(19)

Europäisches
Patentamt

European
Patent Office

Office européen
des brevets

(11) **EP 2 295 543 A1**

(12) **EUROPEAN PATENT APPLICATION**

(43) Date of publication:
**16.03.2011 Bulletin 2011/11**

(51) Int Cl.:
***C12N 7/02*** *(2006.01)*

(21) Application number: **09170077.3**

(22) Date of filing: **11.09.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(71) Applicant: **Max-Planck-Gesellschaft zur Förderung der Wissenschaften e.V. 80539 München (DE)**

(72) Inventor: **The designation of the inventor has not yet been filed**

(74) Representative: **Weiss, Wolfgang et al Weickmann & Weickmann Postfach 860820 81635 München (DE)**

(54) **Method for the preparation of an influenza virus**

(57) The present invention concerns a method for the preparation of influenza viruses. The method includes the manipulation of host cells so that cellular factors that inhibit virus replication are repressed.

**EP 2 295 543 A1**

Printed by Jouve, 75001 PARIS (FR)

**Description**

The present invention relates to a method for the production of a pharmaceutical compositions for the prevention or/and treatment of an influenza virus infection.

Furthermore, the present invention relates to a pharmaceutical composition for the prevention or/and treatment of an influenza virus infection.

In view of the threatening influenza pandemic, there is an acute need to develop and make available lastingly effective drugs. In Germany alone the annual occurrence of influenza causes between 5,000 and 20,000 deaths a year (source: Robert-Koch Institute). The recurring big influenza pandemics are especially feared. The first big pandemic, the so-called "Spanish Flu", cost about 40 million lives in the years 1918-1919 including a high percentage of healthy, middle-aged people. A similar pandemic could be caused by the H5N1 influenza virus (2,3), which at the moment replicates mainly in birds, if acquired mutations enable the virus to be transmitted from person to person. More recently, a novel influenza virus variant has emerged, i.e. the influenza A (H1N1) 'swine flu' strain (4), posing an unpredictable pandemic threat. The probability of a human pandemic has recently grown more acute with the spreading of bird flu (H5N1) worldwide and the infection of domestic animals. It is only a question of time until a highly pathogenic human influenza-recombinant emerges. The methods available at the moment for prophylaxis or therapy of an influenza infection, such as vaccination with viral surface proteins or the use of antiviral drugs (neuraminidase inhibitors or ion channel blockers), have various disadvantages. Already at this early stage resistance is appearing against one of our most effective preparations (Tamiflu), which may make it unsuitable to contain a pandemic. A central problem in the use of vaccines and drugs against influenza is the variability of the pathogen. Up to now the development of effective vaccines has required accurate prediction of the pathogen variant. Drugs directed against viral components can rapidly lose their effectiveness because of mutations of the pathogen.

An area of research which has received little attention up to now is the identification of critical target structures in the host cell. Viruses are dependent on certain cellular proteins to be able to replicate within the host. The knowledge of such cellular factors that are essential for viral replication but dispensable (at least temporarily) for humans could lead to the development of novel drugs. Rough estimates predict about 500 genes in the human genome which are essential for viral multiplication. Of these, 10% at least are probably dispensable temporarily or even permanently for the human organism. Inhibition of these genes and their products, which in contrast to the viral targets are constant in their structure, would enable the development of a new generation of antiviral drugs in the shortest time. Inhibition of such gene products could overcome the development of viral escape mutants that are not longer sensitive to antiviral drugs. Amongst other gene families kinases that are important regulatory proteins within the cell are often hijacked by viruses to manipulate the constitution of the host cell.

Influenza A is a negative-stranded RNA virus that exhibits an array of strategies to facilitate successful survival within mammalian host cells (5). Upon infection, binding of innate immune receptors, such as the cellular protein retinoic acid-inducible gene I (RIG-I), with their cognate ligands triggers the transient expression of dozens of immune and inflammation related genes (6,7). In particular, subsequent induction of type I interferon stimulates the up-regulation of GTPases with intrinsic antiviral activity, such as the myxovirus resistance (Mx) proteins. The antiviral activity of Mx proteins against members of the orthomyxovirus family was first observed in mice (8). The nucleus-located Mx1 protein confers protection against otherwise lethal infections with influenza virus, including strains of the pandemic 1918 and the highly lethal H5N1 influenza viruses (9,10). The human ortholog, MxA, localizes to the cytoplasm and is thought to act by binding and inactivating incoming viral nucleocapsids (11). Interestingly, human MxA reportedly exhibits a protective function in transgenic mice against various RNA viruses (12). To counteract these innate response strategies, influenza viruses employ their NS1 protein; for example, by reducing interferon-β (IFN-β) production or by blocking expression of the antiviral proteins 2'-5' oligoadenylate synthetase (OAS) and protein kinase R (PKR) (13). However, the active suppression of MxA previously observed during influenza A infections *in vitro* and *in vivo* is currently not completely understood.

An intriguing strategy employed by viral agents to regulate their infectious potential is the use of microRNAs (miRNAs); a class of ~22 nt long non-protein-coding short interfering RNA molecules, known as key post-transcriptional regulators of gene expression (14). Viruses with large genomes can encode their own miRNAs to alter host physiology and enhance replication (15). Conversely, the small RNA genome hepatitis C virus can manipulate expression of host cell miR-122 to foster its replication (16).

Common strategies for the production of influenza virus vaccines are based upon influenza virus replication in embryonated hens' eggs or in cell culture. Virus replication in cell culture or embryonated eggs is a time-consuming and expensive procedure. Therefore, it is the problem of the present invention to improve the methods for the influenza vaccine production.

An object of the present invention is a method for the preparation of an influenza virus comprising the steps:

(a) providing a modified cell, a modified embryonated egg or/and a modified non-human organism capable of replicating an influenza virus, wherein the capability of influenza virus replication is increased compared with influenza virus replication in the absence of the modification,

(b) contacting the cell, the embryonated egg or/and the organism of (a) with an influenza virus,

(c) cultivating the cell, the embryonated egg or/and the non-human organism under conditions allowing the replication of the influenza virus, and

(d) isolating the influenza virus or/and at least on component thereof produced in step (c).

From the influenza virus of step (d), a pharmaceutical composition for the prevention or/and treatment of an influenza virus infection may be prepared, optionally together with a pharmaceutically acceptable carrier, adjuvant, diluent or/and additive.

Another object of the present invention is a method for the preparation of a pharmaceutical composition for the prevention or/and treatment of an influenza virus infection, comprising the steps:

(a) providing a modified cell, a modified embryonated egg or/and a modified non-human organism capable of replicating an influenza virus, wherein the capability of influenza virus replication is increased compared with influenza virus replication in the absence of the modification,

(b) contacting the cell, the embryonated egg or/and the organism of (a) with an influenza virus,

(c) cultivating the cell, the embryonated egg or/and the non-human organism under conditions allowing the replication of the influenza virus,

(d) isolating the influenza virus or/and at least one component thereof produced in step (c), and,

(e) preparing the pharmaceutical composition from the influenza virus or/and the components thereof isolated in step (d), optionally together with a pharmaceutically acceptable carrier, adjuvant, diluent or/and additive.

A reference herein to the "method" or "method of the present invention" is a reference to the method for the preparation of an influenza virus and to the method for the preparation of a pharmaceutical composition for the prevention or/and treatment of an influenza virus infection.

The cell employed in step (a) may be any cell capable of being infected with an influenza virus. Cell lines suitable for the production of an influenza virus are known. Preferably the cell is a mammalian cell or an avian cell. Also preferred is a human cell. Also preferred is an epithelial cell, such as a lung epithelial cell. The cell may be a cell line. A suitable lung epithelial cell line is A594. Another suitable cell is the human embryonic kidney cell line 293T. In one embodiment of the present invention, the method of the present invention employs a cell as described herein.

The non-human organism employed in step (a) may be any organism capable of being infected with an influenza virus. Preferably the organism is an organism employed in the production of an influenza vaccine. More preferable, the organism is an embryonated egg, such as an embryonated hen's egg. The person skilled in the art know methods of obtaining such organism. The methods for obtained an embryonated egg by fertilization are known. Inducing influenza virus replication by inoculation with an influenza virus is known. In one embodiment of the present invention, the method of the present invention employs a non-human organism or/and an embryonated egg, as described herein.

Step (a) of the present invention may include the provision of a cell, an embryonated egg or/and a non-human organism modified as described herein, or may include the step of modification.

"Modification of the cell, the embryonated egg or/and non-human organism", as used herein, includes down-regulation or/and upregulation of the expression or/and activity of at least one gene or/and gene product in the cell, the egg or/and the organism.

"Modification of the cell, the embryonated egg or/and the non-human organism", as described herein, may include contacting the cell, the embryonated egg or/and the non-human organism with at least one modulator capable of increasing the influenza virus replication in the cell or/and the organism, compared with influenza virus replication in the absence of the modulator, wherein contacting may be performed before or after step (b), or simultaneously with step (b).

"Modification of the cell, the embryonated egg or/and non-human organism", as described herein, may include the production or/and provision of a recombinant cell, a recombinant embryonated egg or/and recombinant non-human organism, wherein the expression or/and activity of at least one gene or/and gene product is modified so that the capability of the cell, the embryonated egg or/and the non-human organism of replicating an influenza virus is increased compared with influenza virus replication in the absence of the modification.

Preparation of a recombinant cell, a recombinant embryonated egg or/and recombinant non-human organism may include introduction of a nucleic acid molecule into the cell, the embryonated egg or/and the non-human organism, or/and deletion of a nucleic acid sequence in the cell, the egg or/and the organism. The nucleic acid molecule may be incorporated into the genome of the cell, of the embryonated egg or/and of the non-human organism. Thereby, sequences of the cell, the egg or/and the organism may be modified, replaced or/and deleted. The nucleic acid molecule may comprise a sequence heterologous to the cell or/and the organism. Incorporation of the nucleic acid molecule may be performed permanently or transiently. A recombinant embryonated egg or/and recombinant non-human organism may be prepared by manipulation of the germ line. In the context of the present invention, "embryonated egg" in particular refers to the embryo. For instance, "modification of the embryonated egg" is in particular a modification of the embryo.

The person skilled in the art knows methods of introducing a nucleic acid molecule into a cell, an embryonated egg or/and an organism, or/and methods of deletion of a nucleic acid sequence in the cell, the embryonated egg or/and the organism ("recombinant technology", as employed herein). These methods may include transfection employing a suitable vector, such as a plasmid. These methods may also include homologous recombination of the nucleic acid molecule in the genome of the cell or/and the organism. The nucleic acid molecule may also be randomly inserted into the genome of the cell, the embryonated egg or/and the organism.

Table 1a, 1b and 4 describe targets for modulation of influenza virus replication, wherein the targets may be suitable for the modification of the cell, the embryonated egg or/and non-human organism, either by contacting with a modulator, or by recombinant technology, as described herein.

"Modulation" in the context of the present invention may be "activation" or "inhibition".

Examples of genes which upon downregulation increase the influenza virus replication are described in Table 1a. Thus, by increasing expression or/and activity of these genes or/and gene products thereof, the influenza virus replication can be reduced. A decreased expression or/and activity of these genes or/and gene products can be exploited in the method of the present invention by improvement of virus production.

The cell, the embryonated egg or/and non-human organism provided in step (a) may thus be a recombinant cell, a recombinant embryonated egg or/and recombinant non-human organism, wherein the gene expression or/and the activity of a gene selected from Table 1a is downregulated.

Examples of genes which upon downregulation decrease the influenza virus replication are described in Table 1b and 4. Thus, by decreasing expression or/and activity of these genes or/and gene products, the influenza virus replication can be reduced. An increased expression or/and activity of these genes or/and gene products can be exploited in the method of the present invention by improvement of virus production.

The cell, embryonated egg or/and non-human organism provided in step (a) may thus be a recombinant cell, a recombinant embryonated egg or/and recombinant non-human organism, wherein the gene expression or/and the activity of a gene selected from Table 1b and Table 4 is upregulated.

In the context of the present invention, a "target" includes

(a) a nucleotide sequence within a gene or/and a genome, in particular the within a human gene or/and the human genome,
(b) a nucleic acid, or/and a polypeptide encoded by the nucleotide sequence of (a).

The sequence of (a) or/and (b) may be involved in regulation of influenza virus replication in a host cell. The target may be directly or indirectly involved in the regulation of influenza virus replication. In particular, a target is suitable for increasing of influenza virus replication, either by activation of the target or by inhibition of the target.

Examples of targets are genes and partial sequences of genes, such as regulatory sequences. A target according to the present invention also includes a gene product such as RNA, in particular mRNA, tRNA, rRNA, miRNA, piRNA. A target may also include a polypeptide or/and a protein encoded by the target gene. Preferred gene products of a target gene are selected from mRNA, miRNA, polypeptide(s) or/and protein(s) encoded by the target gene. The most preferred gene product is a polypeptide or protein encoded by the target gene. A target protein or a target polypeptide may be posttranslationally modified or not.

A "Gene product" as used herein may be selected from RNA, in particular mRNA, tRNA, rRNA, miRNA, and piRNA. A "Gene product" may also be a polypeptide or/and a protein encoded by said gene.

In the context of the present invention, "activity" of the gene or/and gene product includes transcription, translation, post translational modification, post transcriptional regulation, modulation of the activity of the gene or/and gene product. The activity may be modulated by ligand binding, which ligand may be an activator or inhibitor. The activity may also be modulated by an miRNA molecule, an shRNA molecule, an siRNA molecule, an antisense nucleic acid, a decoy nucleic acid or/and any other nucleic acid, as described herein. The activity of the gene may also be modulated by recombinant technology, as described herein. Modulation may also be performed by a small molecule, an antibody, an aptamer, or/and a spiegelmer (mirror image aptamer).

The method of the present invention may be suitable for the production of a pharmaceutical composition for the prevention or/and treatment of an infection with any influenza virus.

The influenza virus may be any influenza virus suitable for vaccine production. The influenza virus may be an influenza A virus. The influenza A virus may be selected from influenza A viruses isolated so far from avian and mammalian organisms. In particular, the influenza A virus may be selected from H1N1, H1N2, H1N3, H1N4, H1N5, H1N6, H1N7, H1N9, H2N1, H2N2, H2N3, H2N4, H2N5, H2N7, H2N8, H2N9, H3N1, H3N2, H3N3, H3N4, H3N5, H3N6, H3N8, H4N1, H4N2, H4N3, H4N4, H4N5, H4N6, H4N8, H4N9, H5N1, H5N2,H5N3,H5N6,H5N7,H5N8,H5N9,H6N1,H6N2,H6N3,H6N4,H6N5, H6N6, H6N7, H6N8, H6N9, H7N1, H7N2, H7N3, H7N4, H7N5, H7N7, H7N8, H7N9, H9N1, H9N2, H9N3, H9N5, H9N6, H9N7, H9N8, H10N1, H10N3, H10N4, H10N6, H10N7, H10N8, H10N9, H11N2, H11N3, H11N6, H11N9, H12N1, H12N4, H12N5, H12N9, H13N2, H13N6, H13N8, H13N9, H14N5, H15N2, H15N8, H15N9 and H16N3. More particularly, the influenza A virus is selected from H1N1, H3N2, H7N7, H5N1. Even more particularly, the influenza A virus is strain Puerto Rico/8/34, the avian influenza virus isolate H5N1, the avian influenza strain A/FPV/Bratislava/79 (H7N7), strain A/WSN/33 (H1 N1), strain A/Panama/99 (H3N2), or a swine flu strain H1 N1.

The influenza virus may be an influenza B virus. In particular, the influenza B virus may be selected from representatives of the Victoria line and representatives of the Yamagata line.

In the method of the present invention, modification of the cell or/and organism according to step (a) to increase the influenza virus replication includes modulating the expression of a gene selected from Table 1A, Table 1B, and Table 4, or/and a gene product thereof. In particular, modification of the cell or/and organism may activate the expression of a gene selected from Table 1B and Table 4 or/and a gene product thereof, or modification of the cell or/and organism may inhibit the expression of a gene selected from Table 1A or/and a gene product thereof. Modulating the expression may be performed by contacting the cell, the embryonated egg or/and the organism with a modulator as described herein, or may be performed in a recombinant cell, a recombinant embryonated egg or/and recombinant organism, the production of which is described herein.

In the method of the present invention, modification of the cell, the embryonated egg or/and the non-human organism may include inhibition of the expression or/and gene product activity of the MxA gene. The at least one modulator capable of increasing the influenza virus replication may be capable of inhibiting expression or/and gene product activity of the MxA gene. The cell, the embryonated egg or/and the non-human organism, as described herein, may be recombinantly modified so that the expression or/and gene product activity of the MxA gene is inhibited by at least one modulator selected from miR-141, miR-141*, miR-200c, miR-200c*, precursors thereof, derivatives thereof, antisense nucleic acids, siRNAs, shRNAs and influenza virus sequences. In particular, in the method of the present invention, the MxA gene is post-translationally inhibited by at least one modulator selected from miR-141, miR-141*, miR-200c, miR-200c*, precursors thereof, derivatives thereof, antisense nucleic acids, siRNAs, shRNAs and influenza virus sequences.

In the method of the present invention, miR-141, miR-141*, miR-200c, miR-200c*, or/and precursors thereof may be over-expressed in the cell, in the embryonated egg or/and in the non-human organism. Over-expression may be transiently or permanently.

On the RNA level, inhibition may be performed by antisense nucleic acid, siRNA, shRNA, a decoy nucleic acid or/and a derivative thereof. On the level of the MxA polypeptide, inhibition may be performed by a small molecule, an antibody, an aptamer, a spiegelmer (mirror image aptamer).

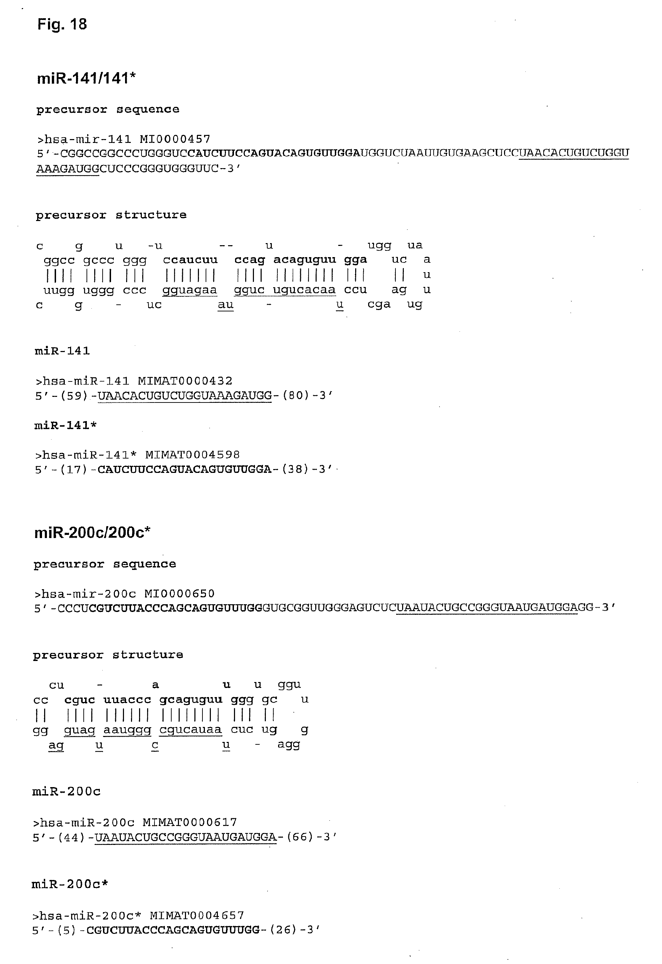

The sequences of miR-141, miR-141*, miR-200c, and miR-200c*, precursors thereof and the hairpin structure of the precursor are described in Fig. 18. The miR-141 and miR-141* may be co-expressed in a cell by a single precursor. The miR-141 and miR-141* comprise complementary sequences which may form the hairpin structure of the precursor. The miR-200c and miR-200c* may be co-expressed in a cell by a single precursor. The miR-200c and miR-200c* comprise complementary sequences which may form the hairpin structure of the precursor.

In the context of the present invention, influenza A sequences have been identified having a high degree of identity to the MxA genes. Thus, in the method of the present invention, the MxA gene may be inhibited or/and the virus replication may be activated by at least one microRNA or/and at least one antisense RNA comprising an influenza A virus sequence or/and a sequence derived from an influenza A virus sequence.

Modification of the cell, of the embryonated egg or/and of the non-human organism may include the inhibition of the expression or/and gene product activity of a gene, wherein the gene comprises

(a) a nucleotide sequence selected from the sequences of Table 1A,
(b) a fragment of the sequence of (a) having a length of at least 70%, at least 80%, at least 90%, at least 95%, at least 98%, at least 99% of the sequence of (a),
(c) a sequence which is at least 70 %, preferably at least 80%, more preferably at least 90% identical to the sequence of (a) or/and (b), or/and
(d) a sequence complementary to a sequence of (a), (b) or/and (c).

Modification of the cell, the embryonated egg or/and the non-human organism may include the activation of the expression or/and gene product activity of a gene, wherein the gene comprises

(i) a nucleotide sequence selected from the sequences of Table 1B and Table 4,
(ii) a fragment of the sequence of (i) having a length of at least 70%, at least 80%, at least 90%, at least 95%, at least 98%, at least 99% of the sequence of (i),
(iii) a sequence which is at least 70 %, preferably at least 80%, more preferably at least 90% identical to the sequence of (i) or/and (ii), or/and
(iv) a sequence complementary to a sequence of (i), (ii) or/and (iii).

The at least one modulator capable of increasing the influenza virus replication may be capable of inhibiting expression or/and gene product activity of a gene, wherein the gene comprises

(a) a nucleotide sequence selected from the sequences of Table 1A,
(b) a fragment of the sequence of (a) having a length of at least 70%, at least 80%, at least 90%, at least 95%, at least 98%, at least 99% of the sequence of (a),
(c) a sequence which is at least 70 %, preferably at least 80%, more preferably at least 90% identical to the sequence of (a) or/and (b), or/and
(d) a sequence complementary to a sequence of (a), (b) or/and (c).

The at least one modulator capable of increasing the influenza virus replication may be capable of activating the expression or/and gene product activity of a gene, wherein the gene comprises

(i) a nucleotide sequence selected from the sequences of Table 1B and Table 4,
(ii) a fragment of the sequence of (i) having a length of at least 70%, at least 80%, at least 90%, at least 95%, at least 98%, at least 99% of the sequence of (i),
(iii) a sequence which is at least 70 %, preferably at least 80%, more preferably at least 90% identical to the sequence of (i) or/and (ii), or/and
(iv) a sequence complementary to a sequence of (i), (ii) or/and (iii).

The cell, the embryonated egg or/and non-human organism may be recombinantly modified, as described herein, so that expression or/and gene product activity of a gene is inhibited, wherein the gene comprises

(a) a nucleotide sequence selected from the sequences of Table 1A,
(b) a fragment of the sequence of (a) having a length of at least 70%, at least 80%, at least 90%, at least 95%, at least 98%, at least 99% of the sequence of (a),
(c) a sequence which is at least 70 %, preferably at least 80%, more preferably at least 90% identical to the sequence of (a) or/and (b), or/and
(d) a sequence complementary to a sequence of (a), (b) or/and (c).

The cell, the embryonated egg or/and non-human organism may be recombinantly modified, as described herein, so that expression or/and gene product activity of a gene is activated, wherein the gene comprises

(i) a nucleotide sequence selected from the sequences of Table 1B and Table 4,
(ii) a fragment of the sequence of (i) having a length of at least 70%, at least 80%, at least 90%, at least 95%, at least 98%, at least 99% of the sequence of (i),
(iii) a sequence which is at least 70 %, preferably at least 80%, more preferably at least 90% identical to the sequence of (i) or/and (ii), or/and
(iv) a sequence complementary to a sequence of (i), (ii) or/and (iii).

Modification (including modulation and recombinant modification) may be a modification of a kinase or/and a modulator of a kinase binding polypeptide, wherein the at least one kinase or/and kinase binding polypeptide is encoded by a nucleic acid or/and gene selected from Table 1A and Table 1B.
In the method of the present invention, the at least one modulator capable of increasing the influenza virus replication may be an activator comprising:

(i) a nucleotide sequence selected from Table 1B and Table 4,
(ii) a fragment of the sequence of (a) having a length of at least 70%, at least 80%, at least 90%, at least 95%, at least 98%, at least 99% of the sequence of (i),
(iii) a sequence which is at least 70 %, preferably at least 80%, more preferably at least 90% identical to the sequence of (i) or/and (ii), or/and
(iv) a sequence complementary to a sequence of (i), (ii) or/and (iii).

The at least one activator may be capable of activating expression or/and gene product activity of a gene comprising sequence (i), (ii) (iii) or/and (iv).

In the method of the present invention, the at least one modulator capable of increasing the influenza virus replication may be an inhibitor comprising:

(a) a nucleotide sequence selected from Table 1A,
(b) a fragment of the sequence of (a) having a length of at least 70%, at least 80%, at least 90%, at least 95%, at least 98%, at least 99% of the sequence of (a),
(c) a sequence which is at least 70 %, preferably at least 80%, more preferably at least 90% identical to the sequence of (a) or/and (b), or/and
(d) a sequence complementary to a sequence of (a), (b) or/and (c).

The at least one inhibitor may be capable of inhibiting expression or/and gene product activity of a gene comprising sequence (a), (b) (c) or/and (d). The at least modulator of influenza virus replication employed in the method of the present invention of the present invention may be selected from the group consisting of nucleic acids, nucleic acid analogues such as ribozymes, peptides, polypeptides, antibodies, aptamers, spiegelmers, small molecules and decoy nucleic acids.

The modulator of influenza virus replication may be a compound having a molecular weight smaller than 1000 Dalton or smaller than 500 Dalton. In the context of the present invention, "small molecule" refers to a compound having a molecular weight smaller than 1000 Dalton or smaller than 500 Dalton. In the method of the present invention, the small molecule may be directed against a polypeptide comprising

(a) an amino acid sequence encoded by a nucleic acid or/and gene selected from Table 1A, Table 1B, and Table 4,
(b) a fragment of the sequence of (a) having a length of at least 70%, at least 80%, at least 90%, at least 95%, at least 98%, at least 99% of the sequence of (a), or/and
(c) an amino acid sequence which is at least 70%, preferably at least 80%, more preferably at least 90% identical to the sequence of (a).

The modulator of the present invention preferably comprises a nucleic acid, wherein the nucleic acid comprises a nucleotide sequence selected from the sequences of Table 2 and Table 4 and fragments thereof.

Preferably, the nucleic acid is selected from

(a) RNA, analogues and derivatives thereof,
(b) DNA, analogues and derivatives thereof , and
(c) combinations of (a) and (b).

Suitable inhibitors are RNA molecules capable of RNA interference. The modulator of the present invention, in particular the inhibitor of the present invention may comprise

(i) an RNA molecule capable of RNA interference, such as siRNA or/and shRNA,
(ii) a miRNA,
(iii) a precursor of the RNA molecule (i) or/and (ii),
(iv) a fragment of the RNA molecule (i), (ii) or/and (iii),
(v) a derivative of the RNA molecule of (i), (ii) (iii) or/and (iv), or/and
(vi) a DNA molecule encoding the RNA molecule of (i), (ii) (iii) or/and (iv).

A preferred modulator is

(i) a miRNA,
(ii) a precursor of the RNA molecule (i), or/and
(iii) a DNA molecule encoding the RNA molecule (i) or/and the precursor (ii).

Yet another preferred modulator is

(i) an RNA molecule capable of RNA interference, such as siRNA or/and shRNA,
(ii) a precursor of the RNA molecule (i), or/and
(iii) a DNA molecule encoding the RNA molecule (i) or/and the precursor (ii).

RNA molecules capable of RNA interference are described in WO 02/44321 the disclosure of which is included herein by reference. MicroRNAs are described in Bartel D (Cell 136:215-233, 2009), the disclosure of which is included herein by reference.

The RNA molecule of the present invention may be a double-stranded RNA molecule, preferably a double-stranded siRNA molecule with or without a single-stranded overhang alone at one end or at both ends. The siRNA molecule may comprise at least one nucleotide analogue or/and deoxyribonucleotide.

The RNA molecule of the present invention may be an shRNA molecule. The shRNA molecule may comprise at least one nucleotide analogue or/and deoxyribonucleotide.

The DNA molecule as employed in the present invention may be a vector.

The nucleic acid employed in the present invention may be an antisense nucleic acid or a DNA encoding the antisense nucleic acid.

The nucleic acid or/and nucleic acid fragment employed in the present invention may have a length of at least 15, preferably at least 17, more preferably at least 19, most preferably at least 21 nucleotides. The nucleic acid or/and the nucleic acid fragment may have a length of at the maximum 29, preferably at the maximum 27, more preferably at the maximum 25, especially more preferably at the maximum 23, most preferably at the maximum 22 nucleotides.

The nucleic acid employed in the present invention may be a microRNA (miRNA), a precursor, a fragment, or a derivative thereof. The miRNA may have the length of the nucleic acid as described herein. The miRNA may in particular have a length of about 22 nucleotides, more preferably 22 nucleotides.

The modulator of the present invention may comprise an antibody, wherein the antibody may be directed against a kinase or/and kinase binding polypeptide.

Preferably the antibody is directed against a kinase or/and kinase binding polypeptide comprising

(a) an amino acid sequence encoded by a nucleic acid or/and gene selected from Table 1A, and Table 1B,
(b) a fragment of the sequence of (a) having a length of at least 70%, at least 80%, at least 90%, at least 95%, at least 98%, at least 99% of the sequence of (a), or/and
(c) an amino acid sequence which is at least 70%, preferably at least 80%, more preferably at least 90% identical to the sequence of (a) or/and (b).

In another preferred embodiment, the antibody is directed against a polypeptide comprising

(a) an amino acid sequence encoded by a nucleic acid or/and gene selected from Table 4,
(b) a fragment of the sequence of (a) having a length of at least 70%, at least 80%, at least 90%, at least 95%, at least 98%, at least 99% of the sequence of (a), or/and
(c) an amino acid sequence which is at least 70%, preferably at least 80%, more preferably at least 90% identical to the sequence of (a) or/and (b).

The antibody of the present invention may be a monoclonal or polyclonal antibody, a chimeric antibody, a chimeric single chain antibody, a Fab fragment or a fragment produced by a Fab expression library.

Techniques of preparing antibodies of the present invention are known by a skilled person. Monoclonal antibodies may be prepared by the human B-cell hybridoma technique or by the EBV-hybridoma technique (Köhler et al., 1975, Nature 256:495-497, Kozbor et al., 1985, J. Immunol. Methods 81,31-42, Cote et al., PNAS, 80:2026-2030, Cole et al., 1984, Mol. Cell Biol. 62:109-120). Chimeric antibodies (mouse/human) may be prepared by carrying out the methods of Morrison et al. (1984, PNAS, 81:6851-6855), Neuberger et al. (1984, 312:604-608) and Takeda et al. (1985, Nature 314:452-454). Single chain antibodies may be prepared by techniques known by a person skilled in the art.

Recombinant immunoglobulin libraries (Orlandi et al, 1989, PNAS 86:3833-3837, Winter et al., 1991, Nature 349:293-299) may be screened to obtain an antibody of the present invention. A random combinatory immunoglobulin library (Burton, 1991, PNAS, 88:11120-11123) may be used to generate an antibody with a related specifity having a different idiotypic composition.

Another strategy for antibody production is the in vivo stimulation of the lymphocyte population.

Furthermore, antibody fragments (containing $F(ab')_2$ fragments) of the present invention can be prepared by protease digestion of an antibody, e.g. by pepsin. Reducing the disulfide bonding of such $F(ab')_2$ fragments results in the Fab fragments. In another approach, the Fab fragment may be directly obtained from an Fab expression library (Huse et al., 1989, Science 254:1275-1281).

Polyclonal antibodies of the present invention may be prepared employing an amino acid sequence encoded by a nucleic acid or/and gene selected from Table 1A and Table 1B or immunogenic fragments thereof as antigen by standard immunization protocols of a host, e.g. a horse, a goat, a rabbit, a human, etc., which standard immunization protocols are known by a person skilled in the art.

The antibody may be an antibody specific for a gene product of a target gene, in particular an antibody specific for a polypeptide or protein encoded by a target gene.

Aptamers and spiegelmers share binding properties with antibodies. Aptamers and spiegelmers are designed for specifically binding a target molecule.

The nucleic acid or the present invention may be selected from (a) aptamers, (b) DNA molecules encoding an aptamer, and (c) spiegelmers.

The skilled person knows aptamers. In the present invention, an "aptamer" may be a nucleic acid that can bind to a target molecule. Aptamers can be identified in combinational nucleic acid libraries (e.g. comprising $>10^{15}$ different nucleic acid sequences) by binding to the immobilized target molecule and subsequent identification of the nucleic acid sequence. This selection procedure may be repeated one or more times in order to improve the specificity. The person skilled in the art knows suitable methods for producing an aptamer specifically binding a predetermined molecule. The aptamer may have a length of a nucleic acid as described herein. The aptamer may have a length of up to 300, up to 200, up to 100, or up to 50 nucleotides. The aptamer may have a length of at least 10, at least 15, or at least 20 nucleotides. The aptamer may be encoded by a DNA molecule. The aptamer may comprise at least one nucleotide analogue or/and at least one nucleotide derivatives, as described herein.

The skilled person knows spiegelmers. In the present invention, a "spiegelmer" may be a nucleic acid that can bind to a target molecule. The person skilled in the art knows suitable methods for production of a spiegelmer specifically binding a predetermined molecule. The spiegelmer comprises nucleotides capable of forming bindings which are nuclease resistant. Preferably the spiegelmer comprises L nucleotides. More preferably, the spiegelmer is an L-oligonucleotide. The spiegelmer may have a length of a nucleic acid as described herein. The spiegelmer may have a length of up to 300, up to 200, up to 100, or up to 50 nucleotides. The spiegelmer may have a length of at least 10, at least 15, or at least 20 nucleotides. The spiegelmer may comprise at least one nucleotide analogue or/and at least one nucleotide derivatives, as described herein.

The skilled person knows decoy nucleic acids. In the present invention, a "decoy" or "decoy nucleic acid" may be a nucleic acid capable of specifically binding a nucleic acid binding protein, such as a DNA binding protein. The decoy nucleic acid may be a DNA molecule, preferably a double stranded DNA molecule. The decoy nucleic acid comprises a sequence termed "recognition sequence" which can be recognized by a nucleic acid binding protein. The recognition sequence preferably has a length of at least 3, at least 5, or at least 10 nucleotides. The recognition sequence preferably has a length of up to 15, up to 20, or up to 25 nucleotides. Examples of nucleic acid binding proteins are transcription factors, which preferably bind double stranded DNA molecules. Transfection of a cell, an embryonated egg, or/and a non-human animal, as described herein, with a decoy nucleic acid may result in reduction of the activity of the nucleic acid binding protein to which the decoy nucleic acid binds. The decoy nucleic acid as described herein may have a length of nucleic acid molecules as described herein. The decoy nucleic acid molecule may have a length of up to 300, up to 200, up to 100, up to 50, up to 40, or up to 30 nucleotides. The decoy nucleic may have a length of at least 3, at least 5, at least 10, at least 15, or at least 20 nucleotides. The decoy nucleic acid may be encoded by a DNA molecule. The decoy nucleic acid may comprise at least one nucleotide analogue or/and at least one nucleotide derivatives, as described herein.

An RNA or/and a DNA molecule as described herein may comprise at least one nucleotide analogue. As used herein, "nucleotide analogue" may refer to building blocks suitable for a modification in the backbone, at least one ribose, at least one base, the 3' end or/and the 5' end in the nucleic acid. Backbone modifications include phosphorothioate linkage (PTs); peptide nucleic acids (PNAs); morpholino nucleic acids; phosphoroamidate-linked DNAs (PAs), which contain backbone nitrogen. Ribose modifications include Locked nucleic acids (LNA) e.g. with methylene bridge joining the 2' oxygen of ribose with the 4' carbon; 2'-deoxy-2'-fluorouridine; 2'-fluoro (2'-F); 2'-O-alkyl-RNAs (2-O-RNAs), e.g. 2'-O-methyl (2'-O-Me), 2'-O-methoxyethyl (2'-O-MOE). A modified base may be 2'-fluoropyrimidine. 5' modifications include 5'-TAMRA-hexyl linker, 5'-Phosphate, 5'-Amino, 5'-Amino-C6 linker, 5'-Biotin, 5'-Fluorescein, 5'-Tetrachloro-fluorescein, 5'-Pyrene, 5'-Thiol, 5'-Amino, (12 Carbon) linker, 5'-Dabcyl, 5'-Cholesterol, 5'-DY547 (Cy3™ alternate). 3' end modifications include 3'-inverted deoxythymidine, 3'-puromycin, 3'-dideoxy-cytidine, 3'-cholesterol, 3'-amino modifier (6 atom), 3'-DY547 (Cy3™ alternate).

In particular, nucleotide analogues as described herein are suitable building blocks in siRNA, antisense RNA, and aptamers.

As used herein, "nucleic acid analogue" refers to nucleic acids comprising at least one nucleotide analogue as described herein. Further, a nucleic acid molecule as described herein may comprise at least one deoxyribonucleotide and at least one ribonucleotide.

An RNA molecule of the present invention may comprise at least one deoxyribonucleotide or/and at least one nucleotide analogue. A DNA molecule of the present invention may comprise at least one ribonucleotide or/and at least one nucleotide analogue.

Derivatives as described herein refers to chemically modified compounds. Derivatives of nucleic acid molecules as described herein refers to nucleic acid molecules which are chemically modified. A modification may be introduced into the nucleic acid molecule, or/and into at least one nucleic acid building block employed in the production of the nucleic acid.

In the present invention the term "fragment" refers to fragments of nucleic acids, polypeptides and proteins.

"Fragment" also refers to partial sequences of nucleic acids, polypeptides and proteins.

Fragments of polypeptides or/and peptides as employed in the present invention, in particular fragments of an amino acid sequence encoded by a nucleic acid or/and gene selected from Table 1A, Table 1B and Table 4 may have a length of at least 5 amino acid residues, at least 10, or at least 20 amino acid residues. The length of said fragments may be 200 amino acid residues at the maximum, 100 amino acid residues at the maximum, 60 amino acid residues at the maximum, or 40 amino acid residues at the maximum.

A fragment of an amino acid sequence as described herein may have a length of at least 70%, at least 80%, at least 90%, at least 95%, at least 98%, at least 99% of the sequence.

A fragment of a nucleotide sequence as described herein may have a length of at least 70%, at least 80%, at least 90%, at least 95%, at least 98%, at least 99% of the sequence.

A fragment of a nucleic acid molecule given in Tables 1A, 1B and 4 may have a length of up to 1000, up to 2000, or up to 3000 nucleotides. A nucleic acid fragment may have a length of an siRNA molecule, an miRNA molecule, an aptamer, a spiegelmer, or/and a decoy as described herein. A nucleic acid fragment may also have a length of up to 300, up to 200, up to 100, or up to 50 nucleotides. A nucleic acid fragment may also have a length of at least 3, at least 5, at least 10, at least 15, or at least 20 nucleotides.

In the method of the present invention, modulating the expression of a gene may be downregulation or upregulation, in particular of transcription or/and translation.

It can easily be determined by a skilled person if a gene is upregulated or downregulated. In the context of the present invention, upregulation (activation) of gene expression may be an upregulation by a factor of at least 2, preferably at least 4. Downregulation (inhibition) in the context of the present invention may be a reduction of gene expression by a factor of at least 2, preferably at least 4. Most preferred is essentially complete inhibition of gene expression, e.g. by RNA interference.

Modulation of the activity of a gene may be decreasing or increasing of the activity. "Inhibition of the activity" may be a decrease of activity of a gene or gene product by a factor of at least 2, preferably at least 4. "Inhibition of the activity" includes essentially complete inhibition of activity. "Activation of the activity" may be an increase of activity of a gene or gene product by a factor of at least 2, preferably at least 4.

In the present invention, specific embodiments of the methods, cells, organisms, and pharmaceutical compositions described herein refer to any individual gene, nucleic acid sequence or/and gene product described in the present application. In a specific embodiment, an individual gene is selected from the genes described in Table 1 and 4. In another specific embodiment, an individual gene produced is selected from the gene products produced by the genes described in Table 1 and 4. In yet another specific embodiment, an individual nucleic acid sequences is selected from the nucleic acid molecules described in Table 1, 2, and 4. Further specific embodiment refer to any combination of genes, gene products and nucleic acid molecules described in the Tables 1, 2, 3, and 4. Table 3 refers to specific combinations of nucleic acid molecules.

Specific embodiments of the present invention refer to the MxA gene, the MxA polypeptide, and fragments thereof. Further specific embodiments refer to miR-141, miR-141*, miR-200c, miR-200c*, and precursors thereof, and DNA molecules encoding miR-141, miR-141*, miR-200c, miR-200c* or/and precursors thereof.

Modification may be performed by a single nucleic acid species or by a combination of nucleic acids comprising 2, 3 4, 5, 6 or even more different nucleic acid species, which may be selected from Tables 1 a, 1b or/and 2 and fragments thereof. Preferred combinations are described in Table 3 (also referred herein as "pools"). Table 3 includes combinations of at least two kinase or/and kinase binding polypeptide genes. It is also preferred that the combination modifies the expression of a single gene, for instance selected from Table 1a and 1b. A combination of two nucleic acid species is preferred. More preferred is a combination of two nucleic acids selected from Table 2. Even more preferred is a combination of two nucleic acids selected from the specific combinations disclosed in Table 2, wherein the two nucleic acids modify the expression of a single gene.

Modification, in particular modulation, may be a knock-down performed by RNA interference. The nucleic acid or the combination of nucleic acid species may be an siRNA, which may comprise a sequence selected from the sequences of Table 2 and Table 4 and fragments thereof. It is preferred that the combination knocks down a single gene, for instance selected from Table 1b and Table 4. A combination of two siRNA species is preferred, which may be selected from those sequences of Table 2, which are derived from genes of Table 1b, and the sequences of Table 4, wherein the combination preferably knocks down a single gene.

"Activation of a gene or/and gene product" or "inhibition of a gene or/and gene product" by recombinant technology, which may be employed in step (a) of the present invention, may include any suitable method the person skilled in the art knows.

Preferred methods of activation of a gene of interest or/and the gene product thereof may be selected from

- introducing at least one further copy of the gene to be activated into the cell or/and organism, either permanently or transiently,

- increasing the transcription,
- introducing a strong promoter into the gene, e.g. a CMV promoter,
- introducing a suitable enhancer,
- inhibition of trancriptionally active microRNA, wherein the microRNA inhibits the activity of the gene to be activated, wherein inhibition may be performed by a suitable nucleic acid molecule,
- deletion of a miRNA binding site,
- improvement of RNA processing including exportation from the nucleus, e.g. by 3' terminally introducing post-transcriptional regulatory elements, e.g. from hepadna viruses, or by 3' terminally introducing of one or more constitutive transport elements, e.g. from type D retroviruses, or/and by employing an intron which can be spliced,
- improvement of translation by improvement of ribosomal binding and optimisation of the coding sequence or/and the 3' UTR, e.g. by deletion of cryptic splicing sites, optimisation of GC content, deletion of killer motives and repeats, optimisation of the structure.

Preferred methods of inhibition of a gene of interest or/and the gene product thereof may be selected from

- deleting at least one further copy of the gene to be inhibited in the cell or/and organism, wherein the gene is deleted completely or partially. For instance, the regulatory sequences or/and the coding sequences are deleted, completely or partially,
- decreasing the transcription,
- deleting an enhancer, if present,
- introduction or/and activation of a trancriptionally active microRNA, wherein the microRNA inhibits the activity of the gene to be inhibited, wherein activation may be an activation of an endogeneous microRNA coding sequence, and introduction may be introduction of an exogeneous microRNA molecule,
- introducing of an miRNA binding site,
- reducing RNA processing including exportation from the nucleus, by deletion or/and modification of 3' terminally introducing post-transcriptional regulatory elements or 3' terminally introducing of one or more constitutive transport elements, if present, or by altering the intron-exon structure,
- reducing translation by modification of ribosomal binding and the coding sequence or/and the 3' UTR, e.g. by introducing of cryptic splicing sites, altering the GC content, introducing of killer motives and repeats.

The gene employed in the various embodiments of the present invention may be selected from any of the Tables 1A, 1B, 2, or 4, or any combination thereof.

Contacting the cell or/and the organism according to step (b) with an influenza virus is known. In the case the non-human organism is an embryonated egg, the skilled person knows suitable methods of inoculating the egg with an influenza virus, for instance at a defined interval after fertilization. Known inoculation techniques may also be applied for administration of the modulator to the embryonated egg or/and for recombinant modification of the embryonated egg.

The skilled person knows methods according to step (c) of cultivating the cell, the embryonated egg or/and the non-human organism under conditions allowing the replication of the influenza virus. Suitable cell culture methods may be applied. In the case the non-human organism is an embryonated egg, the skilled person knows suitable methods, including incubation at elevated temperature, to allow influenza virus replication.

Isolating the influenza virus or/and the components thereof according to step (d) refers to any isolation procedure for viruses or/and components thereof known by a person skilled in the art. "Isolation" includes production of essentially pure or crude preparations or formulations of the virus or/and components thereof. Components of the virus include viral proteins, polypeptids, and nucleic acids encoding viral proteins or/and polypeptides. The life virus may also be isolated.

The person skilled in the art knows methods of preparation of the pharmaceutical composition according to step (e), optionally together with a pharmaceutically acceptable carrier, adjuvant, diluent or/and additive. The pharmaceutical composition produced by the method of the present invention may be an immunogenic composition. The pharmaceutical composition produced by the method of the present invention may also be a vaccine.

Yet another subject of the present invention is a pharmaceutical composition comprising an inhibitor of the expression or/and gene product activity of the MxA gene, optionally together with a pharmaceutically acceptable carrier, adjuvant, diluent or/and additive. The pharmaceutical composition preferably comprises at least one inhibitor selected from miR-141, miR-141*, miR-200c, miR-200c*, precursors and derivatives thereof, optionally together with a pharmaceutically acceptable carrier, adjuvant, diluent or/and additive. The inhibitor may also be selected from antisense nucleic acids, siRNAs, shRNAs, and small molecules.

Yet another subject of the present invention is a pharmaceutical composition comprising an activator of the expression or/and gene product activity of the MxA gene, optionally together with a pharmaceutically acceptable carrier, adjuvant, diluent or/and additive. The pharmaceutical composition preferably comprises at least one inhibitor capable of inhibiting the activity of an miRNA selected from miR-141, miR-141*, miR-200c, miR-200c*, and precursors thereof, optionally together with a pharmaceutically acceptable carrier, adjuvant, diluent or/and additive.

The inhibitor capable of inhibiting the activity of an miRNA selected from miR-141, miR-141*, miR-200c, miR-200c*, and precursors thereof is preferably selected form aptamers, spiegelmers and decoy nucleic acids. Aptamers being preferred inhibitors in the pharmaceutical composition are capable of inhibiting the activity of miR-141, miR-141*, miR-200c or/and miR-200c* and precursors thereof. Spiegelmers being preferred inhibitors in the pharmaceutical composition are capable of inhibiting the activity of miR-141, miR-141*, miR-200c or/and miR-200c* and precursors thereof. Decoy nucleic acid molecules being preferred inhibitors in the pharmaceutical composition comprise a sequence capable of binding a transcription factor involved in the transcription of miR-141, miR-141*, miR-200c or/and miR-200c* and precursors thereof.

The pharmaceutical composition as described herein (either produced by the method of the present invention, or the composition comprising an activator of the expression or/and gene product activity of the MxA gene) is preferably for use in human or veterinary medicine. The pharmaceutical composition is preferably for use for the prevention, alleviation or/and treatment of an influenza virus infection.

The carrier in the pharmaceutical composition may comprise a delivery system. The person skilled in the art knows delivery systems suitable for the pharmaceutical composition of the present invention. The pharmaceutical composition may be delivered in the form of a naked nucleic acid, in combination with viral vectors, non viral vectors including liposomes, nanoparticles or/and polymers. The pharmaceutical composition or/and the nucleic acid may be delivered by electroporation.

Naked nucleic acids include RNA, modified RNA, DNA, modified DNA, RNA-DNA-hybrids, aptamer fusions, plasmid DNA, minicircles, transposons.

Viral vectors include poxviruses, adenoviruses, adeno-associated viruses, vesicular stomatitis viruses, alphaviruses, measles viruses, polioviruses, hepatitis B viruses, retroviruses, and lentiviruses.

Liposomes include stable nucleic acid-lipid particles (SNALP), cationic liposomes, cationic cardiolipin analogue-based liposomes, neutral liposomes, liposome-polycation-DNA, cationic immunoliposomes, immunoliposomes, liposomes containing lipophilic derivatives of cholesterol, lauric acid and lithocholic acid. Examples of compounds suitable for liposome formation are 1,2-dilauroyl-sn-glycero-3-phosphoethanolamine (DLPE); 1,2-dioleoyl-sn-glycero-3-phospho-L-serine (DOPS); cholesterol (CHOL); 1,2-dioleoyl-sn-glycero-3-phosphocholine (DOPC).

Nanoparticles include $CaCO_3$ nanoparticles, chitosan-coated nanoparticle, folated lipid nanoparticle, nanosized nucleic acid carriers.

Polymers include polyethylenimines (PEI), polyester amines (PEA), polyethyleneglycol(PEG)-oligoconjugates, PEG liposomes, polymeric nanospheres.

The pharmaceutical composition may be delivered in combination with atelocollagen, carbon nanotubes, cyclodextrin-containing polycations, fusion proteins (e.g. protamine-antibody conjugates).

An activator of the expression or/and gene product activity of the MxA gene, or/and an inhibitor capable of inhibiting the activity of an miRNA selected from miR-141, miR-141*, miR-200c, miR-200c*, and precursors thereof may be used for the manufacture of a pharmaceutical composition for prevention, alleviation or/and treatment of an influenza virus infection. Delivery systems and delivery methods as described herein may be used.

Another subject of the present invention is the use of an activator of the expression or/and gene product activity of the MxA gene or/and an inhibitor of capable of inhibiting the activity of an miRNA selected from miR-141, miR-141*, miR-200c, miR-200c*, and precursors thereof, for the prevention, alleviation or/and treatment of an influenza virus infection. Delivery systems and delivery methods as described herein may be used.

Yet another subject of the present invention is a method of prevention, alleviation or/and treatment of an influenza virus infection, comprising administering to a subject in need thereof a therapeutically effective amount of an activator of the expression or/and gene product activity of the MxA gene or/and an inhibitor capable of inhibiting the activity of an miRNA selected from miR-141, miR-141*, miR-200c, miR-200c*, and precursors thereof. In the method of prevention, alleviation or/and treatment of an influenza virus infection, delivery systems and delivery methods as described herein may be used.

Yet another subject of the present invention is a recombinant cell produced according to step (a) of the method of the present invention, as described herein.

Yet another subject of the present invention is a recombinant non-human organism produced according to step (a) of the method of the present invention, as described herein.

Yet another subject of the present invention is a recombinant embryonated egg produced according to step (a) of the method of the present invention, as described herein. The recombinant embryonated egg is preferably a recombinant embryonated hen's egg.

The invention is further illustrated by the following figures, tables and examples.

**Figure and Table legends:**

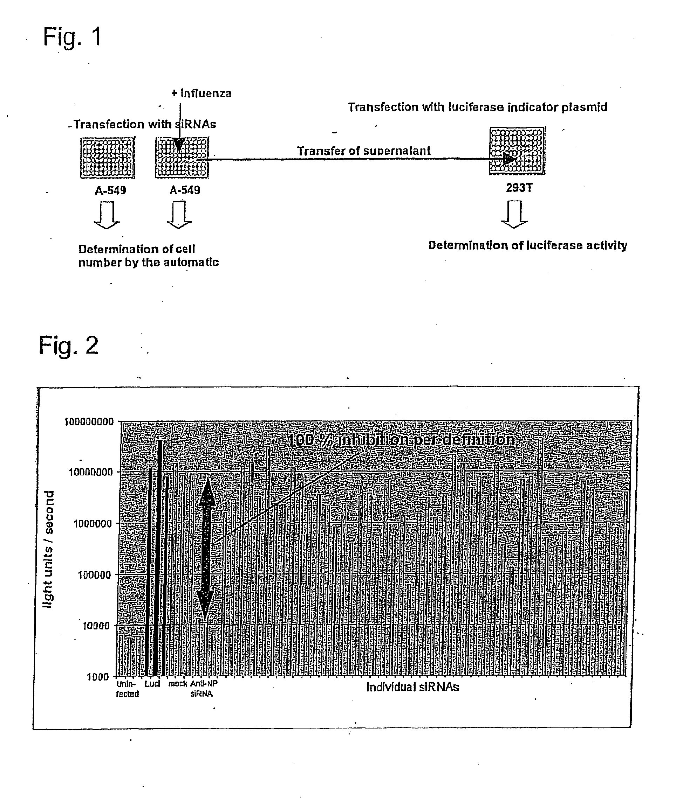

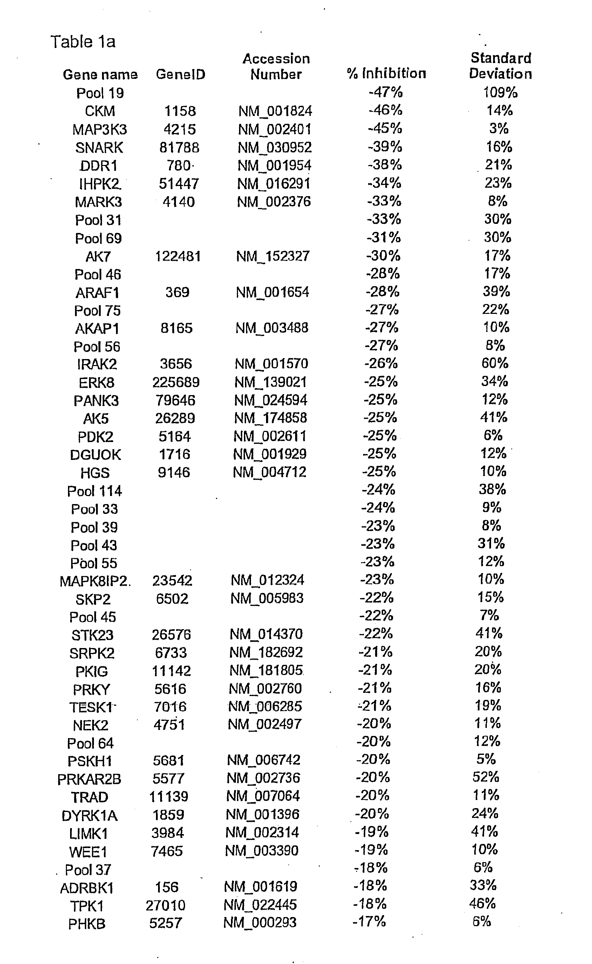

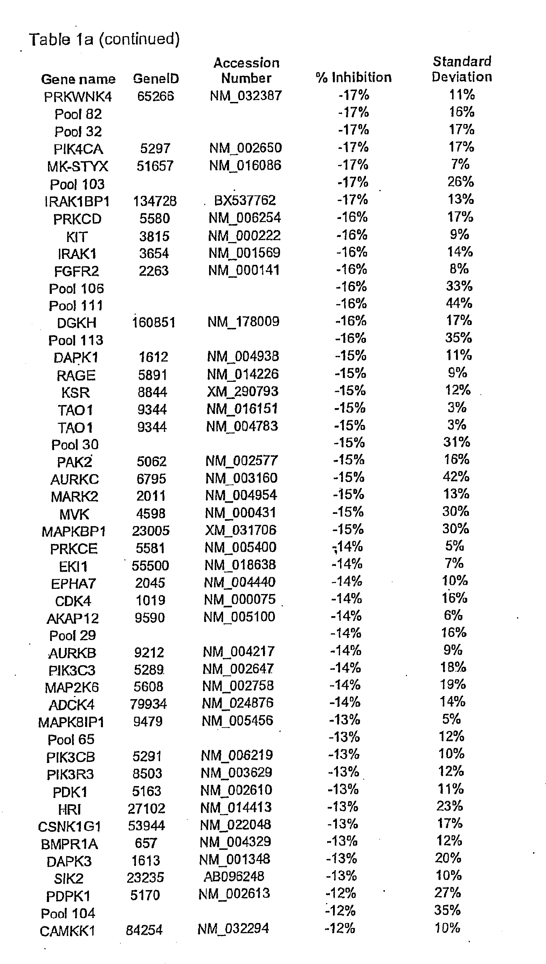

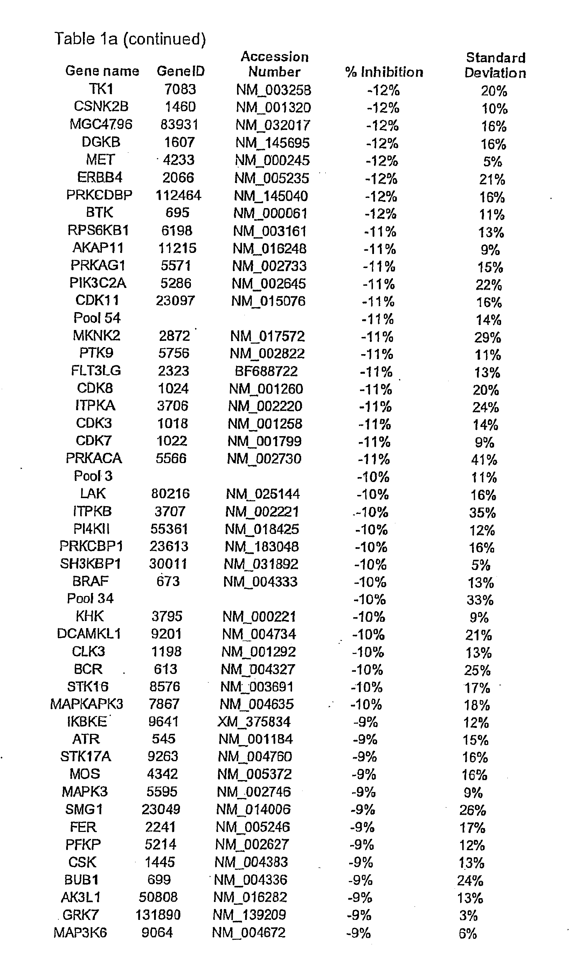

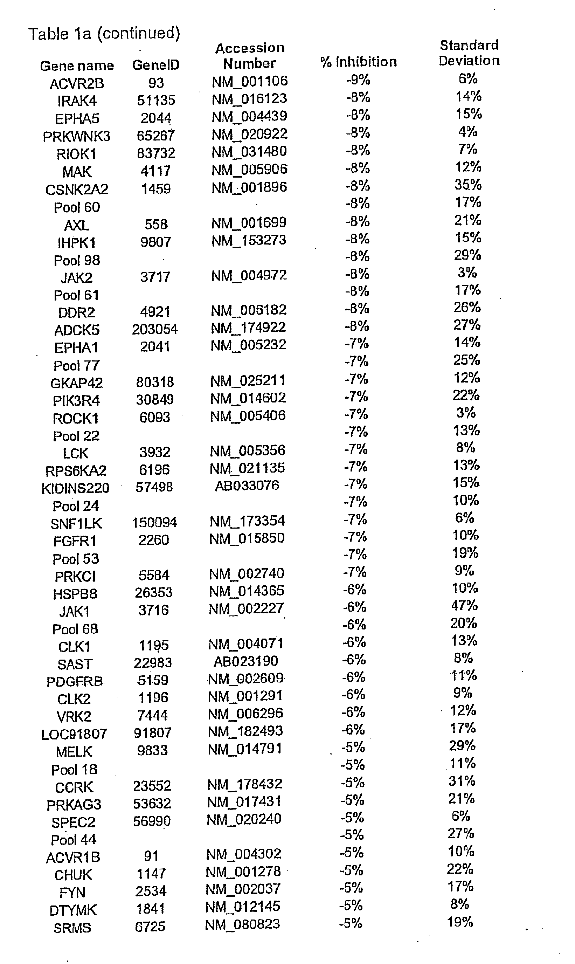

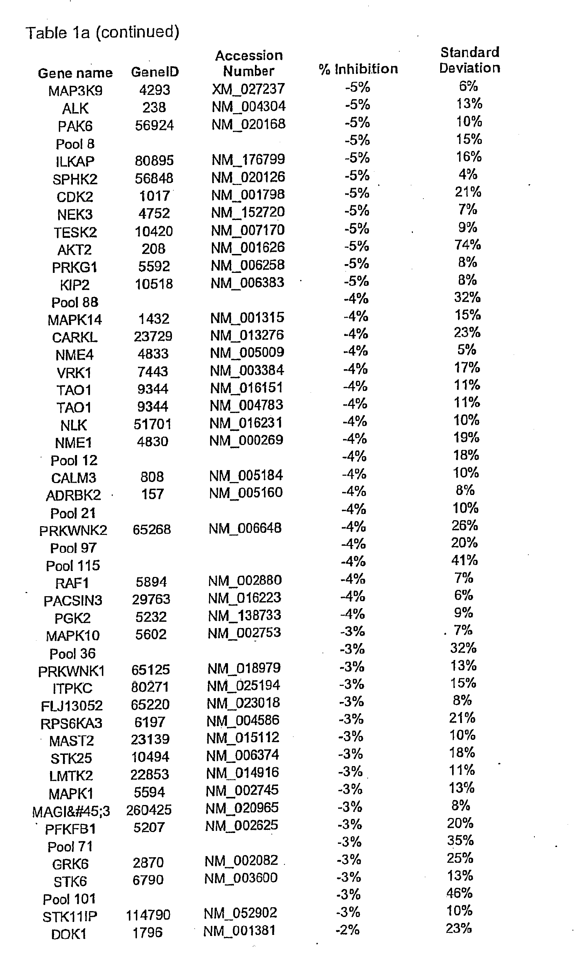

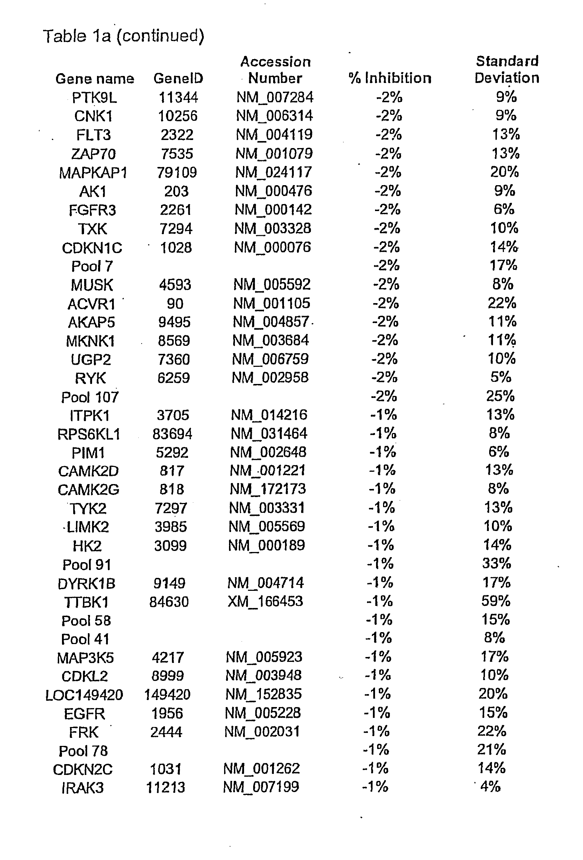

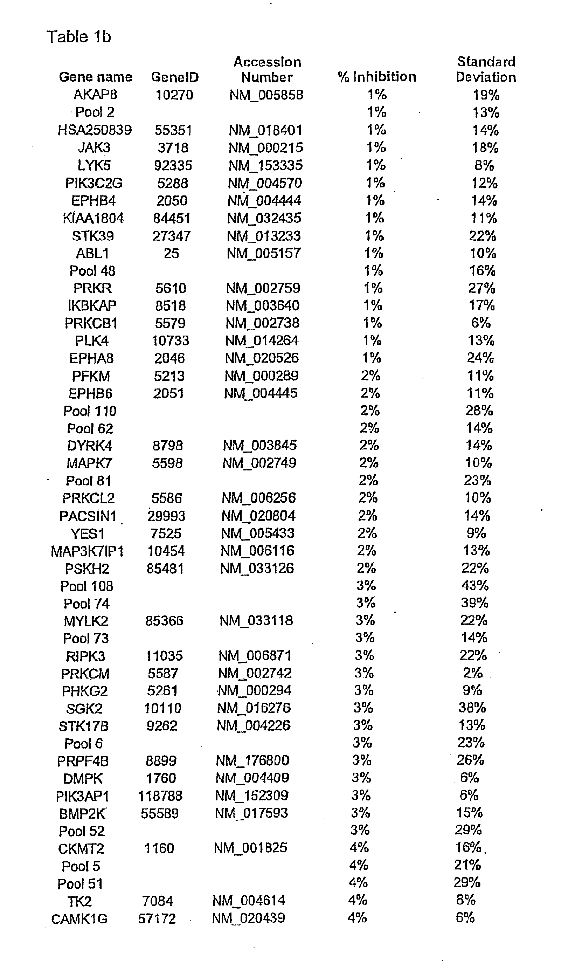

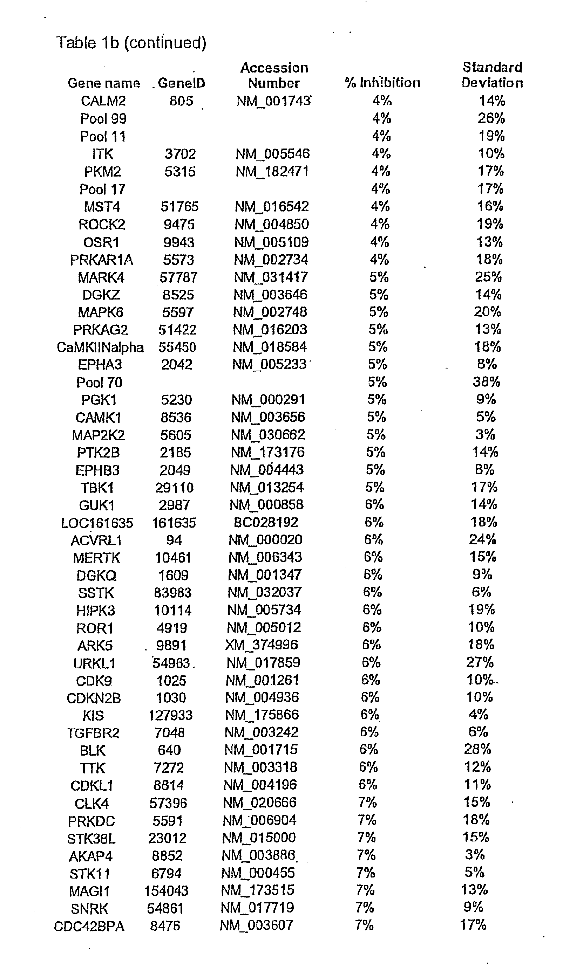

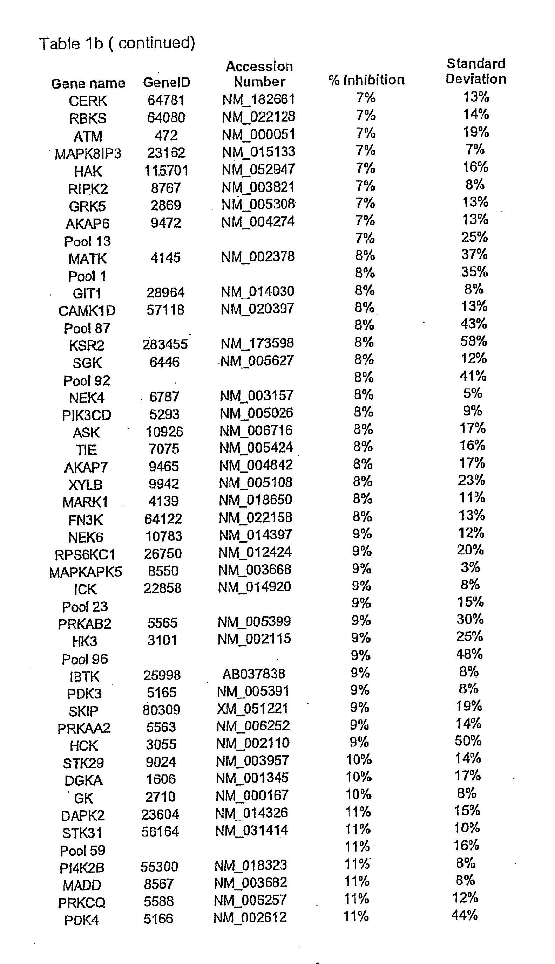

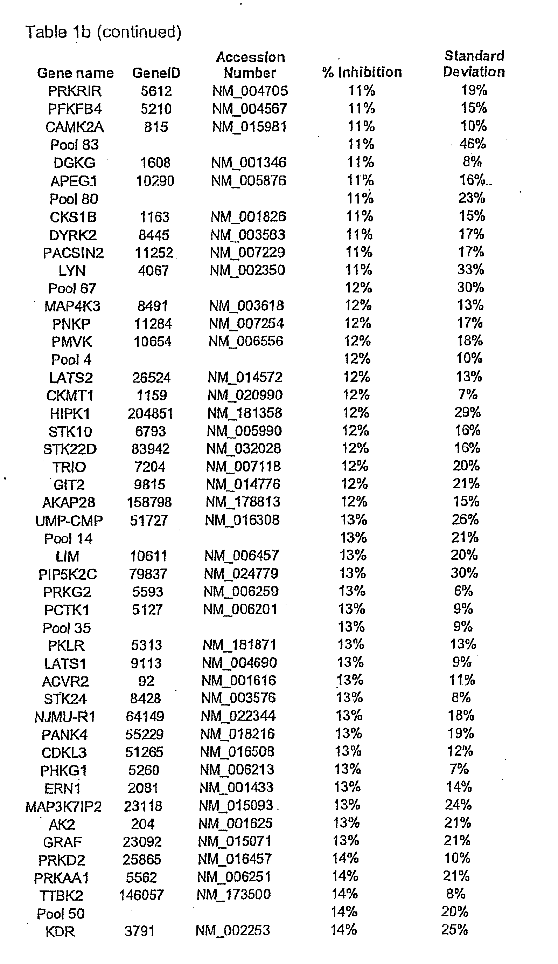

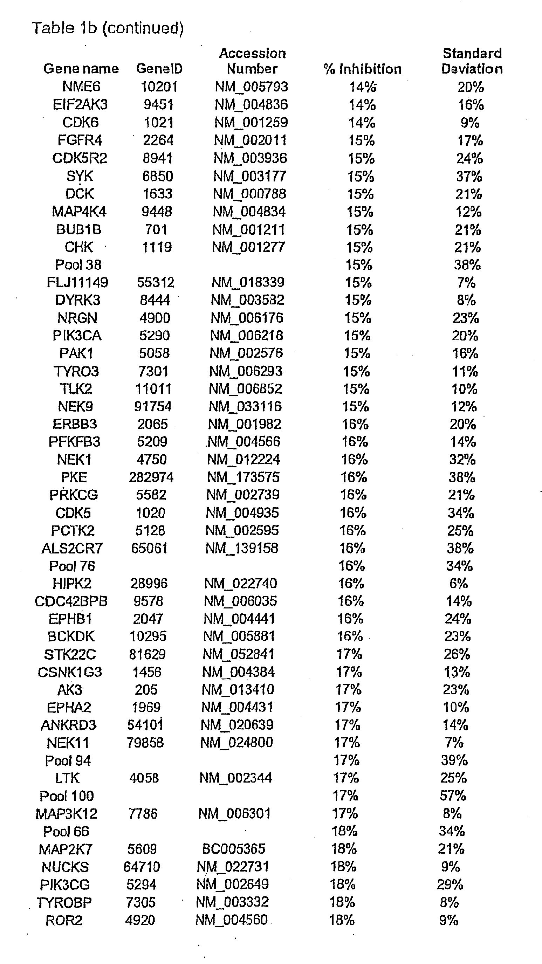

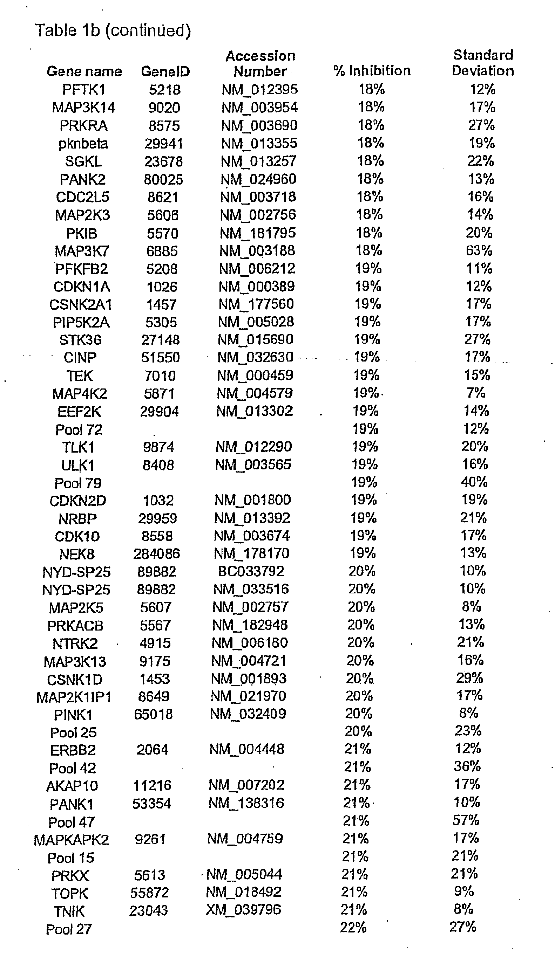

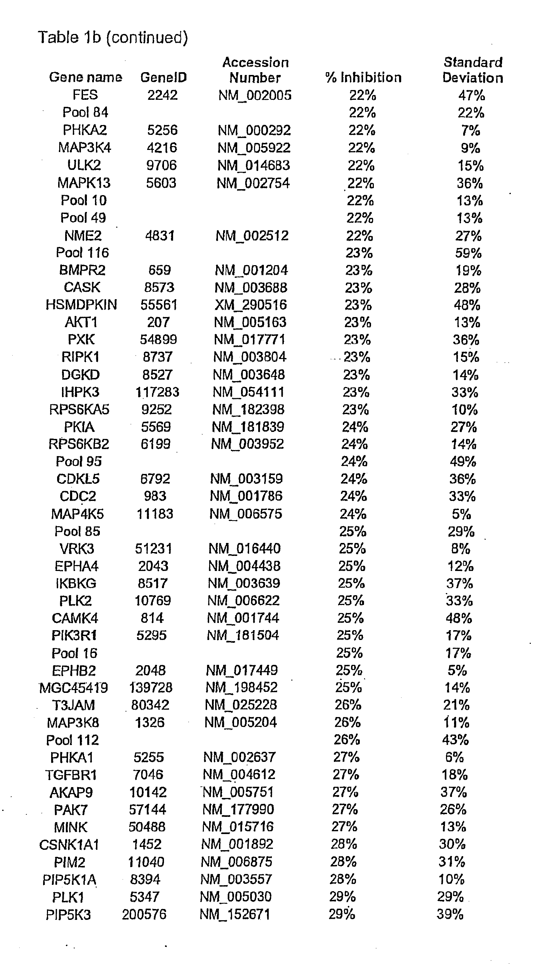

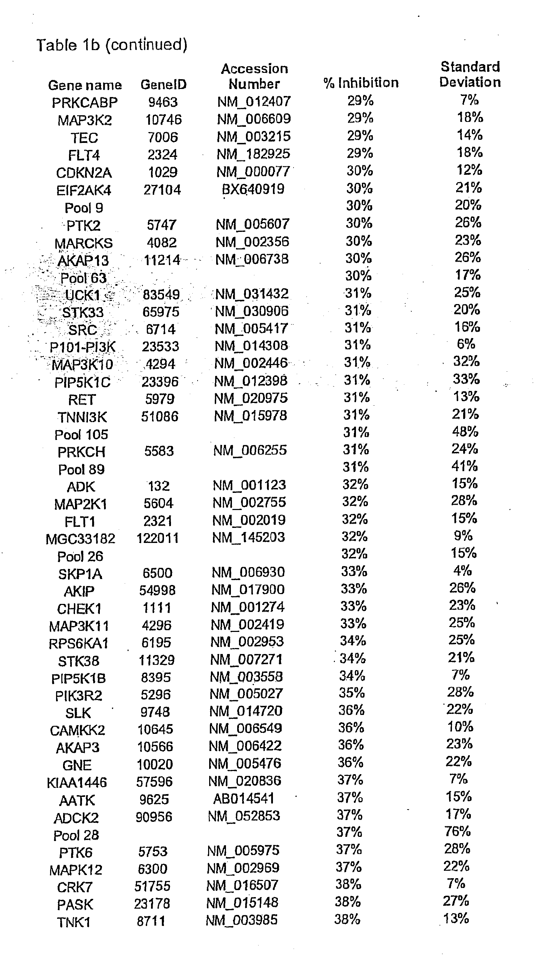

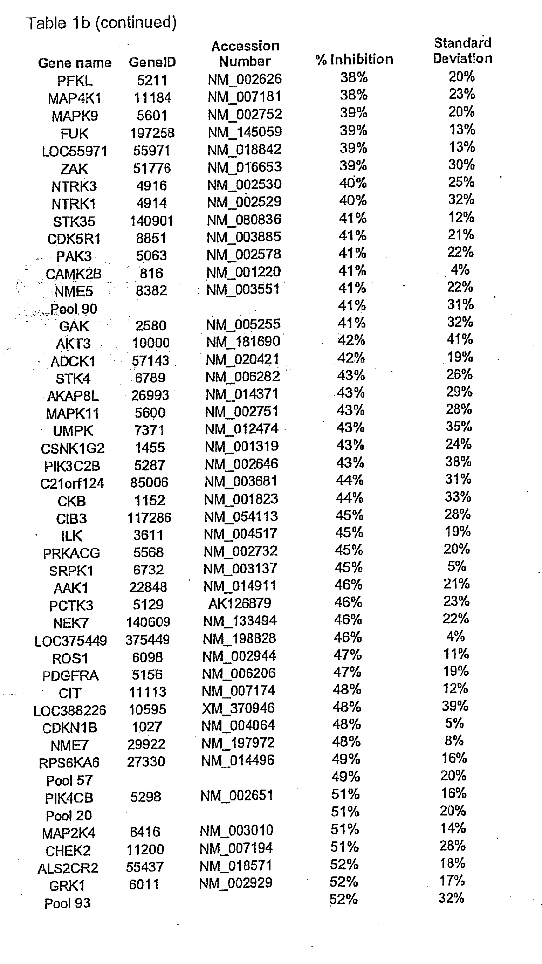

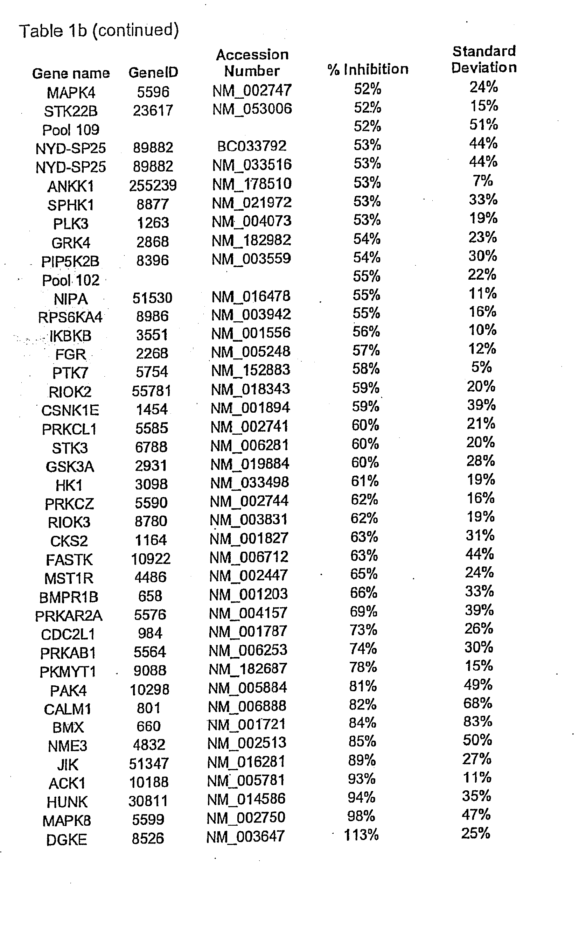

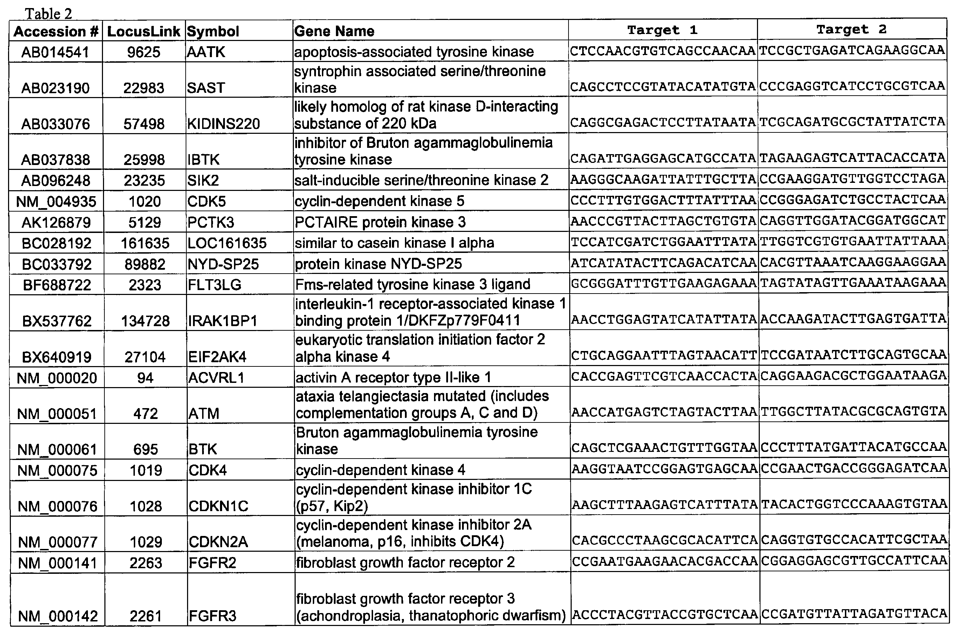

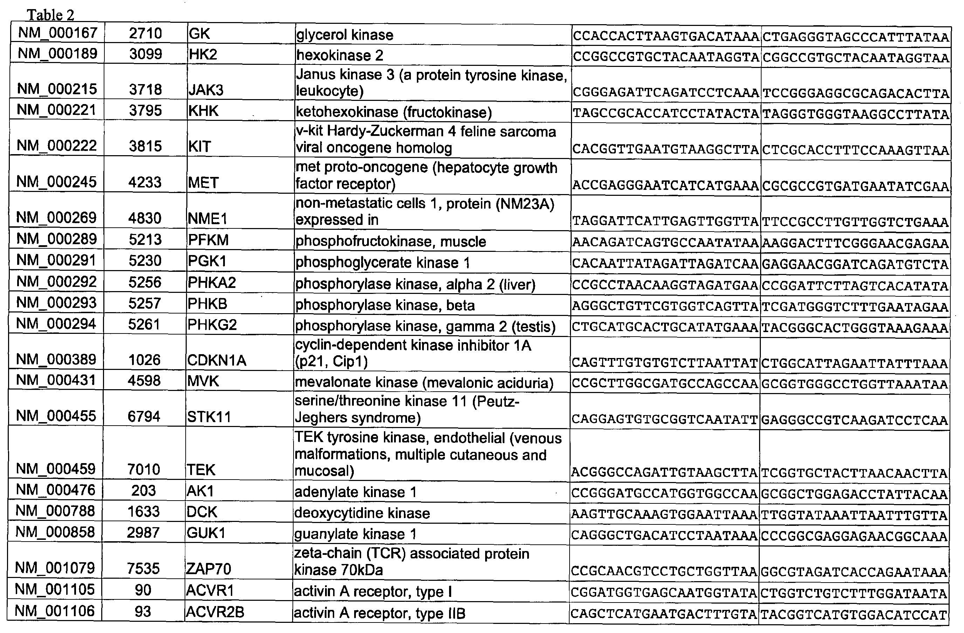

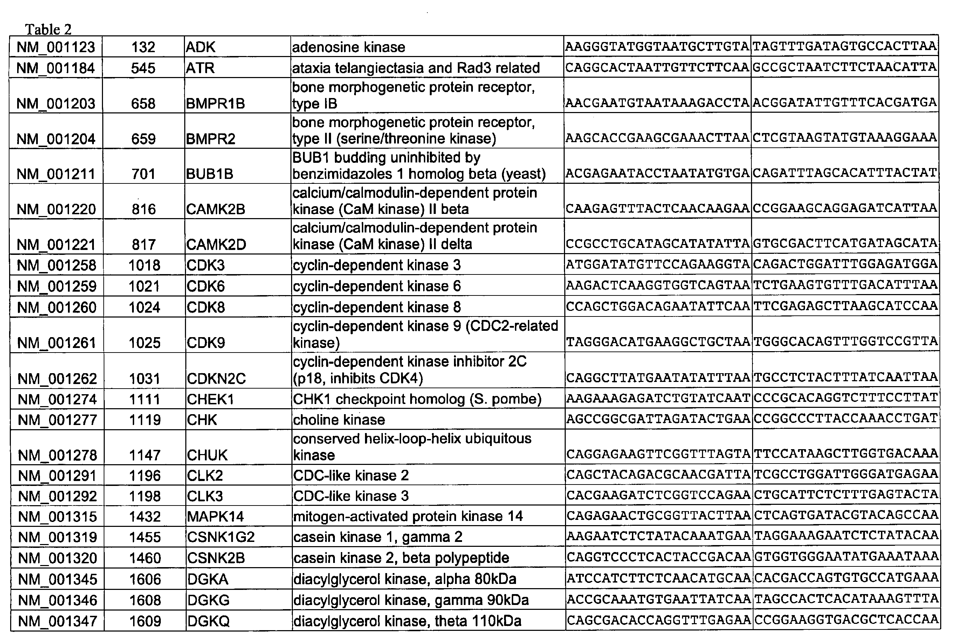

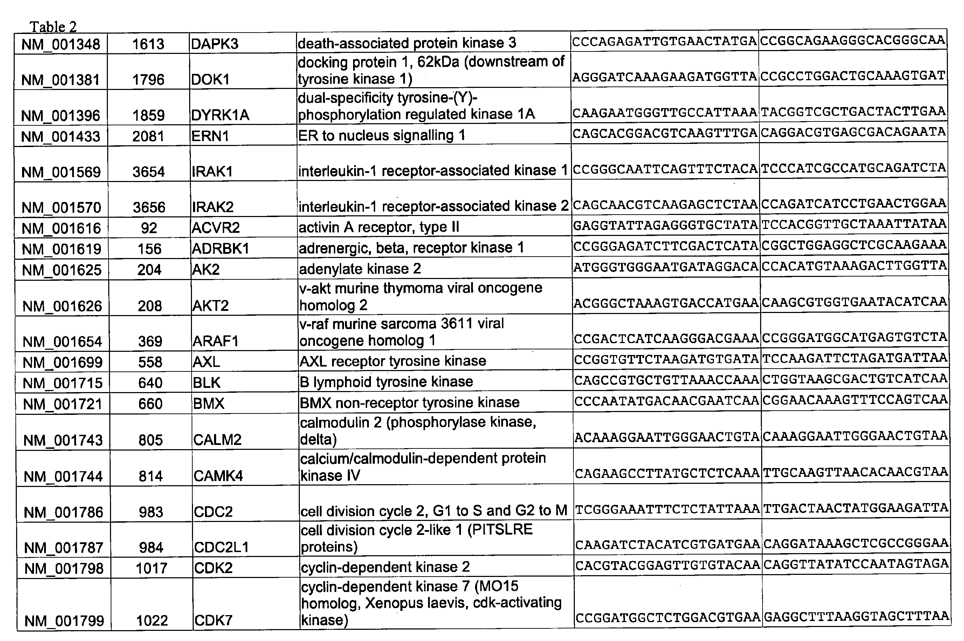

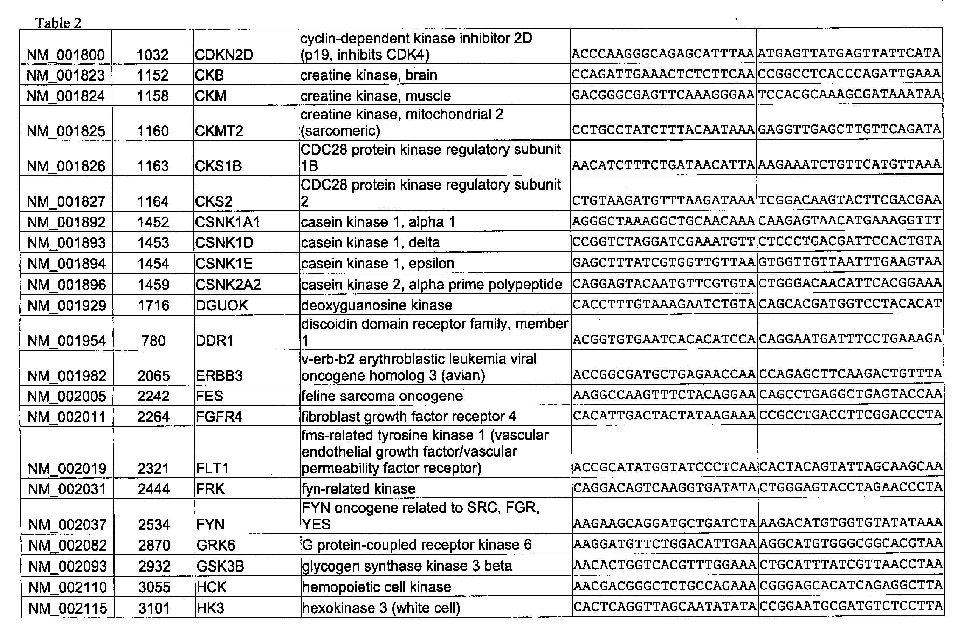

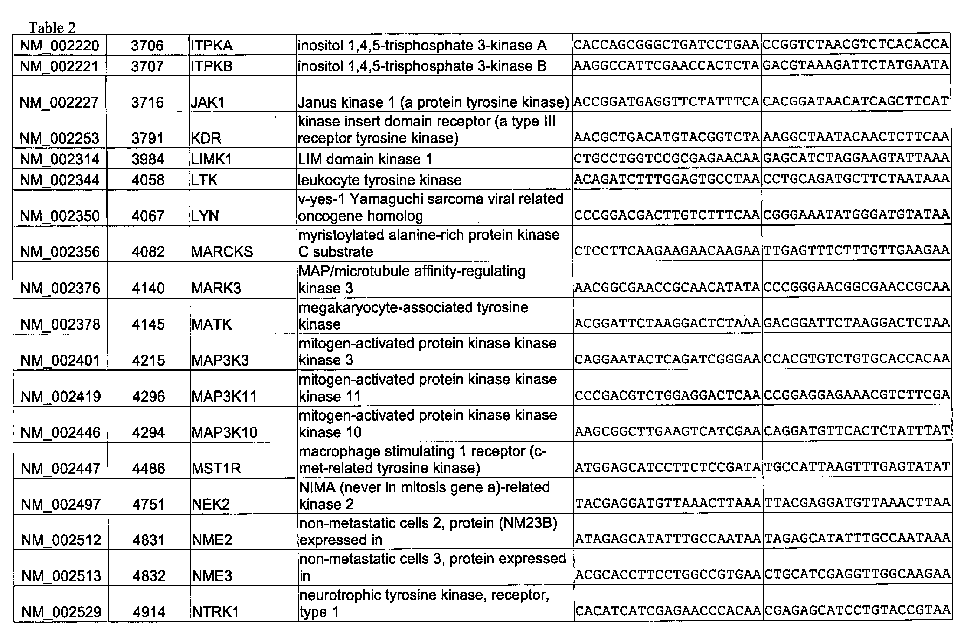

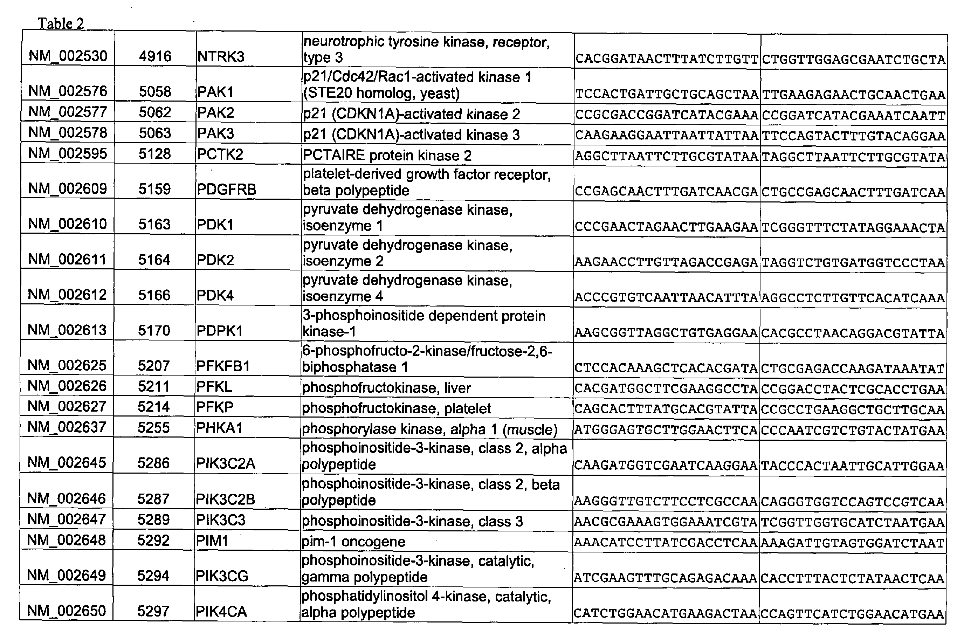

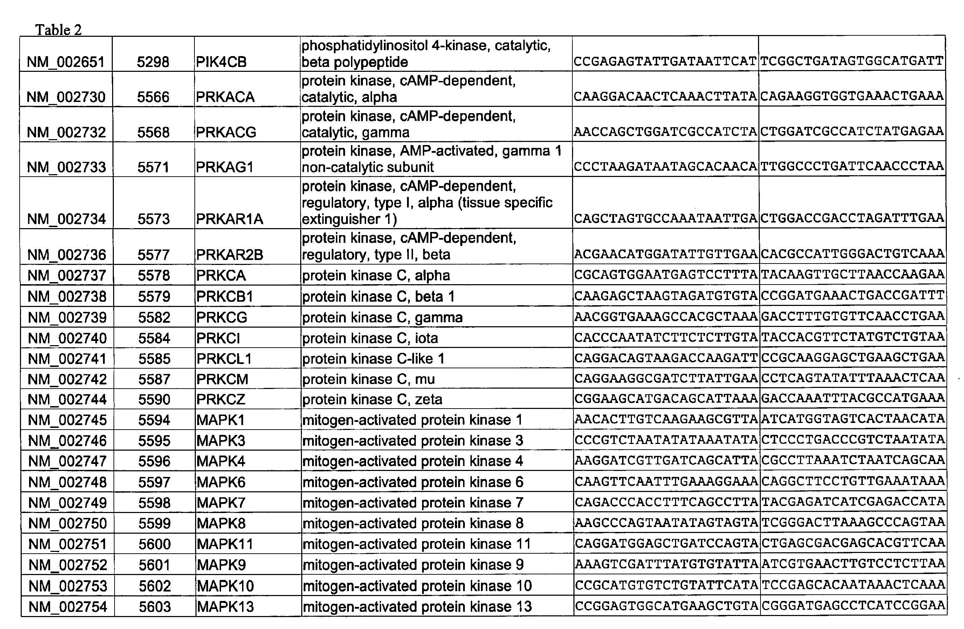

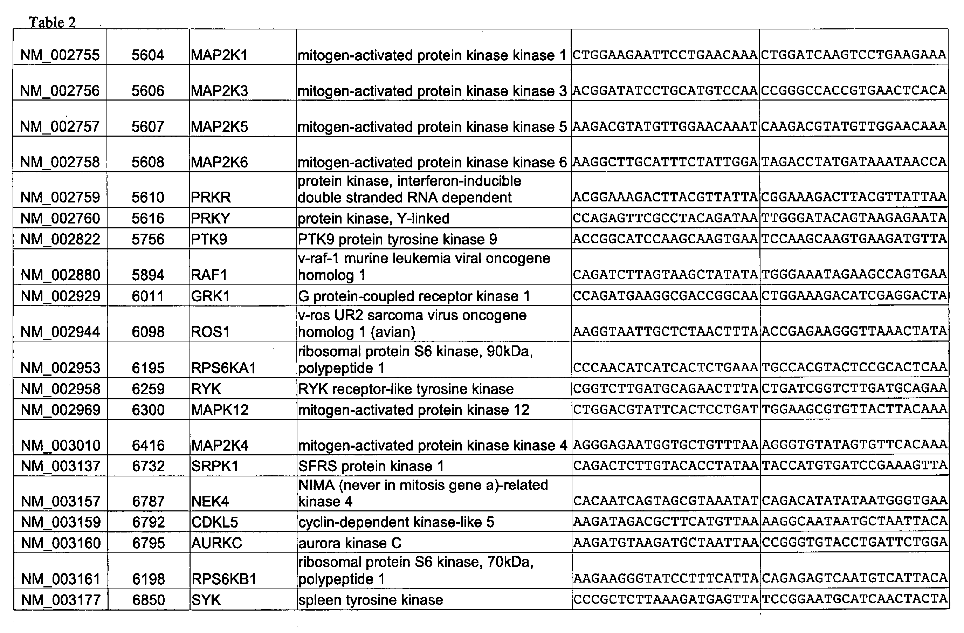

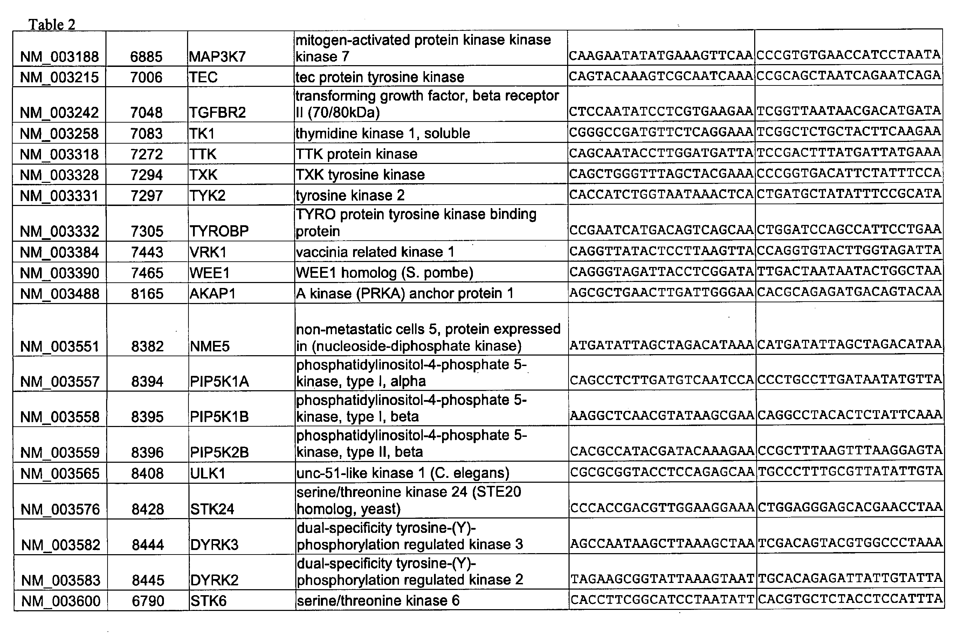

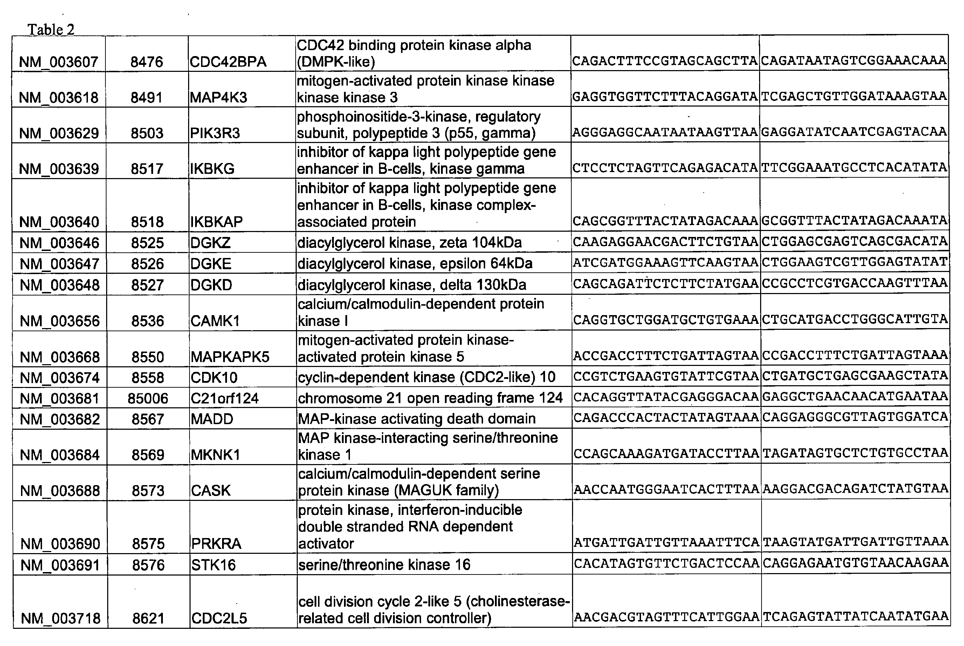

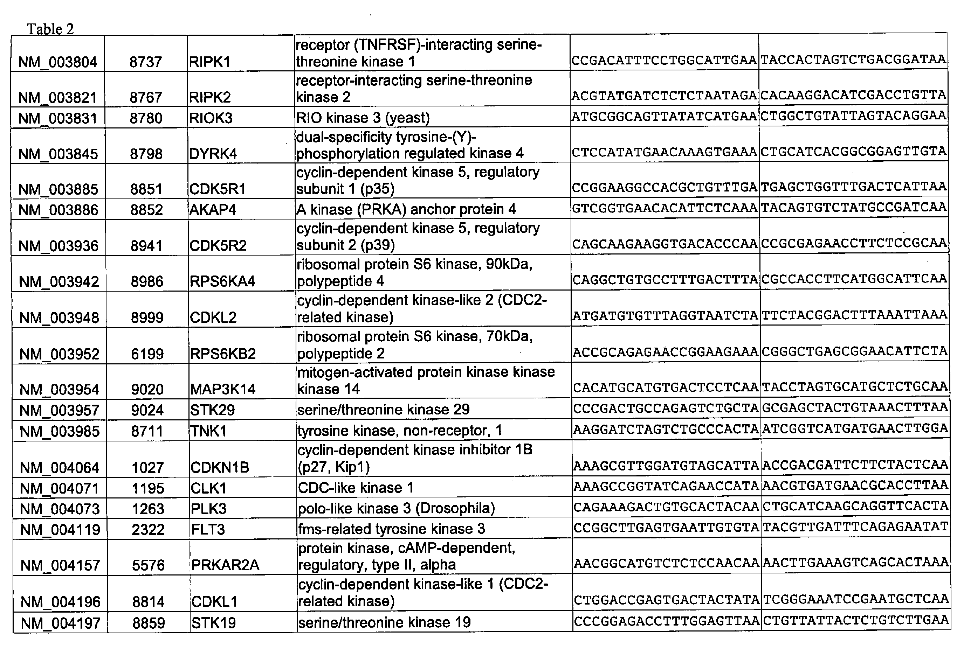

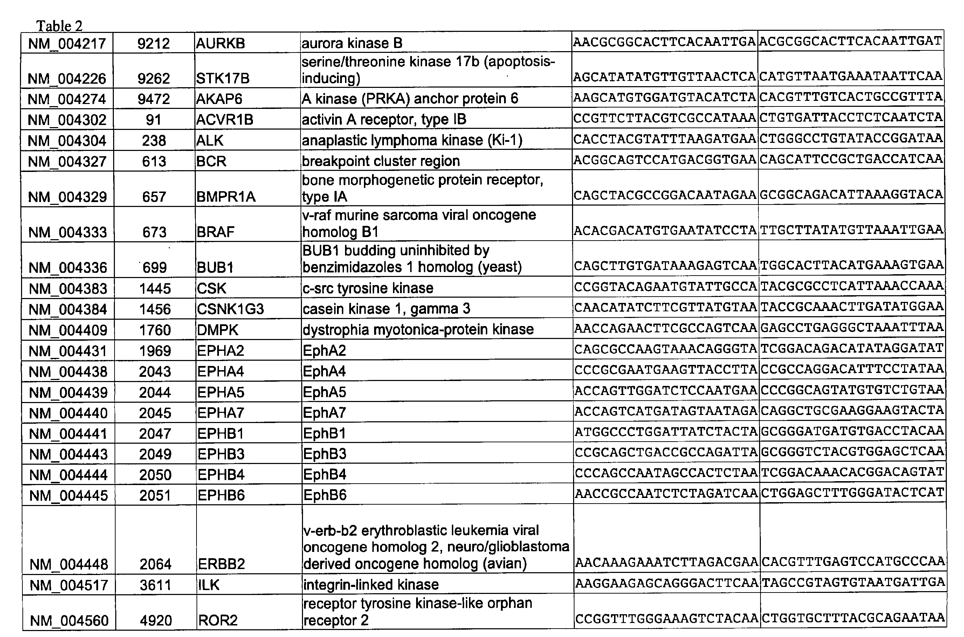

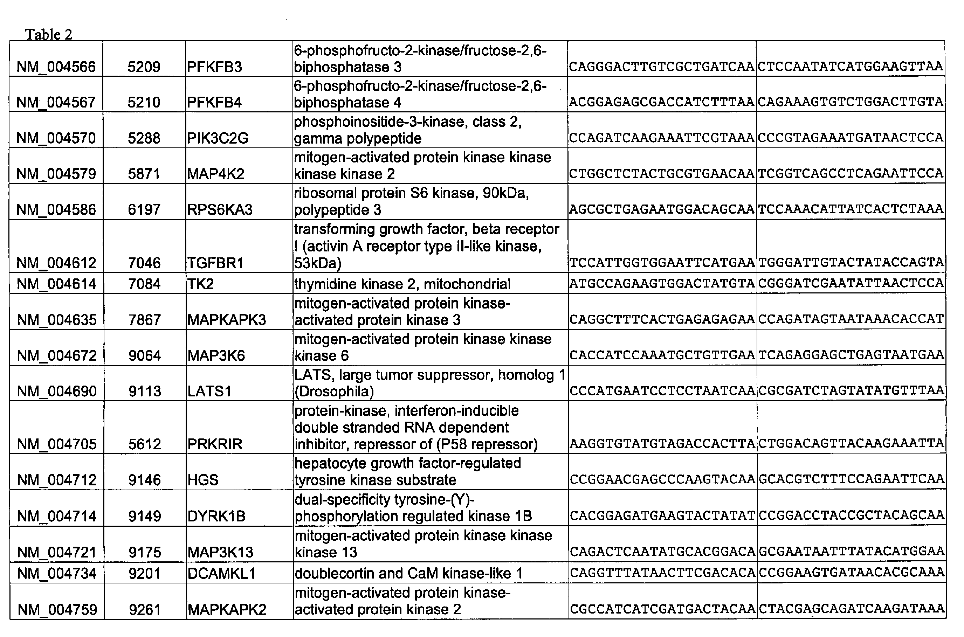

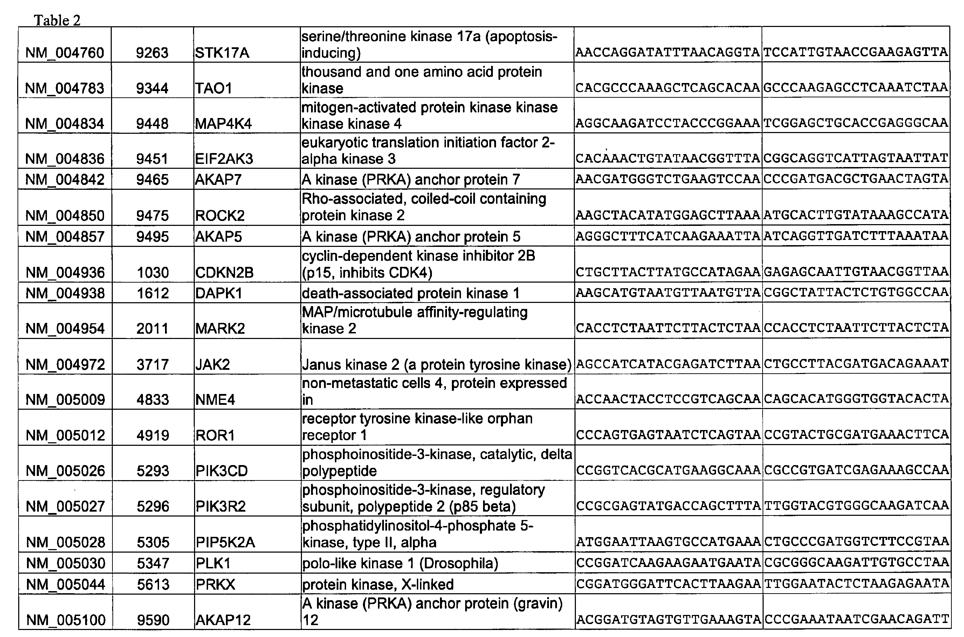

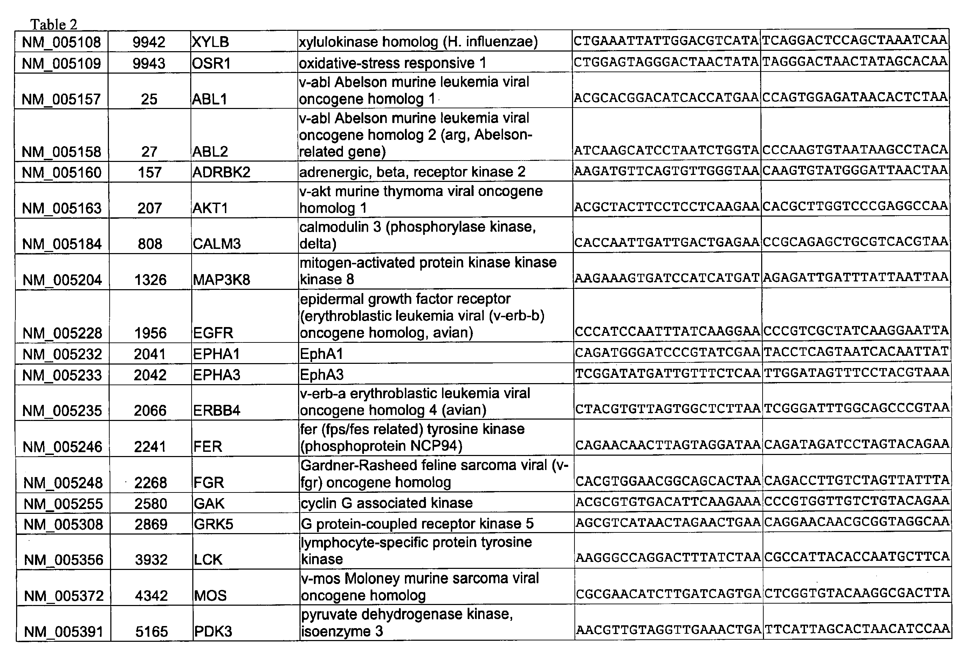

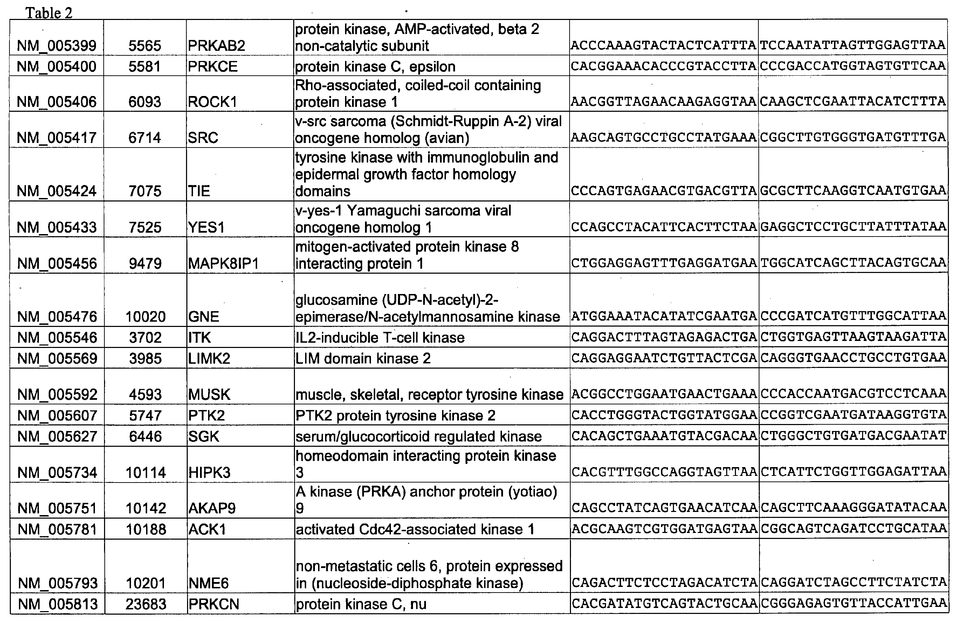

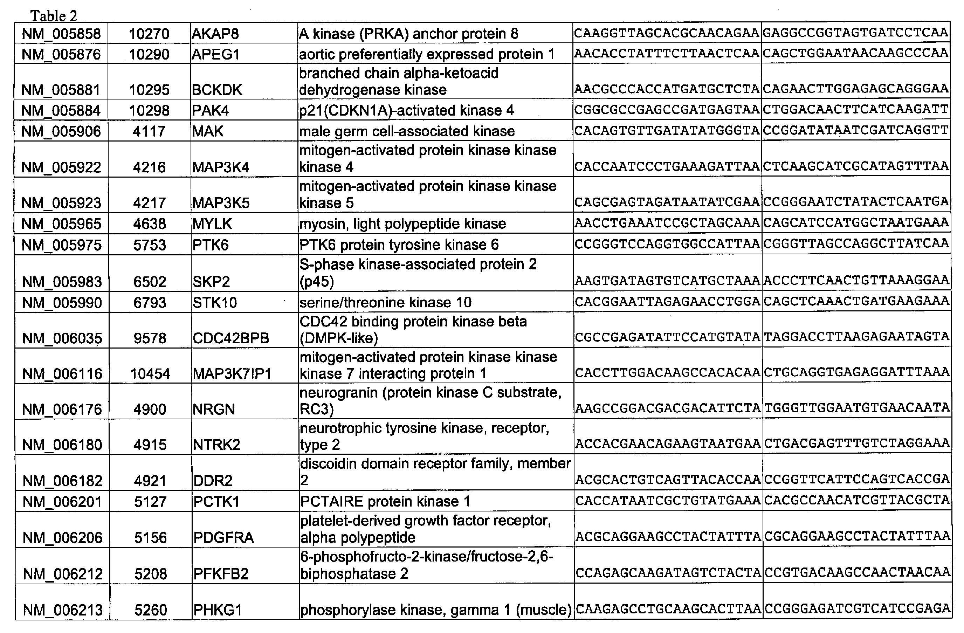

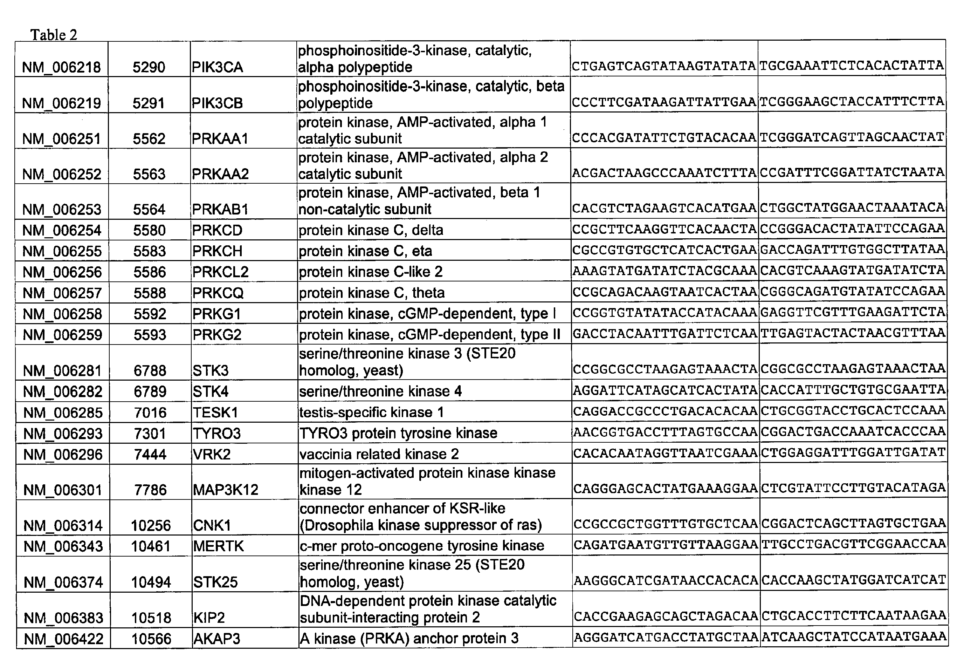

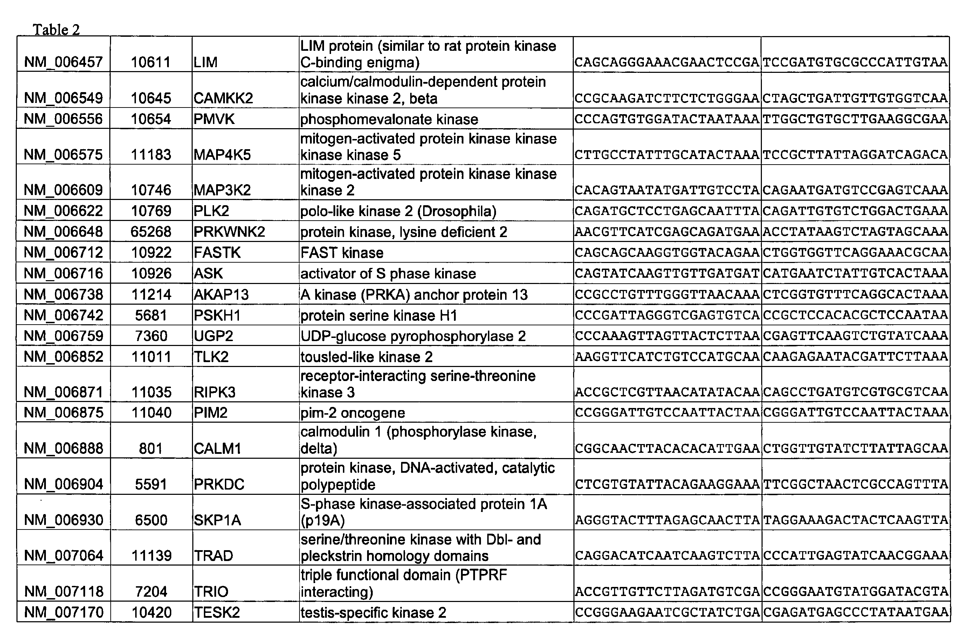

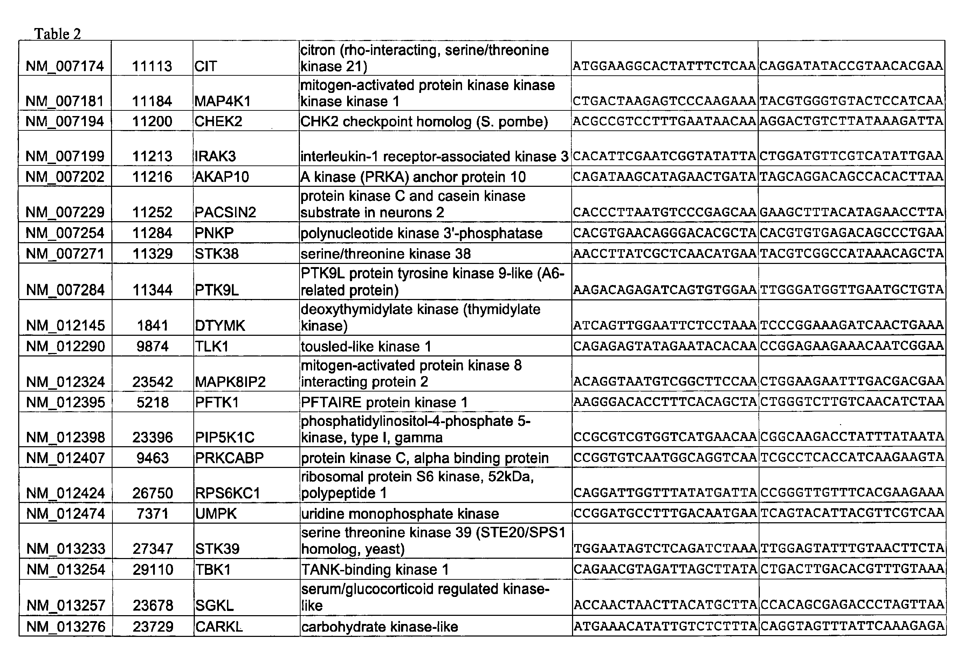

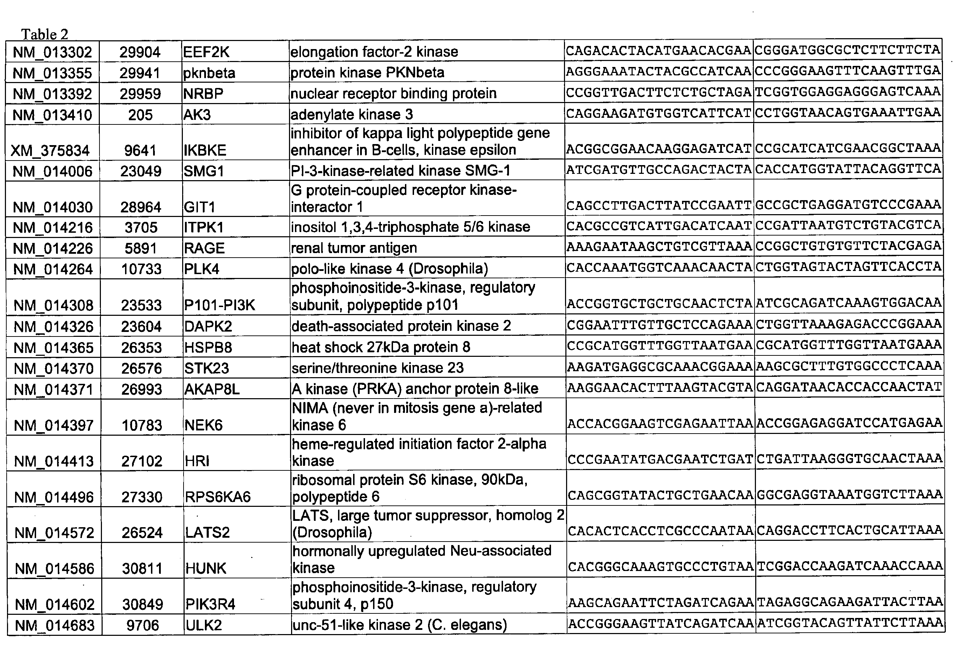

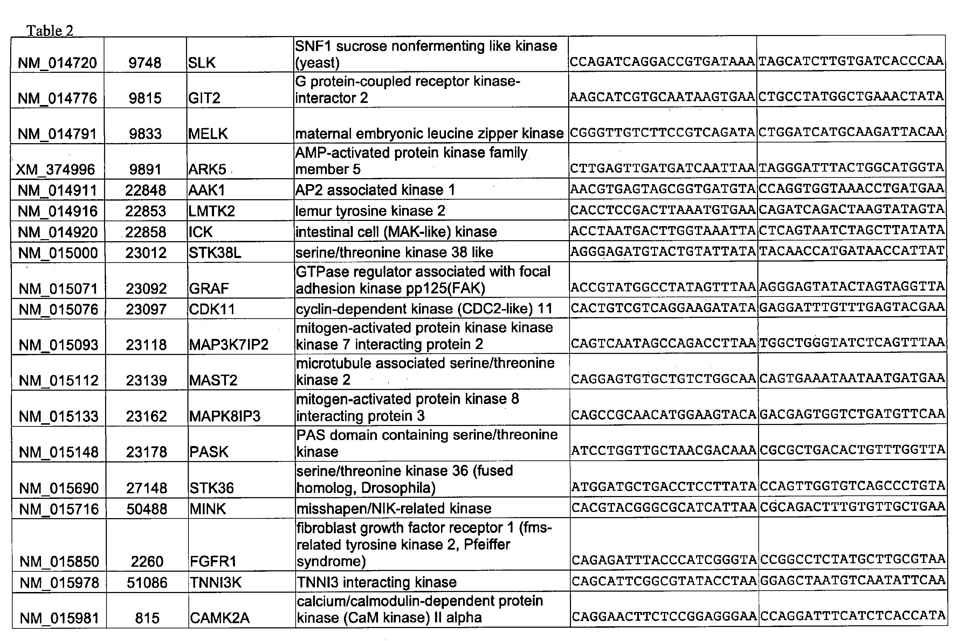

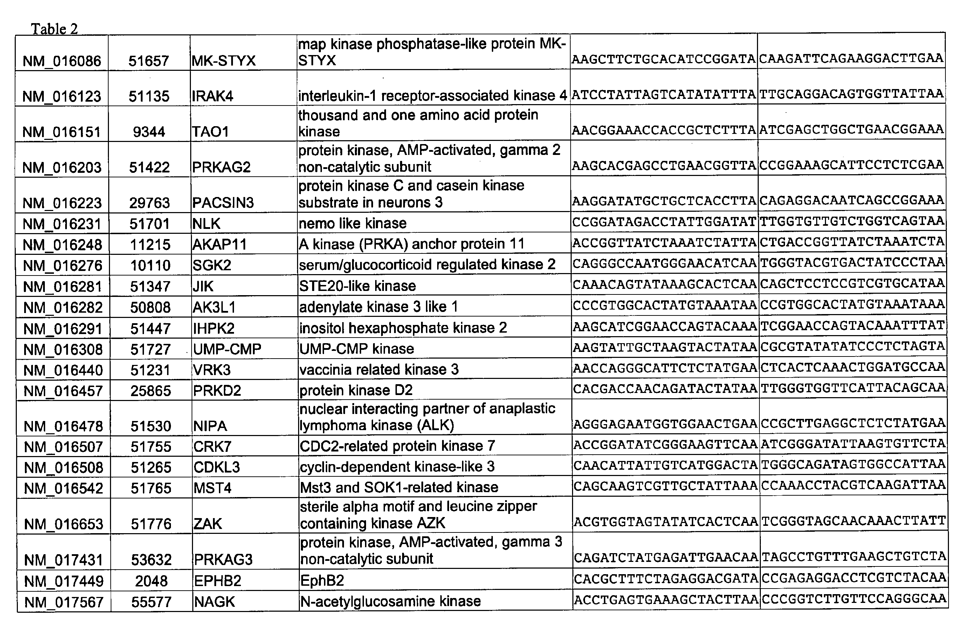

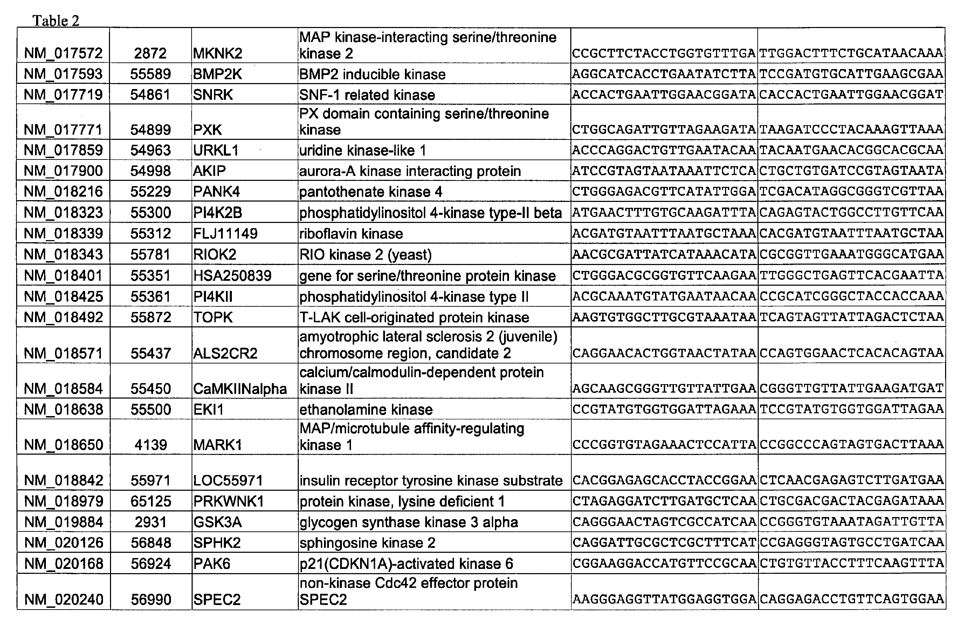

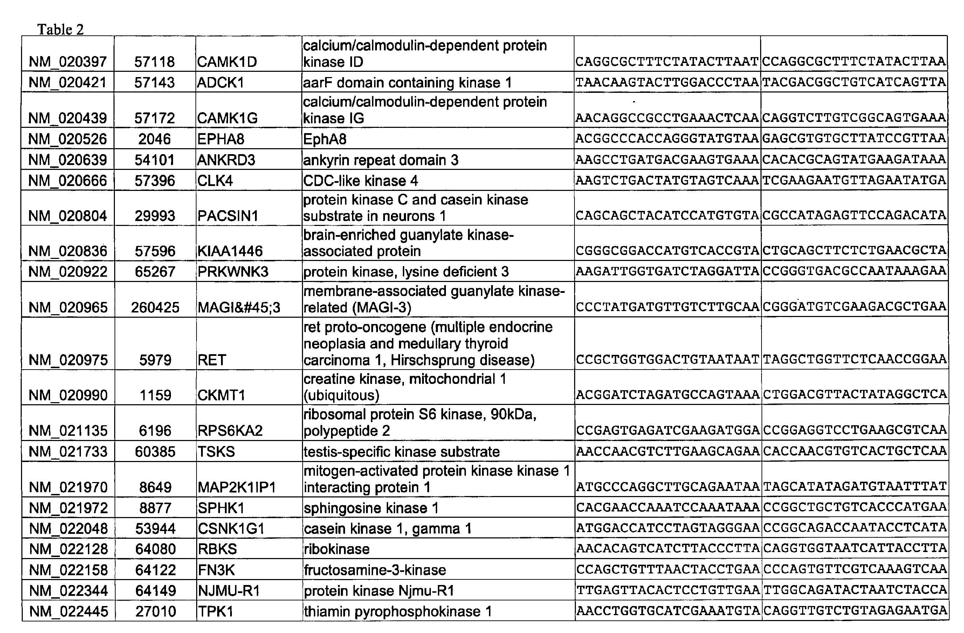

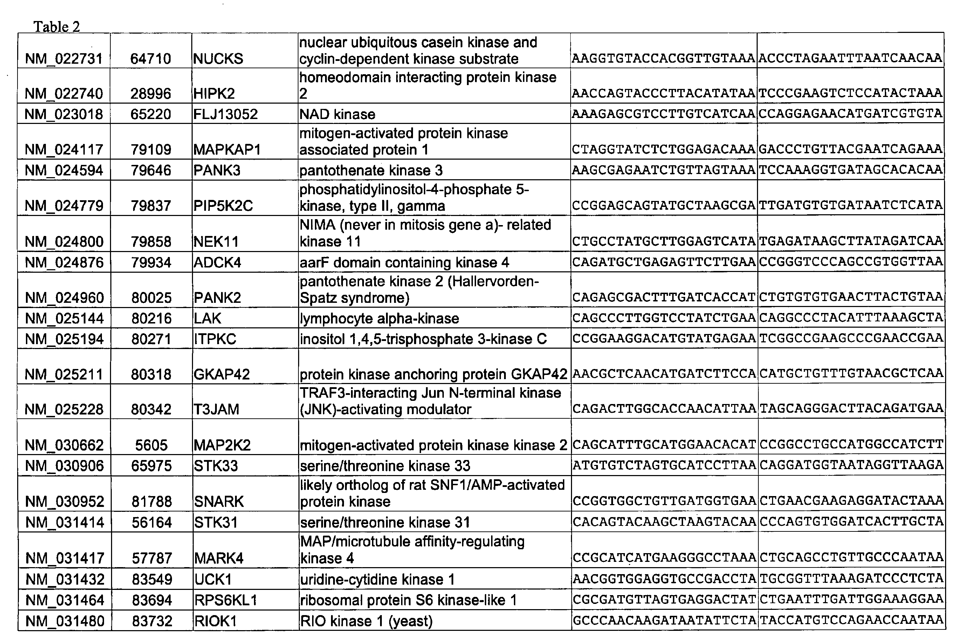

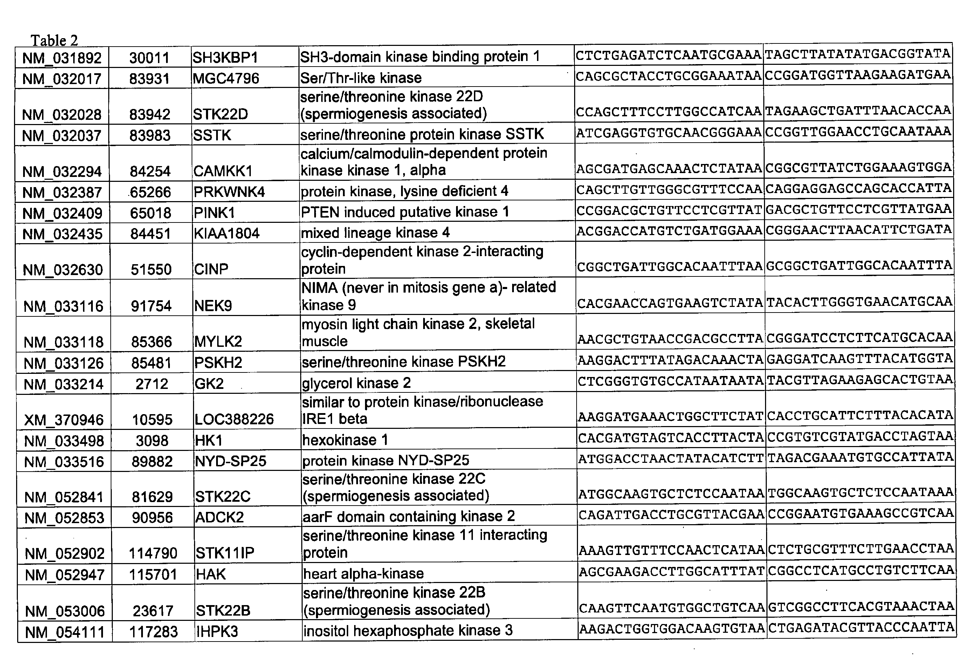

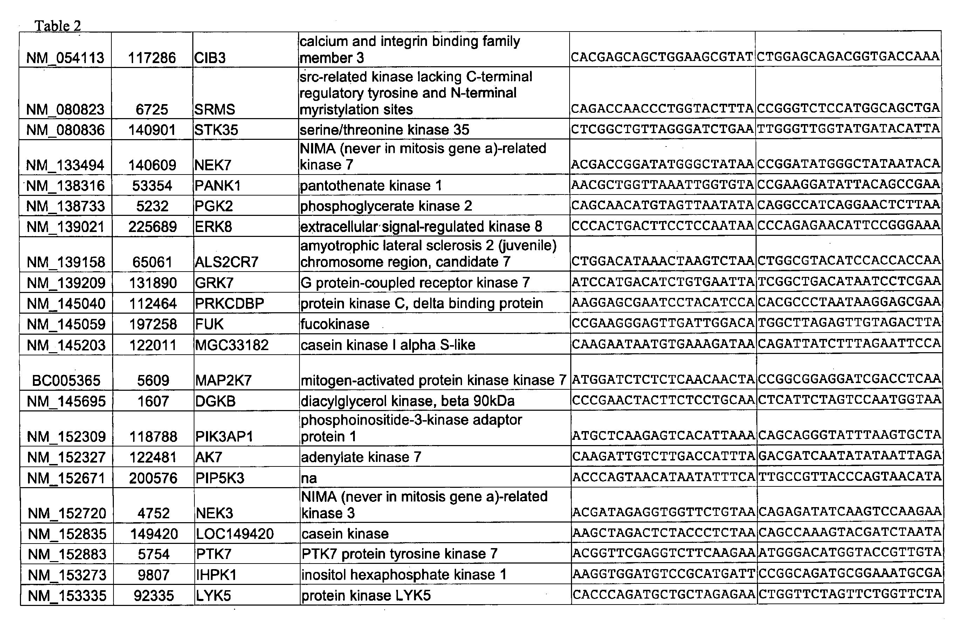

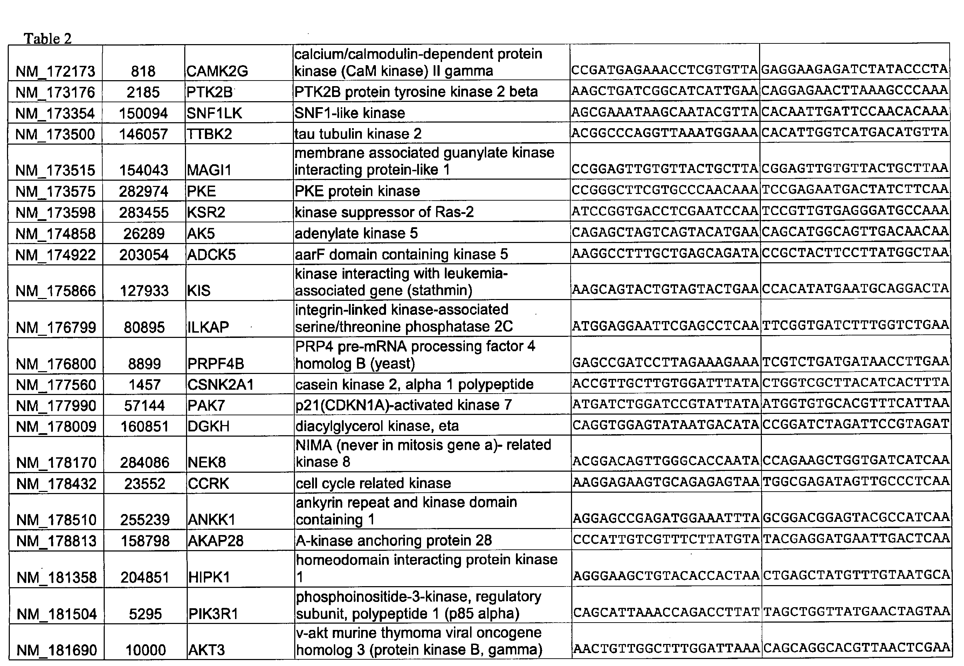

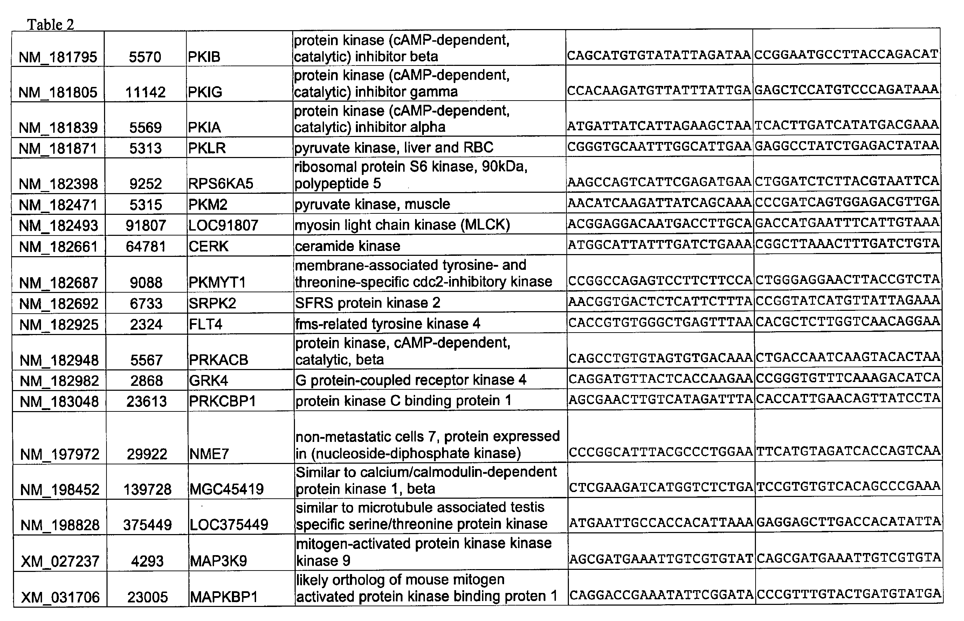

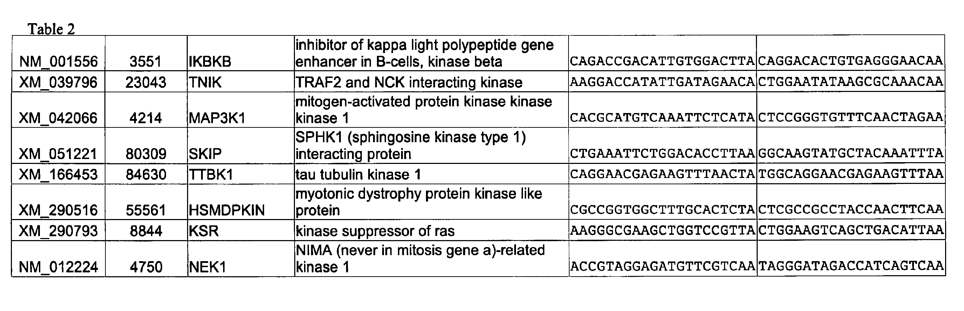

**Figure 1:** The experimental setting of the siRNA kinase screen of the example.

**Figure 2:** The effect of transfected (control)-siRNAs in regard to luminescence data. This diagram shows a typical screening result from one 96 well plate. During all experiments several controls were included in triplets, like uninfected, transfected with a siRNA against luciferase, mock treated and siRNAs against the viral nucleoprotein gene (NP) from influenza A viruses. The difference of the luminescence between cells treated with luciferase siRNAs and anti-NP siRNAs was set to 100 % inhibition per definition.

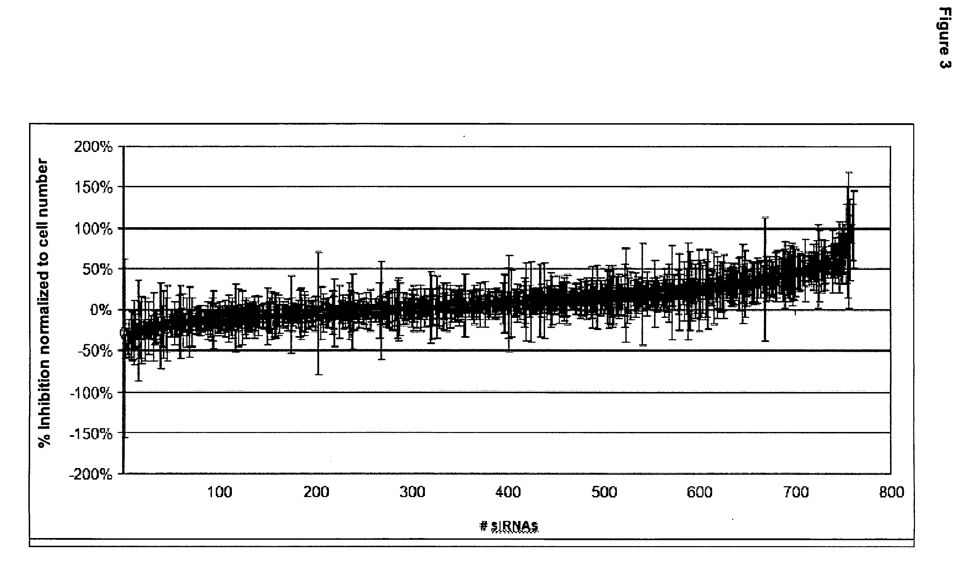

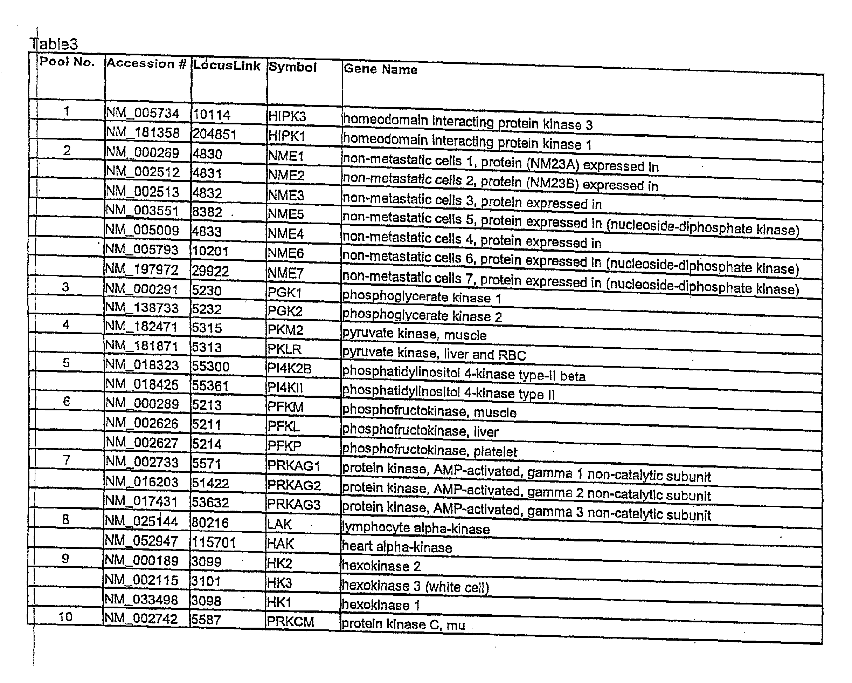

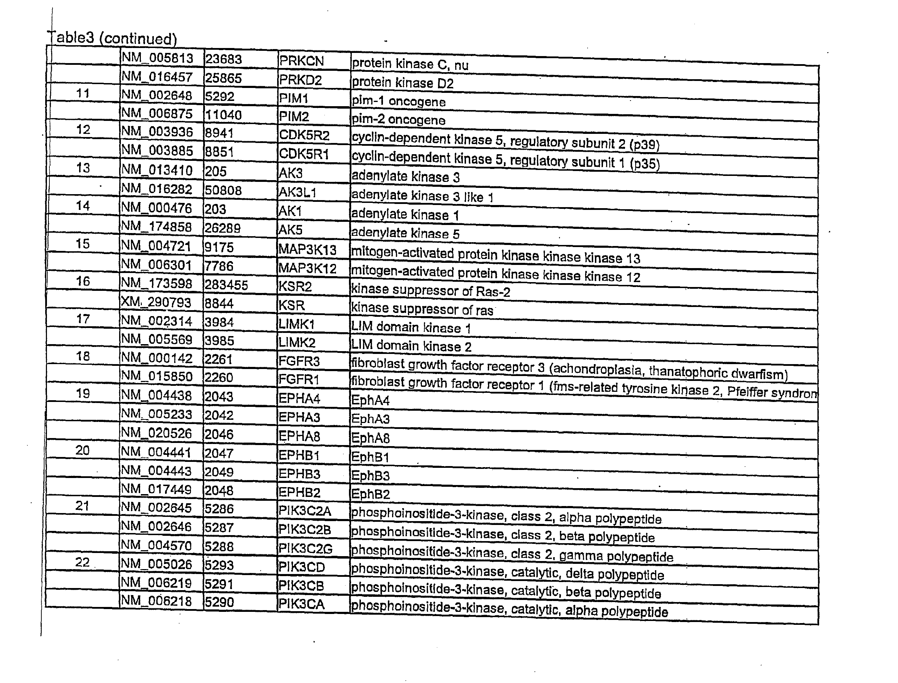

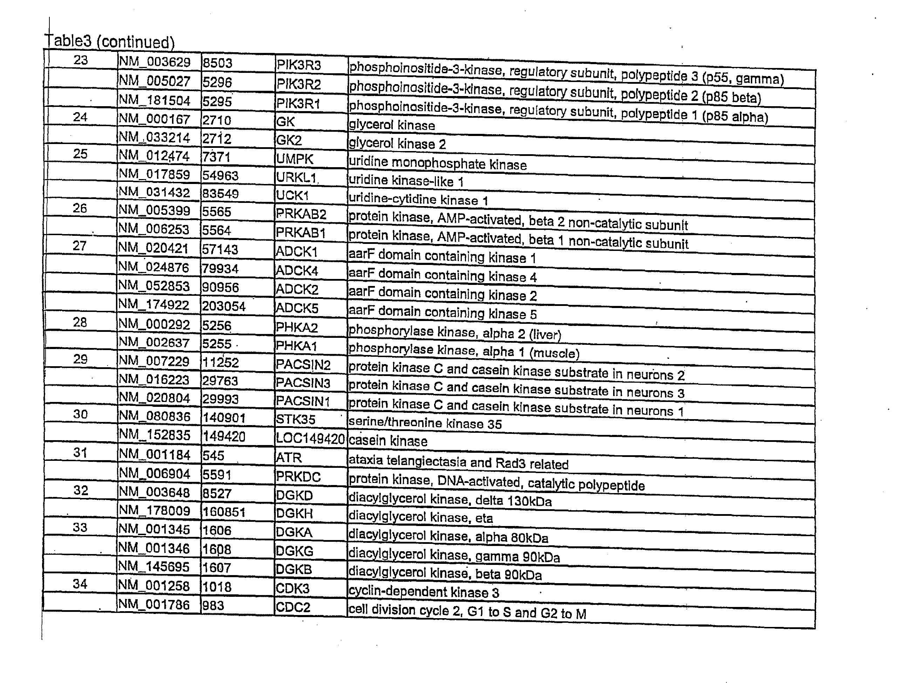

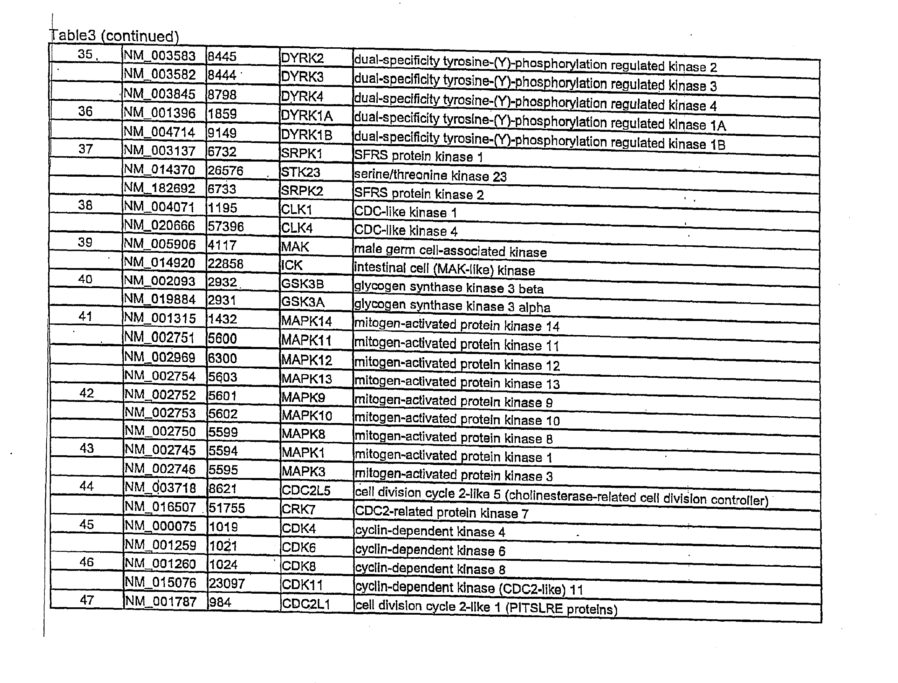

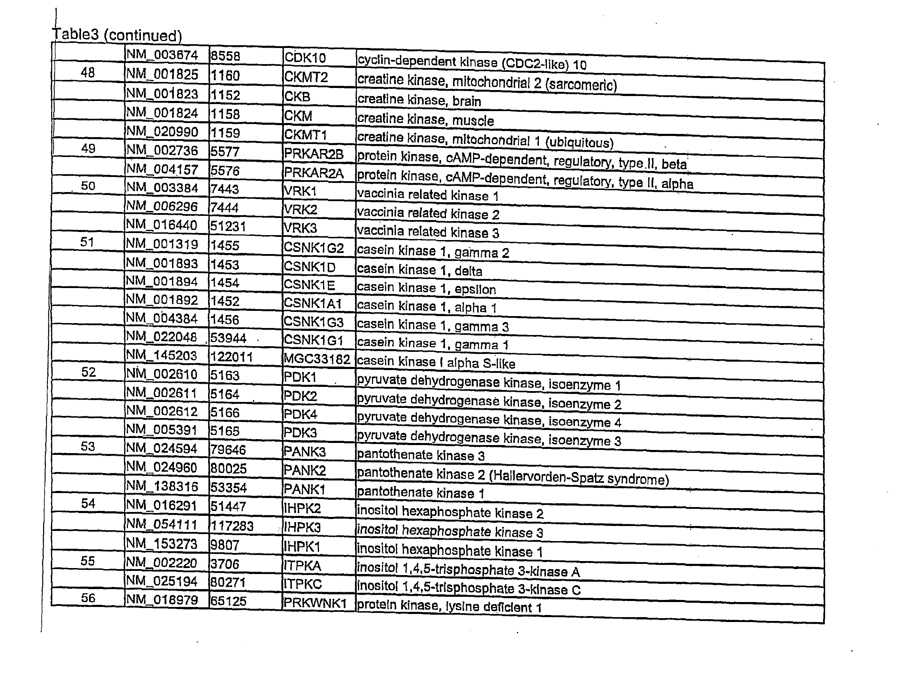

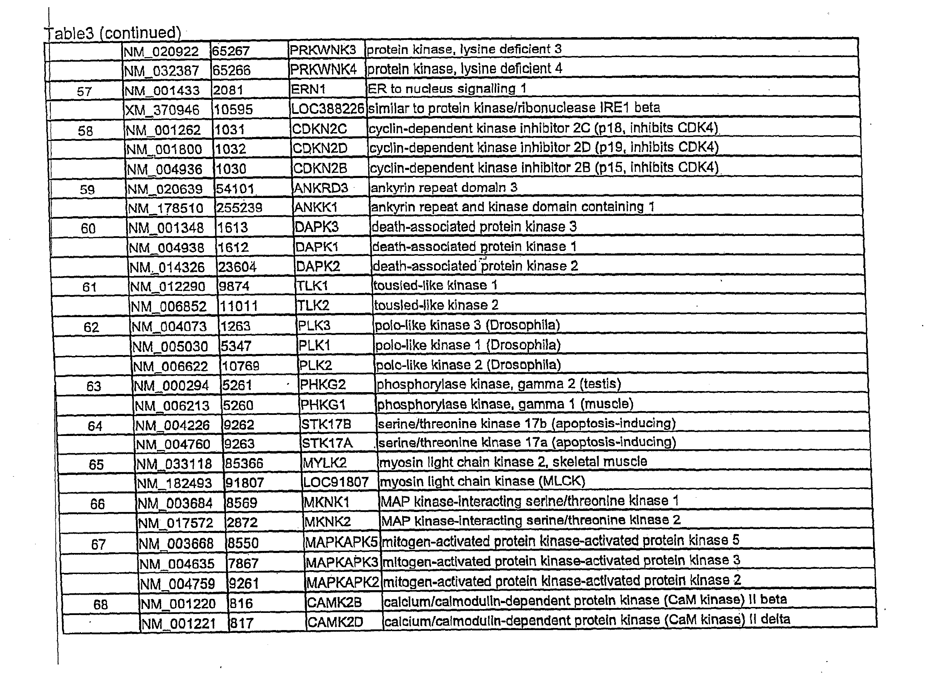

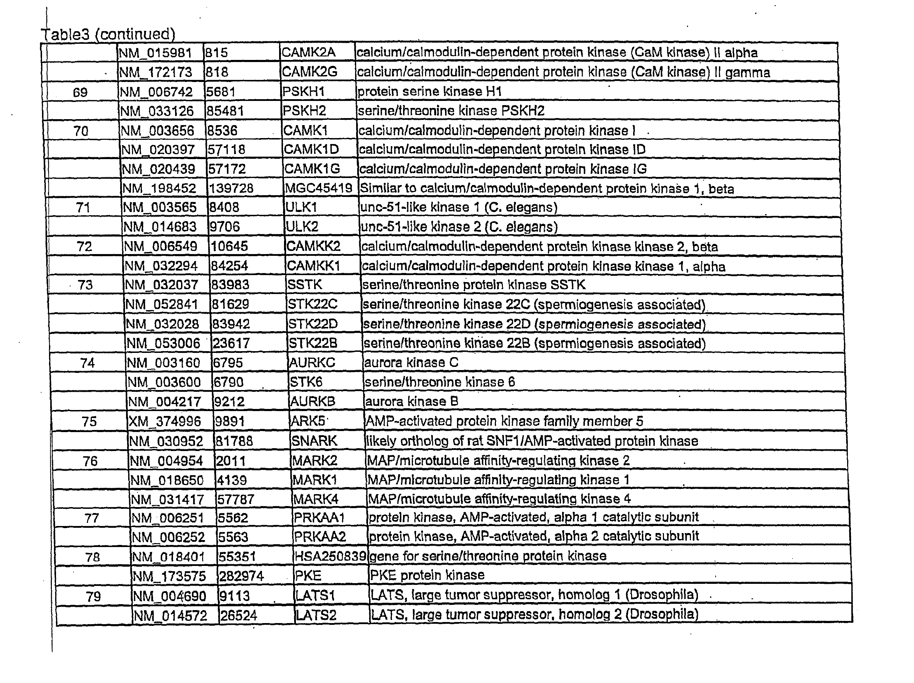

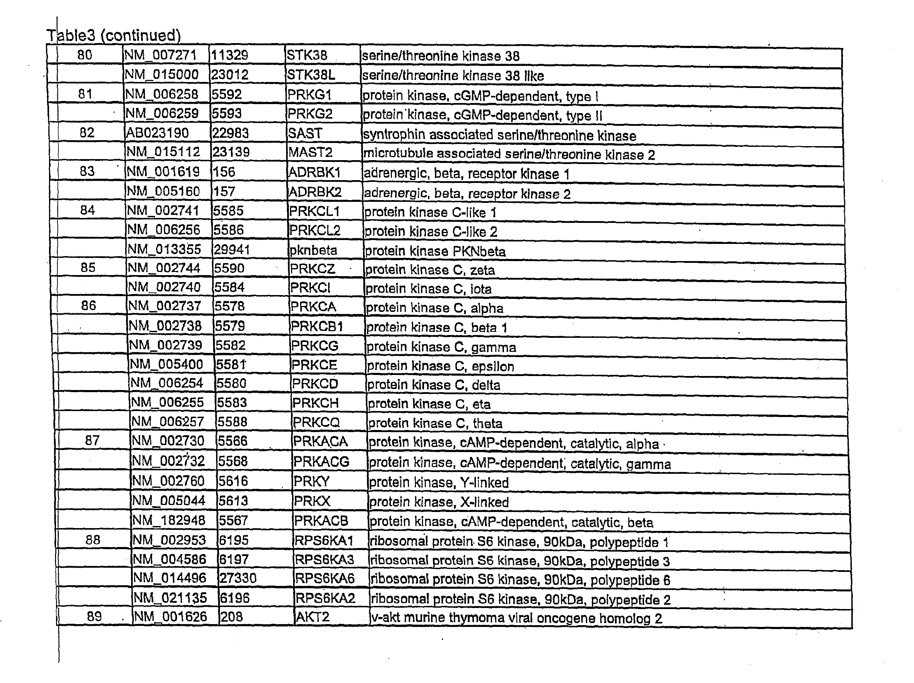

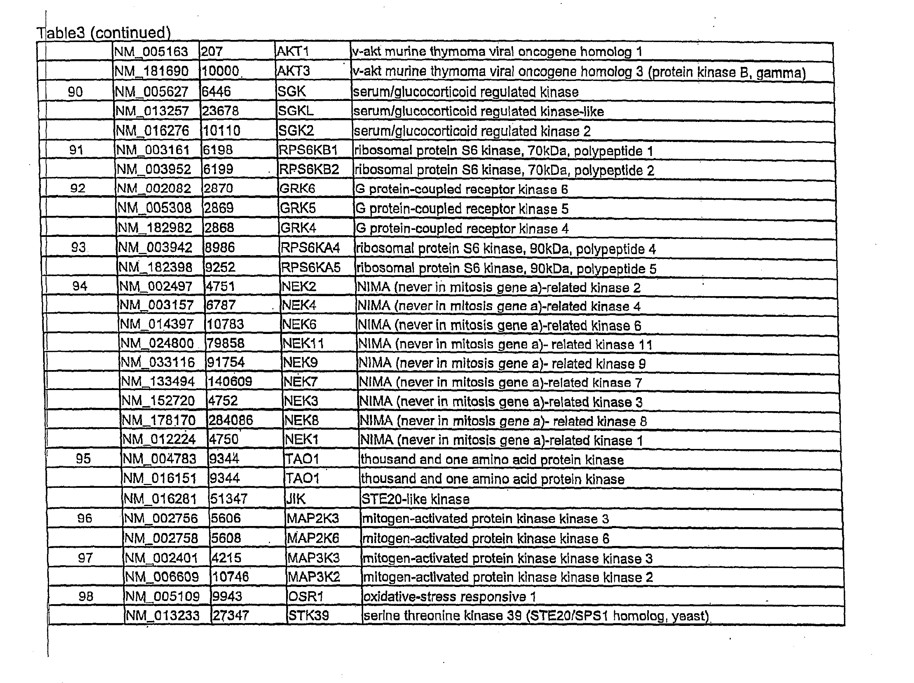

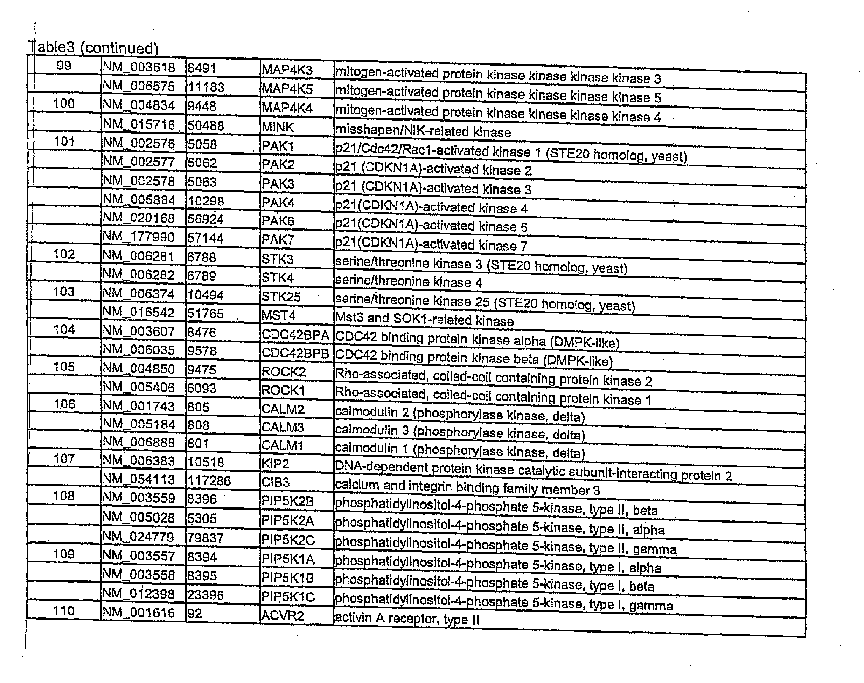

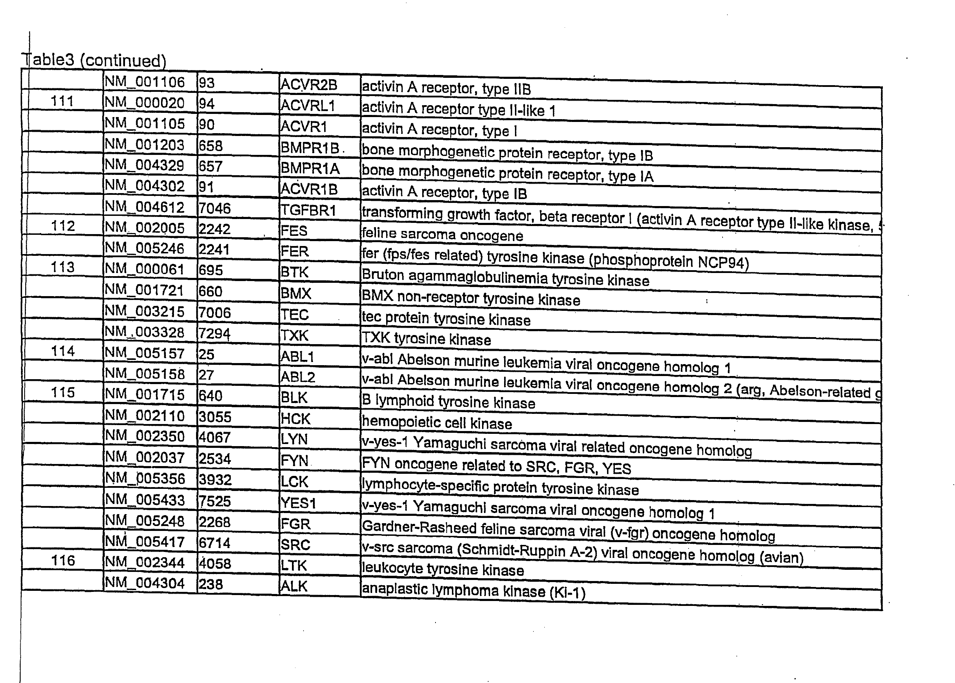

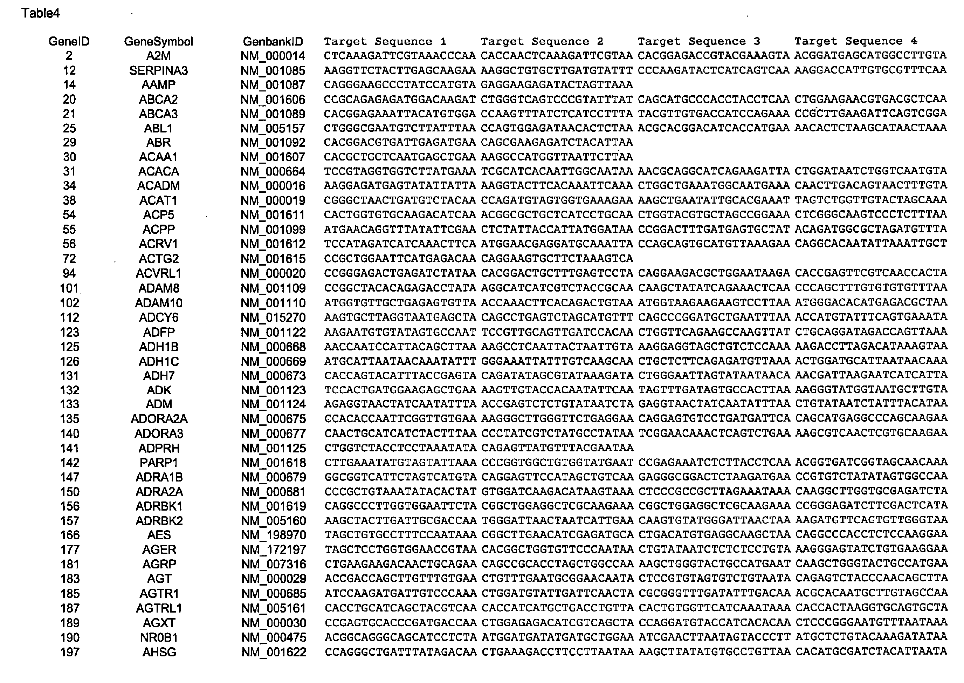

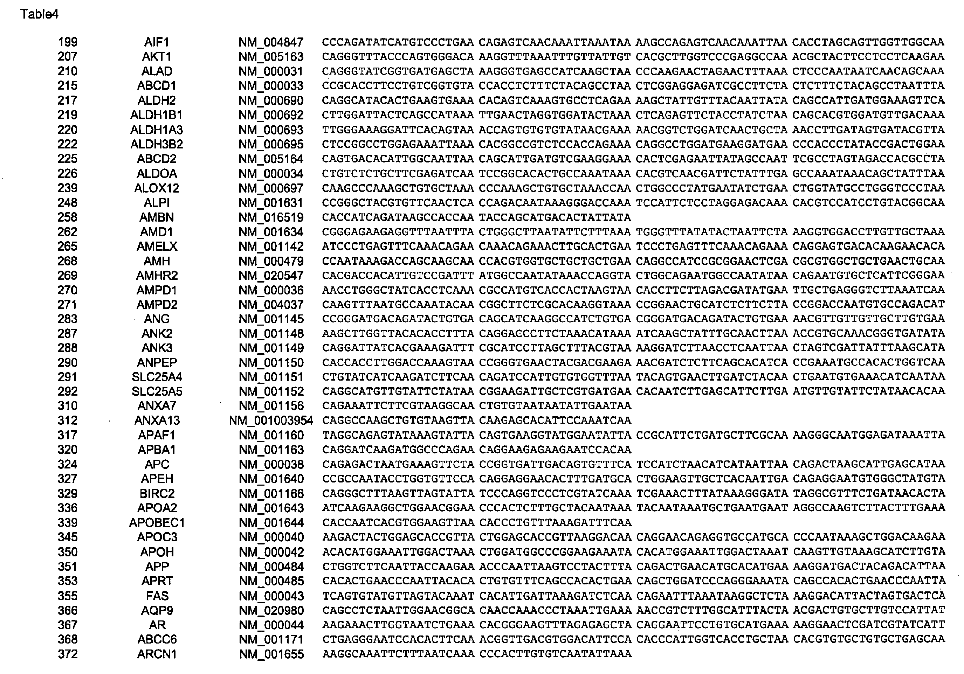

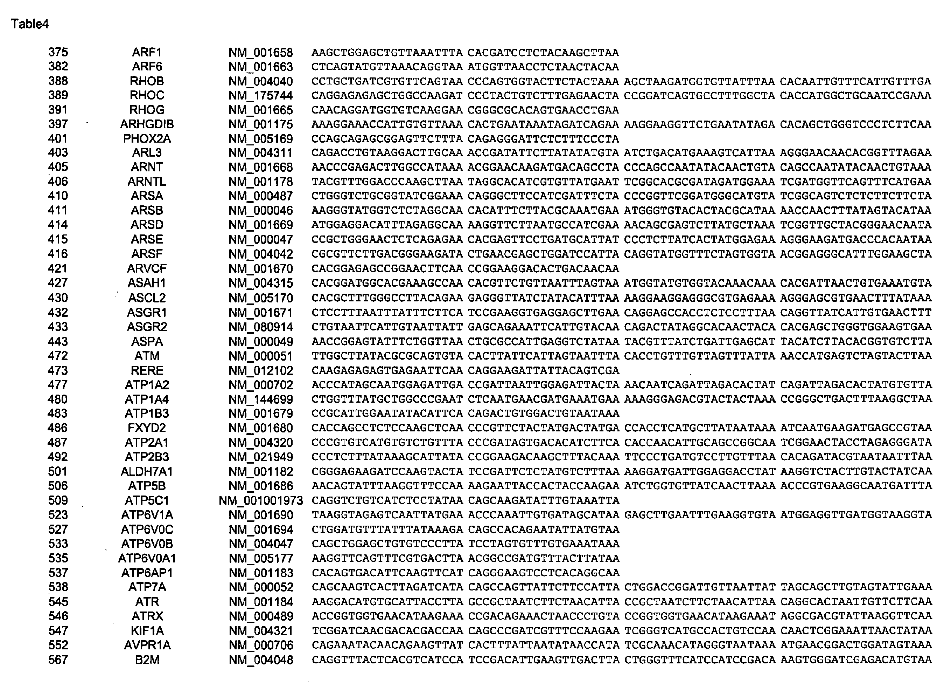

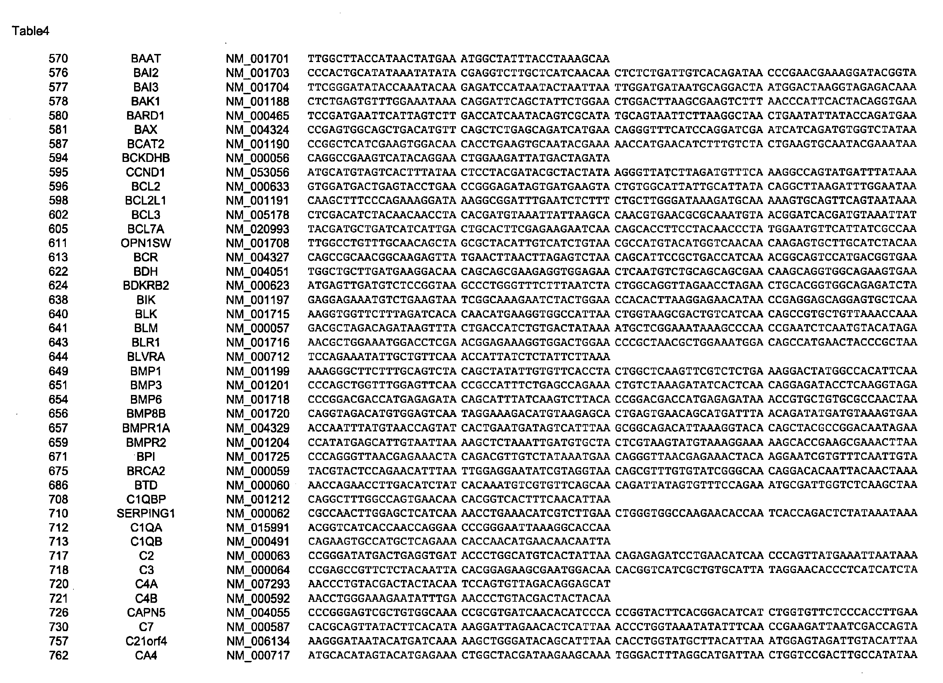

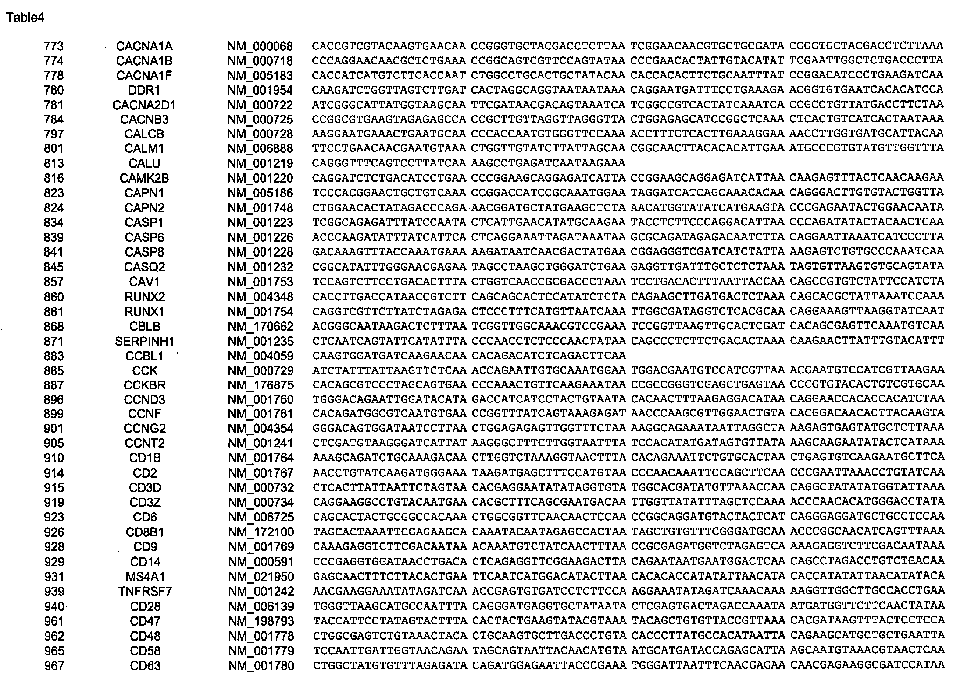

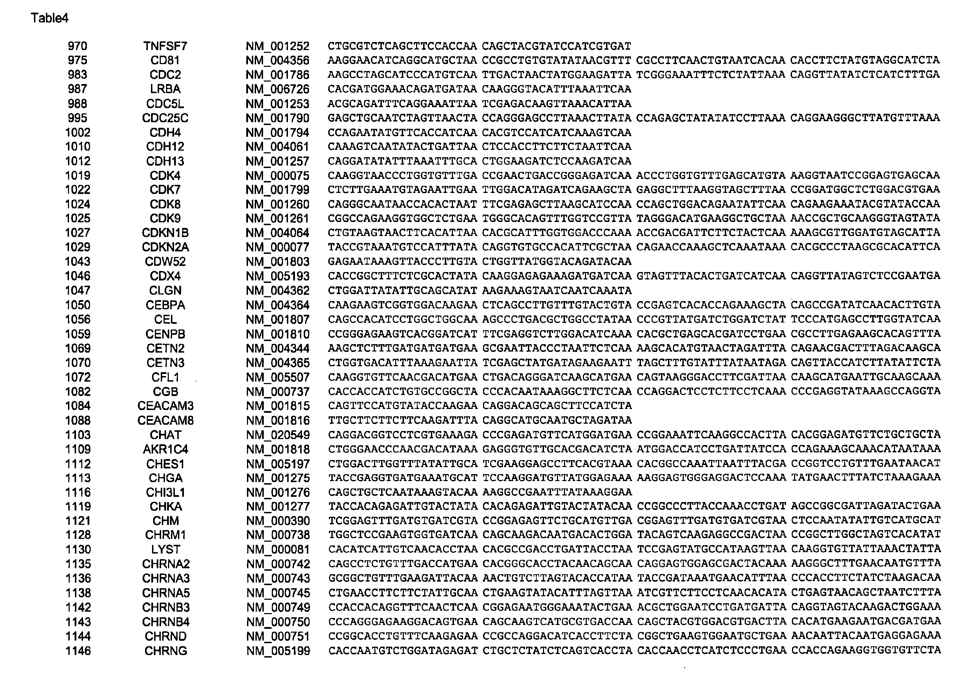

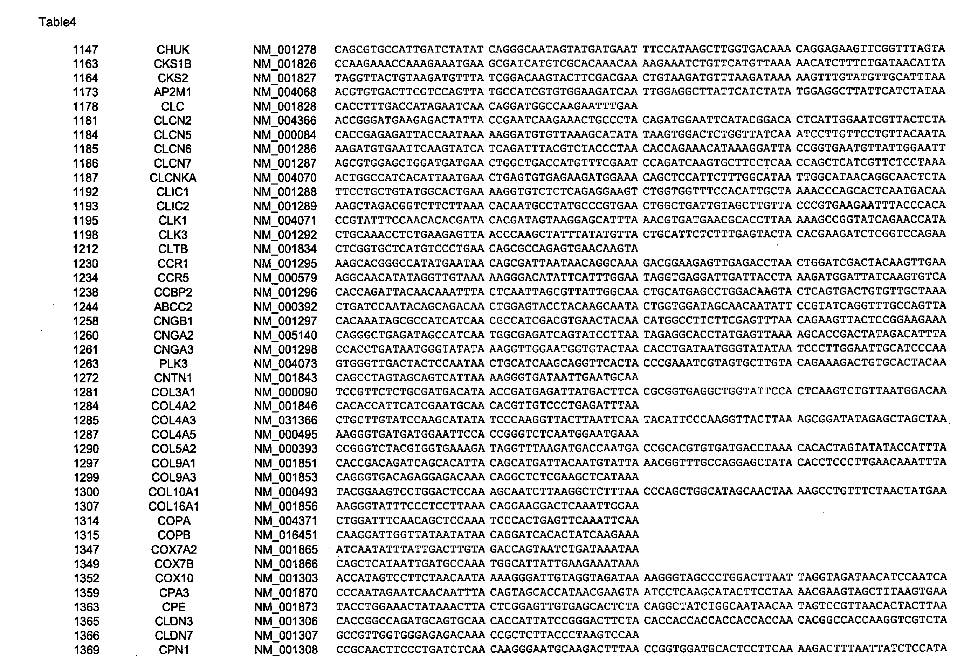

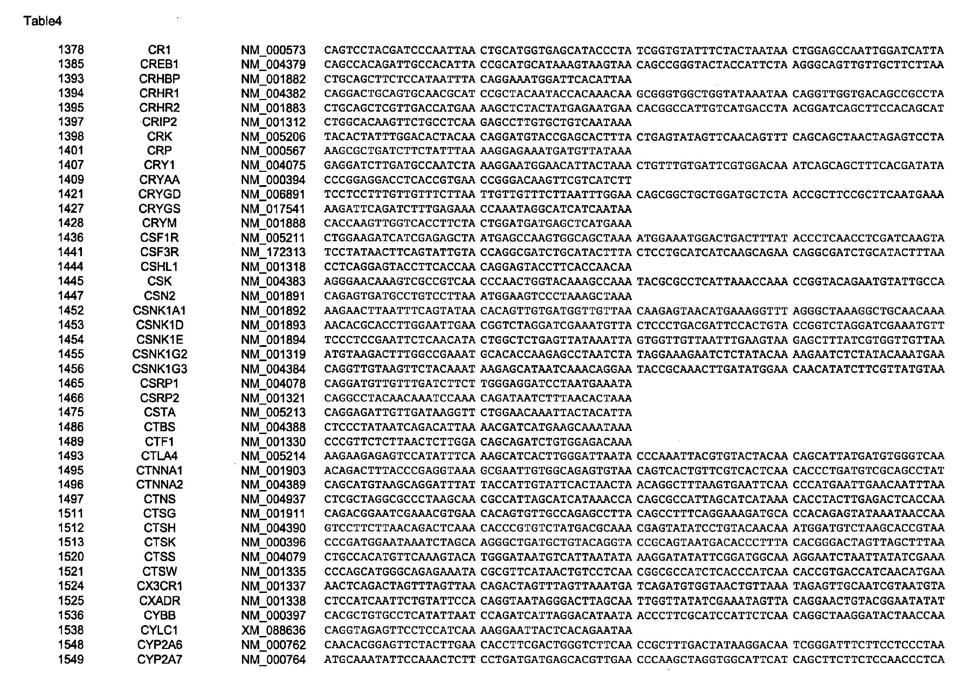

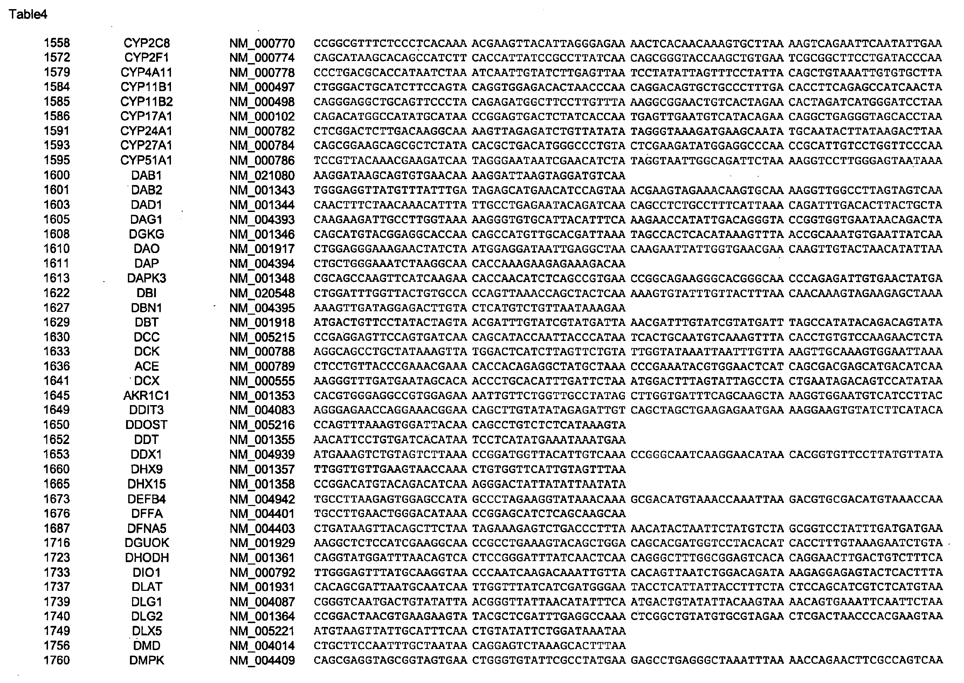

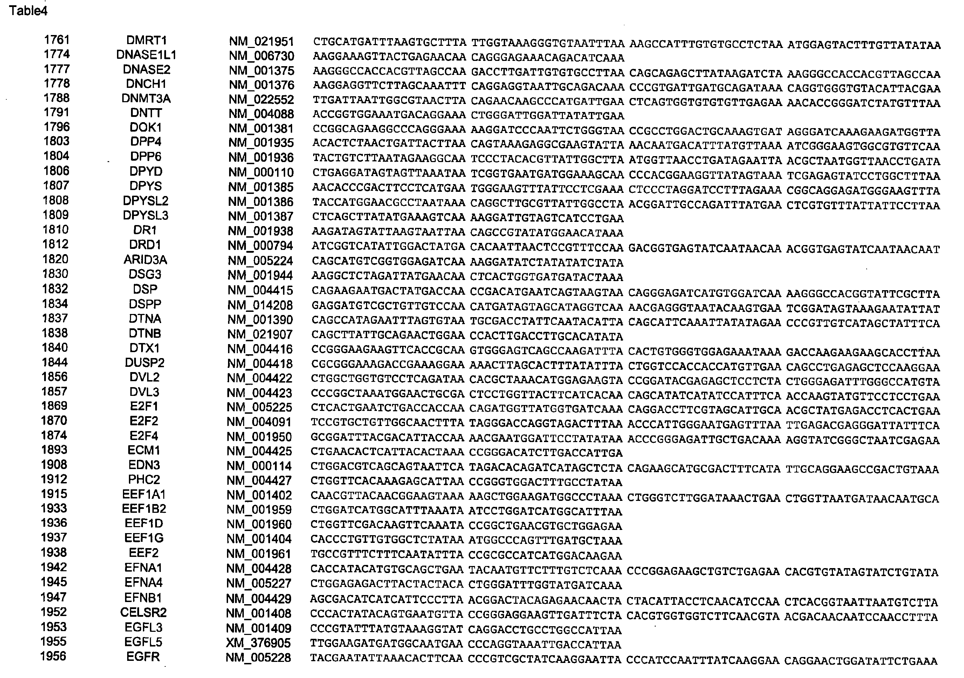

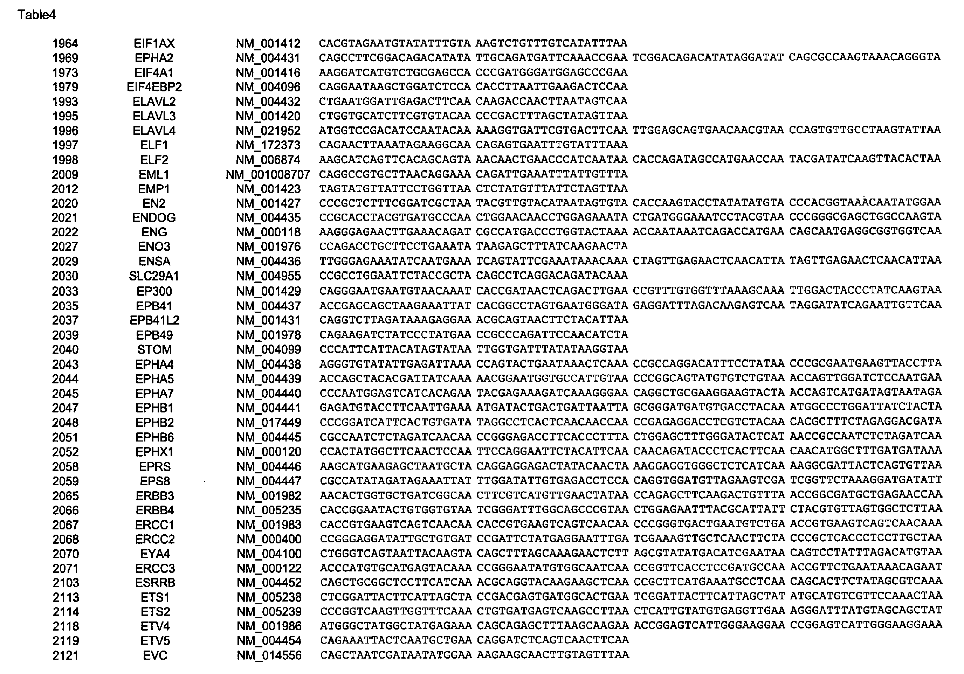

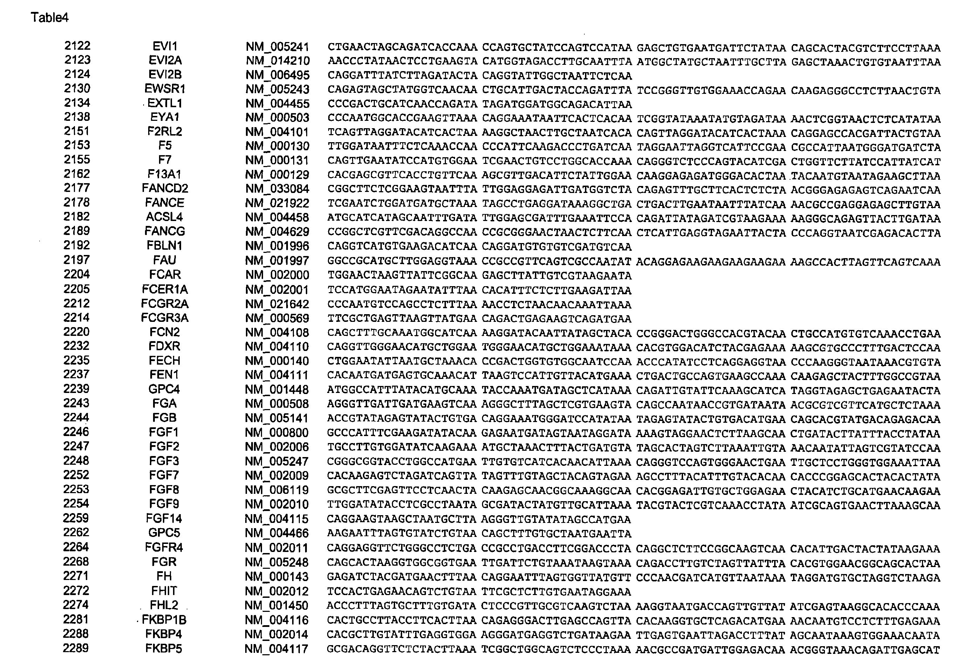

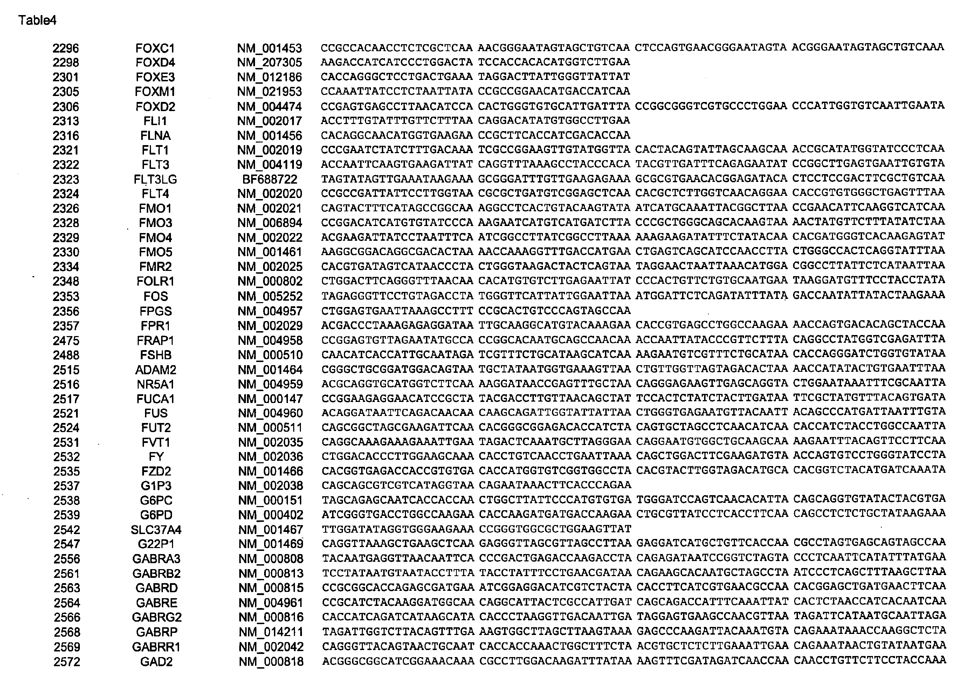

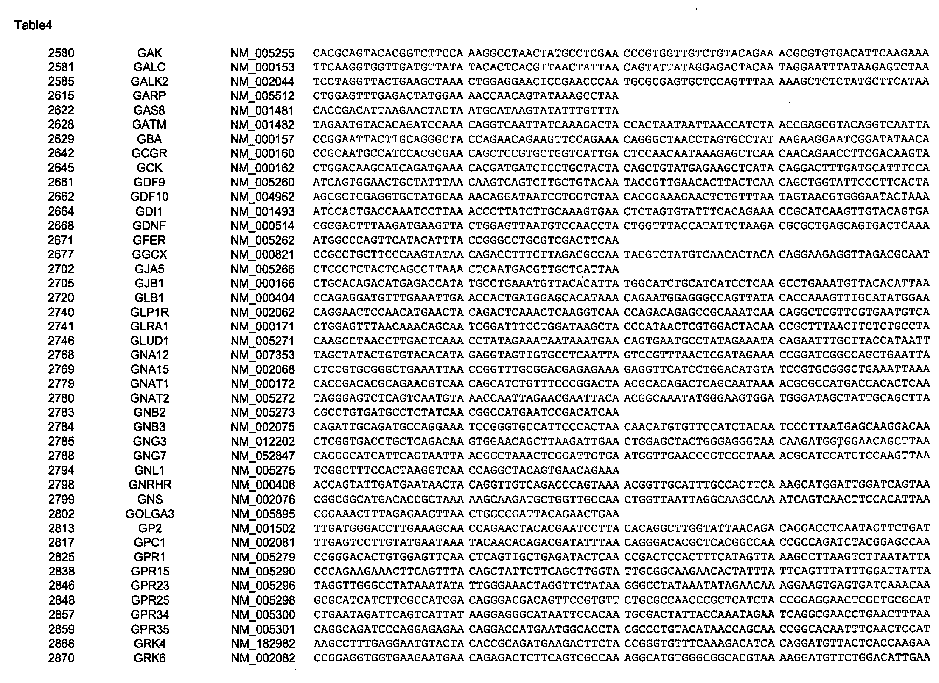

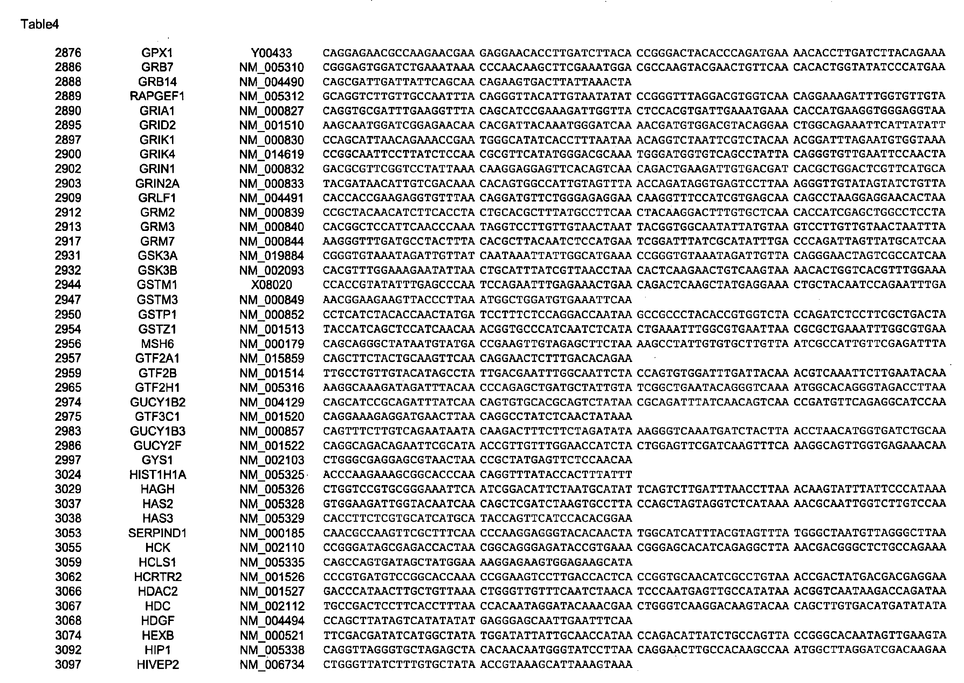

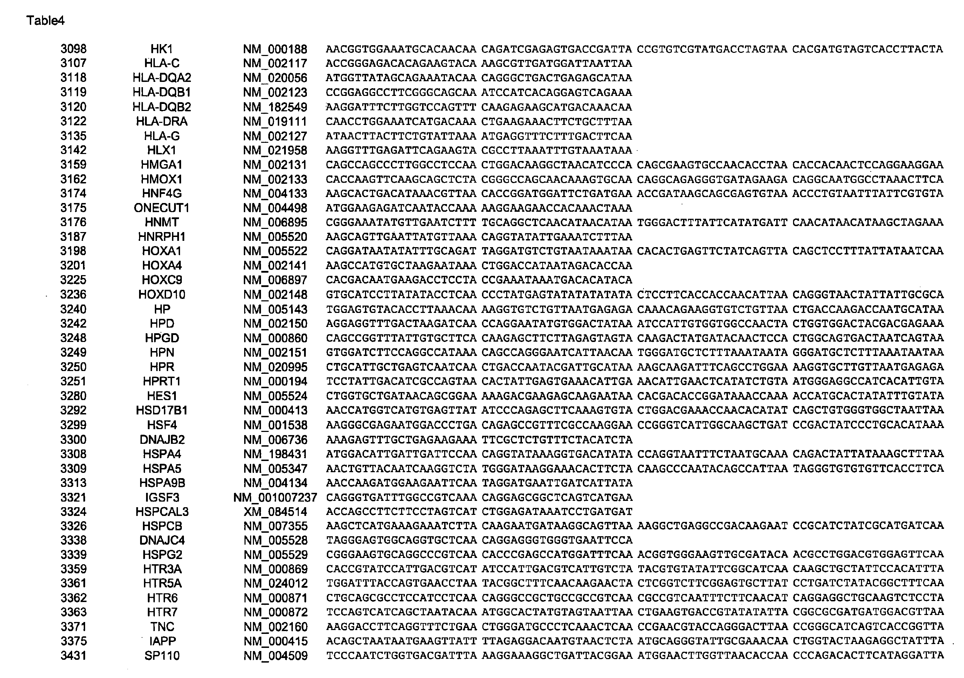

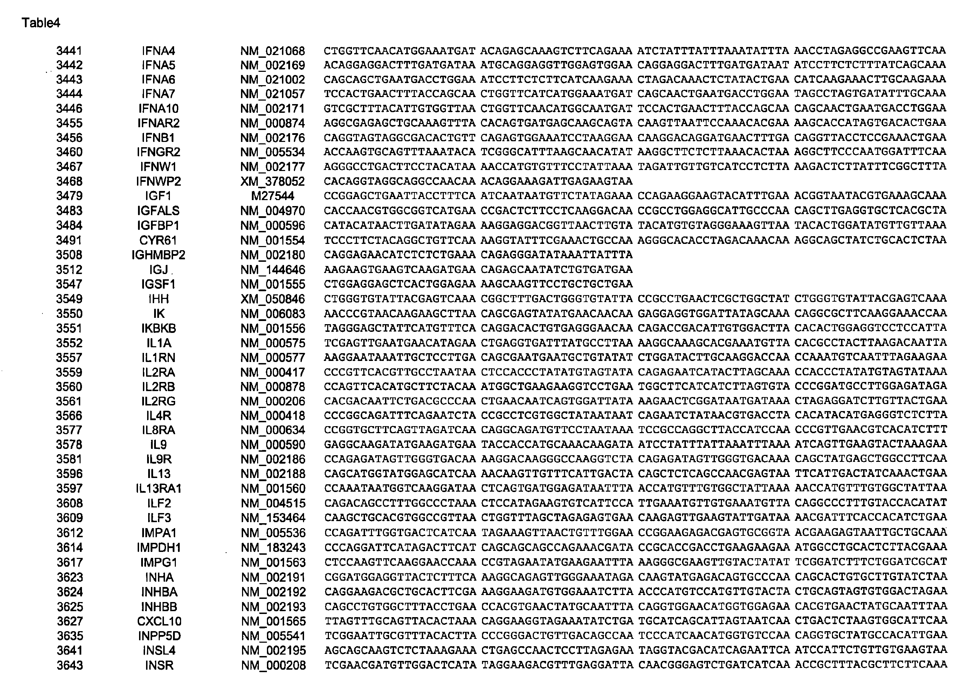

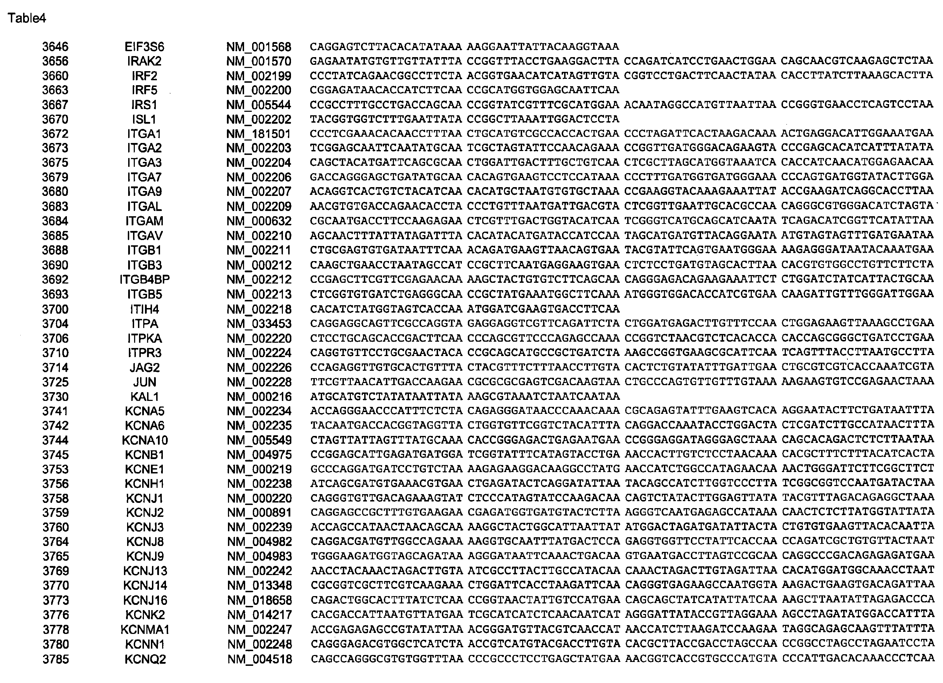

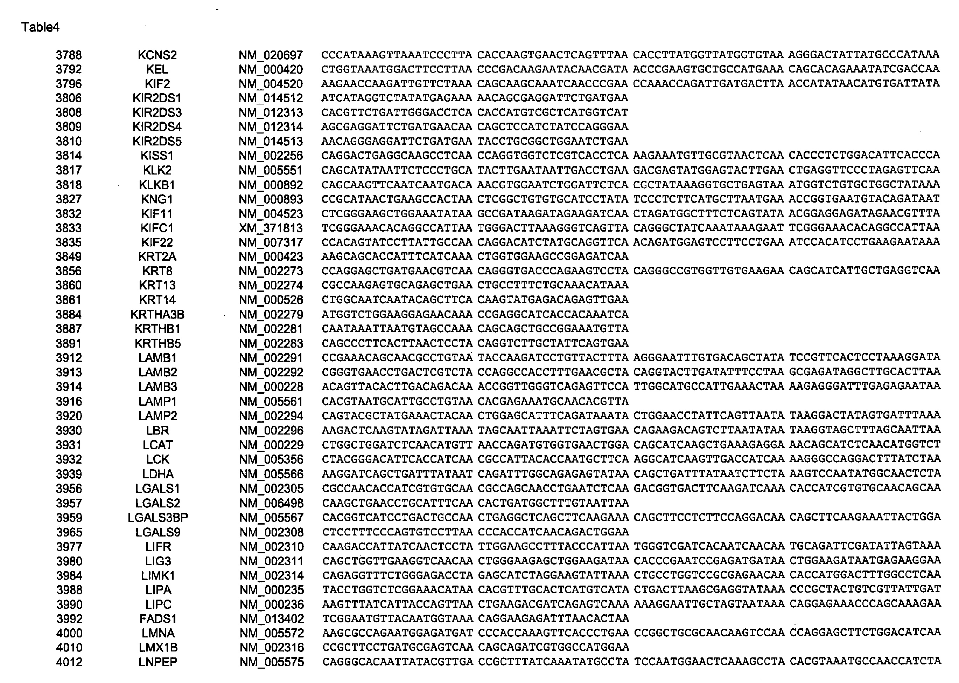

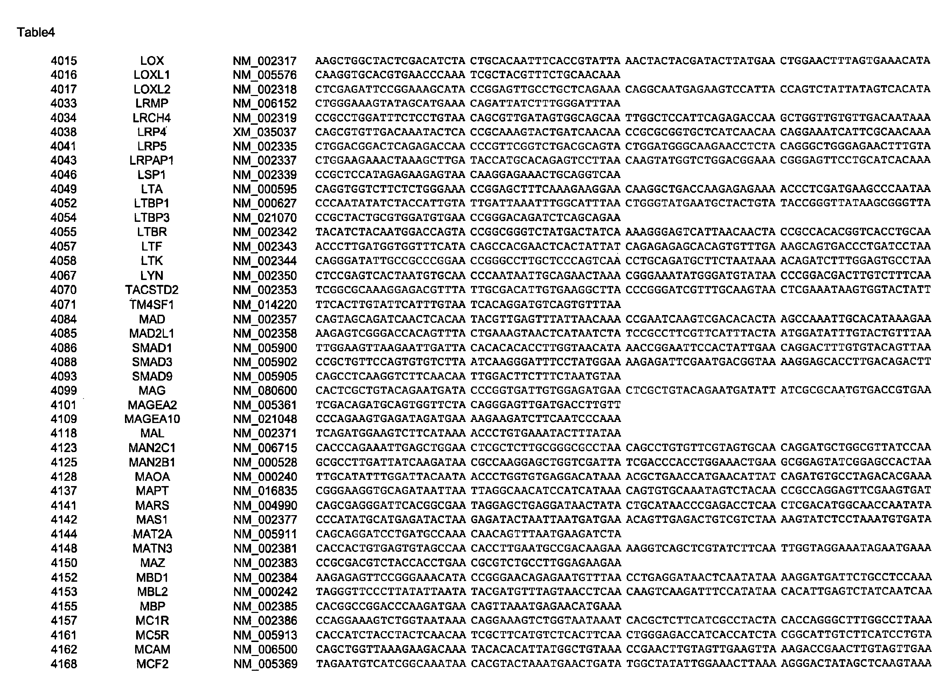

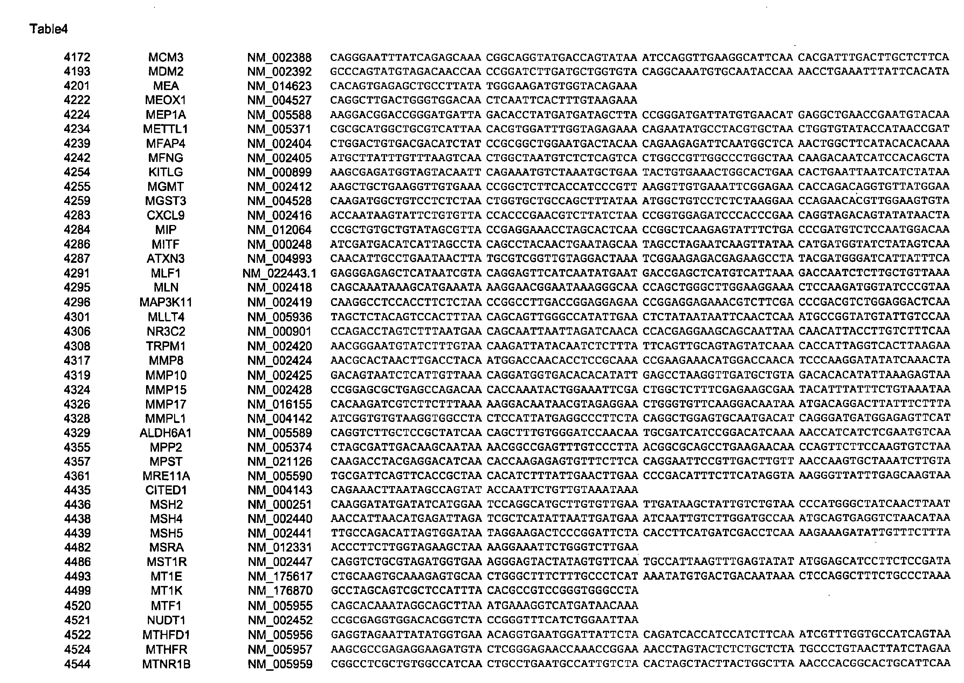

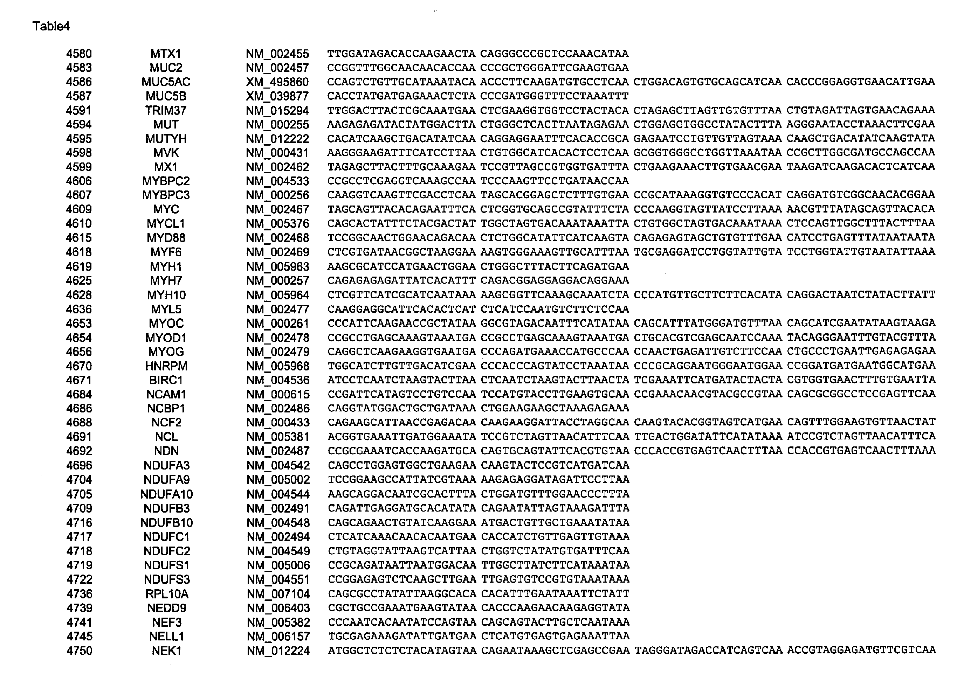

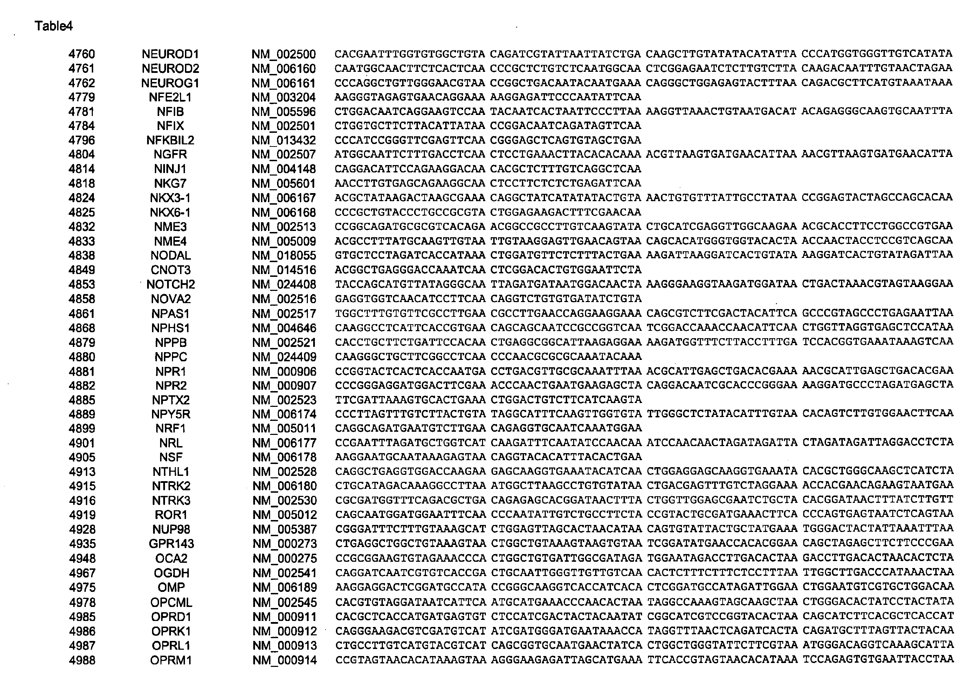

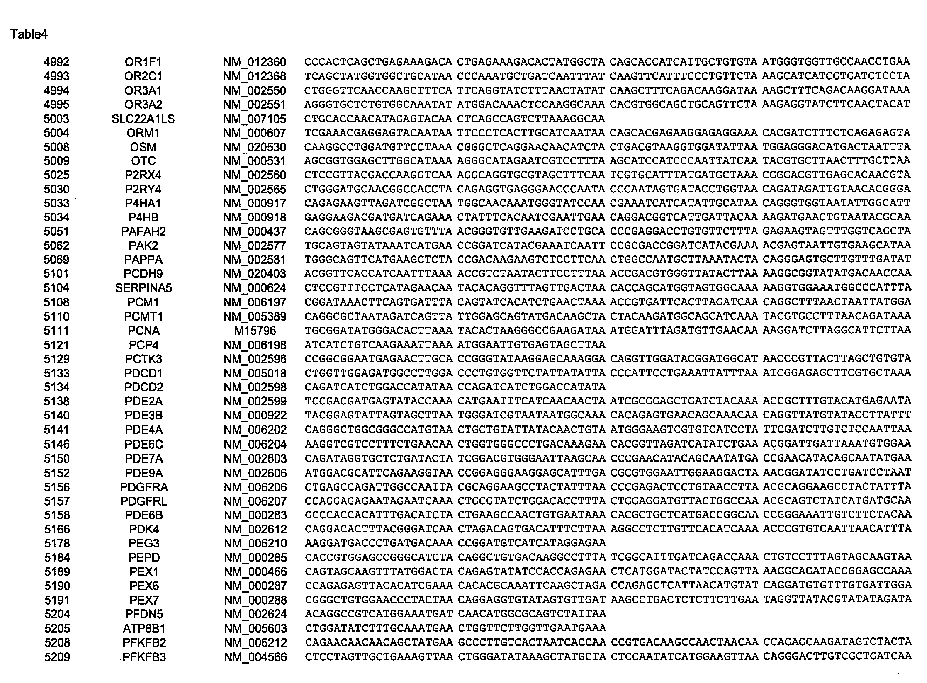

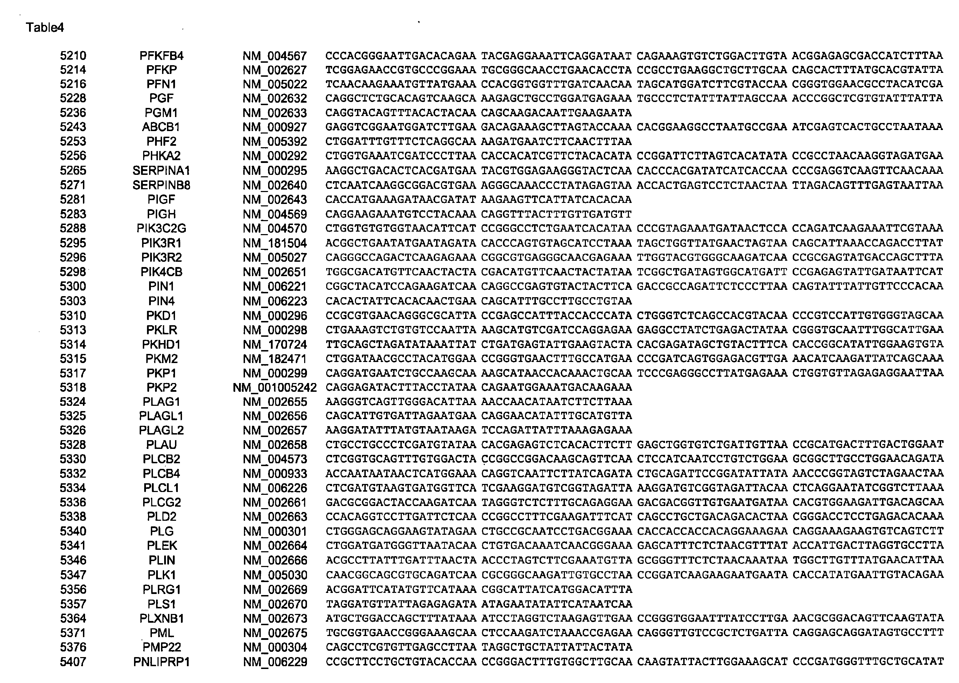

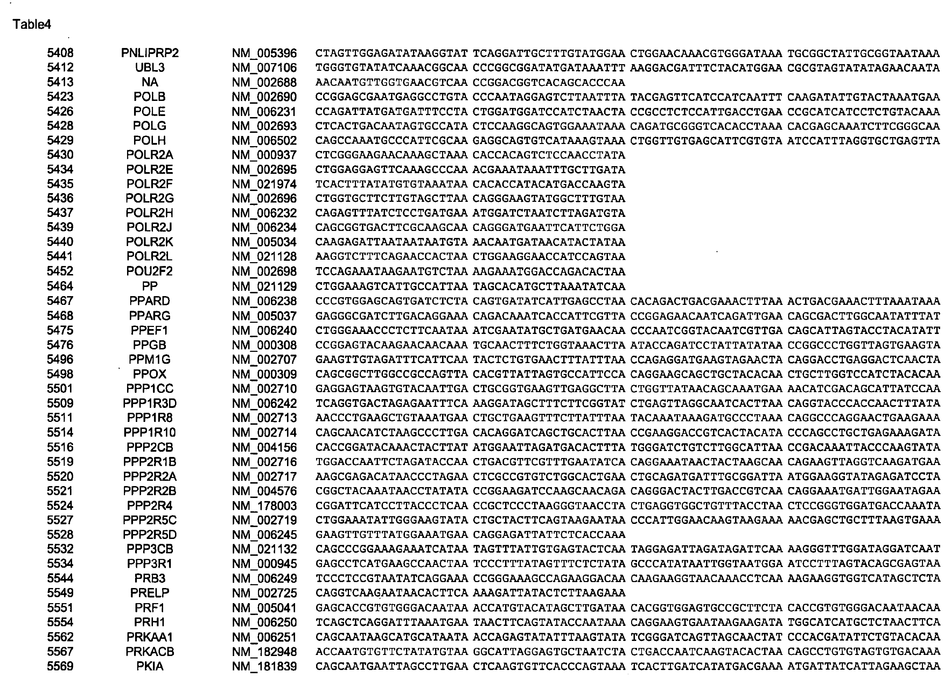

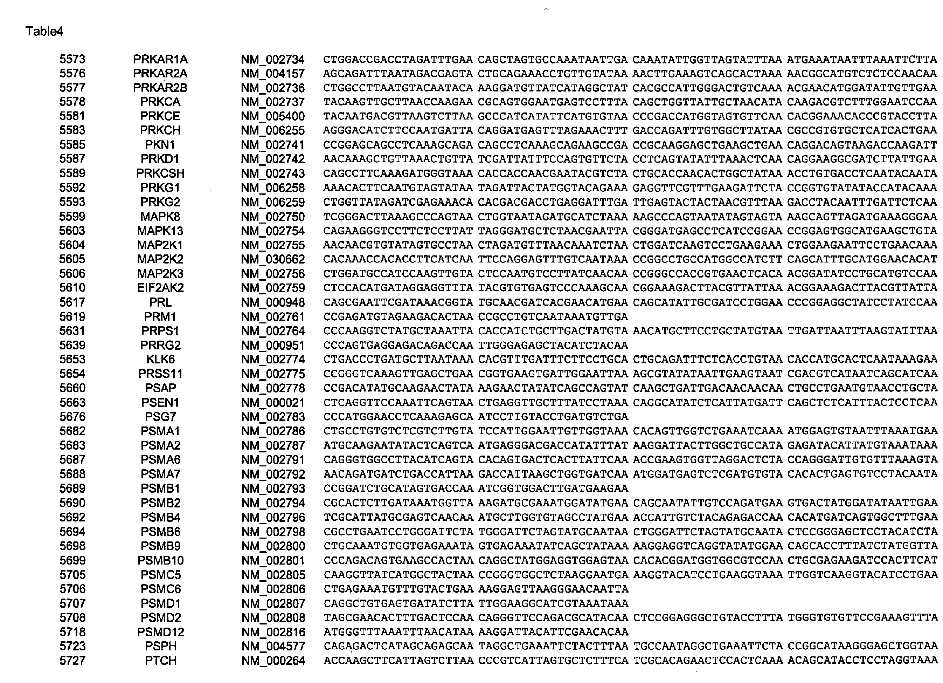

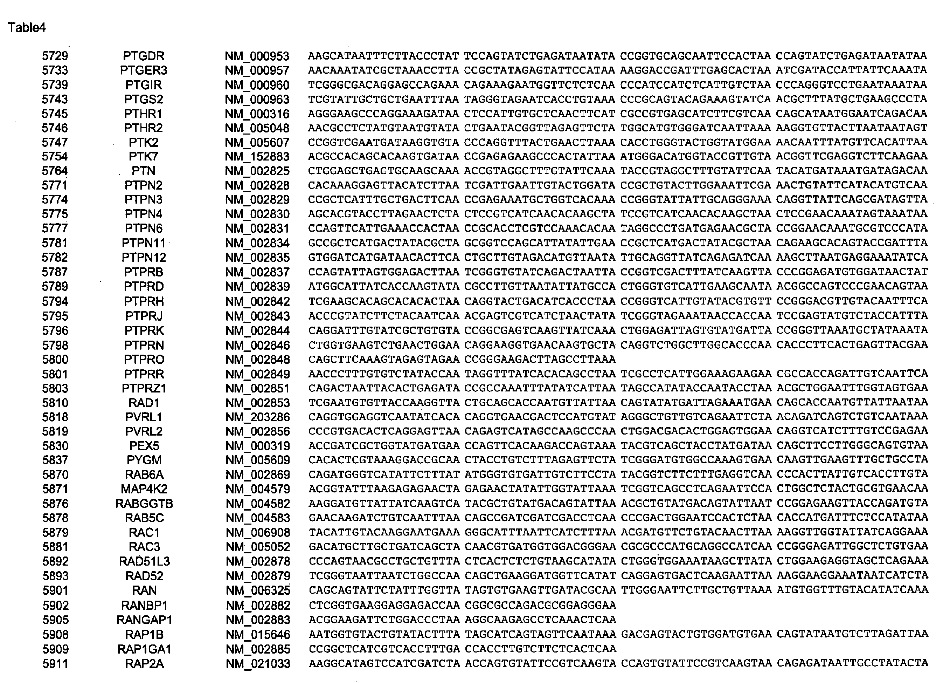

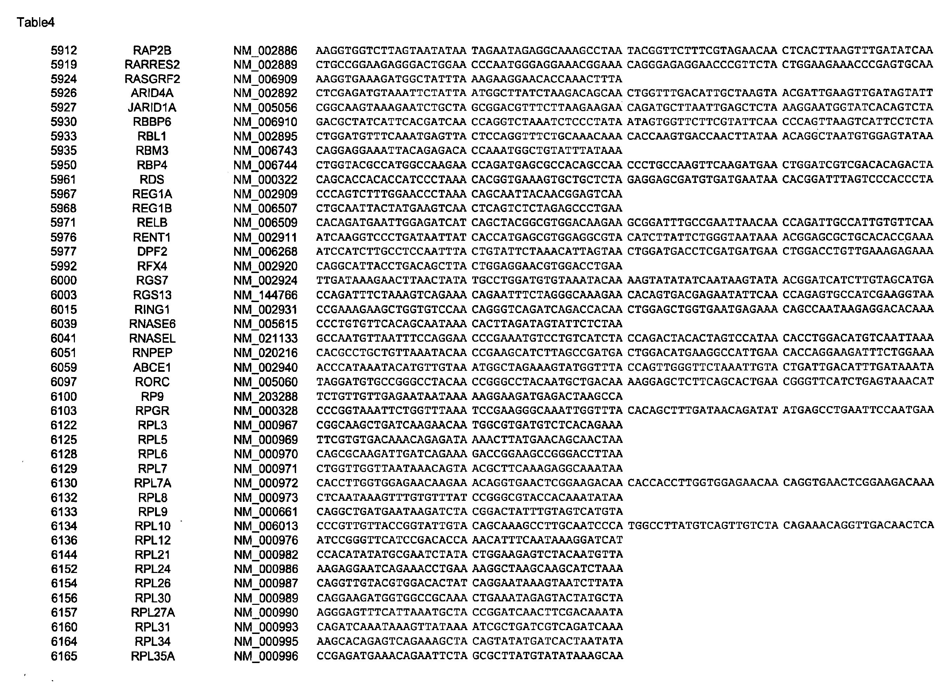

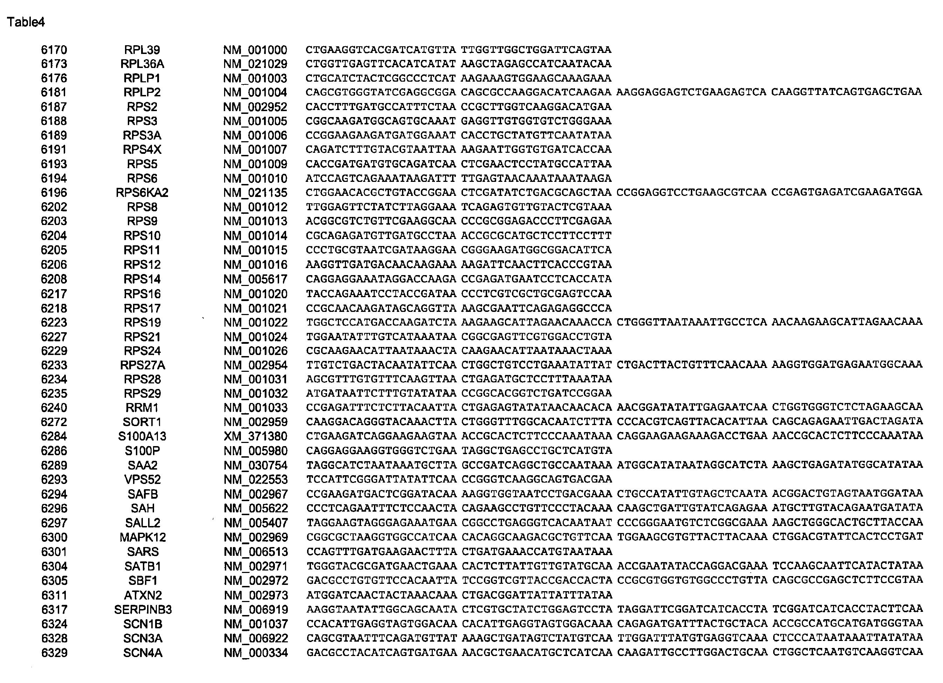

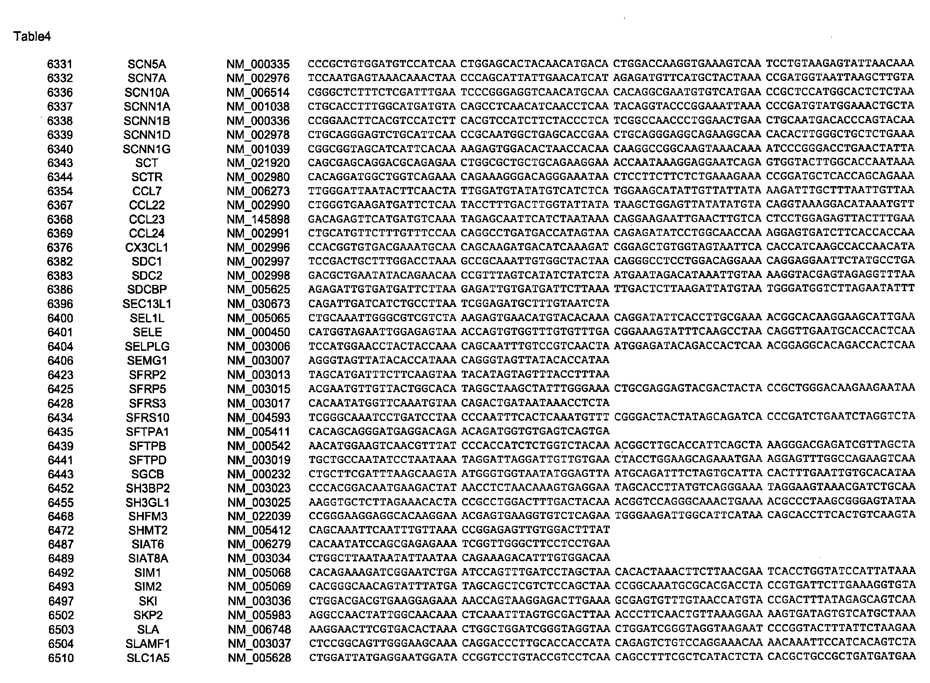

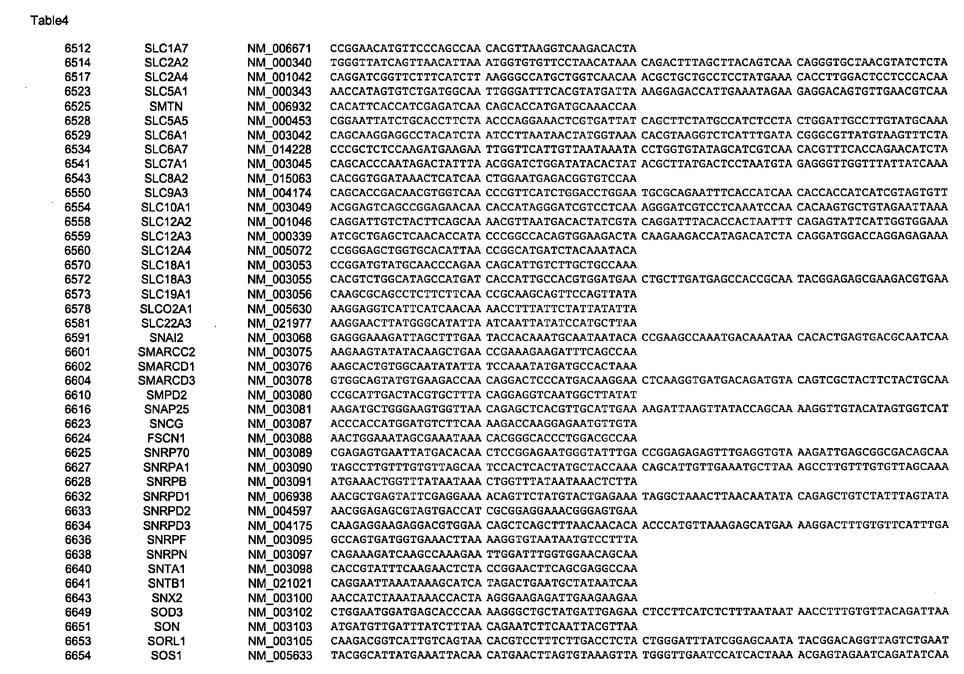

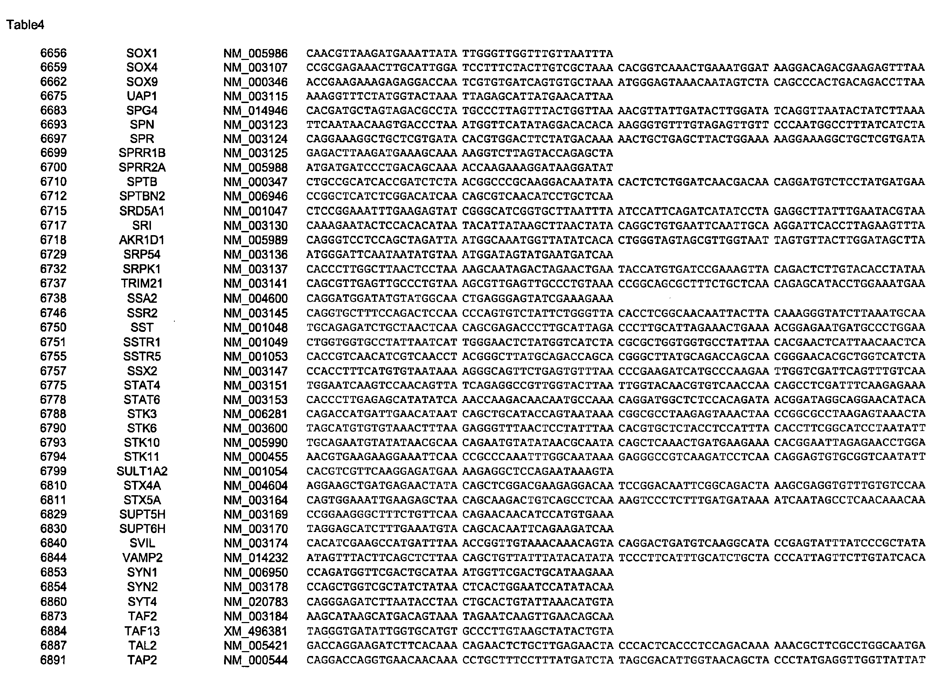

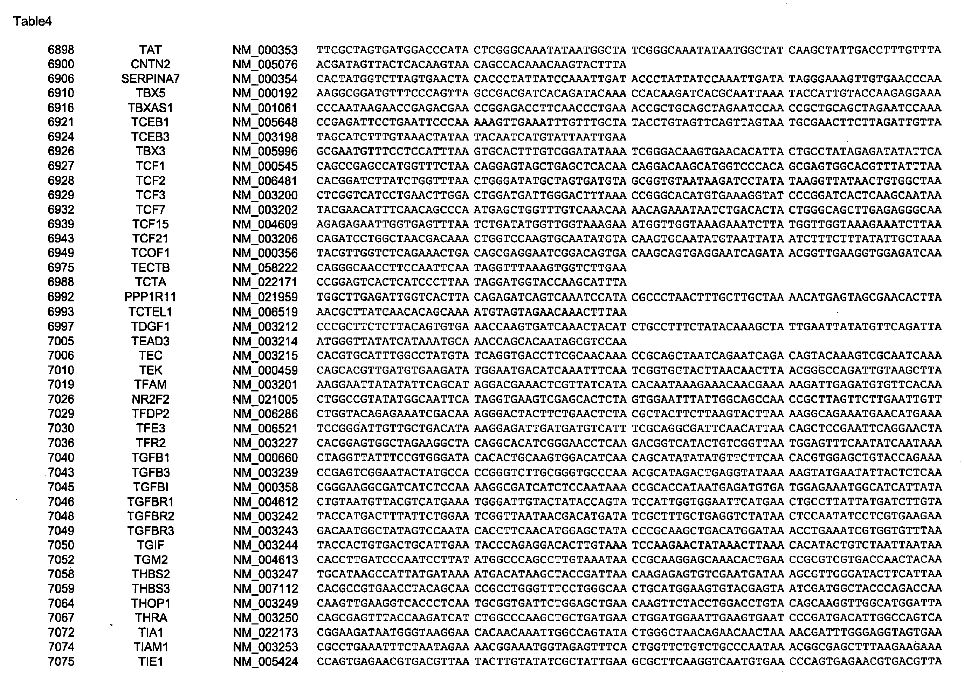

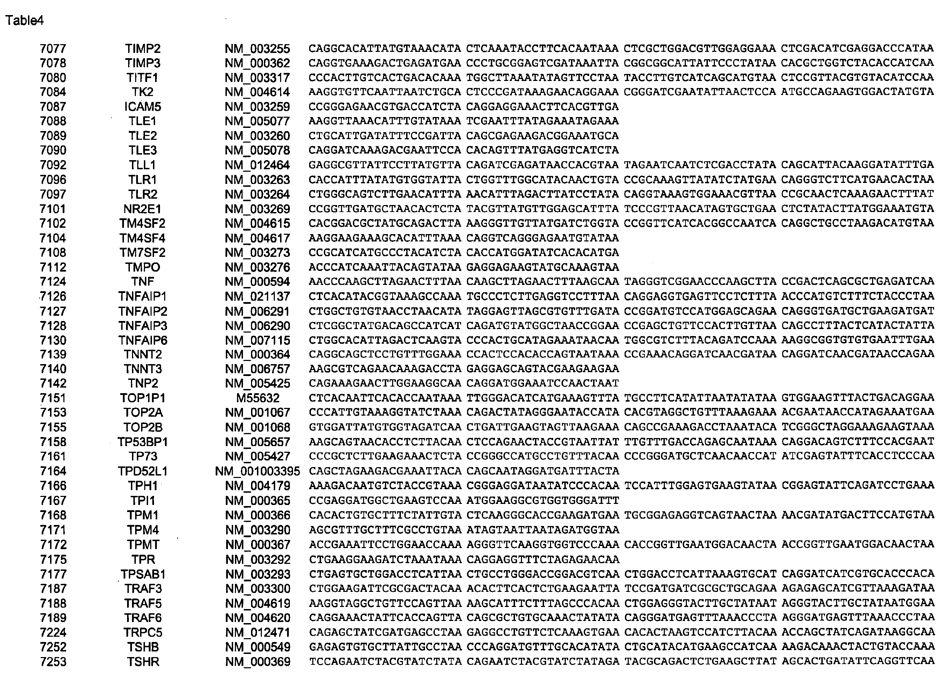

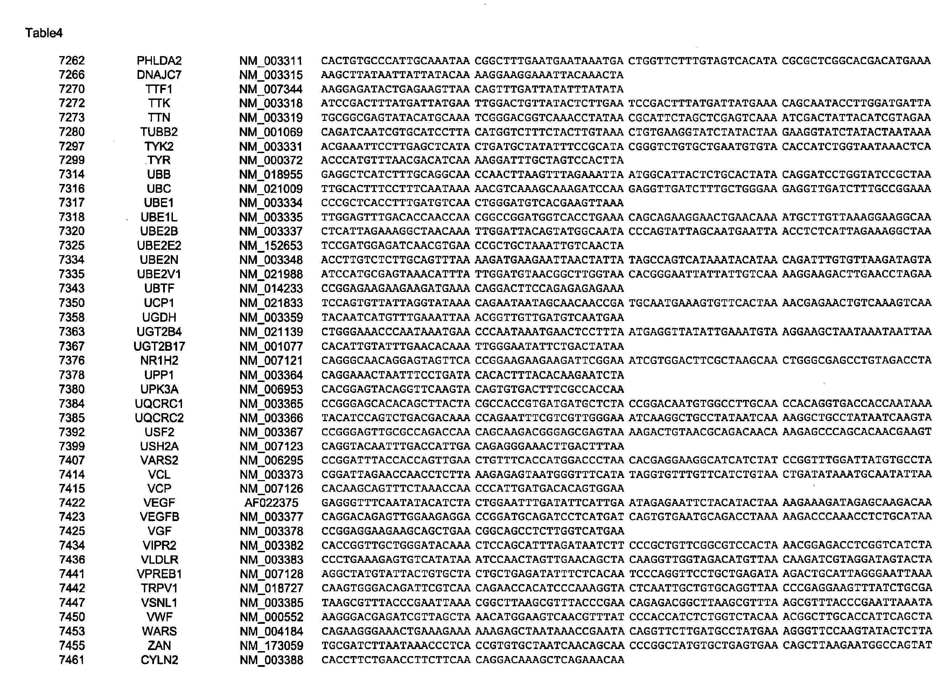

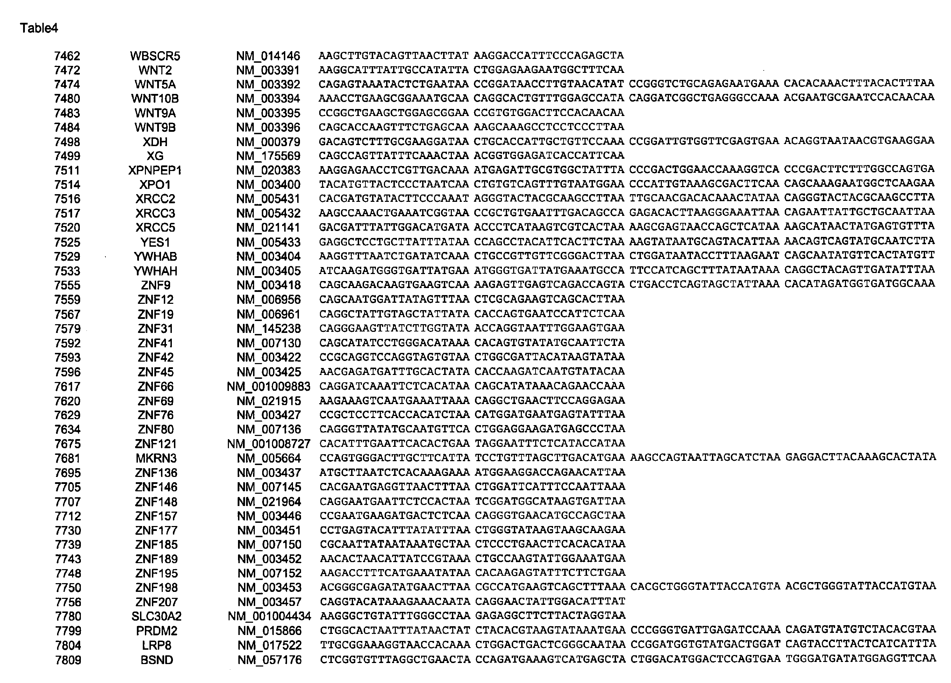

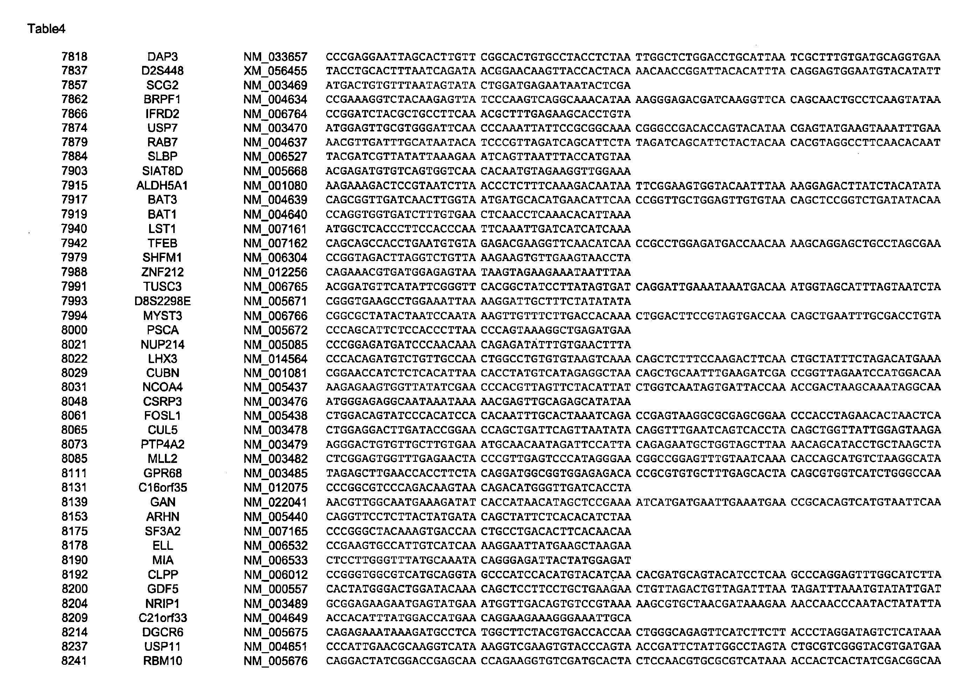

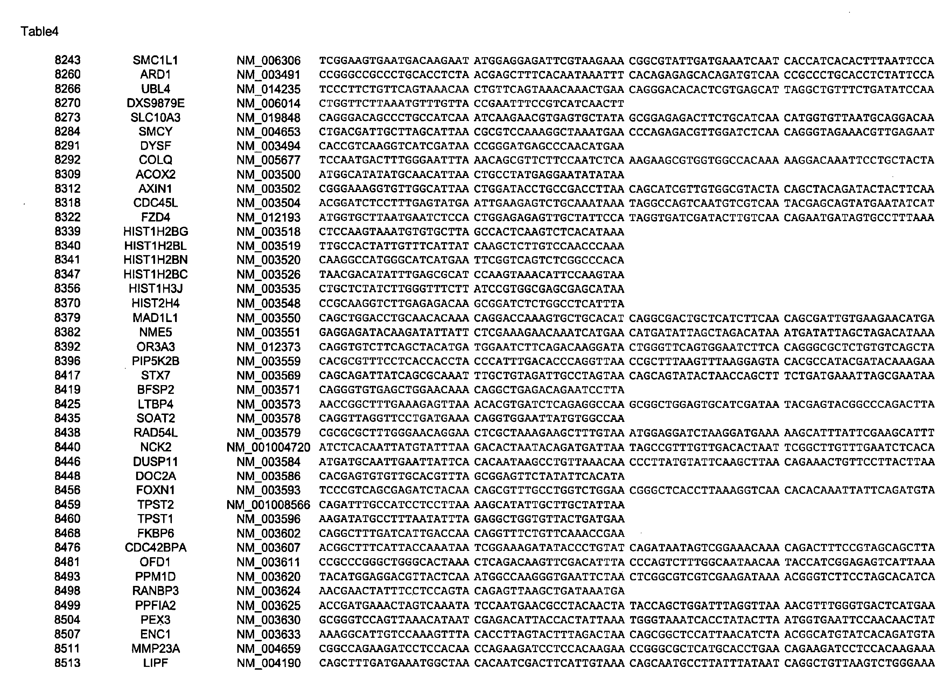

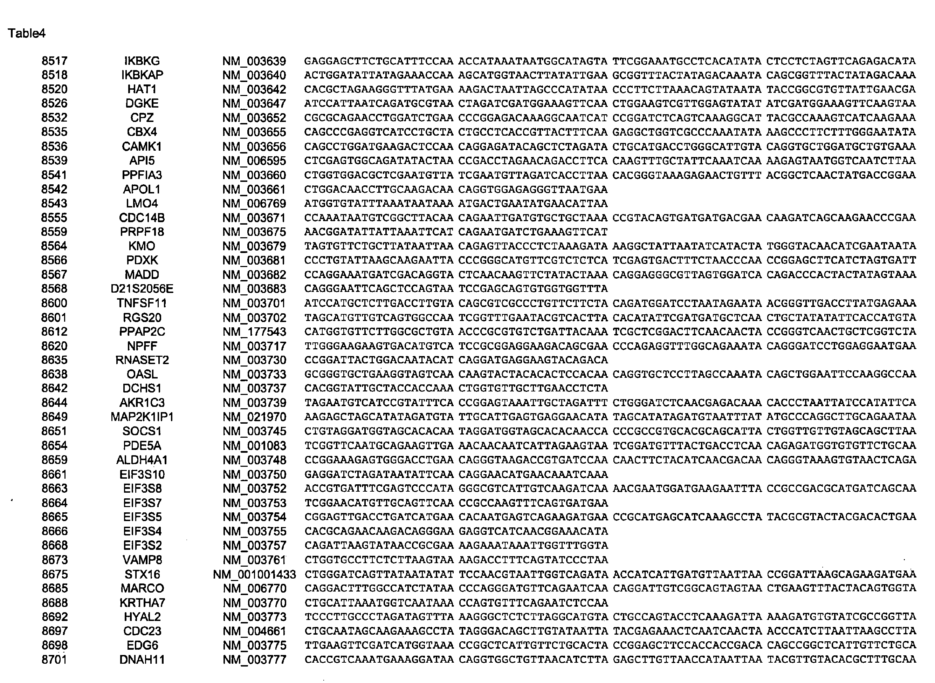

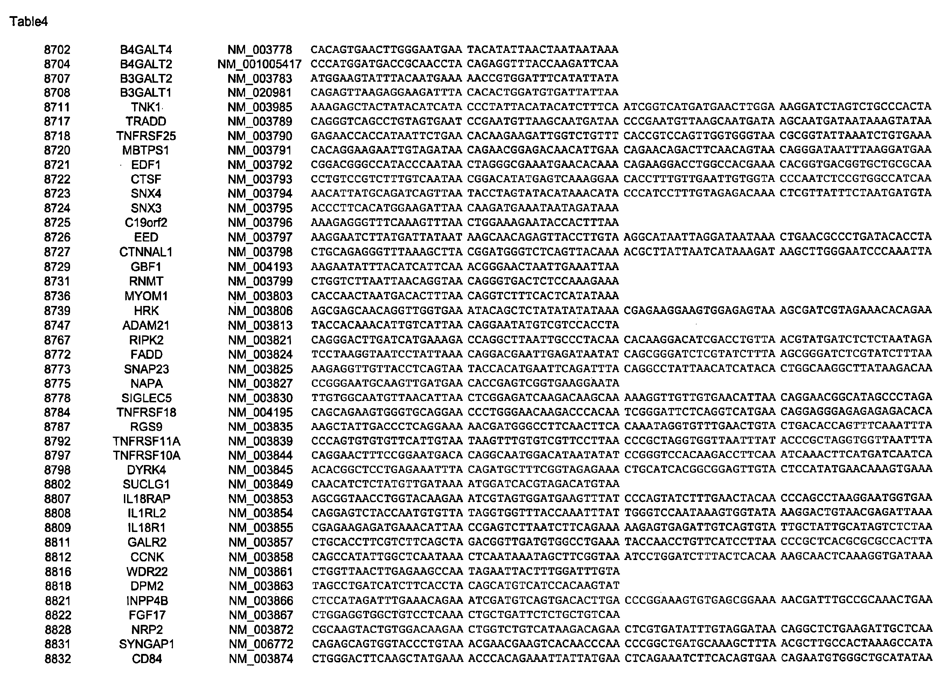

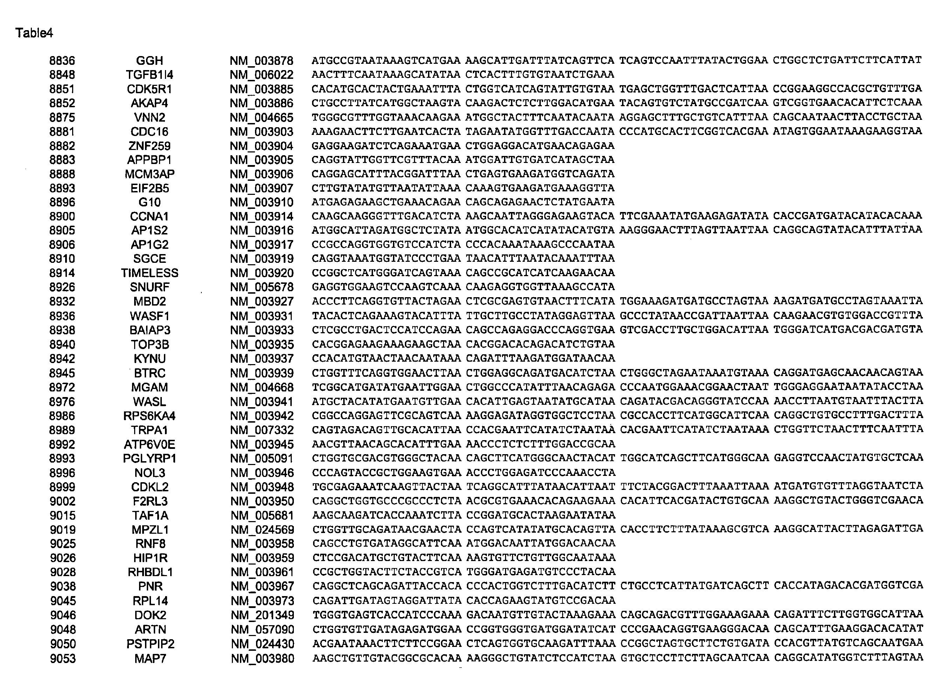

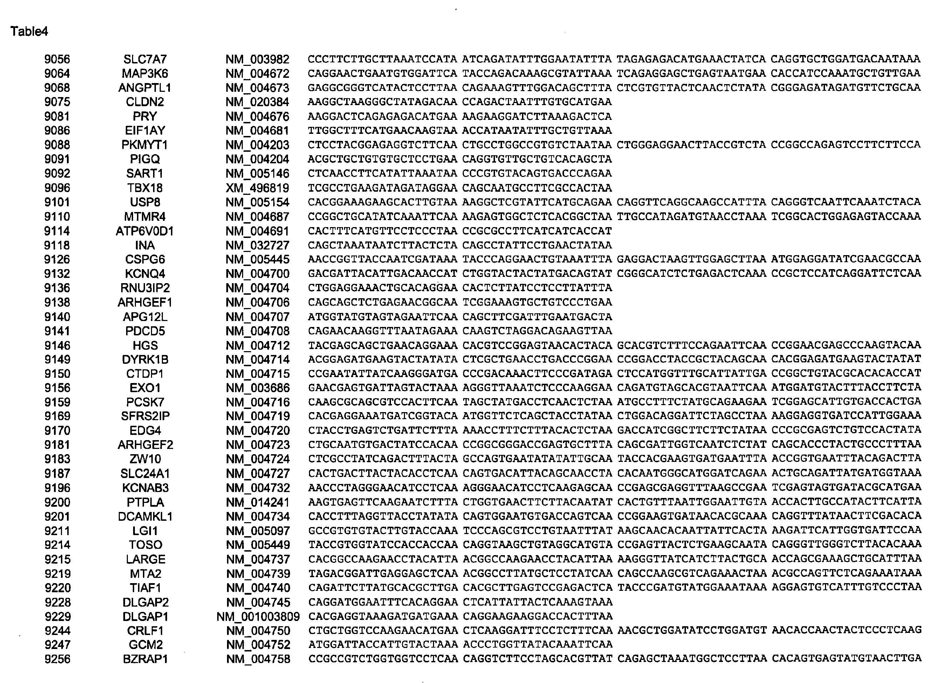

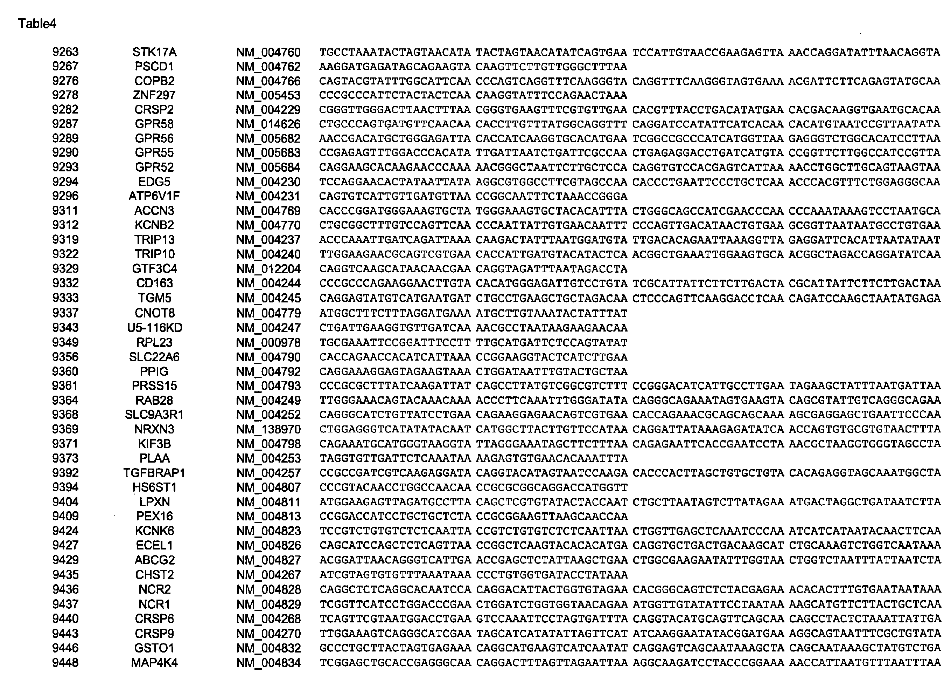

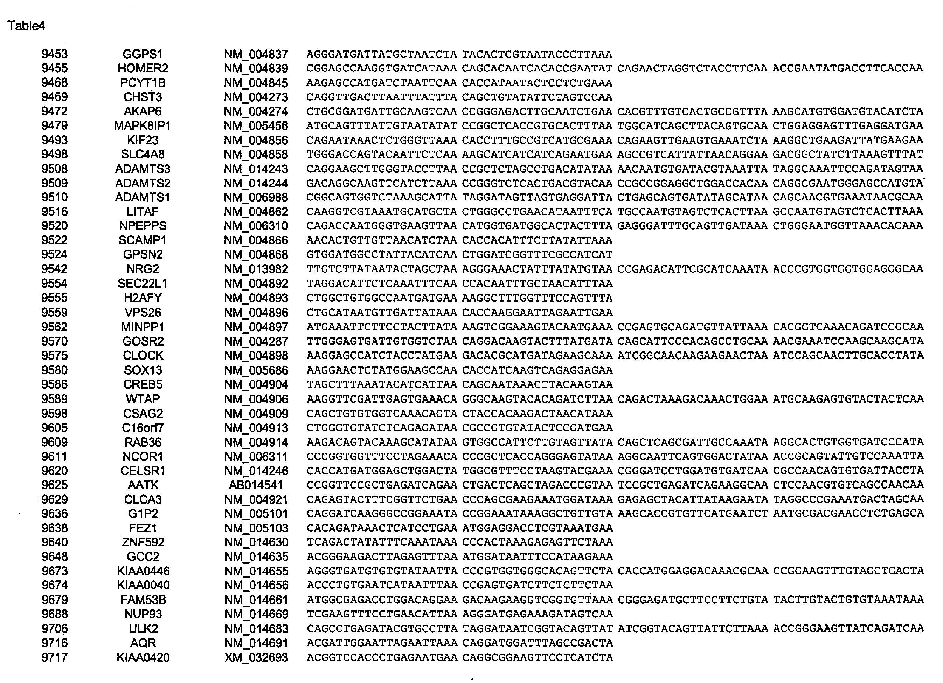

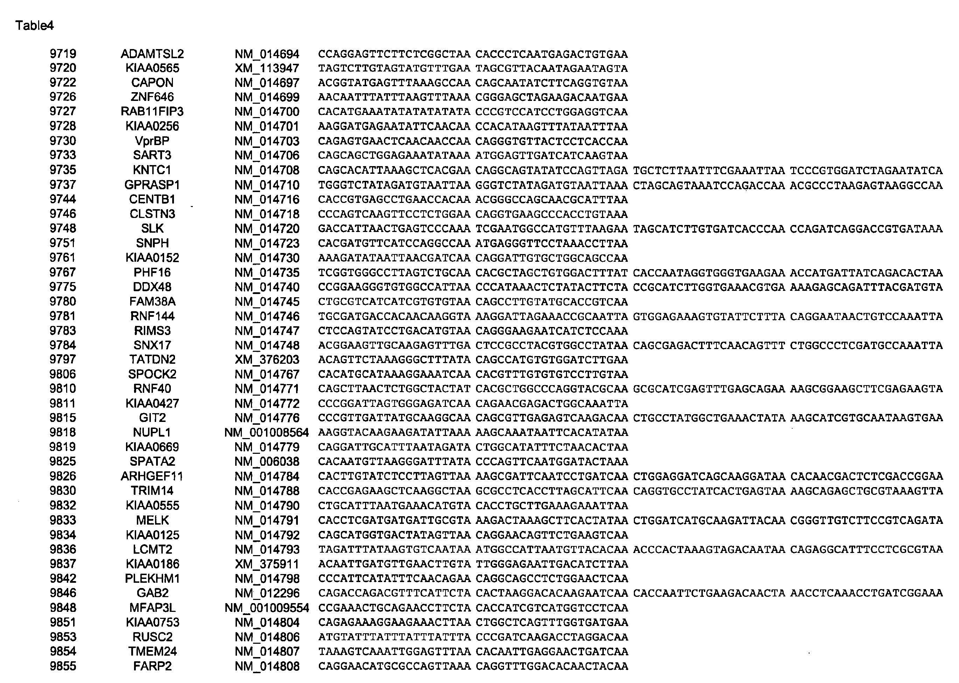

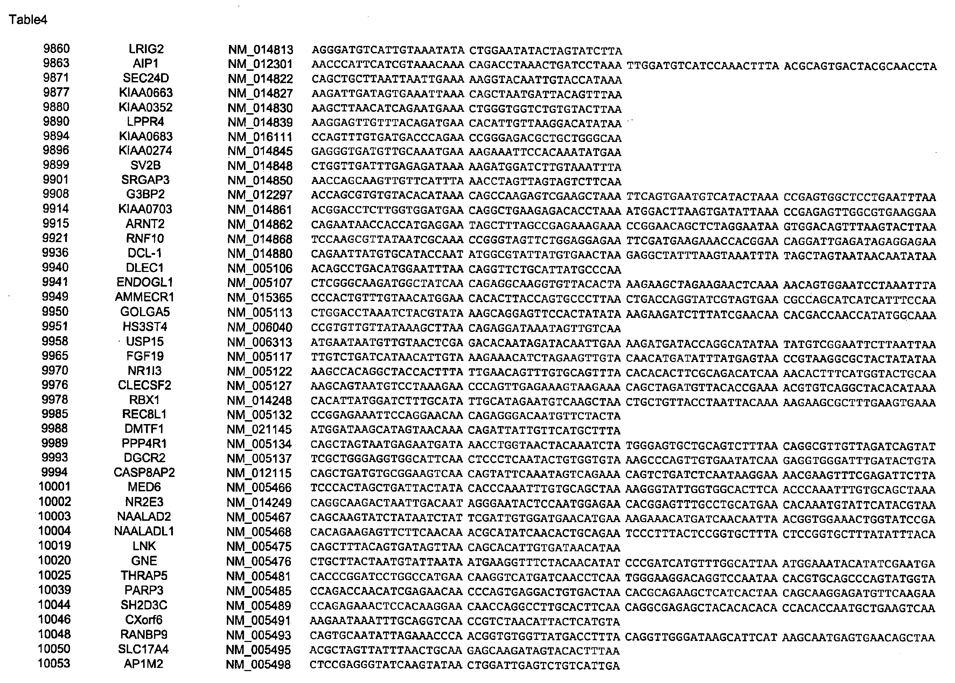

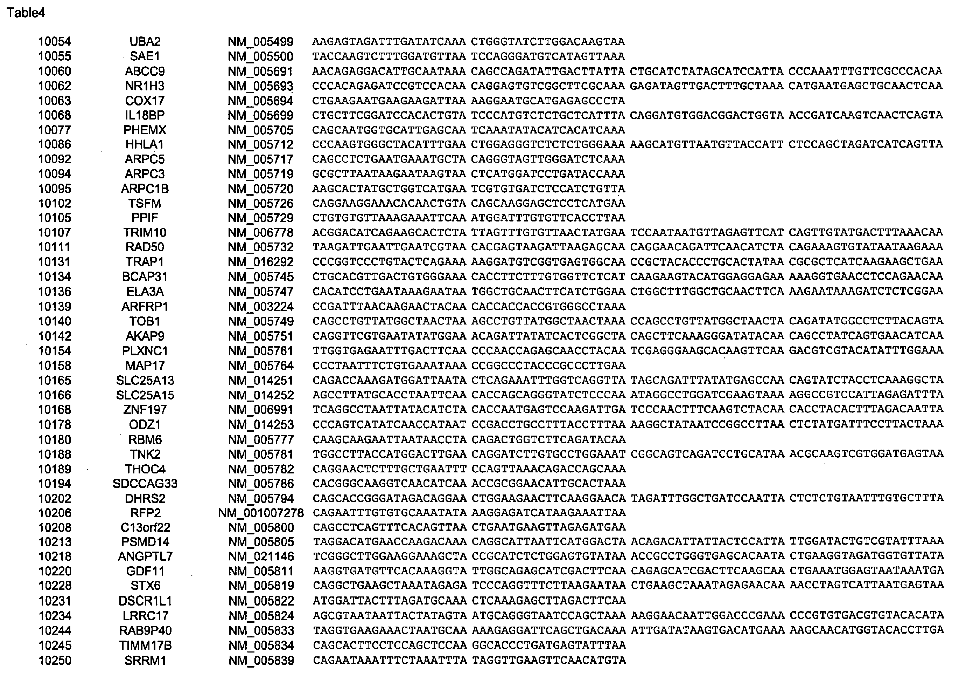

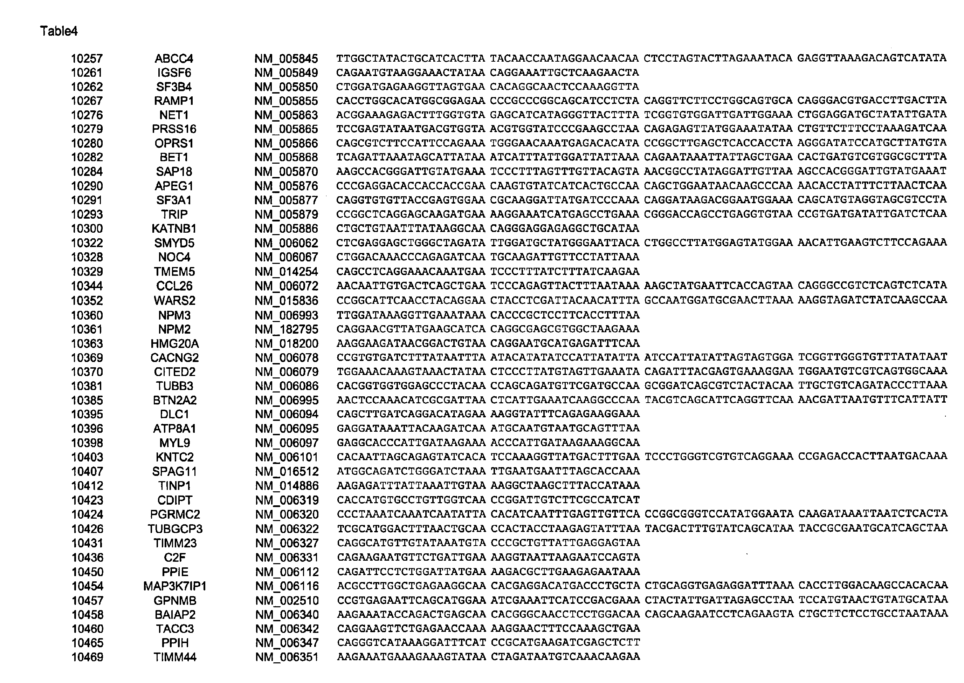

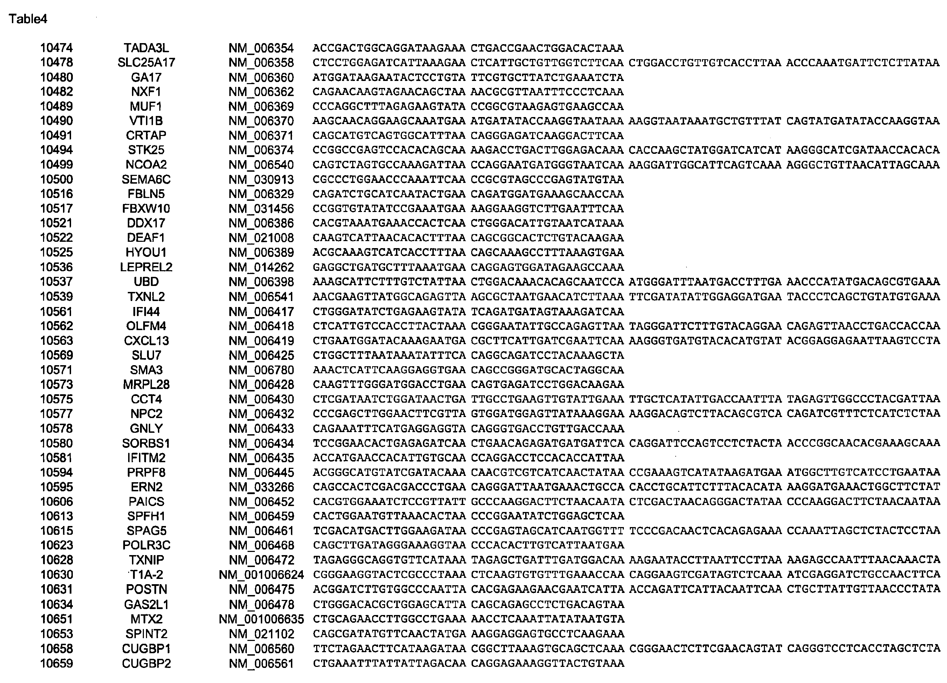

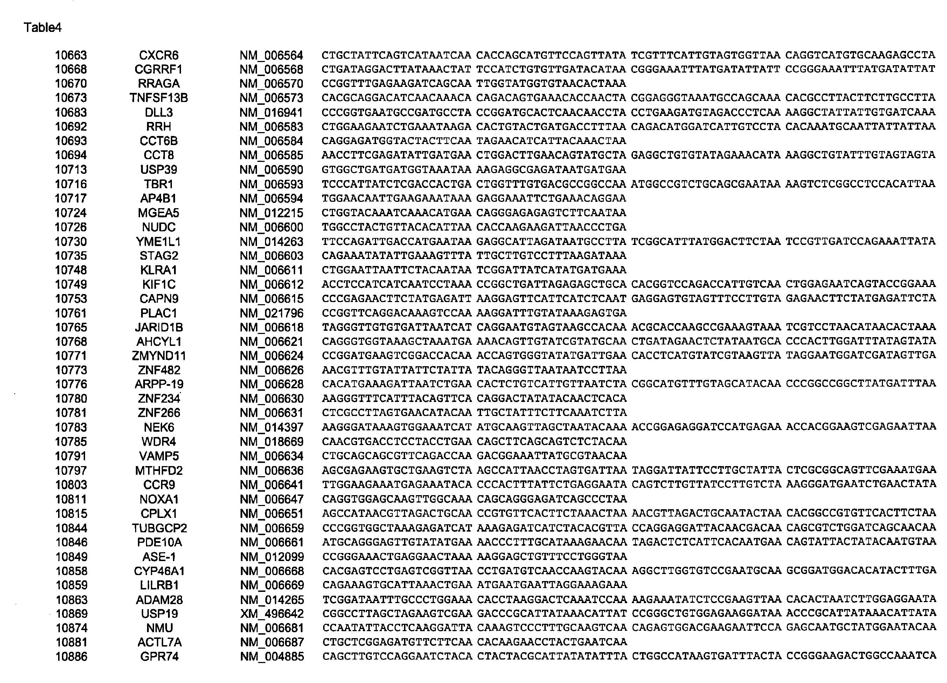

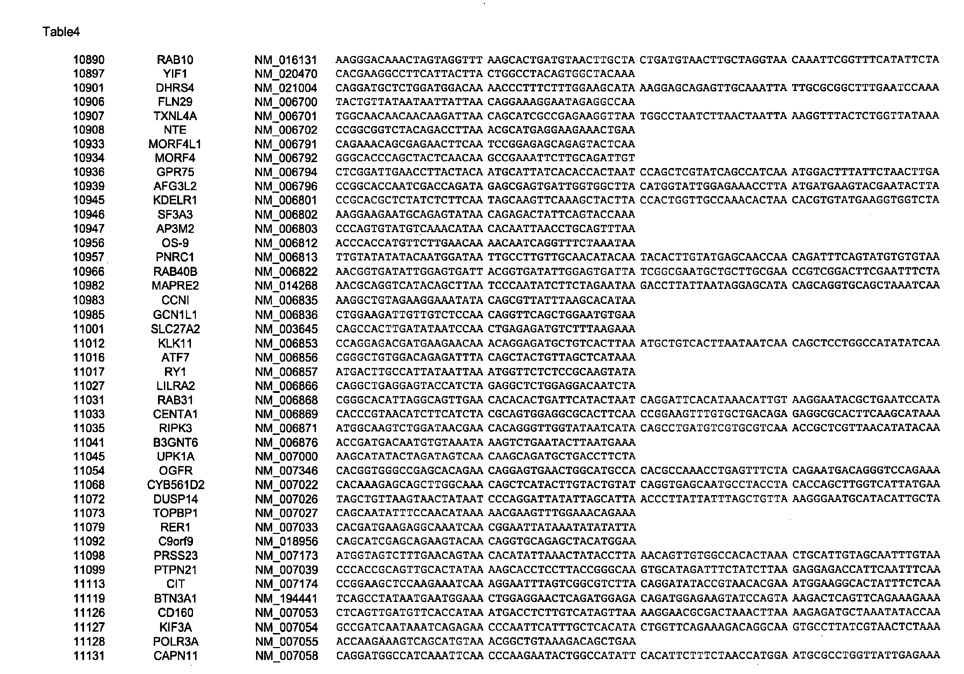

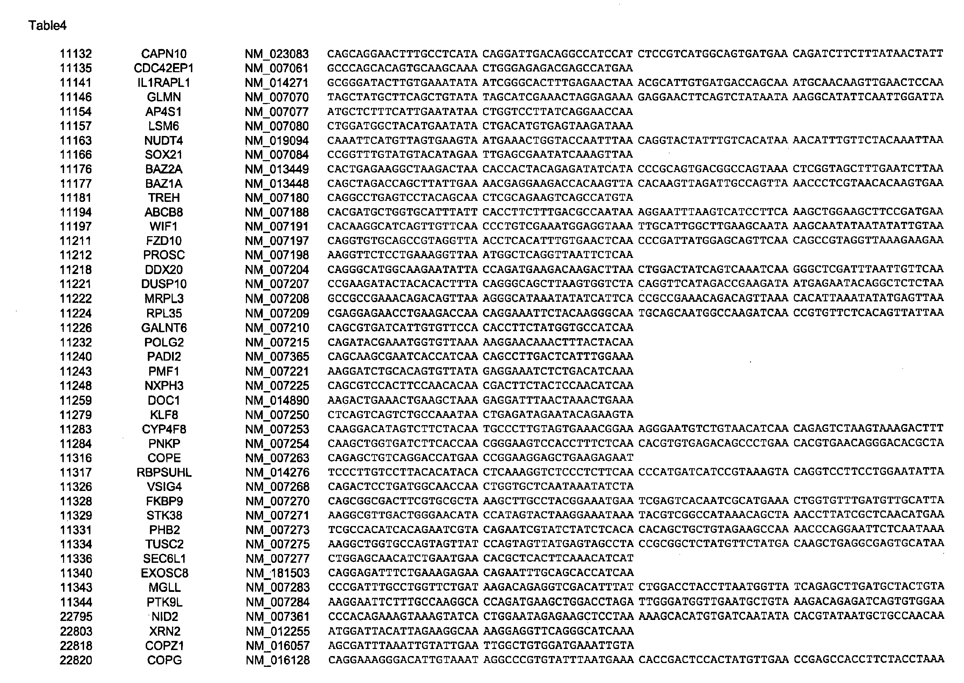

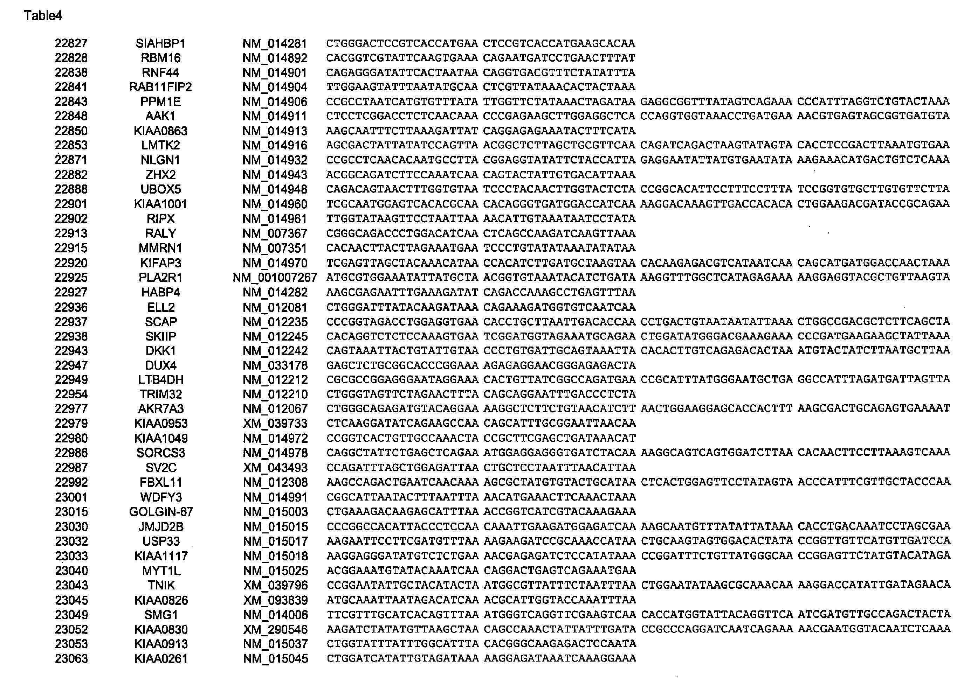

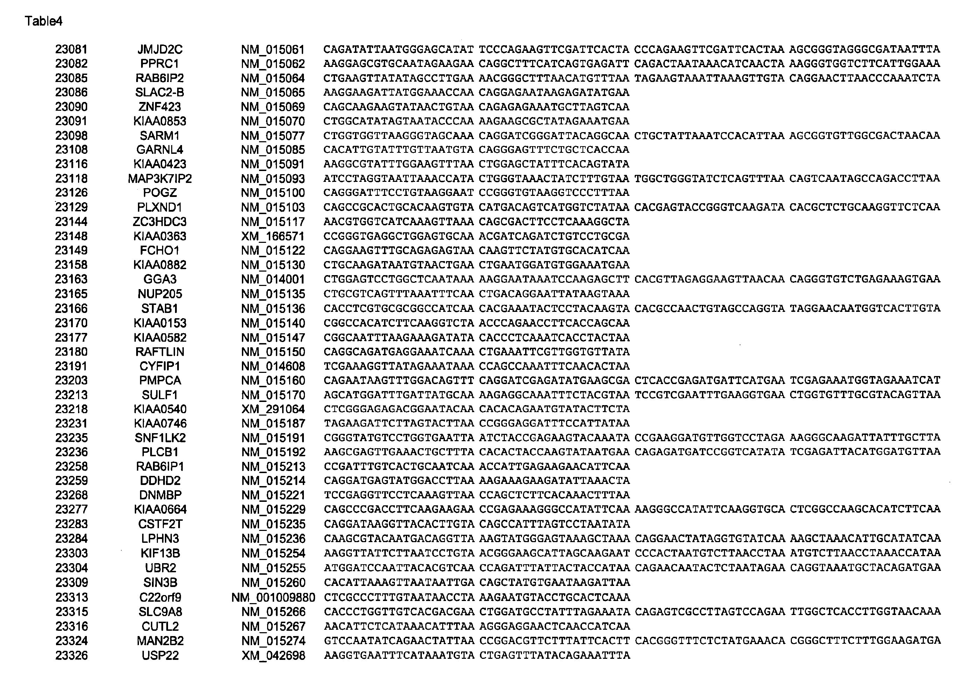

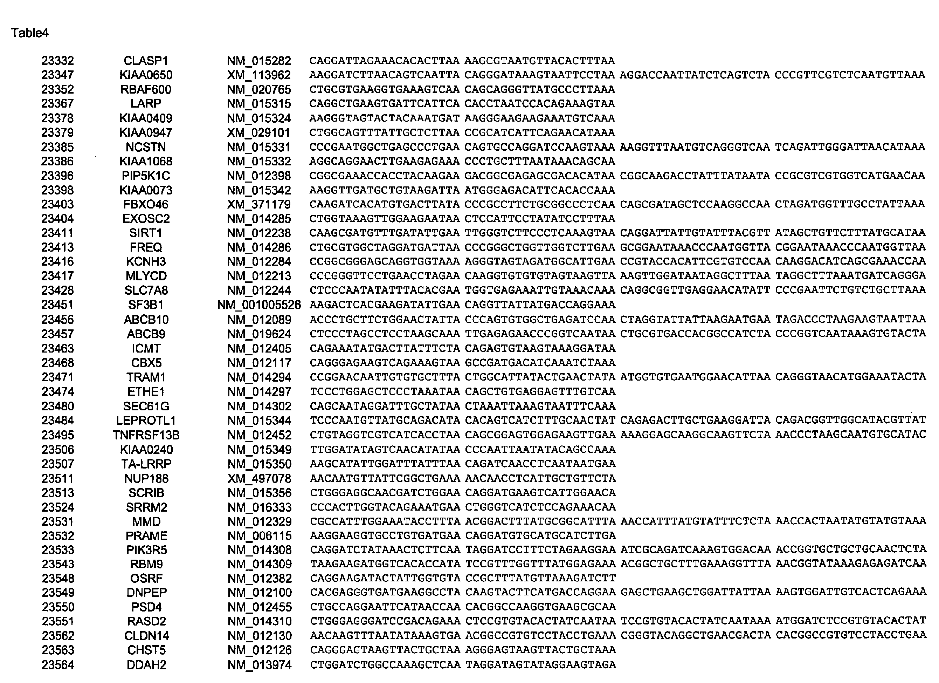

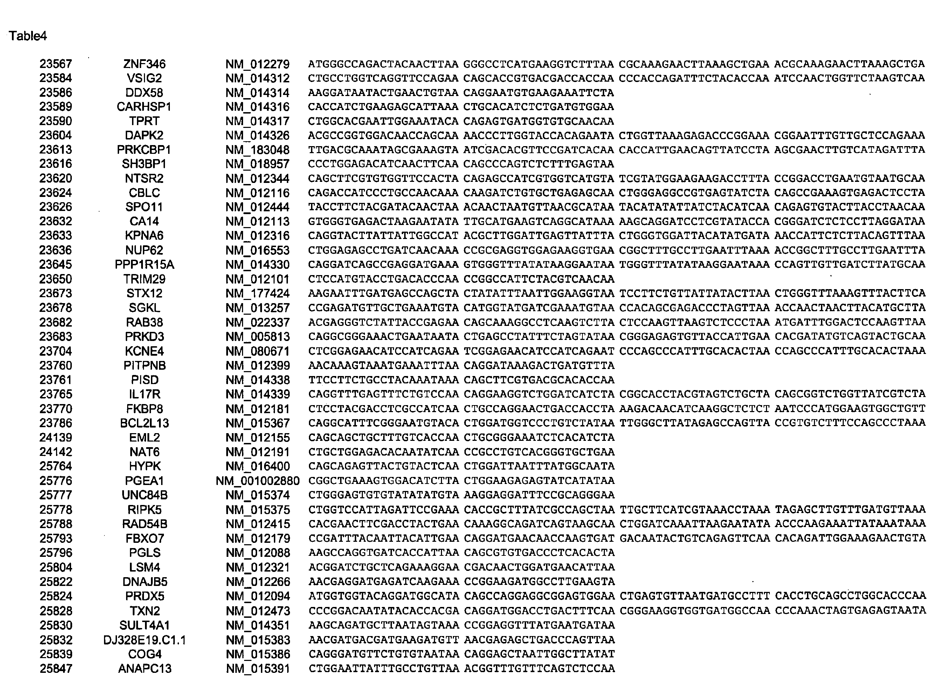

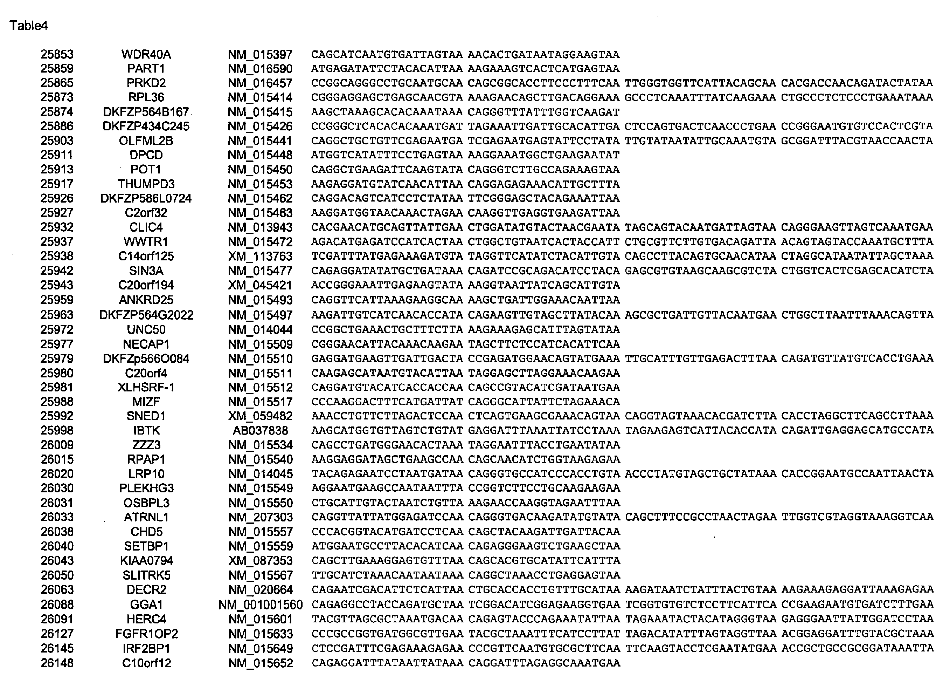

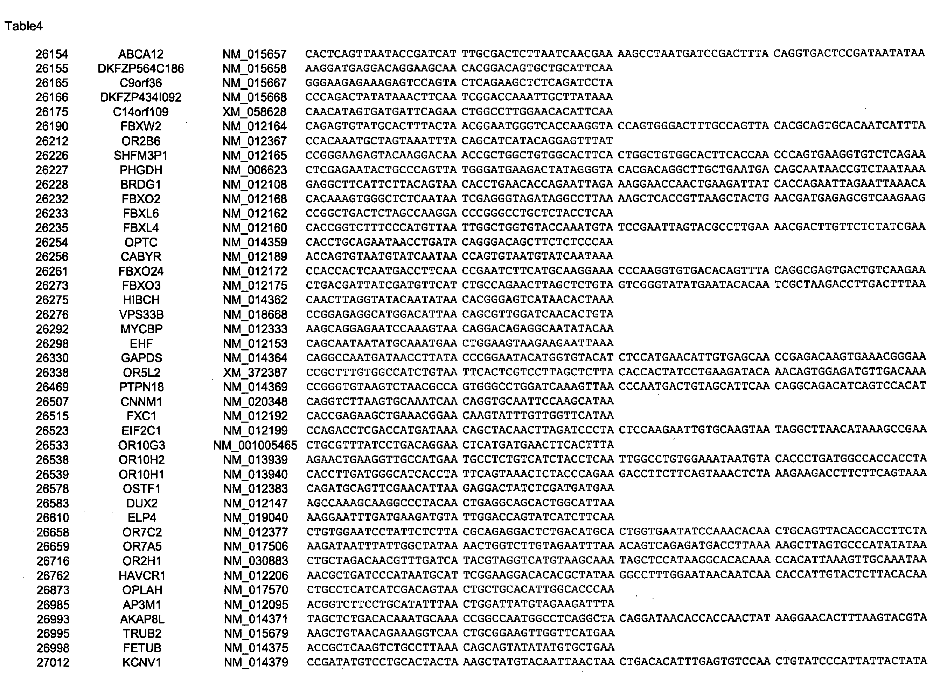

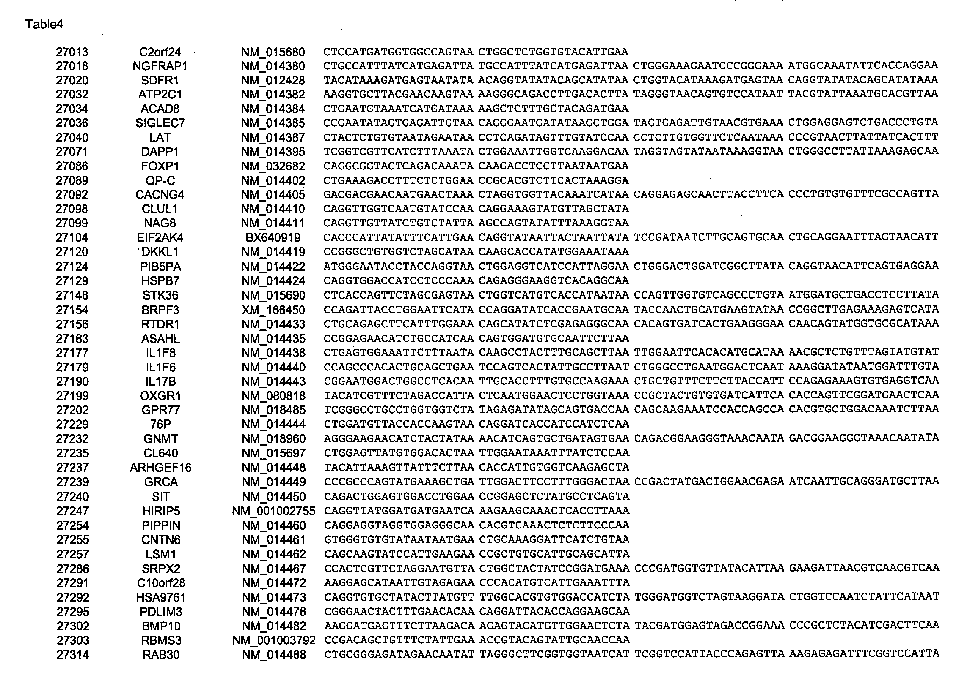

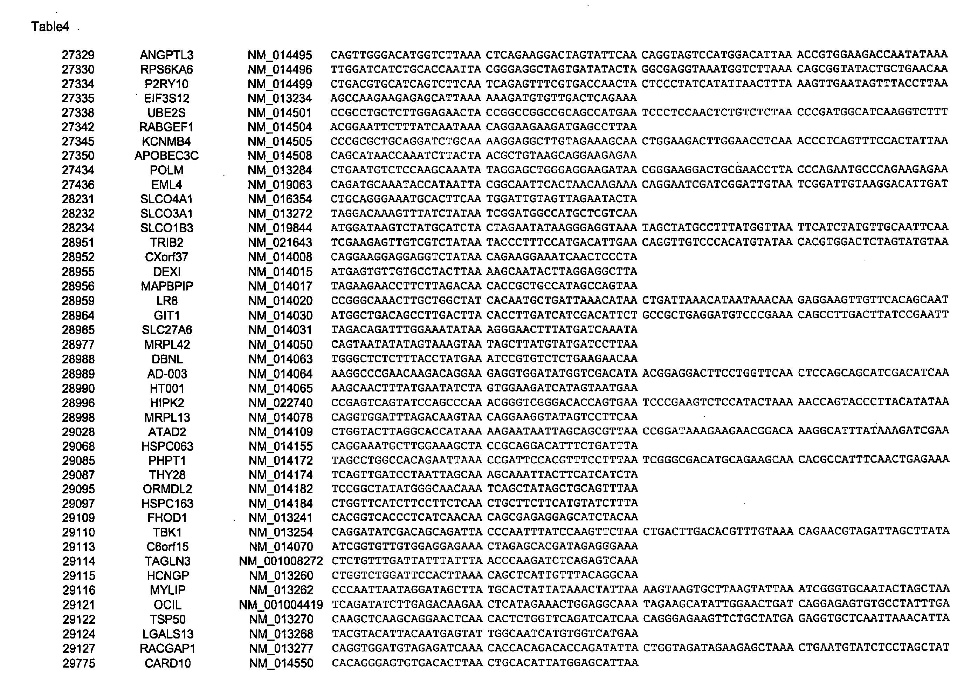

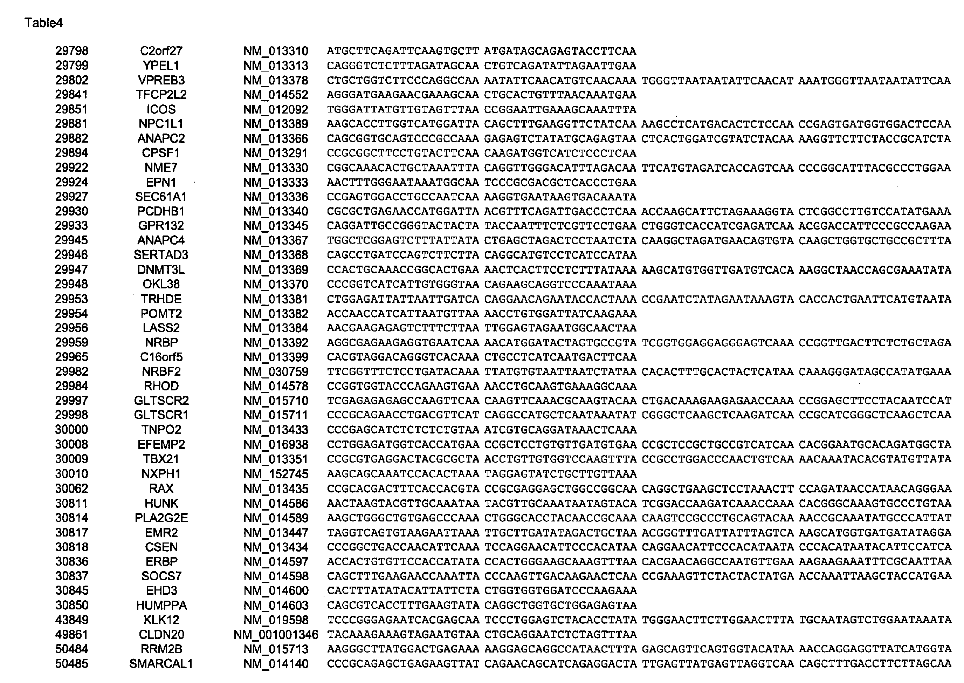

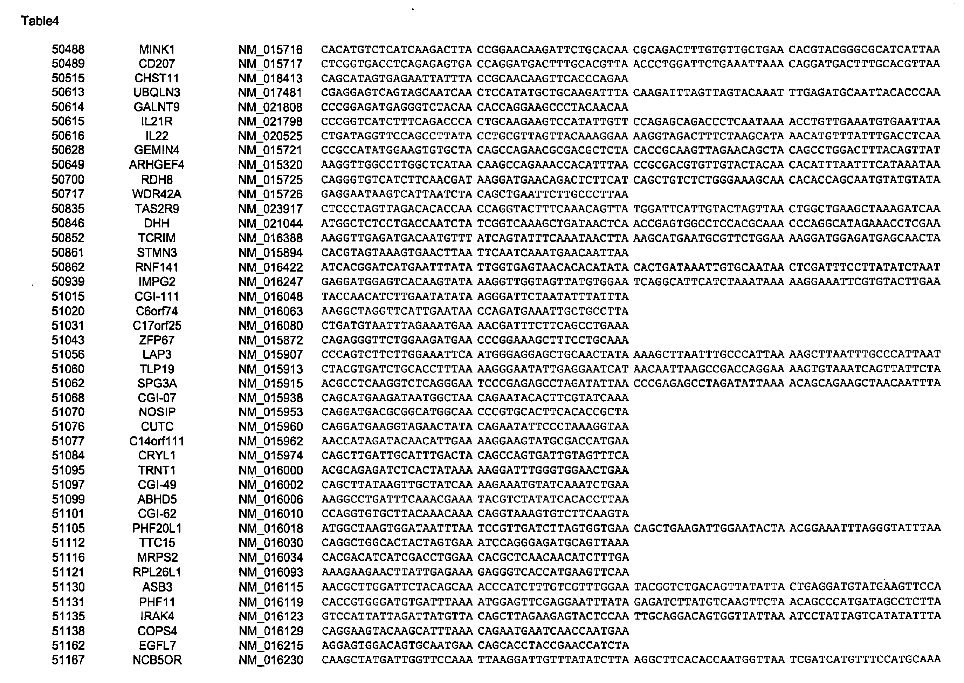

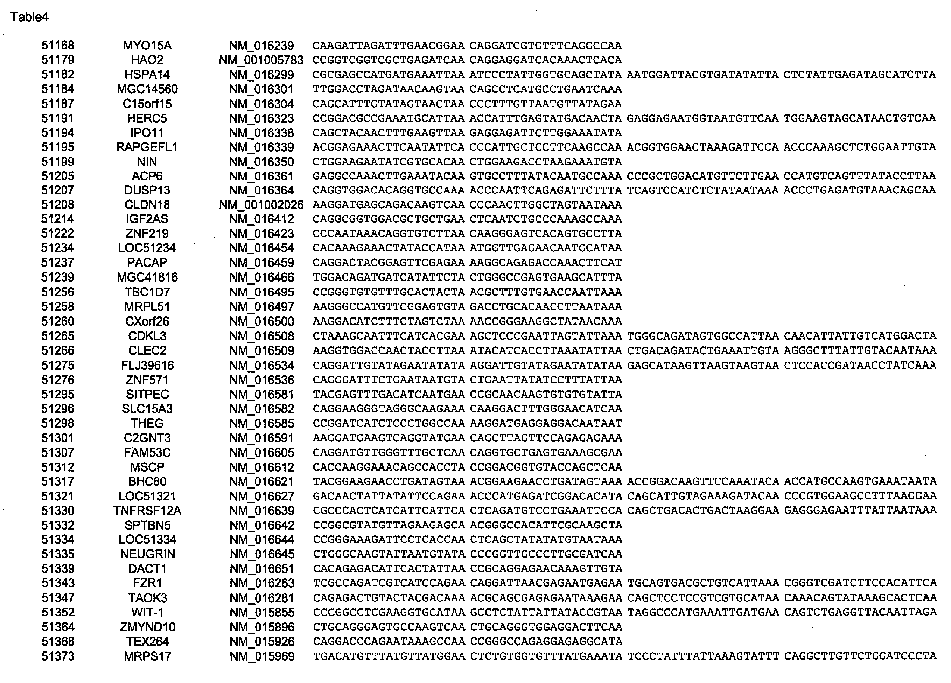

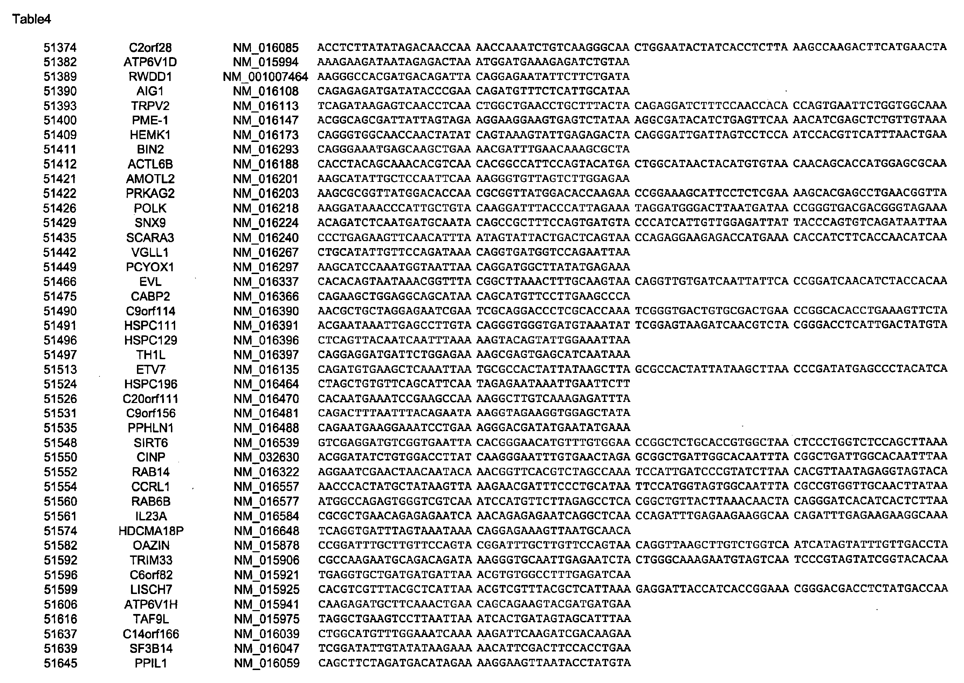

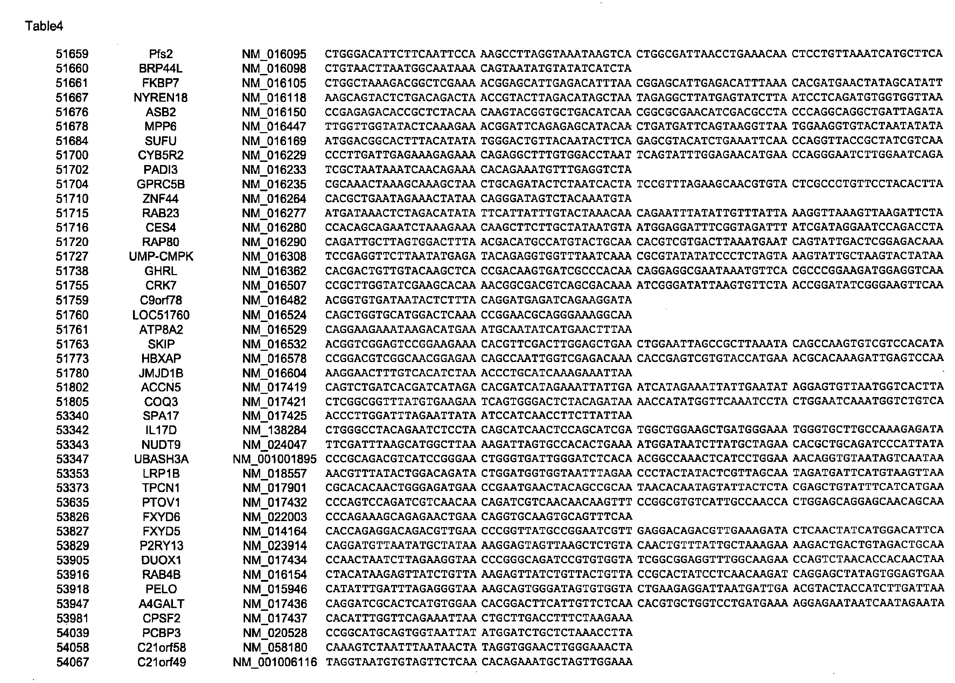

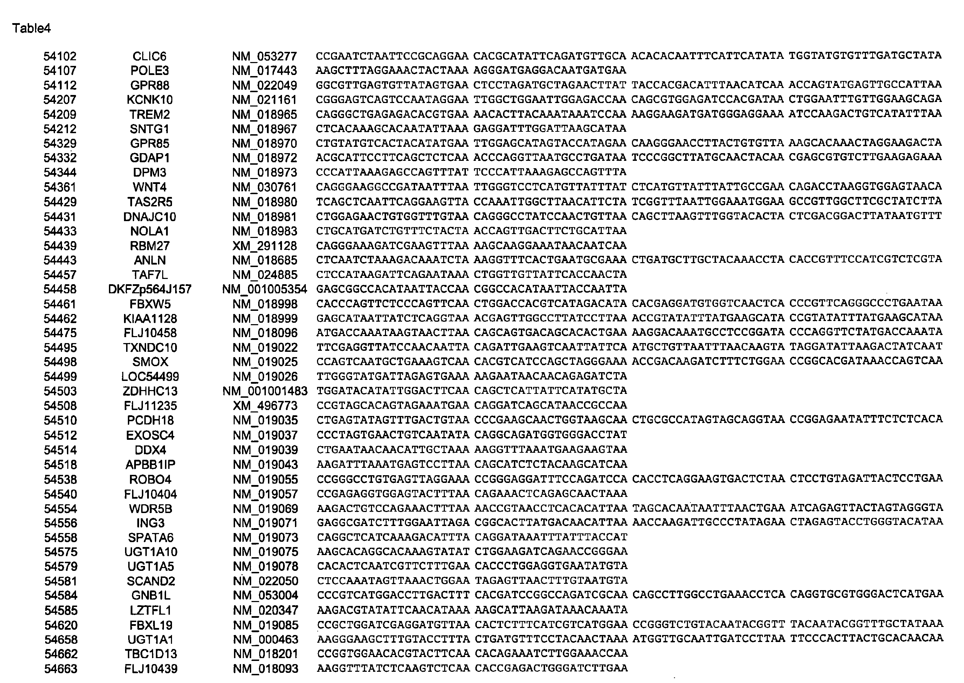

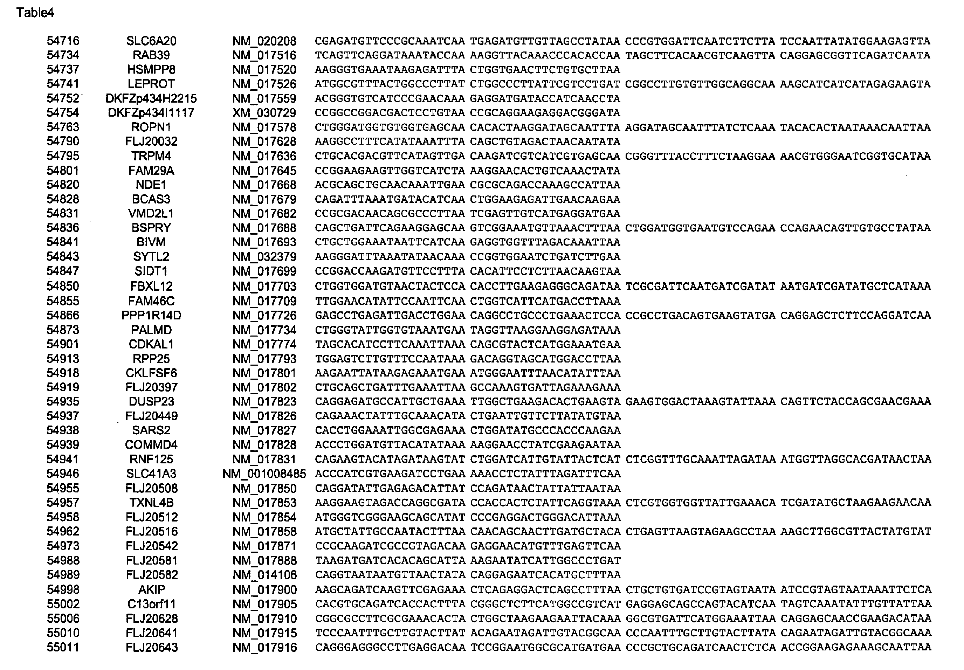

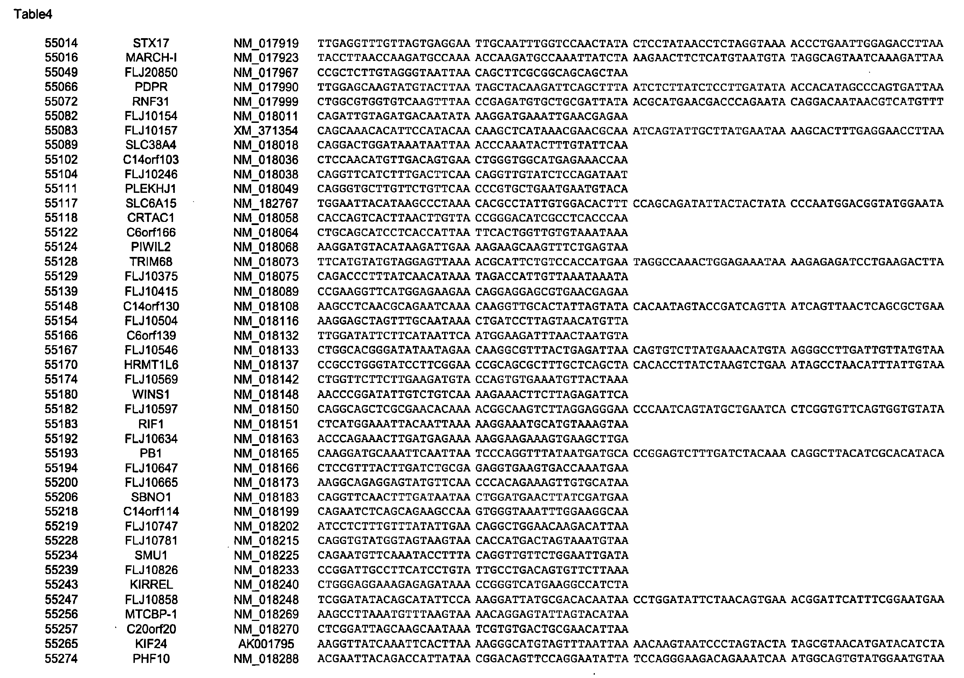

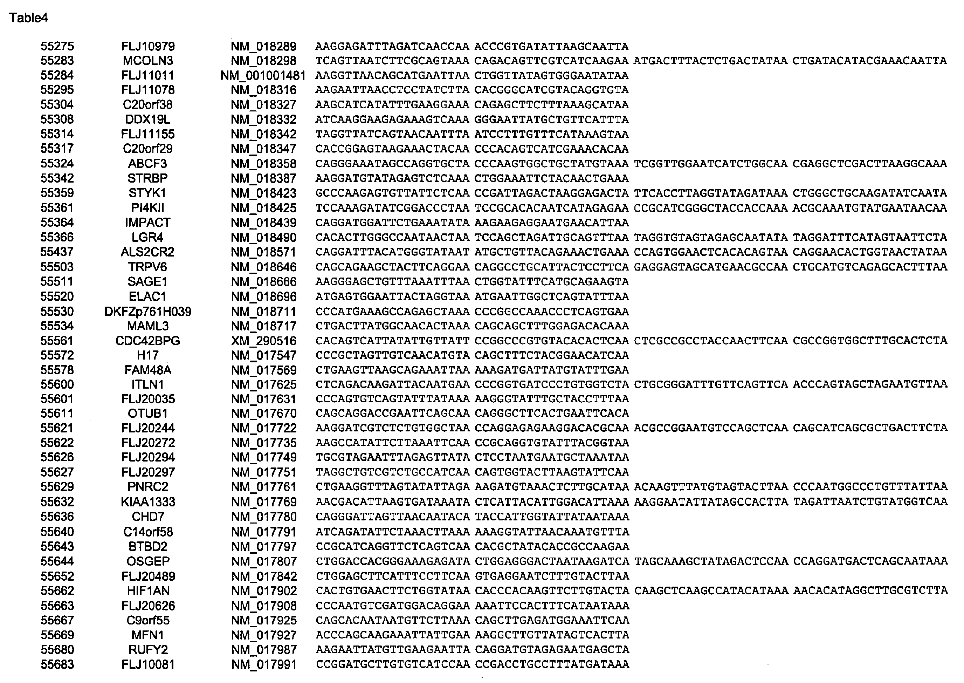

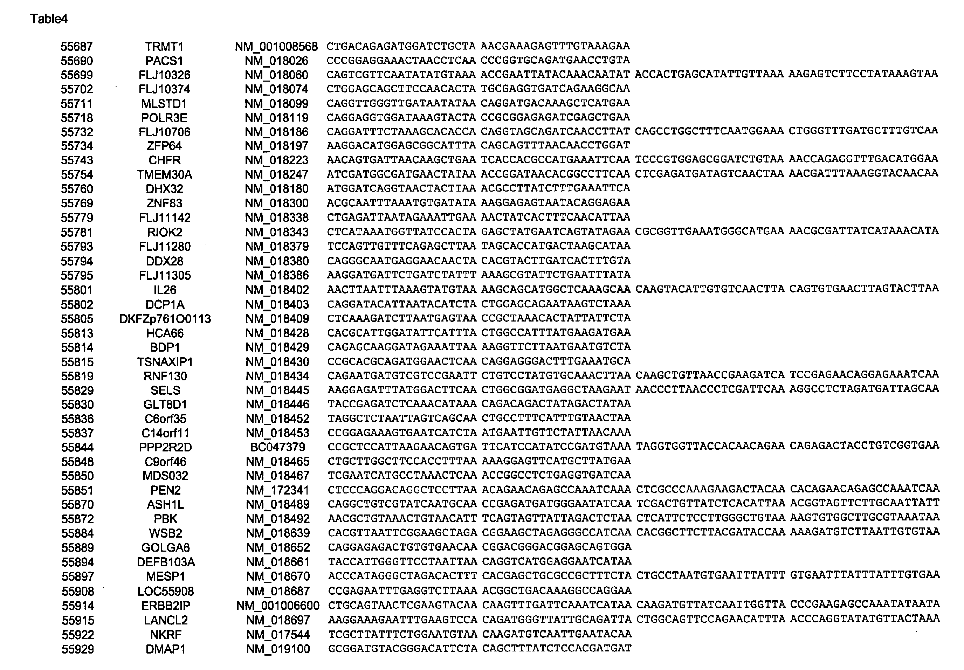

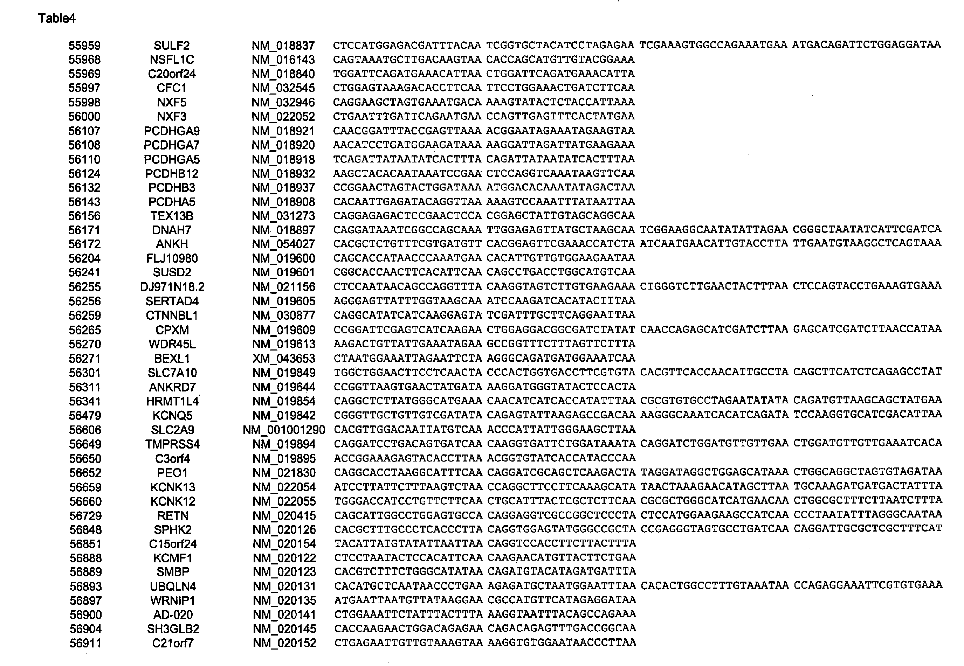

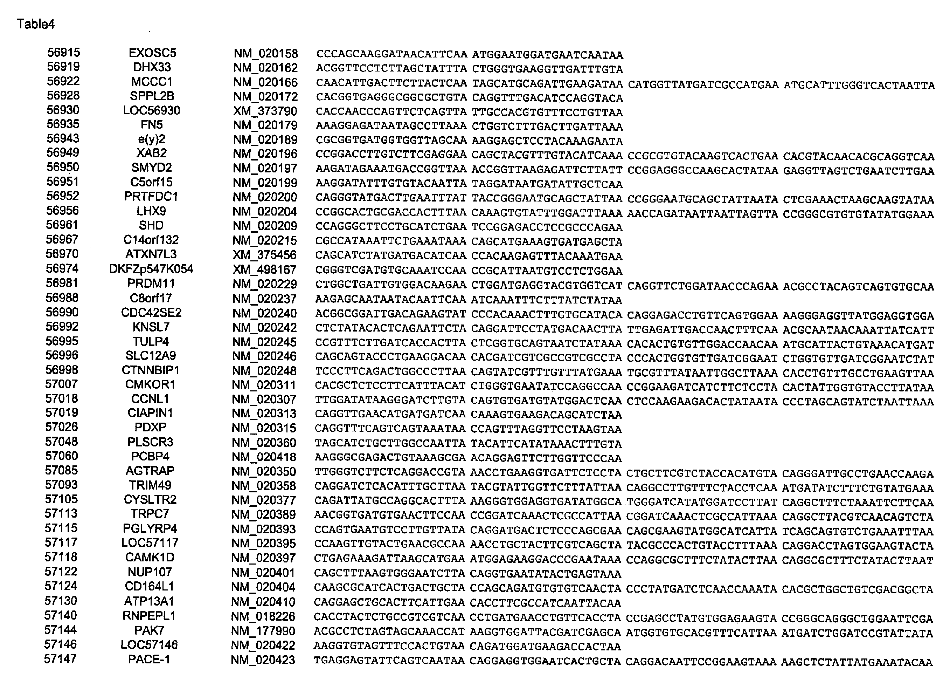

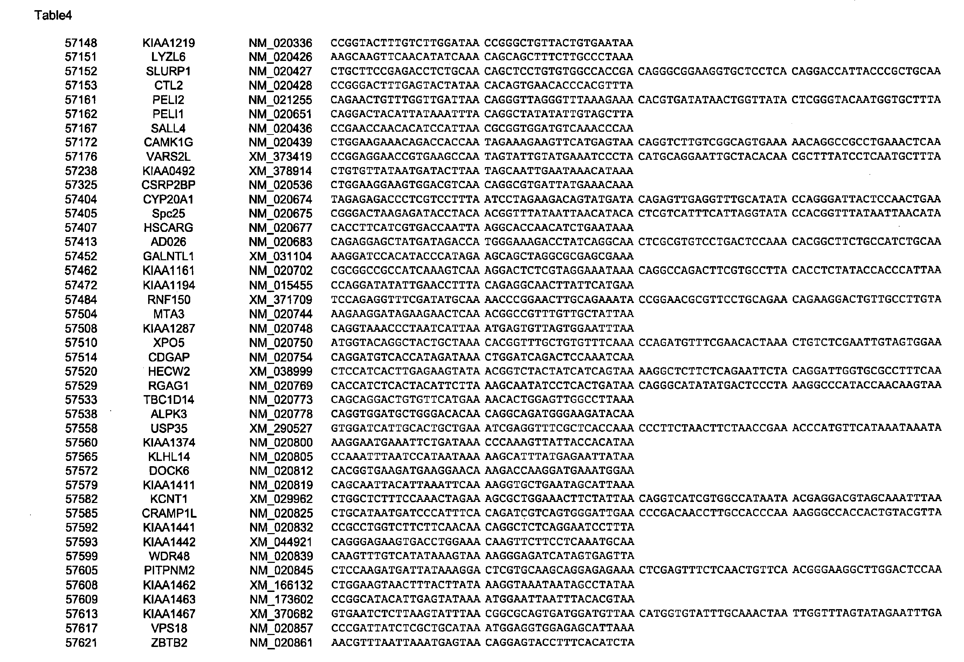

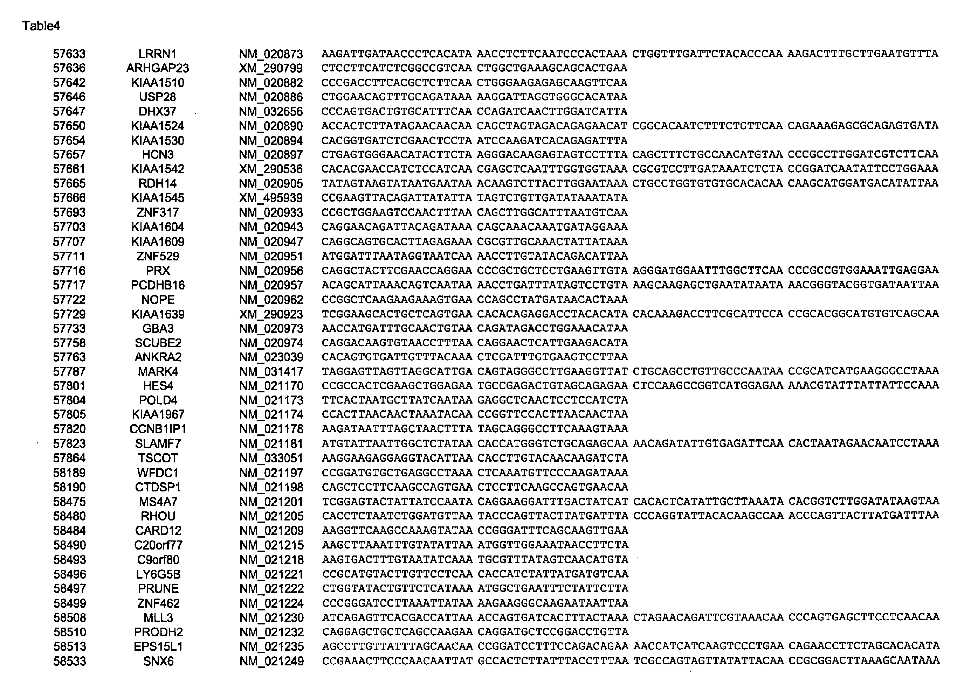

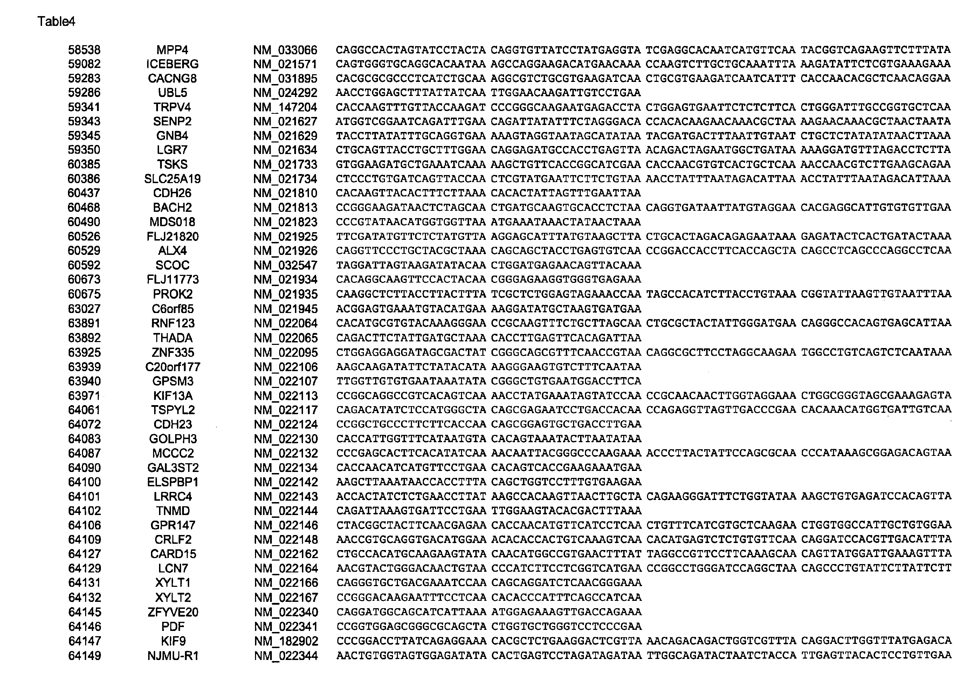

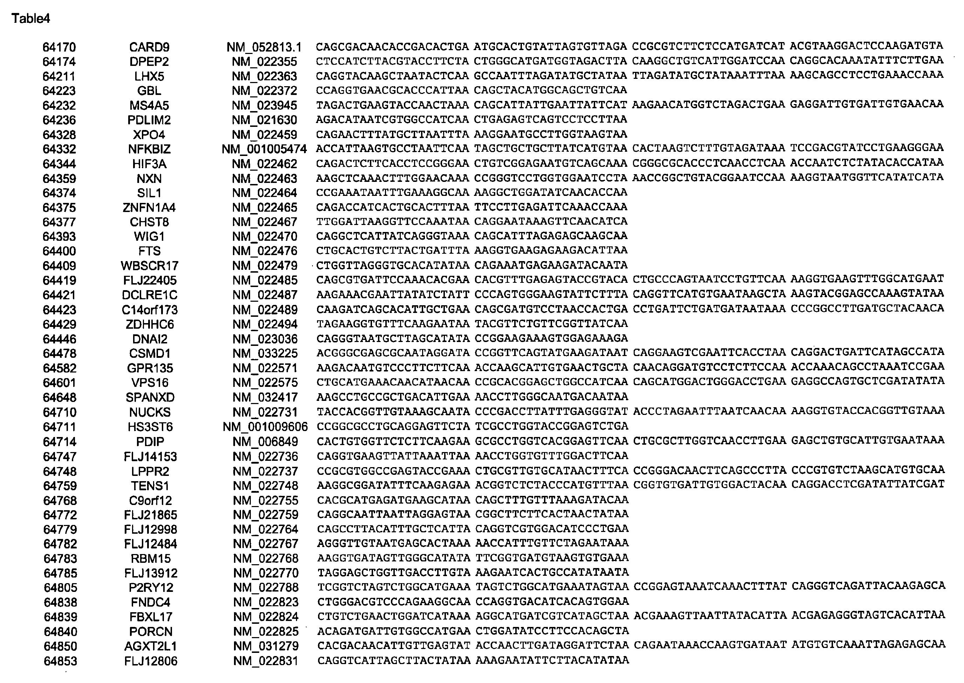

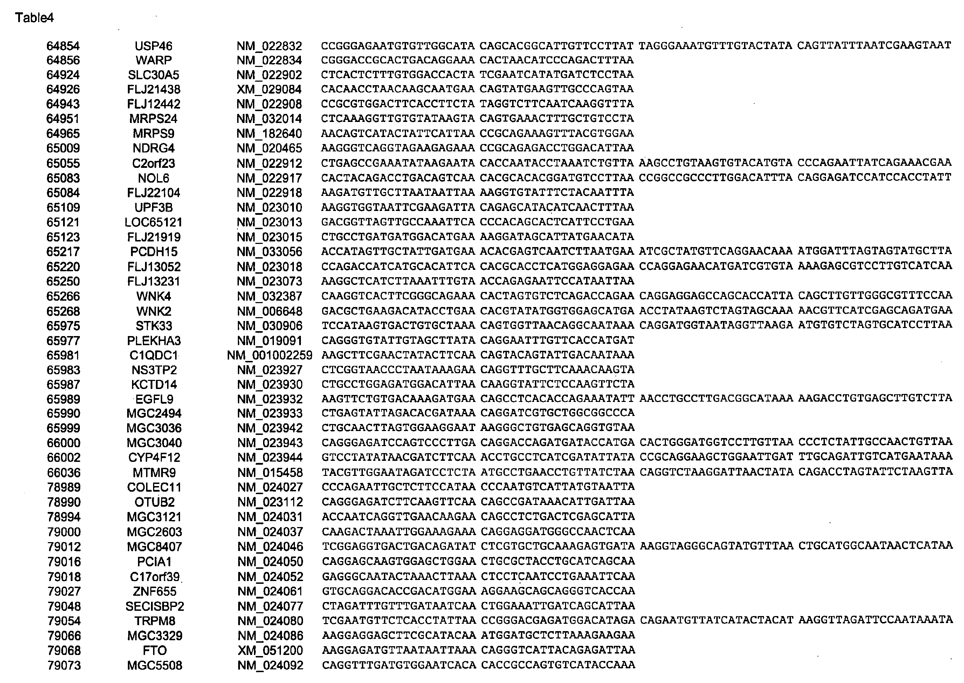

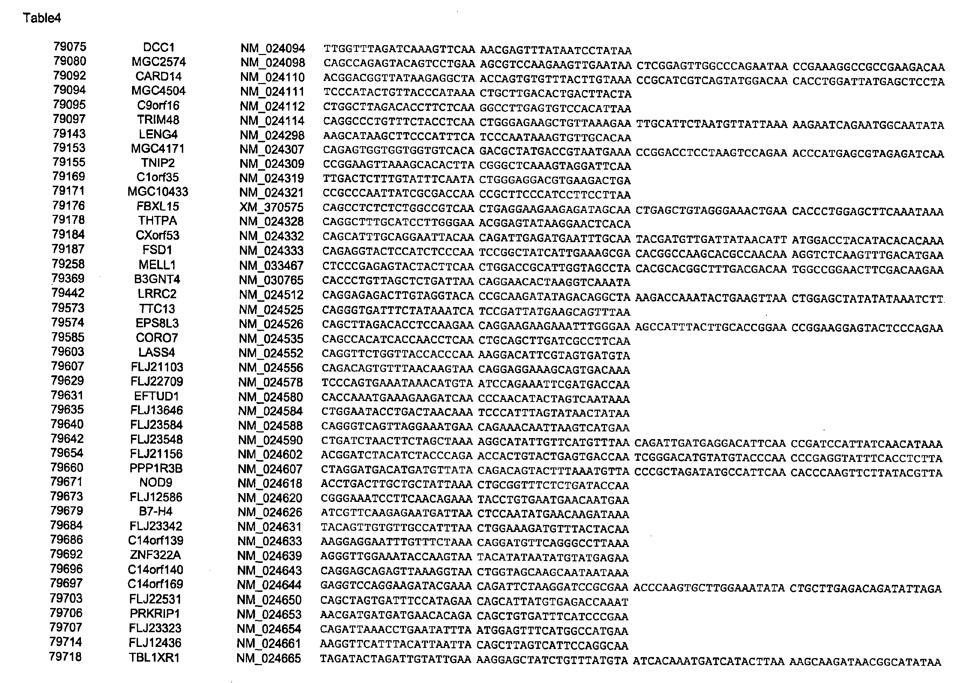

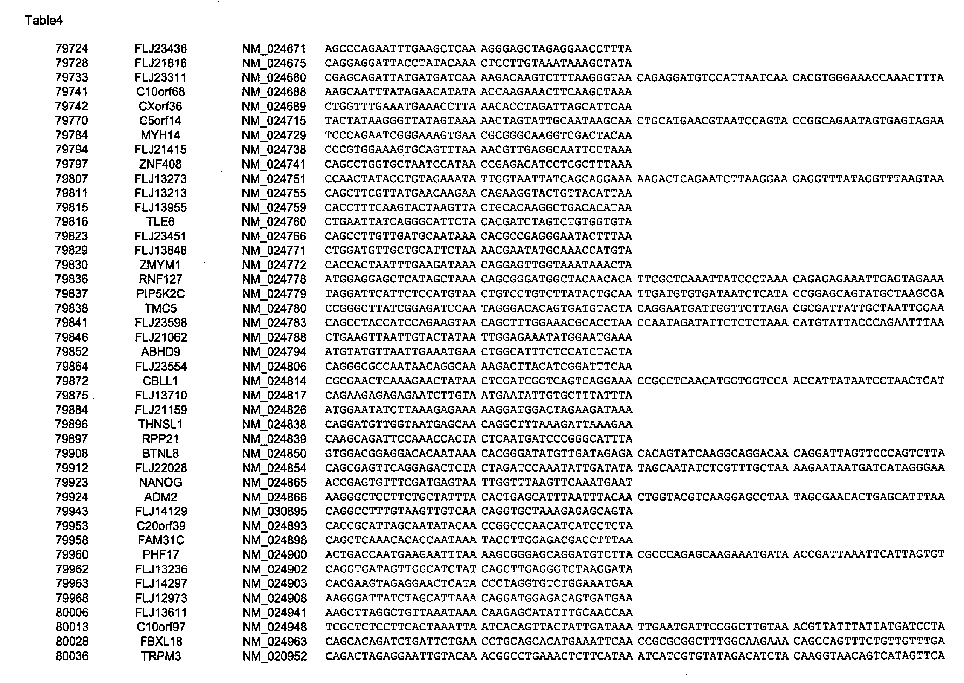

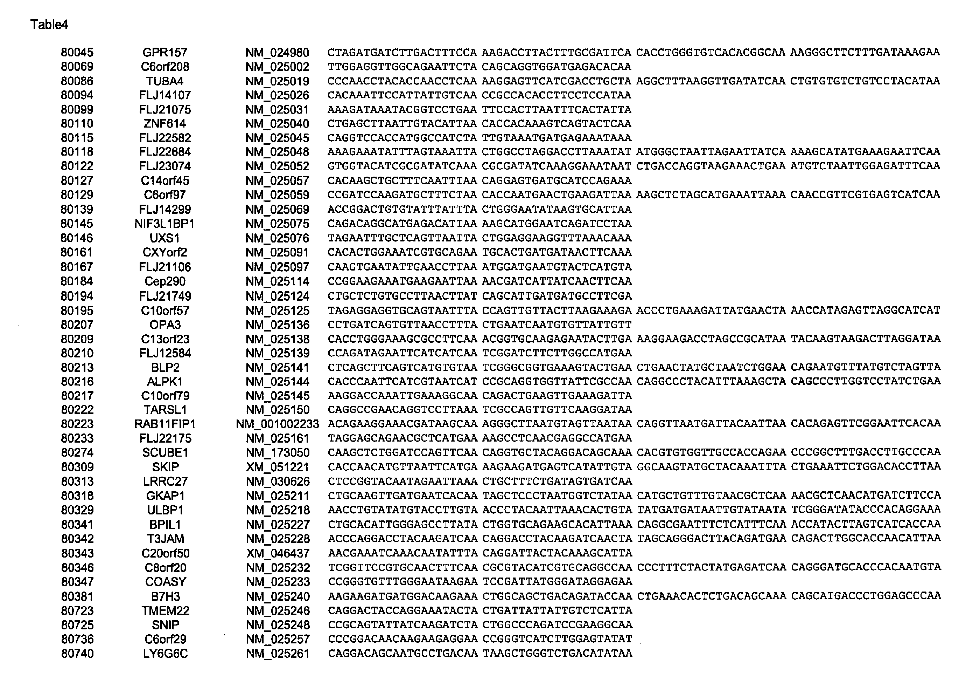

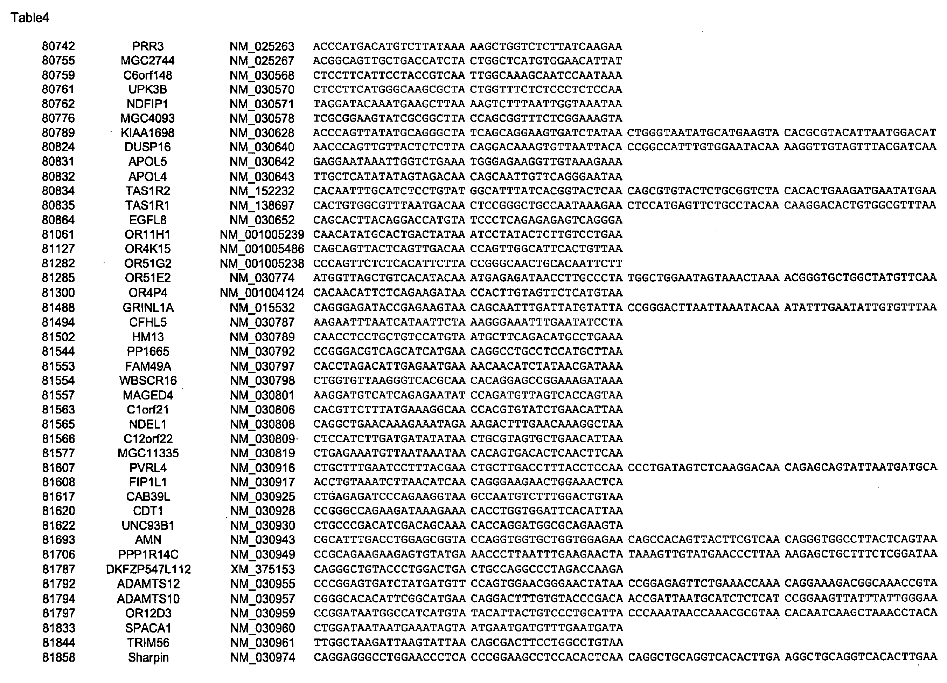

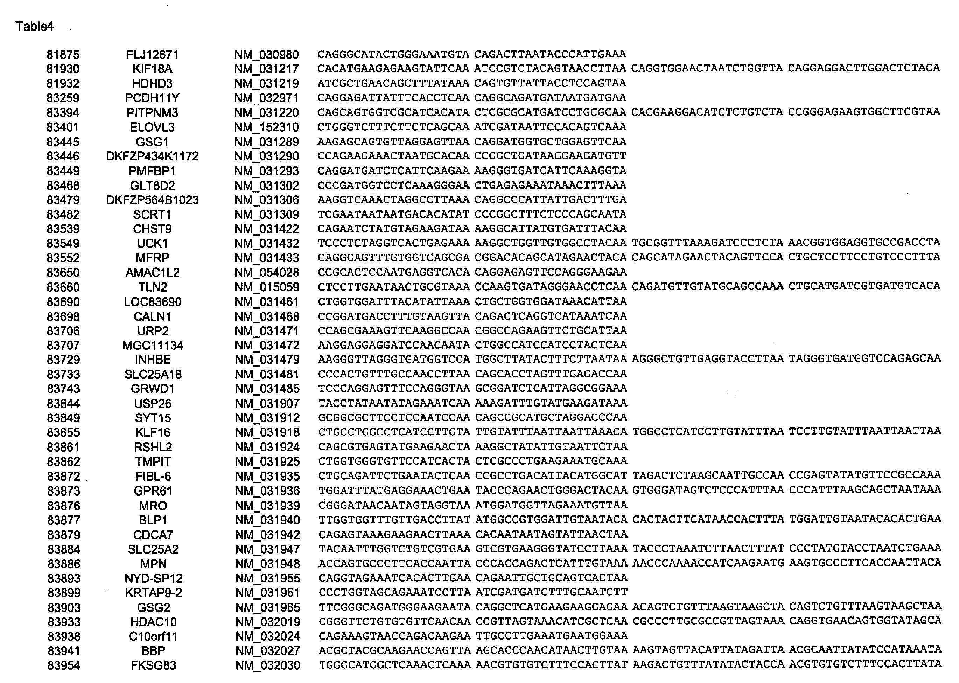

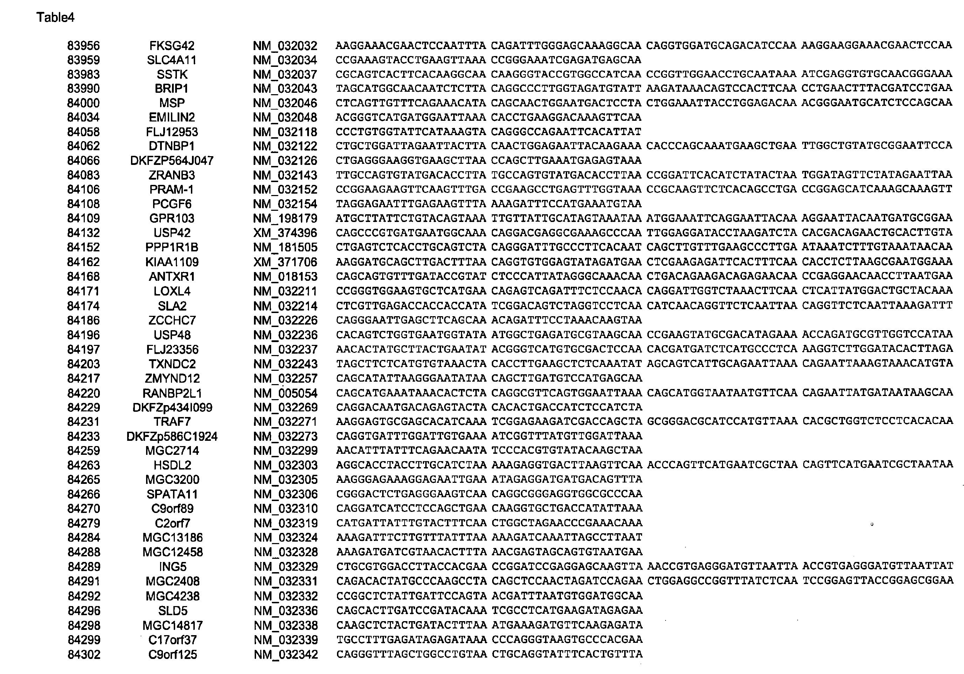

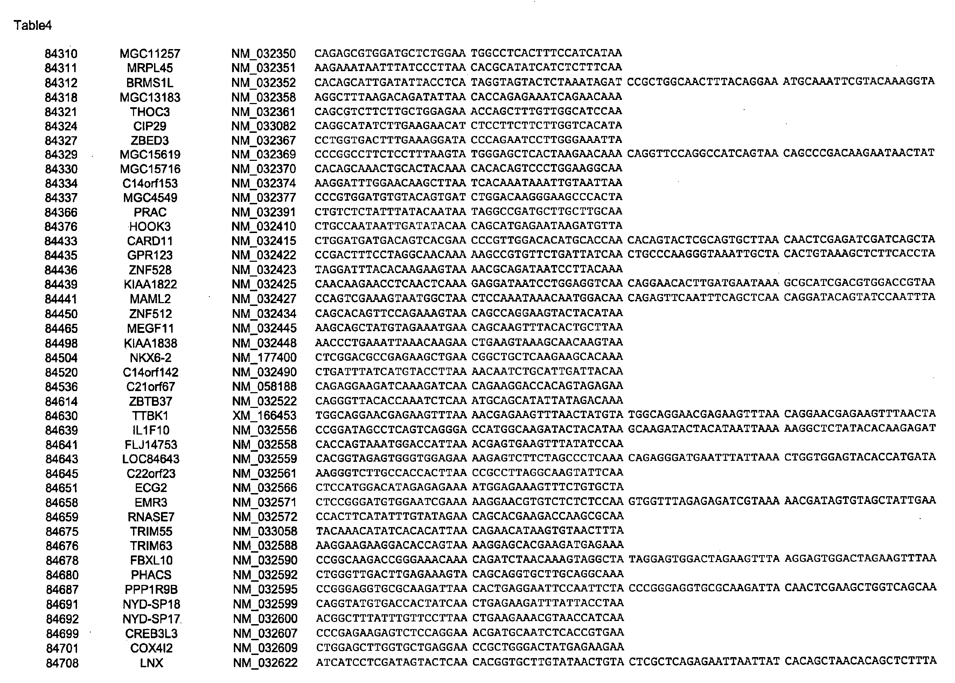

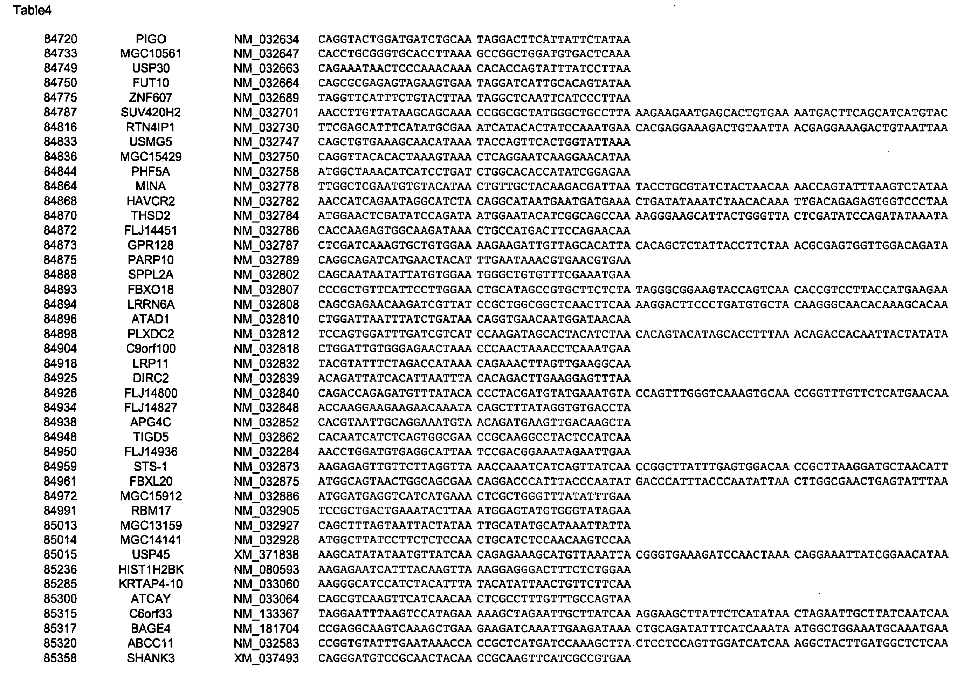

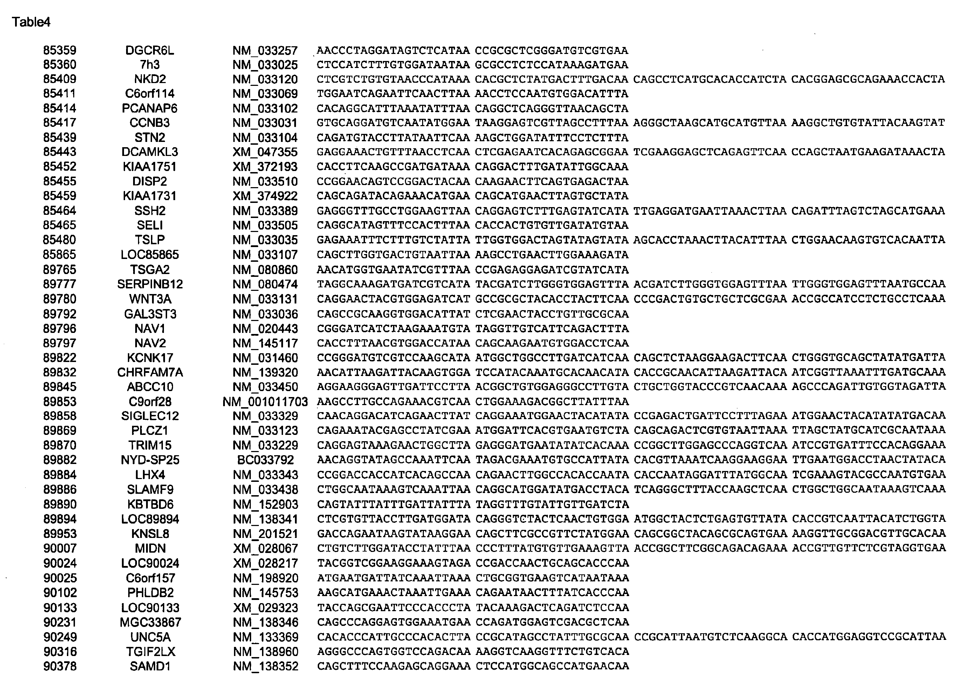

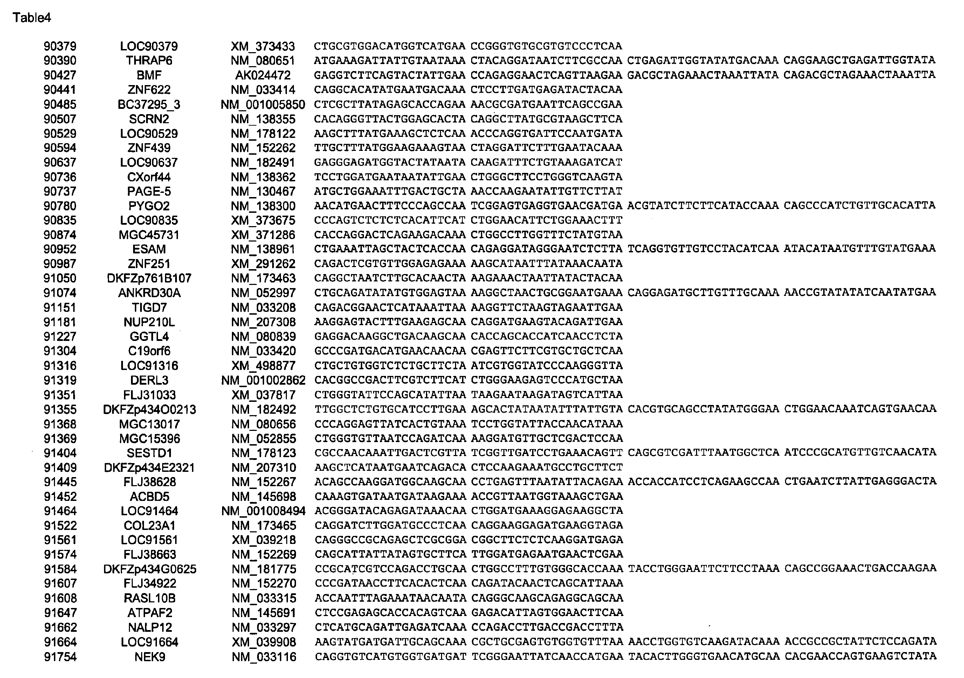

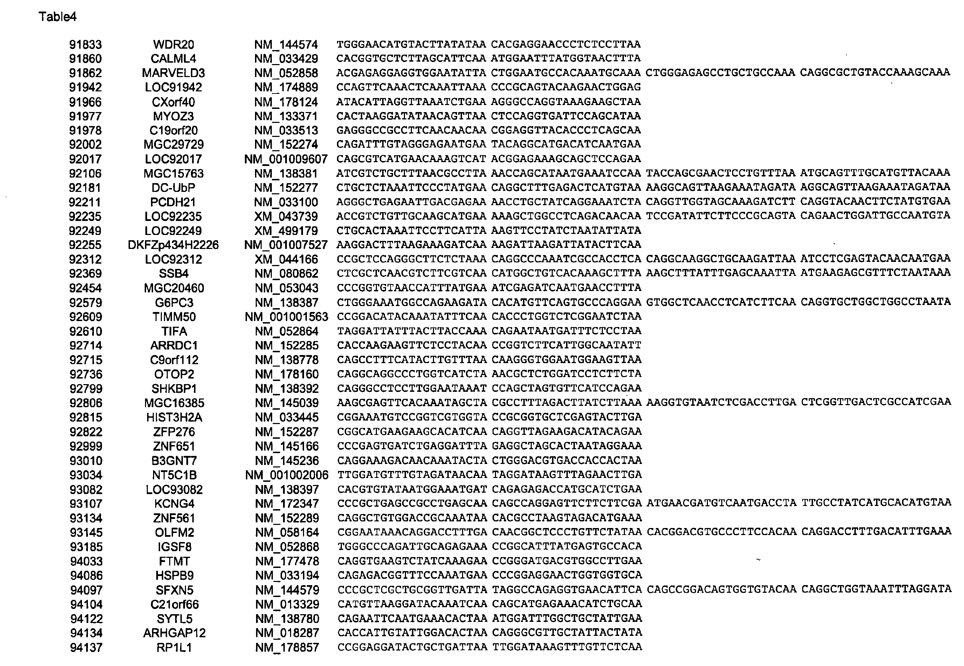

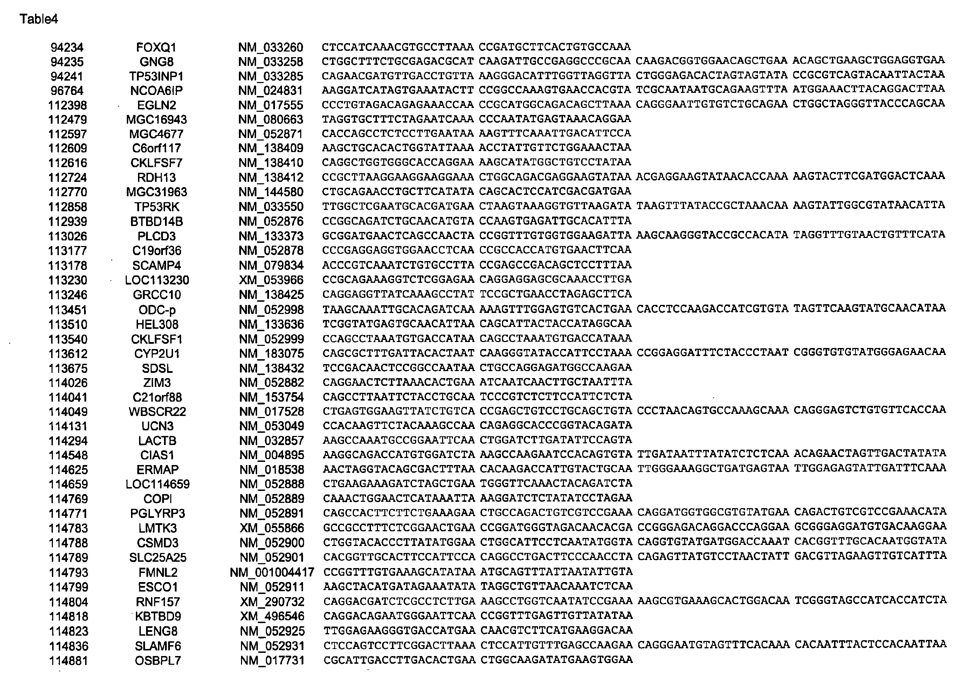

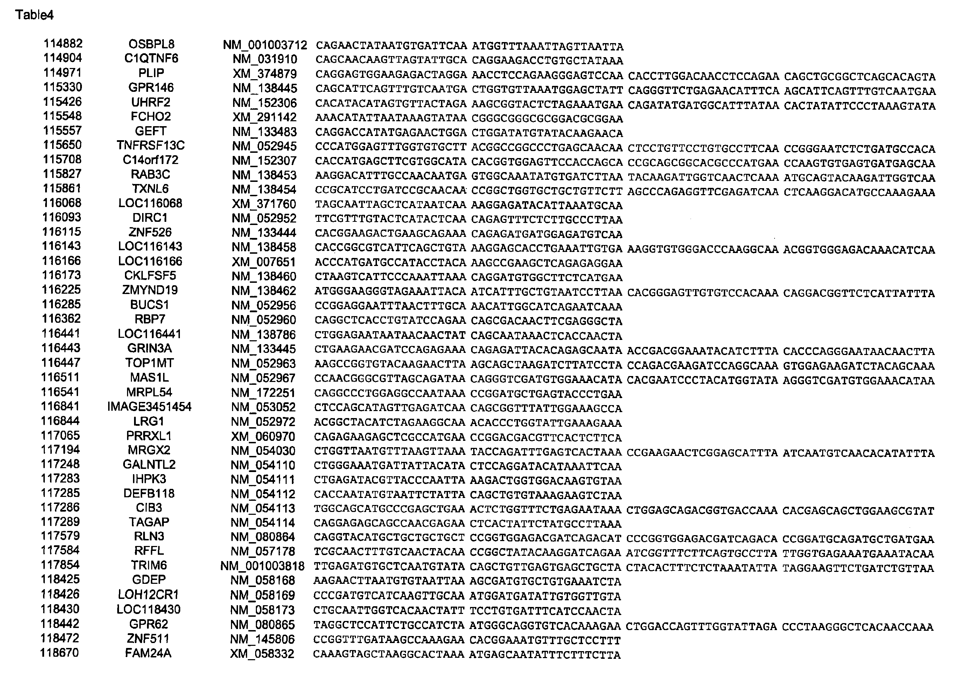

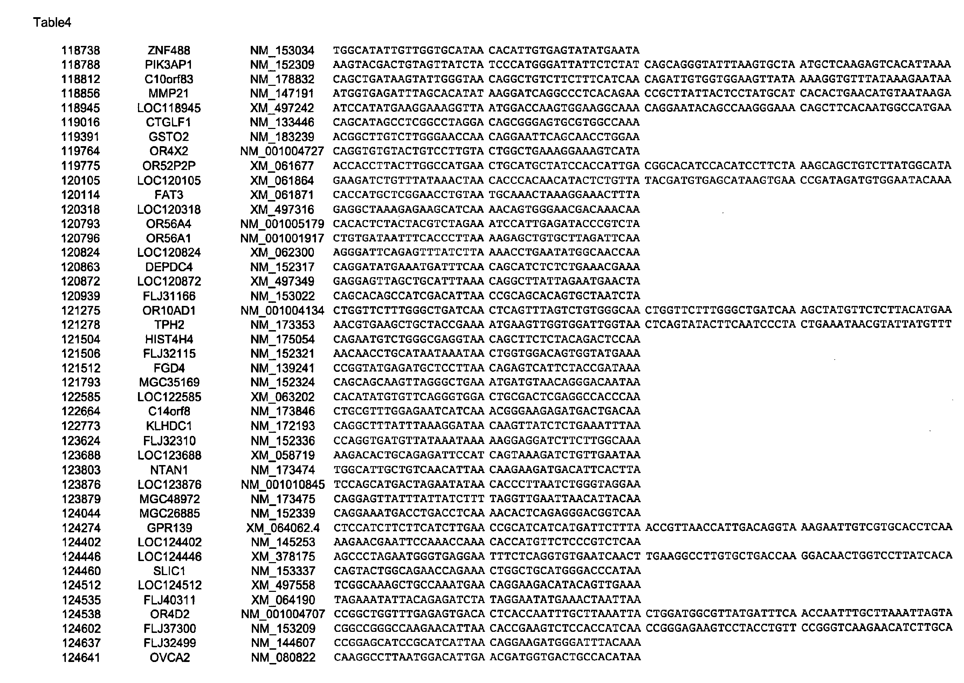

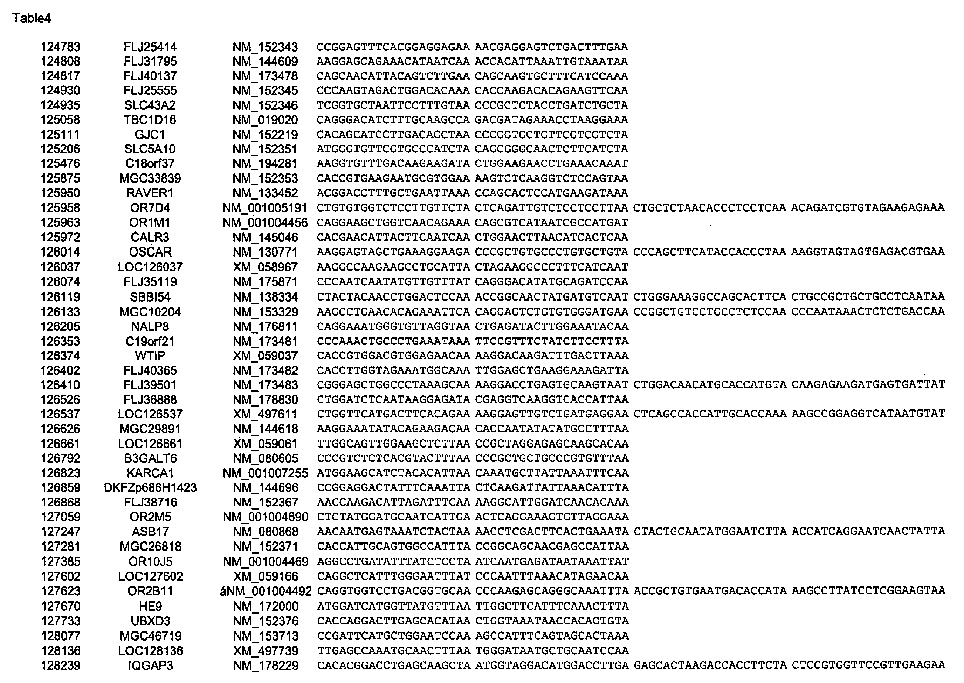

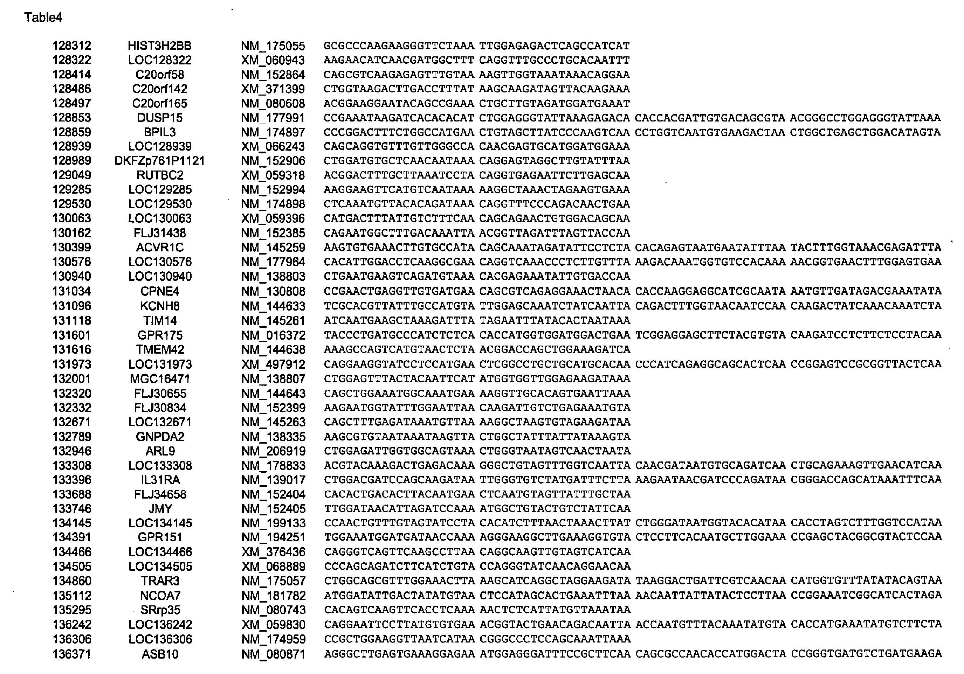

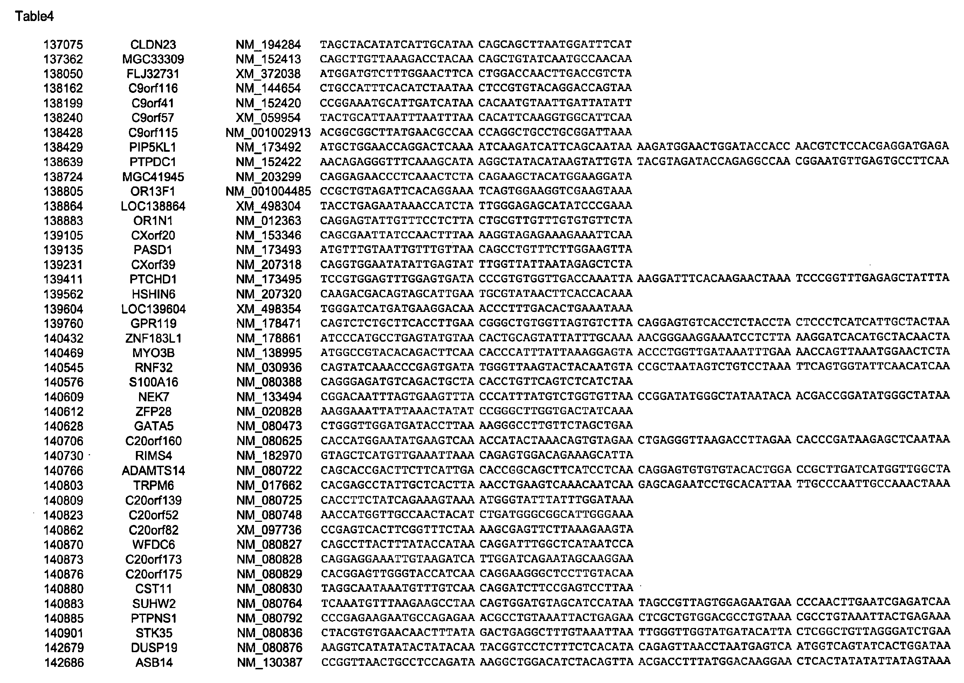

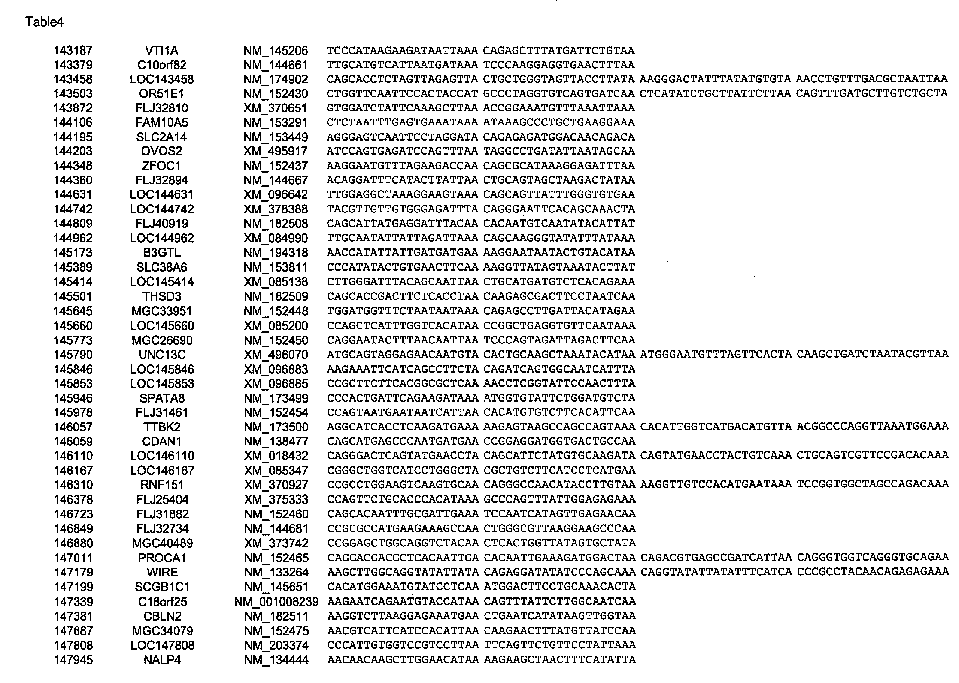

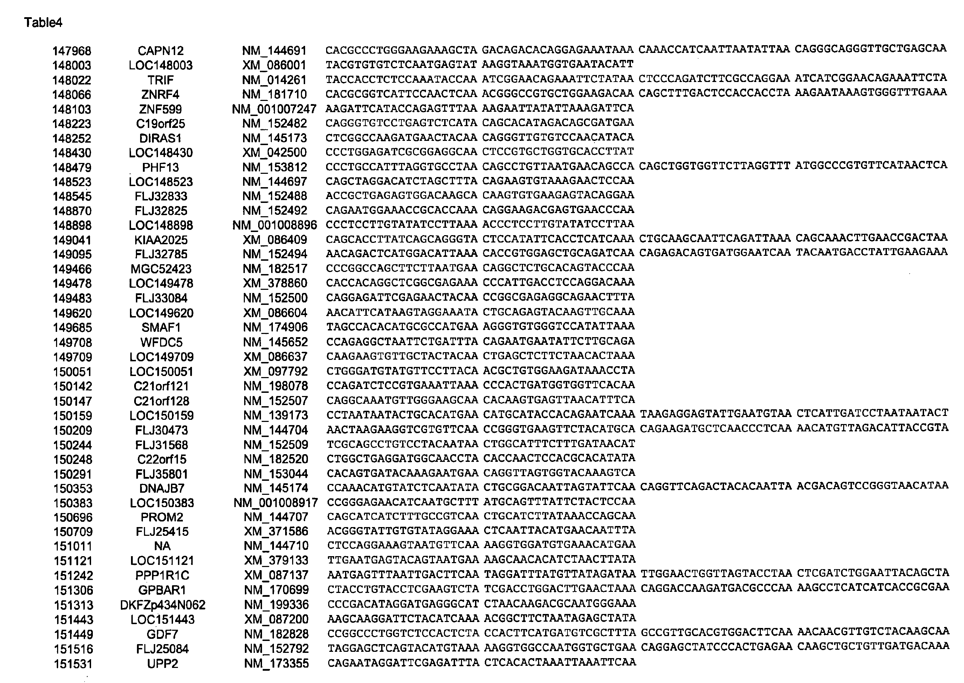

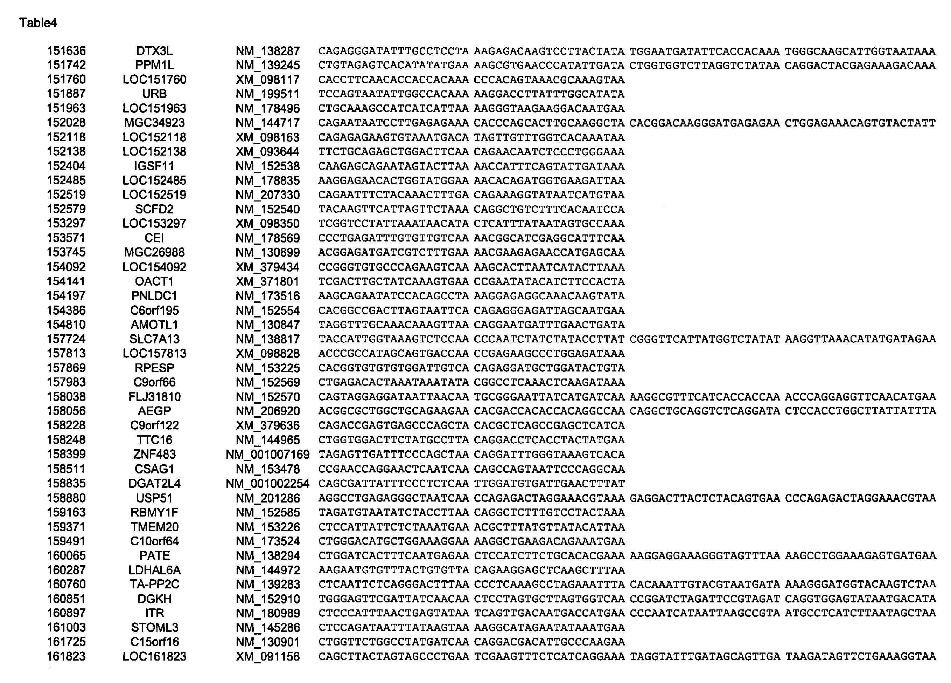

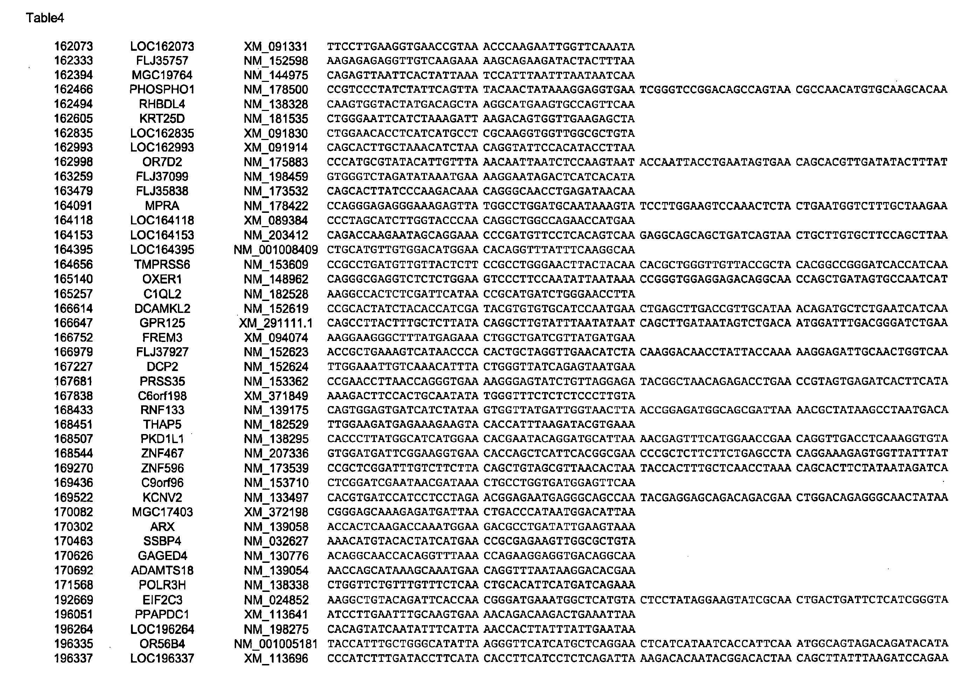

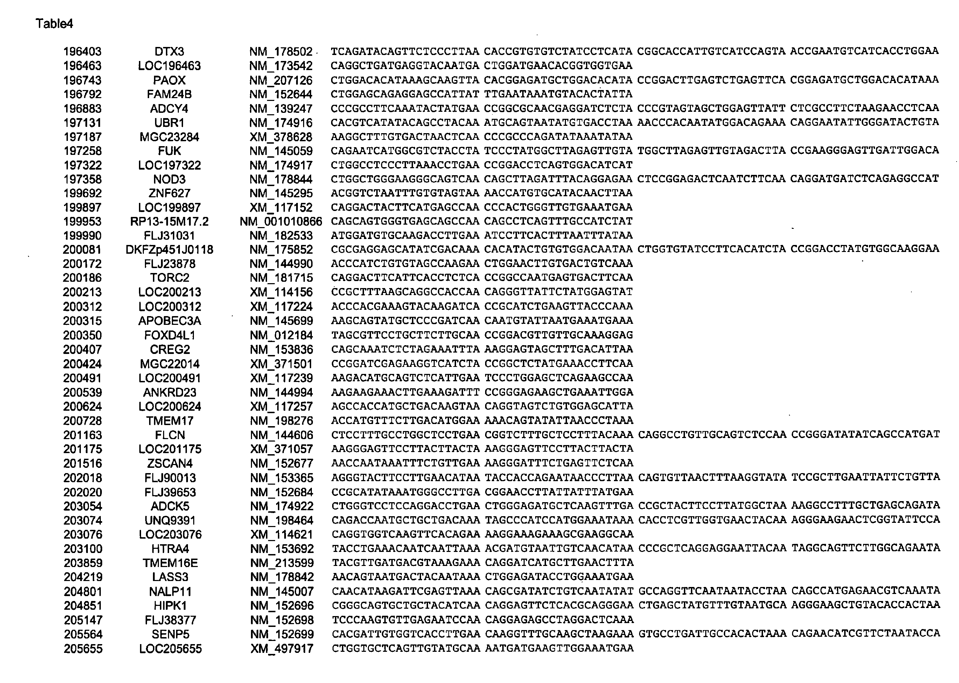

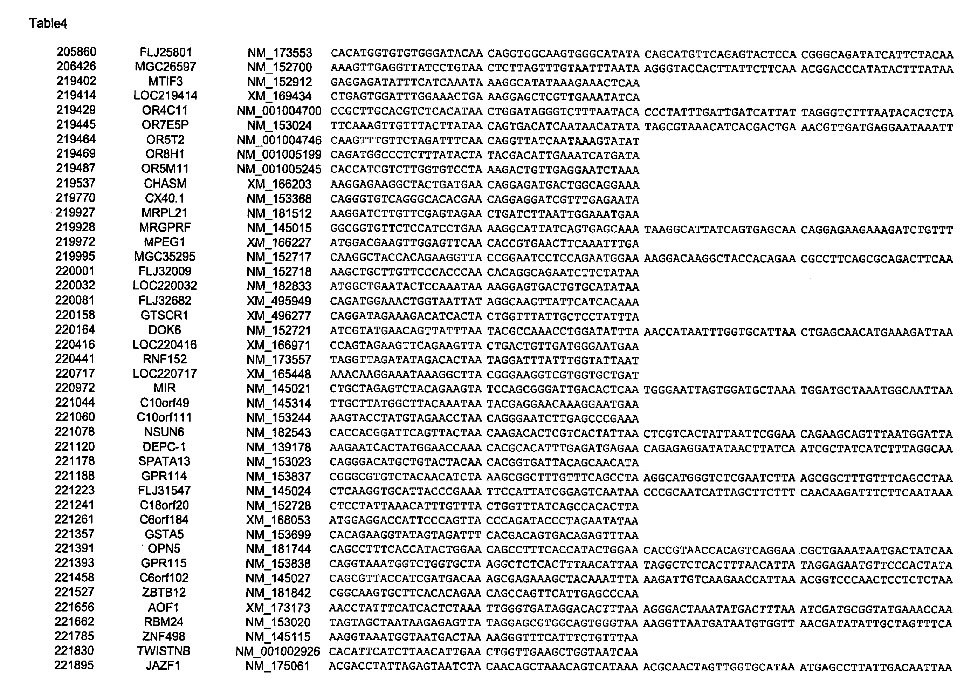

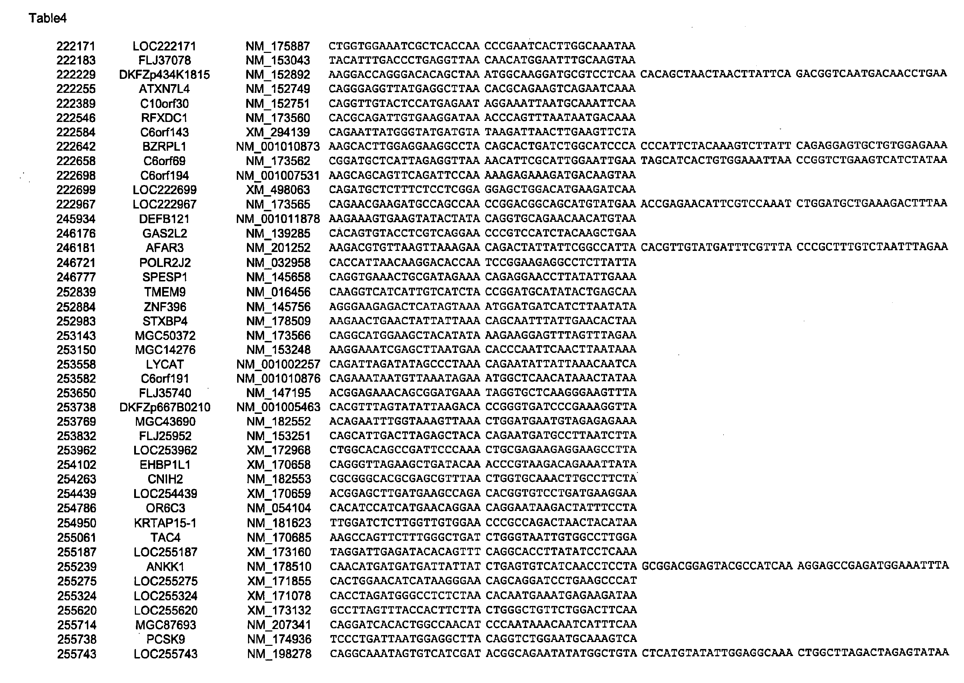

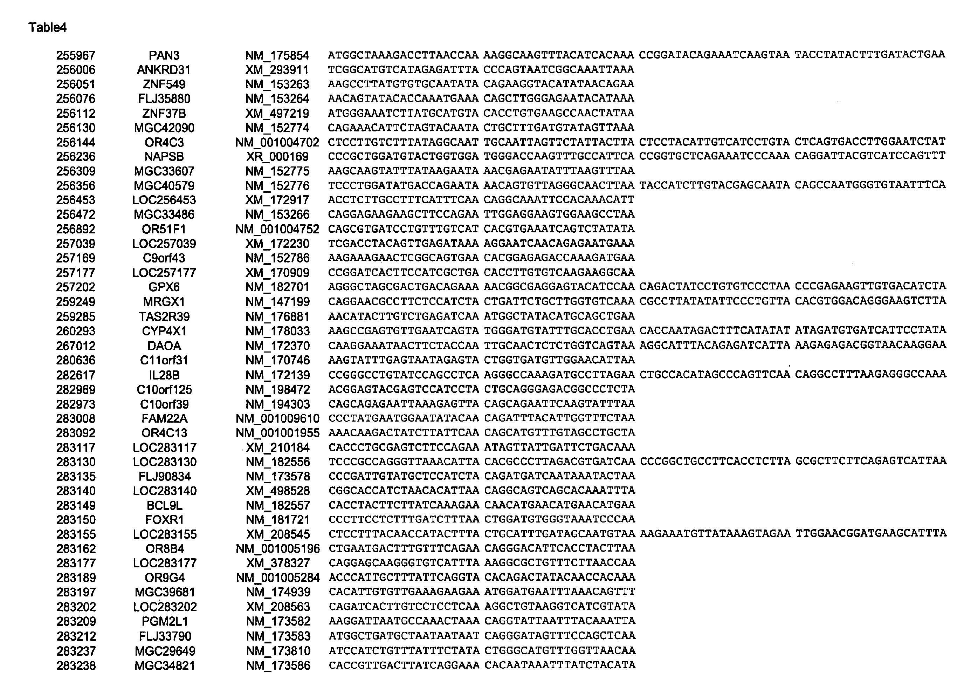

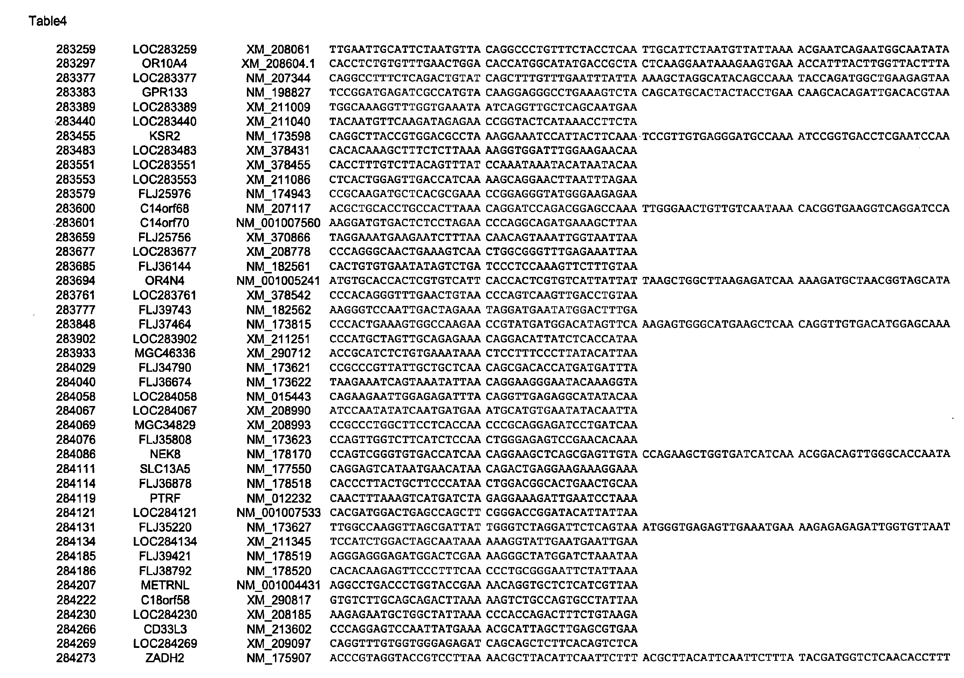

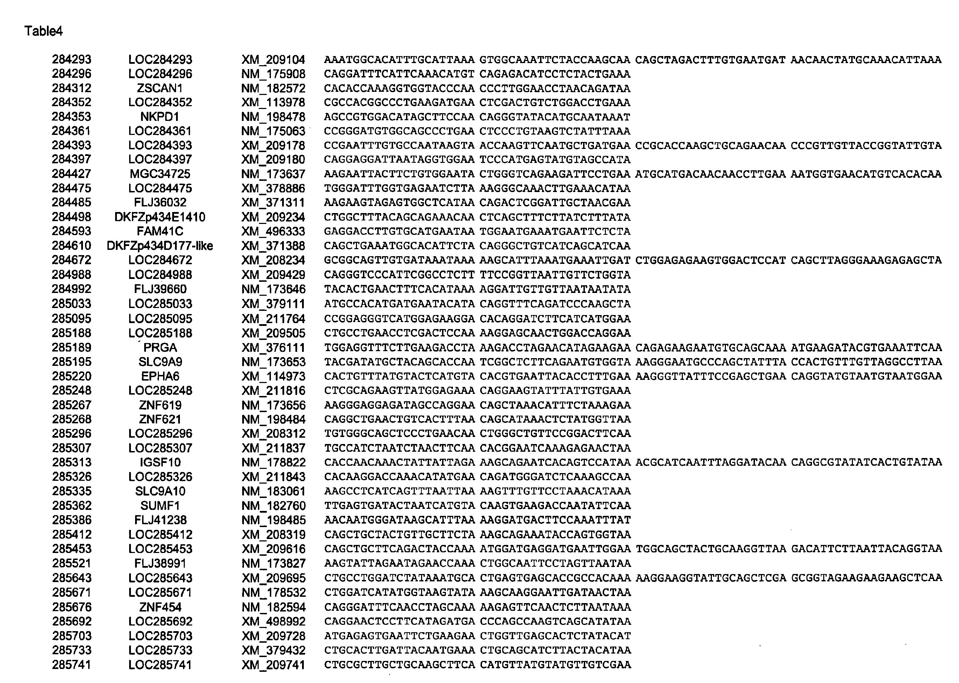

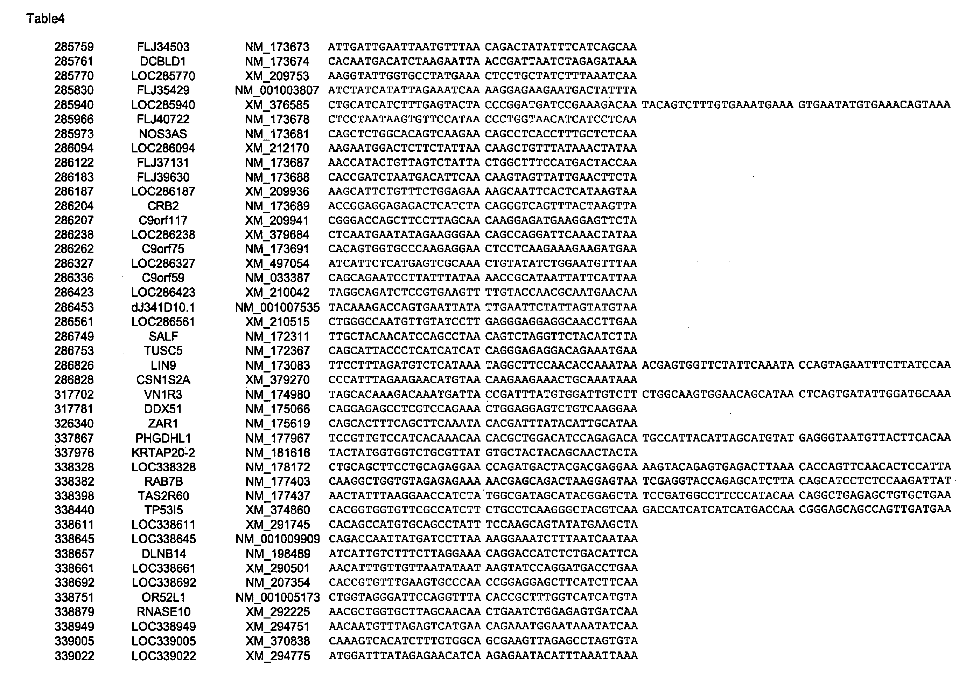

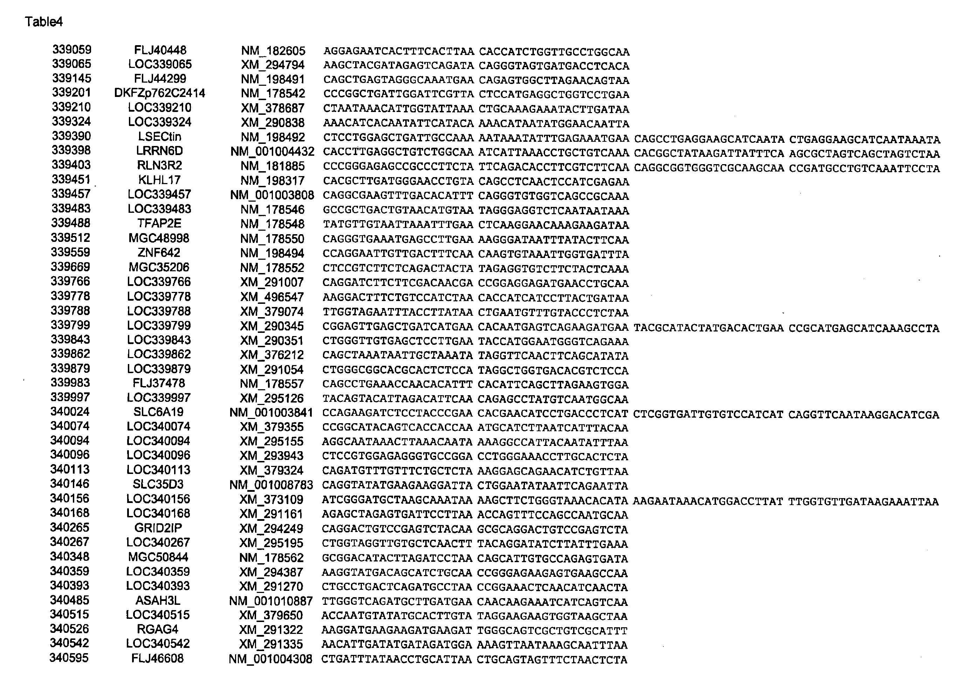

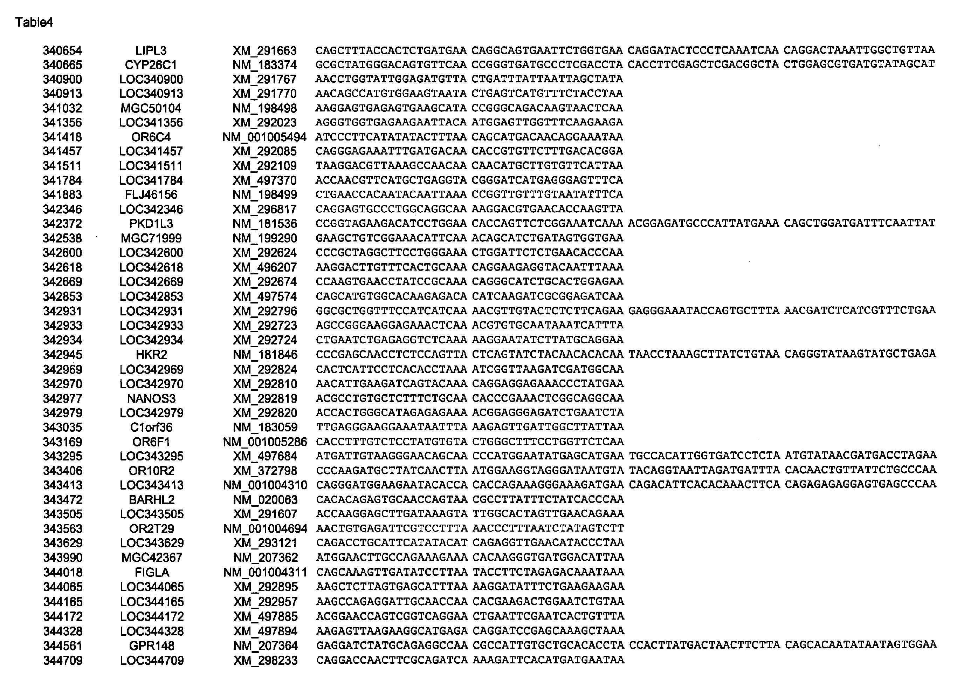

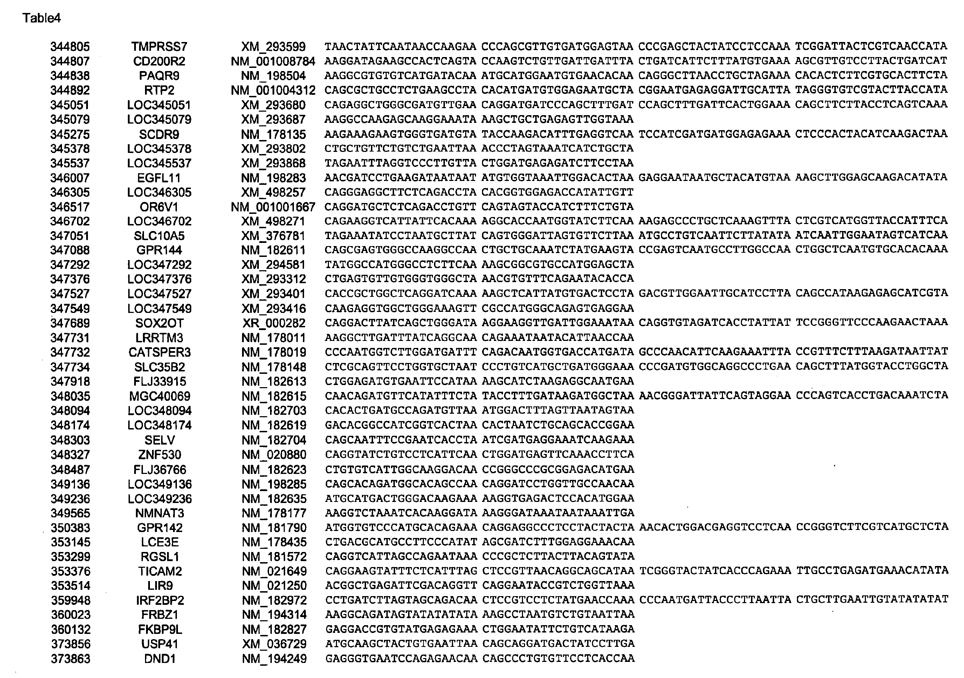

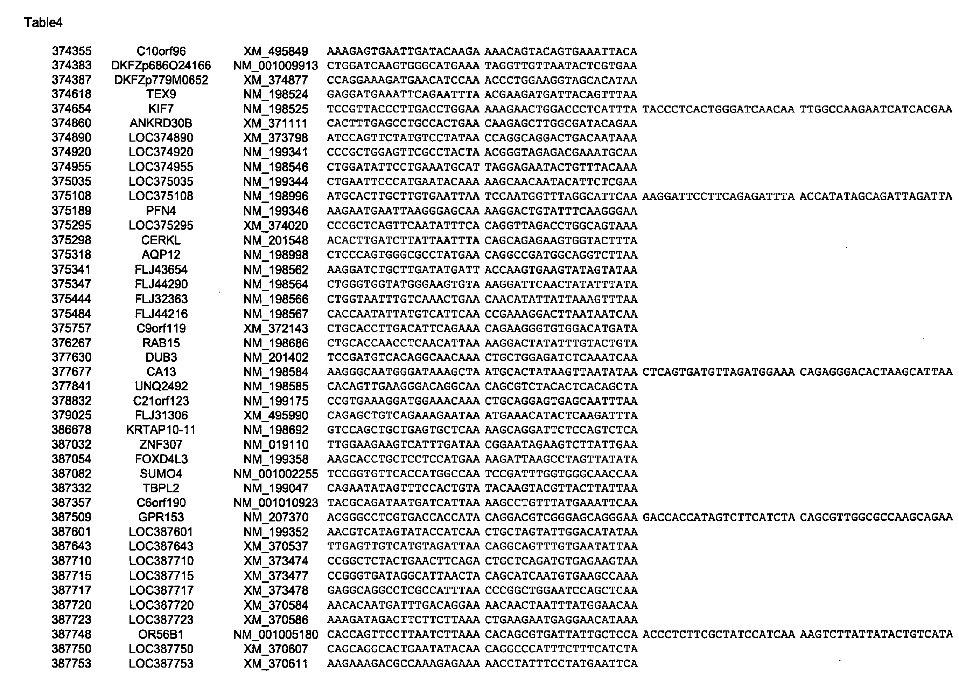

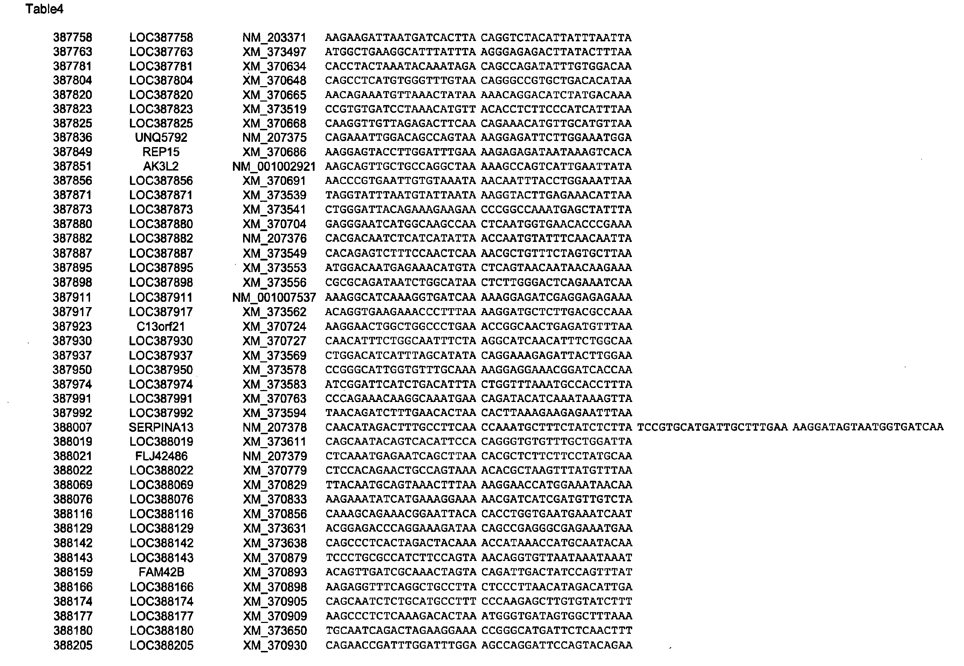

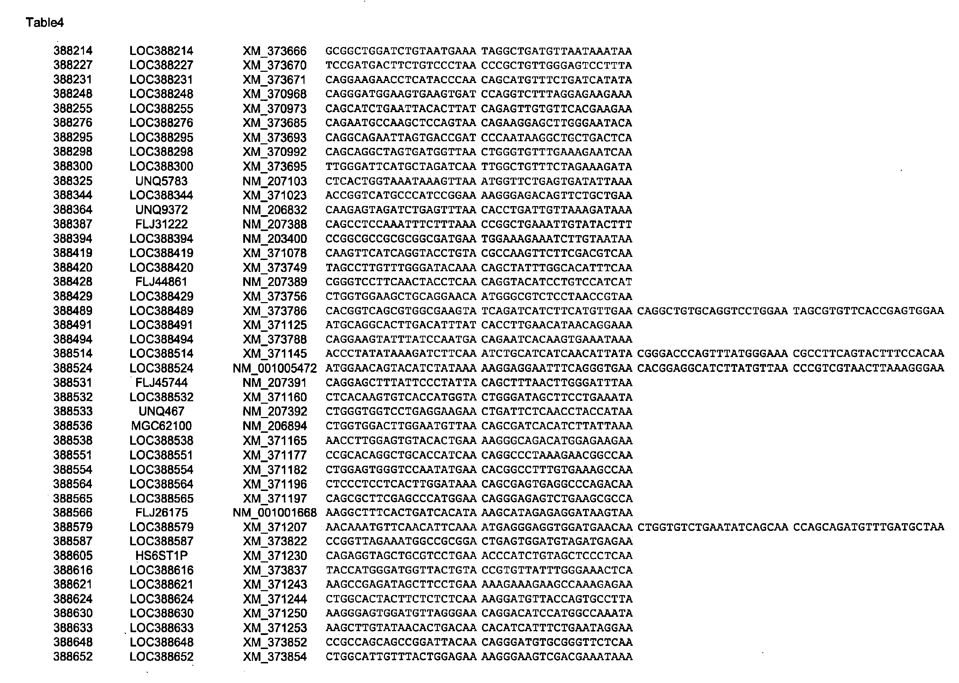

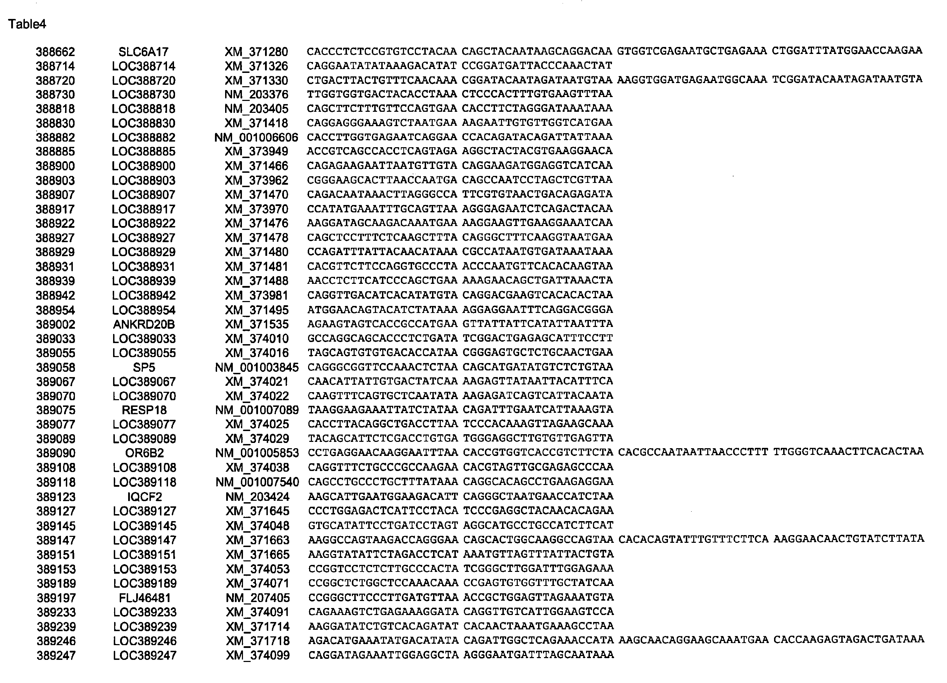

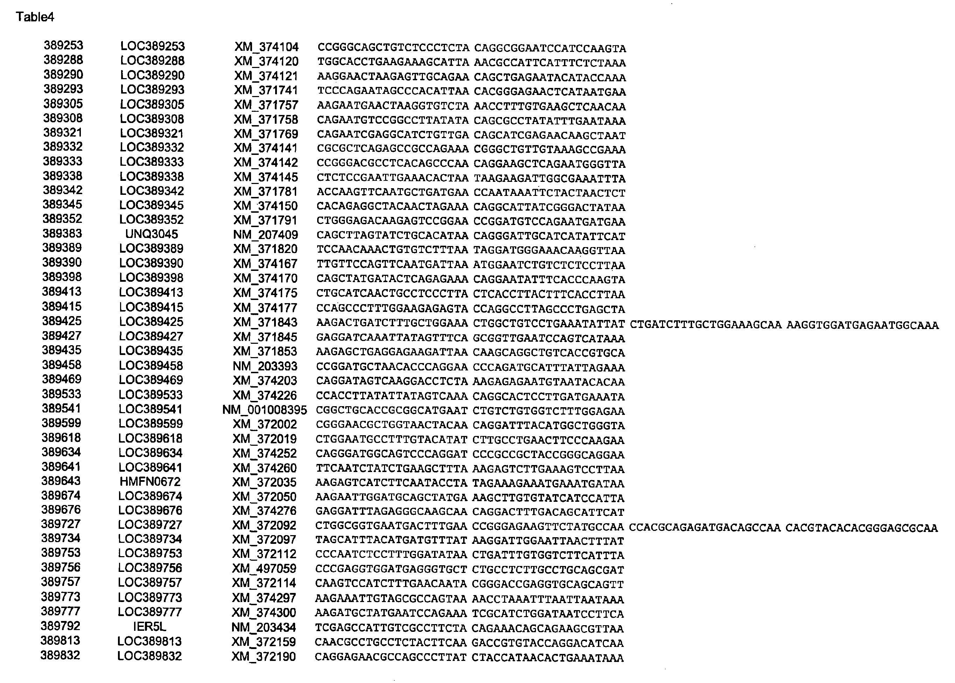

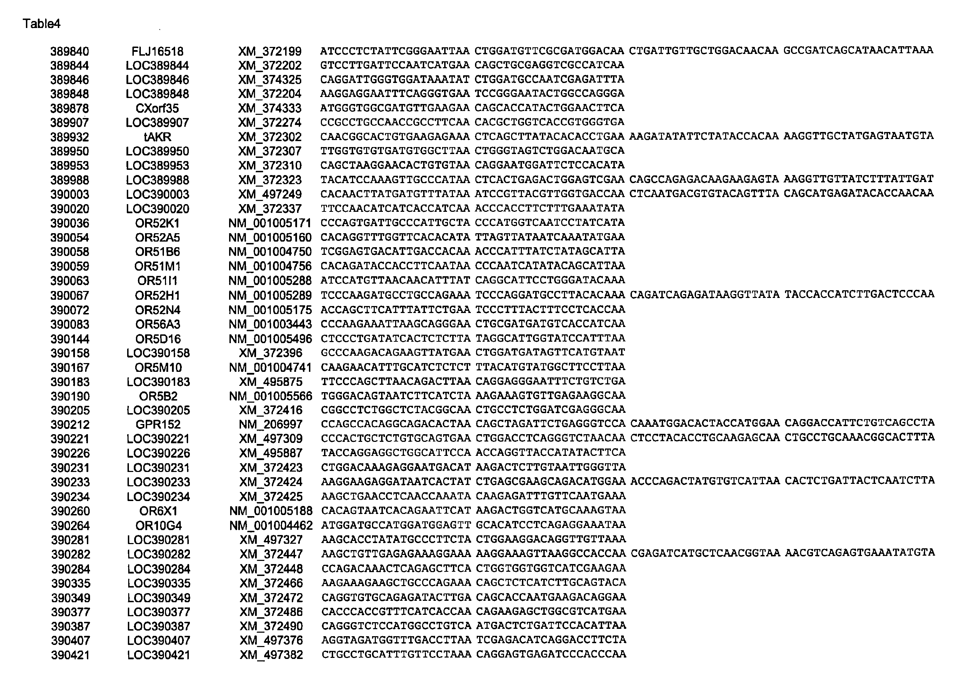

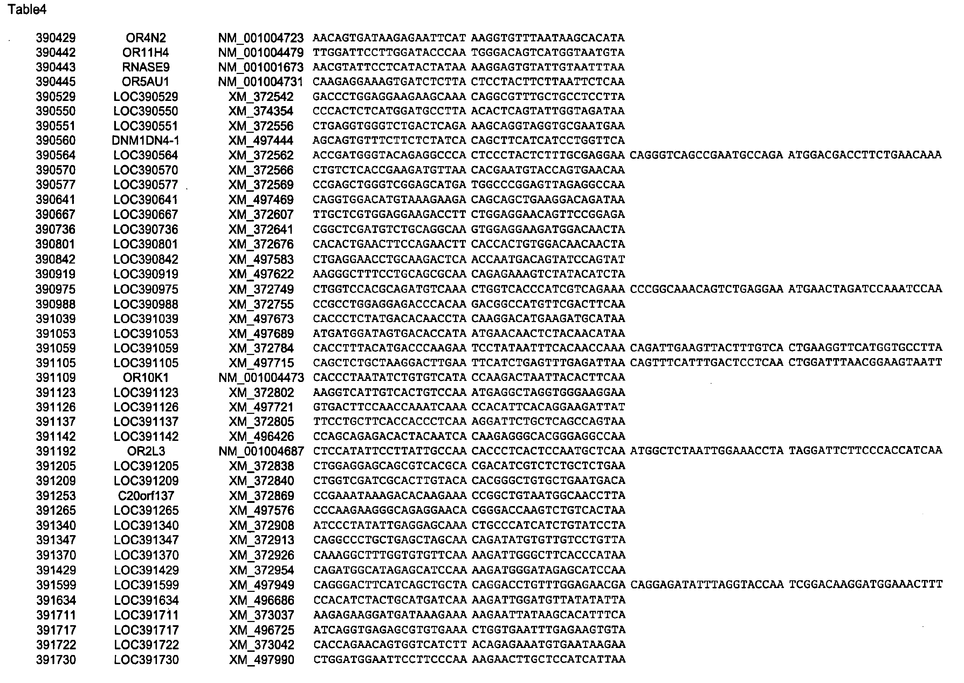

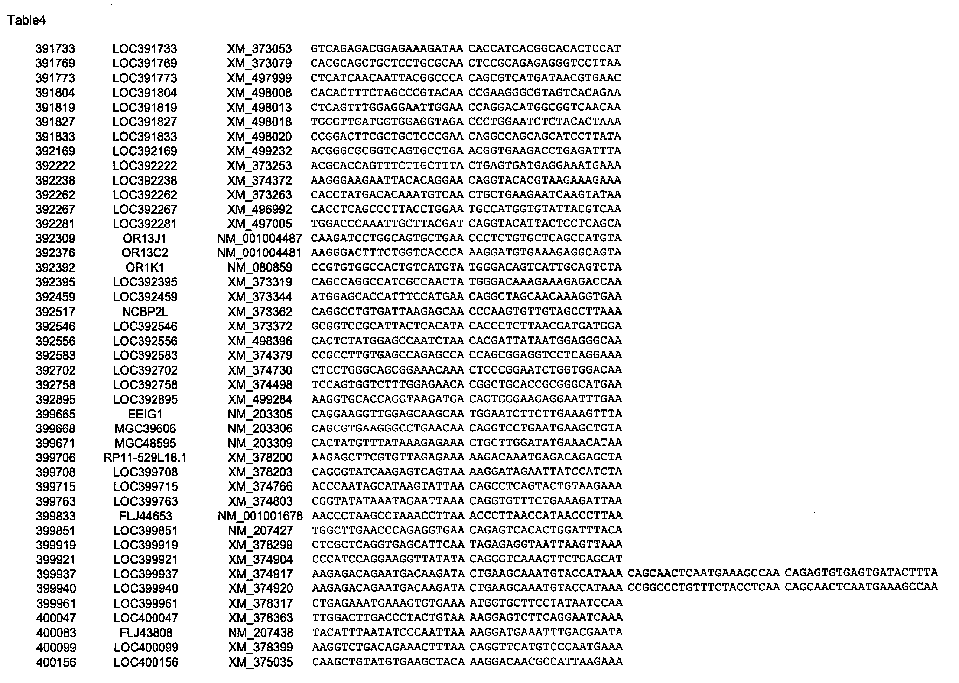

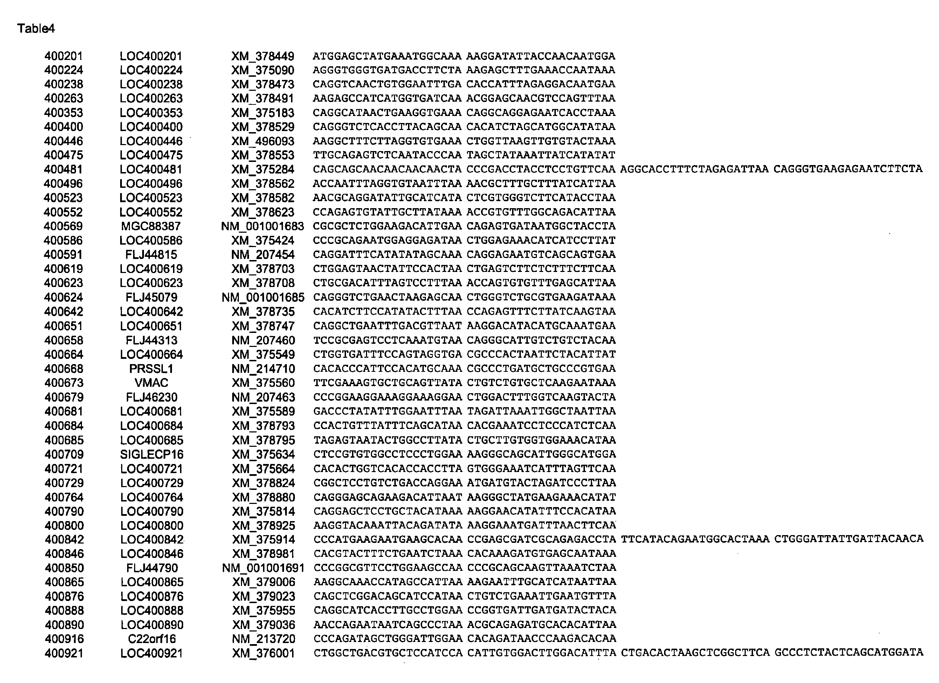

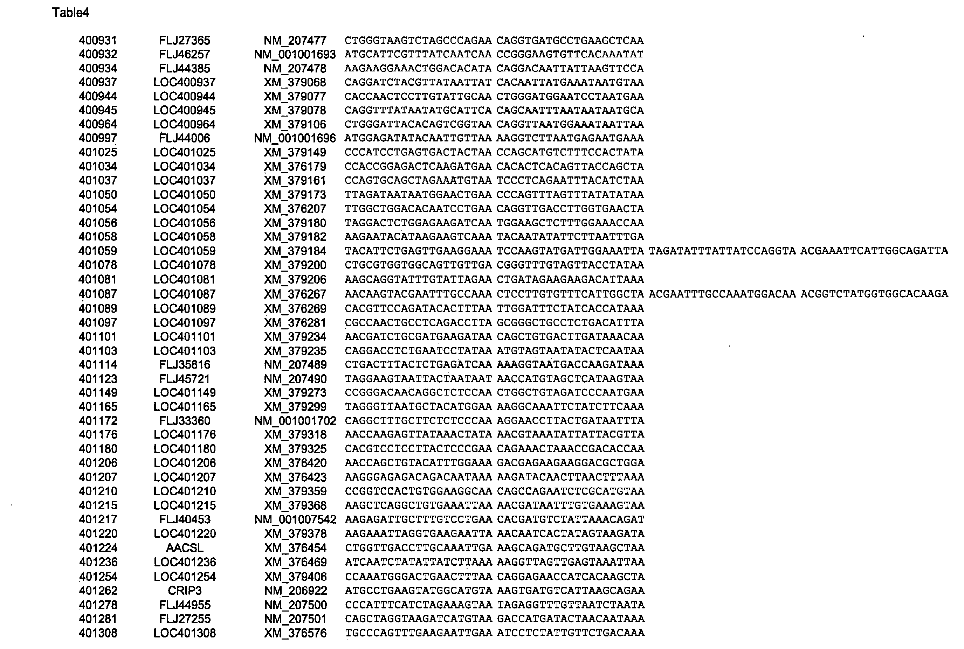

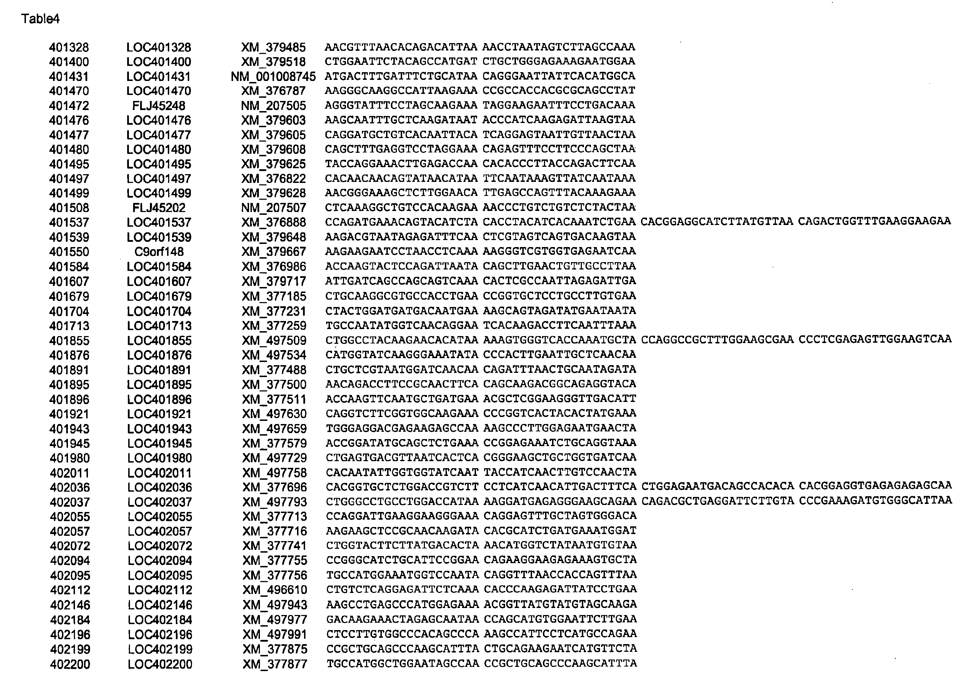

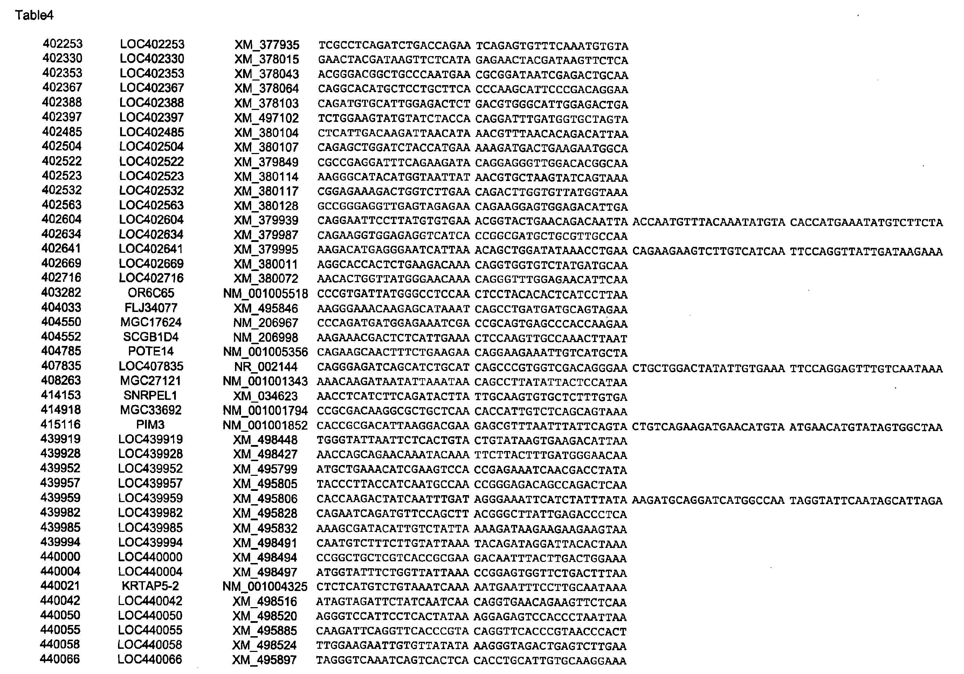

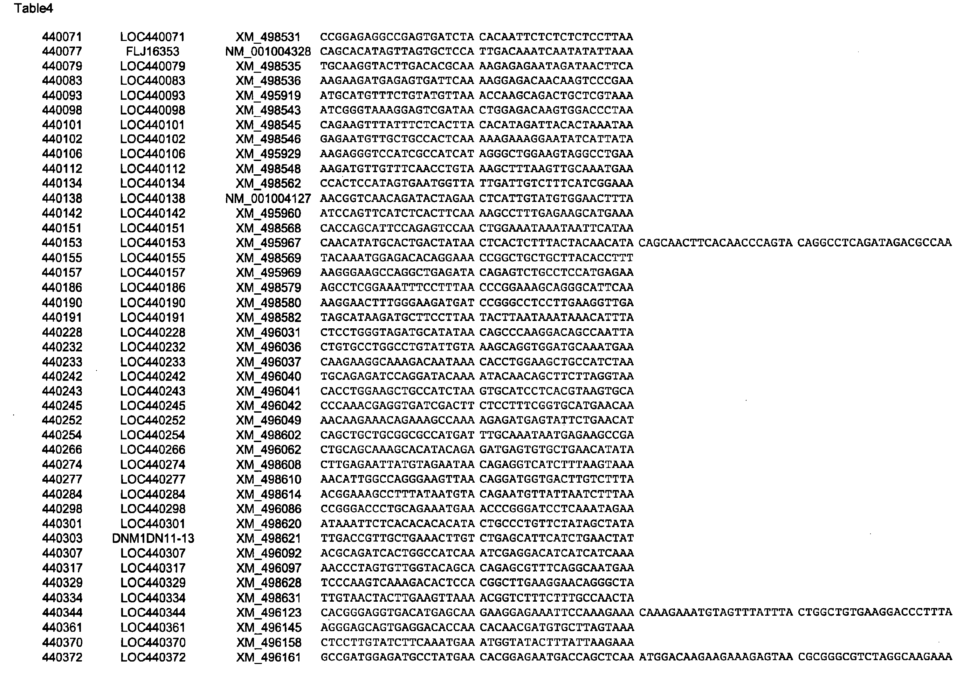

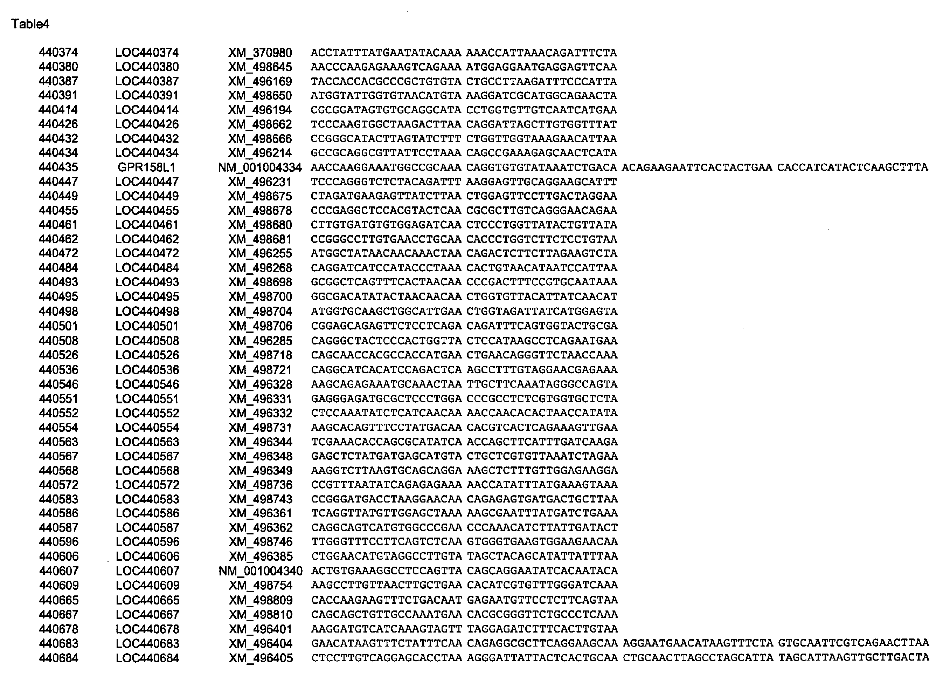

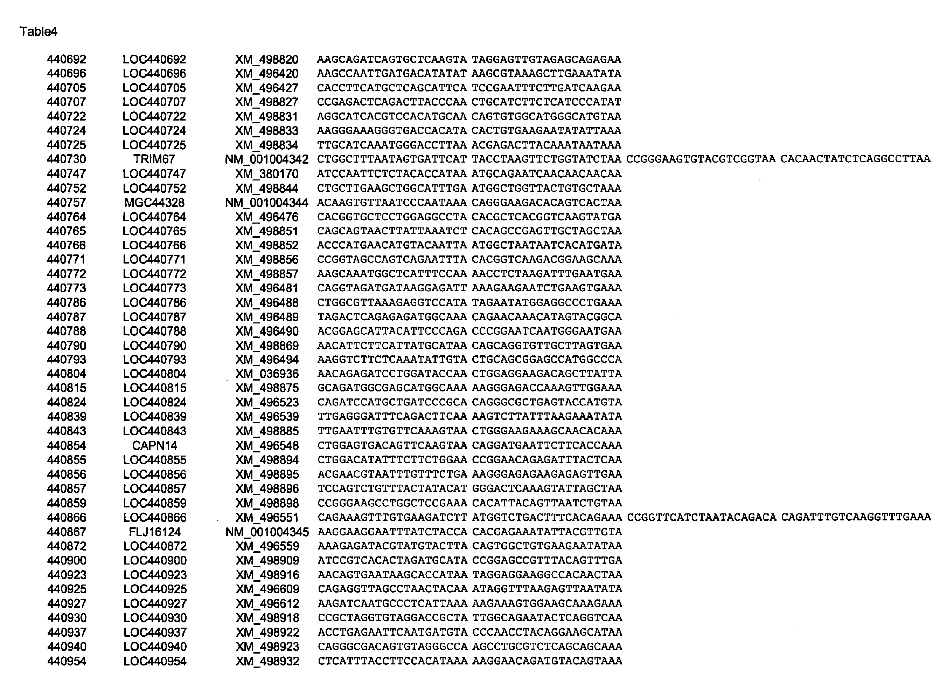

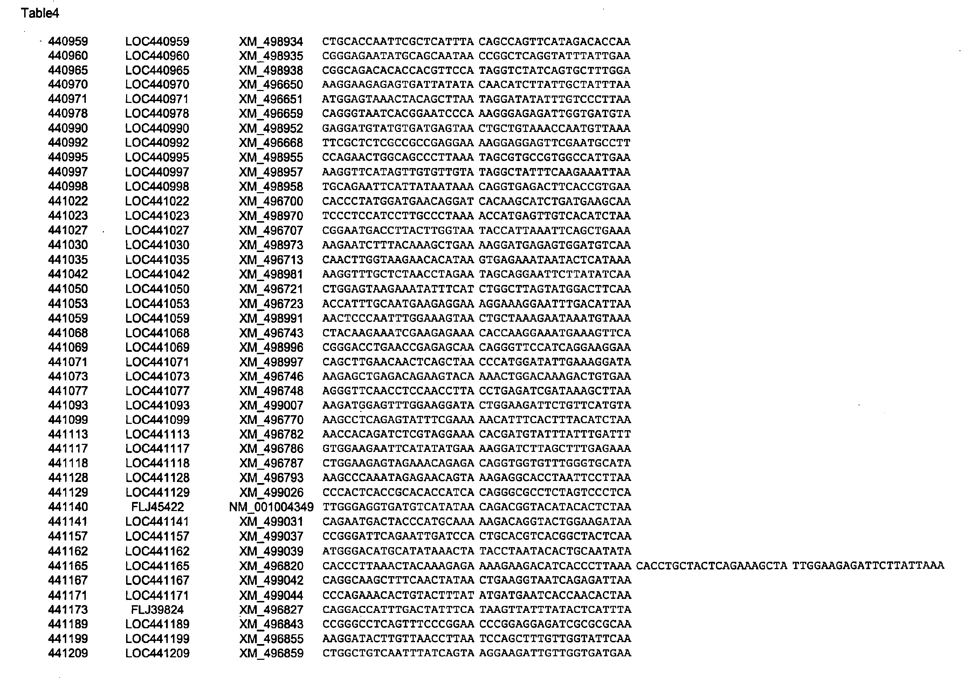

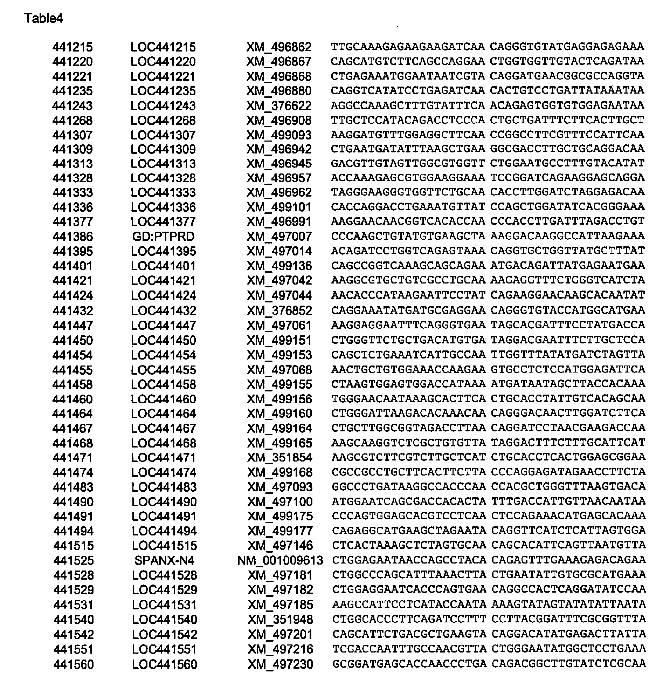

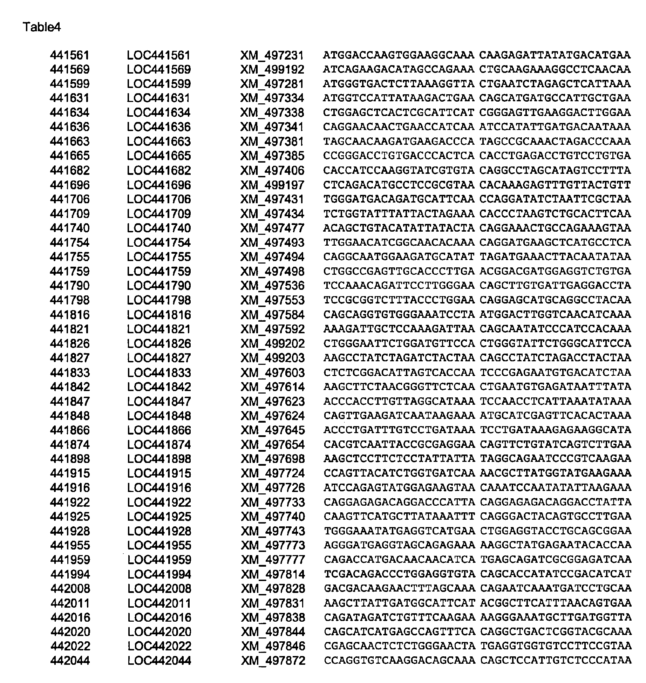

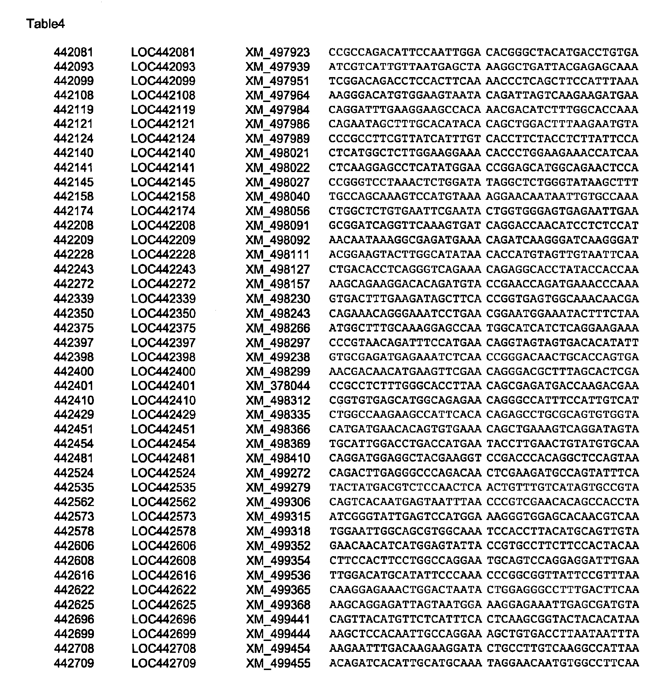

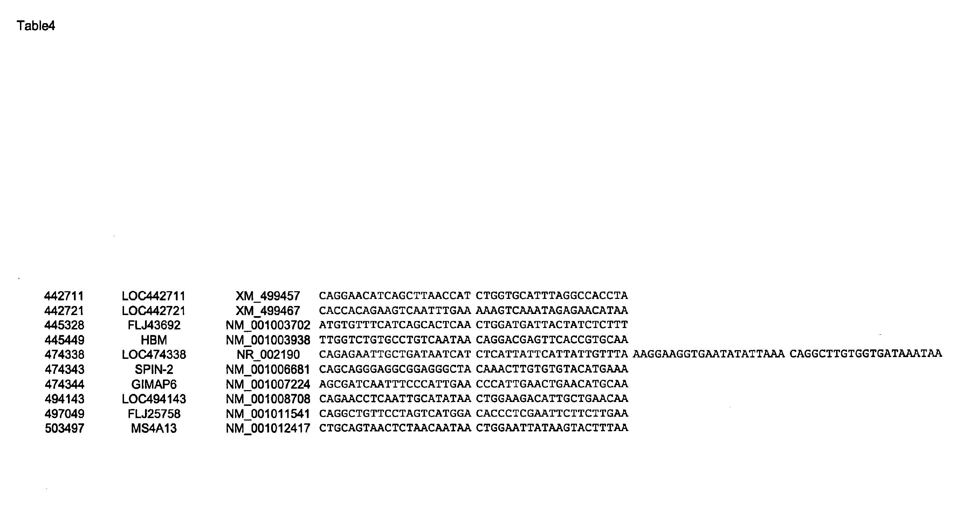

**Figure 3:** The inhibition of influenza virus replication shown for all siRNAs tested in the example.

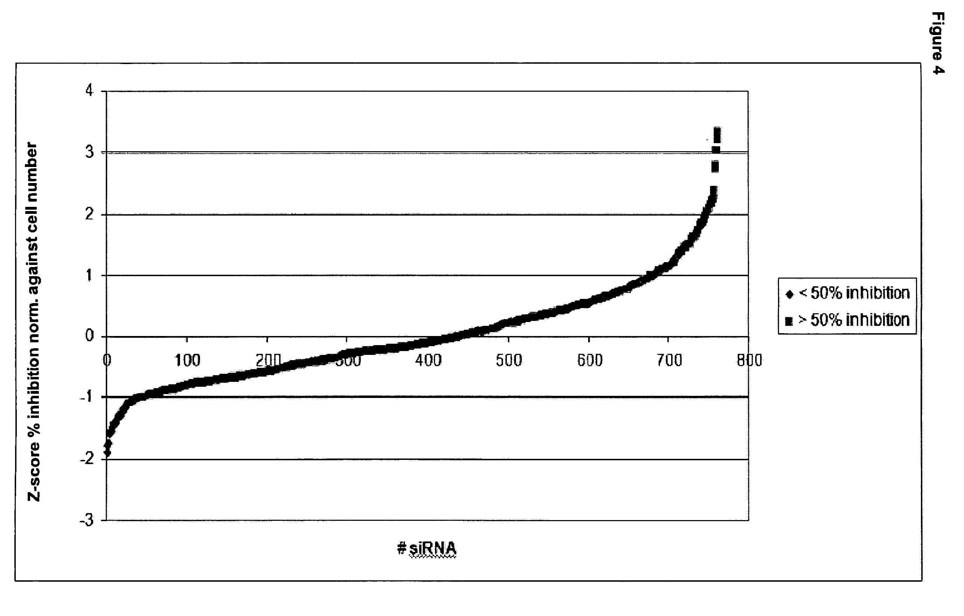

**Figure 4:** The values "% inhibition" from all analyzed siRNAs were used to calculate the z-scores. Highly efficient siRNAs are labelled in pink showing more than 50% inhibition compared to the luciferase siRNA transfected control cells.

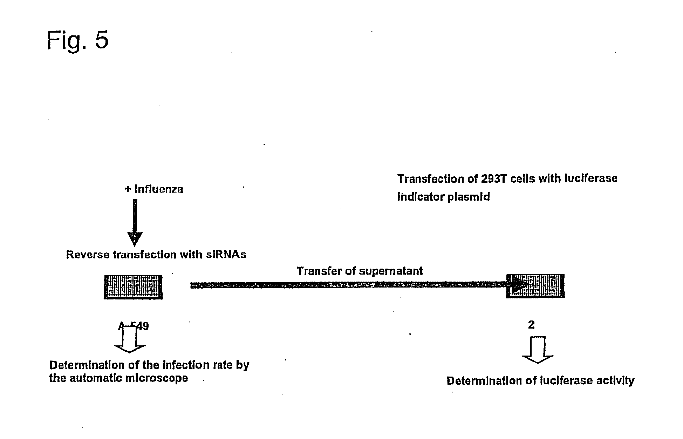

**Figure 5:** The experimental setup of the genome wide siRNA screen (see Example 4).

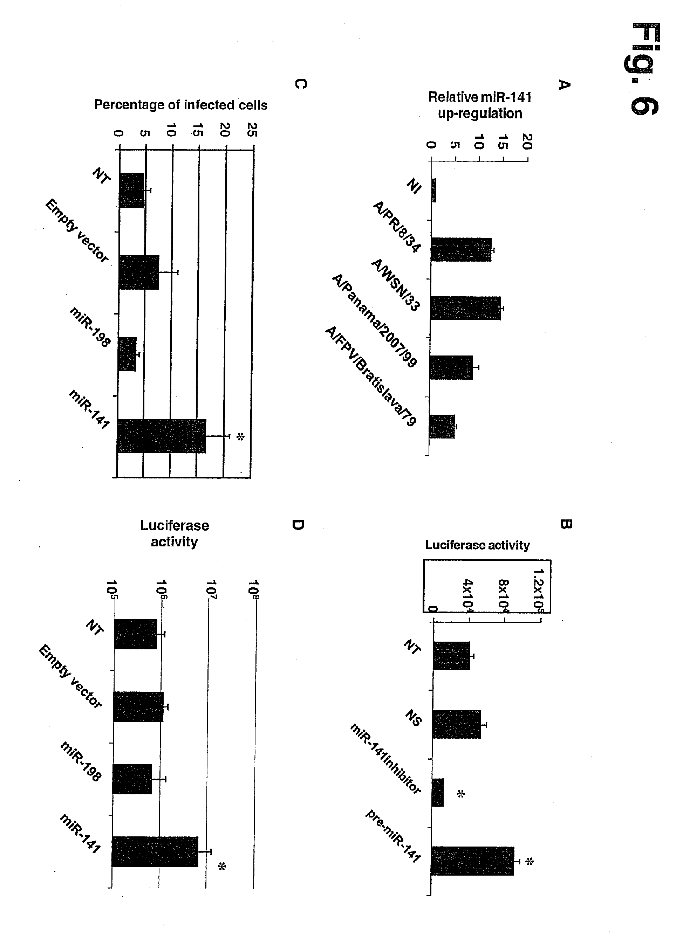

**Figure 6: Expression of miR-141 is increased in human lung epithelial cells upon infection with influenza A and enhances virus replication. (A)** Up-regulation of miR-141 at 24 h p.i. is detected by qRT-PCR upon infections of A549 human lung epithelial cells with the human influenza strains A/WSN/33 (H1N1), A/Puerto Rico/8/34 (H1N1), A/Panama/99 (H3N2) and the avian influenza strain A/FPV/Bratislava/79 (H7N7). Expression of miR-141 is normalized to non-infected (NI) cells. **(B)** Viral propagation (A/WSN/33) in A549 cells (MOI 0.05) after transfection with a miR-141 specific precursor or inhibitor, or a non-specific RNA inhibitor (NS). Virus containing supernatants from transfected and infected A549 cells were quantified using an influenza dependent luciferase assay. **(C, D)** Virus replication in different stably transduced miRNA over-expressing A549 cells. The percentage of infected cells in the primary cell culture **(C)** and the resulting virus progeny **(D)** was quantified using automated microscopy and luciferase assays, respectively. Data are mean +SD of triplicate samples (A, C) or of three independent experiments (B, D). * *t-test* (P<0.05). NT, non-transfected.

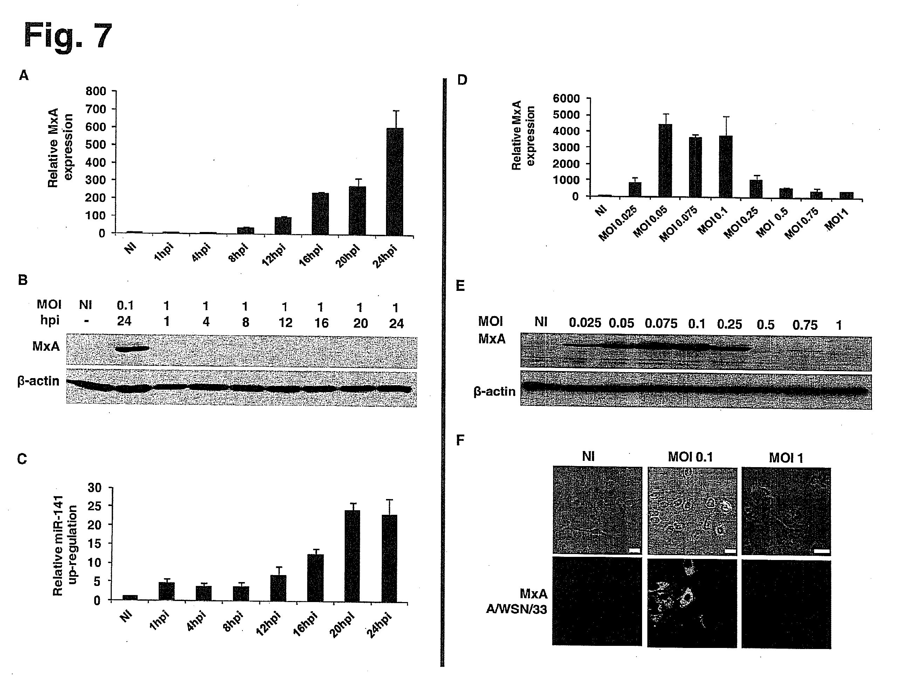

**Figure 7: MxA and miR-141 gene expression upon influenza A infections. (A-C)** To analyze the time-dependent relationship between miR-141 and MxA expression, A549 cells were infected with influenza A/WSN/33 (MOI 1). At the indicated time points, levels of MxA mRNA **(A)**, MxA protein **(B)** and miR-141 **(C)** were determined by qRT-PCR (RNA) and immunoblotting analysis (protein). β-actin serves as a loading control for immunoblotting **(D-F)** A549 cells were infected with influenza A/WSN/33 at the indicated MOIs and at 24 h p.i. cells were lysed. Levels of MxA mRNA **(D)** and MxA protein **(E)** were determined by qRT-PCR and immunoblotting, respectively. β-actin serves as a loading control for immunoblotting. **(F)** Detection of MxA (green) and nucleoprotein (NP) of influenza A/WSN/33 (red) at the indicated MOIs by immunofluorescence (24 h p.i.). Scale bar, 10μ m. MxA expression is relative to non-infected (NI) control in A, C and D. Data are mean + SD of triplicate samples.

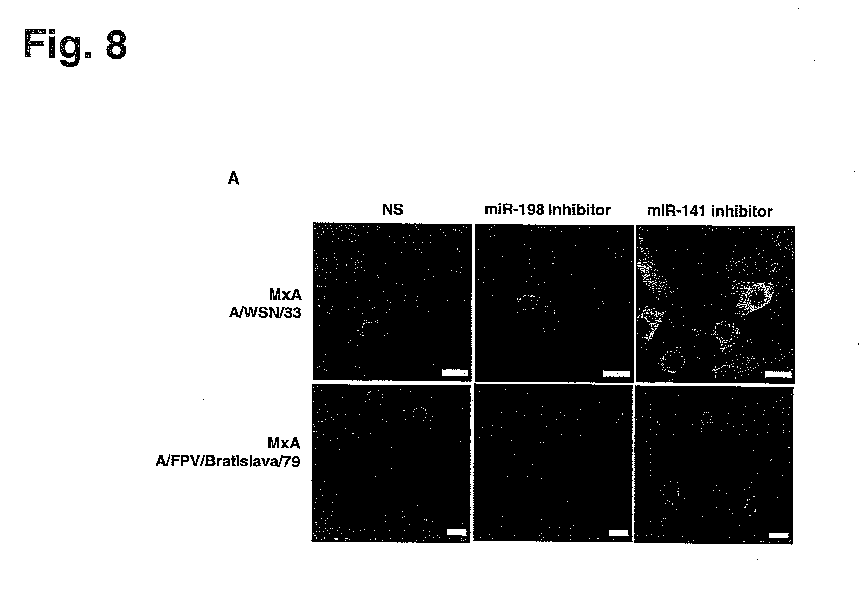

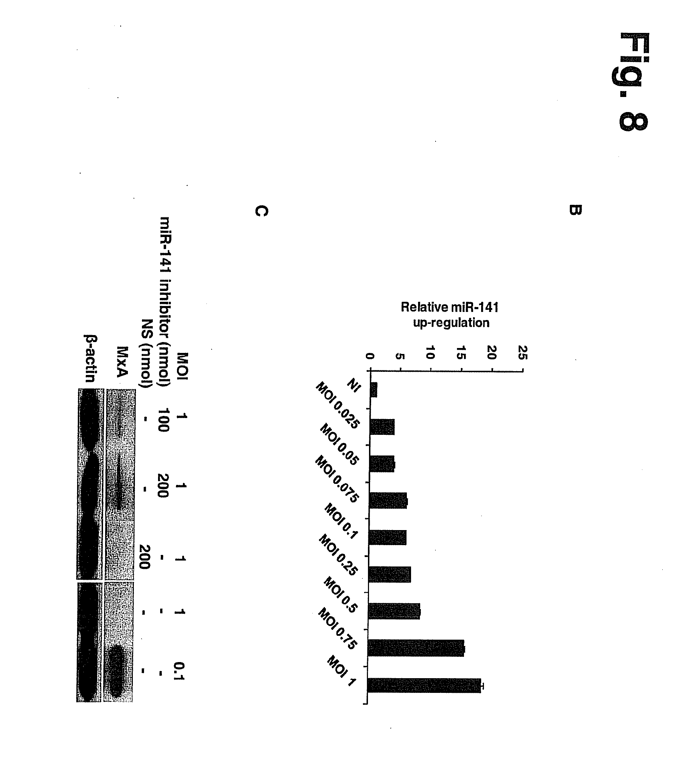

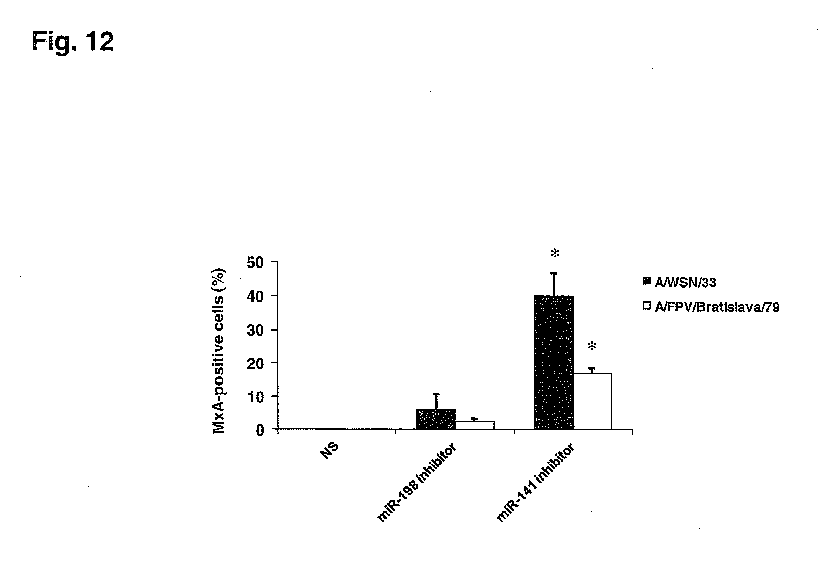

**Figure 8: Inhibition of miR-141 function restores MxA gene expression. (A)** Detection of MxA (green signal) and the nucleoprotein (NP, red signal) in A549 cells (24 h p.i.). Cells were transfected with a miR-141 inhibitor and control inhibitors (miR-198; non-specific, NS), respectively, and 8h later infected with influenza A/WSN/33 or A/FPV/Bratislava/79 (MOI 1). Scale bar, 10 μm. Arrows indicate MxA-positive influenza infected cells. Quantification of MxA-positive influenza infected cells is provided in Fig. 12. **(B)** Levels of miR-141 upon influenza A infections with different MOIs, as quantified by qRT-PCR. Up-regulation is relative to non-infected cells (NI). Data are mean + SD of triplicate samples. **(C)** Immunoblot analysis of MxA gene expression. Cell lysates from A549 cells were transfected with the specified amounts of miR-141 and non-specific inhibitors and infected with influenza A/WSN/33 at the indicated MOIs. β-actin serves as a loading control. Blot is representative of three independent experiments.

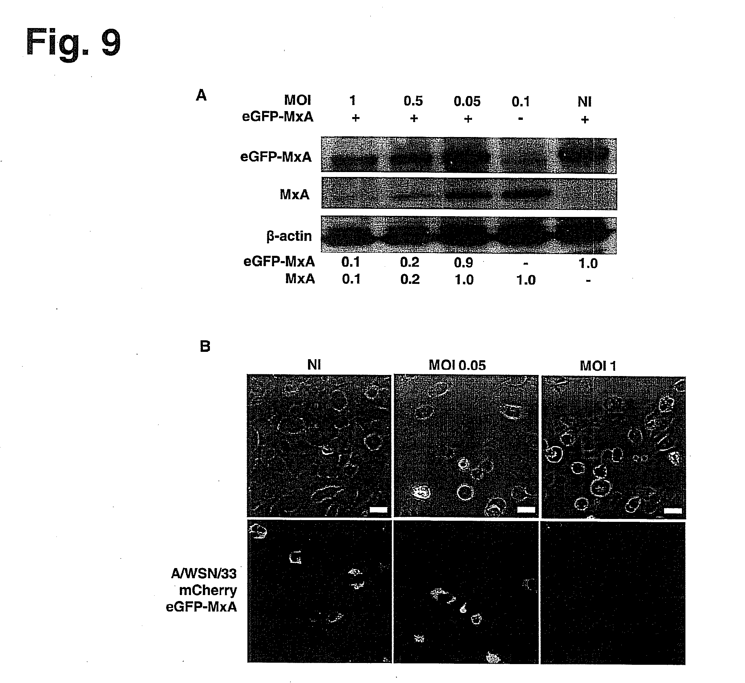

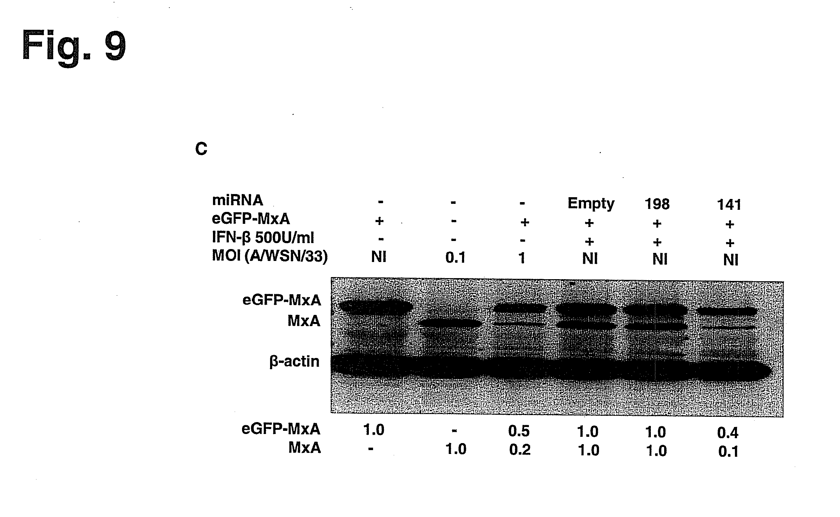

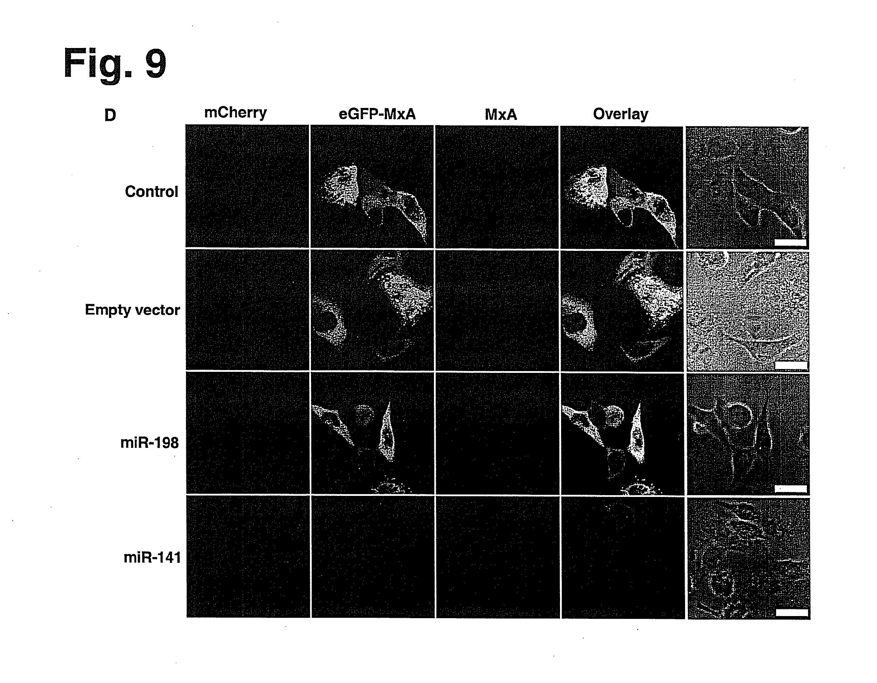

**Figure 9: Down-regulation of MxA is directly mediated by miR-141. (A)** Immunoblot analysis of endogenous MxA protein and the exogenous eGFP-MxA fusion protein (24 h p.i.). A549 cells were infected with influenza A/WSN/33 at the indicated MOIs and 1 h later transfected with pEGFP-MxA. β-action serves as a loading control. Blot depicted is representative of three independent experiments. MxA and eGFP-MxA band intensities are shown below blot **(B)** Representative confocal images of infected and transfected A549 cells at 24 h p.i. with viral NP staining (blue), mCherry expression (red) and eGFP-MxA expression (green). Scale bar, 10 μm. Quantification of eGFP-

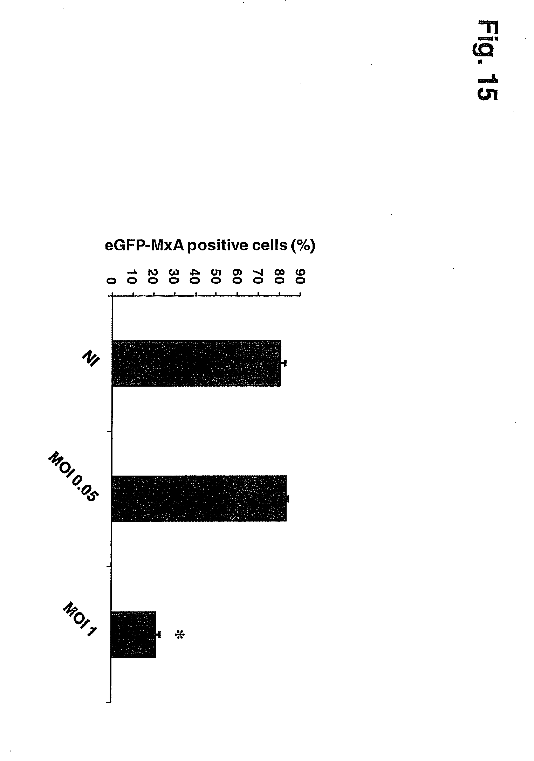

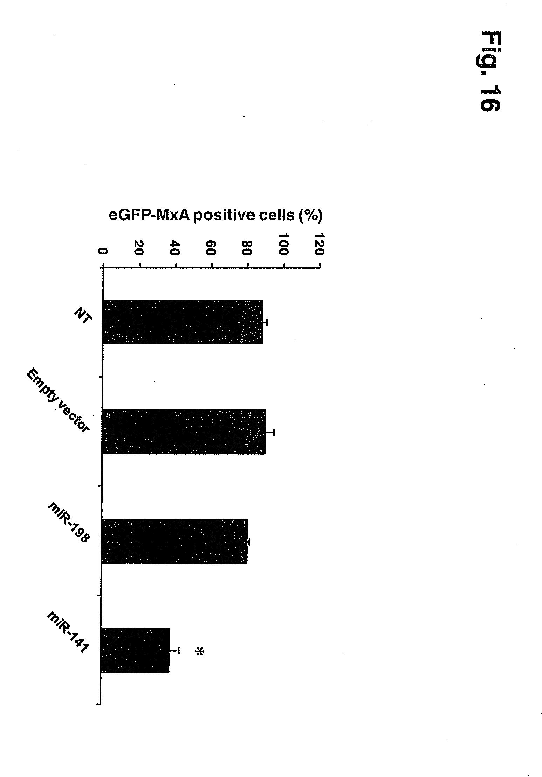

MxA positive cells is provided in Fig. 15. **(C)** Expression of MxA and the eGFP-MxA fusion protein in transfected or influenza A/WSN/33 infected cells. A549 cells were infected at the indicated MOIs and left untreated or transfected with the different miRNA over-expressing plasmids (empty, miR-198 and miR-144) for 6 h, followed by transfections with pEGFP-MxA and simultaneous treatment with IFN-β (500 U/ml). β-actin serves as a loading control. Blot depicted is representative of three independent experiments. MxA and eGFP-MxA band intensities are shown below blot **(D)** Representative confocal micrographs depicting levels of mCherry (red), the exogenous eGFP-MxA (green) and the endogenous MxA (blue) in different miRNA over-expressing or control cells. Arrows indicate transfected cells (mCherry positive) that exhibit both a down-regulation of endogenous MxA and no or weak expression of eGFP-MxA. Scale bar, 10 μm. Quantification of eGFP-MxA positive cells is provided in Fig. 16.

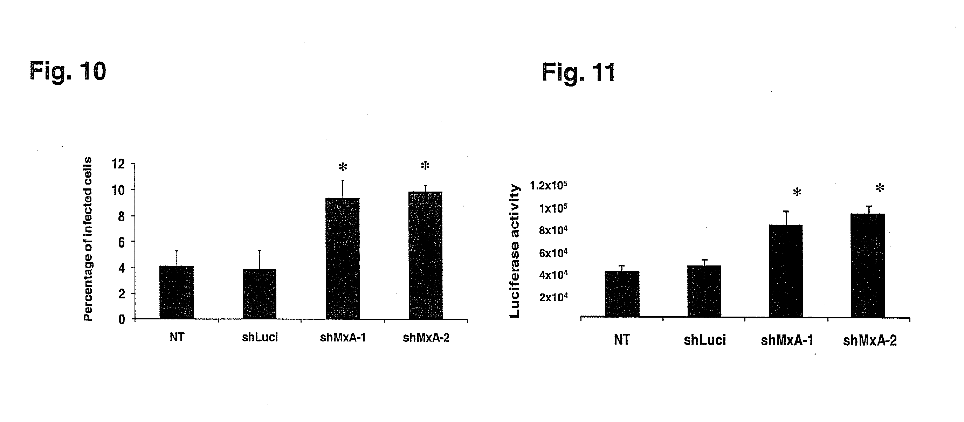

**Figure 10 and 11: Levels of primary infection and virus progeny in MxA deficient, influenza A/WSN/33 infected A549 cells (MOI 0.05).** Primary infection was determined at 24 h p.i. by staining cells with an influenza NP specific antibody, followed by treatment with Hoechst dye to stain for nuclei. Numbers of infected and uninfected cells were quantified using automated microscopy and the percentage of infected cells was calculated (Fig. 10). Virus progeny in cell supernatant was detected using the influenza dependent luciferase assay (Fig. 11). Error bars indicate the standard deviation (SD) of triplicates. NT, non-transfected. * /t-test/ (P<0.05).

**Figure 12: Quantification of endogenous MxA in influenza infected cells.** A549 cells were transfected with miR-141 and control inhibitors (non-specific (NS); miR-181), respectively, and infected with influenza A/WSN/33 or A/FPV/Bratislava/79 (MOI 1) at eight hours post transfection. At 24 h p.i., expression of MxA was analyzed in influenza infected cells (-100 cells per treatment), using immunofluorescence confocal microscopy. Numbers of MxA positive cells depicted as mean percentage of total cell number + SD. Cells transfected with the miR-141 inhibitor significantly increased numbers of MxA positive cells in comparison to control inhibitors after infection at an MOI of 1. * /t-test/ (/P/<0.05).

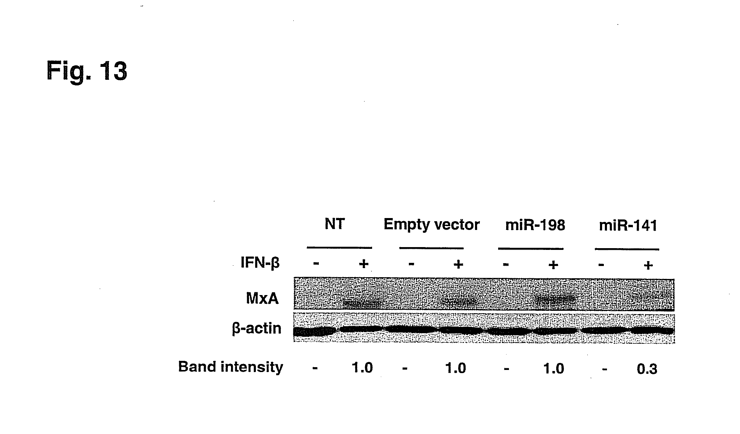

**Figure 13: Expression of MxA in stable miRNA over-expressing A549 cells.** Cells previously transduced with retroviral vectors coding for the indicated miRNAs or the empty control vector, were left untreated or were treated with IFN-β (500U/ml) for 24 h. A representative blot from three independent experiments is depicted. MxA band intensities were determined using the Aida image analyzer program (V.4.03).

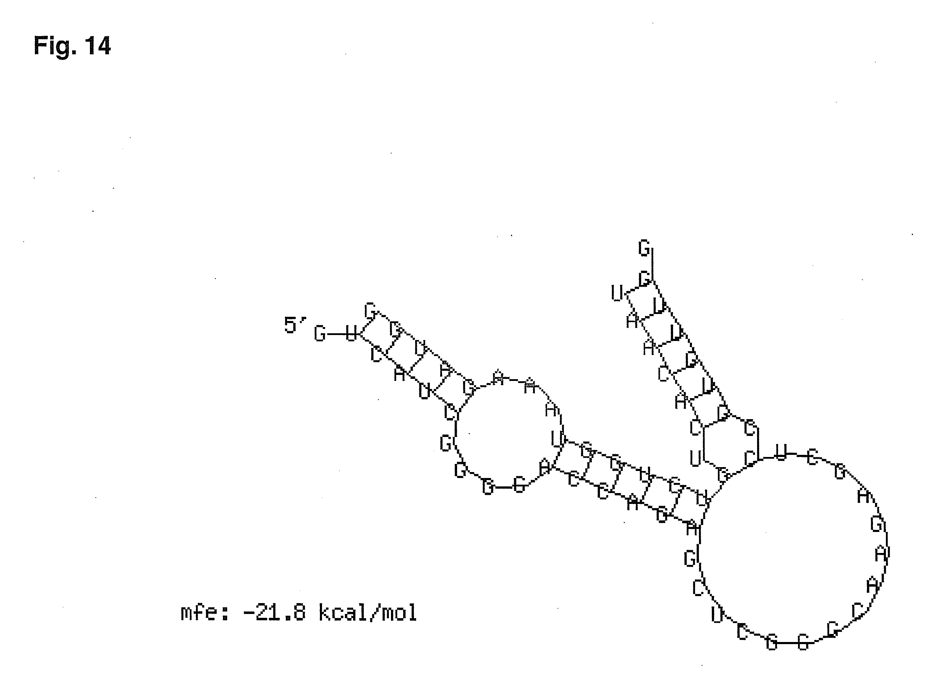

**Figure 14:** Predicted interaction between has-miR-141 (UAACACUGUCUGGUAAAGAUGG, miRBase sequence database (/8/) and the complete mRNA sequence of MxA (NM_002462) with the lowest minimum free energy (mfe) according to the web tool RNAhybrid (/9/).

**Figure 15: Quantification of eGFP-MxA-positive cells.** A549 cells were infected with influenza A/WSN/33 at the indicated MOIs. One hour later cells were co-transfected with an eGFP-MxA fusion plasmid to detect exogenous MxA and a constitutively expressed mCherry construct (molar ratio 6:1) to normalize the transfection efficiency. At 24 h p.i., expression of eGFP-MxA was analyzed in 100 cells per treatment using immunofluorescence confocal microscopy. Numbers of eGFP-MxA and mCherry positive cells depicted as mean percentage of total cell number + SD. At an MOI of 1, the percentage of eGFP-MxA positive cells decreases significantly in comparison to non-infected (NI) and partially infected cell cultures (MOI 0.05). * /t-test/ (/P/<0.05).

**Figure 16: Quantification of eGFP-MxA-positive cells in the absence of infection.** A549 cells were transfected with different over-expressing plasmids (empty, miR-198 and miR-141) for 6 h, followed by transfection with the eGFP-MxA fusion plasmid (to detect exogenous MxA) and simultaneous treatment with IFN-β (500U/ml). Expression of eGFP-MxA was analyzed in -100 mCherry positive cells per treatment using immunofluorescence confocal microscopy. Numbers of eGFP-MxA positive cells depicted as mean percentage of total cell number + SD. Cells transfected with the miR-141 over-expressing plasmid exhibit a significant reduction in eGFP-MxA positive cells in comparison to controls (non-transfected (NT), empty vector and miR-198). * /t-test/ (/P/<0.05).

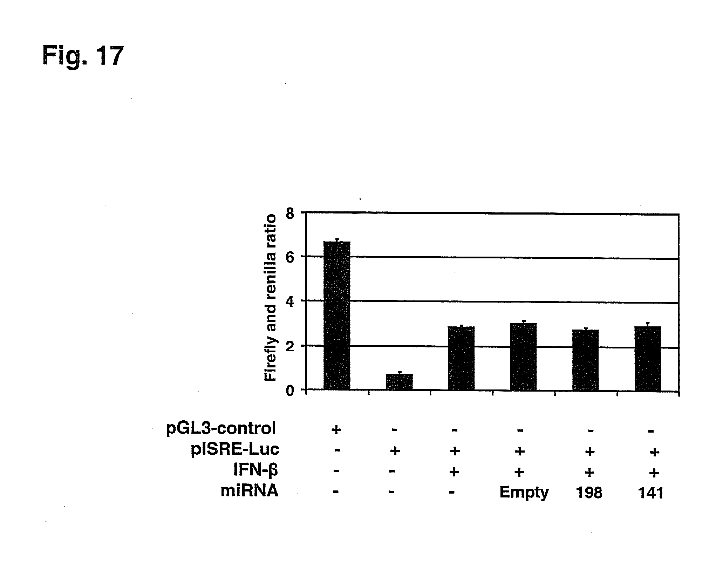

**Figure 17:** The ISRE promoter activity detected by a dual luciferase assay in stably transduced miRNA over-expressing cells upon IFN-β stimulation (1000 U/ml). These A549 cells were transfected with plasmids coding for the firefly luciferase gene driven by either a promoter that contains a ISRE motif within the minimal promoter or driven by a constitutive SV40 promoter (pGL3-control) as a control. In addition, cells were transfected with a /Renilla/ luciferase plasmid as a transfection control, directly followed by stimulation with IFN-β. The ratio of firefly to /Renilla/ luminescent signals is shown. Error bars indicate the standard deviation (SD) of four duplicates.

**Figure 18: Sequences of miR-141/141* and miR-200c/200c*, and precursors thereof.** Further, the hairpin structures of the precursers are described.

**Table 1:** Results of the siRNa kinase screen: **a:** activation ("negative" inhibition) of virus replication in %, normalized against the cell number, and the standard deviation calculated using four independent experiments. **b:** inhibition of virus replication in %, normalized against the cell number, and the standard deviation calculated using four independent experiments. Pool X, wherein X denotes the number of the pool, refers to combinations described in Table 3.

**Table 2:** Oligonucleotide sequences employed in the siRNA kinase screen of example 1. Knock-down of a particular gene was performed (a) by a combination of two oligonucleotide sequences ("target 1" and "target 2") specific for said gene, or (b) by pooled oligonucleotides specific for different genes ("Pool X", wherein X denotes the number of the pool described in Table 3).

**Table 3:** Oligonucleotide pools employed in the siRNA kinase screen of the example.

**Table 4:** Oligonucleotide sequences employed in the siRNA screen of example 4. Up to four oligonucleotide sequences ("target sequence 1", "target sequence 2", "target sequence 3", and "target sequence 4") specific for a gene were employed (each in a separate test).

**Example 1**

Since kinases are one of the most promising candidates that can influence virus progeny we used siRNAs against this group of genes to identify the individual role of each kinase or kinase binding polypeptide in respect of a modified replication of influenza viruses. All siRNAs were tested in four independent experiments. Since siRNAs against kinases can influence the replication of cells or are even cytotoxic, the effect of each individual siRNA transfection in regard to the cell number was analysed by using an automatic microscope. The amount of replication competent influenza viruses was quantified with an influenza reporter plasmid that was constructed using a RNA polymerase I promoter/ terminator cassette to express RNA transcripts encoding the firefly luciferase flanked by the untranslated regions of the influenza A/WSN/33 nucleoprotein (NP) segment. Human embryonic kidney cells (293T) were transfected with this indicator plasmid one day before influenza infection. These cells were chosen, because they show a very strong amplification of the luciferase expression after influenza A virus infection. The cell based assay comprised the following steps (also Fig. 1 which describes the experimental setting of the siRNA kinase screen):

Day 1:   Seeding of A549 cells (lung epithelial cells) in 96-well plates
Day 2:   Transfection with siRNAs directed against kinases or kinase binding proteins
Day 3:   Infection with influenza A/WSN/33 + transfection of 293T cells with the influenza indicator plasmid
Day 4:   Infection of 293T cells with the supernatant of A549 cells + determination of cell number by the automatic microscope
Day 5:   Lysis of the indicator cells and performing the luciferase assay to quantify virus replication

For the identification of influenza relevant kinases the luminescence values were normalised against the cell number (measured after siRNA transfection and virus infection). Thereby unspecific effects due to the lower (or higher) cell numbers can be minimized.

Several controls were included to be able to demonstrate an accurate assay during the whole screening procedure (Fig. 2). The control siRNA against the viral nucleoprotein could nearly reduce the replication to levels of uninfected cells.

The illustration of the inhibition in percentage shows that some siRNAs can enhance the influenza virus replication, whereas others can inhibit the replication stronger (>113%) than the antiviral control siRNA against the influenza NP gene (Fig. 3). Thereby 47 siRNA decreased the replication more than 50%, 9 siRNAs showed more than 80 % inhibition. The list of the results is provided in Table 1a and 1b, showing the activation (Table 1a, "negative" inhibition) and inhibition (Table 1b) of virus replication in %, normalized against the cell number, and the standard deviation calculated using four independent experiments.

Similar results were obtained using the calculation of z-scores. The z-score represents the distance between the raw score and the population mean in units of the standard deviation. The z-scores were calculated using the following equation:

$$z = \frac{X - \mu}{\sigma}.$$

where X is a raw score to be standardized, $\sigma$ is the standard deviation of the population, and $\mu$ is the mean of the population.

**Example 2**

In a future experiment the antiviral effect will be validated in more detail by using individual siRNAs or shRNAs instead of pooled siRNAs. Furthermore new siRNAs (at least two additional siRNAs per identified gene) and shRNAs will be tested using the experimental setting of Example 1. Those confirmed genes that seem to be important for the replication of influenza viruses will then be knocked down in mice using intranasally administered siRNAs or shRNAs. For the evaluation of this antiviral therapy it is of highest importance to determine the efficiency of transportation of compounds to lung epithelial tissue. The success of a therapy depends on the combination of high efficient kinase inhibitors and adequate transport system. A potentially compatible and cost efficient agent is chitosan which we are applying for the delivery of siRNAs or shRNAs in *in vivo* studies successfully. We will apply the compounds either intranasally or administer them directly into the lung.

Efficient siRNAs or shRNAs should lead to a decreased viral titre within the lung tissue and due to this animals should be protected against an otherwise lethal influenza infection.

For testing the biological effect of the kinase inhibitors, we will divide the experiments in four parts:

1. Analysis of the kinase inhibitor distribution in the respiratory apparatus after intranasal application of compound/chitosan nano particles. Optimisation of the compound/chitosan concentration for best effectiveness. Further tests will only be performed in case of success.

2. In LD50 tests the absolute pathogenicity of the virus isolates Influenza A/Puerto Rico/8/34 and the Avian Influenza isolate (for test 4) will be estimated.

3. Test of antiviral effect of selected siRNAs or shRNAs after intranasal application and infection with Influenza A/Puerto Rico/8/34 by analyzing virus titre in lung tissue or survival rate (in certain cases).

4. Test of antiviral effect of selected siRNAs or shRNAs after intranasal application and infection with highly pathogenic Avian Influenza virus isolate (such as H5N1) by analyzing the virus titre in lung tissue or survival rate (in certain cases).

The used virus isolate is dependant on current development and spreading of the Avian Influenza. We aim at inhibiting the replication of the current prevalent strain in vivo efficiently

Kinase inhibitors against the confirmed genes will also be tested in mice regarding to an impaired virus replication.

The Max-Planck-Institut für Infektionsbiologie, Berlin, Germany, has genome-wide RNAi libraries that, in principle, enable the shutting-off of every single human gene in suitable cell cultures (A549 cells). So in the next level the screen will be expanded to a genome wide scale, because many additional cellular factors involved in the attachment, replication and budding of viruses are still unknown.

**Example 3**

Additional siRNAs (not only siRNAs against kinases or kinase binding proteins) and shRNAs will also be validated in regard to a decline of the replication of influenza A viruses. For the evaluation of these siRNAs and shRNAs the same experimental setting will be used as described in example 1, except that the cell number is quantified indirectly by using a commercial cell viability assay (instead of using an automated microscope) and that these siRNAs and shRNAs will be reverse transfected, i.e. cells will be added to the transfection mix already prepared in 384 well plates.

**Example 4**

Among the human genome hundreds of genes are presumably relevant for the replication of influenza viruses. Therefore the screening procedure of kinases and kinase binding factors (described in Example 1) was expanded to a genome wide scale analysing all known human genes by using about 59886 siRNAs.

The experimental setup was performed in a similar way as described in Example 1, except:

- The screen was extended to genome wide level using 59886 siRNAs

- Cells were seeded in 384 well plates.
- Because of the huge number of transfected cells, not all cell numbers could be analysed by automated microscopy.
- siRNAs were reversely transfected in freshly seeded A549 cells using the transfection reagent HiperFect (Qiagen, Hilden, Germany).
- Knock-down of a particular gene was independently performed by up to four siRNAs ("target sequence 1", "target sequence 2", "target sequence 3", and "target sequence 4" in Table 4) specific for a particular gene.
- Additional controls were included: "AllStars Negative Control siRNA" (Qiagen, Hilden, Germany, Order No. 1027280) as negative control, siRNAs directed against PKMYT (GeneID: 9088, GenBank accessionnumber: NM_182687, target sequence: CTGGGAGGAACTTACCGTCTA) as positive control (cellular factor against influenza replication), siRNAs directed against PLK (GeneID: 5347, GenBank accessionnumber: BC014135, target sequence: CCGGATCAAGAAGAATGAATA) as transfection control (cytotoxic after transfection).
- infection rate of transfected A549 cells in selected wells is measured by automated microscopy to be able to dissect the inhibitory effects to early or late events during the infection process.
- Results were analysed by the statistical R-package "cellHTS" software, developed by Michael Butros, Ligia Bras and Wolfgang Huber, using the B-score normalisation method (based on "Allstars Negative Control siRNA" transfected control wells).
- Read-out is inhibition of virus replication.

The siRNAs and corresponding genes that showed a strong antiviral activity (z-scores < -2.0) are listed in Table 4.

The cell based assay comprised the following steps (see also Fig. 5 which describes the experimental setup of the genome wide siRNA screen:

| | |
|---|---|
| Day 1: | Seeding of A549 cells (lung epithelial cells) + reverse transfection of siRNAs |
| Day 3: | Infection with influenza A/WSN/33 + transfection of 293T cells zq with indicator plasmid |
| Day 4: | Infection of 293T cells with the supernatant of A549 cells + fixation of A549 cells with formaldehyde |
| Day 5: | Luciferase Assay to quantify virus replication in 293T cells |
| Day x: | Determination of infection rate by the automated microscope. |

**Example 5**

**Influenza A virus induces endogenous miR-141 to undermine MxA antiviral activity**

Micro-RNAs (miRNAs), known as global regulators in mammalian cells, also represent attractive tools for infecting viruses to usurp host cell functions. To assess the impact of influenza virus on host miRNA function, we performed an expression analysis of miRNAs in A549 human lung epithelial cells. One endogenous miRNA (miR-141) was strongly induced in infected cells and, if over-expressed, enhanced viral replication, whereas inhibition of miR-141 reduced replication. We identified the antiviral MxA host gene as a key target of miR-141. Accordingly, influenza virus infection caused post-transcriptional silencing of MxA, yet allowing interferon-induced MxA synthesis in adjacent non-infected cells. Thus, we have discovered a crucial miRNA-based regulatory mechanism that protects influenza A viruses from the host cell-mediated antiviral response, constituting novel options for antiviral intervention.

We were interested to know whether the influenza A virus also gains an advantage from the use of cellular miRNAs. To begin, changes in the expression pattern of human miRNAs were monitored using two different microarray platforms. Both array types (Ambion and Exiqon) revealed that a single miRNA, miR-141, was significantly up-regulated in infected A549 human lung epithelial cells in comparison to non-infected cells. Microarray results were confirmed by real-time reverse transcription-PCR (qRT-PCR), showing a dramatic increase (5- to 15-fold) of miR-141 after infection of A549 cells with a range of human and avian influenza A virus strains (Fig. 6A).

To investigate if increased cellular miR-141 has an impact on influenza replication, we transfected cells with either a miR-141-specific chemically modified nucleic acid inhibitor or a miR-141 precursor after infection with influenza virus A/WSN/33. Numbers of infectious particles were quantified using a virus-dependent luciferase assay, indicative of the infection efficiency (17). Treatment of A549 cells with the miR-141 precursor significantly increased the formation of infectious particles, whereas the miR-141 inhibitor led to the opposite effect (Fig. 6B). This observation provided the initial evidence that cellular miR-141 plays a role in modulating the efficiency of influenza virus replication.

To confirm that virus replication is enhanced in the presence of elevated levels of miR-141, we generated cell lines stably over-expressing either human miR-141 or different controls (miR-198 or empty vector). Cells were then infected with influenza A/WSN/33 at a low multiplicity of infection (MOI 0.05). Immunofluorescence microscopy revealed that numbers of infected cells at 24 hours post infection (h p.i.) were significantly higher in miR-141 positive cells in comparison to the various controls (Fig. 6C). Quantification of infectious particles in these cell supernatants (Fig. 6D) further confirmed that influenza A viruses can replicate more efficiently in A549 cells with an increased level of miR-141. Together, these data corroborate the stimulatory effect of miR-141 expression on influenza virus replication.

Cellular target genes of miR-141, mostly related to tumor development (18) have previously been reported, but none of these genes provided a plausible explanation for the impact of this miRNA on virus replication. Based on the assumption that miRNAs might not only affect translational repression but also stability of their mRNA targets (19), we compared the mRNA expression profile in miR-141 over-expressing A549 cells versus control cells using commercial whole human genome microarrays. Among the target mRNAs affected by more than threefold in miR-141 over-expressing cells (data not shown), we identified the antiviral factor MxA (12). To confirm the antiviral function of MxA, we generated two stable A549 cells lines exhibiting shRNA-mediated knockdown of MxA (knockdown on protein level each ~85%. Predictably, silencing of MxA significantly increased primary viral replication (Fig. 10) and the titer of infectious virus released to the supernatant (Fig. 11). These observations led us to assume that MxA constitutes a crucial link between miR-141 function and virus replication.

To investigate the molecular basis of miR-141-enhanced viral replication in relation to MxA, we analyzed MxA mRNA, MxA protein and miR-141 levels in A549 cells during the course of infection using qRT-PCR and immunoblotting, respectively (Fig. 7A-C). Because MxA gene expression requires stimulation by type I interferon (20), neither MxA mRNA nor MxA protein was detected in non-infected A549 cells. In cells infected at an MOI of 1, significant levels of MxA RNA were transcribed as early as 8 h p.i. and peaked at 24 h p.i. (Fig. 7A). Surprisingly, no MxA protein was detected using this MOI even after 24 h of infection (Fig. 7B). Over the same infection period, levels of miR-141 increased steadily, leading to a 20-fold up-regulation, in comparison to non-infected A549 cells (Fig. 7C). These observations indicated that a post-transcriptional mechanism is preventing MxA protein synthesis, but not mRNA synthesis, upon infection of cells with influenza virus, concomitant with an increased occurrence of miR-141. However, an intriguing superimposed phenomenon was apparent - at low virus titers (MOI 0.1), we noted a strong MxA protein signal at 24 h p.i. (Fig. 7B). Titration of MxA protein synthesis at 24 h p.i. in response to increasing MOIs revealed a dose-dependent increase of MxA protein levels up to an MOI of 0.1. Increases in MxA protein levels correlated with MxA transcription at low MOIs; in contrast, at MOIs greater than 0.1 MxA protein levels declined sharply despite sustained mRNA transcription (Fig. 7D and 7E). Thus, post-transcriptional repression of MxA protein was evident at elevated MOIs, but not at low MOIs.

To explain this phenomenon, we visualized virus-infected and MxA-expressing cells at the single-cell level using immunofluorescence microscopy. In cells of non-infected (NI) and fully infected cultures (MOI 1), no MxA protein was detected (Fig. 7F). However, MxA protein was detected in the non-infected cells of partially infected cultures (MOI 0.1) (Fig. 7F). The synthesis of MxA in these non-infected cells can be attributed to a paracrine regulatory loop involving type I interferons, known to stimulate MxA synthesis in non-infected cells (21). Together, these data clearly demonstrate that efficient post-transcriptional down-regulation of MxA protein takes place in all virus infected cells; and that infected cells trigger the up-regulation of MxA in non-infected cells, most likely via the production of IFN-$\beta$.

To test if the observed down-regulation of MxA protein in virus infected cells was dependent on post-transcriptional silencing by miR-141, cells were treated with specific miRNA inhibitors (Fig. 8A). Strikingly, MxA positive cells were only found in virus infected cells, i.e. expressing viral nucleoprotein (NP), after treatment with the miR-141 inhibitor (Fig. 8A, see arrows). In the absence of the inhibitor, or upon transfection with an unrelated miR-198 inhibitor, NP and MxA protein expression was mutually exclusive. Similar results were obtained with the H7N7 avian influenza strain A/FPV/Bratislava/79 (Fig. 8A). Quantification of MxA-positive cells corroborated single-cell observations (Fig. 12). Consistent with the notion that miR-141 induces transcriptional silencing of MxA, we observed that miR-141 levels in cell cultures increased steadily with increasing MOI, peaking at an MOI of ~1, i.e. where all cells are infected at 24 h p.i. (Fig. 8B). The crucial role of miR-141 in MxA regulation was further supported by immunoblot analysis, revealing transfection of miR-141 specific inhibitors facilitated MxA expression, even when all cells were infected (Fig. 8C). These findings clearly show miR-141 is pivotal to the virus-induced down-regulation of the antiviral MxA protein.

To substantiate the function of miR-141 in the absence of infection, we used IFN-$\beta$ to stimulate MxA expression in various A549 cell lines over-expressing distinct miRNAs̃ Cells expressing higher levels of miR-141 showed a slighter degree of MxA induction (~30%) in comparison to control cells transfected with miR-198 or an empty vector, suggesting an IFN-$\beta$ dependent, but virus-independent effect of miR-141 on MxA silencing.

To study the molecular basis of miR-141-mediated MxA silencing, we analyzed the MxA mRNA sequence. Typically, miRNAs function by interacting with the 3' untranslated region (UTR) of its target mRNA to exert their regulatory effect on gene expression (22). A549 cells transiently transfected with a luciferase reporter construct linked to the MxA 3'-UTR, however, did not exhibit reduced luciferase activity in response to miR-141 over-expression (data not shown). In addition, sequence alignments of miR-141 and MxA mRNA revealed complementary regions within the coding sequence (CDS) rather than the 3'-UTR region of MxA (Fig. 14). These findings suggested that the CDS was involved in the miR-141-mediated repression of MxA. To rule out a possible indirect regulatory function of miR-141 in MxA down-regulation, we generated a gene fusion between enhanced green fluorescent protein (eGFP) and MxA placed under control of a constitutive cytomegalovirus (CMV) promoter. The eGFP-MxA fusion enabled parallel monitoring of both transgenic and intrinsic MxA proteins, due to their different molecular size. This approach allowed us to discriminate between an indirect regulatory function of miR-141 affecting transcription from the native MxA promoter (e.g. via putative miR-141-regulated transcription factors) and a direct post-transcriptional gene silencing mechanism affecting the MxA transcript, including its 3'-UTR. The eGFP-MxA fusion was readily expressed after transfection of A549 cells; however, its expression upon infection steadily declined with increasing MOIs of influenza A/WSN/33 (Fig. 9A). Similarly, intrinsic MxA, which showed strongest protein levels at an MOI of ~0.1 (compare with Fig. 6E), declined with increasing MOIs (Fig. 9A). Quantification of the Western blot data indicated a ~10-fold decrease of transfected recombinant and intrinsic MxA proteins upon viral infection at an MOI of 1 (Fig. 9A; see band intensity values below blot). Thus, the influenza virus-triggered reduction of transgenic eGFP-MxA and intrinsic MxA were almost identical despite the constitutive CMV promoter directing transcription of eGFP-MxA.

These observations suggesting a direct post-transcriptional interference of MxA synthesis were further supported by single-cell analyses using confocal microscopy: Simultaneous expression of the constitutively expressed mCherry construct and the eGFP-MxA fusion protein (molar ratio 1:6) indicated a successful transfection strategy (Fig. 9B). However, infection of these cells with the influenza virus at increasing MOIs led to a selective reduction of the eGFP-MxA fusion, but not mCherry, protein (Fig. 9B). Quantification of eGFP-MxA positive cells corroborated these observations (Fig. 15). This experiment clearly demonstrates the specific post-transcriptional silencing of MxA upon influenza virus infection.

To collect additional evidence in support of a direct function of miR-141 in the post-transcriptional silencing of MxA expression, we investigated the effect of miRNAs on MxA expression in the absence of viral infection. Since the human MxA promoter contains two functional IFN-stimulated response elements (ISRE) (23), we used IFN-β to induce expression of MxA. Transfection of the retroviral miR-141, but not miR-198 or the empty vector, caused a parallel decrease in expression of co-transfected eGFP-MxA fusion protein and intrinsic, IFN-β-induced MxA (Fig. 9C). Quantification of MxA protein expression indicated a -3-fold decrease of eGFP-MxA and a ~10-fold decrease of intrinsic MxA in response to miR-141 over-expression, in comparison to cells harboring control plasmids (Fig. 9C; see band intensity values below blot). Again, confocal microscopy resolved and confirmed these cross-results on the single-cell level, showing reduced amounts of both exogenous and endogenous MxA in IFN-β stimulated and miR-141 transfected cells (Fig. 9D). Co-transfection of the mCherry plasmid, identical to the experimental settings in Fig. 9B, enabled the tracking of eGFP-MxA transfected cells. Quantification of eGFP-MxA positive cells revealed significantly reduced levels of MxA after transfecting cells with the miR-141 encoding plasmids (Fig. 16). Most notably, individual cells transfected with the retroviral miR-141 construct and that displayed down-regulation of intrinsic MxA also showed no or weak expression of eGFP-MxA (see arrows in Fig. 9D). The direct function of miR-141 was also substantiated by analysis of ISRE promoter activity in IFN-β stimulated A549 cells over-expressing miR-141, or the unrelated miR-198, demonstrating that upstream signaling of MxA is not affected by miR-141 (Fig. 17). Taken together, these data strongly support the notion of a direct effect of miR-141 on MxA synthesis acting on the post-transcriptional level via an RNA interference-based mechanism.

The molecular mechanism underlying active suppression of MxA by viruses (1) has thus far remained enigmatic. Here, we identified the influenza A virus induced miR-141 as a key molecule involved in the down-regulation of the antiviral MxA protein in human cells. Extensive single-cell analyses performed in this study suggest an active miR-141-dependent inhibition of MxA in infected cells; however, infected cells still induce antiviral MxA synthesis in neighboring yet-uninfected cells via a paracrine loop involving β-interferon. Nevertheless, and contrary to most known miRNA translational control mechanisms (24,25), interference by miR-141 is extremely potent, suggesting miR-141 might constitute an excellent target for treating influenza infections. Furthermore, our observations of miR-141 up-regulation in response to a range of human and avian influenza A isolates, holds promise for broad-spectrum treatment options.

**Reference list**

1. T. D. Carroll et al., J. Immunol. 180, 2385 (2008).
2. K. Subbarao et al., Science 279, 393 (1998).
3. E. C. Claas et al., Lancet 351, 472 (1998).
4. C. Fraser et al., Science (2009).
5. T. Wolff et al., Biol. Chem. 389, 1299 (2008).
6. H. Kato et al., Nature 441, 101 (2006).
7. B. Opitz et al., Cell Microbiol. 9, 930 (2007).
8. J. Lindenmann, Virology 16, 203 (1962).
9. O. Haller, H. Arnheiter, I. Gresser, J. Lindenmann, J. Exp. Med. 49, 601 (1979).
10. T. M. Tumpey et al., J. Virol. 81, 10818 (2007).
11. O. Haller, P. Staeheli, G. Kochs, Biochimie 89, 812 (2007).

12. J. Pavlovic et al., J. Virol. 69, 4506 (1995).

13. B. G. Hale, R. E. Randall, J. Ortin, D. Jackson, J. Gen. Virol. 89, 359 (2008).

14. E. Gottwein, B. R. Cullen, Cell Host. Microbe 3, 375 (2008).

15. B. R. Cullen, Nature 457, 421 (2009).

16. C. L. Jopling, M. Yi, A. M. Lancaster, S. M. Lemon, P. Sarnow, Science 309, 1577 (2005).

17. A. Lutz, J. Dyall, P. D. Olivo, A. Pekosz, J. Virol. Methods 126, 13 2005).

18. P. A. Gregory et al., Nat. Cell Biol. 10, 593 (2008).

19. C. Llave, Z. Xie, K. D. Kasschau, J. C. Carrington, Science 297, 053 (2002).

20. D. Holzinger et al., J. Virol. 81, 7776 (2007).

21. T. Ronni et al., J. Immunol. 158, 2363 (1997).

22. B. P. Lewis, C. B. Burge, D. P. Bartel, Cell 120, 15 (2005).

23. T. Ronni et al., J. Interferon Cytokine Res. 18, 773 (1998).

24. M. Selbach et al., Nature 455, 58 (2008).

25. D. Baek et al., Nature 455, 64 (2008).

**Claims**

1. A method for the preparation of an influenza virus, comprising the steps:

   (a) providing a modified cell, a modified embryonated egg or/and a modified non-human organism capable of replicating an influenza virus, wherein the capability of influenza virus replication is increased compared with influenza virus replication in the absence of the modification,
   (b) contacting the cell, the embryonated egg or/and the organism of (a) with an influenza virus,
   (c) cultivating the cell, the embryonated egg or/and the non-human organism under conditions allowing the replication of the influenza virus, and
   (d) isolating the influenza virus or/and at least one component thereof produced in step (c).

2. The method of claim 1, wherein step (a) includes contacting the cell, the embryonated egg or/and the non-human organism with at least one modulator capable of increasing the influenza virus replication in the cell or/and the organism, compared with influenza virus replication in the absence of the modulator.

3. The method of claim 1 or 2, wherein step (a) includes the production or/and provision of a recombinant cell, a recombinant embryonated egg or/and a recombinant non-human organism, wherein the expression or/and activity of at least one gene or/and gene product is modified so that the capability of the cell, the embryonated egg or/and the non-human organism of replicating an influenza virus is increased compared with influenza virus replication in the absence of the modification.

4. The method of any of the preceding claims, wherein the influenza virus is an influenza A virus or/and an influenza B virus, preferably a strain selected from H1N1, H3N2, H7N7, H5N1.

5. The method of any of the preceding claims, wherein modification of the cell, the embryonated egg or/and non-human organism includes inhibition of the expression or/and gene product activity of the MxA gene.

6. The method of claim 5, wherein the expression or/and gene product activity of the MxA gene is inhibited by at least one modulator selected from miR-141, miR-141*, miR-200c, miR-200c*, precursors thereof, derivatives thereof, antisense nucleic acids, siRNAs, shRNAs and influenza virus sequences.

7. The method of claim 5 or 6, wherein at least one of miR-141, miR-141*, miR-200c, miR-200c* and precursors thereof is over-expressed in the cell, in the embryonated egg or/and in the non-human organism.

8. The method of any of the claims 1 to 4, wherein modification of the cell, of the embryonated egg or/and the non-human organism includes the inhibition of the expression or/and gene product activity of a gene, wherein the gene comprises

   (a) a nucleotide sequence selected from the sequences of Table 1A
   (b) a fragment of the sequence of (a) having a length of at least 70%, at least 80%, at least 90%, at least 95%, at least 98%, at least 99% of the sequence of (a),

(c) a sequence which is at least 70 %, preferably at least 80%, more preferably at least 90% identical to the sequence of (a) or/and (b), or/and

(d) a sequence complementary to a sequence of (a), (b) or/and (c).

**9.** The method of any of the claims 1 to 4, wherein modification of the cell, of the embryonated egg or/and of the non-human organism includes the activation of the expression or/and gene product activity of a gene, wherein the gene comprises

(i) a nucleotide sequence selected from the sequences of Table 1 B and Table 4,

(ii) a fragment of the sequence of (i) having a length of at least 70%, at least 80%, at least 90%, at least 95%, at least 98%, at least 99% of the sequence of (i),

(iii) a sequence which is at least 70 %, preferably at least 80%, more preferably at least 90% identical to the sequence of (i) or/and (ii), or/and

(iv) a sequence complementary to a sequence of (i), (ii) or/and (iii).

**10.** The method according to claim 2, wherein the at least one modulator is selected from the group consisting of nucleic acids, nucleic acid analogues, peptides, polypeptides, antibodies, aptamers, spiegelmers, small molecules and decoy nucleic acids.

**11.** The method of claim 10, wherein the nucleic acid is selected from

(a) RNA, analogues and derivatives thereof,

(b) DNA, analogues and derivatives thereof , and

(c) combinations of (a) and (b).

**12.** The method according to any of the claims 10 to 11, wherein the nucleic acid is

(i) an RNA molecule capable of RNA interference, such as siRNA or/and shRNA

(ii) a miRNA,

(iii) a precursor of the RNA molecule (i) or/and (ii),

(iv) a fragment of the RNA molecule (i), (ii) or/and (iii),

(v) a derivative of the RNA molecule of (i), (ii) (iii) or/and (iv), or/and

(vi) a DNA molecule encoding the RNA molecule of (i), (ii) (iii) or/and (iv).

**13.** The method according to any of the claims 10 to 12, wherein the RNA molecule is a double-stranded RNA molecule, preferably a double-stranded siRNA molecule with or without a single-stranded overhang alone at one end or at both ends.

**14.** The method according to any of the claims 10 to 13, wherein the RNA molecule comprises at least one nucleotide analogue or/and deoxyribonucleotide.

**15.** The method according to any of the claims 10 to 14, wherein, the nucleic acid is selected from (a) aptamers, (b) DNA molecules encoding an aptamer, and (c) spiegelmers.

**16.** The method according to any of the claims 10 to 14, wherein the nucleic acid is an antisense nucleic acid orand a DNA encoding the antisense nucleic acid.

**17.** The method according to any of the claims 10 to 13, wherein the nucleic acid has a length of at least 15, preferably at least 17, more preferably at least 19, most preferably at least 21 nucleotides.

**18.** The method according to any of the claims 10 to 14, wherein the nucleic acid has a length of at the maximum 29, preferably at the maximum 27, more preferably at the maximum 25, especially more preferably at the maximum 23, most preferably at the maximum 22 nucleotides.

**19.** The method according to claim 10, wherein the antibody is directed against a polypeptide comprising

(a) an amino acid sequence encoded by a nucleic acid or/and gene selected from Table 1A , Table 1B, and Table 4,

(b) a fragment of the sequence of (a) having a length of at least 70%, at least 80%, at least 90%, at least 95%, at least 98%, at least 99% of the sequence of (a), or/and
(c) an amino acid sequence which is at least 70%, preferably at least 80%, more preferably at least 90% identical to the sequence of (a).

20. The method according to claim 10, wherein the small molecule is directed against a polypeptide comprising

(a) an amino acid sequence encoded by a nucleic acid or/and gene selected from Table 1A , Table 1 B, and Table 4,
(b) a fragment of the sequence of (a) having a length of at least 70%, at least 80%, at least 90%, at least 95%, at least 98%, at least 99% of the sequence of (a),or/and
(c) an amino acid sequence which is at least 70%, preferably at least 80%, more preferably at least 90% identical to the sequence of (a).

21. A recombinant cell produced in the method of claim 3.

22. A recombinant embryonated egg produced in the method of claim 3.

23. A recombinant non-human organism produced in the method of claim 3.

24. A pharmaceutical composition comprising an activator of the expression or/and gene product activity of the MxA gene, optionally together with a pharmaceutically acceptable carrier, adjuvant, diluent or/and additive.

25. The pharmaceutical composition of claim 21, wherein the activator of the expression or/and gene product activity is at least one inhibitor capable of inhibiting the activity of an miRNA selected from miR-141, miR-141*, miR-200c, miR-200c*, and precursors thereof.

## Fig. 1

## Fig. 2

**Figure 3**

**Figure 4**

# Fig. 5

**+ Influenza**

**Transfection of 293T cells with luciferase indicator plasmid**

**Reverse transfection with siRNAs**

**Transfer of supernatant**

A-549

2

**Determination of the infection rate by the automatic microscope**

**Determination of luciferase activity**

Fig. 6

Fig. 7

EP 2 295 543 A1

Fig. 8

Fig. 8

EP 2 295 543 A1

# Fig. 9

A

| MOI | 1 | 0.5 | 0.05 | 0.1 | NI |
|---|---|---|---|---|---|
| eGFP-MxA | + | + | + | - | + |

eGFP-MxA

MxA

β-actin

| eGFP-MxA | 0.1 | 0.2 | 0.9 | - | 1.0 |
| MxA | 0.1 | 0.2 | 1.0 | 1.0 | - |

B

NI            MOI 0.05            MOI 1

A/WSN/33
mCherry
eGFP-MxA

# Fig. 9

C

| miRNA | - | - | - | Empty | 198 | 141 |
|---|---|---|---|---|---|---|
| eGFP-MxA | + | - | + | + | + | + |
| IFN-β 500U/ml | - | - | - | + | + | + |
| MOI (A/WSN/33) | NI | 0.1 | 1 | NI | NI | NI |

eGFP-MxA
MxA
β-actin

| eGFP-MxA | 1.0 | - | 0.5 | 1.0 | 1.0 | 0.4 |
|---|---|---|---|---|---|---|
| MxA | - | 1.0 | 0.2 | 1.0 | 1.0 | 0.1 |

# Fig. 9

Fig. 10

Percentage of infected cells

Fig. 11

Luciferase activity

Fig. 12

## Fig. 13

|  | NT | | Empty vector | | miR-198 | | miR-141 |
|---|---|---|---|---|---|---|---|
| IFN-β | − | + | − | + | − | + | − | + |
| MxA | | | | | | | | |
| β-actin | | | | | | | | |
| Band intensity | − | 1.0 | − | 1.0 | − | 1.0 | − | 0.3 |

**Fig. 14**

mfe: -21.8 kcal/mol

Fig. 15

Fig. 16

## Fig. 17

## Fig. 18


## miR-141/141*

`precursor sequence`

```
>hsa-mir-141 MI0000457
5'-CGGCCGGCCCUGGGUCCAUCUUCCAGUACAGUGUUGGAUGGUCUAAUUGUGAAGCUCCUAACACUGUCUGGU
AAAGAUGGCUCCCGGGUGGGUUC-3'
```


`precursor structure`

```
c     g     u   -u         --     u           -   ugg  ua
 ggcc gccc ggg  ccaucuu  ccag acaguguu gga      uc  a
 |||| |||| |||  |||||||  |||| |||||||| |||       ||  u
 uugg uggg ccc  gguagaa  gguc ugucacaa ccu      ag  u
c     g    -    uc       au     -         u    cga  ug
```


`miR-141`

```
>hsa-miR-141 MIMAT0000432
5'-(59)-UAACACUGUCUGGUAAAGAUGG-(80)-3'
```

`miR-141*`

```
>hsa-miR-141* MIMAT0004598
5'-(17)-CAUCUUCCAGUACAGUGUUGGA-(38)-3'
```


## miR-200c/200c*

`precursor sequence`

```
>hsa-mir-200c MI0000650
5'-CCCUCGUCUUACCCAGCAGUGUUUGGGGUGCGGUUGGGAGUCUCUAAUACUGCCGGGUAAUGAUGGAGG-3'
```


`precursor structure`

```
  cu     -      a         u   u  ggu
cc  cguc uuaccc gcaguguu ggg gc   u
||  |||| |||||| |||||||| ||| ||
gg  guag aauggg cgucauaa cuc ug    g
 ag    u       c         u   -  agg
```


`miR-200c`

```
>hsa-miR-200c MIMAT0000617
5'-(44)-UAAUACUGCCGGGUAAUGAUGGA-(66)-3'
```


`miR-200c*`

```
>hsa-miR-200c* MIMAT0004657
5'-(5)-CGUCUUACCCAGCAGUGUUUGG-(26)-3'
```

Table 1a

| Gene name | GeneID | Accession Number | % Inhibition | Standard Deviation |
|---|---|---|---|---|
| Pool 19 | | | -47% | 109% |
| CKM | 1158 | NM_001824 | -46% | 14% |
| MAP3K3 | 4215 | NM_002401 | -45% | 3% |
| SNARK | 81788 | NM_030952 | -39% | 16% |
| DDR1 | 780 | NM_001954 | -38% | 21% |
| IHPK2 | 51447 | NM_016291 | -34% | 23% |
| MARK3 | 4140 | NM_002376 | -33% | 8% |
| Pool 31 | | | -33% | 30% |
| Pool 69 | | | -31% | 30% |
| AK7 | 122481 | NM_152327 | -30% | 17% |
| Pool 46 | | | -28% | 17% |
| ARAF1 | 369 | NM_001654 | -28% | 39% |
| Pool 75 | | | -27% | 22% |
| AKAP1 | 8165 | NM_003488 | -27% | 10% |
| Pool 56 | | | -27% | 8% |
| IRAK2 | 3656 | NM_001570 | -26% | 60% |
| ERK8 | 225689 | NM_139021 | -25% | 34% |
| PANK3 | 79646 | NM_024594 | -25% | 12% |
| AK5 | 26289 | NM_174858 | -25% | 41% |
| PDK2 | 5164 | NM_002611 | -25% | 6% |
| DGUOK | 1716 | NM_001929 | -25% | 12% |
| HGS | 9146 | NM_004712 | -25% | 10% |
| Pool 114 | | | -24% | 38% |
| Pool 33 | | | -24% | 9% |
| Pool 39 | | | -23% | 8% |
| Pool 43 | | | -23% | 31% |
| Pool 55 | | | -23% | 12% |
| MAPK8IP2 | 23542 | NM_012324 | -23% | 10% |
| SKP2 | 6502 | NM_005983 | -22% | 15% |
| Pool 45 | | | -22% | 7% |
| STK23 | 26576 | NM_014370 | -22% | 41% |
| SRPK2 | 6733 | NM_182692 | -21% | 20% |
| PKIG | 11142 | NM_181805 | -21% | 20% |
| PRKY | 5616 | NM_002760 | -21% | 16% |
| TESK1 | 7016 | NM_006285 | -21% | 19% |
| NEK2 | 4751 | NM_002497 | -20% | 11% |
| Pool 64 | | | -20% | 12% |
| PSKH1 | 5681 | NM_006742 | -20% | 5% |
| PRKAR2B | 5577 | NM_002736 | -20% | 52% |
| TRAD | 11139 | NM_007064 | -20% | 11% |
| DYRK1A | 1859 | NM_001396 | -20% | 24% |
| LIMK1 | 3984 | NM_002314 | -19% | 41% |
| WEE1 | 7465 | NM_003390 | -19% | 10% |
| Pool 37 | | | -18% | 6% |
| ADRBK1 | 156 | NM_001619 | -18% | 33% |
| TPK1 | 27010 | NM_022445 | -18% | 46% |
| PHKB | 5257 | NM_000293 | -17% | 6% |

Table 1a (continued)

| Gene name | GeneID | Accession Number | % Inhibition | Standard Deviation |
|---|---|---|---|---|
| PRKWNK4 | 65266 | NM_032387 | -17% | 11% |
| Pool 82 | | | -17% | 16% |
| Pool 32 | | | -17% | 17% |
| PIK4CA | 5297 | NM_002650 | -17% | 17% |
| MK-STYX | 51657 | NM_016086 | -17% | 7% |
| Pool 103 | | | -17% | 26% |
| IRAK1BP1 | 134728 | BX537762 | -17% | 13% |
| PRKCD | 5580 | NM_006254 | -16% | 17% |
| KIT | 3815 | NM_000222 | -16% | 9% |
| IRAK1 | 3654 | NM_001569 | -16% | 14% |
| FGFR2 | 2263 | NM_000141 | -16% | 8% |
| Pool 106 | | | -16% | 33% |
| Pool 111 | | | -16% | 44% |
| DGKH | 160851 | NM_178009 | -16% | 17% |
| Pool 113 | | | -16% | 35% |
| DAPK1 | 1612 | NM_004938 | -15% | 11% |
| RAGE | 5891 | NM_014226 | -15% | 9% |
| KSR | 8844 | XM_290793 | -15% | 12% |
| TAO1 | 9344 | NM_016151 | -15% | 3% |
| TAO1 | 9344 | NM_004783 | -15% | 3% |
| Pool 30 | | | -15% | 31% |
| PAK2 | 5062 | NM_002577 | -15% | 16% |
| AURKC | 6795 | NM_003160 | -15% | 42% |
| MARK2 | 2011 | NM_004954 | -15% | 13% |
| MVK | 4598 | NM_000431 | -15% | 30% |
| MAPKBP1 | 23005 | XM_031706 | -15% | 30% |
| PRKCE | 5581 | NM_005400 | -14% | 5% |
| EKI1 | 55500 | NM_018638 | -14% | 7% |
| EPHA7 | 2045 | NM_004440 | -14% | 10% |
| CDK4 | 1019 | NM_000075 | -14% | 16% |
| AKAP12 | 9590 | NM_005100 | -14% | 6% |
| Pool 29 | | | -14% | 16% |
| AURKB | 9212 | NM_004217 | -14% | 9% |
| PIK3C3 | 5289 | NM_002647 | -14% | 18% |
| MAP2K6 | 5608 | NM_002758 | -14% | 19% |
| ADCK4 | 79934 | NM_024876 | -14% | 14% |
| MAPK8IP1 | 9479 | NM_005456 | -13% | 5% |
| Pool 65 | | | -13% | 12% |
| PIK3CB | 5291 | NM_006219 | -13% | 10% |
| PIK3R3 | 8503 | NM_003629 | -13% | 12% |
| PDK1 | 5163 | NM_002610 | -13% | 11% |
| HRI | 27102 | NM_014413 | -13% | 23% |
| CSNK1G1 | 53944 | NM_022048 | -13% | 17% |
| BMPR1A | 657 | NM_004329 | -13% | 12% |
| DAPK3 | 1613 | NM_001348 | -13% | 20% |
| SIK2 | 23235 | AB096248 | -13% | 10% |
| PDPK1 | 5170 | NM_002613 | -12% | 27% |
| Pool 104 | | | -12% | 35% |
| CAMKK1 | 84254 | NM_032294 | -12% | 10% |

Table 1a (continued)

| Gene name | GeneID | Accession Number | % Inhibition | Standard Deviation |
|---|---|---|---|---|
| TK1 | 7083 | NM_003258 | -12% | 20% |
| CSNK2B | 1460 | NM_001320 | -12% | 10% |
| MGC4796 | 83931 | NM_032017 | -12% | 16% |
| DGKB | 1607 | NM_145695 | -12% | 16% |
| MET | 4233 | NM_000245 | -12% | 5% |
| ERBB4 | 2066 | NM_005235 | -12% | 21% |
| PRKCDBP | 112464 | NM_145040 | -12% | 16% |
| BTK | 695 | NM_000061 | -12% | 11% |
| RPS6KB1 | 6198 | NM_003161 | -11% | 13% |
| AKAP11 | 11215 | NM_016248 | -11% | 9% |
| PRKAG1 | 5571 | NM_002733 | -11% | 15% |
| PIK3C2A | 5286 | NM_002645 | -11% | 22% |
| CDK11 | 23097 | NM_015076 | -11% | 16% |
| Pool 54 | | | -11% | 14% |
| MKNK2 | 2872 | NM_017572 | -11% | 29% |
| PTK9 | 5756 | NM_002822 | -11% | 11% |
| FLT3LG | 2323 | BF688722 | -11% | 13% |
| CDK8 | 1024 | NM_001260 | -11% | 20% |
| ITPKA | 3706 | NM_002220 | -11% | 24% |
| CDK3 | 1018 | NM_001258 | -11% | 14% |
| CDK7 | 1022 | NM_001799 | -11% | 9% |
| PRKACA | 5566 | NM_002730 | -11% | 41% |
| Pool 3 | | | -10% | 11% |
| LAK | 80216 | NM_025144 | -10% | 16% |
| ITPKB | 3707 | NM_002221 | -10% | 35% |
| PI4KII | 55361 | NM_018425 | -10% | 12% |
| PRKCBP1 | 23613 | NM_183048 | -10% | 16% |
| SH3KBP1 | 30011 | NM_031892 | -10% | 5% |
| BRAF | 673 | NM_004333 | -10% | 13% |
| Pool 34 | | | -10% | 33% |
| KHK | 3795 | NM_000221 | -10% | 9% |
| DCAMKL1 | 9201 | NM_004734 | -10% | 21% |
| CLK3 | 1198 | NM_001292 | -10% | 13% |
| BCR | 613 | NM_004327 | -10% | 25% |
| STK16 | 8576 | NM_003691 | -10% | 17% |
| MAPKAPK3 | 7867 | NM_004635 | -10% | 18% |
| IKBKE | 9641 | XM_375834 | -9% | 12% |
| ATR | 545 | NM_001184 | -9% | 15% |
| STK17A | 9263 | NM_004760 | -9% | 16% |
| MOS | 4342 | NM_005372 | -9% | 16% |
| MAPK3 | 5595 | NM_002746 | -9% | 9% |
| SMG1 | 23049 | NM_014006 | -9% | 26% |
| FER | 2241 | NM_005246 | -9% | 17% |
| PFKP | 5214 | NM_002627 | -9% | 12% |
| CSK | 1445 | NM_004383 | -9% | 13% |
| BUB1 | 699 | NM_004336 | -9% | 24% |
| AK3L1 | 50808 | NM_016282 | -9% | 13% |
| GRK7 | 131890 | NM_139209 | -9% | 3% |
| MAP3K6 | 9064 | NM_004672 | -9% | 6% |

Table 1a (continued)

| Gene name | GeneID | Accession Number | % Inhibition | Standard Deviation |
|---|---|---|---|---|
| ACVR2B | 93 | NM_001106 | -9% | 6% |
| IRAK4 | 51135 | NM_016123 | -8% | 14% |
| EPHA5 | 2044 | NM_004439 | -8% | 15% |
| PRKWNK3 | 65267 | NM_020922 | -8% | 4% |
| RIOK1 | 83732 | NM_031480 | -8% | 7% |
| MAK | 4117 | NM_005906 | -8% | 12% |
| CSNK2A2 | 1459 | NM_001896 | -8% | 35% |
| Pool 60 | | | -8% | 17% |
| AXL | 558 | NM_001699 | -8% | 21% |
| IHPK1 | 9807 | NM_153273 | -8% | 15% |
| Pool 98 | | | -8% | 29% |
| JAK2 | 3717 | NM_004972 | -8% | 3% |
| Pool 61 | | | -8% | 17% |
| DDR2 | 4921 | NM_006182 | -8% | 26% |
| ADCK5 | 203054 | NM_174922 | -8% | 27% |
| EPHA1 | 2041 | NM_005232 | -7% | 14% |
| Pool 77 | | | -7% | 25% |
| GKAP42 | 80318 | NM_025211 | -7% | 12% |
| PIK3R4 | 30849 | NM_014602 | -7% | 22% |
| ROCK1 | 6093 | NM_005406 | -7% | 3% |
| Pool 22 | | | -7% | 13% |
| LCK | 3932 | NM_005356 | -7% | 8% |
| RPS6KA2 | 6196 | NM_021135 | -7% | 13% |
| KIDINS220 | 57498 | AB033076 | -7% | 15% |
| Pool 24 | | | -7% | 10% |
| SNF1LK | 150094 | NM_173354 | -7% | 6% |
| FGFR1 | 2260 | NM_015850 | -7% | 10% |
| Pool 53 | | | -7% | 19% |
| PRKCI | 5584 | NM_002740 | -7% | 9% |
| HSPB8 | 26353 | NM_014365 | -6% | 10% |
| JAK1 | 3716 | NM_002227 | -6% | 47% |
| Pool 68 | | | -6% | 20% |
| CLK1 | 1195 | NM_004071 | -6% | 13% |
| SAST | 22983 | AB023190 | -6% | 8% |
| PDGFRB | 5159 | NM_002609 | -6% | 11% |
| CLK2 | 1196 | NM_001291 | -6% | 9% |
| VRK2 | 7444 | NM_006296 | -6% | 12% |
| LOC91807 | 91807 | NM_182493 | -6% | 17% |
| MELK | 9833 | NM_014791 | -5% | 29% |
| Pool 18 | | | -5% | 11% |
| CCRK | 23552 | NM_178432 | -5% | 31% |
| PRKAG3 | 53632 | NM_017431 | -5% | 21% |
| SPEC2 | 56990 | NM_020240 | -5% | 6% |
| Pool 44 | | | -5% | 27% |
| ACVR1B | 91 | NM_004302 | -5% | 10% |
| CHUK | 1147 | NM_001278 | -5% | 22% |
| FYN | 2534 | NM_002037 | -5% | 17% |
| DTYMK | 1841 | NM_012145 | -5% | 8% |
| SRMS | 6725 | NM_080823 | -5% | 19% |

Table 1a (continued)

| Gene name | GeneID | Accession Number | % Inhibition | Standard Deviation |
|---|---|---|---|---|
| MAP3K9 | 4293 | XM_027237 | -5% | 6% |
| ALK | 238 | NM_004304 | -5% | 13% |
| PAK6 | 56924 | NM_020168 | -5% | 10% |
| Pool 8 | | | -5% | 15% |
| ILKAP | 80895 | NM_176799 | -5% | 16% |
| SPHK2 | 56848 | NM_020126 | -5% | 4% |
| CDK2 | 1017 | NM_001798 | -5% | 21% |
| NEK3 | 4752 | NM_152720 | -5% | 7% |
| TESK2 | 10420 | NM_007170 | -5% | 9% |
| AKT2 | 208 | NM_001626 | -5% | 74% |
| PRKG1 | 5592 | NM_006258 | -5% | 8% |
| KIP2 | 10518 | NM_006383 | -5% | 8% |
| Pool 88 | | | -4% | 32% |
| MAPK14 | 1432 | NM_001315 | -4% | 15% |
| CARKL | 23729 | NM_013276 | -4% | 23% |
| NME4 | 4833 | NM_005009 | -4% | 5% |
| VRK1 | 7443 | NM_003384 | -4% | 17% |
| TAO1 | 9344 | NM_016151 | -4% | 11% |
| TAO1 | 9344 | NM_004783 | -4% | 11% |
| NLK | 51701 | NM_016231 | -4% | 10% |
| NME1 | 4830 | NM_000269 | -4% | 19% |
| Pool 12 | | | -4% | 18% |
| CALM3 | 808 | NM_005184 | -4% | 10% |
| ADRBK2 | 157 | NM_005160 | -4% | 8% |
| Pool 21 | | | -4% | 10% |
| PRKWNK2 | 65268 | NM_006648 | -4% | 26% |
| Pool 97 | | | -4% | 20% |
| Pool 115 | | | -4% | 41% |
| RAF1 | 5894 | NM_002880 | -4% | 7% |
| PACSIN3 | 29763 | NM_016223 | -4% | 6% |
| PGK2 | 5232 | NM_138733 | -4% | 9% |
| MAPK10 | 5602 | NM_002753 | -3% | 7% |
| Pool 36 | | | -3% | 32% |
| PRKWNK1 | 65125 | NM_018979 | -3% | 13% |
| ITPKC | 80271 | NM_025194 | -3% | 15% |
| FLJ13052 | 65220 | NM_023018 | -3% | 8% |
| RPS6KA3 | 6197 | NM_004586 | -3% | 21% |
| MAST2 | 23139 | NM_015112 | -3% | 10% |
| STK25 | 10494 | NM_006374 | -3% | 18% |
| LMTK2 | 22853 | NM_014916 | -3% | 11% |
| MAPK1 | 5594 | NM_002745 | -3% | 13% |
| MAGI&#45;3 | 260425 | NM_020965 | -3% | 8% |
| PFKFB1 | 5207 | NM_002625 | -3% | 20% |
| Pool 71 | | | -3% | 35% |
| GRK6 | 2870 | NM_002082 | -3% | 25% |
| STK6 | 6790 | NM_003600 | -3% | 13% |
| Pool 101 | | | -3% | 46% |
| STK11IP | 114790 | NM_052902 | -3% | 10% |
| DOK1 | 1796 | NM_001381 | -2% | 23% |

## Table 1a (continued)

| Gene name | GeneID | Accession Number | % Inhibition | Standard Deviation |
|---|---|---|---|---|
| PTK9L | 11344 | NM_007284 | -2% | 9% |
| CNK1 | 10256 | NM_006314 | -2% | 9% |
| FLT3 | 2322 | NM_004119 | -2% | 13% |
| ZAP70 | 7535 | NM_001079 | -2% | 13% |
| MAPKAP1 | 79109 | NM_024117 | -2% | 20% |
| AK1 | 203 | NM_000476 | -2% | 9% |
| FGFR3 | 2261 | NM_000142 | -2% | 6% |
| TXK | 7294 | NM_003328 | -2% | 10% |
| CDKN1C | 1028 | NM_000076 | -2% | 14% |
| Pool 7 | | | -2% | 17% |
| MUSK | 4593 | NM_005592 | -2% | 8% |
| ACVR1 | 90 | NM_001105 | -2% | 22% |
| AKAP5 | 9495 | NM_004857 | -2% | 11% |
| MKNK1 | 8569 | NM_003684 | -2% | 11% |
| UGP2 | 7360 | NM_006759 | -2% | 10% |
| RYK | 6259 | NM_002958 | -2% | 5% |
| Pool 107 | | | -2% | 25% |
| ITPK1 | 3705 | NM_014216 | -1% | 13% |
| RPS6KL1 | 83694 | NM_031464 | -1% | 8% |
| PIM1 | 5292 | NM_002648 | -1% | 6% |
| CAMK2D | 817 | NM_001221 | -1% | 13% |
| CAMK2G | 818 | NM_172173 | -1% | 8% |
| TYK2 | 7297 | NM_003331 | -1% | 13% |
| LIMK2 | 3985 | NM_005569 | -1% | 10% |
| HK2 | 3099 | NM_000189 | -1% | 14% |
| Pool 91 | | | -1% | 33% |
| DYRK1B | 9149 | NM_004714 | -1% | 17% |
| TTBK1 | 84630 | XM_166453 | -1% | 59% |
| Pool 58 | | | -1% | 15% |
| Pool 41 | | | -1% | 8% |
| MAP3K5 | 4217 | NM_005923 | -1% | 17% |
| CDKL2 | 8999 | NM_003948 | -1% | 10% |
| LOC149420 | 149420 | NM_152835 | -1% | 20% |
| EGFR | 1956 | NM_005228 | -1% | 15% |
| FRK | 2444 | NM_002031 | -1% | 22% |
| Pool 78 | | | -1% | 21% |
| CDKN2C | 1031 | NM_001262 | -1% | 14% |
| IRAK3 | 11213 | NM_007199 | -1% | 4% |

Table 1b

| Gene name | GeneID | Accession Number | % Inhibition | Standard Deviation |
|---|---|---|---|---|
| AKAP8 | 10270 | NM_005858 | 1% | 19% |
| Pool 2 | | | 1% | 13% |
| HSA250839 | 55351 | NM_018401 | 1% | 14% |
| JAK3 | 3718 | NM_000215 | 1% | 18% |
| LYK5 | 92335 | NM_153335 | 1% | 8% |
| PIK3C2G | 5288 | NM_004570 | 1% | 12% |
| EPHB4 | 2050 | NM_004444 | 1% | 14% |
| KIAA1804 | 84451 | NM_032435 | 1% | 11% |
| STK39 | 27347 | NM_013233 | 1% | 22% |
| ABL1 | 25 | NM_005157 | 1% | 10% |
| Pool 48 | | | 1% | 16% |
| PRKR | 5610 | NM_002759 | 1% | 27% |
| IKBKAP | 8518 | NM_003640 | 1% | 17% |
| PRKCB1 | 5579 | NM_002738 | 1% | 6% |
| PLK4 | 10733 | NM_014264 | 1% | 13% |
| EPHA8 | 2046 | NM_020526 | 1% | 24% |
| PFKM | 5213 | NM_000289 | 2% | 11% |
| EPHB6 | 2051 | NM_004445 | 2% | 11% |
| Pool 110 | | | 2% | 28% |
| Pool 62 | | | 2% | 14% |
| DYRK4 | 8798 | NM_003845 | 2% | 14% |
| MAPK7 | 5598 | NM_002749 | 2% | 10% |
| Pool 81 | | | 2% | 23% |
| PRKCL2 | 5586 | NM_006256 | 2% | 10% |
| PACSIN1 | 29993 | NM_020804 | 2% | 14% |
| YES1 | 7525 | NM_005433 | 2% | 9% |
| MAP3K7IP1 | 10454 | NM_006116 | 2% | 13% |
| PSKH2 | 85481 | NM_033126 | 2% | 22% |
| Pool 108 | | | 3% | 43% |
| Pool 74 | | | 3% | 39% |
| MYLK2 | 85366 | NM_033118 | 3% | 22% |
| Pool 73 | | | 3% | 14% |
| RIPK3 | 11035 | NM_006871 | 3% | 22% |
| PRKCM | 5587 | NM_002742 | 3% | 2% |
| PHKG2 | 5261 | NM_000294 | 3% | 9% |
| SGK2 | 10110 | NM_016276 | 3% | 38% |
| STK17B | 9262 | NM_004226 | 3% | 13% |
| Pool 6 | | | 3% | 23% |
| PRPF4B | 8899 | NM_176800 | 3% | 26% |
| DMPK | 1760 | NM_004409 | 3% | 6% |
| PIK3AP1 | 118788 | NM_152309 | 3% | 6% |
| BMP2K | 55589 | NM_017593 | 3% | 15% |
| Pool 52 | | | 3% | 29% |
| CKMT2 | 1160 | NM_001825 | 4% | 16% |
| Pool 5 | | | 4% | 21% |
| Pool 51 | | | 4% | 29% |
| TK2 | 7084 | NM_004614 | 4% | 8% |
| CAMK1G | 57172 | NM_020439 | 4% | 6% |

Table 1b (continued)

| Gene name | GeneID | Accession Number | % Inhibition | Standard Deviation |
|---|---|---|---|---|
| CALM2 | 805 | NM_001743 | 4% | 14% |
| Pool 99 | | | 4% | 26% |
| Pool 11 | | | 4% | 19% |
| ITK | 3702 | NM_005546 | 4% | 10% |
| PKM2 | 5315 | NM_182471 | 4% | 17% |
| Pool 17 | | | 4% | 17% |
| MST4 | 51765 | NM_016542 | 4% | 16% |
| ROCK2 | 9475 | NM_004850 | 4% | 19% |
| OSR1 | 9943 | NM_005109 | 4% | 13% |
| PRKAR1A | 5573 | NM_002734 | 4% | 18% |
| MARK4 | 57787 | NM_031417 | 5% | 25% |
| DGKZ | 8525 | NM_003646 | 5% | 14% |
| MAPK6 | 5597 | NM_002748 | 5% | 20% |
| PRKAG2 | 51422 | NM_016203 | 5% | 13% |
| CaMKIIalpha | 55450 | NM_018584 | 5% | 18% |
| EPHA3 | 2042 | NM_005233 | 5% | 8% |
| Pool 70 | | | 5% | 38% |
| PGK1 | 5230 | NM_000291 | 5% | 9% |
| CAMK1 | 8536 | NM_003656 | 5% | 5% |
| MAP2K2 | 5605 | NM_030662 | 5% | 3% |
| PTK2B | 2185 | NM_173176 | 5% | 14% |
| EPHB3 | 2049 | NM_004443 | 5% | 8% |
| TBK1 | 29110 | NM_013254 | 5% | 17% |
| GUK1 | 2987 | NM_000858 | 6% | 14% |
| LOC161635 | 161635 | BC028192 | 6% | 18% |
| ACVRL1 | 94 | NM_000020 | 6% | 24% |
| MERTK | 10461 | NM_006343 | 6% | 15% |
| DGKQ | 1609 | NM_001347 | 6% | 9% |
| SSTK | 83983 | NM_032037 | 6% | 6% |
| HIPK3 | 10114 | NM_005734 | 6% | 19% |
| ROR1 | 4919 | NM_005012 | 6% | 10% |
| ARK5 | 9891 | XM_374996 | 6% | 18% |
| URKL1 | 54963 | NM_017859 | 6% | 27% |
| CDK9 | 1025 | NM_001261 | 6% | 10% |
| CDKN2B | 1030 | NM_004936 | 6% | 10% |
| KIS | 127933 | NM_175866 | 6% | 4% |
| TGFBR2 | 7048 | NM_003242 | 6% | 6% |
| BLK | 640 | NM_001715 | 6% | 28% |
| TTK | 7272 | NM_003318 | 6% | 12% |
| CDKL1 | 8814 | NM_004196 | 6% | 11% |
| CLK4 | 57396 | NM_020666 | 7% | 15% |
| PRKDC | 5591 | NM_006904 | 7% | 18% |
| STK38L | 23012 | NM_015000 | 7% | 15% |
| AKAP4 | 8852 | NM_003886 | 7% | 3% |
| STK11 | 6794 | NM_000455 | 7% | 5% |
| MAGI1 | 154043 | NM_173515 | 7% | 13% |
| SNRK | 54861 | NM_017719 | 7% | 9% |
| CDC42BPA | 8476 | NM_003607 | 7% | 17% |

Table 1b ( continued)

| Gene name | GeneID | Accession Number | % Inhibition | Standard Deviation |
|---|---|---|---|---|
| CERK | 64781 | NM_182661 | 7% | 13% |
| RBKS | 64080 | NM_022128 | 7% | 14% |
| ATM | 472 | NM_000051 | 7% | 19% |
| MAPK8IP3 | 23162 | NM_015133 | 7% | 7% |
| HAK | 115701 | NM_052947 | 7% | 16% |
| RIPK2 | 8767 | NM_003821 | 7% | 8% |
| GRK5 | 2869 | NM_005308 | 7% | 13% |
| AKAP6 | 9472 | NM_004274 | 7% | 13% |
| Pool 13 | | | 7% | 25% |
| MATK | 4145 | NM_002378 | 8% | 37% |
| Pool 1 | | | 8% | 35% |
| GIT1 | 28964 | NM_014030 | 8% | 8% |
| CAMK1D | 57118 | NM_020397 | 8% | 13% |
| Pool 87 | | | 8% | 43% |
| KSR2 | 283455 | NM_173598 | 8% | 58% |
| SGK | 6446 | NM_005627 | 8% | 12% |
| Pool 92 | | | 8% | 41% |
| NEK4 | 6787 | NM_003157 | 8% | 5% |
| PIK3CD | 5293 | NM_005026 | 8% | 9% |
| ASK | 10926 | NM_006716 | 8% | 17% |
| TIE | 7075 | NM_005424 | 8% | 16% |
| AKAP7 | 9465 | NM_004842 | 8% | 17% |
| XYLB | 9942 | NM_005108 | 8% | 23% |
| MARK1 | 4139 | NM_018650 | 8% | 11% |
| FN3K | 64122 | NM_022158 | 8% | 13% |
| NEK6 | 10783 | NM_014397 | 9% | 12% |
| RPS6KC1 | 26750 | NM_012424 | 9% | 20% |
| MAPKAPK5 | 8550 | NM_003668 | 9% | 3% |
| ICK | 22858 | NM_014920 | 9% | 8% |
| Pool 23 | | | 9% | 15% |
| PRKAB2 | 5565 | NM_005399 | 9% | 30% |
| HK3 | 3101 | NM_002115 | 9% | 25% |
| Pool 96 | | | 9% | 48% |
| IBTK | 25998 | AB037838 | 9% | 8% |
| PDK3 | 5165 | NM_005391 | 9% | 8% |
| SKIP | 80309 | XM_051221 | 9% | 19% |
| PRKAA2 | 5563 | NM_006252 | 9% | 14% |
| HCK | 3055 | NM_002110 | 9% | 50% |
| STK29 | 9024 | NM_003957 | 10% | 14% |
| DGKA | 1606 | NM_001345 | 10% | 17% |
| GK | 2710 | NM_000167 | 10% | 8% |
| DAPK2 | 23604 | NM_014326 | 11% | 15% |
| STK31 | 56164 | NM_031414 | 11% | 10% |
| Pool 59 | | | 11% | 16% |
| PI4K2B | 55300 | NM_018323 | 11% | 8% |
| MADD | 8567 | NM_003682 | 11% | 8% |
| PRKCQ | 5588 | NM_006257 | 11% | 12% |
| PDK4 | 5166 | NM_002612 | 11% | 44% |

-

Table 1b (continued)

| Gene name | GeneID | Accession Number | % Inhibition | Standard Deviation |
|---|---|---|---|---|
| PRKRIR | 5612 | NM_004705 | 11% | 19% |
| PFKFB4 | 5210 | NM_004567 | 11% | 15% |
| CAMK2A | 815 | NM_015981 | 11% | 10% |
| Pool 83 | | | 11% | 46% |
| DGKG | 1608 | NM_001346 | 11% | 8% |
| APEG1 | 10290 | NM_005876 | 11% | 16% |
| Pool 80 | | | 11% | 23% |
| CKS1B | 1163 | NM_001826 | 11% | 15% |
| DYRK2 | 8445 | NM_003583 | 11% | 17% |
| PACSIN2 | 11252 | NM_007229 | 11% | 17% |
| LYN | 4067 | NM_002350 | 11% | 33% |
| Pool 67 | | | 12% | 30% |
| MAP4K3 | 8491 | NM_003618 | 12% | 13% |
| PNKP | 11284 | NM_007254 | 12% | 17% |
| PMVK | 10654 | NM_006556 | 12% | 18% |
| Pool 4 | | | 12% | 10% |
| LATS2 | 26524 | NM_014572 | 12% | 13% |
| CKMT1 | 1159 | NM_020990 | 12% | 7% |
| HIPK1 | 204851 | NM_181358 | 12% | 29% |
| STK10 | 6793 | NM_005990 | 12% | 16% |
| STK22D | 83942 | NM_032028 | 12% | 16% |
| TRIO | 7204 | NM_007118 | 12% | 20% |
| GIT2 | 9815 | NM_014776 | 12% | 21% |
| AKAP28 | 158798 | NM_178813 | 12% | 15% |
| UMP-CMP | 51727 | NM_016308 | 13% | 26% |
| Pool 14 | | | 13% | 21% |
| LIM | 10611 | NM_006457 | 13% | 20% |
| PIP5K2C | 79837 | NM_024779 | 13% | 30% |
| PRKG2 | 5593 | NM_006259 | 13% | 6% |
| PCTK1 | 5127 | NM_006201 | 13% | 9% |
| Pool 35 | | | 13% | 9% |
| PKLR | 5313 | NM_181871 | 13% | 13% |
| LATS1 | 9113 | NM_004690 | 13% | 9% |
| ACVR2 | 92 | NM_001616 | 13% | 11% |
| STK24 | 8428 | NM_003576 | 13% | 8% |
| NJMU-R1 | 64149 | NM_022344 | 13% | 18% |
| PANK4 | 55229 | NM_018216 | 13% | 19% |
| CDKL3 | 51265 | NM_016508 | 13% | 12% |
| PHKG1 | 5260 | NM_006213 | 13% | 7% |
| ERN1 | 2081 | NM_001433 | 13% | 14% |
| MAP3K7IP2 | 23118 | NM_015093 | 13% | 24% |
| AK2 | 204 | NM_001625 | 13% | 21% |
| GRAF | 23092 | NM_015071 | 13% | 21% |
| PRKD2 | 25865 | NM_016457 | 14% | 10% |
| PRKAA1 | 5562 | NM_006251 | 14% | 21% |
| TTBK2 | 146057 | NM_173500 | 14% | 8% |
| Pool 50 | | | 14% | 20% |
| KDR | 3791 | NM_002253 | 14% | 25% |

Table 1b (continued)

| Gene name | GeneID | Accession Number | % Inhibition | Standard Deviation |
|---|---|---|---|---|
| NME6 | 10201 | NM_005793 | 14% | 20% |
| EIF2AK3 | 9451 | NM_004836 | 14% | 16% |
| CDK6 | 1021 | NM_001259 | 14% | 9% |
| FGFR4 | 2264 | NM_002011 | 15% | 17% |
| CDK5R2 | 8941 | NM_003936 | 15% | 24% |
| SYK | 6850 | NM_003177 | 15% | 37% |
| DCK | 1633 | NM_000788 | 15% | 21% |
| MAP4K4 | 9448 | NM_004834 | 15% | 12% |
| BUB1B | 701 | NM_001211 | 15% | 21% |
| CHK | 1119 | NM_001277 | 15% | 21% |
| Pool 38 | | | 15% | 38% |
| FLJ11149 | 55312 | NM_018339 | 15% | 7% |
| DYRK3 | 8444 | NM_003582 | 15% | 8% |
| NRGN | 4900 | NM_006176 | 15% | 23% |
| PIK3CA | 5290 | NM_006218 | 15% | 20% |
| PAK1 | 5058 | NM_002576 | 15% | 16% |
| TYRO3 | 7301 | NM_006293 | 15% | 11% |
| TLK2 | 11011 | NM_006852 | 15% | 10% |
| NEK9 | 91754 | NM_033116 | 15% | 12% |
| ERBB3 | 2065 | NM_001982 | 16% | 20% |
| PFKFB3 | 5209 | NM_004566 | 16% | 14% |
| NEK1 | 4750 | NM_012224 | 16% | 32% |
| PKE | 282974 | NM_173575 | 16% | 38% |
| PRKCG | 5582 | NM_002739 | 16% | 21% |
| CDK5 | 1020 | NM_004935 | 16% | 34% |
| PCTK2 | 5128 | NM_002595 | 16% | 25% |
| ALS2CR7 | 65061 | NM_139158 | 16% | 38% |
| Pool 76 | | | 16% | 34% |
| HIPK2 | 28996 | NM_022740 | 16% | 6% |
| CDC42BPB | 9578 | NM_006035 | 16% | 14% |
| EPHB1 | 2047 | NM_004441 | 16% | 24% |
| BCKDK | 10295 | NM_005881 | 16% | 23% |
| STK22C | 81629 | NM_052841 | 17% | 26% |
| CSNK1G3 | 1456 | NM_004384 | 17% | 13% |
| AK3 | 205 | NM_013410 | 17% | 23% |
| EPHA2 | 1969 | NM_004431 | 17% | 10% |
| ANKRD3 | 54101 | NM_020639 | 17% | 14% |
| NEK11 | 79858 | NM_024800 | 17% | 7% |
| Pool 94 | | | 17% | 39% |
| LTK | 4058 | NM_002344 | 17% | 25% |
| Pool 100 | | | 17% | 57% |
| MAP3K12 | 7786 | NM_006301 | 17% | 8% |
| Pool 66 | | | 18% | 34% |
| MAP2K7 | 5609 | BC005365 | 18% | 21% |
| NUCKS | 64710 | NM_022731 | 18% | 9% |
| PIK3CG | 5294 | NM_002649 | 18% | 29% |
| TYROBP | 7305 | NM_003332 | 18% | 8% |
| ROR2 | 4920 | NM_004560 | 18% | 9% |

Table 1b (continued)

| Gene name | GeneID | Accession Number | % Inhibition | Standard Deviation |
|---|---|---|---|---|
| PFTK1 | 5218 | NM_012395 | 18% | 12% |
| MAP3K14 | 9020 | NM_003954 | 18% | 17% |
| PRKRA | 8575 | NM_003690 | 18% | 27% |
| pknbeta | 29941 | NM_013355 | 18% | 19% |
| SGKL | 23678 | NM_013257 | 18% | 22% |
| PANK2 | 80025 | NM_024960 | 18% | 13% |
| CDC2L5 | 8621 | NM_003718 | 18% | 16% |
| MAP2K3 | 5606 | NM_002756 | 18% | 14% |
| PKIB | 5570 | NM_181795 | 18% | 20% |
| MAP3K7 | 6885 | NM_003188 | 18% | 63% |
| PFKFB2 | 5208 | NM_006212 | 19% | 11% |
| CDKN1A | 1026 | NM_000389 | 19% | 12% |
| CSNK2A1 | 1457 | NM_177560 | 19% | 17% |
| PIP5K2A | 5305 | NM_005028 | 19% | 17% |
| STK36 | 27148 | NM_015690 | 19% | 27% |
| CINP | 51550 | NM_032630 | 19% | 17% |
| TEK | 7010 | NM_000459 | 19% | 15% |
| MAP4K2 | 5871 | NM_004579 | 19% | 7% |
| EEF2K | 29904 | NM_013302 | 19% | 14% |
| Pool 72 | | | 19% | 12% |
| TLK1 | 9874 | NM_012290 | 19% | 20% |
| ULK1 | 8408 | NM_003565 | 19% | 16% |
| Pool 79 | | | 19% | 40% |
| CDKN2D | 1032 | NM_001800 | 19% | 19% |
| NRBP | 29959 | NM_013392 | 19% | 21% |
| CDK10 | 8558 | NM_003674 | 19% | 17% |
| NEK8 | 284086 | NM_178170 | 19% | 13% |
| NYD-SP25 | 89882 | BC033792 | 20% | 10% |
| NYD-SP25 | 89882 | NM_033516 | 20% | 10% |
| MAP2K5 | 5607 | NM_002757 | 20% | 8% |
| PRKACB | 5567 | NM_182948 | 20% | 13% |
| NTRK2 | 4915 | NM_006180 | 20% | 21% |
| MAP3K13 | 9175 | NM_004721 | 20% | 16% |
| CSNK1D | 1453 | NM_001893 | 20% | 29% |
| MAP2K1IP1 | 8649 | NM_021970 | 20% | 17% |
| PINK1 | 65018 | NM_032409 | 20% | 8% |
| Pool 25 | | | 20% | 23% |
| ERBB2 | 2064 | NM_004448 | 21% | 12% |
| Pool 42 | | | 21% | 36% |
| AKAP10 | 11216 | NM_007202 | 21% | 17% |
| PANK1 | 53354 | NM_138316 | 21% | 10% |
| Pool 47 | | | 21% | 57% |
| MAPKAPK2 | 9261 | NM_004759 | 21% | 17% |
| Pool 15 | | | 21% | 21% |
| PRKX | 5613 | NM_005044 | 21% | 21% |
| TOPK | 55872 | NM_018492 | 21% | 9% |
| TNIK | 23043 | XM_039796 | 21% | 8% |
| Pool 27 | | | 22% | 27% |

Table 1b (continued)

| Gene name | GeneID | Accession Number | % Inhibition | Standard Deviation |
|---|---|---|---|---|
| FES | 2242 | NM_002005 | 22% | 47% |
| Pool 84 | | | 22% | 22% |
| PHKA2 | 5256 | NM_000292 | 22% | 7% |
| MAP3K4 | 4216 | NM_005922 | 22% | 9% |
| ULK2 | 9706 | NM_014683 | 22% | 15% |
| MAPK13 | 5603 | NM_002754 | 22% | 36% |
| Pool 10 | | | 22% | 13% |
| Pool 49 | | | 22% | 13% |
| NME2 | 4831 | NM_002512 | 22% | 27% |
| Pool 116 | | | 23% | 59% |
| BMPR2 | 659 | NM_001204 | 23% | 19% |
| CASK | 8573 | NM_003688 | 23% | 28% |
| HSMDPKIN | 55561 | XM_290516 | 23% | 48% |
| AKT1 | 207 | NM_005163 | 23% | 13% |
| PXK | 54899 | NM_017771 | 23% | 36% |
| RIPK1 | 8737 | NM_003804 | 23% | 15% |
| DGKD | 8527 | NM_003648 | 23% | 14% |
| IHPK3 | 117283 | NM_054111 | 23% | 33% |
| RPS6KA5 | 9252 | NM_182398 | 23% | 10% |
| PKIA | 5569 | NM_181839 | 24% | 27% |
| RPS6KB2 | 6199 | NM_003952 | 24% | 14% |
| Pool 95 | | | 24% | 49% |
| CDKL5 | 6792 | NM_003159 | 24% | 36% |
| CDC2 | 983 | NM_001786 | 24% | 33% |
| MAP4K5 | 11183 | NM_006575 | 24% | 5% |
| Pool 85 | | | 25% | 29% |
| VRK3 | 51231 | NM_016440 | 25% | 8% |
| EPHA4 | 2043 | NM_004438 | 25% | 12% |
| IKBKG | 8517 | NM_003639 | 25% | 37% |
| PLK2 | 10769 | NM_006622 | 25% | 33% |
| CAMK4 | 814 | NM_001744 | 25% | 48% |
| PIK3R1 | 5295 | NM_181504 | 25% | 17% |
| Pool 16 | | | 25% | 17% |
| EPHB2 | 2048 | NM_017449 | 25% | 5% |
| MGC45419 | 139728 | NM_198452 | 25% | 14% |
| T3JAM | 80342 | NM_025228 | 26% | 21% |
| MAP3K8 | 1326 | NM_005204 | 26% | 11% |
| Pool 112 | | | 26% | 43% |
| PHKA1 | 5255 | NM_002637 | 27% | 6% |
| TGFBR1 | 7046 | NM_004612 | 27% | 18% |
| AKAP9 | 10142 | NM_005751 | 27% | 37% |
| PAK7 | 57144 | NM_177990 | 27% | 26% |
| MINK | 50488 | NM_015716 | 27% | 13% |
| CSNK1A1 | 1452 | NM_001892 | 28% | 30% |
| PIM2 | 11040 | NM_006875 | 28% | 31% |
| PIP5K1A | 8394 | NM_003557 | 28% | 10% |
| PLK1 | 5347 | NM_005030 | 29% | 29% |
| PIP5K3 | 200576 | NM_152671 | 29% | 39% |

Table 1b (continued)

| Gene name | GeneID | Accession Number | % Inhibition | Standard Deviation |
|---|---|---|---|---|
| PRKCABP | 9463 | NM_012407 | 29% | 7% |
| MAP3K2 | 10746 | NM_006609 | 29% | 18% |
| TEC | 7006 | NM_003215 | 29% | 14% |
| FLT4 | 2324 | NM_182925 | 29% | 18% |
| CDKN2A | 1029 | NM_000077 | 30% | 12% |
| EIF2AK4 | 27104 | BX640919 | 30% | 21% |
| Pool 9 | | | 30% | 20% |
| PTK2 | 5747 | NM_005607 | 30% | 26% |
| MARCKS | 4082 | NM_002356 | 30% | 23% |
| AKAP13 | 11214 | NM_006738 | 30% | 26% |
| Pool 63 | | | 30% | 17% |
| UCK1 | 83549 | NM_031432 | 31% | 25% |
| STK33 | 65975 | NM_030906 | 31% | 20% |
| SRC | 6714 | NM_005417 | 31% | 16% |
| P101-PI3K | 23533 | NM_014308 | 31% | 6% |
| MAP3K10 | 4294 | NM_002446 | 31% | 32% |
| PIP5K1C | 23396 | NM_012398 | 31% | 33% |
| RET | 5979 | NM_020975 | 31% | 13% |
| TNNI3K | 51086 | NM_015978 | 31% | 21% |
| Pool 105 | | | 31% | 48% |
| PRKCH | 5583 | NM_006255 | 31% | 24% |
| Pool 89 | | | 31% | 41% |
| ADK | 132 | NM_001123 | 32% | 15% |
| MAP2K1 | 5604 | NM_002755 | 32% | 28% |
| FLT1 | 2321 | NM_002019 | 32% | 15% |
| MGC33182 | 122011 | NM_145203 | 32% | 9% |
| Pool 26 | | | 32% | 15% |
| SKP1A | 6500 | NM_006930 | 33% | 4% |
| AKIP | 54998 | NM_017900 | 33% | 26% |
| CHEK1 | 1111 | NM_001274 | 33% | 23% |
| MAP3K11 | 4296 | NM_002419 | 33% | 25% |
| RPS6KA1 | 6195 | NM_002953 | 34% | 25% |
| STK38 | 11329 | NM_007271 | 34% | 21% |
| PIP5K1B | 8395 | NM_003558 | 34% | 7% |
| PIK3R2 | 5296 | NM_005027 | 35% | 28% |
| SLK | 9748 | NM_014720 | 36% | 22% |
| CAMKK2 | 10645 | NM_006549 | 36% | 10% |
| AKAP3 | 10566 | NM_006422 | 36% | 23% |
| GNE | 10020 | NM_005476 | 36% | 22% |
| KIAA1446 | 57596 | NM_020836 | 37% | 7% |
| AATK | 9625 | AB014541 | 37% | 15% |
| ADCK2 | 90956 | NM_052853 | 37% | 17% |
| Pool 28 | | | 37% | 76% |
| PTK6 | 5753 | NM_005975 | 37% | 28% |
| MAPK12 | 6300 | NM_002969 | 37% | 22% |
| CRK7 | 51755 | NM_016507 | 38% | 7% |
| PASK | 23178 | NM_015148 | 38% | 27% |
| TNK1 | 8711 | NM_003985 | 38% | 13% |

Table 1b (continued)

| Gene name | GeneID | Accession Number | % Inhibition | Standard Deviation |
|---|---|---|---|---|
| PFKL | 5211 | NM_002626 | 38% | 20% |
| MAP4K1 | 11184 | NM_007181 | 38% | 23% |
| MAPK9 | 5601 | NM_002752 | 39% | 20% |
| FUK | 197258 | NM_145059 | 39% | 13% |
| LOC55971 | 55971 | NM_018842 | 39% | 13% |
| ZAK | 51776 | NM_016653 | 39% | 30% |
| NTRK3 | 4916 | NM_002530 | 40% | 25% |
| NTRK1 | 4914 | NM_002529 | 40% | 32% |
| STK35 | 140901 | NM_080836 | 41% | 12% |
| CDK5R1 | 8851 | NM_003885 | 41% | 21% |
| PAK3 | 5063 | NM_002578 | 41% | 22% |
| CAMK2B | 816 | NM_001220 | 41% | 4% |
| NME5 | 8382 | NM_003551 | 41% | 22% |
| Pool 90 | | | 41% | 31% |
| GAK | 2580 | NM_005255 | 41% | 32% |
| AKT3 | 10000 | NM_181690 | 42% | 41% |
| ADCK1 | 57143 | NM_020421 | 42% | 19% |
| STK4 | 6789 | NM_006282 | 43% | 26% |
| AKAP8L | 26993 | NM_014371 | 43% | 29% |
| MAPK11 | 5600 | NM_002751 | 43% | 28% |
| UMPK | 7371 | NM_012474 | 43% | 35% |
| CSNK1G2 | 1455 | NM_001319 | 43% | 24% |
| PIK3C2B | 5287 | NM_002646 | 43% | 38% |
| C21orf124 | 85006 | NM_003681 | 44% | 31% |
| CKB | 1152 | NM_001823 | 44% | 33% |
| CIB3 | 117286 | NM_054113 | 45% | 28% |
| ILK | 3611 | NM_004517 | 45% | 19% |
| PRKACG | 5568 | NM_002732 | 45% | 20% |
| SRPK1 | 6732 | NM_003137 | 45% | 5% |
| AAK1 | 22848 | NM_014911 | 46% | 21% |
| PCTK3 | 5129 | AK126879 | 46% | 23% |
| NEK7 | 140609 | NM_133494 | 46% | 22% |
| LOC375449 | 375449 | NM_198828 | 46% | 4% |
| ROS1 | 6098 | NM_002944 | 47% | 11% |
| PDGFRA | 5156 | NM_006206 | 47% | 19% |
| CIT | 11113 | NM_007174 | 48% | 12% |
| LOC388226 | 10595 | XM_370946 | 48% | 39% |
| CDKN1B | 1027 | NM_004064 | 48% | 5% |
| NME7 | 29922 | NM_197972 | 48% | 8% |
| RPS6KA6 | 27330 | NM_014496 | 49% | 16% |
| Pool 57 | | | 49% | 20% |
| PIK4CB | 5298 | NM_002651 | 51% | 16% |
| Pool 20 | | | 51% | 20% |
| MAP2K4 | 6416 | NM_003010 | 51% | 14% |
| CHEK2 | 11200 | NM_007194 | 51% | 28% |
| ALS2CR2 | 55437 | NM_018571 | 52% | 18% |
| GRK1 | 6011 | NM_002929 | 52% | 17% |
| Pool 93 | | | 52% | 32% |

Table 1b (continued)

| Gene name | GeneID | Accession Number | % Inhibition | Standard Deviation |
|---|---|---|---|---|
| MAPK4 | 5596 | NM_002747 | 52% | 24% |
| STK22B | 23617 | NM_053006 | 52% | 15% |
| Pool 109 | | | 52% | 51% |
| NYD-SP25 | 89882 | BC033792 | 53% | 44% |
| NYD-SP25 | 89882 | NM_033516 | 53% | 44% |
| ANKK1 | 255239 | NM_178510 | 53% | 7% |
| SPHK1 | 8877 | NM_021972 | 53% | 33% |
| PLK3 | 1263 | NM_004073 | 53% | 19% |
| GRK4 | 2868 | NM_182982 | 54% | 23% |
| PIP5K2B | 8396 | NM_003559 | 54% | 30% |
| Pool 102 | | | 55% | 22% |
| NIPA | 51530 | NM_016478 | 55% | 11% |
| RPS6KA4 | 8986 | NM_003942 | 55% | 16% |
| IKBKB | 3551 | NM_001556 | 56% | 10% |
| FGR | 2268 | NM_005248 | 57% | 12% |
| PTK7 | 5754 | NM_152883 | 58% | 5% |
| RIOK2 | 55781 | NM_018343 | 59% | 20% |
| CSNK1E | 1454 | NM_001894 | 59% | 39% |
| PRKCL1 | 5585 | NM_002741 | 60% | 21% |
| STK3 | 6788 | NM_006281 | 60% | 20% |
| GSK3A | 2931 | NM_019884 | 60% | 28% |
| HK1 | 3098 | NM_033498 | 61% | 19% |
| PRKCZ | 5590 | NM_002744 | 62% | 16% |
| RIOK3 | 8780 | NM_003831 | 62% | 19% |
| CKS2 | 1164 | NM_001827 | 63% | 31% |
| FASTK | 10922 | NM_006712 | 63% | 44% |
| MST1R | 4486 | NM_002447 | 65% | 24% |
| BMPR1B | 658 | NM_001203 | 66% | 33% |
| PRKAR2A | 5576 | NM_004157 | 69% | 39% |
| CDC2L1 | 984 | NM_001787 | 73% | 26% |
| PRKAB1 | 5564 | NM_006253 | 74% | 30% |
| PKMYT1 | 9088 | NM_182687 | 78% | 15% |
| PAK4 | 10298 | NM_005884 | 81% | 49% |
| CALM1 | 801 | NM_006888 | 82% | 68% |
| BMX | 660 | NM_001721 | 84% | 83% |
| NME3 | 4832 | NM_002513 | 85% | 50% |
| JIK | 51347 | NM_016281 | 89% | 27% |
| ACK1 | 10188 | NM_005781 | 93% | 11% |
| HUNK | 30811 | NM_014586 | 94% | 35% |
| MAPK8 | 5599 | NM_002750 | 98% | 47% |
| DGKE | 8526 | NM_003647 | 113% | 25% |

Table 2

| Accession # | LocusLink | Symbol | Gene Name | Target 1 | Target 2 |
|---|---|---|---|---|---|
| AB014541 | 9625 | AATK | apoptosis-associated tyrosine kinase | CTCCAACGTGTCAGCCAACAA | TCCGCTGAGATCAGAAGGCAA |
| AB023190 | 22983 | SAST | syntrophin associated serine/threonine kinase | CAGCCTCCGTATACATATGTA | CCCGAGGTCATCCTGCGTCAA |
| AB033076 | 57498 | KIDINS220 | likely homolog of rat kinase D-interacting substance of 220 kDa | CAGGCGAGACTCCTTATAATA | TCGCAGATGCGCTATTATCTA |
| AB037838 | 25998 | IBTK | inhibitor of Bruton agammaglobulinemia tyrosine kinase | CAGATTGAGGAGCATGCCATA | TAGAAGAGTCATTACACCATA |
| AB096248 | 23235 | SIK2 | salt-inducible serine/threonine kinase 2 | AAGGGCAAGATTATTTGCTTA | CCGAAGGATGTTGGTCCTAGA |
| NM_004935 | 1020 | CDK5 | cyclin-dependent kinase 5 | CCCTTTGTGGACTTTATTTAA | CCGGGAGATCTGCCTACTCAA |
| AK126879 | 5129 | PCTK3 | PCTAIRE protein kinase 3 | AACCCGTTACTTAGCTGTGTA | CAGGTTGGATACGGATGGCAT |
| BC028192 | 161635 | LOC161635 | similar to casein kinase I alpha | TCCATCGATCTGGAATTTATA | TTGGTCGTGTGAATTATTAAA |
| BC033792 | 89882 | NYD-SP25 | protein kinase NYD-SP25 | ATCATATACTTCAGACATCAA | CACGTTAAATCAAGGAAGGAA |
| BF688722 | 2323 | FLT3LG | Fms-related tyrosine kinase 3 ligand | GCGGGATTTGTTGAAGAGAAA | TAGTATAGTTGAAATAAGAAA |
| BX537762 | 134728 | IRAK1BP1 | interleukin-1 receptor-associated kinase 1 binding protein 1/DKFZp779F0411 | AACCTGGAGTATCATATTATA | ACCAAGATACTTGAGTGATTA |
| BX640919 | 27104 | EIF2AK4 | eukaryotic translation initiation factor 2 alpha kinase 4 | CTGCAGGAATTTAGTAACATT | TCCGATAATCTTGCAGTGCAA |
| NM_000020 | 94 | ACVRL1 | activin A receptor type II-like 1 | CACCGAGTTCGTCAACCACTA | CAGGAAGACGCTGGAATAAGA |
| NM_000051 | 472 | ATM | ataxia telangiectasia mutated (includes complementation groups A, C and D) | AACCATGAGTCTAGTACTTAA | TTGGCTTATACGCGCAGTGTA |
| NM_000061 | 695 | BTK | Bruton agammaglobulinemia tyrosine kinase | CAGCTCGAAACTGTTTGGTAA | CCCTTTATGATTACATGCCAA |
| NM_000075 | 1019 | CDK4 | cyclin-dependent kinase 4 | AAGGTAATCCGGAGTGAGCAA | CCGAACTGACCGGGAGATCAA |
| NM_000076 | 1028 | CDKN1C | cyclin-dependent kinase inhibitor 1C (p57, Kip2) | AAGCTTTAAGAGTCATTTATA | TACACTGGTCCCAAAGTGTAA |
| NM_000077 | 1029 | CDKN2A | cyclin-dependent kinase inhibitor 2A (melanoma, p16, inhibits CDK4) | CACGCCCTAAGCGCACATTCA | CAGGTGTGCCACATTCGCTAA |
| NM_000141 | 2263 | FGFR2 | fibroblast growth factor receptor 2 | CCGAATGAAGAACACGACCAA | CGGAGGAGCGTTGCCATTCAA |
| NM_000142 | 2261 | FGFR3 | fibroblast growth factor receptor 3 (achondroplasia, thanatophoric dwarfism) | ACCCTACGTTACCGTGCTCAA | CCGATGTTATTAGATGTTACA |

Table 2

| | | | | | |
|---|---|---|---|---|---|
| NM_000167 | 2710 | GK | glycerol kinase | CCACCACTTAAGTGACATAAA | CTGAGGGTAGCCCATTTATAA |
| NM_000189 | 3099 | HK2 | hexokinase 2 | CCGGCCGTGCTACAATAGGTA | CGGCCGTGCTACAATAGGTAA |
| NM_000215 | 3718 | JAK3 | Janus kinase 3 (a protein tyrosine kinase, leukocyte) | CGGGAGATTCAGATCCTCAAA | TCCGGGAGGCGCAGACACTTA |
| NM_000221 | 3795 | KHK | ketohexokinase (fructokinase) | TAGCCGCACCATCCTATACTA | TAGGGTGGGTAAGGCCTTATA |
| NM_000222 | 3815 | KIT | v-kit Hardy-Zuckerman 4 feline sarcoma viral oncogene homolog | CACGGTTGAATGTAAGGCTTA | CTCGCACCTTTCCAAAGTTAA |
| NM_000245 | 4233 | MET | met proto-oncogene (hepatocyte growth factor receptor) | ACCGAGGGAATCATCATGAAA | CGCGCCGTGATGAATATCGAA |
| NM_000269 | 4830 | NME1 | non-metastatic cells 1, protein (NM23A) expressed in | TAGGATTCATTGAGTTGGTTA | TTCCGCCTTGTTGGTCTGAAA |
| NM_000289 | 5213 | PFKM | phosphofructokinase, muscle | AACAGATCAGTGCCAATATAA | AAGGACTTTCGGGAACGAGAA |
| NM_000291 | 5230 | PGK1 | phosphoglycerate kinase 1 | CACAATTATAGATTAGATCAA | GAGGAACGGATCAGATGTCTA |
| NM_000292 | 5256 | PHKA2 | phosphorylase kinase, alpha 2 (liver) | CCGCCTAACAAGGTAGATGAA | CCGGATTCTTAGTCACATATA |
| NM_000293 | 5257 | PHKB | phosphorylase kinase, beta | AGGGCTGTTCGTGGTCAGTTA | TCGATGGGTCTTTGAATAGAA |
| NM_000294 | 5261 | PHKG2 | phosphorylase kinase, gamma 2 (testis) | CTGCATGCACTGCATATGAAA | TACGGGCACTGGGTAAAGAAA |
| NM_000389 | 1026 | CDKN1A | cyclin-dependent kinase inhibitor 1A (p21, Cip1) | CAGTTTGTGTGTCTTAATTAT | CTGGCATTAGAATTATTTAAA |
| NM_000431 | 4598 | MVK | mevalonate kinase (mevalonic aciduria) | CCGCTTGGCGATGCCAGCCAA | GCGGTGGGCCTGGTTAAATAA |
| NM_000455 | 6794 | STK11 | serine/threonine kinase 11 (Peutz-Jeghers syndrome) | CAGGAGTGTGCGGTCAATATT | GAGGGCCGTCAAGATCCTCAA |
| NM_000459 | 7010 | TEK | TEK tyrosine kinase, endothelial (venous malformations, multiple cutaneous and mucosal) | ACGGGCCAGATTGTAAGCTTA | TCGGTGCTACTTAACAACTTA |
| NM_000476 | 203 | AK1 | adenylate kinase 1 | CCGGGATGCCATGGTGGCCAA | GCGGCTGGAGACCTATTACAA |
| NM_000788 | 1633 | DCK | deoxycytidine kinase | AAGTTGCAAAGTGGAATTAAA | TTGGTATAAATTAATTTGTTA |
| NM_000858 | 2987 | GUK1 | guanylate kinase 1 | CAGGGCTGACATCCTAATAAA | CCCGGCGAGGAGAACGGCAAA |
| NM_001079 | 7535 | ZAP70 | zeta-chain (TCR) associated protein kinase 70kDa | CCGCAACGTCCTGCTGGTTAA | GGCGTAGATCACCAGAATAAA |
| NM_001105 | 90 | ACVR1 | activin A receptor, type I | CGGATGGTGAGCAATGGTATA | CTGGTCTGTCTTTGGATAATA |
| NM_001106 | 93 | ACVR2B | activin A receptor, type IIB | CAGCTCATGAATGACTTTGTA | TACGGTCATGTGGACATCCAT |

<u>Table 2</u>

| NM_001123 | 132 | ADK | adenosine kinase | AAGGGTATGGTAATGCTTGTA | TAGTTTGATAGTGCCACTTAA |
|---|---|---|---|---|---|
| NM_001184 | 545 | ATR | ataxia telangiectasia and Rad3 related | CAGGCACTAATTGTTCTTCAA | GCCGCTAATCTTCTAACATTA |
| NM_001203 | 658 | BMPR1B | bone morphogenetic protein receptor, type IB | AACGAATGTAATAAAGACCTA | ACGGATATTGTTTCACGATGA |
| NM_001204 | 659 | BMPR2 | bone morphogenetic protein receptor, type II (serine/threonine kinase) | AAGCACCGAAGCGAAACTTAA | CTCGTAAGTATGTAAAGGAAA |
| NM_001211 | 701 | BUB1B | BUB1 budding uninhibited by benzimidazoles 1 homolog beta (yeast) | ACGAGAATACCTAATATGTGA | CAGATTTAGCACATTTACTAT |
| NM_001220 | 816 | CAMK2B | calcium/calmodulin-dependent protein kinase (CaM kinase) II beta | CAAGAGTTTACTCAACAAGAA | CCGGAAGCAGGAGATCATTAA |
| NM_001221 | 817 | CAMK2D | calcium/calmodulin-dependent protein kinase (CaM kinase) II delta | CCGCCTGCATAGCATATATTA | GTGCGACTTCATGATAGCATA |
| NM_001258 | 1018 | CDK3 | cyclin-dependent kinase 3 | ATGGATATGTTCCAGAAGGTA | CAGACTGGATTTGGAGATGGA |
| NM_001259 | 1021 | CDK6 | cyclin-dependent kinase 6 | AAGACTCAAGGTGGTCAGTAA | TCTGAAGTGTTTGACATTTAA |
| NM_001260 | 1024 | CDK8 | cyclin-dependent kinase 8 | CCAGCTGGACAGAATATTCAA | TTCGAGAGCTTAAGCATCCAA |
| NM_001261 | 1025 | CDK9 | cyclin-dependent kinase 9 (CDC2-related kinase) | TAGGGACATGAAGGCTGCTAA | TGGGCACAGTTTGGTCCGTTA |
| NM_001262 | 1031 | CDKN2C | cyclin-dependent kinase inhibitor 2C (p18, inhibits CDK4) | CAGGCTTATGAATATATTTAA | TGCCTCTACTTTATCAATTAA |
| NM_001274 | 1111 | CHEK1 | CHK1 checkpoint homolog (S. pombe) | AAGAAAGAGATCTGTATCAAT | CCCGCACAGGTCTTTCCTTAT |
| NM_001277 | 1119 | CHK | choline kinase | AGCCGGCGATTAGATACTGAA | CCGGCCCTTACCAAACCTGAT |
| NM_001278 | 1147 | CHUK | conserved helix-loop-helix ubiquitous kinase | CAGGAGAAGTTCGGTTTAGTA | TTCCATAAGCTTGGTGACAAA |
| NM_001291 | 1196 | CLK2 | CDC-like kinase 2 | CAGCTACAGACGCAACGATTA | TCGCCTGGATTGGGATGAGAA |
| NM_001292 | 1198 | CLK3 | CDC-like kinase 3 | CACGAAGATCTCGGTCCAGAA | CTGCATTCTCTTTGAGTACTA |
| NM_001315 | 1432 | MAPK14 | mitogen-activated protein kinase 14 | CAGAGAACTGCGGTTACTTAA | CTCAGTGATACGTACAGCCAA |
| NM_001319 | 1455 | CSNK1G2 | casein kinase 1, gamma 2 | AAGAATCTCTATACAAATGAA | TAGGAAAGAATCTCTATACAA |
| NM_001320 | 1460 | CSNK2B | casein kinase 2, beta polypeptide | CAGGTCCCTCACTACCGACAA | GTGGTGGGAATATGAAATAAA |
| NM_001345 | 1606 | DGKA | diacylglycerol kinase, alpha 80kDa | ATCCATCTTCTCAACATGCAA | CACGACCAGTGTGCCATGAAA |
| NM_001346 | 1608 | DGKG | diacylglycerol kinase, gamma 90kDa | ACCGCAAATGTGAATTATCAA | TAGCCACTCACATAAAGTTTA |
| NM_001347 | 1609 | DGKQ | diacylglycerol kinase, theta 110kDa | CAGCGACACCAGGTTTGAGAA | CCGGAAGGTGACGCTCACCAA |

Table 2

| NM_001348 | 1613 | DAPK3 | death-associated protein kinase 3 | CCCAGAGATTGTGAACTATGA | CCGGCAGAAGGGCACGGGCAA |
|---|---|---|---|---|---|
| NM_001381 | 1796 | DOK1 | docking protein 1, 62kDa (downstream of tyrosine kinase 1) | AGGGATCAAAGAAGATGGTTA | CCGCCTGGACTGCAAAGTGAT |
| NM_001396 | 1859 | DYRK1A | dual-specificity tyrosine-(Y)-phosphorylation regulated kinase 1A | CAAGAATGGGTTGCCATTAAA | TACGGTCGCTGACTACTTGAA |
| NM_001433 | 2081 | ERN1 | ER to nucleus signalling 1 | CAGCACGGACGTCAAGTTTGA | CAGGACGTGAGCGACAGAATA |
| NM_001569 | 3654 | IRAK1 | interleukin-1 receptor-associated kinase 1 | CCGGGCAATTCAGTTTCTACA | TCCCATCGCCATGCAGATCTA |
| NM_001570 | 3656 | IRAK2 | interleukin-1 receptor-associated kinase 2 | CAGCAACGTCAAGAGCTCTAA | CCAGATCATCCTGAACTGGAA |
| NM_001616 | 92 | ACVR2 | activin A receptor, type II | GAGGTATTAGAGGGTGCTATA | TCCACGGTTGCTAAATTATAA |
| NM_001619 | 156 | ADRBK1 | adrenergic, beta, receptor kinase 1 | CCGGGAGATCTTCGACTCATA | CGGCTGGAGGCTCGCAAGAAA |
| NM_001625 | 204 | AK2 | adenylate kinase 2 | ATGGGTGGGAATGATAGGACA | CCACATGTAAAGACTTGGTTA |
| NM_001626 | 208 | AKT2 | v-akt murine thymoma viral oncogene homolog 2 | ACGGGCTAAAGTGACCATGAA | CAAGCGTGGTGAATACATCAA |
| NM_001654 | 369 | ARAF1 | v-raf murine sarcoma 3611 viral oncogene homolog 1 | CCGACTCATCAAGGGACGAAA | CCGGGATGGCATGAGTGTCTA |
| NM_001699 | 558 | AXL | AXL receptor tyrosine kinase | CCGGTGTTCTAAGATGTGATA | TCCAAGATTCTAGATGATTAA |
| NM_001715 | 640 | BLK | B lymphoid tyrosine kinase | CAGCCGTGCTGTTAAACCAAA | CTGGTAAGCGACTGTCATCAA |
| NM_001721 | 660 | BMX | BMX non-receptor tyrosine kinase | CCCAATATGACAACGAATCAA | CGGAACAAAGTTTCCAGTCAA |
| NM_001743 | 805 | CALM2 | calmodulin 2 (phosphorylase kinase, delta) | ACAAAGGAATTGGGAACTGTA | CAAAGGAATTGGGAACTGTAA |
| NM_001744 | 814 | CAMK4 | calcium/calmodulin-dependent protein kinase IV | CAGAAGCCTTATGCTCTCAAA | TTGCAAGTTAACACAACGTAA |
| NM_001786 | 983 | CDC2 | cell division cycle 2, G1 to S and G2 to M | TCGGGAAATTTCTCTATTAAA | TTGACTAACTATGGAAGATTA |
| NM_001787 | 984 | CDC2L1 | cell division cycle 2-like 1 (PITSLRE proteins) | CAAGATCTACATCGTGATGAA | CAGGATAAAGCTCGCCGGGAA |
| NM_001798 | 1017 | CDK2 | cyclin-dependent kinase 2 | CACGTACGGAGTTGTGTACAA | CAGGTTATATCCAATAGTAGA |
| NM_001799 | 1022 | CDK7 | cyclin-dependent kinase 7 (MO15 homolog, Xenopus laevis, cdk-activating kinase) | CCGGATGGCTCTGGACGTGAA | GAGGCTTTAAGGTAGCTTTAA |

Table 2

| NM_001800 | 1032 | CDKN2D | cyclin-dependent kinase inhibitor 2D (p19, inhibits CDK4) | ACCCAAGGGCAGAGCATTTAA | ATGAGTTATGAGTTATTCATA |
|---|---|---|---|---|---|
| NM_001823 | 1152 | CKB | creatine kinase, brain | CCAGATTGAAACTCTCTTCAA | CCGGCCTCACCCAGATTGAAA |
| NM_001824 | 1158 | CKM | creatine kinase, muscle | GACGGGCGAGTTCAAAGGGAA | TCCACGCAAAGCGATAAATAA |
| NM_001825 | 1160 | CKMT2 | creatine kinase, mitochondrial 2 (sarcomeric) | CCTGCCTATCTTTACAATAAA | GAGGTTGAGCTTGTTCAGATA |
| NM_001826 | 1163 | CKS1B | CDC28 protein kinase regulatory subunit 1B | AACATCTTTCTGATAACATTA | AAGAAATCTGTTCATGTTAAA |
| NM_001827 | 1164 | CKS2 | CDC28 protein kinase regulatory subunit 2 | CTGTAAGATGTTTAAGATAAA | TCGGACAAGTACTTCGACGAA |
| NM_001892 | 1452 | CSNK1A1 | casein kinase 1, alpha 1 | AGGGCTAAAGGCTGCAACAAA | CAAGAGTAACATGAAAGGTTT |
| NM_001893 | 1453 | CSNK1D | casein kinase 1, delta | CCGGTCTAGGATCGAAATGTT | CTCCCTGACGATTCCACTGTA |
| NM_001894 | 1454 | CSNK1E | casein kinase 1, epsilon | GAGCTTTATCGTGGTTGTTAA | GTGGTTGTTAATTTGAAGTAA |
| NM_001896 | 1459 | CSNK2A2 | casein kinase 2, alpha prime polypeptide | CAGGAGTACAATGTTCGTGTA | CTGGGACAACATTCACGGAAA |
| NM_001929 | 1716 | DGUOK | deoxyguanosine kinase | CACCTTTGTAAAGAATCTGTA | CAGCACGATGGTCCTACACAT |
| NM_001954 | 780 | DDR1 | discoidin domain receptor family, member 1 | ACGGTGTGAATCACACATCCA | CAGGAATGATTTCCTGAAAGA |
| NM_001982 | 2065 | ERBB3 | v-erb-b2 erythroblastic leukemia viral oncogene homolog 3 (avian) | ACCGGCGATGCTGAGAACCAA | CCAGAGCTTCAAGACTGTTTA |
| NM_002005 | 2242 | FES | feline sarcoma oncogene | AAGGCCAAGTTTCTACAGGAA | CAGCCTGAGGCTGAGTACCAA |
| NM_002011 | 2264 | FGFR4 | fibroblast growth factor receptor 4 | CACATTGACTACTATAAGAAA | CCGCCTGACCTTCGGACCCTA |
| NM_002019 | 2321 | FLT1 | fms-related tyrosine kinase 1 (vascular endothelial growth factor/vascular permeability factor receptor) | ACCGCATATGGTATCCCTCAA | CACTACAGTATTAGCAAGCAA |
| NM_002031 | 2444 | FRK | fyn-related kinase | CAGGACAGTCAAGGTGATATA | CTGGGAGTACCTAGAACCCTA |
| NM_002037 | 2534 | FYN | FYN oncogene related to SRC, FGR, YES | AAGAAGCAGGATGCTGATCTA | AAGACATGTGGTGTATATAAA |
| NM_002082 | 2870 | GRK6 | G protein-coupled receptor kinase 6 | AAGGATGTTCTGGACATTGAA | AGGCATGTGGGCGGCACGTAA |
| NM_002093 | 2932 | GSK3B | glycogen synthase kinase 3 beta | AACACTGGTCACGTTTGGAAA | CTGCATTTATCGTTAACCTAA |
| NM_002110 | 3055 | HCK | hemopoietic cell kinase | AACGACGGGCTCTGCCAGAAA | CGGGAGCACATCAGAGGCTTA |
| NM_002115 | 3101 | HK3 | hexokinase 3 (white cell) | CACTCAGGTTAGCAATATATA | CCGGAATGCGATGTCTCCTTA |

EP 2 295 543 A1

Table 2

| Accession | ID | Symbol | Description | Sequence 1 | Sequence 2 |
|---|---|---|---|---|---|
| NM_002220 | 3706 | ITPKA | inositol 1,4,5-trisphosphate 3-kinase A | CACCAGCGGGGCTGATCCTGAA | CCGGTCTCAACGTCTCACACCA |
| NM_002221 | 3707 | ITPKB | inositol 1,4,5-trisphosphate 3-kinase B | AAGGCCATTCGAACCACTCTA | GACGTAAAGATTCTATGAATA |
| NM_002227 | 3716 | JAK1 | Janus kinase 1 (a protein tyrosine kinase) | ACCGGATGAGGTTCTATTTCA | CACGGATAACATCAGCTTCAT |
| NM_002253 | 3791 | KDR | kinase insert domain receptor (a type III receptor tyrosine kinase) | AACGCTGACATGTACGGTCTA | AAGGCTAATACAACTCTTCAA |
| NM_002314 | 3984 | LIMK1 | LIM domain kinase 1 | CTGCCTGGTCCGCGAGAACAA | GAGCATCTAGGAGTATTAAA |
| NM_002344 | 4058 | LTK | leukocyte tyrosine kinase | ACAGATCTTTGGAGTGCCTAA | CCTGCAGATGCTTCTAATAAA |
| NM_002350 | 4067 | LYN | v-yes-1 Yamaguchi sarcoma viral related oncogene homolog | CCCGGACGACTTGTCTTTCAA | CGGGAAATATGGGATGTATAA |
| NM_002356 | 4082 | MARCKS | myristoylated alanine-rich protein kinase C substrate | CTCCTTCAAGAAGAACAAGAA | TTGAGTTTCTTTGTTGAAGAA |
| NM_002376 | 4140 | MARK3 | MAP/microtubule affinity-regulating kinase 3 | AACGGCGAACCGCAACATATA | CCCGGGAACGGCGAACCGCAA |
| NM_002378 | 4145 | MATK | megakaryocyte-associated tyrosine kinase | ACGGATTCTAAGGACTCTAAA | GACGGATTCTAAGGACTCTAA |
| NM_002401 | 4215 | MAP3K3 | mitogen-activated protein kinase kinase kinase 3 | CAGGAATACTCAGATCGGGAA | CCACGTGTCTGTGCACCACAA |
| NM_002419 | 4296 | MAP3K11 | mitogen-activated protein kinase kinase kinase 11 | CCCGACGTCTGGAGGACTCAA | CCGGAGGAGAAACGTCTTCGA |
| NM_002446 | 4294 | MAP3K10 | mitogen-activated protein kinase kinase kinase 10 | AAGCGGCTTGAAGTCATCGAA | CAGGATGTTCACTCTATTTAT |
| NM_002447 | 4486 | MST1R | macrophage stimulating 1 receptor (c-met-related tyrosine kinase) | ATGGAGCATCCTTCTCCGATA | TGCCATTAAGTTTGAGTATAT |
| NM_002497 | 4751 | NEK2 | NIMA (never in mitosis gene a)-related kinase 2 | TACGAGGATGTTAAACTTAAA | TTACGAGGATGTTAAACTTAA |
| NM_002512 | 4831 | NME2 | non-metastatic cells 2, protein (NM23B) expressed in | ATAGAGCATATTTGCCAATAA | TAGAGCATATTTGCCAATAAA |
| NM_002513 | 4832 | NME3 | non-metastatic cells 3, protein expressed in | ACGCACCTTCCTGGCCGTGAA | CTGCATCGAGGTTGGCAAGAA |
| NM_002529 | 4914 | NTRK1 | neurotrophic tyrosine kinase, receptor, type 1 | CACATCATCGAGAGACCCACAA | CGAGAGCATCCTGTACCGTAA |

Table 2

| | | | | | |
|---|---|---|---|---|---|
| NM_002530 | 4916 | NTRK3 | neurotrophic tyrosine kinase, receptor, type 3 | CACGGATAACTTTATCTTGTT | CTGGTTGGAGCGAATCTGCTA |
| NM_002576 | 5058 | PAK1 | p21/Cdc42/Rac1-activated kinase 1 (STE20 homolog, yeast) | TCCACTGATTGCTGCAGCTAA | TTGAAGAGAACTGCAACTGAA |
| NM_002577 | 5062 | PAK2 | p21 (CDKN1A)-activated kinase 2 | CCGCGACCGGATCATACGAAA | CCGGATCATACGAAATCAATT |
| NM_002578 | 5063 | PAK3 | p21 (CDKN1A)-activated kinase 3 | CAAGAAGGAATTAATTATTAA | TTCCAGTACTTTGTACAGGAA |
| NM_002595 | 5128 | PCTK2 | PCTAIRE protein kinase 2 | AGGCTTAATTCTTGCGTATAA | TAGGCTTAATTCTTGCGTATA |
| NM_002609 | 5159 | PDGFRB | platelet-derived growth factor receptor, beta polypeptide | CCGAGCAACTTTGATCAACGA | CTGCCGAGCAACTTTGATCAA |
| NM_002610 | 5163 | PDK1 | pyruvate dehydrogenase kinase, isoenzyme 1 | CCCGAACTAGAACTTGAAGAA | TCGGGTTTCTATAGGAAACTA |
| NM_002611 | 5164 | PDK2 | pyruvate dehydrogenase kinase, isoenzyme 2 | AAGAACCTTGTTAGACCGAGA | TAGGTCTGTGATGGTCCCTAA |
| NM_002612 | 5166 | PDK4 | pyruvate dehydrogenase kinase, isoenzyme 4 | ACCCGTGTCAATTAACATTTA | AGGCCTCTTGTTCACATCAAA |
| NM_002613 | 5170 | PDPK1 | 3-phosphoinositide dependent protein kinase-1 | AAGCGGTTAGGCTGTGAGGAA | CACGCCTAACAGGACGTATTA |
| NM_002625 | 5207 | PFKFB1 | 6-phosphofructo-2-kinase/fructose-2,6-biphosphatase 1 | CTCCACAAAGCTCACACGATA | CTGCGAGACCAAGATAAATAT |
| NM_002626 | 5211 | PFKL | phosphofructokinase, liver | CACGATGGCTTCGAAGGCCTA | CCGGACCTACTCGCACCTGAA |
| NM_002627 | 5214 | PFKP | phosphofructokinase, platelet | CAGCACTTTATGCACGTATTA | CCGCCTGAAGGCTGCTTGCAA |
| NM_002637 | 5255 | PHKA1 | phosphorylase kinase, alpha 1 (muscle) | ATGGGAGTGCTTGGAACTTCA | CCCAATCGTCTGTACTATGAA |
| NM_002645 | 5286 | PIK3C2A | phosphoinositide-3-kinase, class 2, alpha polypeptide | CAAGATGGTCGAATCAAGGAA | TACCCACTAATTGCATTGGAA |
| NM_002646 | 5287 | PIK3C2B | phosphoinositide-3-kinase, class 2, beta polypeptide | AAGGGTTGTCTTCCTCGCCAA | CAGGGTGGTCCAGTCCGTCAA |
| NM_002647 | 5289 | PIK3C3 | phosphoinositide-3-kinase, class 3 | AACGCGAAAGTGGAAATCGTA | TCGGTTGGTGCATCTAATGAA |
| NM_002648 | 5292 | PIM1 | pim-1 oncogene | AAACATCCTTATCGACCTCAA | AAAGATTGTAGTGGATCTAAT |
| NM_002649 | 5294 | PIK3CG | phosphoinositide-3-kinase, catalytic, gamma polypeptide | ATCGAAGTTTGCAGAGACAAA | CACCTTTACTCTATAACTCAA |
| NM_002650 | 5297 | PIK4CA | phosphatidylinositol 4-kinase, catalytic, alpha polypeptide | CATCTGGAACATGAAGACTAA | CCAGTTCATCTGGAACATGAA |

EP 2 295 543 A1

Table 2

| NM_002651 | 5298 | PIK4CB | phosphatidylinositol 4-kinase, catalytic, beta polypeptide | CCGAGAGTATTGATAATTCAT | TCGGCTGATAGTGGCATGATT |
|---|---|---|---|---|---|
| NM_002730 | 5566 | PRKACA | protein kinase, cAMP-dependent, catalytic, alpha | CAAGGACAACTCAAACTTATA | CAGAAGGTGGTGAAACTGAAA |
| NM_002732 | 5568 | PRKACG | protein kinase, cAMP-dependent, catalytic, gamma | AACCAGCTGGATCGCCATCTA | CTGGATCGCCATCTATGAGAA |
| NM_002733 | 5571 | PRKAG1 | protein kinase, AMP-activated, gamma 1 non-catalytic subunit | CCCTAAGATAATAGCACAACA | TTGGCCCTGATTCAACCCTAA |
| NM_002734 | 5573 | PRKAR1A | protein kinase, cAMP-dependent, regulatory, type I, alpha (tissue specific extinguisher 1) | CAGCTAGTGCCAAATAATTGA | CTGGACCGACCTAGATTTGAA |
| NM_002736 | 5577 | PRKAR2B | protein kinase, cAMP-dependent, regulatory, type II, beta | ACGAACATGGATATTGTTGAA | CACGCCATTGGGACTGTCAAA |
| NM_002737 | 5578 | PRKCA | protein kinase C, alpha | CGCAGTGGAATGAGTCCTTTA | TACAAGTTGCTTAACCAAGAA |
| NM_002738 | 5579 | PRKCB1 | protein kinase C, beta 1 | CAAGAGCTAAGTAGATGTGTA | CCGGATGAAACTGACCGATTT |
| NM_002739 | 5582 | PRKCG | protein kinase C, gamma | AACGGTGAAAGCCACGCTAAA | GACCTTTGTGTTCAACCTGAA |
| NM_002740 | 5584 | PRKCI | protein kinase C, iota | CACCCAATATCTTCTCTTGTA | TACCACGTTCTATGTCTGTAA |
| NM_002741 | 5585 | PRKCL1 | protein kinase C-like 1 | CAGGACAGTAAGACCAAGATT | CCGCAAGGAGCTGAAGCTGAA |
| NM_002742 | 5587 | PRKCM | protein kinase C, mu | CAGGAAGGCGATCTTATTGAA | CCTCAGTATATTTAAACTCAA |
| NM_002744 | 5590 | PRKCZ | protein kinase C, zeta | CGGAAGCATGACAGCATTAAA | GACCAAATTTACGCCATGAAA |
| NM_002745 | 5594 | MAPK1 | mitogen-activated protein kinase 1 | AACACTTGTCAAGAAGCGTTA | ATCATGGTAGTCACTAACATA |
| NM_002746 | 5595 | MAPK3 | mitogen-activated protein kinase 3 | CCCGTCTAATATATAAATATA | CTCCCTGACCCGTCTAATATA |
| NM_002747 | 5596 | MAPK4 | mitogen-activated protein kinase 4 | AAGGATCGTTGATCAGCATTA | CGCCTTAAATCTAATCAGCAA |
| NM_002748 | 5597 | MAPK6 | mitogen-activated protein kinase 6 | CAAGTTCAATTTGAAAGGAAA | CAGGCTTCCTGTTGAAATAAA |
| NM_002749 | 5598 | MAPK7 | mitogen-activated protein kinase 7 | CAGACCCACCTTTCAGCCTTA | TACGAGATCATCGAGACCATA |
| NM_002750 | 5599 | MAPK8 | mitogen-activated protein kinase 8 | AAGCCCAGTAATATAGTAGTA | TCGGGACTTAAAGCCCAGTAA |
| NM_002751 | 5600 | MAPK11 | mitogen-activated protein kinase 11 | CAGGATGGAGCTGATCCAGTA | CTGAGCGACGAGCACGTTCAA |
| NM_002752 | 5601 | MAPK9 | mitogen-activated protein kinase 9 | AAAGTCGATTTATGTGTATTA | ATCGTGAACTTGTCCTCTTAA |
| NM_002753 | 5602 | MAPK10 | mitogen-activated protein kinase 10 | CCGCATGTGTCTGTATTCATA | TCCGAGCACAATAAACTCAAA |
| NM_002754 | 5603 | MAPK13 | mitogen-activated protein kinase 13 | CCGGAGTGGCATGAAGCTGTA | CGGGATGAGCCTCATCCGGAA |

EP 2 295 543 A1

Table 2

| NM_002755 | 5604 | MAP2K1 | mitogen-activated protein kinase kinase 1 | CTGGAAGAATTCCTGAACAAA | CTGGATCAAGTCCTGAAGAAA |
|---|---|---|---|---|---|
| NM_002756 | 5606 | MAP2K3 | mitogen-activated protein kinase kinase 3 | ACGGATATCCTGCATGTCCAA | CCGGGCCACCGTGAACTCACA |
| NM_002757 | 5607 | MAP2K5 | mitogen-activated protein kinase kinase 5 | AAGACGTATGTTGGAACAAAT | CAAGACGTATGTTGGAACAAA |
| NM_002758 | 5608 | MAP2K6 | mitogen-activated protein kinase kinase 6 | AAGGCTTGCATTTCTATTGGA | TAGACCTATGATAAATAACCA |
| NM_002759 | 5610 | PRKR | protein kinase, interferon-inducible double stranded RNA dependent | ACGGAAAGACTTACGTTATTA | CGGAAAGACTTACGTTATTAA |
| NM_002760 | 5616 | PRKY | protein kinase, Y-linked | CCAGAGTTCGCCTACAGATAA | TTGGGATACAGTAAGAGAATA |
| NM_002822 | 5756 | PTK9 | PTK9 protein tyrosine kinase 9 | ACCGGCATCCAAGCAAGTGAA | TCCAAGCAAGTGAAGATGTTA |
| NM_002880 | 5894 | RAF1 | v-raf-1 murine leukemia viral oncogene homolog 1 | CAGATCTTAGTAAGCTATATA | TGGGAAATAGAAGCCAGTGAA |
| NM_002929 | 6011 | GRK1 | G protein-coupled receptor kinase 1 | CCAGATGAAGGCGACCGGCAA | CTGGAAAGACATCGAGGACTA |
| NM_002944 | 6098 | ROS1 | v-ros UR2 sarcoma virus oncogene homolog 1 (avian) | AAGGTAATTGCTCTAACTTTA | ACCGAGAAGGGTTAAACTATA |
| NM_002953 | 6195 | RPS6KA1 | ribosomal protein S6 kinase, 90kDa, polypeptide 1 | CCCAACATCATCACTCTGAAA | TGCCACGTACTCCGCACTCAA |
| NM_002958 | 6259 | RYK | RYK receptor-like tyrosine kinase | CGGTCTTGATGCAGAACTTTA | CTGATCGGTCTTGATGCAGAA |
| NM_002969 | 6300 | MAPK12 | mitogen-activated protein kinase 12 | CTGGACGTATTCACTCCTGAT | TGGAAGCGTGTTACTTACAAA |
| NM_003010 | 6416 | MAP2K4 | mitogen-activated protein kinase kinase 4 | AGGGAGAATGGTGCTGTTTAA | AGGGTGTATAGTGTTCACAAA |
| NM_003137 | 6732 | SRPK1 | SFRS protein kinase 1 | CAGACTCTTGTACACCTATAA | TACCATGTGATCCGAAAGTTA |
| NM_003157 | 6787 | NEK4 | NIMA (never in mitosis gene a)-related kinase 4 | CACAATCAGTAGCGTAAATAT | CAGACATATATAATGGGTGAA |
| NM_003159 | 6792 | CDKL5 | cyclin-dependent kinase-like 5 | AAGATAGACGCTTCATGTTAA | AAGGCAATAATGCTAATTACA |
| NM_003160 | 6795 | AURKC | aurora kinase C | AAGATGTAAGATGCTAATTAA | CCGGGTGTACCTGATTCTGGA |
| NM_003161 | 6198 | RPS6KB1 | ribosomal protein S6 kinase, 70kDa, polypeptide 1 | AAGAAGGGTATCCTTTCATTA | CAGAGAGTCAATGTCATTACA |
| NM_003177 | 6850 | SYK | spleen tyrosine kinase | CCCGCTCTTAAAGATGAGTTA | TCCGGAATGCATCAACTACTA |

EP 2 295 543 A1

Table 2

| | | | | | |
|---|---|---|---|---|---|
| NM_003188 | 6885 | MAP3K7 | mitogen-activated protein kinase kinase kinase 7 | CAAGAATATATGAAAGTTCAA | CCCGTGTGAACCATCCTAATA |
| NM_003215 | 7006 | TEC | tec protein tyrosine kinase | CAGTACAAAGTCGCAATCAAA | CCGCAGCTAATCAGAATCAGA |
| NM_003242 | 7048 | TGFBR2 | transforming growth factor, beta receptor II (70/80kDa) | CTCCAATATCCTCGTGAAGAA | TCGGTTAATAACGACATGATA |
| NM_003258 | 7083 | TK1 | thymidine kinase 1, soluble | CGGGCCGATGTTCTCAGGAAA | TCGGCTCTGCTACTTCAAGAA |
| NM_003318 | 7272 | TTK | TTK protein kinase | CAGCAATACCTTGGATGATTA | TCCGACTTTATGATTATGAAA |
| NM_003328 | 7294 | TXK | TXK tyrosine kinase | CAGCTGGGTTTAGCTACGAAA | CCCGGTGACATTCTATTTCCA |
| NM_003331 | 7297 | TYK2 | tyrosine kinase 2 | CACCATCTGGTAATAAACTCA | CTGATGCTATATTTCCGCATA |
| NM_003332 | 7305 | TYROBP | TYRO protein tyrosine kinase binding protein | CCGAATCATGACAGTCAGCAA | CTGGATCCAGCCATTCCTGAA |
| NM_003384 | 7443 | VRK1 | vaccinia related kinase 1 | CAGGTTATACTCCTTAAGTTA | CCAGGTGTACTTGGTAGATTA |
| NM_003390 | 7465 | WEE1 | WEE1 homolog (S. pombe) | CAGGGTAGATTACCTCGGATA | TTGACTAATAATACTGGCTAA |
| NM_003488 | 8165 | AKAP1 | A kinase (PRKA) anchor protein 1 | AGCGCTGAACTTGATTGGGAA | CACGCAGAGATGACAGTACAA |
| NM_003551 | 8382 | NME5 | non-metastatic cells 5, protein expressed in (nucleoside-diphosphate kinase) | ATGATATTAGCTAGACATAAA | CATGATATTAGCTAGACATAA |
| NM_003557 | 8394 | PIP5K1A | phosphatidylinositol-4-phosphate 5-kinase, type I, alpha | CAGCCTCTTGATGTCAATCCA | CCCTGCCTTGATAATATGTTA |
| NM_003558 | 8395 | PIP5K1B | phosphatidylinositol-4-phosphate 5-kinase, type I, beta | AAGGCTCAACGTATAAGCGAA | CAGGCCTACACTCTATTCAAA |
| NM_003559 | 8396 | PIP5K2B | phosphatidylinositol-4-phosphate 5-kinase, type II, beta | CACGCCATACGATACAAAGAA | CCGCTTTAAGTTTAAGGAGTA |
| NM_003565 | 8408 | ULK1 | unc-51-like kinase 1 (C. elegans) | CGCGCGGTACCTCCAGAGCAA | TGCCCTTTGCGTTATATTGTA |
| NM_003576 | 8428 | STK24 | serine/threonine kinase 24 (STE20 homolog, yeast) | CCCACCGACGTTGGAAGGAAA | CTGGAGGGAGCACGAACCTAA |
| NM_003582 | 8444 | DYRK3 | dual-specificity tyrosine-(Y)-phosphorylation regulated kinase 3 | AGCCAATAAGCTTAAAGCTAA | TCGACAGTACGTGGCCCTAAA |
| NM_003583 | 8445 | DYRK2 | dual-specificity tyrosine-(Y)-phosphorylation regulated kinase 2 | TAGAAGCGGTATTAAAGTAAT | TGCACAGAGATTATTGTATTA |
| NM_003600 | 6790 | STK6 | serine/threonine kinase 6 | CACCTTCGGCATCCTAATATT | CACGTGCTCTACCTCCATTTA |

EP 2 295 543 A1

Table 2

| | | | | | |
|---|---|---|---|---|---|
| NM_003607 | 8476 | CDC42BPA | CDC42 binding protein kinase alpha (DMPK-like) | CAGACTTTCCGTAGCAGCTTA | CAGATAATAGTCGGAAACAAA |
| NM_003618 | 8491 | MAP4K3 | mitogen-activated protein kinase kinase kinase kinase 3 | GAGGTGGTTCTTTACAGGATA | TCGAGCTGTTGGATAAAGTAA |
| NM_003629 | 8503 | PIK3R3 | phosphoinositide-3-kinase, regulatory subunit, polypeptide 3 (p55, gamma) | AGGGAGGCAATAATAAGTTAA | GAGGATATCAATCGAGTACAA |
| NM_003639 | 8517 | IKBKG | inhibitor of kappa light polypeptide gene enhancer in B-cells, kinase gamma | CTCCTCTAGTTCAGAGACATA | TTCGGAAATGCCTCACATATA |
| NM_003640 | 8518 | IKBKAP | inhibitor of kappa light polypeptide gene enhancer in B-cells, kinase complex-associated protein | CAGCGGTTTACTATAGACAAA | GCGGTTTACTATAGACAAATA |
| NM_003646 | 8525 | DGKZ | diacylglycerol kinase, zeta 104kDa | CAAGAGGAACGACTTCTGTAA | CTGGAGCGAGTCAGCGACATA |
| NM_003647 | 8526 | DGKE | diacylglycerol kinase, epsilon 64kDa | ATCGATGGAAAGTTCAAGTAA | CTGGAAGTCGTTGGAGTATAT |
| NM_003648 | 8527 | DGKD | diacylglycerol kinase, delta 130kDa | CAGCAGATTCTCTTCTATGAA | CCGCCTCGTGACCAAGTTTAA |
| NM_003656 | 8536 | CAMK1 | calcium/calmodulin-dependent protein kinase I | CAGGTGCTGGATGCTGTGAAA | CTGCATGACCTGGGCATTGTA |
| NM_003668 | 8550 | MAPKAPK5 | mitogen-activated protein kinase-activated protein kinase 5 | ACCGACCTTTCTGATTAGTAA | CCGACCTTTCTGATTAGTAAA |
| NM_003674 | 8558 | CDK10 | cyclin-dependent kinase (CDC2-like) 10 | CCGTCTGAAGTGTATTCGTAA | CTGATGCTGAGCGAAGCTATA |
| NM_003681 | 85006 | C21orf124 | chromosome 21 open reading frame 124 | CACAGGTTATACGAGGGACAA | GAGGCTGAACAACATGAATAA |
| NM_003682 | 8567 | MADD | MAP-kinase activating death domain | CAGACCCACTACTATAGTAAA | CAGGAGGGCGTTAGTGGATCA |
| NM_003684 | 8569 | MKNK1 | MAP kinase-interacting serine/threonine kinase 1 | CCAGCAAAGATGATACCTTAA | TAGATAGTGCTCTGTGCCTAA |
| NM_003688 | 8573 | CASK | calcium/calmodulin-dependent serine protein kinase (MAGUK family) | AACCAATGGGAATCACTTTAA | AAGGACGACAGATCTATGTAA |
| NM_003690 | 8575 | PRKRA | protein kinase, interferon-inducible double stranded RNA dependent activator | ATGATTGATTGTTAAATTTCA | TAAGTATGATTGATTGTTAAA |
| NM_003691 | 8576 | STK16 | serine/threonine kinase 16 | CACATAGTGTTCTGACTCCAA | CAGGAGAATGTGTAACAAGAA |
| NM_003718 | 8621 | CDC2L5 | cell division cycle 2-like 5 (cholinesterase-related cell division controller) | AACGACGTAGTTTCATTGGAA | TCAGAGTATTATCAATATGAA |

Table 2

| NM_003804 | 8737 | RIPK1 | receptor (TNFRSF)-interacting serine-threonine kinase 1 | CCGACATTTCCTGGCATTGAA | TACCACTAGTCTGACGGATAA |
|---|---|---|---|---|---|
| NM_003821 | 8767 | RIPK2 | receptor-interacting serine-threonine kinase 2 | ACGTATGATCTCTCTAATAGA | CACAAGGACATCGACCTGTTA |
| NM_003831 | 8780 | RIOK3 | RIO kinase 3 (yeast) | ATGCGGCAGTTATATCATGAA | CTGGCTGTATTAGTACAGGAA |
| NM_003845 | 8798 | DYRK4 | dual-specificity tyrosine-(Y)-phosphorylation regulated kinase 4 | CTCCATATGAACAAAGTGAAA | CTGCATCACGGCGGAGTTGTA |
| NM_003885 | 8851 | CDK5R1 | cyclin-dependent kinase 5, regulatory subunit 1 (p35) | CCGGAAGGCCACGCTGTTTGA | TGAGCTGGTTTGACTCATTAA |
| NM_003886 | 8852 | AKAP4 | A kinase (PRKA) anchor protein 4 | GTCGGTGAACACATTCTCAAA | TACAGTGTCTATGCCGATCAA |
| NM_003936 | 8941 | CDK5R2 | cyclin-dependent kinase 5, regulatory subunit 2 (p39) | CAGCAAGAAGGTGACACCCAA | CCGCGAGAACCTTCTCCGCAA |
| NM_003942 | 8986 | RPS6KA4 | ribosomal protein S6 kinase, 90kDa, polypeptide 4 | CAGGCTGTGCCTTTGACTTTA | CGCCACCTTCATGGCATTCAA |
| NM_003948 | 8999 | CDKL2 | cyclin-dependent kinase-like 2 (CDC2-related kinase) | ATGATGTGTTTAGGTAATCTA | TTCTACGGACTTTAAATTAAA |
| NM_003952 | 6199 | RPS6KB2 | ribosomal protein S6 kinase, 70kDa, polypeptide 2 | ACCGCAGAGAACCGGAAGAAA | CGGGCTGAGCGGAACATTCTA |
| NM_003954 | 9020 | MAP3K14 | mitogen-activated protein kinase kinase kinase 14 | CACATGCATGTGACTCCTCAA | TACCTAGTGCATGCTCTGCAA |
| NM_003957 | 9024 | STK29 | serine/threonine kinase 29 | CCCGACTGCCAGAGTCTGCTA | GCGAGCTACTGTAAACTTTAA |
| NM_003985 | 8711 | TNK1 | tyrosine kinase, non-receptor, 1 | AAGGATCTAGTCTGCCCACTA | ATCGGTCATGATGAACTTGGA |
| NM_004064 | 1027 | CDKN1B | cyclin-dependent kinase inhibitor 1B (p27, Kip1) | AAAGCGTTGGATGTAGCATTA | ACCGACGATTCTTCTACTCAA |
| NM_004071 | 1195 | CLK1 | CDC-like kinase 1 | AAAGCCGGTATCAGAACCATA | AACGTGATGAACGCACCTTAA |
| NM_004073 | 1263 | PLK3 | polo-like kinase 3 (Drosophila) | CAGAAAGACTGTGCACTACAA | CTGCATCAAGCAGGTTCACTA |
| NM_004119 | 2322 | FLT3 | fms-related tyrosine kinase 3 | CCGGCTTGAGTGAATTGTGTA | TACGTTGATTTCAGAGAATAT |
| NM_004157 | 5576 | PRKAR2A | protein kinase, cAMP-dependent, regulatory, type II, alpha | AACGGCATGTCTCTCCAACAA | AACTTGAAAGTCAGCACTAAA |
| NM_004196 | 8814 | CDKL1 | cyclin-dependent kinase-like 1 (CDC2-related kinase) | CTGGACCGAGTGACTACTATA | TCGGGAAATCCGAATGCTCAA |
| NM_004197 | 8859 | STK19 | serine/threonine kinase 19 | CCCGGAGACCTTTGGAGTTAA | CTGTTATTACTCTGTCTTGAA |

Table 2

| NM_004217 | 9212 | AURKB | aurora kinase B | AACGCGGCACTTCACAATTGA | ACGCGGCACTTCACAATTGAT |
|---|---|---|---|---|---|
| NM_004226 | 9262 | STK17B | serine/threonine kinase 17b (apoptosis-inducing) | AGCATATATGTTGTTAACTCA | CATGTTAATGAAATAATTCAA |
| NM_004274 | 9472 | AKAP6 | A kinase (PRKA) anchor protein 6 | AAGCATGTGGATGTACATCTA | CACGTTTGTCACTGCCGTTTA |
| NM_004302 | 91 | ACVR1B | activin A receptor, type IB | CCGTTCTTACGTCGCCATAAA | CTGTGATTACCTCTCAATCTA |
| NM_004304 | 238 | ALK | anaplastic lymphoma kinase (Ki-1) | CACCTACGTATTTAAGATGAA | CTGGGCCTGTATACCGGATAA |
| NM_004327 | 613 | BCR | breakpoint cluster region | ACGGCAGTCCATGACGGTGAA | CAGCATTCCGCTGACCATCAA |
| NM_004329 | 657 | BMPR1A | bone morphogenetic protein receptor, type IA | CAGCTACGCCGGACAATAGAA | GCGGCAGACATTAAAGGTACA |
| NM_004333 | 673 | BRAF | v-raf murine sarcoma viral oncogene homolog B1 | ACACGACATGTGAATATCCTA | TTGCTTATATGTTAAATTGAA |
| NM_004336 | 699 | BUB1 | BUB1 budding uninhibited by benzimidazoles 1 homolog (yeast) | CAGCTTGTGATAAAGAGTCAA | TGGCACTTACATGAAAGTGAA |
| NM_004383 | 1445 | CSK | c-src tyrosine kinase | CCGGTACAGAATGTATTGCCA | TACGCGCCTCATTAAACCAAA |
| NM_004384 | 1456 | CSNK1G3 | casein kinase 1, gamma 3 | CAACATATCTTCGTTATGTAA | TACCGCAAACTTGATATGGAA |
| NM_004409 | 1760 | DMPK | dystrophia myotonica-protein kinase | AACCAGAACTTCGCCAGTCAA | GAGCCTGAGGGCTAAATTTAA |
| NM_004431 | 1969 | EPHA2 | EphA2 | CAGCGCCAAGTAAACAGGGTA | TCGGACAGACATATAGGGATAT |
| NM_004438 | 2043 | EPHA4 | EphA4 | CCCGCGAATGAAGTTACCTTA | CCGCCAGGACATTTCCTATAA |
| NM_004439 | 2044 | EPHA5 | EphA5 | ACCAGTTGGATCTCCAATGAA | CCCGGCAGTATGTGTCTGTAA |
| NM_004440 | 2045 | EPHA7 | EphA7 | ACCAGTCATGATAGTAATAGA | CAGGCTGCGAAGGAAGTACTA |
| NM_004441 | 2047 | EPHB1 | EphB1 | ATGGCCCTGGATTATCTACTA | GCGGGATGATGTGACCTACAA |
| NM_004443 | 2049 | EPHB3 | EphB3 | CCGCAGCTGACCGCCAGATTA | GCGGGTCTACGTGGAGCTCAA |
| NM_004444 | 2050 | EPHB4 | EphB4 | CCCAGCCAATAGCCACTCTAA | TCGGACAAACACGGACAGTAT |
| NM_004445 | 2051 | EPHB6 | EphB6 | AACCGCCAATCTCTAGATCAA | CTGGAGCTTTGGGATACTCAT |
| NM_004448 | 2064 | ERBB2 | v-erb-b2 erythroblastic leukemia viral oncogene homolog 2, neuro/glioblastoma derived oncogene homolog (avian) | AACAAAGAAATCTTAGACGAA | CACGTTTGAGTCCATGCCCAA |
| NM_004517 | 3611 | ILK | integrin-linked kinase | AAGGAAGAGCAGGGACTTCAA | TAGCCGTAGTGTAATGATTGA |
| NM_004560 | 4920 | ROR2 | receptor tyrosine kinase-like orphan receptor 2 | CCGGTTTGGGAAAGTCTACAA | CTGGTGCTTTACGCAGAATAA |

Table 2

| | | | | | |
|---|---|---|---|---|---|
| NM_004566 | 5209 | PFKFB3 | 6-phosphofructo-2-kinase/fructose-2,6-biphosphatase 3 | CAGGGACTTGTCGCTGATCAA | CTCCAATATCATGGAAGTTAA |
| NM_004567 | 5210 | PFKFB4 | 6-phosphofructo-2-kinase/fructose-2,6-biphosphatase 4 | ACGGAGAGCGACCATCTTTAA | CAGAAAGTGTCTGGACTTGTA |
| NM_004570 | 5288 | PIK3C2G | phosphoinositide-3-kinase, class 2, gamma polypeptide | CCAGATCAAGAAATTCGTAAA | CCCGTAGAAATGATAACTCCA |
| NM_004579 | 5871 | MAP4K2 | mitogen-activated protein kinase kinase kinase kinase 2 | CTGGCTCTACTGCGTGAACAA | TCGGTCAGCCTCAGAATTCCA |
| NM_004586 | 6197 | RPS6KA3 | ribosomal protein S6 kinase, 90kDa, polypeptide 3 | AGCGCTGAGAATGGACAGCAA | TCCAAACATTATCACTCTAAA |
| NM_004612 | 7046 | TGFBR1 | transforming growth factor, beta receptor I (activin A receptor type II-like kinase, 53kDa) | TCCATTGGTGGAATTCATGAA | TGGGATTGTACTATACCAGTA |
| NM_004614 | 7084 | TK2 | thymidine kinase 2, mitochondrial | ATGCCAGAAGTGGACTATGTA | CGGGATCGAATATTAACTCCA |
| NM_004635 | 7867 | MAPKAPK3 | mitogen-activated protein kinase-activated protein kinase 3 | CAGGCTTTCACTGAGAGAGAA | CCAGATAGTAATAAACACCAT |
| NM_004672 | 9064 | MAP3K6 | mitogen-activated protein kinase kinase kinase 6 | CACCATCCAAATGCTGTTGAA | TCAGAGGAGCTGAGTAATGAA |
| NM_004690 | 9113 | LATS1 | LATS, large tumor suppressor, homolog 1 (Drosophila) | CCCATGAATCCTCCTAATCAA | CGCGATCTAGTATATGTTTAA |
| NM_004705 | 5612 | PRKRIR | protein-kinase, interferon-inducible double stranded RNA dependent inhibitor, repressor of (P58 repressor) | AAGGTGTATGTAGACCACTTA | CTGGACAGTTACAAGAAATTA |
| NM_004712 | 9146 | HGS | hepatocyte growth factor-regulated tyrosine kinase substrate | CCGGAACGAGCCCAAGTACAA | GCACGTCTTTCCAGAATTCAA |
| NM_004714 | 9149 | DYRK1B | dual-specificity tyrosine-(Y)-phosphorylation regulated kinase 1B | CACGGAGATGAAGTACTATAT | CCGGACCTACCGCTACAGCAA |
| NM_004721 | 9175 | MAP3K13 | mitogen-activated protein kinase kinase kinase 13 | CAGACTCAATATGCACGGACA | GCGAATAATTTATACATGGAA |
| NM_004734 | 9201 | DCAMKL1 | doublecortin and CaM kinase-like 1 | CAGGTTTATAACTTCGACACA | CCGGAAGTGATAACACGCAAA |
| NM_004759 | 9261 | MAPKAPK2 | mitogen-activated protein kinase-activated protein kinase 2 | CGCCATCATCGATGACTACAA | CTACGAGCAGATCAAGATAAA |

EP 2 295 543 A1

Table 2

| NM_004760 | 9263 | STK17A | serine/threonine kinase 17a (apoptosis-inducing) | AACCAGGATATTTAACAGGTA | TCCATTGTAACCGAAGAGTTA |
|---|---|---|---|---|---|
| NM_004783 | 9344 | TAO1 | thousand and one amino acid protein kinase | CACGCCCAAAGCTCAGCACAA | GCCCAAGAGCCTCAAATCTAA |
| NM_004834 | 9448 | MAP4K4 | mitogen-activated protein kinase kinase kinase kinase 4 | AGGCAAGATCCTACCCGGAAA | TCGGAGCTGCACCGAGGGCAA |
| NM_004836 | 9451 | EIF2AK3 | eukaryotic translation initiation factor 2-alpha kinase 3 | CACAAACTGTATAACGGTTTA | CGGCAGGTCATTAGTAATTAT |
| NM_004842 | 9465 | AKAP7 | A kinase (PRKA) anchor protein 7 | AACGATGGGTCTGAAGTCCAA | CCCGATGACGCTGAACTAGTA |
| NM_004850 | 9475 | ROCK2 | Rho-associated, coiled-coil containing protein kinase 2 | AAGCTACATATGGAGCTTAAA | ATGCACTTGTATAAAGCCATA |
| NM_004857 | 9495 | AKAP5 | A kinase (PRKA) anchor protein 5 | AGGGCTTTCATCAAGAAATTA | ATCAGGTTGATCTTTAAATAA |
| NM_004936 | 1030 | CDKN2B | cyclin-dependent kinase inhibitor 2B (p15, inhibits CDK4) | CTGCTTACTTATGCCATAGAA | GAGAGCAATTGTAACGGTTAA |
| NM_004938 | 1612 | DAPK1 | death-associated protein kinase 1 | AAGCATGTAATGTTAATGTTA | CGGCTATTACTCTGTGGCCAA |
| NM_004954 | 2011 | MARK2 | MAP/microtubule affinity-regulating kinase 2 | CACCTCTAATTCTTACTCTAA | CCACCTCTAATTCTTACTCTA |
| NM_004972 | 3717 | JAK2 | Janus kinase 2 (a protein tyrosine kinase) | AGCCATCATACGAGATCTTAA | CTGCCTTACGATGACAGAAAT |
| NM_005009 | 4833 | NME4 | non-metastatic cells 4, protein expressed in | ACCAACTACCTCCGTCAGCAA | CAGCACATGGGTGGTACACTA |
| NM_005012 | 4919 | ROR1 | receptor tyrosine kinase-like orphan receptor 1 | CCCAGTGAGTAATCTCAGTAA | CCGTACTGCGATGAAACTTCA |
| NM_005026 | 5293 | PIK3CD | phosphoinositide-3-kinase, catalytic, delta polypeptide | CCGGTCACGCATGAAGGCAAA | CGCCGTGATCGAGAAAGCCAA |
| NM_005027 | 5296 | PIK3R2 | phosphoinositide-3-kinase, regulatory subunit, polypeptide 2 (p85 beta) | CCGCGAGTATGACCAGCTTTA | TTGGTACGTGGGCAAGATCAA |
| NM_005028 | 5305 | PIP5K2A | phosphatidylinositol-4-phosphate 5-kinase, type II, alpha | ATGGAATTAAGTGCCATGAAA | CTGCCCGATGGTCTTCCGTAA |
| NM_005030 | 5347 | PLK1 | polo-like kinase 1 (Drosophila) | CCGGATCAAGAAGAATGAATA | CGCGGGCAAGATTGTGCCTAA |
| NM_005044 | 5613 | PRKX | protein kinase, X-linked | CGGATGGGATTCACTTAAGAA | TTGGAATACTCTAAGAGAATA |
| NM_005100 | 9590 | AKAP12 | A kinase (PRKA) anchor protein (gravin) 12 | ACGGATGTAGTGTTGAAAGTA | CCCGAAATAATCGAACAGATT |

EP 2 295 543 A1

Table 2

| NM_005108 | 9942 | XYLB | xylulokinase homolog (H. influenzae) | CTGAAATTATTGGACGTCATA | TCAGGACTCCAGCTAAATCAA |
|---|---|---|---|---|---|
| NM_005109 | 9943 | OSR1 | oxidative-stress responsive 1 | CTGGAGTAGGGACTAACTATA | TAGGGACTAACTATAGCACAA |
| NM_005157 | 25 | ABL1 | v-abl Abelson murine leukemia viral oncogene homolog 1 | ACGCACGGACATCACCATGAA | CCAGTGGAGATAACACTCTAA |
| NM_005158 | 27 | ABL2 | v-abl Abelson murine leukemia viral oncogene homolog 2 (arg, Abelson-related gene) | ATCAAGCATCCTAATCTGGTA | CCCAAGTGTAATAAGCCTACA |
| NM_005160 | 157 | ADRBK2 | adrenergic, beta, receptor kinase 2 | AAGATGTTCAGTGTTGGGTAA | CAAGTGTATGGGATTAACTAA |
| NM_005163 | 207 | AKT1 | v-akt murine thymoma viral oncogene homolog 1 | ACGCTACTTCCTCCTCAAGAA | CACGCTTGGTCCCGAGGCCAA |
| NM_005184 | 808 | CALM3 | calmodulin 3 (phosphorylase kinase, delta) | CACCAATTGATTGACTGAGAA | CCGCAGAGCTGCGTCACGTAA |
| NM_005204 | 1326 | MAP3K8 | mitogen-activated protein kinase kinase kinase 8 | AAGAAAGTGATCCATCATGAT | AGAGATTGATTTATTAATTAA |
| NM_005228 | 1956 | EGFR | epidermal growth factor receptor (erythroblastic leukemia viral (v-erb-b) oncogene homolog, avian) | CCCATCCAATTTATCAAGGAA | CCCGTCGCTATCAAGGAATTA |
| NM_005232 | 2041 | EPHA1 | EphA1 | CAGATGGGATCCCGTATCGAA | TACCTCAGTAATCACAATTAT |
| NM_005233 | 2042 | EPHA3 | EphA3 | TCGGATATGATTGTTTCTCAA | TTGGATAGTTTCCTACGTAAA |
| NM_005235 | 2066 | ERBB4 | v-erb-a erythroblastic leukemia viral oncogene homolog 4 (avian) | CTACGTGTTAGTGGCTCTTAA | TCGGGATTTGGCAGCCCGTAA |
| NM_005246 | 2241 | FER | fer (fps/fes related) tyrosine kinase (phosphoprotein NCP94) | CAGAACAACTTAGTAGGATAA | CAGATAGATCCTAGTACAGAA |
| NM_005248 | 2268 | FGR | Gardner-Rasheed feline sarcoma viral (v-fgr) oncogene homolog | CACGTGGAACGGCAGCACTAA | CAGACCTTGTCTAGTTATTTA |
| NM_005255 | 2580 | GAK | cyclin G associated kinase | ACGCGTGTGACATTCAAGAAA | CCCGTGGTTGTCTGTACAGAA |
| NM_005308 | 2869 | GRK5 | G protein-coupled receptor kinase 5 | AGCGTCATAACTAGAACTGAA | CAGGAACAACGCGGTAGGCAA |
| NM_005356 | 3932 | LCK | lymphocyte-specific protein tyrosine kinase | AAGGGCCAGGACTTTATCTAA | CGCCATTACACCAATGCTTCA |
| NM_005372 | 4342 | MOS | v-mos Moloney murine sarcoma viral oncogene homolog | CGCGAACATCTTGATCAGTGA | CTCGGTGTACAAGGCGACTTA |
| NM_005391 | 5165 | PDK3 | pyruvate dehydrogenase kinase, isoenzyme 3 | AACGTTGTAGGTTGAAACTGA | TTCATTAGCACTAACATCCAA |

EP 2 295 543 A1

Table 2

| NM_ | Gene ID | Symbol | Description | Sequence |
|---|---|---|---|---|
| NM_005399 | 5565 | PRKAB2 | protein kinase, AMP-activated, beta 2 non-catalytic subunit | ACCCAAAGTACTACTCATTTATCCAATATTAGTTGGAGTTAA |
| NM_005400 | 5581 | PRKCE | protein kinase C, epsilon | CACGGAAACACCCGTACCTTACCCGACCATGGTAGTGTTCAA |
| NM_005406 | 6093 | ROCK1 | Rho-associated, coiled-coil containing protein kinase 1 | AACGGTTAGAACAAGAGAGGTAACAAGCTCGAATTACATCTTTA |
| NM_005417 | 6714 | SRC | v-src sarcoma (Schmidt-Ruppin A-2) viral oncogene homolog (avian) | AAGCAGTGCCTGCCTATGAAACGGCTTGTGGGTGATGTTTGA |
| NM_005424 | 7075 | TIE | tyrosine kinase with immunoglobulin and epidermal growth factor homology domains | CCCAGTGAGAACGTGACGTTAGCGCTTCAAGGTCAATGTGAA |
| NM_005433 | 7525 | YES1 | v-yes-1 Yamaguchi sarcoma viral oncogene homolog 1 | CCAGCCTACATTCACTTCTAAGAGGCTCCTGCTTATTTATAA |
| NM_005456 | 9479 | MAPK8IP1 | mitogen-activated protein kinase 8 interacting protein 1 | CTGGAGGAGTTTGAGGATGAATGGCATCAGCTTACAGTGCAA |
| NM_005476 | 10020 | GNE | glucosamine (UDP-N-acetyl)-2-epimerase/N-acetylmannosamine kinase | ATGGAAATACATATCGAATGACCCGATCATGTTTGGCATTAA |
| NM_005546 | 3702 | ITK | IL2-inducible T-cell kinase | CAGGACTTTAGTAGAGACTGACTGGTGAGTTAAGTAAGATTA |
| NM_005569 | 3985 | LIMK2 | LIM domain kinase 2 | CAGGAGGAATCTGTTACTCGACAGGGTGAACCTGCCTGTGAA |
| NM_005592 | 4593 | MUSK | muscle, skeletal, receptor tyrosine kinase | ACGGCCTGGAATGAACTGAAACCCACCAATGACGTCCTCAAA |
| NM_005607 | 5747 | PTK2 | PTK2 protein tyrosine kinase 2 | CACCTGGGTACTGGTATGGAACCGGTCGAATGATAAGGTGTA |
| NM_005627 | 6446 | SGK | serum/glucocorticoid regulated kinase | CACAGCTGAAATGTACGACAACTGGGCTGTGATGACGAATAT |
| NM_005734 | 10114 | HIPK3 | homeodomain interacting protein kinase 3 | CACGTTTGGCCAGGTAGTTAACTCATTCTGGTTGGAGATTAA |
| NM_005751 | 10142 | AKAP9 | A kinase (PRKA) anchor protein (yotiao) 9 | CAGCCTATCAGTGAACATCAACAGCTTCAAAGGGATATACAA |
| NM_005781 | 10188 | ACK1 | activated Cdc42-associated kinase 1 | ACGCAAGTCGTGGATGAGTAACGGCAGTCAGATCCTGCATAA |
| NM_005793 | 10201 | NME6 | non-metastatic cells 6, protein expressed in (nucleoside-diphosphate kinase) | CAGACTTCTCCTAGACATCTACAGGATCTAGCCTTCTATCTA |
| NM_005813 | 23683 | PRKCN | protein kinase C, nu | CACGATATGTCAGTACTGCAACGGGAGAGTGTTACCATTGAA |

Table 2

| Accession | ID | Symbol | Description | | |
|---|---|---|---|---|---|
| NM_005858 | 10270 | AKAP8 | A kinase (PRKA) anchor protein 8 | CAAGGTTAGCACGCAACAGAA | GAGGCCGGTAGTGATCCTCAA |
| NM_005876 | 10290 | APEG1 | aortic preferentially expressed protein 1 | AACACCTATTTCTTAACTCAA | CAGCTGAATAACAAGCCCAA |
| NM_005881 | 10295 | BCKDK | branched chain alpha-ketoacid dehydrogenase kinase | AACGCCCACCATGATGCTCTA | CAGAACTTGGAGAGCAGGGAA |
| NM_005884 | 10298 | PAK4 | p21(CDKN1A)-activated kinase 4 | CGGCGCCGAGCCGATGAGTAA | CTGGACAACTTCATCAAGATT |
| NM_005906 | 4117 | MAK | male germ cell-associated kinase | CACAGTGTTGATATATGGGTA | CCGGATATAATCGATCAGGTT |
| NM_005922 | 4216 | MAP3K4 | mitogen-activated protein kinase kinase kinase 4 | CACCAATCCCTGAAAGATTAA | CTCAAGCATCGCATAGTTTAA |
| NM_005923 | 4217 | MAP3K5 | mitogen-activated protein kinase kinase kinase 5 | CAGCGAGTAGATAATATCGAA | CCGGGAATCTATACTCAATGA |
| NM_005965 | 4638 | MYLK | myosin, light polypeptide kinase | AACCTGAAATCCGCTAGCAAA | CAGCATCCATGGCTAATGAAA |
| NM_005975 | 5753 | PTK6 | PTK6 protein tyrosine kinase 6 | CCGGGTCCAGGTGGCCATTAA | CGGGTTAGCCAGGCTTATCAA |
| NM_005983 | 6502 | SKP2 | S-phase kinase-associated protein 2 (p45) | AAGTGATAGTGTCATGCTAAA | ACCCTTCAACTGTTAAAGGAA |
| NM_005990 | 6793 | STK10 | serine/threonine kinase 10 | CACGGAATTAGAGAACCTGGA | CAGCTCAAACTGATGAAGAAA |
| NM_006035 | 9578 | CDC42BPB | CDC42 binding protein kinase beta (DMPK-like) | CGCCGAGATATTCCATGTATA | TAGGACCTTAAGAGAATAGTA |
| NM_006116 | 10454 | MAP3K7IP1 | mitogen-activated protein kinase kinase kinase 7 interacting protein 1 | CACCTTGGACAAGCCACACAA | CTGCAGGTGAGAGGATTTAAA |
| NM_006176 | 4900 | NRGN | neurogranin (protein kinase C substrate, RC3) | AAGCCGGACGACGACATTCTA | TGGGTTGGAATGTGAACAATA |
| NM_006180 | 4915 | NTRK2 | neurotrophic tyrosine kinase, receptor, type 2 | ACCACGAACAGAAGTAATGAA | CTGACGAGTTTGTCTAGGAAA |
| NM_006182 | 4921 | DDR2 | discoidin domain receptor family, member 2 | ACGCACTGTCAGTTACACCAA | CCGGTTCATTCCAGTCACCGA |
| NM_006201 | 5127 | PCTK1 | PCTAIRE protein kinase 1 | CACCATAATCGCTGTATGAAA | CACGCCAACATCGTTACGCTA |
| NM_006206 | 5156 | PDGFRA | platelet-derived growth factor receptor, alpha polypeptide | ACGCAGGAAGCCTACTATTTA | CGCAGGAAGCCTACTATTTAA |
| NM_006212 | 5208 | PFKFB2 | 6-phosphofructo-2-kinase/fructose-2,6-biphosphatase 2 | CCAGAGCAAGATAGTCTACTA | CCGTGACAAGCCAACTAACAA |
| NM_006213 | 5260 | PHKG1 | phosphorylase kinase, gamma 1 (muscle) | CAAGAGCCTGCAAGCACTTAA | CCGGGAGATCGTCATCCGAGA |

Table 2

| | | | | | |
|---|---|---|---|---|---|
| NM_006218 | 5290 | PIK3CA | phosphoinositide-3-kinase, catalytic, alpha polypeptide | CTGAGTCAGTATAAGTATATA | TGCGAAATTCTCACACTATTA |
| NM_006219 | 5291 | PIK3CB | phosphoinositide-3-kinase, catalytic, beta polypeptide | CCCTTCGATAAGATTATTGAA | TCGGGAAGCTACCATTTCTTA |
| NM_006251 | 5562 | PRKAA1 | protein kinase, AMP-activated, alpha 1 catalytic subunit | CCCACGATATTCTGTACACAA | TCGGGATCAGTTAGCAACTAT |
| NM_006252 | 5563 | PRKAA2 | protein kinase, AMP-activated, alpha 2 catalytic subunit | ACGACTAAGCCCAAATCTTTA | CCGATTTCGGATTATCTAATA |
| NM_006253 | 5564 | PRKAB1 | protein kinase, AMP-activated, beta 1 non-catalytic subunit | CACGTCTAGAAGTCACATGAA | CTGGCTATGGAACTAAATACA |
| NM_006254 | 5580 | PRKCD | protein kinase C, delta | CCGCTTCAAGGTTCACAACTA | CCGGGACACTATATTCCAGAA |
| NM_006255 | 5583 | PRKCH | protein kinase C, eta | CGCCGTGTGCTCATCACTGAA | GACCAGATTTGTGGCTTATAA |
| NM_006256 | 5586 | PRKCL2 | protein kinase C-like 2 | AAAGTATGATATCTACGCAAA | CACGTCAAAGTATGATATCTA |
| NM_006257 | 5588 | PRKCQ | protein kinase C, theta | CCGCAGACAAGTAATCACTAA | CGGGCAGATGTATATCCAGAA |
| NM_006258 | 5592 | PRKG1 | protein kinase, cGMP-dependent, type I | CCGGTGTATATACCATACAAA | GAGGTTCGTTTGAAGATTCTA |
| NM_006259 | 5593 | PRKG2 | protein kinase, cGMP-dependent, type II | GACCTACAATTTGATTCTCAA | TTGAGTACTACTAACGTTTAA |
| NM_006281 | 6788 | STK3 | serine/threonine kinase 3 (STE20 homolog, yeast) | CCGGCGCCTAAGAGTAAACTA | CGGCGCCTAAGAGTAAACTAA |
| NM_006282 | 6789 | STK4 | serine/threonine kinase 4 | AGGATTCATAGCATCACTATA | CACCATTTGCTGTGCGAATTA |
| NM_006285 | 7016 | TESK1 | testis-specific kinase 1 | CAGGACCGCCCTGACACACAA | CTGCGGTACCTGCACTCCAAA |
| NM_006293 | 7301 | TYRO3 | TYRO3 protein tyrosine kinase | AACGGTGACCTTTAGTGCCAA | CGGACTGACCAAATCACCCAA |
| NM_006296 | 7444 | VRK2 | vaccinia related kinase 2 | CACACAATAGGTTAATCGAAA | CTGGAGGATTTGGATTGATAT |
| NM_006301 | 7786 | MAP3K12 | mitogen-activated protein kinase kinase kinase 12 | CAGGGAGCACTATGAAAGGAA | CTCGTATTCCTTGTACATAGA |
| NM_006314 | 10256 | CNK1 | connector enhancer of KSR-like (Drosophila kinase suppressor of ras) | CCGCCGCTGGTTTGTGCTCAA | CGGACTCAGCTTAGTGCTGAA |
| NM_006343 | 10461 | MERTK | c-mer proto-oncogene tyrosine kinase | CAGATGAATGTTGTTAAGGAA | TTGCCTGACGTTCGGAACCAA |
| NM_006374 | 10494 | STK25 | serine/threonine kinase 25 (STE20 homolog, yeast) | AAGGGCATCGATAACCACACA | CACCAAGCTATGGATCATCAT |
| NM_006383 | 10518 | KIP2 | DNA-dependent protein kinase catalytic subunit-interacting protein 2 | CACCGAAGAGCAGCTAGACAA | CTGCACCTTCTTCAATAAGAA |
| NM_006422 | 10566 | AKAP3 | A kinase (PRKA) anchor protein 3 | AGGGATCATGACCTATGCTAA | ATCAAGCTATCCATAATGAAA |

Table 2

| NM_006457 | 10611 | LIM | LIM protein (similar to rat protein kinase C-binding enigma) | CAGCAGGGAAACGAACTCCGA | TCCGATGTGCGCCCATTGTAA |
|---|---|---|---|---|---|
| NM_006549 | 10645 | CAMKK2 | calcium/calmodulin-dependent protein kinase kinase 2, beta | CCGCAAGATCTTCTCTGGGAA | CTAGCTGATTGTTGTGGTCAA |
| NM_006556 | 10654 | PMVK | phosphomevalonate kinase | CCCAGTGTGGATACTAATAAA | TTGGCTGTGCTTGAAGGCGAA |
| NM_006575 | 11183 | MAP4K5 | mitogen-activated protein kinase kinase kinase kinase 5 | CTTGCCTATTTGCATACTAAA | TCCGCTTATTAGGATCAGACA |
| NM_006609 | 10746 | MAP3K2 | mitogen-activated protein kinase kinase kinase 2 | CACAGTAATATGATTGTCCTA | CAGAATGATGTCCGAGTCAAA |
| NM_006622 | 10769 | PLK2 | polo-like kinase 2 (Drosophila) | CAGATGCTCCTGAGCAATTTA | CAGATTGTGTCTGGACTGAAA |
| NM_006648 | 65268 | PRKWNK2 | protein kinase, lysine deficient 2 | AACGTTCATCGAGCAGATGAA | ACCTATAAGTCTAGTAGCAAA |
| NM_006712 | 10922 | FASTK | FAST kinase | CAGCAGCAAGGTGGTACAGAA | CTGGTGGTTCAGGAAACGCAA |
| NM_006716 | 10926 | ASK | activator of S phase kinase | CAGTATCAAGTTGTTGATGAT | CATGAATCTATTGTCACTAAA |
| NM_006738 | 11214 | AKAP13 | A kinase (PRKA) anchor protein 13 | CCGCCTGTTTGGGTTAACAAA | CTCGGTGTTTCAGGCACTAAA |
| NM_006742 | 5681 | PSKH1 | protein serine kinase H1 | CCCGATTAGGGTCGAGTGTCA | CCGCTCCACACGCTCCAATAA |
| NM_006759 | 7360 | UGP2 | UDP-glucose pyrophosphorylase 2 | CCCAAAGTTAGTTACTCTTAA | CGAGTTCAAGTCTGTATCAAA |
| NM_006852 | 11011 | TLK2 | tousled-like kinase 2 | AAGGTTCATCTGTCCATGCAA | CAAGAGAATACGATTCTTAAA |
| NM_006871 | 11035 | RIPK3 | receptor-interacting serine-threonine kinase 3 | ACCGCTCGTTAACATATACAA | CAGCCTGATGTCGTGCGTCAA |
| NM_006875 | 11040 | PIM2 | pim-2 oncogene | CCGGGATTGTCCAATTACTAA | CGGGATTGTCCAATTACTAAA |
| NM_006888 | 801 | CALM1 | calmodulin 1 (phosphorylase kinase, delta) | CGGCAACTTACACACATTGAA | CTGGTTGTATCTTATTAGCAA |
| NM_006904 | 5591 | PRKDC | protein kinase, DNA-activated, catalytic polypeptide | CTCGTGTATTACAGAAGGAAA | TTCGGCTAACTCGCCAGTTTA |
| NM_006930 | 6500 | SKP1A | S-phase kinase-associated protein 1A (p19A) | AGGGTACTTTAGAGCAACTTA | TAGGAAAGACTACTCAAGTTA |
| NM_007064 | 11139 | TRAD | serine/threonine kinase with Dbl- and pleckstrin homology domains | CAGGACATCAATCAAGTCTTA | CCCATTGAGTATCAACGGAAA |
| NM_007118 | 7204 | TRIO | triple functional domain (PTPRF interacting) | ACCGTTGTTCTTAGATGTCGA | CCGGGAATGTATGGATACGTA |
| NM_007170 | 10420 | TESK2 | testis-specific kinase 2 | CCGGGAAGAATCGCTATCTGA | CGAGATGAGCCCTATAATGAA |

EP 2 295 543 A1

Table 2

| NM_007174 | 11113 | CIT | citron (rho-interacting, serine/threonine kinase 21) | ATGGAAGGCACTATTTCTCAA | CAGGATATACCGTAACACGAA |
|---|---|---|---|---|---|
| NM_007181 | 11184 | MAP4K1 | mitogen-activated protein kinase kinase kinase kinase 1 | CTGACTAAGAGTCCCAAGAAA | TACGTGGGTGTACTCCATCAA |
| NM_007194 | 11200 | CHEK2 | CHK2 checkpoint homolog (S. pombe) | ACGCCGTCCTTTGAATAACAA | AGGACTGTCTTATAAAGATTA |
| NM_007199 | 11213 | IRAK3 | interleukin-1 receptor-associated kinase 3 | CACATTCGAATCGGTATATTA | CTGGATGTTCGTCATATTGAA |
| NM_007202 | 11216 | AKAP10 | A kinase (PRKA) anchor protein 10 | CAGATAAGCATAGAACTGATA | TAGCAGGACAGCCACACTTAA |
| NM_007229 | 11252 | PACSIN2 | protein kinase C and casein kinase substrate in neurons 2 | CACCCTTAATGTCCCGAGCAA | GAAGCTTTACATAGAACCTTA |
| NM_007254 | 11284 | PNKP | polynucleotide kinase 3'-phosphatase | CACGTGAACAGGGACACGCTA | CACGTGTGAGACAGCCCTGAA |
| NM_007271 | 11329 | STK38 | serine/threonine kinase 38 | AACCTTATCGCTCAACATGAA | TACGTCGGCCATAAACAGCTA |
| NM_007284 | 11344 | PTK9L | PTK9L protein tyrosine kinase 9-like (A6-related protein) | AAGACAGAGATCAGTGTGGAA | TTGGGATGGTTGAATGCTGTA |
| NM_012145 | 1841 | DTYMK | deoxythymidylate kinase (thymidylate kinase) | ATCAGTTGGAATTCTCCTAAA | TCCCGGAAAGATCAACTGAAA |
| NM_012290 | 9874 | TLK1 | tousled-like kinase 1 | CAGAGAGTATAGAATACACAA | CCGGAGAAGAAACAATCGGAA |
| NM_012324 | 23542 | MAPK8IP2 | mitogen-activated protein kinase 8 interacting protein 2 | ACAGGTAATGTCGGCTTCCAA | CTGGAAGAATTTGACGACGAA |
| NM_012395 | 5218 | PFTK1 | PFTAIRE protein kinase 1 | AAGGGACACCTTTCACAGCTA | CTGGGTCTTGTCAACATCTAA |
| NM_012398 | 23396 | PIP5K1C | phosphatidylinositol-4-phosphate 5-kinase, type I, gamma | CCGCGTCGTGGTCATGAACAA | CGGCAAGACCTATTTATAATA |
| NM_012407 | 9463 | PRKCABP | protein kinase C, alpha binding protein | CCGGTGTCAATGGCAGGTCAA | TCGCCTCACCATCAAGAAGTA |
| NM_012424 | 26750 | RPS6KC1 | ribosomal protein S6 kinase, 52kDa, polypeptide 1 | CAGGATTGGTTTATATGATTA | CCGGGGTTGTTTCACGAAGAAA |
| NM_012474 | 7371 | UMPK | uridine monophosphate kinase | CCGGATGCCTTTGACAATGAA | TCAGTACATTACGTTCGTCAA |
| NM_013233 | 27347 | STK39 | serine threonine kinase 39 (STE20/SPS1 homolog, yeast) | TGGAATAGTCTCAGATCTAAA | TTGGAGTATTTGTAACTTCTA |
| NM_013254 | 29110 | TBK1 | TANK-binding kinase 1 | CAGAACGTAGATTAGCTTATA | CTGACTTGACACGTTTGTAAA |
| NM_013257 | 23678 | SGKL | serum/glucocorticoid regulated kinase-like | ACCAACTAACTTACATGCTTA | CCACAGCGAGACCCTAGTTAA |
| NM_013276 | 23729 | CARKL | carbohydrate kinase-like | ATGAAACATATTGTCTCTTTA | CAGGTAGTTTATTCAAAGAGA |

Table 2

| NM_013302 | 29904 | EEF2K | elongation factor-2 kinase | CAGACACTACATGAACACGAA | CGGGATGGCGCTCTTCTTCTA |
|---|---|---|---|---|---|
| NM_013355 | 29941 | pknbeta | protein kinase PKNbeta | AGGGAAATACTACGCCATCAA | CCCGGGAAGTTTCAAGTTTGA |
| NM_013392 | 29959 | NRBP | nuclear receptor binding protein | CCGGTTGACTTCTCTGCTAGA | TCGGTGGAGGAGGGAGTCAAA |
| NM_013410 | 205 | AK3 | adenylate kinase 3 | CAGGAAGATGTGGTCATTCAT | CCTGGTAACAGTGAAATTGAA |
| XM_375834 | 9641 | IKBKE | inhibitor of kappa light polypeptide gene enhancer in B-cells, kinase epsilon | ACGGCGGAACAAGGAGATCAT | CCGCATCATCGAACGGCTAAA |
| NM_014006 | 23049 | SMG1 | PI-3-kinase-related kinase SMG-1 | ATCGATGTTGCCAGACTACTA | CACCATGGTATTACAGGTTCA |
| NM_014030 | 28964 | GIT1 | G protein-coupled receptor kinase-interactor 1 | CAGCCTTGACTTATCCGAATT | GCCGCTGAGGATGTCCCGAAA |
| NM_014216 | 3705 | ITPK1 | inositol 1,3,4-triphosphate 5/6 kinase | CACGCCGTCATTGACATCAAT | CCGATTAATGTCTGTACGTCA |
| NM_014226 | 5891 | RAGE | renal tumor antigen | AAAGAATAAGCTGTCGTTAAA | CCGGCTGTGTGTTCTACGAGA |
| NM_014264 | 10733 | PLK4 | polo-like kinase 4 (Drosophila) | CACCAAATGGTCAAACAACTA | CTGGTAGTACTAGTTCACCTA |
| NM_014308 | 23533 | P101-PI3K | phosphoinositide-3-kinase, regulatory subunit, polypeptide p101 | ACCGGTGCTGCTGCAACTCTA | ATCGCAGATCAAAGTGGACAA |
| NM_014326 | 23604 | DAPK2 | death-associated protein kinase 2 | CGGAATTTGTTGCTCCAGAAA | CTGGTTAAAGAGACCCGGAAA |
| NM_014365 | 26353 | HSPB8 | heat shock 27kDa protein 8 | CCGCATGGTTTGGTTAATGAA | CGCATGGTTTGGTTAATGAAA |
| NM_014370 | 26576 | STK23 | serine/threonine kinase 23 | AAGATGAGGCGCAAACGGAAA | AAGCGCTTTGTGGCCCTCAAA |
| NM_014371 | 26993 | AKAP8L | A kinase (PRKA) anchor protein 8-like | AAGGAACACTTTAAGTACGTA | CAGGATAACACCACCAACTAT |
| NM_014397 | 10783 | NEK6 | NIMA (never in mitosis gene a)-related kinase 6 | ACCACGGAAGTCGAGAATTAA | ACCGGAGAGGATCCATGAGAA |
| NM_014413 | 27102 | HRI | heme-regulated initiation factor 2-alpha kinase | CCCGAATATGACGAATCTGAT | CTGATTAAGGGTGCAACTAAA |
| NM_014496 | 27330 | RPS6KA6 | ribosomal protein S6 kinase, 90kDa, polypeptide 6 | CAGCGGTATACTGCTGAACAA | GGCGAGGTAAATGGTCTTAAA |
| NM_014572 | 26524 | LATS2 | LATS, large tumor suppressor, homolog 2 (Drosophila) | CACACTCACCTCGCCCAATAA | CAGGACCTTCACTGCATTAAA |
| NM_014586 | 30811 | HUNK | hormonally upregulated Neu-associated kinase | CACGGGCAAAGTGCCCTGTAA | TCGGACCAAGATCAAACCAAA |
| NM_014602 | 30849 | PIK3R4 | phosphoinositide-3-kinase, regulatory subunit 4, p150 | AAGCAGAATTCTAGATCAGAA | TAGAGGCAGAAGATTACTTAA |
| NM_014683 | 9706 | ULK2 | unc-51-like kinase 2 (C. elegans) | ACCGGGAAGTTATCAGATCAA | ATCGGTACAGTTATTCTTAAA |

EP 2 295 543 A1

Table 2

| NM_014720 | 9748 | SLK | SNF1 sucrose nonfermenting like kinase (yeast) | CCAGATCAGGACCGTGATAAA | TAGCATCTTGTGATCACCCAA |
|---|---|---|---|---|---|
| NM_014776 | 9815 | GIT2 | G protein-coupled receptor kinase-interactor 2 | AAGCATCGTGCAATAAGTGAA | CTGCCTATGGCTGAAACTATA |
| NM_014791 | 9833 | MELK | maternal embryonic leucine zipper kinase | CGGGTTGTCTTCCGTCAGATA | CTGGATCATGCAAGATTACAA |
| XM_374996 | 9891 | ARK5 | AMP-activated protein kinase family member 5 | CTTGAGTTGATGATCAATTAA | TAGGGATTTACTGGCATGGTA |
| NM_014911 | 22848 | AAK1 | AP2 associated kinase 1 | AACGTGAGTAGCGGTGATGTA | CCAGGTGGTAAACCTGATGAA |
| NM_014916 | 22853 | LMTK2 | lemur tyrosine kinase 2 | CACCTCCGACTTAAATGTGAA | CAGATCAGACTAAGTATAGTA |
| NM_014920 | 22858 | ICK | intestinal cell (MAK-like) kinase | ACCTAATGACTTGGTAAATTA | CTCAGTAATCTAGCTTATATA |
| NM_015000 | 23012 | STK38L | serine/threonine kinase 38 like | AGGGAGATGTACTGTATTATA | TACAACCATGATAACCATTAT |
| NM_015071 | 23092 | GRAF | GTPase regulator associated with focal adhesion kinase pp125(FAK) | ACCGTATGGCCTATAGTTTAA | AGGGAGTATACTAGTAGGTTA |
| NM_015076 | 23097 | CDK11 | cyclin-dependent kinase (CDC2-like) 11 | CACTGTCGTCAGGAAGATATA | GAGGATTTGTTTGAGTACGAA |
| NM_015093 | 23118 | MAP3K7IP2 | mitogen-activated protein kinase kinase kinase 7 interacting protein 2 | CAGTCAATAGCCAGACCTTAA | TGGCTGGGTATCTCAGTTTAA |
| NM_015112 | 23139 | MAST2 | microtubule associated serine/threonine kinase 2 | CAGGAGTGTGCTGTCTGGCAA | CAGTGAAATAATAATGATGAA |
| NM_015133 | 23162 | MAPK8IP3 | mitogen-activated protein kinase 8 interacting protein 3 | CAGCCGCAACATGGAAGTACA | GACGAGTGGTCTGATGTTCAA |
| NM_015148 | 23178 | PASK | PAS domain containing serine/threonine kinase | ATCCTGGTTGCTAACGACAAA | CGCGCTGACACTGTTTGGTTA |
| NM_015690 | 27148 | STK36 | serine/threonine kinase 36 (fused homolog, Drosophila) | ATGGATGCTGACCTCCTTATA | CCAGTTGGTGTCAGCCCTGTA |
| NM_015716 | 50488 | MINK | misshapen/NIK-related kinase | CACGTACGGGCGCATCATTAA | CGCAGACTTTGTGTTGCTGAA |
| NM_015850 | 2260 | FGFR1 | fibroblast growth factor receptor 1 (fms-related tyrosine kinase 2, Pfeiffer syndrome) | CAGAGATTTACCCATCGGGTA | CCGGCCTCTATGCTTGCGTAA |
| NM_015978 | 51086 | TNNI3K | TNNI3 interacting kinase | CAGCATTCGGCGTATACCTAA | GGAGCTAATGTCAATATTCAA |
| NM_015981 | 815 | CAMK2A | calcium/calmodulin-dependent protein kinase (CaM kinase) II alpha | CAGGAACTTCTCCGGAGGGAA | CCAGGATTTCATCTCACCATA |

Table 2

| NM_016086 | 51657 | MK-STYX | map kinase phosphatase-like protein MK-STYX | AAGCTTCTGCACATCCGGATA | CAAGATTCAGAAGGACTTGAA |
|---|---|---|---|---|---|
| NM_016123 | 51135 | IRAK4 | interleukin-1 receptor-associated kinase 4 | ATCCTATTAGTCATATATTTA | TTGCAGGACAGTGGTTATTAA |
| NM_016151 | 9344 | TAO1 | thousand and one amino acid protein kinase | AACGGAAACCACCGCTCTTTA | ATCGAGCTGGCTGAACGGAAA |
| NM_016203 | 51422 | PRKAG2 | protein kinase, AMP-activated, gamma 2 non-catalytic subunit | AAGCACGAGCCTGAACGGTTA | CCGGAAAGCATTCCTCTCGAA |
| NM_016223 | 29763 | PACSIN3 | protein kinase C and casein kinase substrate in neurons 3 | AAGGATATGCTGCTCACCTTA | CAGAGGACAATCAGCCGGAAA |
| NM_016231 | 51701 | NLK | nemo like kinase | CCGGATAGACCTATTGGATAT | TTGGTGTTGTCTGGTCAGTAA |
| NM_016248 | 11215 | AKAP11 | A kinase (PRKA) anchor protein 11 | ACCGGTTATCTAAATCTATTA | CTGACCGGTTATCTAAATCTA |
| NM_016276 | 10110 | SGK2 | serum/glucocorticoid regulated kinase 2 | CAGGGCCAATGGGAACATCAA | TGGGTACGTGACTATCCCTAA |
| NM_016281 | 51347 | JIK | STE20-like kinase | CAAACAGTATAAAGCACTCAA | CAGCTCCTCCGTCGTGCATAA |
| NM_016282 | 50808 | AK3L1 | adenylate kinase 3 like 1 | CCCGTGGCACTATGTAAATAA | CCGTGGCACTATGTAAATAAA |
| NM_016291 | 51447 | IHPK2 | inositol hexaphosphate kinase 2 | AAGCATCGGAACCAGTACAAA | TCGGAACCAGTACAAATTTAT |
| NM_016308 | 51727 | UMP-CMP | UMP-CMP kinase | AAGTATTGCTAAGTACTATAA | CGCGTATATATCCCTCTAGTA |
| NM_016440 | 51231 | VRK3 | vaccinia related kinase 3 | AACCAGGGCATTCTCTATGAA | CTCACTCAAACTGGATGCCAA |
| NM_016457 | 25865 | PRKD2 | protein kinase D2 | CACGACCAACAGATACTATAA | TTGGGTGGTTCATTACAGCAA |
| NM_016478 | 51530 | NIPA | nuclear interacting partner of anaplastic lymphoma kinase (ALK) | AGGGAGAATGGTGGAACTGAA | CCGCTTGAGGCTCTCTATGAA |
| NM_016507 | 51755 | CRK7 | CDC2-related protein kinase 7 | ACCGGATATCGGGAAGTTCAA | ATCGGGATATTAAGTGTTCTA |
| NM_016508 | 51265 | CDKL3 | cyclin-dependent kinase-like 3 | CAACATTATTGTCATGGACTA | TGGGCAGATAGTGGCCATTAA |
| NM_016542 | 51765 | MST4 | Mst3 and SOK1-related kinase | CAGCAAGTCGTTGCTATTAAA | CCAAACCTACGTCAAGATTAA |
| NM_016653 | 51776 | ZAK | sterile alpha motif and leucine zipper containing kinase AZK | ACGTGGTAGTATATCACTCAA | TCGGGTAGCAACAAACTTATT |
| NM_017431 | 53632 | PRKAG3 | protein kinase, AMP-activated, gamma 3 non-catalytic subunit | CAGATCTATGAGATTGAACAA | TAGCCTGTTTGAAGCTGTCTA |
| NM_017449 | 2048 | EPHB2 | EphB2 | CACGCTTTCTAGAGGACGATA | CCGAGAGGACCTCGTCTACAA |
| NM_017567 | 55577 | NAGK | N-acetylglucosamine kinase | ACCTGAGTGAAAGCTACTTAA | CCCGGTCTTGTTCCAGGGCAA |

Table 2

| NM_017572 | 2872 | MKNK2 | MAP kinase-interacting serine/threonine kinase 2 | CCGCTTCTACCTGGTGTTTGA | TTGGACTTTCTGCATAACAAA |
|---|---|---|---|---|---|
| NM_017593 | 55589 | BMP2K | BMP2 inducible kinase | AGGCATCACCTGAATATCTTA | TCCGATGTGCATTGAAGCGAA |
| NM_017719 | 54861 | SNRK | SNF-1 related kinase | ACCACTGAATTGGAACGGATA | CACCACTGAATTGGAACGGAT |
| NM_017771 | 54899 | PXK | PX domain containing serine/threonine kinase | CTGGCAGATTGTTAGAAGATA | TAAGATCCCTACAAAGTTAAA |
| NM_017859 | 54963 | URKL1 | uridine kinase-like 1 | ACCCAGGACTGTTGAATACAA | TACAATGAACACGGCACGCAA |
| NM_017900 | 54998 | AKIP | aurora-A kinase interacting protein | ATCCGTAGTAATAAATTCTCA | CTGCTGTGATCCGTAGTAATA |
| NM_018216 | 55229 | PANK4 | pantothenate kinase 4 | CTGGGAGACGTTCATATTGGA | TCGACATAGGCGGGTCGTTAA |
| NM_018323 | 55300 | PI4K2B | phosphatidylinositol 4-kinase type-II beta | ATGAACTTTGTGCAAGATTTA | CAGAGTACTGGCCTTGTTCAA |
| NM_018339 | 55312 | FLJ11149 | riboflavin kinase | ACGATGTAATTTAATGCTAAA | CACGATGTAATTTAATGCTAA |
| NM_018343 | 55781 | RIOK2 | RIO kinase 2 (yeast) | AACGCGATTATCATAAACATA | CGCGGTTGAAATGGGCATGAA |
| NM_018401 | 55351 | HSA250839 | gene for serine/threonine protein kinase | CTGGGACGCGGTGTTCAAGAA | TTGGGCTGAGTTCACGAATTA |
| NM_018425 | 55361 | PI4KII | phosphatidylinositol 4-kinase type II | ACGCAAATGTATGAATAACAA | CCGCATCGGGCTACCACCAAA |
| NM_018492 | 55872 | TOPK | T-LAK cell-originated protein kinase | AAGTGTGGCTTGCGTAAATAA | TCAGTAGTTATTAGACTCTAA |
| NM_018571 | 55437 | ALS2CR2 | amyotrophic lateral sclerosis 2 (juvenile) chromosome region, candidate 2 | CAGGAACACTGGTAACTATAA | CCAGTGGAACTCACACAGTAA |
| NM_018584 | 55450 | CaMKIINalpha | calcium/calmodulin-dependent protein kinase II | AGCAAGCGGGTTGTTATTGAA | CGGGTTGTTATTGAAGATGAT |
| NM_018638 | 55500 | EKI1 | ethanolamine kinase | CCGTATGTGGTGGATTAGAAA | TCCGTATGTGGTGGATTAGAA |
| NM_018650 | 4139 | MARK1 | MAP/microtubule affinity-regulating kinase 1 | CCCGGTGTAGAAACTCCATTA | CCGGCCCAGTAGTGACTTAAA |
| NM_018842 | 55971 | LOC55971 | insulin receptor tyrosine kinase substrate | CACGGAGAGCACCTACCGGAA | CTCAACGAGAGTCTTGATGAA |
| NM_018979 | 65125 | PRKWNK1 | protein kinase, lysine deficient 1 | CTAGAGGATCTTGATGCTCAA | CTGCGACGACTACGAGATAAA |
| NM_019884 | 2931 | GSK3A | glycogen synthase kinase 3 alpha | CAGGGAACTAGTCGCCATCAA | CCGGGTGTAAATAGATTGTTA |
| NM_020126 | 56848 | SPHK2 | sphingosine kinase 2 | CAGGATTGCGCTCGCTTTCAT | CCGAGGGTAGTGCCTGATCAA |
| NM_020168 | 56924 | PAK6 | p21(CDKN1A)-activated kinase 6 | CGGAAGGACCATGTTCCGCAA | CTGTGTTACCTTTCAAGTTTA |
| NM_020240 | 56990 | SPEC2 | non-kinase Cdc42 effector protein SPEC2 | AAGGGAGGTTATGGAGGTGGA | CAGGAGACCTGTTCAGTGGAA |

Table 2

| NM_020397 | 57118 | CAMK1D | calcium/calmodulin-dependent protein kinase ID | CAGGCGCTTTCTATACTTAAT | CCAGGCGCTTTCTATACTTAA |
|---|---|---|---|---|---|
| NM_020421 | 57143 | ADCK1 | aarF domain containing kinase 1 | TAACAAGTACTTGGACCCTAA | TACGACGGCTGTCATCAGTTA |
| NM_020439 | 57172 | CAMK1G | calcium/calmodulin-dependent protein kinase IG | AACAGGCCGCCTGAAACTCAA | CAGGTCTTGTCGGCAGTGAAA |
| NM_020526 | 2046 | EPHA8 | EphA8 | ACGGCCCACCAGGGTATGTAA | GAGCGTGTGCTTATCCGTTAA |
| NM_020639 | 54101 | ANKRD3 | ankyrin repeat domain 3 | AAGCCTGATGACGAAGTGAAA | CACACGCAGTATGAAGATAAA |
| NM_020666 | 57396 | CLK4 | CDC-like kinase 4 | AAGTCTGACTATGTAGTCAAA | TCGAAGAATGTTAGAATATGA |
| NM_020804 | 29993 | PACSIN1 | protein kinase C and casein kinase substrate in neurons 1 | CAGCAGCTACATCCATGTGTA | CGCCATAGAGTTCCAGACATA |
| NM_020836 | 57596 | KIAA1446 | brain-enriched guanylate kinase-associated protein | CGGGCGGACCATGTCACCGTA | CTGCAGCTTCTCTGAACGCTA |
| NM_020922 | 65267 | PRKWNK3 | protein kinase, lysine deficient 3 | AAGATTGGTGATCTAGGATTA | CCGGGTGACGCCAATAAAGAA |
| NM_020965 | 260425 | MAGI&#45;3 | membrane-associated guanylate kinase-related (MAGI-3) | CCCTATGATGTTGTCTTGCAA | CGGGATGTCGAAGACGCTGAA |
| NM_020975 | 5979 | RET | ret proto-oncogene (multiple endocrine neoplasia and medullary thyroid carcinoma 1, Hirschsprung disease) | CCGCTGGTGGACTGTAATAAT | TAGGCTGGTTCTCAACCGGAA |
| NM_020990 | 1159 | CKMT1 | creatine kinase, mitochondrial 1 (ubiquitous) | ACGGATCTAGATGCCAGTAAA | CTGGACGTTACTATAGGCTCA |
| NM_021135 | 6196 | RPS6KA2 | ribosomal protein S6 kinase, 90kDa, polypeptide 2 | CCGAGTGAGATCGAAGATGGA | CCGGAGGTCCTGAAGCGTCAA |
| NM_021733 | 60385 | TSKS | testis-specific kinase substrate | AACCAACGTCTTGAAGCAGAA | CACCAACGTGTCACTGCTCAA |
| NM_021970 | 8649 | MAP2K1IP1 | mitogen-activated protein kinase kinase 1 interacting protein 1 | ATGCCCAGGCTTGCAGAATAA | TAGCATATAGATGTAATTTAT |
| NM_021972 | 8877 | SPHK1 | sphingosine kinase 1 | CACGAACCAAATCCAAATAAA | CCGGCTGCTGTCACCCATGAA |
| NM_022048 | 53944 | CSNK1G1 | casein kinase 1, gamma 1 | ATGGACCATCCTAGTAGGGAA | CCGGCAGACCAATACCTCATA |
| NM_022128 | 64080 | RBKS | ribokinase | AACACAGTCATCTTACCCTTA | CAGGTGGTAATCATTACCTTA |
| NM_022158 | 64122 | FN3K | fructosamine-3-kinase | CCAGCTGTTTAACTACCTGAA | CCCAGTGTTCGTCAAAGTCAA |
| NM_022344 | 64149 | NJMU-R1 | protein kinase Njmu-R1 | TTGAGTTACACTCCTGTTGAA | TTGGCAGATACTAATCTACCA |
| NM_022445 | 27010 | TPK1 | thiamin pyrophosphokinase 1 | AACCTGGTGCATCGAAATGTA | CAGGTTGTCTGTAGAGAATGA |

Table 2

| NM_022731 | 64710 | NUCKS | nuclear ubiquitous casein kinase and cyclin-dependent kinase substrate | AAGGTGTACCACGGTTGTAAA | ACCCTAGAATTTAATCAACAA |
|---|---|---|---|---|---|
| NM_022740 | 28996 | HIPK2 | homeodomain interacting protein kinase 2 | AACCAGTACCCTTACATATAA | TCCCGAAGTCTCCATACTAAA |
| NM_023018 | 65220 | FLJ13052 | NAD kinase | AAAGAGCGTCCTTGTCATCAA | CCAGGAGAACATGATCGTGTA |
| NM_024117 | 79109 | MAPKAP1 | mitogen-activated protein kinase associated protein 1 | CTAGGTATCTCTGGAGACAAA | GACCCTGTTACGAATCAGAAA |
| NM_024594 | 79646 | PANK3 | pantothenate kinase 3 | AAGCGAGAATCTGTTAGTAAA | TCCAAAGGTGATAGCACACAA |
| NM_024779 | 79837 | PIP5K2C | phosphatidylinositol-4-phosphate 5-kinase, type II, gamma | CCGGAGCAGTATGCTAAGCGA | TTGATGTGTGATAATCTCATA |
| NM_024800 | 79858 | NEK11 | NIMA (never in mitosis gene a)- related kinase 11 | CTGCCTATGCTTGGAGTCATA | TGAGATAAGCTTATAGATCAA |
| NM_024876 | 79934 | ADCK4 | aarF domain containing kinase 4 | CAGATGCTGAGAGTTCTTGAA | CCGGGTCCCAGCCGTGGTTAA |
| NM_024960 | 80025 | PANK2 | pantothenate kinase 2 (Hallervorden-Spatz syndrome) | CAGAGCGACTTTGATCACCAT | CTGTGTGTGAACTTACTGTAA |
| NM_025144 | 80216 | LAK | lymphocyte alpha-kinase | CAGCCCTTGGTCCTATCTGAA | CAGGCCCTACATTTAAAGCTA |
| NM_025194 | 80271 | ITPKC | inositol 1,4,5-trisphosphate 3-kinase C | CCGGAAGGACATGTATGAGAA | TCGGCCGAAGCCCGAACCGAA |
| NM_025211 | 80318 | GKAP42 | protein kinase anchoring protein GKAP42 | AACGCTCAACATGATCTTCCA | CATGCTGTTTGTAACGCTCAA |
| NM_025228 | 80342 | T3JAM | TRAF3-interacting Jun N-terminal kinase (JNK)-activating modulator | CAGACTTGGCACCAACATTAA | TAGCAGGGACTTACAGATGAA |
| NM_030662 | 5605 | MAP2K2 | mitogen-activated protein kinase kinase 2 | CAGCATTTGCATGGAACACAT | CCGGCCTGCCATGGCCATCTT |
| NM_030906 | 65975 | STK33 | serine/threonine kinase 33 | ATGTGTCTAGTGCATCCTTAA | CAGGATGGTAATAGGTTAAGA |
| NM_030952 | 81788 | SNARK | likely ortholog of rat SNF1/AMP-activated protein kinase | CCGGTGGCTGTTGATGGTGAA | CTGAACGAAGAGGATACTAAA |
| NM_031414 | 56164 | STK31 | serine/threonine kinase 31 | CACAGTACAAGCTAAGTACAA | CCCAGTGTGGATCACTTGCTA |
| NM_031417 | 57787 | MARK4 | MAP/microtubule affinity-regulating kinase 4 | CCGCATCATGAAGGGCCTAAA | CTGCAGCCTGTTGCCCAATAA |
| NM_031432 | 83549 | UCK1 | uridine-cytidine kinase 1 | AACGGTGGAGGTGCCGACCTA | TGCGGTTTAAAGATCCCTCTA |
| NM_031464 | 83694 | RPS6KL1 | ribosomal protein S6 kinase-like 1 | CGCGATGTTAGTGAGGACTAT | CTGAATTTGATTGGAAAGGAA |
| NM_031480 | 83732 | RIOK1 | RIO kinase 1 (yeast) | GCCCAACAAGATAATATTCTA | TACCATGTCCAGAACCAATAA |

Table 2

| NM_031892 | 30011 | SH3KBP1 | SH3-domain kinase binding protein 1 | CTCTGAGATCTCAATGCGAAA | TAGCTTATATATGACGGTATA |
|---|---|---|---|---|---|
| NM_032017 | 83931 | MGC4796 | Ser/Thr-like kinase | CAGCGCTACCTGCGGAAATAA | CCGGATGGTTAAGAAGATGAA |
| NM_032028 | 83942 | STK22D | serine/threonine kinase 22D (spermiogenesis associated) | CCAGCTTTCCTTGGCCATCAA | TAGAAGCTGATTTAACACCAA |
| NM_032037 | 83983 | SSTK | serine/threonine protein kinase SSTK | ATCGAGGTGTGCAACGGGAAA | CCGGTTGGAACCTGCAATAAA |
| NM_032294 | 84254 | CAMKK1 | calcium/calmodulin-dependent protein kinase kinase 1, alpha | AGCGATGAGCAAACTCTATAA | CGGCGTTATCTGGAAAGTGGA |
| NM_032387 | 65266 | PRKWNK4 | protein kinase, lysine deficient 4 | CAGCTTGTTGGGCGTTTCCAA | CAGGAGGAGCCAGCACCATTA |
| NM_032409 | 65018 | PINK1 | PTEN induced putative kinase 1 | CCGGACGCTGTTCCTCGTTAT | GACGCTGTTCCTCGTTATGAA |
| NM_032435 | 84451 | KIAA1804 | mixed lineage kinase 4 | ACGGACCATGTCTGATGGAAA | CGGGAACTTAACATTCTGATA |
| NM_032630 | 51550 | CINP | cyclin-dependent kinase 2-interacting protein | CGGCTGATTGGCACAATTTAA | GCGGCTGATTGGCACAATTTA |
| NM_033116 | 91754 | NEK9 | NIMA (never in mitosis gene a)- related kinase 9 | CACGAACCAGTGAAGTCTATA | TACACTTGGGTGAACATGCAA |
| NM_033118 | 85366 | MYLK2 | myosin light chain kinase 2, skeletal muscle | AACGCTGTAACCGACGCCTTA | CGGGATCCTCTTCATGCACAA |
| NM_033126 | 85481 | PSKH2 | serine/threonine kinase PSKH2 | AAGGACTTTATAGACAAACTA | GAGGATCAAGTTTACATGGTA |
| NM_033214 | 2712 | GK2 | glycerol kinase 2 | CTCGGGTGTGCCATAATAATA | TACGTTAGAAGAGCACTGTAA |
| XM_370946 | 10595 | LOC388226 | similar to protein kinase/ribonuclease IRE1 beta | AAGGATGAAACTGGCTTCTAT | CACCTGCATTCTTTACACATA |
| NM_033498 | 3098 | HK1 | hexokinase 1 | CACGATGTAGTCACCTTACTA | CCGTGTCGTATGACCTAGTAA |
| NM_033516 | 89882 | NYD-SP25 | protein kinase NYD-SP25 | ATGGACCTAACTATACATCTT | TAGACGAAATGTGCCATTATA |
| NM_052841 | 81629 | STK22C | serine/threonine kinase 22C (spermiogenesis associated) | ATGGCAAGTGCTCTCCAATAA | TGGCAAGTGCTCTCCAATAAA |
| NM_052853 | 90956 | ADCK2 | aarF domain containing kinase 2 | CAGATTGACCTGCGTTACGAA | CCGGAATGTGAAAGCCGTCAA |
| NM_052902 | 114790 | STK11IP | serine/threonine kinase 11 interacting protein | AAAGTTGTTTCCAACTCATAA | CTCTGCGTTTCTTGAACCTAA |
| NM_052947 | 115701 | HAK | heart alpha-kinase | AGCGAAGACCTTGGCATTTAT | CGGCCTCATGCCTGTCTTCAA |
| NM_053006 | 23617 | STK22B | serine/threonine kinase 22B (spermiogenesis associated) | CAAGTTCAATGTGGCTGTCAA | GTCGGCCTTCACGTAAACTAA |
| NM_054111 | 117283 | IHPK3 | inositol hexaphosphate kinase 3 | AAGACTGGTGGACAAGTGTAA | CTGAGATACGTTACCCAATTA |

Table 2

| | | | | | |
|---|---|---|---|---|---|
| NM_054113 | 117286 | CIB3 | calcium and integrin binding family member 3 | CACGAGCAGCTGGAAGCGTAT | CTGGAGCAGACGGTGACCAAA |
| NM_080823 | 6725 | SRMS | src-related kinase lacking C-terminal regulatory tyrosine and N-terminal myristylation sites | CAGACCAACCCTGGTACTTTA | CCGGGTCTCCATGGCAGCTGA |
| NM_080836 | 140901 | STK35 | serine/threonine kinase 35 | CTCGGCTGTTAGGGATCTGAA | TTGGGTTGGTATGATACATTA |
| NM_133494 | 140609 | NEK7 | NIMA (never in mitosis gene a)-related kinase 7 | ACGACCGGATATGGGCTATAA | CCGGATATGGGCTATAATACA |
| NM_138316 | 53354 | PANK1 | pantothenate kinase 1 | AACGCTGGTTAAATTGGTGTA | CCGAAGGATATTACAGCCGAA |
| NM_138733 | 5232 | PGK2 | phosphoglycerate kinase 2 | CAGCAACATGTAGTTAATATA | CAGGCCATCAGGAACTCTTAA |
| NM_139021 | 225689 | ERK8 | extracellular signal-regulated kinase 8 | CCCACTGACTTCCTCCAATAA | CCCAGAGAACATTCCGGGAAA |
| NM_139158 | 65061 | ALS2CR7 | amyotrophic lateral sclerosis 2 (juvenile) chromosome region, candidate 7 | CTGGACATAAACTAAGTCTAA | CTGGCGTACATCCACCACCAA |
| NM_139209 | 131890 | GRK7 | G protein-coupled receptor kinase 7 | ATCCATGACATCTGTGAATTA | TCGGCTGACATAATCCTCGAA |
| NM_145040 | 112464 | PRKCDBP | protein kinase C, delta binding protein | AAGGAGCGAATCCTACATCCA | CACGCCCTAATAAGGAGCGAA |
| NM_145059 | 197258 | FUK | fucokinase | CCGAAGGGAGTTGATTGGACA | TGGCTTAGAGTTGTAGACTTA |
| NM_145203 | 122011 | MGC33182 | casein kinase I alpha S-like | CAAGAATAATGTGAAAGATAA | CAGATTATCTTTAGAATTCCA |
| BC005365 | 5609 | MAP2K7 | mitogen-activated protein kinase kinase 7 | ATGGATCTCTCTCAACAACTA | CCGGCGGAGGATCGACCTCAA |
| NM_145695 | 1607 | DGKB | diacylglycerol kinase, beta 90kDa | CCCGAACTACTTCTCCTGCAA | CTCATTCTAGTCCAATGGTAA |
| NM_152309 | 118788 | PIK3AP1 | phosphoinositide-3-kinase adaptor protein 1 | ATGCTCAAGAGTCACATTAAA | CAGCAGGGTATTTAAGTGCTA |
| NM_152327 | 122481 | AK7 | adenylate kinase 7 | CAAGATTGTCTTGACCATTTA | GACGATCAATATATAATTAGA |
| NM_152671 | 200576 | PIP5K3 | na | ACCCAGTAACATAATATTTCA | TTGCCGTTACCCAGTAACATA |
| NM_152720 | 4752 | NEK3 | NIMA (never in mitosis gene a)-related kinase 3 | ACGATAGAGGTGGTTCTGTAA | CAGAGATATCAAGTCCAAGAA |
| NM_152835 | 149420 | LOC149420 | casein kinase | AAGCTAGACTCTACCCTCTAA | CAGCCAAAGTACGATCTAATA |
| NM_152883 | 5754 | PTK7 | PTK7 protein tyrosine kinase 7 | ACGGTTCGAGGTCTTCAAGAA | ATGGGACATGGTACCGTTGTA |
| NM_153273 | 9807 | IHPK1 | inositol hexaphosphate kinase 1 | AAGGTGGATGTCCGCATGATT | CCGGCAGATGCGGAAATGCGA |
| NM_153335 | 92335 | LYK5 | protein kinase LYK5 | CACCCAGATGCTGCTAGAGAA | CTGGTTCTAGTTCTGGTTCTA |

EP 2 295 543 A1

Table 2

| Accession | Gene ID | Symbol | Description | Sequence 1 | Sequence 2 |
|---|---|---|---|---|---|
| NM_172173 | 818 | CAMK2G | calcium/calmodulin-dependent protein kinase (CaM kinase) II gamma | CCGATGAGAAACCTCGTGTTA | GAGGAAGAGATCTATACCCTA |
| NM_173176 | 2185 | PTK2B | PTK2B protein tyrosine kinase 2 beta | AAGCTGATCGGCATCATTGAA | CAGGAGAACTTAAAGCCCAAA |
| NM_173354 | 150094 | SNF1LK | SNF1-like kinase | AGCGAAATAAGCAATACGTTA | CACAATTGATTCCAACACAAA |
| NM_173500 | 146057 | TTBK2 | tau tubulin kinase 2 | ACGGCCCAGGTTAAATGGAAA | CACATTGGTCATGACATGTTA |
| NM_173515 | 154043 | MAGI1 | membrane associated guanylate kinase interacting protein-like 1 | CCGGAGTTGTGTTACTGCTTA | CGGGAGTTGTGTTACTGCTTAA |
| NM_173575 | 282974 | PKE | PKE protein kinase | CCGGGCTTCGTGCCCAACAAA | TCCGAGAATGACTATCTTCAA |
| NM_173598 | 283455 | KSR2 | kinase suppressor of Ras-2 | ATCCGGTGACCTCGAATCCAA | TCCGTTGTGAGGGATGCCAAA |
| NM_174858 | 26289 | AK5 | adenylate kinase 5 | CAGAGCTAGTCAGTACACTGAA | CAGCATGGCAGTTGACAACAA |
| NM_174922 | 203054 | ADCK5 | aarF domain containing kinase 5 | AAGGCCTTTGCTGAGCAGATA | CCGCTACTTCCTTATGGCTAA |
| NM_175866 | 127933 | KIS | kinase interacting with leukemia-associated gene (stathmin) | AAGCAGTACTGTAGTACTGAA | CCACATATGAATGCAGGACTA |
| NM_176799 | 80895 | ILKAP | integrin-linked kinase-associated serine/threonine phosphatase 2C | ATGGAGGAATTCGAGCCTCAA | TTCGGTGATCTTTGGTCTGAA |
| NM_176800 | 8899 | PRPF4B | PRP4 pre-mRNA processing factor 4 homolog B (yeast) | GAGCCGATCCTTAGAAAGAAA | TCGTCTGATGATAACCTTGAA |
| NM_177560 | 1457 | CSNK2A1 | casein kinase 2, alpha 1 polypeptide | ACCGTTGCTTGTGGATTTATA | CTGGTCGCTTACATCACTTTA |
| NM_177990 | 57144 | PAK7 | p21(CDKN1A)-activated kinase 7 | ATGATCTGGATCCGTATTATA | ATGGTGTGCACGTTTCATTAA |
| NM_178009 | 160851 | DGKH | diacylglycerol kinase, eta | CAGGTGGAGTATAATGACATA | CCGGATCTCTAGATTCCGTAGAT |
| NM_178170 | 284086 | NEK8 | NIMA (never in mitosis gene a)- related kinase 8 | ACGGACAGTTGGGCACCAATA | CCAGAAGCTGGTGATCATCAA |
| NM_178432 | 23552 | CCRK | cell cycle related kinase | AAGGAGAAGTGCAGAGAGTAA | TGGCGAGATAGTTGCCCTCAA |
| NM_178510 | 255239 | ANKK1 | ankyrin repeat and kinase domain containing 1 | AGGAGCCGAGATGGAAATTTA | GCGGACGGAGTACGCCATCAA |
| NM_178813 | 158798 | AKAP28 | A-kinase anchoring protein 28 | CCCATTGTGTCGTTTCTTATGTA | TACGAGGATGAATTGACTCAA |
| NM_181358 | 204851 | HIPK1 | homeodomain interacting protein kinase 1 | AGGGAAGCTGTACACCACTAA | CTGAGCTATGTTTGTAATGCA |
| NM_181504 | 5295 | PIK3R1 | phosphoinositide-3-kinase, regulatory subunit, polypeptide 1 (p85 alpha) | CAGCATTAAACCAGACCTTAT | TAGCTGGTTATGAACTAGTAA |
| NM_181690 | 10000 | AKT3 | v-akt murine thymoma viral oncogene homolog 3 (protein kinase B, gamma) | AACTGTTGGCTTTGGATTAAA | CAGCAGGCACGTTAACTCGAA |

Table 2

| NM_181795 | 5570 | PKIB | protein kinase (cAMP-dependent, catalytic) inhibitor beta | CAGCATGTGTATATTAGATAA | CCGGAATGCCTTACCAGACAT |
|---|---|---|---|---|---|
| NM_181805 | 11142 | PKIG | protein kinase (cAMP-dependent, catalytic) inhibitor gamma | CCACAAGATGTTATTTATTGA | GAGCTCCATGTCCCAGATAAA |
| NM_181839 | 5569 | PKIA | protein kinase (cAMP-dependent, catalytic) inhibitor alpha | ATGATTATCATTAGAAGCTAA | TCACTTGATCATATGACGAAA |
| NM_181871 | 5313 | PKLR | pyruvate kinase, liver and RBC | CGGGTGCAATTTGGCATTGAA | GAGGCCTATCTGAGACTATAA |
| NM_182398 | 9252 | RPS6KA5 | ribosomal protein S6 kinase, 90kDa, polypeptide 5 | AAGCCAGTCATTCGAGATGAA | CTGGATCTCTTACGTAATTCA |
| NM_182471 | 5315 | PKM2 | pyruvate kinase, muscle | AACATCAAGATTATCAGCAAA | CCCGATCAGTGGAGACGTTGA |
| NM_182493 | 91807 | LOC91807 | myosin light chain kinase (MLCK) | ACGGAGGACAATGACCTTGCA | GACCATGAATTTCATTGTAAA |
| NM_182661 | 64781 | CERK | ceramide kinase | ATGGCATTATTTGATCTGAAA | CGGCTTAAACTTTGATCTGTA |
| NM_182687 | 9088 | PKMYT1 | membrane-associated tyrosine- and threonine-specific cdc2-inhibitory kinase | CCGGCCAGAGTCCTTCTTCCA | CTGGGAGGAACTTACCGTCTA |
| NM_182692 | 6733 | SRPK2 | SFRS protein kinase 2 | AACGGTGACTCTCATTCTTTA | CCGGTATCATGTTATTAGAAA |
| NM_182925 | 2324 | FLT4 | fms-related tyrosine kinase 4 | CACCGTGTGGGCTGAGTTTAA | CACGCTCTTGGTCAACAGGAA |
| NM_182948 | 5567 | PRKACB | protein kinase, cAMP-dependent, catalytic, beta | CAGCCTGTGTAGTGTGACAAA | CTGACCAATCAAGTACACTAA |
| NM_182982 | 2868 | GRK4 | G protein-coupled receptor kinase 4 | CAGGATGTTACTCACCAAGAA | CCGGGTGTTTCAAAGACATCA |
| NM_183048 | 23613 | PRKCBP1 | protein kinase C binding protein 1 | AGCGAACTTGTCATAGATTTA | CACCATTGAACAGTTATCCTA |
| NM_197972 | 29922 | NME7 | non-metastatic cells 7, protein expressed in (nucleoside-diphosphate kinase) | CCCGGCATTTACGCCCTGGAA | TTCATGTAGATCACCAGTCAA |
| NM_198452 | 139728 | MGC45419 | Similar to calcium/calmodulin-dependent protein kinase 1, beta | CTCGAAGATCATGGTCTCTGA | TCCGTGTGTCACAGCCCGAAA |
| NM_198828 | 375449 | LOC375449 | similar to microtubule associated testis specific serine/threonine protein kinase | ATGAATTGCCACCACATTAAA | GAGGAGCTTGACCACATATTA |
| XM_027237 | 4293 | MAP3K9 | mitogen-activated protein kinase kinase kinase 9 | AGCGATGAAATTGTCGTGTAT | CAGCGATGAAATTGTCGTGTA |
| XM_031706 | 23005 | MAPKBP1 | likely ortholog of mouse mitogen activated protein kinase binding proten 1 | CAGGACCGAAATATTCGGATA | CCCGTTTGTACTGATGTATGA |

Table 2

| NM_001556 | 3551 | IKBKB | inhibitor of kappa light polypeptide gene enhancer in B-cells, kinase beta | CAGACCGACATTGTGGACTTA | CAGGACACTGTGAGGGAACAA |
|---|---|---|---|---|---|
| XM_039796 | 23043 | TNIK | TRAF2 and NCK interacting kinase | AAGGACCATATTGATAGAACA | CTGGAATATAAGCGCAAACAA |
| XM_042066 | 4214 | MAP3K1 | mitogen-activated protein kinase kinase kinase 1 | CACGCATGTCAAATTCTCATA | CTCCGGGTGTTTCAACTAGAA |
| XM_051221 | 80309 | SKIP | SPHK1 (sphingosine kinase type 1) interacting protein | CTGAAATTCTGGACACCTTAA | GGCAAGTATGCTACAAATTTA |
| XM_166453 | 84630 | TTBK1 | tau tubulin kinase 1 | CAGGAACGAGAAGTTTAACTA | TGGCAGGAACGAGAAGTTTAA |
| XM_290516 | 55561 | HSMDPKIN | myotonic dystrophy protein kinase like protein | CGCCGGTGGCTTTGCACTCTA | CTCGCCGCCTACCAACTTCAA |
| XM_290793 | 8844 | KSR | kinase suppressor of ras | AAGGGCGAAGCTGGTCCGTTA | CTGGAAGTCAGCTGACATTAA |
| NM_012224 | 4750 | NEK1 | NIMA (never in mitosis gene a)-related kinase 1 | ACCGTAGGAGATGTTCGTCAA | TAGGGATAGACCATCAGTCAA |

Table3

| Pool No. | Accession # | LocusLink | Symbol | Gene Name |
|---|---|---|---|---|
| 1 | NM_005734 | 10114 | HIPK3 | homeodomain interacting protein kinase 3 |
|  | NM_181358 | 204851 | HIPK1 | homeodomain interacting protein kinase 1 |
| 2 | NM_000269 | 4830 | NME1 | non-metastatic cells 1, protein (NM23A) expressed in |
|  | NM_002512 | 4831 | NME2 | non-metastatic cells 2, protein (NM23B) expressed in |
|  | NM_002513 | 4832 | NME3 | non-metastatic cells 3, protein expressed in |
|  | NM_003551 | 8382 | NME5 | non-metastatic cells 5, protein expressed in (nucleoside-diphosphate kinase) |
|  | NM_005009 | 4833 | NME4 | non-metastatic cells 4, protein expressed in |
|  | NM_005793 | 10201 | NME6 | non-metastatic cells 6, protein expressed in |
|  | NM_197972 | 29922 | NME7 | non-metastatic cells 7, protein expressed in (nucleoside-diphosphate kinase) |
| 3 | NM_000291 | 5230 | PGK1 | phosphoglycerate kinase 1 |
|  | NM_138733 | 5232 | PGK2 | phosphoglycerate kinase 2 |
| 4 | NM_182471 | 5315 | PKM2 | pyruvate kinase, muscle |
|  | NM_181871 | 5313 | PKLR | pyruvate kinase, liver and RBC |
| 5 | NM_018323 | 55300 | PI4K2B | phosphatidylinositol 4-kinase type-II beta |
|  | NM_018425 | 55361 | PI4KII | phosphatidylinositol 4-kinase type II |
| 6 | NM_000289 | 5213 | PFKM | phosphofructokinase, muscle |
|  | NM_002626 | 5211 | PFKL | phosphofructokinase, liver |
|  | NM_002627 | 5214 | PFKP | phosphofructokinase, platelet |
| 7 | NM_002733 | 5571 | PRKAG1 | protein kinase, AMP-activated, gamma 1 non-catalytic subunit |
|  | NM_016203 | 51422 | PRKAG2 | protein kinase, AMP-activated, gamma 2 non-catalytic subunit |
|  | NM_017431 | 53632 | PRKAG3 | protein kinase, AMP-activated, gamma 3 non-catalytic subunit |
| 8 | NM_025144 | 80216 | LAK | lymphocyte alpha-kinase |
|  | NM_052947 | 115701 | HAK | heart alpha-kinase |
| 9 | NM_000189 | 3099 | HK2 | hexokinase 2 |
|  | NM_002115 | 3101 | HK3 | hexokinase 3 (white cell) |
|  | NM_033498 | 3098 | HK1 | hexokinase 1 |
| 10 | NM_002742 | 5587 | PRKCM | protein kinase C, mu |

Table3 (continued)

| | | | | |
|---|---|---|---|---|
| | NM_005813 | 23683 | PRKCN | protein kinase C, nu |
| | NM_016457 | 25865 | PRKD2 | protein kinase D2 |
| 11 | NM_002648 | 5292 | PIM1 | pim-1 oncogene |
| | NM_006875 | 11040 | PIM2 | pim-2 oncogene |
| 12 | NM_003936 | 8941 | CDK5R2 | cyclin-dependent kinase 5, regulatory subunit 2 (p39) |
| | NM_003885 | 8851 | CDK5R1 | cyclin-dependent kinase 5, regulatory subunit 1 (p35) |
| 13 | NM_013410 | 205 | AK3 | adenylate kinase 3 |
| | NM_016282 | 50808 | AK3L1 | adenylate kinase 3 like 1 |
| 14 | NM_000476 | 203 | AK1 | adenylate kinase 1 |
| | NM_174858 | 26289 | AK5 | adenylate kinase 5 |
| 15 | NM_004721 | 9175 | MAP3K13 | mitogen-activated protein kinase kinase kinase 13 |
| | NM_006301 | 7786 | MAP3K12 | mitogen-activated protein kinase kinase kinase 12 |
| 16 | NM_173598 | 283455 | KSR2 | kinase suppressor of Ras-2 |
| | XM_290793 | 8844 | KSR | kinase suppressor of ras |
| 17 | NM_002314 | 3984 | LIMK1 | LIM domain kinase 1 |
| | NM_005569 | 3985 | LIMK2 | LIM domain kinase 2 |
| 18 | NM_000142 | 2261 | FGFR3 | fibroblast growth factor receptor 3 (achondroplasia, thanatophoric dwarfism) |
| | NM_015850 | 2260 | FGFR1 | fibroblast growth factor receptor 1 (fms-related tyrosine kinase 2, Pfeiffer syndrom |
| 19 | NM_004438 | 2043 | EPHA4 | EphA4 |
| | NM_005233 | 2042 | EPHA3 | EphA3 |
| | NM_020526 | 2046 | EPHA8 | EphA8 |
| 20 | NM_004441 | 2047 | EPHB1 | EphB1 |
| | NM_004443 | 2049 | EPHB3 | EphB3 |
| | NM_017449 | 2048 | EPHB2 | EphB2 |
| 21 | NM_002645 | 5286 | PIK3C2A | phosphoinositide-3-kinase, class 2, alpha polypeptide |
| | NM_002646 | 5287 | PIK3C2B | phosphoinositide-3-kinase, class 2, beta polypeptide |
| | NM_004570 | 5288 | PIK3C2G | phosphoinositide-3-kinase, class 2, gamma polypeptide |
| 22 | NM_005026 | 5293 | PIK3CD | phosphoinositide-3-kinase, catalytic, delta polypeptide |
| | NM_006219 | 5291 | PIK3CB | phosphoinositide-3-kinase, catalytic, beta polypeptide |
| | NM_006218 | 5290 | PIK3CA | phosphoinositide-3-kinase, catalytic, alpha polypeptide |

Table3 (continued)

| 23 | NM_003629 | 8503 | PIK3R3 | phosphoinositide-3-kinase, regulatory subunit, polypeptide 3 (p55, gamma) |
|---|---|---|---|---|
| | NM_005027 | 5296 | PIK3R2 | phosphoinositide-3-kinase, regulatory subunit, polypeptide 2 (p85 beta) |
| | NM_181504 | 5295 | PIK3R1 | phosphoinositide-3-kinase, regulatory subunit, polypeptide 1 (p85 alpha) |
| 24 | NM_000167 | 2710 | GK | glycerol kinase |
| | NM_033214 | 2712 | GK2 | glycerol kinase 2 |
| 25 | NM_012474 | 7371 | UMPK | uridine monophosphate kinase |
| | NM_017859 | 54963 | URKL1 | uridine kinase-like 1 |
| | NM_031432 | 83549 | UCK1 | uridine-cytidine kinase 1 |
| 26 | NM_005399 | 5565 | PRKAB2 | protein kinase, AMP-activated, beta 2 non-catalytic subunit |
| | NM_006253 | 5564 | PRKAB1 | protein kinase, AMP-activated, beta 1 non-catalytic subunit |
| 27 | NM_020421 | 57143 | ADCK1 | aarF domain containing kinase 1 |
| | NM_024876 | 79934 | ADCK4 | aarF domain containing kinase 4 |
| | NM_052853 | 90956 | ADCK2 | aarF domain containing kinase 2 |
| | NM_174922 | 203054 | ADCK5 | aarF domain containing kinase 5 |
| 28 | NM_000292 | 5256 | PHKA2 | phosphorylase kinase, alpha 2 (liver) |
| | NM_002637 | 5255 | PHKA1 | phosphorylase kinase, alpha 1 (muscle) |
| 29 | NM_007229 | 11252 | PACSIN2 | protein kinase C and casein kinase substrate in neurons 2 |
| | NM_016223 | 29763 | PACSIN3 | protein kinase C and casein kinase substrate in neurons 3 |
| | NM_020804 | 29993 | PACSIN1 | protein kinase C and casein kinase substrate in neurons 1 |
| 30 | NM_080836 | 140901 | STK35 | serine/threonine kinase 35 |
| | NM_152835 | 149420 | LOC149420 | casein kinase |
| 31 | NM_001184 | 545 | ATR | ataxia telangiectasia and Rad3 related |
| | NM_006904 | 5591 | PRKDC | protein kinase, DNA-activated, catalytic polypeptide |
| 32 | NM_003648 | 8527 | DGKD | diacylglycerol kinase, delta 130kDa |
| | NM_178009 | 160851 | DGKH | diacylglycerol kinase, eta |
| 33 | NM_001345 | 1606 | DGKA | diacylglycerol kinase, alpha 80kDa |
| | NM_001346 | 1608 | DGKG | diacylglycerol kinase, gamma 90kDa |
| | NM_145695 | 1607 | DGKB | diacylglycerol kinase, beta 90kDa |
| 34 | NM_001258 | 1018 | CDK3 | cyclin-dependent kinase 3 |
| | NM_001786 | 983 | CDC2 | cell division cycle 2, G1 to S and G2 to M |

Table3 (continued)

| 35 | NM_003583 | 8445 | DYRK2 | dual-specificity tyrosine-(Y)-phosphorylation regulated kinase 2 |
|---|---|---|---|---|
|  | NM_003582 | 8444 | DYRK3 | dual-specificity tyrosine-(Y)-phosphorylation regulated kinase 3 |
|  | NM_003845 | 8798 | DYRK4 | dual-specificity tyrosine-(Y)-phosphorylation regulated kinase 4 |
| 36 | NM_001396 | 1859 | DYRK1A | dual-specificity tyrosine-(Y)-phosphorylation regulated kinase 1A |
|  | NM_004714 | 9149 | DYRK1B | dual-specificity tyrosine-(Y)-phosphorylation regulated kinase 1B |
| 37 | NM_003137 | 6732 | SRPK1 | SFRS protein kinase 1 |
|  | NM_014370 | 26576 | STK23 | serine/threonine kinase 23 |
|  | NM_182692 | 6733 | SRPK2 | SFRS protein kinase 2 |
| 38 | NM_004071 | 1195 | CLK1 | CDC-like kinase 1 |
|  | NM_020666 | 57396 | CLK4 | CDC-like kinase 4 |
| 39 | NM_005906 | 4117 | MAK | male germ cell-associated kinase |
|  | NM_014920 | 22858 | ICK | intestinal cell (MAK-like) kinase |
| 40 | NM_002093 | 2932 | GSK3B | glycogen synthase kinase 3 beta |
|  | NM_019884 | 2931 | GSK3A | glycogen synthase kinase 3 alpha |
| 41 | NM_001315 | 1432 | MAPK14 | mitogen-activated protein kinase 14 |
|  | NM_002751 | 5600 | MAPK11 | mitogen-activated protein kinase 11 |
|  | NM_002969 | 6300 | MAPK12 | mitogen-activated protein kinase 12 |
|  | NM_002754 | 5603 | MAPK13 | mitogen-activated protein kinase 13 |
| 42 | NM_002752 | 5601 | MAPK9 | mitogen-activated protein kinase 9 |
|  | NM_002753 | 5602 | MAPK10 | mitogen-activated protein kinase 10 |
|  | NM_002750 | 5599 | MAPK8 | mitogen-activated protein kinase 8 |
| 43 | NM_002745 | 5594 | MAPK1 | mitogen-activated protein kinase 1 |
|  | NM_002746 | 5595 | MAPK3 | mitogen-activated protein kinase 3 |
| 44 | NM_003718 | 8621 | CDC2L5 | cell division cycle 2-like 5 (cholinesterase-related cell division controller) |
|  | NM_016507 | 51755 | CRK7 | CDC2-related protein kinase 7 |
| 45 | NM_000075 | 1019 | CDK4 | cyclin-dependent kinase 4 |
|  | NM_001259 | 1021 | CDK6 | cyclin-dependent kinase 6 |
| 46 | NM_001260 | 1024 | CDK8 | cyclin-dependent kinase 8 |
|  | NM_015076 | 23097 | CDK11 | cyclin-dependent kinase (CDC2-like) 11 |
| 47 | NM_001787 | 984 | CDC2L1 | cell division cycle 2-like 1 (PITSLRE proteins) |

EP 2 295 543 A1

Table3 (continued)

| | | | | |
|---|---|---|---|---|
| | NM_003674 | 8558 | CDK10 | cyclin-dependent kinase (CDC2-like) 10 |
| 48 | NM_001825 | 1160 | CKMT2 | creatine kinase, mitochondrial 2 (sarcomeric) |
| | NM_001823 | 1152 | CKB | creatine kinase, brain |
| | NM_001824 | 1158 | CKM | creatine kinase, muscle |
| | NM_020990 | 1159 | CKMT1 | creatine kinase, mitochondrial 1 (ubiquitous) |
| 49 | NM_002736 | 5577 | PRKAR2B | protein kinase, cAMP-dependent, regulatory, type II, beta |
| | NM_004157 | 5576 | PRKAR2A | protein kinase, cAMP-dependent, regulatory, type II, alpha |
| 50 | NM_003384 | 7443 | VRK1 | vaccinia related kinase 1 |
| | NM_006296 | 7444 | VRK2 | vaccinia related kinase 2 |
| | NM_016440 | 51231 | VRK3 | vaccinia related kinase 3 |
| 51 | NM_001319 | 1455 | CSNK1G2 | casein kinase 1, gamma 2 |
| | NM_001893 | 1453 | CSNK1D | casein kinase 1, delta |
| | NM_001894 | 1454 | CSNK1E | casein kinase 1, epsilon |
| | NM_001892 | 1452 | CSNK1A1 | casein kinase 1, alpha 1 |
| | NM_004384 | 1456 | CSNK1G3 | casein kinase 1, gamma 3 |
| | NM_022048 | 53944 | CSNK1G1 | casein kinase 1, gamma 1 |
| | NM_145203 | 122011 | MGC33182 | casein kinase I alpha S-like |
| 52 | NM_002610 | 5163 | PDK1 | pyruvate dehydrogenase kinase, isoenzyme 1 |
| | NM_002611 | 5164 | PDK2 | pyruvate dehydrogenase kinase, isoenzyme 2 |
| | NM_002612 | 5166 | PDK4 | pyruvate dehydrogenase kinase, isoenzyme 4 |
| | NM_005391 | 5165 | PDK3 | pyruvate dehydrogenase kinase, isoenzyme 3 |
| 53 | NM_024594 | 79646 | PANK3 | pantothenate kinase 3 |
| | NM_024960 | 80025 | PANK2 | pantothenate kinase 2 (Hallervorden-Spatz syndrome) |
| | NM_138316 | 53354 | PANK1 | pantothenate kinase 1 |
| 54 | NM_016291 | 51447 | IHPK2 | inositol hexaphosphate kinase 2 |
| | NM_054111 | 117283 | IHPK3 | inositol hexaphosphate kinase 3 |
| | NM_153273 | 9807 | IHPK1 | inositol hexaphosphate kinase 1 |
| 55 | NM_002220 | 3706 | ITPKA | inositol 1,4,5-trisphosphate 3-kinase A |
| | NM_025194 | 80271 | ITPKC | inositol 1,4,5-trisphosphate 3-kinase C |
| 56 | NM_018979 | 65125 | PRKWNK1 | protein kinase, lysine deficient 1 |

Table3 (continued)

| | | | | |
|---|---|---|---|---|
| | NM_020922 | 65267 | PRKWNK3 | protein kinase, lysine deficient 3 |
| | NM_032387 | 65266 | PRKWNK4 | protein kinase, lysine deficient 4 |
| 57 | NM_001433 | 2081 | ERN1 | ER to nucleus signalling 1 |
| | XM_370946 | 10595 | LOC388226 | similar to protein kinase/ribonuclease IRE1 beta |
| 58 | NM_001262 | 1031 | CDKN2C | cyclin-dependent kinase inhibitor 2C (p18, inhibits CDK4) |
| | NM_001800 | 1032 | CDKN2D | cyclin-dependent kinase inhibitor 2D (p19, inhibits CDK4) |
| | NM_004936 | 1030 | CDKN2B | cyclin-dependent kinase inhibitor 2B (p15, inhibits CDK4) |
| 59 | NM_020639 | 54101 | ANKRD3 | ankyrin repeat domain 3 |
| | NM_178510 | 255239 | ANKK1 | ankyrin repeat and kinase domain containing 1 |
| 60 | NM_001348 | 1613 | DAPK3 | death-associated protein kinase 3 |
| | NM_004938 | 1612 | DAPK1 | death-associated protein kinase 1 |
| | NM_014326 | 23604 | DAPK2 | death-associated protein kinase 2 |
| 61 | NM_012290 | 9874 | TLK1 | tousled-like kinase 1 |
| | NM_006852 | 11011 | TLK2 | tousled-like kinase 2 |
| 62 | NM_004073 | 1263 | PLK3 | polo-like kinase 3 (Drosophila) |
| | NM_005030 | 5347 | PLK1 | polo-like kinase 1 (Drosophila) |
| | NM_006622 | 10769 | PLK2 | polo-like kinase 2 (Drosophila) |
| 63 | NM_000294 | 5261 | PHKG2 | phosphorylase kinase, gamma 2 (testis) |
| | NM_006213 | 5260 | PHKG1 | phosphorylase kinase, gamma 1 (muscle) |
| 64 | NM_004226 | 9262 | STK17B | serine/threonine kinase 17b (apoptosis-inducing) |
| | NM_004760 | 9263 | STK17A | serine/threonine kinase 17a (apoptosis-inducing) |
| 65 | NM_033118 | 85366 | MYLK2 | myosin light chain kinase 2, skeletal muscle |
| | NM_182493 | 91807 | LOC91807 | myosin light chain kinase (MLCK) |
| 66 | NM_003684 | 8569 | MKNK1 | MAP kinase-interacting serine/threonine kinase 1 |
| | NM_017572 | 2872 | MKNK2 | MAP kinase-interacting serine/threonine kinase 2 |
| 67 | NM_003668 | 8550 | MAPKAPK5 | mitogen-activated protein kinase-activated protein kinase 5 |
| | NM_004635 | 7867 | MAPKAPK3 | mitogen-activated protein kinase-activated protein kinase 3 |
| | NM_004759 | 9261 | MAPKAPK2 | mitogen-activated protein kinase-activated protein kinase 2 |
| 68 | NM_001220 | 816 | CAMK2B | calcium/calmodulin-dependent protein kinase (CaM kinase) II beta |
| | NM_001221 | 817 | CAMK2D | calcium/calmodulin-dependent protein kinase (CaM kinase) II delta |

Table3 (continued)

| | NM_015981 | 815 | CAMK2A | calcium/calmodulin-dependent protein kinase (CaM kinase) II alpha |
|---|---|---|---|---|
| | NM_172173 | 818 | CAMK2G | calcium/calmodulin-dependent protein kinase (CaM kinase) II gamma |
| 69 | NM_006742 | 5681 | PSKH1 | protein serine kinase H1 |
| | NM_033126 | 85481 | PSKH2 | serine/threonine kinase PSKH2 |
| 70 | NM_003656 | 8536 | CAMK1 | calcium/calmodulin-dependent protein kinase I |
| | NM_020397 | 57118 | CAMK1D | calcium/calmodulin-dependent protein kinase ID |
| | NM_020439 | 57172 | CAMK1G | calcium/calmodulin-dependent protein kinase IG |
| | NM_198452 | 139728 | MGC45419 | Similar to calcium/calmodulin-dependent protein kinase 1, beta |
| 71 | NM_003565 | 8408 | ULK1 | unc-51-like kinase 1 (C. elegans) |
| | NM_014683 | 9706 | ULK2 | unc-51-like kinase 2 (C. elegans) |
| 72 | NM_006549 | 10645 | CAMKK2 | calcium/calmodulin-dependent protein kinase kinase 2, beta |
| | NM_032294 | 84254 | CAMKK1 | calcium/calmodulin-dependent protein kinase kinase 1, alpha |
| 73 | NM_032037 | 83983 | SSTK | serine/threonine protein kinase SSTK |
| | NM_052841 | 81629 | STK22C | serine/threonine kinase 22C (spermiogenesis associated) |
| | NM_032028 | 83942 | STK22D | serine/threonine kinase 22D (spermiogenesis associated) |
| | NM_053006 | 23617 | STK22B | serine/threonine kinase 22B (spermiogenesis associated) |
| 74 | NM_003160 | 6795 | AURKC | aurora kinase C |
| | NM_003600 | 6790 | STK6 | serine/threonine kinase 6 |
| | NM_004217 | 9212 | AURKB | aurora kinase B |
| 75 | XM_374996 | 9891 | ARK5 | AMP-activated protein kinase family member 5 |
| | NM_030952 | 81788 | SNARK | likely ortholog of rat SNF1/AMP-activated protein kinase |
| 76 | NM_004954 | 2011 | MARK2 | MAP/microtubule affinity-regulating kinase 2 |
| | NM_018650 | 4139 | MARK1 | MAP/microtubule affinity-regulating kinase 1 |
| | NM_031417 | 57787 | MARK4 | MAP/microtubule affinity-regulating kinase 4 |
| 77 | NM_006251 | 5562 | PRKAA1 | protein kinase, AMP-activated, alpha 1 catalytic subunit |
| | NM_006252 | 5563 | PRKAA2 | protein kinase, AMP-activated, alpha 2 catalytic subunit |
| 78 | NM_018401 | 55351 | HSA250839 | gene for serine/threonine protein kinase |
| | NM_173575 | 282974 | PKE | PKE protein kinase |
| 79 | NM_004690 | 9113 | LATS1 | LATS, large tumor suppressor, homolog 1 (Drosophila) |
| | NM_014572 | 26524 | LATS2 | LATS, large tumor suppressor, homolog 2 (Drosophila) |

Table3 (continued)

| 80 | NM_007271 | 11329 | STK38 | serine/threonine kinase 38 |
|---|---|---|---|---|
|  | NM_015000 | 23012 | STK38L | serine/threonine kinase 38 like |
| 81 | NM_006258 | 5592 | PRKG1 | protein kinase, cGMP-dependent, type I |
|  | NM_006259 | 5593 | PRKG2 | protein kinase, cGMP-dependent, type II |
| 82 | AB023190 | 22983 | SAST | syntrophin associated serine/threonine kinase |
|  | NM_015112 | 23139 | MAST2 | microtubule associated serine/threonine kinase 2 |
| 83 | NM_001619 | 156 | ADRBK1 | adrenergic, beta, receptor kinase 1 |
|  | NM_005160 | 157 | ADRBK2 | adrenergic, beta, receptor kinase 2 |
| 84 | NM_002741 | 5585 | PRKCL1 | protein kinase C-like 1 |
|  | NM_006256 | 5586 | PRKCL2 | protein kinase C-like 2 |
|  | NM_013355 | 29941 | pknbeta | protein kinase PKNbeta |
| 85 | NM_002744 | 5590 | PRKCZ | protein kinase C, zeta |
|  | NM_002740 | 5584 | PRKCI | protein kinase C, iota |
| 86 | NM_002737 | 5578 | PRKCA | protein kinase C, alpha |
|  | NM_002738 | 5579 | PRKCB1 | protein kinase C, beta 1 |
|  | NM_002739 | 5582 | PRKCG | protein kinase C, gamma |
|  | NM_005400 | 5581 | PRKCE | protein kinase C, epsilon |
|  | NM_006254 | 5580 | PRKCD | protein kinase C, delta |
|  | NM_006255 | 5583 | PRKCH | protein kinase C, eta |
|  | NM_006257 | 5588 | PRKCQ | protein kinase C, theta |
| 87 | NM_002730 | 5566 | PRKACA | protein kinase, cAMP-dependent, catalytic, alpha |
|  | NM_002732 | 5568 | PRKACG | protein kinase, cAMP-dependent, catalytic, gamma |
|  | NM_002760 | 5616 | PRKY | protein kinase, Y-linked |
|  | NM_005044 | 5613 | PRKX | protein kinase, X-linked |
|  | NM_182948 | 5567 | PRKACB | protein kinase, cAMP-dependent, catalytic, beta |
| 88 | NM_002953 | 6195 | RPS6KA1 | ribosomal protein S6 kinase, 90kDa, polypeptide 1 |
|  | NM_004586 | 6197 | RPS6KA3 | ribosomal protein S6 kinase, 90kDa, polypeptide 3 |
|  | NM_014496 | 27330 | RPS6KA6 | ribosomal protein S6 kinase, 90kDa, polypeptide 6 |
|  | NM_021135 | 6196 | RPS6KA2 | ribosomal protein S6 kinase, 90kDa, polypeptide 2 |
| 89 | NM_001626 | 208 | AKT2 | v-akt murine thymoma viral oncogene homolog 2 |

Table3 (continued)

| | | | | |
|---|---|---|---|---|
| | NM_005163 | 207 | AKT1 | v-akt murine thymoma viral oncogene homolog 1 |
| | NM_181690 | 10000 | AKT3 | v-akt murine thymoma viral oncogene homolog 3 (protein kinase B, gamma) |
| 90 | NM_005627 | 6446 | SGK | serum/glucocorticoid regulated kinase |
| | NM_013257 | 23678 | SGKL | serum/glucocorticoid regulated kinase-like |
| | NM_016276 | 10110 | SGK2 | serum/glucocorticoid regulated kinase 2 |
| 91 | NM_003161 | 6198 | RPS6KB1 | ribosomal protein S6 kinase, 70kDa, polypeptide 1 |
| | NM_003952 | 6199 | RPS6KB2 | ribosomal protein S6 kinase, 70kDa, polypeptide 2 |
| 92 | NM_002082 | 2870 | GRK6 | G protein-coupled receptor kinase 6 |
| | NM_005308 | 2869 | GRK5 | G protein-coupled receptor kinase 5 |
| | NM_182982 | 2868 | GRK4 | G protein-coupled receptor kinase 4 |
| 93 | NM_003942 | 8986 | RPS6KA4 | ribosomal protein S6 kinase, 90kDa, polypeptide 4 |
| | NM_182398 | 9252 | RPS6KA5 | ribosomal protein S6 kinase, 90kDa, polypeptide 5 |
| 94 | NM_002497 | 4751 | NEK2 | NIMA (never in mitosis gene a)-related kinase 2 |
| | NM_003157 | 6787 | NEK4 | NIMA (never in mitosis gene a)-related kinase 4 |
| | NM_014397 | 10783 | NEK6 | NIMA (never in mitosis gene a)-related kinase 6 |
| | NM_024800 | 79858 | NEK11 | NIMA (never in mitosis gene a)- related kinase 11 |
| | NM_033116 | 91754 | NEK9 | NIMA (never in mitosis gene a)- related kinase 9 |
| | NM_133494 | 140609 | NEK7 | NIMA (never in mitosis gene a)-related kinase 7 |
| | NM_152720 | 4752 | NEK3 | NIMA (never in mitosis gene a)-related kinase 3 |
| | NM_178170 | 284086 | NEK8 | NIMA (never in mitosis gene a)- related kinase 8 |
| | NM_012224 | 4750 | NEK1 | NIMA (never in mitosis gene a)-related kinase 1 |
| 95 | NM_004783 | 9344 | TAO1 | thousand and one amino acid protein kinase |
| | NM_016151 | 9344 | TAO1 | thousand and one amino acid protein kinase |
| | NM_016281 | 51347 | JIK | STE20-like kinase |
| 96 | NM_002756 | 5606 | MAP2K3 | mitogen-activated protein kinase kinase 3 |
| | NM_002758 | 5608 | MAP2K6 | mitogen-activated protein kinase kinase 6 |
| 97 | NM_002401 | 4215 | MAP3K3 | mitogen-activated protein kinase kinase kinase 3 |
| | NM_006609 | 10746 | MAP3K2 | mitogen-activated protein kinase kinase kinase 2 |
| 98 | NM_005109 | 9943 | OSR1 | oxidative-stress responsive 1 |
| | NM_013233 | 27347 | STK39 | serine threonine kinase 39 (STE20/SPS1 homolog, yeast) |

Table3 (continued)

| 99 | NM_003618 | 8491 | MAP4K3 | mitogen-activated protein kinase kinase kinase kinase 3 |
|---|---|---|---|---|
| | NM_006575 | 11183 | MAP4K5 | mitogen-activated protein kinase kinase kinase kinase 5 |
| 100 | NM_004834 | 9448 | MAP4K4 | mitogen-activated protein kinase kinase kinase kinase 4 |
| | NM_015716 | 50488 | MINK | misshapen/NIK-related kinase |
| 101 | NM_002576 | 5058 | PAK1 | p21/Cdc42/Rac1-activated kinase 1 (STE20 homolog, yeast) |
| | NM_002577 | 5062 | PAK2 | p21 (CDKN1A)-activated kinase 2 |
| | NM_002578 | 5063 | PAK3 | p21 (CDKN1A)-activated kinase 3 |
| | NM_005884 | 10298 | PAK4 | p21(CDKN1A)-activated kinase 4 |
| | NM_020168 | 56924 | PAK6 | p21(CDKN1A)-activated kinase 6 |
| | NM_177990 | 57144 | PAK7 | p21(CDKN1A)-activated kinase 7 |
| 102 | NM_006281 | 6788 | STK3 | serine/threonine kinase 3 (STE20 homolog, yeast) |
| | NM_006282 | 6789 | STK4 | serine/threonine kinase 4 |
| 103 | NM_006374 | 10494 | STK25 | serine/threonine kinase 25 (STE20 homolog, yeast) |
| | NM_016542 | 51765 | MST4 | Mst3 and SOK1-related kinase |
| 104 | NM_003607 | 8476 | CDC42BPA | CDC42 binding protein kinase alpha (DMPK-like) |
| | NM_006035 | 9578 | CDC42BPB | CDC42 binding protein kinase beta (DMPK-like) |
| 105 | NM_004850 | 9475 | ROCK2 | Rho-associated, coiled-coil containing protein kinase 2 |
| | NM_005406 | 6093 | ROCK1 | Rho-associated, coiled-coil containing protein kinase 1 |
| 106 | NM_001743 | 805 | CALM2 | calmodulin 2 (phosphorylase kinase, delta) |
| | NM_005184 | 808 | CALM3 | calmodulin 3 (phosphorylase kinase, delta) |
| | NM_006888 | 801 | CALM1 | calmodulin 1 (phosphorylase kinase, delta) |
| 107 | NM_006383 | 10518 | KIP2 | DNA-dependent protein kinase catalytic subunit-interacting protein 2 |
| | NM_054113 | 117286 | CIB3 | calcium and integrin binding family member 3 |
| 108 | NM_003559 | 8396 | PIP5K2B | phosphatidylinositol-4-phosphate 5-kinase, type II, beta |
| | NM_005028 | 5305 | PIP5K2A | phosphatidylinositol-4-phosphate 5-kinase, type II, alpha |
| | NM_024779 | 79837 | PIP5K2C | phosphatidylinositol-4-phosphate 5-kinase, type II, gamma |
| 109 | NM_003557 | 8394 | PIP5K1A | phosphatidylinositol-4-phosphate 5-kinase, type I, alpha |
| | NM_003558 | 8395 | PIP5K1B | phosphatidylinositol-4-phosphate 5-kinase, type I, beta |
| | NM_012398 | 23396 | PIP5K1C | phosphatidylinositol-4-phosphate 5-kinase, type I, gamma |
| 110 | NM_001616 | 92 | ACVR2 | activin A receptor, type II |

Table3 (continued)

| | NM_001106 | 93 | ACVR2B | activin A receptor, type IIB |
|---|---|---|---|---|
| 111 | NM_000020 | 94 | ACVRL1 | activin A receptor type II-like 1 |
| | NM_001105 | 90 | ACVR1 | activin A receptor, type I |
| | NM_001203 | 658 | BMPR1B | bone morphogenetic protein receptor, type IB |
| | NM_004329 | 657 | BMPR1A | bone morphogenetic protein receptor, type IA |
| | NM_004302 | 91 | ACVR1B | activin A receptor, type IB |
| | NM_004612 | 7046 | TGFBR1 | transforming growth factor, beta receptor I (activin A receptor type II-like kinase, |
| 112 | NM_002005 | 2242 | FES | feline sarcoma oncogene |
| | NM_005246 | 2241 | FER | fer (fps/fes related) tyrosine kinase (phosphoprotein NCP94) |
| 113 | NM_000061 | 695 | BTK | Bruton agammaglobulinemia tyrosine kinase |
| | NM_001721 | 660 | BMX | BMX non-receptor tyrosine kinase |
| | NM_003215 | 7006 | TEC | tec protein tyrosine kinase |
| | NM_003328 | 7294 | TXK | TXK tyrosine kinase |
| 114 | NM_005157 | 25 | ABL1 | v-abl Abelson murine leukemia viral oncogene homolog 1 |
| | NM_005158 | 27 | ABL2 | v-abl Abelson murine leukemia viral oncogene homolog 2 (arg, Abelson-related g |
| 115 | NM_001715 | 640 | BLK | B lymphoid tyrosine kinase |
| | NM_002110 | 3055 | HCK | hemopoietic cell kinase |
| | NM_002350 | 4067 | LYN | v-yes-1 Yamaguchi sarcoma viral related oncogene homolog |
| | NM_002037 | 2534 | FYN | FYN oncogene related to SRC, FGR, YES |
| | NM_005356 | 3932 | LCK | lymphocyte-specific protein tyrosine kinase |
| | NM_005433 | 7525 | YES1 | v-yes-1 Yamaguchi sarcoma viral oncogene homolog 1 |
| | NM_005248 | 2268 | FGR | Gardner-Rasheed feline sarcoma viral (v-fgr) oncogene homolog |
| | NM_005417 | 6714 | SRC | v-src sarcoma (Schmidt-Ruppin A-2) viral oncogene homolog (avian) |
| 116 | NM_002344 | 4058 | LTK | leukocyte tyrosine kinase |
| | NM_004304 | 238 | ALK | anaplastic lymphoma kinase (Ki-1) |

Table4

| GeneID | GeneSymbol | GenbankID | Target Sequence 1 | Target Sequence 2 | Target Sequence 3 | Target Sequence 4 |
|---|---|---|---|---|---|---|
| 2 | A2M | NM_000014 | CTCAAAGATTCGTAAACCCAA | CACCCAACTCAAAGATTCGTAA | CACGGAGACCGTACGAAAGTA | ACGGATGAGCATGGCCTTGTA |
| 12 | SERPINA3 | NM_001085 | AAGGTTCTACTTGAGCAAGAA | AAGGCTGTGCTTGATGTATTT | CCCAAGATACTCATCAGTCAA | AAGGACCATTGTGCGTTTCAA |
| 14 | AAMP | NM_001087 | CAGGGAAGCCCTATCCATGTA | GAGGAAGAGATACTAGTTAAA | | |
| 20 | ABCA2 | NM_001606 | CCGCAGAGAGATGGACAAGAT | CTGGGTCAGTCCCGTATTTAT | CAGCCATGCCCACCTACCTCAA | CTGGAAGAACGTGACGCTCAA |
| 21 | ABCA3 | NM_001089 | CACGGAGAAATTACATGTGGA | CCAAGTTTATCTCATCCTTTA | TACGTTGTGACCATCCAGAAA | CCGCTTGAAGATTCAGTCGGA |
| 25 | ABL1 | NM_005157 | CTGGGCGAATGTCTTATTTAA | CCAGTGGAGATAACACTCTAA | ACGCACGGACATCACCATGAA | AACACTCTAAGCATAACTAAA |
| 29 | ABR | NM_001092 | CACGGACGTGATTGAGATGAA | CAGCGAAGAGATCTACATTAA | | |
| 30 | ACAA1 | NM_001607 | CACGCTGCTCAATGAGCTGAA | AAGGCCATGGTTAATTCTTAA | | |
| 31 | ACACA | NM_000664 | TCCGTAGGTGGTCTTATGAAA | TCGCATCACAATTGGCAATAA | AACGCAGGCATCAGAAGATTA | CTGGATAATCTGGTCAATGTA |
| 34 | ACADM | NM_000016 | AAGGAGATGAGTATATTATTA | AAGGTACTTCACAAATTCAAA | CTGGCTGAAATGGCAATGAAA | CAACTTGACAGTAACTTTGTA |
| 38 | ACAT1 | NM_000019 | CGGGCTAACTGATGTCTACAA | CCAGATGTAGTGGTGAAAGAA | AAGCTGAATATTGCACGAAAT | TAGTCTGGTTGTACTAGCAAA |
| 54 | ACP5 | NM_001611 | CACTGGTGTGCAAGACATCAA | ACGGCGCTGCTCATCCTGCAA | CTGGTACGTGCTAGCCGGAAA | CTCGGGCAAGTCCCTCTTTAA |
| 55 | ACPP | NM_001099 | ATGAACAGGTTTATATTCGAA | CTCTATTACCATTATGGATAA | CCGGACTTTGATGAGTGCTAT | ACAGATGGCGCTAGATGTTTA |
| 56 | ACRV1 | NM_001612 | TCCATAGATCATCAAACTTCA | ATGGAACGAGGATGCAAATTA | CCAGCAGTGCATGTTAAAGAA | CAGGCACAATATTAAATTGCT |
| 72 | ACTG2 | NM_001615 | CCGCTGGAATTCATGAGACAA | CAGGAAGTGCTTCTAAAGTCA | | |
| 94 | ACVRL1 | NM_000020 | CCGGGAGACTGAGATCTATAA | CACGGACTGCTTTGAGTCCTA | CAGGAAGACGCTGGAATAAGA | CACCGAGTTCGTCAACCACTA |
| 101 | ADAM8 | NM_001109 | CCGGCTACACAGAGACCTATA | AGGCATCATCGTCTACCGCAA | CAAGCTATATCAGAAACTCAA | CCCAGCTTTGTGTGTGTTTAA |
| 102 | ADAM10 | NM_001110 | ATGGTGTTGCTGAGAGTGTTA | ACCAAACTTCACAGACTGTAA | ATGGTAAGAAGAAGTCCTTAA | ATGGGACACATGAGACGCTAA |
| 112 | ADCY6 | NM_015270 | AAGTGCTTAGGTAATGAGCTA | CAGCCTGAGTCTAGCATGTTT | CAGCCCGGATGCTGAATTTAA | ACCATGTATTTCAGTGAAATA |
| 123 | ADFP | NM_001122 | AAGAATGTGTATAGTGCCAAT | TCCGTTGCAGTTGATCCACAA | CTGGTTCAGAAGCCAAGTTAT | CTGCAGGATAGACCAGTTAAA |
| 125 | ADH1B | NM_000668 | AACCAATCCATTACAGCTTAA | AAGCCTCAATTACTAATTGTA | AAGGAGGTAGCTGTCTCCAAA | AAGACCTTAGACATAAAGTAA |
| 126 | ADH1C | NM_000669 | ATGCATTAATAACAAATATTT | GGGAAATTATTTGTCAAGCAA | CTGCTCTTCAGAGATGTTAAA | ACTGGATGCATTAATAACAAA |
| 131 | ADH7 | NM_000673 | CACCAGTACATTTACCGAGTA | CAGATATAGCGTATAAAGATA | CTGGGAATTAGTATAATAACA | AACGATTAAGAATCATCATTA |
| 132 | ADK | NM_001123 | TCCACTGATGGAAGAGCTGAA | AAGTTGTACCACAATATTCAA | TAGTTTGATAGTGCCACTTAA | AAGGGTATGGTAATGCTTGTA |
| 133 | ADM | NM_001124 | AGAGGTAACTATCAATATTTA | ACCGAGTCTCTGTATAATCTA | GAGGTAACTATCAATATTTAA | CTGTATAATCTATTTACATAA |
| 135 | ADORA2A | NM_000675 | CCACACCAATTCGGTTGTGAA | AAGGGCTTGGGTTCTGAGGAA | CAGGAGTGTCCTGATGATTCA | CAGCCATGAGGCCCAGCAAGAA |
| 140 | ADORA3 | NM_000677 | CAACTGCATCATCTACTTTAA | CCCTATCGTCTATGCCTATAA | TCGGAACAAACTCAGTCTGAA | AAGCGTCAACTCGTGCAAGAA |
| 141 | ADPRH | NM_001125 | CTGGTCTACCTCCTAAATATA | CAGAGTTATGTTTACGAATAA | | |
| 142 | PARP1 | NM_001618 | CTTGAAATATGTAGTATTAAA | CCCGGTGGCTGTGGTATGAAT | CCGAGAAATCTCTTACCTCAA | ACGGTGATCGGTAGCAACAAA |
| 147 | ADRA1B | NM_000679 | GGCGGTCATTCTAGTCATGTA | CAGGAGTTCCATAGCTGTCAA | GAGGGCGGACTCTAAGATGAA | CCGTGTCTATATAGTGGCCAA |
| 150 | ADRA2A | NM_000681 | CCCGCTGTAAATATACACTAT | GTGGATCAAGACATAAGTAAA | CTCCCGCCGCTTAGAAATAAA | CAAGGCTTGGTGCGAGATCTA |
| 156 | ADRBK1 | NM_001619 | CAGGCCCTTGGTGGAATTCTA | CGGCTGGAGGCTCGCAAGAAA | CGGCTGGAGGCTCGCAAGAAA | CCGGGAGATCTTCGACTCATA |
| 157 | ADRBK2 | NM_005160 | AAGCTACTTGATTGCGACCAA | TGGGATTAACTAATCATTGAA | CAAGTGTATGGGATTAACTAA | AAGATGTTCAGTGTTGGGTAA |
| 166 | AES | NM_198970 | TAGCTGTGCCTTTCCAATAAA | CGGCTTGAACATCGAGATGCA | CTGACATGTGAGGCAAGCTAA | CAGGCCCACCTCTCCAAGGAA |
| 177 | AGER | NM_172197 | TAGCTCCTGGTGGAACCGTAA | CACGGCTGGTGTTCCCAATAA | CTGTATAATCTCTCTCCTGTA | AAGGGAGTATCTGTGAAGGAA |
| 181 | AGRP | NM_007316 | CTGAAGAAGACAACTGCAGAA | CAGCCGCACCTAGCTGGCCAA | AAGCTGGGTACTGCCATGAAT | CAAGCTGGGTACTGCCATGAA |
| 183 | AGT | NM_000029 | ACCGACCAGCTTGTTTGTGAA | CTGTTTGAATGCGGAACAATA | CTCCGTGTAGTGTCTGTAATA | CAGAGTCTACCCAACAGCTTA |
| 185 | AGTR1 | NM_000685 | ATCCAAGATGATTGTCCCAAA | CTGGATGTATTGATTCAACTA | CGCGGGTTTGATATTTGACAA | ACGCACAATGCTTGTAGCCAA |
| 187 | AGTRL1 | NM_005161 | CACCTGCATCAGCTACGTCAA | CACCATCATGCTGACCTGTTA | CACTGTGGTTCATCAAATAAA | CACCACTAAGGTGCAGTGCTA |
| 189 | AGXT | NM_000030 | CCGAGTGCACCCGATGACCAA | CTGGAGAGACATCGTCAGCTA | CCAGGATGTACCATCACACAA | CTCCCGGGAATGTTTAATAAA |
| 190 | NR0B1 | NM_000475 | ACGGCAGGGCAGCATCCTCTA | ATGGATGATATGATGCTGGAA | ATCGAACTTAATAGTACCCTT | ATGCTCTGTACAAAGATATAA |
| 197 | AHSG | NM_001622 | CCAGGGCTGATTTATAGACAA | CTGAAAGACCTTCCTTAATAA | AAGCTTATATGTGCCTGTTAA | CACATGCGATCTACATTAATA |

Table4

| # | Gene | Accession | Sequence |
|---|---|---|---|
| 199 | AIF1 | NM_004847 | CCCAGATATCATGTCCCTGAA CAGAGTCAACAAATTAAATAA AAGCCAGAGTCAACAAATTAA CACCTAGCAGTTGGTTGGCAA |
| 207 | AKT1 | NM_005163 | CAGGGTTTACCCAGTGGGACA AAGGTTTAAATTTGTTATTGT CACGCTTGGTCCCGAGGCCAA ACGCTACTTCCTCCTCAAGAA |
| 210 | ALAD | NM_000031 | CAGGGTATCGGTGATGAGCTA AAGGGTGAGCCATCAAGCTAA CCCAAGAACTAGAACTTTAAA CTCCCAATAATCAACAGCAAA |
| 215 | ABCD1 | NM_000033 | CCGCACCTTCCTGCGGTGTA CACCTCTTCTACAGCCTAA CTCGGAGGAGATCGCCTTCTA CTCTTTCTACAGCCTAATTTA |
| 217 | ALDH2 | NM_000690 | CAGGCATACACTGAAGTGAAA CACAGTCAAAGTGCCTCAGAA AAGCTATTGTTTACAATTATA CAGCCATTGATGAAAGTTCA |
| 219 | ALDH1B1 | NM_000692 | CTTGGATTACTCAGCCATAAA TTGAACTAGGTGGATACTAAA CTCAGAGTTCTACCTATCTAA CAGCAGTGTGGGTCTGACAAA |
| 220 | ALDH1A3 | NM_000693 | TTGGAAAGGATTCACAGTAA ACCAGTGTGTGTATAACGAAA AACGGTCTGGATCAACTGCTA AACCTTGATAGTGATACGTTA |
| 222 | ALDH3B2 | NM_000695 | CTCCGGCCTGGAGAAATTAAA CACGGCCGTCTCCACCAGAAA CAGGCCTGGATGAAGGATGAA CCCACCCTATACCGACTGGAA |
| 225 | ABCD2 | NM_005164 | CAGTGACACATTGCAATTAA CAGCATTGATGTCGAAGGAAA CACTCGAGAATTATAGCCAAT TCGCCTAGTAGACCACGCCTA |
| 226 | ALDOA | NM_000034 | CTGTCTCTGCTTCCGAGATCAA TCCGCACACTGCCAAATAAA CACGTCAACGATTCTATTTGA GCCAAATAAACAGCTATTTAA |
| 239 | ALOX12 | NM_000697 | CAAGCCCAAAGCTGTGCTAAA CCCAAAGTCTGCTAAACCAA CTGGCCCTATGAATATCTGAA CTGGTATGCCTGGGTCCCTAA |
| 248 | ALPI | NM_001631 | CCGGGCTACGTTGTTCAACTCA CCAGACAATAAAGGACCAAA TCCATTCTCCTAGGAGACAAA CACGTCCATCCTGTACGGCAA |
| 258 | AMBN | NM_016519 | CACCATCAGATAAGCCACCAA TACCAGCATGACACTATTATA |
| 262 | AMD1 | NM_001634 | CGGGAGAAGAGGTTTAATTTA CAAACAGAAACTTGCACTGAA TGGGTTTATATACTAATTCTA AAGGTGGACCTTGTTGCTAAA |
| 265 | AMELX | NM_001142 | ATCCCTGAGTTTCAAACAGAA CAAACAGAAACTTGCACTGAA TCCCTGAGTTCAAACAGAAA CAGGAGTGACACAGAACACA |
| 268 | AMH | NM_000479 | CCAATAAAGACCAGCAAGCAA CCACGTGGTGCTGCTGCTGAA CGCGTGGCTGCGAACTGCAA |
| 269 | AMHR2 | NM_020547 | CACGACCACATTGTCCGATTT ATGGCCAATATAAACCAGTA CTGGCAGAATGGCCAATATAA CAGAATGTGCTCATTCGGGAA |
| 270 | AMPD1 | NM_000036 | AACCTGGGCTATCACCTCAAA CGCCATGTCCACCTAAGTAA CACCTTCTTAGACGATATGAA TTGCTGAGGGTCTTAAATCAA |
| 271 | AMPD2 | NM_004037 | CAAGTTTAATGCCAAATACAA CGGCTTCTCGCACAAGGTAA CCGGACCATGTCCATCTCTTA CCGGACCAATGTGCCAGACAT |
| 283 | ANG | NM_001145 | CCGGGATGACAGATACTGTGA CAGCATCAAGGCCATCTGTGA CGGGATGACAGATACTGTGA AACGTTGTTGTGTTGCTTGTGAA |
| 287 | ANK2 | NM_001148 | AAGCTTGGTTACACACCTTTA CAGGACCCTTCTAAACATAAA ATCAAGCTATTGCAACTTAA ACCGTGCAAACGGGTGATATA |
| 288 | ANK3 | NM_001149 | CAGGATTATCACGAAAGATTT CGCATCCTTAGCTTTACGTAA AAGGATCTTAACCTCAATTAA CTAGTCGATTATTAAGCCATA |
| 290 | ANPEP | NM_001150 | CACCACCTTGGACCAAAGTAA CCGGGTGAACTACGACGAAGA AACGATCTCTTCAGCACATCA CCGAAATGCCACACTGGTCAA |
| 291 | SLC25A4 | NM_001151 | CTGTATCATCAAGATCTTCAA CAGATCCATTGTGTGGTTTAA TACAGTGAACTTGATCTACAA CTGAATGTGAAACATCAATAA |
| 292 | SLC25A5 | NM_001152 | CAGGCATGTTGTATTCTATAA CGGAAGATTGCTCGTCGTGAA CACAATCTTGAGCATTCTTGA ATGTTGTATTCTATAACACAA |
| 310 | ANXA7 | NM_001156 | CAGAAATTCTTCGTAAGGCAA CTGTGTAATAATATTGAATAA |
| 312 | ANXA13 | NM_001003954 | CAGGCCAAGCTGTGTAAGTTA CAAGAGCACATTCCAAATCAA |
| 317 | APAF1 | NM_001160 | TAGGCAGAGTATAAAGTATTA CAGTGAAGGTATGGAAATATTA CCGCATTCTGATGCTTCGCAA AAGGGCAATGGAGATAAATTA |
| 320 | APBA1 | NM_001163 | CAGGATCAAGAATGGCCCCAGAA CAGGAAGAGAAGAATCCACAA |
| 324 | APC | NM_000038 | CAGAGACTAATGAAAGTTCTA CCGGTGATTGACAGTGTTCA TCCATCTAACATCATAATTAA CAGACTAAGCATTGAGCATAA |
| 327 | APEH | NM_001640 | CCGCCAATACCTGGTGTTCCA CAGGAGGAACACTTTGATGCA CTGGAAGTTGCTCACAATTGA CAGAGGAATGTGGGCTATGTA |
| 329 | BIRC2 | NM_001166 | CAGGGCTTTAAGTTAGTATTA TCCCAGGTCCCTCGTATCAAA TCGAAACTTTATAAAGGGATA TAGGCGTTTCTGATAACACTA |
| 336 | APOA2 | NM_001643 | ATCAAGAAGGCTGGAACGGAA CCCACTCTTTGCTACAATAAA TACAATAAATGCTGAATGAAT AGGCCAAGTCTTACTTTGAAA |
| 339 | APOBEC1 | NM_001644 | CACCAATCACGTGGAAGTTAA CACCCTGTTTAAAGATTTCAA |
| 345 | APOC3 | NM_000040 | AAGACTACTGGAGCACCGTTA CTGGAGCACCGTTAAGGACAA CAGGAACAGAGGTGCCATGCA CCCAATAAAGCTGGACAAGAA |
| 350 | APOH | NM_000042 | ACACATGGAAATTGGACTAAA ACCCAATTAAGTCCTACTTTA CAAGTTGTAAAGCATCTTGTA |
| 351 | APP | NM_000484 | CTGTCTTCAATTACCAAGAA ACCCAATTAAGTCCTACTTTA CAGCTGAACATGACACCATGAA AAGGATGAACTACAGACATTAA |
| 353 | APRT | NM_000485 | CACACTGAACCAATTACACA CTGTGTTTCAGCCACACTGAA CAGCCACACTGAACCCAATTA |
| 355 | FAS | NM_000043 | TCAGTGTATGTTAGTACAAAT CACATTGATTAAAGATCTCAA CAGAATTAAATAAGGCTCTA AAGGACATTACTAGTGACTCA |
| 366 | AQP9 | NM_020980 | CAGCCTCTAATTGAACGGCA CAACAAACCCTAAATTGAAA AACCGTCTTTGGCATTTACTA ACGACTGTGCTTGTCCATTAT |
| 367 | AR | NM_000044 | AAGAAACTTGGTAATCTGAAA CACGGGAAGTTTAGAGAGCTA CAGGAATTCCTGTGCATGAAA AAGGAACTCGATCGTATCATT |
| 368 | ABCC6 | NM_001171 | CTGAGGGAATCCACACTTCAA ACGGTTGACGTGGACATTCCA CACCCATTGGTCACCTGCTAA CACGTGTGTGCTGAGCAA |
| 372 | ARCN1 | NM_001655 | AAGGCAAATTCTTAATCAAA CCCACTTGTGTCAATATTAAA |

Table4

| | | | | | | |
|---|---|---|---|---|---|---|
| 375 | ARF1 | NM_001658 | AAGCTGGAGCTGTTAAATTTA CACGATCCTCTCACAAGCTTAA | | | |
| 382 | ARF6 | NM_001663 | CTCAGTATGTTAAACAGGTAA ATGGTTAACCTCTAACTACAA | | | |
| 388 | RHOB | NM_004040 | CTGCTGATCGTGTTCAGTAA CCCAGTGGTACTTCTACTAAA AGCTAAGATGTGTTATTTAA CACAATTGTTTCATTGTTTGA | | | |
| 389 | RHOC | NM_175744 | CAGGAGAGAGCTGGCCAAGAT CCCTACTGTCTTTGAGAACTA CCGGATCAGTGCCTTTGGCTA CACCATGGCTGCAATCCGAAA | | | |
| 391 | RHOG | NM_001665 | CAACAGGATGGTGTCAAGGAA CGGGCGCCACAGTGAACCTGAA | | | |
| 397 | ARHGDIB | NM_001175 | AAAGGAAACCATTGTGTTAAA CACTGAATAAATAGATCAGAA AAGGAAGGTTCTGAATATAGA CACAGCTGGGTCCCTCTTCAA | | | |
| 401 | PHOX2A | NM_005169 | CCAGCAGAGCGGAGTTCTTTA CAGAGGGATTCTCTTTCCCTA | | | |
| 403 | ARL3 | NM_004311 | CAGACCTGTAAGGACTTGCAA ACCGATATTCTTATATATGTA ATCTGACATGAAAGTCATTAA AGGGAACAACACGGTTTAGAA | | | |
| 405 | ARNT | NM_001668 | AACCCGAGACTTGGCCATAAA ACGGAACAAGATGACAGCCTA CCCAGCCAATATACAACTGTA CAGCCAATATACAACTGTAAA | | | |
| 406 | ARNTL | NM_001178 | TACGTTTGGACCCAAGCTTAA TAGGCACATCGTGTTATGAAT TCGGCACGCGATAGATGGAAA TCGATGGTTCAGTTTCATGAA | | | |
| 410 | ARSA | NM_000487 | CTGGGTCTGCGGTATCGGAAA CAGGGCTTCCATCGATTTCTA CCCGGTTCGATGGGCATGTA TCGGCAGTCTCTCTTCTTCTA | | | |
| 411 | ARSB | NM_000046 | AAGGGTATGGTCTCTAGGCAA CACATTTCTTACGCAAATGAA ATGGGTGTACACTACGCATAA AACCAACTTTATAGTACATAA | | | |
| 414 | ARSD | NM_001669 | ATGGAGGACATTTAGAGGCAA AAGGTTCTTAATGCCATCGAA AACAGCGAGTCTTATGCTAAA TCGGTTGCTACGGAACAATA | | | |
| 415 | ARSE | NM_000047 | CCGCTGGGAACTCTCAGAGAA CACGAGTTCCTGATGCATTAT CCCTCTTATCACTATGGAGAA AGGGAAGATGACCCACAATAA | | | |
| 416 | ARSF | NM_004042 | CGCGTTCTTGACGGGAAGATA CTGAACGAGCTGGATCCATTA CAGGTATGGTTTCTAGTGGTA ACGGAGGGCATTGGAAGCTA | | | |
| 421 | ARVCF | NM_001670 | CACGGAGAGCCGGAACTTCAA CCGGAAGGACACTGACAACAA | | | |
| 427 | ASAH1 | NM_004315 | CACGGATGGCACGAAAGCCAA CACGTTCTGTTAATTTAGTAA ATGGTATGTGGTACAAACAAA CACGATTAACTGTGAAATGTA | | | |
| 430 | ASCL2 | NM_005170 | CACGCTTTGGGCCTTACAGAA GAGGGTTATCTATACATTTAA AAGGAAGGAGGGCGTGAGAAA AGGGAGCGTGAACTTTATAAA | | | |
| 432 | ASGR1 | NM_001671 | CTCCTTTAATTTATTTCTTCA TCCGAAGGTGAGGAGCTTGAA CAGGAGCCACCTCCTTTAA CAGTTTATCATTGTGAACTTT | | | |
| 433 | ASGR2 | NM_080914 | CTGTAATTCATTGTAATTATT GAGCAGAAATTCATTGTACAA CAGACTATAGGCACAACTACA CACGAGCTGGGTGAAGTGAA | | | |
| 443 | ASPA | NM_000049 | AACCGGAGTATTTCTGGTAA CTGCGCCATTGAGGTCTATAA TACGTTTATCTGATTGAGCAT TACATCTTACACGGTGTCTTA | | | |
| 472 | ATM | NM_000051 | TTGGCTTATACGCGCAGTGTA CACTTATTCATTAGTAATTTA CACCTGTTTGTTAGTTTATTA AACCATGAGTCTAGTACTTAA | | | |
| 473 | RERE | NM_012102 | CAAGAGAGAGTGAGAATTCAA CAGGAGATTATTACAGTCGA | | | |
| 477 | ATP1A2 | NM_000702 | ACCCATAGCAATGGAGAATTGA CCGATTAATTGGAGATTACTA AACAATCAGATTAGACACTAT CAGATTAGACACTATGTGTTA | | | |
| 480 | ATP1A4 | NM_144699 | CTGGTTTATGCTGGCCCGAAT CTCAATGAACGATGAAATGAA AAAGGGAGACGTACTACTACAA CCGGGCTGACTTTAAGGCTAA | | | |
| 483 | ATP1B3 | NM_001679 | CCGCATTGGAATATACATTCA CAGACTGTGGACTGTAATAAA | | | |
| 486 | FXYD2 | NM_001680 | CACCAGCCTCTCCAAGCTCAA CCCGTTCTACTATGACTATGA CCACCTCATGCTTATAATAAA ATCAATGAAGATGAGCCGTAA | | | |
| 487 | ATP2A1 | NM_004320 | CCCGTGTCATGTGTCTGTTTA CCCGATAGTGACACATCTTCA CACCAACATTGCAGCCGGCAA TCGGAACTACCTAGAGGGATA | | | |
| 492 | ATP2B3 | NM_021949 | CCCTCTTTATAAAGCATTATA CCGGAAGACAAGCTTTACAAA TTCCCTGATGTCCTTGTTTAA CACAGATACGTAATAATTTAA | | | |
| 501 | ALDH7A1 | NM_001182 | CGGGAAGAGATCCAAGTACTA TCCGATTCTCTATGTCTTTAA AAGGATGATTGGAGGACCTAT AAGGTCTACTTGTACTATCAA | | | |
| 506 | ATP5B | NM_001686 | AACAGTATTTAAGGTTTCCAA AAGAATTACCCACTACCAAGAA ATCTGGTGTTATCAACCTTAAA ACCCGTGAAGGCAATGATTTA | | | |
| 509 | ATP5C1 | NM_001001973 | CAGGTCTGTCATCTCCTATAA CAGCAAGGATATTTGTAAATTA | | | |
| 523 | ATP6V1A | NM_001690 | TAAGGTAGAGTCAATTATGAA ACCCAAATTGTGATAGCATAA GAGCTTGAATTGAAGGTGTA ATGGAGGTTGATGGTAAGGTA | | | |
| 527 | ATP6V0C | NM_001694 | CTGGATGTTTATTTATAAAGA CAGCCACAGAAATATTATGTAA | | | |
| 533 | ATP6V0B | NM_004047 | CAGCTGGAGCTGTGTCCCTTA TCCTAGTGTTTGTGAAATAAA | | | |
| 535 | ATP6V0A1 | NM_005177 | AAGGTTCAGTTTCGTGACTTA ACGGCCGATGTTTACTTATAA | | | |
| 537 | ATP6AP1 | NM_001183 | CACAGTGACATTCAAGTTCAT CAGGGAAGTCCTCACAGGCAA | | | |
| 538 | ATP7A | NM_000052 | CAGCAAGTCACTTAGATCATA GCGCCAGTTATTCTTCCATTA CCGCTAATCTTCTAACATTA CGGCACTACTTGTTCTTCAA | | | |
| 545 | ATR | NM_001184 | AAGGACATGTGCATTACCTTA CCGGTGGTGAACATAAGAAA CCGCTAATCTTCTAACATTA CAGGCACTACTTGTTCTTCAA | | | |
| 546 | ATRX | NM_000489 | ACCGGTGGTGAACATAAGAAA CCGACAGAAACTAACCCTGTA CCGGTGGTGAACATAAGAAAT AGGCGACGTATTAAGGTTCAA | | | |
| 547 | KIF1A | NM_004321 | TCGGATCAACGACACGACCAA CAGCCCGATCGTTTCCAAGAA TCGGGTCATGCCACTGTCCAA CAACTCGGAAATTAACTATAA | | | |
| 552 | AVPR1A | NM_000706 | CAGAAATACAACAGAAGTTAT CACTTTATTAATATAACCATA TCGCAAACATAGGTAATAAA ATGAACGGACTGGATAGTAAA | | | |
| 567 | B2M | NM_004048 | CAGGTTTACTCACGTCATCCA TCCGACATTGAAGTTGACTTA CTGGGTTTCATCCATCCGACA AAGTGGGATCGAGACATGTAA | | | |

Table4

| 570 | BAAT | NM_001701 | TTGGCTTACCATAACTATGAA ATGGCTATTTACCTAAAGCAA |
| 576 | BAI2 | NM_001703 | CCCACTGCATATAAATATATA CGAGGTCTTGCTCATCAACAA CTCTCTGATTGTCACAGATAA CCCGAACGAAAGGATACGGTA |
| 577 | BAI3 | NM_001704 | TTCGGGATATACCAAATACAA GAGATCCATAATACTAATTAA TTGGATGATAATGCAGGACTA ATGGACTAAGGTAGAGACAAA |
| 578 | BAK1 | NM_001188 | CTCTGAGTGTTTGGAAATAAA CAGGATTCAGCTATTCTGGAA CTGGACTTAAGCGAAGTCTTT AACCCATTCACTACAGGTGAA |
| 580 | BARD1 | NM_000465 | TCCGATGAATTCATTAGTCTT GACCATCAATACAGTCGCATA TGCAGTAATTCTTAAGGCTAA CTGAATATTATACCAGATGAA |
| 581 | BAX | NM_004324 | CCGAGTGGCAGCTGACATGTT CAGCTCTGAGCAGATCATGAA CAGGGTTTCATCCAGGATCGA ATCATCAGATGTGGTCTATAA |
| 587 | BCAT2 | NM_001190 | CCGGCTCATCGAAGTGGACAA CACCTGAAGTGCAATACGAAA AACCATGAACATCTTTGTCTA CTGAAGTGCAATACGAAATAA |
| 594 | BCKDHB | NM_000056 | CAGGCCGAAGTCATACAGGAA CTGGAAGATTATGACTAGATA |
| 595 | CCND1 | NM_053056 | ATGCATGTAGTCACTTTATAA CTCCTACGATACGCTACTATA AGGGTTATCTTAGATGTTTCA AAGGCCAGTATGATTATAAA |
| 596 | BCL2 | NM_000633 | GTGGATGACTGAGTACCTGAA CCGGGAGATAGTGATGAAGTA CTGTGGCATTATTGCATTATA CAGGCTTAAGATTTGGAATAA |
| 598 | BCL2L1 | NM_001191 | CAAGCTTTCCCAGAAAGGATA AAGGCGGATTTGAATCTCTTT CTGCTTGGGATAAAGATGCAA AAAGTGCAGTTCAGTAATAAA |
| 602 | BCL3 | NM_005178 | CTCGACATCTACAACAACCTA CACGATGTAAATTATTAAGCA CAACGTGAACGCGCAAATGTA ACGGATCACGATGTAAATTAT |
| 605 | BCL7A | NM_020993 | TACGATGCTGATCATCATTGA CTGCACTTCGAGAAGAATCAA CAGCACCTTCCTACAACCCTA TGGAATGTTCATTATCGCCAA |
| 611 | OPN1SW | NM_001708 | TTGGCCTGTTTGCAACAGCTA GCGCTACATTGTCATCTGTAA CGCCATGTACATGGTCAACAA CAAGAGTGCTTGCATCTACAA |
| 613 | BCR | NM_004327 | CAGCCGCAACGGCAAGAGTTA TGAACTTAACTTAGAGTCTAA CAGCATTCCGCTGACCATCAA ACGGCAGTCCATGACGGTGAA |
| 622 | BDH | NM_004051 | TGGCTGCTTGATGAAGGACAA CAGCAGCGAAGAGGTGGAGAA CTCAATGTCTGCAGCAGCGAA CAAGCAGGTGGCAGAAGTGAA |
| 624 | BDKRB2 | NM_000623 | ATGAGTTGATGTCTCCGGTAA GCCCTGGGTTTCTTTAATCTA CTGGCAGGTTAGAACCTAGAA CTGCACGGTGGCAGAGATCTA |
| 638 | BIK | NM_001197 | GAGGAGAAATGTCTGAAGTAA TCGGCAAAGAAATCTACTGGAA CCACACTTAAGGAGAACATAA CCGAGGAGCAGGAGTGCTCAA |
| 640 | BLK | NM_001715 | AAGGTGGTTCTTTAGATCACA CAACATGAAGGTGGCCATTAA CTGGTAAGCGACTGTCATCAA CAGCCGTGCTGTTAAACCAAA |
| 641 | BLM | NM_000057 | GACGCTAGACAGATAAGTTTA CTGACCATCTGTGACTATAAA ATGCTCGGAAATAAAGCCCAA CCGAATCTCAATGTACATAGA |
| 643 | BLR1 | NM_001716 | AACGCTGGAAATGGACCTCGA ACGGAGAAAGGTGGACTGGAA CCCGCTAACGCTGGAAATGGA CAGCCATGAACTACCCGCTAA |
| 644 | BLVRA | NM_000712 | TCCAGAAATATTGCTGTTCAA ACCATTATCTCTATTCTTAAA |
| 649 | BMP1 | NM_001199 | AAAGGGCTTCTTTGCAGTCTA CAGCTATATTGTGTTCACCTA CTGGCTCAAGTTCGTCTCTGA AAGGACTATGGCCACATTCAA |
| 651 | BMP3 | NM_001201 | CCCAGCTGGTTTGGAGTTCAA CCGCCATTTCTGAGCCAGAAA CTGTCTAAAGATATCACTCAA CAGGAGATACCTCAAGGTAGA |
| 654 | BMP6 | NM_001718 | CCCGGACGACCATGAGAGATA CAGCATTTATCAAGTCTTACA CCGGACGACCATGAGAGATAA ACCGTGCTGTGCGCCAACTAA |
| 656 | BMP8B | NM_001720 | CAGGTAGACATGTGGAGTCAA TAGGAAAGACATGTAAGAGCA CTGAGTGAACAGCATGATTTA ACAGATATGATGTAAAGTGAA |
| 657 | BMPR1A | NM_004329 | ACCAATTTATGTAACCAGTAT CACTGAATGATAGTCATTTAA GCGGCAGACATTAAAGGTACA CAGCTACGCCGGACAATAGAA |
| 659 | BMPR2 | NM_001204 | CCATATGAGCATTGTAATTAA AAGCTCTAAATTGATGTGCTA CTCGTAAGTATGTAAAGGAAA AAGCACCGAAGCGAAACTTAA |
| 671 | BPI | NM_001725 | CCCAGGGTTAACGAGAAACTA CAGACGTTGTCTATAAATGAA CAGGGTTAACGAGAAACTACA AGGAATCGTGTTTCAATTGTA |
| 675 | BRCA2 | NM_000059 | TACGTACTCCAGAACATTTAT TTGGAGGAATATCGTAGGTAA CAGCGTTTGTGTATCGGGCAA CAGGACACAATTACAACTAAA |
| 686 | BTD | NM_000060 | AACCAGAACCTTGACATCTAT CACAAATGTCGTGTTCAGCAA CAGATTATAGTGTTTCCAGAA ATGCGATTGGTCTCAAGCTAA |
| 708 | C1QBP | NM_001212 | CAGGCTTTGGCCAGTGAACAA CACGGTCACTTTCAACATTAA |
| 710 | SERPING1 | NM_000062 | CGCCAACTTGGAGCTCATCAA AACCTGAAACATCGTCTTGAA CTGGGTGGCCAAGAACACCAA TCACCAGACTCTATAAATAAA |
| 712 | C1QA | NM_015991 | ACGGTCATCACCAACCAGGAA CCCGGGAATTAAAGGCACCAA |
| 713 | C1QB | NM_000491 | CAGAAGTGCCATGCTCAGAAA CACCAACATGAACAACAATTA |
| 717 | C2 | NM_000063 | CCGGGATATGACTGAGGTGAT ACCCTGGCATGTCACTATTAA CAGAGAGATCCTGAACATCAA CCCAGTTATGAAATTAATAAA |
| 718 | C3 | NM_000064 | CCGAGCCGTTCTCTACAATTA CACGGAGAAGCGAATGGACAA CACGGTCATCGCTGTGCATTA TAGGAACACCCTCATCATCTA |
| 720 | C4A | NM_007293 | AACCCTGTACGACTACTACAA TCCAGTGTTAGACAGGAGCAT |
| 721 | C4B | NM_000592 | AACCTGGGAAAGAATATTTGA AACCCTGTACGACTACTACAA |
| 726 | CAPN5 | NM_004055 | CCCGGGAGTCGCTGTGGCAAA CCGCGTGATCAACACATCCCA CCGGTACTTCACGGACATCAT CTGGTGTTCTCCCACCTTGAA |
| 730 | C7 | NM_000587 | CACGCAGTTATACTTCACATA AAGGATTAGAACACTCATTAA ACCCTGGTAAATATATTTCAA CCGAAGATTAATCGACCAGTA |
| 757 | C21orf4 | NM_006134 | AAGGGATAATACATGATCAAA AAGCTGGGATACAGCATTTAA CACCTGGTATGCTTACATTAA ATGGAGTAGATTGTACATTAA |
| 762 | CA4 | NM_000717 | ATGCACATAGTACATGAGAAA CTGGCTACGATAAGAAGCAAA TGGGACTTTAGGCATGATTAA CTGGTCCGACTTGCCATATAA |

Table4

| No. | Gene | Accession | Sequence |
|---|---|---|---|
| 773 | CACNA1A | NM_000068 | CACCGTGTACAAGTGAACAA CCGGGTGCTACGACCTCTTAA TCGGAACAACGTGCTGCGATA CGGGTGCTACGACCTCTTAAA |
| 774 | CACNA1B | NM_000718 | CCCAGGAACAACGCTCTGAAA CCGGCAGTCGTTCCAGTATAA CCCGAACAACTATTGTACATAT TCGAATTGGCTCTGACCCTTA |
| 778 | CACNA1F | NM_005183 | CACCATCATGTCTTCACCAAT CTGGCCTGCACTGCTATACAA CACCACACTTCTGCAATTTAT CCGGACATCCCTGAAGATCAA |
| 780 | DDR1 | NM_001954 | CAAGATCTGGTTAGTCTTGAT CACTAGGCAGGTAATAATAAA CAGGAATGATTTCCTGAAAGA ACGGTGTGAATCACACATCCA |
| 781 | CACNA2D1 | NM_000722 | ATCGGGCATTATGGTAAGCAA TTCGATAAACGACAGTAAATCA TCGGCCGTCACTATCAAATCA CGCCTGTTATGACCTTCTAA |
| 784 | CACNB3 | NM_000725 | CCGGCGTGAAGTAGAGAGCCA CCGCTTGTTAGGTTAGGGTTA CTGGAGAGCATCCGGCTCAAA CTCACTGTCATCACTAATAAA |
| 797 | CALCB | NM_000728 | AAGGAATGAAACTGAATGCAA CCCACCAATGTGGTTCCAAA ACCTTTGTCACTGAAAGGAA AACCTTGGTGATGCATTACAA |
| 801 | CALM1 | NM_006888 | TTCCTGAACAACGAAGTGTAAA CTGGTTGTATCTTATTAGCAA CGGCAACTTACACACATTGAA ATGCCCGTGTATGTTGGTTTA |
| 813 | CALU | NM_001219 | CAGGGTTTCAGTCCTTATCAA AGCCTGAGATCAATAAGAAA |
| 816 | CAMK2B | NM_001220 | CAGGATCTCTGACATCCTGAA CCCGGAAGCAGGAGATCATTAA CAAGAGTTTACTCAACAAGAA |
| 823 | CAPN1 | NM_005186 | TCCCACGGAACTGCTGTCAAA CCGGACCATCCGCAAATGGAA TAGGATCATCAGCAAACACAA CAGGGACTTGTGTACTGGTTA |
| 824 | CAPN2 | NM_001748 | CTGGAACACTATAGACCCAGA AACGGATGCTATGAAGCTCTA AACATGTATATCATGAAGTA CCCGAGAATACTGGAACAATA |
| 834 | CASP1 | NM_001223 | TCGGCAGAGATTATCCAATA CTCATTGAACATATGCAAGAA TACCTCTTCCCAGGACATTAA CCCAGATATACTACAACTCAA |
| 839 | CASP6 | NM_001226 | ACCCAAGAATATTATCATTCA CTCAGGAAATTAGATAAATAA GCGCAGATAGAGACAATCTTA CAGGAATTAAATCATCCCTTA |
| 841 | CASP8 | NM_001228 | GACAAAGTTTACCAAATGAAA AAGATAATCAACGACTATGAA CGGAGGGTCGATCATCTATTA AAGAGTCTGTGCCCAAATCAA |
| 845 | CASQ2 | NM_001232 | CGGCATATTTGGGAACGAGAA TAGCCTAAGCTGGGATCTGAA GAGGTTGATTTGCTCTCTAAA TAGTGTTAAGTGTGCAGTATA |
| 857 | CAV1 | NM_001753 | TCCAGTCTTCCTGACACTTTA CTGGTCAACCGCGACCCTAAA TCCTGACACTTTAATTACCAA CAGCCGTGTCTATTCCATCTA |
| 860 | RUNX2 | NM_004348 | CACCTTGACCATAACCGTCTT CAGCAGCACTCCATATCTCTA CAGAAGCTTGATGACTCTAAA CAGCACGCTATTAAATCCAAA |
| 861 | RUNX1 | NM_001754 | CAGTCGTCTTATCTAGAGA CTCCCTTTCATGTTAATCAAA TTGGCGATAGGTCTCACGCAA CAGGAAAGTTAAGGTATCAAT |
| 868 | CBLB | NM_170662 | ACGGGCAATAAGACTCTTTAA TCGGTTGGCAAACGTCCGAAA TCCGGTTAAGTGCACTCGAT CACAGCGAGTTCAAATGTCAA |
| 871 | SERPINH1 | NM_001235 | CTCAATCAGTATTCATATTTA CCCAACCTCTCCCAACTATAA CAGCCCTCTTCTGACACTAAA CAAGAACTTATTGTACATTT |
| 883 | CCBL1 | NM_004059 | CAAGTGGATGATCAAGAACAA CACAGACATCTCAGACTTCAA |
| 885 | CCK | NM_000729 | ATCTATTTATTAAGTTCTCAA ACCAGAATGTGCAAATGAA TGGACGAATGTCCATCGTTAA ACGAATGTCCATCGTTAAGAA |
| 887 | CCKBR | NM_176875 | CACAGCGTCCCTAGCAGTGAA CCCAAACTGTTCAAGAAATAA CCGCCGGGTCGAGCTGAGTAA CCGTGTACACTGTCGTGCAA |
| 896 | CCND3 | NM_001760 | TGGGACAGAATTGGATACATA GACCATCATCCTACTGTAATA CACAACTTAAGAGGACATTAA CAGGAACCACCACCACATCTAA |
| 899 | CCNF | NM_001761 | CACAGATGGCGTCAATGTGAA CCGGTTTATCAGTAAAGAGAT AACCCAAGCGTTGGAACTGTA CACGGACAACACTTACAAGTA |
| 901 | CCNG2 | NM_004354 | GGGACAGTGGATAATCCTTAA CTGGAGAGAGTTGGTTTCTAA AAGGCAGAAATAATTAGGCTA AAGAGTGAGTATGCTCTTAAA |
| 905 | CCNT2 | NM_001241 | CTCGATGTAAGGGATCATTAT AAGGGCTTTCTTGGTAATTTA TCCACATATGATAGTGTTATA AGCAAGAATATACTCATAAA |
| 910 | CD1B | NM_001764 | AAGCAGATCTGCAAAGACAA CTTGGTCTCAAAGGTAACTTTA CACAGAAATTCTGTGCACTAA CTGAGTGTCAAGAATGCTTCA |
| 914 | CD2 | NM_001767 | AACCTGTATCAAGATGGGAAA TAAGATGAGCTTTCCATGTAA CCCAACAAATTCCAGCTTCAA CCCGAATTAAACCTGTATCAA |
| 915 | CD3D | NM_000732 | CTCACTTATTAATTCTAGTAA CACGAGGAATATATAGGTGTA TGGCACGATATGTTAAACCAA CAGGCTATATATGGTATTAAA |
| 919 | CD3Z | NM_000734 | CAGGAAGGCCTGTACAATGAA CCGGTTTATCAGCGAATGACAA TTGGTTATATTTAGCTCCAAA ACCAACAACATGGGACCTATA |
| 923 | CD6 | NM_006725 | CAGCACTACTGCGGCCACAAA CTGGCGGTTCAACAACTCCAA CCGGCAGGATGTACTACTCAT CAGGGAGGATGCTGCCTCCAA |
| 926 | CD8B1 | NM_172100 | TAGCACTAAATTCGAGAAGCA CAAATACAATAGAGCCACTAA TAGCTGTGTTTCGGGATGCAA ACCCGGCAACATCAGTTTAAA |
| 928 | CD9 | NM_001769 | CAAAGAGGTCTTCGACAATAA ACAAATGTCTATCAACTTTAA CCGCGAGATGGTCTAGAGTCA AAAGAGGTCTTCGACAATAAA |
| 929 | CD14 | NM_000591 | CCCGAGGTGGATAACCTGACA CTCAGAGGTTCGGAAGACTTA CAGAATAATGAATGGACTCAA CAGCCTAGACCTGTCTGACAA |
| 931 | MS4A1 | NM_021950 | GAGCAACTTTCTTACACTGAA TTCAATCATGGACATACTTAA CACACACCATATTAACATATACA |
| 939 | TNFRSF7 | NM_001242 | AACGAAGGAAATATAGATCAA ACCGAGTGTGATCCTCTTCCA AGGAAATATAGATCAAACAAA AAGGTTGGCTTGCCACCTGAA |
| 940 | CD28 | NM_006139 | TGGGTTAAGCATGCCAATTTA CAGGGATGAGGTGCTATAATA CTCGAGTGACTAGACCAAATA ATGATGGTTCTTCAACTATAA |
| 961 | CD47 | NM_198793 | TACCATTCCTATAGTACTTTA CACTACTGAAGTATACGTAAA TACAGCTGTGTTACCGTTAAA CACGATAAGTTTACTCCTCCA |
| 962 | CD48 | NM_001778 | CTCGGCGAGTCTGTAAACTACA CTGCAAGTGCTTGACCCTGTA CACCCTTATGCCACATAATTA CAGAAGCATGCTGCTGAATTA |
| 965 | CD58 | NM_001779 | TCCAATTGATTGGTAACAGAA TAGCAGTAATTACAACATGTA ATGCATGATACCAGAGCATTA AGCAATGTAAACGTAACTCAA |
| 967 | CD63 | NM_001780 | CTGGCTATGTGTTTAGAGATA CAGATGGAGAATTACCCGAAA TGGGATTAATTTCAACGAGAA CAACGAGAAGGCGGATCCATAA |

Table4

| 970 | TNFSF7 | NM_001252 | CTGCGTCTCAGCTTCCACCAA CAGCTACGTATCCATCGTGAT |
| 975 | CD81 | NM_004356 | AAGGAACATCAGGCATGCTAA CCGCCTGTGTATATAACGTTT CGCCTTCAACTGTAATCACAA CACCTTCTATGTAGGCATCTA |
| 983 | CDC2 | NM_001786 | AAGCCTAGCATCCCATGTCAA TTGACTAACTATGGAAGATTA TCGGGAAATTTCTCTATTAAA CAGGTTATATCTCATCTTTGA |
| 987 | LRBA | NM_006726 | CACGATGGAAACAGATGATAA CAAGGGTACATTTAAATTCAA |
| 988 | CDC5L | NM_001253 | ACGCAGATTTCAGGAAATTAA TCGAGACAAGTTAAACATTAA |
| 995 | CDC25C | NM_001790 | GAGCTGCAATCTAGTTAACTA CCAGGGAGCCTTAAACTTATA CCAGAGCTATATATCCTTAAA CAGGAAGGGCTTATGTTTAAA |
| 1002 | CDH4 | NM_001794 | CCAGAATATGTTCACCATCAA CACGTCCATCATCAAAGTCAA |
| 1010 | CDH12 | NM_004061 | CAAAGTCAATATACTGATTAA CTCCACCTTCTTCTAATTCAA |
| 1012 | CDH13 | NM_001257 | CAGGATATATTTAAATTTGCA CTGGAAGATCTCCAAGATCAA |
| 1019 | CDK4 | NM_000075 | CAAGGTAACCCTGGTGTTTGA CCGAACTGACCGGGAGATCAA ACCCTGGTGTTTGAGCATGTA AAGGTAATCCGGAGTGAGCAA |
| 1022 | CDK7 | NM_001799 | CTCTTGAAATGTAGAATTGAA TTGGACATAGATCAGAAGCTA GAGGCTTTAAGGTAGCTTTAA CCGGATGGCTCTGGACGTGAA |
| 1024 | CDK8 | NM_001260 | CAGGGCAATAACCACACTAAT TTCGAGAGCTTAAGCATCCAA CCAGCTGGACAGAATATTCAA CAGAAGAAATACGTATACCAA |
| 1025 | CDK9 | NM_001261 | CGGCCAGAAGGTGGCTCTGAA TGGGCACAGTTTGGTCCGTTA TAGGGACATGAAGGCTGCTAA AACCGCTGCAAGGGTAGTATA |
| 1027 | CDKN1B | NM_004064 | CTGTAAGTAACTTCACATTAA CACGCATTTGGTGGACCCAAA ACCGACGATTCTTCTACTCAA AAAGCGTTGGATGTAGCATTA |
| 1029 | CDKN2A | NM_000077 | TACCGTAAATGTCCATTTATA CAGGTGTGCCACATTCGCTAA CAGAACCAAAGCTCAAATAAA CACGCCCTAAGCGCACATTCA |
| 1043 | CDW52 | NM_001803 | GAGAATAAAGTTACCCTTGTA CTGGTTATGGTACAGATACAA |
| 1046 | CDX4 | NM_005193 | CACCGGCTTTCTCGCACTATA CAAGGAGAGAAAGATGATCAA GTAGTTTACACTGATCATCAA CAGGTTATAGTCTCCGAATGA |
| 1047 | CLGN | NM_004362 | CTGGATTATATTGCAGCATAT AAGAAAGTAATCAATCAAATA |
| 1050 | CEBPA | NM_004364 | CAAGAAGTCGGTGGACAAGAA CTCAGCCTTGTTTGTACTGTA CCGAGTCACACCAGAAAGCTA CAGCCGATATCAACACTTGTA |
| 1056 | CEL | NM_001807 | CAGCCACATCCTGGCTGGCAA AGCCCTGACGCTGGCCTATAA CCCGTTATGATCTGGATCTAT TCCCATGAGCCTTGGTATCAA |
| 1059 | CENPB | NM_001810 | CCGGGAGAAGTCACGGATCAT TTCGAGGTCTTGGACATCAAA CACGCTGAGCACGATCCTGAA CGCCTTGAGAAGCACAGTTTA |
| 1069 | CETN2 | NM_004344 | AAGCTCTTTGATGATGATGAA GCGAATTACCCTAATTCTCAA AAGCACATGTAACTAGATTTA CAGAACGACTTTAGACAAGCA |
| 1070 | CETN3 | NM_004365 | CTGGTGACATTTAAAGAATTA TCGAGCTATGATAGAAGAATT TAGCTTTGTATTTATAATAGA CAGTTACCATCTTATATTCTA |
| 1072 | CFL1 | NM_005507 | CAAGGTGTTCAACGACATGAA CTGACAGGGATCAAGCATGAA CAGTAAGGGACCTTCGATTAA CAAGCATGAATTGCAAGCAAA |
| 1082 | CGB | NM_000737 | CACCACCATCTGTGCCGGCTA CCCACAATAAAGGCTTCTCAA CCAGGACTCCTCTTCCTCAAA CCCGAGGTATAAAGCCAGGTA |
| 1084 | CEACAM3 | NM_001815 | CAGTTCCATGTATACCAAGAA CAGGACAGCAGCTTCCATCTA |
| 1088 | CEACAM8 | NM_001816 | TTGCTTCTTCTTCAAGATTTA CAGGCTGCCAATGCTAGATAA |
| 1103 | CHAT | NM_020549 | CAGGACGGTCCTCGTGAAAGA CCCGAGATGTTCATGGATGAA CCGGAAATTCAAGGCCACTTA CACGGAGATGTTCTGCTGCTA |
| 1109 | AKR1C4 | NM_001818 | CTGGGAACCCAACGACATAAA GAGGGTGTTGCACGACATCTA ATGGACCATCCTGATTATCCA CCAGAAAGCAAACATAATAAA |
| 1112 | CHES1 | NM_005197 | CTGGACTTGGTTTATATTGCA TCGAAGGAGCCTTCACGTAAA CACGGCCAAATTAATTTACGA CCGGTCCTGTTTGAATAACAT |
| 1113 | CHGA | NM_001275 | TACCGAGGTGATGAAATGCAT TCCAAGGATGTTATGGAGAAA AAGGAGTGGGAGGACTCCAAA TATGAACTTTATCTAAAGAAA |
| 1116 | CHI3L1 | NM_001276 | CAGCTGCTCAATAAAGTACAA AAGGCCGAATTTATAAAGGAA |
| 1119 | CHKA | NM_001277 | TACCACAGAGATTGTACTATA CACAGAGATTGTACTATACAA CCGGCCCTTACCAAACCTGAT AGCCGGCGATTAGATACTGAA |
| 1121 | CHM | NM_000390 | TCGGAGTTTGATGTGATCGTA CCGGAGAGTTCTGCATGTTGA CGGAGTTTGATGTGATCGTAA CTCCAATATATTGTCATGCAT |
| 1128 | CHRM1 | NM_000738 | TGGCTCCGAAGTGGTGATCAA CAGCAAGACAATGACACTGGA TACAGTCAAGAGGCCGACTAA CCGGCTTGGCTAGTCACATAT |
| 1130 | LYST | NM_000081 | CACATCATTGTCAACACCTAA CACGCCGACCTGATTACCTAA TCCGAGTATGCCATAAGTTAA CAAGGTGTTATTAAACTATTA |
| 1135 | CHRNA2 | NM_000742 | CAGCCTCTGTTTGACCATGAA CACGGGCACCTACAACAGCAA CAGGAGTGGAGCGACTACAAA AAGGGCTTTGAACAATGTTTA |
| 1136 | CHRNA3 | NM_000743 | GCGGCTGTTTGAAGATTACAA AACTGTCTTAGTACACCATAA TACCGATAAATGAACATTTAA CCCACCTTCTATCTAAGACAA |
| 1138 | CHRNA5 | NM_000745 | CTGAACCTTCTTCTATTGCAA CTGAAGTATACATTTAGTTAA ATCGTTCTTCCTCAACACATA CTGAGTAACAGCTAATCTTTA |
| 1142 | CHRNB3 | NM_000749 | CCACCACAGGTTTCAACTCAA CGGAGAATGGGAAATACTGAA ACGCTGGAATCCTGATGATTA CAGGTAGTACAAGACTGGAAA |
| 1143 | CHRNB4 | NM_000750 | CCCAGGGAGAAGGACAGTGAA CAGCAAGTCATGCGTGACCAA CAGCTACGTGGACGTGACTTA CACATGAAGAATGACGATGAA |
| 1144 | CHRND | NM_000751 | CCGGCACCTGTTTCAAGAGAA CCGCCAGGACATCACCTTCTA CGGCTGAAGTGGAATGCTGAA AACAATTACAATGAGGAGAAA |
| 1146 | CHRNG | NM_005199 | CACCAATGTCTGGATAGAGAT CTGCTCTATCTCAGTCACCTA CACCAACCTCATCTCCCTGAA CCACCAGAAGGTGGTGTTCTA |

Table4

| ID | Gene | Accession | Sequence |
|---|---|---|---|
| 1147 | CHUK | NM_001278 | CAGCGTGCCATTGATCTATAT CAGGGCAATAGTATGATGAAT TTCCATAAGCTTGGTGACAAA CAGGAGAAGTTCGGTTAGTA |
| 1163 | CKS1B | NM_001826 | CCAAGAAACCAAGAAATGAA GCGATCATGTCGCACAAACAA AAGAAATCTGTTCATGTTAAA AACATCTTTCTGATAACATTA |
| 1164 | CKS2 | NM_001827 | TAGGTTACTGTAAGATGTTTA TCGGACAAGTACTTCGACGAA CTGTAAGATGTTTAAGATAAA AAGTTTGTATGTTGCAITTAA |
| 1173 | AP2M1 | NM_004068 | ACGTGTGACTTCGTCCAGTTA TGCCATCGTGTGGAAGATCAA TTGGAGGCTTATTCATCTATA TGGAGGCTTATTCATCTATAA |
| 1178 | CLC | NM_001828 | CACCTTTGACCATAGAATCAA CAGGATGGCCAAGAAGTTGAA |
| 1181 | CLCN2 | NM_004366 | ACCGGGATGAAGAGACTATTA CCGAATCAAGAAACTGCCCTA CAGATGGAATTCATACGGACA CTCATTGGAATCGTTACTCTA |
| 1184 | CLCN5 | NM_000084 | CACCGAGAGATTACCAATAAA AAGGATGTGTTAAAGCATATA TAAGTGGACTCTGGTTATCAA ATCCTTGTTCCTGTTACAATA |
| 1185 | CLCN6 | NM_001286 | AAGATGTGAATTCAAGTATCA TCAGATTTACGTCTACCCTAA CACCAGAAACATAAAGGATTA CCGGTGAATGTTATTGGAATT |
| 1186 | CLCN7 | NM_001287 | AGCGTGGAGCTGGATGATGAA CTGGCTGACCATGTTTCGAAT CCAGATCAAGTGCTTCCTCAA CCAGCTCAAGTCGTTCTCCTAA |
| 1187 | CLCNKA | NM_004070 | ACTGGCCATCACATTAATGAA CTGAGTGTGAGAAGATGGAAA CAGCTCCATTCTTTGGCATAA TTGGCATAAACAGCCAACTCTA |
| 1192 | CLIC1 | NM_001288 | TTCCTGCTGTATGGCACTGAA AAGGTGTCTCTCAGAGGAAGT CTGGTGGTTTCCACATTGCTA AAACCCAGCACTCAATGACAA |
| 1193 | CLIC2 | NM_001289 | AAGCTAGAGCGGTCTTCTTAAA CACAATGCCTATGCCCGTGAA CTGGCTGATTGTAGCTTGTTA CCCGTGAAGAATTTACCCACA |
| 1195 | CLK1 | NM_004071 | CCGTATTTCCAACACACGATA CACGATAGTAAGGAGCATTTA AACGTGATGAACGCACCTTAA AAAGCCGGTATCAGAACCATA |
| 1198 | CLK3 | NM_001292 | CTGCAAACCTCTGAAGAGTTA ACCCAAGCTATTTATATGTTA CTGCATTCTCTTTGAGTACTA CACGAAGATCTCGGTCCAGAA |
| 1212 | CLTB | NM_001834 | CTCGGTGCTCATGTCCCTGAA CAGCGCCAGAGTGAACAAGTA |
| 1230 | CCR1 | NM_001295 | AAGCACGGGCCATATGAATAA CAGCGATTAATAACAGCCAAA GACGAAGAGTTGAGACCTAA CTGATCGCCTACACAAGTTGAA |
| 1234 | CCR5 | NM_000579 | AGGCAACATATAGGTTGTAAA AAGGGACATATTCATTTGGAA TAGGTGAGGATTGATTACCTA AAGATTGGATTATCAAGTGTCA |
| 1238 | CCBP2 | NM_001296 | CACCAGATTACAACAAATTTA CTCAATTAGCGTTATTGGCAA CTGCATGAGCCTGGACAAGTA CTCAGTGACTGTGTTGCTAAA |
| 1244 | ABCC2 | NM_000392 | CTGATCCAATACAGCAGACAA CGCCATCGACGTGAACTACAA CTGGTGGATAGCAACAATATT CCGTATCAGGTTTGCCAGTTA |
| 1258 | CNGB1 | NM_001297 | CACAAATAGCGCCATCATCAA CATGGCCTTCTTCGAGTTTAA CAGAAGTTACTCCGGAAGAAA |
| 1260 | CNGA2 | NM_005140 | CCAACTGAGATAGCCATCAA TGGGGAGATCAGTATCCTTAA TAGAGCACCTATGAGTTAAA AGCACCGACTATAGACATTTA |
| 1261 | CNGA3 | NM_001298 | CCAACTGATAATGGGTATATA AAGGTTGGAATGGTGTACTAA CACCTGATAATGGGTATATAA TCCCTTGGAATTGCATCCCAA |
| 1263 | PLK3 | NM_004073 | GTGGGTTGACTACTCCAATAA CTGCATCAAGCAGGTTCACTA CCCGAAATCGTAGTGCTTGTA CAGAAAGACTGTGCACTACAA |
| 1272 | CNTN1 | NM_001843 | CAGCCTAGTAGCAGTCATTAA AAGGGTGATAATTGAATGCAA |
| 1281 | COL3A1 | NM_000090 | TCCGTTCTCTGCGATGACATA ACCGATGAGATTATGACTTCA CGCGGTGAGGCTGGTATTCCA CTCAAGTCTGTTAATGGACAA |
| 1284 | COL4A2 | NM_001846 | CACACCATTCATCGAATGCAA CACGTTGTCCCTGAGATTTAA |
| 1285 | COL4A3 | NM_031366 | CTGCTTGTATCCAAGCATATA TCCCAAGGTTACTTAATTCAA TACATTCCCAAGGTTACTTAA AGCGGATATAGAGCTAGCTAA |
| 1287 | COL4A5 | NM_000495 | AAGGGTGATGATGGAATTCCA CCGGGTCTCAATGGAATGAAA |
| 1290 | COL5A2 | NM_000393 | CCGGGTCTACGTGGTGAAAGA TAGGTTAAGATGACCAATGA CCGACACGTGTGATGACCTAAA CACACTAGTATATACCATTTA |
| 1297 | COL9A1 | NM_001851 | CACCGACAGATCAGCACATTA CAGCATGATTACAATGTATTA AACGGTTTGCCAGGAGCTATA CACCTCCCTTGAACAAATTTA |
| 1299 | COL9A3 | NM_001853 | CAGGGTGACAGAGGAGGACAAA CAGGCTCTCGAAGCTCATAAA |
| 1300 | COL10A1 | NM_000493 | TACGGAAGTCCTGGACTCCAA AGCAATCTTAAGGCTCTTTAA CCCAGCTGGCATAGCAACTAA AAGCCTGTTTCTAACTATGAA |
| 1307 | COL16A1 | NM_001856 | AAGGGTATTTCCCTCCTTAAA CAGGAAGGACTCAAATTGGAA |
| 1314 | COPA | NM_004371 | CTGGATTTCAACAGCTCCAAA TCCCACTGAGTTCAAATTCAA |
| 1315 | COPB | NM_016451 | CAAGGATTGGTTATAATATAA CAGGATCACACTATCAAGAAA |
| 1347 | COX7A2 | NM_001865 | ATCAATATTTATTGACTTGTA GACCAGTAATCTGATAAATAA |
| 1349 | COX7B | NM_001866 | CAGCTTCATAATTGATGCCAAA TGGCATTATTGAAGAAATAAA |
| 1352 | COX10 | NM_001303 | ACCATAGTCCTTCTAACAATA AAAGGATTGTAGGTAGAATAA AAGGGTAGCCCTGGACTTAAT TAGGTAGAATAACATCCAATCA |
| 1359 | CPA3 | NM_001870 | CCCAATAGAATCAACAATTTA CAGTAGCACCATAACGAAGTA ATCCTCAAGCATACTTCCTAA AACGAAGTAGCTTTAAGTGAA |
| 1363 | CPE | NM_001873 | TACCTGGAAACTATAAACTTA CTCGGAGTTGTGAGCACTCTA CAGGCTATCTGGCAATAACAA TAGTCCGTTAACACTACTTAA |
| 1365 | CLDN3 | NM_001306 | CACCGGCCAGATGCAGTGCAA CACCATTATCCGGGACTTCTA CACCACCACCACCACCACCAA |
| 1366 | CLDN7 | NM_001307 | GCCGTTGGTGGGGAGAGACAAA CCGCTCTTACCCTAAGTCCAA |
| 1369 | CPN1 | NM_001308 | CCGCAACTTCCCTGATCTCAA CAAGGGAATGCAAGACTTTAA CCGGTGGATGCACTCCTTCAA AAGACTTTAATTATCTCCATA |

Table4

| | | | |
|---|---|---|---|
| 1378 | CR1 | NM_000573 | CAGTCCTACGATCCCAATTAA CTGCCATGGTGAGCATACCCTA TCGGTGTATTTCTACTAATAA CTGGAGCCAATTGGATCATTA |
| 1385 | CREB1 | NM_004379 | CAGCCACAGATGCCACATTA CCGCATGCATAAAGTAAGTAA AGGCCGGGTACTACCATTCTA AGGGCAGTTGTTGCTTCTTAA |
| 1393 | CRHBP | NM_001882 | CTGCAGCTTCTCCATAATTTA CAGGAAAATGGATTCACATTAA |
| 1394 | CRHR1 | NM_004382 | CAGGACTGCAGTGCAACGCAT CCGCTACAATACCACAAACAA GCGGGTGGCTGGTATAAATAA CAGGTTGGTGACAGCCGCCTA |
| 1395 | CRHR2 | NM_001883 | CTGCAGCTCGTTGACCATGAA AGTCTACTATGAGAATGAA CACGGCCATTGTCATGACCTA ACGGATCAGCTTCCACAGCAT |
| 1397 | CRIP2 | NM_001312 | CTGGCACAAGTTCTGCCTCAA GAGCCTTGTTGCTGTCAATAAA |
| 1398 | CRK | NM_005206 | TACACTATTTGGACACTACAA CAGGATGTACCGAGCACTTTA CTGAGTAGTAGTTCAACAGTTT CAGCAGCTAACTAGAGTCCTA |
| 1401 | CRP | NM_000567 | AAGCGCTGATCTTCTATTTAA AAGGAGAAATGATGTTATAAA |
| 1407 | CRY1 | NM_004075 | GAGGATCTTGATGCCAATCTA AAGGAATGGAACATTACTAAA CTGTTTGTGATTCGTGGACAA ATCAGCAGCTTTCACGATATA |
| 1409 | CRYAA | NM_000394 | CCCGGAGGACCTCACCGTGAA CCGGGACAAGTTCGTCATCTT |
| 1421 | CRYGD | NM_006891 | TCCTCCTTTGTTGTTTCTTAA TTGTTGTTCTTAATTTGGAA CAGCGGCTGCTGGATGCTCTA ACCGCTTCCGCTTCAATGAAA |
| 1427 | CRYGS | NM_017541 | AAGATTCAGATCTTTGAGAAA CCAAATAGGCATCATCAATAA |
| 1428 | CRYM | NM_001888 | CACCAAGTTGGTCACCTTCTA CTGGATGATGAGCTCATGAAA |
| 1436 | CSF1R | NM_005211 | CTGGAAGATCATCGAGAGCTA ATGAGCCAAGTGGCAGCTAAA ATGGAAATGGACTGACTTTAT ACCCTCAACCTCGATCAAGTA |
| 1441 | CSF3R | NM_172313 | TCCTATAACTTCAGTATTGTA CCAGGCGATCTGCATACTTTA CTCCTGCATCATCAAGCAGAA CAGGCGATCTGCATACTTTAA |
| 1444 | CSHL1 | NM_001318 | CCTCAGGAGTACCTTCACCAA CAGGAGTACCTTCACCAACAA |
| 1445 | CSK | NM_004383 | AGGGAACAAAGTCGCCGTCAA CCCAACTGGTACAAAGCCAAA TACGCGCCTCATTAAACCAAA CCGTACAGAGAATGTATTGCCA |
| 1447 | CSN2 | NM_001891 | CAGAGTGATGCCTGTCCTTAA ATGGAAGTCCCTAAAGCTAAA |
| 1452 | CSNK1A1 | NM_001892 | AAGAACTTAATTCAGTATATAA CACAGTTGTGATGGTGTTAA CAAGAGTAACATGAAAGGTTT AGGGCTAAAGGCTGCAACAAA |
| 1453 | CSNK1D | NM_001893 | AACACGCACCTTGAATTGAA CGGTCTAGGATCGAAATGTTA CTCCCTGACGATTCCACTGTA CCGGTCTAGGATCGAAATGTT |
| 1454 | CSNK1E | NM_001894 | TCCCTCCGAATTCTCAACATA CTGGCTCTGAGTTATAAATTA GTGGTTGTTAATTTGAAGTAA GAGCTTTATCGTGTTGTTAA |
| 1455 | CSNK1G2 | NM_001319 | ATGTAAGACTTTGGCCGAAAT GCACACCAAGAGCCTAATCTA TAGGAAAGAATCTCTATACAA AAGAATCTCTATACAAATGAA |
| 1456 | CSNK1G3 | NM_004384 | CAGGTTGTAAGTTCTACAAAT AAGAGCATAATCAAACAGGAA TACCGCAAACTTGATATGGAA CAACATATCTTCGTTATGTAA |
| 1465 | CSRP1 | NM_004078 | CAGGATGTTGTTGATCTTCT TGGGAGGATCCTAATGAAATA |
| 1466 | CSRP2 | NM_001321 | CAGGCCTACACAACAAATCCAAA CAGATAATCTTTAACACTAAA |
| 1475 | CSTA | NM_005213 | CAGGAGATTGTTGATAAGGTT CTGGAACAAATTACTACATTA |
| 1486 | CTBS | NM_004388 | CTCCCTATAATCAGACATTAA AACGATCATGAAGCAAATAAA |
| 1489 | CTF1 | NM_001330 | CCCGTTCTCTTAACTCTTGGA CAGCAGATCTGTGGAGACAAA |
| 1493 | CTLA4 | NM_005214 | AAGAAGAGAGTCCATATTTCA AAGCATCACTTGGGATTAATA CCCAAATTACGTGTACTACAA CAGCATTATGATGTGGGTCAA |
| 1495 | CTNNA1 | NM_001903 | ACAGACTTTACCCGAGGTAAA GCGAATTGTGGCAGAGTGTAA CAGTCACTGTTCGTCACTCAA CACCCTGATGTGCCAGCCTAT |
| 1496 | CTNNA2 | NM_004389 | CAGCAGTGAAGCAGGATTTAT TACCATTGTATTCACTAACTA ACAGGCTTAAGTGAATTCAA CCATGAATTGAACAATTTAA |
| 1497 | CTNS | NM_004937 | CTCGCTAGGCGCCCTAAGCAA CGCCATTAGCATCATAAACCA CAGCGCCATTAGCATCATAAA CACCTACTTGAGACTCACCAA |
| 1511 | CTSG | NM_001911 | CAGACGGAATCGAAACGTGAA CACAGTGTTGCCAGAGCCTTA CAGCCTTTCAGGAAAGATGCA CCACAGAGTATAAATAACCAA |
| 1512 | CTSH | NM_004390 | GTCCTTCTTAACAGACTCAAA CACCCGTGTCTATGACGCAAA CGAGTATATCCTGTACAACAA ATGGATGTCTAAGCACCGTAA |
| 1513 | CTSK | NM_000396 | CCCGATGGAATAAATCTAGCA AGGGCTGATGCGTACAGGTA CCGCAGTAATGACACCCTTTA CACGGGACTAGTTAGCTTTAA |
| 1520 | CTSS | NM_004079 | CTGCCACATGTTCAAAGTACA TGGGATAATGTCATTAATATA AAGGATATATTCGGATGGCAA AGGAATCTAATTATATCGAAA |
| 1521 | CTSW | NM_001335 | CCCAAGCATGGGCAGAGAAATA CGCGTTCATAACTGTCCTCAA CGGCCGCCATCTCACCCATCAA CACCGTGACCATCAACATGAA |
| 1524 | CX3CR1 | NM_001337 | AACTCAGACTAGTTTAGTTAA CAGACTAGTTTAGTTAAATGA TCAGATGTGGTAACTGTTAAA TAGAGTTGCAATCGTAATGTA |
| 1525 | CXADR | NM_001338 | CTCCATCAATTCTGTATTCCA CAGTAATAGGGACTTAGCAA TTGGTTATATCGAAATAGTTA CAGGAACTGTACGGAATATAT |
| 1536 | CYBB | NM_000397 | CACGGTGCCTCATATTAAT CCAGATCACTTAGGACATAATA ACCCTTCGCATCCATTCTCAA CAGCTAAGGATACTAACCAA |
| 1538 | CYLC1 | XM_088636 | CAGGTAGAGTTCCTCACTTGAA AAGGAATTACTCACAGAATAA |
| 1548 | CYP2A6 | NM_000762 | CAACACGGAGTTCTACTTGAA CACCTTCGACTGGGTCTTCAA CCGCTTTGACTATAAGGACAA TCGGGATTTCTTCTCCCTAA |
| 1549 | CYP2A7 | NM_000764 | ATGCAAATATTCCAAACTCTT CCTGATGATGAGCACGTTGAA CCCAAGCTAGGTGGCATTCAT CAGCTTCTTCTTCTCCAACCCTCA |

Table4

| 1558 | CYP2C8 | NM_000770 | CCGGCGTTTCTCCCTCACAAA ACGAAGTTACATTAGGGAGAA AACTCACAACAAAGTGCTTAA AAGTCAGAATTCAATATTGAA |
|---|---|---|---|
| 1572 | CYP2F1 | NM_000774 | CAGCATAAGCACAGCCATCTT CACCATTATCCGCCTTATCAA CAGCGGGTACCAAGCTGTGAA TCGCGGCTTCCTGATACCCAA |
| 1579 | CYP4A11 | NM_000778 | CCCTGACGCACCATAATCTAA ATCAATTGTATCTTGAGTTAA TCCTATATTAGTTTCCTATTA CAGCTGTAAATTGTGTGCTTA |
| 1584 | CYP11B1 | NM_000497 | CTGGGACTGCATCTTCCAGTA CAGGTGGAGACACTAACCCAA CAGGACAGTGCTGCCCTTTGA CACCTTCAGAGCCATCAACTA |
| 1585 | CYP11B2 | NM_000498 | CAGGGAGGCTGCAGTTCCCTA CAGAGATGGCTTCCTTGTTTA AAGGCGGAACTGTCACTAGAA CACTAGATCATGGGATCCTAA |
| 1586 | CYP17A1 | NM_000102 | CAGACATGGCCATATGCATAA CCGGAGTGACTCTATCACCAA TGAGTTGAATGTCATACAGAA CAGGCTGAGGGTAGCACCTAA |
| 1591 | CYP24A1 | NM_000782 | CTCGGACTCTTGACAAGGCAA AAGTTAGAGATCTGTTATATA TAGGGTAAAGATGAAGCAATA TGCAATACTTATAAGACTTAA |
| 1593 | CYP27A1 | NM_000784 | CAGCGGAAGCAGCGCTCTATA CACGCTGACATGGGCCCTGTA CTCGAAGATATGGAGGCCCAA CCGCATTGTCCTGGTTCCCAA |
| 1595 | CYP51A1 | NM_000786 | TCCGTTACAAACGAAGATCAA TAGGGAATAATCGAACATCTA TAGGTAATTGGCAGATTCTAA AAGGTCCTTGGGAGTAATAAA |
| 1600 | DAB1 | NM_021080 | AAGGATAAGCAGTGTGAACAA AAGGATTAAGTAGGGATGTCAA |
| 1601 | DAB2 | NM_001343 | TGGGAGGTTATGTTTATTTGA TAGAGCATGAACATCCAGTAA ACGAAGTAGAAACAAGTGCAA AAGGTTGGCCTTAGTAGTCAA |
| 1603 | DAD1 | NM_001344 | CAACTTTCTAACAAACATTTA TTGCCTGAGAATACAGATCAA CAGCCTCTGCCTTTCATTAAA CAGATTTGACACTTACTGCTA |
| 1605 | DAG1 | NM_004393 | CAAGAAGATTGCCTTGGTAAA AAGGGTGTGCATTACATTTCA AAGAACCATATTGACAGGGTA CCGGTGGTGAATAACAGACTA |
| 1608 | DGKG | NM_001346 | CAGCATGTACGGAGGCACCAA CAGCCATGTTGCACGATTAAA TAGCCACTCACATAAAGTTTA ACCGCAAATGTGAATTATCAA |
| 1610 | DAO | NM_001917 | CTGGAGGGAAAGAACTATCTA ATGGAGGATAATTGAGGCTAA CAAGAATTATTGGTGAACGAA CAAGTTGTACTAACATATTAA |
| 1611 | DAP | NM_004394 | CTGCTGGGAAATCTAAGGCAA CACCAAAGAAGAGAAAGACAA |
| 1613 | DAPK3 | NM_001348 | CGCAGCCAAGTTCATCAAGAA CACCAACATCTCAGCCGTGAA CCGGCAGAAGGGCACGGGCAA CCCAGAGATTGTGAACTATGA |
| 1622 | DBI | NM_020548 | CTGGATTTGGTTACTGTGCCA CCAGTTAAACCAGCTACTCAA AAAGTGTATTTGTTACTTTAA CAACAAAGTAGAAGAGCTAAA |
| 1627 | DBN1 | NM_004395 | AAAGTTGATAGGAGACTTGTA CTCATGTCTGTTAATAAAGAA |
| 1629 | DBT | NM_001918 | ATGACTGTTCCTATACTAGTA ACGATTTGTATCGTATGATTA AACGATTTGTATCGTATGATT TAGCCATATACAGACAGTATA |
| 1630 | DCC | NM_005215 | CCGAGGAGTTCCAGTGATCAA CAGCATACCAATTACCCATAA TCACTGCAATGTCAAAGTTTA CACCTGTGTCCAAGAACTCTA |
| 1633 | DCK | NM_000788 | AGGCAGCCTGCTATAAAGTTA TGGACTCATCTTAGTTCTGTA TTGGTATAAATTAATTTGTTA AAGTTGCAAAGTGGAATTAAA |
| 1636 | ACE | NM_000789 | CTCCTGTTACCCGAAACGAAA CACCACAGAGGCTATGCTAAA CCCGAAATACGTGGAACTCAT CAGCGACGAGCATGACATCAA |
| 1641 | DCX | NM_000555 | AAGGGTTTGATGAATAGCACA ACCCTGCACATTTGATTCTAA ATGGACTTTAGTATTAGCCTA CTGAATAGACAGTCCATATAA |
| 1645 | AKR1C1 | NM_001353 | CACGTGGGAGGCCGTGGAGAA AATTGTTCTGGTTGCCTATAG CTTGGTGATTTCAGCAAGCTA AAGGTGGAATGTCATCCTTAC |
| 1649 | DDIT3 | NM_004083 | AGGGAGAACCAGGAAACGGAA CAGCTTGTATATAGAGATTGT CAGCTAGCTGAAGAGAATGAA AAGGAAGTGTATCTTCATACA |
| 1650 | DDOST | NM_005216 | CCAGTTTAAAGTGGATTACAA CAGCCTGTCTCTCATAAAGTA |
| 1652 | DDT | NM_001355 | AACATTCCTGTGATCACATAA TCCTCATATAGAAATAAATGAA |
| 1653 | DDX1 | NM_004939 | ATGAAAGTCTGTAGTCTTAAA CCGGATGGTTACATTGTCAAA CCGGGCAATCAAGGAACATAA CACGGTGTTCCTTATGTTATA |
| 1660 | DHX9 | NM_001357 | TTGGTTGTTGAAGTAACCAAA CTGTGGTTCATTGTAGTTTAA |
| 1665 | DHX15 | NM_001358 | CCGGACATGTACAGACATCAA AGGGACTATTATATTAATATA |
| 1673 | DEFB4 | NM_004942 | TGCCTTAAGAGTGGAGCCATA GCCCTAGAAGGTATAAACAAA GCGACATGTAAACCAAATTAA GACGTGCGACATGTAAACCAA |
| 1676 | DFFA | NM_004401 | TGCCTTGAACTGGGACATAAA CCGGAGCATCTCAGCAAGCAA |
| 1687 | DFNA5 | NM_004403 | CTGATAAGTTACAGCTTCTAA TAGAAAGAGTCTGACCCTTTA AACATACTAATTCTATGTCTA GCGGTCCTATTTGATGATGAA |
| 1716 | DGUOK | NM_001929 | AAGGCTCTCCATCGAAGGCAA CCGCCTGAAAGTACAGCTGGA CAGCACGATGGTCCTACACAT CACCTTTGTAAAGAATCTGTA |
| 1723 | DHODH | NM_001361 | CAGGTATGGATTTAACAGTCA CTCCGGGATTTATCAACTCAA CAGGGCTTTGGCGGAGTCACA CAGGAACTTGACTGTCTTTCA |
| 1733 | DIO1 | NM_000792 | TTGGGAGTTTATGCAAGGTAA CCCAATCAAGACAAATTGTTA CACAGTTAATCTGGACAGATA AAGAGGAGAGTACTCACTTTA |
| 1737 | DLAT | NM_001931 | CACAGCGATTAATGCAATCAA TTGGTTTATCATCGATGGGAA TACCTCATTATTACCTTTCTA CTCCAGCATCGTCTCATGTAA |
| 1739 | DLG1 | NM_004087 | CGGGTCAATGACTGTATATTA ACGGGTTATTAACATATTTCA ATGACTGTATATTACAAGTAA AACAGTGAAATTCAATTCTAA |
| 1740 | DLG2 | NM_001364 | CCGGACTAACGTGAAGAAGTA TACGCTCGATTTGAGGCCAAA CTCGGCTGTATGTGCGTAGAA CTCGACTAACCCACGAAGTAA |
| 1749 | DLX5 | NM_005221 | ATGTAAGTTATTGCATTTCAA CTGTATATTCTGGATAAATAA |
| 1756 | DMD | NM_004014 | CTGCTTCCAATTTGCTAATAA CAGGAGTCTAAAGCACTTTAA |
| 1760 | DMPK | NM_004409 | CAGCGAGGTAGCGGTAGTGAA CTGGGTGTATTCGCCTATGAA GAGCCTGAGGGCTAAATTTAA AACCAGAACTTCGCCAGTCAA |

Table4

| # | Gene | Accession | Sequence |
|---|------|-----------|----------|
| 1761 | DMRT1 | NM_021951 | CTGCATGATTAAGTGCTTTA TTGGTAAAGGGTGTAATTTAA AAGCCATTTGTGCCTCTCAA ATGGAGTACTTTGTTATATAA |
| 1774 | DNASE1L1 | NM_006730 | AAGGAAAGTTACTGAGAACAA CAGGGAGAAACAGACATCAA |
| 1777 | DNASE2 | NM_001375 | AAGGGCCACCACGTTAGCCAA GACCTTGATTGTGTGCCTTAA |
| 1778 | DNCH1 | NM_001376 | AAGGAGTTCTTAGCCAAATTT CAGGAGGTAATTGCAGACAAA CAGCAGAGCTTATAAGATCTA AAGGGCCACCACGTTAGCCAA |
| 1788 | DNMT3A | NM_022552 | TTGATTAATTGGCGTAACTTA CAGAACAAGCCCATGATTGAA CCCGTGATTGATGCAGATAAA CAGTGGGTGTACATTACGAA |
| 1791 | DNTT | NM_004088 | ACCGGTGAAATGACAGGGAAA CTGGGATTGGATTATATTGAA CTCAGTGGTGTGTTGAGAA AACACCGGGATCTATGTTTAA |
| 1796 | DOK1 | NM_001381 | CCGGCAGAAGGCCCAGGGAA AAGGATCCCAATTCTGGGTAA CCGCCTGGACTGCAAAGTGAT AGGGATCAAAGAAGAAGATGGTTA |
| 1803 | DPP4 | NM_001935 | ACACTCTAACTGATTACTTAA CAGTAAAGAGGCGAAGTATTA AACAATGACATTTATGTTAAA ATCGGGAAGTGGCGTGTTCAA |
| 1804 | DPP6 | NM_001936 | TACTGTCTTAATAGAAGGCAA TCCCTACACGTTATTGGCTTA ATGGTTAACCTGATAGAATTA ACGCTAATGGTTAACCTGATA |
| 1806 | DPYD | NM_000110 | CTGAGGATAGTAGTTAAATAA TCGGTGAATGATGGAAAGCAA CCCACGAAGGTTATAGTAAA TCGAGAGTATCCTGGCTTTAA |
| 1807 | DPYS | NM_001385 | AACACCCGACTTCCTCATGAA TGGGAAGTTTATTCCTCGAAA CTCCCTAGGATCCTTTAGAAA CGGCAGGAGATGGGAAGTTTA |
| 1808 | DPYSL2 | NM_001386 | TACCATGGAACGCCTAATAAA CAGGCTTGCGTTATTGGCCTA ACGGATTGCCAGATTTATGAA CTCGTGTTTATTATTCCTTAA |
| 1809 | DPYSL3 | NM_001387 | CTCAGCTTATATGAAAGTCAA AAGGATTGTAGTCATCCTGAA |
| 1810 | DR1 | NM_001938 | AAGATAGTATTAAGTAATTAA CAGCCGTATATGGAACATAAA |
| 1812 | DRD1 | NM_000794 | ATCGGTCATATTGGACTATGA CACAATTAACTCCGTTCCAA GACGGTGAGTATCAATAACAA ACGGTGAGTATCAATAACAAT |
| 1820 | ARID3A | NM_005224 | CAGGCTCTAGATTGAGACAA AAGGATATCTATATATCTATA |
| 1830 | DSG3 | NM_001944 | AAGGCCTCAGATTGAACAA CTCACTGGTGATGATACTAAA |
| 1832 | DSP | NM_004415 | CAGAAGAATGACTATGACCAA CCGACATGAATCAGTAAGTAA CAGGGAGATCATGTGGATCAA AAGGGCCACGGTATTCGCTTA |
| 1834 | DSPP | NM_014208 | GAGGATGTCGCTGTTGTCCAA CATGATAGTAGCATAGGTCAA AACGAGGGTAATACAAGTGAA TCGGATAGTAGTAAAGAATATAT |
| 1837 | DTNA | NM_001390 | CAGCCATAGAATTTAGTGTAA TGCGACCTATTCAATACATTA CAGCATTCAAATTATATAGAA CCCGTTGTCATAGCTATTTCA |
| 1838 | DTNB | NM_021907 | CAGCTTATTGCAGAACTGGAA CCACTTGACCTTGCACATATA |
| 1840 | DTX1 | NM_004416 | CCGGGAAGAAGTTCACCGCAA GTGGGAGTCAGCCAAGATTTA CACTGTGGGTGGAGAAATAAA GACCAAGAAGAAGCACCTTAA |
| 1844 | DUSP2 | NM_004418 | CGCGGGAAAGACCGAAAGGAA AAACTTAGCACTTTATATTTA CTGGTCCACCACCATGTTGAA CAGCCTGAGAGCTCAAGGAA |
| 1856 | DVL2 | NM_004422 | CTGGCTGGTGTCCTCAGATAA CACGCTAAACATGGAGAAGTA CCGGATACGAGAGCTCCTCTA CTGGGAGATTGGGCCATGTA |
| 1857 | DVL3 | NM_004423 | CCCGGCTAAATGGAACTGCGA CTCCTGGTTACTTCATCACAA CAGCATATCATATCCATTTCA ACCAAGTATGTTCCTCCTGAA |
| 1869 | E2F1 | NM_005225 | CTCACTGAATCTGACCACCAA CAGATGGTTATGGTGATCAAA CAGGACCTTCGTAGCATTGCA ACGCTATGAGACCTCACTGAA |
| 1870 | E2F2 | NM_004091 | TCCGTGCTGTTGGGCAACTTTA TAGGGACCAGGTAGACTTTAA ACCCATTGGAATGAGTTTAA TTGAGACGAGGGATTATTTCA |
| 1874 | E2F4 | NM_001950 | GCGGATTTACGACATTACCAA ACGAATGAATTCCTATATAAA ACCCGGGAGATTGCTGACAAA AGGTATCGGGCTAATCGAGAA |
| 1893 | ECM1 | NM_004425 | CTGAACACTCATTACACTAAA CCGGGACATCTTGACCATTGA |
| 1908 | EDN3 | NM_000114 | CTGGACGTCAGCAGTAATTCA TAGACACAGATCATAGCTCTA CAGAAGCATGCGACTTTCATA TTGCAGGAAGCCGACTGTAAA |
| 1912 | PHC2 | NM_004427 | CTGGTTCACAAAGAGCATTAA CCGGGTGGACTTTGCCTATAA |
| 1915 | EEF1A1 | NM_001402 | CAACGTTACAACGGAAGTAAA AAGCTGGAAGATGGCCCTAAA CTGGTTAATGATAACAATGCA |
| 1933 | EEF1B2 | NM_001959 | CTGGATCATGGCATTTAAATA ATCCTGGATCATGCATTTAA |
| 1936 | EEF1D | NM_001960 | CTGGTTCGACAAGTTCAAATA CCGGCTGAACGTGCTGGAGAA |
| 1937 | EEF1G | NM_001404 | CACCCTGTTGTGGGCTCTATAA ATGGCCCAGTTTGATGCTAAA |
| 1938 | EEF2 | NM_001961 | TGCCGTTTCTTTCAATATTTA CCGCGCCATCATGGACAAGAA |
| 1942 | EFNA1 | NM_004428 | CACCATACATGTGCAGCTGAA TACAATGTTCTTTGTCTCAAA CCCGGAGAAGCTGTCTGAGAA CACGTGTATAGTAGTATCGTATA |
| 1945 | EFNA4 | NM_005227 | CTGGAGAGCTTACTACTACA CTGGATTTGGTATGATCAAA |
| 1947 | EFNB1 | NM_004429 | AGCGACATCATCATTCCCTTA ACGGACTACAGAGAACAACTA CTACATTACCTCAACATCCAA CTCACGCGGTAATTAATGTCTTA |
| 1952 | CELSR2 | NM_001408 | CCCACTATACAGTGAATGTTA CCGGGAGGAAGTGATTTCTA CACGTGGTGTCTTCAACGTA ACGACAACAATCCAACCTTTA |
| 1953 | EGFL3 | NM_001409 | CCCGTATTTATGTAAAGGTAT CAGGACCCCAGTTTGATGCTAAA |
| 1955 | EGFL5 | XM_376905 | TTGGAAGATGATGGCAATGAA CCCAGTAAAATTGACCATTAA |
| 1956 | EGFR | NM_005228 | TACGAATATTAAACACTTCAA CCCATCCAATTTATCAAGGAATTA CAGGAACTGGATATTCTGAAA |

Table4

| | Gene | Accession | Sequence 1 | Sequence 2 | Sequence 3 | Sequence 4 |
|---|---|---|---|---|---|---|
| 1964 | EIF1AX | NM_001412 | CACGTAGAAATGTATATTTGTA | AAGTCTGTTGTTGTCATATTTAA | | |
| 1969 | EPHA2 | NM_004431 | CAGCCTTCGGACACAGACATATA | TTGCAGATGATTCAAAACCGAA | TCGGACAGAGACATATAGGATAT | CAGCGCCAAGTAAACAGGGTA |
| 1973 | EIF4A1 | NM_001416 | AAGGATCATGTCTGCGAGCCA | CCCGATGGGATGGAGCCCGAA | | |
| 1979 | EIF4EBP2 | NM_004096 | CAGGAATAAGCTGGATCTCCA | CACCTTAATTGAAGACTCCAA | | |
| 1993 | ELAVL2 | NM_004432 | CTGAATGGATTGAGACTTCAA | CAAGACCAACTTAATAGTCAA | | |
| 1995 | ELAVL3 | NM_001420 | CTGGTGCATCTTCGTGTACAA | CCCGACTTTAGCTATAGTTAA | | |
| 1996 | ELAVL4 | NM_021952 | ATGTCCGACATCCAATACAA | AAAGGTGATTCGTGACTTCAA | TTGGAGCAGTGAACAACGTAA | CCAGTCGTTGCCTAAGTATTAA |
| 1997 | ELF1 | NM_172373 | CAGAACTTAAATAGAAGGCAA | CAGAGTGAATTTGTATTTAAA | | |
| 1998 | ELF2 | NM_006874 | AAGCATCAGTTCACAGCAGTA | AACAACTGAACCCATCAATAA | CACCAGATAGCCATGAACCAA | TACGATATCAAGTTACACTAA |
| 2009 | EML1 | NM_001008707 | CAGGCCGTGCTTAACAGGAAA | CAGATTGAAATTTATTGTTTA | | |
| 2012 | EMP1 | NM_001423 | TAGTATGTTATTCCTGGTTAA | CTCTATGTTTATTCTAGTTAA | | |
| 2020 | EN2 | NM_001427 | CCCGCTCTTTCGGATCGCTAA | TACGTTGTACATAATAGTGTA | CACCAAGTACCTATATATGTA | CCCACGTAAACAATATGGAA |
| 2021 | ENDOG | NM_004435 | CGCACCTACGTGATGCCCAA | CTGGAACAACCTGGAGAAATA | CTGATGGGAAATCCTACGTAA | CCCGGGCGAGCTGGCCAAGTA |
| 2022 | ENG | NM_000118 | AAGGGAGAACTTGAAACAGAT | CGCCATGACCCTGGTACTAAA | ACCAATAAATCAGACCATGAA | CAGCAATGAGGCGGTGGTCAA |
| 2027 | ENO3 | NM_001976 | CCAGACCTGCTTCCTGAAATA | TAAGAGCTTTATCAAGAACTA | | |
| 2029 | ENSA | NM_004436 | TTGGAGAAATATCAATGAAA | TCAGTATTCGAAATAAACAAA | CTAGTTGAGAACTCAACATTA | TAGTTGAGAACTCAACATTAA |
| 2030 | SLC29A1 | NM_004955 | CCGCCTGGAATTCTACCGCTA | CAGCCTCAGGACAGATACAAA | | |
| 2033 | EP300 | NM_001429 | CAGGAATGAATGTAACAAAT | CACCGATAACTCAGACTTGAA | CCGTTTGTGGTTTAAAGCAAA | TTGGACTACCCTATCAAGTAA |
| 2035 | EPB41 | NM_004437 | ACCGAGCAGCTAAGAAATTAT | CACGGCCTAGTGAATGGGATA | GAGGATTTAGACAAGAGTCAA | TAGGATATCAGAATTGTTCAA |
| 2037 | EPB41L2 | NM_001431 | CAGGTCTTAGATAAAGAGGAA | ACGCAGTAACTTCTACATTAA | | |
| 2039 | EPB49 | NM_001978 | CAGAAGATCTATCCCTATGAA | CCGCCCAGATTCCAACATCTA | | |
| 2040 | STOM | NM_004099 | CCCATTCATTACACATAGTATAA | TTGGTGATTTATATAAGGTAA | | |
| 2043 | EPHA4 | NM_004438 | AGGGTGTATATTGAGATTAAA | CCAGTACTGAATAAACTCAAA | CCGCCAGGACATTTCCTATAA | CCCGCGAATGAAGTTACCTTA |
| 2044 | EPHA5 | NM_004439 | ACCAGCTACACGATTATCAAA | AACGGAATGGTGCCATTGTAA | CCCGGCAGTATGTGTCTGTAA | ACCAGTTGGATCTCCAATGAA |
| 2045 | EPHA7 | NM_004440 | CCCAATGAGTCATCACAGAA | TACGAGAAAGATCAAAGGGAA | CAGGCTGCGAAGGAAGTACTA | ACCAGTCATGATAGTAATAGA |
| 2047 | EPHB1 | NM_004441 | GAGATGTACCTTCAATTGAAA | ATGATACTGACTGATTAATTA | GCGGGATGATGTGACCTACAA | ATGGCCCTGGATTATCTACTA |
| 2048 | EPHB2 | NM_017449 | CCCGGATCATTCACTGTGATA | TAGGCCTCACTCAACAACCAA | CACGCTTTCTAGAGGACGATA | |
| 2051 | EPHB6 | NM_004445 | CGCCAATCTCTAGATCAACAA | CCGGGAGACCTTCACCCTTTA | CTGGAGCTTTGGGATACTCAT | AACCGCCAATCTCTAGATCAA |
| 2052 | EPHX1 | NM_000120 | CCACTATGGCTTCAACTCCAA | TTCCAGGAATTCTACATTCAA | CAACAGATACCCTCACTTCAA | CAACATGCTTTGATGATAAA |
| 2058 | EPRS | NM_004446 | AAGCATGAAGAGCTAATGCTA | CAGGAGGAGACTATACAACTA | AAGGAGGTGGGCTCTCATCAA | AAGGCGATTACTCAGTGTTAA |
| 2059 | EPS8 | NM_004447 | CGCCATATAGATAGAAATTAT | TTGGATATTGTGAGACCTCCA | CAGGTGGATGTTAGAAGTCGA | TCGGTTCTAAAGGATGATATT |
| 2065 | ERBB3 | NM_001982 | AACACTGGTGCTGATCGGCAA | CTTCGTCATGTTGAACTATAA | CCAGAGCTTCAAGACTGTTTA | ACCGGCGATGCTGAGAACCAA |
| 2066 | ERBB4 | NM_005235 | CACCGGAATACTGTGGTGTAA | TCGGGATTTGGCAGCCCGTAA | CTGGAGAATTTACGCATTATT | CTACGTGTTTAGTGGCTCTTAA |
| 2067 | ERCC1 | NM_001983 | CACCGTGAAGTCAGTCAACAA | CACCGGGTGACTGAATGTCGA | CCCGGGTGACTGAATGTCTGA | ACCGTGAAGTCAGTCAACAAA |
| 2068 | ERCC2 | NM_000400 | CGGGAGGATATTGCTGTGAT | CCGATTCTCATGAGGAATTTGA | TCGAAAGTTGCTCAACTTCTA | CCCGCTCACCCTCCTTGCTAA |
| 2070 | EYA4 | NM_004100 | CTGGGTCAGTAATTACAAGTA | CAGCTTTAGCAAAGAAACTCTT | AGCGTATATGACATCGAATAA | CAGTCCTATTTAGAACATGTAA |
| 2071 | ERCC3 | NM_000122 | ACCCATGTCCATGAGTCACAAA | CCGGTTCACCTCCGATGCCAA | CCGGTTCCACCTCGATGCCAA | ACCGTTCTGAATAAACAGAAT |
| 2103 | ESRRB | NM_004452 | CAGCTGCGGCTCCTTCATCAA | AGCAGGCTTTGGTGTTTCAAA | CCGCTTCATGAAATGCCTCAA | CAGCACTTCTATAGCGTCAAA |
| 2113 | ETS1 | NM_005238 | CTCGGATTACTTCATTAGCTA | CCGACGAGTGATGGCACTGAA | TCGGATTACTTCATTAGCTAT | ATGCATGTCGTTCCAAACTAA |
| 2114 | ETS2 | NM_005239 | CCCGGTCAAGTTGGTTTCAAA | CTGTGATGAGTCAAGCCTTAA | CTCATTGTATGTGAGGTTGAA | AGGGATTTATGTAGCAGCTAT |
| 2118 | ETV4 | NM_001986 | ATGGGCTATGGCTATGACAAA | AGGGATTTATGTAGCAGCTAT | AGGGATTATGTAGCAGCTAT | ACCGGAGTCATTGGGAAGGAA |
| 2119 | ETV5 | NM_004454 | CAGAAATTACTCAATGGGAA | CAGGATCTCAGTCAACTTCAA | CCGGAGTCATTGGGAAGGAA | |
| 2121 | EVC | NM_014556 | CAGCTAATCGATAATATGGAA | AAGAAGCAACTTGTAGTTTAA | | |

Table4

| No. | Gene | Accession | Sequence |
|---|---|---|---|
| 2122 | EVI1 | NM_005241 | CTGAACTAGCAGATCACCAAA CCAGTGCTCTATCCAGTCCATAA GAGCTGTGAATGATTCTATAA CAGCACTACGTCTTCCTTAAA |
| 2123 | EVI2A | NM_014210 | AACCCTATAAACTCCTGAAGTA CATGGCTAGACCTTGCAATTTA ATGGCTATGCTAATTTGCTTA GAGCTAAACTGTGTAATTTAA |
| 2124 | EVI2B | NM_006495 | CAGGATTTATCTTAGATACTA CAGGTATTGGCTAATTCTCAA |
| 2130 | EWSR1 | NM_005243 | CAGAGTAGCTATGGTCAACCAA CTGCATTGACTACCAGATTTA TCCGGGTTGTGGAAACCAGAA CAAGAGGGCCTCTTAACTGTA |
| 2134 | EXTL1 | NM_004455 | CCCGACTGCATCAACCAGATA TAGATGGATGGCAGACATTAA |
| 2138 | EYA1 | NM_000503 | CCCATTGGCACCGAAGTTAAA CAGGAAATAATTCACTCACAA TCGGTATAAATATGTAGATAA AACTCGGTAACTCTCATATAA |
| 2151 | F2RL2 | NM_004101 | TCAGTTAGGATACATCACTAA AAGGCTAACTTGCTAATCACA CAGTTAGGATACATCACTAAA CAGGAGCCACGATTACTGTAA |
| 2153 | F5 | NM_000130 | TTGGATAATTTCTCAAACCAA CCCATTCAAGACCCTGATCAA TAGGAATTAGGTCATTCCGAA CGCCATTAATGGATGATCTA |
| 2155 | F7 | NM_000131 | CAGTTGAATATCCATGTGGAA TCGAACTGTCCTGGCACCAAA CAGGGTCTCCCAGTACATCGA CTGGTTCTTATCCATTATCAT |
| 2162 | F13A1 | NM_000129 | CACGAGCGTTCACCTGTTCAA AGCGTTGACATTCTATTGGAA CAAGGAGGATGGGACACTAA TACAATGTAATAGAAGCTTAA |
| 2177 | FANCD2 | NM_033084 | CGGCTTCTCGGAAGTAATTTA TTGGAGGAGATTGATGGTCTA CAGAGTTTGCTTCACTCTCTA ACGGGAGGAGTCAGAATCAA |
| 2178 | FANCE | NM_021922 | TCGAATCTGGATGATGCTAAA TAGCCTGAGGATAAAGGCTGA CTGACTTGAATAATTTATCAA AACGCCGAGGAGAGCTTGTAA |
| 2182 | ACSL4 | NM_004458 | ATGCATCATAGCAATTTGATA TTGGAGCCGATTTGAAATTCCA CAGATTATAGATCGTAAGAAA AAGGGCAGAGTTACTTGATAA |
| 2189 | FANCG | NM_004629 | CCGGCTCGTTCGACAGGCCAA CCGCGGGAACTAACTCTTCAA CTCATTGAGGTAGAATTACTA CCCAGGTAATCGAGACACTTA |
| 2192 | FBLN1 | NM_001996 | CAGTCATGTGAAGACATCAA CAGGATGTGTGTCGATGTCAA |
| 2197 | FAU | NM_001997 | GGCCGCATGCTTGGAGGTAAA CCGCCGTTCAGTCGCCAATAT ACAGGAGGAAGAAGAAGAA AAGCCACTTAGTTCAGTCAAA |
| 2200 | FCAR | NM_002000 | TGGAACTAAGTTATTCGGCAA GAGCTTATTGTCGTAAGAATA |
| 2205 | FCER1A | NM_002001 | TCCATGGAATAGAATATTTAA CACATTTCTCTTGAAGATTAA |
| 2212 | FCGR2A | NM_021642 | CCCAATGTCCAGCCTCTTTAA AACCCTCAACAACAAATTAAA |
| 2214 | FCGR3A | NM_000569 | TTCGCTCGTAAGTTATGAA AACCCTGAGAAGTCAGATGAA |
| 2220 | FCN2 | NM_004108 | CAGCTTTGCAAATGGCATCAA AAGGATACAATTATAGCTACA CCGGGACTGGGCCACGTACAA CTGCCATGTGTCAAACCTGAA |
| 2232 | FDXR | NM_004110 | CAGGTTGGGAACATGCTGAA TGGGAACATGCTGGAAATAAA CACGTGGACATCCTCAGGAGGTAA AAGCGTGCCCTTTGACTCCAA |
| 2235 | FECH | NM_000140 | CTGGAATATTAATGCTAAACA CCGACTGGTGTGGCAATCCAA ACCCATATCCTCAGGAGGTAA CCCAAGGGTAATAAACGTGTA |
| 2237 | FEN1 | NM_004111 | CACAATGATGAGTGCAAACAT TAAGTCCATTGTTACATGAAA CTGACTGCCAGTGAAGCCAAA CAAGAGCTACTTGGCCGTAA |
| 2239 | GPC4 | NM_001448 | ATGGCCATTATACATGCAAA TACCAAATGATAGCTCATAAA CAGATTGTATTCAAAGCATCA TAGGTAGAGCTGAGAATACTA |
| 2243 | FGA | NM_000508 | AGGGTTGATTGATGAAGTCAA AGGGCTTTAGCTCGTGAAGTA CAGCCAATAACCGTGATAATA ACGCGTCGTTCATGCTCTAAA |
| 2244 | FGB | NM_005141 | ACCGTATAGAGTATACTGTGA CAGGAAATGGGATCCCATATAA TAGAGTATACTGTGACATGAA CAGCACGTATGACAGAGACAA |
| 2246 | FGF1 | NM_000800 | GCCCATTTCGAAGATACACAA GAGAATGATAGTAATAGGATA AAAGTAGGAACTCTTAAGCAA CTGATACTTATTTACCTATAA |
| 2247 | FGF2 | NM_002006 | TGCCTTGTGGATATCAAGAAA ATGCTAAACTTTACTGATGTA TAGCACTAGTCTTAAATTGTA AACAATATTAGTCGTATCCAA |
| 2248 | FGF3 | NM_005247 | CGGGCGGTACCTGGCCATGAA TTGTGTCATCACAACATTAAA CAGGGTCCAGTGGGAACTGAA TTGCTCCTGGGTGGAAATTAA |
| 2252 | FGF7 | NM_002009 | CACAAGAGTCTAGATCAGTTA TAGTTTGTAGCTACAGTAGAA AGCCTTTACATTGTACACAA CACCCGGAGCACTACACTATA |
| 2253 | FGF8 | NM_006119 | GCGCTTCGAGTTCCTCAACTA CAAGAGCAACGGCAAAGGCAA CACGGAGATTGTGCTGGAGAA CTACATCTGCATGAACAAGAA |
| 2254 | FGF9 | NM_002010 | TTGGATATACCTCGCCTAATA TCGGATAGCTAGTTGCATTAAA TACGTACTCGTCAAACCTATA ATCGCAGTGAACTTAAAGCAA |
| 2259 | FGF14 | NM_004115 | CAGGAAGTAAGTCAATGCTTA AGGGTTGTATATAGCCATGAA |
| 2262 | GPC5 | NM_004466 | AAGAATTTAGTGTATCGTGAA CAGCTTGTGCTAATGAATTA |
| 2264 | FGFR4 | NM_002011 | CAGGAGGTTCTGGGCCTCTGA CCGCCTGACCTTCGGACCCTA CACATTGACTACTATAAGAAA |
| 2268 | FGR | NM_005248 | CAGCACTAAGGTGGCGGTGAA TTGATTCTGTAAATAAGTAAA CAGACCTTGTCTAGTTATTTA CACGTGGACACGGCAGCACTAA |
| 2271 | FH | NM_000143 | GAGATCTACGATGAACTTTAA CAGGAATTTAGTGGTTATGTT CCCAACGATCATGTTAATAAA TAGGATGTGTCTAGGTCTAAGA |
| 2272 | FHIT | NM_002012 | TCCACTGAACAGTCTGTAA TTCGCTCTTGTGAATAGGAAA |
| 2274 | FHL2 | NM_001450 | ACCCTTTAGTGCTTTGTGATA CTCCCGTTGCGTCAAGTCTAA AAGTAATGACCAGTTGTTAT ATCGAGTAAGGCACACCCAAA |
| 2281 | FKBP1B | NM_004116 | CACTGCCTTACCTTCACTTAA CAGAGGGACTTGAGCCAGTTA CACAAGGTGCTCAGACATGAA AACAATGTCCTCTTTGAGAAA |
| 2288 | FKBP4 | NM_002014 | CACGCTTGTATTTGAGGTGGA AGGGATGAGGGTCTGATAAGAA TTGAGTGAATTAGACCTTTAT AGCAATAAAGTGGAAACAATA |
| 2289 | FKBP5 | NM_004117 | GCGACAGGTTCTCTACTTAAA TCGGCTGGCAGTCTCCCTAAA AACGCCGATGATTGGAGACAA ACGGGTAAAACAGATTGAGCAT |

Table4

| | | | |
|---|---|---|---|
| 2296 | FOXC1 | NM_001453 | CCGCCACAACCTCTCGCTCAA AACGGGAATAGTAGCTGTCAA CTCCAGTGAACGGGAATAGTA ACGGGAATAGTAGCTGTCAAA |
| 2298 | FOXD4 | NM_207305 | AAGACCATCATCCCTGGACTA TCCACCACACATGGTCTTGAA |
| 2301 | FOXE3 | NM_012186 | CACCAGGGCTCCTGACTGAAA TAGGACTTATTGGGTTATTAT |
| 2305 | FOXM1 | NM_021953 | CCAAATTATCCTCTAATTATA CCGCCGGAACATGACCATCAA |
| 2306 | FOXD2 | NM_004474 | CCGAGTGAGCCTTAACATCCA CACTGGGTGTGCATTGATTTA CCGGCGGGTCGTGCCCTGGAA CCCATTGGTGTCAATTGAATA |
| 2313 | FLI1 | NM_002017 | ACCTTTGTATTTGTTCTTTAA CAGGACATATGTGGCCTTGAA |
| 2316 | FLNA | NM_001456 | CACAGGCAACATGGTGAAGAA CCGCTTCACCATCGACACCAA |
| 2321 | FLT1 | NM_002019 | CCCGAATCTATCTTTGACAAA TCGCCGGAAGTTGTATGGTTA CACTACAGTATTAGCAAGCAA ACCGCATATGGTATCCCTCAA |
| 2322 | FLT3 | NM_004119 | ACCAATTCAAGTGAAGATTAT CAGGTTTAAAGCCTACCCACA TACGTTGATTTCAGAGAATAT CCGGCTTGAGTGAATTGTGTA |
| 2323 | FLT3LG | BF688722 | TAGTATAGTTGAAATAAGAAA GCGGGATTTGTTGAAGAGAAA GCGCGTGAACACGGAGATACA CTCCTCCGACTTCGCTGTCAA |
| 2324 | FLT4 | NM_002020 | CCGCCGATTATTCCTTGGTAA CGCGCTGATGTCGGAGCTCAA CACGCTCTTGGTCAACAGGAA CACCGTGTGGGCTGAGTTTAA |
| 2326 | FMO1 | NM_002021 | CAGTACTTTCATAGCCGGCAA AGGCCTCACTGTACAAGTATA ATCATGCAAATTACGGCTTAA CCGAACATTCAAGGTCATCAA |
| 2328 | FMO3 | NM_006894 | CCGGACATCATGTGTATCCCA AAGAATCATGTCATGATCTTA CCCGCTGGGCAGCACAAGTAA AACTATGTTCTTTATATCTAA |
| 2329 | FMO4 | NM_002022 | ACGAAGATTATCCTAATTTCA ATCGGCCTTATCGGCCTTAAA AAAGAAGATATTTCTATACAA CACGATGGGTCACAAGAGTAT |
| 2330 | FMO5 | NM_001461 | AAGGCGGACAGGCGACACTAA AACCCAAAGGTTTGACCATGAA CTGAGTCAGCATCCAACCTTA CTGGGCCACTCAGGTATTTAA |
| 2334 | FMR2 | NM_002025 | CACGTGATAGTCATAACCCTA CTGGGTAAGACTACTCAGTAA TAGGAACTAATTAAACATGGA CGGCCTTATTCTCATAATTAA |
| 2348 | FOLR1 | NM_000802 | CTGGACTTCAGGGTTTAACAA CACATGTGTCTTGAGAATTAT CCCACTGTTCTGTGCAATGAA TAAGGATGTTTCCTACCTATA |
| 2353 | FOS | NM_005252 | TAGAGGGTTCCTGTAGACCTA TGGGTTCATTATTGGAATTAA ATGGATTCTCAGATATTTATA GACCAATATTATACTAAGAAA |
| 2356 | FPGS | NM_004957 | CTGGAGTGAATTAAAGCCTTT CCGCACTGTCCCAGTAGCCAA |
| 2357 | FPR1 | NM_002029 | ACGACCCTAAAGAGAGGATAA TTGCAAGGCATGTACAAAGAA CACCGTGAGCCTGGCCAAGAA AACCAGTGACACAGCTACCAA |
| 2475 | FRAP1 | NM_004958 | CCGGAGTGTTAGAATATGCCA CCGGCACAATGCAGCCAACAA ACCAATTATACCCGTTCTTTA CAGGCCTATGGTCGAGATTTA |
| 2488 | FSHB | NM_000510 | CAACATCACCATTGCAATAGA TCGTTTCTGCATAAGCATCAA AAGAATGTCGTTTCTGCATAA CACCAGGGATCTGGTGTATAA |
| 2515 | ADAM2 | NM_001464 | CGGGCTGCGGATGGACAGTAA TGCTATAATGGTGAAAGTTAA CTGTTGGTTAGTAGACACTAA AACCATATACTGTGAATTTAA |
| 2516 | NR5A1 | NM_004959 | ACGCAGGTGCATGGTCTTCAA AAGGATAACCGAGTTTGCTAA CAGGGAGAAGTTGAGCAGGTA CTGGAATAAATTTCGCAATTA |
| 2517 | FUCA1 | NM_000147 | CCGGAAGAGGAACATCCGCTA TACGACCTTGTTAACAGCTAT TCCACTCTATCTACTTGATAA TTCGCTATGTTTACAGTGATA |
| 2521 | FUS | NM_004960 | ACAGGATAATTCAGACAACAA CAAGCAGATTGGTATTATTAA CTGGGTGAGAATGTTACAATT ACAGCCCATGATTAATTTGTA |
| 2524 | FUT2 | NM_000511 | CAGCGGCTAGCGAAGATTCAA CACGGGCGGAGACACCATCTA CAGTGCTAGCCTCAACATCAA CACCATCTACCTGGCCAATTA |
| 2531 | FVT1 | NM_002035 | CAGGCAAAGAAAGAAATTGAA TAGACTCAAATGCTTAGGGAA CAGGAATGTGGCTGCAAGCAA AAGAATTTACAGTTCCTTCAA |
| 2532 | FY | NM_002036 | CTGGACACCCTTGGAAGCAAA CACCTGTCAACCTGAATTAAA CAGCTGGACTTCGAAGATGTA ACCAGTGTCCTGGGTATCCTA |
| 2535 | FZD2 | NM_001466 | CACGGTGAGACCACCGTGTGA CACCATGGTGTCGGTGGCCTA CACGTACTTGGTAGACATGCA CACGGTCTACATGATCAAATA |
| 2537 | G1P3 | NM_002038 | CAGCAGCGTCGTCATAGGTAA CAGAATAAACTTCACCCAGAA |
| 2538 | G6PC | NM_000151 | TAGCAGAGCAATCACCACCAA CTGGCTTATTCCCATGTGTGA TGGGATCCAGTCAACACATTA CAGCAGGTGTATACTACGTGA |
| 2539 | G6PD | NM_000402 | ATCGGGTGACCTGGCCAAGAA CACCAAGATGATGACCAAGAA CTGCGTTATCCTCACCTTCAA CAGCCTCTCTGCTATAAGAAA |
| 2542 | SLC37A4 | NM_001467 | TTGGATATAGGTGGGAAGAAA CCGGGTGGCGCTGGAAGTTAT |
| 2547 | G22P1 | NM_001469 | CAGGTTAAAGCTGAAGCTCAA GAGGGTTAGCGTTAGCCTTAA GAGGATCATGCTGTTCACCAA CGCCTAGTGAGCAGTAGCCAA |
| 2556 | GABRA3 | NM_000808 | TACAATGAGGTTAACAATTCA CCCGACTGAGACCAAGACCTA CAGAGATAATCCGGTCTAGTA CCCTCAATTCATATTTATGAA |
| 2561 | GABRB2 | NM_000813 | TCCTATAATGTAATACCTTTA TACCTATTTCCTGAACGATAA CAGAAGCACAATGCTAGCCTA ATCCCTCAGCTTTAAGCTTAA |
| 2563 | GABRD | NM_000815 | CCGCGGCACCAGAGCGATGAA ATCGGAGGACATCGTCTACTA CACCTTCATCGTGAACGCCAA CACGGAGCTGATGAACTTCAA |
| 2564 | GABRE | NM_004961 | CCGCATCTACAAGGATGGCAA CAGGCATTACTCGCCATTGAT CAGCAGACCATTTCAAATTAT CACTCTAACCATCACAATCAA |
| 2566 | GABRG2 | NM_000816 | CACCATCAGATCATAAGCATA CACCCTAAGGTTGACAATTGA TAGGAGTGAAGCCAACGTTAA TAGATTCATAATGCAATTAGA |
| 2568 | GABRP | NM_014211 | TAGATTGGTCTTACAGTTTGA AAGTGGCTTAGCTTAAGTAAA GAGCCCAAGATTACAAATGTA CAGAAATAAACCAAGGCTCTA |
| 2569 | GABRR1 | NM_002042 | CAGGGTTACAGTAACTGCAAT CACCACCAAACTGGCTTTCTA ACGTGCTCTCTTGAAATTGAA CAGAAATAACTGTATAATGAA |
| 2572 | GAD2 | NM_000818 | ACGGGCGGCATCGGAAACAAA CGCCTTGGACAAGATTTATAA AAGTTTCGATAGATCAACCAA CAACCTGTTCTTCCTACCAAA |

Table4

| No. | Gene | Accession | Sequence |
|---|---|---|---|
| 2580 | GAK | NM_005255 | CACGCAGTACACGGTCTTCCA AAGGCCTAACTATGCCTCGAA CCCGTGGTTGTCTCGTACAGAA ACGCGGTGTGACATTCAAGAAA |
| 2581 | GALC | NM_000153 | TTCAAGGTGGTTGATGTTATA TACACTCACGTTAACTATTAA CAGTATTATAGGAGACTACAA TAGGAATTTATAAGAGTCTAA |
| 2585 | GALK2 | NM_002044 | TCCTAGTTACTGAAGCTAAA CTGGAGGAACTCCGAACCCAA TGCGCGAGTGCTCCAGTTTAA AAAGCTCTCTATGCTTCATAA |
| 2615 | GARP | NM_005512 | CTGGAGTTTGAGACTATGGAA AACCAACAGTATAAAGCCTAA |
| 2622 | GAS8 | NM_001481 | CACCGACATTAAGAACTACTA ATGCATAAGTATATTTGTTTA |
| 2628 | GATM | NM_001482 | TAGAATGTACACAGATCCAAA CCACTAATAATTAACCATCTA ACCGAGCCGTACAGGTCAATTA |
| 2629 | GBA | NM_000157 | CCGGAATTACTTGCAGGGCTA CCAGAACAGAAGTTCCAGAAA CAGGGCTAACCTAGTGCCTAT AAGAAGGAATCGGATATAACA |
| 2642 | GCGR | NM_000160 | CCGCAATGCCATCCACGCGAA CAGCTCCGTGCTGGTCATTGA CTCCAACAATAAAGAGCTCAA CAACAGAACCTTCGACAAGTA |
| 2645 | GCK | NM_000162 | CTGGACAAGCATCAGATGAAA CACGATGATCTCCTGCTACTA CAGCTGTATGAGAAGCTCATA CAGGACTTTGATGCATTTCCA |
| 2661 | GDF9 | NM_005260 | ATCAGTGGAACTGCTATTTAA CAAGTCAGTCTTGCTGTACAA TACCGTTGAACACTTACTCAA CAGCTGGTATTCCCTTCACTA |
| 2662 | GDF10 | NM_004962 | AGCGCTCGAGGTGCTATGCAA AACAGGATAATCGTGGTGTAA CACGGAAAGAACTCTGTTTAA TAGTAACGTGGGAATACTAAA |
| 2664 | GDI1 | NM_001493 | ATCCACTGACCAAATCCTTAA ACCCTTATCTTGCAAAGTGAA CTCTAGTGTATTTCACAGAAA CCGCATCAAGTTGTACAGTGA |
| 2668 | GDNF | NM_000514 | CGGGACTTTAAGATGAAGTTA CTGGAGTTAATGTCCAACCTA CTGGTTTACCCATATTCTAAGA CGCGCTGAGCAGTGACTCAAA |
| 2671 | GFER | NM_005262 | ATGGCCCAGTTCATACATTTA CCGGGCCTGCGTCGACTTCAA |
| 2677 | GGCX | NM_000821 | CCGCCTGCTTCCCAAGTATAA CAGACCTTTCTTAGACGCCAA TACGTCTATGTCAACACTACA CAGGAAGAGAGTTAGACGCAAT |
| 2702 | GJA5 | NM_005266 | CTCCCTCTACTCAGCCTTAAA CTCAATGACGTTGCTCATTAA |
| 2705 | GJB1 | NM_000166 | CTGCACAGACATGAGGACCATA TGCCTGAAAATGTTACACATTA GCCTGAAAATGTTACACATTAA |
| 2720 | GLB1 | NM_000404 | CCAGATGGTTGAAATTGA ACCACTGATGGAGCACATAAA CAGAATGAGGGCCAGTTATA CACCAAAGTTTGCATATGGAA |
| 2740 | GLP1R | NM_002062 | CAGGAACTCCAACATGAACTA CAGATTCAAACTCAAGTCAA CCCATAACTCGTGGACTACAA CCGCTTTAACTTCTCTGCCTA |
| 2741 | GLRA1 | NM_000171 | CTGGAGTTAACAAACAGCAA TCGGATTTCCTGGATAAGCTA CAGTGAATGCCTATAGAAATA CAGAATTGCTTACCATAATT |
| 2746 | GLUD1 | NM_005271 | CAAGCCTAACCTTGACTCAAA CCTATAGAAAATAAATAAATGAA GTCCGTTTAACTGCGTAAGAA CAGAATTGCTTACCATAATT |
| 2768 | GNA12 | NM_007353 | TAGCTATACTTGTGTACACATA GAGGTAGTTGTGCCTCAATTA GTTCGTTAACTCGATAGAAA CCGGATCGGCCAGCTGAATTA |
| 2769 | GNA15 | NM_002068 | CTCCGTGCGGCTGAAATTAA CCGGTTTGCGGACGAGAGAAA CAGCATCTGTTTCCGGACTA TCCGTGCGGGCTGAAATTAAA |
| 2779 | GNAT1 | NM_000172 | CACCGACACGCAGAACGTCAA GAGGTTCATCCTGGACATGTA ACGCACAGACTCAGCAATAAA ACGCGCCATGACCAATAAA |
| 2780 | GNAT2 | NM_005272 | TAGGGAGTCTCAGTCAATGTA CAGCAGACTCAGCAATAAA AACCAATTAGAACGAATTACA ACGGCAAATATGGGAAGTGGA |
| 2783 | GNB2 | NM_005273 | CGCCTGTGATGCCTCTATCAA CGGCCATGAATCCGACATCAA CCAGAACTACACGAATCCTTA CAACATGTTGTTCCATCTACAA |
| 2784 | GNB3 | NM_002075 | CAGATTGCAGATGCCAGGAAA TCCGGGTGCCATTCCCACTAA GTGGAACAGCTTAAGATTGAA CTGGAGCTACTGGGAGGGTAA |
| 2785 | GNG3 | NM_012202 | CTCGGTGACCTGCTCAGACAA GTGGAACAGCTTAAGATTGAA ACGGCTAAACTCGGATTGTGA CAAGATGGTGAACAGCTTAA |
| 2788 | GNG7 | NM_052847 | CAGGGCATCATTCAGTAATTA ACGGCTAAACTCGGATTGTGA ATGGTTGAACCCGTCGCTAAA ACGCATCCATCTCCAAGTTAA |
| 2794 | GNL1 | NM_005275 | TCGGCTTTCCACTAAGGTCAA CCAGGCTACAGTGAACAGAAA ACGGTTGCATTTGCCACTTCA |
| 2798 | GNRHR | NM_000406 | ACCAGTATTGATGAATAACTA CAGGTTGTCAGACCCAGTAAA AAGCATGGATTGGATCAGTAA |
| 2799 | GNS | NM_002076 | CGGCGGCCATGACACCGCTAAA AAGCAAGATGCTGGTTGCCAA CTGGTTAATTAGGCAAGCCAA ATCAGTCAACTTCCACATTAA |
| 2802 | GOLGA3 | NM_005895 | CGGAAAACTTTAGAGAAGTTAA CTGGTTAATTAGGCAAGCCAA CTGGCCGATTACAGAACTGAA |
| 2813 | GP2 | NM_001502 | TTGATGGGACCTTGAAAGCAA CCAGAACTACACGAATCCTTA CACAGGCTTGGTATTAACAGA CAGGACCTTCAATAGTTCTGAT |
| 2817 | GPC1 | NM_002081 | TTGAGTCCTTGTATGAATAAA TACAACACAGACGATATTTAA CAGGGACACGCTCACGGCAA CCGCCAGATCTACGGAGCCAA |
| 2825 | GPR1 | NM_005279 | CCCGGACACTGTGGAGTTCAA CTCAGTTGCTGAGATACTCAA CGACTCCACTTTCATAGTTA AAGCCTTAAGTCTTAATATTA |
| 2838 | GPR15 | NM_005290 | CCCAGAAGAAACTTCAGTTTA TTGGCAAGAACACTATTTA TTGCCAAGAACACTATTTA TTCAGTTATTTGGATTATTA |
| 2846 | GPR23 | NM_005296 | TAGGTTGGGCCTATAAATATA TTGGGAAACTAGGTTCTATAA GGGCCTATAAATATAGAACAA AGGAAGTGAGTGATCAAACAA |
| 2848 | GPR25 | NM_005298 | GCGCATCATCTTCGCCATCGA CAGGGACGACAGTTCCGTGTT CTGCGCCAACCCGCTCATCTA CCGGAGGAACTCGCTGCGCAT |
| 2857 | GPR34 | NM_005300 | CTGAATAGATTCAGTCATTAT TGCGACTATTACCAAATAGAA TGCGACTATTACCAAATAGAA TCAGGCGAACCTGAACTTTAA |
| 2859 | GPR35 | NM_005301 | CAGGCAGAATCCCAGGAGAA CAGGACCATGAATGGCACCTA CGCCCTGTACATAACCAGCAA CCGGCACAATTTCAACTCCAT |
| 2868 | GRK4 | NM_182982 | AAGCCTTTGAGGAATGTACTA CCGGGTGTTTCAAAGACATCA CCGGGTGTTTCAAAGACATCA CAGGATGTTACTCACCAAGAA |
| 2870 | GRK6 | NM_002082 | CCGGAGGTGGTGAAGAATGAA AGGCATGTGGCGGCCACGTAA AGCCATGTGGCGGCCACGTAA AAGGATGTTCTGGACATTGAA |

114

Table4

| | | | Sequence |
|---|---|---|---|
| 2876 | GPX1 | Y00433 | CAGGAGAACGCCAAGAACGAA GAGGAACACCCTTGATCTTACA CCGGGACTACACCCAGATGAA AACACCTTGATCTTACAGAAA |
| 2886 | GRB7 | NM_005310 | CGGGAGTGGATCTGAAATAAA CCCAACAAGCTTCGAAATGGA CGCCAAGTACGAACTGTTCAA CACACTGTGTATCCCATGAA |
| 2888 | GRB14 | NM_004490 | CAGCGATTGATTATTCAGCAA CAGAAGTGACTTATTAAACTA |
| 2889 | RAPGEF1 | NM_005312 | GCAGGTCTTGTTGCCAATTTA CAGGGTTACATTGTAATATAT CCGGGTTTAGGACGTGGTCAA CAGGAAAGATTTGGTGTTGTA |
| 2890 | GRIA1 | NM_000827 | CAGGTGCGATTGAAGGTTTA CAGCATCCGAAAGATTGGTTA CTCCACGTGATTGAAATGAAA CACCATGAAGGTGGGAGGTAA |
| 2895 | GRID2 | NM_001510 | AAGCAATGGATCGGAGAACAA CACGATTACAAAATGGGATCAA AACGATGTGGACGTACAGGAA CTGGCAGAAATTCATTATATT |
| 2897 | GRIK1 | NM_000830 | CCAGCATTAACAGAAACCGAA TGGGCATATCACCTTTAATAA ACAGGTCTAATTCGTCTACAA ACGGATTTAGAATGTGGTAAA |
| 2900 | GRIK4 | NM_014619 | CCGGCAATTCCTTATCTCCAA CGCGTTCATATGGAGCGCAAA TGGGATGGTGTCAGCCTATTA CAGGGTGTTGAATTCCAACTA |
| 2902 | GRIN1 | NM_000832 | GACGCGTTCGGTCCTATTAAA CAAGGAGGAGTTCACAGTCAA CAGACTGAAGATTGTGACGAT CACGCTGGACTCGTTCATGCA |
| 2903 | GRIN2A | NM_000833 | TACGATAAACATTGTCGACAAA CACAGTGGCCATTGTAGTTTA ACCAGATAGGTGAGTCCTTAA AGGGTTGTATAGTATCTGTTA |
| 2909 | GRLF1 | NM_004491 | CACCACCGAAGAGGTGTTTAA CAGGATGTTCTGGGAGAGGAA CAAGGTTTCCATCGTGAGCAA CAGCCTAAGGAGGAACACTAA |
| 2912 | GRM2 | NM_000839 | CCGCTACAACATCTTCAACCTA CTGCACGCTTTATGCCTTCAA CTACAAGGACTTGTTGCTCAA CACCATCGAGCTGGCCTCCTA |
| 2913 | GRM3 | NM_000840 | CACGGCTCCATTCAACCCAAA TAGTCCTTGTTGTAACTAAT TACGGTGGCAATATTATGTAA GTCCTTGTTGTAACTAATTTA |
| 2917 | GRM7 | NM_000844 | AAGGGTTTGATGCCTACTTTA CACGCTTACAAATCTCCATGAA CCCAGATTATCGCATATTGA CCCAGATTAGTTATGCATCAA |
| 2931 | GSK3A | NM_019884 | CGGGTGTAAAATAGATTGTTAT CAATAAATTATTGGCATGAAA CCGGGTGTAAATAGATTGTTA CAGGGAACTAGTCGCCATCAA |
| 2932 | GSK3B | NM_002093 | CACGTTTGGAAAGAATATTAA CTGCATTTATCGTTAACCTAA CACTCAAGAACTGTCAAGTAA AACACTGGTCACGTTTGGAAA |
| 2944 | GSTM1 | X08020 | CCACCGTATATTTGAGCCCAA TCCAGAATTTGAGAAACTGAA CTGCTACAATCCAGAATTTGA |
| 2947 | GSTM3 | NM_000849 | AACGGAAGAAGTTACCCTTAA ATGGCTGGATGTGAAATTCAA |
| 2950 | GSTP1 | NM_000852 | CCTCATCTACACCAACTATGA TCCTTTCTCCAGGACCAATAA GCCGCCCTACACCGTGGTCTA CCAGATCTCCTTCGCTGACTA |
| 2954 | GSTZ1 | NM_001513 | TACCATCAGCTCCATCAACAA ACGGTGCCCATCAATCTCATA CTGAAATTGGCGTGAATTAA CGCGCTGAAATTTGGCGTGAA |
| 2956 | MSH6 | NM_000179 | CAGCAGGGCTATAATGTATGA CCGAAGTTGTAGAGCTTCTAA AAGCCTATTGTGTGCTTGTTA ATCGCCATTGTTCGAGATTTA |
| 2957 | GTF2A1 | NM_015859 | CAGCTTCTACTGCAAGTTCAA CAGGAACTCTTTGACACAGAA |
| 2959 | GTF2B | NM_001514 | TTGCCGTTGTACATAGCCTA TTGACGAATTGGCAATTCTA CCAGTGTGTGATTGATTACAA ACGTCAAATTCTTGAATACAA |
| 2965 | GTF2H1 | NM_005316 | AAGGCAAAGATAGATTTACAA CCCGAGCGATGCTATTGTA TCGGCTGAATACAGGGTCAAA ATGGCACAGGGTAGACCTTAA |
| 2974 | GUCY1B2 | NM_004129 | CAGCATCCGCAGATTTATCAA CAGTGTGCACGCAGTCAATAA CGCAGATTTATCAACAGTCAA CCGATGTTCAGAGGCATCCAA |
| 2975 | GTF3C1 | NM_001520 | CAGGAAAGAGGATGAACTTAA CAGGCCTATCTCAACTATAAA |
| 2983 | GUCY1B3 | NM_000857 | CAGTTTCTTGTCAGAATAATA CAAGACTTTCTTCTAGATATA AAGGGTCAAATGATCTACTTA ACCTAACATGGTGATCTGCAA |
| 2986 | GUCY2F | NM_001522 | CAGGCAGACAGAATTCGCATA ACCGTTGTTTGGAACCATCTA CTGGAGTTCGATCAAGTTTCA AAGGCAGTTGGTGAGAAACAA |
| 2997 | GYS1 | NM_002103 | CTGGGCGAGGAGCGTAACTAA CCGCTATGAGTTCTCCAACAA |
| 3024 | HIST1H1A | NM_005325 | ACCCAAGAAAGCGGCACCCAA CAGTTTTATACCACTTTATTT |
| 3029 | HAGH | NM_005326 | CTGGTCCGTGCGGGAAATTCA ATCGGACATTCTAATGCATAT TCAGTCTTGATTTAACCTTAA ACAAGTATTATTCCCATAAA |
| 3037 | HAS2 | NM_005328 | GTGGAAGATTGGTACAATCAA CAGCTCGATCTAAGTGCCTTA CCAGCTAGTAGTCTCATAAAA AACGCAATTGGTCTTGTCCAA |
| 3038 | HAS3 | NM_005329 | CACCTTCTCGTGCATCATGCA TACCAGTTCATCCACACGAA |
| 3053 | SERPIND1 | NM_000185 | CAACGCCAAGTTGCTTTCAA CCCAAGAGGGTACACAACTA TGGCATCATTTACGTAGTTTA TGGGCTAAGTGTTAGGCTTAA |
| 3055 | HCK | NM_002110 | CCGGGATAGCGAGACCACTAA CGGCAGGAGATACCGTGAAA CGGGAGCACACTCAGAGGCTTA AACGACGGGCTCTGCCAGAAA |
| 3059 | HCLS1 | NM_005335 | CAGCCAGTGATAGCTATGGAA AAGGAGAAGAAGTGGAGAAGCATA |
| 3062 | HCRTR2 | NM_001526 | CCCGTGATGTCCGGCACCAAA CCGGAAGTCCTTGACCACTCA CCGGTGCAACATCGCCTGTAA ACCGACTATGACGACGAGGAA |
| 3066 | HDAC2 | NM_001527 | GACCCCATAACTTGCTGTTAAA CTGGGTTGTTTCAATCTAACA TCCCAATGAGTTGCCATATAA ACGGTCAATAAGACCAGATAA |
| 3067 | HDC | NM_002112 | TGCCGACTCCTTCACCTTTAA CCACAATAGGATACAAACGAA CTGGGTCAAGGACAAGTACAA CAGCTTGTGACATGATATATA |
| 3068 | HDGF | NM_004494 | CCAGCTTATAGTCATATATAT GAGGGAGCAATTGAATTTCAA |
| 3074 | HEXB | NM_000521 | TTCGACGATATCATGGCTATA TGGATATTATTGCAACCATAA CCAGACATTATATCTGCCAGTTA CCGGGCACAATAGTTGAAGTA |
| 3092 | HIP1 | NM_005338 | CAGTTAGGGTGCTAGAGCTA CACAACAATGGGTATCCTTAA CAGGAACTTGCCACAAGCCAA ATGGCTTAGGATCGACAAGAA |
| 3097 | HIVEP2 | NM_006734 | CTGGGTTATCTTTGTGCTATA ACCGTAAAGCATTAAAGTAAA |

Table4

| | | | |
|---|---|---|---|
| 3098 | HK1 | NM_000188 | AACGGTGGAAATGCACAACAA CAGATCGAGAGTGACCGATTA CCGTGTCGTATGACCTAGTAA CACGATGTAGTCACCTTACTA |
| 3107 | HLA-C | NM_002117 | ACCGGGAGACACAGAAGTACA AAGCGTTGATGGATTAATTAA |
| 3118 | HLA-DQA2 | NM_020056 | ATGGTTATAGCAGAAATACAA CAGGGCTGACTGAGAGCATAA |
| 3119 | HLA-DQB1 | NM_002123 | CCGGAGGCCTTCGGGCAGCAA ATCCATCACAGGAGTCAGAAA |
| 3120 | HLA-DQB2 | NM_182549 | AAGGATTTCTTGGTCCAGTTT CAAGAGAAGCATGACAAACAA |
| 3122 | HLA-DRA | NM_019111 | CAACCTGGAAATCATGACAAA CTGAAGAAACTTCTGCTTTAA |
| 3135 | HLA-G | NM_002127 | ATAACTTACTTCTGTATTAAA ATGAGGTTTCTTTGACTTCAA |
| 3142 | HLX1 | NM_021958 | AAGGTTTGAGATTCAGAAGTA CGCCTTAAATTTGTAAATAAA |
| 3159 | HMGA1 | NM_002131 | CAGCCAGCCCTTGGCCTCCAA CTGGACAAGGCTAACATCCCA CAGCGAAGTGCCAACACCTAA CACCCACAACTCCAGGAAGGAA |
| 3162 | HMOX1 | NM_002133 | CACCAAGTTCAAGCAGCTCTA CGGGCCAGCAACAAAGTGCAA CAGGCAGAGGGTGATAGAAGA CAGGCAATGGCCTAAACTTCA |
| 3174 | HNF4G | NM_004133 | AAGCACTGACATAAACGTTAA CACCGGATGGATTCTGATGAA ACCGATAAGCAGCGAGTGTAA ACCCTGTAATTTATTCGTGTA |
| 3175 | ONECUT1 | NM_004498 | ATGGAAGAGATCAATACCAAA AAGGAAGAACCACAAACTAAA |
| 3176 | HNMT | NM_006895 | CGGGAAATATGTTGAATCTTT TGCAGGCTCAACATAACATAA TGGGACTTTATTCATATGATT CAACATAACATAAGCTAGAAA |
| 3187 | HNRPH1 | NM_005520 | AAGCAGTTGAATTATGTTAAA CAGGTATATTGAAATCTTTAA |
| 3198 | HOXA1 | NM_005522 | CAGGATAATATATTTGCAGAT TAGGATGTCTGTAATAAATAA CACACTGAGTTCTATCAGTTA CAGCTCCTTTATTATAATCAA |
| 3201 | HOXA4 | NM_002141 | AAGCCATGTGCTAAGAATAAA CTGGACCATAATAGACACCAA |
| 3225 | HOXC9 | NM_006897 | CACGACAATGAAGACCTCCTA CCGAAATAAATGACACATACA |
| 3236 | HOXD10 | NM_002148 | GTGCATCCTTATATACCTCAA CCCTATGAGTATATATATATA CTCCTTCACCACCAACATTAA CAGGGTAACTATTATTGCGCA |
| 3240 | HP | NM_005143 | TGGAGTGTACACCTTAAACAA AAGGTGTCTGTTAATGAGAGA CAAACAGAAGGTGTCTGTTAA CTGACCAAGACCAATGCATAA |
| 3242 | HPD | NM_002150 | AGGAGGTTTGACTAAGATCAA CCAGGAATATGTGGACTATAA ATCCATTGTGGTGGCCAACTA CTGGTGGACTACGACGAGAAA |
| 3248 | HPGD | NM_000860 | CAGCCGGTTTATTGTGCTTCA CAAGAGCTTCTTAGAGTAGTA CAAGACTATGATACAACTCCA CTGGCAGTGACTAATCAGTAA |
| 3249 | HPN | NM_002151 | GTGGATCTTCCAGGCCATAAA CAGCCAGGGAATCATTAACAA TGGGATGCTCTTTAAATAATA GGGATGCTCTTTAAATAATAA |
| 3250 | HPR | NM_020995 | CTGCATTGCTGAGTCAATCAA CTGACCAATACGATTGCATAA AAGCAAGATTTCAGCCTGGAA AAGGTGCTTGTTAATGAGAGA |
| 3251 | HPRT1 | NM_000194 | TCCTATTGACATCGCCAGTAA CACTATTGAGTGAAACATTGA AACATTGAACTCATATCTGTA ATGGGAGGCCATCACATTGTA |
| 3280 | HES1 | NM_005524 | CTGGTGCTGATAACAGCGGAA AAAGACGAAGAGCAAGAATAA CACGACACCGGATAAACCAAA ACCATGCACTATATTTGTATA |
| 3292 | HSD17B1 | NM_000413 | AACCATGGTCATGTGAGTTAT ATCCCAGAGCTTCAAAGTGTA CTGGACGAAACCAACACATAT CAGCTGTGGGTGGCTAATTAA |
| 3299 | HSF4 | NM_001538 | AAGGGCGAGAATGGACCCTGA CAGAGCCGTTTCGCCAAGGAA CCGGGTCATTGGCAAGCTGAT CCGACTATCCCTGCACATAAA |
| 3300 | DNAJB2 | NM_006736 | AAAGAGTTTGCTGAGAAGAAA TTCGCTCTGTTTCTACATCTA |
| 3308 | HSPA4 | NM_198431 | ATGGACATTGATTGATTCCAA CAGGTATAAAGGTGACATATA CCAGGTAATTTCTAATGCAAA CAGACTATTATAAAGCTTTAA |
| 3309 | HSPA5 | NM_005347 | AACTGTTACAATCAAGGTCTA TGGGATAAGGAAACACTTCTA CAAGCCCAATACAGCCATTAA TAGGGTGTGTGTTCACCTTCA |
| 3313 | HSPA9B | NM_004134 | AACCAAGATGGAAGAATTCAA TAGGATGAATTGATCATTATA |
| 3321 | IGSF3 | NM_001007237 | CAGGGTGATTTGGCCGTCAAA CAGGAGCGGCTCAGTCATGAA |
| 3324 | HSPCAL3 | XM_084514 | ACCAGCCTTCTTCCTAGTCAT CTGGAGATAAATCCTGATGAT |
| 3326 | HSPCB | NM_007355 | AAGCTCATGAAAGAAATCTTA CAAGAATGATAAGGCAGTTAA AAGGCTGAGGCCGACAAGAAT CCGCATCTATCGCATGATCAA |
| 3338 | DNAJC4 | NM_005528 | TAGGGAGTGGCAGGTGCTCAA CAGGAGGGTGGGTGAATTCCA |
| 3339 | HSPG2 | NM_005529 | CGGGAAGTGCAGGCCCGTCAA CACCCGAGCCATGGATTTCAA ACGGTGGGAAGTTGCGATACA ACGCCTGGACGTGGAGTTCAA |
| 3359 | HTR3A | NM_000869 | CACCGTATCCATTGACGTCAT ATCCATTGACGTCATTGTCTA TACGTGTATATTCGGCATCAA CAAGCTGCTATTCCACATTTA |
| 3361 | HTR5A | NM_024012 | TGGATTTACCAGTGAACCTAA TACGGCTTTCAACAAGAACTA CTCGGTCTTCGGAGTGCTTAT CCTGATCTATACGGCTTTCAA |
| 3362 | HTR6 | NM_000871 | CTGCAGCGCCTCCATCCTCAA CAGGGCCGCTGCCGCCGTCAA CGCCGTCAATTTCTTCAACAT CAGGAGGCTGCAAGTCTCCTA |
| 3363 | HTR7 | NM_000872 | TCCAGTCATCAGCTAATACAA ATGGCACTATGTAGTAATTAA CTGAAGTGACCGTATATATTA CGGCGCGATGATGGACGTTAA |
| 3371 | TNC | NM_002160 | AAGGACCTTCAGGTTTCTGAA CTGGGATGCCCTCAAACTCAA CCGAACGTACCAGGGACTTAA CCGGGCATCAGTCACCGGTTA |
| 3375 | IAPP | NM_000415 | ACAGCTAATAATGAAGTTATT TTAGAGGACAATGTAACTCTA ATGCAGGGTATTGCGAAACAA CTGGTACTAAGAGGCTATTTA |
| 3431 | SP110 | NM_004509 | TCCCAATCTGGTGACGATTTA AAGGAAAGGCTGATTACGGAA ATGGAACTTGGTTAACACCAA CCCAGACACTTCATAGGATTA |

Table4

| No. | Symbol | Accession | Sequences |
|---|---|---|---|
| 3441 | IFNA4 | NM_021068 | CTGGTTCAACATGGAAATGAT ACAGAGCAAAGTCTTCAGAAA ATCTATTTATTTAAATATTTA AACCTAGAGGCCGAAGTTCAA |
| 3442 | IFNA5 | NM_002169 | ACAGGAGGACTTTGATGATAA ATGCAGGAGGTTGGAGTGGAA CAGGAGGACTTTGATGATAAT ATCCTTCTCTTTATCAGCAAA |
| 3443 | IFNA6 | NM_021002 | CAGCAGCTGAATGACCTGGAA ATCCTTCTCTTCATCAGAAA CTAGACAAACTCTATACTGAA CATCAAGAAACTTGCAAGAAA |
| 3444 | IFNA7 | NM_021057 | TCCACTGAACTTTACCAGCAA CTGGTTCATCATGGAAATGAT CAGCAACTGAATGACCTGGAA TAGCCTAGTGATATTGCAAA |
| 3446 | IFNA10 | NM_002171 | GTCGCTTTACATTGTGGTTAA CTGGTTCAACATGGCAATGAT TCCACTGAACTTTACCAGCAA CAGCAACTGAATGACCTGGAA |
| 3455 | IFNAR2 | NM_000874 | AGGCGGAGAGCTGCAAAGTTTA CACAGTGATGAGCAAGCAGTA CAAGTTAATTCCAAACACGAA AAGCACCATAGTGACACTGAA |
| 3456 | IFNB1 | NM_002176 | CAGGTAGTAGGCGACACTGTT CAGAGTGGGAAATCCTAAGGAA CAAGGACAGGATGAACTTTGA CAGGTTACCTCCGAAACTGAA |
| 3460 | IFNGR2 | NM_005534 | ACCAAGTGCAGTTAAATACA TCGGGCATTTAAGCAACATAT AAGGCTTCTCTTAAACACTAA AGGCTTCCCAATGGATTTCAA |
| 3467 | IFNW1 | NM_002177 | AGGGCCTGACTTCCTACATAA AACCATGTGTTTCCTATTAAA TAGATTGTGTCATCCTCTTA AAGACTCTTATTTCGGCTTTA |
| 3468 | IFNWP2 | XM_378052 | CACAGGTAGGCAGGCCAACAA ACAGGAAAGATTGAGAAGTAA |
| 3479 | IGF1 | M27544 | CCGGAGCTGAATTACCTTTCA ATCAATAATGTTCTATAGAAA CCAGAAGGAGTACATTTGAA ACGGTAATACGTGAAAGCAAA |
| 3483 | IGFALS | NM_004970 | CACCAACGTGGCGGTCATGAA CCGACTCTTCCTCAAGGACAA CCGCCTGGAGGCATTGCCCAA CAGCTTGAGGTGCTCACGCTA |
| 3484 | IGFBP1 | NM_000596 | CATACATAACTTGATATAGAA AAGGAGGACGGTTAACTTGTA TACATGTGAGGGAAAGTTAA TACACTGATATGTTGTTAAA |
| 3491 | CYR61 | NM_001554 | TCCCTTCTACAGGCTGTTCAA AAGGTATTTCGAAACTGCCAA AGGGCACACCTAGACAAACAA AGGCAGCTATCTGCACTCTAA |
| 3508 | IGHMBP2 | NM_002180 | CAGGAGAACATCTCTCTGAAA CAGAGGGATATAAATTATTTA |
| 3512 | IGJ | NM_144646 | AAGAAGTGAAGTCAAGATGAA CAGAGCAATATCTGTGATGAA |
| 3547 | IGSF1 | NM_001555 | CTGGAGGAGCTCACTGGAGAA AAGCAAGTTCCTGCTGCTGAA |
| 3549 | IHH | XM_050846 | CTGGGTGTATTACGAGTCAAA CGGCTTTGACTGGGTGTATTA CCGCCTGAACTCGCTGGCTAT CTGGGTGTATTACGAGTCAAA |
| 3550 | IK | NM_006083 | AACCCGTAACAAGAAGCTTAA CAGCGAGTATATGAACAACAA GAGGAGGTGGATTATAGCAAA CAGGCGCTTCAAGGAAACCAA |
| 3551 | IKBKB | NM_001556 | TAGGGAGCTATTCATGTTTCA CAGGACACTGTGAGGGAACAA CAGACCGACATTGTGGACTTA CACACTGGAGGTCCTCCATTA |
| 3552 | IL1A | NM_000575 | TCGAGTTGAATGAACATAGAA CTGAGTGATTTATGCCTTAA AAGGCAAAGCACGAAATGTTA CACGCCTACTTAAGACAATTA |
| 3557 | IL1RN | NM_000577 | AAGGAATAAAATTGCTCCTTGA CAGCGAATGAATGCTGTATAT CTGGATACTTGCAAGGACCAA CCAAATGTCAATTTAGAAGAA |
| 3559 | IL2RA | NM_000417 | CCCGTTCACGTTGCCTAATAA CTCCACCCTATATGTAGTATA CAGAGAATCATACTTAGCAAA CCACCCTATATGTAGTATAGA |
| 3560 | IL2RB | NM_000878 | CCAGTTCACGTGCTTCTACAA ATGGCTGGAAGAGGTCCTGAA TGGCTTCATCATCTTAGTGTA CCCGATGCCTTGGAGATAGA |
| 3561 | IL2RG | NM_000206 | CACGACAATTCTGACGCCCAA CTGAACAATCAGTGGATTATA AAGAACTCGGATAATGATAAA CTAGAGGATCTTGTTACTGAA |
| 3566 | IL4R | NM_000418 | CCCGGCAGATTTCAGAAATCTA CAGGCAGATGTTCCTAATAAA CAGAATCTATAACGTGACCTA CACATACATGAGGGTCTCTTA |
| 3577 | IL8RA | NM_000634 | CCGGTGCTTCAGTTAGAATCAA TACCACCATGCAAACAAGATA TCCGCCAGGCTTACCATCCAA CCGTTGAACGTCACATCTTT |
| 3578 | IL9 | NM_000590 | GAGGCAAGATATGAAGATGAA AAGGACAAGGGCCAAGGTCTA ATCCTATTTATTAAATTTAAA ATCAGTTGAAGTACTAAAGAA |
| 3581 | IL9R | NM_002186 | CCAGAGATAGTTGGGTGACAA CAGAGATAGTTGGGTGACAAA CAGCTATGAGCTGGCCTTCAA |
| 3596 | IL13 | NM_002188 | CAGCATGGTATGGAGCATCAA AACAAGTTGTTTCATTGACTA TTCATTGACTATCAAACTGAA |
| 3597 | IL13RA1 | NM_001560 | CCAAATAATGGTCAAGGATAA CTCAGTGATGAGGAGATAATTTA AACCATGTTTGTGGCTATTAA |
| 3608 | ILF2 | NM_004515 | CAGACAGCCTTTGGCCCTAAA CTCCATAGAGAAGTGTCATTCCA TTGAAATGTTGTGAAAATGTTA CAGGCCCTTTGTACCACACATAT |
| 3609 | ILF3 | NM_153464 | CAAGGTCGCACGTGGCCGTTAA CTGGTTTAGCTAGGAGTGAA CAAGAGTTGAAGTATTGATAA AACGATTCACCACATCTGAA |
| 3612 | IMPA1 | NM_005536 | CCAGATTTGGTGACTCATCAA TAGAAAGTTAACTGTTTGGAA CCGGAAGAGACGAGTGCGGTA ACGAAGAGTAATTGCTGCAAA |
| 3614 | IMPDH1 | NM_183243 | CCCAGGATTCATAGACTTCAT CAGCAGCAGCCAGAAACGATA CCGCACCGACCTGAAGAAGAA ATGGCCTGCACTCTTACGAAA |
| 3617 | IMPG1 | NM_001563 | CTCCAAGTTCAAGGAACCAAA CCGTAGAAATATGAAGAATTTA AAGGGCGAAGTTGTACTATAT TCGGATCTTTCTGGATCGCAT |
| 3623 | INHA | NM_002191 | CGGATGGAGGTTACTCTTTCA AAGGCAGAGTTGGGAAATAGA CAAGTATGAGACAGTGCCCAA CAGCACTGTGCTTGTATCTAA |
| 3624 | INHBA | NM_002192 | CAGGAAGACGCTGCACTTCGA AAGGAAGATGTGGAAATCTTA ACCCATGTCCATGTTGTACTA CTGCAGTAGTGTGGACTAGAA |
| 3625 | INHBB | NM_002193 | CAGCCTGTGGCTTTACCTGAA CCACGTGAACTATGCAATTTA CAGTGGAACATGGTGGAGAA CACTGACTCTCAAGTGCATTCAA |
| 3627 | CXCL10 | NM_001565 | TTAGTTTGCAGTTACACTAAA CAGGAAGGTAGAAAATATCTGA TGCCATCAGCATTAGTAATCAA CTGACTCTCAAGTGCCACATTGAA |
| 3635 | INPP5D | NM_005541 | TCGGAATTGCGTTTACACTTA CCCGGGACTGTTGACAGCCAA TCCCATCAACATGGTGTCCAA CAGTGCCTATGCCACATTGAA |
| 3641 | INSL4 | NM_002195 | AGCAGCAAGTCTCTAAAGAAA CTGAGCCAACTCCTTAGAGAA TAGGTACGACATCAGAATTCA ATCCATTCGTGTTGTGAAGTAA |
| 3643 | INSR | NM_000208 | TCGAACGATGTTGGACTCATA TAGGAAGACGTTTGAGGATTA CAACGGAGTCTGATCATCAA ACCGCTTTACGCTTCTTCAA |

Table4

| ID | Gene | Accession | Sequences |
|---|---|---|---|
| 3646 | EIF3S6 | NM_001568 | CAGGAGTCTTACACATATAAA AAGGAATTATTACAAGGTAAA |
| 3656 | IRAK2 | NM_001570 | GAGAATATGTGTTGTTATTTA CCGGTTTACCTGAAGGACTTA CCAGATCATCCTGAACTGGAA CAGCAACGTCAAGAGCTCTAA |
| 3660 | IRF2 | NM_002199 | CCCTATCAGAACGGCCTTCTA ACGGTGAACATCATAGTTGTA CGGTCCTGACTTCAACTATAA CACCTTATCTTAAAGCACTTA |
| 3663 | IRF5 | NM_002200 | CGGAGATAACACCATCTTCAA CCGCATGGTGGAGCAATTCAA |
| 3667 | IRS1 | NM_005544 | CCGCCTTTGCCTGACCAGCAA CCGGTATCGTTTCGCATGGAA ACAATAGGCCATGTTAATTAA CCGGGTGAACCTCAGTCCTAA |
| 3670 | ISL1 | NM_002202 | TACGGTGGTCTTTGAATTATA CCGGCTTAAATTGGACTCCTA |
| 3672 | ITGA1 | NM_181501 | CCCTGAAACACAACCTTTAA CTGCATGTCGCCACCACTGAA CCCTAGATTCACTAAGACAAA ACTGAGGACATTGGAAATGAA |
| 3673 | ITGA2 | NM_002203 | TCGGAGCAATTCAATATGCAA TCGCTAGTATTCCAACAGAAA CCGGTTGATGGGACAGAAGTA CCCGAGCACATCATTTATATA |
| 3675 | ITGA3 | NM_002204 | CAGCTACATGATTCAGCGCAA CTGGATTGACTTTGCTGTCAA CTCGCTTAGCATGGTAAATCA CACCATCAACATGGAGAACAA |
| 3679 | ITGA7 | NM_002206 | GACCAGGAGCTGATATGCAA CACAGTGAAGTCCTCCATAAA CCCTTTGATGGTGATGGGAAA CCCAGTGATGGTATACTTGGA |
| 3680 | ITGA9 | NM_002207 | ACAGGTCACTGTCTACATCAA CACATGTCAATGTGTGCTAAA CCGAAGGTACAAAGAAATTAT ACCGAAGATCAGGCACCTTAA |
| 3683 | ITGAL | NM_002209 | AACGTGTGACCAGAACACCTA CCCTGTTTAATGATTGACGTA CTCGGTTGAATTGCACGCCAA CAGGGCGTGGGACATCTAGTA |
| 3684 | ITGAM | NM_000632 | CGCAATGACCTTCCAAGAGAA CTCGTTTGACTGGTACATCAA TCGGGTCATGCAGCATCAATA TCAGACATCGGTTCATATTAA |
| 3685 | ITGAV | NM_002210 | AGCAACTTTATTATAGATTTA CACATACATGATACCATCCAA TAGCATGATGTTACAGGAATA ATGTAGTAGTTTGATGAATAA |
| 3688 | ITGB1 | NM_002211 | CTGCGAGTGTGATAATTTCAA ACAGATGAAGTTAACAGTGAA TACGTATTCAGTGAATGGGAA AAGAGGGATAATACAAATGAA |
| 3690 | ITGB3 | NM_000212 | CAAGCTGAACCTAATAGCCAT CCGCTTCAATGAGGAAGTGAA CTCTCCTGATGTAGCACTTAA CACGTGTGGCCTGTTCTTCTA |
| 3692 | ITGB4BP | NM_002212 | CCGAGCTCGTTCGAGAACAA AAGCTACTGTGTCTTCAGCAA CAGGGAGACAGAAGAAATTCT CTGGATCTATCATTACTGCAA |
| 3693 | ITGB5 | NM_002213 | CTCGGTGTGATCGTAGGGCAA CGCTATGAAATGGCTTCAAA ATGGGTGGACACCATCGTGAA CAAGATTGTTTGGGATTGGAA |
| 3700 | ITIH4 | NM_002218 | CACATCATGGTAGTCACCAA ATGGATCGAAGTGAGCCTTCAA |
| 3704 | ITPA | NM_033453 | CAGGAGGCAGTTCGCCAGGTA GAGGAGGTCGTTCAGATTCTA CTGGATGAGACTTGTTTCCAA CTGGAGAAGTTAAAGCCTGAA |
| 3706 | ITPKA | NM_002224 | CTCCTGCAGCACCGACTTCAA CCCAGCGTTCCCAGAGCCAAA CCGGTCTAACGTCTCCACACCA CACCAGCGGGCTGATCTGAA |
| 3710 | ITPR3 | NM_002226 | CAGGTGTTCCTGCGAACTACA CCGCAGCATGCGCCTGATCTA AAGCCGGTGAAGCGCATTCAA TCAGTTTACCTTAATGCCTTA |
| 3714 | JAG2 | NM_002228 | CCAGAGGTGTGCACTGTTTA CTACGTTTCTTTAACCTTGTA CACTCTGTATATTTGATTGAA CTGCGGTCGTCACCAAATCGTA |
| 3725 | JUN | NM_000216 | TTCGTTAACATTGACCAAGAA CGCGCGCGAGTCGACAAGTAA AAGCGTAAATCTAATCAATAA |
| 3730 | KAL1 | NM_002234 | ATGCATGTCTATATAATTATA AACCAGGGAACCCATTTCTCTA CAGAGGGATAAACCCAAACAAA AGGAATACTTCTGATAATTTA |
| 3741 | KCNA5 | NM_002235 | ACCAGGGAACCCATTTCTCTA CAGAGGGATAAACCCAAACAAA TACAATGACCACGTAGGTTA CTGGTGTTCGGTCTCACATTTA CAGGACCAAATACCTGGACTA CTCGATCTTGCCATAACTTTA |
| 3742 | KCNA6 | NM_005549 | CTAGTTATTAGTTTATGCAAA CACCGGGAGACTGAGAATGAA CCGGAGGATAGGGAGCTAAA CAGCACAGACTCTCTTAATAA |
| 3744 | KCNA10 | NM_004975 | CCGGAGCATTGAGATGATGGA TCGGTATTCATAGTACCTGA AACCACTTGTCTCCTAACAAA CACGCTTTCTTTACATCACTA |
| 3745 | KCNB1 | NM_000219 | GCCCAGGATGATCCTGTCTAA AAGAGAAGGACAAGGCCTATG AACCATCTGGCCATAGAACAA AACTGGGATTCTTCGGCTTCT |
| 3753 | KCNE1 | NM_002238 | ATCAGCGATGTGAAACGTGAA CTGAGATACTCAGGATATTAA TACAGCCATCTTGGTCCCTTA TCGGCGGTCCAATGATACTAA |
| 3756 | KCNH1 | NM_000220 | CAGGGTGTTGACAGAAAGTAT CTCCCATAGTATCCAAGACAA CAGTCTATACTTGGAGTTATA TACGTTTAGACAGAGGCTAAA |
| 3758 | KCNJ1 | NM_000891 | CAGGAGCCGCTTTGTGAAGAA CGAGATGGTGATGTACTCTTA AGGGTCAATGAGAGCCATAAA CAACTCTCTTATGGTATTATA |
| 3759 | KCNJ2 | NM_002239 | ACCAGCCATAACTAACAGCAA AAGGCTACTGGCATTAATTAT ATGGACTAGATGATATTACTA CTGTGTGAAGTTACACAATTA |
| 3760 | KCNJ3 | NM_004982 | CAGGACGATGTTGGCCAGAAA AAGGTGCAATTTATGACTCCA GAGGTGGTTCCTATTCACCAA CCAGATGCCTGTGTTACTAAT |
| 3764 | KCNJ8 | NM_004983 | TGGGAAGATGTTGGTAGCAGATAA TCGGTATTCAAACTGACCAA GTGAATGACCTTAGTCCGCAA CAGGCCCGACAGAGAGATGAA |
| 3765 | KCNJ9 | NM_002242 | AACCTACAAACTAGACTTGTA ATCGCCTTACTTGCCCATACAA CAAACTAGACTTGTAGATTAA CACATGGATGGCAAACCTAAT |
| 3769 | KCNJ13 | NM_013348 | CGCGGTCGCTTCGTCAAGAAA CTGGATTCACCTAAGATTCAA CAGGGTGAGAAGCCAATGGTA AAGACTGAAGTGACAGATTAA |
| 3770 | KCNJ14 | NM_018658 | CAGACTGGCACTTTATCTCAA CCGGTAACTATTGTCCATGAA CAGCAGCTATCATATTATCAA AAGCTTAATATTAGAGACCCA |
| 3773 | KCNJ16 | NM_014217 | CACGACCATTAATGTTATGAA TCGCATCACTCAACAATCAT AGGGATTATACCGTTAGGAAA AGCCTAGATATGGACCATTTA |
| 3776 | KCNK2 | NM_002247 | ACCGAGAGAGCCGTATATTAA ACGGGATGTTACGTCAACCAT AACCATCTTAAGATCCAAGAA TAGGCAGAGCAAGTTTATTTA |
| 3778 | KCNMA1 | NM_002248 | CAGGGAGACGTGGCTCATCTA ACCGTCACCGTACGACCTTGTA CACGCTTACCGACCTAGCCAA CCGGCCTAGCCTAGAATCCTA |
| 3780 | KCNN1 | NM_002248 | CAGGGAGGACGCTGGTGTGGTTAA CCCGCCCTCCTGAGCTATGAA AACGGTCACCGTGCCCATGTA CCCATTGACACAAACCCTCAA |
| 3785 | KCNQ2 | NM_004518 | CAGCCAGGGCGTGGTGGTTAA CCCGCCCTCCTGAGCTATGAA AACGGTCACCGTGCCCATGTA CCCATTGACACAAACCCTCAA |

Table4

| # | Gene | Accession | Sequences |
|---|------|-----------|-----------|
| 3788 | KCNS2 | NM_020697 | CCCATAAAGTTAAATCCCTTA CACCAAGTGAACTCAGTTAA CACCTTATGGTTATGGTGTAA AGGGACTATTATGCCCATAAA |
| 3792 | KEL | NM_000420 | CTGGTAAATGGACTTCCTTAA CCCGACAAGAATACAACGATA ACCCGAAGTGCTGCCATGAAA CAGCACAGAAATATCGACCAA |
| 3796 | KIF2 | NM_004520 | AAGAACCAAGATTGTTCTAAA CAGCAAGCAAATCAACCCGAA CCAAACCAGATTGATGACTTA ACCATATAACATGTGATTATA |
| 3806 | KIR2DS1 | NM_014512 | ATCATAGGTCTATATGAGAAA AACAGCGAGGATTCTGATGAA |
| 3808 | KIR2DS3 | NM_012313 | CACGTTCTGATTGGGACCTCA CACCATGTCGCTCATGGTCAT |
| 3809 | KIR2DS4 | NM_012314 | AGCGAGGATTCTGATGAACAA CAGCTCCATCTATCCAGGGAA |
| 3810 | KIR2DS5 | NM_014513 | AACAGGAGGATTCTGATGAA TACCTGCGGCTGGAATCTGAA |
| 3814 | KISS1 | NM_002256 | CAGGATGAGGCAGCCCTCAA AAGAAATGTTGCGTAACTCAA CACCCTCTGACATTCACCCA |
| 3817 | KLK2 | NM_005551 | CAGCATATAATTCTCCCTGCA GACGGTATGGAGTACTTGAA CTGAGGTTCCCTAGAGTTCAA |
| 3818 | KLKB1 | NM_000892 | CAGCAAGTTCAATCAATGACA AACGTGGAATCTGGATTCTCA CGCTATAAAGGTGCTGAGTAA ATGGTCTGTGCTGGCTATAAA |
| 3827 | KNG1 | NM_000893 | CCGCATAAACTGAAGCCACTAA CTCGGCTGTGTGCATCCTATA TCCCTCTTCATGCTTAATGAA ACCGGTGAATGTACAGATAAT |
| 3832 | KIF11 | NM_004523 | CTCGGGAAGCTGGAAATATAA GCCGATAAGATAGAAGATCAA CTAGATGGCTTTCTCAGTATA ACGGAGGAGATAGAAACGTTTA |
| 3833 | KIFC1 | XM_371813 | TCGGGAAACACAGGCCATTAA TGGGACTTAAAGGGTCAGTTA CAGGGCTATCAAATAAAGAAT TCGGGAAACACAGGCCATTAA |
| 3835 | KIF22 | NM_007317 | CCACAGTATCCTTATTGCCAA CAGGACATCTATGCAGGTTCA ACAGATGGAGTCCTTCCTGAA ATCCACATCCTGAAGAATAAA |
| 3849 | KRT2A | NM_000423 | AAGCAGCACCATTTCATCAAA CTGGTGGAAGCCGGAGATCAA |
| 3856 | KRT8 | NM_002273 | CCAGGAGCTGATGAACGTCAA CAGGGTGACCCAGAAGTCCTA CAGGGCCGTGGTTGTGAAGAA CAGCATCATTGCTGAGGTCAA |
| 3860 | KRT13 | NM_002274 | CGCCAAGAGTGCGAGAGCTGAA CTGCCTTTCTGCAAACATAAA |
| 3861 | KRT14 | NM_000526 | CTGGCAATCAATACAGCTTCA CAAGTATGAGACAGAGTTGAA |
| 3884 | KRTHA3B | NM_002279 | ATGGTCTGGAAGGAGAACAAA CCGAGGCATCACCACAAATCA |
| 3887 | KRTHB1 | NM_002281 | CAATAAATTAATGTAGCCAAA CAGCAGCTTGCCAAATGTTTA |
| 3891 | KRTHB5 | NM_002283 | CAGCCCTTCACTTAACTCCTA CAGGTCTTGCTATTCAGTGAA |
| 3912 | LAMB1 | NM_002291 | CCGAAACAGCAACGCCTGTAA TACCAAGATCCGTGTTACTTTA AGGGAATTTGTGACAGCTATA TCCGTTCACTCCTAAAGGATA |
| 3913 | LAMB2 | NM_002292 | CGGGTGAACCTGACCTCGTCTA CAGGCCCACCTTTGAACGCTA CAGGTACTTGATATTTCCTAA GCGAGATAGGCTTGCACTTAA |
| 3914 | LAMB3 | NM_000228 | ACAGTTACACTTGACAGACAA ACCGGTTGGCTGAGTTCCA TTGGCATGCCATTGAAACTAA AAGAGGGATTTGAGAGAATAA |
| 3916 | LAMP1 | NM_005561 | CACGTAATGCATTGCATCCTA CAGGTCTTGCTATTCAGTGAA |
| 3920 | LAMP2 | NM_002294 | CAGTACGCTATGAAAACTACAA CTGGAACCTATTCAGTTAATA TAAGGACTATAGTGATTTAAA |
| 3930 | LBR | NM_002296 | AAGACTCAAGTATAGATTAAA TAGCAATTAAATTCTAGTGAA CAGAAGACAGTCTTAATATAA TAAGGTAGCTTTAGCAATTAA |
| 3931 | LCAT | NM_000229 | CTGGCTGGATCTCAACATGTT AACCAGATGTGGTGAACTGGA CAGCATCAAGCTGAAAGAGGA AACAGCATCTCAACATGGTCT |
| 3932 | LCK | NM_005356 | CTACGGGACATTCACCATCAA CGCCATTACACCAATGCTTCA AGGCATCAAGTCAGCCATCAA AAGGGCCAGGACTTTATCTAA |
| 3939 | LDHA | NM_005566 | AAGGATCAGCTGATTTATAAT CAGATTTGGCAGAGAGTATAA CAGCTGATTTATAATCTTCTA AGTCCAATATGCAACTCTA |
| 3956 | LGALS1 | NM_002305 | CGCCAACACCATCGTGTGCAA CGCCAGCAACCTGAATCTCAA CACCATCGTGTGCAACATCAAA CACCATGCGTGTGCAACAGCAA |
| 3957 | LGALS2 | NM_006498 | CAAGCTGAACCTGCATTTCAA CACTGATGGCTTTGTAATTAA |
| 3959 | LGALS3BP | NM_005567 | CACGTCATCCTGACTGCCAA CTGAGGCTCAGCTTCAAGAAA CAGCTTCCTCCTTCCAGGACAA CAGCTTCAAGAAATTACTGGA |
| 3965 | LGALS9 | NM_002308 | CTCCTTTCCCAGTGTCCTTAA CCCACCATCAACAGACTGAA |
| 3977 | LIFR | NM_002310 | CAAGACCATTATCAACTCCTA TTGGAAGCCTTTACCCATTAA TGGGTCGATCACAATCAACAA TGCAGATTCGATATTAGTAAA |
| 3980 | LIG3 | NM_002311 | CAGCTGGTTGAAGGTCAACAA CTGGAAGAGAGCTGGAAGATAA CACCCGAATCCGAGATGATAA CTGGAAGATAATGAGAAGGAA |
| 3984 | LIMK1 | NM_002314 | CAGAGGTTTCTGGGAGACCTA GAGCATCTAGGAAGTATTAAA CTGCCTGGTCCGCGAGAACAA CACCGACTTTGGCCTCAA |
| 3988 | LIPA | NM_000235 | TACCTGGTCTCGGAAACATAA CACGTTTGCACTCATGTCATA CTGAAGCGAGGTATAAA CCCGCTACTGTCGTTATTGAT |
| 3990 | LIPC | NM_000236 | AAGTTTATCATTACCAGTTAA CTGAAGACGATCAGAGTCAAA AAAGGAATTGCTAGTAATAAA CAGGAGAAACCCAGCAAAGAA |
| 3992 | FADS1 | NM_013402 | TCGGAATGTTACAATGGTAAA CAGGAAGAGATTAACACTAA |
| 4000 | LMNA | NM_005572 | AAGCCAGAATGGAGATGAT CCCACCAAAGTTCACCCTGAA CCGGCTGCGCAACAAGTCCAA CCAGGAGCTTCTGGACATCAA |
| 4010 | LMX1B | NM_002316 | CCGCTTCCTGAGCTGAGTCAA CAGCAGATCGTGGCCATGGAA |
| 4012 | LNPEP | NM_005575 | CAGGGCACAATTATACGTTGA CCGCTTTATCAAATATGCCTA TCCAATGGAACTCAAAGCCTA CACGTAAATGCCAACCATCTA |

Table4

| | | | |
|---|---|---|---|
| 4015 | LOX | NM_002317 | AAGCTGGCTACTCGACATCTA CTGCACAATTTCACCGTATTA AACTACTACGATACTTATGAA CTGGAACTTTAGTGAAACATA |
| 4016 | LOXL1 | NM_005576 | CAAGGTGCACGTGAACCCAAA TCGCTACGTTTCTGCAACAAA |
| 4017 | LOXL2 | NM_002318 | CTCGAGATTCCGGAAAGCATA CCGGAGTTGCCTGCTCAGAAA CAGGCAATGAGAAGTCCATTA CCAGTCTATTATAGTCACATA |
| 4033 | LRMP | NM_006152 | CTGGGAAAGTATAGCATGAAA CAGATTATCTTTGGGATTTAA |
| 4034 | LRCH4 | NM_002319 | CCGCCTGGATTTCTCCTGTAA CAGCCGTTGATAGTGGCAGCAA TTGGCTCCATTCAGAGACCAA GCTGGTTGTGTTGACAATAAA |
| 4038 | LRP4 | XM_035037 | CAGCGTGTTGACAAATACTCA CCGCAAAGTACTGATCAACAA CCGCGCGGTGCTCATCAACAA CAGGAAATCATTCGCAACAAA |
| 4041 | LRP5 | NM_002335 | CTGGACGGACTCAGAGACCAA CCCGTTCGGTCTGACGCAGTA CTGGATGGGCAAGAACCTCTA CAGGGCTGGGAGAACTTTGTA |
| 4043 | LRPAP1 | NM_002337 | CTGGAAGAAACTAAAGCTTGA TACCATGCACAGAGTCCTTAA CAAGTATGGTCTGGACGGAAA CGGGAGTTCCTGCATCACAAA |
| 4046 | LSP1 | NM_002339 | CCGCTCCATAGAGAAGAGTAA CAAGGAGAAACTGCAGGTCAA |
| 4049 | LTA | NM_000595 | CAGGTGGTCTTCTCTGGGAAA CCGGAGCTTTCAAAGAAGGAA CAAGGCTGACCAAGAGAGAAA ACCCTCGATGAAGCCCAATAA |
| 4052 | LTBP1 | NM_000627 | CCCAATATATCTACCATTGTA TTGATTAAATTTGGCATTTAA CTGGGTATGAATGCTACTGTA TACCGGGTTATAAGCGGGTTA |
| 4054 | LTBP3 | NM_021070 | CCGCTACTGCGTGGATGTGAA CCGGGACAGATCTCAGCAGAA |
| 4055 | LTBR | NM_002342 | TACATCTACAATGGACCAGTA CCGGCGGGTCTATGACTATCA AAAGGGAGTCATTAACAACTA CCGCCACACGGTCACCTGCAA |
| 4057 | LTF | NM_002343 | ACCCTTGATGGTGGTTTCATA CAGCCACGAACTCACTATTAT CAGAGAGAGCACAGTGTTTGA AAGCAGTGACCCTGATCCTAA |
| 4058 | LTK | NM_002344 | CAGGGATATTGCCGCCCGGAA CCGGGCCTTGCTCCCAGTCAA CCTGCAGATGCTTCTAATAAA ACAGATCTTTGGAGTGCCTAA |
| 4067 | LYN | NM_002350 | CTCCGAGTCACTAATGTGCAA CCCAATAATTGCAGAACTAAA CGGGAAATATGGGATGTATAA CCCGGACGACTTGTCTTTCAA |
| 4070 | TACSTD2 | NM_002353 | TCGGCGCAAAGGAGACGTTTA TTGCGACATTGTGAAGGCTTA CCCGGGATCGTTTGCAAGTAA CTCGAAATAAGTGGTACTATT |
| 4071 | TM4SF1 | NM_014220 | TTCACTTGTATTCATTTGTAA TCACAGGATGTCAGTGTTTAA |
| 4084 | MAD | NM_002357 | CAGTAGCAGATCAACTCACAA TACGTTGAGTTTATTAACAAA CCGAATCAAGTCGACACACTA AGCCAAATTGCACATAAAGAA |
| 4085 | MAD2L1 | NM_002358 | AAGAGTCGGGACCACAGTTTA CTGAAAGTAACTCATAATCTA TCCGCCTTCGTTCATTTACTA ATGGATATTTGTACTGTTTAA |
| 4086 | SMAD1 | NM_005900 | TTGGAAGTTAAGAATTGATTA CACACACACCTTGGTAACATA AACCGGAATTCCACTATTGAA CAGGACTTTGTGTACAGTTAA |
| 4088 | SMAD3 | NM_005902 | CCGCTGTTCCAGTGTGTCTTA ATCAAGGGATTTCCTATGGAA AAGAGATTCGAATGACGGTAA AAGGAGCACCTTGACAGACTT |
| 4093 | SMAD9 | NM_005905 | CAGCCTCAAGGTCTTCAACAA TTGGACTTCTTTCTAATGTAA |
| 4099 | MAG | NM_080600 | CACTCGCTGTACAGAATGATA CCCGGTGATTGTGGAGATGAA CTCGCTGTACAGAATGATATT ATCGCGCAATGTGACCGTGAA |
| 4101 | MAGEA2 | NM_005361 | TCGACAGATGCAGTGGTTCTA CAGGGAGTTGATGACCTTGTT |
| 4109 | MAGEA10 | NM_021048 | CCCAGAAGTGAGATAGATGAA AAGAAGATCTTCAATCCCAAA |
| 4118 | MAL | NM_002371 | TCAGATGGAAGTCTTCATAAA ACCCTGTGAAATACTTTATAA |
| 4123 | MAN2C1 | NM_006715 | CACCCAGAAATTGAGCTGGAA CTCGCTCTTGCGGGCGCCTAA CAGCCTGTGTTCGTAGTGCAA CAGGATGCTGGCGTTATCCAA |
| 4125 | MAN2B1 | NM_000528 | GCGCCTTGATTATCAAGATAA CGCCAAGGAGCTGGTCGATTA TCGACCCACCTGGAAACTGAA GCGGAGTATCGGAGCCACTAA |
| 4128 | MAOA | NM_000240 | TTGCATATTTGGATTACAATA ACCCTGGTGTGAGGACATAAA ACGCTGAACCATGAACATTAT CAGATGTGCCTAGACACGAAA |
| 4137 | MAPT | NM_016835 | CGGGAAGGTGCAGATAATTAA TTAGGCAACATCCATCATAAA CAGTGTGCAAATAGTCTACAA CCGCCAGGAGTTCGAAGTGAT |
| 4141 | MARS | NM_004990 | CAGCGAGGGATTCACGGCGAA TAGGAGCTGAGGATAACTATA CTGCATAACCCGAGACCTCAA CTCGACATGGCAACCAATATA |
| 4142 | MAS1 | NM_002377 | CCCATATGCATGAGATACTAA GAGATACTAATTAATGATGAA ACAGTTGAGACTGTCGTCTAA AAGTATCTCCTAAATGTGATA |
| 4144 | MAT2A | NM_005911 | CAGCAGGATCCTGATGCCAAA CAACAGTTTAATGAAGATCTA |
| 4148 | MATN3 | NM_002381 | CACCACTGTGAGTGTAGCCAA CACCTTGAATGCCGACAAGAA AAGGTCAGCTCGTATCTTCAA TTGGTAGGAAATAGAATGAAA |
| 4150 | MAZ | NM_002383 | CCGCGACGTCTACCACCTGAA CGCGTCTGCCTTGGAGAAGAA |
| 4152 | MBD1 | NM_002384 | AAGAGAGTTCCGGGAAACATA CCGGGAACAGAGAATGTTTAA CCTGAGGATAACTCAATATAA AAGGATGATTCTGCCTCCAAA |
| 4153 | MBL2 | NM_000242 | TAGGGTTCCCTTATATTAATA TACGATGTTTAGTAACCTCAA CAAGTCAAGATTTCCATATAA CACATTGAGTCTATCAATCAA |
| 4155 | MBP | NM_002385 | CACGGCCGGACCCAAGATGAA CAGTTAAATGAGAACATGAAA |
| 4157 | MC1R | NM_002386 | CCAGGAAAGTCTGGTAATAAA CAGGAAAGTCTGGTAATAAAT CACGCTCTTCATCGCCTACTA CACCAGGGCTTTGGCCTTAAA |
| 4161 | MC5R | NM_005913 | CACCATCTACCTACTCAACAA TCGCTTCATGTCTCACTTCAA CTGGGAGACCATCACCATCTA CGGCATTGTCTTCATCCTGTA |
| 4162 | MCAM | NM_006500 | CAGCTGGTTAAAGAAGACAAA TACACACATTATGGCTGTAAA CCGAACTTGTAGTTGAAGTTA AAGACCGAACTTGTAGTTGAA |
| 4168 | MCF2 | NM_005369 | TAGAATGTCATCGGCAAATAA CACGTACTAAATGAACTGATA TGGCTATATTGGAAACTTAAA AGGGACTATAGCTCAAGTAAA |

Table4

| | | | Sequences | | | |
|---|---|---|---|---|---|---|
| 4172 | MCM3 | NM_002388 | CAGGGAATTTATCAGAGCAAA | CGGCAGGTATGACCAGTATAA | ATCCAGGTTGAAGGCATTCAA | CACGATTTGACTTGCTCTTCA |
| 4193 | MDM2 | NM_002392 | GCCCAGTATGTAGACAACCAA | CCGGATCTTGATGCTGGTGTA | CAGGCAAATGTGCAATACCAA | AACCTGAAATTTATTCACATA |
| 4201 | MEA | NM_014623 | CACAGTGAGAGCTGGCCTTATA | TGGGAAGATGTGGTACAGAAA | | |
| 4222 | MEOX1 | NM_004527 | CAGGCTTGACTGGGTGGACAA | CTCAATTCACTTGTAAGAAA | | |
| 4224 | MEP1A | NM_005588 | AAGGACGGACCGGGATGATTA | GACACCTATGATGATAGCTTA | CCGGGATGATTATGTGAACAT | GAGGCTGAACCGAATGTACAA |
| 4234 | METTL1 | NM_005371 | CGCGCATGGCTGCGTCATTAA | CACGTGGATTTGGTAGAGAAA | CAGAATATGCCTACGTGCTAA | CTGGTGTATACCATAACCGAT |
| 4239 | MFAP4 | NM_002404 | CTGGACTGTGACGACATCTAT | CCGCGGCTGGAATGACTACAA | CAGAAGAGATTCAATGGCTCA | AACTGGCTTCATACACACAAA |
| 4242 | MFNG | NM_002405 | ATGCTTATTTGTTAAGTCAA | CTGGCTAATGTCTCCAGTCA | CTGGCCGTTGGCCCTGGCTAA | CAAGACAATCATCCACAGCTA |
| 4254 | KITLG | NM_000899 | AAGCGAGATGGTAGTACAATT | CAGAAATGTCTAAATGCTGAA | TACTGTGAAACTGGCACTGAA | CACTGAATTAATCATCTATAA |
| 4255 | MGMT | NM_002412 | AAGCTGCTAGAGGTTGTGAAA | CCGGTTCTTCACCATCCCGTT | AAGGTTGTGAAATTCGGAGAA | CACCAGACAGGTGTTATGGAA |
| 4259 | MGST3 | NM_004528 | CAAGATGGCTGTCCTCTCTAA | CTGGTGCTGCCAGCTTATAA | ATGGCTGTCCTCTCTAAGGAA | CCGAACACGTTGGAAGTGTA |
| 4283 | CXCL9 | NM_002416 | ACCAATAAGTATTCTGTGTTA | CCACCCGAACGTCTTATCTAA | CCGTGGAGATCCCACCCGAA | CAGGTAGAACAGTATATAACTA |
| 4284 | MIP | NM_012064 | CCGCTGTGCTGTATAGCGTTA | CCGAGGAAACCTAGCACTCAA | CCGCATGGTCTCCAATGGACAA | |
| 4286 | MITF | NM_000248 | ATCGATGACATCATTAGCCTA | CAGCCTACAACTGAATAGCAA | TAGCCTAGAATCAAGTTATAA | CATGATGGTATCTATAGTCAA |
| 4287 | ATXN3 | NM_004993 | CAACATTGCCTGAATAACTTA | TGCGTCGGTTGTAGGACTAAA | TCGGAAGAGACGAGAAGCCTA | TACGATGGGATCATTATTTCA |
| 4291 | MLF1 | NM_022443.1 | GAGGGAGAGCTCATAATCGTA | CAGGAGTTCATCAATATGAAT | GACCGAGCTCATGTCATTAAA | GACCAATCTCTTGCTGTTAAA |
| 4295 | MLN | NM_002418 | CAGCAAATAAAGCATGAAATA | CCGGCCTTGACCGGAGGAGAA | CCGAGGAGAAACGTCTTCGA | CTCCAAGATGGTATCCCGTAA |
| 4296 | MAP3K11 | NM_002419 | CAAGGCCTCCACCTTCTCTAA | CAGCAGTTGGGCCATATTGAA | ATGCCGGTATGTATTGTCCAA | CCCGACGCTCGGAGGACTCAA |
| 4301 | MLLT4 | NM_005936 | TAGCTCTACAGTCCCACTTTAA | CAGCAGATCAATTAATTAGAT | CTCTATAATAATTCAACTCAA | |
| 4306 | NR3C2 | NM_000901 | CCAGACCTAGTCTTTAATGAA | CAGCAATTAATTAGATCAACA | CCACGAGGAAGCAGCAATTAA | CAACATTACCTTGTCTTTCAA |
| 4308 | TRPM1 | NM_002420 | AACGGGAATGTATCTTTGTAA | CAAGATTATACAACATCTTTA | TTCAGTTGCAGTAGTATCAAA | CACCATTAGGTCACTTAAGAA |
| 4317 | MMP8 | NM_002424 | AACGCCACTAACTTGACCTACA | ATGGACCAACCACCTCCGCAAA | CCGAAGAAACATGGACCAACA | TCCCAAGGATATATCAAACTA |
| 4319 | MMP10 | NM_002425 | CCGGAGCGCTGAGCCAGACAA | CAGGATGGTGACACACATATT | GAGCCTAAGGTTGATGCTGTA | GACACACATATTAAAGAGTAA |
| 4324 | MMP15 | NM_002428 | CACAAGATCGTCTTCTTTAAA | CACCAAATACTGGAAATTCGA | CTGGCTCTTTCAAGGAAATAA | TACATTTATTTCTGTAAATAA |
| 4326 | MMP17 | NM_016155 | ATCGGTGTAAGGTGGCCTA | CTCCATTATGAGGCCCTTCTA | CTGGTGTTCAAGGACAATAA | ATGACAGGACCTATTTCTTTA |
| 4328 | MMPL1 | NM_004142 | CAGGTCTTGCTCCGTCATCAA | CAGCTTGTGGTCCCCTTCTCAA | TGCGATCATCCGGACATCAAA | AACCATCATCCGAATGTCAA |
| 4329 | ALDH6A1 | NM_005589 | CTAGCGATTGACAAGCAATAA | AACGGCCGAGTTTGTCCCTTA | ACGGCGCCAGCCTGAAGAACAA | CCAGTTCTTCCAAGTGTCTAA |
| 4355 | MPP2 | NM_005374 | CAAGACCTACGAGGACATCAA | CACCAAGCAGACCATCAA | CACATCTTTATTGAACTTGAA | AACCAAGTGCTAAATCTTGTA |
| 4357 | MPST | NM_021126 | TGCGATTCAGTTCACCGCTAA | CCCGACCATTCTTCATAGGTA | CACACATTCTTCATAGGTA | AAGGGTTATTTGAGCAAGTAA |
| 4361 | MRE11A | NM_005590 | CAGAAACTTAATAGCCAGTAT | ACCAATTCTGTTGTAAATAAA | | |
| 4435 | CITED1 | NM_004143 | CAAGGATATGATATCATGGAA | TCCAGGCATGCTTGTGTTGAA | TTGATAAGCTATTGTCTGTAA | CCCATGGCTATCAACTTAAT |
| 4436 | MSH2 | NM_000251 | AACCATTAAACATGAGATTAGA | TCGCTCATATTAATTGATGAA | ATCAATTGTCTTGGATGCCAA | ATGCAGTGAGGTCTAACATAA |
| 4438 | MSH4 | NM_002440 | TTGCCAGACATTAGTGGATAA | TAGGAAGACTCCCGATTCTA | CACCTTCATGATCGACCTCAA | AAGAAAGATATTGTTTCTTTA |
| 4439 | MSH5 | NM_002441 | ACCCTTCTTGGTAGAAGCTAA | AAGGAAATTCTGGGTCTTGAA | | |
| 4482 | MSRA | NM_012331 | CAGGTCTGCGTAGATGGTGAA | AGGGAGTACTATAGTGTTCAA | TGCCATTAAGTTTGAGTATAT | ATGGAGCATCCTTCTCCGATA |
| 4486 | MST1R | NM_002447 | CTGCAAGTGCAAAGAGTGCAA | CTGGGCTTTCTTTCTGCCCTCAT | AAATATGTGACTGACAATAAA | CTCCAGGCTTTCTGCCCTAAA |
| 4493 | MT1E | NM_175617 | GCCTAGCAGTCGCTCCATTTA | CACGCCGTCCGGGTGGGCCTA | | |
| 4499 | MT1K | NM_176870 | CCGCACAAATAGGCACGGTCTA | CCGGGTTTCATCTGGAATTAA | | |
| 4520 | MTF1 | NM_005955 | GAGGTAGAATTATATGGTGAA | ACAGGTGAATGGATTATTCTA | CAGATCACCATCCATCTTCAA | ATCGTTTGGTGCCATCAGTAA |
| 4521 | NUDT1 | NM_002452 | AAGCGCCGAGAGGAAGATGTA | CTCGGGAGAACCAAACCGGAA | AACCTAGTACTCTCTGCTCTA | TGCCCTGTAACTTATCTAGAA |
| 4522 | MTHFD1 | NM_005956 | | | | |
| 4524 | MTHFR | NM_005957 | | | | |
| 4544 | MTNR1B | NM_005959 | CGGCCTCGCTGTGGCCATCAA | CTGCCTGAATGCCATTGCTA | CACTAGCTACTTACTGGCTTA | AACCCACGCACTGCCATTCAA |

Table4

| | | | |
|---|---|---|---|
| 4580 | NM_002455 | MTX1 | TTGGATAGACACCAAGAACTA CAGGGCCCGCTCCAAACATAA |
| 4583 | NM_002457 | MUC2 | CCGGTTTGGCAACAACACCAA CCCGCTGGGATTCGAAGTGAA |
| 4586 | XM_495860 | MUC5AC | CCAGTCTGTTGCATAAATACA ACCTTCAAGATGTGCCTCAA CTGGACAGTGTGCGAGCATCAA CACCCGGAGGTGAACATTGAA |
| 4587 | XM_039877 | MUC5B | CACCTATGATGAGAAACTCTA CCCGATGGGTTTCCTAAATTT |
| 4591 | NM_015294 | TRIM37 | TTGGACTTACTCGCAAATGAA CTCGAAGGTGGTCCTACTACA CTAGAGCTTAGTTGTGTTTAA CTGTAGATTAGTGAACAGAAA |
| 4594 | NM_000255 | MUT | AAGAGAGATACTATGGACTTA CTGGGCTCACTTAATAGAGAA CTGGAGCTGGCCTATACTTTA AGGGAATACCTAAACTTCGAA |
| 4595 | NM_012222 | MUTYH | CACATCAAGCTGACATATCAA CAGGAGGAATTTCACCACGCA GAGAATCCTGTTGTTAGTAAA CAAGCTGACATATCAAGTATA |
| 4598 | NM_000431 | MVK | AAGGAAGATTTCATCCTTAA CTGTGGCATCACACTCCTCAA GCGGTGGGCCTGGTTAAATAA CCGCTTGGCGATGCCAGCCAA |
| 4599 | NM_002462 | MX1 | TAGAGCTTACTTTGCAAAGAA TCCGTTAGCCGTGGTATTTTA CTGAAGAAACTTGTGAACGAA TAAGATCAAGACACTCATCAA |
| 4606 | NM_004533 | MYBPC2 | CCGCCTCGAGGTCAAAGCCAA TCCCAAGTTCCTGATAACCAA |
| 4607 | NM_000256 | MYBPC3 | CAAGGTCAAGTTCGACCTCAA TAGCACGGAGCTCTTTGTGAA CCGCATAAAGGTGTCCCACAT CAGGATGTCGGCAACACGGAA |
| 4609 | NM_002467 | MYC | TAGCAGTTACACAGAATTTCA CTCGGTGCAGCCGTATTTCTA CCCAAGGTAGTTATCCTTAAA AACGTTTATAGCAGTTACACA |
| 4610 | NM_005376 | MYCL1 | CAGCACTATTTCTTACGACTAT TGGCTAGTGACAAATAAATTA CTGTGGCTAGTGACAAATAAA CTCCAGTTGGCTTTACTTTAA |
| 4615 | NM_002468 | MYD88 | TCCGCAACTGGAACAGACAA CTCTGGCATATTCATCAAGTA CAGAGAGTAGCTGTGTTTGAA CATCCTGAGTTTATAATAATA |
| 4618 | NM_002469 | MYF6 | CTCGTGATAACGGCTAAGGAA AAGTGGGAAAGTTGCATTTAA TGCGAGGATCCTGGTATTGTA TCCTGGTATTGTAATATTAAA |
| 4619 | NM_005963 | MYH1 | AAGCGCATCCATGAACTGGAA CTGGGCTTTACTTCAGATGAA |
| 4625 | NM_000257 | MYH7 | CAGAGAGATTATCACATTT CAGACGGAGGAGGACAGGAAA |
| 4628 | NM_005964 | MYH10 | CTCGTTCATCGCATCAATAAA AAGCGGTTCAAAGCAAATCTA CCCATGTTGCTTCTTCACATA CAGGACTAATCTATACTTATT |
| 4636 | NM_002477 | MYL5 | CAAGGAGGCATTCACACTCAT CTCATCCAATGTCTTCTCCAA |
| 4653 | NM_000261 | MYOC | CCCATTCAAGAACCGCTATAA GGCGTAGACAATTTCATATAA CAGCATTATGGGATGTTTAA CAGCATCGAATATAAGTAAGA |
| 4654 | NM_002478 | MYOD1 | CCGCCTGAGCAAGTAAATGA CCCGCTGAGCAAGTAAATGA CTGCACGTCGAGCAATCCAAA TACAGGGAATTTGTACGTTTA |
| 4656 | NM_002479 | MYOG | CAGCTCAAGAAGGTGAATGA CCCGGATGTGCTTCCAA CCAACTGAGATTGTCTTCCAA CTGCCCTGAATTGAGAGAGAA |
| 4670 | NM_005968 | HNRPM | TGGCATCTTGTTGACATCGAA CCCACCCAGTATCCTAAATAA CCCGCAGGAATGGAATGGAA CCGGATGATGGAATGGCATGAA |
| 4671 | NM_004536 | BIRC1 | ATCCTCAATCTAAGTACTTAA CTCAATCTAAGTACTTAACTA TCGAAATTCATGATACTACTA CGTGGTGAACTTTGTGAATTA |
| 4684 | NM_000615 | NCAM1 | CCGATTCATAGTCCTGTCCAA TCCATGTACCTTGAAGTGCAA CCGAAACAACGTACGCCGTAA CAGCGCGGCCTCCGAGTTCAA |
| 4686 | NM_002486 | NCBP1 | CAGTATGGACTGCTGATAAA CTGGAAGAAGCTAAAGAGAAA |
| 4688 | NM_000433 | NCF2 | CAGAAGCATTAACCGAGACAA CAAGAAGGATTACCTAGGCAA CAAGTACACGGTAGTCATGAA CAGTTTGGAAGTGTTAACTAT |
| 4691 | NM_005381 | NCL | ACGGTGAAATTGATGGAAATA TCCGTCTAGTTAACATTTCAA TTGACTGGATATTCATATAAA ATCCGTCTAGTTAACATTTCA |
| 4692 | NM_002487 | NDN | CCCGAAATCACCAAGATGCA CAGTGCAGTATTCACGTGTAA CCACCGTGAGTCAACTTTAA CCACCGTGAGTCAACTTTAAA |
| 4696 | NM_004542 | NDUFA3 | CAGCCTGGAGTGGCTGAAGAA CAAGTACTCCGTCATGATCAA |
| 4704 | NM_005002 | NDUFA9 | TCCGGAAGCCATTATCGTAAA AAGAGAGGATAGATTCCTTAA |
| 4705 | NM_004544 | NDUFA10 | AAGCAGGACAATCGCACTTTA CTGGATGTTTGGAACCCTTTA |
| 4709 | NM_004491 | NDUFB3 | CAGATTGAGGATGCACATATA CAGAATATTAGTAAAGATTTA |
| 4716 | NM_004548 | NDUFB10 | CAGCAGAACTGTATCAAGGAA ATGACTGTTGCTGAAATATAA |
| 4717 | NM_002494 | NDUFC1 | CTCATCAAACAACACAATGAA CACCATCTGTTGAGTTGTAAA |
| 4718 | NM_004549 | NDUFC2 | CTGTAGGTATTAAGTCATTAA CTGGTCTATATGTGATTTCAA |
| 4719 | NM_005006 | NDUFS1 | CCGCAGATAATTAATGGACAA TTGGCTTATCTTCATAAATAA |
| 4722 | NM_004551 | NDUFS3 | CCGGAGAGTCTCAAGCTTGAA TTGAGTGTCCGTGTAAATAAA |
| 4736 | NM_007104 | RPL10A | CAGCGCCTATATTAAGGCACA CACATTGAATAAATTCTATT |
| 4739 | NM_006403 | NEDD9 | CGTGCCGAAATGAAGTATAA CACCCAAGAACAAGAGGTATA |
| 4741 | NM_005382 | NEF3 | CCCAATCACAATATCCAGTAA CAGCAGTACTTGCTCAATAAA |
| 4745 | NM_006157 | NELL1 | TGCGAGAAAGATATTGATGAA CTCATGTGAGTGAGAATTAA |
| 4750 | NM_012224 | NEK1 | ATGGCTCTCTCTACATAGTAA CAGAATAAAGCTCGAGCCGAA TAGGGATAGACCATCAGTCAA ACCGTAGGAGATGTTCGTCAA |

Table4

| No. | Accession | Gene | Sequence |
|---|---|---|---|
| 4760 | NM_002500 | NEUROD1 | CACGAATTTGGTGTGGCTGTA CAGATCGTATTAATTATCTGA CAAGCTTGTATATACATATTA CCCATGGTGGGTTGTCATATA |
| 4761 | NM_006160 | NEUROD2 | CAATGGCAACTTCTCACTCAA CCCGCTCGTCTCAATGGCAA CTCGGAGAATCTCTTGTCTTA CAAGACAATTTGTAACTAGAA |
| 4762 | NM_006161 | NEUROG1 | CCCAGGCTGTTGGGAACGTAA CCGGCTGACAATACAATGAAA CAGGGCTGGAGAGTACTTTAA CAGACGCTTCATGTAAATAAA |
| 4779 | NM_003204 | NFE2L1 | AAGGGTAGAGTGAACAGGAAA AAGGAGAATTCCCAATATTCAA |
| 4781 | NM_005596 | NFIB | CTGGACAATCAGGAAGTCCAA TACAATCACTAATTCCCTTAA AAGGTTAAACTGTAATGACAT ACAGAGGGCAAGTGCAATTTA |
| 4784 | NM_002501 | NFIX | CTGGTGCTTCTTACATTATAA CGGGACAATCAGATAGTTCAA |
| 4796 | NM_013432 | NFKBIL2 | CCCATCCGGGTTCGAGTTCAA CGGGAGCTCAGTGTAGCTGAA |
| 4804 | NM_002507 | NGFR | ATGGCAATTCTTTGACCTCAA CTCCTGAAACTTACACACAAA ACGTTAAGTGATGAACATTAA AACGTTAAGTGATGAACATTA |
| 4814 | NM_004148 | NINJ1 | CAGGACATTCCAGAAGGACAA CACGCTCTTTGTCAGGCTCAA |
| 4818 | NM_005601 | NKG7 | AACCTTGTGAGCAGAAGGCAA CTCCTTCTCTCTGAGATTCAA |
| 4824 | NM_006167 | NKX3-1 | ACGCTATAAGACTAAGCGAAA CAGGCTATCATATATACTGTA AACTGTGTTTATTGCCTATAA CCGGAGTACTAGCCAGCACAA |
| 4825 | NM_006168 | NKX6-1 | CCCGCTGTACCCTGCCGCGTA CTGGAGAAGACTTTCGAACAA |
| 4832 | NM_002513 | NME3 | CCGGCAGATGCGCGTCACGA ACGGCGCCTTGTCAAGTATA CTGCATCGAGGTTGGCAAGAA ACGCACCTTCCTGGCCGTGAA |
| 4833 | NM_005009 | NME4 | ACGCCTTTATGCAAGTTGTAA TTGTAAGGAGTGAACAGTAA CAGCACATGGGTGGTACACTA ACCAACTACCTCCGTCAGCAA |
| 4838 | NM_018055 | NODAL | GTGCTCCTAGATCACCATAAA CTGGATGTTCTCTTTACTGAA AAGATTAAGGATCACTGTATA AAGGATCACTGTATAGATTAA |
| 4849 | NM_014516 | CNOT3 | ACGCTGAGGGACCAAATCAA CTCGGACACTGTGGAATTCTA |
| 4853 | NM_024408 | NOTCH2 | TACCAGCATGTTATAGGGCAA TTAGATGATAATGGACAACTA AAGGGAAGGTAAGATGGATAA CTGACTAAACGTAGTAAGGAA |
| 4858 | NM_002516 | NOVA2 | GAGGTGGTCAACATCCTTCAA CAGGTCTGTGTGATATCTGTA |
| 4861 | NM_002517 | NPAS1 | TGGCTTTGTGTTCGCCTTGAA CGCCTTGAACCAGGAAGGAAA CAGCGTCTTCGACTACATTCA GCCCGTAGCCCTGAGAATTAA |
| 4868 | NM_004646 | NPHS1 | CAAGGCCTCATTCACCGTGAA CAGCAGCAATCCGCCGGTCAA TCGGACCAAACCAACATTCAA CTGGTTAGGTGAGCTCCATAA |
| 4879 | NM_002521 | NPPB | CACCTGCTTCTGATTCCACAA CTGAGGCGGCATTAAGAGGAA AAGATGGTTTCTTACCTTTGA TCCACGGTGAAATAAAGTCAA |
| 4880 | NM_024409 | NPPC | CAAGGCTGCTTCGGCCTCAA CCCAACGCGCGCAAATACAAA |
| 4881 | NM_000906 | NPR1 | CCGGTACTCACCTCACCAATGA CCTGAGCGTGCGCAAATTTAA ACGCATTGAGCTGACACGAAA AACGCATTGAGCTGACACGAA |
| 4882 | NM_000907 | NPR2 | CCCGGAGGATGGACTTCGAA ACCCAACTGAATGAAGACTA CAGGACAATCGCACCCGGGAA AAGGATGCCCTAGATGAGCTA |
| 4885 | NM_002523 | NPTX2 | TTCGATTAAAGTGCACTGAAA CTGGACTGTCTTCATCAAGTA |
| 4889 | NM_006174 | NPY5R | CCCTTAGTTTGTCTTACTGTA TAGGCATTTCAAGTTGGTGTA TTGGGCTCTATACATTTGTAA CACAGTCTTGTGGAACTTCAA |
| 4899 | NM_005011 | NRF1 | CAGGCAGATGAATGTCTTGAA CAGAGGTGCAATCAAATGGAA |
| 4901 | NM_006177 | NRL | CCGAATTTAGATGCTGGTCAT CAAGATTTCAATATCCAACAA ATCCAACAACTAGATAGATTA CTAGATAGATTAGGAGACCTA |
| 4905 | NM_006178 | NSF | AAGGAATGCAATAAAGAGTAA CAGTGTACACATTTACACTGAA |
| 4913 | NM_002528 | NTHL1 | CAGGCTGAGGTGGACCAAGAA GAGCAAGGTGAAATACATCAA CTGGAGGAGCAAGGTGAAATA CACGCTGGCAAGCTCATCTA |
| 4915 | NM_006180 | NTRK2 | CTGCATAGACAAAGGCCTTAA ATGGCTTAAGCCTGTGTATAA CTGGAGAGTTGTCTAGGGAAA ACCACGAACAGAAGTAATGAA |
| 4916 | NM_002530 | NTRK3 | CGCGATGGTTTCAGAGCGTGA CAGAGAGCACGGATAACTTTA CTGGTTGGAGCGAATCTGCTA CACGGATAAACTTTATCTTGTT |
| 4919 | NM_005012 | ROR1 | CAGCAATGGATGGAATTTCAA CCCAATATTGTCTGCCTTCTA CCGTACTGCGATGAAACTTCA CCCAGTGAGTAATCTCAGTAA |
| 4928 | NM_005387 | NUP98 | CGGGATTTCTTTGTAAAGCAT CTGGAGTTAGCACTAACATAA CAGTGTATTACTGCTATGAAA TGGGACTACTATTAAATTTAA |
| 4935 | NM_000273 | GPR143 | CTGAGGCTGGCTGTAAAGTAA CTGGCTGTAAAGTAAGTGTAA TCGGATATGAACCACACGGAA CAGCTAGAGTCTTCTTCCCGAA |
| 4948 | NM_000275 | OCA2 | CGCCGGAAGTGTAGAAACCCA CTGGCTGTGATTGGCGATAGA TGGAATAGACCTTGACACTAA GACCTTGACACTAACACTCTA |
| 4967 | NM_002541 | OGDH | CAGGATCAATCCGTGTCACCGA CCGGCAAGGTCACCATCCA CACTCTTTCTTTCCTCCCTTAA TTGGCTTGCACCCATAAACTAA |
| 4975 | NM_006189 | OMP | AAGGAGGACTCGGATGCCATA CTCGGATGCCATAGATTGGAA CTGGAATGTCGTGCTGACAA |
| 4978 | NM_002545 | OPCML | CACGTGTAGGATAATCATTCA ATGCATGAAAACCAACACTAA CTGGAAAGTAGCAAGCTAA CTGGGACACTATCTCACTATA |
| 4985 | NM_000911 | OPRD1 | CACGCTCACCATGGATGAGTGT CTCCATCGACTACTACAATAT CGGCATCGTCCGGTACACTAA CAGCATCTTCACGCTCACCAT |
| 4986 | NM_000912 | OPRK1 | CAGGGAAGACGTCGATGTCAT ATCGATGGGATGAATAAACCA TAGGTTAACTCAGATCACTA CAGATGCTTTAGTTACTACAA |
| 4987 | NM_000913 | OPRL1 | CTGCCTTGTCATGTACGTCAT CAGCGGTGCAATGAACTATCA CTGGCTGGGTATTCTTCGTAA ATGGGACAGGTCAAAGCATTA |
| 4988 | NM_000914 | OPRM1 | CCGTAGTAACACATAAAGTAA AGGGAAGAGATTAGCATGAAA TTCACCGTAGTAACACATAAA TCCAGAGTGTGAATTACCTAA |

Table4

| # | Symbol | Accession | Sequence |
|---|--------|-----------|----------|
| 4992 | OR1F1 | NM_012360 | CCCACTCAGCTGAGAAAGACA CTGAGAAAGACACTATGGCTA CAGCACCATCATTGCTGTGTA ATGGGTGGTTGCCAACCTGAA |
| 4993 | OR2C1 | NM_012368 | TCAGCTATGTGGCTGCATAA CCCAAATGCTGATCAATTTAT CAAGTTCATTCCCGTGTTCTA AAGCATCATCGTGATCTCCTA |
| 4994 | OR3A1 | NM_002550 | CTGGGTTCAACCAAGCTTTCA TTCAGTGTATCTTAACTATAT CAAGCTTTCAGACAAGGATAA AAGCTTTCAGACAAGGATAAA |
| 4995 | OR3A2 | NM_002551 | AGGGTGCTCTGTGGCAAATAT ATGGACAAACTCCAAGGCAAA CACGTGGCAGCTGCAGTTCTA AAGAGGTATCTTCAACTACAT |
| 5003 | SLC22A1LS | NM_007105 | CTGCAGCAACATAGAGTACAA CTCAGCCAGTCTTAAAGGCAA |
| 5004 | ORM1 | NM_000607 | TCGAAACGAGGAGTACAATAA TTCCCTCACTTGCATCAATAA CAGCACGAGAAGGAGGAGAAA CACGATCTTTCTCAGAGAGTA |
| 5008 | OSM | NM_020530 | CAAGGCCTGGATGTTCCTAAA CGGGCTCAGGAACAACATCTA CTGACGTAAGGTGGATATTAA TGGAGGGACATGGACTAATTTA |
| 5009 | OTC | NM_000531 | AGCGGTGGAGCTTGGCATAAA AGGGCATAGAATCGTCCTTTA AGCATCCATCCAATTATCAA TACGTGCTTAACTTTGCTTAA |
| 5025 | P2RX4 | NM_002560 | CTCCGTTACGACCAAGGTCAA AGGCAGTGCGTAGCTTTCAA TCGTGCATTTATGATGCTAAA CGGGACGTTGAGCACAACGTA |
| 5030 | P2RY4 | NM_002565 | CTGGGATGCAACGGCCACCTA CAGAGGTGAGGGAACCCAATA CCCAATAGTGATACCTGCTAA CAGATAGATTGTAACACGGGA |
| 5033 | P4HA1 | NM_000917 | CAGAGAAGTTAGATCGGCTAA TGGCAACAAATGGGTATCCAA CGAAATCATCATATTGCATAA CAGGTGGTAATATTGGCATT |
| 5034 | P4HB | NM_000918 | GAGAAGACGACGAGTGTTTA CTATTTCACAATCGAATTGAA CAGGACGACCTGTGTTCTTTA AAGTGAACTGTAATACGCAA |
| 5051 | PAFAH2 | NM_000437 | CAGCGGTAAGCGAGTGTTTTA CCCGAGGACCTGTGTTCTTTA GAGAAGTAGTTTGGTCAGCTA |
| 5062 | PAK2 | NM_002577 | TGCAGTAGTATAAAATCATGAA CCGGATCATACGAAATCAATT CCGCGACCGGATCATACGAAA ACGAGTAATTGTGAAGCATAA |
| 5069 | PAPPA | NM_002581 | TGGGCAGTTCATGAAGCTCTA CCGACAAGAAGTCTCCTTCAA CTGGCCAATGCTTAAATACTA CAGGGAGTGCTTGTTGTGATAT |
| 5101 | PCDH9 | NM_020403 | ACGGTTCACCATCAATTTAAA ACCGTCTAATACTTCCTTAAA ACCGACGTGGGTTATACTTAA AAGGCGGTATATGACAACCAA |
| 5104 | SERPINA5 | NM_000624 | CTCCGTTCCTCATAGAACAA TACACAGGTTTAGTTGACTAA CAGTATCACATCTGAACTAAA CAGCTTTAACTAATAATATGGA |
| 5108 | PCM1 | NM_006197 | CGGATAAACTTCAGTGATTTA CAGTATCACATCTGAACTAAA ACCGTGATTCACTTAGATCAA TACGTGCCTTAACAGATAAA |
| 5110 | PCMT1 | NM_005389 | CAGGCGCTAATAGATCAGTTA TTGGAGCAGTATGACAAGCTA CTACAAGATGGCAGCATCAAA TACGTCCTTAACAGATAAA |
| 5111 | PCNA | M15796 | TGCGGATATGGCACACTTAAA TACACTAAGGGCCGAAGATAAA ATGGATTTAGATGTTGAACAA AAGGATCTTAGGCATTCTTAA |
| 5121 | PCP4 | NM_006198 | ATCATCTGTCAAGAAAATTAAA ATGGAATTGTGAGTAGCTTAA |
| 5129 | PCTK3 | NM_002596 | CCGGCGGAATGAGAACTTGCA CCGGGTATAAGGAGCAAAGGA CAGGTTGGATACGGATGGCAT AACCCGTTACTTAGCTGTGTA |
| 5133 | PDCD1 | NM_005018 | CTGGTTGGAGATGGCCTTGGA CCCTGTGGTTCTATTATATTA CCCATTCCTGAAATTATTTAA ATCGGAGAGCTTCGTGCTAAA |
| 5134 | PDCD2 | NM_002598 | CAGATCATCTGGACCAATATAA CATGAATTTCATCAACAACTA |
| 5138 | PDE2A | NM_002599 | TCCGACGATGAGTATACCAAA TGGGATCGTAATAATGGCAAA ATCGCGGAGCTGATCTACAAA ACCGCTTTGTACATGAGAATA |
| 5140 | PDE3B | NM_000922 | TACGGAGTATTAGTAGCTTAA CTGCTGTATTATACAACTGTA CACAGAGTGAACAGCAAACAA CAGGTTATGTATACCTTATTT |
| 5141 | PDE4A | NM_006202 | CAGGGCTGGCGGGCCATGTAA CTGGTGGGCCCTGACAAAGAA ATGGGAAGTCGTGTCATCCTA TTCGATCTTGTCTCCAATTAA |
| 5146 | PDE6C | NM_006204 | AAGGTCGTCCTTTCTGAACAA TCGGACGTGGGAATTAAGCAA CACGGTTAGATCATATCTGAA ACGGATTGATTAAATGTGGAA |
| 5150 | PDE7A | NM_002603 | CAGATAGGTGCTCTGATACTA CCGGAGGAAGGAGCATTTGA CCCGAACATACAGCAATATGA CCGAACATACAGCAATATGAA |
| 5152 | PDE9A | NM_002606 | ATGGACGCATTCAGAAGGTAA CGCGAGACTCCTGTAACCTTA AACGGATAATCCTGATCCTAAT |
| 5156 | PDGFRA | NM_006206 | CTGAGCCAGATTGGCCAATTA CCCGAGACTCCTGTAACCTTA ACGCAGGAAGCCTACTATTTA |
| 5157 | PDGFRL | NM_006207 | CCAGGAGAGAATAGAATCAAA CTGGAGGATGTTACTGGCCAA CACGCTGCTGCTCATGATGCAA CCGGGAAATTGTCTTCTACAA |
| 5158 | PDE6B | NM_000283 | GCCCACCACATTTGACATCTA CTGAAGCCAACTGACATTTCTTAA CACGCTCTTGTTCACATCAAA ACCCGTGTCAATTAACATTTA |
| 5166 | PDK4 | NM_002612 | CAGGACACTTTACGGGATCAA CTAGACAGTGACATTTCTTAA |
| 5178 | PEG3 | NM_006210 | AAGGATGACCCTGATGACAAA CCGGATGTCATCATAGGAGAA |
| 5184 | PEPD | NM_000285 | CACCGTGGAGCCGGCATCTA CAGGCTGTGACAAGGCCTTTA TCGGCATTTGATCAGACCAAA CTGTCCTTAGTAGCAAGTAA |
| 5189 | PEX1 | NM_000466 | CAGTAGCAAGTTTATGGACTA CTCATGGATACTATCCACCAGAGA CTCATGGATACTATCCAGTA AAGGCAGATACCGGAGCCAAA |
| 5190 | PEX6 | NM_000287 | CCAGAGAGTTACACATCGAAA CACACGCAAATTCAAGCTAGA CCAGAGCTCATTAACATGTAT CAGGATGTGTTTGTGATTGGA |
| 5191 | PEX7 | NM_000288 | CGGGCTGTGGAACCCTACTAA CAGGAGGTGTATAGTGTTGAT AAGCCTGACTCTCTTCTTGAA TAGGTTATACGTATATAGATA |
| 5204 | PFDN5 | NM_002624 | ACAGGCCGTCATGGAAATGAT CAACATGGCGCAGTCTATTAA |
| 5205 | ATP8B1 | NM_005603 | CTGGATATCTTTGCAAATGAA CTGGTTCTTGGTTGAATGAAA |
| 5208 | PFKFB2 | NM_006212 | CAGAACAACAACAGCTATGAA CCGTGACAAGCCAACTAACAA CCAGAGCAAGATAGTCTACTA |
| 5209 | PFKFB3 | NM_004566 | CTCCTAGTTGCTGAAAGTTAA CTGGGATATAAAGCTATGCTA CTCCAATATCATGAAGTAA CAGGGACTTGTCGCTGATCAA |

Table4

| | Gene | Accession | Sequence |
|---|---|---|---|
| 5210 | PFKFB4 | NM_004567 | CCCACGGGAATTGACACAGAA TACGAGGAAATTCAGGATAAT CAGAAAGTGTCTGGACTTGTA ACGGAGAGGCGACCATCTTTAA |
| 5214 | PFKP | NM_002627 | TCGGAGAACCGTGCCGGAAA TGCGGGCAACCTGAACACCTA CGCCTGAAGGCTGCTTGCAA CAGCACTTTATGCACGTATTA |
| 5216 | PFN1 | NM_005022 | TCAACAAGAAATGTTATGAAA CCACGGTGGTTTGATCAACAA TAGCATGGATCTTCGTACCAA CGGGTTGGAACGCCTACATCGA |
| 5228 | PGF | NM_002632 | CAGGCTCTGCACAGTCAAGCA AAGAGCTGCCTGGATGAGAAA TGCCCTCTATTTATTAGCCAA ACCCGGCTCGTGTATTTATTA |
| 5236 | PGM1 | NM_002633 | CAGGTACAGTTTACACTACAA CAGCAAGACAATTGAAGAATA |
| 5243 | ABCB1 | NM_000927 | GAGGTCGGAATGGATCTTGAA GACAGAAAGCTTAGTACCAAA CACGGAAGGCCTAATGCCGAA ATCGAGTCACTGCCTAATAAA |
| 5253 | PHF2 | NM_005392 | CTGGATTTGTTTCTCAGGCAA AAGATGAATCTTCAACTTTAA |
| 5256 | PHKA2 | NM_000292 | CTGGTGAAATGCATCCCTTAA CACCACATCGTTCTACACATA CCGGATTCTTAGTCACATATA CCGCCTAACAAGGTAGATGAA |
| 5265 | SERPINA1 | NM_000295 | AAGGCTGACACTCACGATGAA TACGTGGAGAGGTACTCAA CACCCAGATATCATCACCAA CCCGAGGTCAAGTTCAACAAA |
| 5271 | SERPINB8 | NM_002640 | CTCAATCAAGGCGGACGTGAA AGGGCAAACCCTATAGAGTAA ACCACTGAGTCCTCTAACTAA TTAGACAGTTGAGTAATTAA |
| 5281 | PIGF | NM_002643 | CACCATGAAAGATAACGATAT AAGAAGTTCATTATCACACAA |
| 5283 | PIGH | NM_004569 | CAGGAAGAAAATGTCCTACAAA CAGGTTTACTTTGTTGATGTT |
| 5288 | PIK3C2G | NM_004570 | CTGGTGTGTGGTAACATTCAT CCGGGCCTCTGAATCACATAA CCCGTAGAAATGATAACTCCA CCAGATCAAGAAATTCGTAAA |
| 5295 | PIK3R1 | NM_181504 | ACGGCTGAATATGAATAGATA CACCCAGTGTAGCATCCTAAA TAGCTGGTTATGAACTAGTAA CAGCATTAAACCAGACCTTAT |
| 5296 | PIK3R2 | NM_005027 | CAGGGCCAGACTCAAGAGAAA CGGCGTGAGGCAACGAGAAA TTGGTACGTGGGCAAGATCAA CCGCGAGTATGACCAGCTTTA |
| 5298 | PIK4CB | NM_002651 | TGGGACACATGTTCAACTACTA CGACATGTTCAACTACTATAA TCGGCTGATAGTGGCATGATT CCGAGAGTATTGATAATTCAT |
| 5300 | PIN1 | NM_006221 | CGGCTACATCCAGAAGATCAA CAGGCCGAGTGTACTACTTCA GACCGCCAGATTCTCCCTTAA CAGTATTTATTGTTCCCACAA |
| 5303 | PIN4 | NM_006223 | CACACTATTCACACAACTGAA CAGCATTTGCCTTGCCTGTAA |
| 5310 | PKD1 | NM_000296 | CCGCTGAACACAGGGCGCATTA CCGAGCCATTACCACCCATA CTGGGTCTCAGCCACGTACAA CCCGTCCATTGTGGGTAGCAA |
| 5313 | PKLR | NM_000298 | CTGAAAGTCTGTGTCCAATTA AAGCATGTCGATCCAGGAGAA GAGGCCTATCTGAGACTATAA CGGGTGCAATTGGCATTGAA |
| 5314 | PKHD1 | NM_170724 | TTGCAGCTAGATATAAATTAT CTGATGAGTATTGAAGTACTA CACCGAGATAGCTGTACTTTCA CACCGGCATATTGGAAGTGTA |
| 5315 | PKM2 | NM_182471 | CTGATAACGCCTACATGGAA CCGGGTGAACTTTGCCATGAA CCCGATCAGTGGAGACGTTGA AACATCAAGATTATCAGCAAA |
| 5317 | PKP1 | NM_002299 | CAGGATGAATCTGCCAAGCAA AGCATAACCACAAACTGCAA TCCCGAGGGCCTTATGAGAAA CTGGTGTTAGAGAGGAATTAA |
| 5318 | PKP2 | NM_001005242 | CAGGAGACTACTTTACCTATAA CAGAATGAAATGACAAGAAA |
| 5324 | PLAG1 | NM_002655 | AAGGGTCAGTTGGGACATTAA AACCAACATAATCTTCTTAAA |
| 5325 | PLAGL1 | NM_002656 | CAGCATTGTGATTAGAATGAA CAGGAACACATATTTGCATGTTA |
| 5326 | PLAGL2 | NM_002657 | AAGGATATTTATGTAATAAGA TCCAGATTATTTAAAGAGAAA |
| 5328 | PLAU | NM_002658 | CTGCCTGCCCTCGATGTATAAA CACGAGAGTCTCACACTTCTT GAGCTGGTGTCTGATTGTTAA CCGCATGACTTTGACTGGAAT |
| 5330 | PLCB2 | NM_004573 | CTCGGTGCAGTTTGTGGACTA CCGGCCGACAAGCAGTTCAA CTCCATCAATCCTGTCTGGAA GCGGCTTGCCTGGAACAGATA |
| 5332 | PLCB4 | NM_000933 | ACCAATAATAACTCATGGAAA CAGGTCAATTCTTATCAGATA CTGCAGATTCCGGATATTATA AACCCGGTAGTCTAGAACTAA |
| 5334 | PLCL1 | NM_006226 | CTCGATGTAAGTGATGGTTCA TCGAAGGATGTGCGGTAGATTA AAGGATGTCGGTAGATTACAA CTCAGGAATATCGGTCTTAAA |
| 5336 | PLCG2 | NM_002661 | GACGCGGACTACCAAGATCAA TAGGGTCTCTTTGCCAGAGGAA GACGACGGTTGAATGATAA CACGTGGAAGATTGACAGCAA |
| 5338 | PLD2 | NM_002663 | CCACAGTCCTTGATTCTCAA CCGGCCTTCGCAATCCTGACGGAAA CAGCCTGCTGCACAGACACTAA CGGGACCTCCTGAGACACAAA |
| 5340 | PLG | NM_000301 | CTGGGAGCAGGAAGTAGATAGAA CTGCCGCAATCCTGACGGAAA CACCACCACCAGGAAAGAA CAGGAAAGAAGTGTCAGTCTT |
| 5341 | PLEK | NM_002664 | CTGGATGATGGGTTAATACAA CTGTGACAAATCAACGGGAAA GAGCATTTCTTAACGTTTAT ACCATTGACTTAGGTGCCTTA |
| 5346 | PLIN | NM_002666 | CAGCCTTATTTGATTTAACTA ACCCTAGTCTTCGAAATGTTA GCGGGTTTCTTCTAACAAATAA TGGCTTGTTTTATGAACATTAA |
| 5347 | PLK1 | NM_005030 | CAACGGCAGCGTGCAGATCAA CGCGGGCAAGATTGTGCCTAA CCGGATCAAGAAGAATGAATA CACCATATGAATTGTACAGAA |
| 5356 | PLRG1 | NM_002669 | ACGGATTCATATGTTCATAAA ATGGCATTATCATGGACATTTA |
| 5357 | PLS1 | NM_002670 | TAGGATGTTATTAGAGAGATA ATAGAATATATTCATAAATCAA |
| 5364 | PLXNB1 | NM_002673 | ATGGTGACCAGCTTTATAAA ATCCTAGGTCTAAGAGTTGAA CCGGGTGGAATTATCCTTGA AACGCGGACAGCAGTTCAAGTATA |
| 5371 | PML | NM_002675 | TGCGGTGAACCGGGAAAGCAA CTCCAAGATCTAAACCGAGAA CAGGGTTGTCCGCTCTGATTA CAGGAGCAGGAGTAGTGCCTTT |
| 5376 | PMP22 | NM_000304 | CAGCCTCGTTGTTGAGCCTTAA TAGGCTGCTATTATTACTATA |
| 5407 | PNLIPRP1 | NM_006229 | CCGCTTCCTGCTGTACACCAA CCGGGACTTTGTTGGCTTGCAA CAAGTATTACTTGGAAAGCAT CCCGATGGGTTTGCTGCATAT |

Table4

| | | | | | | |
|---|---|---|---|---|---|---|
| 5408 | PNLIPRP2 | NM_005396 | CTAGTTGGAGATATAAGGTAT | TCAGGATTGCTTGTGATGGAA | CTGGAACAAACGTGGGATAAA | TGCGGCTATTGCGGTAATAAA |
| 5412 | UBL3 | NM_007106 | TGGGTGTATATCAAACGGCAA | CCCGGCGGATATGATAAATTT | AAGGACGATTTCTACATGGAA | CGCGTAGTAGTATAGAACAATA |
| 5413 | NA | NM_002688 | AACAATGTTGGTGAACGTCAA | CCGGACGGTCACAGCACCCAA | | |
| 5423 | POLB | NM_002690 | CCGGAGCGAATGAGGCCTGTA | CCCAATAGGAGTCTTAATTTA | TACGAGTTCATCCATCAATTT | CAAGATATTGTACTAAATGAA |
| 5426 | POLE | NM_006231 | CCAGATTATGATTTCCTA | CTGGATGGATCCATCTAACTA | CCGCCTCTCCATTGACCTGAA | CCGCATCATCCTCGTACAAA |
| 5428 | POLG | NM_002693 | CTCACTGACAATAGTGCCATA | CTCCAAGGCAGTGAAATAAA | CAGATGCGGGTCACACCTAAA | CACGAGCAAATCTTCGGGCAA |
| 5429 | POLH | NM_006502 | CAGCCAAATGCCCATTCGCAA | GAGGCAGTGTCATAAAGTAAA | CTGGTTGTGACATTCGTGTA | ATCCATTAGGTGCTGAGTTA |
| 5430 | POLR2A | NM_000937 | CTCGGGAAGAACAAAGCTAAA | CACCACAGTCTCAACCTATA | | |
| 5434 | POLR2E | NM_002695 | CTGGAGGAGTTCAAAGCCCAA | ACGAAATAAATTGCTTGATA | | |
| 5435 | POLR2F | NM_021974 | TCACTTATATGTGTAAAATAA | CACACCATACACATGACCAAGTA | | |
| 5436 | POLR2G | NM_002696 | CTGGTGCTTCTTGTAGCTTAA | CAGGGAAGTATGGCTTTGTAA | | |
| 5437 | POLR2H | NM_006232 | CAGAGTTTATCTCCTGATGAA | ATGGATCTAATCTTAGATGTA | | |
| 5439 | POLR2J | NM_006234 | CAGCGGTGACTTCGCAAGCAA | CAGGGATGAATTCATTCTGA | | |
| 5440 | POLR2K | NM_005034 | CAAGAGATTAATAATAATGTA | AACAATGATAACATACTATAA | | |
| 5441 | POLR2L | NM_021128 | AAGGTCTTTCAGAACCACTAA | CTGGAAGGAACCATCCAGTAA | | |
| 5452 | POU2F2 | NM_002698 | TCCAGAAATAAGAATGTCTAA | AAGAAAATGGACCAGACACTAA | | |
| 5464 | PP | NM_021129 | CTGGAAAGTCATTGCCATTAA | TAGCACATGCTTAAATATCAA | | |
| 5467 | PPARD | NM_006238 | CCCGTGGAGCAGTGATCTCTA | CAGTGATATCATTGAGCCTAA | CACAGACTGACGAAACTTTAA | ACTGACGAAACTTTAAATAAA |
| 5468 | PPARG | NM_005037 | GAGGGCGATCTTGACAGGAAA | CAGACAAATCACCATTCGTTA | CCGGAGAACAATCAGATTGAA | CAGCGACTTGGCAATATTTAT |
| 5475 | PPEF1 | NM_006240 | CTGGGAAACCCTCTTCAATAA | ATCGAATATGCTGATGAACAA | CCCAATCGGTACAATCGTTGA | CAGCATTAGTACCTACATATT |
| 5476 | PPGB | NM_000308 | CCGGAGTACAAGAACAACAAA | TGCAACTTTCTGTAAACTTA | ATACCAGATCCTATTATATAA | CCGGCCCTGGTTAGTGAAGTA |
| 5496 | PPM1G | NM_002707 | GAAGTTGTAGATTTCATTCAA | TACTCTGTGAACTTTATTTAA | CCAGAGGATGAAGTAGAACTA | CAGGACCTGAGGACTCAACTA |
| 5498 | PPOX | NM_000309 | CAGCGGCTTGGCCGCCAGTTA | CACGTTATTAGTGCCATTCCA | CAGGAAGCAGCTGCTACACAA | CTGCTTGGTCCATCTACACAA |
| 5501 | PPP1CC | NM_002710 | GAGGAGTAAGTGTACAATTGA | CTGCGGTGAAGTTGAGGCTTA | CTGGTTATACACGCAAATGAA | AACATCGACAGCATTATCCAA |
| 5509 | PPP1R3D | NM_006242 | TCAGGTGACTAGAGAATTTCA | AAGGATAGCTTTCTTCGGTAT | CTGAGTTAGGCAATCACTTAA | CAGGTACCCACCAACTTTATA |
| 5511 | PPP1R8 | NM_002713 | AACCCTGAAGCTGTAAATGAA | CTGCTGAAGTTTCTTATTTAA | TACAAATAAAGATGCCCTAAA | CAGGCCCAGGAACTGAAGAAA |
| 5514 | PPP1R10 | NM_002714 | CAGCAACACTCAAGCCCTTGA | CAGGATAACAACTACTTAT | CCGAAGGACCGTCACTCACATA | CCCAGCCTGTCGAGAAAGATA |
| 5516 | PPP2CB | NM_004156 | CACCGGATACAAACTACTTAT | ATGGAATTAGATGACACTTTA | TGGGATCTGTCTTGGCATTGA | CCGACAAATTACCCAAGTATA |
| 5519 | PPP2R1B | NM_002716 | TGGACCAATTCTAGATACCAA | CTGACGTTCGTTTGAATATCA | CAGGAAATAACTACTAAGCAA | CAGAAGTTAGGTCAAGATGAA |
| 5520 | PPP2R2A | NM_002717 | AAGCAGAGACATAACCCTAGAA | CTCGCCGTGTCTGGCACTGAA | CTGCAGTGATTTGCGGATTA | ATGGAAGGTATAGAGATCCTA |
| 5521 | PPP2R2B | NM_004576 | CGGCTACAAATAACCTATATA | CCGGAAGATCCAAGCAACAGA | CAGGGACTACTTGACCGTCAA | CAGGAAATGATTGGAATAGAA |
| 5524 | PPP2R4 | NM_178003 | CGGATTCATCCTTACCCTCAA | CCGCTCCCTAAGGGTAACCTA | CTGAGGTGGCTGTTTACCTAA | CTCCGGGTGGATGACCAAATA |
| 5527 | PPP2R5C | NM_002719 | CTGGAAATATTGGGAAGTATA | CTGCTACTTCAGTAAGAATAA | CCCATTGAACAAGTAAGAAA | AACGAGCTGCTTAAGTGAAA |
| 5528 | PPP2R5D | NM_006245 | GAAGTTGTTTATGGAAATGAA | CAGGAGATTATTCTCACCAAA | | |
| 5532 | PPP3CB | NM_021132 | CAGCCCGGAAAGAAAATCATAA | TAGTTTATTGTGAGTACTCAA | TAGGAGATTAGATAGATTCAA | AAGGGTTTGGATAGATCAAT |
| 5534 | PPP3R1 | NM_000945 | GAGCCTCATGAAGCCAACTAA | TCCCTTTATAGTTTCTCTATA | GCCCATATAATTGGTAATGGA | ATCCTTTAGTACAGCGAGTAA |
| 5544 | PRB3 | NM_006249 | TCCCTCCGTAATATCAGGAAA | CCGGGAAAGCCAGAAGGACAA | CAAGAAGGTAACAAACCTCAA | AGAAGGTGGTCATAGCTCTA |
| 5549 | PRELP | NM_002725 | CAGGTCAAGAATAACACTTCA | AAAGATTATACTCTTAAGAAA | | |
| 5551 | PRF1 | NM_005041 | GAGCACCGTGTGGGACAATAA | ACCATGTACATAGCTTGATAA | CACGGTGGAGTGCCGCTTCTA | CACCGTGTGGGACAATAACAA |
| 5554 | PRH1 | NM_006250 | TCAGCTCAGGATTTAAATGAA | TAACTTCAGTATACCAATAAA | CAGGAAGTGAATAAGAAGATA | TGGCATCATGCTCTAACTTCA |
| 5562 | PRKAA1 | NM_006251 | CAGCAATAAGCATGCATAATA | ACCAGAGTATATTTAAGTAATA | TCGGGATCAGTTAGCAACTAT | CCCACGATATTCTGTACACAA |
| 5567 | PRKACB | NM_182948 | ACCAATGTGTTCTATATGTAA | GGCATTAGGAGTGCTAATCTA | CTGACCAATCAAGTACACTAA | CAGCCTGTGTAGTGTGACAAA |
| 5569 | PKIA | NM_181839 | CAGCAATGAATTAGCCCTGAA | CTCAAGTGTTCACCCAGTAAA | TCACTTGATCATATGACGAAA | ATGATTATCATTAGAAGCTAA |

Table4

| | | | Sequence |
|---|---|---|---|
| 5573 | PRKAR1A | NM_002734 | CTGGACCGACCTAGATTTGAA CAGCTAGTGCCAAATAATTGA CAAATATTGGTTAGTATTTAA ATGAAATAATTTAAATTCTTA |
| 5576 | PRKAR2A | NM_004157 | AGCAGATTAATAGACGAGTA CTGCAGAAACCTGTTGTATAA AACTTGAAAGTCAGCACTAA AACGGCATGTCTCTCCAACAA |
| 5577 | PRKAR2B | NM_002736 | CTGGCCTTAATGTACAATACA AAGGATGTTATCATAGGCTAT CACGCCATTGGGACTGTCAAA ACGAACATGGATATTGTTGAA |
| 5578 | PRKCA | NM_002737 | TACAAGTTGCTTAACCAAGAA CGCAGTGAATGAGTCCTTTA GCCATCATATTCATGTGTAA CAAGACGTCTTTGGAATCCAA |
| 5581 | PRKCE | NM_005400 | TACAATGACGTTAAGTCTTAA GCCATCATATTCATGTGTAA CCCGACCATGGTAGTGTTCAA CACGGAAACACCCGTACCTTA |
| 5583 | PRKCH | NM_006255 | AGGGACATCTTCCAATGATTA CAGGATGAGTTTAGAAAACTTT GACCAGATTTGTGGCTTATAA CGCCCGTGTGCTCATCACTGAA |
| 5585 | PKN1 | NM_002741 | CCGGAGCAGCCTCAAAGCAGA CAGCCTCAAAGCAGAAGCCGA CCGCAAGGAGCTGAAGCTGAA CAGGACAGTAAGACCAAGATT |
| 5587 | PRKD1 | NM_002742 | AACAAAGCTGTTAAACTGTTA TCGATTATTTCCAGTGTTCTA CCTCAGTATATTTAAACTCAA CAGGAAGGCGATCTTATTGAA |
| 5589 | PRKCSH | NM_002743 | CAGCCTTCAAAGATGGGTAAA CACCACCAACGAATACGTCTA CTGCACCAACACTGGCTATAA ACCTGTGACCTCAATACAATA |
| 5592 | PRKG1 | NM_006258 | AAACACTTCAATGTAGTATAA TAGATTACTATGGTACAGAAA GAGGTTCGTTGAAGATTCTA CCGGTGTATATACCATACAAA |
| 5593 | PRKG2 | NM_006259 | CTGTTTATAGATCGAGAAACA CACGCGACCTGAGGATTTGA TTGAGTACTACTAACGTTTAA GACCTACAATTGATTCTCAA |
| 5599 | MAPK8 | NM_002750 | TCGGGAGTTAAAGCCGTAA AAGCCCAGTAATATAGTAGTA AAGCAGTTAGATGAAAGGGAA |
| 5603 | MAPK13 | NM_002754 | CAGAAGGGTCCTTCTCCTTAT TAGGGATGTCTCTAACGAATTA CGGGATGAGCCTCATCCGGAA CCGGAGTGGCATGAAGCTGTA |
| 5604 | MAP2K1 | NM_002755 | AACAAACGTGTATAGTGCCTAA CTAGATGTTTAACAAATCTAA CTGGAAGAAATTCCTGAACAAA |
| 5605 | MAP2K2 | NM_030662 | CACAAACCACACCTTCATCAA TTCCAGGAGTTTGTCAATAAA CCGGCCTGCCATGGCCATCTT CAGCATTTGCATGAAGAACACAT |
| 5606 | MAP2K3 | NM_002756 | CTGGATGCCATCCAAGTTGTA CTCCAATGTCCTTATCAACAA CCGGGCCACCGTGAACTCACA ACGGATATCCTGCATGTCCAA |
| 5610 | EIF2AK2 | NM_002759 | CTCCACATGATAGGAGGTTTA TACGTGTGAGTCCCAAAGCAA CGGAAAGACTTACGTTATTAA ACGAAAGACTTACGTTATTA |
| 5617 | PRL | NM_000948 | CAGCGAATTCGATAAAACGGTA TGCAACGATCACGAACATGAA CAGCATATTGCGATCCTGGAA CCCGGAGCCTATCCTATCCAA |
| 5619 | PRM1 | NM_002761 | CCGAGATGTAGAAGCACTAA CCGCCTGTCAATAAAATGTGA |
| 5631 | PRPS1 | NM_002764 | CCCAAGGTCTATGCTAAATTA CACCATCTGCTTGACTATGTA AACATGCTTCCTGCTATGTAA TTGATTAATTTAAGTATTTAA |
| 5639 | PRRG2 | NM_000951 | CCCAGTGAGGAGACAGACCAA TTGGGAGAGCTACATCTACAA |
| 5653 | KLK6 | NM_002774 | CTGACCCTGATGCTTAATAAA CACGTTTGATTTCTTCCTGCA CTGCGAGATTTCTCACCTGTAA CACCATGCACTCAATAAAGAA |
| 5654 | PRSS11 | NM_002775 | CCGGTCAAAGTTGAGCTGAA CGTGAAGTTGATTGGAATTAA AGCGTATATATAGTGAATTAAT CGACGTCATAATCAGCATCAA |
| 5660 | PSAP | NM_002778 | CCGACATATGCAAGAACTATA AAGAACTATATCAGCCAGTAT CAAGCTGATTGACAACAACAA CTGCCTGAATGTAACCTGCTA |
| 5663 | PSEN1 | NM_000021 | CTCAGTTCCAAATTCAGTAA CTGAGGTTGCTTTATCCTAAA CAGGCATATCTCATTATGATT CAGCTCTCATTTACTCCTCAA |
| 5676 | PSG7 | NM_002783 | CCCATGGAACCTCAAAGAGCA ATCCTTGTACCTGATGTCTGA |
| 5682 | PSMA1 | NM_002786 | CTGCCTGTGTCTCGTCTTGTA TCCATTGGAATTGTTGGGTAAA CACAGTTGGTCTGAAATCAAA ATGGAGTGTAATTAAATGAA |
| 5683 | PSMA2 | NM_002787 | ATGCAAGAATATACTCAGTCA ATGAGGGACGACGACCATATTAT AAGGATTACTTGGCTGCCATA GAGATACATTATGTAAATAA |
| 5687 | PSMA6 | NM_002791 | CAGGGTGGCCTTACATCAGTA CACAGTGACTCACTTATTCAA ACCGAAGTGGTTAGGACTCTA CCAGGGATTGTGTTTAAAGTA |
| 5688 | PSMA7 | NM_002792 | AACAGATGATCTGACCATTAA GACCATTAAGCTGGTGATCAA ATGGATGAGTCTCGATGTGTA CACACTGAGTGTCCTACAATA |
| 5689 | PSMB1 | NM_002793 | CCGGATCTGCATAGTGACCAA ATCGGTGGACTTGATGAAGAA |
| 5690 | PSMB2 | NM_002794 | CGCACTCTTGATAAATGGTTA AAGATGCGAAATGGAATATGAA CAGCAATATTGTCCAGATGAA GTGACTATGGATATAATTGAA |
| 5692 | PSMB4 | NM_002796 | TCGCATTATGCGAGTCAACAA ATGCTTGGTGTAGCCTATGAA ACCATTGGTGTGCTGCCATA CACATGATCAGTGGCTTTGAA |
| 5694 | PSMB6 | NM_002798 | CGCCTGAATCCTGGGATTCTA TGGGATTCTCAGTATGCAATAA CTGGGATTCTCAGTATGCAATAA CTCCGGGAGCTCCTACATCTA |
| 5698 | PSMB9 | NM_002800 | CTGCAAAATGTGGTGAGAAATA GTGAGAAATATCAGCTATAAA AAGGAGGTCAGGTATATGGAA CAGCACCTTTATCTATGGTTA |
| 5699 | PSMB10 | NM_002801 | CCCAGACAGTGAAGCCACTAA CAGGCTATGGAGGTGGAGTAA CACACGGATGGTGGCGTCCAA CTGCGAGAAGATCCACTTCAT |
| 5705 | PSMC5 | NM_002805 | CAAGGTTATCATGGCTACTAA CCGGGTGGCTCTAAGGAATGA AAGGTACATCCTGAAGGTAAA TTGGTCAAGGTACATCCTGAA |
| 5706 | PSMC6 | NM_002806 | CTGAGAAATGTTTGTACTGAA AAGGAGTTAAGGGAACAATTA |
| 5707 | PSMD1 | NM_002807 | CAGGCTGTGAGTGAATCTTA TTGGAAGGCATCGTAAATAAA |
| 5708 | PSMD2 | NM_002808 | TAGCGAACACTTGACTCCAA CAGGGTTCCAGCGCCATACAA CTCCGGAGGGCTGTACCTTTA TGGGTGTGTTCCGAAAGTTTA |
| 5718 | PSMD12 | NM_002816 | ATGGGTTTAAATTAACATAA AAGGATTACATTCGAACACAA |
| 5723 | PSPH | NM_004577 | CAGAGACTCATAGCGAGCAA TAGGCTGAAATTCTACTTTAA TGCCAATAGGCTGAAATTCTA CCGGCATAAGGGAGCTGGTAA |
| 5727 | PTCH | NM_000264 | ACCAAGCTTCATTAGTCTTCA CCCGTCATTAGTCTCTTTCA TCGCACAGAACTCCACTCAAA ACAGCATACCTCCTAGGTAAA |

Table4

| | Gene | Accession | Sequence |
|---|---|---|---|
| 5729 | PTGDR | NM_000953 | AAGCATAATTTCTTACCCTAT TCCAGTATCTGAGATAATATA CCGGTGCAGCAATTCCACTAA CCAGTATCTGAGATAATATAA |
| 5733 | PTGER3 | NM_000957 | AACAAATATCGTAAACCTTA CGGCTATAGAGTATTCCATAA AAGGACCGATTTGAGCACTAA ATCGATACCATTATTCAAATA |
| 5739 | PTGIR | NM_000960 | TCGGGCGACAGGAGCCAGAAA CAGAAAGAAATGGTTCTCTCAA CCCATCCATCTCATTGTCTAA CCCAGGGTCCTGAATAAATAA |
| 5743 | PTGS2 | NM_000963 | TCGTATTGCTGCTGAATTTAA TAGGGTAGAAATCACCTGTAA CCCGCAGTACAGAAAGTATCA ACGCTTTATGCTGAAGCCCTA |
| 5745 | PTHR1 | NM_000316 | AGGGAAGCCCAGGAAAGATAA CTCCATTGTGCTCAACTTCAT CGCCGTGAGCATCTTCGTCAA CAGCATAATGGAATCAGACAA |
| 5746 | PTHR2 | NM_005048 | AACGCCTCTATGTAATGTATA CTGAATACGGTTAGAGTTCTA TGGCATGTGGGATCAATTAAA AAGGTGTTACTTAATAATAGT |
| 5747 | PTK2 | NM_005607 | CCGGTCGAATGATAAGGTGTA CCCAGGTTACTGAACTTAAA CACCTGGGTACTGGTATGGAA AACAATTTATGTTCACATTAA |
| 5754 | PTK7 | NM_152883 | ACGCCACAGCACAAGTGATAA CCGAGAGAAGCCCACTATTAA ATGGGACATGCTACCGTTGTA ACGGTTCGAGGTCTTCAAGAA |
| 5764 | PTN | NM_002825 | CTGGAGCTGAGTGCAAGCAAA CCTAGGCTTTGTATTCAATAA TACCGTAGGCTTTGTATTCGA TACATGATAAATAGTAGACAA |
| 5771 | PTPN2 | NM_002828 | CACAAAGGAGTTACATCTTAA TCGATTGAATTGTACTGGATA CCGCTGTACTTGGAAATTCGA AACTGTATTCATACATGTCAA |
| 5774 | PTPN3 | NM_002829 | CCGCTCATTTGCTGACTTCAA CCGAGAAATGCTGGTCACAAA CCGGGTATTATTGCAGGGAAA CAGGTTATTCAGCGGATAGTTA |
| 5775 | PTPN4 | NM_002830 | AGCACGTACCTTAGAACTCTA CTCCGTCATCAACACAAGCTA TCCGTCATCAACACAAGCTAA CTCCGAACAAATAGTAAATAA |
| 5777 | PTPN6 | NM_002831 | CCAGTTCATTGAAACCACTAA CCGCACCTCGTCCAAACACAA TAGGCCCTGATGAGAACGCTA CCGGAACAAATGCTCCCATA |
| 5781 | PTPN11 | NM_002834 | GCCGCTCATGACTATACGCTA GCGGTCCAGCATTATATTGAA CCGCTCATGACTATACGCTAA CAGAAGCACAGTACCGATTTA |
| 5782 | PTPN12 | NM_002835 | GTGGATCATGATAAACACTTCA CTGCTTGTAGACATGTTAATA TTGCAGGTTATCAGAGATCAA AAGCTTAATGAGGAAATATCA |
| 5787 | PTPRB | NM_002837 | CAGTATTAGTGGAGACTTAA TCGGGTGTATCAGACTAATTA CCGGTCGACTTTATCAAGTTA CCCGGAGATGTGGATAACTAT |
| 5789 | PTPRD | NM_002839 | ATGGCATTATCACCAAGTATA CGCCTTGTTAATATTATGCCA CTGGGTGTCATTGAAGCAATA ACGGCCAGTCCCGACAGTAA |
| 5794 | PTPRH | NM_002842 | TCGAAGCACAGCACACACTAA CAGGTACTGACATCACCCTAA CCCGGTGTGTATACGTGTT CCGGGACGTTGTACAATTTCA |
| 5795 | PTPRJ | NM_002843 | ACCCGTATCTTCTACAATCAA ACGAGTCGTCATCTAACTATA TCGGGTAGAAATAACCACCAA TCCGAGTATGTCTACCATTTA |
| 5796 | PTPRK | NM_002844 | CAGGATTTGTATCGCTGTGTA CCGGCGAGTCAAGTTATCAAA CTGGAGATTAGTGTATGATTA CCGGGTTAAATGCTATAAATA |
| 5798 | PTPRN | NM_002846 | CTGGTGAAGTCTGAACTGGAA CAGGAAGGTGAACAAGTGCTA CAGGTCTGGCTTGGCACCCAA CACCCTTCACTGAGTTACGAA |
| 5800 | PTPRO | NM_002848 | CAGCTTCAAAGTAGAGTAGAA CCGGGAAGACTTAGCCTTAAA | |
| 5801 | PTPRR | NM_002849 | AACCCTTTGTGTCTATACCAA TAGGTTTATCACACAGCCTAA TAGCCATATACCAATACCTAA ACGCTGGAATTTGGTAGTGAA |
| 5803 | PTPRZ1 | NM_002851 | CAGATCAATTACACTGAGATA CGCCCAAATTTATATCATTAA CAGTATATGATTAGAAATGAA CAGCACCAATGTTATTAATAA |
| 5810 | RAD1 | NM_002853 | TCGAATGTGTTACCAAGGTTA CTGCAGCACCAATGTTATTAA TCGGTCAGCCTCAGAATTCCA CTGGCTCTACTGCGTGAACAA |
| 5818 | PVRL1 | NM_203286 | CAGGTGGACAATATCACA CAGGTGAACGAACTCCAGTAT ACGCTGTATGACAGTATTAAT CCGGAGAAGTTACCAGATGTA |
| 5819 | PVRL2 | NM_002856 | CCCGTGACACTCAGGAGTTAA CAGATCATAGCCAAGACCCAA CTGGACGACACTGGAGTGGAA CAGGTCATCTTTGTCCGAGAA |
| 5830 | PEX5 | NM_000319 | ACCGATCGCTGGTATGATGAA CCAGTTCACAAGACCAGTAAA TACGTCAGCTACCTATGATAA AAGGTTGGTATTATCAGGAAA |
| 5837 | PYGM | NM_005609 | CACACTCGTAAAGACCGCAA CTACCGTGTCTTTAGAGTTCTA TCGGGATGTGGCCAAAGTGAA CAAGTTGAAGTTTGCTGCCTA |
| 5870 | RAB6A | NM_002869 | CAGATGGGTCATATTCTTTAT ATGGGTGTGATTGTCTTCCTA TACGGTCTTCTTTGAGGTCAA CCCACTTATTGTCACCTTGTA |
| 5871 | MAP4K2 | NM_004579 | ACGGTATTTAAGAGAGAACTA GAGAACTATATTGGTATTAAA TCGGTCAGCCTCAGAATTCCA CTGGCTCTACTGCGTGAACAA |
| 5876 | RABGGTB | NM_004582 | AAGGATGTTATTATCAAGTCA TACGCTGTATGACAGTATTAA ACGCTGTATGACAGTATTAAT CCGGAGAAGTTACCAGATGTA |
| 5878 | RAB5C | NM_004583 | GAACAAGATCTGTCAATATCACA CAGGTGGACGATCATCAGCTAA CCCGACTGGAATCCACTCTAA CACCATGATTCTCCATATAA |
| 5879 | RAC1 | NM_006908 | TACATTGTACAAGGAATGAAA GGGCATTAATTCATCTTTAA ACGATGTTCTGTACAACTTAA AAGGTTGGTATTATCAGGAAA |
| 5881 | RAC3 | NM_005052 | GACATGCTTGCTGATCAGCTA CAACGTAGCTGTGGACGGGAA CGCGCCCATGCAGGCCATCAA CCGGGAGATTGGCTCTGTGAA |
| 5892 | RAD51L3 | NM_002878 | CCCAGTAACGCCTGCTGTTTA CTCACTCTCTGTAAGCATATA CTGGGTGGAAATAAGCTTATA CTGGAAGAGGTAGCTCAGAAA |
| 5893 | RAD52 | NM_002879 | TCGGGTAATTAATCTGGCCAA CAGCTGAAGGATGGTTCATAT CAGGAGTGACTCAAGAATTAA AAGGAAGGAAATAATCATCTA |
| 5901 | RAN | NM_006325 | CAGCAGTATTCTATTTGGTTA TAGTGTGAAGTTGATACGCAA TTGGGAATTCTTGCTGTTAAA ATGTGGTTTGTACATATCAAA |
| 5902 | RANBP1 | NM_002882 | CTCGGTGAAGGAGGAGACCAA CGGCGCCAGACGCGGAGGGAA | |
| 5905 | RANGAP1 | NM_002883 | ACGGAAGATTCTGGACCCTAA AGCAAGAGCCTCAAACTCAA | |
| 5908 | RAP1B | NM_015646 | AATGTGTACTGTATACTTTA TAGCATCAGTAGTTCAATAAA GACGAGTACTGTGGATGTGAA CAGTATATAATGTCTTAGATTAA |
| 5909 | RAP1GA1 | NM_002885 | CCGGCTCATCGTCACCTTTGA CACCTTGTCTTCTCACTCAA | |
| 5911 | RAP2A | NM_021033 | AAGGCATAGTCCATCGATCTA ACCAGTGTATTCCGTCAAGTA CCAGTGTATTCCGTCAAGTAA CAGAGATAATTGCCTACTACTA |

128

Table4

| | | | |
|---|---|---|---|
| 5912 | RAP2B | NM_002886 | AAGGTGGTCTTAGTAATATAA TAGAATAGAGGCAAAGCCTAA TACGGTTCTTCGTAGAACAA CTCACTTAAGTTTGATATCAA |
| 5919 | RARRES2 | NM_002889 | CTGCCGGAAGAGGGACTGGAA CCCAATGGGAGGAAACGGAAA CAGGGAGGAGGAACCCGTTCTA CTGGAAGAAACCCGAGTGCAA |
| 5924 | RASGRF2 | NM_006909 | AAGGTGAAAGATGGCTATTTA AAGAAGGAACACCAAACTTTA |
| 5926 | ARID4A | NM_002892 | CTCGAGATGTAAATTCTATTA ATGGCTTATCTAAGACAGCAA CTGGTTTGACATTGCTAAGTA ACGATTGAAGTTGATAGTATT |
| 5927 | JARID1A | NM_005056 | CGGCAAGTAAAGAAATCTGCTA GCGGACGTTTCTTAAGAAGAA CAGATGCTTAATTGAGCTCTA AAGGAATGGTATCACAGTCTA |
| 5930 | RBBP6 | NM_006910 | GACGCTATCATTCACGATCAA CCAGGTCTAAAATCTCCCTATA ATAGTGGTTCTTCGTATTCAA CCCAGTTAAGTCATTCCTCTA |
| 5933 | RBL1 | NM_002895 | CTGGATGTTTCAAATGAGTTA CTCCAGGTTTCTGCAAACAAA CACCAAGTGACCAACTTATAA ACAGGCTAATGTGGAGTATAA |
| 5935 | RBM3 | NM_006743 | CAGGAGGAAATTACAGAGACA CCAAATGGCGTGATTTATAAA |
| 5950 | RBP4 | NM_006744 | CTGGTACGCCATGGCCAAGAA CCAGATGAGCGCCACAGCCAA CCCTGCCAAGTTCAAGATGAA CTGGATCGTCGACACAGACTA |
| 5961 | RDS | NM_000322 | CAGCACCACCACCATCCCTAAA CACGGTGAAAGTGCTGCTCTA GAGGAGCGATGTGATGAATAA CACGGATTTAGTCCCACCCTA |
| 5967 | REG1A | NM_002909 | CCCAGTCTTTGGAACCCTAAA CTCAGTCTCTAGAGCCCTGAA |
| 5968 | REG1B | NM_006507 | CTGCAATTACTATGAAGTCAA CTCAGTCTCTAGAGCCCTGAA |
| 5971 | RELB | NM_006509 | CACAGATGAATTGGAGATCAT CAGCTACGGCGTGGACAAGAA GCGGATTTGCCGAATTAACAA CCAGAATTGCCATTGTGTTCAA |
| 5976 | RENT1 | NM_002911 | ATCAAGGTCCCTGATAATTAT CACCATGAGCGTGGAGGCGTA CATCTTATTCTGGGTAATAAA ACGGAGCGCTGCACACCGAAA |
| 5977 | DPF2 | NM_006268 | ATCCATCTTGCCTCCAATTTA CTGTATTCTAAACATTAGTAA CTGGATGACCTCGATGATGAA CTGGACCTGTTGAAAGAGAAA |
| 5992 | RFX4 | NM_002920 | CAGGCATTACCTGACAGCTTA CTGGAGGAACGTGGACCTGAA |
| 6000 | RGS7 | NM_002924 | TTGATAAAGAACTTAACTATA TGCCTGGATGTGTAAATACAA AAGTATATATCAATAAGTATA ACGGATCATCTTGTAGCATGA |
| 6003 | RGS13 | NM_144766 | CCAGATTTCTAAAGTCAGAAA CAGAATTTCTAGGGCCAAAGAA CACAGTGACGAGAATATTCAA CCAGAGTGCCATCGAAGGTAA |
| 6015 | RING1 | NM_002931 | CCGAAAGAAGCTGGTGTCCAA CAGGGTCAGATCAGTGATGAA CTGGAGCTGGTGAATGAGAAA CAGCCAATAAGAGGACACAAA |
| 6039 | RNASE6 | NM_005615 | CCCTGTGTTCACAGCAATAAA CACTTAGATAGTATTCTCTAA |
| 6041 | RNASEL | NM_021133 | GCCAATGTTAATTTCCAGGAA CCCGAAATGTCCTGTCATCTA CACCTGGACATGTCAATTAAA |
| 6051 | RNPEP | NM_020216 | CACGCCTGCTGTTAAATACAA CCGGAAGCATCTTAGCCGATGA CTGGACATGAAGGCCATTGAA CACCAGGAAGATTTCTGGAAA |
| 6059 | ABCE1 | NM_002940 | ACCCATAAATACATGTTGTAA ATGGCTAGAAAGTATGGTTTA CCAGTTGGGTTCTAAATTGTA CTGATTGACATTTGATAAATA |
| 6097 | RORC | NM_005060 | TAGGATGTGCCGGGCCTACAA CCGGGCCTACAATGCTGACAA AAGGAGCTCTTCAGCACTGAA CGGGTTCATCTGAGTAAACAT |
| 6100 | RP9 | NM_203288 | TCTGTTGTTGGAATAATAAA AAGGAAGATGAGACTAAGCCA |
| 6103 | RPGR | NM_000328 | CCCGGTAAATTCTGGTTTAAA TCCGAAGGGCAAATTGGTTTA CACAGCTTTGATAACAGATAT ATGAGCCTGAATTCCAATGAA |
| 6122 | RPL3 | NM_000967 | CGGCAAGCTGATCAAGAACAA TGGCGTGATGTCCACAGAAA |
| 6125 | RPL5 | NM_000969 | TTCGTGTGACAAACAGAGATA AAACTTATGAACAGCAACTAA |
| 6128 | RPL6 | NM_000970 | CAGCGCAAGATTGATCAGAAA GACCGGAAGCCGGGACCTTAA |
| 6129 | RPL7 | NM_000971 | CTGGTTGGTTAATAAACAGTA ACGCTTCAAAGAGGCAAATAA |
| 6130 | RPL7A | NM_000972 | CACCTTGGTGGAGAACAAGAA ACAGGTGAACTCGGAAGACAA CACCACCTTGGTGGAGAACAA CAGGTGAACTCGGAAGACAAA |
| 6132 | RPL8 | NM_000973 | CTCAATAAAGTTTGTGTTTAT CCGGGCGTACCACAAATATAA |
| 6133 | RPL9 | NM_000661 | CAGGCTGATGAATAAGATCTA CGGACTATTTGTAGTCATGTA |
| 6134 | RPL10 | NM_006013 | CCCGGTTTACCGTATTGTA GCAATGGTGAATGCCTGCAATCCCA TGGCCTTATGTCAGTTGTCTA CAGAAACAGGTTGCAACTCA |
| 6136 | RPL12 | NM_000976 | ATCCGGGTTCATCCGACACCA AACATTTCAATAAAGGATCAT |
| 6144 | RPL21 | NM_000982 | CCACATATATGCGAATCTATA CTGGAAGAGTCTACAATGTTA |
| 6152 | RPL24 | NM_000986 | AAGAGGAATCAGAAACCTGAA AAGGCTAAGCAAGCATCTAAA |
| 6154 | RPL26 | NM_000987 | CAGGTTGTACGTGGACACTAT CAGGAATAAAGTAATCTTATA |
| 6156 | RPL30 | NM_000989 | CAGGAAGATGGTGGCCGCAAA CTGAAATAGAGTACTATGCTA |
| 6157 | RPL27A | NM_000990 | AGGGAGTTTCATTAAATGCTA CCGGATCAACTTCGACAAATA |
| 6160 | RPL31 | NM_000993 | CAGATCAAATAAAGTTATAAA ATCGCTGATCGTCAGATCAAA |
| 6164 | RPL34 | NM_000995 | AAGCACAGAGTCAGAAAGCTA CAGTATATGATCACTAATATA |
| 6165 | RPL35A | NM_000996 | CCGAGATGAAACAGAATTCTA GCGCTTATGTATGTATAAAGCAA |

Table4

| | | | |
|---|---|---|---|
| 6170 | RPL39 | NM_001000 | CTGAAGGTCACGATCATGTTA TTGGTTGGCTGGATTCAGTAA |
| 6173 | RPL36A | NM_021029 | CTGGTTGAGTTCACATCATAT AAGCTAGAGCCATCAATACAA |
| 6176 | RPLP1 | NM_001003 | CTGCATCTACTCGGCCCTCAT AAGAAAGTGGAAGCAAAGAAA |
| 6181 | RPLP2 | NM_001004 | CAGCGTGGGTATCGAGGCGGA CAGCGCCAAGGACATCAAGAA AAGGAGGAGTCTGAAGAGTCA CAAGGTTATCAGTGAGCTGAA |
| 6187 | RPS2 | NM_002952 | CACCTTTGATGCCATTTCTAA CCGCTTGGTCAAGGACATGAA |
| 6188 | RPS3 | NM_001005 | CGGCAAGATGGCAGTGCAAAT GAGGTTGTGGTGTCTGGGAAA |
| 6189 | RPS3A | NM_001006 | CCGGAAGAAGATGATGGAAAT CACCTGCTATGTTCAATATAA |
| 6191 | RPS4X | NM_001007 | CAGATCTTTGTACGTAATTAA AAGAATTGGTGTGATCACCAA |
| 6193 | RPS5 | NM_001009 | CACCGATGATGTGCAGATCAA CTCGAACTCCTATGCCATTAA |
| 6194 | RPS6 | NM_001010 | ATCCAGTCAGAAATAAGATTT TTGAGTAACAAATAAATAAGA |
| 6196 | RPS6KA2 | NM_021135 | CTGGAACACGCTGTACCGGAA CTCGATATCTGACGCAGCTAA CCGGAGGTCCTGAAGCGTCAA CCGAGTGAGATCGAAGATGGA |
| 6202 | RPS8 | NM_001012 | TTGGAGTTCTATCTTAGGAAA TCAGAGTGTTGTACTCGTAAA |
| 6203 | RPS9 | NM_001013 | ACGGCGTCTGTTCGAAGGCAA CCCGCGGAGACCCTTCGAGAA |
| 6204 | RPS10 | NM_001014 | CGCAGAGATGTTGATGCCTAA ACCGCGCATGCTCCTTCCTTT |
| 6205 | RPS11 | NM_001015 | CCCTGCGTAATCGATAAGGAA CGGGAAGATGGCGGACATTCA |
| 6206 | RPS12 | NM_001016 | AAGGTTGATGACAACAAGAAA AAGATTCAACTTCACCCGTAA |
| 6208 | RPS14 | NM_005617 | CAGGAGGAAATAGGACCAAGA CCGAGATGAATCCTCACCATA |
| 6217 | RPS16 | NM_001020 | TACCAGAAATCCTACCGATAA CCCTCGTCGCTGCGAGTCCAA |
| 6218 | RPS17 | NM_001021 | CCGCAACAAGATAGCAGGTTA AAGCGAATTCAGAGAGGCCCA |
| 6223 | RPS19 | NM_001022 | TGGCTCCATGACCAAGATCTA AAGAAGCATTAGAACAAACCA CTGGGTTAATAAATTGCCTCA AACAAGAAGCATTAGAACAAA |
| 6227 | RPS21 | NM_001024 | TGGAATATTTGTCATAAATAA CGGCGAGTTCGTGGACCTGTA |
| 6229 | RPS24 | NM_001026 | CGCAAGAACATTAATAAACTA CAAGAACATTAATAAACTAAA |
| 6233 | RPS27A | NM_002954 | TTGTCTGACTACAATATTCAA CTGGCTGTCCTGAAATATTAT CTGACTTACTGTTTCAACAAA AAGGTGGATGAGAATGGCAAA |
| 6234 | RPS28 | NM_001031 | AGCGTTTGTGTTTCAAGTTAA CTGAGATGCTCCTTTAAATAA |
| 6235 | RPS29 | NM_001032 | ATGATAATTCTTTGTATATAA CCGGCACGGTCTGATCCGGAA |
| 6240 | RRM1 | NM_001033 | CCGAGATTTCTCTTACAATTA CTGAGAGTATATAACAACACA AACGGATATATTGAGAATCAA CTGGTGGGTCTCTAGAAGCAA |
| 6272 | SORT1 | NM_002959 | CAAGGACAGGGTACAAACTTA CTGGGTTTGGCACAATCTTTA CCCACGTCAGTTACACATTAA CAGCAGAGAATTGACTAGATA |
| 6284 | S100A13 | XM_371380 | CTGAAGATCAGGAAGAAGTAA ACCGCACTCTTCCCAAATAAA CAGGAAGAAGAAAGACCTGAA AACCGCACTCTTCCCAAATAA |
| 6286 | S100P | NM_005980 | CAGGAGGAAGGTGGGTCTGAA TAGGCTGAGCCTGCTCATGTA |
| 6289 | SAA2 | NM_030754 | TAGGCATCTAATAAATGCTTA GCCGATCAGGCTGCCAATAAA ATGGCATATAATAGGCATCTA AAGCTGAGATATGGCATATAA |
| 6293 | VPS52 | NM_022553 | TCCATTCGGGATTATATTCAA CCGGGTCAAGGCAGTGACGAA |
| 6294 | SAFB | NM_002967 | CCGAAGATGACTCGGATACAA AAGGTGGTAATCCTGACGAAA CTGCCATATTGTAGCTCAATA ACGGACTGTAGTAATGGATAA |
| 6296 | SAH | NM_005622 | CCCTCAGAATTTCTCCAACTA CAGAAGCCTGTTCCCTACAAA CAAGCTGATTGTATCAGAGAA ATGCTTGTACAGAATGATATA |
| 6297 | SALL2 | NM_005407 | TAGGAAGTAGGGAGAAATGAA CGGCCTGAGGGTCACAATAAT CCCGGGAATGTCTCGGCGAAA AAGCTGGGCACTGCTTACCAA |
| 6300 | MAPK12 | NM_002969 | CGGCGCTAAGGTGGCCATCAA CACAGGCAAGACGCTGTTCAA TGGAAGCGTGTTACTTACAAA CTGGACGTATTCACTCCTGAT |
| 6301 | SARS | NM_006513 | CCAGTTTGATGAAGAACTTTA CTGATGAAACCATGTAATAAA |
| 6304 | SATB1 | NM_002971 | TGGGTACGCGATGAACTGAAA CACTCTTATTGTTGTATGCAA ACCGAATATACCAGGACGAAA TCCAAGCAATTCATACTATAA |
| 6305 | SBF1 | NM_002972 | GACGCCTGTGTTCCACAATTA TCCGGTCGTTACCGACCACTA CCGCGTGGTGTGGCCCTGTTA CAGCGCCGAGCTCTTCCGTAA |
| 6311 | ATXN2 | NM_002973 | ATGGATCAACTACTAAACAAA CTGACGGATTATTATTTATAA |
| 6317 | SERPINB3 | NM_006919 | AAGGTAATATTGGCAGCAATA CTCGTGCTATCTGGAGTCCTA TAGGATTCGGATCATCACCTA TCGGATCATCACCTACTTCAA |
| 6324 | SCN1B | NM_001037 | CCACATTGAGGTAGTGGACAA CACATTGAGGTAGTGGACAAA CAGAGATGATTTACTGCTACA ACCGCCATGCATGATGGGTAA |
| 6328 | SCN3A | NM_006922 | CAGCGTAATTTCAGATGTTAT AAAGCTGATAGTCTATGTCAA TTGGATTTATGTGAGGTCAAA CTCCCATAATAAATTATATAA |
| 6329 | SCN4A | NM_000334 | GACGCCTACATCAGTGATGAA AACGCTGAACATGCTCATCAA CAAGATTGCCTTGGACTGCAA CTGGCTCAATGTCAAGGTCAA |

130

EP 2 295 543 A1

Table4

| 6331 | SCN5A | NM_000335 | CCCGCTGTGGATGTCCATCAA CTGGAGCACTACAACATGACA CTGGACCAAGGTGAAAGTCAA TCCTGTAAGAGTATTAACAAA |
| 6332 | SCN7A | NM_002976 | TCCAATGAGTAAACAAACTAA CCCAGCATTATTGAACATCAT AGAGATGTTCATGCTACTAAA CCGATGGTAATTAAGCTTGTA |
| 6336 | SCN10A | NM_006514 | CGGGCTCTTTCTCGATTTGAA TCCCGGGAGGTCAACATGCAA CACAGGCGAATGTGTCATGAA CCGCTCCATGGCACTCTCTAA |
| 6337 | SCNN1A | NM_001038 | CTGCACCTTTGGCATGATGTA CAGCCTCAACATCAACCTCAA TACAGGTACCCGGAAATTAAA CCCGATGTATGGAAACTGCTA |
| 6338 | SCNN1B | NM_000336 | CCGGAACTTCACGTCCATCTT CACGTCCATCTTCTACCCTCA TCGGCCAACCCTGGAACTGAA CTGCAATGACACCCAGTACAA |
| 6339 | SCNN1D | NM_002978 | CTGCAGGGAGTCTGCATTCAA CCGCAATGGCTGAGCACCGAA CTGCAGGGAGGCAGAAGGCAA CACACTTGGGCTGCTCTGAAA |
| 6340 | SCNN1G | NM_001039 | CGGCGGTAGCATCATTCACAA AAGAGTGGACACTAACCACAA CAAGGCCGGCAAGTAAACAAA ATCCCGGGACCTGAACTATTA |
| 6343 | SCT | NM_021920 | CAGCGAGCAGGACGCAGAGAA CTGGCGCTGCTGCAGAAGGAA ACCAATAAAGGAGGAATCAGA GTGGTACTTGGCACCAATAAA |
| 6344 | SCTR | NM_002980 | CACAGGATGGCTGGTCAGAAA CAGAAAGGGACAGGGAAATAA CTCCTTCTTCTCTGAAAGAAA CCGGATGCTCACCAGCAGAAA |
| 6354 | CCL7 | NM_006273 | TTGGGATTAATACTTCAACTA TTGGATGTATATGTCATCTCA TGGAAGCATATTGTTATTATA AAGATTTGCTTTAATTGTTAA |
| 6367 | CCL22 | NM_002990 | CTGGGTGAAGATGATTCTCAA TACCTTTGACTTGGTATTATA TAAGCTGGAGTTATATATGTA CAGGTAAAGGACATAAATGTT |
| 6368 | CCL23 | NM_145898 | GACAGAGTTCATGATGTCAAA TAGAGCAATTCATCTAATAAA CAGGAAGAATTGAACTTGTCA CTCCTGGAGAGTTACTTTGAA |
| 6369 | CCL24 | NM_002991 | CTGCATGTTCTTTGTTTCCAA CAGGCCTGATGACCATAGTAA CAGAGATATCCTGGCAACCAA AGGAGTGATCTTCACCACCAA |
| 6376 | CX3CL1 | NM_002996 | CCACGGTGTGACGAAATGCAA CAGCAAGATGACATCAAAGAT CGGAGCTGTGGTAGTAATTCA CACCATCAAGCCACCAACATA |
| 6382 | SDC1 | NM_002997 | TCCGACTGCTTTGGACCTAAA GCCGCAAATTGTGGCTACTAA CAGGGCCTCCTGGACAGGAAA CAGGAGGAATTCTATGCCTGA |
| 6383 | SDC2 | NM_002998 | GACGCTGAATATACAGAACAA CCGTTTAGTCATATCTATCTA ATGAATAGACATAAATTGTAA AAGGTACGAGTAGAGGTTTAA |
| 6386 | SDCBP | NM_005625 | AGAGATTGTGATGATTCTTAA GAGATTGTGATGATTCTTAAA TTGACTCTTAAGATTATGTAA TGGGATGGTCTTAGAATATTT |
| 6396 | SEC13L1 | NM_030673 | CAGATTGATCATCTGCCTTAA TCGGAGATGCTTTGTAATCTA |
| 6400 | SEL1L | NM_005065 | CTGCAAATTGGGCGTCGTCTA AAGAGTGAACATGTACACAAA CAGGATATTCACCTTGCGAAA ACGGCACAAGGAAGCATTGAA |
| 6401 | SELE | NM_000450 | CATGGTAGAATTGGAGAGTAA ACCAGTGTGGTTTGTGTTTGA CGGAAAGTATTTCAAGCCTAA CAGGTTGAATGCACCACTCAA |
| 6404 | SELPLG | NM_003006 | TCCATGGAACCTACTACCAAA CAGCAATTTGTCCGTCAACTA ATGGAGATACAGACCACTCAA ACGGAGGCACAGACCACTCAA |
| 6406 | SEMG1 | NM_003007 | AGGGTAGTTATACACCATAAA CAGGGTAGTTATACACCATAA |
| 6423 | SFRP2 | NM_003013 | TAGCATGATTTCTTCAAGTAA TACATAGTAGTTTACCTTTAA |
| 6425 | SFRP5 | NM_003015 | ACGAATGTTGTTACTGGCACA TAGGCTAAGCTATTTGGGAAA CTGCGAGGAGTACGACTACTA CCGCTGGGACAAGAAGAATAA |
| 6428 | SFRS3 | NM_003017 | CACAATATGGTTCAAATGTAA CAGACTGATAATAAACCTCTA |
| 6434 | SFRS10 | NM_004593 | TCGGGCAAATCCTGATCCTAA CCCAATTTCACTCAAATGTTT CGGGACTACTATAGCAGATCA CCCGATCTGAATCTAGGTCTA |
| 6435 | SFTPA1 | NM_005411 | CACAGCAGGGATGAGGACAGA ACAGATGGTGTGAGTCAGTGA |
| 6439 | SFTPB | NM_000542 | AACATGGAAGTCAACGTTTAT CCCACCATCTCTGGTCTACAA ACGGCTTGCACCATTCAGCTA AAGGGACGAGATCGTTAGCTA |
| 6441 | SFTPD | NM_003019 | TGCTGCCAATATCCTAATAAA TAGGATTAGGATTGTTGTGAA CTACCTGGAAGCAGAAATGAA AGGAGTTTGGCCAGAAGTCAA |
| 6443 | SGCB | NM_000232 | CTGCTTCGATTTAAGCAAGTA ATGGGTGGTAATATGGAGTTA ATGCAGATTTCTAGTGCATTA CACTTTGAATTGTGCACATAA |
| 6452 | SH3BP2 | NM_003023 | CCCACGGACAATGAAGACTAT AACCTCTAACAAAGTGAGGAA TAGCACCTTATGTCAGGGAAA TAGGAAGTAAACGATCTGCAA |
| 6455 | SH3GL1 | NM_003025 | AAGGTGCTCTTAGAACACTAA CCGCCTGGACTTTGACTACAA ACGGTCCAGGGCAAACTGAAA ACGCCCTAAGCGGGAGTATAA |
| 6468 | SHFM3 | NM_022039 | CCGGGAAGGAGGCACAAGGAA ACGAGTGAAGGTGTCTCAGAA TGGGAAGATTGGCATTCATAA CAGCACCTTCACTGTCAAGTA |
| 6472 | SHMT2 | NM_005412 | CAGCAAATTCAATTTGTTAAA CCGGAGAGTTGTGGACTTTAT |
| 6487 | SIAT6 | NM_006279 | CACAATATCCAGCGAGAGAAA TCGGTTGGGCTTCCTCCTGAA |
| 6489 | SIAT8A | NM_003034 | CTGGCTTAATAATATTAATAA CAGAAAGACATTTGTGGACAA |
| 6492 | SIM1 | NM_005068 | CACAGAAAGATCGGAATCTGA ATCCAGTTTGATCCTAGCTAA CACACTAAACTTCTTAACGAA TCACCTGGTATCCATTATAAA |
| 6493 | SIM2 | NM_005069 | CACGGGCAACAGTATTTATGA TAGCAGCTCGTCTCCAGCTAA CCGGCAAATGCGCACGACCTA CCGTGATTCTTGAAAGGTGTA |
| 6497 | SKI | NM_003036 | CTGGACGACGTGAAGGAGAAA AACCAGTAAGGAGACTTGAAA GCGAGTGTTTGTAACCATGTA CCGACTTTATAGAGCAGTCAA |
| 6502 | SKP2 | NM_005983 | AGGCCAACTATTGGCAACAAA CTCAAATTTAGTGCGACTTAA ACCCTTCAACTGTTAAAGGAA AAGTGATAGTGTCATGCTAAA |
| 6503 | SLA | NM_006748 | AAGGAACTTCGTGACACTAAA CTGGCTGGATCGGGTAGGTAA CTGGATCGGGTAGGTAAGAAT CCCGGTACTTTATTCTAAGAA |
| 6504 | SLAMF1 | NM_003037 | CTCCGGCAGTTGGGAAGCAAA CAGGACCCTTGCACCACCATA CAGAGTCTGTCCAGGAAACAA AACAAATTCCATCACAGTCTA |
| 6510 | SLC1A5 | NM_005628 | CTGGATTATGAGGAATGGATA CCGGTCCTGTACCGTCCTCAA CAGCCTTTCGCTCATACTCTA CACGCTGCCGCTGATGATGAA |

Table4

| | | | Sequences |
|---|---|---|---|
| 6512 | SLC1A7 | NM_006671 | CCGGAACATGTTCCAGCCAA CACGTTAAGGTCAAGACACTA |
| 6514 | SLC2A2 | NM_000340 | TGGGTTATCAGTTAACATTAA ATGGTGTGTTCCTAACATAAA CAGACTTTAGCTTACAGTCAA CAGGGTGCTAACGTATCTCTA |
| 6517 | SLC2A4 | NM_001042 | CAGGATCGGTTCTTTCATCTT AAGGGCCATGCTGTCAACAA ACGCTGCTGCCTCCTATGAAA CACCTTGGACTCCTCCCACAA |
| 6523 | SLC5A1 | NM_000343 | AACCATAGTGTCTGATGGCAA TTGGGATTTCACGTATGATTA AAGGAGACCATTGAAATAGAA GAGGACAGTGTTGAACGTCAA |
| 6525 | SMTN | NM_006932 | CACATTCACCATCGAGATCAA CAGCACCATGATGCAAACCAA |
| 6528 | SLC5A5 | NM_000453 | CGGAATTATCTGCACCTTCTA ACCCAGGAAACCTGTGATTAT CAGCTTCTATGCCATCTCCTA CTGGAATTGCCTTGTATGCAAA |
| 6529 | SLC6A1 | NM_003042 | CAGCAAGGAGGCCTACATCTA ATCCTTAATAACTATGGTAAA CACGTAAGGTCTCATTTGATA CGGGCGGTTATGTAAGTTTCTA |
| 6534 | SLC6A7 | NM_014228 | CCCGCTCTCCAAGATGAAGAA TTGGTTCATTGTTAATAAATA CCTGGTGTATAGCATCGTCAA CACGTTTCACCAGAACATCTA |
| 6541 | SLC7A1 | NM_003045 | CAGCACCCAATAGACTATTTA ACGGATCTGGATATACACTAT ACGCTTATGACTCCTAATGTA GAGGGTTGGTTTATTATCAAA |
| 6543 | SLC8A2 | NM_015063 | CACGGTGGATAAACTCATCAA CTGGAATGAGACGGTGTCCAA |
| 6550 | SLC9A3 | NM_004174 | CAGCACCGACAACGTGGTCAA CCCGTTCATCTGGACCTGGAA TGCGCAGAATTTCACCATCAA CACCACCATCATCGTAGTGTT |
| 6554 | SLC10A1 | NM_003049 | ACGGAGTCAGCCGGAGAACAA CACCATAGGGATCGTCCTCAA AGGGATCGTCTCCAAATCCAA CACAAGTGCTGTAGAATTAAA |
| 6558 | SLC12A2 | NM_001046 | CAGGATTGTCTACTTCAGCAA AACGTTAATGACACTATCGTA CAGGATTACACCACTAATTT CAGAGTATTCATTGGTGGAA |
| 6559 | SLC12A3 | NM_000339 | ATCGCTGAGCTCAACACCATA CCCGGCCACAGTGAAGACTA CAAGAAGACCATAGACATCTA CAGGATGGACCAGGAGAGAAA |
| 6560 | SLC12A4 | NM_005072 | CCGGGAGCTGGTGCACATTAA CCGGCATGATCTACAAATACA |
| 6570 | SLC18A1 | NM_003053 | CCGGATGTATGCAACCCAGAA CAGCATTGTCTTGCTGCCAAA |
| 6572 | SLC18A3 | NM_003055 | CACGTCTGGCATAGCCATGAT CACCATTGCCACGTGGATGAA CTGCTTGATGAGCCACGCCAA TACGGAGAGCGAAGACGTGAA |
| 6573 | SLC19A1 | NM_003056 | CAAGCGCAGCCTCTTCTTCAA CCGCAAGCAGTTCCAGTTATA |
| 6578 | SLCO2A1 | NM_005630 | AAGGAGGTCATTCATCAACAA AACCTTTATTCTATTATATTA |
| 6581 | SLC22A3 | NM_021977 | AAGGAACTTATGGGCATATTA ATCAATTATATCCATGCTTAA |
| 6591 | SNAI2 | NM_003068 | GAGGGAAAGATTAGCTTTGAA TACCACAAATGCAATAATACA CCGAAGCCAAATGACAAATAA CACACTGAGTGACGCAATCAA |
| 6601 | SMARCC2 | NM_003075 | AAGAAGTATATACAAGCTGAA CCGAAAGAAGATTTCAGCCAA |
| 6602 | SMARCD1 | NM_003076 | AAGCACTGTGCAATATATTA TCCAAATATGATGCCACTAAA |
| 6604 | SMARCD3 | NM_003078 | GTGGCAGTATGTGAAGACCAA CAGGACTCCCATGACAAGGAA CTCAAGGTGATGACAGATGTA CAGTCGCTACTTCTCTACTGCAA |
| 6610 | SMPD2 | NM_003080 | CCGCATTGACTACGTGCTTTA CAGGAGGTCAATGGCTTATAT |
| 6616 | SNAP25 | NM_003081 | AAGATGCTGGGAAGTGGTTAA CAGAGCTCACGTTGCATTGAA AAGGTTGTACATAGTGGTCAT |
| 6623 | SNCG | NM_003087 | ACCCACCATGGATGTCTTCAA AAGACCAAGGAGAATGTTGTA |
| 6624 | FSCN1 | NM_003088 | AACTGGAAATAGCGAAATAAA CACGGGCACCCTGACGCCAA |
| 6625 | SNRP70 | NM_003089 | CGAGAGTGAATTATGACACAA CTCCGGAGAATGGGTATTTGA CCGGAGAGAGTTTGAGGTGTA AAGATTGAGCGGCGACAGCAA |
| 6627 | SNRPA1 | NM_003090 | TAGCCTTGTTGTGTTAGCAA TCCACTCACTATGCTACCAAA CAGCATTGTTGAAATGCTTAA AGCCTTGTTTGTGTTAGCAAA |
| 6628 | SNRPB | NM_003091 | ATGAAACTGGTTTATAATAAA CTGGTTTATAATAAACTCTTA |
| 6632 | SNRPD1 | NM_006938 | AACGCTGAGTATTCGAGGAAA ACAGTTCTATGTACTGAGAAA TAGGCTAAACTTAACAATATA CAGAGCTGTCTATTTAGTATA |
| 6633 | SNRPD2 | NM_004597 | AACGGAGAGCGTAGTGACCAT CGCGGAGGAAACGGGAGTGAA |
| 6634 | SNRPD3 | NM_004175 | CACAGGAAGAGGACGTGGAA CAGCTCAGCTTAACAACACA ACCCATGTTAAAGAGCATGAA AAGGACTTTGTGTTCATTTGA |
| 6636 | SNRPF | NM_003095 | GCCAGTGATGGTGAAACTTAA AAGGTGTAATAATGTCCTTTA |
| 6638 | SNRPN | NM_003097 | CAGAAAGATCAAGCCAAAGAA TTGGATTTGGTGAACAGCAA |
| 6640 | SNTA1 | NM_003098 | CACCGTATTTCAAGAACTCTA CGGAACTTCAGCGAGCCAA |
| 6641 | SNTB1 | NM_021021 | CAGGAATTAAATAAAGCATCA TAGACTGAATGCTATAATCAA |
| 6643 | SNX2 | NM_003100 | AACCATCTAAATAAACCACCTA AGGGAAGAGATTGAAGAAGAA |
| 6649 | SOD3 | NM_003102 | CTGGAATGGATGGCACCCAA AAGGGCTGCTATGATTGAGAA CTCCTTCATCTCTTTAATAAT AACCTTTGTTGTTACAGATTAA |
| 6651 | SON | NM_003103 | ATGATGTTGATTTATCTTTAA CAGAAATCTTCAATTACGTTAA |
| 6653 | SORL1 | NM_003105 | CAAGACGGTCATTGTCAGTAA CACGTCCTTTCTTGACCTCTA CTGGGATTTATCGGAGCAATA TACGGACAGGTTAGTCTGAAT |
| 6654 | SOS1 | NM_005633 | TACGGCATTATGAAATTACAA CATGAACTTAGTGTAAAGTTA TGGGTTGAATCCATCACTAAA ACGAGTAGAATCAGATATCAA |

Table4

| | | | |
|---|---|---|---|
| 6656 | SOX1 | NM_005986 | CAACGTTAAGATGAAATTATA TTGGGTTGGTTTGTTAATTTA |
| 6659 | SOX4 | NM_003107 | CCGCGAGAAACTTGCATTGGA TCCTTTCTACTTGTCGCTAAA CACGGTCAAACTGAAATGGAT AAGGACAGACGAAGAGTTTAA |
| 6662 | SOX9 | NM_000346 | ACCGAAGAAAGAGAGGACCAA TCGTGTGATCAGTGTGCTAAA ATGGGAGTAAACAATAGTCTA CAGCCCACTGACAGACCTTAA |
| 6675 | UAP1 | NM_003115 | AAAGGTTTCTATGGTACTAAA TTAGAGCATTATGAACATTAA |
| 6683 | SPG4 | NM_014946 | CACGATGCTAGTAGACGCCTA TGCCCTTAGTTTACTGGTTAA AACGTTATTGATACTTGGATA TCAGTTAATACTATCTTAAA |
| 6693 | SPN | NM_003123 | TTCAATAACAAGTGACCCTAA ATGGTTCATATAGGACACACA AAGGGTGTTTGTAGAGTTGTT CCCAATGGCCTTATCATCTA |
| 6697 | SPR | NM_003124 | CAGGAAAGGCTGCTCGTGATA CACGTGGACTTCTATGACAAA AACTGCTGAGCTTACTGGAAA AAGGAAAGGCTGCTCGTGATA |
| 6699 | SPRR1B | NM_003125 | GAGACTTAAGATGAAAGCAAA AAGGTCTTAGTACCAGAGCTA |
| 6700 | SPRR2A | NM_005988 | ATGATGATCCCTGACAGCAAA ACCAAGAAAGGATAAGGATAT |
| 6710 | SPTB | NM_000347 | CTGCCGCATCACCGATCTCTA ACGGCCCGCAAGGACAATATA CACTCTCTGGATCAACGACAA CAGGATGTCTCCTATGATGAA |
| 6712 | SPTBN2 | NM_006946 | CCGGCTCATCTCGGACATCAA CAGCGTCAACATCCTGCTCAA |
| 6715 | SRD5A1 | NM_001047 | CTCCGGAAATTTGAAGAGTAT CGGGCATCGGTGCTTAATTTA ATCCATTCAGATACATATCCTA GAGGCTTATTTGAATACGTAA |
| 6717 | SRI | NM_003130 | CAAAGAATACTTCCACACATAA TACATTATAAGCTTAACTATA CAGGCTGTGAATTCAATTGCA AGGATTCACCTTAGAAGTTTA |
| 6718 | AKR1D1 | NM_005989 | CAGGGTCCTCCAGCTAGATTA ATGGCAAATGGTTATATCACA CTGGGTAGTAGCGTTGGTAAT TAGTGTTACTTGGATAGCTTA |
| 6729 | SRP54 | NM_003136 | ATGGGATTCAATAATATATGTAA ATGGATAGTAGTATGAATGATCAA |
| 6732 | SRPK1 | NM_003137 | CACCCTTGGCTTAACTCCTAA AAGCAATAGACTAGAACTGAA TACCATGTGATCCGAAAGTTA CAGACTCTTGTACACCTATAA |
| 6737 | TRIM21 | NM_003141 | CAGCGTTGAGTTGCCCTGTAA AGCGTTGAGTTGCCCTGTAAA CCGGCAGCGCTTTCTGCTCAA CAGAGCATACCTGGAAATGAA |
| 6738 | SSA2 | NM_004600 | CAGGATGGATATGTATGGCAA CTGAGGGAGTATCGAAAGAAA |
| 6746 | SSR2 | NM_003145 | CAGGTGCTTTCCAGACTCCAA CCCAGTGTCTATTCTGGGTTA CACCTCCGCAACAATTACTTA CAAAGGGTATCTTAAATGCAA |
| 6750 | SST | NM_001048 | TGCAGAGATCTGCTAACTCAA CAGCGAGACCCTTGCATTAGA CCCTTGGTGGTGCCTATTAA ACGGAGAATGATGCCCTGGAA |
| 6751 | SSTR1 | NM_001049 | CTGGTGGTGCCTATTAATCAT TGGGAACTCTATGTCATCTA CGCGCTGGTGCCTATTAA CACGAACTCATTAACAACTCA |
| 6755 | SSTR5 | NM_001053 | CACCGTCAACATCGTCAACCT ACGGGCTTATGCAGACCAGCA CGGGAACACGCTGGTCATCTA |
| 6757 | SSX2 | NM_003147 | CCACCTTTCATGTGTAATAAA AGGGCAGTTGCTGGTGTTTAA TTGGTCGATTCAGTTTGTCAA |
| 6775 | STAT4 | NM_003151 | TGGAATCAAGTCCAACAGTTA TCAGAGACCGTTGGTGCTTAA TTGGTACAACGTGTCAACCAA CACCCTCGATTTCAAGAGAAA |
| 6778 | STAT6 | NM_003153 | CACCCTTGAGAGCATATATCA AACCAAGACAACAATGCCAAA CAGGATGGCTCTCCACAGATA ACGGATAGGCAGGAACATACA |
| 6788 | STK3 | NM_006281 | CAGACCATGATTGAACATAAT CAGCTGCATACCAGTAATAAA CGGGCGCCTAAGAGTAAACTA |
| 6790 | STK6 | NM_003600 | TAGCATGTGTGTAAACTTTAA GAGGGTTTAACTCCTATTTAA CACGTGCTCTACCTCCATTTA CACCTTCGGCATCCTAATATT |
| 6793 | STK10 | NM_005990 | TGCAGAATGTATATAACGCAA CAGAATGTATATAAACGCAATA CAGCTCAAACTGATGAAGAAA CACGGAATTAGAGAACCTGGA |
| 6794 | STK11 | NM_000455 | AACGTGAAGAAGAAATTCAA CCGCCCAAATTGGCAATAAA GAGGGCCGTCAAGATCCTCAA CAGGAGTGTGCGGTCAATATT |
| 6799 | SULT1A2 | NM_001054 | CACGTCGTTCAAGGAGATGAA AAGAGGCTCCAGAATAAGTA |
| 6810 | STX4A | NM_004604 | AGGAAGCTGATGAGAACTATA CAGCTCGGACGAAGAGGACAA TCCGGACAATTCGGCAGACTA AAGCGGAGGTGTTTGTCCAA |
| 6811 | STX5A | NM_003164 | CAGTGGAAATTGAAGAGCTAA CAGCAAGACTGTCAGCCTCAA AAGTCCCTTCTTTGATGATAAA ATCAATAGCCTCAACAAACAA |
| 6829 | SUPT5H | NM_003169 | CCGGAAGGGCTTTCTGTTCAA CAGAACAACATCCATGTGAAA |
| 6830 | SUPT6H | NM_003170 | TAGGAGCATCTTTGAAATGTA CAGCACAATTCAGAAGATCAA |
| 6840 | SVIL | NM_003174 | CACATCGAAGCCATGATTTAA ACCGGTTGTAAACAAACAGTA CAGGACTGATGTCAAGGCATA CCGAGTATTATTCCCGCTATA |
| 6844 | VAMP2 | NM_014232 | ATAGTTTACTTCAGCTCTTAA CAGCTGTTATTTATACATATA TCCCTTCATTTGCATCTGCTA CCCATTAGTTCTTGTATCACA |
| 6853 | SYN1 | NM_006950 | CCAGATGGTTCGACTGCATAA ATGTTCGACTGCATAAGAAA |
| 6854 | SYN2 | NM_003178 | CCAGCGTCTCTATCTATAA CTCACTGGAATCCATATACAA |
| 6860 | SYT4 | NM_020783 | CAGGGAGATCTTAATACTCTAA CTGCACTGTTAAACATGTA |
| 6873 | TAF2 | NM_003184 | AAGCATAAGCATGACAGTAAA TAGAATCAAGTTGAACAGCAA |
| 6884 | TAF13 | XM_496381 | TAGGGTGATATTGGTGCATGT GCCCTTGTAAGCTATACTGTA |
| 6887 | TAL2 | NM_005421 | GACCAGGAAGATCTTCACAAA CAGAACTCTGCTTGAGAACTA CCCACTCACCCTTCCAGACAAA AAACGCTTCGCCTGGCAATGA |
| 6891 | TAP2 | NM_000544 | CAGGACCAGGTGAACAACAAA CCTGCTTTCCTTTATGATCTA TAGCGACATTGGTAACAGCTA CCCTATGAGGTTGGTTATTAT |

133

Table4

| # | Gene | Accession | Sequence |
|---|------|-----------|----------|
| 6898 | TAT | NM_000353 | TTCGCTAGTGATGATGGACCCATA CTCGGCAAATATAATGGCTA TCGGGCAAATATAATGGCTAT CAAGCTATTGACCTTTGTTTA |
| 6900 | CNTN2 | NM_005076 | ACGATAGTTACTCACAAGTAA CAGCCACAAACAAGTACTTTA |
| 6906 | SERPINA7 | NM_000354 | CACTATGGTCTTAGTGAACTA ACCCTATTATTATCCAAATTGATA ACCCTATTATTATCCAAATTGATA TAGGGAAAGTTGTGAACCCAA |
| 6910 | TBX5 | NM_000192 | AAGGCGGATGTTTCCCAGTTA CCACAAGATCACGCAATTAAA CCACAAGATCACGCAATTAAA TACCATTGTACCAAGAGGAAA |
| 6916 | TBXAS1 | NM_001061 | CCCAATAAGAACCGACGAA CCGGAGACCTTCAACCCTGAA ACCGCTGCAGCTAGAATCCAA CCGCTGCAGCTAGAATCCAA |
| 6921 | TCEB1 | NM_005648 | CCGAGATTCCTGAATTCCCAA AAAGTTGAAATTTGTTTGCTA TACCTGTAGTTCAGTTAGTAA TGCGAACTTCTTAGATTGTTA |
| 6924 | TCEB3 | NM_003198 | TAGCATCTTTGTAAACTATAA TACAATCATGTATTAATTGAA |
| 6926 | TBX3 | NM_005996 | GCGAATGTTTCCTCCATTTAA GTGCACTTTGTCGGATATAAA TCGGGACAAGTGAACACATTA CTGCCTATAGAGATATATTCA |
| 6927 | TCF1 | NM_000545 | CAGCCGAGCCATGGTTTCTAA GTGCACTTTGTCGGATATAAA CAGGACAAGCATGTCCCACA GCGAGTGGCACGTTTATTTAA |
| 6928 | TCF2 | NM_006481 | CACGGATCTTATCTGGTTTAA CTGGGATATGCTAGTGATGTA GCGGTGTAATAAGATCCTATA TAAGGTTATAACTGTGGCTAA |
| 6929 | TCF3 | NM_003200 | CTCGGTCATCCTGAACTTGGA CTGGATGATTGGGACTTTAAA CCGGGCACATGTGAAAGGTAT CCCGGATCACTCAAGCAATAA |
| 6932 | TCF7 | NM_003202 | TACGAACATTTCAACAGCCCA ATGAGCTGGTTTGTCAAACAA AACAGAAATAATCTGACACTA CTGGGCAGCTTGAGAGGGCAA |
| 6939 | TCF15 | NM_004609 | AGAGAGAATTGGTGAGTTTAA TCTGATATGGTTGGTAAAGAA ATGGTTGGTAAAGAAAATCTTA TGGTTGGTAAAGAAAATCTTAA |
| 6943 | TCF21 | NM_003206 | CAGATCCTGGCTAACGACAAA CTGGTCCAAGTGCAATATGTA CAAGTGCAATATGTAATTATA ATCTTTCTTTATATTGCTAAA |
| 6949 | TCOF1 | NM_000356 | TACGTTGGTCTCAGAAACTGA CAGCGAGGAATCGGACAGTGA CAAGCAGTGAGGAATCAGATA ACGGTTGAAGGTGGAGATCAA |
| 6975 | TECTB | NM_058222 | CAGGGCAACCTTCCAATTCAA TAGGTTTAAAGTGGTCTTGAA |
| 6988 | TCTA | NM_022171 | CCGGAGTCACTCATCCCTTAA TAGGATGGTACCAAGCATTTA |
| 6992 | PPP1R11 | NM_021959 | TGGCTTGAGATTGGTCACTTA CAGAGATCAGTCAAATCCATA CGCCCTAACTTTGCTTGCTAA AACATGAGTAGCGAACACTTA |
| 6993 | TCTEL1 | NM_006519 | AACGCTTATCAACACAGCAAA ATGTAGTAGAACAAACTTTAA |
| 6997 | TDGF1 | NM_003212 | CCCGCTTCTCTTACAGTGTGA AACCAAGTGATCAAACTACAT CTGCCTTTCTATACAAAGCTA TTGAATTATATGTTCAGATTA |
| 7005 | TEAD3 | NM_003214 | ATGGGTTATATCATAAATGCA AACCAGCACAATAGCGTCCAA |
| 7006 | TEC | NM_003215 | CACGTGCCATTGGCCTATGTA TCAGGTGACCTTCGCAACAAA CCGCAGCTAATCAGAATCAGA CAGTACAAAGTCGCAATCAAA |
| 7010 | TEK | NM_000459 | CAGCACGTTGATGTGAAGATA AGGACGAAACTCGTTATCATA TCGGTGCTACTTAACAACTTA ACGGGCCAGATTGTAAGCTTA |
| 7019 | TFAM | NM_003201 | AAGGAATTATATATTCAGCAT AGGACGAAACTCGTTATCATA CACAATAAAGAAACAACGAAA AAGATTGAGATGTGTTCACAA |
| 7026 | NR2F2 | NM_021005 | CTGGCCGTATATGGCAATTCA TAGGTGAAGTCGAGCACTCTA GTGGAATTTATTGGCAGCCAA CCGCTTAGTTCTTGAATTGTT |
| 7029 | TFDP2 | NM_006286 | CTGGTACAGAGAAATCGACAA AGGGACTACTTCTGAACTCTA CGCTACTTCTTAAGTACTTAA AAGGCAGAAAATGAACATGAAA |
| 7030 | TFE3 | NM_006521 | TCCGGGATTGTTGCTGACATA AAGGAGATTGATGATGTCATT TCGCAGGCGATTCAACATTAA CAGCTCCGAATTCAGGAACTA |
| 7036 | TFR2 | NM_003227 | CACGGAGTGGCTAGAAGGCTA CAGGCACATCGGGAACCTCAA GACGGTCATACTGTCGGTTAA TGGAGTTTCAATATCAATAAA |
| 7040 | TGFB1 | NM_000660 | CTAGGTTATTTCCGTGGGATA CACACTGCAAGTGGACATCAA CAGCATATATATGTTCTTCAA CACGTGGAGCTGTACCAGAAA |
| 7043 | TGFB3 | NM_003239 | CCGAGTCGGAATACTATGCCA CCGGGTCTTGCGGGTGCCCAA ACGCATAGACTGAGGTATAAA AAGTATGAATATTACTCTCAA |
| 7045 | TGFBI | NM_000358 | CGGGAAGGCGATCATCTCCAA AAGGCGGATCATCTCCAATAAA CCGCACCCATAATGAGATGTGA TGGAGAAATGGCATCATTATA |
| 7046 | TGFBR1 | NM_004612 | CTGTAAGTTACGTCATGAAA TGGGATTGTACTATACCAGTA TCCATTGGTGGAATTCATGAA CTGCCTTATTATGATCTTGTA |
| 7048 | TGFBR2 | NM_003242 | TACCATGACTTTATTCTGGAA TCGGTTAATAACGACACATGATA CTCCAATATCCTCGTGAAGAA CTCCAATATCCTCGTGAAGAA |
| 7049 | TGFBR3 | NM_003243 | GACAATGGCTATAGTCCAATA CACCTTCAACATGGAGCTATA CCCGCAAGCTGACATGGATAA ACCTGAAATCGTGGTGTTTAA |
| 7050 | TGIF | NM_003244 | TACCACTGTGACTGCATTGAA TACCCAGAGGACACTTGTAAA TCCAAGAACTATAAACTTAAA CACATACTGTCTAATTAATAA |
| 7052 | TGM2 | NM_004613 | CACCTTGATCCCAATCCTTAT ATGGCCCAGCCTTGTAAATAA CCGCAAGGAGCAAACACTGAA CCGCGTCGTGACCAACTACAA |
| 7058 | THBS2 | NM_003247 | TGCATAAGCCATTATGATAAA ATGACATAAGCTACCGATTAA CAAGAGAGTGTCGAATGATAA AGCGTTGGGATACTTCATTAA |
| 7059 | THBS3 | NM_007112 | CACGCCGTGAACCTACAGCAA CTGCATGGAAGTGTACGAGTA CTGCATGGAAGTGTACGAGTA ATCGATGGCTACCCAGACCAA |
| 7064 | THOP1 | NM_003249 | CAAGTGAAGGTCACCCTCCAA CAAGTTCTACCTGGACCTGTA CAGCAAGGTTGGCATGGATTA CAGCAAGGTTGGCATGATTA |
| 7067 | THRA | NM_003250 | CAGCGAGTTTACCAAGATCAT CTGGCCCAAGCTGCTGATGAA CTGGATGAATTGAAGTGAAT CCCGGTGACAGTGGCCAGTCA |
| 7072 | TIA1 | NM_022173 | CGGAAGATAAATGGTAGGGAA CACAACAAATTGGCCAGAACA CTGGGCTAACAGAACAACTAA AACGATTTGGGAGGTAGTGAA |
| 7074 | TIAM1 | NM_003253 | CGCCTGAAATTTCTAATAGAA AACGGAAATGGTAGAGTTTCA CTGGTTCTGTCTGCCCAATAA ACGGCGAGCTTTAAGAAGAAA |
| 7075 | TIE1 | NM_005424 | CCAGTGAGAACGTGACGTTAA TACTTGTATATCGCTATTGAA GCGCTTCAAGGTCAATGTGAA CCCAGTGAGAACGTGACGTTA |

Table4

| | | | | | | |
|---|---|---|---|---|---|---|
| 7077 | TIMP2 | NM_003255 | CAGGCACATTATGTAAACATA | CTCAAATACCTTCACAATAAA | CTCGCTCTGGACGTTGGAGGAAA | CTCGACATCGAGAGACCCATAA |
| 7078 | TIMP3 | NM_000362 | CAGGTGAAAGACTGAGGATGAA | CCCTGCCGGATCGCGATAAATTA | CGGCGGCATTATTCCCTATAA | CACGCTGGTCTCACACCATCAA |
| 7080 | TITF1 | NM_003317 | CCCACTTGTCACTGACACAAA | TGGCTTAAATATAGTTCCTAA | TACCTTGTCATCAGCATGTAA | CTCCGTTACGTGTACATCCAA |
| 7084 | TK2 | NM_004614 | AAGGTGTTCAATTAAATCTGCA | CTCCCGATAAAGAACAGGAAA | CGGGATCGAATATTAACTCCA | ATGCCAGAAGTGGACTATGTA |
| 7087 | ICAM5 | NM_003259 | CCGGGAGAACGTGACCATCTA | CAGGAGGAAACTTCACGTTGA | | |
| 7088 | TLE1 | NM_005077 | AAGGTTAAACATTTGTATAAA | TCGAATTTATAGAAATAGAAA | | |
| 7089 | TLE2 | NM_003260 | CTGCATTGATATTTCCGATTA | CAGCGAGAAGACGGAAATGCA | | |
| 7090 | TLE3 | NM_005078 | CAGGATCAAAGACGAATTCCA | CACAGTTTATGAGGTCATCTA | | |
| 7092 | TLL1 | NM_012464 | GAGGCGTTATTCCTTATGTTA | CAGATCGAGATAACCACGTAA | TAGAATCAATCTCGACCTATA | CAGCATTACAAGGATATTTGA |
| 7096 | TLR1 | NM_003263 | CACCATTTATATGTGGTATTA | CTGGTTTGGCATACAACTGTA | CCGCAAAGTTATATCTCATGAA | CAGGGTCTTCATGAACACTAA |
| 7097 | TLR2 | NM_003264 | CTGGGCAGTCTTGAACATTTA | AACATTTAGACTTATCCTATA | CAGGTAAAGTGGAAACGTTAA | CCGCAACTCAAAGAACTTTAT |
| 7101 | NR2E1 | NM_003269 | CCGTTGATGCTAACACTCTA | TACGTTATGTTGGAGCATTTA | TCCCGTTAACATAGTGCTGAA | CTCTATACTTATGGAAATGTA |
| 7102 | TM4SF2 | NM_004615 | CACGGACGCTATGCAGACTTA | AAGGGTTGTTATGATCTGGTA | CCGGTTCATCACGGCCAATCA | CAGGCTGCCTAAGACATGTAA |
| 7104 | TM4SF4 | NM_004617 | AAGGAAGAAAGCACATTTAAA | CAGGTCAGGGAGAATGTATAA | | |
| 7108 | TM7SF2 | NM_003273 | CCGCATCATGCCCTACATCTA | CACCATGGATATCACACATGA | | |
| 7112 | TMPO | NM_003276 | ACCCATCAAATTACAGTATAA | GAGGAGAAGTATGCAAAGTAA | | |
| 7124 | TNF | NM_000594 | AACCCAAGCTTAGAACTTTAA | CAAGCTTAGAACTTTAAGCAA | TAGGGTCGGAACCCAAGCTTA | CCGACTCAGCGCTGAGATCAA |
| 7126 | TNFAIP1 | NM_021137 | CTCACATACGGTAAAGCCAAA | TGCCCTCTTGAGGTCCTTTAA | CAGGAGGTGAGTTCCTCTTTA | ACCCATGTCTTTCTACCCTAA |
| 7127 | TNFAIP2 | NM_006291 | CTGGCTGTGTAACCTAACATA | TAGGAGTTAGCGTGTTTGATA | CCGGATGTCCATGGAGCAGAA | CAGGGTGATGCTGAAGATGAT |
| 7128 | TNFAIP3 | NM_006290 | CTCGGCTATGACAGCCATCAT | CAGATGTATGGCTAACCGGAA | CGAGCTGTTCCACTTGTTAA | CAGCCTTTACTCATCTCATATTA |
| 7130 | TNFAIP6 | NM_007115 | CTGGCACATTAGACTCAAGTA | CCACTCCACCAGTAATAACAA | TGGCGTCTTTACAGATCCAAA | AAGGCGGTGTGTGAATTTGAA |
| 7139 | TNNT2 | NM_000364 | CAGGCAGCTCCTGTTTGGAAA | CCATCCACCAGTAATAAC | CCGAAACAGGATCAACGATAA | CAGGATCAACGATAACCAGAA |
| 7140 | TNNT3 | NM_006757 | AAGCGTCAGAACAAAGACCTA | GAGGAGCAGTACGAAGAAGAA | | |
| 7142 | TNP2 | NM_005425 | CAGAAAGAACTTGGAAGGCAA | CAGGATGAAAATCCAACTAAT | | |
| | | M55632 | CTCACAATTCACACCAATAAA | TTGGGACATCATGAAAGTTTA | TGCCTTCATATTAATATATAA | GTGGAAGTTTACTGACAGGAA |
| 7151 | TOP1P1 | NM_001067 | CCCATTGTAAAGGTATCTAAA | CAGACTATAGGGAATACCATA | CACGTAGGCTGTTTAAAGAAA | ACGAATAACCATAGAAATGAA |
| 7153 | TOP2A | NM_001068 | GTGGATTATGTGGTAGATCAA | CTGATTGAAGTAGTTAAGAAA | CTGGGCTAGGAGAAGAAGTAA | TCGGGCTAGGAGAAGAAGTAA |
| 7155 | TOP2B | NM_005657 | AAGCAGTAACACCTCTTACAA | CTCCAGAACTACCGTAATTAT | CAGGACAGTCTTTCCACGAAT | |
| 7158 | TP53BP1 | NM_005427 | CCCGCTCTTGAAGAAACTCTA | CCGGGCCATGCCTGTTTACAA | ATCGAGTATTCACCTCCCAA | |
| 7161 | TP73 | NM_001003395 | CAGCTAGAAGACGAAAATTACA | CAGCAATAGGATGATTACTA | | |
| 7164 | TPD52L1 | NM_004179 | AAAGACAATGTCTACCGTAAA | CGGGAGGATAATATCCCACAA | TCCATTTGGAGTGAAGTATAA | CGGAGTATTCAGATCCTGAAA |
| 7166 | TPH1 | NM_000365 | CCGAGGATGGCTGAAGTCAA | ATGGAAGGCGTGGTGGGATTT | | |
| 7167 | TPI1 | NM_000366 | CACACTGTGCTTTCTATTGTA | CTCAAGGGCACCGAAGATGAA | TGCGGAGAGGTCAGTAACTAA | AACGATATGACTTCCATGTAA |
| 7168 | TPM1 | NM_003290 | AGCGTTTGCTTTCGCCCTGTAA | ATAGTAATTAATAGATGGTAA | | |
| 7171 | TPM4 | NM_000367 | ACCGAAATTCCTGGAACCAAA | AGGGTTCAAGTGGTCCCAAA | CACCGGTTGAATGGACAACTA | ACCGGTTGAATGGACAACTAA |
| 7172 | TPMT | NM_003292 | CTGAAGGAAGATCTAAATAAA | CAGGAGGTTTCTAGAGAACAA | | |
| 7175 | TPR | NM_003293 | CTGAGTGCTGGACCTCATTAA | CTGCCTGGGACCGGACGTCAA | CTGGACCTCATTAAAGTGCAT | CAGGATCATCGTGCACCCACA |
| 7177 | TPSAB1 | NM_003300 | CTGGAAGATTCGCGACTACAA | ACACTTCACTCTGAAGAATTA | TCCGATGATCGGCGCTGCAGAA | AGAGAGCATCGTTAAAGATAA |
| 7187 | TRAF3 | NM_004619 | AAGGTAGGCTGTTCCAGTTAA | AAGCATTTCTTTAGCCCACAA | CTGGAGGGTACTTGCTATAAT | AGGGTACTTGCTATAATGGAA |
| 7188 | TRAF5 | NM_004620 | CAGGAAACTATTCACCAGTTA | CAGCGCTGTCAAACTATATA | CAGGAGTGAGTTTAAACCCTA | ACCAGCTATCAGATAAGGCAA |
| 7189 | TRAF6 | NM_012471 | CAGGATCATCGATGAGCCTAA | GAGGCCTGTTCTCAAAGTGAA | CACACTAAGTCCATCTTCAAA | ACCAGCTATCCATCTTCAAA |
| 7224 | TRPC5 | NM_000549 | CAGAGTGTGCTTATTGCCTAA | CCCAGGATGTTTGCACATATA | CTGCATACATGAAGCCATCAA | AAGACAAACTACTGTACCAAA |
| 7252 | TSHB | NM_000369 | GAGAGTTGCTTATGCCTAA | CCCAGGATGTTTGCACATATA | CTGCATACATGAAGCCATCAA | AAGACAAACTACTGTACCAAA |
| 7253 | TSHR | NM_000369 | TCCAGAATCTACGTATCTATA | CAGAATCTACGTATCTATAGA | TACGCAGACTCTGAAGCTTAT | AGCACTGATATTCAGGTTCAA |

135

Table4

| No. | Gene | Accession | Sequence |
| --- | --- | --- | --- |
| 7262 | PHLDA2 | NM_003311 | CACTGTGCCCATTGCAAATAA CGGCTTGAATGAATAAATGA CTGGTTCTTTGTAGTCACATA CGCGCTCGGCACGACATGAAA |
| 7266 | DNAJC7 | NM_003315 | AGCTTATAATTATTATTATACAA AAGGAAGGAAATTACAAACTA |
| 7270 | TTF1 | NM_007344 | AAGGAGATACTGAGAAGTTAA CAGTTTGATTATATTTATATA |
| 7272 | TTK | NM_003318 | ATCCGACTTTATGATTATGAA TTGGACTGTTATACTCTTGAA TCCGACTTTATGATTATGAAA CAGCAATATACCTTGGATGATTA |
| 7273 | TTN | NM_003319 | TGCGGCGAGTATACATGCAAA TCGGGACGGTCAAACCTATAA CGCATTCTAGCTCGAGTCAAA ATCGACTATTACATCGTAGAA |
| 7280 | TUBB2 | NM_001069 | CAGATCAATCGTGCATCCTTA CATGGTCTTTCTACTTGTAAA CTGTGAAGGTATCTATACTAA GAAGGTATCTATACTAATAAA |
| 7297 | TYK2 | NM_003331 | ACGAAATTCCTTGAGCTCATA CTGATGCTATATTCCGCATA CGGGTCTGTGCTGAATGTGTA CACCATCTGGTAATAAACTCA |
| 7299 | TYR | NM_000372 | ACCCATGTTTAACGACATCAA AAGGATTTGCTAGTCCACTTA |
| 7314 | UBB | NM_018955 | GAGGCTCATCTTTGCAGGCAA CCAACTTAAGTTTAGAAATTA ATGGCATTACTCTGCACTATA CAGGATCCTGGTATCCGCTAA |
| 7316 | UBC | NM_021009 | TTGCACTTTCCTTTCAATAAA AACGTCAAAGCAAAGATCCAA GAGGTTGATCTTTGCTGGGAA GAGGTTGATCTTTGCCGGAAA |
| 7317 | UBE1 | NM_003334 | CCCGCTCACCTTTGATGTCAA CTGGGATGTCACGAAGTAAA |
| 7318 | UBE1L | NM_003335 | TTGGAGTTTGACACCACCAA CGGCCGGATGGTCACCTGAAA CAGCAGAAGGAACTGAACAAA ATGCTTGTTAAAGGAAGGCAA |
| 7320 | UBE2B | NM_003337 | CTCATTAGAAAGGCTAACAAA TTGGATTACAGTATGGCAATA CCCAGTATTAGCAATGAATTA ACCTCTCATTAGAAAAGGCTAA |
| 7325 | UBE2E2 | NM_152653 | TCCGATGGAGATCAACGTGAA CCGCTGCTAAATTGTCAACTA |
| 7334 | UBE2N | NM_003348 | ACCTTGTCTCTTGCAGTTTAA AAGATGAAGAATTAACTATTA TAGCCAGTCATAAATACATAA CAGATTTGTGTTAAGATAGTA |
| 7335 | UBE2V1 | NM_021988 | ATCCATGCGAGTAAACATTTA TTGGATGTAACGGCTTGGTAA CACGGGAATTATTATTGTCAA AAGGAAGACTTGAACCTAGAA |
| 7343 | UBTF | NM_014233 | CCGGAGAAGAAGAAGATGAAA CAGGACTTCCAGAGAGAGAAA |
| 7350 | UCP1 | NM_021833 | TCCAGTGTTATTAGGTATAAA CAGAATAAATAGCAACAACCGA TGCAATGAAAGTGTTCACTAA AACGAGAACTGTCAAGTCAA |
| 7358 | UGDH | NM_003359 | TACAATCATGTTTGAAATTAA ACGGTTGTTGATGTCAATGAA |
| 7363 | UGT2B4 | NM_021139 | CTGGGAAACCCAATAAATGAA CCCAATAAATGAACTCCTTTA ATGAGGTTATATTGAAATGTA AGGAAGCTAATAAATAATTAA |
| 7367 | UGT2B17 | NM_001077 | CACATTGTATTTGAACACAAA TTGGGAATATTCTGACTATAA |
| 7376 | NR1H2 | NM_007121 | CAGGCAACAGAGGAGTAGTTCA CCGGAAGAAGAATTCGGAA ATCGTGGACTTCGCTAAGCAA CTGGGCGAGCCTGTAGACCTA |
| 7378 | UPP1 | NM_007123 | CAGGAAAACTAATTTCCTGATA CACACTTTACACAAGAATCTA |
| 7380 | UPK3A | NM_006953 | CACGGAGTACAGGTTCAAGTA CAGTGTGACTTTCGCCACCAA |
| 7384 | UQCRC1 | NM_003365 | CCGGAGCACACAGCTTACTA CGCCACCGTGATGATGCTCTA CCGGACAATGTGGCCTTGCAA CCACAGGTGACCACCAATAAA |
| 7385 | UQCRC2 | NM_003366 | TACATCCAGTCTGACGACCAAA CCAGAATTTCGTCGTTGGGAA ATCAAGGCTGCCTATAATCAA AAGGCTGCCTATAATCAAGTA |
| 7392 | USF2 | NM_003367 | CCGGGAGTTGCGCCAGACCAA CAGGGGAAACTTGACTTTAA AAGACTGTAACGCAGCAACA AAGAGCCCAGCAACCAACGAAGT |
| 7399 | USH2A | NM_007123 | CAGGTACAATTGACCATTGA CAGAGGGAAACTTGACTTTAA |
| 7407 | VARS2 | NM_006295 | CCGGATTTACCACCAGTTGAA CTGTTTCACCATGGACCCTAA CACGAGGAAGGCATCATCTAT CCGGTTTGGATTATGTGCCTA |
| 7414 | VCL | NM_003373 | CGGATTAGAACCAACCTCTTA AAGAGAGTAATGGGTTTCATA TAGGTGTTGTTCATCTGTAA CTGATATAAATGCAATATTAA |
| 7415 | VCP | NM_007126 | CACAAGCAGTTTCTAAACCAA CCCATTGATGACACAGTGAA |
| 7422 | VEGF | AF022375 | GAGGGTTTCAATATACATCTA CTGGAATTTGATATTCATTGA ATAGAGAATTCTACACATCTAA AAGAAAGATAGAGCAAGACAA |
| 7423 | VEGFB | NM_003377 | CAGGACAGAGTTGGAAGAGGA CCGGATGCAGATCCTCATGAT CAGTGTGAATGCAGACCTAAA AAGACCCAAACCTCTGCATAA |
| 7425 | VGF | NM_003378 | CCGGAGGAAGAAGCAGCTGAA CGGCAGCCTCTTGGTCATGAA |
| 7434 | VIPR2 | NM_003382 | CACCGGTTGCTGGGGATACAAA CTCCAGCATTAGATAATCTT CCCGCTGTTCGGCGTCCACTA AACGGAGACCTCGGTCATCTA |
| 7436 | VLDLR | NM_003383 | CCCTGAAAGAGTGTCATATAA ATCCAACTAGTTGAACAGCTA CAAGGTTGGTAGACATGTTAA CAAGATCGTAGGATAGTACTA |
| 7441 | VPREB1 | NM_007128 | AGGCTATGTTATTACTGTGCTA CTGCTGAGATATTTCTCACAA TCCCAGTTCCTGCTGAGATA AGACTGCATTAGGGAATTAAA |
| 7442 | TRPV1 | NM_018727 | CAAGTGGGACAGATTCGTCAA CAGAACCACATCCCAAAGTA CTCAATTGCTGTGCAGGTTAA CCCGAGGAAGTTTATCTGCGA |
| 7447 | VSNL1 | NM_003385 | TAAGCGTTTACCCGAATTAAA CGGCTTAAGCGTTTACCCGAA AGCGTTTACCGAATTAAATA |
| 7450 | VWF | NM_000552 | AAGGACGAAACTGAGAAGAAA AAAGAGCTAATAAACCGAATA CCCACCATCTCTGGTCTACAA ACGGCTTGCCACCATTCAGCTA |
| 7453 | WARS | NM_004184 | CAGAAGGAAACTGAAGAAGAA AAAGAGCTAATAAACCGAATA CAGGTTCTTGATGCCTATGAA AGGGTTCCAAGTATACTCTTA |
| 7455 | ZAN | NM_173059 | TGCGATCTTAATAAACCCTCA CCGTGTGCTAATCAACAGCAA CCCGGCTATGTGCTGAGTGAA CAGCTTAAGAATGGCCAGTAT |
| 7461 | CYLN2 | NM_003388 | CACCTTCTGAACCTTCTTCAA CAGGACAAAGCTCAGAAACA |

Table4

| No. | Gene | Accession | Sequences |
|---|---|---|---|
| 7462 | WBSCR5 | NM_014146 | AAGCTTGTACAGTTAACTTAT AAGGACCATTCCCAGAGCTA |
| 7472 | WNT2 | NM_003391 | AAGGCATTTATTGCCATATTA CTTGGAGAAGAATGGCTTCAA |
| 7474 | WNT5A | NM_003392 | CAGAGTAAATACTCTGAATAA CCGGATAAACCTTGTAACATAT CCGGGTCTGCAGAGAATGAAA CACACAAACTTTACACTTTAA |
| 7480 | WNT10B | NM_003394 | AAACCTGAAGCGGAAATGCAA CAGGCACTGTTTGGAGCCATA CAGGATCGGCTGAGGGCCAAA ACGAATGCGAATCCACAACAA |
| 7483 | WNT9A | NM_003395 | CCGGCTGAAGCTGGAGCGGAA CCGTGTGGACTTCCACAACAA |
| 7484 | WNT9B | NM_003396 | CAGCACCAAGTTTCTGAGCAA AAGCAAAGCCTCCTCCCTTAA |
| 7498 | XDH | NM_000379 | GACAGTCTTTGCGAAGGATAA CTGCACCATTGCTGTTCCAAA CCGGATTGTGTGGTTCGAGTGAA ACAGGTAATAACGTGAAGGAA |
| 7499 | XG | NM_175569 | CAGCCAGTTATTTCAAACTAA ACGGTGGAGATCACCATTCAA |
| 7511 | XPNPEP1 | NM_020383 | AAGGAGAACCTCGTTGACAAA ATGAGATTGCGTGGCTATTTA CCCGACTGGAACCAAAGGTCA CCCGACTTCTTTGGCCAGTGA |
| 7514 | XPO1 | NM_003400 | TACATGTTACTCCCTAATCAA CTGTGTCAGTTTGTAATGAA CCCATTGTAAAGCGACTTCAA CAGCAAAGAATGGCTCAAGAA |
| 7516 | XRCC2 | NM_005431 | CACGATGTATACTTCCCAAAT AGGGTACTACGCAAGCCTTAA TTGCAACGACACAAACTATAA CAGGGTACTACGCAAGCCTTA |
| 7517 | XRCC3 | NM_005432 | AAGCCAAACTGAAATCGGTAA CCGCTGTGAATTTGACAGCCA GAGACACTTAAGGGAAATTAA CAGAATTATTGCTGCAATTAA |
| 7520 | XRCC5 | NM_021141 | GACGATTTATTGGACATGATA ACCCTCATAAGTCGTCACTAA AAGCGAGTAACCAGCTCATAA AAGCATAACTATGAGTGTTTA |
| 7525 | YES1 | NM_005433 | GAGGCTCCTGCTTATTTATAA CCAGCCTACATTCACTTCTAA AAGTATAATGCAGTACATTAA AACAGTCAGTATGCAATCTTA |
| 7529 | YWHAB | NM_003404 | AAGTTTAATCTGATATCAAA CTGCCGTTGTTCGGGACTTAA CTGGATAATACCTTTAAGAAT CAGCAATATGTTCACTATGTT |
| 7533 | YWHAH | NM_003405 | ATCAAGATGGGTGATTATGAA ATGGGTGATTATGAAATGCCA TTCCATCAGCTTTATAATAAA CAGGCTACAGTTGATATTTAA |
| 7555 | ZNF9 | NM_003418 | CAGCAAGACAAGTGAAGTCAA AAGAGTTGAGTCAGACCAGTA CTGACCTCAGTAGCTATTAAA CACATAGATGGTGATGGCAAA |
| 7559 | ZNF12 | NM_006956 | CAGCAATGGATTATAGTTTAA CTCCAGAAGTCAGCACTTAA |
| 7567 | ZNF19 | NM_006961 | CAGGCTATTGTAGCTATTATA CACCAGTGAATCCATTCTCAA |
| 7579 | ZNF31 | NM_145238 | CAGGGAAGTTATCTTGGTATA ACCAGGTAATTTGGAAGTGAA |
| 7592 | ZNF41 | NM_007130 | CAGCATATCCTGGGACATAAA CACAGTGTATATGCAATTCTA |
| 7593 | ZNF42 | NM_003422 | CCGCAGGTCCAGGTAGTGTAA CACAGTGCGATTACATAAGTATAA |
| 7596 | ZNF45 | NM_003425 | AACGAGATGATTTGCACTATA CACCAAGATCAATGTATACAA |
| 7617 | ZNF66 | NM_001009883 | CAGGATCAAATTCTCACCATA CAGCATAAAACAGAACCAAA |
| 7620 | ZNF69 | NM_021915 | AAGAAAGTCAATGAAATTAAA CAGGCTGAACTTCCAGGAGAA |
| 7629 | ZNF76 | NM_003427 | CCGCTCCTTCACCACATCTAA CATGGATGAATGAAGTATTTAA |
| 7634 | ZNF80 | NM_007136 | CAGGGTTATATGCAATGTTCA CTGGAGGAAGAATGAGCCCTAA |
| 7675 | ZNF121 | NM_001008727 | CACATTTGAATTCACACTGAA TAGGAATTTCTCATACCATAA CCAGTGGGACTTGCTTCATTA TCCTGTTTAGCTTGACATGAA AAGCCAGTAATTAGCATCTAA GAGGACTTACAAAGCACTATA |
| 7681 | MKRN3 | NM_005664 | ATGCTTAAATCTCACAAAGAAA ATGGAAGGACCAGAACATTAA |
| 7695 | ZNF136 | NM_003437 | ACGAATGAGGTTAACTTTAA CTGGATTCATTTCCAATTAAA |
| 7705 | ZNF146 | NM_007145 | CAGGAATGAATTCTCCACTAA TCGGATGGCATAAGTGATTAA |
| 7707 | ZNF148 | NM_021964 | CCGAATGAAGATGACTCTCAA CAGGGTGAACATGCCAGCTAA |
| 7712 | ZNF157 | NM_003446 | CCTGAGTACATTTATATTTAA CTGGGTATAAGTAAGCAAGAA |
| 7730 | ZNF177 | NM_003451 | CGCAATTATAATAAATGCTAA CTCCCTGAACTTCACACATAA |
| 7739 | ZNF185 | NM_007150 | AACACTAACATTATCCGTAAA CTGCCAAGTATTGGAAATGAA |
| 7743 | ZNF189 | NM_003452 | AAGACCTTTCATGAAATATAA CACAAGAGTATTTCTTCTGAA |
| 7748 | ZNF195 | NM_007152 | ACGGGCCGAGATATGAACTAA CGCCATGAAGTCAGCTTTAAA CACGCTGGGTATTACCATGTA ACGCTGGGTATTACCATGTAA |
| 7750 | ZNF198 | NM_003453 | CAGGTACATAAAGAAACAATA CAGGAACTATTGGACATTTAT |
| 7756 | ZNF207 | NM_003457 | AAGGGCTGTATTTGGGCCTAA GAGAGCGTTCTTACTAGGTAA |
| 7780 | SLC30A2 | NM_001004434 | CTGCACTAATTTATAACTAT CTACACGTAAGTATAAATGAA CCCGGGTGATTGAGATCCAAA CAGATGTATGTCTACACGTAA |
| 7799 | PRDM2 | NM_015866 | TTGCGGAAAGTAACCACAAA CTGGACTGACTCGGGCAATAA CCGGATGGTGTATGACTGGAT CAGTACCTTACTCATCATTTA |
| 7804 | LRP8 | NM_017522 | CTCGGTGTGTTTAGGCTGAACTA CCAGATGAAAGTCATGAGCTA CTGGACAATGAGACTCCAGTGAA TGGGATGATGAGGTTCAA |
| 7809 | BSND | NM_057176 | |

Table4

| | | | |
|---|---|---|---|
| 7818 | DAP3 | NM_033657 | CCCGAGGAGAATTAGCACTTGTT CGGCACTGTGCTGCCTACCTCTAA TTGGCTCTGGACCTGCATTAA TCGCTTTGTGATGCAGGTGAA |
| 7837 | D2S448 | XM_056455 | TACCTGCACTTAATCAGATA ACGGAACAAGTTACCACTACA AACAACCGATTACACATTTA CAGGAGTGGAATGTACATATT |
| 7857 | SCG2 | NM_003469 | ATGACTGTGTTAATAGTATA CTGGATGAGAATAATACTCGA |
| 7862 | BRPF1 | NM_004634 | CCGAAAGGTCTACAAGAGTTA TCCCAAGTCAGGCAAACATAA AAGGGAGACGATCAAGGTTCA CAGCAACTGCCTCAAGTATAA |
| 7866 | IFRD2 | NM_006764 | CCGGATCTACGCTGCCTTCAA ACGCTTTGAGAAGCACCTGTA |
| 7874 | USP7 | NM_003470 | ATGGAGTTGCGTGGGATTCAA CCCAAATTATTCCGCGGCAAA CGGGCCGACACCAGTACATAA CGAGTATGAAGTAAATTTGAA |
| 7879 | RAB7 | NM_004637 | AACGTTGATTTGCATAATACA TCCCGTTAGATCAGCATTCTA TAGATCAGCATTCTACTACAA CACGTAGGCCTTCAACACAAT |
| 7884 | SLBP | NM_006527 | TACGATCGTTATATTAAAGAA ATCAGTTAATTTACCATGTAA |
| 7903 | SIAT8D | NM_005668 | ACGAGATGTCAGTGGTCAA CACAATGTAGAAGGTTGGAAA |
| 7915 | ALDH5A1 | NM_001080 | AAGAAAGACTCCGTAATCTTA ACCCTCTTTCAAGACAATAA TTCGGAAGTGGTACAATTTAA AAGGAGACTTATCTACATATA |
| 7917 | BAT3 | NM_004639 | CAGCGGTTGATCAACTTGGTA ATGATGCACATGAACATTCAA CCGGTTGCTGGAGTTGTGTAA CAGCTCCGGTCTGATATACAA |
| 7919 | BAT1 | NM_004640 | CCAGGTGGTGATCTTTGTGAA CTCAACCTCAAACACATTAAA |
| 7940 | LST1 | NM_007161 | ATGGCTCACCCTTCCACCCAA TTCAAATTGATCATCATCAAA |
| 7942 | TFEB | NM_007162 | CAGCAGCCACCTGAATGTGTA GAGACGAAGGTTCAACATCAA CCGCCTGGAGATGACCAACAA AAGCAGGAGCGTGCCTAGCGAA |
| 7979 | SHFM1 | NM_006304 | CCGGTAGACTTAGGTCTGTTA AAGAAGTGTTGAAGTAACCTA |
| 7988 | ZNF212 | NM_012256 | CAGAAACGTGATGGAGAGTAA TAAGTAGAAGAAATAATTTAA |
| 7991 | TUSC3 | NM_006765 | ACGGATGTTCATATTCGGGTT CACGGCTATCCTTATAGTGAT CAGGATTGAAATAAATGACAA ATGGTAGCATTTAGTAATCTA |
| 7993 | D8S2298E | NM_005671 | CGGGTGAAGCCTGGAAATTAA AAGGATTGCTTTCTATATATA |
| 7994 | MYST3 | NM_006766 | CGGCGCTATACTAATCCAATA AAGTTGTTTCTTGACCACAAA CTGGACTTCCGTAGTGACCAA CAGCTGAATTTGCGACCTGTA |
| 8000 | PSCA | NM_005672 | CCCAGCATTCTCCACCCTTAA CCCAGTAAAGGCTGAGATGAA |
| 8021 | NUP214 | NM_005085 | CCCGGAGATGATCCCAACAAA CAGAGATATTTGTGAACTTTA |
| 8022 | LHX3 | NM_014564 | CCCACAGATGTCGTTGCCAA CTGGCCTGTGTGTAAGTCAAA CAGCTCTTTCCAAGACTTCAA CTGCTATTTCTAGACATGAAA |
| 8029 | CUBN | NM_001081 | CGGAACCATCTCTCACATTAA CACCTATGTCATAGAGGCTAA CAGCTGCAATTGAAGATCGA CCGGTTAGAATCCATGGACAA |
| 8031 | NCOA4 | NM_005437 | AAGAGAAGTGGTTATATCGAA CCCACGTTAGTTCTACATTAT CTGGTCAATAGTGATTACCAA ACCGACTAAGCAAATAGGCAA |
| 8048 | CSRP3 | NM_003476 | ATGGGAGAGGCAATAATAGAA AACGAGTTGCACATATAA |
| 8061 | FOSL1 | NM_005438 | CTGGACAGTATCCCACATCCA CACAATTTGCACTAAATCAGA CCGAGTAAGGCGCGAGCGGAA CCCACCTAGAACACTAACTCA |
| 8065 | CUL5 | NM_003478 | CTGGAGGACTTGATACCGGAA CCAGCTGATTCAGTTAATATA CAGGTTTGAATCAGTCACCTA CAGCTGGTTATTGGAGTAAGA |
| 8073 | PTP4A2 | NM_003479 | AGGGACTGTGTTGCTTGTGAA ATGCAACAATAGATTCCATTA CAGAGAATGCTGGTAGCTTAA AACAGCATACCTGCTAAGCTA |
| 8085 | MLL2 | NM_003482 | CTCGGAGTGGTTTGAGAACTA CCCGTTGAGTCCCATAGGGAA CGGCCGGAGTTTGTAATCAAA CACCAGCATGTCTAAGGCATA |
| 8111 | GPR68 | NM_003485 | TAGAGCTTGAACCACCTTCTA CAGGATGGCGGTGGAGAGACA CCGCGTGTGCTTTGAGCACTA CAGCGTGGTCATCTGGGCCAA |
| 8131 | C16orf35 | NM_012075 | CCCGGCGTCCCAGCACAAGTAA CAGACACTGGGTTGATCACCTA |
| 8139 | GAN | NM_022041 | AACGTTGGCAATGAAAGATAT CACCATAACATAGCTCCGAAA ATCATGATGAATTGAAATGAA CCGCACAGTCATGTAATTCAA |
| 8153 | ARHN | NM_005440 | CAGTTCCTCTTACTATGATA CAGCTATTCTCACACATCTAA |
| 8175 | SF3A2 | NM_007165 | CCCGGGCTACAAAGTGACCAA CTGCCTGACACTTCACAACAA |
| 8178 | ELL | NM_006532 | CCGAAGTGCCATTGTCATCAA AAGGAATTATGAAGCTAAGAA |
| 8190 | MIA | NM_006533 | CTCCTTGGGTTTATGCAAATA CAGGGAGATTACTATGGAGAT |
| 8192 | CLPP | NM_006012 | CACGGTGGCGTCATGCAGGTA GCCCATCCACATGTACATCAA CACGATGCAGTACATCCTCAA GCCCAGGAGTTTGGCATCTTA |
| 8200 | GDF5 | NM_000557 | CACTATGGGACTGGATACAAA CAGCTCCTTCCTGCTGAAGAA CTGTTAGACTGTTAGATTTAA TAGATTAAATGTATATTGAT |
| 8204 | NRIP1 | NM_003489 | GCGGAAGAAATGAGTATGAA ATGGTTGACAGTGTCCGTAAA AAGCGTGCTAACGATAAAGAA AACCAACCAATACTATATTA |
| 8209 | C21orf33 | NM_004649 | ACCACATTTATGGACCATGAA CAGGAAGAAAGGAAATTGCA |
| 8214 | DGCR6 | NM_005675 | CAGAAAATAAAGATGCCTCA TGGCTTCTACGTGACCACCAA CTGGGCAGAGTCATCTTCTT ACCCTAGGATAGTCTCATAAA |
| 8237 | USP11 | NM_004651 | CCCATTGAACGCAAGGTCATA AAGGTCGAAGTGTACCCAGTA ACCGATTCTATTGGCCTAGTA CTGCGTCGGGTACGTGATGAA |
| 8241 | RBM10 | NM_005676 | CAGGACTATCGGACCGAGCAA CCAGAAGGTGTCGATGCACTA CTCCAACGTGCGCGTCATAAA ACCACTCACTATCGACGGCAA |

Table4

| | Gene | Accession | Sequences |
|---|---|---|---|
| 8243 | SMC1L1 | NM_006306 | TCGGAAGTGAATGACAAGAAT ATGGAGGAGATTCGTAAGAAA CGGCGTATTGATGAAATCAAT CACCATCACACTTTAATTCCA |
| 8260 | ARD1 | NM_003491 | CCGGGCCGCCCTGCACCTCTA ACGAGCTTTCACAATAAATTT CACAGAGAGCACAGATGTCAA CCGCCCCTGCACCTCTATTCCA |
| 8266 | UBL4 | NM_014235 | TCCCTTCTGTTCAGTAAACAA CTGTTCAGTAAACAAACTGAA CAGGGACACACTCGTGAGCAT TAGGCTGTTTCTGTATCCAA |
| 8270 | DXS9879E | NM_006014 | CTGGTTCTTAAAATGTTGTTA CCGAATTTCCGTCATCAACTT |
| 8273 | SLC10A3 | NM_019848 | CAGGGACAGCCCTGCCATCAA ATCAAGAACGTGAGTGCTATA GCGGAGAGACTTCTGCATCAA CATGGTGTTAATGCAGGACAA |
| 8284 | SMCY | NM_004653 | CTGACGATTGCTTAGCATTAA CGCGTCCAAAGGCTAAATGAA CCCAGAGACGTTGGATCTCAA CAGGGTAGAAACGTTGAGAAT |
| 8291 | DYSF | NM_003494 | CACCGTCAAGGTCATCGATAA CCGGGATGAGCCCAACATGAA |
| 8292 | COLQ | NM_005677 | TCCAATGACTTTGGGAATTTA AACAGCGTTCTTCCAATCTCA AAGAAGCGTGGTGGCCACAAA AAGGACAAATTCCTGCTACTA |
| 8309 | ACOX2 | NM_003500 | ATGGCATATATGCAACATTAA CTGCCTATGAGGAATATATAA |
| 8312 | AXIN1 | NM_003502 | CGGGAAAGGTGTTGGCATTAA CTGATACCTGCCGACCTTAA CAGCTACAGATACTACTTCAA |
| 8318 | CDC45L | NM_003504 | ACGGATCTCCTTTGAGTATGA ATTGAAGAGTCTGCAAATAAA TAGGCCAGTCAATGTCGTCAA TACGAGCAGTATGAATATCAT |
| 8322 | FZD4 | NM_012193 | ATGGTGCTTAATGAATCTCCA CTGGAGAGAGTTGCTATTCCA TAGGTGATCGATACTTGTCAA CAGAATGATAGTGCCTTTAAA |
| 8339 | HIST1H2BG | NM_003518 | CTCCAAGTAAAATGTGCTTA GCCACTCAAGTTCACATAAA |
| 8340 | HIST1H2BL | NM_003519 | TTGCCACTATTGTTCATTAT CAAGCTCTTGTCCAACCCAAA |
| 8341 | HIST1H2BN | NM_003520 | CAAGGCCATGGCATCATGAA TTCGGTCAGTCTCGGCCCACA |
| 8347 | HIST1H2BC | NM_003526 | TAACGACATATTTGAGCGCAT CCAAGTAAACATTCCAAGTAA |
| 8356 | HIST1H3J | NM_003535 | CTGCTCTATCTTGGGTTTCTT ATCCGTGGCGAGCGAGCATAA |
| 8370 | HIST2H4 | NM_003548 | CCGCAAGGTCTTGAGAGACAA GCGGATCTCTGGCCTCATTTA |
| 8379 | MAD1L1 | NM_003550 | CAGCTGGACCTGCCAACACAA CAGGACCAAAGTGCTGCACAT CAGGCGACTGCTCATCTTCAA CAGCGCGATTGTGAAGAACATGA |
| 8382 | NME5 | NM_003551 | GAGGAGATACAAGATATTATT CTCGAAAGAACAAATCATGAA CATGATGGTTCAGTGGAATCTTCA ATGATATTAGCTAGACATAAA |
| 8392 | OR3A3 | NM_012373 | CAGGTGTCTTCAGCTACATGA TGGAATCTTCAGACAAGGATA CTGGGTTCAGTGGAATCTTCA CAGGGCGCTCGTGTGTCAGCTA |
| 8396 | PIP5K2B | NM_003559 | CACGCGTTTCCTCACCACCTA CCCATTTGACACCCAGGTTAA CCGCTTAAGTTAAGGAGTA CACGCCATACGATACAAAGAA |
| 8417 | STX7 | NM_003569 | CAGCAGATTATCAGCGCAAAT TTGCTGTAGATTGCCTAGTAA CAGCAGTATACTAACCAGCTT TCTGATGAAATTAGCGAATAA |
| 8419 | BFSP2 | NM_003571 | CAGGGTGTGAGCTGGAACAAA CAGCCTGAGACAGAATCCTTA |
| 8425 | LTBP4 | NM_003573 | AACCGGCTTTGAAAGAGTTAA ACACGTGATCTCAGAGGCCAA GCGGCTGGAGTGCATCGATAA TACGAGTACGGCCCAGACTTA |
| 8435 | SOAT2 | NM_003578 | CAGGTTAGGTTCCTGATGAAA CAGTGGAATTATGTGGCCAA |
| 8438 | RAD54L | NM_003579 | CGCGCCGTTTGGGACACAGGAA CTCGCTAAAGAAGCTTTGTAA ATGGAGGATCTAAGGATGAAA AAGCATTTATTCGAAGCATTT |
| 8440 | NCK2 | NM_001004720 | ATCTCACAATTATGTATTTAA GACACTAATACAGATGAATTAA TAGCCGTTTGTTGACACTAAT TCGGCTTGTGTTTGAATCTCACA |
| 8446 | DUSP11 | NM_003584 | ATGATGCAATTGAATTATTCA CACAATAAGCCTGTTAAACAA CCCTTATGTATTCAAGCTTAA CAGAAAACTGTTCCTTACTTAA |
| 8448 | DOC2A | NM_003586 | CACGAGTGTGTTGCACGTTTA GCGGAGTTCTATATTCACATA |
| 8456 | FOXN1 | NM_003593 | TCCCGTCAGCGAGATCTACAA CAGCGTTTGCCTGGTCTGGAA CGGGCTCACCTTAAAGGTCAA CACACAAATTATTCAGATGTA |
| 8459 | TPST2 | NM_001008566 | CAGATTTGCCATCCTCCTTAA AAGCATATTGCTTGCTATTAA |
| 8460 | TPST1 | NM_003596 | AAGATATGCCTTTAATATTTA GAGGCTGGTGTTACTGATGAA |
| 8468 | FKBP6 | NM_003602 | CAGGCTTTGATCATTGACCAA CAGGTTTCTGTTCAAACCGAA |
| 8476 | CDC42BPA | NM_003607 | ACGGCTTTCATTACCAAATAA TCGGAAAGATATACCCTGTAT CAGAATAATAGTCGGAAACAAA CAGACTTTCCGTAGCAGCTTA |
| 8481 | OFD1 | NM_003611 | CCGCCCGGGCTGGGCACTAAA CTCAGACAAGTTCGACATTTA TACCATCGGAGAGTCATTAAA |
| 8493 | PPM1D | NM_003620 | TACATGGAGGACGTTACTCAA ATGGCCAAGGGTGAATTCTAA CTCGGCGTCGTCGAAGATAAA ACGGGTCTTCCTAGCACATCA |
| 8498 | RANBP3 | NM_003624 | AACGAACTATTTCCTCCAGTA CAGAGTTAAGCTGATAAATGA |
| 8499 | PPFIA2 | NM_003625 | ACCGATGAAACTAGTCAAATA TCCAATGAACGCCTACAACTA TACCAGCTGGATTTAGGTTAA AACGTTTGGGTGACTCATGAA |
| 8504 | PEX3 | NM_003630 | GCGGGTCCAGTAAACATAAT CGAGACATTACCACTATTAAA TGGGTAAATCACCTATACTTA ATGGTGAATTCCAACAACTAT |
| 8507 | ENC1 | NM_003633 | AAAGGCATTGTCCAAAGTTTA CACCTTAGTACTTTAGACTAA CAGCGGCTCCATTAACATCTA ACGGCATGTATCACAGATGTA |
| 8511 | MMP23A | NM_004659 | CGGCCAGAAGATCCTCCACAA CCGGAGATCCTCGCACCTGAA CAGAAGATCCTCCACAAGAAA |
| 8513 | LIPF | NM_004190 | CAGCTTTGATGAAATGGCTAA CACAATCGCACTTCATTGTAAA CAGCGTGTTAAGTCTGTGGGAAA |

Table4

| ID | Accession | Gene | | | | |
|---|---|---|---|---|---|---|
| 8517 | NM_003639 | IKBKG | GAGGAGCTTCTGCATTCCAA | ACCATAAATAATGGCATAGTA | TTCGGAAATGCCTCACATATA | CTCCTCTAGTTCAGAGACATA |
| 8518 | NM_003640 | IKBKAP | ACTGGATATTATAGAAACCAA | AGCATGGTAACTTATATTGAA | GCGGTTTACTATAGACAAATA | CAGCGGTTTACTATAGACAAA |
| 8520 | NM_003642 | HAT1 | CACGCTAGAAGGGTTTATGAA | AAGACTAATTAGCCCATATAA | CCCTTCTTAAACAGTATAATA | TACCGGCGTGTTATTGAACGA |
| 8526 | NM_003647 | DGKE | ATCCATTAATCAGATGCGTAA | CTAGATCGATGGAAAGTTCAA | CTGAAGTCGTTGGAGTATATT | ATCGATGGAAAGTTCAAGTAA |
| 8532 | NM_003652 | CPZ | CGCGCCGAGTCATCCTGCTA | CCCGGAGACAAAGGCAATCAT | TACGCCAAAGTCATCAAGAAA | TACGCCAAAGTCATCAAGAAA |
| 8535 | NM_003655 | CBX4 | CAGCCCGAGTGAAGACTCCAA | CTGCCTCACCGTTACTTTCAA | GAGGCTGGTCGCCCAAATATA | AAGCCCTTCTTTGGGAATATA |
| 8536 | NM_003656 | CAMK1 | CTCGAGTGGCAGATATACTAA | CAGGAGATACAGCTCTAGATA | CTGCATGACCTGGGCATTGTA | CAGGTGCTGGATGCTGTGAAA |
| 8539 | NM_006595 | API5 | CTGGTGGACGCTCGAATGTTA | CCGACCTAGAACAGACCTTCA | CAAGTTTGCTATTCAAATCAA | AAGAGTAATGGTCAATCTTAA |
| 8541 | NM_003660 | PPFIA3 | CTGGACAACCTTGCAGACAA | CAGTGGAGAGGGTTAATGAA | CACGGGTAAAGAGAACTGTTT | ACGGCTCAACTATGACCGGAA |
| 8542 | NM_003661 | APOL1 | ATGTGTATTTAAATAATAAA | ATGACTGAATATGAACATTAA | | |
| 8543 | NM_006769 | LMO4 | CCAAATAATGTCGGCTTACAA | CAGAATTGATGTGCTGCTAAA | CCGTACAGTGATGATGACGAA | CAAGATCAGCAAGAACCCGAA |
| 8555 | NM_003671 | CDC14B | AACGGATATTATTAAATTCAT | CAGAATGATCTGAAAGTTCAT | | |
| 8559 | NM_003675 | PRPF18 | TAGTGTTCTGCTTATAATTAA | CAGAGTTACCCTCTAAAGATA | AAGGCTATTAATATCATACTA | TGGGTACAACATCGAATAATA |
| 8564 | NM_003679 | KMO | CCCTGTATTAAGCAAGAATTA | CCCGGGCATGTTCGTCTCTCA | TCGAGTGACTTTCTAACCCAA | CCGGAGCTTCATCTAGTGATT |
| 8566 | NM_003681 | PDXK | CCAGGAAATGATCGACAGGTA | CTCAACAAGTTCTATACTAAA | CAGGAGGGCGTTAGTGGATCA | CAGACCCACTACTACTATAAA |
| 8567 | NM_003682 | MADD | CAGGGAATTCAGCTCCAGTAA | TCCGAGCAGTGTGTGTTTA | | |
| 8568 | NM_003683 | D21S2056E | ATCCATGCTCTTGACCTTGTA | CAGCGTCGCCCGTGTTCTCTA | CAGATGGATCCTAATAGAATA | ACGGGTTGACCTTATGAGAAA |
| 8600 | NM_003701 | TNFSF11 | TAGCATGTTGTCAGTGGCCAA | TCGGTTGAATACGTCACTTA | CACATATTCGATGATGCTCAA | CTGCTATATATTCACCATGTA |
| 8601 | NM_003702 | RGS20 | CATGGTGTTCTTGGCCTGTA | ACCCGCGTGTCTGATTACAAA | TCGCTCGGACTTCAACAACTA | CCGGGTCAACTGCTCGTCTA |
| 8612 | NM_177543 | PPAP2C | TTGGGAAGAAGTGACATGTCA | TCCGCGGAGGAAGACAGCGAA | CCCAGAGGTTTGGCAGAAATA | CAGGGATCCTGGAGGAATGAA |
| 8620 | NM_003717 | NPFF | CCGGATTACTGGACAATACAT | CAGGATGAGGAAGTACAGACA | | |
| 8635 | NM_003730 | RNASET2 | GCGGGTGCTGAAGGTAGTCAA | CAAGTACTACACACTCCACAA | CAGGTGCTCCTTAGCCAAATA | CAGCTGGAATTCCAAGGCCAA |
| 8638 | NM_003733 | OASL | CACCGTATTGCTACCACCAAA | CTGGTGTTGCTTGAACCTCTA | | |
| 8642 | NM_003737 | DCHS1 | TAGAATGTCATCCGTATTTCA | CCGGAGTAAATTGCTAGATTT | CTGGGATCTCAACGAGACAAA | CACCCTAATTATCCATATTCA |
| 8644 | NM_003739 | AKR1C3 | AAGAGCTAGCATATAGATGTA | TTGCATTGAGTGAGGAACACA | TAGCATATAGATGTAATTTAT | ATGCCCAGGCTTGCAGAATAA |
| 8649 | NM_021970 | MAP2K1IP1 | CTGTAGGATGGTAGCACACAA | TAGGATGGTAGCACACAACCA | CCCGCCGTGCACGCAGCATTA | CTGGTTGTTGTAGCAGCTTAA |
| 8651 | NM_003745 | SOCS1 | TCGGTTCAATGCAGAAGTTGA | AACAACAATCATTAGAAGTAA | TCGGATGTTTACTGACCTCAA | CAGAGATGGTGTGTTCTGCAA |
| 8654 | NM_001083 | PDE5A | CCGGAAAGAGTGGGACCTGAA | CAGGGTAAGACCGTGATCCAA | CAACTTCTACATCAACGACAA | CAGGGTAAAGTGTAACTCAGA |
| 8659 | NM_003748 | ALDH4A1 | GAGGATCTAGATAATATTCAA | CAGGAACATGAACAAATCAAA | | |
| 8661 | NM_003750 | EIF3S10 | ACCGTGATTTCGAGTCCCATA | GGGCGTCATTGTCAAGATCAA | AACGAATGGATGAAGAATTTA | CCGCCGACGCATGATCAGCAA |
| 8663 | NM_003752 | EIF3S8 | TCGGAACATGTTGCAGTTCAA | CCGCCAAGTTTCAGTGATGAA | | |
| 8664 | NM_003753 | EIF3S7 | CGGAGTTGACCTGATCATGAA | CACAATGAGTCAGAAGATGAA | CCGCATGAGCATCAAAGCCTA | TACGCGTACTACGACACTGAA |
| 8665 | NM_003754 | EIF3S5 | CACGCAGAACAAGACAGGGAA | GAGGTCATCAACGGAAACATA | | |
| 8666 | NM_003755 | EIF3S4 | CAGATTAAGTATAACCGCGAA | AAGAAATAAATTGGTTTGGTA | | |
| 8668 | NM_003757 | EIF3S2 | CTGGTGCCTTCTCTTAAGTAA | AAGACCTTTCAGTATCCCTAA | | |
| 8673 | NM_003761 | VAMP8 | CTGGGATCAGTTATAAATATAT | TCCAACGTAATTGGTCAGATA | ACCATCATTGATGTTAATTAA | CCGGATTAAGCAGAAGATGAA |
| 8675 | NM_001001433 | STX16 | CAGGACTTTGGCCATCTATAA | CCCAGGGATGTTCAGAATCAA | CAGGATTGTCGGCAGTAGTAA | CTGAAGTTTACTACAGTGGTA |
| 8685 | NM_006770 | MARCO | CTGCCATTAAATGTCAATAAA | CCAGTGTTTCAGAATCTCCAA | | |
| 8688 | NM_003770 | KRTHA7 | TCCCTTGCCCTAGATAGTTTA | AAGGGCTCTCTAGGCATGTA | CTGCCAGTACCTCAAAGATTA | AAAGATGTGTATCGCCGTTA |
| 8692 | NM_003773 | HYAL2 | CTGCCAATAGCAAGAAAGCCTA | TAGGACAGCTTGTATAATTA | TACAGAAAACTCAATCAACTA | ACCCATCTTAATTAAGCCTTA |
| 8697 | NM_004661 | CDC23 | TTGAAGTTCGATCATGGTAAA | CCGGCTCATTGTCCCACCGACA | CCGGAGCTTCCACCACCGACA | CAGCCGGCTCATTGTTCTGCA |
| 8698 | NM_003775 | EDG6 | CCGGCTCATTGTTCTGCACTA | CAGGTGGCTGTTAACCATAATTAA | | |
| 8701 | NM_003777 | DNAH11 | CACCGTCAAATGAAAGGATAA | GAGCTTGTTAACCATAATTAA | TACGTTGTTACACGCGTTTGCAA | |

Table4

| ID | Gene | Accession | Sequence |
|---|---|---|---|
| 8702 | B4GALT4 | NM_003778 | CACAGTGAACTTGGGAATGAA TACATATTAACTAATAATAAA |
| 8704 | B4GALT2 | NM_001005417 | CCCATGGATGACCGCAACCTA CAGAGGTTTACCAAGATTCAA |
| 8707 | B3GALT2 | NM_003783 | ATGGAAGTATTTACAATGAAA AACCGTGGATTTCATATTATA |
| 8708 | B3GALT1 | NM_020981 | CAGAGTTAAGAGGAAGATTTA CACACTGGATGTGATTATTAA |
| 8711 | TNK1 | NM_003985 | AAAGAGCTACTATACATCATA CCCTATTACATACATCTTTCA ATCGGTCATGATGATGAACTTGGA AAGGATCTAGTCTGCCACTA |
| 8717 | TRADD | NM_003789 | CAGGGTCAGCCTGTAGTGAAT CCGAATGTTAAGCAATGATAA CCCGAATGTTAAGCAATGATA AGCAATGATAATAAAGTATAA |
| 8718 | TNFRSF25 | NM_003790 | GAGAACCACCATAATCTGAA CACAAGAAGATTGGTCTGTTT CACCGTCCAGTTGGTGGGTAA CGCGGTATTAAATCTGTGAAA |
| 8720 | MBTPS1 | NM_003791 | CACAGGAAGAATTGTAGATAA CAGAACGGAGCAACATTGAA CAGAACAGACTTCAACAGTAA CAGGGATAATTTAAGGATGAA |
| 8721 | EDF1 | NM_003792 | CGGACGGGCCATACCCAATAA CTAGGGCGAAATGAACACAAA CAGAAGGACCTGGCCACGAAA CACGGTGACGGTGCTGCGCAA |
| 8722 | CTSF | NM_003793 | CCTGTCCGTCTTTGTCAATAA CGGACATATGAGTCAAAGGAA CACCTTTGTTGAATTGTGGTA CCCAATCTCCGTGGCCATCAA |
| 8723 | SNX4 | NM_003794 | AACATTATGCAGATCAGTTAA TACCTAGTATACATAAAACATA CCCATCCTTTGTAGAGACAAA CTCGTTATTTCTAATGATGTA |
| 8724 | SNX3 | NM_003795 | ACCCTTCACATGGAAGATTAA CAAGATGAAAATAATAGATAAA |
| 8725 | C19orf2 | NM_003796 | AAAGAGGGTTTCAAAGTTTAA CTGGAAAGAAATACCACTTTAA |
| 8726 | EED | NM_003797 | AAGGAATCTTATGATTATAAT AAGCAACAGAGTTACCTTGTA AGGCATAATTAGGATAATAAA CTGAACGCCCTGATACACCTA |
| 8727 | CTNNAL1 | NM_003798 | CTGCAGAGGGTTTAAAGCTTA CGGATGGGTCTCAGTTACAAA ACGCTTATTAATCATAAAGAT AAGCTTGGGAATCCCAAATTA |
| 8729 | GBF1 | NM_004193 | AAGAATATTTACATCATTCAA ACGGGAACTAATTGAAATTAA |
| 8731 | RNMT | NM_003799 | CTGGTCTTAATTAACAGGTAA CAGGGTGACTCTCCAAAGAAA |
| 8736 | MYOM1 | NM_003803 | CACCAACTAATGACACTTTAA CAGTCTTTCACTCATATAAA |
| 8739 | HRK | NM_003806 | AGCAGCAACAGTTGGTGAA ATACAGCTCTATATATAAA CGAGAAGGAAGTGGAGAGTAA AGCGATCGTAGAAACACAGAA |
| 8747 | ADAM21 | NM_003813 | TACCAAAACATTGTCATTAA CAGGAATATGTCTCCACCTA |
| 8767 | RIPK2 | NM_003821 | CAGGGACTTGATCATGAAAGA CCAGGCTTAATTGCCCTACAA CACAAGGACATCGACCTGTTA ACGTATGATCTCTCTAATAGA |
| 8772 | FADD | NM_003824 | TCCTAAGGTAATCCTATTAAA CAGGACGAATTGAGATAATAT AGCGGGGATCTCGTATCTTTAA |
| 8773 | SNAP23 | NM_003825 | AAGAGGTTGTTACCTCAGTAA TACCACATGAATTCAGATTTA CAGGCCTATTAACATCATACA CTGGCAAGGCTTATAAGACAA |
| 8775 | NAPA | NM_003827 | CCGGGAATGCAAGTTGATGAA CACCGAGTCGGTGAAGGAATA |
| 8778 | SIGLEC5 | NM_003830 | TTGTGGCAATGTTAACATTAA CTCGGAGATCAAGACAAGCAA AAAGGTTGTTGTGAACATTAA CAGGAACGGCATAGCCCTAGA |
| 8784 | TNFRSF18 | NM_004195 | CAGCAGAAGTGGGTGCAGGAA CCCTGGGAACAAGACCCACAA TCGGGATTCTCAGTCATGAA CAGGAGGGAGAGAGACACA |
| 8787 | RGS9 | NM_003835 | AAGCTATTGACCCTCAGGAAA AACGATGGGCCTTCAACTTCA CAAATAGGTGTTGAACTGTA CTGACACCAGTTTCAAATTTA |
| 8792 | TNFRSF11A | NM_003839 | CCCAGTGTGTGTTCATTGTAA TAAGTTTGTGTCGTTCCTTAA CCCGCTAGGTGGTTAATTTAT ACCCGCTAGGTGGTTAATTTA |
| 8797 | TNFRSF10A | NM_003844 | CAGGAACTTTCCGGAATGACA CAGGCAATGGACATAATATAT CCGGGTCCACAAGACCTTCAA ATCAAACTTCATGATCAATCA |
| 8798 | DYRK4 | NM_003845 | ACACGGCTCCTGAGAAATTTA CAGATGCTTTCGGTAGAGAAA CTGCATCACGGCGGAGTTGTA CTCCATATGAACAAAGTGAAA |
| 8802 | SUCLG1 | NM_003849 | CAACATCTCTATGTTGATAAA ATGGATCACGTAGACATGTAA |
| 8807 | IL18RAP | NM_003853 | AGCGGTAACCTGGTACAAGAA ATCGTAGTGGATGAAGTTTAT CCCAGTGTATCTTTGAACTACAA CCCAGCCTTAAGGAATGGTGAA |
| 8808 | IL1RL2 | NM_003854 | CGGAGTCTACCAATGTGTTA TAGGTGGTTTACCAAATTTAT TGGGTCCAATAAAGTGTATA AAGGACTGTAACGAGATTAAA |
| 8809 | IL18R1 | NM_003855 | CGAGAAGAGATGAAACATTAA CCGAGTCTTAATCTTCAGAAA AAGAGTGAGATTGTCAGTGTA TTGCTATTGCATAGTCTCTAA |
| 8811 | GALR2 | NM_003857 | CTGCACCTTCGTCTTCAGCTA CAGCGTTGATGTGGCCTGAAA TACCAACCTGTTCATCCTTAA CCCGCTCACGCGCCCACTTA |
| 8812 | CCNK | NM_003858 | CAGCCATATTGGCTCAATAAA CTCAATAAAATAGCTTCGGTAA ATCCTGGATCTTTACTCACAA AAGCAACTCAAAGGTGATAAA |
| 8816 | WDR22 | NM_003861 | CTGGTTAACCTGAGAAGCCAA TAGAATTACTTTGGATTTGTA |
| 8818 | DPM2 | NM_003863 | TAGCCTGATCATCTTCACCTA CAGAATGTCATCCACAAGTAT |
| 8821 | INPP4B | NM_003866 | CTCCATAGATTTGAAACAGAA ATCGATGTCAGTGACACTTGA CCCGAAAGTGTGAGCGCGAAA AACGATTTGCCGCAAACTGAA |
| 8822 | FGF17 | NM_003867 | CTGGAGGTGGCTGTCCTCAAA CTGCTGATTCTCTGCTGTCAA |
| 8828 | NRP2 | NM_003872 | CGCAAGTACTGTGGGACAAGAA CTGGTCTGTATTGTAGGATAA CTCGTGATATTTGTAGGGATAA CAGGCTCTGAAGATTGCTCAA |
| 8831 | SYNGAP1 | NM_006772 | CAGAGCAGTGGTACCCGTGTAA ACGAACGAAGTCACAACCCAA CCCGGCTGATGCAAAGCTTTA ACGCTTGCCACTAAAGCCATA |
| 8832 | CD84 | NM_003874 | CTGGGACTTCAAGCTATGAA ACCCACAGAAATTATTATGAA CTCAGAAATCTTCACAGTGAA CAGAATGTGGGCTGCATATAA |

Table4

| No. | Gene | Accession | Sequence |
|---|---|---|---|
| 8836 | GGH | NM_003878 | ATGCCGTAATAAAGTCATGAA AAGCATTGATTTATCAGTTCA TCAGTCCAATTTATACTGGAA CTGGCTCTGATTCTTCATTAT |
| 8848 | TGFB1I4 | NM_006022 | AACTTTCAATAAAGCATATAA CTCACTTTGTGTAATCTGAAA |
| 8851 | CDK5R1 | NM_003885 | CACATGCACTACTGAAATTTA CTGGTCATCAGTATTGTGTAA TGAGCTGGTTGACTCATTGA |
| 8852 | AKAP4 | NM_003886 | CTGCCTTATCATGCTAAGTA CAAGACTCTCTTGGACATGAA CCGGAAGGCCACGCTGTTTGA |
| 8875 | VNN2 | NM_004665 | TGGGCGTTTGGTAAACAAGAA ATGGCTACTTTCAATACAATA GTCGGTGAACAATTCTCAAA |
| 8881 | CDC16 | NM_003903 | AAAGAACTTCTTGAATCACTA TAGAATATGGTTTGACCAATA AGGAGCTTTGCTGTCATTTAA CAGCAATAACTTACCTGCTAA |
| 8882 | ZNF259 | NM_003904 | GAGGAAGATCTCAGAAATGAA CTGGAGGACATGAACAGAGAA CCCATGCACTTCGGTCACGAA ATAGTGGAATAAAGAAGGTAA |
| 8883 | APPBP1 | NM_003905 | CAGGTATTGGTTCGTTTACAA ATGGATTGTGATCATAGCTAA |
| 8888 | MCM3AP | NM_003906 | CAGGAGCATTTACGGATTTAA CTGAGTGAAGATGGTCAGATA |
| 8893 | EIF2B5 | NM_003907 | CTTGTATATGTTAATATTAAA CAAAGTGAAGATGAAAGGTTA |
| 8896 | G10 | NM_003910 | ATGAGAGAAGCTGAAACAGAA CAGCAGAGAACCTCATGAATA |
| 8900 | CCNA1 | NM_003914 | CAACAAGGGTTTGACATCTA AAGCAATTAGGAGAAGTACA TTCGAAATATGAAGAGATATA CACCGATGATACATACACAAA |
| 8905 | AP1S2 | NM_003916 | ATGGCATTAGATGCTCTATA ATGGCACATCATATACATGTA AAGGGAACTTTAGTTAATTAA CAGGCAGTAGTATACATTTATTAA |
| 8906 | AP1G2 | NM_003917 | CCGCCAGGTGGTGTCCATCTA CCCACAAATAAAGCCCAATAA |
| 8910 | SGCE | NM_003919 | CAGGTAAATGTATCCCTGAA TAACATTTAATACAAATTTAA |
| 8914 | TIMELESS | NM_003920 | CCGGCTCATGGGATCAGTAAA CAGCCGCATCATCAAGAACAA |
| 8926 | SNURF | NM_005678 | GAGGTGGAAGTCCAAGTCAAA CAAGAGGTGGTTAAAGCCATA |
| 8932 | MBD2 | NM_003927 | ACCCTTCAGGTGTTACTAGAA CTCGCGAGTGTAACTTTCATA TGGAAAGATGATGCCTAGTAA AAGATGATGCCTAGTAAATTA |
| 8936 | WASF1 | NM_003931 | TACACTCAGAAAGTACATTTA TTGCTTGCCTATAGGAGTTAA GCCCTATAAACCGATTAATTAA CAAGAACGTGTGGACCGTTTA |
| 8938 | BAIAP3 | NM_003933 | CTCGCCTGACTCCATCCAGAA CAGCCAGAGGACCCAGGTGAA GTCGACCTTGCTGGACATTAA TGGGATCATGACGACGATGTA |
| 8940 | TOP3B | NM_003935 | CACGGAGAAGAAAGAAGCTAA CACGGACACAGACATCTGTAA |
| 8942 | KYNU | NM_003937 | CCACATGTAACTAACAATAAA CAGATTTAAGATGGATAACAA |
| 8945 | BTRC | NM_003939 | CTGGTTTCAGGTGAACTTAA CTGGAGGCAGATGACATCTAA CTGGGCTAGAATAAATGTAAA CAGGATGAGCAACAACAGTAA |
| 8972 | MGAM | NM_004668 | TCGGCATGATATGAATTGGAA CTGGCCCATATTAACAGAGA CCCAATGGAAACGGAACTAAT TGGGAGGAATATATACCTAA |
| 8976 | WASL | NM_003941 | ATGCTACATATGAATGTTCAA CACATTGAGTAATATGCATAA AACCTTAATGTAATTTACTTA |
| 8986 | RPS6KA4 | NM_003942 | CGGCCAGGAGTTGCAGTCAA AAGGAGATAGGTGCTCCTAA CGCCACCTTCATGGCATTCAA CAGGCTGTGCCTTTGACTTTA |
| 8989 | TRPA1 | NM_007332 | CAGTAGACAGTTGCACATTAA CCACGAATTCATATCTAATAAA CACGAATTCATATCTAATAAA CTGGTTCTAACTTTCAATTTA |
| 8992 | ATP6V0E | NM_003945 | AACGTTAACACGACACATTTGAA AACCCTCTCTTTGACCGCAA |
| 8993 | PGLYRP1 | NM_005091 | CTGGTGCGACGTGGCTACAA CAGCTTCATGGGCAACTACAT TGGCATCAGCTTCATGGGCAA GAGGTCCAACTATGTGCTCAA |
| 8996 | NOL3 | NM_003946 | CCCAGTACCGCTGGAAGTGAA ACCCTGGAGATCCCAAACCTA |
| 8999 | CDKL2 | NM_003948 | TGCGAGAAATCAAGTTACTAA TCAGGCATTTATAACATTAAT TTCTACGGACTTTAAATTAAA ATGATGTGTTAGGTAATCTA |
| 9002 | F2RL3 | NM_003950 | CAGGCTGGTGCCCGCCCTCTA ACGCGTGAAACACAGAAGAAA CACATTCAGCGATACTGTGCAA AAGGCTGTACTGGGTCGAACA |
| 9015 | TAF1A | NM_005681 | AAGCAAGATCACCAAATCTTA CCGGATGCACTAAGAATATAA |
| 9019 | MPZL1 | NM_024569 | CTGGTTGCAGATAACGAACTA CCAGTCATATATGCACAGTTA CACCTTTCTTTATAAAGCGTCA AAGGCATTACTTAGAGATTGA |
| 9025 | RNF8 | NM_003958 | CAGCCTGTGTAGGCATTCAA ATGGACAATTATGGACAACAA |
| 9026 | HIP1R | NM_003959 | CTCCGACATGCTGTACTTCAA AAGTGTTCTGTTGGCAATAAA |
| 9028 | RHBDL1 | NM_003961 | CCGCTGGTACTTCTCACCGTCA TGGGATGGAGATGCCCCTACAA |
| 9038 | PNR | NM_003967 | CAGGCTCAGCAGATTACCACA CCACATGGTCTTTGACATCTT CTGCCTCATTATGATCAGCTT CACCATAGACACGATGGTCGA |
| 9045 | RPL14 | NM_003973 | CAGATTGATAGTAGGATTATA CACCAGAAGTATGTCCGACAA CAGCAGACGTTTGGAAAGAAA CAGATTTCTTGGTGGCATTAA |
| 9046 | DOK2 | NM_201349 | TGGGTGAGTCACCATCCCAAA GACAATGTTGTACTAAAGAAA CCCGAACAGGTGAAGGGACAA CAGCATTTGAAGGACACATAT |
| 9048 | ARTN | NM_057090 | CTGGTGTTGATAGAGATGGAA CCGGTGGTGGTGATGATATCAT CCGGCTAGTGCTTCTGTGATA CCACGTTATGTCAGCAATGAA |
| 9050 | PSTPIP2 | NM_024430 | ACGAATAAACTTCTTCCGGAA CTCAGTGGTGCAAGATTTAAA CCGGCTAGTGCTTCTGTGATA CAGGCATATGTCTTTAGTGAA |
| 9053 | MAP7 | NM_003980 | AAGCTGTTGTACGGCGCACAA AAGGGCTGTATCTCCATCTAA GTGCTCCTTCTTAGCAATCAA CAGGCATATGTCTTTAGTAA |

Table4

| | | | |
|---|---|---|---|
| 9056 | SLC7A7 | NM_003982 | CCCTTCTTGCTTAAATCCATA ATCAGATATTTGGAATATTTA TAGAGAGACATGAAACTATCA CAGGTGCTGGATGACAATAAA |
| 9064 | MAP3K6 | NM_004672 | CAGGAACTGAATGTGGATTCA TACCAGACAAAGCGTATTAAA TCAGAGGAGCTGAGTAATGAA CACCATCCAAATGCTGTTGAA |
| 9068 | ANGPTL1 | NM_004673 | GAGGCGGGTCATACTCCTTAA CAGAAAGTTTGGACAGCTTTA CTCGTGTTACTCAACTCTATA CGGGAGATAGATGTTCTGCAA |
| 9075 | CLDN2 | NM_020384 | AAGGCTAAGGGCTATAGACAA CCAGACTAATTTGTGCATGAA |
| 9081 | PRY | NM_004676 | AAGGACTCAGAGAGACATGAA AAGAAGGATCTTAAAGACTCA |
| 9086 | EIF1AY | NM_004681 | TTGGCTTTCATGAACAAGTAA ACCATAATATTTGCTGTTAAA |
| 9088 | PKMYT1 | NM_004203 | CTCCTACGGAGAGGTCTTCAA CTGCCTGGCCGTGTCTAATAA CTGGGAGGAACTTACCGTCTA CCGGCCAGAGTCCTTCTTCCA |
| 9091 | PIGQ | NM_004204 | ACGCTGCTGTGTGCTCCTGAA CAGGTGTTGCTGTCACAGCTA |
| 9092 | SART1 | NM_005146 | CTCAACCTTCATATTAAATAA CCCGTGTACAGTGACCCAGAA |
| 9096 | TBX18 | XM_496819 | TCGCCTGAAGATAGATAGGAA CAGCAATGCCTTCGCCACTAA |
| 9101 | USP8 | NM_005154 | CACGGAAAGAAGCACTTGTAA AAGGCTCGTATTCATGCAGAA CAGGTTCAGGCAAGCCATTTA CAGGGTCAATTCAAATCTACA |
| 9110 | MTMR4 | NM_004687 | CCGGCTGCATATCAAATTCAA AAGAGTGGCTCTCACGGCTAA TTGCCATAGATGTAACCTAAA TCGGCACTGGAGAGTACCAAA |
| 9114 | ATP6V0D1 | NM_004691 | CACTTTCATGTTCCTCCCTAA CCGCGCCTTCATCATCACCAT |
| 9118 | INA | NM_032727 | CAGCTAAATAATCTTACTCTA CAGCCTATTCCTGAACTATAA |
| 9126 | CSPG6 | NM_005445 | AACCGGTTACCAATCGATAAA TACCCAGGAACTGTAAATTTA GAGGACTAAGTTGGAGCTTAA ATGGAGGATATCGAACGCCAA |
| 9132 | KCNQ4 | NM_004700 | GACGATTACATTGACAACCAT CTGGTACTACTATGACAGTAT CGGGCATCTCTGAGACTCAAA CCGCTCCATCAGGATTCTCAA |
| 9136 | RNU3IP2 | NM_004704 | CTGGAGGAAACTGCACAGGAA CACTCTTATCCTCCTTATTTA |
| 9138 | ARHGEF1 | NM_004706 | CAGCAGCTCTGAGAACGGCAA TCGGAAAGTGCTGTCCCTGAA |
| 9140 | APG12L | NM_004707 | ATGGTATGTAGTAGAATTCAA CAGCTTCGATTTGAATGACTA |
| 9141 | PDCD5 | NM_004708 | CAGAACAAGGTTTAATAGAAA CAAGTCTAGGACAGAAGTTAA |
| 9146 | HGS | NM_004712 | TACGAGCAGCTGAACAGGAAA CACGTCCGGAGTAACACTACA GCACGTCTTTCCAGAATTCAA CCGGAACGAGCCCAAGTACAA |
| 9149 | DYRK1B | NM_004714 | ACGGAGATGAAGTACTATATA CTCGCTGAACCTGACCCGGAA CCGGACCTACCGCTACAGCAA CACGGAGATGAAGTACTATAT |
| 9150 | CTDP1 | NM_004715 | CCGAATATTATCAAGGGATGA CCCGACAAACTTCCCGATAGA CTCCATGGTTTGCATTATTGA CCGGCTGTACGCACACACCAT |
| 9156 | EXO1 | NM_003686 | GAACGAGTGATTAGTACTAAA AGGGTTAAATCTCCCAAGGAA CAGATGTAGCACGTAATTCAA ATGGATGTACTTTACCTTCTA |
| 9159 | PCSK7 | NM_004716 | CAAGCGCAGCGTCCACTTCAA TAGCTATGACCTCAACTCTAA ATGCCTTTCTATGCAGAAGAA TCGGAGCATTGTGACCACTGA |
| 9169 | SFRS2IP | NM_004719 | CACGAGGAAATGATCGGTACA ATGGTTCTCAGCTACCTATAA CTGGACAGGATTCTAGCCTAA AAGGAGGTGATCCATTGGAAA |
| 9170 | EDG4 | NM_004720 | CTACCTGAGTCTGATTCTTTA AAACCTTTCTTTACACTCTAA GACCATCGGCTTCTTCTATAA CCCGCGAGTCTGTCCACTATA |
| 9181 | ARHGEF2 | NM_004723 | CTGCAATGTGACTATCCACAA CCGGCGGGACCGAGTGCTTTA CAGCGATTGGTCAATCTCTAT CAGCACCCTACTGCCCTTTAA |
| 9183 | ZW10 | NM_004724 | CTCGCCTATCAGACTTTACTA GCCAGTGAATATATATTGCAA TACCACGAAGTGATGAATTTA ACCGGTGAATTTACAGACTTA |
| 9187 | SLC24A1 | NM_004727 | CACTGACTTACTACACCTCAA CAGTGACATTACAGCAACCTA CACAATGGGCATGGATCAGAA ACTGCAGATTATGATGGTAAA |
| 9196 | KCNAB3 | NM_004732 | AACCCTAGGGAACATCCTCAA AGGGAACATCCTCAAGAGCAA CCGAGCGAGGTTTAAGCCGAA TCGAGTAGTGATACGCATGAA |
| 9200 | PTPLA | NM_014241 | AAGTGAGTTCAAGACTCTTTA CTGGTGAACTTCTTACAAAT CACTGTTTAATTGGAATTGTA ACCACTTGCCATACTTCATTA |
| 9201 | DCAMKL1 | NM_004734 | CACCTTTAGGTTACCTATATA CAGTGGAATGTGACCAGTCAA CCGGAAGTGATAACACGCAAA CAGGTTTATAACTTCGACACA |
| 9211 | LGI1 | NM_005097 | GCCGTGTGTACTTGTACCAAA TCCCAGCGTCCTGTAATTTAT AAGCAACACAATTATTCACTA AAGATTCATTGGTGATTCCAA |
| 9214 | TOSO | NM_005449 | TACCGTGGTATCCACCACCAA CAGGTAAGCTGTAGGCATGTA CCGAGTTACTCTGAAGCAATA CAGGGTTGGGTCTTACACAAA |
| 9215 | LARGE | NM_004737 | CACGGCCAAGAACCTACATTA ACGGCCAAGAACCTACATTAA AAGGGTTATCATCTTACTGCA ACCAGCGAAAGCTGCATTTAA |
| 9219 | MTA2 | NM_004739 | TAGACGGATTGAGGAGCTCAA ACGGCCTTATGCTCCTATCAA CAGCCAAGCGTCAGAAACTAA ACGCCAGTTCTCAGAAATAAA |
| 9220 | TIAF1 | NM_004740 | CAGATTCTTATGCACGCTTGA CACGCTTGAGTCCGAGACTCA TACCCGATGTATGGAAATAAA AGGAGTGTCATTTGTCCCTAA |
| 9228 | DLGAP2 | NM_004745 | CAGGATGGAATTTCACAGGAA CTCATTATTACTCAAAGTAAA |
| 9229 | DLGAP1 | NM_001003809 | CACGAGGTAAAGATGATGAAA CAGGAAGAAGGACCACTTTAA |
| 9244 | CRLF1 | NM_004750 | CTGCTGGTCCAAGAACATGAA CTCAAGGATTTCCTCTTTCAA AACGCTGGATATCCTGGATGT AACACCAACTACTCCCTCAAG |
| 9247 | GCM2 | NM_004752 | ATGGATTACCATTGTACTAAA ACCCTGGTTATACAAATTCAA |
| 9256 | BZRAP1 | NM_004758 | CCGCCGTCTGGTGGTCCTCAA CAGGTCTTCCTAGCACGTTAT CAGAGCTAAATGGCTCCTTAA CACAGTGAGTATGTAACTTGA |

EP 2 295 543 A1

Table4

| No. | Gene | Accession | Sequences |
|---|---|---|---|
| 9263 | STK17A | NM_004760 | TGCCTAAATACTAGTAACATA TACTAGTAACATATCAGTGAA TCCATTGTGAACCGAAGAGTTA AACCAGGATATTTAACAGGTA |
| 9267 | PSCD1 | NM_004762 | AAGGATGAGATAGCAGAAGTA CAAGTTCTTGTTGGGCTTTAA |
| 9276 | COPB2 | NM_004766 | CAGTACGTATTTGGCATTCAA CCCAGTCAGGTTCAAGGGTA CAGGTTTCAAGGGTAGTGAAA ACGATTCTTCAGAGTATGCAA |
| 9278 | ZNF297 | NM_005453 | CCCGCCCATTCTACTACTCAA CAAGGTATTTCCAGAACTAAA |
| 9282 | CRSP2 | NM_004229 | CGGGTTGGGACTTAACTTTAA CGGGTGAAGTTTCGTGTTGAA CACGTTTACCTGACATATGAA CACGACAAGGTGAATGCACAA |
| 9287 | GPR58 | NM_014626 | CTGCCCAGTGATGTTCAACAA CACCTTGTTTATGGCAGGTTT CAGGATCCATATTCATCACAA CACATGTAATCCGTTAATATA |
| 9289 | GPR56 | NM_005682 | AACCGACATGCTGGGAGATTA CACCATCAAGGTGCACATGAA TCGGCCGCCCATCATGGTTAA GAGGGTCTGGCACATCCTTAA |
| 9290 | GPR55 | NM_005683 | CCGAGAGTTTGGACCCACATA TTGATTAATCTGATTCGCCAA CTGAGAGGACCTGATCATGTA CCGGTTCTTGGCCATCCGTTA |
| 9293 | GPR52 | NM_005684 | CAGGAAGCACAAGAACCCAAA AACGGGCTAATTCTTGTCTCCA CAGGTGTCCACGAGTCATTAA AACTGGCTTGCAGTAAGTAA |
| 9294 | EDG5 | NM_004230 | TCCAGGAACACTATAATTATA AGGCGTGGCCTTCGTAGCCAA ACCCACGTTTCTGGAGGGCAA |
| 9296 | ATP6V1F | NM_004231 | CAGTGTCATTGTTGATGTTAA CCGGCAATTTCTAAACCGGGA |
| 9311 | ACCN3 | NM_004769 | CACCCGGATGGGAAAGTGCTA TGGGAAAGTGCTACACATTTA CCCAAATAAAGTCCTAATGCA |
| 9312 | KCNB2 | NM_004770 | CTGCGGCTTTGTCCAGTTCAA CCCAATTATTGTGAACAATTT CCCAGTTGACATAACTGTGAA GCGGTTAATAATGCCTGTGAA |
| 9319 | TRIP13 | NM_004237 | ACCCAAATTGATCAGATAAA CAAGACTATTTAATGGATGTA TTGACACAGAATAAAGGTTA GAGGATTCACATTAATATAAT |
| 9322 | TRIP10 | NM_004240 | TTGGAAGAACGCAGTCGTGAA CACCATTGATGTACATACTCA ACGGCTGAAATTGGAAGTGCA ACGGCTAGACCAGGATATCAA |
| 9329 | GTF3C4 | NM_012204 | CAGGTCAAGCATAACAACGAA CAGGTAGATTTAATAGACCTA |
| 9332 | CD163 | NM_004244 | CCCGCCCAGAAGGAACTTGTA CACATGGGAGATTGTCCGTA TCGCATTATTCTTCTTGACTA CGCATTATTCTTCTTGACTAA |
| 9333 | TGM5 | NM_004245 | CAGGAGTATGTCATGAATGAT CTGCCTGAAGCTGCTAGACAA CTCCCAGTTCAAGGACCTCAA CAGATCCAAGGACCCTCAA |
| 9337 | CNOT8 | NM_004779 | ATGGCTTTCTTTAGGATGAAA ATGTCTGTAAATACTATTTAT |
| 9343 | U5-116KD | NM_004247 | CTGATTGAAGGTGTTGATCAA AACGCCTAATAAGAAGAACAA |
| 9349 | RPL23 | NM_000978 | TGCGAAATTCCGGATTTCCTT TTGCATGATTCTCCAGTATAT |
| 9356 | SLC22A6 | NM_004790 | CACCAGAACCACATCATTAAA CGGAAGTTACTCATCTTGAA |
| 9360 | PPIG | NM_004792 | CAGGAAAGGAGTAGAAGTAAA CTGGATAATTTGTACTGCTAA |
| 9361 | PRSS15 | NM_004793 | CCCGCGCTTTATCAAGATTAT CAGCCTTATGTCGGCGTCTTT CCGGGACATCATTGCCTTGAA TAGAAGCTATTTAATGATTAA |
| 9364 | RAB28 | NM_004249 | TTTGGGAAACAGTACAAACAAA ACCCTTCAAATTTGGGATATA CAGGGCAGAAATAGTGAAGTA CAGCCTATTGTCAGGGCAGAA |
| 9368 | SLC9A3R1 | NM_004252 | CAGGGCATCGTATCCTGAA CAGAAGGAGAACAGTCGTGAA CACCAGAAACGCAGCAGCAAA AGCGAGGAGCTGAATTCCCAA |
| 9369 | NRXN3 | NM_138970 | CTGGAGGGGTCATATATACAAT CATGGCTTACTTGTTCCATAA CAGGATTATAAAGAGATATCA ACCAGTGTCGTGTAACTTTA |
| 9371 | KIF3B | NM_004798 | CAGAAATGCATGGGTAAGGTA TTAGGGAAATAGCTTCTTTAA CAGAGAATTCACCGAATCCTA AACGCTAAGGTGGGTAGCCTA |
| 9373 | PLAA | NM_004253 | TAGTGTTGATTCTCAAATAA AAGAGTGTGAACACAAATTTA |
| 9392 | TGFBRAP1 | NM_004257 | CCGCCGATCGTCAAGAGGATA CAGGTACATAGTAATCCAAGA CACCCACTTAGCTGTGCTGTA CACAGAGGTAGCAAATGGCTA |
| 9394 | HS6ST1 | NM_004807 | CCCGTACACCTGGCCAACAA CGCGCGGCAGGACCATGGTT |
| 9404 | LPXN | NM_004811 | ATGGAAGAGTTAGATGCCTTA CAGCTCGTGTATACTACCAAT CTGCTTAATAGTCTTATAGAA ATGACTAGGCTGATAATCTTA |
| 9409 | PEX16 | NM_004813 | CCGGACCATCCTGCTGCTCTA CGCGGAAGTTAAGCAACCAA |
| 9424 | KCNK6 | NM_004823 | TCCGTCGTGTGTCTCTCAATTA CCGTCTGTGTCTCTCAATTAA CTGGTTGAGCTCAAATCCCAA ATCATCATAATACAACTTCAA |
| 9427 | ECEL1 | NM_004826 | CAGCATCCAGCTCTCAGTTAA CCGGCTCAAGTACACACATGA CAGGTGCTGACTGACAAGCAT CTGCAAAGTCTGGTCAATAAA |
| 9429 | ABCG2 | NM_004827 | ACGGATTAACAGGGTCATTGA ACCGAGCTCTATTAAGCTGAA CTGGCGAAGAATATTTGGTAA CTGGTCTAATTATTATTAATCTA |
| 9435 | CHST2 | NM_004267 | ATCGTAGTGTGTTTAAATAAA CCCTGTGGTGATACCTATAAA |
| 9436 | NCR2 | NM_004828 | CAGGCTCTCAGGCACAATCCA CAGGACATTACTGGTGTAGAA CACGGGCAGTCTTCTACGAGAA ACACACTTTGTGAATAATAAA |
| 9437 | NCR1 | NM_004829 | TCGGTTCATCCTGGACCCGAA CTGGATCTGGTGGTAACAGAA ATGGTTGTATATTCCTAATAA AAGCATGTTCTTACTGCTCAA |
| 9440 | CRSP6 | NM_004268 | TCAGTTCGTAATGGACCTGAA GTCCAAATTCCTAGTGATTTA CAGTACATGCAGTTCAGCAA CAGCCTACTCTAAATTATTGA |
| 9443 | CRSP9 | NM_004270 | TTGGAAAGTCAGGGCATCGAA TAGCATCATATATTAGTTCAT ATCAAGGAATATACGGATGAA AGGCAGTAATTCGCTGTATA |
| 9446 | GSTO1 | NM_004832 | GCCCTGCTTACTAGTGAGAAA CAGGAGTCAGCAATAAAGCTA CAGCATGAAGTCATCAATAT CAGCAATAAAGCTATGTCTGA |
| 9448 | MAP4K4 | NM_004834 | TCGGAGCTGCACCGAGGGCAA CAGGACTTTAGTTAGAATTAA AGGCAAGATCCTACCCGGAAA AACCATTAATTAATGTTTAATTTAA |

Table4

| | | | |
|---|---|---|---|
| 9453 | GGPS1 | NM_004837 | AGGGATGATTATGCTAATCTA TACACTCGTAATACCCTTAAA |
| 9455 | HOMER2 | NM_004839 | CGGAGCCAAGGTGATCATAAA CAGCACAATCACACCGAATAT CAGAACTAGGTCTACCTTCAA ACCGAATATGACCTTCACCAA |
| 9468 | PCYT1B | NM_004845 | AAGAGCCATGATCTAATTCAA CACCATAATACTCCTCTGAAA |
| 9469 | CHST3 | NM_004273 | CAGGTTGACTTAATTTATTTA CAGCTGTATATTCTAGTCCAA |
| 9472 | AKAP6 | NM_004274 | CTGCGGATGATTGCAAGTCAA CCGGGAGACTTGCAATCTGAA CACGTTTGTCACTGCCGTTTA AAGCATGTGGATGTACATCTA |
| 9479 | MAPK8IP1 | NM_005456 | ATGCAGTTTATTGTAATATAT CCGGCTCACCGTGCACTTTAA TGGCATCAGCTTACAGTGCAA CTGGAGGAGTTTGAGGATGAA |
| 9493 | KIF23 | NM_004856 | CAGAATAAACTCTGGGTTAAA CACCTTTGCCGTCATGCGAAA CAGAAGTTGAAGTGAAATCTA AAGGCTGAAGATTATGAAGAA |
| 9498 | SLC4A8 | NM_004858 | TGGGACCAGTACAATTCTCAA AAGCATCATCATCAGAATGAA AGCCGTCATTATTAACAGGAA GACGGCTATCTTAAAGTTTAT |
| 9508 | ADAMTS3 | NM_014243 | CAGGAAGCTTGGGTACCTTAA CCGCTCTAGCCTGACATATAA AACAATGTGATACGTAAATTA TAGGCAAATTCCAGATAGTAA |
| 9509 | ADAMTS2 | NM_014244 | GACAGGCAAGTTCATCTTAAA CCGGGTCTCACTGACGTACAA CCGCCGGAGGCTGGACCACAA CAGGCGAATGGGAGCCATGTA |
| 9510 | ADAMTS1 | NM_006988 | CGGCAGTGGTCTAAAGCATTA TAGGATAGTTAGTGAGGATTA CTGAGCAGTGATATAGCATAA CAGCAACGTGAAATAACGCAA |
| 9516 | LITAF | NM_004862 | CAAGGTCGTAAATGCATGCTA CTGGGCCTGAACATAATTTCA TGCCAATGTAGTCTCACTTAA GCCAATGTAGTCTCACTTAAA |
| 9520 | NPEPPS | NM_006310 | CAGACCAATGGGTGAAGTTAA CATGGTGATGGCACTACTTTA GAGGGATTTGCAGTTGATAAA CTGGGAATGGTTAAACACAAA |
| 9522 | SCAMP1 | NM_004866 | AACACTGTTGTTAACATCTAA CACCACATTTCTTATATTAAA |
| 9524 | GPSN2 | NM_004868 | GTGGATGGCCTATTACATCAA CTGGATCGGTTTCGCCATCAT |
| 9542 | NRG2 | NM_013982 | TTGTCTTATAATACTAGCTAA AGGGAAACTATTTATATGTAA CCGAGACATTCGCATCAAATA ACCCGTGGTGGTGGAGGGCAA |
| 9554 | SEC22L1 | NM_004892 | TAGGACATTCTCAAATTTCAA CCACAATTTGCTAACATTTAA |
| 9555 | H2AFY | NM_004893 | CTGGCTGTGGCCAATGATGAA AAGGCTTTGGTTTCCAGTTTA |
| 9559 | VPS26 | NM_004896 | CTGCATAATGTTGATTATAAA CACCAAGGAATTAGAATTGAA |
| 9562 | MINPP1 | NM_004897 | ATGAAATTCTTCCTACTTATA AAGTCGGAAAGTACAATGAAA CCGAGTGCAGATGTTATTAAA CACGGTCAAACAGATCCGCAA |
| 9570 | GOSR2 | NM_004287 | TTGGGAGTGATTGTGGTCTAA CAGGACAAGTACTTTATGATA CAGCATTCCCACAGCCTGCAA AACGAAATCCAAGCAAGCATA |
| 9575 | CLOCK | NM_004898 | AAGGAGCCATCTACCTATGAA GACACGCATGATAGAAGCAAA ATCGGCAACAAGAAGAACTAA ATCCAGCAACTTGCACCTATA |
| 9580 | SOX13 | NM_005686 | AAGGAACTCTATGGAAGCCAA CACCATCAAGTCAGAGGAGAA |
| 9586 | CREB5 | NM_004904 | TAGCTTTAAATACATCATTAA CAGCAATAAACTTACAAGTAA |
| 9589 | WTAP | NM_004906 | AAGGTTCGATTGAGTGAAACA GGGCAAGTACACAGATCTTAA CAGACTAAAGACAAACTGGAA ATGCAAGAGTGTACTACTCAA |
| 9598 | CSAG2 | NM_004909 | CAGCTGTGTGGTCAAACAGTA CTACCACAAGACTAACATAAA |
| 9605 | C16orf7 | NM_004913 | CTGGGTGTATCTCAGAGATAA CGCCGTGTATACTCCGATGAA |
| 9609 | RAB36 | NM_004914 | AAGACAGTACAAAGCATATAA GTGGCCATTCTTGTAGTTATA CAGCTCAGCGATTGCCAAATA AGGCACTGTGGTGATCCCATA |
| 9611 | NCOR1 | NM_006311 | CCCGGTGGTTCCTAGAAACA CCCGCTCACCAGGGAGTATAA AGGCAATTCAGTGGACTATAA ACCGCAGTATTGTCCAAATTA |
| 9620 | CELSR1 | NM_014246 | CACCATGATGGAGCTGGACTA TGGCGTTTCCTAAGTACGAAA CGGGATCCTGGATGTGATCAA CGCCAACAGTGTGATTACCTA |
| 9625 | AATK | AB014541 | CCGGTTCCGCTGAGATCAGAA CTGACTCAGCTAGACCCGTAA TCCGCTGAGATCAGAAGGCAA CTCCAACGTGTCAGCCAACAA |
| 9629 | CLCA3 | NM_004921 | CAGAGTACTTTCGGTTCTGAA CCCAGCGAAGAAATGGATAAA GAGAGCTACATTATAAGAATA TAGGCCCGAAATGACTAGCAA |
| 9636 | G1P2 | NM_005101 | CAGGATCAAGGGCCGGAAATA CCGGAAATAAAGGCTGTTGTA AAGCACCGTGTTCATGAATCT AATGCGACGAACCTCTGAGCA |
| 9638 | FEZ1 | NM_005103 | CACAGATAAACTCATCCTGAA ATGGAGGACCTCGTAAATGAA |
| 9640 | ZNF592 | NM_014630 | TCAGACTATATTTCAAATAAA CCCACTAAAGAGAGTTCTAAA |
| 9648 | GCC2 | NM_014635 | ACGGGAAGACTTAGAGTTTAA ATGGATAATTTCCATAAGAAA |
| 9673 | KIAA0446 | NM_014655 | AGGGTGATGTGTGTATAATTA CCCGTGGTGGGCACAGTTCTA CACCATGGAGGACAAACGCAA CCGGAAGTTTGTAGCTGACTA |
| 9674 | KIAA0040 | NM_014656 | ACCCTGTGAATCATAATTTAA CCGAGTGATCTTCTCTTCTAA |
| 9679 | FAM53B | NM_014661 | ATGGCGAGACCTGGACAGGAA GACAAGAAGGTCGGTGTTAAA CGGGAGATGCTTCCTTCTGTA TACTTGTACTGTGTAAATAAA |
| 9688 | NUP93 | NM_014669 | TCGAAGTTTCCTGAACATTAA AGGGATGAGAAAGATAGTCAA |
| 9706 | ULK2 | NM_014683 | CAGCCTGAGATACGTGCCTTA TAGGATAATCGGTACAGTTAT ATCGGTACAGTTATTCTTAAA ACCGGGAAGTTATCAGATCAA |
| 9716 | AQR | NM_014691 | ACGATTGGAATTAGAATTAAA CAGGATGGATTTAGCCGACTA |
| 9717 | KIAA0420 | XM_032693 | ACGGTCCACCCTGAGAATGAA CAGGCGGAAGTTCCTCATCTA |

EP 2 295 543 A1

Table4

| 9719 | ADAMTSL2 | NM_014694 | CCAGGAGTTCTTCTCGGCTAA CACCCTCAATGAGACTGTGAA |
| 9720 | KIAA0565 | XM_113947 | TAGTCTTGTAGTATGTTTGAA TAGCGTTACAATAGAATAGTA |
| 9722 | CAPON | NM_014697 | ACGGTATGAGTTTAAAGCCAA CAGCAATATCTTCAGGTGTAA |
| 9726 | ZNF646 | NM_014699 | AACAATTTATTTAAGTTTAAA CGGGAGCTAGAAGACAATGAA |
| 9727 | RAB11FIP3 | NM_014700 | CACATGAAATATATATATATA CCCGTCCATCCTGGAGGTCAA |
| 9728 | KIAA0256 | NM_014701 | AAGGATGAGAATATTCAACAA CCACATAAGTTTATAATTTAA |
| 9730 | VprBP | NM_014703 | CAGAGTGAACTCAACAACCAA CAGGGTGTTACTCCTCACCAA |
| 9733 | SART3 | NM_014706 | CAGCAGCTGGAGAAATATAAA ATGGAGTTGATCATCAAGTAA |
| 9735 | KNTC1 | NM_014708 | CAGCACATTAAAGCTCACGAA CAGGCAGTATATCCAGTTAGA TGCTCTTAATTTCGAAATTAA TCCCGTGGATCTAGAATATCA |
| 9737 | GPRASP1 | NM_014710 | TGGGTCTATAGATGTAATTAA GGGTCTATAGATGTAATTAAA CTAGCAGTAAATCCAGACCAA ACGCCCTAAGAGTAAGGCCAA |
| 9744 | CENTB1 | NM_014716 | CACCGTGAGCCTGAACCACAA ACGGGCCAGCAACGCATTTAA |
| 9746 | CLSTN3 | NM_014718 | CCCAGTCAAGTTCCTCTGGAA CAGGTGAAGCCCACCTGTAAA |
| 9748 | SLK | NM_014720 | GACCATTAACTGAGTCCCAAA TCGAATGGCCATGTTTAAGAA TAGCATCTTGTGATCACCCAA CCAGATCAGGACCGTGATAAA |
| 9751 | SNPH | NM_014723 | CACGATGTTCATCCAGGCCAA ATGAGGGTTCCTAAACCTTAA |
| 9761 | KIAA0152 | NM_014730 | AAAGATATAATTAACGATCAA CAGGATTGTGCTGGCAGCCAA |
| 9767 | PHF16 | NM_014735 | TCGGTGGGCCTTAGTCTGCAA CACGCTAGCTGTGGACTTTAT CACCAATAGGTGGGTGAAGAA ACCATGATTATCAGACACTAA |
| 9775 | DDX48 | NM_014740 | CCGGAAGGGTGTGGCCATTAA CCCATAAACTCTATACTTCTA CCGCATCTTGGTGAAACGTGA AAAGAGCAGATTTACGATGTA |
| 9780 | FAM38A | NM_014745 | CTGCGTCATCATCGTGTGTAA CAGCCTTGTATGCACCGTCAA |
| 9781 | RNF144 | NM_014746 | TGCGATGACCACAACAAGGTA AAGGATTAGAAACCGCAATTA GTGGAGAAAGTGTATTCTTTA CAGGAATAACTGTCCAAATTA |
| 9783 | RIMS3 | NM_014747 | CTCCAGTATCCTGACATGTAA CAGGGAAGAATCATCTCCAAA |
| 9784 | SNX17 | NM_014748 | ACGGAAGTTGCAAGAGTTTGA CTCCGCCTACGTGGCCTATAA CAGCGAGACTTTCAACAGTTT CTGGCCCTCGATGCCAAATTA |
| 9797 | TATDN2 | XM_376203 | ACAGTTCTAAAGGGCTTTATA CAGCCATGTGTGGATCTTGAA |
| 9806 | SPOCK2 | NM_014767 | CACATGCATAAAGGAAATCAA CACGTTTGTGTGTCCTTGTAA |
| 9810 | RNF40 | NM_014771 | CAGCTTAACTCTGGCTACTAT CACGCTGGCCCAGGTACGCAA GCGCATCGAGTTTGAGCAGAA AAGCGGAAGCTTCGAGAAGTA |
| 9811 | KIAA0427 | NM_014772 | CCCGGATTAGTGGGAGATCAA CAGAACGAGACTGGCAAATTA |
| 9815 | GIT2 | NM_014776 | CCCGTTGATTATGCAAGGCAA CAGCGTTGAGAGTCAAGACAA CTGCCTATGGCTGAAACTATA AAGCATCGTGCAATAAGTGAA |
| 9818 | NUPL1 | NM_001008564 | AAGGTACAAGAAGATATTAAA AAGCAAATAATTCACATATAA |
| 9819 | KIAA0669 | NM_014779 | CAGGATTGCATTTAATAGATA CTGGCATATTTCTAACACTAA |
| 9825 | SPATA2 | NM_006038 | CACAATGTTAAGGGATTTATA CCCAGTTCAATGGATACTAAA |
| 9826 | ARHGEF11 | NM_014784 | CACTTGTATCTCCTTAGTTAA AAGCGATTCAATCCTGATCAA CTGGAGGATCAGCAAGGATAA CACAACGACTCTCGACCGGAA |
| 9830 | TRIM14 | NM_014788 | CACCGAGAAGCTCAAGGCTAA GCGCCTCACCTTAGCATTCAA CAGGTGCCTATCACTGAGTAA AAGCAGAGCTGCGTAAAGTTA |
| 9832 | KIAA0555 | NM_014790 | CTGCATTTAATGAAACATGTA CACCTGCTTGAAAGAAATTAA |
| 9833 | MELK | NM_014791 | CACCTCGATGATGATTGCGTA AAGACTAAAGCTTCACTATAA CTGGATCATGCAAGATTACAA CGGGTTGTCTTCCGTCAGATA |
| 9834 | KIAA0125 | NM_014792 | CAGCATGGTGACTATAGTTAA CAGGAACAGTTCTGAAGTCAA |
| 9836 | LCMT2 | NM_014793 | TAGATTATAAGTGTCAATAA ATGGCCATTAATGTTACACAA ACCCACTAAAGTAGACAATAA CAGAGGCATTTCCTCGCGTAA |
| 9837 | KIAA0186 | XM_375911 | ACAATTGATGTTGAACTTGTA TTGGGAGAATTGACATCTTAA |
| 9842 | PLEKHM1 | NM_014798 | CCCATTCATATTTCAACAGAA CAGGCAGCCTCTGGAACTCAA |
| 9846 | GAB2 | NM_012296 | CAGACCAGACGTTTCATTCTA CACTAAGGACACAAGAATCAA CACCAATTCTGAAGACAACTA AACCTCAAACCTGATCGGAAA |
| 9848 | MFAP3L | NM_001009554 | CCGAAACTGCAGAACCTTCTA CACCATCGTCATGGTCCTCAA |
| 9851 | KIAA0753 | NM_014804 | CAGAGAAAGGAAGAAACTTAA CTGGCTCAGTTTGGTGATGAA |
| 9853 | RUSC2 | NM_014806 | ATGTATTTATTTATTTATTTA CCCGATCAAGACCTAGGACAA |
| 9854 | TMEM24 | NM_014807 | TAAAGTCAAATTGGAGTTTAA CACAATTGAGGAACTGATCAA |
| 9855 | FARP2 | NM_014808 | CAGGAACATGCGCCAGTTAAA CAGGTTTGGACACAACTACAA |

Table4

| | | | | | | |
|---|---|---|---|---|---|---|
| 9860 | LRIG2 | NM_014813 | AGGGATGTCATTGTAAATATA | CTGGAATATACTAGTATCTTA | | |
| 9863 | AIP1 | NM_012301 | AACCCATTCATCGTAAACAAA | CAGACCTAAACTGATCCTAAA | TTGGATGTCATCCAAACTTTA | ACGCAGTGACTACGCAACCTA |
| 9871 | SEC24D | NM_014822 | CAGCTGCTTAATTAATTGAAA | AAGGTACAATTGTACCATAAA | | |
| 9877 | KIAA0663 | NM_014827 | AAGATTGATAGTGAAATTAAA | CAGCTAATGATTACAGTTTAA | | |
| 9880 | KIAA0352 | NM_014830 | AAGCTTAACATCAGAATGAAA | CTGGGTGGTCTGTGTACTTAA | | |
| 9890 | LPPR4 | NM_014839 | AAGGAGTTGTTTACAGATGAA | CACATTGTTAAGGACATATAA | | |
| 9894 | KIAA0683 | NM_016111 | CCAGTTTGTGATGACCCAGAA | CCGGGAGACGCTGCTGGGCAA | | |
| 9896 | KIAA0274 | NM_014845 | GAGGGTGATGTTGCAAATGAA | AAGAAATTCCACAAATATGAA | | |
| 9899 | SV2B | NM_014848 | CTGGTTGATTTGAGAGATAAA | AAGATGGATCTTGTAAATTTA | | |
| 9901 | SRGAP3 | NM_014850 | AACCAGCAAGTTGTTCATTTA | AACCTAGTTAGTAGTCTTCAA | | |
| 9908 | G3BP2 | NM_012297 | ACCAGCGTGTGTACACATAAA | CAGCCAAGAGTCGAAGCTAAA | TTCAGTGAATGTCATACTAAA | CCGAGTGGCTCCTGAATTTAA |
| 9914 | KIAA0703 | NM_014861 | ACGGACCTCTTGGTGGATGAA | CAGGCTGAAGAGACACCTAAA | ATGGACTTAAGTGATATTAAA | CCGAGAGTTGGCGTGAAGGAA |
| 9915 | ARNT2 | NM_014862 | CAGAATAACCACCATGAGGAA | TAGCTTTAGCCGAGAAAGAAA | CCGGAACAGCTCTAGGAATAA | GTGGACAGTTTAAGTACTTAA |
| 9921 | RNF10 | NM_014868 | TCCAAGCGTTATAATCGCAAA | CCGGGTAGTTCTGGAGGAGAA | TTCGATGAAGAAACCACGGAA | CAGGATTGAGATAGAGGAGAA |
| 9936 | DCL-1 | NM_014880 | CAGAATTATGTGCATACCAAT | ATGGCGTATTATGTGAACTAA | GAGGCTATTTAAGTAAATTTA | TAGCTAGTAATAACAATATAA |
| 9940 | DLEC1 | NM_005106 | ACAGCCTGACATGGAATTTAA | CAGGTTCTGCATTATGCCCAA | | |
| 9941 | ENDOGL1 | NM_005107 | CTCGGGCAAGATGGCTATCAA | CAGAGGCAAGGTGTTACACTA | AAGAAGCTAGAAGAACTCAAA | AACAGTGGAATCCTAAATTTA |
| 9949 | AMMECR1 | NM_015365 | CCCACTGTTTGTAACATGGAA | CACACTTACCAGTGCCCTTAA | CTGACCAGGTATCGTAGTGAA | CGCCAGCATCATCATTTCCAA |
| 9950 | GOLGA5 | NM_005113 | CTGGACCTAAATCTACGTATA | AAGCAGGAGTTCCACTATATA | AAGAAGATCTTTATCGAACAA | CACGACCAACCATATGGCAAA |
| 9951 | HS3ST4 | NM_006040 | CCGTGTTGTTATAAAGCTTAA | CAGAGGATAAATAGTTGTCAA | | |
| 9958 | USP15 | NM_006313 | ATGAATAATGTTGTAACTCGA | GACACAATAGATACAATTGAA | AAGATGATACCAGGCATATAA | TATGTCGGAATTCTTAATTAA |
| 9965 | FGF19 | NM_005117 | TTGTCTGATCATAACATTGTA | AAGAAACATCTAGAAGTTGTA | CAACATGATATTTATGAGTAA | CCGTAAGGCGCTACTATATAA |
| 9970 | NR1I3 | NM_005122 | AAGCCACAGGCTACCACTTTA | TTGAACAGTTTGTGCAGTTTA | CACACACTTCGCAGACATCAA | AACACTTTCATGGTACTGCAA |
| 9976 | CLECSF2 | NM_005127 | AAGCAGTAATGTCCTAAAGAA | CCCAGTTGAGAAAGTAAGAAA | CAGCTAGATGTTACACCGAAA | ACGTGTCAGGCTACACATAAA |
| 9978 | RBX1 | NM_014248 | CACATTATGGATCTTTGCATA | TTGCATAGAATGTCAAGCTAA | CTGCTGTTACCTAATTACAAA | AAGAAGCGCTTTGAAGTGAAA |
| 9985 | REC8L1 | NM_005132 | CCGGAGAAATTCCAGGAACAA | CAGAGGGACAATGTTCTACTA | | |
| 9988 | DMTF1 | NM_021145 | ATGGATAAGCATAGTAACAAA | CAGATTATTGTTCATGCTTTA | | |
| 9989 | PPP4R1 | NM_005134 | CAGCTAGTAATGAGAATGATA | AACCTGGTAACTACAAATCTA | TGGGAGTGCTGCAGTCTTTAA | CAGGCGTTGTTAGATCAGTAT |
| 9993 | DGCR2 | NM_005137 | TCGCTGGGAGGTGGCATTCAA | CTCCCTCAATACTGTGGTGTA | AAGCCCAGTTGTGAATATCAA | GAGGTGGGATTTGATACTGTA |
| 9994 | CASP8AP2 | NM_012115 | CAGCTGATGTGCGGAAGTCAA | CAGTATTCAAATAGTCAGAAA | CAGTCTGATCTCAATAAGGAA | AACGAAGTTTCGAGATTCTTA |
| 10001 | MED6 | NM_005466 | TCCCACTAGCTGATTACTATA | CACCCAAATTTGTGCAGCTAA | AAGGGTATTGGTGGCACTTCA | ACCCAAATTTGTGCAGCTAAA |
| 10002 | NR2E3 | NM_014249 | CAGGCAAGACTAATTGACAAT | AGGGAATACTCCAATGGAGAA | CACGGAGTTTGCCTGCATGAA | CACAAATGTATTCATACGTAA |
| 10003 | NAALAD2 | NM_005467 | CAGCAAGTATCTATAATCTAT | TCGATTGTGGATGAACATGAA | AAGAAACATGATCAACAATTA | ACGGTGGAAACTGGTATCCGA |
| 10004 | NAALADL1 | NM_005468 | CACAGAAGAGTTCTTCAACAA | ACGCATATCAACACTGCAGAA | TCCCTTTACTCCGGTGCTTTA | CTCCGGTGCTTTATATTTACA |
| 10019 | LNK | NM_005475 | CAGCTTTACAGTGATAGTTAA | CAGCACATTGTGATAACATAA | | |
| 10020 | GNE | NM_005476 | CTGCTTACTAATGTATTAATA | ATGAAGGTTTCTACAACATAT | CCCGATCATGTTTGGCATTAA | ATGGAAATACATATCGAATGA |
| 10025 | THRAP5 | NM_005481 | CACCCGGATCCTGGCCATGAA | CAAGGTCATGATCAACCTCAA | TGGGAAGGACAGGTCCAATAA | CACGTGCAGCCCAGTATGGTA |
| 10039 | PARP3 | NM_005485 | CCAGACCAACATCGAGAACAA | CCCAGTGAGGACTGTGACTAA | CACGCAGAAGCTCATCACTAA | CAGCAAGGAGATGTTCAAGAA |
| 10044 | SH2D3C | NM_005489 | CCAGAGAAACTCCACAAGGAA | CAACCAGGCCTTGCACTTCAA | CAGGCGAGAGCTACACACACA | CCACACCAATGCTGAAGTCAA |
| 10046 | CXorf6 | NM_005491 | AAGAATAAATTTGCAGGTCAA | CCGTCTAACATTACTCATGTA | | |
| 10048 | RANBP9 | NM_005493 | CAGTGCAATATTAGAAACCCA | ACGGTGTGGTTATGACCTTTA | CAGGTTGGGATAAGCATTCAT | AAGCAATGAGTGAACAGCTAA |
| 10050 | SLC17A4 | NM_005495 | ACGCTAGTTATTTAACTGCAA | GAGCAAGATAGTACACTTTAA | | |
| 10053 | AP1M2 | NM_005498 | CTCCGAGGGTATCAAGTATAA | CTGGATTGAGTCTGTCATTGA | | |

Table4

| | | | | | | |
|---|---|---|---|---|---|---|
| 10054 | UBA2 | NM_005499 | AAGAGTAGAGATTTGATATCAAA | CTGGGTATCTTGGACAAGTAA | | |
| 10055 | SAE1 | NM_005500 | TACCAAGTCTTTGGATGTTAA | TCCAGGGATGTCATAGTTAAA | | |
| 10060 | ABCC9 | NM_005691 | AACAGAGAGGACATTGCAATAAA | CAGCCAGATATTGACTTATTA | CTGCATCTATAGCATCCATTA | CCCAAATTTGTTCGCCCACAA |
| 10062 | NR1H3 | NM_005693 | CCCACAGAATGAGAAGATTAA | CAGGAGTGTCGGCTTCGCAA | GAGATAGTTGACTTTGCTAAA | CATGAATGAGCTGCAACTCAA |
| 10063 | COX17 | NM_005694 | CTGAAGAATGAAGAAGATTAA | AAGGAATGCATGAGAGCCCTA | | |
| 10068 | IL18BP | NM_005699 | CTGCTTCGGATCCACACTGTA | TCCCATGTCTCTGCTCATTTA | CAGGATGTGGACGGACTGGTA | ACCGATCAAGTCAACTCAGTA |
| 10077 | PHEMX | NM_005705 | CAGCAATGGTGCATTGAGCAA | TCAAATATACATCACATCAAA | | |
| 10086 | HHLA1 | NM_005712 | CCCAAGTGGGCTACATTTGAA | CTGGAGGGTCTCTCTGGGAAA | AAGCATGTTAATGTTACCATT | CTCCAGCTAGATCATCAGTTA |
| 10092 | ARPC5 | NM_005717 | CAGCCTCTGAATGAAATGCTA | CAGGGTAGTTGGGATCTCAAA | | |
| 10094 | ARPC3 | NM_005719 | GCGCTTAATAAGAATAAGTAA | CTCATGATCCTGATACCAAA | | |
| 10095 | ARPC1B | NM_005720 | AAGCACTATGCTGTCATGAA | TCGTGTGATCTCCATCTGTTA | | |
| 10102 | TSFM | NM_005726 | CAGGAAGGAAACACCAACTGTA | CAGCAAGGAGCTCCTCATGAA | | |
| 10105 | PPIF | NM_005729 | CTGTGTGTTAAAGAAATTCAA | ATGGATTTGTGTTCACCTTAA | | |
| 10107 | TRIM10 | NM_006778 | ACGGACACTAGAAGCACTCTA | TTAGTTTGTTAACTATGAA | TCCAATAAATGTTAGAGTTCAT | CAGTTGTGTATGACTTAAACAA |
| 10111 | RAD50 | NM_005732 | TAAGATTGAATTCGTAA | CACGAGTAAGATTAAGAGCAA | CAGGAACAGATTCAACATCTA | CAGAAAGTGTATAATAAGAAA |
| 10131 | TRAP1 | NM_016292 | CCCGGTCCCTGACTTCAGAAA | AAGGATGTCGGTGAGTGGCAA | CCGCTACACCCTGCACTATAA | CGCGCTCATCAAGAAGCTGAA |
| 10134 | BCAP31 | NM_005745 | CTGCACGTTGACTGTGGGAAA | CACCTTCTTTGTGGTTCTCAT | CAAGAAGTACATGGAGGAGAA | AAAGGTGAACCTCCAGAACAA |
| 10136 | ELA3A | NM_005747 | CACATCCTGAATAAAGAAATAA | TGGCTGCAACTTCATCTGGAA | CTGGCTTTGGCTGCAACTTCA | AAGAATAAAGATCTCTCGGAA |
| 10139 | ARFRP1 | NM_003224 | CCGATTTAACAAGAACTACAA | CACCACCACCGTGGGCCTAAA | | |
| 10140 | TOB1 | NM_005749 | CAGCCTGTTATGGCTAACTAA | AGCCTGTTATGGCTAACTAAA | CCAGCCTGTTATGGCTAACTA | CAGATATGGCCTCTTACAGTA |
| 10142 | AKAP9 | NM_005751 | CAGGTTCGTGAATATATGGAA | ACAGATTATATCACTCGGCTA | CAGCTTCAAAGGGATATACAA | CAGCCTATCAGTGAACATCAA |
| 10154 | PLXNC1 | NM_005761 | TTGGTGAGAATTTGACTTCAA | CCCAACCAGAGCAACCTACAA | TCGAGGGAAGCACAAGTTCAA | GACGTCGTACATATTTGGAAA |
| 10158 | MAP17 | NM_005764 | CCCTAATTTCTGTGAAATAAA | CCGGCCCTACCCGCCCTTGAA | | |
| 10165 | SLC25A13 | NM_014251 | CAGACCAAAGATGGATTAATA | CTCAGAAATTTGGTCAGGTTA | TAGCAGAGATTTATATGAGCCAA | CAGTATCTACCTCAAAGGCTA |
| 10166 | SLC25A15 | NM_014252 | AGCCTTATGCACCTAATTCAA | CACCAGCAGGGTATCTCCCAA | ATAGGCCTGGATCGAAGTAAA | AGCCGTCCATTAGAGATTTA |
| 10168 | ZNF197 | NM_006991 | TCAGGCCTAATTATACATCTA | CACCAATGAGTCCAAGATTGA | TCCCAACTTTCAAGTCTCACAA | CACCTACACTTTAGACAATTA |
| 10178 | ODZ1 | NM_014253 | CCCAGTCATATCAACCATAAT | CCGACCTGCCTTACCTTTAA | AAGGCTATAATCCGGCCTTAA | CTCTATGATTTCCTTACTAAA |
| 10180 | RBM6 | NM_005777 | CAAGCAAGAATTAATAACCTA | CAGACTGGTCTTCAGATACAA | | |
| 10188 | TNK2 | NM_005781 | TGGCCTTACCATGGACTTGAA | CAGGATCTTGTGCCTGGAAAT | CGGCAGTCAGATCCTGCATAA | ACGCAAGTCGTGGATGAGTAA |
| 10189 | THOC4 | NM_005782 | CAGGAACTCTTTGCTGAATTT | CCAGTTAAAACAGACCAGCAAA | | |
| 10194 | SDCCAG33 | NM_005786 | CACGGGCAAGGTCAACATCAA | ACCGCGGAACATTGCACTAAA | | |
| 10202 | DHRS2 | NM_005794 | CAGCACCGGGATAGACAGGAA | CTGGAAGAACTTCAAGGAACA | TAGATTTGGCTGATCCAATTA | CTCTCTCGTAATTTGTGCTTTA |
| 10206 | RFP2 | NM_001007278 | CAGAATTTGTGTGCAAATATA | AAGGAGATCATAAGAAATTAA | | |
| 10208 | C13orf22 | NM_005800 | CAGCCTCAGTTTCACAGTTAA | CTGAATGAAGTTAGAGATGAA | | |
| 10213 | PSMD14 | NM_005805 | TAGGACATGAACCAAGACAAA | CAGGCATTAATTCATGGACTA | TTGGATACTGTCGTATTTAAA | |
| 10218 | ANGPTL7 | NM_021146 | TCGGGTTGGAAGGAAAGCTA | CCGCATCCTCGGAGTGTATAA | ACCGCCTGGGTGAGCACAATA | CTGAAGGTAGATGGTGTATA |
| 10220 | GDF11 | NM_005811 | AAGGTGATGTTCACAAGGTA | TTGGCAGAGCATCGACTTCAA | CAGAGCATCGACTTCAAGCAA | CTGAAATGAGTAATAAATGA |
| 10228 | STX6 | NM_005819 | CAGGCTGAAGCTAAATAGAGA | TCCCAGGTTTCTTAAGAATAA | CTGAAGCTAAATAGAGAACAA | AACCTAGTCATTAATGAGTAA |
| 10231 | DSCR1L1 | NM_005822 | ATGGATTACTTTAGATGCAAA | CTCAAAGAGCTTAGACTTCAA | | |
| 10234 | LRRC17 | NM_005824 | AGCGTAATAATTACTATAGTA | ATGCAGGGTAATCCAGCTAAA | AAGGAACAATTGGACCCGAAA | CCCGTGTGTGACGTGTACACATA |
| 10244 | RAB9P40 | NM_005833 | TAGGTGAAGAAACTAAATGCAA | AAAGAGGATTCAGCTGACAAA | ATTGATATAGTGACATGAAA | AAGCAACATGGTACACCTTGA |
| 10245 | TIMM17B | NM_005834 | CAGCACTTCCTCCAGCTCCAA | GGCACCCTGATGAGTATTTAA | | |
| 10250 | SRRM1 | NM_005839 | CAGAATAAATTTCTAAATTTA | TAGGTTGAAGTTCAACATGTA | | |

Table4

| 10257 | ABCC4 | NM_005845 | TTGGCTATACTGCATCACTTA TACAACCAATAGGAACAACAA CTCCTAGTACTTAGAAATACA GAGGTTAAAGACAGTCATATA |
|-------|-------|-----------|-----|
| 10261 | IGSF6 | NM_005849 | CAGAATGTAAGGAAACTATAA CAGGAAATTGCTCAAGAACTA |
| 10262 | SF3B4 | NM_005850 | CTGGATGAGAAGGTTAGTGAA CACAGGCAACTCCAAAGGTTA |
| 10267 | RAMP1 | NM_005855 | CACCTGGCACATGGCGGAGAA CCCGCCCGGCAGCATCCTCTA CAGGTTCTTCCTGGCAGTGCA CAGGGACGTGACCTTGACTTA |
| 10276 | NET1 | NM_005863 | ACGGAAAGAGACTTTGGTGTA GAGCATCATAGGGTTACTTTA TCGGTGTGGATTGATTGGAAA CTGGAGGATGCTATATTGATA |
| 10279 | PRSS16 | NM_005865 | TCCGAGTATAATGACGTGGTA ACGTGGTATCCCGAAGCCTAA CAGAGAGTTATGGAAATATAA CTGTTCTTTCCTAAAGATCAA |
| 10280 | OPRS1 | NM_005866 | CAGCGTCTTCCATTCCAGAAA TGGGAACAAATGAGACACATA CCGGCTTGAGCTCACCACCTA AGGGATATCCATGCTTATGTA |
| 10282 | BET1 | NM_005868 | TCAGATTAAATAGCATTATAA ATCATTTATTGGATTATTAAA CAGAATAAATTATTAGCTGAA CACTGATGTCGTGGCGCTTTA |
| 10284 | SAP18 | NM_005870 | AAGCCACGGGATTGTATGAAA TCCCTTTAGTTTGTTACAGTA AACGGCCTATAGGATTGTTAA AGCCACGGGATTGTATGAAAT |
| 10290 | APEG1 | NM_005876 | CCCGAGGACACCACCACCGAA CAAGTGTATCATCACTGCCAA CAGCTGGAATAACAAGCCCAA AACACCTATTTCTTAACTCAA |
| 10291 | SF3A1 | NM_005877 | CAGGTGTGTTACCGAGTGGAA CGCAAGGATTATGATCCCAAA CAGGATAAGACGGAATGGAAA CAGCATGTAGGTAGCGTCCTA |
| 10293 | TRIP | NM_005879 | CCGGCTCAGGAGCAAGATGAA AAGGAAATCATGAGCCTGAAA CGGGACCAGCCTGAGGTGTAA CCGTGATGATATTGATCTCAA |
| 10300 | KATNB1 | NM_005886 | CTGCTGTAATTTATAAGGCAA CAGGGAGGAGAGGCTGCATAA |
| 10322 | SMYD5 | NM_006062 | CTCGAGGAGCTGGGCTAGATA TTGGATGCTATGGGAATTACA CTGGCCTTATGGAGTATGGAA AACATTGAAGTCTTCCAGAAA |
| 10328 | NOC4 | NM_006067 | CTGGACAAACCCAGAGATCAA TGCAAGATTGTTCCTATTAAA |
| 10329 | TMEM5 | NM_014254 | CAGCCTCAGGAAACAAATGAA TCCCTTTATCTTTATCAAGAA |
| 10344 | CCL26 | NM_006072 | AACAATTGTGACTCAGCTGAA TCCCAGAGTTACTTTAATAAA AAGCTATGAATTCACCAGTAA CAGGGCCGTCTCAGTCTCATA |
| 10352 | WARS2 | NM_015836 | CCGGCATTCAACCTACAGGAA CTACCTCGATTACAACATTTA GCCAATGGATGCGAACTTAAA AAGGTAGATCTATCAAGCCAA |
| 10360 | NPM3 | NM_006993 | TTGGATAAAGGTTGAAATAAA CACCCGCTCCTTCACCTTTAA |
| 10361 | NPM2 | NM_182795 | CAGGAACGTTATGAAGCATCA CAGGCGAGCGTGGCTAAGAAA |
| 10363 | HMG20A | NM_018200 | AAGGAAGATAACGGACTGTAA CAGGAATGCATGAGATTTCAA |
| 10369 | CACNG2 | NM_006078 | CCGTGTGATCTTTATAATTTA ATACATATATCCATTATATTA ATCCATTATATTAGTAGTGGA TCGGTTGGGTGTTTATATAAT |
| 10370 | CITED2 | NM_006079 | TGGAAACAAAGTAAACTATAA CTCCCTTATGTAGTTGAAATA CAGATTTACGAGTGAAAGGAA TGGAATGTCGTCAGTGGCAAA |
| 10381 | TUBB3 | NM_006086 | CACGGTGGTGGAGCCCTACAA CCAGCAGATGTTCGATGCCAA GCGGATCAGCGTCTACTACAA TTGCTGTCAGATACCCTTAAA |
| 10385 | BTN2A2 | NM_006995 | AACTCCAAACATCGCGATTAA CTCATTGAAATCAAGGCCCAA TACGTCAGCATTCAGGTTCAA AACGATTAATGTTTCATTATT |
| 10395 | DLC1 | NM_006094 | CAGCTTGATCAGGACATAGAA AAGGTATTTCAGAGAAGGAAA |
| 10396 | ATP8A1 | NM_006095 | GAGGATAAATTACAAGATCAA ATGCAATGTAATGCAGTTTAA |
| 10398 | MYL9 | NM_006097 | GAGGCACCCATTGATAAGAAA ACCCATTGATAAGAAAGGCAA |
| 10403 | KNTC2 | NM_006101 | CACAATTAGCAGAGTATCACA TCCAAAGGTTATGACTTTGAA TCCCTGGGTCGTGTCAGGAAA CCGAGACCACTTAATGACAAA |
| 10407 | SPAG11 | NM_016512 | ATGGCAGATCTGGGATCTAAA TTGAATGAATTTAGCACCAAA |
| 10412 | TINP1 | NM_014886 | AAGAGATTTATTAAATTGTAA AAGGCTAAGCTTTACCATAAA |
| 10423 | CDIPT | NM_006319 | CACCATGTGCCTGTTGGTCAA CCGGATTGTCTTCGCCATCAT |
| 10424 | PGRMC2 | NM_006320 | CCCTAAATCAAATCAATATTA CACATCAATTTGAGTTGTTCA CCGGCGGGTCCATATGGAATA CAAGATAAATTAATCTCACTA |
| 10426 | TUBGCP3 | NM_006322 | TCGCATGGACTTTAACTGCAA CCACTACCTAAGAGTATTTAA TACGACTTTGTATCAGCATAA TACCGCGAATGCATCAGCTAA |
| 10431 | TIMM23 | NM_006327 | CAGGCATGTTGTATAAATGTA CCCGCTGTTATTGAGGAGTAA |
| 10436 | C2F | NM_006331 | CAGAAGAATGTTCTGATTGAA AAGGTAATTAAGAATCCAGTA |
| 10450 | PPIE | NM_006112 | CAGATTCCTCTGGATTATGAA AAGACGCTTGAAGAGAATAAA |
| 10454 | MAP3K7IP1 | NM_006116 | ACGCCTTGGCTGAGAAGGCAA CACGAGGACATGACCCTGCTA CTGCAGGTGAGAGGATTTAAA CACCTTGGACAAGCCACACAA |
| 10457 | GPNMB | NM_002510 | CCGTGAGAATTCAGCATGGAA ATCGAAATTCATCCGACGAAA CTACTATTGATTAGAGCCTAA TCCATGTAACTGTATGCATAA |
| 10458 | BAIAP2 | NM_006340 | AAGAAATACCAGACTGAGCAA CACGGGCAACCTCCTGGACAA CAGCAAGAATCCTCAGAAGTA CTGCTTCTCCTGCCTAATAAA |
| 10460 | TACC3 | NM_006342 | CAGGAAGTTCTGAGAACCAAA AAGGAACTTTCCAAAGCTGAA |
| 10465 | PPIH | NM_006347 | CAGGGTCATAAAGGATTTCAT CCGCATGAAGATCGAGCTCTT |
| 10469 | TIMM44 | NM_006351 | AAGAAATGAAAGAAAGTATAA CTAGATAATGTCAAACAAGAA |

Table4

| | | | |
|---|---|---|---|
| 10474 | TADA3L | NM_006354 | ACCGACTGCAGGATAAGAAA CTGACCGAACTGACACTAAA |
| 10478 | SLC25A17 | NM_006358 | CTCCTGGAGATCATTAAAGAA CTCATTGCTGTTGGTCTTCAA ACCCAAATGATTCTCTTATAA |
| 10480 | GA17 | NM_006360 | ATGGATAAGAATACTCCCTGTA TTCGTGCTTATCTGAAATCTA |
| 10482 | NXF1 | NM_006362 | CAGAACAAGTAGAACAGCTAA AACGCGTTAATTTCCCTCAAA |
| 10489 | MUF1 | NM_006369 | CCCAGGCTTTAGAGAAGTATA CCGGCGTAAGAGTGAAGCCAA |
| 10490 | VTI1B | NM_006370 | AAGCAACAGGAAGCAAATGAA ATGATAATACCAAGGTAATAAA AAGGTAATAAAATGCTGTTTAT CAGTATGATATACCAAGGTAA |
| 10491 | CRTAP | NM_006371 | CAGCATGTCAGTGGCATTTAA CAGGGAGGATCAAGGACTTCAA |
| 10494 | STK25 | NM_006374 | CCGGCCGAGTCCACACAGCAA AAGACCTGACTTGGAGACAAA CACCAAGCTATGGATCATCAT AAGGGCATCGATAACCACACA |
| 10499 | NCOA2 | NM_006540 | CAGTCTAGTGCCAAAGATTAA CCAGGAATGATGGGTAATCAA AAGGATTGGCATTCAGTCAAA AGGGCTGTTAACATTAGCAAA |
| 10500 | SEMA6C | NM_030913 | CGCCCTGGAACCCAAATTCAA CCGCGTAGCCCGAGTATGTAA |
| 10516 | FBLN5 | NM_006329 | CAGATCTGCATCAATACTGAA CAGATGGATGAAAGCAACCAA |
| 10517 | FBXW10 | NM_031456 | CCGGTGTATATCCGAAATGAA AAGGAAGGTCTTGAATTTCAA |
| 10521 | DDX17 | NM_006386 | CACGTAAATGAAACCACTCAA CTGGGACATTGTAATCATAAA |
| 10522 | DEAF1 | NM_021008 | CAAGTCATTAACACACTTTAA CAGCGGCACTCTGTACAAGAA |
| 10525 | HYOU1 | NM_006389 | ACGCAAAGTCATCACCTTTAA CAGCAAAGCCTTTAAAGTGAA |
| 10536 | LEPREL2 | NM_014262 | GAGGCTGATGCTTTAAATGAA CAGGAGTGGATAGAAGCCAAA |
| 10537 | UBD | NM_006398 | AAAGCATTCTTTGTCTATTAA CTGGACAAACACAGCAATCCA ATGGGATTAATGACCTTTGA AACCCATATGACAGCGTGAAA |
| 10539 | TXNL2 | NM_006541 | AACGAAGTTATGGCAGAGTTA AGCGCTAATGAACATCTTAAA TTCGATATATTGGAGGATGAA TACCCTCAGCTGTATGTGAAA |
| 10561 | IFI44 | NM_006417 | CTGGGATATCTGAGAAGTATA TCAGATGATAGTAAAGATCAA |
| 10562 | OLFM4 | NM_006418 | CTCATTGTCCACCTTACTAAA CAGGAATATTGCCAGAGTTAA TAGGGATTCTTTGTACAGGAA CAGAGTTAACCTGACCACCAA |
| 10563 | CXCL13 | NM_006419 | CTGAATGGATACAAAGAATGA CGCTTCATTGATCGAATTCAA AAGGGTGATGTACACATGTAT ACGGAGGAGAATTAAGTCCTA |
| 10569 | SLU7 | NM_006425 | CTGGCTTAATAAATATTTCA CAGGCAGATCCTACAAAGCTA |
| 10571 | SMA3 | NM_006780 | AAACTCATTCAAGGAGGTGAA CAGCCGGGATGCACTAGGCAA |
| 10573 | MRPL28 | NM_006428 | CAAGTTGGGATGGACCCTGAA CAGTGAGATCCTGGACAAGAA |
| 10575 | CCT4 | NM_006430 | CTCGATAATCTGGATAACTGA TTGCTCATATTGACCAATTTA TAGAGTTGGCCCTACGATTAA |
| 10577 | NPC2 | NM_006432 | CCCGAGCTTGGAACTTCGTTA GTGGATGGAGTTATAAAGGAA AAGGACAGTCTTACAGCGTCA CAGATCGTTTCTCATCTCTAA |
| 10578 | GNLY | NM_006433 | CAGAAATTTCATGAGGAGGTA CAGGGTGACCTGTTGACCAAA |
| 10580 | SORBS1 | NM_006434 | TCCGGACACTGAGAGATCAA CTGAACAGAGATGATGATTCA ACCCGGCAACACGAAAGCAAA |
| 10581 | IFITM2 | NM_006435 | ACCATGAACCACATTGTGCAA CCAGGACCTCCACACCATTAA |
| 10594 | PRPF8 | NM_006445 | ACGGGCATGTATCGATACAAA CAACGTCGTCATCAACTATAA CCGAAAGTCATATAAGATGAA ATGGCTTGTCATCCTGAATAA |
| 10595 | ERN2 | NM_033266 | CAGCCACTCGACGACCCTGAA CAGGGATTAATGAAACTGCCA CACCTGCATTCTTTACACATA AAGGATGAAACTGGCTTCTAT |
| 10606 | PAICS | NM_006452 | CACGTGGAAATCTCCGTTATT GCCCAAGGACTTCTAACAATA CTCGACTAACAGGGACTATAA CCCAAGGACTTCTAACAATAA |
| 10613 | SPFH1 | NM_006459 | CACTGGAATGTTAAACACTAA CCCGGAATATCTGGAGCTCAA |
| 10615 | SPAG5 | NM_006461 | TCGACATGACTTGGAAGATAA CCCGAGTAGCATCAATGGTTT TCCCGACAACTCACAGAGAAA CCAAATTAGCTCTACTCCTAA |
| 10623 | POLR3C | NM_006468 | CAGCTTGATAGGGAAAGGTAA CCCACACTTGTCATTAATGAA |
| 10628 | TXNIP | NM_006472 | TAGAGGGCAGGTGTTCATAAA TAGAGCTGATTTGATGGACAA AAGAATACCTTAATTCCTTAA AAGAGCCAATTAACAAACTA |
| 10630 | T1A-2 | NM_001006624 | CGGGAAGGTACTCGCCCTAAA CTCAAGTGTGTTTGAAACCAA CAGGAAGTCGATAGTCTCAAA ATCGGGATCTGCCAACTTCA |
| 10631 | POSTN | NM_006475 | ACGGATCTTGTGCCCAATTA CACGAGAAGAACGAATCATTA ACCAGATTCATTACAATTCAA CTGCTTATTGTTAACCCTATA |
| 10634 | GAS2L1 | NM_006478 | CTGGGACACGCTGGAGCATTA CAGCAGAGCCTCTGACAGTAA |
| 10651 | MTX2 | NM_001006635 | CTGCAGAACCTTGGCCTGAAA AACCTCAAATTATATAATGTA |
| 10653 | SPINT2 | NM_021102 | CAGCGATATGTTCAACTATGA AAGGAGGAGGTGCCTCAAGAA |
| 10658 | CUGBP1 | NM_006560 | TTCTAGAACTTCATAAGATAA CGGCTTAAAGTGCAGCTCAA CGGGAACTCTTCGAACAGTAT CAGGGTCCTCACCTAGCTCTA |
| 10659 | CUGBP2 | NM_006561 | CTGAAATTTATTATTAGACAA CAGGAAAGGTTACTGTAAA |

Table4

| | | | | | | |
|---|---|---|---|---|---|---|
| 10663 | CXCR6 | NM_006564 | CTGCTATTCAGTCATAATCAA | CACCAGCATGTCCAGTTATA | TCGTTTCATTGTGAGTGGTTAA | CAGGTCATGTGCAAGAGCCTA |
| 10668 | CGRRF1 | NM_006568 | CTGATAGGACTTATAAACTAT | TCCATCTGTGTTGATACATAA | CGGGAAATTTATGATATATATT | CCGGGAAATTTATGATATTAT |
| 10670 | RRAGA | NM_006570 | CCGGTTTGAGAAGATCAGCAA | TTGGTATGGTGTAACACTAAA | | |
| 10673 | TNFSF13B | NM_006573 | CACGCAGGACATCAACAAACA | CAGACAGTGAAACACCAACTA | CGGAGGGTAAATGCCAGCAAA | CACGCCTTACTTCTTGCCTTA |
| 10683 | DLL3 | NM_016941 | CCCGGTGAATGCCGATGCCTA | CCGGATGCACTCAACAACCTA | AAGGCTATTATTGTGATCAAA | |
| 10692 | RRH | NM_006583 | CTGGAAGAATCTGAAATAAGA | CACTGTACTGATGACCTTTAA | CACAAATGCAATTATTATTAA | |
| 10693 | CCT6B | NM_006584 | CAGGAGGATGTACTACTTCAA | TAGAACATCATTACAAACTAA | | |
| 10694 | CCT8 | NM_006585 | AACCTTCGAGATATTGATGAA | CTGGACTTGAACAGTATGCTA | GAGGCTGTGTATAGAAACATA | AAGGCTGTATTTGTAGTAGTA |
| 10713 | USP39 | NM_006590 | GTGGCTGATGATGGTAAATAA | AAGAGGCGAGATAATGATGAA | | |
| 10716 | TBR1 | NM_006593 | TCCCATTATCTCGACCACTGA | CTGGTTTGTGACGCCGGCCAA | ATGGCCGTCTCGCAGCGAATAA | AAGTCTCGGCCCTCCACATTAA |
| 10717 | AP4B1 | NM_006594 | TGGAACAATTGAAGAAATAAA | GAGGAAATTCTGAAACAGGAA | | |
| 10724 | MGEA5 | NM_012215 | CTGGTACAAATCAAACATGAA | CAGGGAGAGAGTCTTCAATAA | | |
| 10726 | NUDC | NM_006600 | TGGCCTACTGTTACACATTAA | CACCAAGAAGATTAACCCTGA | | |
| 10730 | YME1L1 | NM_014263 | TTCCAGATTGACCATGAATAA | GAGGCATTAGATAATGCCTTA | TCGGCATTTATGGACTTCTAA | TCCGTTGATCCAGAAATTATA |
| 10735 | STAG2 | NM_006603 | CAGAAATATATTGAAAGTTTA | TTGCTTGTCCTTTAAGATAAA | | |
| 10748 | KLRA1 | NM_006611 | CTGGAATTAATTCTACAATAA | TCGGATTATCATATGATGAAA | | |
| 10749 | KIF1C | NM_006612 | ACCTCCATCATCAATCCTAAA | CCGGCTGATTAGAGAGCTGCA | CTGGAGAATCAGTACCGGAAA | |
| 10753 | CAPN9 | NM_006615 | CCCGAGAACTTCTATGAGATT | AAGGAGTTCATTCATCTCAAT | GAGGAGTGTAGTTTCCTTGTA | GAGAACTTCTATGAGATTCTA |
| 10761 | PLAC1 | NM_021796 | CCGGTTCAGGACAAAGTCCAA | AAGGATTTGTATAAAGAGTGA | | |
| 10765 | JARID1B | NM_006618 | TAGGGTTGTGTGATTAATCAT | CAGGAATGTAGTAAGCCACAA | ACGCACCAAGCGAAAGTAAA | TCGTCCTAACATAACACTAAA |
| 10768 | AHCYL1 | NM_006621 | CAGGTGGTAAAGCTAAATGA | AAACAGTTGTATCGTATGCAA | CTGATAGAACTCTATAATGCA | CCCACTTGGATTTATAGTATA |
| 10771 | ZMYND11 | NM_006624 | CCGGATGAAGTCGGACCACAA | ACCAGTGGGTATATGATTGAA | CACCTCATGTATCGTAAGTTA | TAGGAATGGATCGATAGTTGA |
| 10773 | ZNF482 | NM_006626 | AACGTTTGTATTATTCTATTA | TACAGGGTTAATAATCCTTAA | | |
| 10776 | ARPP-19 | NM_006628 | CACATGAAAGATTAATCTGAA | CACTCTGTCATTGTTAATCTA | CCCGGCCATGTTTGTTAGCATACAA | CCCGGCCTTATGATTTAA |
| 10780 | ZNF234 | NM_006630 | AAGGGTTTCATTTACAGTTCA | CAGGACTATATACAACTCACA | | |
| 10781 | ZNF266 | NM_006631 | CTCGCCTTAGTGAACATACAA | TTGCTATTTCTTCAAATCTTA | | |
| 10783 | NEK6 | NM_014397 | AAGGGATAAAGTGGAAATCAT | ATGCAAGTTAGCTAATACAAA | ACCGGAGAGGATCCATGAGAA | ACCACGGAAGTCGAGAATTAA |
| 10785 | WDR4 | NM_018669 | CAACGTGACCTCCTACCTGAA | CAGCTTCAGCAGTCTCTACAA | | |
| 10791 | VAMP5 | NM_006634 | CTGCAGCAGCGTTCAGACCAA | GACGGAAATTATGCGTAACAA | | |
| 10797 | MTHFD2 | NM_006636 | AGCGAGAAGTGCTGAAGTCTA | AGCCATTAACCTAGTGATTAA | TAGGATTATTCCTTGCTATTA | CTCGCGGCAGTTCGAAATGAA |
| 10803 | CCR9 | NM_006641 | TTGGAAGAAATGAGAAAATACA | CCCACTTTATTCTGAGGAATA | CAGTCTTGTTATCCTTGTCTA | AAGGGATGAAATCTGAACTATA |
| 10811 | NOXA1 | NM_006647 | CAGGTGGAGCAAGTTGGCAAA | CAGCAGGGAGATCAGCCCTAA | | |
| 10815 | CPLX1 | NM_006651 | AGCCATAAACGTTAGACTGCAA | CCGTGTTCACTTCTAAACTAA | AACGTTAGACTGCAATACTAA | CACGGCCGTGTTCACTTCTAA |
| 10844 | TUBGCP2 | NM_006659 | CCCGGTGGCTAAAGAGATCAT | AAAGAGATCATCTACACGTTA | CCAGGAGGATTACAACGACAA | CAGCGTCTGGATCAGCAACAA |
| 10846 | PDE10A | NM_006661 | ATGCAGGGAGTTGTATATGAA | AACCCTTTGCATAAGAACCAA | TAGACTCTCATTCACAATGAA | CAGTATTACTATACAATGTAA |
| 10849 | ASE-1 | NM_012099 | CCGGAAACTGAGGAACTAAA | AAGGAGCTGTTTCCTGGGTAA | | |
| 10858 | CYP46A1 | NM_006668 | CACGAGTCCTGAGTCCGGTTAA | CCTGATGTCAACGAAGTCAA | AGGCTTGGTGTCCGAATGCAA | GCGGATGGACACACTACTTTGA |
| 10859 | LILRB1 | NM_006669 | CAGAAAGTGCATTAAACTGAA | ATGAATGAATTAGGAAAGAAA | | |
| 10863 | ADAM28 | NM_014265 | TCGGATAAATTTGCCCTGGAAA | CACCTAAGGACTCAAATCCAA | AAGAAATATCTCCGAAGTTAA | CACACTAATCTTGGAGGAATA |
| 10869 | USP19 | XM_498642 | CGGCCTTAGCTAGAAGTCGAA | GACCCGCATTATAAACATTAT | CCGGGCTGTGGAGAAGGATAA | ACCCGCATTATAAACATTATA |
| 10874 | NMU | NM_006681 | CCAATATTACCTCAAGGATTA | CAAAGTCCCTTGCAAGTCAA | CAGAGTGGACGAAGAATTCCA | GAGCAATGCTATGAATACAA |
| 10881 | ACTL7A | NM_006687 | CTGCTCGGAGATGTTCTTCAA | CACAAGAACCTACTGAATCAA | | |
| 10886 | GPR74 | NM_004885 | CAGCTTGTCCAGGAATCTACA | CTACTACGCATTATATATTTA | CTGGCCATAAGTGATTTACTA | CCGGGAAGACTGGCCAAATCA |

151

Table4

| | | | |
|---|---|---|---|
| 10890 | RAB10 | NM_016131 | AAGGGACAAACTAGTAGGTTT AAGCACTGATGTAACTGCTA CTGATGTAACTTGCTAGGTAA CAAATTCGGTTTCATATTCTA |
| 10897 | YIF1 | NM_020470 | CACGAAGGCCTTCATTACTTA CTGGCCTACAGTGCTACAAA |
| 10901 | DHRS4 | NM_021004 | CAGGATGCTCTGGATGGACAA AACCCTTTCTTTGGAAGCATA TTGCGCGGCTTTGAATCCAAA |
| 10906 | FLN29 | NM_006700 | TACTGTTATAATAATTATTAA CAGGAAAGGAATAGAGGCCAA |
| 10907 | TXNL4A | NM_006701 | TGGCAACAACAACAAGATTAA CAGCATCGCCGAGAAGGTTAA TGGCCTAATCTTAACTAATTA AAGGTTTACTCTGGTTATAAA |
| 10908 | NTE | NM_006702 | CCGGCGGTCTACAGACCTTAA ACGCATGAGGAAGAAACTGAA |
| 10933 | MORF4L1 | NM_006791 | CAGAAACAGCGAGAACTTCAA TCCGGAGAGCAGAGTACTCAA |
| 10934 | MORF4 | NM_006792 | GGGCACCCAGCTACTCAACAA GCCGAAATTCTTGCAGATTGT |
| 10936 | GPR75 | NM_006794 | CTCGGATTGAAACCTTACTACA ATGCATTATCACCACCACTAAT CCAGCTCGTATCAGCCATCAA ATGGACTTTATTCTAACTTGA |
| 10939 | AFG3L2 | NM_006796 | CCGGCACCAATCGACCAGATA GAGCGAGTGATTGGTGGCTTA CATGGTATTGGAGAAAACCTTA ATGATGAAGTACGAATACTTA |
| 10945 | KDELR1 | NM_006801 | CCGCACGCTCTATCTCTTCAA TAGCAAGTTCAAAGCTACTTA CCACTGGTTGCCAAACACTAA CACGTGTATGAAGGTGGTCTA |
| 10946 | SF3A3 | NM_006802 | AAGGAAGAATGCAGAGTATAA CAGAGACTATTCAGTACCAAA |
| 10947 | AP3M2 | NM_006803 | CCCAGTGTATGTCAAACATAA CACAATTAACCTGCAGTTTAA |
| 10956 | OS-9 | NM_006812 | ACCCACCATGTTCTTGAACAA AACAATCAGGTTTCTAAATAA |
| 10957 | PNRC1 | NM_006813 | TTGTATATATACAATGGATAA TTGCCTTGTTGTGCAACATCAA CAGATTTCAGTATGTGTGTAA |
| 10966 | RAB40B | NM_006822 | AACGGTGATATTGGAGTGATT ACGGTGATATTGGAGTGATTA TCGGCGAATGCTGCTTGCGAA CCGTCGGACTTCGAATTTCTA |
| 10982 | MAPRE2 | NM_014268 | AACGCAGGTCATACAGCTTAA TCCCAATATCTTCTAGAATAA GACCTTATTAATAGGAGCATA CAGCAGGTGCAGCTAAATCAA |
| 10983 | CCNI | NM_006835 | AAGGCTGTAGAAGGAAATATA CAGCGTTATTTAAGCACATAA |
| 10985 | GCN1L1 | NM_006836 | CTGGAAGATTGTTGTCTCCAA CAGTTCAGCTGGAATGTGAA |
| 11001 | SLC27A2 | NM_003645 | CAGCCACTTGATATAATCCAA CTGAGAGATGTCTTTAAGAAA |
| 11012 | KLK11 | NM_006853 | CCAGGAGACGATGAAGAACAA ACAGGAGAGATGCTGTCACTTAA CAGCTCCTGGCCATATATCAA |
| 11016 | ATF7 | NM_006856 | CGGGCTGTGGGACAGAGATTTA CAGCTACTGTTAGCTCATAAA |
| 11017 | RY1 | NM_006857 | ATGACTTGCCATTATAATTAA ATGGTTCTCTCCGCAAGTATA |
| 11027 | LILRA2 | NM_006866 | CAGGCTGAGGAGTACCATCTA GAGGCTCTGGAGGACAATCTA |
| 11031 | RAB31 | NM_006868 | CGGGCACATTAGGCAGTTGAA CACACACTGATTCATACTAAT CAGGATTCACATAAACATTGT AAGGAATACGCTGAATCCATA |
| 11033 | CENTA1 | NM_006869 | CACCCGTAACATCTTCATCTA CGCAGTGGAGCCCACTTCAA CCGGAAGTTTGTGCTGACAGA GAGGCGCACTTCAAGCATAAA |
| 11035 | RIPK3 | NM_006871 | ATGGCAAGTCTGGATAACGAA CACAGGTTGGTATAATCATA CAGCCTGATGTCTGCGTCAA ACCGCTGTCGTTAACATATACAA |
| 11041 | B3GNT6 | NM_006876 | ACCGATGACAATGTGTAAATA AAGTCTGAATACTTAATGAAA |
| 11045 | UPK1A | NM_007000 | AAGCATATACTAGATAGTCAA CAAGCAGATGCTGACCTTCTA |
| 11054 | OGFR | NM_007346 | CACGGTGGGCCGAGCACAGAA CAGGAGTGAACTGGCATGCCA CAGAATGACAGGGTCCAGAAA |
| 11068 | CYB561D2 | NM_007022 | CACAAAGAGCAGCTTGGCAAA CAGCTCATACTTGTACTGTAT CAGGTGAGCAATGCCTACCTA CACCAGCTTGGTCATTATGAA |
| 11072 | DUSP14 | NM_007026 | TAGCTGTTAAGTAACTATAAT CCCAGGATTATATTAGCATTA ACCCTTATTATTTAGCTGTTA AAGGGAATGCATACATTGCTA |
| 11073 | TOPBP1 | NM_007027 | CAGCAATATTTCCAACATAAA AACGAAGTTTGGAAACAGAAA |
| 11079 | RER1 | NM_007033 | CACGATGAAGAGGCAAATCAA CGGAATTATAAAATATATATTA |
| 11092 | C9orf9 | NM_018956 | CAGCATCGAGCAGAAGTACAA CAGTGCAGAGCTACATGGAA |
| 11098 | PRSS23 | NM_007173 | ATGGTAGTCTTTGAACAGTAA CACATATTAAACTATACCTTA AACAGTTGTGGCCACACTAAA CTGCATTGTAGCAATTTGTAA |
| 11099 | PTPN21 | NM_007039 | CCCACCGCAGTTGCACTATAA AAGCACCTCCTTACCGGGCAA GTGCATAGATTTCTATCTTAA GAGGAGGACCATTCAATTTCAA |
| 11113 | CIT | NM_007174 | CCGGAAGCTCCAAGAAATCAA AGGAATTTAGTCGGCGTCTTA CAGGATATACCGTAACACGAA ATGGAAGGCACTATTTCTCAA |
| 11119 | BTN3A1 | NM_194441 | TCAGCCTATAATGAATGGAAA CTGGAGGAACTCAGATGGAGA CAGATGGAGAAGTATCCAGTA AAGACTCAGTTCAGAAAGAAA |
| 11126 | CD160 | NM_007053 | CTCAGTTGATGTTCACCATAA ATGACCTCTTGTCATAGTTAA AAGGAACGCGACTAAACTTAA AAGAGATGCTAAATATACCAA |
| 11127 | KIF3A | NM_007054 | GCCGATCAATAAATCAGAGAA CCCAATTCATTTGCTCACATA CTGGTTCAGAAAGACAGGCAA GTGCCTTATCGTAACTCTAAA |
| 11128 | POLR3A | NM_007055 | ACCAAGAAAGTCAGCATGTAA ACGGCTGTAAAGACAGCTGAA |
| 11131 | CAPN11 | NM_007058 | CAGGATGGCCATCAAATTCAA CCCAAGAATACTGGCCATATT CACATTCTTTCTAACCATGGA ATGCGCCTGTTATTGAGAAA |

Table4

| No. | Gene | Accession | Sequence |
|---|---|---|---|
| 11132 | CAPN10 | NM_023083 | CAGGAGGAACTTGCCTCATA CAGGATTGACAGGCCATCCAT CTCCGTCATGGCAGTGATGAA CAGATCTTCTTTATAACTATT |
| 11135 | CDC42EP1 | NM_007061 | GCCCAGCCACAGTGCAAGCAAA CTGGGAGGACGAGCCATGAA |
| 11141 | IL1RAPL1 | NM_014271 | GCGGGATACTTGTGAAATATA ATCGGGCACTTTGAGAACTAA ACGCATTGTGATGACCAGCAA ATGCAACAAGTTGAACTCCAA |
| 11146 | GLMN | NM_007070 | TAGCTATGCTTCAGCTGTATA TAGCATCGAAACTAGGAGAAA GAGGAACTTCAGTCTATAATA AAGGCATATTCAATTGGATTA |
| 11154 | AP4S1 | NM_007077 | ATGCTCTTTCATTGAATATAA CTGGTCCTTATCAGGAACCAA |
| 11157 | LSM6 | NM_007080 | CTGGATGGCTACATGAATATA CTGACATGTGAGTAAGATAAA |
| 11163 | NUDT4 | NM_019094 | CAAATTCATGTTAGTGAAGTA ATGAAACTGGTACCCAATTTAA CAGTACTATTTGTCACATAA AACATTTGTTCTCACAAATTAA |
| 11166 | SOX21 | NM_007084 | CCGGTTGTATGTACATAGAA TTGAGCGAATATCAAAGTTAA |
| 11176 | BAZ2A | NM_013449 | CACTGAGAAGGCTAAGACTAA CACCACTACAGAGATATCATA CCCGCAGTGACGGCCAGTAAA CTCGGTAGCTTTGAATCTTAA |
| 11177 | BAZ1A | NM_013448 | CAGCTAGACCAGCTTATTGAA AACGAGGAAGACCACAAGTTA CACAAGTTAGAATTGCCAGTTA AACCCTCGTAACACAAGTGAA |
| 11181 | TREH | NM_007180 | CAGGCCTGAGTCCTACAGCAA CTCGCAGAAGTCAGCCATGTA |
| 11194 | ABCB8 | NM_007188 | CACGATGCTGGTGCATTTATT CACCTTCTTTGACGCCAATAA AGGAATTTAAGTCATCCTTCA AAGCTGGAAGCTTCCGATGAA |
| 11197 | WIF1 | NM_007191 | CACAAGGCATCAGTTGTTCAA CCCTGTCGAAATGGAGGTAAA TTGCATTGGCTTGAAGCAATA AAGCAATATAATATATTGTAA |
| 11211 | FZD10 | NM_007197 | CAGGTGTGCAGCCGTAGGTTA ACCTCACATTTGTGAACTCAA CCCGATTATGGAGCAGTTCAA CAGCCGTAGGTTAAAGAAGAA |
| 11212 | PROSC | NM_007198 | AAGGTTCTCCTGAAAGGTTAA ATGGCTCAGGTTAATTCTCAA |
| 11218 | DDX20 | NM_007204 | CAGGCATGGCAAGAAATATTA CCAGATGAAGACAAGACTTAA GGGCTCGATTAATTGTTCAA |
| 11221 | DUSP10 | NM_007207 | CCGAAGATACTACACACTTTA CAGGGCAGCTTAAGTGGTCTA CAGGTTCATAGACCGAAGATA ATGAGAATACAGGCTCTCTAA |
| 11222 | MRPL3 | NM_007208 | GCCGCCGAAACAGACAGTTAA AGGGCATAAATATATCATTCA CCGCCGAAACAGACAGTTAAA CACATTAAATATGAGTTAA |
| 11224 | RPL35 | NM_007209 | CGAGGAACCTGAAGACCAA CAGGAAATTCTACAAGGGCAA TGCAGCAATGGCCAAGATCAA CCGTGTTCTCACAGTTATTAA |
| 11226 | GALNT6 | NM_007210 | CAGCGTGATCATTGTGTTCCA CACCTTCTATGGTGCCATCAA |
| 11232 | POLG2 | NM_007215 | CAGATACGAAATGGTGTTAAA AAGGAACAAACTTTACTACAA |
| 11240 | PADI2 | NM_007365 | CAGCAAGCGAATCACCATCAA CAGCCTTGACTCATTTGGAAA |
| 11243 | PMF1 | NM_007221 | AAGGATCTGCACAGTTGTTATA GAGGAAATCTCTGACCATCAAA |
| 11248 | NXPH3 | NM_007225 | CAGCGTCCACTTCCAACACAA CGACTTCTACTCCAACATCAA |
| 11259 | DOC1 | NM_014890 | AAGACTGAAACTGAAGCTAAA GAGGATTTAACTAAACTGAAA |
| 11279 | KLF8 | NM_007250 | CTCAGTCAGTCTGCCAAATAA CTGAGATAGAATACAGAAGTA |
| 11283 | CYP4F8 | NM_007253 | CAAGGACATAGTCTTCTACAA TGCCCTTGTAGTGAAACGGAA AGGGAATGTCTGTAACATCAA CAGAGTCTAAGTAAAGACTTT |
| 11284 | PNKP | NM_007254 | CAAGCTGGTGATCTTCACCAA CGGGAAGTCCACCTTTCTCAA CACGTGTGAGACAGCCCTGAA CACGTGAACAGGACACGCTA |
| 11316 | COPE | NM_007263 | CAGAGCTGTCAGGACCATGAA CCGGAAGGAGCTGAAGAGAAT |
| 11317 | RBPSUHL | NM_014276 | TCCCTTGTCCTTACACATACA CTCAAAGGTCTCCCCTCTTCAA CCCATGATCATCCGTAAAGTA CAGGTCCTTCCTGGAATATTA |
| 11326 | VSIG4 | NM_007268 | CAGACTCCTGATGGCAACCAA CTGGTGCTCAATAAATATCTA |
| 11328 | FKBP9 | NM_007270 | CAGCGGCGACTTCGTGCGCTA AAGCTTGCCTACGGAAATGAA TCGAGTCACAATCGCATGAAA CTGGTGTTTGATGTTGCATTA |
| 11329 | STK38 | NM_007271 | AAGGCGTTGACTGGGAACATA CCATAGTACTAAGGAAATAAA TACGTCGGCCATAAACAGCTA AACCCAGGAATTCTCAATAAA |
| 11331 | PHB2 | NM_007273 | TCGCCACATCACAGAATCGTA CAGAGTTATGAGTAGCCTA CCGCGGCTATGTTCTATGA CAAGCTGAGGCGAGTGCATAA |
| 11334 | TUSC2 | NM_007275 | AAGGCTTGGTGCCAGTAGTTAT CCAGTAGTTATGAGTAGCCTA CACAGCTGCTGTAGAAGCCAA |
| 11336 | SEC6L1 | NM_007277 | CTGGAGCAACATCTGAATGAA CACGCTCACTTCAAACATCAT |
| 11340 | EXOSC8 | NM_181503 | CAGGAGATTTCTGAAAGAGAA CAGAATTGCAGCACCATCAA |
| 11343 | MGLL | NM_007283 | CCCGATTTGCCTGGTTCTGAT AAGACAGAAGGTCGACATTTAT CTGGACCTACCTTAATGGTTA TCAGAGCTTGATGCTACTGTA |
| 11344 | PTK9L | NM_007284 | AAGGAATTCTTTGCCAAGGCA CCAGATGAAGCTGGACCTAGA TTGGGGATGGTTGAATGCTGTA AAGACAGAGATCAGTGTGGAA |
| 22795 | NID2 | NM_007361 | CCCACAGAAAGTAAAGTATCA CTGGAATAGAGAAGCTCCTAA AAAGCACATGTGATCAATATA CACGTATAATGCTGCCAACAA |
| 22803 | XRN2 | NM_012255 | ATGGATTACATTAGAAGGCAA AAGGAGGTTCAGGGCATCAAA |
| 22818 | COPZ1 | NM_016057 | AGCGATTTAAATTGTATTGAA TTGGCTGTGGATGAAATTGTA |
| 22820 | COPG | NM_016128 | CAGGAAAGGGACATTGTAAAT AGGCCCGTGTATTTAATGAAA CACCGACTCCACTATGTTGAA CCGAGCCCACCTTCTACCTAAA |

Table4

| 22827 | SIAHBP1 | NM_014281 | CTGGGACTCCGTCACCATGAA CTCCGTCACCATGAAGCACAA |
|---|---|---|---|
| 22828 | RBM16 | NM_014892 | CACGGTCGTATTCAAGTGAAA CAGAATGATCCTGAACTTTAT |
| 22838 | RNF44 | NM_014901 | CAGAGGGATATTCACTAATAA CAGGTGACGTTTCTATATTTA |
| 22841 | RAB11FIP2 | NM_014904 | TTGGAAGTATTTAATATGCAA CTCGTTATAAACACTACTAAA |
| 22843 | PPM1E | NM_014906 | CCGCCTAATCATGTGTTTATA TTGGTTCTATAAACTAGATAA GAGGCGGTTTATAGTCAGAAA CCCATTTAGGTCTGTACTAAA |
| 22848 | AAK1 | NM_014911 | CTCCTCGGACCTCTCAACAAA CCCGAGAAGCTTGGAGGCTCA CCAGGTGGTAAACCTGATGAA AACGTGAGTAGCGGTGATGTA |
| 22850 | KIAA0863 | NM_014913 | AAGCAATTTCTTAAAGATTAT CAGGAGAGAAATACTTTCATA |
| 22853 | LMTK2 | NM_014916 | AGCGACTATTATATCCAGTTA ACGGCTCTTAGCTGCGTTCAA CAGATCAGACTAAGTATAGTA CACCTCCGACTTAAATGTGAA |
| 22871 | NLGN1 | NM_014932 | CCGCCTCAACACAATGCCTTA CGGAGGTATATTCTACCATTA GAGGAATATTATGTGAATATA AAGAAACATGACTGTCTCAAA |
| 22882 | ZHX2 | NM_014943 | ACGGCAGATCTTCCAAATCAA CAGTACTATTGTGACATTAAA |
| 22888 | UBOX5 | NM_014948 | CAGACAGTAACTTTGGTGTAA TCCCTACAACTTGGTACTCTA CCGGCACATTCCTTTCCTTTA TCCGGTGTGCTTGTGTTCTTA |
| 22901 | KIAA1001 | NM_014960 | TCGCAATGGAGTCACACGCAA CACAGGGTGATGGACCATCAA AAGGACAAAGTTGACCACACA CTGGAAGACGATACCGCAGAA |
| 22902 | RIPX | NM_014961 | TTGGTATAAGTTCCTAATTAA AACATTGTAAATAATCCTATA |
| 22913 | RALY | NM_007367 | CGGGCAGACCCTGGACATCAA CTCAGCCAAGATCAAGTTAAA |
| 22915 | MMRN1 | NM_007351 | CACAACTTACTTAGAAATGAA TCCCTGTATATAAATATATAA |
| 22920 | KIFAP3 | NM_014970 | TCGAGTTAGCTACAAACATAA CCACATCTTGATGCTAAGTAA CACAAGAGACGTCATAATCAA CAGCATGATGGACCAACTAAA |
| 22925 | PLA2R1 | NM_001007267 | ATGCGTGGAAATATTATGCTA ACGGTGTAAATACATCTGATA AAGGTTTGGCTCATAGAGAAA AAGGAGGTACGCTGTTAAGTA |
| 22927 | HABP4 | NM_014282 | AAGCGAGAATTTGAAAGATAT CAGACCAAAGCCTGAGTTTAA |
| 22936 | ELL2 | NM_012081 | CTGGGATTTATACAAGATAAA CAGAAAGATGGTGTCAATCAA |
| 22937 | SCAP | NM_012235 | CCCGGTAGACCTGGAGGTGAA CACCTGCTTAATTGACACCAA CCTGACTGTAATAATATTAAA CTGGCCGACGCTCTTCAGCTA |
| 22938 | SKIIP | NM_012245 | CACAGGTCTCTCCAAAGTGAA TCGGATGGTAGAAATGCAGAA CTGGATATGGGACGAAAGAAA CCCGATGAAGAAGCTATTAAA |
| 22943 | DKK1 | NM_012242 | CAGTAAATTACTGTATTGTAA CCCTGTGATTGCAGTAAATTA CACACTTGTCAGAGACACTAA ATGTACTATCTTAATGCTTAA |
| 22947 | DUX4 | NM_033178 | GAGCTCTGCGGCACCCGGAAA AGAGAGGAACGGGAGAGACTA |
| 22949 | LTB4DH | NM_012212 | CGCGCCGGAGGGAATAGGAAA CACTGTTATCGGCCAGATGAA CCGCATTTATGGGAATGCTGA GGCCATTTAGATGATTAGTTA |
| 22954 | TRIM32 | NM_012210 | CTGGGTAGTTCTAGAACTTTA CAGCAGGAATTTGACCCTCTA |
| 22977 | AKR7A3 | NM_012067 | CTGGGCAGAGATGTACAGGAA AAGGCTCTTCTGTAACATCTT AACTGGAAGGAGCACCACTTT AAGCGACTGCAGAGTGAAAAT |
| 22979 | KIAA0953 | XM_039733 | CTCAAGGATATCAGAAGCCAA CAGCATTTGCGGAATTAACAA |
| 22980 | KIAA1049 | NM_014972 | CCGGTCACTGTTGCCAAACTA CCGCTTCGAGCTGATAAACAT |
| 22986 | SORCS3 | NM_014978 | CAGGCTATTCTGAGCTCAGAA ATGGAGGAGGGTGATCTACAA AAGGCAGTCAGTGGATCTTAA CACAACTTCCTTAAAGTCAAA |
| 22987 | SV2C | XM_043493 | CCAGATTTAGCTGGAGATTAA CTGCTCCTAATTTAACATTAA |
| 22992 | FBXL11 | NM_012308 | AAGCCAGACTGAATCAACAAA AGCGCTATGTGTACTGCATAA CTCACTGGAGTTCCTATAGTA ACCCATTTCGTTGCTACCCAA |
| 23001 | WDFY3 | NM_014991 | CGGCATTAATACTTTAATTTA AACATGAAACTTCAAACTAAA |
| 23015 | GOLGIN-67 | NM_015003 | CTGAAAGACAAGAGCATTTAA ACCGGTCATCGTACAAAGAAA |
| 23030 | JMJD2B | NM_015015 | CCCGGCCACATTACCCTCCAA CAAATTGAAGATGGAGATCAA AAGCAATGTTTATATTATAAA CACCTGACAAATCCTAGCGAA |
| 23032 | USP33 | NM_015017 | AAGAATTCCTTCGATGTTTAA AAGAAGATCCGCAAACCATAA CTGCAAGTAGTGGACACTATA CCGGTTGTTCATGTTGATCCA |
| 23033 | KIAA1117 | NM_015018 | AAGGAGGGATATGTCTCTGAA AACGAGAGATCTCCATATAAA CCGGATTTCTGTTATGGGCAA CCGGAGTTCTATGTACATAGA |
| 23040 | MYT1L | NM_015025 | ACGGAAATGTATACAAATCAA CAGGACTGAGTCAGAAATGAA |
| 23043 | TNIK | XM_039796 | CCGGAATATTGCTACATACTA ATGGCGTTATTTCTAATTTAA CTGGAATATAAGCGCAAACAA AAGGACCATATTGATAGAACA |
| 23045 | KIAA0826 | XM_093839 | ATGCAAATTAATAGACATCAA ACGCATTGGTACCAAATTTAA |
| 23049 | SMG1 | NM_014006 | TTCGTTTGCATCACAGTTTAA ATGGGTCAGGTTCGAAGTCAA CACCATGGTATTACAGGTTCA ATCGATGTTGCCAGACTACTA |
| 23052 | KIAA0830 | XM_290546 | AAGATCTATATGTTAAGCTAA CAGCCAAACTATTATTTGATA CCGCCCAGGATCAATCAGAAA AACGAATGGTACAATCTCAAA |
| 23053 | KIAA0913 | NM_015037 | CTGGTATTTATTTGGCATTTA CACGGGCAAGAGACTCCAATA |
| 23063 | KIAA0261 | NM_015045 | CTGGATCATATTGTAGATAAA AAGGAGATAAATCAAAGGAAA |

Table4

| | | | |
|---|---|---|---|
| 23081 | JMJD2C | NM_015061 | CAGATATTAATGGGAGCATAT TCCCAGAAGTTCGATTCACTA CCCAGAAGTTCGATTCACTAA AGCGGGTAGGGCGATAATTTA |
| 23082 | PPRC1 | NM_015062 | AAGGAGCGTGCAATAGAAGAA CAGGCTTTCATCAGTGAGATT CAGACTAATAAACATCAACTA AAGGGTGGTCTTCATTGGAAA |
| 23085 | RAB6IP2 | NM_015064 | CTGAAGTTATATAGCCTTGAA AACGGGCTTTAACATGTTTAA TAGAAGTAAATTAAAGTTGTA CAGGAACTTAACCCAAATCTA |
| 23086 | SLAC2-B | NM_015065 | AAGGAAGATTATGGAAACCAA CAGGAGAATAAGAGATATGAA |
| 23090 | ZNF423 | NM_015069 | CAGCAAGAAGTATAACTGTAA CAGAGAGAAATGCTTAGTCAA |
| 23091 | KIAA0853 | NM_015070 | CTGGCATATAGTAATACCCAA AAGAAGCGCTATAGAAATGAA |
| 23098 | SARM1 | NM_015077 | CTGGTGGTTAAGGGTAGCAAA CAGGATCGGGATTACAGGCAA CTGCTATTAAATCCACATTAA AGCGGTGTTGGCGACTAACAA |
| 23108 | GARNL4 | NM_015085 | CACATTGTATTTGTTAATGTA CAGGGAGTTTCTGCTCACCAA |
| 23116 | KIAA0423 | NM_015091 | AAGGCGTATTTGGAAGTTTAA CTGGAGCTATTTCACAGTATA |
| 23118 | MAP3K7IP2 | NM_015093 | ATCCTAGGTAATTAAACCATA CTGGGTAAACTATCTTTGTAA TGGCTGGGTATCTCAGTTTAA CAGTCAATAGCCAGACCTTAA |
| 23126 | POGZ | NM_015100 | CAGGGATTTCCTGTAAGGAAT CCGGGTGTAAGGTCCCTTTAA |
| 23129 | PLXND1 | NM_015103 | CAGCCGCACTGCACAAGTGTA CATGACAGTCATGGTCTATAA CACGAGTACCGGGTCAAGATA CACGCTCTGCAAGGTTCTCAA |
| 23144 | ZC3HDC3 | NM_015117 | AACGTGGTCATCAAAGTTAAA CAGCGACTTCCTCAAAGGCTA |
| 23148 | KIAA0363 | XM_166571 | CCGGGTGAGGCTGGAGTGCAA ACGATCAGATCTGTCCTGCGA |
| 23149 | FCHO1 | NM_015122 | CAGGAAGTTTGCAGAGAGTAA CAAGTTCTATGTGCACATCAA |
| 23158 | KIAA0882 | NM_015130 | CTGCAAGATAATGTAACTGAA CTGAATGGATGTGGAAATGAA |
| 23163 | GGA3 | NM_014001 | CTGGAGTCCTGGCTCAATAAA AAGGAATAAATCCAAGAGCTT CACGTTAGAGGAAGTTAACAA CAGGGTGTCTGAGAAAGTGAA |
| 23165 | NUP205 | NM_015135 | CTGCGTCAGTTTAAATTTCAA CTGACAGGAATTATAAGTAAA |
| 23166 | STAB1 | NM_015136 | CACCTCGTGCGCGGCCATCAA CACGAAATACTCCTACAAGTA CACGCCAACTGTAGCCAGGTA TAGGAACAATGGTCACTTGTA |
| 23170 | KIAA0153 | NM_015140 | CGGCCACATCTTCAAGGTCTA ACCCAGAACCTTCACCAGCAA |
| 23177 | KIAA0582 | NM_015147 | CGGCAATTTAAGAAAGATATA CACCCTCAAATCACCTACTAA |
| 23180 | RAFTLIN | NM_015150 | CAGGCAGATGAGGAAATCAAA CTGAAATTCGTTGGTGTTATA |
| 23191 | CYFIP1 | NM_014608 | TCGAAAGGTTATAGAAATAAA CCAGCCAAATTTCAACACTAA |
| 23203 | PMPCA | NM_015160 | CAGAATAAGTTTGGACAGTTT CAGGATCGAGATATGAAGCGA CTCACCGAGATGATTCATGAA TCGAGAAATGGTAGAAATCAT |
| 23213 | SULF1 | NM_015170 | AGCATGGATTTGATTATGCAA AAGAGGCAAATTTCTACGTAA TCCGTCGAATTTGAAGGTGAA CTGGTGTTTGCGTACAGTTAA |
| 23218 | KIAA0540 | XM_291064 | CTCGGGAGAGACGGAATACAA CACACAGAATGTATACTTCTA |
| 23231 | KIAA0746 | NM_015187 | TAGAAGATTCTTAGTACTTAA CCGGGAGGATTTCCATTATAA |
| 23235 | SNF1LK2 | NM_015191 | CGGGTATGTCCTGGTGAATTA ATCTACCGAGAAGTACAAATA CCGAAGGATGTTGGTCCTAGA AAGGGCAAGATTATTTGCTTA |
| 23236 | PLCB1 | NM_015192 | AAGCGAGTTGAAACTGCTTTA CACACTACCAAGTATAATGAA CAGAGATGATCCGGTCATATA TCGAGATTACATGGATGTTAA |
| 23258 | RAB6IP1 | NM_015213 | CCGATTTGTCACTGCAATCAA ACCATTGAGAAGAACATTCAA |
| 23259 | DDHD2 | NM_015214 | CAGGATGAGTATGGACCTTAA AAGAAAGAAGATATTAAACTA |
| 23268 | DNMBP | NM_015221 | TCCGAGGTTCCTCAAAGTTAA CCAGCTCTTCACAAACTTTAA |
| 23277 | KIAA0664 | NM_015229 | CAGCCCGACCTTCAAGAAGAA CCGAGAAAGGGCCATATTCAA AAGGGCCATATTCAAGGTGCA CTCGGCCAAGCACATCTTCAA |
| 23283 | CSTF2T | NM_015235 | CAGGATAAGGTTACACTTGTA CAGCCATTTAGTCCTAATATA |
| 23284 | LPHN3 | NM_015236 | CAAGCGTACAATGACAGGTTA AAGTATGGGAGTAAAGCTAAA CAGGAACTATAGGTGTATCAA AAGCTAAACATTGCATATCAA |
| 23303 | KIF13B | NM_015254 | AAGGTTATTCTTAATCCTGTA ACGGGAAGCATTAGCAAGAAT CCCACTAATGTCTTAACCTAA ATGTCTTAACCTAAACCATAA |
| 23304 | UBR2 | NM_015255 | ATGGATCCAATTACACGTCAA CCAGATTTATTACTACCATAA CAGAACAATACTCTAATAGAA CAGGTAAATGCTACAGATGAA |
| 23309 | SIN3B | NM_015260 | CACATTAAAGTTAATAATTGA CAGCTATGTGAATAAGATTAA |
| 23313 | C22orf9 | NM_001009880 | CTCGCCCTTTGTAATAACCTA AAGAATGTACCTGCACTCAAA |
| 23315 | SLC9A8 | NM_015266 | CACCCTGGTTGTCACGACGAA CTGGATGCCTATTTAGAAATA CAGAGTCGCCTTAGTCCAGAA TTGGCTCACCTTGGTAACAAA |
| 23316 | CUTL2 | NM_015267 | AACATTCTCATAAACATTTAA AGGGAGGAACTCAACCATCAA |
| 23324 | MAN2B2 | NM_015274 | GTCCAATATCAGAACTATTAA CCGGACGTTCTTTATTCACTT CACGGGTTTCTCTATGAAACA CGGGCTTTCTTTGGAAGATGA |
| 23326 | USP22 | XM_042698 | AAGGTGAATTTCATAAATGTA CTGAGTTTATACAGAAATTTA |

EP 2 295 543 A1

Table4

| | Gene | Accession | Sequences |
|---|---|---|---|
| 23332 | CLASP1 | NM_015282 | CAGGATTAGAAACACACTTAA AAGCGTAATGTTACACTTTAA |
| 23347 | KIAA0650 | XM_113962 | AAGGATCTTAACAGTCAATTA CAGGGATAAAGTAATTCCTAA AGGACCAATTATCTCAGTCTA CCCGTTCGTCTCTCAATGTTAAA |
| 23352 | RBAF600 | NM_020765 | CTGCGTGAAGGTGAAAGTCAA CAGCAGGGTTATGCCCTTAAA |
| 23367 | LARP | NM_015315 | CAGGCTGAAGTGATTCATTCA CACCTAATCCACAGAAAGTAA |
| 23378 | KIAA0409 | NM_015324 | AAGGGTAGTACTACAAATGAT AAGGGAAGAAGAAATGTCAAA |
| 23379 | KIAA0947 | XM_029101 | CTGGCAGTTTATTGCTCTTAA CCGCATCATTCAGAACATAAA |
| 23385 | NCSTN | NM_015331 | CCCGAATGCTGAGCCCTGAA CAGTGCCAGATCCAAGTAAA AAGGTTTAATGTCAGGGTCAA TCAGATTGGGATTAACATAAA |
| 23386 | KIAA1068 | NM_015332 | AGGCAGGAACTTGAAGAGAA CCCTGCTTAATAAACAGCAA |
| 23396 | PIP5K1C | NM_012398 | CGGCGAAACCACCTACAAGAA GACGGCGAGAGCGACACATAA CGGCAAGAGACCTATTTATAATA CCGCGTCGTGGTCATGAACAA |
| 23398 | KIAA0073 | NM_015342 | AAGGTTGATGCTGTAAGATTA ATGGGAGACATTCACACCAAA |
| 23403 | FBXO46 | XM_371179 | CAAGATCACATGTGACTTATA CCCGCCTTCTGCGGCCCTCAA CAGCGATAGCTCCAAGGCCAA CTAGATGGTTGCCTATTAAA |
| 23404 | EXOSC2 | NM_014285 | CTGGTAAAGTTGGAAGAATAA CTCCATTCCTATATCCTTTAA |
| 23411 | SIRT1 | NM_012238 | CAAGGATTATTGTGATATTGAA TTGGGTCTTCCCTCAAAGTAA CAGGATTATTGTATTTACGTT ATAGCTGTTCTTTATGCATAA |
| 23413 | FREQ | NM_014286 | CTGCGTGGCTAGGATGATTAA CCCGGGCTGGTTGGTCTTGAA GCGGAATAAACCCAATGGTTA CGGAATAAACCCAATGGTTAA |
| 23416 | KCNH3 | NM_012284 | CCGGCGGGAGCAGGTGGTAAA AGGGTAGTAGATGGCATTGAA CCGTACCCACATTCGTGTCCAA CAAGGACATCAGCGAAACCAA |
| 23417 | MLYCD | NM_012213 | CCCGGGTTCCTGAACCTAGAA CAAGGTGTGTGTAGTAAGTTA AAGTTGGATAAATAGGCTTTAA TAGGCTTTAAATGATCAGGGA |
| 23428 | SLC7A8 | NM_012244 | CTCCAATATATTTACACGAA TGGTGAGAAATTGTAAACAAA CAGGCGGTTGAGGAACATATT CCCGAATTCTGTCTGCTTAAA |
| 23451 | SF3B1 | NM_001005526 | AAGACTCACGAAGATATTGAA CAGGTTATTATGACCAGGAAA |
| 23456 | ABCB10 | NM_012089 | ACCCTGCTTCTGGAACTATTA CCCAGTGTGGCTGAGATCCAA CTAGGTATTATTAAGAATGAA TAGACCCTAAGAAGTAATTAA |
| 23457 | ABCB9 | NM_019624 | CTCCCTAGCCTCCTAAGCAAA TTGAGAGAACCCGTCAATAA CTGCGTGACCACGCCATCTA CCCGGTCAATAAAGTGTACTA |
| 23463 | ICMT | NM_012405 | CAGAAAATGACTTATTTCTA CAGAGTGTAAGTAAAGGATAA |
| 23468 | CBX5 | NM_012117 | CAGGGAGAAGTCAGAAAGTAA GCCGATGACATCAAATCTAAA |
| 23471 | TRAM1 | NM_014294 | CCGGAACAATTGTGTGCTTTA CTGGCATTATACTGAACTATA ATGGTGTGAATGGAACATTAA CAGGGTAACATGGAAATACTA |
| 23474 | ETHE1 | NM_014297 | TCCCTGGAGCTCCCTAAATAA CAGCTGTGAGGAGTTTGTCAA |
| 23480 | SEC61G | NM_014302 | CAGCAATAGGATTTGCTATAA CTAAATTAAAGTAATTTCAAA |
| 23484 | LEPROTL1 | NM_015344 | TCCCAATGTTATGCAGACATA CACAGTCATCTTTGCAACTAT CAGAGACTTGCTGAAGGATTA CAGACGGTTGGCATACGTTAT |
| 23495 | TNFRSF13B | NM_012452 | CGTGTAGGTCGTCATCACCTAA CAGCGGAGTGGAGAAGTTGAA AAAGGAGCAAGGCAAGTTCTA AACCCTAAGCAATGTGCATAC |
| 23506 | KIAA0240 | NM_015349 | TTGGATATAGTCAACATATAA CCCAATTAATATACAGCCAAA |
| 23507 | TA-LRRP | NM_015350 | AAGCATATTGGATTTATTTAA CAGATCAACCTCAATAATGAA |
| 23511 | NUP188 | XM_497078 | AACAATGTTATTCGGCTGAAA AACAACCTCATTGCTGTTCTA |
| 23513 | SCRIB | NM_015356 | CTGGGAGGCAACGATCTGGAA CAGGATGAAGTCATTGGAACA |
| 23524 | SRRM2 | NM_016333 | CCCACTTGGTACAGAAATGAA CTGGGTCATCTCCAGAAACAA |
| 23531 | MMD | NM_012329 | CGCCATTTGGAAATACCTTTA ACGGACTTTATGCGGCATTTA AACCACTAATATGTATGTAAA |
| 23532 | PRAME | NM_006115 | AAGGAAGGTGCCTGTGATGAA CAGGATGTGCATGCATCCTTGA |
| 23533 | PIK3R5 | NM_014308 | CAGGATCTATAAACTCTTCAA TAGGATCCTTTCTAGAAGGAA ATCGCAGATCAAAGTGGACAA ACCGGTGCTGCTGCAACTCTA |
| 23543 | RBM9 | NM_014309 | TAAGAAGATGGTCACACCATA TCCGTTGGTTTATGGAGAAAA ACGGCTGCTTTGAAAGGTTTA AACGGTATAAAGAGAGATCAA |
| 23548 | OSRF | NM_012382 | CAGGAAGATACTATTGGTGTA CCGCTTTATGTTAAAGATCTT |
| 23549 | DNPEP | NM_012100 | CACGAGGGTGATGAAGGCCTA CAAGTACTTCATGACCAGGAA GAGCTGAAGCTGGATTATTAA AAGTGGATTGTCACTCAGAAA |
| 23550 | PSD4 | NM_012455 | CTGCCAGGAATTCATAACCAA CACGGCCAAGGTGAAGCGCAA |
| 23551 | RASD2 | NM_014310 | CTGGGAGGGATCCGACAGAAA CTCCGTGTACACTATCAATAA ATGGATCTCCGTGTACACTAT |
| 23562 | CLDN14 | NM_012130 | AACAAGTTTAATATAAAGTGA ACGGCCGTGTCCTACCTGAAA CGGGTACAGGCTGAACGACTA CACGGCCGTGTCCTACCTGAA |
| 23563 | CHST5 | NM_012126 | CAGGGAGTAAGTTACTGCTAA AGGGAGTAAGTTACTGCTAAA |
| 23564 | DDAH2 | NM_013974 | CTGGATCTGGCCAAAGCTCAA TAGGATAGTAGTATAGGAAGTAGA |

156

Table4

| 23567 | ZNF346 | NM_012279 | ATGGGCCAGACTACAACTTAA GGGCCTCATGAAGGTCTTTAA CGCAAAGAACTTAAAGCTGAA ACGCAAAGAACTTAAAGCTGA |
| 23584 | VSIG2 | NM_014312 | CTGCCTGGTCAGGTTCCAGAA CAGCACCGTGACGACCACCAA CCCACCAGATTTCTACACCAA ATCCAACTGGTTCTAAGTCAA |
| 23586 | DDX58 | NM_014314 | AAGGATAATACTGAACTGTAA CAGGAATGTGAAGAAATTCTA |
| 23589 | CARHSP1 | NM_014316 | CACCATCTGAAGAGCATTAAA CTGCACATCTCTGATGTGGAA |
| 23590 | TPRT | NM_014317 | CTGGCACGAATTGGAAATACA CAGAGTGATGGTGTGCAACAA |
| 23604 | DAPK2 | NM_014326 | ACGCCGGTGGACAACCAGCAA AACCCTTGGTACCACAGAATA CTGGTTAAAGAGACCCGGAAA CGGAATTTGTTGCTCCAGAAA |
| 23613 | PRKCBP1 | NM_183048 | TTGACGCAAATAGCGAAAGTA ATCGACACGTTCCGATCACAA CACCATTGAACAGTTATCCTA AGCGAACTTGTCATAGATTTA |
| 23616 | SH3BP1 | NM_018957 | CCCTGGAGACATCAACTTCAA CAGCCCAGTCTCTTTGAGTAA |
| 23620 | NTSR2 | NM_012344 | CAGCTTCGTGTGGTTCCACTA CAGAGCCATCGTGGTCATGTA TCGTATGGAAGAAGACCTTTA CCGGACCTGAATGTAATGCAA |
| 23624 | CBLC | NM_012116 | CAGACCATCCCTGCCAACAAA CAAGATCTGTGCTGAGAGCAA CTGGGAGGCCGTGAGTATCTA CAGCCGAAAGTGAGACTCCTA |
| 23626 | SPO11 | NM_012444 | TACCTTCTACGATACAACTAA ACAACTAATGTTAACGCATAA TACATATATTATCTACATCAA CAGAGTGTACTTACCTAACAA |
| 23632 | CA14 | NM_012113 | GTGGGTGAGACTAAGAATATA TTGCATGAAGTCAGGCATAAA AAGCAGGATCCTCGTATACCA CGGGATCTCTCCTTAGGATAA |
| 23633 | KPNA6 | NM_012316 | CAGGTACTTATTATTGGCCAT ACGCTTGGATTGAGTTATTTA CTGGGTGGATTACATATGATA AACCATTCTCTTACAGTTTAA |
| 23636 | NUP62 | NM_016553 | CTGGAGAGCCTGATCAACAAA CCGCGAGGTGGAGAAGGTGAA CGGCTTTGCCTTGAATTTAAA ACCGGCTTTGCCTTGAATTTA |
| 23645 | PPP1R15A | NM_014330 | CAGGATCAGCCGAGGATGAAA GTGGGTTTATATAAGGAATAA TGGGTTTATATAAGGAATAAA CCAGTTGTTGATCTTATGCAA |
| 23650 | TRIM29 | NM_012101 | CTCCATGTACCTGACACCCAA CCGGCCATTCTACGTCAACAA |
| 23673 | STX12 | NM_177424 | AAGAATTTGATGAGCCAGCTA CTATATTTAATTGGAAGGTAA TCCTTCTGTTATTATACTTAA CTGGGTTTAAAGTTTACTTCA |
| 23678 | SGKL | NM_013257 | CCGAGATGTTGCTGAAATGTA CATGGTATGATCGAAATGTAA CCACAGCGAGACCCTAGTTAA ACCAACTAACTTACATGCTTA |
| 23682 | RAB38 | NM_022337 | ACGAGGGTCTATTACCGAGAA CAGCAAAGGCCTCAAGTCTTA CTCCAAGTTAAGTCTCCCTAA ATGATTTGGACTCCAAGTTAA |
| 23683 | PRKD3 | NM_005813 | CAGGCGGGAAACTGAATAATA CTGAGCCTATTTCTAGTATAA CGGGAGAGTGTTACCATTGAA CACGATATGTCAGTACTGCAA |
| 23704 | KCNE4 | NM_080671 | CTCGGAGAACATCCATCAGAA TCGGAGAACATCCATCAGAAT CCCAGCCCATTTGCACACTAA CCAGCCCATTTGCACACTAAA |
| 23760 | PITPNB | NM_012399 | AACAAAGTAAATGAAATTTAA CAGGATAAAGACTGATGTTTA |
| 23761 | PISD | NM_014338 | TTCCTTCTGCCTACAAATAAA CAGCTTCGTGACGCACACCAA |
| 23765 | IL17R | NM_014339 | CAGGTTTGAGTTTCTGTCCAA CAGGAAGGTCTGGATCATCTA CGGCACCTACGTAGTCTGCTA CAGCGGGTCTGGTTATCGTCTA |
| 23770 | FKBP8 | NM_012181 | CTCCTACGACCTCGCCATCAA CTGCCAGGAACTGACCACCTA AAGACAACATCAAGGCTCTCT AATCCCATGGAAGTGGCTGTT |
| 23786 | BCL2L13 | NM_015367 | CAGGCATTTCGGGAATGTACA CTGGATGGTCCCTGTCTATAA TTGGGCTTATAGAGCCAGTTA CCGTGTCTTTCCAGCCCTAAA |
| 24139 | EML2 | NM_012155 | CAGCAGCTGCTTTGTCACCAA CTGCGGGAAATCTCACATCTA |
| 24142 | NAT6 | NM_012191 | CTGCTGGAGACACAATATCAA CCGCCTGTCACGGGTGCTGAA |
| 25764 | HYPK | NM_016400 | CAGCAGAGTTACTGTACTCAA CTGGATTAATTTATGGCAATA |
| 25776 | PGEA1 | NM_001002880 | CGGCTGAAAGTGGACATCTTA CTGGAAGAGAGTATCATATAA |
| 25777 | UNC84B | NM_015374 | CTGGGAGTGTGTATATATGTA AAGGAGGATTTCCGCAGGGAA |
| 25778 | RIPK5 | NM_015375 | CTGGTCCATTAGATTCCGAAA CACCGCTTTATCGCCAGCTAA TTGCTTCATCGTAAACCTAAA TAGAGCTTGTTTGATGTTAAA |
| 25788 | RAD54B | NM_012415 | CACGAACTTCGACCTACTGAA CAAAGGCAGATCAGTAAGCAA CTGGATCAAATTAAGAATATA ACCCAAGAAATTATAAATAAA |
| 25793 | FBXO7 | NM_012179 | CCGATTTACAATTACATTGAA CAGGATGAACAACCAAGTGAT GACAATACTGTCAGAGTTCAA CACAGATTGGAAAGAACTGTA |
| 25796 | PGLS | NM_012088 | AAGCCAGGTGATCACCATTAA CAGCCGTGTGACCCTCACACTA |
| 25804 | LSM4 | NM_012321 | ACGGATCTGCTCAGAAAGGAA CGACAACTGGATGAACATTAA |
| 25822 | DNAJB5 | NM_012266 | AACGAGGATGAGATCAAGAAA CCGGAAGATGGCCTTGAAGTA |
| 25824 | PRDX5 | NM_012094 | ATGGTGGTACAGGATGGCATA CAGCCAGGAGGCGGAGTGGAA CTGAGTGTTAATGATGCCTTT CACCTGCAGCCTGGCACCCAA |
| 25828 | TXN2 | NM_012473 | CCCGGACAATATACACCACGA CAGGATGGACCTGACTTTCAA CGGGAAGGTGGTGATGGCCAA CCCAAACTAGTGAGAGTAATA |
| 25830 | SULT4A1 | NM_014351 | AAGCAGATGCTTAATAGTAAA CCGGAGGTTTATGAATGATAA |
| 25832 | DJ328E19.C1.1 | NM_015383 | AACGATGACGATGAAGATGTT AACGAGAGCTGACCCAGTTAA |
| 25839 | COG4 | NM_015386 | CAGGGATGTTCTGTGTAATAA CAGGAGCTAATTGGCTTATAT |
| 25847 | ANAPC13 | NM_015391 | CTGGAATTATTTGCCTGTTAA ACGGTTTGTTTCAGTCTCCAA |

EP 2 295 543 A1

Table4

| | | | Sequence |
|---|---|---|---|
| 25853 | WDR40A | NM_015397 | CAGCATCAATGTGATTAGTAA AACACTGATAATAGGAAGTAA |
| 25859 | PART1 | NM_016590 | ATGAGATATTCTACACATTAA AAGAAAGTCACTCATGAGTAA |
| 25865 | PRKD2 | NM_016457 | CCGGCAGGGCCTGCAATGCAA CAGCGGCCACCTTCCCTTCAA TTGGGTGGTTCATTACAGCAA CACGACCAACAGATACTATAA |
| 25873 | RPL36 | NM_015414 | CGGGAGGAGCTGAGCAACGTA AAAGAACAGCTTGACAGGAAA GCCCTCAAATTTATCAAGAAA CTGCCCTCTCCCTGAAATAAA |
| 25874 | DKFZP564B167 | NM_015415 | AAGCTAAAGCACACAAATAAA CAGGGTTTATTTGGTCAAGAT |
| 25886 | DKFZP434C245 | NM_015426 | CCGGGCTCACCACAAATGAT TAGAAATTGATTGCACATTGA CCCGGAATGTGTCCACTCGTA |
| 25903 | OLFML2B | NM_015441 | CAGCGTCGTGTTCGAGAATGA TCGAGAATGAGTATTCCTATA TTGTATAATATTGCAAATGTA GCGGATTTACGTAACCAACTA |
| 25911 | DPCD | NM_015448 | ATGGTCATATATTCCTGAGTAA AAGGAAATGGCTGAAGAATAT |
| 25913 | POT1 | NM_015450 | CAGGCTGAAGATTCAAGTATA CAGGGTCTTGCCAGAAAGTAA |
| 25917 | THUMPD3 | NM_015453 | AAGAGGATGTATCAACATTAA CAGGAGAGAAACATTGCTTTA |
| 25926 | DKFZP586L0724 | NM_015462 | CAGGACAGTCATCCTCTATAA TTCGGGAGCTACAGAAATTAA |
| 25927 | C2orf32 | NM_015463 | AAGGATGGTAACAAACTAGAA CAAGGTTGAGGTGAAGATTAA |
| 25932 | CLIC4 | NM_013943 | CACGAACATGCAGTTATTGAA CTGGATATGTACTAACGAATA TAGCAGTACAATGATTAGTAA CAGGGAAGTTAGTCAAATGAA |
| 25937 | WWTR1 | NM_015472 | AGACATGAGATCCATCACTAA CTGGCTGTAATCACTACCATT CTGCGTTCTTGTGACAGATTA ACAGTAGTACCAAATGCTTTA |
| 25938 | C14orf125 | XM_113763 | TCGATTTATGAGAAAGATGTA TAGGTTCATATCTACATTGTA CAGCCTTACAGTGCAACATAA CTAGGCATAATATTAGCTAAA |
| 25942 | SIN3A | NM_015477 | CAGAGGATATATGCTGATAAA CAGATCCGAGACATCCTACA GAGCGTGTAAGCAAGCGTCTA CTGGTCACTCGAGCACATCTA |
| 25943 | C20orf194 | XM_045421 | ACCGGGAAATTGAGAAGTATA AAGGTAATTATCAGCATTGTA |
| 25959 | ANKRD25 | NM_015493 | CAGGTTCATTAAAGAAGGCAA AAGCTGATTGGAAACAATTAA |
| 25963 | DKFZP564G2022 | NM_015497 | AAGATTGTCATCAACACCATA CAGAAGTTGTAGCTTATACAA AGCGCTGATTGTTACAATGAA CTGGCTTAATTTAAACAGTTA |
| 25972 | UNC50 | NM_014044 | CCGGCTGAAACTGCTTTCTTA AAGAAAGAGCATTTAGTATAA |
| 25977 | NECAP1 | NM_015509 | CGGGAACATTACAAACAAGAA TAGCTTCTCCATCACATTCAA |
| 25979 | DKFZp566O084 | NM_015510 | GAGGATGAAGTTGATTGACTA CCGAGATGGAACAGTATGAAA TTGCATTTGTTGAGACTTTAA CAGATGTTATGTCACCTGAAA |
| 25980 | C20orf4 | NM_015511 | CAAGAGCATAATGTACATTAA TAGGAGCTTAGGAAACAAGAA |
| 25981 | XLHSRF-1 | NM_015512 | CAGGATGTACATCACCACCAA CAGCCGTACATCGATAATGAA |
| 25988 | MIZF | NM_015517 | CCCAAGGACTTTCATGATTAT CAGGGCATTATTCTAGAAACA |
| 25992 | SNED1 | XM_059482 | AAACCTGTTCTTAGACTCCAA CTCAGTGAAGCAAACAGTAA CAGGTAGTAAACACGATCTTA CACCTAGGCTTCAGCCTTAAA |
| 25998 | IBTK | AB037838 | AAGCATGGTGTTAGTCTGTAT GAGGATTTAAATTATCCTAAA TAGAAGAGTCATTACACCATA CAGATTGAGGAGCATGCCATA |
| 26009 | ZZZ3 | NM_015534 | CAGCCTGATGGGAACACTAAA TAGGAATTTACCTGAATATAA |
| 26015 | RPAP1 | NM_015540 | AAGGAGGATAGCTGAAGCCAA CAGCAACATCTGGTAAGAGAA |
| 26020 | LRP10 | NM_014045 | TACAGAGAATCCTAATGATAA CAGGGTGCCATCCCACCTGTA ACCCTATGTAGCTGCTATAAA CACCGGAATGCCAATTAACTA |
| 26030 | PLEKHG3 | NM_015549 | AGGAATGAAGCCAATAATTTA CCGGTCTTCCTGCAAGAAGAA |
| 26031 | OSBPL3 | NM_015550 | CTGCATTGTACTAATCTGTTA AAGAACCAAGGTAGAATTTAA |
| 26033 | ATRNL1 | NM_207303 | CAGGTTATTATGGAGATCCAA CAGGGTGACAAGATATGTATA CAGCTTTCCGCCTAACTAGAA TTGGTGTCGTAGGTAAAGGTCAA |
| 26038 | CHD5 | NM_015557 | CCCAACGTGACATGATCCTCAA CAGCTACAAGATTGATTACAA |
| 26040 | SETBP1 | NM_015559 | ATGGAATGCCTTACACATCAA CAGAGGGAAGTCTGAAGCTAA |
| 26043 | KIAA0794 | XM_087353 | CAGCTTGAAAGGAGTGTTTAA CAGCACGTGCATATTCATTTA |
| 26050 | SLITRK5 | NM_015567 | TTGCATCTAAACAATAATAAA CAGGCTAAACCTGAGGAGTAA |
| 26063 | DECR2 | NM_020664 | CAGAATCGACATTCTCATTAA CTGCACCACCTGTTTGCATAA AAGATAATCTATTTACTGTAA AAGAAAGAGGATTAAAGAGAA |
| 26088 | GGA1 | NM_001001560 | CAGAGGCCTACCAGATGCTAA TCGGACATCGGAGAAGGTGAA TCGGTGTGTCTCCTTCATTCA CCGAAGAATGTGATCTTTGAA |
| 26091 | HERC4 | NM_015601 | TACGTTAGCGCTAAATGACAA CAGAGTACCCAGAAATATTAA TAGAAATACTACATAGGGTAA GAGGAATTATTGGATCCTAA |
| 26127 | FGFR1OP2 | NM_015633 | CCCGCCGGTGATGGCGTTGAA CTGCTAAATTTCATCCTTAT TAGACATATTTAGTAGGTTAA ACGGAGGATTTGTACGCTAAA |
| 26145 | IRF2BP1 | NM_015649 | CTCGATTTCGAGAAAGAGAA TTCAAGTACCTCGAATATGAA TTCAAGTGCGCTTCAA ACCGCTGCCGCGGATAAATTA |
| 26148 | C10orf12 | NM_015652 | CAGAGGATTTATTAATTATAAA CAGGATTTAGAGGCAAATGAA |

Table4

| | | | |
|---|---|---|---|
| 26154 | ABCA12 | NM_015657 | CACTCAGTTAATACCGATCAT TTGCGACTCTTAATCAACGAA AAGCCTAATGATCCGACTTTA CAGGTGACTCCGATAATATAA |
| 26155 | DKFZP564C186 | NM_015658 | AAGGATGAGGACAGGAAGCAA CACGGACAGTGCTGCATTCAA |
| 26165 | C9orf36 | NM_015667 | GGGAAGAGAAAGAGTCCAGTA CTCAGAAGCTCTCAGATCCTA |
| 26166 | DKFZP434I092 | NM_015668 | CCCAGACTATATAAACTTCAA TCGGACCAAATTGCTTATAAA |
| 26175 | C14orf109 | XM_058628 | CAACATAGTGATGATTCAGAA CTGGCCTTGGAACACATTCAA |
| 26190 | FBXW2 | NM_012164 | CAGAGTGATGCACTTTACTA ACGGAATGGGTCACCAAGGTA CCAGTGGGACTTTGCCAGTTA CACGCAGTGCACAATCATTTA |
| 26212 | OR2B6 | NM_012367 | CCACAAATGCTAGTAAATTTA CAGCATCATACAGGAGTTTAT |
| 26226 | SHFM3P1 | NM_012165 | CCGGGAAGAATACGTGCCAGTTA ACCGCTGGCTGTGCACTTCACCAA CCCAGTGAAGGTGTCTCAGAA |
| 26227 | PHGDH | NM_006623 | CTCGAGAATACTGCCCAGTTA TGGGATGAAGCATATAGGGTA CACGACAGGCTTGCTGAATGA CAGCAATAACCGTCTAATAAA |
| 26228 | BRDG1 | NM_012108 | GAGGCTTCATTCTTACAGTAA CACCTGAACACCAGAATTAGA AAGGAACCAACTGAAGATTAT CACCAGAATTAGAATTAAACA |
| 26232 | FBXO2 | NM_012168 | CACAAAGTGGGCTCTCAATAA TCGAGGGTAGATAGGCCTTAA AAGCTCACCGTTAAGCTACTG AACGATGAGAGCGTCAAGAAG |
| 26233 | FBXL6 | NM_012162 | CCGGCTGACTCTAGCCAAGGA CCCGGGCCTGCTCTACCTCAA |
| 26235 | FBXL4 | NM_012160 | CACCGGTCTTTCCCATGTTAA TTGGCTGGTGTACCAAATGTA TCCGAATTAGTACGCCTTGAA AACGACTTGTTCTCTATCGAA |
| 26254 | OPTC | NM_014359 | CACCTGCAGAATAAACCTGATA CAGGGACAGCTTCTCTCCCAA |
| 26256 | CABYR | NM_012189 | ACCAGTGTAATGTATCAATAA CCAGTGTAATGTATCAATAAA |
| 26261 | FBXO24 | NM_012172 | CCACCACTCAATGACCTTCAA CCGAATCTTCATGCAAGGAAA CCCAAGGTGTGACACAGTTTA CAGGCGAGTGACTGTCAAGAA |
| 26273 | FBXO3 | NM_012175 | CTGACGATTATCGATGTTCAT CTGCCAGAACTTAGCTCTGTA GTCGGGTATATGAATACACAA TCGCTAAGACCTTGACTTTAA |
| 26275 | HIBCH | NM_014362 | CAACTTAGGTATACAATATAA CACGGGAGTCATAACACTAAA |
| 26276 | VPS33B | NM_018668 | CCGGAGAGGCATGGACATTAA CAGCGTTGGATCAACACTGTA |
| 26292 | MYCBP | NM_012333 | AAGCAGGAGAATCCAAAGTAA CAGGACAGAGGCAATATACAA |
| 26298 | EHF | NM_012153 | CAGCAATAATATGCAAATGAA CTGGAAGTAAGAAGAATTAAA |
| 26330 | GAPDS | NM_014364 | CAGGCCAATGATAACCTTATA CCCGGAATACACATGGTGACAT CTCCATGAACATTGTGAGCAA CCGAGACAAGTGAAACGGGAA |
| 26338 | OR5L2 | XM_372387 | CCGCTTTGTGGCCATCTGTAA TTCACTCGTCCTTAGCTCTTA CACCACTATCCTGAAGATACA AACAGTGGAGATGTTGACAAA |
| 26469 | PTPN18 | NM_012169 | CCGGTGTAAGTCAACGCCA GTGGGCCTGATCAAGTTAA CCCAATGACTGTAGCATTCAA CAGGCAGCACATCAGTCCACAT |
| 26507 | CNNM1 | NM_020348 | CAGGTCTTAAGTGCAAATCAA CAGGTGCAATTCCAAGCATAA |
| 26515 | FXC1 | NM_012192 | CACCGAGAAGCTGAAAACGGAA CAAGTATTTGTTGGTTCATAA |
| 26523 | EIF2C1 | NM_012199 | CCAGACCTCGACCATGATAAA CAGCTACACAACTTAGATCCCTA |
| 26533 | OR10G3 | NM_001005465 | CTGCGTTTATCCTGACAGGAA CTCATGATGAACTTCACTTTA |
| 26538 | OR10H2 | NM_013939 | AGAACTGAAGGTTGCCATGAA TGCCTCTGTCATCTACCTCAA TTGGCCTGTGTGGAAATAATGTA CACCCTGATGGCCACCACCTA |
| 26539 | OR10H1 | NM_013940 | CACCTTGATGGGCATCACCTA TTCAGTAAACTCTACCCAGAA GACCTTCTTCAGTAAACTCTA AAGAAGACCTTCTTCAGTAAA |
| 26578 | OSTF1 | NM_012383 | CAGATGCAGTTCGAACATTAA GAGGACTATCTCGATGATGAA |
| 26583 | DUX2 | NM_012147 | AGCCAAAGCAAGGCCCTACAA CTGAGGCAGCACTGGCATTAA |
| 26610 | ELP4 | NM_019040 | AAGGAATTTGATGAAGATGTA TTGGACCAGTATCATCTTCAA |
| 26658 | OR7C2 | NM_012377 | CTGTTGAATCCTATTCTCTTA CGCAGAGACTCTGACATGCA CTGCAGTTACACCACCTTCTA |
| 26659 | OR7A5 | NM_017506 | AAGATAATTTATTGGCTATAA AACTGGTCTTGTAGAATTTAA ACAGTCAGAGATGACCTTAAA AAGCTTAGTTGCCCATATATAA |
| 26716 | OR2H1 | NM_030883 | CTGCTAGACAACGTTTGATCA TACGTAGGTCATGTAAGCAAA CCACATTAAAGTTGCAAATAA |
| 26762 | HAVCR1 | NM_012206 | AACGCTGATCCCATAATGCAT TCGGAAGGACACACGCTATAA TAGCTCCATAAGGCACACAAA CACCATTGTACTCTTACACAA |
| 26873 | OPLAH | NM_017570 | CTGCCTCATCATCGACAGTAA CTGCTGCACATTGGCACCCAA GGCCTTTGGAATAAACAATCAA |
| 26985 | AP3M1 | NM_012095 | ACGGTCTTCCCTGCATATTTAA CTGGATTATGTAGAAGATTTA |
| 26993 | AKAP8L | NM_014371 | TAGTCTGACCAAATGCAAA CCGGCCAATGGCCTCAGGCTA CAGGATAACCACCAACTAT AAGGAACACTTTAAGTACGTA |
| 26995 | TRUB2 | NM_015679 | AAGCTGTAACAGAAAGGTCAA CTGCGGAAGTTGGTTCATGAA |
| 26998 | FETUB | NM_014375 | ACCGCTAAGTCTGCCTTAAA CAGCAGTATATATGTCTGAA |
| 27012 | KCNV1 | NM_014379 | CCGATATGTCCTGCACTACTA AAGCTATGTACAATTAACTAA CTGACACATTTGAGTGTCCAA CTGTATCCCATTATTACTATA |

Table4

| | | | |
|---|---|---|---|
| 27013 | C2orf24 | NM_015680 | CTCCATGATGGTGGCCAGTAA CTGGCTCTCTGGTGTACATTGAA |
| 27018 | NGFRAP1 | NM_012380 | CTGCCATTTATCATGAGATTA TGCCATTTATCATGAGATTAA ATGGCAAATATTCACCAGGAA |
| 27020 | SDFR1 | NM_012428 | TACATAAAGATGAGTAATATA ACAGTATATACAGCATATAA CTGGTACATAAAGATGAGTAA CAGGTATATACAGCATATAAA |
| 27032 | ATP2C1 | NM_014382 | AAGGTGCTTACGAACAGTAA AAGGGCAGACCTTGACACTTA TAGGGTAACAGTGTCCATAAT TACGTATTAAATGCACGTTAA |
| 27034 | ACAD8 | NM_014384 | CTGAATGTAAATCATGATAAA AAGCTCTTTGCTACAGATGAA |
| 27036 | SIGLEC7 | NM_014385 | CCGAATATAGTGAGATTGTAA CAGGGAATGATATAAGCTGGA TAGTGAGATTGTAACGTGAAA CTGGAGGAGTCTGACCCTGTA |
| 27040 | LAT | NM_014387 | CTACTCTGTGTAATAGAATAA CCTCAGATAGTTGTATCCAA CCGTAACTTATTATCACTTT |
| 27071 | DAPP1 | NM_014395 | TCGGTCGTTCATCTTTAAATA CTGGAAATTGGTCAAGGACAA TAGGTAGTATAATAAAGGTAA CTGGGCCTTATTAAAGAGCAA |
| 27086 | FOXP1 | NM_032682 | CAGGCGGTACTCAGACAAATA CAAGACCTCCTTAATAATGAA |
| 27089 | QP-C | NM_014402 | CTGAAAGACCTTTCTCTGGAA CCGCACGTCTTCACTAAAGA |
| 27092 | CACNG4 | NM_014405 | GACGACGAACAATGAACTAAA CTAGTGGTTACAAATCATAA CAGGAGAGCAACTTACCTTCA CCCTGTGTGTTTCGCCAGTTA |
| 27098 | CLUL1 | NM_014410 | CAGGTTGGTCAATGTATCCAA CAGGAAAGTATGTTAGCTATA |
| 27099 | NAG8 | NM_014411 | CAGGTTGTTATCTGTCTATTA AGCCAGTATATTTAAAGGTAA |
| 27104 | EIF2AK4 | BX640919 | CACCCATTATATTCATTGAA CAGTATAATTACTAATTATA TCCGATAATCTTGCAGTGCAA CTGCAGGAATTTAGTAACATT |
| 27120 | DKKL1 | NM_014419 | CCGGGCTGTGGTCTAGCATAA CAAGCACCATATGGAAATAAA |
| 27124 | PIB5PA | NM_014422 | ATGGGAATACCTACCAGTAA CTGGAGGTCATCCATTAGGAA CTGGGACTGGATCGGCTTATA CAGGTAACATTCAGTGAGGAA |
| 27129 | HSPB7 | NM_014424 | CAGGTGGACCATCCTCCCAAA CAGAGGGAAGGTCACAGGCAA |
| 27148 | STK36 | NM_015690 | CTCACCAGTTCTAGCGAGTAA CTGGTCATGTCACCATAATAA CCAGTTGGTGTGTCAGCCTGTA ATGGATGCTGACCTCCTTATA |
| 27154 | BRPF3 | XM_166450 | CCAGATTACCTGGAATTCATA CCAGATATCACCGAATGCAA TACCAACTGCATGAAGTATAA CCGGCTTGAGAAAGAGTCATA |
| 27156 | RTDR1 | NM_014433 | CTGCAGAGCTTCATTTGGAAA CAGCATATCTCGAGAGGGCAA CACAGTGATCACTGAAGGGAA CAACAGTATGGTGCGCATAAA |
| 27163 | ASAHL | NM_014435 | CCGGAGAACATCTGCCATCAA CAGTGGATGTGCAATTCTTAA |
| 27177 | IL1F8 | NM_014438 | CTGAGTGGAAATTCTTTAATA CAAGCCTACTTTGCAGCTTAA TTGGAATTCACACATGCATAA AACGCTCTCGTTTAGTATGTAT |
| 27179 | IL1F6 | NM_014440 | CCAGCCCACACTGCAGCTGAA TCCAGTCACTATTGCCTTAAT CTGGGCCTGAATGGACTCAAT AAAGGATATAATGGATTTGTA |
| 27190 | IL17B | NM_014443 | CGGAATGGACTGGCCTCACAA TTGCCACCTTTGTGCCAAGAAA CTGCTGTTTCTTCTTACCATT CCAGAGAAAGTGTGAGGTCAA |
| 27199 | OXGR1 | NM_080818 | TACATCGTTTCTAGACCATTA CTCAATGGAACTCCTGGTAAA CCGCTACTGTGTGATCATTCA CACCAGTTCGGATGAACTCAA |
| 27202 | GPR77 | NM_018485 | TCGGGCCTGCCTGGTGGTCTA TAGAGATATAGCAGTGACCAA CAGCAAGAAAATCCACCAGCCA CACGTGCTGGACAAATCTTAA |
| 27229 | 76P | NM_014444 | CTGGATGTTACCACCAGTAA CAGGATCACCATCCATCTCAA |
| 27232 | GNMT | NM_018960 | AGGGAAGAACATCTACTATAA AACATCAGTGCTGATAGTGAA CAGACGGAAGGGTAAACAATA GACGGAAGGGTAAACAATATA |
| 27235 | CL640 | NM_015697 | CTGGAGTTATGTGGACACTAA TTGGAATAAATTTATCTCCAA |
| 27237 | ARHGEF16 | NM_014448 | TACATTAAAGTTATTTCTTAA CACCATTGTGGTCAAGAGCTA |
| 27239 | GRCA | NM_014449 | CCCGCCCAGTAGAAAGCTGA TTGGACTTCCTTTGGGACTAA ATCAATTGCAGGGGATGCTTAA |
| 27240 | SIT | NM_014450 | CAGACTGGAGTGGACCTGGAA CCGGAGCTCTATGCCTCAGTA |
| 27247 | HIRIP5 | NM_001002755 | CAGGTTATGGATGATGAATCA AAGAAGCAAACTCACCTTAAA |
| 27254 | PIPPIN | NM_014460 | CAGGAGGTAGGTGAGGGCAA CACGTCAAAGCTCTCTTCCCAA |
| 27255 | CNTN6 | NM_014461 | GTGGGTGTGTATAATAATGAA CTGCAAAGGATTCATCTGTAA |
| 27257 | LSM1 | NM_014462 | CAGCAAGTATCCATTGAAGAA CCGCTGTGCATTGCAGCATTA |
| 27286 | SRPX2 | NM_014467 | CCACTCGTTCTTAGGAATGTTA CTGGCTACTATCCGGATGAAA CCGATGGTGTTATACATTAA GAAGATTAACGTCAACGTCAA |
| 27291 | C10orf28 | NM_014472 | AAGGAGCATAATTGTAGAGAA CCCACATGTCATTGAAATTTA |
| 27292 | HSA9761 | NM_014473 | CAGGTGCTATACTTATGTT TTGGCACGTGTGGACCATCTA TGGGATGGTCTAGTAAGGATA CTGGTCCAATCTATTCATAAT |
| 27295 | PDLIM3 | NM_014476 | CGGGAACTACTTTGAACACAA CAGGATTACACCAGGAAGCAA |
| 27302 | BMP10 | NM_014482 | AAGGATGAGTTTCTTAAGACA AGAGTACATGTTGGAACTCTA TACGATGAGTAGACCGGAAA CCCGCTCTACATCGACTTCAA |
| 27303 | RBMS3 | NM_001003792 | CCGACAGCGTGTTTCTATTGAA ACCGTACAGTATTGCAACCAA |
| 27314 | RAB30 | NM_014488 | CTCGCGGGAGATAGAACAATAT TAGGGCTTCGGTGGTAATCAT TCGGTCCATTACCCAGAGTTA AAGAGAGATTTCGGTCCATTA |

Table4

| | | | |
|---|---|---|---|
| 27329 | ANGPTL3 | NM_014495 | CAGTTGGGACATGGTCTTAAA CTCAGAAGGACTAGTATTCAA CAGGTAGTCCATGGACATTAA ACCGTGGAAGACCAATATAAA |
| 27330 | RPS6KA6 | NM_014496 | TTGGATCATCTGCACCAATTA CGGGAGGCTAGTGATATACTA GGCGAGGTATACTGCTGAACAA |
| 27334 | P2RY10 | NM_014499 | CTGACGTGCATCAGTCTTCAA TCAGAGTTTCGTGACCAACTA CTCCCTATCATATTAACTTTA AAGTTGAATAGTTTACCTTAA |
| 27335 | EIF3S12 | NM_013234 | AGCCAAGAAGAGAGCATTAAA AAAGATGTGTTGACTCAGAAA |
| 27338 | UBE2S | NM_014501 | CCGCCTGCTCTTGGAGAACTA CCGGCCGGCCGCAGCCATGAA TCCCTCCAACTCTGTCTCTCAA CCCGATGGCATCAAGGTCTTT |
| 27342 | RABGEF1 | NM_014504 | ACGGAATTCTTTATCAATAAA CAGGAAGAAGATGAGCCTTAA |
| 27345 | KCNMB4 | NM_014505 | CCCGCGCTGCAGGATCTGCAA AAGGAGGCTTGTAGAAAGCAA CTGGAAGACTTGGAACCTCAA ACCCTCAGTTTCCACTATTAA |
| 27350 | APOBEC3C | NM_014508 | CAGCATAACCAAATCTTACTA ACGCTGTAAGCAGAAGATAA |
| 27434 | POLM | NM_013284 | CTGAATGTCTCCAAGCAATTA TAGGAGCTGGGAGGAAGATAA CGGGAAGGACTGCGAACCTTA CCCAGAATGCCCAGAAGAGAA |
| 27436 | EML4 | NM_019063 | CAGATGCAAAATACCATAATTA CGGCAATTCACTAACAAGAAA CAGGAAATCGATCGGATTGTAA TCGGATTGTAAGGACATTGAT |
| 28231 | SLCO4A1 | NM_016354 | CTGCAGGGAAATGCACTTCAA TGGATTGTAGTTAGAATACTA |
| 28232 | SLCO3A1 | NM_013272 | TAGGACAAAGTTTATCTATAA TCGGATGGCCATGCTCGTCAA |
| 28234 | SLCO1B3 | NM_019844 | ATGGATAAGTCTATGCATCTA CTAGAATATAAGGAGGTAAA TAGCTATGCCTTTATGGTTAA TTCATCTATGTTGCAATTCAA |
| 28951 | TRIB2 | NM_021643 | TCGAAGAGTTGTCGTCTATAA TACCCTTTCCATGACATTGAA CAGGTTGTCCCACATGTATAA CACGTGGACTCTAGTATGTAA |
| 28952 | CXorf37 | NM_014008 | CAGGAAGGAGGAGGTCTATAA CAGAAGGAAATCAACTCCCTA |
| 28955 | DEXI | NM_014015 | ATGAGTGTTGTGCCTACTTAA AAGCAATACTTAGGAGGCTTA |
| 28956 | MAPBPIP | NM_014017 | TAGAAGAACCTTCTTAGACAA CACCGCTGCCATAGCCAGTAA |
| 28959 | LR8 | NM_014020 | CCCGGCCAAACTTGCTGGCTAT CACAATGCTGATTAAACATAA CTGATTAAACATAATAAACAA GAGGAAGTGTTCACAGCAAT |
| 28964 | GIT1 | NM_014030 | ATGGCTGACAGCCTTGACTTA CACCTTGATCATCGACATTCT GCCGCCTGAGGATGTCCCGAAA CAGCCTTGACTTATCCGAATT |
| 28965 | SLC27A6 | NM_014031 | TAGACAGATTTGAAAATATAA AGGGAACTTTATGATCAAATA |
| 28977 | MRPL42 | NM_014050 | CAGTAATATATAGTAAAGTAA TAGCTTATGTATGATCCTTAA |
| 28988 | DBNL | NM_014063 | TGGGCTCTCTTTACCTATGAA ATCCGTGTCTCTGAAGAACAA |
| 28989 | AD-003 | NM_014064 | AAGGCCCGAACAAGACAGGAA GAGGTGGATATGGTCGACATA ACGGAGGGACTTCCTGGTTCAA CTCCAGCAGCATCGACACATCAA |
| 28990 | HT001 | NM_014065 | AAGCAACTTTATGAATATCTA GTGGAAGATCATAGTAATGAA |
| 28996 | HIPK2 | NM_022740 | CCGAGTCAGTATCCAGCCCAA CAGGAAGCACCAGTGAA TCCCGAAGTCTCCATACTAAA AACCAGTACCCTTACATATAA |
| 28998 | MRPL13 | NM_014078 | CAGGTGGATTTAGACAAGTAA CAGGAAGGTATAGTCCTTCAA |
| 29028 | ATAD2 | NM_014109 | CTGGTACTTAGGCACCATAAA AAGAATAATTAGCAGCGTTAA CCGGATAAAGAAGAACGACA AAGGCATTATAAAGATCGAA |
| 29068 | HSPC063 | NM_014155 | CAGGAAATGCTTGGAAAGCTA CCGCAGGACATTTCTGATTTA |
| 29085 | PHPT1 | NM_014172 | TAGCCTGGCCACAGAATTAAA CCGATTCCACGTTTCCTTTAA TCGGGCGACATGCAGAAGCAA CACGCCCATTCAACTGAGAAA |
| 29087 | THY28 | NM_014174 | TCAGTTGATCCTAATTAGCAA AGCAAATTACTTCATCATCTA |
| 29095 | ORMDL2 | NM_014182 | TCCGGCTATATGGCCAACAAA TCAGCTATAGCTGCAGTTTAA |
| 29097 | HSPC163 | NM_014184 | CTGGTTCATCTTCCTTCTCAA CTGCTTCTTCATGTATCTTTA |
| 29109 | FHOD1 | NM_013241 | CACGGTCACCCTCATCAACAA CAGCGAGAGGAGCATCTACAA |
| 29110 | TBK1 | NM_013254 | CAGGATAATCGACAGCAGATTA CCCAATTTATCCAAGTTCTAA CTGACTTGACACGTTTGTAAA CAGAACGTAGATTAGCTTATA |
| 29113 | C6orf15 | NM_014070 | ATCGGTGTGTGTGGAGGAGAAA CTAGAGCACGATAGAGGGAAA |
| 29114 | TAGLN3 | NM_001008272 | CTCTGTTTGATTATTTATTTA ACCCAAGATCTCAGAGTCAAA |
| 29115 | HCNGP | NM_013260 | CTGGTCTCGGATTCCACTTAAA CAGCTCATTGTTTACAGGCAA |
| 29116 | MYLIP | NM_013262 | CCCAATTAATAGGATAGCTTA TGCACTATTATAAACTATTAA AAGTAAGTGCTTAAGTATTAA ATCGGGTGCAATACTAGCTAA |
| 29121 | OCIL | NM_001004419 | TCAGATATCTTGAGACAAGAA CTCATAGAAACTGGAGGCAAA TAGAAGCATATTGGAACTGAT CAGGAGAGTGTGCCTATTTGA |
| 29122 | TSP50 | NM_013270 | CAAGTCAAGCAGGAACTCAA CACTCTGGTTCAGATCATCAA CAGGGAGAAGTTCTGCTATGA GAGGTGCTCAATTAAACATTA |
| 29124 | LGALS13 | NM_013268 | TACGTACATTACAATGAGTAT TGGCAATCATGTGGTCATGAA |
| 29127 | RACGAP1 | NM_013277 | CAGGTGGATGTAGAGATCAAA CACCACAGACACCAGTATTA CTGGTAGATAGAAGAGCTAAA CTGAATGTATCTCCTAGCTAT |
| 29775 | CARD10 | NM_014550 | CACAGGGGAGTGTGACACTTAA CTGCACATTATGGAGCATTAA |

Table4

| | | | |
|---|---|---|---|
| 29798 | C2orf27 | NM_013310 | ATGCTTCAGATTCAAGTGCTT ATGATAGCAGAGTACCTTCAA |
| 29799 | YPEL1 | NM_013313 | CAGGGTCTCTTTAGATAGCAA CTGTCAGATATTAGAATTGAA |
| 29802 | VPREB3 | NM_013378 | CTGCTGGTCTTCCCAGGCCAA AATATTCAACATGTCAACAAA TGGGTTAATAATATTCAACAT AAATGGGTTAATAATATTCAA |
| 29841 | TFCP2L2 | NM_014552 | AGGGATGAAGAACGAAAGCAA CTGCACTGTTTAACAAATGAA |
| 29851 | ICOS | NM_012092 | TGGGATTATGTTGTAGTTTAA CCGGAATTGAAAGCAAATTTA |
| 29881 | NPC1L1 | NM_013389 | AAGCACCTTGGTCATGGATTA CAGCTTTGAAGGTTCTATCAA AAGCCTCATGACACTCTCCAA CCGAGTGATGGTGGACTCCAA |
| 29882 | ANAPC2 | NM_013366 | CAGCGGTGCAGTCCCGCCAAA GAGAGTCTATATGCAGAGTAA CTCACTGGATCGTATCTACAA AAGGTTCTTCTACCGCATCTA |
| 29894 | CPSF1 | NM_013291 | CCGCGGCTTCCTGTACTTCAA CAAGATGGTCATCTCCCTCAA |
| 29922 | NME7 | NM_013330 | CGGCAAACACTGCTAAATTTA CAGGTTGGGACATTTAGACAA TTCATGTAGATCACCAGTCAA CCCGGCATTTACGCCCTGGAA |
| 29924 | EPN1 | NM_013333 | AACTTTGGGAATAAATGGCAA TCCCGCGACGCTCACCCTGAA |
| 29927 | SEC61A1 | NM_013336 | CCGAGTGGACCTGCCAATCAA AAGGTGAATAAGTGACAAATA |
| 29930 | PCDHB1 | NM_013340 | CGCGCTGAGAACCATGGATTA ACGTTTCAGATTGACCCTCAA ACCAAGCATTCTAGAAAGGTA CTCGGCCTTGTCCATATGAAA |
| 29933 | GPR132 | NM_013345 | CAGGATTGCCGGGTACTACTA TACCAATTTCTCGTTCCTGAA CTGGGTCACCATCGAGATCAA ACGGACCATTCCCGCCAAGAA |
| 29945 | ANAPC4 | NM_013367 | TGGCTCGGAGTCTTTATTATA CTGAGCTAGACTCCTAATCTA CAAGGCTAGATGAACAGTGTA CAAGCTGGTGCTGCCGCTTTA |
| 29946 | SERTAD3 | NM_013368 | CAGCCTGATCCAGTCTTCTTA CAGGCATGTCCTCATCCATAA |
| 29947 | DNMT3L | NM_013369 | CCACTGCAAACCGGCACTGAA AACTCACTTCCTCTTTATAAA AAGCATGTGGTTGATGTCACA AAGGCTAACCAGCGAAATATA |
| 29948 | OKL38 | NM_013370 | CCCGGTCATCATTGTGGGTAA CAGAAGCAGGTCCCAAATAAA |
| 29953 | TRHDE | NM_013381 | CTGGAGATTATTAATTGATCA CAGGAACAGAATACCACTAAA CCGAATCTATAGAATAAAGTA CACCACTGAATTCATGTAATA |
| 29954 | POMT2 | NM_013382 | ACCAACCATCATTAATGTTAA AACCTGTGGATTATCAAGAAA |
| 29956 | LASS2 | NM_013384 | AACGAAGAGAGTCTTTCTTAA TTGGAGTAGAATGGCAACTAA |
| 29959 | NRBP | NM_013392 | AGGCGAGAAGAGGTGAATCAA AACATGGATACTAGTGCCGTA TCGGTGGAGGAGGGAGTCAAA CCGGTTGACTTCTCTGCTAGA |
| 29965 | C16orf5 | NM_013399 | CACGTAGGACAGGGTCACAAA CTGCCTCATCAATGACTTCAA |
| 29982 | NRBF2 | NM_030759 | TTCGGTTTCTCCTGATACAAA TTATGTGTAATTAATCTATAA CACACTTTGCACTACTCATAA CAAAGGGATAGCCATATGAAA |
| 29984 | RHOD | NM_014578 | CCGGTGGTACCCAGAAGTGAA AACCTGCAAGTGAAAGGCAAA |
| 29997 | GLTSCR2 | NM_015710 | TCGAGAGAGAGCCAAGTTCAA CAAGTTCAAACGCAAGTACAA CTGACAAAGAAGAGAACCAAA CCGGAGCTTCCTACAATCCAT |
| 29998 | GLTSCR1 | NM_015711 | CCCGCAGAACCTGACGTTCAT CAGGCCATGCTCAATAAATAT CGGGCTCAAGCTCAAGATCAA CCGCATCGGGCTCAAGCTCAA |
| 30000 | TNPO2 | NM_013433 | CCCGAGCATCTCTCTCTGTAA ATCGTGCAGGATAAACTCAAA |
| 30008 | EFEMP2 | NM_016938 | CCTGGAGATGGTCACCATGAA CCGCTCCTGTGTTGATGTGAA CCGCTCCGCTGCCGTCATCAA CACGGAATGCACAGATGGCTA |
| 30009 | TBX21 | NM_013351 | CCGCGTGAGGACTACGCGCTA ACCTGTTGTGGTCCAAGTTTA CCGCCTGGACCCAACTGTCAA AACAAATACACGTATGTTATA |
| 30010 | NXPH1 | NM_152745 | AAGCAGCAAATCCACACTAAA TAGGAGTATCTGCTTGTTAAA |
| 30062 | RAX | NM_013435 | CCGCACGACTTTCACCACGTA CCGCGAGGAGCTGGCCGGCAA CAGGCTGAAGCTCCTAAACTT CCAGATAACCATAACAGGGAA |
| 30811 | HUNK | NM_014586 | AACTAAGTACGTTGCAAATAA TACGTTGCAAATAATAGTACA TCGGACCAAGATCAAACCAAA CACGGGCAAAGTGCCCTGTAA |
| 30814 | PLA2G2E | NM_014589 | AAGCTGGGCTGTGAGCCCAAA CTGGGCACCTACAACCGCAAA CAAGTCCGCCCTGCAGTACAA AACCGCAAATATGCCCATTAT |
| 30817 | EMR2 | NM_013447 | TAGGTCAGTGTAAGAATTAAA TTGCTTGATATAGACTGCTAA ACGGGTTTGATTATTTAGTCA AAGCATGGTGATGATATAGGA |
| 30818 | CSEN | NM_013434 | CCCGGCTGACCAACATTCAAA TCCAGGAACATTCCCACATAA CAGGAACATTCCCACATAATA CCCACATAATACATTCCATCA |
| 30836 | ERBP | NM_014597 | ACCACTGTGTTCCACCATATA CCACTGGGAAGCAAAGTTTAA CACGAACAGGCCAATGTTGAA AAGAAGAAATTTCGCAATTAA |
| 30837 | SOCS7 | NM_014598 | CAGCTTTGAAGAACCAAATTA CCCAAGTTGACAAGAACTCAA CCGAAAGTTCTACTACTATGA ACCAAATTAAGCTACCATGAA |
| 30845 | EHD3 | NM_014600 | CACTTTATATACATTATTCTA CTGGTGGTGGATCCCAAGAAA |
| 30850 | HUMPPA | NM_014603 | CAGCGTCACCTTTGAAGTATA CAGGCTGGTGCTGGAGAGTAA |
| 43849 | KLK12 | NM_019598 | TCCCGGGAGAATCACGAGCAA TCCCTGGAGTCTACACCTATA TGGGAACTTCTTGGAACTTTA TGCAATAGTCTGGAATAAATA |
| 49861 | CLDN20 | NM_001001346 | TACAAAGAAAGTAGAATGTAA CTGCAGGAATCTCTAGTTTAA |
| 50484 | RRM2B | NM_015713 | AAGGGCTTATGGACTGAGAAA AAGGAGCAGGCCATAACTTTA GAGCAGTTCAGTGGTACATAA AACCAGGAGGTTATCATGGTA |
| 50485 | SMARCAL1 | NM_014140 | CCCGCAGAGCTGAGAAGTTAT CAGAACAGCATCAGAGGACTA TTGAGTTATGAGTTAGGTCAA CAGCTTTGACCTTCTTAGCAA |

Table4

| No. | Gene | Sequences | Accession |
|---|---|---|---|
| 50488 | MINK1 | CACATGTCTCATCAAGACTTA CCGGAACAAGATTCTGCACAA CGCAGACTTTGTGTTGCTGAA CACGTACGGGCGCATCATTAA | NM_015716 |
| 50489 | CD207 | CTCGGTGACCTCAGAGAGTGA CCAGGATGACTTTGCACGTTA ACCCTGGATTCTGAAATTAAA CAGGATGACTTTGCACGTTAA | NM_015717 |
| 50515 | CHST11 | CAGCATAGTGAGAATTATTTA CCGCAACAAGTTCACCCAGAA | NM_018413 |
| 50613 | UBQLN3 | CGAGGAGTCAGTAGCAATCAA CTCCATATGCTGCAAGATTTA CAAGATTTAGTTAGTACAAAT TTGAGATGCAATTACACCCAA | NM_017481 |
| 50614 | GALNT9 | CCCGGAGATGAGGGTCTACAA CACCAGGAAGCCTACAACAA | NM_021808 |
| 50615 | IL21R | CCCGGTCATCTTTCAGACCCA CTGCAAGAGAGTCCATATTGTT CCAGAGCAGACCCTCAATAAA ACCTGTTGAAATGTGAATTAA | NM_021798 |
| 50616 | IL22 | CTGATAGGTTCCAGCCTTATA CCTGCGTTAGTTACAAAGGAA AAGGTAGAGACTTTCTAAGCATA AACATGTTATTTGACCTCAA | NM_020525 |
| 50628 | GEMIN4 | CCGCCATATGGAAGTGTGCTA CAGCCAGAACGCGACGCTCTA CAGCCGCAAGTTAGAACAGCTA CAGCCTGGACTTTACAGTTAT | NM_015721 |
| 50649 | ARHGEF4 | AAGGTTGGCCTTGGCTCATAA CAAGCCAGAAACCACATTTAA CCGCGACGTGTTGTACTACAA CACATTTAATTTCATAAATAA | NM_015320 |
| 50700 | RDH8 | CAGGGTGTCATCTTCAACGAT AAGGATGAACAGACTCTTCAT CAGCTGTCTCTGGGAAAGCAA CACACCAGCAATGTATGTATA | NM_015725 |
| 50717 | WDR42A | GAGGAATAAGTCATTAATCTA CAGCTGAATTCTTGCCCTTAA | NM_015726 |
| 50835 | TAS2R9 | CTCCCAGTTAGACACACCAA CCAGGTACTTTCAAACAGTTA TGGATTCATTGTACTAGTTAA CTGGCTGAAGCTAAAGATCAA | NM_023917 |
| 50846 | DHH | ATGGCCTCTCCTGACCAATCTA TCGGTCAAAGCTGATAACTCA ACCGAGTGGCCTCCACGCAAA CCCAGGCATAGAAAACCTCGAA | NM_021044 |
| 50852 | TCRIM | AAGGTTGAGATGACAATGTTT ATCAGTATTTCAAATAACTTA AAGCATGAATGCGTTCTGGAA AAGGATGGAGATGAGCAACTA | NM_016388 |
| 50861 | STMN3 | CACGTAGTAAAGTGAACTTAA TTCAATCAAATGAACAATTAA | NM_015894 |
| 50862 | RNF141 | ATCACGGATCATGAATTTATA TTGGTGAGTAACACACATATA CACTGATAAATTGTGCAATAA CTCGATTTCCTTATATCTAAT | NM_016422 |
| 50939 | IMPG2 | GAGGATGGAGTCACAAGTATA AAGGTTGGTAGTTATGTGGAA TCAGGCATTCATCTAAATAAA AAGGAAATTCGTGTACTTGAA | NM_016247 |
| 51015 | CGI-111 | TACCAACATCTTGAATATATA AGGGATTCTAATATTTATTTA | NM_016048 |
| 51020 | C6orf74 | AAGGCTAGTTCATTGAAATAA CCAGATGAAATTGCTGCCTTA | NM_016063 |
| 51031 | C17orf25 | CTGATGTAATTTAGAAATGAA AACGATTTCTTCAGCCTGAAA | NM_016080 |
| 51043 | ZFP67 | CAGAGGGTTCTGGAAGATGAA CCCGGAAAGCTTTCCTGCAAA | NM_015872 |
| 51056 | LAP3 | CCCAGTCTCTTCTTGGAAATTCA ATGGGAGGAGCTGCAACTATA AAAGCTTAATTTGCCCATTAAT AAGCTTAATTTGCCCATTAAT | NM_015907 |
| 51060 | TLP19 | CTACGTGATCTGCACCTTTAA AAGGGAATATTGAGGAATCAT AACAATTAAGCCGACCAGGAA AAGTGTAAATCAGTTATTCTA | NM_015913 |
| 51062 | SPG3A | ACGCCTCAAGGTCTCAGGGAA TCCCGAGAGCCTAGATATTAA CCCGAGAGCCTAGATATTAAA ACAGCAGAAGCTAACAATTTA | NM_015915 |
| 51068 | CGI-07 | CAGCATGAAGATAATGGCTAA CAGAATACACTTCGTATCAAA | NM_015938 |
| 51070 | NOSIP | CAGGATGACGCGGCATGGCAA CCCGTGCACTTCACACCGCTA | NM_015953 |
| 51076 | CUTC | CAGGATGAAGGTAGAACTATA CAGAATATTCCCTAAAGGTAA | NM_015960 |
| 51077 | C14orf111 | AACCATAGTACAACACATTGAA AAGGAAGTATGCGACCATGAA CAGCTGAAGAGATTGGAATACTA ACGGAAATTTAGGGTATTTAA | NM_015962 |
| 51084 | CRYL1 | CAGCTTGATTTGCATTTGACTA CAGCCAGTGATTGTAGTTTCA | NM_015974 |
| 51095 | TRNT1 | ACGCAGAGATCTCACTATAAA AAGGATTTGGGTGGAACTGAA | NM_016000 |
| 51097 | CGI-49 | CAGCTTATAAGTTGCTATCAA AAGAAATGTATCAAATCTGAA | NM_016002 |
| 51099 | ABHD5 | AAGGCCTGATTTCAAACGAAA TACGTCTATATCACACCTTAA | NM_016006 |
| 51101 | CGI-62 | CCAGGTGTGCTTACAAACAAA CAGGTAAAGTGTCTTCAAGTA | NM_016010 |
| 51105 | PHF20L1 | ATGGCTAAGTGGATAATTTAA TCCGTTGATCTTAGTGGTGAA CAGCTGAAGATTGGAATACTA ACGGAAATTTAGGGTATTTAA | NM_016018 |
| 51112 | TTC15 | CAGGCTGGCACTACTAGTGAA ATCCAGGGAGATGCAGTTAAA | NM_016030 |
| 51116 | MRPS2 | CACGACCATCGACCTGGAA CACGCTCAACAACATCTTTGA | NM_016034 |
| 51121 | RPL26L1 | AAAGAAGAACTTATTGAGAAA GAGGGTCACCATGAAGTTCAA | NM_016093 |
| 51130 | ASB3 | ACGCTTGGATTCTACAGCAA ACCCATCTTTGTCGTTTGGAA TACGGTCTGACAGTTATATTA CTGAGGATGTATGAAGTTCCA | NM_016115 |
| 51131 | PHF11 | CACCTGGGATGTGATTTAAA ATGGAGTTCGAGGAATTTATA GAGATCTTATGTCAAGTTCTA ACAGCCCATGATAGCCTCTTA | NM_016119 |
| 51135 | IRAK4 | GTCCATTATTAGATTATGTTA CAGCTTAGAGAGTACTCCAA TTGCAGGACAGTGGTTATTAA ATCCTATTAGTCATATATTTA | NM_016123 |
| 51138 | COPS4 | CAGGAAGTACAAGCATTTAAA CAGAATGAATCAACCAATGAA | NM_016129 |
| 51162 | EGFL7 | AGGAGTGGCACAGTGCAATGAA CAGCACCTACCGAACCATCTA | NM_016215 |
| 51167 | NCB5OR | CAAGCTATGATTGGTTCCAAA TTAAGGATTGTTTATATCTTA AGGCTTCACACCAATGTTGTTAA TCGATCATGTTCCATGCAAA | NM_016230 |

163

Table4

| # | Gene | Accession | Seq1 | Seq2 | Seq3 | Seq4 |
|---|------|-----------|------|------|------|------|
| 51168 | MYO15A | NM_016239 | CAAGATTAGATTTGAACGGAA | CAGGATCGTGTTTCAGGCCAA | | |
| 51179 | HAO2 | NM_001005783 | CCGGTCGGTCGCTGAGATCAA | CAGGAGGATCACAAACTCACA | | |
| 51182 | HSPA14 | NM_016299 | CGCGAGCCATGATGAAATTAA | ATCCCTATTGGTGCAGCTATA | AATGGATTACGTGATATATTA | CTCTATTGAGATAGCATCTTA |
| 51184 | MGC14560 | NM_016301 | TTGGACCTAGATAACAAGTAA | CAGCCTCATGCCTGAATCAAA | | |
| 51187 | C15orf15 | NM_016304 | CAGCATTGTATAGTAACTAA | CCCTTTGTTAATGTTATAGAA | | |
| 51191 | HERC5 | NM_016323 | CCGGACGCCGAAATGCATTAA | ACCATTGAGTATGACAACTA | TGGAAGTAGCATAACTGTCAA | |
| 51194 | IPO11 | NM_016338 | CAGCTACAACTTTGAAGTTAA | GAGGAGATTCTTGAAATATA | | |
| 51195 | RAPGEFL1 | NM_016339 | ACGGAGAAACTTCAATATTCA | CCCATTGCTCCTTCAAGCCAA | ACGGTGGAACTAAAGATTCCA | ACCCAAAGCTCTGGAATTGTA |
| 51199 | NIN | NM_016350 | CTGGAAGAATATCGTGCACAA | CTGGAAGACCTAAGAAATGTA | | |
| 51205 | ACP6 | NM_016361 | GAGGCCAAACTTGAAATACAA | GTGCCTTTATACAATGCCAAA | CCATGTCAGTTTATACCTTAA | |
| 51207 | DUSP13 | NM_016364 | CAGGTGGACACAGGTGCCAAA | ACCCAATTCAGAGATTCTTTA | TCAGTCCATCTCTATAATAAA | ACCCTGAGATGTAAACAGCAA |
| 51208 | CLDN18 | NM_001002026 | AAGGATGAGCAGACAAGTCAA | CCCAACTTGGCTAGTAATAAA | | |
| 51214 | IGF2AS | NM_016412 | CAGGCGGTGGACGCTGCTGAA | CTCAATCTGCCCAAAGCCAAA | | |
| 51222 | ZNF219 | NM_016423 | CCCAATAAACAGGTGTCTTAA | CAAGGAGTCACAGTGCCTTA | | |
| 51234 | LOC51234 | NM_016454 | CACAAAGAAACTATACCATAA | ATGGTTGAGAACAATGCAAAA | | |
| 51237 | PACAP | NM_016459 | CAGGACTACGGAGTTCGAGAA | AAGGCAGGAGACCAAACTTCAT | | |
| 51239 | MGC41816 | NM_016466 | TGGACAGATGATCATCATTCTA | CTGGGCCGAGTGAAGCATTTA | | |
| 51256 | TBC1D7 | NM_016495 | CCGGGTGTGTTTGCACTACTA | ACGCTTTGTGAACCAATTAAA | | |
| 51258 | MRPL51 | NM_016497 | AAGGGCCATGTTCGGAGTGTA | GACCTGCACAACCTTAATAAA | | |
| 51260 | CXorf26 | NM_016500 | AAGGACATCTTTCTAGTCTAA | AACCGGAGAGGCTATAACAAA | | |
| 51265 | CDKL3 | NM_016508 | CTAAAGCAATTTCATCACGAA | AGCTCCCGAATTAGTATTAAA | TGGGCAGATAGTGGCCATTAA | CAACATTATTGTCATGGACTA |
| 51266 | CLEC2 | NM_016509 | AAGTGGACCAACTACCTTAA | ATACATCACCTTAAATATTAA | CTGACAGATACTGAAATTGTA | AGGGCTTTATTGTACAATAAA |
| 51275 | FLJ39616 | NM_016534 | CAGGATTGTATAGAATATATA | AGGATTGTATAGAATATATAA | GAGCATAAGTTAAGTAAGTAA | CTCCACCGATAACCTATCAAA |
| 51276 | ZNF571 | NM_016536 | CAGGGATTTCTGAATAAATGTA | CTGAATTATATCCTTTATTAA | | |
| 51295 | SITPEC | NM_016581 | TACGAGTTTGACATCAATGAA | CCGCAACAAGTGTGTGTATTA | | |
| 51296 | SLC15A3 | NM_016582 | CAGGAAGGGTAGGGCAAGAAA | CAAGGACTTTGGAACATCAA | | |
| 51298 | THEG | NM_016585 | CCGGATCATCTCCCTGGCCAA | AAGGATGAGGAGGACAATAAT | | |
| 51301 | C2GNT3 | NM_016591 | AAGGATGAAGTCAGGTATGAA | CAGCTTAGTTCCAGAGAGAAA | | |
| 51307 | FAM53C | NM_016605 | CAGGATGTTGGGTTTGCTCAA | CAGGTGCTGAGTGAAAGCGAA | | |
| 51312 | MSCP | NM_016612 | CACCAAGGAAACAGCCACCTA | CCGGACGGTGTACCAGCTCAA | | |
| 51317 | BHC80 | NM_016621 | TACGAAGAACCTGATAGTAA | ACGGAAGAACCTGATAGTAAA | ACCGGACAAGTTCCAAATACA | ACCATGCCAAGTGAAATAATA |
| 51321 | LOC51321 | NM_016627 | GACAACTATTATATTCCAGAA | ACCCATGAGATCGGACACATA | CCCGTGTAGAAAGATACAA | CCCGTGGAAGCCTTTAAGGAA |
| 51330 | TNFRSF12A | NM_016639 | CGCCCACTCATCATTCATTCA | CTCAGATGTCCTGAAATTCCA | CAGCTGACACTGACTAAGGAA | GAGGAGAATTTATTAATAAA |
| 51332 | SPTBN5 | NM_016642 | CCGGGTATGTTAGAAGAGCA | CTCAGCTATATATGTAATAAA | | |
| 51334 | LOC51334 | NM_016645 | CCGGGAAAGATTCCTCACCAA | CTCAGCTATATATGTAATAAA | | |
| 51335 | NEUGRIN | NM_016651 | CTGGGCAAGTATTAATGTATA | CCCGGTTGCCCTTGCGATCAA | | |
| 51339 | DACT1 | NM_016263 | CACAGAGACATTCACTATTAA | CCGCAGGAGAACAAAGTTGTA | | |
| 51343 | FZR1 | NM_016281 | TCGCCAGATCGTCATCCAGAA | CAGGATTAACGAGAATGAGAA | TGCAGTGACGCTGTCATTAAA | CGGGTCGATCTTCCACATTCA |
| 51347 | TAOK3 | NM_015855 | CAGAGACTGTACTACGACAAA | ACGCAGCGAGAATAAAGAA | CAGCTCCTCCGTCGTGCATAA | CAAACAGTATAAAGCACTCAA |
| 51352 | WIT-1 | NM_015896 | CCCGGCCTCGAAGGTGCATAA | GCCTCTATTATTATACCGTAA | TAGGCCCATGAAATTGATGAA | CAGTCTGAGGTTACAATTAGA |
| 51364 | ZMYND10 | NM_015926 | CTGCAGGGAGTGCCAAGTCAA | CTGCAGGGTGGAGGACTTCAA | | |
| 51368 | TEX264 | NM_015926 | CAGGACCCAGAATAAAGCCAA | CCGGGCCAGAGGAGAGGCATA | | |
| 51373 | MRPS17 | NM_015969 | TGACATGTTTATGTTTATGGAA | CTCTGTGGTGTTTATGAAATA | TCCCTATTATTAAAGTATT | CAGGCTTGTTCTGGATCCCTA |

Table4

| | | | |
|---|---|---|---|
| 51374 | C2orf28 | NM_016085 | ACCTCTTATATAGACAACCAA AACCAAATCTGTCAAGGGCAA CTGGAATACTATCACCTCTTA AAGCCAAGACTTCATGAACTA |
| 51382 | ATP6V1D | NM_015994 | AAAGAAGATAATAGAGACTAA ATGGATGAAAGAGATCTGTAA |
| 51389 | RWDD1 | NM_001007464 | AAGGGCCACGATGACAGATTA CAGGAGAATATTCTTCTGATA |
| 51390 | AIG1 | NM_016108 | CAGAGAGATGATATACCCGAA CAGATGTTTCTCATTGCATAA |
| 51393 | TRPV2 | NM_016113 | TCAGATAAGAGTCAACCTCAA CTGGCTGAACCTGCTTTACTA CAGAGGATCTTTCCAACCACA CCAGTGAATTCTGGTGGCAAA |
| 51400 | PME-1 | NM_016147 | ACGGCAGCGATTATTAGTAGA AGGAAGGAAGTGAGTCTATAA AGGCGATACATCTGAGTTCAA AACATCGAGCTCTGTTGTAAA |
| 51409 | HEMK1 | NM_016173 | CAGGGTGGCAACCAACTATAT CAGTAAAGTATTGAGAGACTA CAGGGATTGATTAGTCCTCCA ATCCACGTTCATTTAACTGAA |
| 51411 | BIN2 | NM_016293 | CAGGGAAATGAGCAAGCTGAA AACGATTTGAACAAAGCGCTA |
| 51412 | ACTL6B | NM_016188 | CACCTACAGCAAACACGTCAA CACGGCCATTCCAGTACATGA CTGGCATAACTACACATGTAA CAACAGCACCATGGAGCGCAA |
| 51421 | AMOTL2 | NM_016201 | AAGCATATTGCTCCAATTCAA AAGGGTGTTAGTCTTGGAGAA |
| 51422 | PRKAG2 | NM_016203 | AAGCGCGGTTATGGACACCAA CGCGGTTATGGACACCAAGAA CCGGAAAGCATTCCTCTCGAA AAGCACGAGCCTGAACGGTTA |
| 51426 | POLK | NM_016218 | AAGGATAAACCCATTGCTGTA CAAGGATTTACCCATTAGAAA TAGGATGGGACTTAATGATAA CCGGGTGACGACGGGTAGAAA |
| 51429 | SNX9 | NM_016224 | ACAGATCTCAATGATGCAATA CAGCCGCTTTCCAGTGATGTA CCCATCATTGTTGGAGATTAT TACCCAGTGTCAGATAATTAA |
| 51435 | SCARA3 | NM_016240 | CCCTGAGAAGTTCAACATTTA ATAGTATTACTGACTCAGTAA CCAGAGGAAGAGACCATGAAA CACCATCTTCACCAACATCAA |
| 51442 | VGLL1 | NM_016267 | CTGCATATTGTTCCAGATAAA CAGGTGATGGTCCAGAATTAA |
| 51449 | PCYOX1 | NM_016297 | AAGCATCCAAATGTAATTAA CAGGATGGCTTATATGAGAAA |
| 51466 | EVL | NM_016337 | CACACAGTAATAAACGGTTTA CGGCTTAAACTTTGCAAGTAA CAGGTTGTGATCAATTATTCA CCGGATCAACATCTACCACAA |
| 51475 | CABP2 | NM_016366 | CAGAAGCTGGAGGCAGCATAA CAGCATGTTCCTTGAAGCCCA |
| 51490 | C9orf114 | NM_016390 | AACGGTTCTAGGAGAATCGAA TCGCAGGACCCTGCACCAAA CCGGCACACCTGAAAGTTCTA |
| 51491 | HSPC111 | NM_016391 | ACGAATAAATTGAGCCTTGTA CAGGGTGGGTGATGTAAATAT TCGGAGTAAGATCAACGTCTA CGGGACCTCATTGACTATGTA |
| 51496 | HSPC129 | NM_016396 | CTCAGTTACAAATCAATTTAAA AGTACAGTATTGGAAATTAA |
| 51497 | TH1L | NM_016397 | CAGGAGGATGATTCTGGAGAA AAGCGAGTGAGCATCAATAAA |
| 51513 | ETV7 | NM_016135 | CAGATGTGAAGCTCAAATTAA TGCGCCACTATTATAAGCTTA GCGCCACTATTATAAGCTTAA CCCGATATGAGCCCTACATCA |
| 51524 | HSPC196 | NM_016464 | CTAGCTGTGTTCAGCATTCAA TAGAGAATAAATTGAATTCTT |
| 51526 | C20orf111 | NM_016470 | CACAATGAAATCCGAAGCCAA AAGGCTTGTCAAAGAGATTTA |
| 51531 | C9orf156 | NM_016481 | CAGACTTTAATTTACAGAATA AAGGTAGAAGGTGGAGCTATA |
| 51535 | PPHLN1 | NM_016488 | CAGAATGAAGGAAAATCCTGAA AGGGACGATATGAATATGAAA |
| 51548 | SIRT6 | NM_016539 | GTCGAGGATGTCGGTGAATTA CACGGGAACACATGTTTGTGGAA CCGGCTCTGCACCGTGGCTAA CTCCCTGGTCTCCAGCTTAAA |
| 51550 | CINP | NM_032630 | ACGGATATCGTGGACCTTAT CAAGGGAATTGTGAACTAGA GCGGCTGATTGGCACAATTTA CGGCTGATTGGCACAATTTAA |
| 51552 | RAB14 | NM_016322 | AGGAATCGAACTAACAATACA AACGGTTCACGTCTAGCCAAA TCCATTGATCCCGTATCTTAA CACGTTAATAGAGGTAGTACA |
| 51554 | CCRL1 | NM_016557 | AACCCACTATGCTATAAGTTA AAGAACGATTTCCCTGCATAA TTCCATGGTAGTGGCAATTTA CGCCGTGGTTGCAACTCACTTAA |
| 51560 | RAB6B | NM_016577 | ATGGCCAGAGTGGGTCGTCAA ATCCATGTTCTTAGAGCCTCA CGGCTGTTACTTAAACAACTA CAGGGATCACATCACTCTTAA |
| 51561 | IL23A | NM_016584 | CGCCTGAACAGGAAGAATCAA AACAGAGAAAGTTAATGCAACA CCAGATTTGAGGAAGAAGGCAA CAGATTTGAGAAGAAGGCAAA |
| 51574 | HDCMA18P | NM_016648 | TCAGGTGATTTAGTAAATAAA CAGGAGAAAGTTAATGCAACA |
| 51582 | OAZIN | NM_015878 | CCGGATTTGCTTGTTCCAGTA CGGATTTGCTTGTTCCAGTAA CAGGTTAAGCTTGTCTGGTCA ATCATAGTATTTGTTGACCTA |
| 51592 | TRIM33 | NM_015906 | CGCCAAGAATGCAGACAGATA AAGGGTGCAATTGAGAATCTA CTGGCAAAGAATGTAGTCAA TCCCGTAGTATCGGTACACAA |
| 51596 | C6orf82 | NM_015921 | TGAGGTGCTGATGATGATTAA ACGTGTGGCCTTTGAGATCAA |
| 51599 | LISCH7 | NM_015925 | CAGTCGTTTACGCTCATTAA ACGTCGTTTACGCTCATTAAA GAGGATTACCATCACCGGAAA CGGGACGACCTCTATGACCAA |
| 51606 | ATP6V1H | NM_015941 | CAAGAGATGCTTCAAACTGAA ATCACTGATAGTAGCATTTAA |
| 51616 | TAF9L | NM_015975 | TAGGCTGAAGTCCTTAATTAA ATCACTGATAGTAGCATTTAA |
| 51637 | C14orf166 | NM_016039 | CTGGATATTGTATATAAGAAA AACATTCAAGATCGACAAGAA |
| 51639 | SF3B14 | NM_016047 | TCGGATATTGTATATAAGAAA AACATTCGACTTCCACCTGAA |
| 51645 | PPIL1 | NM_016059 | CAGCTTCTAGATGCACATAGAA AAGGAAGTTAATACCTATGTA |

Table4

| SEQ | Gene | Accession | Sequence(s) |
|---|---|---|---|
| 51659 | Pfs2 | NM_016095 | CTGGGACATTCTTCAATTCCA AAGCCTTAGGTAAATAAGTCA CTGGCGATTAACCTGAAACAA CTCCTGTTAAATCATGCTTCA |
| 51660 | BRP44L | NM_016098 | CTGTAACTTAATGGCAATAAA CAGTAATATGTATATCATCTA |
| 51661 | FKBP7 | NM_016105 | CTGGCTAAAGACGGCTCGAAA ACGGAGCATTGAGACATTTAA CGGAGCATTGAGACATTTAAA CACGATGAACTATAGCATATT |
| 51667 | NYREN18 | NM_016118 | AAGCAGTACTCTGACAGACTA ACCGTACTTAGACATAGCTAA TAGAGGCTTATGAGTATCTTA ATCCTCAGATGTGGTGGTTAA |
| 51676 | ASB2 | NM_016150 | TTGGTTGGTATACTCAAAGAA ACGGATTCAGAGAGCATACAA CGGCGCGAACATCGACGCCTA CCCAGGCAGGCTGATTAGATA |
| 51678 | MPP6 | NM_016447 | ATGGACGGCACTTTACATATA TGGGACTGTTACAATACTTCA CTGATGATTCAGTAAGGTTAA TGGAAGGTGTACTAATATATA |
| 51684 | SUFU | NM_016169 | CCCTTGATTGAGAAAGAGAAA CAGGAGCCTTTGTGGACCTAAT GAGCGTACATCTGAAATTCAA CCAGGTACCGTCATCGTCAA |
| 51700 | CYB5R2 | NM_016229 | TCGCTAATAAATCAACAGAAA CACAGAAATGTTTGAGGTCTA TCAGTATTTGGAGAACATGAA CCAGGAATCTTGGAATCGA |
| 51702 | PADI3 | NM_016233 | CGCAAACTAAAGCAAAGCTAA CTGCAGATACTCTAATCACTA TCCGTTTAGAAGCAACGTGTA CTCGCCCCTGTTCCTACACTTA |
| 51704 | GPRC5B | NM_016235 | CACGCTGAATAGAAAACTATAA CAGGGATAGTCTACAAATGTA |
| 51710 | ZNF44 | NM_016264 | ATGATAAACTCTAGACATATA TTCATTATTGTACTAAAACAA CAGAATTTATATTGTTTATTA AAGTTAAAGTTAAGATTCTA |
| 51715 | RAB23 | NM_016277 | CCACAGCAGAATCTAAAGAAA CAAGCTTCTTGCTATAATGTA ATGGAGGATTTCGGTAGATTT ATCGATAGGAATCCAGACCTA |
| 51716 | CES4 | NM_016280 | CAGATTGCTTAGTGGACTTTA ACGACATGCCATGTACTGCAA CACGTCGTGACTTAAATGAAT CAGTATTGACTCGGAGACAAA |
| 51720 | RAP80 | NM_016290 | TCCGAGGTTCTTAATATGAGA TACAGAGGTGGTTTAATCAAA CGCTATATATCCCTCTAGTA AAGTATTGCTAAGTACTATAA |
| 51727 | UMP-CMPK | NM_016308 | CACGACTGTTGTACAAGCTCA CCGACAAGTGATCGCCCACAA CAGGAGGCGAATAAAATGTTCA CGCCCGGAAGATGGAGGTCAA |
| 51738 | GHRL | NM_016362 | CCGCTTGGTATCGAAGCACAA AACGGCGACGTCAGCGACAAA ATCGGGATATTAAGTGTTCTA ACCGGATATCGGGAAGTTCAA |
| 51755 | CRK7 | NM_016507 | ACGGTGTGATAATACTCTTTA CAGGATGAGATCAGGAAGGATA |
| 51759 | C9orf78 | NM_016482 | CAGCTGGTGCATGGACTCAAA CCGGAACGCAGGGAAAGGCAA |
| 51760 | LOC51760 | NM_016524 | CAGGAAGAAATAAGACATGAA ATGCAATATCATGAACTTTAA |
| 51761 | ATP8A2 | NM_016529 | ACGGTCGGAGTCCGGAAGAAA CACGTTCGACTTGGAGCTGAA CTGGAATTAGCGCGCTTAAATA CAGCCAAGTGTCGTCCACATA |
| 51763 | SKIP | NM_016532 | CTCGGACGTCGGCAACGGAGAA CAGCCAATTGGTCGAGACAAA CACCGAGTCGTGTACCATGAA ACGCACAAAGATTGAGTCCAA |
| 51773 | HBXAP | NM_016578 | AAGGAACTTTGTCACATCTAA ACCCTGCATCAAAGAAATTAA |
| 51780 | JMJD1B | NM_016604 | CAGTCTGATCACGATCATAGA CACGATCATAGAAAATTATTGA ATCATAGAAAATTATTGAATAT AGGAGTGTTAATGGTCACTTA |
| 51802 | ACCN5 | NM_017419 | CTCGGCGGTTAGAATTATGAGAA TCAGTGGGACTTCACAGATAA AACCATATGGTTCAAATCCTA CTGGAATCAAATGGTCTGTCA |
| 51805 | COQ3 | NM_017421 | ACCCTTGGATTAGAATTATA ATCCATCAACCTTCTTATTAA |
| 53340 | SPA17 | NM_017425 | CTGGGCCTACAGAAATCTCCTA CAGCATCAACTCCAGCATCGA TGGCTGGAAGCTGATGGGAAA TGGGTGCTGCCAAAGAGATA |
| 53342 | IL17D | NM_138284 | TTCGATTTAAGCATGGCTTAA AAGATTAGTGCCACACTGAAA ATGGATAATCTTATGCTAGAA CACGCTGCAGATCCCATTATA |
| 53343 | NUDT9 | NM_024047 | CCCGCAGACGTCATCCGGGAA CTGGGTGATTGGGATCTCACA ACGGCCAAACTCATCCTGAAA AACAGTGTAATAGTCAATAA |
| 53347 | UBASH3A | NM_001001895 | AACGTTTATACTGGACAGATA CTGGATGGTGGTAATTTAGAA CCCTACTATACTCGTTAGCAA TAGATGATTCATGTAAGTTAA |
| 53353 | LRP1B | NM_018557 | CGCACACAACTGGGAGATGAA CCGAATGAACTACAGCCGCAA TAACACAATAGTATTACTCTA CGAGCTGTATTTCATCATGAA |
| 53373 | TPCN1 | NM_017901 | CCCTACTATACTCGTTAGCAA CGAGCTGTATTTCATCATGAA |
| 53635 | PTOV1 | NM_017432 | CCCAGTCCAGATCGTCAACAA CAGATCGTCAACAACAAGTTT CCGGCGTGTCATTGCCACCA CTGGAGCAGGAGCAACAGCAA |
| 53826 | FXYD6 | NM_022003 | CCAGAAAGCAGAGAACTGAA CAGGTGCAAGTGCAGTTTCAA |
| 53827 | FXYD5 | NM_014164 | CACCAGGACAGACGTTGAA CCCGGTTATGCCGGAATCGTT GAGGACAGACGTTGAAAGATA CTCAACTATCATGGACATTCA |
| 53829 | P2RY13 | NM_023914 | CAGGATGTTAATATGCTATAA AAGGAGTAGTTAAGCTCTGTA CAACTGTTTATTGCTAAAGAA AAGACTGACTGTAGACTGCAA |
| 53905 | DUOX1 | NM_017434 | CAACTAATCTTAGAAGGTAA CCCGGGCAGATCCGTGGGTA TCGGCGGAGGTTTGGCAAGAA CCAGTCTAACACCACAACTAA |
| 53916 | RAB4B | NM_016154 | CTACATAAGAGTTATCTGTTA AAGAGTTATCTGTTTACTGTTA CCGCACTATCCTCAACAAGAT CAGGAGCTATAGTGGAGTGAA |
| 53918 | PELO | NM_015946 | CATATTTGATTTAGAGGGTAA AAGCAGTGGGATAGTGTGGTA CTGAAGAGGATTAATGATTGA ACGTACTACCATCTTGATTAA |
| 53947 | A4GALT | NM_017436 | CAGGATCGCCACTCATGTGGAA CACGGACTTCATTGTTCTCAA CACGTGCTGGTCCTGATGAAA AGGAGAATAATCAATAGAATA |
| 53981 | CPSF2 | NM_017437 | CACATTTGGTTCAGAAATTAT ATGGATCTGCTTAAACCTTA |
| 54039 | PCBP3 | NM_020528 | CCGGCATGCAGGTAATTAT TAGGTGGAACTTGGGAAACTA |
| 54058 | C21orf58 | NM_058180 | CAAAGTCTAATTTAATAACTA TAGGTGGAACTTGGGAAACTA |
| 54067 | C21orf49 | NM_001006116 | TAGGTAATGTGTAGTTCTCAA CACAGAAATGTGGAAA |

Table4

| | | | |
|---|---|---|---|
| 54102 | CLIC6 | NM_053277 | CCGAATCTAATTCCGCAGGAA CACGCCATATTCAGATGTTGCA ACACACAATTTCATTCATATA TGGTATGTGTTTGATGCTATA |
| 54107 | POLE3 | NM_017443 | AAGCTTTAGGAAACTACTAAA AGGGATGAGGACAATGATGAA |
| 54112 | GPR88 | NM_022049 | GGCGTTGAGTGTTATAGTGAA CTCCTAGATGCTAGAACTTAT TACCACGACATTAACATCAA ACCAGTATGAGTTGCCATTAA |
| 54207 | KCNK10 | NM_021161 | CGGGAGTCAGTCCAATAGGAA TTGGCGTGGAATTGGAGACCAA CAGCGTGGAGATCCACGATAA CTGGAATTGTTGGAAGCAGA |
| 54209 | TREM2 | NM_018965 | CAGGGCTGAGAGACACGTGAA AACACTTACAAATAAATCCAA AAGGAAGATGATGGGAGGAAA ATCCAAGACTGTCATATTAA |
| 54212 | SNTG1 | NM_018967 | CTCACAAAGCACAATATTAAA GAGGATTTGGATTAAGCATAA |
| 54329 | GPR85 | NM_018970 | CTGTATGTCACTACATATGAA TTGGAGCATAGTACCATAGAA CAAGGGAACCTTACTGTGTTA AAGCACAAACTAGGAAGACTA |
| 54332 | GDAP1 | NM_018972 | ACGCATTCCTTCAGCTCTCAA ACCCAGTTAATGCCTGATAA TCCCGGCTTATGCAACTACAA CGAGCGTGTCTTGAAGAGAAA |
| 54344 | DPM3 | NM_018973 | CCCATTAAAGAGCCAGTTTAT TCCCATTAAAGAGCCAGTTTA |
| 54361 | WNT4 | NM_030761 | CAGGGAAGGCCGATAATTTAA TTGGGTCCTCATGTTATTTAT CTCATGTTATTATTGCCGAA CAGACCTAAGGTGGAGTAACA |
| 54429 | TAS2R5 | NM_018980 | TCAGCTCAATTCAGGAAGTTA CCAAATTGGCTTAACATTCTA TCGGTTTAATTGGAAATGGAA GCCGTTGGCTTCGCTATCTTA |
| 54431 | DNAJC10 | NM_018981 | CTGGAGAACTGTGGTTGTGAA CAGGGCCTATCCAACTGTTAA CAGCTTAAGTTTGGTACACTA CTCGACGACTTATAATGTTT |
| 54433 | NOLA1 | NM_018983 | CTGCATGATCTGTTTCTACTA ACCAGTTGACTTCTGCATTAA |
| 54439 | RBM27 | XM_291128 | CAGGGAAAGATCGAAGTTTAA AAGCAAGGAAATAACAATCAA |
| 54443 | ANLN | NM_018685 | CTCAATCTAAAGACAAATCTA AAGGTTTCACTGAATGCGAAA CTGATGCTTGCTACAAACCTA CACCGTTTCCATCGTCTCGTA |
| 54457 | TAF7L | NM_024885 | CTCCATAAGATTCAGAATAAA CTGGTTGTTATTCACCAACTA |
| 54458 | DKFZp564J157 | NM_001005354 | GAGCGGCCACATAATTACCAA CGGCCACATAATTACCAATTA |
| 54461 | FBXW5 | NM_018998 | CACCCAGTTCTCCCAGTTCAA CTGGACCACGTCATAGACATA CACGAGGATGTGTCAACTCA CCCGTTCAGGGCCCTGAATAA |
| 54462 | KIAA1128 | NM_018999 | GAGCATAATTATCTCAGGTAA ACGAGTTGGCCTTATCCTTAA ACCGTATATTTATGAAGCATA CCGTATATTTATGAAGCATAA |
| 54475 | FLJ10458 | NM_018096 | ATGACCAAATAAGTAACCTAA CAGCAGTGACAGCACACTGAA AAGGACAAATGCCTCCGATA CCCAGGTTCTATGACCAAATA |
| 54495 | TXNDC10 | NM_019022 | TTCGAGGTTATCCAACAATTA CAGATTGAAGTCAATTATTCA ATGCTGTTAATTAACAAGTA TAGGATATTAAGACTATCAAT |
| 54498 | SMOX | NM_019025 | CCAGTCAATGCTGAAAGTCAA CACGTCATCCAGCTAGGGAAA ACCGACAAGATCTTTCTGGAA CCGGCACGATAAACCAGTCAA |
| 54499 | LOC54499 | NM_019026 | TTGGGTATGATTAGAGTGAAA AAGAATAACAACAGAGATCTA |
| 54503 | ZDHHC13 | NM_001001483 | TGGATACATATTGGACTTCAA CAGCTCATTATTCATATGCTA |
| 54508 | FLJ11235 | XM_496773 | CCGTAGCACAGTAGAAATGAA CAGGATCAGCATAACCGCCAA |
| 54510 | PCDH18 | NM_019035 | CTGAGTATAGTTTGACTGTAA CCCGAAGCAACTGGTAAGCAA CTGCGCCATAGTAGCAGGTAA CCGGAGAATATTTCTCTCACA |
| 54512 | EXOSC4 | NM_019037 | CCCTAGTGAACTGTCAATATA CAGGCAGATGGTGGGACCTAT |
| 54514 | DDX4 | NM_019039 | CTGAATAACAACATTGCTAAA AAGGTTTAAATGAAGAAGTAA |
| 54518 | APBB1IP | NM_019043 | AAGATTTAAATGAGTCCTTAA CAGCATCTCTACAAGCATCAA |
| 54538 | ROBO4 | NM_019055 | CCGGGCCTGTGAGTTAGGAAA CCGGGAGGATTTCCAGATCCA CTCCCTGTAGATTACTCCTGAA |
| 54540 | FLJ10404 | NM_019057 | CCGAGAGGTGGAGTACTTTAA CAGCATGGTCAGCAGTGCAA |
| 54554 | WDR5B | NM_019069 | AAGACTGTCCAGAAACTTTAA AACCGTAACCTCACACATTAA TAGCACAATAATTAACTGAA ATCAGAGTTACTAGTAGGGTA |
| 54556 | ING3 | NM_019071 | GAGGCGATCTTTGGAATTAGA CGGCACTTATGACAACATTAA AACCAAGAGATTGCCCTATAGAA CTAGAGAGTACCTGGGTACATAA |
| 54558 | SPATA6 | NM_019073 | CAGGCTCATCAAAGACATTTA CAGGATAAATTTATTTACCAT |
| 54575 | UGT1A10 | NM_019075 | AAGCACAGGCACAAAGTATAT CTGGAAGATCAGAACCGGGAA |
| 54579 | UGT1A5 | NM_019078 | CACACTCAATCGTTCTTTGAA CACCCTGGAGGTGAATATGTA |
| 54581 | SCAND2 | NM_022050 | CTCCAAATAGTTAAACTGAAA TAGAGTTAACTTTGTAATGTA |
| 54584 | GNB1L | NM_053004 | CCCGTCAATGGACCTTGACTTT CACGATCCGGCCAGATCGCAA CAGGTGCGTGGGACTCATGAA |
| 54585 | LZTFL1 | NM_020347 | AAGACGTATATTCAACATAAA AAGCATTAAGATAAACAAATA |
| 54620 | FBXL19 | NM_019085 | CCGCTGGATCGAGGATGTTAA CACTCTTTCATCGTCATGGAA CCGGGTCTGTACAATACGGTT TACAATACGGTTTGCTATAAA |
| 54658 | UGT1A1 | NM_000463 | AAGGGAAGCTTTGTACCTTTA CTGATGTTTCCTACAACTAAA ATGGTTGCAATTGATCCTTAA TTCCCACTTACTGCACAACAA |
| 54662 | TBC1D13 | NM_018201 | CCGGTGGAACACGTACTTCAA CACAGAAAATCTTGGAAACCAA |
| 54663 | FLJ10439 | NM_018093 | AAGGTTTATCTCAAGTCTCAA CACCGAGACTGGGATCTTGAA |

Table4

| | | | |
|---|---|---|---|
| 54716 | SLC6A20 | NM_020208 | CGAGATGTTCCCGCAAATCAA TGAGATGTTGTTAGCCTATAA CCCGTGGGATTCAATCTTCTTA TCCAATTATATGGAAGAGTTA |
| 54734 | RAB39 | NM_017516 | TCAGTTCAGGATAAATACCAA AAGGTTACAAACCCACACCAA TAGCTTCACAACGTCAAGTTA CAGGAGCGGTTCAGATCAATA |
| 54737 | HSMPP8 | NM_017520 | AAGGGTGAAATAAGAGATTTA CTGGTGAACTTCTGTGCTTAA |
| 54741 | LEPROT | NM_017526 | ATGGCGTTTACTGGCCCTTAT CTGGCCCTTATTCGTCCTGAT CGGCCTTGTGTTGGCAGGCAA AAGCATCATCATAGAGAAGTA |
| 54752 | DKFZp434H2215 | NM_017559 | ACGGGTGTCATCCCGAACAAA GAGGATGATACCATCAACCTA |
| 54754 | DKFZp434I1117 | XM_030729 | CCGGCCGGACGACTCCTGTAA CCGCAGGAAGAGGACGGGATA |
| 54763 | ROPN1 | NM_017578 | CTGGGATGGTGTGGTGAGCAA CACACTAAGGATAGCAATTTA AGGATAGCAATTTATCTCAAA TACACACTAATAAACAATTAA |
| 54790 | FLJ20032 | NM_017628 | AAGGCCTTTCATATAAATTTA CAGCTGTAGACTAACAATATA |
| 54795 | TRPM4 | NM_017636 | CTGCACGACGTTCATAGTTGA CAAGATCGTCATCGTGAGCAA CGGGTTTACCTTTCTAAGGAA AACGTGGGAATCGGTGCATAA |
| 54801 | FAM29A | NM_017645 | CCGGAAGAAGTTGGTCATCTA AAGGAACACTGTCAAACTATA |
| 54820 | NDE1 | NM_017668 | ACGCAGCTGCAACAAATTGAA CGCGCAGACCAAAGCCATTAA |
| 54828 | BCAS3 | NM_017679 | CAGATTTAAATGATACATCAA CTGGAAGAGATTGAACAAGAA |
| 54831 | VMD2L1 | NM_017682 | CCGCGACAACAGCGCCCTTAA TCGAGTTGTCATGAGGATGAA |
| 54836 | BSPRY | NM_017688 | CAGCTGATTCAGAAGGAGCAA GTCGGAAATGTTAAACTTTAA CTGGATGGTGAATGTCCAGAA CCAGAACAGTTGTGCCTATAA |
| 54841 | BIVM | NM_017693 | CTGCTGGAAATAATTCATCAA GAGGTGGTTTAGACAAATTAA |
| 54843 | SYTL2 | NM_032379 | AAGGGATTTAAATATAACAAA CCGGTGGAATCTGATCTTGAA |
| 54847 | SIDT1 | NM_017699 | CCGGACCAAGATGTTCCTTTA CACATTCCTCTTAACAAGTAA |
| 54850 | FBXL12 | NM_017703 | CTGGTGGATGTAACTACTCCA CACCTTGAAGAGGGCAGATAA TCGCGATTCAATGATCGATAT AATGATCGATATGCTCATAAA |
| 54855 | FAM46C | NM_017709 | TTGGAACATATTCCAATTCAA CTGGTCATTCATGACCTTAAA |
| 54866 | PPP1R14D | NM_017726 | GAGCCTGAGATTGACCTGGAA CAGGCCTGCCCTGAAACTCCA CCGCCTGACAGTGAAGTATGA CAGGAGCTCTTCCAGGATCAA |
| 54873 | PALMD | NM_017734 | CTGGGTATTGGTGTAAATGAA TAGGTTAAGGAAGGAGATAAA |
| 54901 | CDKAL1 | NM_017774 | TAGCACATCCTTCAAATTAAA CAGCGTACTCATGGAAATGAA |
| 54913 | RPP25 | NM_017793 | TGGAGTCTTGTTTCCAATAAA GACAGGTAGCATGGACCTTAA |
| 54918 | CKLFSF6 | NM_017801 | AAGAATTATAAGAGAAATGAA ATGGGAATTTAACATATTTAA |
| 54919 | FLJ20397 | NM_017802 | CTGCAGCTGATTTGAAATTAA GCCAAAGTGATTAGAAAGAAA |
| 54935 | DUSP23 | NM_017823 | CAGGAGATGCCATTGCTGAAA TTGGCTGAAGACACTGAAGTA GAAGTGGACTAAAGTATTAAA CAGTTCTACCAGCGAACGAAA |
| 54937 | FLJ20449 | NM_017826 | CAGAAACTATTTGCAAACATA CTGAATTGTTCTTATATGTAA |
| 54938 | SARS2 | NM_017827 | CACCTGGAAATTGGCGAGAAA CTGGATATGCCCACCCAAGAA |
| 54939 | COMMD4 | NM_017828 | ACCCTGGATGTTACATATAAA AAGGAACCTATCGAAGAATAA |
| 54941 | RNF125 | NM_017831 | CAGAAGTACATAGATAAGTAT CTGGATCATTGTATTACTCAT CTCGGTTTGCAAATTAGATAA ATGGTTAGGCACGATAACTAA |
| 54946 | SLC41A3 | NM_001008485 | ACCCATCGTGAAGATCCTGAA AAACCTCTATTTAGATTTCAA |
| 54955 | FLJ20508 | NM_017850 | CAGGATATTGAGAGACATTAT CCAGATAACTATTATTAATAA |
| 54957 | TXNL4B | NM_017853 | AAGGAAGTAGACCAGGCGATA CCACCACTCTATTCAGGTAAA CTCGTGGTGGTTATTGAAACA TCGATATGCTAAGAAGAACAA |
| 54958 | FLJ20512 | NM_017854 | ATGGGTCGGGAAGCAGCATAT CCCGAGGACTGGGACATTAAA |
| 54962 | FLJ20516 | NM_017858 | ATGCTATTGCCAATACTTTAA CAACAGCAACTTGATGCTACA CTGAGTTAAGTAGAAGCCTAA AAGCTTGGCGTTACTATGTAT |
| 54973 | FLJ20542 | NM_017871 | CCGCAAGATCGCCGTAGACAA GAGGAACATGTTTGAGTTCAA |
| 54988 | FLJ20581 | NM_017888 | TAAGATGATCACACAGCATTA AAGAATATCATTGGCCCTGAT |
| 54989 | FLJ20582 | NM_014106 | CAGGTAATAATGTTAACTATA CAGGAGAATCACATGCTTTAA |
| 54998 | AKIP | NM_017900 | AAGCAGATCAAGTTCGAGAAA CTCAGAGGACTCAGCCTTTAA CTGCTGTGATCCGTAGTAATA ATCCGTAGTAATAAATTCTCA |
| 55002 | C13orf11 | NM_017905 | CACGTGCAGATCACCACCTTTA CGGGCTCTTCATGGCCGTCAT GAGGAGCAGCCAGTACATCAA TAGTCAAATATTTGTTATTAA |
| 55006 | FLJ20628 | NM_017910 | CGGCGCCTTCGCGAAACACTA CTGGCTAAGAAGAATTACAAA GGCGTGATTCATGGAAATTAA CAGGAGCAACCGAAGACATAA |
| 55010 | FLJ20641 | NM_017915 | TCCCAATTTGCTTGTACTTAT ACAGAATAGATTGTACGGCAA CCCAATTTGCTTGTACTTATA CAGAATAGATTGTACGGCAAA |
| 55011 | FLJ20643 | NM_017916 | CAGGGAGGGCCTTGAGGACAA TCCGGAATGGCGCATGATGAA CCCGCTGCAGATCAACTCTCA ACCGGAAGAGAAAGCAATTAA |

EP 2 295 543 A1

Table4

| | Gene | Accession | Sequence |
|---|---|---|---|
| 55014 | STX17 | NM_017919 | TTGAGGTTTGTTAGTGAGGAA TTGCAATTGGTCCAACTATA CTCCTATAACCTCTCAGGTAAA ACCCTGAATTGGAGACCTTAA |
| 55016 | MARCH-I | NM_017923 | TACCTTAACCAAGATGCCAAA ACCAAGATGCCAAATTATCTA AAGAACTTCTCATGTAATGTA TAGGCAGTAATCAAAGATTAA |
| 55049 | FLJ20850 | NM_017967 | CCGCTCTTGTGAGGTAATTAA CAGCTTCGCGGCAGCAGCTAA |
| 55066 | PDPR | NM_017990 | TTGGAGCAAGTATGTACTTAA TAGCTACAAGATTCAGCTTTA ATCTCTTATCTCCTTGATATA ACCACATAGCCCAGTGATTAA |
| 55072 | RNF31 | NM_017999 | CTGGCGTGGTGTCAAGTTTAA CCGAGATGTGCTGCGATTATA ACGCATGAACGACCCAGAATA CAGGACAATAACGTCATGTTT |
| 55082 | FLJ10154 | NM_018011 | CAGATTGTAGATGACAATATA AAGGATGAAATTGAACGAGAA |
| 55083 | FLJ10157 | XM_371354 | CAGCAAACACATTCCATACAA CAAGCTCATAAACGAACGCAA ATCAGTATTGCTTATGAATAA AAGCACTTTGAGGAACCTTAA |
| 55089 | SLC38A4 | NM_018018 | CAGGACTGGATAAATAATTAA ACCCAAAATACTTTGTATTCAA |
| 55102 | C14orf103 | NM_018036 | CTCCAACATGTTGACAGTGAA CTGGGTGGCATGAGAAACCAA |
| 55104 | FLJ10246 | NM_018038 | CAGGTTCATCTTTGACTTCAA CAGGTTGTATCTCCAGATAAT |
| 55111 | PLEKHJ1 | NM_018049 | CAGGGTGCTGTTGTTCTGTTCAA CCCGTGCTGAATGAATGTACA |
| 55117 | SLC6A15 | NM_182767 | TGGAATTACATAAGCCCTAAA CACGCCTATTGTGGACACTTT CCAGCAGATATTACTACTATA CCCAATGACGGTATGGAATA |
| 55118 | CRTAC1 | NM_018058 | CACCAGTCACTTAACTTGTTA CCGGGACATCGCCTCACCCAA |
| 55122 | C6orf166 | NM_018064 | CTGCAGCATCCTCACCATTAA TTCACTGGTTGTGTAAATAAA |
| 55124 | PIWIL2 | NM_018068 | AAGGATGTACATAAGATTGAA AAGAAGCAAGTTTCTGAGTAA |
| 55128 | TRIM68 | NM_018073 | TTCATGTATGTAGGAGTTAAA ACGCATTCGTCCCACCATGAA TAGGCCAAACTGGAGAAATAA AAGAGAGATCCTGAAGACTTA |
| 55129 | FLJ10375 | NM_018075 | CAGACCCTTTATCAACATAAA TAGACCATTGTTAAATAAATA |
| 55139 | FLJ10415 | NM_018089 | CCGAAGGTTCATGGAGAAGAA CAGGAGGAGCGTGAACGAGAA |
| 55148 | C14orf130 | NM_018108 | AAGCCTCAACGCAGAATCAAA CAAGGTTGCACTATTAGTATA CACAATAGTACCGATCAGTTA ATCAGTTAACTCAGCGCTGAA |
| 55154 | FLJ10504 | NM_018116 | AAGGAGCTAGTTTGCAATTAAA CTGATCCTTAGTAACATGTTA |
| 55166 | C6orf139 | NM_018132 | TTGGATATTCTTCATAATTCA ATGGAAGATTAACTAATGTA |
| 55167 | FLJ10546 | NM_018133 | CTGGCACGGGATATAATAGAA CAAGGCGTTTACTGAGATTAA CAGTGTCTTATGAAACATGTA AGGGCCTTGATTGTTATGTAA |
| 55170 | HRMT1L6 | NM_018137 | CCGCCTGGGTATCCTTCGGAA CCGCAGCGCTTTGCTCAGCTA CACACCTTATCTAAGTCTGAA ATAGCCTAACATTTATTGTAA |
| 55174 | FLJ10569 | NM_018142 | CTGGTTCTTCTTGAAGATGTA CCAGTGTGAAATGTTACTAAA |
| 55180 | WINS1 | NM_018148 | AACCCGGATATTGTCTGTCAA AAGAAACTTCTTAGAGATTCA |
| 55182 | FLJ10597 | NM_018150 | CAGGCAGCTCGCGAACACAAA ACGGCAAGTCTTAGGAGGGAA CCCAATCAGTATGCTGAATCA CTCGGTGTGTTCAGTGGTGTATA |
| 55183 | RIF1 | NM_018151 | CTCATGGAAATTACAATTAAA AAGGAAATGCATGTAAAGTAA |
| 55192 | FLJ10634 | NM_018163 | ACCCAGAAAACTTGATGAGAA AAGGAAGAAAGTGAAGCTTGA |
| 55193 | PB1 | NM_018165 | CAAGGATGCAAATTCAATTAA TCCCAGGTTTATAATGATGCA CCGGAGTCTTTGATCTACAAA CAGGCTTACACATCGCACATACA |
| 55194 | FLJ10647 | NM_018166 | CTCCGTTTACTTGATCTGCGA GAGGTGAAGTGACCAAATGAA |
| 55200 | FLJ10665 | NM_018173 | AAGGCAGAGGAGTATGTTCAA CCCACAGAAAGTTGTGCATAA |
| 55206 | SBNO1 | NM_018183 | CAGGTTCAACTTTGATAATAA CTGGATGAACTTATCGATGAA |
| 55218 | C14orf114 | NM_018199 | CAGAATCTCAGCAGAAGCCAA GTGGGTAAATTTGGAAGGCAA |
| 55219 | FLJ10747 | NM_018202 | ATCCTCTTTGTTTATATTGAA CAGGCTGGAACAAGACATTAA |
| 55228 | FLJ10781 | NM_018215 | CAGGTGTATGGTAGTAAGTAA CACCATGACTAGTAAATGTAA |
| 55234 | SMU1 | NM_018225 | CAGAATGTTCAAATACCTTTA CAGGTTGTTCTGGAATTGATA |
| 55239 | FLJ10826 | NM_018233 | CCGGATTGCCTTCATCCTGTA TTGCCTGACAGTGTTCTTAAA |
| 55243 | KIRREL | NM_018240 | CTGGAGGAAAGAGAGGATAAA CCGGGTCATGAAGGCCATCTA |
| 55247 | FLJ10858 | NM_018248 | TCGGATATACAGCATATTCCA AAGGATTATGCGACACAATAA CCTGGATATTCTTAACAGTGAA ACGGATTCATTCGGAATGAA |
| 55256 | MTCBP-1 | NM_018269 | AAGCCTTAAATGTTTAAGTAA AACAGGAGTATTAGTACAATAA AACAAGTAATCCCTAGTACTA TAGCGTAACATGATACATCTA |
| 55257 | C20orf20 | NM_018270 | CTCGGATTAGCAAGCAATAAA TCGTGTGACTGCGAACATTAA |
| 55265 | KIF24 | AK001795 | AAGGTTATCAAATTCACTTAA AAGGGCATGTAGTTTAATTAA |
| 55274 | PHF10 | NM_018288 | ACGAATTACAGACCATTATAA CGGACAGTTCCAGGAATATTA TCCAGGGAAGACAGAAATCAA ATGGCAGTGTATGGAATGTAA |

Table4

| ID | Gene | Accession | Sequence |
|---|---|---|---|
| 55275 | FLJ10979 | NM_018289 | AAGGAGATTAGATCAACCAA ACCCGTGATATTAAGCAATTA |
| 55283 | MCOLN3 | NM_018298 | TCAGTTAATCTTCGCAGTAAA CAGACAGTTCGTCATCAAGAA ATGACTTTACTCTGACTATAA CTGATACATACGAAACAATTA |
| 55284 | FLJ11011 | NM_001001481 | AAGGTTAACAGCATGAATTAA CTGGTTATAGTGGAATATAA |
| 55295 | FLJ11078 | NM_018316 | AAGAATTAACCTCCTATCTTA CACGGGCATCGTACAGGTGTA |
| 55304 | C20orf38 | NM_018327 | AAGCATCATATTTGAAGGAAA CAGAGCTTCTTTAAAGCATAA |
| 55308 | DDX19L | NM_018332 | ATCAAGGAAGAGAAAGTCAAA GGGAATTATGCTGTTCATTTA |
| 55314 | FLJ11155 | NM_018342 | TAGTTATCAGTAACAATTTA ATCCTTTGTTTCATAAAGTAA |
| 55317 | C20orf29 | NM_018347 | CACCGGAGTAAGAAAACTACAA CCCACAGTCATCGAAACACAA |
| 55324 | ABCF3 | NM_018358 | CAGGGAAATAGCCAGGTGCTA CCCAAGTGGCTGCTATGTAAA TCGGTTGGAATCATCTGGCAA CGAGGGCTCGACTTAAGGCAAA |
| 55342 | STRBP | NM_018387 | AAGGATGTATAGAGTCTCAAA CTGGAAATTCTACAACTGAAA |
| 55359 | STYK1 | NM_018423 | GCCCAAGAGTGTTATTCTCAA CCGATTAGACTAAGGAGACTA TTCACCTTAGGTATAGATAAA CTGGGCTGCAAGATATCAATA |
| 55361 | PI4KII | NM_018425 | TCCAAAGATATCGGACCCTAA TCCGCACACAATCATAGAGAA CCGCATCGGGCTACCACCAAA ACGCAAATGTATGAATAACAA |
| 55364 | IMPACT | NM_018439 | CAGGATGGGATTCTGAAATATA AAGAAGAGGAATGAACATTAA |
| 55366 | LGR4 | NM_018490 | CACACTTGGGCAATAACTAA TCCAGCTAGATTGCAGTTTAA TAGGTGTAGTAGAGCAATATA TAGGATTTCATAGTAATTCTA |
| 55437 | ALS2CR2 | NM_018571 | CAGGATTTACATGGGTATAAT ATGCTGTTACAGAAACTGAAA CCAGTGGAACTCACACAGTAA CAGGAACACTGGTAACTATAA |
| 55503 | TRPV6 | NM_018646 | CAGCAGAAGCTACTTCAGGAA CAGGCCTGCATTACTCCTTCA GAGGAGTAGCATGAACGCCAA CTGCATGTCAGAGCACTTTAA |
| 55511 | SAGE1 | NM_018666 | AAGGGAGCTGTTTAAATTTAA CTGGTATTTCATGCAGAAGTA |
| 55520 | ELAC1 | NM_018696 | ATGAGTGGAATTACTAGGTAA ATGAATTGGCTCAGTATTTAA |
| 55530 | DKFZp761H039 | NM_018711 | CCCATGAAAGCCAGAGCTAAA CCCGGCCAAACCCTCAGTGAA |
| 55534 | MAML3 | NM_018717 | CTGACTTATGGCAACACTAAA CAGCAGTTTGGAGACACAAA |
| 55561 | CDC42BPG H17 | XM_290516 | CACAGTCATTATATTGTTATT CCGGCCCGTGTACACACTCAA CTCGCCGCGCCTACCAACTCAA CGCCGGTGGCTTTGCACTCTA |
| 55572 | FAM48A | NM_017547 | CCCGCTAGTTGTCAACATGTA CAGCTTTCTACGGAACATCAA |
| 55578 | ITLN1 | NM_017569 | CTGAAGTTAAGCAGAAATTAA AAAGATGATTATGTATTTGAA |
| 55600 | FLJ20035 | NM_017625 | CTCAGACAAGATTACAATGAA CCCGGTGATCCCTGGTCTA CTGCGGGATTTGTTCAGTTCA ACCCAGTAGCTAGAATGTTAA |
| 55601 | OTUB1 | NM_017631 | CCCAGTGTCAGTATTTATAAA AAGGGTATTTGCTACCTTTAA |
| 55611 | FLJ20244 | NM_017670 | CAGCAGGACCGAATTCAGCAA CAGGGCTTCACTGAATTCACA |
| 55621 | FLJ20272 | NM_017722 | AAGGATCGTCTCTGTGGCTAA CCAGGAGAAGGACACGCAA ACGCCGGAATGTCCAGCTCAA CAGCATCAGCGCTGACTTCTA |
| 55622 | FLJ20294 | NM_017735 | AAGCCATATTCTTAAATTCAA CCGCAGTGTATTTACGGTAA |
| 55626 | FLJ20297 | NM_017749 | TGCGTAGAATTTAGAGTTATA CTCCTAATGAATGCTAAATAA |
| 55627 | PNRC2 | NM_017751 | TAGGCTGTCGTCTGCCATCAA CAGTGGTACTTAAGTATTCAA |
| 55629 | KIAA1333 | NM_017761 | CTGAAGTTTAGTATATTAGA AAGATGTAAACTCTTGCATAA ACAAGTTTATGTAGTAGTACTTAA CCCAATGGCCCTGTTTATTAA |
| 55632 | CHD7 | NM_017769 | AACGACATTAAGTGATAAATA CTCATTACATTGGACATTAAA AAGGAATATTATAGCCACTTA TAGATTAATCTGTATGGTCAA |
| 55636 | C14orf58 | NM_017780 | CAGGGATTAGTTAACAATACA TACCATTGGTATTATAATAAA |
| 55640 | BTBD2 | NM_017791 | ATCAGATATTCTAAACTTAAA AAAGTATTAACAAAATGTTTA |
| 55643 | OSGEP | NM_017797 | CCGCATCAGGTTCTCAGTCAA CACGCTATACACCGCCAAGAA |
| 55644 | FLJ20489 | NM_017807 | CTGGACCACGGGAAAGAGATA CTGGAGGGACTAATAAGATCA TAGCAAAGCTATAGAGACTCCAA CCAGGATGACTCAGCAATAAA |
| 55652 | HIF1AN | NM_017842 | CTGGAGCTTCATTTCCTTCAA GTGAGGAATCTTTGTACTTAA |
| 55662 | FLJ20626 | NM_017902 | CACTGTGAACTTCTTGTATAA CACCCACAAGTTCTTGTACTA CAAGCTCAAGCCATACATAAA AACACATAGGCTTGCGTCTTA |
| 55663 | C9orf55 | NM_017908 | CCCAATGTCGATGGACAGGAA AAATTCCACTTTCATAATAAA |
| 55667 | MFN1 | NM_017925 | CAGCACAATAATGTTCTTAAA CAGCTTGAGATGGAAATTCAA |
| 55669 | RUFY2 | NM_017927 | ACCCAGCAAGAAATTATTGAA AAGGCTTGTTATAGTCACTTA |
| 55680 | FLJ10081 | NM_017987 | AAGAATTATGTTGAAGAATTA CAGGATGTAGAGAATGAGCTA |
| 55683 |  | NM_017991 | CCGGATGCTTGTGTCATCCAA CGGACCTGCCTTTATGATAAA |

Table4

| | | | Sequence |
|---|---|---|---|
| 55687 | TRMT1 | NM_001008568 | CTGACAGAGATGGATCTGCTA AACCAAAGAGTTTGTAAAGAA |
| 55690 | PACS1 | NM_018026 | CCCGGAGGAAACTAACCTCAA CCCGGTGCAGATGAACCTGTA |
| 55699 | FLJ10326 | NM_018060 | CAGTCGTTCAATATATGTAAA ACCGAATTATACAAACAATAT ACCACTGAGCATATTGTTAAA AAGAGTCTTCCTATAAAGTAA |
| 55702 | FLJ10374 | NM_018074 | CTGGAGCAGCTTCCAACACTA TGCGAGGTGATCAGAAGGCAA |
| 55711 | MLSTD1 | NM_018099 | CAGGTTGGGTTGATAATATGA CAGGATGACAAAGCTCATGAA |
| 55718 | POLR3E | NM_018119 | CAGGAGGTGGATAAAGTACTA CCCGCGAGAGATGGAGCTGAA |
| 55732 | FLJ10706 | NM_018186 | CAGGATTTCTAAAGCACACCA CAGGTAGCAGATCAACCTTAT CAGCCTGGCTTTCAATGAAAA CTGGGTTTGATGCTTTGTCAA |
| 55734 | ZFP64 | NM_018197 | AAGGACATGGAGCGGCATTTA CAGCAGTTTAACAACCTGGAT |
| 55743 | CHFR | NM_018223 | AACAGTGATTAACAAGCTGAA TCACCACGCCATGAAATTCAA TCCCGTGGAGCGGATCGTGAA AACCAGAGGTTTGACATGGAA |
| 55754 | TMEM30A | NM_018247 | ATCGATGGCGATGAACTATAA ACCGGATAACACGGCCTTCAA CTCGAGATGATAGTCAACTAA AACGATTTAAAGGTACAACAA |
| 55760 | DHX32 | NM_018180 | ATGGATCAGGTAACTACTTAA ACGCCTTATCTTTGAAATTCA |
| 55769 | ZNF83 | NM_018300 | ACGCAATTTAAATGTGATATA AAGGAGAGTAATACAGGAGAA |
| 55779 | FLJ11142 | NM_018338 | CTGAGATTAATAGAAATTGAA AACTATCACTTTCAACATTAA |
| 55781 | RIOK2 | NM_018343 | CTCATAAAATGGTTATCCACTA GAGTATGAATCAGTATAGAA CGCGGTTGAAATGGGCATGAA AACGCGATTATCATAAACATA |
| 55793 | FLJ11280 | NM_018379 | TCCAGTTGTTTCAGAGCTTAA TAGCACCATGACTAAGCATAA |
| 55794 | DDX28 | NM_018380 | CAGGCAATGAGGAACAACTA CACGTACTTGATCACTTTGTA |
| 55795 | FLJ11305 | NM_018386 | AAGGATGATTCTGATCTATTT AAAGCGTATTCTGAATTATA |
| 55801 | IL26 | NM_018402 | AACTTAATTTAAAGTATGTAA AAGCAGCATGGCTCAAAGCAA CAAGTACATTGTGTCAACTTA CAGTGTGAACTTAGTACTTAA |
| 55802 | DCP1A | NM_018403 | CAGATACATTAATACATCTA CTGGAGCAGAATAAGTCTAAA |
| 55805 | DKFZp761O0113 | NM_018409 | CTCAAAGATCTTAATGAGTAA CCCGTCAAACACTATTATTCTA |
| 55813 | HCA66 | NM_018428 | CACCATTGGATATTCATTTA CTGGCCCATTTATGAAGATGAA |
| 55814 | BDP1 | NM_018429 | CAGAGCAAGGATAGAAATTAA AAGGTTCTTAATGAATGTCTA |
| 55815 | TSNAXIP1 | NM_018430 | CCGCACGCAGATGGAACTCAA CAGGAGGGACTTTGAAATGCA |
| 55819 | RNF130 | NM_018434 | CAGAATGATGTCGTCCGAATT CTGTCCTATGTGCAAACTTAA CAAGCTGTTAACCGAAGATCA TCCGAGAACAGGAGAAATCAA |
| 55829 | SELS | NM_018445 | AAGGAGATTTATGGACTTCAA CTGGCGGATGAGGCTAAGAAT AACCCTTAACCCTCGATTCAA AGGCCTCTAGATGATTAGCAA |
| 55830 | GLT8D1 | NM_018446 | TACCGAGATCTCAAACATAAA CAGCAGACTATAGACTATAA |
| 55836 | C6orf35 | NM_018452 | TAGGCTCTAATTAGTCAGCAA CTGCCTTTCATTTGTAACTAA |
| 55837 | C14orf11 | NM_018453 | CCGGAGAAAGTGAATCATCTA ATGAATTGTTCTATTAACAAA |
| 55844 | PPP2R2D | BC047379 | CCGCTCCATTAAGAACAGTGA TTCATCCATATCCGATGTAAA TAGGTGGTTACCACAACAGAA CAGAGACTACCTGTCGGTGAA |
| 55848 | C9orf46 | NM_018465 | CTGCTTGGCTTCCACCTTTAA AAAGGAGTTCATGCTTATGAA |
| 55850 | MDS032 | NM_018467 | TCGAATCATGCCTAAACTCAA ACCGGCCTCTGAGGTGATCAA |
| 55851 | PEN2 | NM_172341 | CTCCCAGGACAGGCTCCTTAA ACAGAACAGAGCCAAATCAAA CTCGCCCAAAGAAGACTACAA CACAGAACAGAGCCAAATCAA |
| 55870 | ASH1L | NM_018489 | CAGGCTGTGTATCAATGCAA CGAGATGATGGGAATATCAA TCGACTGTTATCTCACATTAA ACGGTAGTTCTTGCAATTATT |
| 55872 | PBK | NM_018492 | AACGCTGTAAACTGTAACATT TCAGTAGTTATTAGACTCTAA CTCATTCTTCCTTGGGCTGTAA AGTGTGGCTTGCGTAAATAA |
| 55884 | WSB2 | NM_018639 | CACGTTAATTCGGAAGCTAGA CGGAAGCTAGAGGGCCATCAA CACGGGCTTCTTACGATACCAA AAAGATGTCTTAATTGTGTAA |
| 55889 | GOLGA6 | NM_018652 | CAGGAGAGACTGTGTGAACAA CGGACGGGACGGGAGCAGTGGA |
| 55894 | DEFB103A | NM_018661 | TACCATTGGGTTCCTAATTAA CAGTTCATGGGAGGAATCATAA |
| 55897 | MESP1 | NM_018670 | ACCCATAGGGCTAGACACTTT CACGAGCTGCGCCGCTTTCTA CTGCCTAATGTGAATTTATTT GTGAATTTATTTATTTGTGAA |
| 55908 | LOC55908 | NM_018687 | CCGAGAATTTGAGGTCTTAAA ACGGCTGACAAAGGCCAGAA |
| 55914 | ERBB2IP | NM_001006600 | CTGCAGTAACTCGAAGTACAA CAAGTTTGATTCAAATCATAA CAAGATGTTATCAATTGGTTA CCCGAAGAGCCAAATATAATA |
| 55915 | LANCL2 | NM_018697 | AAGGAAAGAATTTGAAGTCCA CAGATGGGTTATTGCAGATTA CTCGCAGTTCCAGAACATTTA ACCCAGGTATATGTTACTAAA |
| 55922 | NKRF | NM_017544 | TCGCTTATTTCTGGAATGTAA CAAGATGTCAATTGAATACAA |
| 55929 | DMAP1 | NM_019100 | GCGGATGTACGGGACATTCTA CAGCTTTATCTCCACGATGAT |

Table4

| | | | Sequence |
|---|---|---|---|
| 55959 | NM_018837 | SULF2 | CTCCATGGAGACGATTACAA TCGGTGCTACATCCTAGAGAA TCGAAAGTGGCCAGAAATGAA ATGACAGATTCTGGAGGATAA |
| 55968 | NM_016143 | NSFL1C | CAGTAAATGCTTGACAAGTAA CACCAGCATGTTGTACGGAAA |
| 55969 | NM_018840 | C20orf24 | TGGATTCAGATGAAACATTAA CTGGATTCAGATGAAACATTA |
| 55997 | NM_032545 | CFC1 | CAGGAAGCTAGTGAAATGACA AAAGTATACTCTACCATTAAA |
| 55998 | NM_032946 | NXF5 | CTGAAATTGATTCAGAATGAA CCAGTTGAGTTTCCACTATGAA |
| 56000 | NM_022052 | NXF3 | CAACGGATTACCGAGTTAAA ACGGAATAGAAATAGAAGTAA |
| 56107 | NM_018921 | PCDHGA9 | AACATCCTGATGGAAGATAAA AAGGATTAGATTATGAAGAAA |
| 56108 | NM_018920 | PCDHGA7 | TCAGATTATAATATCACTTTA CAGATTATAATATCACTTTAA |
| 56110 | NM_018918 | PCDHGA5 | AAGCTACACAATAAATCCGAA CTCCAGGTCAAATAAGTTCAA |
| 56124 | NM_018932 | PCDHB12 | CCGGAACTAGTACTGGATAAA ATGGACACAAATATAGACTAA |
| 56132 | NM_018937 | PCDHB3 | CACAATTGAGATACAGGTTAA AAAGTCCAAATTTATAATTAA |
| 56143 | NM_018908 | PCDHA5 | CAGGAGAGACTCCGAACTCCA CGGAGCTATTGTAGCAGGCAA |
| 56156 | NM_031273 | TEX13B | CAGGATAAATCGGCCAGCAAA TTGGAGAGTTATGCTAAGCAA TCGGAAGGCAATATATTAGAA CGGGCTAATATCATTCGATCA |
| 56171 | NM_018897 | DNAH7 | CACGCTCTGTTTCGTGATGTT CACGGAGTTCGAAACCATCTA ATCAATGAACATTGTACCTTA TTGAATGTAAGGCTCAGTAAA |
| 56172 | NM_054027 | ANKH | CAGCACCATAACCCAAATGAA CACATTGTTGTGGAAGAATAA |
| 56204 | NM_019600 | FLJ10980 | CGGCACCAACTTCACATTCAA CAGCCTGACCTGGCATGTCAA |
| 56241 | NM_019601 | SUSD2 | CTCCAATAACAGCCAGGTTTA CAAGGTAGTCTTGTGAAGAAA CTGGGTCTTGAACTACTTTAA CTCCAGTACCTGAAAGTGAAA |
| 56255 | NM_021156 | DJ971N18.2 | AGGGAGTTATTTGTAAGCAA ATCCAAGATCACACATCTTTAA |
| 56256 | NM_019605 | SERTAD4 | CAGGCATATCATCAAGGAGTA TCGATTTGCTTCAGGAATTAA |
| 56259 | NM_030877 | CTNNBL1 | CCGGATTCGAGTCATCAAGAA CTGGAGGACGGCGATCTATAT CAACCAGAGCATCGATCTTAA GAGCATCGATCTTAACCATAA |
| 56265 | NM_019609 | CPXM | AAGACTGTTATTGAAAATAGAA GCCGGTTTCTTTAGTTCTTTA |
| 56270 | NM_019613 | WDR45L | CTAATGGAAATTAGAATTCTA AGGGCAGATGATGGAAATCAA |
| 56271 | XM_043653 | BEXL1 | TGGCTGGAACTTCCTCAACTA CCCACTGGTGACCTTCGTGTA CACGTTCACCAACATTGCCTA CAGCTTCATCTCAGAGCCTAT |
| 56301 | NM_019849 | SLC7A10 | CCGGTTAAGTGAACTATGATA AAGGATGGTATACTCCACTA |
| 56311 | NM_019644 | ANKRD7 | CAGGCTCTTATGGGCATGAAA CAACATCATCACCATATTTAA CGCGTGTGCCTAGAATATATA CAGATGTTAAGCAGCTATGAA |
| 56341 | NM_019854 | HRMT1L4 | CGGGTTGCTGTTGTCGATATA CAGAGTATTAAGAGCCGACAA AAGGGCAAATCACATCAGATA TCCAAGGTGCATCGACATTAA |
| 56479 | NM_019842 | KCNQ5 | CACGTTGGACAATTATGTCAA ACCCATTATTGGGAAGCTTAA |
| 56606 | NM_001001290 | SLC2A9 | CACGTTGGACAATTATGTCAA CAAGGTGATTCTGGATAAATA CAGGATCTGGATGTTGTTGAA CTGGATGTGTTGTTGAAATCACA |
| 56649 | NM_019894 | TMPRSS4 | CAGGATCCTGACAGTGATCAA CAAGGTGATTCTGGATAAATA CAGGATCTGGATGTTGTTGAA CTGGATGTGTTGTTGAAATCACA |
| 56650 | NM_019895 | C3orf4 | ACCGGAAAGAGTACACCTTAA ACGGTGTATCACCATACCCAA |
| 56652 | NM_021830 | PEO1 | CAGGCACCTAAGGCATTTCAA CAGGATCGCAGCTCAAGACTA TAGGATAGGCTGGAGCATAAA CTGGCAGGCTAGTGTAGATAA |
| 56659 | NM_022054 | KCNK13 | ATCCTTATTCTTTAAGTCTAA CCAGGTTCCTTCAAAGCATA TAACTAAAGACATAGCTTAA TGCAAAGATGATGACTATTTA |
| 56660 | NM_022055 | KCNK12 | TGGGACCATCCTGTCTCTTCAA CTGCATTATCTCGCTCTTCAA CGCGCTGGGCATCATGAACAA CTGGCGCTTTCTTAATCTTTA |
| 56729 | NM_020415 | RETN | CAGCATTGGCCTGAGTGCCA CAGGAGGTCGCCGGCTCCCTA CTCCATGGAAGAAGCCATCAA CCCTAATATATTAGGGCAATAA |
| 56848 | NM_020126 | SPHK2 | CACGCTTTGCCCTCACCCTTA CAGGTGGAGTATGGGCCGCTA CCGAGGGTAGTGCCTGATCAA CAGGATTGCGCTCGCTTTCAT |
| 56851 | NM_020154 | C15orf24 | TACATTATGTATATTAATTAA CAGGTCCACCTTCTTACTTTA |
| 56888 | NM_020122 | KCMF1 | CTCCTAATACTCCACATTCAA CAAGAACATGTTACTTCTGAA |
| 56889 | NM_020123 | SMBP | CACGTCTTTCTGGGCATATAA CAGATGTACATAGATGATTTA |
| 56893 | NM_020131 | UBQLN4 | CACACTGCCCTTTGTAAATAA CCAGAGAGAAATTCGTGTGAAA |
| 56897 | NM_020135 | WRNIP1 | CACATGCTCAATAACCCTGAA AGAGATGCTAATGAATTTAA |
| 56900 | NM_020141 | AD-020 | ATGAATTAATGTTATAAGGAA CGCCATGTTCATAGAGGATAA |
| 56904 | NM_020145 | SH3GLB2 | CTGGAAATTCTATTTACTTTA AAGGTAATTTACAGCCAGAAA |
| 56911 | NM_020152 | C21orf7 | CACCAAGAACTGGACAGAGAA CAGACAGAGTTTGACCGGCAA |

Table4

| | | | |
|---|---|---|---|
| 56915 | EXOSC5 | NM_020158 | CCCAGCAAGGATAACATTCAA ATGGAATGGATGAATCAATAA |
| 56919 | DHX33 | NM_020162 | ACGGTTCCTCTTAGCTATTTA CTGGGTGAAGGTTGATTTGTA |
| 56922 | MCCC1 | NM_020166 | CAACATTGACTTCTTACTCAA TAGCATGCAGATTGAAGATAA CATGGTTATGATCGCCATGAA ATGCATTGGGTCACTAATTA |
| 56928 | SPPL2B | NM_020172 | CACGGTGAGGGCGGCGCTGTA CAGGTTTGACATCCAGGTACA |
| 56930 | LOC56930 | XM_373790 | CACCAACCCAGTTCTCAGTTA TTGCCACGTGTTCCTGTTAA |
| 56935 | FN5 | NM_020179 | AAAGGAGAATAATAGCCTTAAA CTGGTCTTTGACTTGATTAAA |
| 56943 | e(y)2 | NM_020189 | CGCGGTGATGGTGGTTAGCAA AAGGAGCTCCTACAAAGAATA |
| 56949 | XAB2 | NM_020196 | CCGGACCTTGTCTTGTGTACAA CCGCGTGTACAAGTCACTGAA CACGTACAACACGCAGGTCAA |
| 56950 | SMYD2 | NM_020197 | AAGATAGAAATGACCGGTTAA ACCGGTTAAGAGATTCTTATT CCGGAGGGCCAAGCACTATAA GAGGTTAGTCTGAATCTTGAA |
| 56951 | C5orf15 | NM_020199 | AAGGATATTTGTGTACAATTA TAGGATAATGATATTGCTCAA |
| 56952 | PRTFDC1 | NM_020200 | CAGGGTATGACTTGAATTTAT TACCGGGAATGCAGCTATTAA CTCGAAAACTAAGCAAGTATAA |
| 56956 | LHX9 | NM_020204 | CCGGCACTGCGACCACTTTAA CAAAGTGTATTTGGATTTAAA AACCAGATAATTAATTAGTTA CCGGGGCGTGTGTATGGAAA |
| 56961 | SHD | NM_020209 | CCAGGGCTTCCTGCATCTGAA TCCGGAGACCTCCGCCAGAA |
| 56967 | C14orf132 | NM_020215 | CGCCATAAATTCTGAAATAAA CAGCATGAAAGTGATGAGCTA |
| 56970 | ATXN7L3 | XM_375456 | CAGCATCTATGATGACATCAA CCACAAGAGTTTACAAATGAA |
| 56974 | DKFZp547K054 | XM_498167 | CGGGTCGATGTGCAAATCCAA CCGCATTAATGTCCTCTGGAA |
| 56981 | PRDM11 | NM_020229 | CTGGCTGATTGTGGACAAGAA CTGGATTGAGGTACGTGGTCAT CAGGTTCTTGGATAACCCAGAA ACGCCTACAGTCAGTGTGCAA |
| 56988 | C8orf17 | NM_020237 | AAGAGCAATAATACAAATTCAA ATCAAATTTCTTTATCTATAA |
| 56990 | CDC42SE2 | NM_020240 | ACGGCGGATTGACAGAGTAT CCCACAAACTTTGTGCATACA CAGGAGACCTGTTCAGTGGAA AAGGGAGGTTATGGAGGTGGA |
| 56992 | KNSL7 | NM_020242 | CTCTATACACTCAGAATTCTA CAGGATTCCTATGACAACTTA TTGAGATTGACCAACTTTCAA ACGCAATAACAAATTATCATT |
| 56995 | TULP4 | NM_020245 | CCGTTTCTTGATCACCACTTA CTCGGTGCAGTAATCTATAAA CACACTGTGTTGGACCAACAA ATGCATTACTGTAAACATGAT |
| 56996 | SLC12A9 | NM_020246 | CAGCAGTACCCTGAAGGACAA CAGATCGTCGCCGTCGCCTA CCCACTGGTGTTGATCGGAAT CTGGTGTTGATCGGAATCTAT |
| 56998 | CTNNBIP1 | NM_020248 | TCCCTTCAGACTGGCCCTAA CAGTATCGTTTGTTTATGAAA TGCGTTTATAATTGGCTTAAA CACCTGTTTGCCTGAAGTTAA |
| 57007 | CMKOR1 | NM_020311 | CACGCTCTCCTTCATTTACAT CTGGGTGAATATCCAGGCCAA CCGGAAGATCATCTTCTCCTA CACTATTGGTGTACCTTATAA |
| 57018 | CCNL1 | NM_020307 | TTTGGATATAAGGGATCTTGTA CAGTGTGATGTATGGACTCAA CTCCAAGAAGACACTATAATA CCCTAGCAGTATCTAATTAAA |
| 57019 | CIAPIN1 | NM_020313 | CAGGTTGAACATGATGATCAA CAAAGTGAAGACAGCATCTAA |
| 57026 | PDXP | NM_020315 | CAGGTTTCAGTCAGTAAATAA CCAGTTTAGGTTCCTAAGTAA |
| 57048 | PLSCR3 | NM_020360 | TAGCATCTGCTTGGCCAATTA TACATTCATATAAACTTTGTA |
| 57060 | PCBP4 | NM_020418 | AAGGGCGAGACTGTAAAGCGA ACAGGAGTTCTTGGTTCCAA |
| 57085 | AGTRAP | NM_020350 | TTGGGTCTTCTCCAGGACCGTA AACCTGAAGGTGATTCTCCTA CTGCTTCGTCGTCTACCACATGTA CAGGGATTGCCTGAACCAAGA |
| 57093 | TRIM49 | NM_020358 | CAGGATCTCACATTTGCTTAA TACGTATTGGTTCTTTATTAA CAGGCCTTGTTTCTTCTACCTCAA ATGATATCTTTCTGTATGAAA |
| 57105 | CYSLTR2 | NM_020377 | CAGATTATGCCAGGCACTTTA AAGGGTGGAGGTGATATGGCA TGGGATCATATGGATCCTTAT CAGGCTTTCTAAATTCTTCAA |
| 57113 | TRPC7 | NM_020389 | AACCGGTGATGTGAACTTCCAA CCGGATCAAACTCGCCATTAAA CAGGCTTACGTCAACAGTCTA |
| 57115 | PGLYRP4 | NM_020393 | CCAGTGAATGTCCTTGTTATA CAGGATGATGGCATCATTA TCAGCAGTGTCTGAAATTTAA |
| 57117 | LOC57117 | NM_020395 | CCAAGTTGTACTGAACGCCAA AACCTGCTACTTCGTCAGCTA TACGCCCACTGTACCTTTAAA CAGGACCTAGTGGAAGTACTA |
| 57118 | CAMK1D | NM_020397 | CTGAAAGATTAAGCATGAA ATGGAGAAGGACCCGAATAAA CCAGGCGCTTTCTATACTTAAA CAGGCGCTTTCTATACTTAAT |
| 57122 | NUP107 | NM_020401 | CAGCTTTAAGTGGGAATCTTA CAGTGAATATACTGAGTAAA |
| 57124 | CD164L1 | NM_020404 | CAAGCCATCACTGACTGCTA CCCTATGATCTCAACCAAATA CACGCTGGCTGTCGACGGCTA |
| 57130 | ATP13A1 | NM_020410 | CAGGAGCTGCACTTCATTGAA CACCTTCGCCATCAATTACAA |
| 57140 | RNPEPL1 | NM_018226 | CACCTACTCTGCCGTCGTCAA CCTGATGAACCTGTTCACCTA CCGAGCCTATGTGGAGAAGTA CCGGGCCAGGCTGGAATTCGA |
| 57144 | PAK7 | NM_177990 | ACGCCCTCAGTAGCAAACCAT AAGGTGGATTACGATCGAGCA ATGGTGTGCACGTTTCATTAA ATGATCTGGATCCGTATTATA |
| 57146 | LOC57146 | NM_020422 | AAGGTGTAGTTTCCACTGTAA CAGATGGATGAAGACCACTAA |
| 57147 | PACE-1 | NM_020423 | TGAGGAGTATTCAGTCAATAA CAGGAGGTGGAATCACTGCTA CAGGACAATTCCGGAAGTAAA AAGCTCTATTATGAAATACAA |

173

Table4

| | | | |
|---|---|---|---|
| 57148 | KIAA1219 | NM_020336 | CCGGTACTTTGTCTTGGATAA CCGGGCTGTTACTGTGAATAA |
| 57151 | LYZL6 | NM_020426 | AAGCAAGTTCAACATATCAAA CAGCAGCTTTCTTGCCCTAAA |
| 57152 | SLURP1 | NM_020427 | CTGCTTCCGAGACCTCTGCAA CAGCTCCTGTGTGGCCACCGA CAGGGCGGAAGGTGCTCCTCA CAGGACCATTACCCGCTGCAA |
| 57153 | CTL2 | NM_020428 | CCGGGACTTTGAGTACTATAA CACAGTGAACACCCACGTTTA |
| 57161 | PELI2 | NM_021255 | CAGAACTGTTTGGTTGATTAA CAGGGTTAGGGTTTAAAGAAA CACGTGATATAACTGGTTATA CTCGGGTACAATGGTGCTTTA |
| 57162 | PELI1 | NM_020651 | CAGGACTACATTATAAATTTA CAGGCTATATATTGTAGCTTA |
| 57167 | SALL4 | NM_020436 | CCGAACCAACACATCCATTAA CGCGGTGGATGTCAAACCCAA |
| 57172 | CAMK1G | NM_020439 | CTGGAAGAAACAGACCACCAA TAGAAAGAAGTTCATGAGTAA CAGGTCTTGTCGGCAGTGAAA AACAGGCCGCCTGAAACTCAA |
| 57176 | VARS2L | XM_373419 | CCGGAGGAACCGTGAAGCCAA TAGTATTGTATGAAATCCCTA CATGCAGGAATTGCTACACAA CGCTTTATCCTCAATGCTTTA |
| 57238 | KIAA0492 | XM_378914 | CTGTGTTATAATGATACTTAA TAGCAATTGAATAAACATAAA |
| 57325 | CSRP2BP | NM_020536 | CTGGAAGGAAGTGGACGTCAA CAGGCGTGATTATGAAACAAA |
| 57404 | CYP20A1 | NM_020674 | TAGAGAGACCCTCGTCCTTTA ATCCTAGAAGACAGTATGATA CAGAGTTGAGGTTTGCATATA CCAGGGATTACTCCAACTGAA |
| 57405 | Spc25 | NM_020675 | CGGGACTAAGAGATACCTACA ACGGTTTATAATTAACATACA CTCGTCATTTCATTAGGTATA CCACGGTTTATAATTAACATA |
| 57407 | HSCARG | NM_020677 | CACCTTCATCGTGACCAATTA AGGCACCAACATCTGAATAAA |
| 57413 | AD026 | NM_020683 | CAGAGGAGCTATGATAGACCA TGGGAAAGACCTATCAGGCAA CTCGCGTGTCCTGACTCCAAA CACGGCTTCTGCCATCTGCAA |
| 57452 | GALNTL1 | XM_031104 | AAGGATCCACATACCCATAGA AGCAGCTAGGCGCGAGCGAAA |
| 57462 | KIAA1161 | NM_020702 | CGCGGCCGCCATCAAAGTCAA AGGACTCTCGTAGGAAATAAA CAGGCCAGACTTCGTGCCTTA CACCTCTATACCACCCATTAA |
| 57472 | KIAA1194 | NM_015455 | CCAGGATATATTGAACCTTTA CAGAGGCAACTTATTCATGAA |
| 57484 | RNF150 | XM_371709 | TCCAGAGGTTTCGATATGCAA AACCCGGAACTTGCAGAAATA CCGGAACGCGTTCCTGCAGAA CAGAAGGACTGTTGCCTTGTA |
| 57504 | MTA3 | NM_020744 | AAGAAGGATAGAAGAACTCAA ACGGCCGTTTGTTGCTATTAA |
| 57508 | KIAA1287 | NM_020748 | CAGGTAAACCCTAATCATTAA ATGAGTGTTAGTGGAATTTAA |
| 57510 | XPO5 | NM_020750 | ATGGTACAGGCTACTGCTAAA CACGGTTTGCTGTGTTTCAAA CCAGATGTTTCGAACACTAAA CTGTCTCGAATTGTAGTGGAA |
| 57514 | CDGAP | NM_020754 | CAGGATGTCACCATAGATAAA CTGGATCAGACTCCAAATCAA |
| 57520 | HECW2 | XM_038999 | CTCCATCACTTGAGAAGTATA ACGGTCTACTATCATCAGTAA AAGGCTCTTCTCAGAATTCTA CAGGATTGGTGCGCCTTTCAA |
| 57529 | RGAG1 | NM_020769 | CACCATCTCACTACATTCTTA AAGCAATATCCTCACTGATAA CAGGGCATATATGACTCCCTA AAGGCCCATACCAACAAGTAA |
| 57533 | TBC1D14 | NM_020773 | CAGCAGGACTGTGTTCATGAA AACACTGGAGTTGGCCTTAAA |
| 57538 | ALPK3 | NM_020778 | CAGGTGGATGCTGGGACACAA CAGGCAGATGGGAAGATACAA |
| 57558 | USP35 | XM_290527 | GTGGATCATTGCACTGCTGAA ATCGAGGTTTCGCTCACCAAA CCCTTCTAACTTCTAACCGAA ACCCATGTTCATAAATAAATA |
| 57560 | KIAA1374 | NM_020800 | AAGGAATGAAATTCTGATAAA CCCAAAGTTATTACCACATAA |
| 57565 | KLHL14 | NM_020805 | CCAAATTTAATCCATAATAAA AAGCATTTATGAGAATTATAA |
| 57572 | DOCK6 | NM_020812 | CACGGTGAAGATGAAGGAACA AAGACCAAGGATGAAATGGAA |
| 57579 | KIAA1411 | NM_020819 | CAGCAATTACATTAAATTCAA AAGGTGCTGAATAGCATTAAA |
| 57582 | KCNT1 | XM_029962 | CTGGCTCTTTCCAAACTAGAA AGCGCTGGAAACTTCTATTAA CAGGTCATCGTGGCCATAATA ACGAGGACGTAGCAAATTAA |
| 57585 | CRAMP1L | NM_020825 | CTGCATAATGATCCCATTTCA CAGATCGTCAGTGGGATTGAA CCCGACAACCTTGCCACCCAA AAGGGCCACCACTGTACGTTA |
| 57592 | KIAA1441 | NM_020832 | CCGCCTGGTCTTCTTCAACAA CAGGCTCTCAGGAATCCTTTA |
| 57593 | KIAA1442 | XM_044921 | CAGGGAGAAGTGACCTGGAAA CAAGTTCTTCCTCAAATGCAA |
| 57599 | WDR48 | NM_020839 | CAAGTTTGTCATATAAAGTAA AAGGGAGATCATAGTGAGTTA |
| 57605 | PITPNM2 | NM_020845 | CTCCAAGATGATTATAAAGGA CTCGTGCAAGCAGGAGAGAAA CTCGAGTTTCTCAACTGTTCA ACGGGAAGGCTTGGACTCCAA |
| 57608 | KIAA1462 | XM_166132 | CTGGAAGTAACTTTACTTATA AAGGTAAATAATAGCCTATAA |
| 57609 | KIAA1463 | NM_173602 | CCGGCATACATTGAGTATAAA ATGGAATTAATTTACACGTAA |
| 57613 | KIAA1467 | XM_370682 | GTGAATCTCTTAAGTATTTAA CGGCGCAGTGATGGGATGTTAA CATGGTGTATTTGCAAACTAA TTGGTTTAGTATAGAATTTGA |
| 57617 | VPS18 | NM_020857 | CCCGATTATCTCGCTGCATAA ATGGAGGTGGAGAGCATTAAA |
| 57621 | ZBTB2 | NM_020861 | AACGTTTAATTAAATGAGTAA CAGGAGTACCTTTCACATCTA |

Table4

| | | | |
|---|---|---|---|
| 57633 | LRRN1 | NM_020873 | AAGATTGATAACCCTCACATA AACCTCTTCAATCCCACTAAA CTGGTTTGATTCTACACCCAA AAGACTTTGCTTGAATGTTTA |
| 57636 | ARHGAP23 | XM_290799 | CTCCTTCATCTCGGCCGTCAA CTGGCTGAAAGCAGCACTGAA |
| 57642 | KIAA1510 | NM_020882 | CCCGACCTTCACGCTCTTCAA CTGGGAAGAGAGCAAGTTCAA |
| 57646 | USP28 | NM_020886 | CTGGAACAGTTTGCAGATAAA AAGGATTAGGTGGGCACATAA |
| 57647 | DHX37 | NM_032656 | CCCAGTGACTGTGCATTTCAA CCAGATCAACTTGGATCATTA |
| 57650 | KIAA1524 | NM_020890 | ACCACTCTTATAGAACAACAA CAGCTAGTAGACAGAGAACAT CGGCACAATCTTTCTGTTCAA CAGAAAGAGCGCAGAGTGATA |
| 57654 | KIAA1530 | NM_020894 | CACGGTGATCTCGAACTCCTA ATCCAAGATCACAGAGATTTA |
| 57657 | HCN3 | NM_020897 | CTGAGTGGGAACATACTTCTA AGGGACAAGAGTAGTCCTTTA CAGCTTTCTGCCAACATGTAA CCCGCCTTGGATCGTCTTCAA |
| 57661 | KIAA1542 | XM_290536 | CACACGAACCATCTCCATCAA CGAGCTCAATTTGGTGGTAAA CGCGTCCTTGATAAATCTCTA CCGGATCAATATTCCTGGAAA |
| 57665 | RDH14 | NM_020905 | TATAGTAAGTATAATGAATAA ACAAGTCTTACTTGGAATAAA CTGCCTGGTGTGTGCACACAA CAAGCATGGATGACATATTAA |
| 57666 | KIAA1545 | XM_495939 | CCGAAGTTACAGATTATATTA TAGTCTGTTGATATAAATATA |
| 57693 | ZNF317 | NM_020933 | CCGCTGGAAGTCCAACTTTAA CAGCTTGGCATTTAATGTCAA |
| 57703 | KIAA1604 | NM_020943 | CAGGAACAGATTACAGATAAA CAGCAAACAAATGATAGGAAA |
| 57707 | KIAA1609 | NM_020947 | CAGGCAGTGCACTTAGAGAAA CGCGTTGCAAACTATTATAAA |
| 57711 | ZNF529 | NM_020951 | ATGGATTTAATAGGTAATCAA AACCTTGTATACAGACATTAA |
| 57716 | PRX | NM_020956 | CAGGCTACTTCGAACCAGGAA CCCGCTGCTCCTGAAGTTGTA AGGGATGGAATTTGGCTTCAA CCCGCCGTGGAAATTGAGGAA |
| 57717 | PCDHB16 | NM_020957 | ACAGCATTAAACAGTCAATAA AACCTGATTTATAGTCCTGTA AAGCAAGAGCTGAATATAATA AACGGGTACGGTGATAATTAA |
| 57722 | NOPE | NM_020962 | CCGGCTCAAGAAGAAAGTGAA CCAGCCTATGATAACACTAAA |
| 57729 | KIAA1639 | XM_290923 | TCGGAAGCACTGCTCAGTGAA CACACAGAGGACCTACACATA CACAAAGACCTTCGCATTCCA CCGCACGGCATGTGTCAGCAA |
| 57733 | GBA3 | NM_020973 | AACCATGATTTGCAACTGTAA CAGATAGACCTGGGAAACATAA |
| 57758 | SCUBE2 | NM_020974 | CAGGACAAGTGTAACCTTTAA CAGGAACTCATTGAAGACATA |
| 57763 | ANKRA2 | NM_023039 | CACAGTGTGATTGTTTACAAA CTCGATTTGTGAAGTCCTTAA |
| 57787 | MARK4 | NM_031417 | TAGGAGTTAGTTAGGCATTGA CAGTAGGGCCTTGAAGGTTAT CTGCAGCCTGTTGCCCAATAA CCGCATCATGAAGGGCCTAAA |
| 57801 | HES4 | NM_021170 | CCGCCACTCGAAGCTGGAGAA TGCCGAGACTGTAGCAGAGAA CTCCAAGCCGGTCATGGAGAA AAACGTATTTATTATTCCAAA |
| 57804 | POLD4 | NM_021173 | TTCACTAATGCTTATCAATAA GAGGCTCAACTCCTCCATCTA |
| 57805 | KIAA1967 | NM_021174 | CCACTTAACAACTAAATACAA CCGGTTCCCACTTAACAACTAA |
| 57820 | CCNB1IP1 | NM_021178 | AAGATAATTTAGCTAACTTTA TAGCAGGGCCTTCAAAGTAAA |
| 57823 | SLAMF7 | NM_021181 | ATGTATTAATTGGCTCTATAA CACCATGGGTCTGCAGAGCAA AACAGATATTGTGAGATTCAA CACTAATAGAACAATCCTAAA |
| 57864 | TSCOT | NM_033051 | AAGGAAGAGGAGGTACATTAA CACCTTGTACAACAAGATCTA |
| 58189 | WFDC1 | NM_021197 | CCGGATGTGCTGAGGCCTAAA CTCAAATGTTCCCAAGATAAA |
| 58190 | CTDSP1 | NM_021198 | CAGCTCCTTCAAGCCAGTGAA CTCCTTCAAGCCAGTGAACAA |
| 58475 | MS4A7 | NM_021201 | TCGGAGTACTATTATCCAATA CAGGAAGGATTTGACTATCAT CACACTCATATTGCTTAAATA CACGGTCTTGGATATAAGTAA |
| 58480 | RHOU | NM_021205 | CACCTCTAATCTGGATGTTAA TACCCAGTTACTTATGATTTA CCCAGGTATTACACAAGCCAA ACCCAGTTACTTATGATTTAA |
| 58484 | CARD12 | NM_021209 | AAGGTTCAAGCCAAAGTATAA CCGGGATTTCAGCAAGTTGAA |
| 58490 | C20orf77 | NM_021215 | AAGCTTAAATTTGTATATTAA ATGGTTGGAAATAACCTTCTA |
| 58493 | C9orf80 | NM_021218 | AAGTGACTTTGTAATATCAAA TGCGTTTATAGTCAACATGTA |
| 58496 | LY6G5B | NM_021221 | CCGCATGTACTTGTTCCTCAA CACCATCTATTATGATGTCAA |
| 58497 | PRUNE | NM_021222 | CTGGTATACTGTTCTCATAAA ATGGCTGAATTTCTATTCTTA |
| 58499 | ZNF462 | NM_021224 | CCCGGGATCCTTAAATTATAA AAGAAGGGCAAGAATAATTAA |
| 58508 | MLL3 | NM_021230 | ATCAGAGTTCACGACCATTAA ACCAGTGATCACTTTACTAAA CTAGAACAGATTCGTAAACAA CCCAGTGAGCTTCCTCAACAA |
| 58510 | PRODH2 | NM_021232 | CAGGAGCTGCTCAGCCAAGAA CAGGATGCTCCGGACCTGTTA |
| 58513 | EPS15L1 | NM_021235 | AGCCTTGTTATTTAGCAACAA CCGGATCCTTTCCAGACAGAA AACCATCATCAAGTCCCTGAA CAGAACCTTCTAGCACACATA |
| 58533 | SNX6 | NM_021249 | CCGAAACTTCCCAACAATTAT GCCACTCTTATTTACCTTTAA TCGCCAGTAGTTATATTACAA CCGCGGACTTAAAGCAATAAA |

Table4

| # | Gene | Accession | Sequences |
|---|------|-----------|-----------|
| 58538 | MPP4 | NM_033066 | CAGGCCACTAGTATCCTACTA CAGGTGTTATCCTATGAGGTA TCGAGGCACAATCATGTTCAA TACGGTCAGAAGTTCTTTATA |
| 59082 | ICEBERG | NM_021571 | CAGTGGGTGCAGGCACAATAA AGCCAGGAAGACATGAACCAA CCAAGTCTTGCTGCAAATTTA AAGATATTCTCGTGAAAGAAA |
| 59283 | CACNG8 | NM_031895 | CACGCCGCCCTCATCTGCAA AGGCGTCTGCGTGAAGATCAA CTGCGTGAAGATCAATCATTT CACCAACACGCTCAACAGGAA |
| 59286 | UBL5 | NM_024292 | AACCTGGAGCTTTATTATCAA TTGGAACAAGATTGTCCTGAA |
| 59341 | TRPV4 | NM_147204 | CACCAAGTTGTTACCCAAGAT CCCGGGCAAGAATGAGACCTA CTGGGAGTGAATTCTCTCTTCA CTGGGATTTGCCGGTGCTCAA |
| 59343 | SENP2 | NM_021627 | ATGGTCGGAATCAGATTTGAA CAGATTATATTTCTAGGGACA CCACACAAGAACAAACGCTAA AAGAACAAACGCTAACTAATA |
| 59345 | GNB4 | NM_021629 | TACCTTATATTTGCAGGTGAA AAAGTAGGTAATAGCATATAA TACGATGACTTAATTGTAAT CTGCTCTATATAATAACTTAAA |
| 59350 | LGR7 | NM_021634 | CTGCAGTTACCTGCTTTGAA CAGGAGATGCCACCTGAGTTA ACAGACTAGAATGGCTGATAA AAAGGATGTTTAGACCCTCTTA |
| 60385 | TSKS | NM_021733 | GTGGAAGATGCTGAAATCAAA AAGCTGTTCACCGGCATCGAA CACCAACGTGTCACTGCTCAA AACCAACGTCTTGAAGCAGAA |
| 60386 | SLC25A19 | NM_021734 | CTCCCTGATCAGTTACCAA CTCGTATGAATTCTTCTGTAA AACCTATTTAATAGACACATTAA ACCTATTTAATAGACATTAAA |
| 60437 | CDH26 | NM_021810 | CACAAGTTACACTTTCTTAAA CACACTATTAGTTTGAATTAA |
| 60468 | BACH2 | NM_021813 | CCGGGAAGATAACTCTAGCAA CTGATGCAAGTGCACCTCTAA CAGGTGATAATTATGTAGGAA CACGAGGCATTGTGTTGAA |
| 60490 | MDS018 | NM_021823 | CCCGTATAAACATGGTGGTTAA ATGAAATAAACTATAACTAAA |
| 60526 | FLJ21820 | NM_021925 | TTCGATATGTTCTCTATGTTA AGGAGCATTTATGTAAGCTTA CTGCACTAGACAGAGAATAAA GAGATACTCACTGATACTAAA |
| 60529 | ALX4 | NM_021926 | CAGGTTCCCTGCTACGCTAAA CAGCAGCTACCTGAGTGTCAA CCGGACCACCTTCACCAGCTA CAGCCCTCAGCCCAGGCCTCAA |
| 60592 | SCOC | NM_032547 | TAGGATTAGTAAGATATACAA CTGGATGAGAACAGTTACAAA |
| 60673 | FLJ11773 | NM_021934 | CACAGGCAAGTTCCACTACAA CGGGAGAAGGTGGGTGAGAAA |
| 60675 | PROK2 | NM_021935 | CAAGGCTCTTACCTTACTTTA TCGCTCTGGAGTAGAAACCAA TAGCCACATCTTACCTGTAAA CGGTATTAAGTTGTAATTTAA |
| 63027 | C6orf85 | NM_021945 | ACGGAGTGAAATGTACATGAA AAGGATATGCTAAGTGATGAA |
| 63891 | RNF123 | NM_022064 | CACATGCGTGTACAAAGGGAA CCGCAAGTTTCTGCTTAGCAA CTGCGCTACTATTGGGATGAA CAGGGCCACAGTGAGCATTAA |
| 63892 | THADA | NM_022065 | CAGACTTCTATTGATGCTAAA CACCTTGAGTTCACAGATTAA |
| 63925 | ZNF335 | NM_022095 | CTGGAGGAGGATAGCGACTAT CGGGCAGCGTTCAACCGTAA TGGCCTGTCAGTCTCAATAAA |
| 63939 | C20orf177 | NM_022106 | AAGCAAGATATTCTATACATA AAGGGAAGTGTCTTTCAATAA |
| 63940 | GPSM3 | NM_022107 | TTGGTTGTGAATAAAATATA CGGGCTGTGAATGGACCTTCA |
| 63971 | KIF13A | NM_022113 | CCGGCAGGCCGTCACAGTCAA AACCTATGAAAATAGTATCCAA CCGCAACAACTGGTAGGAAA CTGGCGGGTAGCGAAAGAGTA |
| 64061 | TSPYL2 | NM_022117 | CAGACATATCTCCATGGGCTA CAGCGAGAATCCTGACCACAA CCAGAGGTTAGTTGACCCGAA CACAAACATGGTGATTGTCAA |
| 64072 | CDH23 | NM_022124 | CCGGCTGCCCTTCTTCACCAA CAGCGGAGTGCTGACCTTGAA |
| 64083 | GOLPH3 | NM_022130 | CACCATTGGTTTCATAATGTA CACAGTAAATACTTAATATAA |
| 64087 | MCCC2 | NM_022132 | CCCGAGCACTTCACATATCAA AACAATTACGGGCCCAAGAAA ACCCTTACTATTCCAGCGCAA CCCATAAAGCGGAGACAGTAA |
| 64090 | GAL3ST2 | NM_022134 | CACCAACATCATGTTCCTGAA CACAGTCACCGAAGAAATGAA |
| 64100 | ELSPBP1 | NM_022142 | AAGCTAAATAACCACCTTTA CAGCTGGTCCTTTGTGAAGAA |
| 64101 | LRRC4 | NM_022143 | ACCACTATCTCTGAACCTTAT AAGCCACAAGTTAACTTGCTA CAGAAGGGATTTCTGGTATAA AAGCTGTGAGATCCACAGTTA |
| 64102 | TNMD | NM_022144 | CAGATTAAAGTGATTCCTGAA TTGGAAGTACACGACTTTAAA |
| 64106 | GPR147 | NM_022146 | CTACGGCTACTTCAACGAGAA CACCAACATGTTCATCCTCAA CTGTTTCATCGTGCTCAAGAA CTGGTGGCCATTGTGCTGTGGAA |
| 64109 | CRLF2 | NM_022148 | AACCGTGCAGGTGACATGGAA ACACACCACTGTCAAAGTCAA CACATGAGTCTCTGTGTTCAA CAGGATCCACGTTGACATTTA |
| 64127 | CARD15 | NM_022162 | CTGCCACATGCAAGAAGTATA CAACATGGCCGTGAACTTTAT TAGGCCGTTCCTCAAAGCAA CAGTTATGGATTGAAAGTTTA |
| 64129 | LCN7 | NM_022164 | AACGTACTGGGACAACTGTAA CCCATCTTCCTCGGTCATGAA CCGGCCTGGGATCCCAGGCTAA CAGCCCTGTATTCTTATTCTT |
| 64131 | XYLT1 | NM_022166 | CAGGGTGCTAGCGAAATCCAA CAGCAGGATCTCAACGGGAAA |
| 64132 | XYLT2 | NM_022167 | CCGGGAACAAGAATTTCCTCAA CACACCCATTCAGCCATCAA |
| 64145 | ZFYVE20 | NM_022340 | CAGGATGCAGCATCATTAAA ATGGAGAAAGTTGACCAGAAA |
| 64146 | PDF | NM_022341 | CCGGTGGAGCGGGCGCAGCTA CTGGTGCTGGGTCCTCCCGAA |
| 64147 | KIF9 | NM_182902 | CCCGGACCTTATCAGAGGAAA CACGCTCTGAAGGACTCGTTA AACAGACAGACTGGTCGTTTA CAGGACTTGGTTTATGAGACA |
| 64149 | NJMU-R1 | NM_022344 | AACTGTGTAGTGGAGATATA CACTGAGTCCTAGATAGATAA TTGGCAGATACTAATCTACCA TTGAGTTACACTCCTGTTGAA |

Table4

| | | | | | | |
|---|---|---|---|---|---|---|
| 64170 | CARD9 | NM_052813.1 | CAGCGACAACACCGACACTGA | ATGCACTGTATTAGTGTTAGA | CCGCGTCTTCTCCATGATCAT | ACGTAAGGACTCCAAGATGTA |
| 64174 | DPEP2 | NM_022355 | CTCCATCTTACGTACCTTCTA | CTGGGCATGATGGTAGACTTA | CAAGGCTGTCATTGGATCCAA | CAGGCACAAATATTTCTTGAA |
| 64211 | LHX5 | NM_022363 | CAGGTACAAGCTAATACTCAA | GCCAATTTAGATATGCTATAA | TTAGATATGCTATAAATTTAA | AAGCAGCCTCCTGAAACCAAA |
| 64223 | GBL | NM_022372 | CCAGGTGAACGCACCCATTAA | CAGCTACATGGCAGCTGTCAA | | |
| 64232 | MS4A5 | NM_023945 | TAGACTGAAGTACCAACTAAA | CAGCATTATTGAATTATTCAT | AAGAACATGGTCTAGACTGAA | GAGGATTGTGATTGTGAACAA |
| 64236 | PDLIM2 | NM_021630 | AGACATAATCGTGGCCATCAA | CTGAGAGTCAGTCCTCCTTAA | | |
| 64328 | XPO4 | NM_022459 | CAGAACTTTATGCTTAATTTA | AAGGAATGCCTTGGTAAGTAA | | |
| 64332 | NFKBIZ | NM_001005474 | ACCATTAAGTGCCTAATTCAA | TAGCTGCTGCTTATCATGTAA | CACTAAGTCTTTGTAGATAAA | TCCGACGTATCCTGAAGGGAA |
| 64344 | HIF3A | NM_022462 | CAGACTCTTCACCTCCGGGAA | CTGTCGGAGAATGTCAGCAAA | CGGGCGCACCCTCAACCTCAA | ACCAATCTCTATACACCATAA |
| 64359 | NXN | NM_022463 | AAGCTCAAACTTTGGAACAAA | CCGGGTCCTGGTGGAATCCTA | AACCGGCTGTACGGAATCCAA | AAGGTAATGGTTCATATCATA |
| 64374 | SIL1 | NM_022464 | CCGAAATAATTTGAAAGGCAA | AAGGCTGGATATCAACACCAA | | |
| 64375 | ZNFN1A4 | NM_022465 | CAGACCATCACTGCACTTTAA | TTCCTTGAGATTCAAACCAAA | | |
| 64377 | CHST8 | NM_022467 | TTGGATTAAGGTTCCAAATAA | CAGGAATAAAGTTCAACATCA | | |
| 64393 | WIG1 | NM_022470 | CAGGCTCATTATCAGGGTAAA | CAGCATTTAGAGAGCAAGCAA | | |
| 64400 | FTS | NM_022476 | CTGCACTGTCTTACTGATTTA | AAGGTGAAGAGAAGACATTAA | | |
| 64409 | WBSCR17 | NM_022479 | CTGGTTAGGGTGCACATATAA | CAGAAATGAGAAGATACAATA | | |
| 64419 | FLJ22405 | NM_022485 | CAGCGTGATTCCAAACACGAA | CACGTTTGAGAGTACCGTACA | CTGCCCAGTAATCCTGTTCAA | AAGGTGAAGTTTGGCATGAAT |
| 64421 | DCLRE1C | NM_022487 | AAGAAACGAATTATATCTATT | CCCAGTGGGAAGTATTCTTTA | CAGGTTCATGTGAATAAGCTA | AAGTACGGAGCCAAAGTATAA |
| 64423 | C14orf173 | NM_022489 | CAAGATCAGCACATTGCTGAA | CAGCGATGTCCTAACCACTGA | CCTGATTCTGATGATAATAAA | CCCGGCCTTGATGCTACAACA |
| 64429 | ZDHHC6 | NM_022494 | TAGAAGGTGTTTCAAGAATAA | TACGTTCTGTTCGGTTATCAA | | |
| 64446 | DNAI2 | NM_023036 | CAGGGTAATGCTTAGCATATA | CCGGAAGAAAGTGGAGAAAGA | | |
| 64478 | CSMD1 | NM_033225 | ACGGGCGAGCGCAATAGGATA | CCGGTTCAGTATGAAGATAAT | CAGGAAGTCGAATTCACCTAA | CAGGACTGATTCATAGCCATA |
| 64582 | GPR135 | NM_022571 | AAGACAATGTCCCTTCTTCAA | ACCAAGCATTGTGAACTGCTA | CAACAGGATGTCCTCTTCCAA | ACCAAACAGCCTAAATCCGAA |
| 64601 | VPS16 | NM_022575 | CTGCATGAAACAACATAACAA | CCGCACGGAGCTGGCCATCAA | CAGCATGGACTGGGACCTGAA | GAGGCCAGTGCTCGATATATA |
| 64648 | SPANXD | NM_032417 | AAGCCTGCCGCTGACATTGAA | AACCTTGGGCAATGACAATAA | | |
| 64710 | NUCKS | NM_022731 | TACCACGGTTGTAAAGCAATA | CCCGACCTTATTTGAGGGTAT | ACCCTAGAATTTAATCAACAA | AAGGTGTACCACGGTTGTAAA |
| 64711 | HS3ST6 | NM_001009606 | CCGGCGCCTGCAGGAGTTCTA | TCGCCTGGTACCGGAGTCTGA | | |
| 64714 | PDIP | NM_006849 | CACTGTGGTTCTCTTCAAGAA | GCGCCTGGTCACGGAGTTCAA | CTGCGCTTGGTCAACCTTGAA | GAGCTGTGCATTGTGAATAAA |
| 64747 | FLJ14153 | NM_022736 | CAGGTGAAGTTATTAAATTAA | AACCTGGTGTTTGGACTTCAA | | |
| 64748 | LPPR2 | NM_022737 | CCGCGTGGCCGAGTACCGAAA | CTGCGTTGTGCATAACTTTCA | CCGGGACAACTTCAGCCCTTA | CCCGTGTCTAAGCATGTGCAA |
| 64759 | TENS1 | NM_022748 | AAGGCGGATATTTCAAGAGAA | ACGGTCTCTACCCATGTTTAA | CGGTGTGATTGTGGACTACAA | CAGGACCTCGATATTATCGAT |
| 64768 | C9orf12 | NM_022755 | CACGCATGAGATGAAGCATAA | CAGCTTTGTTTAAAGATACAA | | |
| 64772 | FLJ21865 | NM_022759 | CAGGCAATTAATTAGGAGTAA | CGGCTTCTTCACTAACTATAA | | |
| 64779 | FLJ12998 | NM_022764 | CAGCCTTACATTTGCTCATTA | CAGGTCGTGGACATCCCTGAA | | |
| 64782 | FLJ12484 | NM_022767 | AGGGTTGTAATGAGCACTAAA | AACCATTTGTTCTAGAATAAA | | |
| 64783 | RBM15 | NM_022768 | AAGGTGATAGTTGGGCATATA | TTCGGTGATGTAAGTGTGAAA | | |
| 64785 | FLJ13912 | NM_022770 | TAGGAGCTGGTTGACCTTGTA | AAGAATCACTGCCATATAATA | | |
| 64805 | P2RY12 | NM_022788 | TCGGTCTAGTCTGGCATGAAA | TAGTCTGGCATGAAATAGTAA | CCGGAGTAAATCAAACTTTAT | CAGGGTCAGATTACAAGAGCA |
| 64838 | FNDC4 | NM_022823 | CTGGGACGTCCCAGAAGGCAA | CCAGGTGACATCACAGTGGAA | | |
| 64839 | FBXL17 | NM_022824 | CTGTCTGAACTGGATCATAAA | AGGCATGATCGTCATAGCTAA | ACGAAAGTTAATTATACATTA | ACGAGAGGGTAGTCACATTAA |
| 64840 | PORCN | NM_022825 | ACAGATGATTGTGGCCATGAA | CTGGATATCCTTCCACAGCTA | | |
| 64850 | AGXT2L1 | NM_031279 | CACGACAACATTGTTGAGTAT | ACCAACTTGATAGGATTCTAA | CAGAATAAACCAAGTGATAAT | ATGTGTCAAATTAGAGAGCAA |
| 64853 | FLJ12806 | NM_022831 | CAGGTCATTAGCTTACTATAA | AAAGAATATTCTTACATATAA | | |

Table4

| | | | |
|---|---|---|---|
| 64854 | USP46 | NM_022832 | CCGGGAGGAATGTGTTGGCATA CAGCACGGCATTGTTCCTTAT TAGGGAAATGTTTGTACTATA CAGTTATTTAATCGAAGTAAT |
| 64856 | WARP | NM_022834 | CGGGACCGCACTGACAGGAAA CACTAACATCCCAGACTTTAA |
| 64924 | SLC30A5 | NM_022902 | CTCACTCTTTGTGGACCACTA TCGAATCATATGATCTCCTAA |
| 64926 | FLJ21438 | XM_029084 | CACAACCTAACAAGCAATGAA CAGTATGAAGTTGCCCAGTAA |
| 64943 | FLJ12442 | NM_022908 | CCGCGTGGACTTCACCTTCTA TAGTCTTCAATCAAGGTTTA |
| 64951 | MRPS24 | NM_032014 | CTCAAAGGTTGTGTATAAGTA CAGTGAAACTTTGCTGTCCTA |
| 64965 | MRPS9 | NM_182640 | AACAGTCATACTATTCATTAA CCGCAGAAAGTTTACGTGGAA |
| 65009 | NDRG4 | NM_020465 | AAGGGTCAGGTAGAAGAGAAA CCGCAGAGACCTGGACATTAA |
| 65055 | C2orf23 | NM_022912 | CTGAGCCGAAATATAAGAATA CACCAATACCTAAATCTGTTA AAGCCTGTAAGTGTACATGTA CCCAGAATTATCAGAAACGAA |
| 65083 | NOL6 | NM_022917 | CACTACAGACCTGACAGTCAA CACGCACACGATGTCCTTAA CCGGCCGCCCTTGGACATTTA CAGGAGATCCATCCACCTATT |
| 65084 | FLJ22104 | NM_022918 | AAGATGTTGCTTAATAATTAA AAGGTGTATTTCTACAATTTA |
| 65109 | UPF3B | NM_023010 | AAGGTGGTAATTCGAAGATTA CAGAGCATACATCAACTTTAA |
| 65121 | LOC65121 | NM_023013 | GACGGTTAGTTGCCAAATTCA CCCACAGCACTCATTCCTGAA |
| 65123 | FLJ21919 | NM_023015 | CTGCCTGATGATGGACATGAA AAGGATAGCATTATGAACATA |
| 65217 | PCDH15 | NM_033056 | ACCATAGTTGCTATTGATGAA ACACGAGTCAATCTTAATGAA ATCGCTATGTTCAGGAACAAA ATGGATTTAGTAGTATGCTTA |
| 65220 | FLJ13052 | NM_023018 | CCAGACCATCATGCACATTCA CACGCACCTCATGGAGGAGAA CCAGGAGAACATGATCGTGTA AAAGAGCGTCCTTGTCATCAA |
| 65250 | FLJ13231 | NM_023073 | AAGGCTCATCTTAAATTTGTA ACCAGAGAATTCCATAATTAA |
| 65266 | WNK4 | NM_032387 | CAAGGTCACTTCGGGCAGAAA CACTAGTGTCTCAGACCAGAA CAGGAGGAGCCAGCACCATTA CAGCTTGTTGTGGCGTTTCCAA |
| 65268 | WNK2 | NM_006648 | GACGCTGAAGACATACCTGAA CACGTATATGGTGGAGCATGA ACCTATAAGTCTAGTAGCAAA AACGTTCATCGAGCAGATGAA |
| 65975 | STK33 | NM_030906 | TCCATAAGTGACTGTCTAAA CAGTGGTTAACAGGCAATAAA CAGGATGGTAATAGGTTAAGA ATGTGTCTAGTGCATCCTTAA |
| 65977 | PLEKHA3 | NM_019091 | CAGGGTGTATTGTAACCTATA CAGGAATTTGTTCACCATGAT |
| 65981 | C1QDC1 | NM_001002259 | AAGCTTCGAACTATACTTCAA CAGTACAGTATTGACAATAAA |
| 65983 | NS3TP2 | NM_023927 | CTCGGTAACCCTAATAAAGAA CAGGTTTGCTTCAAACAAGTA |
| 65987 | KCTD14 | NM_023930 | CTGCCTGGAGATGGACATTAA CAAGGTATTCTCCAAGTTCTA |
| 65989 | EGFL9 | NM_023932 | AAGTTCTGTGACAAAGATGAA CAGCCTCACACCAGAAATATT AACCTGCCTTGACGGCATAAA AAGACCTGTGAGCTTGTCTTA |
| 65990 | MGC2494 | NM_023933 | CTGAGTATTAGACACGATAAA CAGGATCGTGCTGGCGGCCCA |
| 65999 | MGC3036 | NM_023942 | CTGCAACTTAGTGGAAGGAAT AAGGGCTGTGAGCAGGTGTAA |
| 66000 | MGC3040 | NM_023943 | CAGGGAGATCCAGTCCCTTGA CAGGACCAGATGATACCATGA CCCTCTCTATTGCCAACTGTTAA CACTGGGATGGTCCTTGTTAA |
| 66002 | CYP4F12 | NM_023944 | GTCCTATATAACGATCTTCAA ACCTGCCTCATCGATATTATA TTGCAGATTGTCATGAATAAA CCGCAGGAAGCTGGAATTGAT |
| 66036 | MTMR9 | NM_015458 | TACGTTGGAATAGATCCTCTA ATGCCTGAACCTGTTATCTAA CAGACCTAGTATTCTAAGTTA CAGGTCTCAAGGATTAACTATA |
| 78989 | COLEC11 | NM_024027 | CCCAGAATTGCTCTTCCATAA CCCAATGTCATTATGTAATTA |
| 78990 | OTUB2 | NM_023112 | CAGGGAGATCTTCAAGTTCAA CAGCCGATAAACATTGATTAA |
| 78994 | MGC3121 | NM_024031 | ACCAATCAGGTTGAACAAGAA CAGCCTCTGACTCGAGCATTA |
| 79000 | MGC2603 | NM_024037 | CAAGACTAAAATTGGAAAGAAA CAGGAGGATGGGCCAACTCAA |
| 79012 | MGC8407 | NM_024046 | TCGGAGGTGACTGACAGATAT CTCGTGCTGCAAAGAGTGATA AAGGTAGGGCAGTATGTTAA CTGCATGGCAATAACTCATAA |
| 79016 | PCIA1 | NM_024050 | CAGGAGCAAGTGGAGCTGGAA CTGCGCTACCTGCATCAGCAA |
| 79018 | C17orf39 | NM_024052 | GAGGGCAATACTAAACTTAAA CTCCTCAATCCTGAAATTCAA |
| 79027 | ZNF655 | NM_024061 | GTGCAGGACACCGACATGGAA AGGAAGCAGCAGGGTCACCAA |
| 79048 | SECISBP2 | NM_024077 | CTAGATTTGTTTGATAATCAA CTGGAAATTGATCAGCATTAA |
| 79054 | TRPM8 | NM_024080 | TCGAATGTTCTCACCTATTAA CCGGACGAGATGGACATAGA CAGAATGTTATCATACTACAT AAGGTTAGATTCCAATAAATA |
| 79066 | MGC3329 | NM_024086 | AAGGAGGAGCTTCGCATACAA ATGGATGCTCTTAAAGAAGAA |
| 79068 | FTO | XM_051200 | AAGGAGATGTTAATAATTAAA CAGGGTCATTACAGAGATTAA |
| 79073 | MGC5508 | NM_024092 | CAGGTTTGATGTGGAATCACA CACCGCCAGTGTCATCACCAAA |

178

Table4

| ID | Gene | Accession | Sequence |
|---|---|---|---|
| 79075 | DCC1 | NM_024094 | TTGGTTTAGATCAAAGTTCAA AACGAGTTTATAATCCTATAA |
| 79080 | MGC2574 | NM_024098 | CAGCCAGAGTACAGTCCTGAA AGCGTCCAAGAAGTTGAATAA CCGAAAGGCCGCCGAAGACAA |
| 79092 | CARD14 | NM_024110 | ACGGACGGTTATAGAGGCTA ACCAGTGTTTTACTTGTAAA CTCGGAGGTTGGCCCAGAATAA |
| 79094 | MGC4504 | NM_024111 | TCCCATACTGTTACCCATAAA CTGCTTGACACTGACTACTA CCGCATCGTCAGTATGGACAA CACCTGGATTATGAGCTCCTA |
| 79095 | C9orf16 | NM_024112 | CTGGCTTAGACACCTTCTCAA GGCCTTGAGTGTCCACATTAA |
| 79097 | TRIM48 | NM_024114 | CAGGCCCTGTTTCTACCTCAA CTGGGAGAAGCTGTTAAAGAA TTGCATTCTAATGTTATTAAA AAGAATCAGAATGGCAATATA |
| 79143 | LENG4 | NM_024298 | AAGCATAAGCTTCCCATTTCA TCCCAATAAAGTGTTGCACAA |
| 79153 | MGC4171 | NM_024307 | CAGAGTGGTGGTGTGTCACA GACGCTATGACCGTAATGAAA ACCCATGAGCGTAGAGATCAA |
| 79155 | TNIP2 | NM_024309 | CCGGAAGTTAAAGCACACTTA CGGGCTCAAAGTAGGATTCAA |
| 79169 | C1orf35 | NM_024319 | TTGACTCTTTGTATTTCAATA CTGGGAGGACGTGAAGACTGA |
| 79171 | MGC10433 | NM_024321 | CCGCCAATTATCGCGACCAA CCGCTTCCCATCCTTCCTTAA |
| 79176 | FBXL15 | XM_370575 | CAGCCTCTCTCTGGCCGTCAA CTGAGGAAGAAGAGATAGCAA CTGAGCTGTGAGGAAACTGAA CACCCTGGAGCTTCAAATAAA |
| 79178 | THTPA | NM_024328 | CAGGCTTTGCATCCTTGGGAA ACGGAGTATAAGGAACTCACA |
| 79184 | CXorf53 | NM_024332 | CAGCATTTGCAGGAATTACAA CAGATTGAGATGAATTTGCAA TACGATGTTGATTATAAACATT ATGGACCTACATACACACAAA |
| 79187 | FSD1 | NM_024333 | CAGAGGTACTCCATCTCCCAA TCCGGCTATCATTGAAAGCGA CACGGCCAAGCACGCCAACAA AGGTCTCAAGTTTGACATGAA |
| 79258 | MELL1 | NM_033467 | CTCCCGAGAGTACTACTTCAA CTGGACCCGCATTGGTAGCCTA CACGCACGGCTTTGACGACAA TGGCCGGAACTTCGACAAGAA |
| 79369 | B3GNT4 | NM_030765 | CACCCGTTAGCTCTGATTAA CAGGAACACTAAGGTCAAATA |
| 79442 | LRRC2 | NM_024512 | CAGGAGAGACTTGTAGGTACA CCGCAAGATATAGACAGGCTA AAGACCAAATACTGAAGTTAA CTGGAGCTATATATAAAATCTT |
| 79573 | TTC13 | NM_024525 | CAGGGTGATTTCTATAAATCA TCCGATTATGAAGCAGTTTAA |
| 79574 | EPS8L3 | NM_024526 | CAGCTTAGACACCTCCAAGAA CAGGAAGAGAAGAAATTTGGGAA AGCCATTTACTTGCACCGGAA CCGGAAGGAGTACTCCCAGAA |
| 79585 | CORO7 | NM_024535 | CAGCCACATCACCAACCTCAA CTGCAGCTTGATCGCCTTCAA |
| 79603 | LASS4 | NM_024552 | CAGGTTCTGGTTACCACCCAA AAGGACATTCGTAGTGATGTA |
| 79607 | FLJ21103 | NM_024556 | CAGACAGTGTTTAACAAGTAA CAGGAGGAAAGCAGTGACAAA |
| 79629 | FLJ22709 | NM_024578 | TCCCAGTGAAATAAACATGTA ATCCAGAAATTCGATGACCAA |
| 79631 | EFTUD1 | NM_024580 | CACCAAATGAAAGAAGATCAA CCCAACATACTAGTCAATAAA |
| 79635 | FLJ13646 | NM_024584 | CTGGAATACCTGACTAACAAA TCCCATTTAGTATAACTATAA |
| 79640 | FLJ23584 | NM_024588 | CAGGGTCAGTTAGGAAATGAA CAGAAACAATTAAGTCATGAA |
| 79642 | FLJ23548 | NM_024590 | CTGATCTAACTTCTAGCTAAA AGCCATATTGTTCATGTTAA CAGATTGATGAGGACATTCAA CCGATCCATTATCAACATAAA |
| 79654 | FLJ21156 | NM_024602 | ACGGATCTACATCTACCCAGA ACCACTGTACTGAGTGACCAA TCGGGACATGTATGTACCCAA CCCGAGGTATTTCACCTCTTA |
| 79660 | PPP1R3B | NM_024607 | CTAGGATGACATGATGTTATA CAGACAGTACTTAAATGTTA CCCGCTAGATATGCCATTCAA CACCCAAGTTCTTATACGTTA |
| 79671 | NOD9 | NM_024618 | ACCTGACTTGCTGCTATTAAA CTGCGGTTTCTCTGATACCAA |
| 79673 | FLJ12586 | NM_024620 | CGGGAAATCCTTCAACAGAAA TACCTGTGAATGAACAATGAA |
| 79679 | B7-H4 | NM_024626 | ATCGTTCAAGAAGTAGATTAA CTCCAATATGAACAAGATAAA |
| 79684 | FLJ23342 | NM_024631 | TACAGTTGTGTTGCCATTTAA CTGGAAAGATGTTTACTACAA |
| 79686 | C14orf139 | NM_024633 | AAGGAGGAATTTGTTTCTAAA CAGCAGTGTTCAGGGCCTTAAA |
| 79692 | ZNF322A | NM_024639 | AGGGTTGGCAGAATACCAAGTAA TACATATAAATATGTATGAGAA |
| 79696 | C14orf140 | NM_024643 | CAGGAGCAGAAGATACGAAA CTGGTAGCAAGCAATAATAAA |
| 79697 | C14orf169 | NM_024644 | GAGGTCCAGGAAGATACGAAA CAGATTCTAAGGATCCGCGAA ACCCAAGTGCTTGGAAATATA CTGCTTGAGACAGATATTAGA |
| 79703 | FLJ22531 | NM_024650 | CAGCTAGTGTATTTCCATAGAA CAGCATTATGTGACCAAAT |
| 79706 | PRKRIP1 | NM_024653 | AACGATGATGAACACAGA CAGCTGTGATTTCATCCCGAA |
| 79707 | FLJ23323 | NM_024654 | CAGATTAAAACCTGAATATTTA ATGGAGTTTCATGGCCATGAA |
| 79714 | FLJ12436 | NM_024661 | AAGGTTCATTTACATTAATTA CAGCTTAGTCATTCCAGGCAA |
| 79718 | TBL1XR1 | NM_024665 | TAGATACTAGATTGTATTGAA AAGGAGCTATCTGTTTATGTA ATCACAAATGATCATACTTAA AAGCAAGATAACGGCATATAA |

Table4

| | | | Sequence |
|---|---|---|---|
| 79724 | FLJ23436 | NM_024671 | AGCCCAGAATTTGAAGCTCAA AGGGAGCTAGAGAGAACCTTTA |
| 79728 | FLJ21816 | NM_024675 | CAGGAGCGATTACCTATACAAA CTCCTGTAAATAAAGCTATA |
| 79733 | FLJ23311 | NM_024680 | CGAGCAGATTATGATCAA AAGACAAGTCTTAAGGGTAA CAGAGGATGTCCATTAATCAA CACGTGGGAAACCAAACTTTA |
| 79741 | C10orf68 | NM_024688 | AAGCAATTTATAGAACATATA ACCAAGAAACTTCAAGCTAAA |
| 79742 | CXorf36 | NM_024689 | CTGGTTTGAAATGAAACCTTA AACACCTAGATTAGCATTCAA |
| 79770 | C5orf14 | NM_024715 | TACTATAAGGGTTATAGTAAA AACTAGTATTGCAATAAGCAA CTGCATGAACGTAATCCAGTA CCGGCAGAATAGTGAGTAGAA |
| 79784 | MYH14 | NM_024729 | TCCCAGAATCGGGAAAGTGAA CGCGGGCAAGGTCGACTACAA |
| 79794 | FLJ21415 | NM_024738 | CCCGTGGAAAGTGCAGTTTAA AACGTTGAGGCAATTCCTAAA |
| 79797 | ZNF408 | NM_024741 | CAGCCTGGTGCTAATCCATAA CCGAGACATCCTCGCTTTAAA |
| 79807 | FLJ13273 | NM_024751 | CCAACTATACCTGTAGAAATA TTGGTAATTATCAGCAGGAAA AAGAACTCAGAAATCTTAAGGAA GAGGTTTATAGGTTTAAGTAA |
| 79811 | FLJ13213 | NM_024755 | CAGCTTCGTTATGAACCAAGAA CAGAAGGTACTGTTACATTAA |
| 79815 | FLJ13955 | NM_024759 | CACCTTTCAAGTACTAAGTTA CTGCACAAGGCTGACACATAA |
| 79816 | TLE6 | NM_024760 | CTGAATTATCAGGGCATTCTA CACGATCTAGTCTGTGGTGTA |
| 79823 | FLJ23451 | NM_024766 | CAGCCTTGTTGATGCAATAAA CACGCCGAGGGAATACTTTAA |
| 79829 | FLJ13848 | NM_024771 | CTGGATGTTGCTGCATTCTAA AACGAATATGCAAACCATGTA |
| 79830 | ZMYM1 | NM_024772 | CACCACTAATTTGAAGATAAA CAGGAGTTGGTAAATAAACTA |
| 79836 | RNF127 | NM_024778 | ATGGAGGAGCTCATAGCTAAA CAGCGGGATGGCTACAACACA TTCGCTCAAATTATCCCTAAA CAGAGAGAGAAATTGAGTAGAAA |
| 79837 | PIP5K2C | NM_024779 | TAGGATTCATTCTCCATGTAA CTGTCCTGTCTTATACTGAA TTGATGTGTGATAAATCTCATA CCGGAGCAGTATGCTAAGCGA |
| 79838 | TMC5 | NM_024780 | CCGGGCTTATCGGAGTCCAA TAGGACACAGTGATGTACTA CAGGAATGATTGGTTCTTAGA CGCGATTATTGCTAATTGGAA |
| 79841 | FLJ23598 | NM_024783 | CAGCCTACCATCCAGAAGTAA CAGCTTGGAAACGCACCTAA CCAATAGATATTCTCTCTAAA CATGTATTACCCAGAATTTAA |
| 79846 | FLJ21062 | NM_024788 | CTGAAGTTAATTGTACTATAA TTGGAGAAATATGGAATGAAA |
| 79852 | ABHD9 | NM_024794 | ATGTATGTTAATTGAAAATGAA CTGGCATTTCTCCATCTACTA |
| 79864 | FLJ23554 | NM_024806 | CAGGGCGCCAATAACAGGCAA AAGACTTACATCGGATTTCAA |
| 79872 | CBLL1 | NM_024814 | CGCGAACTCAAAGAACTATAA CTCGATCGGTCAGTCAGGAAA CCGCCTCAACATGGTGGTCCA ACCATTATAATCCTAACTCAT |
| 79875 | FLJ13710 | NM_024817 | CAGAAGAGAGAAATCTTGTA ATGAATATTGTGCTTTATTTA |
| 79884 | FLJ21159 | NM_024826 | ATGGAATATCTTAAAGAGAAA AAGGATGGACTAGAAGATAAA |
| 79896 | THNSL1 | NM_024838 | CAGGATGTTGGTAATGAGCAA CAGCTTTAAAGATTAAAGAA |
| 79897 | RPP21 | NM_024839 | CAAGGCAGATTCCAAACCACTA CTCAATGATCCCGGGCATTTA |
| 79908 | BTNL8 | NM_024850 | GTGGACGGAGGACACAATAAA CACGGGATATGTTGATAGAGA CACAGTATCAAGGCAGGACAA CAGGATTAGTTCCCAGTCTTA |
| 79912 | FLJ22028 | NM_024854 | CAGCGAGTTCAGGAGACTCTA CTAGATCCAAATATTGATATA TAGCAATATCTCGTTTGCTAA AAGAATAATGATCATAGGGAA |
| 79923 | NANOG | NM_024865 | ACCGAGTGTTTCGATGAGTAA TTGGTTTAAGTTCAAATGAAT |
| 79924 | ADM2 | NM_024866 | AAGGGCTCCTTCTGCTATTTA CACTGAGCATTTAATTTACAA CTGGTACGTCAAGGAGCCTAA TAGCGAACACTGAGCATTTAA |
| 79943 | FLJ14129 | NM_030895 | CAGGCCTTTGTAAGTTGTCAA CAGTGCTAAAGAGAGCAGTA |
| 79953 | C20orf39 | NM_024893 | CACCGCATTAGCAAATATACAA CCGGCCCAACATCATCCTCTA |
| 79958 | FAM31C | NM_024898 | CAGCTCAAACACACCAATAAA TACCTTGGACACAGACCTTTAA |
| 79960 | PHF17 | NM_024900 | ACTGACCAATGAAGAATTTAA AAGCGGGAGCAGGAGATGTCTTA CGCCCAGAGCAAGAAATGATA ACCGATTAAATTCATTAGTGT |
| 79962 | FLJ13236 | NM_024902 | CAGGTGATAGTTGGCATCTAT CAGCTTGAGGGTCTAAGGATA |
| 79963 | FLJ14297 | NM_024903 | CACGAAGTAGAGGAACTCATA CCCTAGTGTCTGGAAATGAA |
| 79968 | FLJ12973 | NM_024908 | AAGGGATTATCTAGCATTAAA CAGGATGGAGACAGTGATGAA |
| 80006 | FLJ13611 | NM_024941 | AAGCTTAGGCTGTTAAATAAA CAAGAGCATATTTGCAACCAA |
| 80013 | C10orf97 | NM_024948 | TCGCTCTCCTTCACTAAATTA ATCACAGTTACTATTGATAAA TTGAATGATTCCGGCTTGTAA ACGTTATTTATTATGATCCTA |
| 80028 | FBXL18 | NM_024963 | CAGCACAGATCTGATTCTGAA CCTGCAGCACATGAAATTCAA CGCGCGGCCTTTGGCAAGAAA CAGCCAGTTTCTGTGTTTGA |
| 80036 | TRPM3 | NM_020952 | CAGACTAGAGGAATTGTACAA ACGGCCTGAAACTCTTCATAA ATCATCGTGTATAGACATCTA CAAGGTAACAGTCATAGTTCA |

Table4

| ID | Gene | Accession | Sequence |
|---|---|---|---|
| 80045 | GPR157 | NM_024980 | CTAGAGATGATCTTGACTTTCCA AAGACCTTACTTTGCGATTCA CACCTGGGTGTCACACGGCAA AAGGGCTTCTTTGATAAAGAA |
| 80069 | C6orf208 | NM_025002 | TTGGAGGTTGGCAGAATTCTA CAGCAGGTGGATGAGACACAA |
| 80086 | TUBA4 | NM_025019 | CCCAACCTACCACCAACCTCAA AAGGAGTTCATCGACCTGCTA AGGCTTTAAGGTTGATATCAA CTGTGTGTCGTCCTACATAA |
| 80094 | FLJ14107 | NM_025026 | CACAAATTCCATTATTGTCAA CCGCCACACCTTCCTCCATAA |
| 80099 | FLJ21075 | NM_025031 | AAAGATAAATACGGTCCTGAA TTCCACTTAATTTCACTATTA |
| 80110 | ZNF614 | NM_025040 | CTGAGCTTAATTGTACATTAA CACCACAAAGTCAGTACTCAA |
| 80115 | FLJ22582 | NM_025045 | CAGGTCCACCATGGCCATCTA TTGTAAATGATGAGAAATAAA |
| 80118 | FLJ22684 | NM_025048 | AAAGAAATATTTAGTAAATTA CTGGCCTAGGACCTTAAATAT ATGGGCTAATTAGAATTATCA AAAGCATATGAAAGAATTCAA |
| 80122 | FLJ23074 | NM_025052 | GTGGTACATCGCGATATCAAA CGCGATATCAAAGGAAATAAT CTGACCAGTAAGAAACTGAA ATGTCTAATTGGAGATTTCAA |
| 80127 | C14orf45 | NM_025057 | CACAAGCTGCTTTCAATTTAA CAGGAGTGATGCATCCAGAAA |
| 80129 | C6orf97 | NM_025059 | CCGATCCAAGATGCTTTCTAA CACCAATGAACTGAAGATTAA AAGCTCTAGCATGAAATTAAA CAACCGTTCGTGAGTCATCAA |
| 80139 | FLJ14299 | NM_025069 | ACCGGACTGTGTATTTATTTA CTGGGAATATAAGTGCATTAA |
| 80145 | NIF3L1BP1 | NM_025075 | CAGACAGGCATGAGACATTAA AAGCATGGAATCAGATCCTAA |
| 80146 | UXS1 | NM_025076 | TAGAATTTGCTCAGTTAATTA CTGGAGGAAGTTAAACAAA |
| 80161 | CXYorf2 | NM_025091 | CACACTGGAAATCGTGCAGAA TGCACTGATGATAACTTCAAA |
| 80167 | FLJ21106 | NM_025097 | CAAGTGAATATTGAACCTTAA ATGGATGAATGTACTCATGTA |
| 80184 | Cep290 | NM_025114 | CCGGAAGAAATGAAGAATTAA AACGATCATTATCAACTTCAA |
| 80194 | FLJ21749 | NM_025124 | CTGCTTCTGTGCCTTAACTTAT CAGCATTGATGATGCCTTCGA |
| 80195 | C10orf57 | NM_025125 | TAGAGGAGGTGCAGTAATTTA CCAGTTGTTACTTAAGAAAGA ACCCTGAAAGATTATGAACTA AACCATAGAGTTAGGCATCAT |
| 80207 | OPA3 | NM_025136 | CCTGATCAGTGTTAACCTTTA CTGAATCAATGTGTTATTGTT |
| 80209 | C13orf23 | NM_025138 | CACCTGGGAAAGCGCCTTCAA ACGGTGCAAGAGAATACTTGA AAGGAAGACCTAGCCGCATAA TACAAGTAAGACTTAGGATAA |
| 80210 | FLJ12584 | NM_025139 | CCAGATAGAATTCATCATCAA TCGGATCTTCTTGGCCATGAA |
| 80213 | BLP2 | NM_025141 | CTCAGCTTCAGTCATGTGTAA TCGGGCGGTGAAAGTACTGAA CTGAACTATGCTAATCTGGAA CAGAATGTTTATGTCTAGTTA |
| 80216 | ALPK1 | NM_025144 | CACCCAATTCATCGTAATCAT CCGCAGGTGGTTATTCGCCAA CAGGCCCTACATTTAAAGCTA CAGCCCTTGGTCCTATCTGAA |
| 80217 | C10orf79 | NM_025145 | AAGGACCAAATTGAAAGGCAA CAGACTGAAGTTGAAAGATTA |
| 80222 | TARSL1 | NM_025150 | CAGGCCGAACAGGTCCTTAAA TCGCCAGTTGTTCAAGGATAA |
| 80223 | RAB11FIP1 | NM_001002233 | ACAGAAGGAAAACGATAAGCAA AGGGCTTAATGTAGTTAATAA CAGGTTAATGATTACAATTAA CACAGAGTTCGGAATTCACAA |
| 80233 | FLJ22175 | NM_025161 | TAGGAGCAGAACGCTCATGAA AAGCCTCAACGAGGCCATGAA |
| 80274 | SCUBE1 | NM_173050 | CAAGCTCTGGATCCAGTTCAA CAGGTGCTACAGGACAGCAAA CCCGGCTTTGACCTTGCCCAA |
| 80309 | SKIP | XM_051221 | CACCAACATGTTAATTCATGA AAGAAGATGAGTCATATTGTA GGCAAGTATGCTACAAATTTA CTGAAATTCTGGACACCTTAA |
| 80313 | LRRC27 | NM_030626 | CTCCGGTACAATAGAATTAAA CTGCTTTCTGATAGTGATCAA |
| 80318 | GKAP1 | NM_025211 | CTGCAAGTTGATGAATCACAA TAGCTCCCTAATGGTCTATAA CATGCTGTTTGTAACGCTCAA AACGCTCAACATGATCTTCCA |
| 80329 | ULBP1 | NM_025218 | AACCTGTATATGTACCTTGTA ACCCTACAATAAACACTGTA TATGATGATAATGTATAATA TCGGGATATACCCACCAGGAAA |
| 80341 | BPIL1 | NM_025227 | CTGCACATTGGGAGTCCTTAA CTGGTGCAAGAACCATTCAA CAGGCGAATTCTCATTTCAA ACCATACTTAGTCATCACCAA |
| 80342 | T3JAM | NM_025228 | ACCCAGGACCTACAAGATCAA CAGGACCTACAAGATCAACTA TAGCAGGGACTTACAGATGAA CAGACTTGGCACCACCATTAA |
| 80343 | C20orf50 | XM_046437 | AACGAAATCAAACAATATTTA CAGGATTACTACAAAGCATTA |
| 80346 | C8orf20 | NM_025232 | TCGGTTCCGTGCAACTTTCAA CGCGTACATCGTGCAGGCCAA CCCTTTCTACTATGAGATCAA CAGGGATGACCCACCAAATGTA |
| 80347 | COASY | NM_025233 | CCGGGTGTTTGGGAATAAGAA TCCGATTATGGGATAGGAGAA |
| 80381 | B7H3 | NM_025240 | AAGAAGATGATGGACAAGAAA CTGGCAGCTGACAGATACCAA CTGAAACACTCTGACAGCAAA CAGCATGACCCTGGAGCCCAA |
| 80723 | TMEM22 | NM_025246 | CAGGACTACCAGGAAATACTA CTGATTATTATTGTCTCATTA |
| 80725 | SNIP | NM_025248 | CCGGACAACAAGAAGAGAGAA CCGGGTCATCTTGGAGTATAT |
| 80736 | C6orf29 | NM_025257 | CCCGGACAACAAGAAGAGGAA CCGGGTCATCTTGGAGTATAT |
| 80740 | LY6G6C | NM_025261 | CAGGACAGCAATGCCTGACAA TAAGCTGGGTCTGACATATAA |

Table4

| | | | | |
|---|---|---|---|---|
| 80742 | PRR3 | NM_025263 | ACCCATGACATGTCTTATAAA AAGCTGGTCTCTTATCAAGAA | |
| 80755 | MGC2744 | NM_025267 | ACGGCAGTTGCTGACCATCTA CTGGCTCATGTGGAACATTAT | |
| 80759 | C6orf148 | NM_030568 | CTCCTTCATTCCTACCGTCAA TTGGCAAAGCAATCCAATAAA | |
| 80761 | UPK3B | NM_030570 | CTCCTTCATGGCAAGCGCTA CTGGTTTCTCCCTCTCCAA | |
| 80762 | NDFIP1 | NM_030571 | TAGGATACAAATGAAGCTTAA AAGTCTTTAATTGGTAAATAA | |
| 80776 | MGC4093 | NM_030578 | TCGCGGAAGTATCGCGGCTTA CCAGCGGTTTCTCGAAAGTA | |
| 80789 | KIAA1698 | NM_030628 | ACCCAGTTATATGCAGGGCTA TCAGCAGGAAGTGATCTATAA | CTGGGTAATATGCATGAAGTA CACGCGTACATTAATGGACAT |
| 80824 | DUSP16 | NM_030640 | AACCCAGTTGTTACTCTCTTA CAGGACAAAGTGTTAATTACA | CCGGCCATTTGTGGAATACAA AAGGTTGTAGTTTACGATCAA |
| 80831 | APOL5 | NM_030642 | GAGGAATAAATTGGTCTGAAA TGGGAGAAGGTTGTAAAGAAA | |
| 80832 | APOL4 | NM_030643 | TTGCTCATATATAGTAGACAA CAGCAATTGTTCAGGGAATAA | |
| 80834 | TAS1R2 | NM_152232 | CACAATTTGCATCTCCTGTAT GGCATTTATCACGGTACTCAA | CAGCCGTGTACTCTGCCGGTCTA CACACTGAAGGATGAATATGAA |
| 80835 | TAS1R1 | NM_138697 | CACTGTGGCGTTTAATGACAA CTCCGGGCTGCCAATAAAGAA | CTCCATGAGTTCTGCCTACAA CAAGGACACTGTGGCGTTTAA |
| 80864 | EGFL8 | NM_030652 | CAGCACTTACAGGACCATGTA TCCCTCAGAGAGTCAGGGA | |
| 81061 | OR11H1 | NM_001005239 | CAACATATGCACTGACTATAA ATCCTATACTCTTGTCCTGAA | |
| 81127 | OR4K15 | NM_001005486 | CAGCAGTTACTCAGTTGACAA CCAGTTGGCATTCACTGTTAA | |
| 81282 | OR51G2 | NM_001005238 | CCCAGTTCTCTCCACATTCTTA CAGGCAACTGCACAATTCTT | |
| 81285 | OR51E2 | NM_030774 | ATGGTTAGCTGCACCATACAA ATGAGAGATAACCTTGCCCTA | TGGCTGGAATAGTAAACTAAA ACGGGGTGCTGGCTATGTTCAA |
| 81300 | OR4P4 | NM_001004124 | CACAACATTCTCAGAAGATAA CCACTTGTAGTTCTCATGTAA | |
| 81488 | GRINL1A | NM_015532 | CAGGGAGATACCGAGAAGTAA CAGCAATTTGATTATGTATTA | CCGGGACTTAATTAAATACAA ATATTTGAATATTGTGTTTAA |
| 81494 | CFHL5 | NM_030787 | AAGAATTTAATCATAATTCTA AAGGGAAATTTGAATATCCTA | |
| 81502 | HM13 | NM_030789 | CAACCTCCTGCTGTCCATGTA ATGCTTCAGACATGCCTGAAA | |
| 81544 | PP1665 | NM_030792 | CCGGGACGTCAGCATCATGAA CAGGCCTGCCTCCATGCTTAA | |
| 81553 | FAM49A | NM_030797 | CACCTAGACATTGAGAATGAA AACAACATCTATAACGATAAA | |
| 81554 | WBSCR16 | NM_030798 | CTGGTGTTAAGGGTCACGCAA CACAGGAGCCGGAAAGATAAA | |
| 81557 | MAGED4 | NM_030801 | AAGGATGTCATCAGAGAATAT CCAGATGTTAGTCACCAGTAA | |
| 81563 | C1orf21 | NM_030806 | CACGTTCTTTATGAAAGGCAA CCACGTGTATCTGAACATTAA | |
| 81565 | NDEL1 | NM_030808 | CAGGCTGAACAAGGAAATAGA AAGACTTTGAACAAAGGCTAA | |
| 81566 | C12orf22 | NM_030809 | CTCCATCTTGATGATATATAA CTGCGTAGTGCTGAACATTAA | |
| 81577 | MGC11335 | NM_030819 | CTGAGAAATGTTAATAAAATAA CACAGTGACACTCAACTTCAA | |
| 81607 | PVRL4 | NM_030916 | CTGCTTTGAATCCTTTACGAA CTGCTTGACCTTTACCTCCAA | CCCTGATAGTCTCAAGGACAA CAGAGCAGTATTAATGATGCA |
| 81608 | FIP1L1 | NM_030917 | ACCTGTAAATCTTAACATCAA CAGGGAAGAAACTGGAAACTCA | |
| 81617 | CAB39L | NM_030925 | CTGAGAGATCCCAGAAGGTAA GCCAATGTCTTTGGACTGTAA | |
| 81620 | CDT1 | NM_030928 | CCGGGCCAGAAGATAAAGAAA CACCTGGTGATTCACATTAA | |
| 81622 | UNC93B1 | NM_030930 | CTGCCCGACATGCACAGCAA CCAGGATGCGCAGAAGTA | |
| 81693 | AMN | NM_030943 | CGCATTTGACCTGGACGGTA CCAGGTGGCTGGTGGTGGAA | CAGCCACAGTTACTTCGTCAA CAGGGTGGCCTTACTCAGTAA |
| 81706 | PPP1R14C | NM_030949 | CCGCAGAAGAAGAGTGTATGA AACCCTTAATTGAAGAACTA | TAAAGTTGTATGAACCCTTAA AAGAGCTGCTTTCTCGGATAA |
| 81787 | DKFZP547L112 | XM_375153 | CAGGGCTGTACCCTGGACTGA CTGCCAGGCCCTAGACCAAGA | |
| 81792 | ADAMTS12 | NM_030955 | CCCGGAGTGATCTATGATGTT CCAGTGGAACGGGAACTATAA | CCGGAGAGTTCTGAAACCAAA CAGGAAAGACGGCAAACCGTA |
| 81794 | ADAMTS10 | NM_030957 | CGGGCACACATTCGGCATGAA CAGGACTTTGTGTACCCGACA | ACGATTAATGCATCTCTCAT CCGGAAGTTATTATTGGGAA |
| 81797 | OR12D3 | NM_030959 | CCGGATAATGGCCATCATGTA TACATTACTGTCCCTGCATTA | CCCAAATAACCAAACGCGTAA CACAATCAAGCTAAACCTACA |
| 81833 | SPACA1 | NM_030960 | CTGGATAATAATGAAATAGTA ATGAATGATGTTTGAATGATA | |
| 81844 | TRIM56 | NM_030961 | TTGGCTAAGATTAAGTATTAA CAGCGACTTCCTGGCCTGTAA | |
| 81858 | Sharpin | NM_030974 | CAGGAGGGCCTGGAACCCTCA CCCGGAAGCCTCCACACTCAA | CAGGCTGCAGGTCACACTTGA AGGCTGCAGGTCACACTTGAA |

Table4

| | | | Sequence |
|---|---|---|---|
| 81875 | FLJ12671 | NM_030980 | CAGGGCATACTGGGAAATGTA CAGACTTAATACCCATTGAAA |
| 81930 | KIF18A | NM_031217 | CACATGAAGAGAAGTATTCAA ATCCGTCTACAGTAACCTTAA CAGGTGGAACTAATCTGGTTA CAGGAGGACTTGGACTCTACA |
| 81932 | HDHD3 | NM_031219 | ATCGCTGAACAGCTTTATAAA CAGTGTTATTACCTCCAGTAA |
| 83259 | PCDH11Y | NM_032971 | CAGGAGATTATTCACCTCAA CAGGCAGATGATAATGATGAA |
| 83394 | PITPNM3 | NM_031220 | CAGCAGTGGTCGCATCACATA CTCGCGCATGATCCTGCGCAA CACGAAGGACATCTCTGTCTA CCGGGAGGAAGTGGCTTCGTAA |
| 83401 | ELOVL3 | NM_152310 | CTGGGTCTTCTTCTCAGCAA ATCGATAATTCCACAGTCAAA |
| 83445 | GSG1 | NM_031289 | AAGAGCAGTGTTAGGAGTTAA CAGGATGGTGCTGGAGTTCAA |
| 83446 | DKFZP434K1172 | NM_031290 | CCAGAGAAACTAATGCACAA CGGGTCGTGATAAGGAAGATGTT |
| 83449 | PMFBP1 | NM_031293 | CAGGATGATCTCATTCAAGAA AAGGGTGATCATTCAAAGGTA |
| 83468 | GLT8D2 | NM_031302 | CCCGATGGTCCTCAAAGGGAA CTGAGAGAAAATAAACTTTAAA |
| 83479 | DKFZP564B1023 | NM_031306 | AAGGTCAAACTAGGCCTTAAA CAGGCCCATTATTGACTTTGA |
| 83482 | SCRT1 | NM_031309 | TCGAATAATAATGACACATAT CCCGGCTTTCTCCCAGCAATA |
| 83539 | CHST9 | NM_031422 | CAGAATCTATGTAGAAGATAA AAGGCATTATGTGATTTACAA |
| 83549 | UCK1 | NM_031432 | TCCCTCTAGGTCACTGAGAAA AAGGCTGGTTGTGGCCTACAA TGCGGTTTAAAGATCCCTCTA AACGGTGGAGGTGCCGACCTA |
| 83552 | MFRP | NM_031433 | CAGGGAGTTTGTGGTCAGCGA CGGACACAGCATAGAACTACA CAGCATAGAACTACAGTTCCA CTGCTCCTTCCTGTCCCTTTA |
| 83650 | AMAC1L2 | NM_054028 | CCGCACTCCAATGAGGTCACA CAGGAGAGTTCCAGGGAAGAA |
| 83660 | TLN2 | NM_015059 | CTCCTTGAATAACTGCGTAAA CCAAGTGATAGGGAACCTCAA CAGATGTTGTATGCAGCCAAA CTGCATGATCGTGATGTCACA |
| 83690 | LOC83690 | NM_031461 | CTGGTGGATTTACATATTAAA CTGCTGGTGGATAAACATTAA |
| 83698 | CALN1 | NM_031468 | CCGGATGACCTTTGTAAGTTA CAGACTCAGGTCATAAATCAA |
| 83706 | URP2 | NM_031471 | CCAGGAAAGTTCAAGGCCAA CGGCCAGAAGTTCTGCATTAA |
| 83707 | MGC11134 | NM_031472 | AAGGAGGAGGATCCAACAATA CTGGCCATCCATCCTACTCAA |
| 83729 | INHBE | NM_031479 | AAGGGTTAGGGTGATGGTCCA TGGCTTATACTTTCTTAATAA AGGGCTGTTGAGGTACCTTAA TAGGGTGATGGTGCCAGAGCAA |
| 83733 | SLC25A18 | NM_031481 | CCCACTGTTTGCCAACCTTAA CAGCACCTAGTTTGAGACCAA |
| 83743 | GRWD1 | NM_031485 | TCCCAGGAGTTCCAGGGTAA GCGGATCTCATTAGGCGGAAA |
| 83844 | USP26 | NM_031907 | TACCTATAATATAGAAAATCAA AAAGATTTGTATGAAGATAAA |
| 83849 | SYT15 | NM_031912 | GCGGCGCTTCCTCCAATCCAA CAGCCCATGCTAGGACCCCAA |
| 83855 | KLF16 | NM_031918 | CTGCCTGGCCTCATCCTTGTA TTGTATTAATTAATTAAACA TGGCCTCATCCTTGTATTAA TCCTTGTATTTAATTAATTAA |
| 83861 | RSHL2 | NM_031924 | CAGCGTGAGTATGAAGAACTA AAGGCTATATTGTAATTCTAA |
| 83862 | TMPIT | NM_031925 | CTGGTGGGTGTTCCATCACTA CTCGCCCTGAAGAAATGCAAA |
| 83872 | FIBL-6 | NM_031935 | CTGCAGATTCTGAATACTCAA CCGCCTGACATTACATGGCAT TAGACTCTAAGCAATTGCCAA CCGAGTATATGTTCCGCCAAA |
| 83873 | GPR61 | NM_031936 | TGGATTTATGAGGAAACTGAA TACCCAGAACTGGGACTACAA GTGGGATAGTCTCCCATTTAA CCCATTTAAGCAGCTAATAAA |
| 83876 | MRO | NM_031939 | CGGGATAACAATAGTAGGTAA ATGGATGGTTAGAAATGTTAA |
| 83877 | BLP1 | NM_031940 | TTGGTGGTTTGTTGACCTTAT ATGGCCGTGGATTGTAATACA CACTACTTCATAACCACTTTA TGGATTGTAATACACACTGAA |
| 83879 | CDCA7 | NM_031942 | CAGAGTAAAGAAGAACTTAAA CACAATAATAGTATTAACTAA |
| 83884 | SLC25A2 | NM_031947 | TACAATTTGGTCTGTCGTGAA GTCGTGAAGGGTATCCTTAAA TACCCTAAATCTTAACTTTAT CCTATGTACCTAATCTGAAA |
| 83886 | MPN | NM_031948 | ACCAGTGCCCTTCACCAATTA CCCACCAGACTCATTTGTAAA AACCCAAAACCATCAAGAATG AAGTGCCCTTCACCAATTACA |
| 83893 | NYD-SP12 | NM_031955 | CAGGTAGAAATCACACTTGAA CAGAATTGCTGCAGTCACTAA |
| 83899 | KRTAP9-2 | NM_031961 | CCCTGGTAGCAGAAATCCTTA ATCGATGATCTTTGCAATCTT |
| 83903 | GSG2 | NM_031965 | TTCGGGCAGAATGGGAAGAATA CAGGCTCATGAAGAAGGAGAA ACAGTCTGTTTAAGTAAGCTA CAGTCTGTTTAAGTAAGCTAA |
| 83933 | HDAC10 | NM_032019 | CGGGTTCTGTGTGTTCAACAA CCGTTAGTAAACATCGCTCAA CGCCCTTGCGCCGTTAGTAAA CAGGTGAACAGTGGTATAGCA |
| 83938 | C10orf11 | NM_032024 | CAGAAAGTAACCAGACAAGAA TTGCCTTGAAATGAATGGAAA |
| 83941 | BBP | NM_032027 | ACGCTACGCAAGAACCAGTTA AGCACCCAACATAACTTGTAA AAGTAGTTACATTATAGATTA ACGCAATTATATCCATAAATA |
| 83954 | FKSG83 | NM_032030 | TGGGCATGGCTCAAACTCAAA AACGTGTGTCTTTCCACTTAT AAGACTGTTTATATACTACCA ACGTGTGTCTTTCCACTTATA |

Table4

| | | | Sequence |
|---|---|---|---|
| 83956 | FKSG42 | NM_032032 | AAGGAAACGAACTCCAATTTA CAGATTTGGGAGCAAAGGCAA CAGGTGGATGCAGACATCCAA AAGGAAGGAAACGAACTCCAA |
| 83959 | SLC4A11 | NM_032034 | CCGAAAGTACCTGAAGTTAAA CCGGGAAATCGAGATGAGCAA |
| 83983 | SSTK | NM_032037 | CGCAGTCACTTCACAAGCCAA CAAGGGTACCGTGGCCATCAA CCGGTTGGAACCTGCAATAAA ATCGAGGTGTGCAACGGGAAA |
| 83990 | BRIP1 | NM_032043 | TAGCATGGCAACAATCTCTTA CAGGCCCTTGGTAGATGTATT AAGATAAACAGTCCACTTCAA CCTGAACTTTACGATCCTGAA |
| 84000 | MSP | NM_032046 | CTCAGTGTTTCAGAAACATA CAGCAACTGGAATGACTCCTA CTGGAAATTACCTGGAGACAA ACGGGAATGCATCTCCAGCAA |
| 84034 | EMILIN2 | NM_032048 | ACGGGTCATGATGGAATTAAA CACCTGAAGGACAAAGTTCAA |
| 84058 | FLJ12953 | NM_032118 | CCCTGTGTGTATTCATAAAGTA CAGGGCCAGAATTCACATTAT |
| 84062 | DTNBP1 | NM_032122 | CTGCTGGATTAGAATTACTTA CAACTGGAGAATTACAAGAAA CACCCAGCAAATGAAGCTGAA TTGGCTGTATGCGAATTCCA |
| 84066 | DKFZP564J047 | NM_032126 | CTGAGGGAAGGTGAAGCTTAA CCAGCTTGAAATGAGAGTAAA |
| 84083 | ZRANB3 | NM_032143 | TTGCCAGTGTATGACACCTTA TGCCAGTGTATGACACCTTAA TGGATAGTTCTATAGAATTAA |
| 84106 | PRAM-1 | NM_032152 | CCGGAAGAAGTTCAAGTTTGA CCGAAGCCTGAGTTTGGTAAA CCGGAGCATCAAAGCAAAGTT |
| 84108 | PCGF6 | NM_032154 | TAGGAGAATTTGAGAAGTTTA AAAGATTTCCATGAAATGTAA |
| 84109 | GPR103 | NM_198179 | ATGTCTTATTCTGTACAGTAAA TTGTTATTGCATAGTAAATAA AGGAATTACAATGATGCGGAA |
| 84132 | USP42 | XM_374396 | CAGCCCGTGATGAATGGCAAA CAGGACGAGGCGAAAGCCCAA TTGGAGGATACCTAAGATCTA CACGACAGAACTGCACTTGTA |
| 84152 | PPP1R1B | NM_181505 | CTGAGTTCACCTGCAGTCTA CAGGGATTTGCCCTTCACAAT CAGCTTGTTTGAAGCCCTTGA ATAAATCTTTGTAAATAACAA |
| 84162 | KIAA1109 | XM_371706 | AAGGATGCAGCTTGACTTTAA CAGGTGTGGAGTATAGATGAA CTCGAAGAGATTCACTTTCAA CACCTCTTAAGCGAATGGAAA |
| 84168 | ANTXR1 | NM_018153 | CAGCAGTGTTTGATACCGTAT CTCCCATTATAGGGCAAACAA CTGACAGAAGACAGAGAACAA CCGAGGAACAACCTTAATGAA |
| 84171 | LOXL4 | NM_032211 | CCGGGTGGAAGTGCTCATGAA CAGAGTCAGATTTCTCCAACA CAGGATTGGTCTAAACTTCAA CTCATTATGGACTGCTACAAA |
| 84174 | SLA2 | NM_032214 | CTCGTTGAGACCACCACCATA TCGGACAGTCTAGGTCCTCAA CATCAACAGGTTCTCAATTAA CAGGTTCTCAATTAAAGATTT |
| 84186 | ZCCHC7 | NM_032226 | CAGGAATTGAGCTTCAAGTAA ACAGATTTCCTAAACAAGTAA |
| 84196 | USP48 | NM_032236 | CACAGTTGGTGAATGGTATA ATGGCTGAGATGCGTAAGCAA CCGAAGTATGCGACATAGAAA ACCAGATGCGTTGGTCCATAA |
| 84197 | FLJ23356 | NM_032237 | AACACTATGCTTACTGAATAT ACGGGTCATGTGCGACTCCAA AAGGTCTTGGATACACTTAGA |
| 84203 | TXNDC2 | NM_032243 | TAGCTTCTCATGTGTAAACTA CACCTTGAAGCTCTCAAATAT AGCAGTCATTGCAGAATTAAA CAGAATTAAAGTAAACATGTA |
| 84217 | ZMYND12 | NM_032257 | CAGCATATTAAGGGAATATAA CAGCTTGATGTGTCCATGAGCAA |
| 84220 | RANBP2L1 | NM_005054 | CAGCATGGTAATAAACACTCTA CAGGCGTTCAGTGGAATTAAA CAGAATTATGATAATAAGCAA |
| 84229 | DKFZp434I099 | NM_032269 | CAGGACAATGACAGAGTACTA CACACTGACCATCTCCATCTA |
| 84231 | TRAF7 | NM_032271 | AAGGAGTGCGAGCACATCAAA TCGGAGAAGATCGACCAGCTA GCGGGACGCATCCATGTTAAA CACGCTGGTCTCTCCACACAA |
| 84233 | DKFZp586C1924 | NM_032273 | CAGGTGATTGGATTGTGAAA ATCGGTTTATGTTGGATTAAA |
| 84259 | MGC2714 | NM_032299 | AACATTTATTTCAGAACAATA TCCCACGTGTATACAAGCTAA |
| 84263 | HSDL2 | NM_032303 | AGGGAGAAAGGAGAATTGAA AAAGAGGTGACTTAAGTTCAA ACCCAGTTCATGAATCGCTAA CAGTTCATGAATCGCTAATAA |
| 84265 | MGC3200 | NM_032305 | AAGGGAGAAAGGAGAATTGAA ATAGAGGATGATGACAGTTTA |
| 84266 | SPATA11 | NM_032306 | CGGGACTCTGAGGGAAGTCAA CAGGCGGGAGGTGGCGCCCAA |
| 84270 | C9orf89 | NM_032310 | CAGGATCATCCTCCAGCTGAA CAAGGTGCTGACCATATTAAA |
| 84279 | C2orf7 | NM_032319 | CATGATTATTTGTACTTTCAA CTGGCTAGAACCCGAAACAAA |
| 84284 | MGC13186 | NM_032324 | AAAGATTTCTTGTTTATTTAA AAAGATCAAATTAGCCTTAAT |
| 84288 | MGC12458 | NM_032328 | AAAGATGATCGTAACACTTTA AACGAGTAGCAGTGTAATGAA |
| 84289 | ING5 | NM_032329 | CTGCGTGGACCTTACCACGAA CCGGATCCGAGGAGCAAGTTA AACCGTGAGGGGATGTTAATTA ACCGTGAGGGATGTTAATTAT |
| 84291 | MGC2408 | NM_032331 | CAGACACTATGCCCAAGCCTA CAGCTCCAACTAGATCCAGAA CTGGAGGCCGGTTTATCTCAA TCCGGAGTTACCGGAGCCGAA |
| 84292 | MGC4238 | NM_032332 | CCGGCTCTATTGATTCCAGTA ACGATTTAAATGGATGGCAA |
| 84296 | SLD5 | NM_032336 | CAGCACTTGATCCGATACAAA TCGCCTCATGAAGATAGAGAA |
| 84298 | MGC14817 | NM_032338 | CAAGCTCTACTGACTTTCAA ATGAAAGATGTTCAAGAGATA |
| 84299 | C17orf37 | NM_032339 | TGCCTTTGAGATAGAGATAAA CCCAGGGTAAGTGCCCACGAA |
| 84302 | C9orf125 | NM_032342 | CAGGGTTTAGCTGGCCTGTAA CTGCAGGTATTTCACTGTTTA |

184

Table4

| | | | |
|---|---|---|---|
| 84310 | MGC11257 | NM_032350 | CAGAGCGTGGATGCTCTGGAA TGGCCTCACTTTCCATCATAA |
| 84311 | MRPL45 | NM_032351 | AAGAAATAATTTATCCCTTAA CACGCATATCATCTCTTTCAA |
| 84312 | BRMS1L | NM_032352 | CACAGCATTGATATTACCTCA TAGGTAGTACTCTAAATAGAT CCGCTGGCAACTTTACAGGAA ATGCAAATTCGTACAAAGGTA |
| 84318 | MGC13183 | NM_032358 | AGGCTTTAAGACAGATATTAA CACCAGAGAAATCAGAACAAA |
| 84321 | THOC3 | NM_032361 | CAGCGTCTTCTTGCTGGAGAA ACCAGCTTTGTTGGCATCCAA |
| 84324 | CIP29 | NM_033082 | CAGGCATATCTTGAAGAACAT CTCCTTCTTCTTGGTCACATA |
| 84327 | ZBED3 | NM_032367 | CCTGGTGACTTTGAAAGGATA CCCAGAATCCTTGGGAAATTA |
| 84329 | MGC15619 | NM_032369 | CCCGGCCTTCTCCTTTAAGTA TGGGAGCTCACTAAGAACAAA CAGGTTCCAGGCCATCAGTAA CAGCCCGACAAGAATAACTAT |
| 84330 | MGC15716 | NM_032370 | CACAGCAAACTGCACTACAAA CACACAGTCCCTGGAAGGCAA |
| 84334 | C14orf153 | NM_032374 | AAGGATTTGGAACAAGCTTAA TCACAAATAAATTGTAATTAA |
| 84337 | MGC4549 | NM_032377 | CCCGTGGATGTGTACAGTGAT CTGGACAAGGGAAGCCCACTA |
| 84366 | PRAC | NM_032391 | CTGTCTCTATTTATACAATAA TAGGCCGATGCTTGCTTGCAA |
| 84376 | HOOK3 | NM_032410 | CTGCCAATAATTGATATACAA CAGCATGAGAATAAGATGTTA |
| 84433 | CARD11 | NM_032415 | CTGGATGATGACAGTCACGAA CCCGTTGGACACATGCACCAA CACAGTACTCGCAGTGCTTAA CAACTCGAGATCGATCAGCTA |
| 84435 | GPR123 | NM_032422 | CCGACTTTCCTAGGCAACAAA AAGCCGTGTTCTGATTATCAA CTGCCCAAGGGTAAATTGCTA CACTGTAAAGCTCTTCACCTA |
| 84436 | ZNF528 | NM_032423 | TAGGATTTACACAAGAAGTAA AACGCAGATAATCCTTACAAA |
| 84439 | KIAA1822 | NM_032425 | CAACAAGAACCTCAACTCAAA GAGGATAATCCTGGAGGTCAA CAGGAACACTTGATGAATAAA GCGCATCGACGTGGACCGTAA |
| 84441 | MAML2 | NM_032427 | CCAGTCGAAAGTAATGGCTAA CTCCAAATAAACAATGGACAA CAGAGTTCAATTTCAGCTCAA CAGGATACAGTATCCAATTTA |
| 84450 | ZNF512 | NM_032434 | CAGCACAGTTCCAGAAAGTAA CAGCCAGGAAGTACTACATAA |
| 84465 | MEGF11 | NM_032445 | AAGCAGCTATGTAGAAATGAA CAGCAAGTTTACACTGCTTAA |
| 84498 | KIAA1838 | NM_032448 | AACCCTGAAATTAAACAAGAA CTGAAGTAAAGCAACAAGTAA |
| 84504 | NKX6-2 | NM_177400 | CTCGGACGCCGAGAAGCTGAA CGGCTGCTCAAGAAGCACAAA |
| 84520 | C14orf142 | NM_032490 | CTGATTTATCATGTACCTTAA AACAATCTGCATTGATTACAA |
| 84536 | C21orf67 | NM_058188 | CAGAGGAAGATCAAAGATCAA CAGAAGGACCACAGTAGAGAA |
| 84614 | ZBTB37 | NM_032522 | CAGGGTTACACCAAATCTCAA ATGCAGCATATTATAGACAAA |
| 84630 | TTBK1 | XM_166453 | TGGCAGGAACGAGAAGTTTAA AACGAGAAGTTTAACTATGTA TGGCAGGAACGAGAAGTTTAA CAGGAACGAGAAGTTTAACTA |
| 84639 | IL1F10 | NM_032556 | CCGGATAGCCTCAGTCAGGGA CCATGGCAAGATACTACATAA GCAAGATACTACATAATTAAA AAGGCTCTATACACAAGAGAT |
| 84641 | FLJ14753 | NM_032558 | CACCAGTAAATGGACCATTAA ACGAGTGAAGTTTATATCCAA |
| 84643 | LOC84643 | NM_032559 | CACGGTAGAGTGGGTGGAGAA AAGAGTCTTCTAGCCCTCAAA CAGAGGGATGAATTTATTAAA CTGGTGGAGTACACCATGATA |
| 84645 | C22orf23 | NM_032561 | AAGGGTCTTGCCACCACTTAA CCGCCTTAGGCAAGTATTCAA |
| 84651 | ECG2 | NM_032566 | CTCCATGGACATAGAGAGAAA ATGGAGAAAGTTTCTGTGCTA |
| 84658 | EMR3 | NM_032571 | CTCCGGGATGTGGAATCGAAA AAGGAACGTGTCTCTCTCCAA GTGGTTTAGAGAGATCGTAAA AACGATAGTGTAGCTATTGAA |
| 84659 | RNASE7 | NM_032572 | CCACTTCATATTTGTATAGAA CAGCACGAAGACCAAGCGCAA |
| 84675 | TRIM55 | NM_033058 | TACAAACATATCACACATTAA CAGAACATAAGTGTAACTTTA |
| 84676 | TRIM63 | NM_032588 | AAGGAAGAAGGACACCAGTAA AAGGAGCACGAAGATGAGAAA |
| 84678 | FBXL10 | NM_032590 | CCGGCAAGACCGGGAAACAAA CAGATCTAACAAAGTAGGCTA TAGGAGTGGACTAGAAGTTTA AGGAGTGGACTAGAAGTTTAA |
| 84680 | PHACS | NM_032592 | CTGGGGTTGACTTGAGAAAGTA CAGCAGGTGCTTGCAGGCAAA |
| 84687 | PPP1R9B | NM_032595 | CCGGGAGGTGCGCAAGATTAA CACTGAGGAATTCCAATTCTA CCCGGGAGGTGCGCAAGATTA CAACTCGAAGCTGGTCAGCAA |
| 84691 | NYD-SP18 | NM_032599 | CAGGTATGTGACCACTATCAA CTGAGAAGATTTATTACCTAA |
| 84692 | NYD-SP17 | NM_032600 | ACGGCTTTATTTGTTCCTTAA CTGAAGAAACGTAACCATCAA |
| 84699 | CREB3L3 | NM_032607 | CCCGAGAAGAGTCTCCAGGAA ACGATGCAATCTCACCGTGAA |
| 84701 | COX4I2 | NM_032609 | CTGGAGCTTGGTGCTGAGGAA CCGCTGGGACTATGAGAAGAA |
| 84708 | LNX | NM_032622 | ATCATCCTCGATAGTACTCAA CACGGTGCTTGTATAACTGTA CTCGCTCAGAGAATTAATTAT CACAGCTAACACAGCTCTTTA |

Table4

| | | | | | | |
|---|---|---|---|---|---|---|
| 84720 | PIGO | NM_032634 | CAGGTACTGATGATCTGCAA | TAGGACTTCATTATTCTATAA | | |
| 84733 | MGC10561 | NM_032647 | CACCTGCGGGTGCACCTTAAA | GCCGGCTGGATGTGACTCAAA | | |
| 84749 | USP30 | NM_032663 | CAGAAATAACTCCCAAACAAA | CACACCAGTATTTATCCTTAA | | |
| 84750 | FUT10 | NM_032664 | CAGCGCGAGAGTAGAAGTGAA | TAGGATCATTGCACAGTATAA | | |
| 84775 | ZNF607 | NM_032689 | TAGGTTCATTTCTGTACTTAA | TAGGCTCAATTCATCCTTAA | | |
| 84787 | SUV420H2 | NM_032701 | AACCTTGTTATAAGCAGCAAA | CCGGCGCTATGGGCTGCCTTA | AAGAAGAATGAGCACTGTGAA | AATGACTTCAGCATCATGTAC |
| 84816 | RTN4IP1 | NM_032730 | TTCGAGCATTTCATATGCGAA | ATCATACACTATCCAAATGAA | CACGAGGAAAGACTGTAATTA | ACGAGGAAAGACTGTAATTAA |
| 84833 | USMG5 | NM_032747 | CAGCTGTGAAAGCAACATAAA | TACCAGTTCACTGGTATTAAA | | |
| 84836 | MGC15429 | NM_032750 | CAGGTTACACACTAAAGTAAA | CTCAGGAATCAAGGAACATAA | | |
| 84844 | PHF5A | NM_032758 | ATGGCTAAACATCATCCTGAT | CTGGCACCACCATATCGGAGAA | | |
| 84864 | MINA | NM_032778 | TTGGCTGCGAATGTCACATAA | CTGTTGCTACAAGACGATTAA | TACCTGCGTATCTACTAACAA | AACCAGTATTTAAGTCTATAA |
| 84868 | HAVCR2 | NM_032782 | AACCATCAGAATAGGCATCTA | CAGGCATAATGAATGATGAAA | TTGACAGAGAGTGGTCCCTAA | |
| 84870 | THSD2 | NM_032784 | ATGGAACTCGATATCCAGATA | ATGGAATACATCGGCAGCCAA | AAGGGAAGCATTACTGGGTTA | CTCGATATCCAGATATAAATA |
| 84872 | FLJ14451 | NM_032786 | CACCAAGAGTGGCAAGATAAA | CTGCCATGACTTCCAGAACAA | | |
| 84873 | GPR128 | NM_032787 | CTCGATCAAAGTGCTGTGGAA | AAGAAGATTGTTAGCACATTA | ACGCGAGTGGTTGGACAGATA | |
| 84875 | PARP10 | NM_032789 | CAGGCAGATCATGAACTACAT | TTGAATAAACGTGAACGTGAA | | |
| 84888 | SPPL2A | NM_032802 | CAGCAATAATATTATGTGGAA | TGGGCTGTGTTTCGAAATGAA | | |
| 84893 | FBXO18 | NM_032807 | CCCGCTGTTCATTCCTTGGAA | CTGCATAGCCGTGCTTCTCTA | TAGGGCGGAAGTACCAGTCAA | CACCGTCCTTACCATGAAGAA |
| 84894 | LRRN6A | NM_032808 | CAGCGAGAACAAGATCGTTAT | CCGCTGGCGGCTCAACTTCAA | CAAGGGCAACACAAAGCACAA | |
| 84896 | ATAD1 | NM_032810 | CTGGATTAATTTATCTGATAA | CAGTTGAACAATGATAACAA | | |
| 84898 | PLXDC2 | NM_032812 | TCCAGTGGGATTTGATCGTCAT | CCAAGATAGCACTACATCTAA | ACAGACCACAATTACTATATA | |
| 84904 | C9orf100 | NM_032818 | CTGGATTGTGGGAGAACTAAA | CCCAACTAAACCTCAAATGAA | | |
| 84918 | LRP11 | NM_032832 | TACGTATTTCTAGACCATAAA | CAGAAACTTAGTTGAAGGCAA | | |
| 84925 | DIRC2 | NM_032839 | ACAGATTATCACATTAATTTA | CACAGACTTGAAGGAGTTTAA | | |
| 84926 | FLJ14800 | NM_032840 | CAGACCAGAGATGTTTATACA | CCCTACGATGTATGAAAATGTA | CAGTTTGGGTCAAAGTGCAA | CCGGTTTGTTCTCATGAACAA |
| 84934 | FLJ14827 | NM_032848 | ACCAAGGAAGAAGAACAAATA | CAGCTTTATAGGTGTGACCTA | | |
| 84938 | APG4C | NM_032852 | CACGTAATTGCAGGAAATGTA | ACAGATGAAGTTGACAAGCTA | | |
| 84948 | TIGD5 | NM_032862 | CACAATCATCTCAGTGGCGAA | CCGCAAGGCCTACTCCATCAA | | |
| 84950 | FLJ14936 | NM_032284 | AACCTGGATGTGAGGCATTAA | TCCGACGGAAATAGAATTGAA | | |
| 84959 | STS-1 | NM_032873 | AAGAGAGTTGTTCTTAGGTTA | AACCAAATCATCAGTTATCAA | CCGGCTTATTTGAGTGGACAA | CCGCTTAAGGATGCTAACATT |
| 84961 | FBXL20 | NM_032875 | ATGGCAGTAACTGGCAGCGAA | CAGGACCCATTTACCCAATAT | CTTGGCGAACTGAGTATTTAA | |
| 84972 | MGC15912 | NM_032886 | ATGGATGAGGTCATCATGAAA | CTCGCTGGGTTTATATTTGAA | | |
| 84991 | RBM17 | NM_032905 | TCCGCTGACTGAAATACTTAA | ATGGAGTATGTGGGTATAGAA | | |
| 85013 | MGC13159 | NM_032927 | CAGCTTAGTAATTACATATAAA | TTGCATATGCATAAATTATTA | | |
| 85014 | MGC14141 | NM_032928 | ATGGCTTATCCTTCTCTCCAA | CTGCATCTCCAACAAGTCCAA | | |
| 85015 | USP45 | XM_371838 | AAGCATATATAATGTTATCAA | CAGAGAAAGCATGTTAAATTA | CGGGTGAAAGATCCAACTAAA | CAGGAAATTATCGGAACATAA |
| 85236 | HIST1H2BK | NM_080593 | AAGAGAATCATTTACAAGTTA | AAGGAGGGACTTTCTCTGGAA | | |
| 85285 | KRTAP4-10 | NM_033060 | AAGGGCATCCATCTACATTTA | TACATATTAACTGTTCTTCAA | | |
| 85300 | ATCAY | NM_033064 | CAGCGTCAAGTTCATCAACAA | CTCGCCTTTGTTTGCCAGTAA | | |
| 85315 | C6orf33 | NM_133367 | TAGGAATTTAAGTCCATAGAA | AAAGCTAGAATTGCTTATCAA | AGGAAGCTTATTCTCATATAA | CTAGAATTGCTTATCAATCAA |
| 85317 | BAGE4 | NM_181704 | CCGAGGCAAGTCAAAGCTGAA | GAAGATCAAATTGAAGATAAA | CTGCAGATATTTCATCAAATA | ATGGCTGGAAATGCAAATGAA |
| 85320 | ABCC11 | NM_032583 | CCGGTGTATTTGAATAAACCA | CCGCTCATGATCCAAAGCTTA | CTCCTCCAGTTGGATCATCAA | AGGCTACTTGATGGCTCTCAA |
| 85358 | SHANK3 | XM_037493 | CAGGGATGTCCGCAACTACAA | CCGCAAGTTCATCGCCGTGAA | | |

Table4

| ID | Gene | Accession | Sequence |
|---|---|---|---|
| 85359 | DGCR6L | NM_033257 | AACCCTAGGATAGTCTCATAA CCGCGCTCGGGATGTCGTGAA |
| 85360 | 7h3 | NM_033025 | CTCCATCTTTGTGGATAATAA GCGCCCTCCATAAAGATGAA |
| 85409 | NKD2 | NM_033120 | CTCGTCTGTGTAACCCATAAA CACGCTCCATGACTTTGACAA CAGCCTCCATGCACCATCTA CACGGAGCGCGAGAAACCACTA |
| 85411 | C6orf114 | NM_033069 | TGGAATCAGAATTCAACTAA AACCTCCAATGTGGACATTTA |
| 85414 | PCANAP6 | NM_033102 | CACAGGCATTTAAATATTTAA CAGGCTCAGGGTTAACAGCTA |
| 85417 | CCNB3 | NM_033031 | GTGCAGGATGTCAATATGGAA TAAGGAGTCGTTAGCCTTTAA AGGGCTAAGCATGCATGTTAA AAGGCTGTGTATTACAAGTAT |
| 85439 | STN2 | NM_033104 | CAGATGTACCTTATAATTCAA AAGCTGGATATTCCTCTTTA |
| 85443 | DCAMKL3 | XM_047355 | GAGGAAACTGTTTAACCTCAA CTCGAGAATCACAGAGCGGAA TCGAAGGAGTCAGAGTTCAA CCAGCTAATGAGAGATAAACTA |
| 85452 | KIAA1751 | XM_372193 | CACCTTCAAGCCGATGATAAA CAGGACTTTGTATATTGGCAAA |
| 85455 | DISP2 | NM_033510 | CCGGAACAGTCCGGACTACAA CAAGAACTTCAGTGAGACTAA |
| 85459 | KIAA1731 | XM_374922 | CAGCAGATACAGAAACATGAA CAGCATGAACTTAGTGCTATA |
| 85464 | SSH2 | NM_033389 | GAGGGTTTGCCTGGGAGTTAA CAGGAGTCTTTGAGTATCATA TTGAGGATGAATAAACTAA CAGATTTAGTCTAGCATGAAA |
| 85465 | SELI | NM_033505 | CAGGCATAGTTTCCACTTTAA CACCACTGTGTTGATATGTAA |
| 85480 | TSLP | NM_033035 | GAGAAATTTCTTTGTCTATTA TTGGTGGACTAGTATAGTATA AGCACCTAAACTTACATTTAA CTGGAACAAGTGTCACAATTA |
| 85865 | LOC85865 | NM_033107 | CAGCTTGGTGACTGTAATTAA AAGCCTGAACTTGGAAAGATA |
| 89765 | TSGA2 | NM_080860 | AACATGGTGAATATCGTTTAA CCGAGAGGAGATCGTATCATA |
| 89777 | SERPINB12 | NM_080474 | TAGGCAAAGATGATCGTCATA TACGATCTTGGGTGGAGTTTA TTGGGTGGAGTTTAATGCCAA ACGATCTTGGGTGGAGTTTA |
| 89780 | WNT3A | NM_033131 | CAGGAACTACGTGGAGATCAT GCCGCCGCTACACCTACTTCAA CCCGACTGTGCTGCTCGCGAA ACCGCCATCCTCGCCTCAAA |
| 89792 | GAL3ST3 | NM_033036 | CAGCCGCAAGGTGGACATTAT CTCGAACTACCTGTTGCGCAA |
| 89796 | NAV1 | NM_020443 | CGGGATCATCTAAGAAAGTA TAGGTTGTCATTCAGACTTTA |
| 89797 | NAV2 | NM_145117 | CACCTTTAACGTGGACCATAA CAGCAAGAATGTGGACCTCAA |
| 89822 | KCNK17 | NM_031460 | CCGGGATGTCGTCCAAGCATA ATGGCTGGCCTGATCATCAA CAGCTCTAAGGAAGACTTCAA CTGGGTGCAGCTATATGATTA |
| 89832 | CHRFAM7A | NM_139320 | AACATTAAGATTACAAGTGGA TCCATACAAATGCACAACATA CACCGCAACATTAAGATTACA ATCGGTTAAATTTGATGCAAA |
| 89845 | ABCC10 | NM_033450 | AGGAAGGAGGTTGATTCCTTA ACGGCTGTGGAGGGCGCCTGTA CTGCTGGTACCCGTCAACAAA AGCCCAGATTGTGGTAGATTA |
| 89853 | C9orf28 | NM_001011703 | AGCCCTTGCCAGAAACGTCAA CTGGAAAGACGGCTTATTTAA |
| 89858 | SIGLEC12 | NM_033329 | CAACAGGACATCAGAACTTAT CAGGAAATGGAACTACACATATA CCGAGACTGATTCCTTTAGAA ATGGAACTACATATATGACAA |
| 89869 | PLCZ1 | NM_033123 | CAGAAATACGAGCCTATCGAA ATGGATTCACGTGAATGTCTA CAGCAGACTCGTGTAATTAAA TTAGCTATGCATCGCAATAAA |
| 89870 | TRIM15 | NM_033229 | CAGGAGTAAAGAACTGGCTTA GAGGGATGAATATATCACAAA CCGGCTTGGAGCCCAGGTCAA ATCCGTGATTTCCACAGGAAA |
| 89882 | NYD-SP25 | BC033792 | AACAGGTATAGCCAAATTCAA TAGACGAAATGTGCCATTATA CACGTTAAATCAAGGAAGGAA TTGAATGGACCTAACTATACA |
| 89884 | LHX4 | NM_033343 | CCGGACCACCATCACAGCCAA CAGAACTTGGCCACACCAATA CACCAATAGGATTTATGGCAA TCGAAAGTACGCCAATGTGAA |
| 89886 | SLAMF9 | NM_033438 | CTGGCAATAAAGTCAAATTAA CAGGCATGATATGACCTACA TCAGGGCTTTACCAAGCTACA CTGGCTGGCAATAAAGTCAAA |
| 89890 | KBTBD6 | NM_152903 | CAGTATTTATTTGATTATTTA TAGGTTTGTATTGTTGATCTA |
| 89894 | LOC89894 | NM_138341 | CTCGTGTTACCTTGATGGATA CAGGGTCTACTCAACTGTGGA ATGGCTACTCTGAGTGTTATA CACCGTCAATTACATCTGGTA |
| 89953 | KNSL8 | NM_201521 | GACCAGAATAAGTATAAGGAA CAGCTTCGCCGTTCTATGGAA CAGCGGCTACAGCGCAGTGAA AAGGTTGCGGACGTTGCACAA |
| 90007 | MIDN | XM_028067 | CTGTCTTGGATACCTATTTAA CCCTTTATGTGTGAAAGTTA ACCGGCTTCGGCGAGACAGAAA ACCGTTGTTCTCGTAGGTGAA |
| 90024 | LOC90024 | XM_028217 | TACGGTCGGAAGGAAAGTAGA CCGACCAACTGCAGCACCCAA |
| 90025 | C6orf157 | NM_198920 | ATGAATGATTATCAAATTAAA CTGCGGTGAAGTCATAATAAA |
| 90102 | PHLDB2 | NM_145753 | AAGCATGAAACTAAATTGAAA CAGAATAACTTTATCACCCAA |
| 90133 | LOC90133 | XM_029323 | TACCAGCGAATTCCCACCCTA TACAAAGACTCAGATCTCCAA |
| 90231 | MGC33867 | NM_138346 | CACGCCAGGAGTGGAAATGAA CCAGATGGAGTCGACGCTCAA |
| 90249 | UNC5A | NM_133369 | CACACCCATTGCCCACACTTA CCGCATAGCCTATTTGCGCAA CCGCATTAATGTCTCAAGGCA CACCATGGGAGGTCCGCATTAA |
| 90316 | TGIF2LX | NM_138960 | AGGGCCCAGTGGTCCAGACAA AAGGTCAAGGTTTCTGTCACA |
| 90378 | SAMD1 | NM_138352 | CAGCTTTCCAAGAGCAGGAAA CTCCATGGCAGCCATGAACAA |

Table4

| | | | |
|---|---|---|---|
| 90379 | LOC90379 | XM_373433 | CTGCGTGGACATGGTCATGAA CCGGGTGTGCGTGTCCTCAA |
| 90390 | THRAP6 | NM_080651 | ATGAAAGATATTGTAATAAA CTACAGGATAATCTTCGCCAA CTGAGATTGGTATATGACAAA CAGGAAGCTGAGATTGGTATA |
| 90427 | BMF | AK024472 | GAGGTCTTCAGTACTATTGAA CCAGAGGAACTCAGTTAAGAA GACGCTAGAAACTAAATTATA CAGACGCTAGAAACTAAATTA |
| 90441 | ZNF622 | NM_033414 | CAGGCACATATGAATGACAAA CTCCTTGATGAGATACACAA |
| 90485 | BC37295_3 | NM_001005850 | CTCGCTTATAGAGCACCAGAA AACGCGATGAATTCAGCCGAA |
| 90507 | SCRN2 | NM_138355 | CACAGGGTTACTGGAGCACTA CAGGCTTATGCGTAAGCTTCA |
| 90529 | LOC90529 | NM_178122 | AAGCTTTATGAAAGCTCTCAA ACCCAGGTGATTCCAATGATA |
| 90594 | ZNF439 | NM_152262 | TTGCTTTATGGAAGAAAGTAA CTAGGATTCTTTGAATACAAA |
| 90637 | LOC90637 | NM_182491 | GAGGGAGATGGTACTATAATA CAAGATTTCTGTAAAGATCAT |
| 90736 | CXorf44 | NM_138362 | TCCTGGATGAATAAATATTGAA CTGGGCTTCCTGGGTCAAGTA |
| 90737 | PAGE-5 | NM_130467 | ATGCTGGAAATTTGACTGCTA AACCAAGAATATTGTTCTTAT |
| 90780 | PYGO2 | NM_138300 | AACATGAACTTTCCCAGCCAA TCGGAGTGAGGTGAACGATGA ACGTATCTTCTTCATACCAAA CAGCCCATCTGTTGCACATTA |
| 90835 | LOC90835 | XM_373675 | CCCAGTCTCTCTCACATTCAT CTGGAACATTCTGGAAACTTT |
| 90874 | MGC45731 | XM_371286 | CACCAGGACTCAGAAGACAAA CTGGCCTTGGTTTCTATGTAA |
| 90952 | ESAM | NM_138961 | CTGAAATTAGCTACTCACCAA CAGAGGATAGGGAATCTCTTA TCAGGTGTGTCCTACATCAA ATACATAATGTTTGTATGAAA |
| 90987 | ZNF251 | XM_291262 | CAGACTCGTGTTGGAGAGAAA AAGCATAATTTATAAACAATA |
| 91050 | DKFZp761B107 | NM_173463 | CAGGCTAATCTTGCACAACTA AAGAAACTAATTATACTACAA |
| 91074 | ANKRD30A | NM_052997 | CTGCAGATATATGTGGAGTAA AAGGCTAACTGCGGAATGAAA CAGGAGATGCTTGTTTGCAAA AACCGTATATATCAATATGAA |
| 91151 | TIGD7 | NM_033208 | CAGACGGAACCCATAAATTAA AAGGTTCTAAGTAGAATTGAA |
| 91181 | NUP210L | NM_207308 | AAGGAGTACTTTGAAGAGCAA CAGGATGAAGTACAGATTGAA |
| 91227 | GGTL4 | NM_080839 | GAGGACAAGGCTGACAAGCAA CACCAGCACCATCAACCTCTA |
| 91304 | C19orf6 | NM_033420 | GCCCGATGACATGAACAACAA CGAGTTCTTCGTGTGCTCCAA |
| 91316 | LOC91316 | XM_498877 | CTGCTGTGGTCTCCTGCTTCTA ATCGTGGTATCCCAAGGGTTA |
| 91319 | DERL3 | NM_001002862 | CACGGCCGACTTCGTCTTCAT CTGGGAAGAGTCCCATGCTAA |
| 91351 | FLJ31033 | XM_037817 | CTGGGTATTCCAGCATATTAA TAAGAATAAGATAGTCATTAA |
| 91355 | DKFZp434O0213 | NM_182492 | TTGGCTCTGTGCATCCTTGAA AGCACTATAATATTTATTGTA CACGTGCAGCCTATATGGGAA CTGGAACAAATCAGTGAACAA |
| 91368 | MGC13017 | NM_080656 | CCCAGGAGTTATCACTGTAAA TCCTGGTATTACCAACATAAA |
| 91369 | MGC15396 | NM_052855 | CTGGGTGTTAATCCAGATCAA AAGGATGTTGCTCGACTCCAA |
| 91404 | SESTD1 | NM_178123 | CGCCAACAAATTGACTCGTTA TCGGTTGATCCTGAAACAGTT CAGCCGTCGATTAATGGCTCA ATCCCGCATGTTGTCAACATA |
| 91409 | DKFZp434E2321 | NM_207310 | AAGCTCATAATGAATCAGACA CTCCAAGAAATGCCTGCTTCT |
| 91445 | FLJ38628 | NM_152267 | ACAGCCAAGGATGGCAAGCAA CCTGAGTTTAATATTACAGAA ACCACCATCCTCAGAAGCCAA CTGAATCTTATTGAGGGACTA |
| 91452 | ACBD5 | NM_145698 | CAAAGTGATAATGATAAGAAA ACCGTTAATGGTAAAGCTGAA |
| 91464 | LOC91464 | NM_001008494 | ACGGGATACAGAGATAAACAA CTGGATGAAAGGAGAAGGCTA |
| 91522 | COL23A1 | NM_173465 | CAGGATCTTGGATGCCCTCAA CAGGAAGGAGATGAAGGTAGA |
| 91561 | LOC91561 | XM_039218 | CAGGGCCGCAGAGCTCGCGGA CGGCTTCTCTCAAGGATGAGA |
| 91574 | FLJ38663 | NM_152269 | CAGCATTATTATAGTGCTTCA TTGGATGAGAATGAACTCGAA |
| 91584 | DKFZp434G0625 | NM_181775 | CCGCATCGTCCAGGAGCTCAA CTGGCCTTTGTGGGCACCAAA TACCTGGGAATTCTTCCTAAA CAGCCGGAAACTGACCAAGAA |
| 91607 | FLJ34922 | NM_152270 | CCCGATAACCTTCACACTCAA CAGATACAACTCAGCATTAAA |
| 91608 | RASL10B | NM_033315 | ACCAATTAGAAATAACAATA CAGGGCAAGCAGAGGCAGCAA |
| 91647 | ATPAF2 | NM_145691 | CTCCGAGAGCACCACAGTCAA GAGACATTAGTGGAACTTCAA |
| 91662 | NALP12 | NM_033297 | CTCATGCAGATTGAGATCAAA CCAGACCTTGACCGACCTTTA |
| 91664 | LOC91664 | XM_039908 | AAGTATGATGATTGCAGCAAA CGCTGCGAGTGTGGTGTTTAA AACCTGGTGTCAAGATACAAA ACCGCCGCTATTCTCCAGATA |
| 91754 | NEK9 | NM_033116 | CAGGTGTCATGTGGTGATGAT TCGGGAATTATCAACCATGAA TACACTTGGGTGAACATGCAA CACGAACCAGTGAAGTCTATA |

Table4

| | | | |
|---|---|---|---|
| 91833 | WDR20 | NM_144574 | TGGGAACATGTACTTATATAA CACGAGGAACCCTCTCCTTAA |
| 91860 | CALML4 | NM_033429 | CACGGTGCTCTTAGCATTCAA ATGGAATTTATGGTAACTTTA |
| 91862 | MARVELD3 | NM_052858 | ACGAGAGGAGGTGGAATATTA CTGGAATGCCACAAATGCAAA CTGGGAGAGCCTGCTGCCAAA CAGGCGCTGTACCAAAGCAAA |
| 91942 | LOC91942 | NM_174889 | CCAGTTCAAACTCAAATTAAA CCCGCAGTACAAGAACTGGAG |
| 91966 | CXorf40 | NM_178124 | ATACATTAGGTTAAATCTGAA AGGGCCAGGTAAAGAAGCTAA |
| 91977 | MYOZ3 | NM_133371 | CACTAAGGATATAACAGTTAA CTCCAGGTGATTCCAGCATAA |
| 91978 | C19orf20 | NM_033513 | GAGGGCCGCCTTCAACAACAA CGGAGGTTACACCCTCAGCAA |
| 92002 | MGC29729 | NM_152274 | CAGATTTGTAGGGAGAATGAA TACAGGCATGACATCAATGAA |
| 92017 | LOC92017 | NM_001009607 | CAGCGTCATGAACAAAGTCAT ACGGAGAAAGCAGCTCCAGAA |
| 92106 | MGC15763 | NM_138381 | ATCGTCTGCTTTAACGCCTTA AACCAGCATAATGAAATCCAA TACCAGCGAACTCCTGTTTAA ATGCAGTTTGCATGTTACAAA |
| 92181 | DC-UbP | NM_152277 | CTGCTCTAAATTCCCTATGAA CAGGCTTTGAGACTCATGTAA AAGGCAGTTAAGAAATAGATA AGGCAGTTAAGAAATAGATAA |
| 92211 | PCDH21 | NM_033100 | AGGGCTGAGAATTGACGAGAA AACCTGCTATCAGGAAATCTA CAGGTTGGTAGCAAAGATCTT CAGGTACAACTTCTATGTGAA |
| 92235 | LOC92235 | XM_043739 | ACCGTCTGTTGCAAGCATGAA AAAGCTGGCCTCAGACAACAA TCCGATATTCTTCCCGCAGTA CAGAACTGGATTGCCAATGTA |
| 92249 | LOC92249 | XM_499179 | CTGCACTAAATTCCTTCATTA AAGTTCCTATCTAATATTATA |
| 92255 | DKFZp434H2226 | NM_001007527 | AAGGACTTTAAGAAAGATCAA AAGATTAAGATTATACTTCAA |
| 92312 | LOC92312 | XM_044166 | CCGCTCCAGGGCTTCTCTAAA CAGGCCCAAATCGCCACCTCA CAGGCAAGGCTGCAAGATTAA ATCCTCGAGTACAACAATGAA |
| 92369 | SSB4 | NM_080862 | CTCGCTCAACGTCTTCGTCAA CATGGCTGTCACAAAGCTTTA AAGCTTTATTTGAGCAAATTA ATGAAGAGCGTTTCTAATAAA |
| 92454 | MGC20460 | NM_053043 | CCCGGTGTAACCATTTATGAA ATCGAGATCAATGAACCTTTA |
| 92579 | G6PC3 | NM_138387 | CTGGGAAATGGCCAGAAGATA CACATGTTCAGTGCCCAGGAA GTGGCTCAACCTCATCTTCAA CAGGTGCTGGCTGGCCTAATA |
| 92609 | TIMM50 | NM_001001563 | CCGGACATACAAATATTTCAA CACCCTGGTCTCGGAATCTAA |
| 92610 | TIFA | NM_052864 | TAGGATTATTTACTTACCAAA CAGAATAATGATTTCTCCTAA |
| 92714 | ARRDC1 | NM_152285 | CACCAAGAAGTTCTCCTACAA CCGGTCTTCATTGGCAATATT |
| 92715 | C9orf112 | NM_138778 | CAGCCTTTCATACTTGTTTAA CAAGGGTGGAATGGAAGTTAA |
| 92736 | OTOP2 | NM_178160 | CAGGCAGGCCCTGGTCATCTA AACGCTCTGGATCCTCTTCTA |
| 92799 | SHKBP1 | NM_138392 | CAGGGCCTCCTTGGAATAAAT CCAGCTAGTGTTCATCCAGAA |
| 92806 | MGC16385 | NM_145039 | AAGCGAGTTCACAAATAGCTA CGCCTTTAGACTTATCTTAAA AAGGTGTAATCTCGACCTTGA CTCGGTTGACTCGCCATCGAA |
| 92815 | HIST3H2A | NM_033445 | CGGAAATGTCCGGTCGTGGTA CCGCGGTGCTCGAGTACTTGA |
| 92822 | ZFP276 | NM_152287 | CGGCATGAAGAAGCACATCAA CAGGTTAGAAGACATACAGAA |
| 92999 | ZNF651 | NM_145166 | CCCGAGTGATCTGAGGATTTA GAGGCTAGCACTAATAGGAAA |
| 93010 | B3GNT7 | NM_145236 | CAGGAAAGACAACAAATACTA CTGGGACGTGACCACCACTAA |
| 93034 | NT5C1B | NM_001002006 | TTGGATGTTTGTAGATAACAA TAGGATAAGTTTAGAACTTGA |
| 93082 | LOC93082 | NM_138397 | CACGTGTATAATGGAAATGAT CAGAGAGACCATGCATCTGAA |
| 93107 | KCNG4 | NM_172347 | CCCGCTGAGCCGCCTGAGCAA CAGCCAGGAGTTCTTCTTCGA ATGAACGATGTCAATGACCTA TTGCCTATCATGCACATGTAA |
| 93134 | ZNF561 | NM_152289 | CAGGCTGTGGACCGCAAATAA CACGCCTAAGTAGACATGAAA |
| 93145 | OLFM2 | NM_058164 | CGGAATAAACAGGACCTTTGA CAACGGCTCCCTGTTCTATAA CACGGACGTGCCCTTCCACAA CAGGACCTTTGACATTTGAAA |
| 93185 | IGSF8 | NM_052868 | TGGGCCCAGATTGCAGAGAAA CCGGCATTTATGAGTGCCACA |
| 94033 | FTMT | NM_177478 | CAGGTGAAGTCTATCAAAGAA CCGGGATGACGTGGCCTTGAA |
| 94086 | HSPB9 | NM_033194 | CAGAGACGGTTTCCAAATGAA CCCGGAGGAACTGGTGGTGCA |
| 94097 | SFXN5 | NM_144579 | CCCGCTCGCTGCGGTTGATTA TAGGCCAGAGGTGAACATTCA CAGCCGGACAGTGGTGTACAA CAGGCTGGTAAATTTAGGATA |
| 94104 | C21orf66 | NM_013329 | CATGTTAAGGATACAAATCAA CAGCATGAGAAACATCTGCAA |
| 94122 | SYTL5 | NM_138780 | CAGAATTCAATGAAACACTAA ATGGATTTGGCTGCTATTGAA |
| 94134 | ARHGAP12 | NM_018287 | CACCATTGTATTGGACACTAA CAGGGCGTTGCTATTACTATA |
| 94137 | RP1L1 | NM_178857 | CCGGAGGATACTGCTGATTAA TTGGATAAAGTTTGTTCTCAA |

Table4

| | | | |
|---|---|---|---|
| 94234 | FOXQ1 | NM_033260 | CTCCATCAAACGTGCCTTAAA CCGATGCTTCACTGTGCCAAA |
| 94235 | GNG8 | NM_033258 | CTGGCTTTCTGCGAGACGCAT CAAGATTGCCGAGGCCCGCAA CAAGACGGTGGAACAGCTGAA ACAGCTGAAGCTGGAGGTGAA |
| 94241 | TP53INP1 | NM_033285 | CAGAACGATGTTGACCTGTTA AAGGGACATTTGGTTAGGTTA CTGGGAGACACTAGTAGTATA CCGCGTCAGTACAATTACTAA |
| 96764 | NCOA6IP | NM_024831 | AAGGATCATAGTGAAATACTT CCGGCCAAAGTGAACCACGTA TCGCAATAATGCAGAAGTTTA ATGGAAACTTACAGGACTTAA |
| 112398 | EGLN2 | NM_017555 | CCCTGTAGACAGAGAAACCAA CCGCATGGCAGACAGCTTAAA CAGGGAATTGTGTCTGCAGAA CTGGCTAGGGTTACCCAGCAA |
| 112479 | MGC16943 | NM_080663 | TAGGTGCTTTCTAGAATCAAA CCCAATATGAGTAAACAGGAA |
| 112597 | MGC4677 | NM_052871 | CACCAGCCTCTCCTTGAATAA AAGTTTCAAATTGACATTCCA |
| 112609 | C6orf117 | NM_138409 | AAGCTGCACACTGGTATTAAA ACCTATTGTTCTGGAAACTAA |
| 112616 | CKLFSF7 | NM_138410 | CAGGCTGGTGGGCACCAGGAA AAGCATATGGCTGTCCTATAA |
| 112724 | RDH13 | NM_138412 | CCGCTTAAGGAAGGAAGGAAA CTGGCAGACGAGGAAGTATAA ACGAGGAAGTATAACACCAAA AAGTACTTCGATGGACTCAAA |
| 112770 | MGC31963 | NM_144580 | CTGCAGAACCTGCTTCATATA CAGCACTCCATCGACGATGAA |
| 112858 | TP53RK | NM_033550 | TTGGCTCGAATGCACGATGAA CTAAGTAAAGGTGTTAAGATA TAAGTTTATACCGCTAAACAA AAGTATTGGCGTATAACATTA |
| 112939 | BTBD14B | NM_052876 | CCGGCAGATCTGCAACATGTA CCAAGTGAGATTGCACATTTA |
| 113026 | PLCD3 | NM_133373 | GCGGATGAACTCAGCCAACTA CCGGTTTGTGGTGGAAGATTA AAGCAAGGGTACCGCCACATA TAGGTTTGTAACTGTTTCATA |
| 113177 | C19orf36 | NM_052878 | CCCGAGGAGGTGGAACCTCAA CCGCCACCATGTGAACTTCAA |
| 113178 | SCAMP4 | NM_079834 | ACCCGTCAAATCTGTGCCTTA CCGAGCCGACAGCTCCTTTAA |
| 113230 | LOC113230 | XM_053966 | CCGCAGAAAGGTCTCGGAGAA CAGGAGGAGCGCAAACCTTGA |
| 113246 | GRCC10 | NM_138425 | CAGGAGGTTATCAAAGCCTAT TCCGCTGAACCTAGAGCTTCA |
| 113451 | ODC-p | NM_052998 | TAAGCAAATTGCACAGATCAA AAAGTTTGGAGTGTCACTGAA CACCTCCAAGACCATCGTGTA TAGTTCAAGTATGCAACATAA |
| 113510 | HEL308 | NM_133636 | TCGGTATGAGTGCAACATTAA CAGCATTACTACCATAGGCAA |
| 113540 | CKLFSF1 | NM_052999 | CCAGCCTAAATGTGACCATAA CAGCCTAAATGTGACCATAAA |
| 113612 | CYP2U1 | NM_183075 | CAGCGCTTTGATTACACTAAT CAAGGGTATACCATTCCTAAA CCGGAGGATTTCTACCCTAAT CGGGTGTGTATGGGAGAACAA |
| 113675 | SDSL | NM_138432 | TCCGACAACTCCGGCCAATAA CTGCCAGGAGATGGCCAAGAA |
| 114026 | ZIM3 | NM_052882 | CAGGAACTCTTAAACACTGAA ATCAATCAACTTGCTAATTTA |
| 114041 | C21orf88 | NM_153754 | CAGCCTTAATTCTACCTGCAA TCCCGTCTCTTCCATTCTCTA |
| 114049 | WBSCR22 | NM_017528 | CTGAGTGGAAGTTATCTGTCA CCGAGCTGTCCTGCAGCTGTA CCCTAACAGTGCCAAAGCAAA CAGGGAGTCTGTGTTCACCAA |
| 114131 | UCN3 | NM_053049 | CCACAAGTTCTACAAAGCCAA CAGAGGCACCCGGTACAGATA |
| 114294 | LACTB | NM_032857 | AAGCCAAATGCCGGAATTCAA CTGGATCTTGATATTCCAGTA |
| 114548 | CIAS1 | NM_004895 | AAGGCAGACCATGTGGATCTA AAGCCAAGAATCCACAGTGTA TTGATAATTTATATCTCTCAA ACAGAACTAGTTGACTATATA |
| 114625 | ERMAP | NM_018538 | AACTAGGTACAGCGACTTTAA CACAAGACCATTGTACTGCAA TTGGGAAAGGCTGATGAGTAA TTGGAGAGTATTGATTTCAAA |
| 114659 | LOC114659 | NM_052888 | CTGAAGAAAGATCTAGCTGAA TGGGTTCAAACTACAGATCTA |
| 114769 | COPI | NM_052889 | CAAACTGGAACTCATAAATTA AAGGATCTCTATATCCTAGAA |
| 114771 | PGLYRP3 | NM_052891 | CAGCCACTTCTTCTGAAAGAA CTGCCAGACTGTCGTCCGAAA CAGGATGGTGGCGTGTATGAA CAGACTGTCGTCCGAAACATA |
| 114783 | LMTK3 | XM_055866 | GCCGCCTTTCTCGGAACTGAA CCGGATGGGTAGACAACACGA CCGGGAGACAGGACCCAGGAA GCGGGAGGATGTGACAAGGAA |
| 114788 | CSMD3 | NM_052900 | CTGGTACACCCTTATATGGAA CTGGCATTCCTCAATATGGTA CAGGTGTATGATGGACCAAAT CACGGTTTGCACAATGGTATA |
| 114789 | SLC25A25 | NM_052901 | CACGGTTGCACTTCCATTCCA CAGGCCTGACTTCCCAACCTA CAGAGTTATGTCCTAACTATT GACGTTAGAAGTTGTCATTTA |
| 114793 | FMNL2 | NM_001004417 | CCGGTTTGTGAAAGCATATAA ATGCAGTTTATTAATATTGTA |
| 114799 | ESCO1 | NM_052911 | AAGCTACATGATAGAAATATA TAGGCTGTTAACAAATCTCAA |
| 114804 | RNF157 | XM_290732 | CAGGACGATCTCGCCTCTTGA AAGCCTGGTCAATATCCGAAA AAGCGTGAAAGCACTGGACAA TCGGGTAGCCATCACCATCTA |
| 114818 | KBTBD9 | XM_496546 | CAGGACAGAATGGGAATTCAA CCGGTTTGAGTTGTTATATAA |
| 114823 | LENG8 | NM_052925 | TTGGAGAAGGGTGACCATGAA CAACGTCTTCATGAAGGACAA |
| 114836 | SLAMF6 | NM_052931 | CTCCAGTCCTTCGGACTTAAA CTCCATTGTTTGAGCCAAGAA CAGGGAATGTAGTTTCACAAA CACAATTTACTCCACAATTAA |
| 114881 | OSBPL7 | NM_017731 | CGCATTGACCTTGACACTGAA CTGGCAAGATATGAAGTGGAA |

Table4

| | | | |
|---|---|---|---|
| 114882 | OSBPL8 | NM_001003712 | CAGAACTATAATGTGATTCAA ATGGTTTAAATTAGTTAATTA |
| 114904 | C1QTNF6 | NM_031910 | CAGCAACAAGTTAGTATTGCA CAGGAAGACCTGTGCTATAAA |
| 114971 | PLIP | XM_374879 | CAGGAGTGGAAGAGACTAGGA AACCTCCAGAAGGGAGTCCAA CACCTTGGACAACCTCCAGAA CAGCTGCGGCTCAGCACAGTA |
| 115330 | GPR146 | NM_138445 | CAGCATTCAGTTTGTCAATGA CTGGTGTTAAATGGAGCTATT CAGGGTTCTGAGAACATTTCA AGCATTCAGTTTGTCAATGAA |
| 115426 | UHRF2 | NM_152306 | CACATACATAGTGTTACTAGA AAGCGGTACTCTAGAAATGAA CAGATATGATGGCATTTATAA CACTATATTCCCTAAAGTATA |
| 115548 | FCHO2 | XM_291142 | AAACATATTAATAAAGTATAA CGGGCGGGCGCGGACGCGGAA |
| 115557 | GEFT | NM_133483 | CAGGACCATATGAGACTGGA CTGGAATATGTATACAAGAACA |
| 115650 | TNFRSF13C | NM_052945 | CCCATGGAGTTGGTGTGCTT ACGGCCGGCCCTGAGCAACAA CTCCTGTTCCTGTGCCTTCAA CCGGGAATCTCTGATGCCACA |
| 115708 | C14orf172 | NM_152307 | CACCATGAGCTTCGTGGCATA CACGGTGGAGTTCCACCAGCA CCGCAGCGGCACGCGCCATGAA CCAAGTGTGAGTGATGAGCAA |
| 115827 | RAB3C | NM_138453 | AAGGACATTTGCCAACAATGA GTGGCAAATATGTGATCTTAA TACAAGATTGGTCAACTCAAA ATGCAGTACAAGATTGGTCAA |
| 115861 | TXNL6 | NM_138454 | CCGCATCCTGATCCGCAACAA CCGGCTGGTGCTGCTGTTCTT AGCCCAGAGGTTCGAGATCAA CTCAAGGACATGCCAAAGAAA |
| 116068 | LOC116068 | XM_371760 | TAGCAATTAGCTCATAATCAA AAGGAGATACATTAAATGCAA |
| 116093 | DIRC1 | NM_052952 | TTCGTTTGTACTCATACTCAA CAGAGTTTCTCTTGCCCTTAA |
| 116115 | ZNF526 | NM_133444 | CACGGAAGACTGAAGCAGAAA CAGAGATGATGGAGATGTCAA |
| 116143 | LOC116143 | NM_138458 | CACCGGCGTCATTCAGCTGTA AAGGAGCACCTGAAATTGTGA AAGGTGTGGGACCCAAGGCAA ACGGTGGGAGACAAACATCAA |
| 116166 | LOC116166 | XM_007651 | ACCCATGATGCCATACCTACA AAGCCGAAGCTCAGAGAGAA |
| 116173 | CKLFSF5 | NM_138460 | CTAAGTCATTCCCAAATTAAA CAGGATGTGGCTTCTCATGAA |
| 116225 | ZMYND19 | NM_138462 | ATGGGAAGGGTAGAAATTACA ATCATTTGCTGTAATCCTTAA CACGGGGAGTTGTGTCCACAA CAGGACGGTTCTCATTATTA |
| 116285 | BUCS1 | NM_052956 | CCGGAGGAATTTAACTTTGCA AACATTGGCATCAGAATCAAA |
| 116362 | RBP7 | NM_052960 | CAGGCTCACCTGTATCCAGAA CAGCGACAACTTCGAGGGCTA |
| 116441 | LOC116441 | NM_138786 | CTGGAGAATAATAACAACTAT CAGCAATAAACTCACCAACTA |
| 116443 | GRIN3A | NM_133445 | CTGAAGAACGATCCAGAGAAA CAGAGATTACACAGAGCAATA ACCGACGGAAATACATCTTTA CACCCAGGAATAACAACTTA |
| 116447 | TOP1MT | NM_052963 | AAGCCGGTGTACAAGAACTTA AGCAGCTAAGATCTTATCCTA CCAGACGAAGATCCAGGCAAA GTGGAGAAGATCTACAGCAAA |
| 116511 | MAS1L | NM_052967 | CCAACGGCGTTAGCAGATAAA CAGGGTCGATGTGAAACATA CACGAATCCCTACATGGTATA AGGGTCGATGTGAAACATAA |
| 116541 | MRPL54 | NM_172251 | CAGGCCCTGGAGGCCAATAAA CCGATGCTGAGTACCCTGAA |
| 116841 | IMAGE3451454 | NM_053052 | CTCCAGCATAGTTGAGATCAA CAGCGGTTTATTGGAAAGCCA |
| 116844 | LRG1 | NM_052972 | ACGGCTACATCTAGAAGGCAA ACACCCTGGTATTGAAAGAAA |
| 117065 | PRRXL1 | XM_060970 | CAGAGAAGAGCTCGCCATGAA CCGGACGACGTTCACTCTTCA |
| 117194 | MRGX2 | NM_054030 | CTGGTTAATGTTTAAGTTAAA TACCAGATTTGAGTCACTAAA CCGAAGAACTCGGAGCATTTA ATCAATGTCAACACATATTTA |
| 117248 | GALNTL2 | NM_054110 | CTGGAAATGATTATTACATA CTCCAGGATACATAAATTCAA |
| 117283 | IHPK3 | NM_054111 | CTGAGATACGTTACCCAATTA AAGACTGGTGGACAAGTGTAA |
| 117285 | DEFB118 | NM_054112 | CACCAATATGTAATTCTATTA CAGCTGTGTAAAGAAGTCTAA |
| 117286 | CIB3 | NM_054113 | TGGCAGCATGCCCGAGCTGAA ACTCTGGTTTCTGAGAATAAA CTGGAGCAGCGGTGACCAAA CACGAGCGAGCTGAAGCGTAT |
| 117289 | TAGAP | NM_054114 | CAGGAGAGCAGCCAACGAGAA CTCACTATTCTATGCCTTAAA |
| 117579 | RLN3 | NM_080864 | CAGGTACATGCTGCTGCTGCT CCGGTGGAGACGATCAGACAT CCCGGTGGAGACGATCAGACA CCGGATGCAGATGCTGATGAA |
| 117584 | RFFL | NM_057178 | TCGCAACTTTGTCAACTACAA CCGGCTATACAAGGATCAGAA ATCGGTTTCTTCAGTGCCTTA TTGGTGAGAAATGAAATACAA |
| 117854 | TRIM6 | NM_001003818 | TTGAGATGTGCTCAATGTATA CTACACTTTCTCTAAATATTA TAGGAAGTTCTGATCTGTTAA |
| 118425 | GDEP | NM_058168 | AAGAACTTAATGTGTAATTAA AGCGATGTGCTGTGAAATCTA |
| 118426 | LOH12CR1 | NM_058169 | CCGATGTCATCAAGTTGCAA ATGGATGATATTGTGGTTGTA |
| 118430 | LOC118430 | NM_058173 | CTGCAATTGGTCACAACTATT TCCTGTGATTTCATCCAACTA |
| 118442 | GPR62 | NM_080865 | TAGGCTCCATTCTGCCATCTA ATGGGCAGGTGTCACAAAGAA CTGGACCAGTTTGGTATTAGA CCCTAAGGGCTCACAACCAAA |
| 118472 | ZNF511 | NM_145806 | CCGTTTGATAAGGCACTAAA CACGGAAATGTTTGCTCCTTT |
| 118670 | FAM24A | XM_058332 | CAAAGTAGCTAAGGCACTAAA ATGAGCAATATTTCTTTCTTA |

Table4

| | | | | | | |
|---|---|---|---|---|---|---|
| 118738 | ZNF488 | NM_153034 | TGGCATATTGTTGGTGCATAA | CACATTGTGAGTATATGAATA | | |
| 118788 | PIK3AP1 | NM_152309 | AAGTACGACTGTAGTTATCTA | TCCCATGGGATTATTCTCTAT | CAGCAGGGTATTTAAGTGCTA | ATGCTCAAGAGTCACATTAAA |
| 118812 | C10orf83 | NM_178832 | CAGCTGATAAGTATTGGGTAA | CAGGCTGTCTTCTTTCATCAA | CAGATTGTGGTGGAAGTTATA | AAAGGTGTTTATAAAGAATAA |
| 118856 | MMP21 | NM_147191 | ATGGTGAGATTTAGCACATAT | AAGGATCAGGCCCTCACAGAA | CCGCTTATTACTCCTATGCAT | CACACTGAACATGTAATAAGA |
| 118945 | LOC118945 | XM_497242 | ATCCATATGAAGGAAAGGTTA | ATGGACCAAGTGGAAGGCAAA | CAGGAATACAGCCAAGGGAAA | CAGCTTCACAATGGCCATGAA |
| 119016 | CTGLF1 | NM_133446 | CAGCATAGCCTCGGCCTAGGA | CAGCGGGAGTGCGTGGCCAAA | | |
| 119391 | GSTO2 | NM_183239 | ACGGCTTGTCTTGGGAACCAA | CAGGAATTCAGCAACCTGGAA | | |
| 119764 | OR4X2 | NM_001004727 | CAGGTGTGTACTGTCCTTGTA | CTGGCTGAAAGGAAAGTCATA | | |
| 119775 | OR52P2P | XM_061677 | ACCACCTTACTTGGCCATGAA | CTGCATGCTATCCACCATTGA | CGGCACATCCACATCCTTCTA | AAGCAGCTGTCTTATGGCATA |
| 120105 | LOC120105 | XM_061864 | GAAGATCTGTTTATAAACTAA | CACCCACAACATACTCTGTTA | TACGATGTGAGCATAAGTGAA | CCGATAGATGTGGAATACAAA |
| 120114 | FAT3 | XM_061871 | CACCATGCTCGGAACCTGTAA | TGCAAACTAAAGGAAACTTTA | | |
| 120318 | LOC120318 | XM_497316 | GAGGCTAAAGAGAAGCATCAA | AACAGTGGGAACGACAAACAA | | |
| 120793 | OR56A4 | NM_001005179 | CACACTCTACTACGTCTAGAA | ATCCATTGAGATACCCGTCTA | | |
| 120796 | OR56A1 | NM_001001917 | CTGTGATAATTTCACCCTTAA | AAGAGCTGTGCTTAGATTCAA | | |
| 120824 | LOC120824 | XM_062300 | AGGGATTCAGAGTTTATCTTA | AAACCTGAATATGGCAACCAA | | |
| 120863 | DEPDC4 | NM_152317 | CAGGATATGAAATGATTTCAA | CAGCATCTCTCTGAAACGAAA | | |
| 120872 | LOC120872 | XM_497349 | GAGGAGTTAGCTGCATTTAAA | CAGGCTTATTAGAATGAACTA | | |
| 120939 | FLJ31166 | NM_153022 | CAGCACAGCCATCGACATTAA | CCGCAGCACAGTGCTAATCTA | | |
| 121275 | OR10AD1 | NM_001004134 | CTGGTTCTTTGGGCTGATCAA | CTCAGTTTAGTCTGTGGGCAA | CTGGTTCTTTGGGCTGATCAA | AGCTATGTTCTCTTACATGAA |
| 121278 | TPH2 | NM_173353 | AACGTGAAGCTGCTACCGAAA | ATGAAGTTGGTGGATTGGTAA | CTCAGTATACTTCAATCCCTA | CTGAAATAACGTATTATGTTT |
| 121504 | HIST4H4 | NM_175054 | CAGAATGTCTGGGCGAGGTAA | CAGCTTCTCTACAGACTCCAA | | |
| 121506 | FLJ32115 | NM_152321 | AACAACCTGCATAATAAATAA | CTGGTGGACAGTGGTATGAAA | | |
| 121512 | FGD4 | NM_139241 | CCGGTATGAGATGCTCCTTAA | CAGAGTCATTCTACCGATAAA | | |
| 121793 | MGC35169 | NM_152324 | CAGCAGCAAGTTAGGGCTGAA | ATGATGTAACAGGGACAATAA | | |
| 122585 | LOC122585 | XM_063202 | CACATATGTGTTCAGGGTGGA | CTGCGACTCGAGGCCACCCAA | | |
| 122664 | C14orf8 | NM_173846 | CTGCGTTTGGAGAATCATCAA | ACGGGAAGAGATGACTGACAA | | |
| 122773 | KLHDC1 | NM_172193 | CAGGCTTTATTTAAAGGATAA | CAAGTTATCTCTGAAATTTAA | | |
| 123624 | FLJ32310 | NM_152336 | CCAGGTGATGTTATAAATAAA | AAGGAGGATCTTCTTGGCAAA | | |
| 123688 | LOC123688 | XM_058719 | AAGACACTGCAGAGATTCCAT | CAGTAAAGATCTGTTGAATAA | | |
| 123803 | NTAN1 | NM_173474 | TGGCATTGCTGTCAACATTAA | CAAGAAGATGACATTCACTTA | | |
| 123876 | LOC123876 | NM_001010845 | TCCAGCATGACTAGAATATAA | CACCCTTAATCTGGGTAGGAA | | |
| 123879 | MGC48972 | NM_173475 | CAGGAGTTATTTATTATCTTT | TAGGTTGAATTAACATTACAA | | |
| 124044 | MGC26885 | NM_152339 | CAGGAAATGACCTGACCTCAA | AACACTCAGAGGGACGGTCAA | | |
| 124274 | GPR139 | XM_064062.4 | CTCCATCTTCTTCATCTTGAA | CCGCATCATCATGATTCTTTA | ACCGTTAACCATTGACAGGTA | AAGAATTGTCGTGCACCTCAA |
| 124402 | LOC124402 | NM_145253 | AAGAACGAATTCCAAACCAAA | CACCATGTTCTCCCGTCTCAA | | |
| 124446 | LOC124446 | XM_378175 | AGCCCTAGAATGGGTGAGGAA | TTTCTCAGGTGTGAATCAACT | TGAAGGCCTTGTGCTGACCAA | GGACAACTGGTCCTTATCACA |
| 124460 | SLIC1 | NM_153337 | CAGTACTGGCAGAACCAGAAA | CTGGCTGCATGGGACCCATAA | | |
| 124512 | LOC124512 | XM_497558 | TCGGCAAAGCTGCCAAATGAA | CAGGAAGACATACAGTTGAAA | | |
| 124535 | FLJ40311 | XM_064190 | TAGAAATATTACAGAGATCTA | TAGGAATATGAAACTAATTAA | | |
| 124538 | OR4D2 | NM_001004707 | CCGGCTGGTTTGAGAGTGACA | CTCACCAATTTGCTTAAATTA | CTGGATGGCGTTATGATTTCA | ACCAATTTGCTTAAATTAGTA |
| 124602 | FLJ37300 | NM_153209 | CGGCCGGGCCAAGAACATTAA | CACCGAAGTCTCCACCATCAA | CCGGGGAGAAGTCCTACCTGTT | CCGGGTCAAGAACATCTTGCA |
| 124637 | FLJ32499 | NM_144607 | CCGGAGCATCCGCATCATTAA | CAGGAAGATGGGATTTACAAA | | |
| 124641 | OVCA2 | NM_080822 | CAAGGCCTTAATGGACATTGA | ACGATGGTGACTGCCACATAA | | |

EP 2 295 543 A1

Table4

| | | | | |
|---|---|---|---|---|
| 124783 | FLJ25414 | NM_152343 | CCGGAGTTCACGGAGGAGAA AACGAGGAGTCTGACTTTGAA | |
| 124808 | FLJ31795 | NM_144609 | AAGGAGCAGAAACATAATCAA ACCACATTAAATTGTAAATAA | |
| 124817 | FLJ40137 | NM_173478 | CAGCAACATTACAGTCTTGAA CAGCAAGTGCTTTCATCCAAA | |
| 124930 | FLJ25555 | NM_152345 | CCCAAGTAGACTGGACACAAA CACCAAGACACAGAAGTTCAA | |
| 124935 | SLC43A2 | NM_152346 | TCGGTGCTAATTCCTTGTAA CCCGCTCTACCTGATCTGCTA | |
| 125058 | TBC1D16 | NM_019020 | CAGGGACATCTTTGCAAGCCA GACGATAGAAACCTAAGGAAA | |
| 125111 | GJC1 | NM_152219 | CACAGCATCCTTGACAGCTAA CCCGGTGCTGTTCGTCGTCTA | |
| 125206 | SLC5A10 | NM_152351 | ATGGGTGTTCGTGCCCATCTA CAGCGGGCAACTCTTCATCTA | |
| 125476 | C18orf37 | NM_194281 | AAGGTGTTTGACAAGAAGATA CTGGAAGAACCTGAAACAAAT | |
| 125875 | MGC33839 | NM_152353 | CACCGTGAAGAATGCGTGGAA AAGTCTCAAGGTCTCCAGTAA | |
| 125950 | RAVER1 | NM_133452 | ACGGACCTTTGCTGAATTAAA CCAGCACTCCATGAAGATAAA | |
| 125958 | OR7D4 | NM_001005191 | CTGTGTGGTCTCCTTGTTCTA CTCAGATTGTCTCCTCCTTAA | CTGCTCTAACACCCTCCTCAA ACAGATCGTGTAGAAGAGAAA |
| 125963 | OR1M1 | NM_001004456 | CAGGAAGCTGGTCAACAGAAA CAGCGTCATAATCGCCATGAT | |
| 125972 | CALR3 | NM_145046 | CACGAACATTACTTCAATCAA CTGGAACTTAACATCACTCAA | |
| 126014 | OSCAR | NM_130771 | AAGGAGTAGCTGAAAGGAAGA CCCGCTGTGCCCTGTGCTGTA | CCCAGCTTCATACCACCCTAA AAGGTAGTAGTGAGACGTGAA |
| 126037 | LOC126037 | XM_058967 | AAGGCCAAGAAGCCTGCATTA CTAGAAGGCCCTTTCATCAAT | |
| 126074 | FLJ35119 | NM_175871 | CCCAATCAATATGTTGTTTAT CAGGGACATATGCAGATCCAA | |
| 126119 | SBBI54 | NM_138334 | CTACTACAACCTGGACTCCAA ACCGGCAACTATGATGTCAAT | CTGGGAAAGGCCAGCACTTCA CTGCCGCTGCTGCCTCAATAA |
| 126133 | MGC10204 | NM_153329 | AAGCCTGAACACAGAAATTCA CAGGAGTCTGTGTGGGATGAA | CCGGCTGTCCTGCCTCTCCAA CCCAATAAACTCTCTGACCAA |
| 126205 | NALP8 | NM_176811 | CAGGAAATGGGTGTTAGGTAA CTGAGATACTTGGAAATACAA | |
| 126353 | C19orf21 | NM_173481 | CCCAAACTGCCCTGAAATAAA TTCCGTTTCTATCTTCCTTTA | |
| 126374 | WTIP | XM_059037 | CACCGTGGACGTGGAGAACAA AAGGACAAGATTTGACTTAAA | |
| 126402 | FLJ40365 | NM_173482 | CACCTTGGTAGAAATGGCAAA TTGGAGCTGAAGGAAAGATTA | |
| 126410 | FLJ39501 | NM_173483 | CGGGAGCTGGCCCTAAAGCAA AAGGACCTGAGTGCAAGTAAT | CTGGACAACATGCACCATGTA CAAGAGAAGATGAGTGATTAT |
| 126526 | FLJ36888 | NM_178830 | CTGGATCTCAATAAGGAGATA CGAGGTCAAGGTCACCATTAA | |
| 126537 | LOC126537 | XM_497611 | CTGGTTCATGACTTCACAGAA AAGGAGTTGTCTGATGAGGAA | CTCAGCCACCATTGCACCAAA AAGCCGGAGGTCATAATGTAT |
| 126626 | MGC29891 | NM_144618 | AAGGAAATATACAGAAGACAA CACCAATATATGCCTTTAA | |
| 126661 | LOC126661 | XM_059061 | TTGGCAGTTGGAAGCTCTTAA CCGCTAGGAGAGCAAGCACAA | |
| 126792 | B3GALT6 | NM_080605 | CCCGTCTCTCACGTACTTTAA CCCGCTGCTGCCCGTGTTTAA | |
| 126823 | KARCA1 | NM_001007255 | ATGGAAGCATCTACACATTAA CAAATGCTTATTAAATTTCAA | |
| 126859 | DKFZp686H1423 | NM_144696 | CCGGAGGACTATTTCAAATTA CTCAAGATTATTAAACATTTA | |
| 126868 | FLJ38716 | NM_152367 | AACCAAGACATTAGATTTCAA AAGGCATTGGATCAACACAAA | |
| 127059 | OR2M5 | NM_001004690 | CTCTATGGATGCAATCATTGA ACTCAGGAAAGTGTTAGGAAA | |
| 127247 | ASB17 | NM_080868 | AACAATGAGTAAATCTACTAA AACCTCGACTTCACTGAAATA | CTACTGCAATATGGAATCTTA ACCATCAGGAATCAACTATTA |
| 127281 | MGC26818 | NM_152371 | CACCATTGCAGTGGCCATTTA CCGGCAGCAACGAGCCATTAA | |
| 127385 | OR10J5 | NM_001004469 | AGGCCTGATATTTATCTCCTA ATCAATGAGATAATAAATTAT | |
| 127602 | LOC127602 | XM_059166 | CAGGCTCATTTGGGAATTTAT CCCAATTTAAACATAGAACAA | |
| 127623 | OR2B11 | aNM_001004492 | CAGGTGGTCCTGACGGTGCAA CCCAAGAGCAGGGCAAATTTA | ACCGGCTGTGAATGACACCATA AAGCCTTATCCTCGGAAGTAA |
| 127670 | HE9 | NM_172000 | ATGGATCATGGTTATGTTTAA TTGGCTTCATTTCAAACTTTA | |
| 127733 | UBXD3 | NM_152376 | CACCAGGACTTGAGCACATAA CTGGTAAATAACCACAGTGTA | |
| 128077 | MGC46719 | NM_153713 | CCGATTCATGCTGGAATCCAA AGCCATTTCAGTAGCACTAAA | |
| 128136 | LOC128136 | XM_497739 | TTGAGCCAAATGCAACTTTAA TGGGATAATGCTGCAATCCAA | |
| 128239 | IQGAP3 | NM_178229 | CACACGGACCTGAGCAAGCTA ATGGTAGGACATGGACCTTGA | GAGCACTAAGACCACCTTCTA CTCCGTGGTTCCGTTGAAGAA |

Table4

| ID | Gene | Accession | Sequence |
|---|---|---|---|
| 128312 | HIST3H2BB | NM_175055 | GCGCCCAAGAAGGTTCTAAA TTGGAGAGACTCAGCCATCAT |
| 128322 | LOC128322 | XM_060943 | AAGAACATCAACGATGGCTTT CAGGTTTGCCCTGCACAATTT |
| 128414 | C20orf58 | NM_152864 | CAGCGTCAAGAGAGTTTGTAA AAGTTGGTAAATAAACAGGAA |
| 128486 | C20orf142 | XM_371399 | CTGGTAAGACTTGACCTTTAT AAGCAAGATAGTTACAAGAAA |
| 128497 | C20orf165 | NM_080608 | ACGGAAGGAATACAGCCGAAA CTGCTTGTAGATGGATGAAAT |
| 128853 | DUSP15 | NM_177991 | CCGAAATAAGATCACACACAT CTGGAGGGTATTAAAGAGACA CACCACGATTGTGACAGCGTA ACGGGCCTGGAGGGTATTAAA |
| 128859 | BPIL3 | NM_174897 | CCCGGACTTTCTGGCCATGAA CTGTAGCTTTATCCCAAGTCAA CCTGGTCAATGTGAAGACTAA CTGGCCTGAGCTGGACATAGTA |
| 128939 | LOC128939 | XM_066243 | CAGCAGGTGTTTGTTGGGCCA CAACGAGTGCATGGATGGAAA |
| 128989 | DKFZp761P1121 | NM_152906 | CTGGATGTGCTCAACAATAAA CAGGAGTAGGCTTGTATTTAA |
| 129049 | RUTBC2 | XM_059318 | ACGGACTTTGCTTAAATCCTA CAGGTGAGAATTCTTGAGCAA |
| 129285 | LOC129285 | NM_152994 | AAGGAAGTTCATGTCAATAAA AAGGCTAAACTAGAAGTGAAA |
| 129530 | LOC129530 | NM_174898 | CTCAAATGTTACACAGATAAA CAGGTTTCCCAGACAACTGAA |
| 130063 | LOC130063 | XM_059396 | CATGACTTTATTGTCTTTCAA CAGCAGAACTGTGGACAGCAA |
| 130162 | FLJ31438 | NM_152385 | CAGAATGGCTTTGACAAATTA ACGGTTAGATTTAGTTACCAA |
| 130399 | ACVR1C | NM_145259 | AAGTGTGAAACTTGTGCCATA CAGCAAATAGATATTCCTCTA CACAGAGTAATGAATATTTAA TACTTTGGTAAACGAGATTTA |
| 130576 | LOC130576 | NM_177964 | CACATTGGACCTCAAGGCGAA CAGGTCAAACCCTCTTGTTTA AAGACAAATGGTGTCCACAAA AACGGTGAACTTTGGAGTGAA |
| 130940 | LOC130940 | NM_138803 | CTGAATGAAGTCAGATGTAAA CACGAGAAATATTGTGACCAA |
| 131034 | CPNE4 | NM_130808 | CCGAACTGAGGTTGTGATGAA CAGCGTCAGAGAAACTAACA CACCAAGGAGGCATCGCAATA AATGTTGATAGACGAAATATA |
| 131096 | KCNH8 | NM_144633 | TCGCACGTTATTTGCCATGTA TTGGAGCAAATCTATCAATTA CAGACTTTGGTAACAATCCAA CAAGACTATCAAACAAATCTA |
| 131118 | TIM14 | NM_145261 | ATCAATGAAGCTAAAGATTTA TAGAATTTATACACTAATAAA |
| 131601 | GPR175 | NM_016372 | TACCCTGATGCCCATCTCTCA CACCATGGTGGATGGACTGAA TCGGAGGAGCTTCTACGTGTA CAAGATCCTCTTCTCCTACAA |
| 131616 | TMEM42 | NM_144638 | AAAGCCAGTCATGTAACTCTA ACGGACCAGCTGGAAAGATCA |
| 131973 | LOC131973 | XM_497912 | CAGGAAGGTATCCTCCATGAA CTCGGCCTGCTGCATGCACAA CCCATCAGAGGCAGCACTCAA CCGGAGTCCGCGGTTACTCAA |
| 132001 | MGC16471 | NM_138807 | CTGGAGTTTACTACAATTCAT ATGGTGGTTGGAGAAGATAAA |
| 132320 | FLJ30655 | NM_144643 | CAGCTGGAAATGGCAAATGAA AAGGTTGCACAGTGAATTAAA |
| 132332 | FLJ30834 | NM_152399 | AAGAATGGTATTTGGAATTAA CAAGATTGTCTGAGAAATGTA |
| 132671 | LOC132671 | NM_145263 | CAGCTTTGAGATAAATGTTAA AAGGCTAAGTGTAGAAGATAA |
| 132789 | GNPDA2 | NM_138335 | AAGCGTGTAATAAATAAGTTA CTGGCTATTTATTATAAAGTA |
| 132946 | ARL9 | NM_206919 | CTGGAGATTGGTGGCAGTAAA CTGGGTAATAGTCAACTAATA |
| 133308 | LOC133308 | NM_178833 | ACGTACAAAGACTGAGACAAA GGGCTGTAGTTTGGTCAATTA CAACGATAAATGTGCAGATCAA CTGCAGAAAGTTGAACATCAA |
| 133396 | IL31RA | NM_139017 | CTGGACGATCCAGCAAGATAA TTGGGTGTCTATGATTTCTTA AAGAATAACGATCCCAGATAA CGGGACCAGCATAAAATTTCAA |
| 133688 | FLJ34658 | NM_152404 | CACACTGACACTTACAATGAA CTCAATGTAGTTATTTGCTAA |
| 133746 | JMY | NM_152405 | TTGGATAACATTAGATCCAAA ATGGCTGTACTGTCTATTCAA |
| 134145 | LOC134145 | NM_199133 | CCAACTGTTTGTAGTATCCTA CACATCTTTAACTAAACTTAT CTGGATAATGGTACACATAA CACCTAGTCTTTGGTCCATAA |
| 134391 | GPR151 | NM_194251 | TGGAAATGGATGATAACCAAA AGGGAAGGCTTGAAAGGTGTA CTCCTTCACAATGCTTGGAAA CCGAGCTACGGCGTACTCCAA |
| 134466 | LOC134466 | XM_376436 | CAGGGTCAGTTCAGCCTTAA CCAGGCAAGTTGAGTCATCAA |
| 134505 | LOC134505 | XM_068889 | CCCAGCAGATCTTCATCTGTA CCAGGTATCAACAGGAACAA |
| 134860 | TRAR3 | NM_175057 | CTGGCAGCGTTGGAAACTTA AAGCATCAGGCTAGGAAGATA TAAGGACTGATTCGTCAACAA CATGGTGTTTATATACAGTAA |
| 135112 | NCOA7 | NM_181782 | ATGGATATTGACTATATGTAA CTCCATAGCACTGAAATTTAA AACAATTATTATACTCCTTAA CCGGAAATCGGCATCACTAGA |
| 135295 | SRrp35 | NM_080743 | CACAGTCAAGTTCACCTCAAA AACTCTCATTATGTTAAATAA |
| 136242 | LOC136242 | XM_059830 | CAGGAATTCCTTATGTGTGAA ACGGTACTGAACAGACAATTA CACCAATGTTTACAAATATGTA CACCATGAAATATGTCTTCTA |
| 136306 | LOC136306 | NM_174959 | CCGCTGGAAGGTTAATCATAA CGGGCCCTCCAGCAAATTAAA |
| 136371 | ASB10 | NM_080871 | AGGGCTTGAGTGAAAGGAGAA ATGGAGGGATTCCGCTTCAA CAGCGCCAACACCATGGACTA CCGGGTGATGTCTGATGAAGA |

Table4

| | | | |
|---|---|---|---|
| 137075 | CLDN23 | NM_194284 | TAGCTACATATCATTGCATAA CAGCAGCTTAATGGATTTCAT |
| 137362 | MGC33309 | NM_152413 | CAGCTTGTTAAAGACCTACAA CAGCTGTATCAATGCCAACAA |
| 138050 | FLJ32731 | XM_372038 | ATGGATGTCTTTGGAACTTCA CTGGACCAACTTGACCGTCTA |
| 138162 | C9orf116 | NM_144654 | CTGCCATTTCACATCTAATAA CTCCGTGTACAGGACCAGTAA |
| 138199 | C9orf41 | NM_152420 | CCGGAAATGCATTGATCATAA CACAATGTAATTGATTATATT |
| 138240 | C9orf57 | XM_059954 | TACTGCATTAATTTAATTTAA CACATTCAAGGTGGCATTCAA |
| 138428 | C9orf115 | NM_001002913 | ACGGCGGCTTATGAACGCCAA CCAGGCTGCCTGCGGATTAAA |
| 138429 | PIP5KL1 | NM_173492 | ATGCTGGAACCAGGACTCAAA ATCAAGATCATTCAGCAATAA AAGATGGAACTGGATACCACC AACGTCTCCACGAGGATGAGA |
| 138639 | PTPDC1 | NM_152422 | AACAGAGGGTTTCAAAGCATA AGGCTATACATAAGTATTGTA TACGTAGATACCAGAGGCCAA CGGAATGTTGAGTGCCTTCAA |
| 138724 | MGC41945 | NM_203299 | CAGGAGAACCCTCAAACTCTA CAGAAGCTACATGGAAGGATA |
| 138805 | OR13F1 | NM_001004485 | CCGCTGTAGATTCACAGGAAA TCAGTGGAAGGTCGAAGTAAA |
| 138864 | LOC138864 | XM_498304 | TACCTGAGAATAAACCATCTA TTGGGAGAGCATATCCCGAAA |
| 138883 | OR1N1 | NM_012363 | CAGGAGTATTGTTTCCTCTTA CTGCGTTGTTTGTGTGTTCTA |
| 139105 | CXorf20 | NM_153346 | CAGCGAATTATCCAACTTTAA AAGGTAGAGAAAGAAATTCAA |
| 139135 | PASD1 | NM_173493 | ATGTTTGTAATTGTTTGTTAA CAGCCTGTTTCTTGGAAGTTA |
| 139231 | CXorf39 | NM_207318 | CAGGTGGAATATATTGAGTAT TTGGTTATTAATAGAGCTCTA |
| 139411 | PTCHD1 | NM_173495 | TCCGTGGAGTTTGGAGTGATA CCCGTGTGGTTGACCAAATTA AAGGATTTCACAAGAACTAAA TCCCGGTTTGAGAGCTATTTA |
| 139562 | HSHIN6 | NM_207320 | CAAGACGACAGTAGCATTGAA TGCGTATAACTTCACCACAAA |
| 139604 | LOC139604 | XM_498354 | TGGGATCATGATGAAGGACAA ACCCTTTGACACTGAAATAAA |
| 139760 | GPR119 | NM_178471 | CAGTCTCTGCTTCACCTTGAA CGGGCTGTGGTTAGTGTCTTA CAGGAGTGTCACCTCTACCTA CTCCCTCATCATTGCTACTAA |
| 140432 | ZNF183L1 | NM_178861 | ATCCCATGCCTGAGTATGTAA CACTGCAGTATTATTTGCAAA AACGGGAAGGAAATCCTCTTA AAGGATCACATGCTACAACTA |
| 140469 | MYO3B | NM_138995 | ATGGCCGTACACAGACTTCAA CACCCATTTATTAAAGGAGTA ACCCTGGTTGATAAATTTGAA AACCAGTTAAATGGAACTCTA |
| 140545 | RNF32 | NM_030936 | CAGTATCAAACCCGAGTGATA TGGGTTAAGTACTACAATGTA CCGCTAATAGTCTGTCCTAAA TTCAGTGGTATTCAACATCAA |
| 140576 | S100A16 | NM_080388 | CAGGGAGATGTCAGACTGCTA CACCTGTTCAGTCTCATCTAA |
| 140609 | NEK7 | NM_133494 | CGGACAATTTAGTGAAGTTTA CCCATTTATGTCTGGTGTTAA CCGGATATGGGCTATAATACA ACGACCGGATATGGGCTATAA |
| 140612 | ZFP28 | NM_020828 | AAGGAAATTATTAAACTATAT CCGGGCTTGGTGACTATCAAA |
| 140628 | GATA5 | NM_080473 | CTGGGTTGGATGATACCTTAA AAGGGCCTTGTTCTAGCTGAA |
| 140706 | C20orf160 | NM_080625 | CACCATGGAATATGAAGTCAA ACCATACTAAACAGTGTAGAA CTGAGGGTTAAGACCTTAGAA CACCCGATAAGAGCTCAATAA |
| 140730 | RIMS4 | NM_182970 | GTAGCTCATGTTGAAATTAAA CAGAGTGGACAGAAAGCATTA |
| 140766 | ADAMTS14 | NM_080722 | CAGCACCGACTTCTTCATTGA CACCGGCAGCTTCATCCTCAA CAGGAGTGTGTGTACACTGGA CCGCTTGATCATGGTTGGCTA |
| 140803 | TRPM6 | NM_017662 | CACGAGCCTATTGCTCACTTA AACCTGAAGTCAAACAATCAA GAGCAGAATCCTGCACATTAA TTGCCCAATTGCCAAACTAAA |
| 140809 | C20orf139 | NM_080725 | CACCTTCTATCAGAAAGTAAA ATGGGTATTTATTTGGATAAA |
| 140823 | C20orf52 | NM_080748 | AACCATGGTTGCCAACTACAT CTGATGGGCGGCATTGGGAAA |
| 140862 | C20orf82 | XM_097736 | CCGAGTCACTTCGGTTTCTAA AAGCGAGTTCTTAAAGAAGTA |
| 140870 | WFDC6 | NM_080827 | CAGCCTTACTTTATACCATAA CAGGATTTGGCTCATAATCCA |
| 140873 | C20orf173 | NM_080828 | CAGGAGGAAATTGTAAGATCA TTGGATCAGAATAGCAAGGAA |
| 140876 | C20orf175 | NM_080829 | CACGGAGTTGGGTACCATCAA CAGGAAGGGCTCCTTGTACAA |
| 140880 | CST11 | NM_080830 | TAGGCAATAAATGTTTGTCAA CAGGATCTTCCGAGTCCTTAA |
| 140883 | SUHW2 | NM_080764 | TCAAATGTTTAAGAAGCCTAA CAGTGGATGTAGCATCCATAA TAGCCGTTAGTGGAGAATGAA CCCAACTTGAATCGAGATCAA |
| 140885 | PTPNS1 | NM_080792 | CCCGAGAAGAATGCCAGAGAA ACGCCTGTAAATTACTGAGAA CTCGCTGTGGACGCCTGTAAA CGCCTGTAAATTACTGAGAAA |
| 140901 | STK35 | NM_080836 | CTACGTGTGAACAACTTTATA GACTGAGGCTTTGTAAATTAA TTGGGTTGGTATGATACATTA CTCGGCTGTTAGGGATCTGAA |
| 142679 | DUSP19 | NM_080876 | AAGGTCATATATACTATACAA TACGGTCCTCTTTCTCACATA CAGAGTTAACCTAATGAGTCA ATGGTCAGTATCACTGGATAA |
| 142686 | ASB14 | NM_130387 | CCGGTTAACTGCCTCCAGATA AAGGCTGGACATCTACAGTTA ACGACCTTTATGGACAAGGAA CTCACTATATATTATAGTAAA |

Table4

| | | | | |
|---|---|---|---|---|
| 143187 | VTI1A | NM_145206 | TCCCATAAGAAGATAATTAAA CAGAGCTTTATGATTCTGTAA | |
| 143379 | C10orf82 | NM_144661 | TTGCATGTCATTAATGATAAA TCCCAAGGAGGTGAACTTTAA | |
| 143458 | LOC143458 | NM_174902 | CAGCACCTCTAGTTAGAGTTA CTGCTGGGTAGTTACCTTATA AAGGGACTATTTATATGTGTA AACCTGTTTGACGCTAATTAA |
| 143503 | OR51E1 | NM_152430 | CTGGTTCAATTCCACTACCAT GCCCTAGGTGTCAGTGATCAA CTCATATCTGCTTATTCTTAA CAGTTTGATGCTTGTCTGCTA |
| 143872 | FLJ32810 | XM_370651 | GTGGATCTATTCAAAGCTTAA ACCGGAAATGTTTAAATTAAA | |
| 144106 | FAM10A5 | NM_153291 | CTCTAATTTGAGTGAAATAAA ATAAAGCCCTGCTGAAGGAAA | |
| 144195 | SLC2A14 | NM_153449 | AGGGAGTCAATTCCTAGGATA CAGAGAGATGGGACAACAGACA | |
| 144203 | OVOS2 | XM_495917 | ATCCAGTGAGATCCAGTTTAA TAGGCCTGATATTAATAGCAA | |
| 144348 | ZFOC1 | NM_152437 | AAGGAATGTTTAGAAGACCAA CAGCGCATAAAGGAGATTTAA | |
| 144360 | FLJ32894 | NM_144667 | ACAGGATTTCATACTTATTAA CTGCAGTAGCTAAGACTATAA | |
| 144631 | LOC144631 | XM_096642 | TTGGAGGCTAAAGGAAGTAAA CAGCAGTTATTTGGGTGTGAA | |
| 144742 | LOC144742 | XM_378388 | TACGTTGTTGTGGGAGATTTA CAGGGAATTCACAGCAAACTA | |
| 144809 | FLJ40919 | NM_182508 | CAGCATTATGAGGATTTACAA CACAATGTCAATATACATTAT | |
| 144962 | LOC144962 | XM_084990 | TTGCAATATTATTAGATTAAA CAGCAAGGGTATATTTATAAA | |
| 145173 | B3GTL | NM_194318 | AACCATATTATTGATGATGAA AAGGAATAATACTGTACATAA | |
| 145389 | SLC38A6 | NM_153811 | CCCATATACTGTGAACTTCAA AAGGTTATAGTAAATACTTAT | |
| 145414 | LOC145414 | XM_085138 | CTTGGGATTTACAGCAATTAA CTGCATGATGTCTCACAGAAA | |
| 145501 | THSD3 | NM_182509 | CAGCACCGACTTCTCACCTAA CAAGAGCGACTTCCTAATCAA | |
| 145645 | MGC33951 | NM_152448 | TGGATGGTTTCTAATAATAAA CAGAGCCTTGATTACATAGAA | |
| 145660 | LOC145660 | XM_085200 | CCAGCTCATTTGGTCACATAA CCGGCTGAGGTGTTCAATAAA | |
| 145773 | MGC26690 | NM_152450 | CAGGAATACTTTAACAATTAA TCCCAGTAGATTAGACTTCAA | |
| 145790 | UNC13C | XM_496070 | ATGCAGTAGGAGAACAATGTA CACTGCAAGCTAAATACATAA ATGGGAATGTTTAGTTCACTA CAAGCTGATCTAATACGTTAA |
| 145846 | LOC145846 | XM_096883 | AAGAAATTCATCAGCCTTCTA CAGATCAGTGGCAATCATTTA | |
| 145853 | LOC145853 | XM_096885 | CCGCTTCTTCACGGCGCTCAA AACCTCGGTATTCCAACTTTA | |
| 145946 | SPATA8 | NM_173499 | CCCACTGATTCAGAAGATAAA ATGGTGTATTCTGGATGTCTA | |
| 145978 | FLJ31461 | NM_152454 | CCAGTAATGAATAATCATTAA CACATGTGTCTTCACATTCAA | |
| 146057 | TTBK2 | NM_173500 | AGGCATCACCTCAAGATGAAA AAGAGTAAGCCAGCCAGTAAA CACATTGGTCATGACATGTTA ACGGCCCAGGTTAAATGGAAA |
| 146059 | CDAN1 | NM_138477 | CAGCATGAGCCCAATGATGAA CCGGAGGATGGTGACTGCCAA | |
| 146110 | LOC146110 | XM_018432 | CAGGGACTCAGTATGAACCTA CAGCATTCTATGTGCAAGATA CAGTATGAACCTACTGTCAAA CTGCAGTCGTTCCGACACAAA |
| 146167 | LOC146167 | XM_085347 | CGGGCTGGTCATCCTGGGCTA CGCTGTCTTCATCCTCATGAA | |
| 146310 | RNF151 | XM_370927 | CCGCCTGGAAGTCAAGTGCAA CAGGGCCAACATACCTTGTAA AAGGTTGTCCACATGAATAAA TCCGGTGGCTAGCCAGACAAA |
| 146378 | FLJ25404 | XM_375333 | CCAGTTCTGCACCCACATAAA GCCCAGTTTATTGGAGAGAAA | |
| 146723 | FLJ31882 | NM_152460 | CAGCACAATTTGCGATTGAAA TCCAATCATAGTTGAGAACAA | |
| 146849 | FLJ32734 | NM_144681 | CCGCGCCATGAAGAAAGCCAA CTGGGCGTTAAGGAAGCCCAA | |
| 146880 | MGC40489 | XM_373742 | CCGGAGCTGGCAGGTCTACAA CTCACTGGTTATAGTGCTATA | |
| 147011 | PROCA1 | NM_152465 | CAGGACGACGCTCACAATTGA CACAATTGAAAGATGGACTAA CAGACGTGAGCCGATCATTAA CAGGGTGGTCAGGGTGCAGAA |
| 147179 | WIRE | NM_133264 | AAGCTTGGCAGGTATATTATA CAGAGGATATATCCCAGCAAA CAGGTATATTATATTTCATCA CCCGCCTACAACAGAGAGAAA |
| 147199 | SCGB1C1 | NM_145651 | CACATGGAAATGTATCCTCAA ATGGACTTCCTGCAAACACTA | |
| 147339 | C18orf25 | NM_001008239 | AAGAATCAGAATGTACCATAA CAGTTTATTCTTGGCAATCAA | |
| 147381 | CBLN2 | NM_182511 | AAGGTCTTAAGGAGAAATGAA CTGAATCATATAAGTTGGTAA | |
| 147687 | MGC34079 | NM_152475 | AACGTCATTCATCCACATTAA CAAGAACTTTATGTTATCCAA | |
| 147808 | LOC147808 | NM_203374 | CCCATTGTGGTCCGTCCTTAA TTCAGTTCTGTTCCTATTAAA | |
| 147945 | NALP4 | NM_134444 | AACAACAAGCTTGGAACATAA AAGAAGCTAACTTTCATATTA | |

Table4

| | | | |
|---|---|---|---|
| 147968 | CAPN12 | NM_144691 | CACGCCCTGGGAAGAAAGCTA GACAGACACAGGAGAAATAAA CAAACCATCAATTAATATTAA CAGGGCAGGGTTGCTGAGCAA |
| 148003 | LOC148003 | XM_086001 | TACGTGTGTCTCAATGAGTAT AAGGTAAATGGTGAATACATT |
| 148022 | TRIF | NM_014261 | TACCACCTCTCCAAATACCAA ATCGGAACAGAAATTCTATAA CTCCCAGATCTTCGCCAGGAA ATCATCGGAACAGAAATTCTA |
| 148066 | ZNRF4 | NM_181710 | CACGCGGTCATTCCAACTCAA ACGGGCCGTGCTGGAAGACAA CAGCTTTGACTCCACCACCTA AAGAATAAAGTGGGTTTGAAA |
| 148103 | ZNF599 | NM_001007247 | AAGATTCATACCAGAGTTTAA AAGAATTATATTAAAGATTCA |
| 148223 | C19orf25 | NM_152482 | CAGGGTGTCCTGAGTCTCATA CAGCACATAGACAGCGATGAA |
| 148252 | DIRAS1 | NM_145173 | CTCGGCCAAGATGAACTACAA CAGGGTTGTGTCCAACATACA |
| 148430 | LOC148430 | XM_042500 | CCCTGGAGATCGCGGAGGCAA CTCCGTGCTGGTGCACCTTAT |
| 148479 | PHF13 | NM_153812 | CCCTGCCATTTAGGTGCCTAA CAGCCTGTTAATGAACAGCCA CAGCTGGTGGTTCTTAGGTTT ATGGCCCGTGTTCATAACTCA |
| 148523 | LOC148523 | NM_144697 | CAGCTAGGACATCTAGCTTTA CAGAAGTGTAAAGAACTCCAA |
| 148545 | FLJ32833 | NM_152488 | ACCGCTGAGAGTGGACAAGCA CAAGTGTGAAGAGTACAGGAA |
| 148870 | FLJ32825 | NM_152492 | CAGAATGGAAACCGCACCAAA CAGGAAGACGAGTGAACCCAA |
| 148898 | LOC148898 | NM_001008896 | CCCTCCTTGTATATCCTTAAA ACCCTCCTTGTATATCCTTAA |
| 149041 | KIAA2025 | XM_086409 | CAGCACCTTATCAGCAGGGTA CTCCATATTCACCTCATCAAA CTGCAAGCAATTCAGATTAAA CAGCAAACTTGAACCGACTAA |
| 149095 | FLJ32785 | NM_152494 | AACAGACTCATGGACATTAAA CACCGTGGAGCTGCAGATCAA CAGAGACAGTGATGGAATCAA TACAATGACCTATTGAAGAAA |
| 149466 | MGC52423 | NM_182517 | CCCGGCCAGCTTCTTAATGAA CAGGCTCTGCACAGTACCCAA |
| 149478 | LOC149478 | XM_378860 | CACCACAGGCTCGGCCGAGAAA CCCATTGACCTCCAGGACAAA |
| 149483 | FLJ33084 | NM_152500 | CAGGAGATTCGAGAACTACAA CCGGCGAGAGGCAGAACTTTA |
| 149620 | LOC149620 | XM_086604 | AACATTCATAAGTAGGAAATA CTGCAGAGTACAAGTTGCAAA |
| 149685 | SMAF1 | NM_174906 | TAGCCACACATGCGCCATGAA AGGGTGTGGGTCCATATTAAA |
| 149708 | WFDC5 | NM_145652 | CCAGAGGCTAATTCTGATTTA CAGAATGAATATTCTTGCAGA |
| 149709 | LOC149709 | XM_086637 | CAAGAAGTGTTGCTACTACAA CTGAGCTCTTCTAACACTAAA |
| 150051 | LOC150051 | XM_097792 | CTGGGATGTATGTTCCTTACA ACGCTGTGGAAGATAAACCTA |
| 150142 | C21orf121 | NM_198078 | CCAGATCTCCGTGAAATTAAA CCCACTGATGGTGGTTCACAA |
| 150147 | C21orf128 | NM_152507 | CAGGCAAATGTTGGGAAGCAA CACAAGTGAGTTAACATTTCA |
| 150159 | LOC150159 | NM_139173 | CCTAATAATACTGCACATGAA CATGCATACCACAGAATCAAA TAAGAGGAGTATTGAATGTAA CTCATTGATCCTAATAATACT |
| 150209 | FLJ30473 | NM_144704 | AACTAAGAAGGTCGTGTTCAA CCGGGTGAAGTTCTACATGCA CAGAAGATGCTCAACCCTCAA AACATGTTAGACATTACCGTA |
| 150244 | FLJ31568 | NM_152509 | TCGCAGCCTGTCCTACAATAA CTGGCATTTCTTTGATAACAT |
| 150248 | C22orf15 | NM_182520 | CTGGCTGAGGATGGCAACCTA CACCAACTCCACGCACATATA |
| 150291 | FLJ35801 | NM_153044 | CACAGTGATACAAAGAATGAA CAGGTTAGTGGTACAAAGTCA |
| 150353 | DNAJB7 | NM_145174 | CCAAACATGTATCTCAATATA CTGCGGACAATTAGTATTCAA CAGGTTCAGACTACACAATTA ACGACAGTCCGGGTAACATAA |
| 150383 | LOC150383 | NM_001008917 | CCGGGAGAACATCAATGCTTT ATGCAGTTTATTCTACTCCAA |
| 150696 | PROM2 | NM_144707 | CAGCATCATCTTTGCCGTCAA CTGCATCTTATAAACCAGCAA |
| 150709 | FLJ25415 | XM_371586 | ACGGGTATTGTGTATAGGAAA CTCAATTACATGAACAATTTA |
| 151011 | NA | NM_144710 | CTCCAGGAAAGTAATGTTCAA AAGGTGGATGTGAAACATGAA |
| 151121 | LOC151121 | XM_379133 | TTGAATGAGTACAGTAATGAA AAGCAACACATCTAACTTATA |
| 151242 | PPP1R1C | XM_087137 | AATGAGTTTAATTGACTTCAA TAGGATTTATGTTATAGATAA TTGGAACTGGTTAGTACCTAA CTCGATCTGGAATTACAGCTA |
| 151306 | GPBAR1 | NM_170699 | CTACCTGTACCTCGAAGTCTA TCGACCTGGACTTGAACTAAA CAGGACCAAGATGACGCCCAA AAGCCTCATCATCACCGCGAA |
| 151313 | DKFZp434N062 | NM_199336 | CCCGACATAGGATGAGGGCAT CTAACAAGACGCAATGGGAAA |
| 151443 | LOC151443 | XM_087200 | AAGCAAGGATTCTACATCAAA ACGGCTTCTAATAGAGCTATA |
| 151449 | GDF7 | NM_182828 | CCGGCCCTGGTCTCCACTCTA CCACTTCATGATGTCGCTTTA GCCGTTGCACGTGGACTTCAA AACAACGTTGTCTACAAGCAA |
| 151516 | FLJ25084 | NM_152792 | TAGGAGCTCAGTACATGTAAA AAGGTGGCCAATGGTGCTGAA CAGGAGCTATCCCACTGAGAA CAAGCTGCTGTTGATGACAAA |
| 151531 | UPP2 | NM_173355 | CAGAATAGGATTCGAGATTTA CTCACACTAAATTAAATTCAA |

Table4

| | | | |
|---|---|---|---|
| 151636 | DTX3L | NM_138287 | CAGAGGGATATTTGCCTCCTA AAGAGACAAGTCCTTACTATA TGGAATGATATTCACCACAAA TGGGCAAGCATTGGTAATAAA |
| 151742 | PPM1L | NM_139245 | CTGTAGAGTCACATATATGAA AAGCGTGAACCCATATTGATA CTGGTGGTCTTAGGTCTATAA CAGGACTACGAGAAAGACAAA |
| 151760 | LOC151760 | XM_098117 | CACCTTCAACACCACCACAAA CCCACAGTAAACGCAAAGTAA |
| 151887 | URB | NM_199511 | TCCAGTAATATTGGCCACAAA AAGGACCTTATTTGGCATATA |
| 151963 | LOC151963 | NM_178496 | CTGCAAAGCCATCATCATTAA AAGGGTAAGAAGGACAATGAA |
| 152028 | MGC34923 | NM_144717 | CAGAATAATCCTTGAGAGAAA CACCCAGCACTTGCAAGGCTA CACGGACAAGGGATGAGAGAA CTGGAGAAACAGTGTACTATT |
| 152118 | LOC152118 | XM_098163 | CAGAGAGAAGTGTAAATGACA TAGTTGTTTGGTCACAAATAA |
| 152138 | LOC152138 | XM_093644 | TTCTGCAGAGCTGGACTTCAA CAGAACAATCTCCCTGGGAAA |
| 152404 | IGSF11 | NM_152538 | CAAGAGCAGAATAGTACTTAA AACCATTTCAGTATTGATAAA |
| 152485 | LOC152485 | NM_178835 | AAGGAGAACACTGGTATGGAA AACACAGATGGTGAAGATTAA |
| 152519 | LOC152519 | NM_207330 | CAGAATTTCTACAAACTTTGA CAGAAAGGTATAATCATGTAA |
| 152579 | SCFD2 | NM_152540 | TACAAGTTCATTAGTTCTAAA CAGGCTGTCTTTCACAATCCA |
| 153297 | LOC153297 | XM_098350 | TCGGTCCTATTAAATAACATA CTCATTTATAATAGTGCCAAA |
| 153571 | CEI | NM_178569 | CCCTGAGATTTGTGTTGTCAA AACGGCATCGAGGCATTTCAA |
| 153745 | MGC26988 | NM_130899 | ACGGAGATGATCGTCTTTGAA AACGAAGAGAACCATGAGCAA |
| 154092 | LOC154092 | XM_379434 | CCGGGTGTGCCCAGAAGTCAA AAGCACTTAATCATACTTAAA |
| 154141 | OACT1 | XM_371801 | TCGACTTGCTATCAAAGTGAA CCGAATATACATCTTCCACTA |
| 154197 | PNLDC1 | NM_173516 | AAGCAGAATATCCACAGCCTA AAGGAGAGGCAAACAAGTATA |
| 154386 | C6orf195 | NM_152554 | CACGGCCGACTTAGTAATTCA CAGAGGGAGATTAGCAATGAA |
| 154810 | AMOTL1 | NM_130847 | TAGGTTTGCAAACAAAGTTAA CAGGAATGATTTGAACTGATA |
| 157724 | SLC7A13 | NM_138817 | TACCATTGGTAAAGTCTCCAA CCCAATCTATCTATACCTTAT CGGGTTCATTATGGTCTATAT AAGGTTAAACATATGATAGAA |
| 157813 | LOC157813 | XM_098828 | ACCCGCCATAGCAGTGACCAA CCGAGAAGCCCTGGAGATAAA |
| 157869 | RPESP | NM_153225 | CACGGTGTGTGTGGATTGTCA CAGAGGATGCTGGATACTGTA |
| 157983 | C9orf66 | NM_152569 | CTGAGACACTAAATAAATATA CGGCCTCAAACTCAAGATAAA |
| 158038 | FLJ31810 | NM_152570 | CAGTAGGAGGATAATTAACAA TGCGGGAATTATCATGATCAA AAGGCGTTTCATCACCACCAA ACCCAGGAGGTTCAACATGAA |
| 158056 | AEGP | NM_206920 | ACGGCGCTGGCTGCAGAAGAA CACGACCACACCACAGGCCAA CAGGCTGCAGGTCTCAGGATA CTCCACCTGGCTTATTATTTA |
| 158228 | C9orf122 | XM_379636 | CAGACCGAGTGAGCCCAGCTA CACGCTCAGCCGAGCTCATCA |
| 158248 | TTC16 | NM_144965 | CTGGTGGACTTCTATGCCTTA CAGGACCTCACCTACTATGAA |
| 158399 | ZNF483 | NM_001007169 | TAGAGTTGATTTCCCAGCTAA CAGGATTTGGGTAAAGTCACA |
| 158511 | CSAG1 | NM_153478 | CCGAACCAGGAACTCAATCAA CAGCCAGTAATTCCCAGGCAA |
| 158835 | DGAT2L4 | NM_001002254 | CAGCGATTATTTCCCTCTCAA TTGGATGTGATTGAACTTTAT |
| 158880 | USP51 | NM_201286 | AGGCCTGAGAGGGCTAATCAA CCAGAGACTAGGAAACGTAAA GAGGACTTACTCTACAGTGAA CCCAGAGACTAGGAAACGTAA |
| 159163 | RBMY1F | NM_152585 | TAGATGTAATATCTACCTTAA CAGGCTCTTTGTCCTACTAAA |
| 159371 | TMEM20 | NM_153226 | CTCCATTATTCTCTAAATGAA ACGCTTTATGTTATACATTAA |
| 159491 | C10orf64 | NM_173524 | CTGGGACATGCTGGAAAGGAA AAGGCTGAAGACAGAAATGAA |
| 160065 | PATE | NM_138294 | CTGGATCACTTTCAATGAGAA CTCCATCTTCTGCACACGAAA AAGGAGGAAAGGGTAGTTTAA AAGCCTGGAAAGAGTGATGAA |
| 160287 | LDHAL6A | NM_144972 | AAGAATGTGTTTACTGTGTTA CAGAAGGAGCTCAAGCTTTAA |
| 160760 | TA-PP2C | NM_139283 | CTCAATTCTCAGGGACTTTAA CCCTCAAAGCCTAGAAATTTA CACAAATTGTACGTAATGATA AAAGGGATGGTACAAGTCTAA |
| 160851 | DGKH | NM_152910 | TGGGAGTTCGATTATCAACAA CTCCTAGTGCTTAGTGGTCAA CCGGATCTAGATTCCGTAGAT CAGGTGGAGTATAATGACATA |
| 160897 | ITR | NM_180989 | CTCCCATTTAACTGAGTATAA TCAGTTGACAATGACCATGAA CCCAATCATAATTAAGCCGTA ATGCCTCATCTTAATAGCTAA |
| 161003 | STOML3 | NM_145286 | CTCCAGATAATTTATAAGTAA AAGGCATAGAATATAAATGAA |
| 161725 | C15orf16 | NM_130901 | CTGGTTCTGGCCTATGATCAA CAGGACGACATTGCCCAAGAA |
| 161823 | LOC161823 | XM_091156 | CAGCTTACTAGTAGCCCTGAA TCGAAGTTTCTCATCAGGAAA TAGGTATTTGATAGCAGTTGA TAAGATAGTTCTGAAAGGTAA |

EP 2 295 543 A1

Table4

| | | | | | | |
|---|---|---|---|---|---|---|
| 162073 | LOC162073 | XM_091331 | TTCCTTGAAGGTGAACCGTAA | ACCCAAGAAGAATTGGTTCAAATA | | |
| 162333 | FLJ35757 | NM_152598 | AAGAGAGAGGTTGTCAAGAAA | AAGCAGAAGAATACTACTTTAA | | |
| 162394 | MGC19764 | NM_144975 | CAGAGTTAATTCACTATTAAA | TCCATTTAATTTAATAATCAA | | |
| 162466 | PHOSPHO1 | NM_178500 | CCGTCCCTATCTATTCAGTTA | TACAACTATAAAGGAGGTGAA | TCGGGTCCGGACAGCCAGTAA | CGCCAACATGTGCAAGCACAA |
| 162494 | RHBDL4 | NM_138328 | CAAGTGGTACTATGACAGCTA | AGGCATGAAGTGCCAGTTCAA | | |
| 162605 | KRT25D | NM_181535 | CTGGGAATTCATCTAAAGATT | AAGACAGTGGTTGAAGAGCTA | | |
| 162835 | LOC162835 | XM_091830 | CTGGAACACCTCATCATGCCT | CGCAAGGTGGTTGGCGCTGTA | | |
| 162993 | LOC162993 | XM_091914 | CAGCACTTGCTAAACATCTAA | CAGGTATTCCACATACCTTAA | | |
| 162998 | OR7D2 | NM_175883 | CCCATGCGTATACATTGTTTA | AACAATTAATCTCCAAGTAAT | ACCAATTACCTGAATAGTGAA | CAGCACGTTGATATACTTTAT |
| 163259 | FLJ37099 | NM_198459 | GTGGGTCTAGATATAAATGAA | AAGGAATAGACTCATCACATA | | |
| 163479 | FLJ35838 | NM_173532 | CAGCACTTATCCAAGACAAA | CAGGGCAACCTGAGATAACAA | | |
| 164091 | MPRA | NM_178422 | CCAGGGAGAGGGAAAGAGTTA | TGGCCTGGATGCAATAAAGTA | TCCTTGGAAGTCCAAACTCTA | CTGAATGGTCTCTTGCTAAGAA |
| 164118 | LOC164118 | XM_089384 | CCCTAGCATCTTGGTACCCAA | CAGGCTGGCCAGAACCATGAA | | |
| 164153 | LOC164153 | NM_203412 | CAGACCAAGAATAGCAGGAAA | CCCGATGTTCCTCACAGTCAA | GAGGCAGCAGCTGATCAGTAA | CTGCTTGTTGCTTCCAGCTTAA |
| 164395 | LOC164395 | NM_0001008409 | CTGCATGTTGTGGACATGGAA | CACAGGTTTATTTCAAGGCAA | | |
| 164656 | TMPRSS6 | NM_153609 | CCGCCTGATGTTGTTACTCTT | CCGCCTGGGAACTTACTACAA | CACGGCCGGGATCACCATCAA | |
| 165140 | OXER1 | NM_148962 | CAGGGCGAGGTCTTCTCTGGAA | GTCCCTTCCAATATTAATAAA | CCGGGTGGAGGAGACAGGCAA | CCAGCTGATAGTGCCAATCAT |
| 165257 | C1QL2 | NM_182528 | AAGGCCACTCTCGATTCATAA | CCGCATGATCTGGGAACCTTA | | |
| 166614 | DCAMKL2 | NM_182619 | CCGCACTATCTACACCATCGA | TACGTGTGTGCATCCAATGAA | CTGAGCTTGACCGTTGCATAA | ACAGATGCTCTGAATCATCAA |
| 166647 | GPR125 | XM_291111.1 | CAGCCTTACTTTGCTCTTATA | CAGGCTTGTGATTTAATATAAT | CAGCTTGATAATAGTCTGACA | ATGGATTTGACGGGATCTGAA |
| 166752 | FREM3 | XM_094074 | AAGGAAGGGCTTTATGAGAAA | CTGGCTGATCGTTATGATGAA | | |
| 166979 | FLJ37927 | NM_152623 | ACCGCTGAAAGTCATCAACCCA | CACTGCTAGGTTGAACATCTA | CAAGGACAACCTATTACCAAA | AAGGAGATTGCAACTGGTCAA |
| 167227 | DCP2 | NM_152624 | TTGGAAATTGTCAAACATTTA | CTGGGTTATCAGAGTAATGAA | | |
| 167681 | PRSS35 | NM_153362 | CCGAACCTTAACCAGGGTGAA | AAGGGAGTATCTGTTAGGAGA | TACGGCTAACAGAGACCTGAA | CCGTAGTGAGATCACTTCATA |
| 167838 | C6orf198 | XM_371849 | AAAGACTTCCACTGCAATATA | TGGGTTTCTCTCTCCCTTGTA | | |
| 168433 | RNF133 | NM_139175 | CAGTGGAGTGATCATCTATAA | GTGGTTATGATTGGTAACTTA | ACCGGAGATGGCAGCGATTAA | AACGCTATAAGCCTAATGACA |
| 168451 | THAP5 | NM_182529 | TTGGAAGATGAGAAAGAAGTA | CACCATTTAAGATACGTGAAA | | |
| 168507 | PKD1L1 | NM_138295 | CACCCTTATGGCATCATGGAA | CACGAATACAGGATGCATTAA | AACGAGTTTCATGGAACCGAA | CAGGTTGACCTCAAAGGTGTA |
| 168544 | ZNF467 | NM_207336 | GTGGATGATTCGGAAGGTGAA | CACCAGCTCATTCACGGCGAA | CCCGCTCTTCTTCTGAGCCTA | CAGGAAAAGAGTGGTTATTTAT |
| 169270 | ZNF596 | NM_173539 | CCGCTCGGATTTGTCTTCTTA | CAGCTGTAGCGTTAACACTAA | TACCACTTTGCTCAACCTAAA | CAGCACTTCTATAATAGATCA |
| 169436 | C9orf96 | NM_153710 | CTCGGATCGAATAACGATAAA | CTGCCTGGTGATGGAGTTCAA | | |
| 169522 | KCNV2 | NM_133497 | CACGTGATCCATCCTCCTCAGA | ACGGAGAATGAGGGCAGCCAA | TACGAGGAGCAGCAGACGAA | CTGGACAGAGGGCAACTATAA |
| 170082 | MGC17403 | XM_372198 | CGGGAGCAAAGAGATGATTAA | CTGACCCATAATGGACATTAA | | |
| 170302 | ARX | NM_139058 | ACCACTCAAGACCAAATGGAA | GACGCCTGATATTGAAGTAAA | | |
| 170463 | SSBP4 | NM_032627 | AAACATGTACACTATCATGAA | CCGCGAGAAGTTGGCGCTGTA | | |
| 170626 | GAGED4 | NM_130776 | ACAGGCAACCACCAGGTTTAAA | CCGAAGGAGGTGACAGGCAA | | |
| 170692 | ADAMTS18 | NM_139054 | AACCAGCATAAAGCAAATGAA | CAGGTTTAATAAGGACACGAA | | |
| 171568 | POLR3H | NM_138338 | CTGGTTGTTGTTTTCTCAA | CTGCACATTCATGATCAGAAA | | |
| 192669 | EIF2C3 | NM_024852 | AAGGCTGTACAGATTCACCAA | CGGGATGAAATGGCTCATGTA | CTCCTATAGGAAGTATCGCAA | CTGACTGATTCTCATCGGGTA |
| 196051 | PPAPDC1 | XM_113641 | ATCCTTGAATTTGCAAGTGAA | AACAGACAAGACTGAAATTAA | | |
| 196264 | LOC196264 | NM_198275 | CACAGTATCAATATTTCATTA | AACCACTTATTTATTGAATAA | | |
| 196335 | OR56B4 | NM_0001005181 | TACCATTTGCTGGGCATATTA | AGGGTTCATCATGCTCAGGAA | CTCATCATAATCACCATTCAA | ATGGCAGTAGAGACAGATACATA |
| 196337 | LOC196337 | XM_113696 | CCCATCTTTGATACCTTCATA | CACCTTCATCCTCTCAGATTA | AAGACACAATACGGACACTAA | CAGCTTATTTATTAAGATCCAGAA |

Table4

| | | | | | |
|---|---|---|---|---|---|
| 196403 | DTX3 | NM_178502 | TCAGATACAGTTCTCCCTTAA | CACCGTGTGTCTATCCTCATA | CGGCACCATTGTCATCCAGTA ACCGAATGTCATCACCTGGAA |
| 196463 | LOC196463 | NM_173542 | CAGGCTGATGAGGTACAATGA | CTGGATGAACACGGTGGTGAA | |
| 196743 | PAOX | NM_207126 | CTGGACAATAAAGCAAGTTA | CACGGAGATGCTGGACACATA | CCGGACTTGAGCTGAGTTCA CGGAGATGCTGGACACATAAA |
| 196792 | FAM24B | NM_152644 | CTGGAGCAGAGAGGAGCCATTAT | TTGAATAAATGTACACTATTA | |
| 196883 | ADCY4 | NM_139247 | CCCGCCTTCAAATACTATGAA | CCGGCGCAACGAGGATCTCTA | CCCGTAGTAGCTGGAGTTATT CTCGCCTTCTAAGAACCTCAA |
| 197131 | UBR1 | NM_174916 | CACGTCATATACAGCCTACAA | ATGCAGTAATATGTGACCTAA | AACCCACAATATGGACAGAAA CAGGAATATTGGGATACTGTA |
| 197187 | MGC23284 | XM_378628 | AAGGCTTTGTGACTAACTCAA | CCCGCCCAGATATAAATATAA | |
| 197258 | FUK | NM_145059 | CAGAATCATGGCGTCTACCTA | TCCCTATGGCTTAGAGTTGTA | TGGCTTAGAGTTGTAGACTTA CCGAAGGGAGTTGATTGGACA |
| 197322 | LOC197322 | NM_174917 | CTGGCCTCCCTTAAACCTGAA | CCGGACCTCAGTGGACATCAT | |
| 197358 | NOD3 | NM_178844 | CTGGCTGGGAAGGGCAGTCAA | CAGCTTAGATTTACAGGAGAA | CTCCGGAGACTCAATCTTCAA CAGGATGATCTTCAGAGGCCAT |
| 199692 | ZNF627 | NM_145295 | ACGGTCTAATTTGTTGTAGTAA | AACCATGTGCATACAACTTAA | |
| 199897 | LOC199897 | XM_117152 | CAGGACTACTTCATGAGCCAA | CCCACTGGGTTGTGAAATGAA | |
| 199953 | RP13-15M17.2 | NM_001010866 | CAGCAGTGGGTGAGCAGCCAA | CAGCCTCAGTTTGCCATCTAT | |
| 199990 | FLJ31031 | NM_182533 | ATGGATGTGCAAGAACCTTGAA | ATCCTTCACTTTAATTTATAA | |
| 200081 | DKFZp451J0118 | NM_175852 | CGCGGAGGAGCAATATCGACAAA | CACATACTGTGTGACAATAA | CTGGTGTATCCTTCACATCTA CCGGACCTATGTGGCAAGGAA |
| 200172 | FLJ23878 | NM_144990 | ACCCATCTGTAGCCAAGAA | CTGGAACCTTGTGACTGTCAAA | |
| 200186 | TORC2 | NM_181715 | CAGGACTTCATTCACCTCTCA | CCGGCCAATGAGTGACTTCAA | |
| 200213 | LOC200213 | XM_114156 | CCGCTTTAAGCAGGCCACCAA | CAGGGTTATTCTATGGAGTAT | |
| 200312 | LOC200312 | XM_117224 | ACCCACGAAAGTACAAGATCA | CCGCATCTGAAGTTACCCAAA | |
| 200315 | APOBEC3A | NM_145699 | AAGCAGTATGCTCCGATCAA | CAATGTATTAATGAAATGAAA | |
| 200350 | FOXD4L1 | NM_012184 | TAGCGTTCCTGCTTCTTGCAA | CCGGACGTGTTGCAAAGGAG | |
| 200407 | CREG2 | NM_153836 | CAGCAAATCTCTAGAAATTTA | AAGGAGTAGCTTTGACATTAA | |
| 200424 | MGC22014 | XM_371501 | CCGGATCGAGAAGGTCATCTA | CCGGCTCTATGAAACCTTCAA | |
| 200491 | LOC200491 | XM_117239 | AAGACATGCAGTCTCATTGAA | TCCCTGGAGCTCAGAAGCCAA | |
| 200539 | ANKRD23 | NM_144994 | AAGAAGAAACTTGAAAGATTT | CCGGGAGAAGCTGAAATTGGA | |
| 200624 | LOC200624 | XM_117257 | AGCCACCATGCTGACAAGTAA | CAGGTAGTCTGTGGAGCATTA | |
| 200728 | TMEM17 | NM_198276 | ACCATGTTTCTTGACATGGAA | AAACAGTATATTAACCCTAAA | |
| 201163 | FLCN | NM_144606 | CTCCTTTGCCTGGCTCCTGAA | CGGTCTTTGCTCCTTTACAAA | CAGGCCTGTTGCAGTCTCCAA CCGGGATATATCAGCCATGAT |
| 201175 | LOC201175 | XM_371057 | AAGGGAGTTCCTTACTTACTA | AAGGGATTTCCTTACTTACTA | |
| 201516 | ZSCAN4 | NM_152677 | AACCAATAAATTTCTGTTGAA | AAGGGATTTCTGAGTCTCAA | |
| 202018 | FLJ90013 | NM_153365 | AGGGTACTTCCTTGAACATAA | TACCACCAGAATACCCTTAA | CAGTGTTAACTTTAAGGTATA TCCGCTTGAATTATTCTGTTA |
| 202020 | FLJ39653 | NM_152684 | CCGCATATAAATGGGCCTTGA | CGGAACCTTATTATTTATGAA | |
| 203054 | ADCK5 | NM_174922 | CTGGGTCCTCCAGGACCTGAA | CTGGGAGATGCTCAAGTTTGA | CCGCTACTTCCTTATGGCTAA AAGGCCTTTGCTGAGCAGATA |
| 203074 | UNQ9391 | NM_198464 | CAGACCAATGCTGCTGACAAA | TAGCCCATCCATGAAATAAA | CACCTCGTTGGTGAACTACAA AGGGAAGAACTCGGTATTCCA |
| 203076 | LOC203076 | XM_114621 | CAGGTGGTCAAGTTCACAGAA | AAGGAAAGAAAGCGAAGGCAA | |
| 203100 | HTRA4 | NM_153692 | TACCTGAAACAATCAATTAAA | ACGATGTAATTGTCAACATAA | CCCGCTCAGGAGGAATTACAA TAGGCAGTTCTTGGCAGAATA |
| 203859 | TMEM16E | NM_213599 | TACGTTGATGACGTAAAGAAA | CAGGATCATGCTTGAACTTTA | |
| 204219 | LASS3 | NM_178842 | AACAGTAATGACTACAATAAA | CTGGAGATACCTGGAAATGAA | |
| 204801 | NALP11 | NM_145007 | CAACATAAGATTCGAGTTAAA | CAGCGATATCTGTCAATATAT | GCCAGGTTCAATAATACCTAA CAGCCATGAGAACGTCAAATA |
| 204851 | HIPK1 | NM_152696 | CGGGCAGTGCTGCTACATCAA | CAGGAGTTCTCACGCAGGGAA | CTGAGCTATGTTTGTAATGCA AGGGAAGCTGTACACCACTAA |
| 205147 | FLJ38377 | NM_152698 | TCCCAAGTGTTGAGAATCCAA | CAGGAGAGCCTAGGACTCAAA | |
| 205564 | SENP5 | NM_152699 | CACGATTGTGGTCACCTTGAA | CAAGTTTGCAAGCTAAGAAA | GTGCCTGATTGCCACCACTAAA CAGAACAACATCGTTCTAATACCA |
| 205655 | LOC205655 | XM_497917 | CTGGTGCTCAGTTGTATGCAA | AATGATGAAGTTGGAAATGAA | |

200

Table4

| 205860 | FLJ25801 | NM_173553 | CACATGGTGTGTGGGATACAA CAGGTGGCAAGTGGGCATATA CAGCATGTTCAGAGTACTCCA CGGGCAGATATCATTCTACAA |
| 206426 | MGC26597 | NM_152700 | AAAGTTGAGGTTATCCTGTAA CTCTTAGTTTGTAATTTAATA AGGGTACCACTTATTCTTCAA ACGGACCCATATACTTTATAA |
| 219402 | MTIF3 | NM_152912 | GAGGAGATATTTCATCAAATA AAGGCATATAAAGAAACTCAA |
| 219414 | LOC219414 | XM_169434 | CTGAGTGGATTTGGAAACTGA AAGGAGCTCGTTGAAATATCA |
| 219429 | OR4C11 | NM_001004700 | CCGCTTGCACGTCTCACATAA CTGGATAGGGTCTTTAATACA CCCTATTTGATTGATCATTAT TAGGGTCTTTAATACACTCTA |
| 219445 | OR7E5P | NM_153024 | TTCAAAGTTGTTTACTTATAA CAGTGACATCAATAACATATA TAGCGTAAACATCACGACTGA AACGTTGATGAGGAATAAATT |
| 219464 | OR5T2 | NM_001004746 | CAAGTTTGTTCTAGATTTCAA CAGGTTATCAATAAAGTATAT |
| 219469 | OR8H1 | NM_001005199 | CAGATGGCCCTCTTTATACTA TACGACATTGAAATCATGATA |
| 219487 | OR5M11 | NM_001005245 | CACCATCGTCTTGGTGTCCTA AAGACTGTTGAGGAATCTAAA |
| 219537 | CHASM | XM_166203 | AAGGAGAAGGCTACTGATGAA CAGGAGATGACTGGCAGGAAA |
| 219770 | CX40.1 | NM_153368 | CAGGGTGTCAGGGCACACGAA CAGGAGGATCGTTTGAGAATA |
| 219927 | MRPL21 | NM_181512 | AAGGATCTTGTTCGAGTAGAA CTGATCTTAATTGGAAATGAA |
| 219928 | MRGPRF | NM_145015 | GGCGGTGTTCTCCATCCTGAA AAGGCATTATCAGTGAGCAAA TAAGGCATTATCAGTGAGCAA CAGGAGAAGAAAGATCTGTTT |
| 219972 | MPEG1 | XM_166227 | ATGGACGAAGTTGGAGTTCAA CACCGTGAACTTCAAATTTGA |
| 219995 | MGC35295 | NM_152717 | CAAGGCTACCACAGAAGGTTA CCGGAATCCTCCAGAATGGAA AAGGACAAGGCTACCACAGAA CGCCTTCAGCGCAGACTTCAA |
| 220001 | FLJ32009 | NM_152718 | AAGCTGCTTGTTCCCACCCAA CACAGGCAGAATCTTCTATAA |
| 220032 | LOC220032 | NM_182833 | ATGGCTGAATACTCCAAATAA AAGGAGTGACTGTGCATATAA |
| 220081 | FLJ32682 | XM_495949 | CAGATGGAAACTGGTAATTAT AGGCAAGTTATTCATCACAAA |
| 220158 | GTSCR1 | XM_496277 | CAGGATAGAAAGACATCACTA CTGGTTTATTGCTCCTATTTA |
| 220164 | DOK6 | NM_152721 | ATCGTATGAACAGTTATTTAA TACGCCAAACCTGGATATTTA AACCATAATTTGGTGCATTAA CTGAGCAACATGAAAGATTAA |
| 220416 | LOC220416 | XM_166971 | CCAGTAGAAGTTCAGAAGTTA CTGACTGTTGATGGGAATGAA |
| 220441 | RNF152 | NM_173557 | TAGGTTAGATATAGACACTAA TAGGATTTATTTGGTATTAAT |
| 220717 | LOC220717 | XM_165448 | AAACAAGGAAATAAAGGCTTA CGGGAAGGTCGTGGTGCTGAT |
| 220972 | MIR | NM_145021 | CTGCTAGAGTCTACAGAAGTA TCCAGCGGGATTGACACTCAA TGGGAATTAGTGGATGCTAAA TGGATGCTAAATGGCAATTAA |
| 221044 | C10orf49 | NM_145314 | TTGCTTATGGCTTACAAATAA TACGAGGAACAAAGGAATGAA |
| 221060 | C10orf111 | NM_153244 | AAGTACCTATGTAGAACCTAA CAGGGAATCTTGAGCCCGAAA |
| 221078 | NSUN6 | NM_182543 | CACCACGGATTCAGTTACTAA CAAGACACTCGTCACTATTAA CTCGTCACTATTAATTCGGAA CAGAAGCAGTTTAATGGATTA |
| 221120 | DEPC-1 | NM_139178 | AAGAATCACTATGGAACCAAA CACGCACATTTGAGATGAGAA CAGAGAGGATATAACTTATCA ATCGCTATCATCTTTAGGCAA |
| 221178 | SPATA13 | NM_153023 | CAGGGACATGCTGTACTACAA CACGGTGATTACAGCAACATA |
| 221188 | GPR114 | NM_153837 | CGGGCGTGTCTACAACATCTA AAGCGGCTTTGTTTCAGCCTA AGGCATGGGTCTCGAATCTTA AGCGGCTTTGTTTCAGCCTAA |
| 221223 | FLJ31547 | NM_145024 | CTCAAGGTGCATTACCCGAAA TTCCATTATCGGAGTCAATAA CCCGCAATCATTAGCTTCTTT CAACAAGATTTCTTCAATAAA |
| 221241 | C18orf20 | NM_152728 | CTCCTATTAAACATTTGTTTA CTGGTTTATCAGCCACACTTA |
| 221261 | C6orf184 | XM_168053 | ATGGAGGACCATTCCCAGTTA CCCAGATACCCTAGAATATAA |
| 221357 | GSTA5 | NM_153699 | CACAGAAGGTATAGTAGATTT CACGACAGTGACAGAGTTTAA |
| 221391 | OPN5 | NM_181744 | CAGCCTTTCACCATACTGGAA CAGCCTTTCACCATACTGGAA CACCGTAACCACAGTCAGGAA CGCTGAAATAATGACTATCAA |
| 221393 | GPR115 | NM_153838 | CAGGTAAATGGTCTGGTGCTA AGGCTCTCACTTTAACATTAA TAGGCTCTCACTTTAACATTA TAGGAGAATGTTCCCACTATA |
| 221458 | C6orf102 | NM_145027 | CAGCGTTACCATCGATGACAA AGCGAGAAAGCTACAAATTTA AAGATTGTCAAGAACCATTAA ACGGTCCCAACTCCTCTCTAA |
| 221527 | ZBTB12 | NM_181842 | CGGCAAGTGCTTCACACAGAA CAGCCAGTTCATTGAGCCCAA |
| 221656 | AOF1 | XM_173173 | AACCTATTTCATCACTCTAAA TTGGGTGATAGGACACTTTAA AGGGACTAAATATGACTTTAA ATCGATGCGGTATGAAACCAA |
| 221662 | RBM24 | NM_153020 | TAGTAGCTAATAAGAGAGTTA TAGGAGCGTGGCAGTGGGTAA AAGGTTAATGATAATGTGGTT AACGATATATTGCTAGTTTCA |
| 221785 | ZNF498 | NM_145115 | AAGGTAAATGGTAATGACTAA AAGGGGTTTCATTTCTGTTTAA |
| 221830 | TWISTNB | NM_001002926 | CACATTCATCTTAACATTGAA CTGGTTGAAGCTGGTAATCAA |
| 221895 | JAZF1 | NM_175061 | ACGACCTATTAGAGTAATCTA CAACAGCTAAACAGTCATAAA ACGCAACTAGTTGGTGCATAA ATGAGCCTTATTGACAATTAA |

Table4

| | | | |
|---|---|---|---|
| 222171 | LOC222171 | NM_175887 | CTGGTGGAAATCGCTCACCAA CCCGAATCACTTGGCAAATAA |
| 222183 | FLJ37078 | NM_153043 | TACATTTGACCCTGAGGTTAA CAACATGGAATTTGCAAGTAA |
| 222229 | DKFZp434K1815 | NM_152892 | AAGGACCAGGGACACAGCTAA ATGGCAAGGATGCGTCCTCAA CACAGCTAACTAACTTATTCA GACGGTCAATGACAACCTGAA |
| 222255 | ATXN7L4 | NM_152749 | CAGGGAGGTTATGAGGCTTAA CACGCAGAAGTCAGAATCAAA |
| 222389 | C10orf30 | NM_152751 | CAGGTTGTACTCCATGAGAAT AGGAAATTAATGCAAATTCAA |
| 222546 | RFXDC1 | NM_173560 | CACGCAGATTGTGAAGGATAA ACCCAGTTTAATAATGACAAA |
| 222584 | C6orf143 | XM_294139 | CAGAATTATGGGTATGATGTA TAAGATTAACTTGAAGTTCTA |
| 222642 | BZRPL1 | NM_001010873 | AAGCACTTGGAGGAAGGCCTA CAGCACTGATCTGGCATCCCA CCCATTCTACAAAGTCTTATT CAGAGGAGTGCTGTGGAGAAA |
| 222658 | C6orf69 | NM_173562 | CGGATGCTCATTAGAGGTTAA AACATTCGCATTGGAATTGAA TAGCATCACTGTGGAAATTAA CCGGTCTGAAGTCATCTATAA |
| 222698 | C6orf194 | NM_001007531 | AAGCAGCAGTTCAGATTCCAA AAAGAGAAAGATGACAAGTAA |
| 222699 | LOC222699 | XM_498063 | CAGATGCTCTTTCTCCTCGGA GGAGCTGGACATGAAGATCAA |
| 222967 | LOC222967 | NM_173565 | CAGAACGAAGATGCCAGCCAA CCGGACGGCAGCATGTATGAA ACCGAGAACATTCGTCCAAAT CTGGATGCTGAAAGACTTTAA |
| 245934 | DEFB121 | NM_001011878 | AAGAAAGTGAAGTATACTATA CAGGTGCAGAACAACATGTAA |
| 246176 | GAS2L2 | NM_139285 | CACAGTGTACCTCGTCAGGAA CCCGTCCATCTACAAGCTGAA |
| 246181 | AFAR3 | NM_201252 | AAGACGTGTTAAGTTAAAGAA CAGACTATTATTCGGCCATTA CACGTTGTATGATTTCGTTTA CCCGCTTTGTCTAATTTAGAA |
| 246721 | POLR2J2 | NM_032958 | CACCATTAACAAGGACACCAA TCCGGAAGAGGCCTCTTATTA |
| 246777 | SPESP1 | NM_145658 | CAGGTGAAACTGCGATAGAAA CAGAGGAACCTTATATTGAAA |
| 252839 | TMEM9 | NM_016456 | CAAGGTCATCATTGTCATCTA CCGGATGCATATACTGAGCAA |
| 252884 | ZNF396 | NM_145756 | AGGGAAGAGACTCATAGTAAA ATGGATGATCATCTTAATATA |
| 252983 | STXBP4 | NM_178509 | AAGAACTGAACTATTATTAAA CAGCAATTTATTGAACACTAA |
| 253143 | MGC50372 | NM_173566 | CAGGCATGGAAGCTACATATA AAGAAGGAGTTTAGTTTAGAA |
| 253150 | MGC14276 | NM_153248 | AAGGAAATCGAGCTTAATGAA CACCCAATTCAACTTAATAAA |
| 253558 | LYCAT | NM_001002257 | CAGATTAGATATAGCCCTAAA CAGAATATTATTAAACAATCA |
| 253582 | C6orf191 | NM_001010876 | CAGAAATAATGTTAAATAGAA ATGGCTCAACATAAACTATAA |
| 253650 | FLJ35740 | NM_147195 | ACGGAGAAACAGCGGATGAAA TAGGTGCTCAAGGGAAGTTTA |
| 253738 | DKFZp667B0210 | NM_001005463 | CACGTTTAGTATATTAAGACA CCGGGTGATCCCGAAAGGTTA |
| 253769 | MGC43690 | NM_182552 | ACAGAATTTGGTAAAGTTAAA CTGGATGAATGTAGAGAGAAA |
| 253832 | FLJ25952 | NM_153251 | CAGCATTGACTTAGAGCTACA CAGAATGATGCCTTAATCTTA |
| 253962 | LOC253962 | XM_172968 | CTGGCACAGCCGATTCCCAAA CTGCGAGAAGAGGAAGCCTTA |
| 254102 | EHBP1L1 | XM_170658 | CAGGGTTAGAAGCTGATACAA ACCCGTAAGACAGAAATTATA |
| 254263 | CNIH2 | NM_182553 | CGCGGGCACGCGAGCGTTTAA CTGGTGCAAACTTGCCTTCTA |
| 254439 | LOC254439 | XM_170659 | ACGGAGCTTGATGAAGCCAGA CACGGTGTCCTGATGAAGGAA |
| 254786 | OR6C3 | NM_054104 | CACATCCATCATGAACAGGAA CAGGAATAAGACTATTTCCTA |
| 254950 | KRTAP15-1 | NM_181623 | TTGGATCTCTTGGTTGTGGAA CCCGCCAGACTAACTACATAA |
| 255061 | TAC4 | NM_170685 | AAGCCAGTTCTTTGGGCTGAT CTGGGTAATTGTGGCCTTGGA |
| 255187 | LOC255187 | XM_173160 | TAGGATTGAGATACACAGTTT CAGGCACCTTATATCCTCAAA |
| 255239 | ANKK1 | NM_178510 | CAACATGATGATGATTATTAT CTGAGTGTCATCAACCTCCTA GCGGACGGAGTACGCCATCAA AGGAGCCGAGATGGAAATTTA |
| 255275 | LOC255275 | XM_171855 | CACTGGAACATCATAAGGGAA CAGCAGGATCCTGAAGCCCAT |
| 255324 | LOC255324 | XM_171078 | CACCTAGATGGGCCTCTCTAA CACAATGAAATGAGAAGATAA |
| 255620 | LOC255620 | XM_173132 | GCCTTAGTTACCACTTCTTA CTGGGCTGTTCTGGACTTCAA |
| 255714 | MGC87693 | NM_207341 | CAGGATCACACTGGCCAACAT CCCAATAAACAATCATTTCAA |
| 255738 | PCSK9 | NM_174936 | TCCCTGATTAATGGAGGCTTA CAGGTCTGGAATGCAAAGTCA |
| 255743 | LOC255743 | NM_198278 | CAGGCAAATAGTGTCATCGAT ACGGCAGAATATATGGCTGTA CTCATGTATATTGGAGGCAAA CTGGCTTAGACTAGAGTATAA |

Table4

| 255967 | PAN3 | NM_175854 | ATGGCTAAAGACCTTAACCAA AAGGCAAGTTTACATCACAAA CCGGATACAGAAATCAAGTAA TACCTATACTTTGATACTGAA |
| 256006 | ANKRD31 | XM_293911 | TCGGCATGTCATAGAGATTTA CCCAGTAATCGGCAAATTAAA |
| 256051 | ZNF549 | NM_153263 | AAGCCTTATGTGTGCAATATA CAGAAGGTACATATAACAGAA |
| 256076 | FLJ35880 | NM_153264 | AACAGTATACACCAAATGAAA CAGCTTGGGAGAATACATAAA |
| 256112 | ZNF37B | XM_497219 | ATGGGAAATCTTATGCATGTA CACCTGTGAAGCCAACTATAA |
| 256130 | MGC42090 | NM_152774 | CAGAAACATTCTAGTACAATA CTGCTTTGATGTATAGTTAAA |
| 256144 | OR4C3 | NM_001004702 | CTCCTTGTCTTTATAGGCAAT TGCAATTAGTTCTATTACTTA CTCCTACATTGTCATCCTGTA CTCAGTGACCTTGGAATCTAT |
| 256236 | NAPSB | XR_000169 | CCCGCTGGATGTACTGGTGGA TGGGACCAAGTTTGCCATTCA CCGGTGCTCAGAAATCCCAAA CAGGATTACGTCATCCAGTTT |
| 256309 | MGC33607 | NM_152775 | AAGCAAGTATTTATAAGAATA AACGAGAATATTTAAGTTTAA |
| 256356 | MGC40579 | NM_152776 | TCCCTGGATATGACCAGAATA AACAGTGTTAGGGCAACTTAA TACCATCTTGTACGAGCAATA CAGCCAATGGGTGTAATTTCA |
| 256453 | LOC256453 | XM_172917 | ACCTCTTGCCTTTCATTTCAA CAGGCAAATTCCACAAACATT |
| 256472 | MGC33486 | NM_153266 | CAGGAGAAGAAGCTTCCAGAA TTGGAGGAAGTGGAAGCCTAA |
| 256892 | OR51F1 | NM_001004752 | CAGCGTGATCCTGTTTGTCAT CACGTGAAATCAGTCTATATA |
| 257039 | LOC257039 | XM_172230 | TCGACCTACAGTTGAGATAAA AGGAATCAACAGAGAATGAAA |
| 257169 | C9orf43 | NM_152786 | AAGAAAGAACTCGGCAGTGAA CACGGAGAGACCAAAGATGAA |
| 257177 | LOC257177 | XM_170909 | CCGGATCACTTCCATCGCTGA CACCTTGTGTCAAGAAGGCAA |
| 257202 | GPX6 | NM_182701 | AGGGCTAGCGACTGACAGAAA AACGGCGAGGAGTACATCCAA CAGACTATCCTGTGTCCCTAA CCCGAGAAGTTGTGACATCTA |
| 259249 | MRGX1 | NM_147199 | CAGGAACGCCTTCTCCATCTA CTGATTCTGCTTGGTGTCAAA CGCCTTATATATTCCCTGTTA CACGTGGACAGGGAAGTCTTA |
| 259285 | TAS2R39 | NM_176881 | AACATACTTGTCTGAGATCAA ATGCCTATACATGCAGCTGAA |
| 260293 | CYP4X1 | NM_178033 | AAGCCGAGTGTTGAATCAGTA TGGGATGTATTTGCACCTGAA CACCAATAGACTTTCATATAT ATAGATGTGATCATTCCTATA |
| 267012 | DAOA | NM_172370 | CAAGGAAATAACTTCTACCAA TTGCAACTCTCTGGTCAGTAA AGGCATTTACAGAGATCATTA AAGAGAGACGGTAACAAGGAA |
| 280636 | C11orf31 | NM_170746 | AAGTATTTGAGTAATAGAGTA CTGGTGATGTTGGAACATTAA |
| 282617 | IL28B | NM_172139 | CCGGGCCTGTATCCAGCCTCA AGGGCCAAAGATGCCTTAGAA CTGCCACATAGCCCAGTTCAA CAGGCCTTTAAGAGGGCCAAA |
| 282969 | C10orf125 | NM_198472 | ACGGAGTACGAGTCCATCCTA CTGCAGGGAGACGGCCCTCTA |
| 282973 | C10orf39 | NM_194303 | CAGCAGAGAATTAAAGAGTTA CAGCAGAATTCAAGTATTTAA |
| 283008 | FAM22A | NM_001009610 | CCCTATGAATGGAATATACAA CAGATTTACATTGGTTTCTAA |
| 283092 | OR4C13 | NM_001001955 | AAACAAGACTATCTTATTCAA CAGCATGTTTGTAGCCTGCTA |
| 283117 | LOC283117 | XM_210184 | CACCCTGCGAGTCTTCCAGAA ATAGTTATTGATTCTGACAAA |
| 283130 | LOC283130 | NM_182556 | TCCCGCCAGGGTTAAACATTA CACGCCCTTAGACGTGATCAA CCCGGCTGCCTTCACCTCTTA GCGCTTCTTCAGAGTCATTAA |
| 283135 | FLJ90834 | NM_173578 | CCCGATTGTATGCTCCATCTA CAGATGATCAATAAATACTAA |
| 283140 | LOC283140 | XM_498528 | CGGCACCATCTAACACATTAA CAGGCAGTCAGCACAAATTTA |
| 283149 | BCL9L | NM_182557 | CACCTACTTCTTATCAAAGAA CAACATGAACATGAACATGAA |
| 283150 | FOXR1 | NM_181721 | CCCTTCCTCTTTGATCTTTAA CTGGATGTGGGTAAATCCCAA |
| 283155 | LOC283155 | XM_208545 | CTCCTTTACAACCATACTTTA CTGCATTTGATAGCAATGTAA AAGAAATGTTATAAAGTAGAA TTGGAACGGATGAAGCATTTA |
| 283162 | OR8B4 | NM_001005196 | CTGAATGACTTTGTTTCAGAA CAGGGACATTCACCTACTTAA |
| 283177 | LOC283177 | XM_378327 | CAGGAGCAAGGGTGTCATTTA AAGGCGCTGTTTCTTAACCAA |
| 283189 | OR9G4 | NM_001005284 | ACCCATTGCTTTATTCAGGTA CACAGACTATACAACCACAAA |
| 283197 | MGC39681 | NM_174939 | CACATTGTGTTGAAAGAAGAA ATGGATGAATTTAAACAGTTT |
| 283202 | LOC283202 | XM_208563 | CAGATCACTTGTCCTCCTCAA AGGCTGTAAGGTCATCGTATA |
| 283209 | PGM2L1 | NM_173582 | AAGGATTAATGCCAAACTAAA CAGGTATTAATTTACAAATTA |
| 283212 | FLJ33790 | NM_173583 | ATGGCTGATGCTAATAATAAT CAGGGATAGTTTCCAGCTCAA |
| 283237 | MGC29649 | NM_173810 | ATCCATCTGTTTATTTCTATA CTGGGCATGTTTGGTTAACAA |
| 283238 | MGC34821 | NM_173586 | CACCGTTGACTTATCAGGAAA CACAATAAATTTATCTACATA |

EP 2 295 543 A1

Table4

| | | | |
|---|---|---|---|
| 283259 | LOC283259 | XM_208061 | TTGAATTGCATTCTAATGTTA CAGGCCCTGTTTCTACCTCAA TTGCATTCTAATGTTATTAAA ACGAATCAGAATGGCAATATA |
| 283297 | OR10A4 | XM_208604.1 | CACCTCTGTGTTTGAACTGGA CACCATGGCATATGACCGCTA CTCAAGGAATAAAGAAGTGAA ACCATTTACTTGGTTACTTTA |
| 283377 | LOC283377 | NM_207344 | CAGGCCTTTCTCAGACTGTAT CAGCTTTGTTTGAATTTATTA AAAGCTAGGCATACAGCCAAA TACCAGATGGCTGAAGAGTAA |
| 283383 | GPR133 | NM_198827 | TCCGGATGAGATCGCCATGTA CAAGGAGGGCCTGAAAGTCTA CAGCATGCACTACTACCTGAA CAAGCACAGATTGACACGTAA |
| 283389 | LOC283389 | XM_211009 | TGGCAAAGGTTTGGTGAAATA ATCAGGTTGCTCAGCAATGAA |
| 283440 | LOC283440 | XM_211040 | TACAATGTTCAAGATAGAGAA CCGGTACTCATAAACCTTCTA |
| 283455 | KSR2 | NM_173598 | CAGGCTTACCGTGGACGCCTA AAGGAAATCCATTACTTCAAA TCCGTTGTGAGGGATGCCAAA ATCCGGTGACCTCGAATCCAA |
| 283483 | LOC283483 | XM_378431 | CACACAAAGCTTTCTCTTAAA AAGGTGGATTTGGAAGAACAA |
| 283551 | LOC283551 | XM_378455 | CACCTTTGTCTTACAGTTTAT CCAAATAAATACATAATACAA |
| 283553 | LOC283553 | XM_211086 | CTCACTGGAGTTGACCATCAA AAGCAGGAACTTAATTTAGAA |
| 283579 | FLJ25976 | NM_174943 | CCGCAAGATGCTCACGCGAAA CCGGAGGGTATGGGAAGAGAA |
| 283600 | C14orf68 | NM_207117 | ACGCTGCACCTGCCACTTAAA CAGGATCCAGACGGAGCCAAA TTGGGAACTGTTGTCAATAAA CACGGTGAAGGTCAGGATCCA |
| 283601 | C14orf70 | NM_001007560 | AAGGATGTGACTCTCCTAGAA CCCAGGCAGATGAAAGCTTAA |
| 283659 | FLJ25756 | XM_370866 | TAGGAAATGAAGAATCTTTAA CAACAGTAAATTGGTAATTAA |
| 283677 | LOC283677 | XM_208778 | CCCAGGGCAACTGAAAGTCAA CTGGCGGGTTTGAGAAATTAA |
| 283685 | FLJ36144 | NM_182561 | CACTGTGTGAATATAGTCTGA TCCCTCCAAAGTTCTTTGTAA |
| 283694 | OR4N4 | NM_001005241 | ATGTGCACCACTCGTGTCATT CACCACTCGTGTCATTATTAT TAAGCTGGCTTAAGAGATCAA AAAGATGCTAACGGTAGCATA |
| 283761 | LOC283761 | XM_378542 | CCCACAGGGTTTGAACTGTAA CCCAGTCAAGTTGACCTGTAA |
| 283777 | FLJ39743 | NM_182562 | AAGGGTCCAATTGACTAGAAA TAGGATGAATATGGACTTTGA |
| 283848 | FLJ37464 | NM_173815 | CCCACTGAAAGTGGCCAAGAA CCGTATGATGGACATAGTTCA AAGAGTGGGCATGAAGCTCAA CAGGTTGTGACATGGAGCAAA |
| 283902 | LOC283902 | XM_211251 | CCCATGCTAGTTGCAGAGAAA CAGGACATTATCTCACCATAA |
| 283933 | MGC46336 | XM_290712 | ACCGCATCTCTGTGAAATAAA CTCCTTTCCCTTATACATTAA |
| 284029 | FLJ34790 | NM_173621 | CCGCCCGTTATTGCTGCTCAA CAGCGACACCATGATGATTTA |
| 284040 | FLJ36674 | NM_173622 | TAAGAAATCAGTAAATATTAA CAGGAAGGGAATACAAAGGTA |
| 284058 | LOC284058 | NM_015443 | CAGAAGAATTGGAGAGATTTA CAGGTTGAGAGGCATATACAA |
| 284067 | LOC284067 | XM_208990 | ATCCAATATATCAATGATGAA ATGCATGTGAATATACAATTA |
| 284069 | MGC34829 | XM_208993 | CCGCCCTGGCTTCCTCACCAA CCCGCAGGAGATCCTGATCAA |
| 284076 | FLJ35808 | NM_173623 | CCAGTTGGTCTTCATCTCCAA CTGGGAGAGTCCGAACACAAA |
| 284086 | NEK8 | NM_178170 | CCAGTCGGGTGTGACCATCAA CAGGAAGCTCAGCGAGTTGTA CCAGAAGCTGGTGATCATCAA ACGGACAGTTGGGCACCAATA |
| 284111 | SLC13A5 | NM_177550 | CAGGAGTCATAATGAACATAA CAGACTGAGGAAGAAAGGAAA |
| 284114 | FLJ36878 | NM_178518 | CACCCTTACTGCTTCCCATAA CTGGACGGCACTGAACTGCAA |
| 284119 | PTRF | NM_012232 | CAACTTTAAAGTCATGATCTA GAGGAAAGATTGAATCCTAAA |
| 284121 | LOC284121 | NM_001007533 | CACGATGGACTGAGCCAGCTT CGGGACCGGATACATTATTAA |
| 284131 | FLJ35220 | NM_173627 | TTGGCCAAGGTTAGCGATTAT TGGGTCTAGGATTCTCAGTAA ATGGGTGAGAGTTGAAATGAA AAGAGAGAGATTGGTGTTAAT |
| 284134 | LOC284134 | XM_211345 | TCCATCTGGACTAGCAATAAA AAAGGTATTGAATGAATTGAA |
| 284185 | FLJ39421 | NM_178519 | AGGGAGGGAGATGGACTCGAA AAGGGCTATGGATCTAAATAA |
| 284186 | FLJ38792 | NM_178520 | CACACAAGAGTTCCCTTTCAA CCCTGCGGGAATTCTATTAAA |
| 284207 | METRNL | NM_001004431 | AGGCCTGACCCTGGTACCGAA AACAGGTGCTCTCATCGTTAA |
| 284222 | C18orf58 | XM_290817 | GTGTCTTGCAGCAGACTTAAA AAGTCTGCCAGTGCCTATTAA |
| 284230 | LOC284230 | XM_208185 | AAGAGAATGCTGGCTATTAAA CCCACCAGACTTTCTGTAAGA |
| 284266 | CD33L3 | NM_213602 | CCCAGGAGTCCAATTATGAAA ACGCATTAGCTTGAGCGTGAA |
| 284269 | LOC284269 | XM_209097 | CAGGTTTGTGGTGGGAGAGAT CAGCAGCTCTTCACAGTCTCA |
| 284273 | ZADH2 | NM_175907 | ACCCGTAGGTACCGTCCTTAA AACGCTTACATTCAATTCTTT ACGCTTACATTCAATTCTTTA TACGATGGTCTCAACACCTTT |

Table4

| 284293 | LOC284293 | XM_209104 | AAATGGCACATTTGCATTAAA GTGGCAAATTCTACCAAGCAA CAGCTAGACTTTGTGAATGAT AACAACTATGCAAACATTAAA |
| 284296 | LOC284296 | NM_175908 | CAGGATTTCATTCAAACATGT CAGAGACATCCTCTACTGAAA |
| 284312 | ZSCAN1 | NM_182572 | CACACCAAAGGTGGTACCCAA CCCTTGAACCTAACAGATAA |
| 284352 | LOC284352 | XM_113978 | CGCCACGGCCCTGAAGATGAA CTCGACTGTCTGGACCTGAAA |
| 284353 | NKPD1 | NM_198478 | AGCCGTGGACATAGCTTCCAA CAGGGTATACATGCAATAAAT |
| 284361 | LOC284361 | NM_175063 | CCGGGATGTGGCAGCCCTGAA CTCCCTGTAAGTCTATTTAAA |
| 284393 | LOC284393 | XM_209178 | CCGAATTTGTGCCAATAAGTA ACCAAGTTCAATGCTGATGAA CCGCACCAAGCTGCAGAACAA CCCGTTGTTACCGGTATTGTA |
| 284397 | LOC284397 | XM_209180 | CAGGAGGATTAATAGGTGGAA TCCCATGAGTATGTAGCCATA |
| 284427 | MGC34725 | NM_173637 | AAGAATTACTTCTGTGGAATA CTGGGTCAGAAGATTCCTGAA ATGCATGACAACAACCTTGAA AATGGTGAACATGTCACACAA |
| 284475 | LOC284475 | XM_378886 | TGGGATTTGGTGAGAATCTTA AAGGGCAAACTTGAAACATAA |
| 284485 | FLJ36032 | XM_371311 | AAGAAGTAGAGTGGCTCATAA CAGACTCGGATTGCTAACGAA |
| 284498 | DKFZp434E1410 | XM_209234 | CTGGCTTTACAGCAGAAACAA CTCAGCTTCTTATCTTTATA |
| 284593 | FAM41C | XM_496333 | GAGGAACCTTGTCATGAAATAA TGGAATGAAATGAATTCTCTA |
| 284610 | DKFZp434D177-like | XM_371388 | CAGCTGAAATGGCACATTCTA CAGGGCTGTCATCAGCATCAA |
| 284672 | LOC284672 | XM_208234 | GCGGCAGTTGTGATAAATAAA AAGCATTTAAATGAAATTGAT CTGGAGAGAAGTGGACTCCAT CAGCTTAGGGAAAGAGAGCTA |
| 284988 | LOC284988 | XM_209429 | CAGGGTCCCATTCGGCCTCTT TTCCGGTTAATTGTTCTGGTA |
| 284992 | FLJ39660 | NM_173646 | TACACTGAACTTTCACATAAA AGGATTGTTGTTAATAATATA |
| 285033 | LOC285033 | XM_379111 | ATGCCACATGATGAATACATA CAGTTTCAGATCCCAAGCTA |
| 285095 | LOC285095 | XM_211764 | CCGGAGGGTCATGGAGAAGGA CACAGATCTTCATCATGGAA |
| 285188 | LOC285188 | XM_209505 | CTGCCTGAACCTCGACTCCAA AAGGAGCAACTGACCAGGAA |
| 285189 | PRGA | XM_376111 | TGGAGGTTTCTTGAAGACCTA AAGACCTAGAACATAGAAGAA CAGAGAAGAATGTGCAGCAAA ATGAAGATACGTGAAATTCAA |
| 285195 | SLC9A9 | NM_173653 | TACGATATGCTACAGCACCAA TCGGCTCTTCAGAATGTGGTA AAGGGAATGCCCAGCTATTTA CCACTGTTTGTTAGGCCTTAA |
| 285220 | EPHA6 | XM_114973 | CACTGTTTATGTACTCATGTA CACGTGAATTACACCTTTGAA AAGGGGTTATTTCCGAGCTGAA CAGGTATGTAATGTAATGGAA |
| 285248 | LOC285248 | XM_211816 | CTCGCAGAAGTTATGGAGAAA CAGGAAGTATTTATTGTGAAA |
| 285267 | ZNF619 | XM_173656 | AAGGGAGGAGATAGCCAGGAA CAGCTAAACATTTCTAAAGAA |
| 285268 | ZNF621 | NM_198484 | CAGGCTGAACTGTCACTTTAA CAGCATAAACTCTATGGTTAA |
| 285296 | LOC285296 | XM_208312 | TGTGGGCAGCTCCCTGAACAA CTGGGCTGTTCCGGACTTCAA |
| 285307 | LOC285307 | XM_211837 | TGCCATCTCAATCTAACTTCAA CACCGAATCAAAGAGAACTAA |
| 285313 | IGSF10 | NM_178822 | CACCAACAAACTATTATTAGGA AAGCAGAATCACAGTCCATAA ACGCATCAATTTAGGATACAA CAGGCGTATATCACTGTATAA |
| 285326 | LOC285326 | XM_211843 | CACAAGGACCAAACATATGAA CAGATGGGATCTCAAAGCCAA |
| 285335 | SLC9A10 | NM_183061 | AAGCCTCATCAGTTTAATTAA AAGTTTGTTCCTAAACATAAA |
| 285362 | SUMF1 | NM_182760 | TTGAGTGATACTAATCATGTA CAAGTGAAGACCAATATTCAA |
| 285386 | FLJ41238 | NM_198485 | AACAATGGATAAGCATTTAA AAGGATGACTTCCAAATTTAT |
| 285412 | LOC285412 | XM_208319 | CAGCTGCTACTGTTGCTTCTA AAGCAGAAATACCAGTGGTAA |
| 285453 | LOC285453 | XM_209616 | CAGCTGCTTCAGACTACCAAA ATGGATGAGGATGAATTGGAA TGGCAGCTACTGCGAAGGTTAA GACATTCTTAATTACAGGTAA |
| 285521 | FLJ38991 | NM_173827 | AAGTATTAGAATAGAACCAAA CTGGCAATTCCTAGTTAATAA |
| 285643 | LOC285643 | XM_209695 | CTGCCTGGATCTATAAATGCA CTGAGTGAGCACCGCCACAAA AAGGAAGGTATTGCAGCTCGA GCGGTAGAAGAAGAAGCTCAA |
| 285671 | LOC285671 | NM_178532 | CTGGATCATATGGTAAGTATA AAGCAAGGAATTGATAACTAA |
| 285676 | ZNF454 | NM_182594 | CAGGGATTTCAACCTAGCAAA AAGAGTTCAACTCTTAATAAA |
| 285692 | LOC285692 | XM_498992 | CAGGAACTCCTTCAACATGA CCCAGCCAAGTCAGCATATAA |
| 285703 | LOC285703 | XM_209728 | ATGAGAGTGAATTCTGAAGAA CTGGTTGAGCACTCTATACAT |
| 285733 | LOC285733 | XM_379432 | CTGCACTTGATTACAATGAAA CTGCAGCATCTTACTACATAA |
| 285741 | LOC285741 | XM_209741 | CTGCGCTTGCTGCTCAAGCTTCA CATGTTATGTATGTTGTCGAA |

Table4

| ID | Gene | Accession | Sequence |
|---|---|---|---|
| 285759 | FLJ34503 | NM_173673 | ATTGATTGAATTAATGTTTAA CAGACTATATTCATCAGCAA |
| 285761 | DCBLD1 | NM_173674 | CACAATGACATCTAAGAATTA ACCGATTAATCTAGAGATAAA |
| 285770 | LOC285770 | XM_209753 | AAGGTATTGGTGCCTATGAAA CTCCTGCTATCTTTAAATCAA |
| 285830 | FLJ35429 | NM_001003807 | ATCTATCATATTAGAAATCAA AAGGAGAAGAATGACTATTTA |
| 285940 | LOC285940 | XM_376585 | CTGCATCATCTTTGAGTACTA CCCGATGATCCGAAAGACAA TACAGTCTTTGTGAAATGAAA GTGAATATGTGAAACAGTAAA |
| 285966 | FLJ40722 | NM_173678 | CTCCTAATAAGTGTTCCATAA CCTGGTAACATCATCCTCAA |
| 285973 | NOS3AS | NM_173681 | CAGCTCTGGCACAGTCAAGAA CAGCCTCACCTTGCTCTCAA |
| 286094 | LOC286094 | XM_212170 | AAGAATGGACTCTTCTATTAA CAAGCTGTTTATAAACTATAA |
| 286122 | FLJ37131 | NM_173687 | AACCATACTGTTAGTCTATTA CTGGCTTTCCATGACTACCAA |
| 286183 | FLJ39630 | NM_173688 | CACCGATCTAATGACATTCAA CAAGTAGTTATTGAACTTCTA |
| 286187 | LOC286187 | XM_209936 | AAGCATTCTGTTTCTGGAGAA AAGCAATTCACTCATAAGTAA |
| 286204 | CRB2 | NM_173689 | ACCGGAGGAGAGACTCATCTA CAGGGTCAGTTACTAAGTTA |
| 286207 | C9orf117 | XM_209941 | CGGGACCAGCTTCCTTAGCAA CAAGGAGATGAAGGAGTTCTA |
| 286238 | LOC286238 | XM_379684 | CTCAATGAATATAGAAGGGAA CAGCCAGGATTCAAACTATAA |
| 286262 | C9orf75 | NM_173691 | CACAGTGGTGCCCAAGAGGAA CTCCTCAAGAAAGAAGATGAA |
| 286327 | LOC286327 | XM_497054 | ATCATTCTCATGAGTCGCAAA CTGTATATCTGGAATGTTTAA |
| 286336 | C9orf59 | NM_033387 | CAGCAGAATCCTTATTTATAA AACCGCATAATTATTCATTAA |
| 286423 | LOC286423 | XM_210042 | TAGGCAGATCTCCGTGAAGTT TTGTACCAACGCAATGAACAA |
| 286453 | dJ341D10.1 | NM_001007535 | TACAAAGACCAGTGAATTATA TTGAATTCTATTAGTATGTAA |
| 286561 | LOC286561 | XM_210515 | CTGGGCCAATGTTGTATCCTT GAGGGAGGAGGCAACCTTGAA |
| 286749 | SALF | NM_172311 | TTGCTACAACATCCAGCCTAA CAGTCTAGGTTCTACATCTTA |
| 286753 | TUSC5 | NM_172367 | CAGCATTACCCTCCATCATCAT CAGGGAGAGGACAGAAATGAA |
| 286826 | LIN9 | NM_173083 | TTCCTTTAGATGTCTCCATAAA TAGGCTTCCAACACCAAATAA ACGAGTGGTTCTATTCAAATA CCAGTAGAATTTCTTATCCAA |
| 286828 | CSN1S2A | XM_379270 | CCCATTTAGAAGAACATGTAA CAAGAAGAAACTGCAAATAAA |
| 317702 | VN1R3 | NM_174980 | TAGCACAAAGACAAATGATTA CCGATTTATGTGGATTGTCTT CTGGCAAGTGGAACAGCATAA CTCAGTGATATTGGATGCAAA |
| 317781 | DDX51 | NM_175066 | CAGGAGAGCCTCGTCCAGAAA CTGGAGGAGTCTGTCAAGGAA |
| 326340 | ZAR1 | NM_175619 | CAGCACTTTCAGCTTCAAATA CACGATTTATACATTGCATAA |
| 337867 | PHGDHL1 | NM_177967 | TCCGTTGTCCATCACAAAACAA CACGCTGGACATCCAGAGACA TGCCATTACATTAGCATGTAT GAGGGTAATGTTACTTCACAA |
| 337976 | KRTAP20-2 | NM_181616 | TACTATGGTGGTCTGCGTTAT GTGCTACTACAGCAACTACTA |
| 338328 | LOC338328 | NM_178172 | CTGCAGCTTCCTGCAGAGGAA CCAGTGACTACGACGAGGAA AAGTACAGAGTGAGACTTAAA CACCAGTTCAACACTCCATTA |
| 338382 | RAB7B | NM_177403 | CAAGGCTGGTGTAGAGAGAAA AACGAGCAGACTAAGGAGTAA TCGAGTACCAGAGCATCTTA CAGCATCCTCTCCAAGATTAT |
| 338398 | TAS2R60 | NM_177437 | AACTATTTAAGGAACCATCTA TGGCGATAGCATACGGAGCTA TCCGATGGCCTTCCCATACAA CAGGCTGAGAGCTGTGCTGAA |
| 338440 | TP53I5 | XM_374860 | CACGGTGGTGTTCGCCATCTT CTGCCTCAAGGGTACGTCAA GACCATCATCATCATGACCAA CGGGAGCAGCCAGTTGATGAA |
| 338611 | LOC338611 | XM_291745 | CACAGCCATGTGACGCCTATT TCCAAGCAGTATATGAAGCTA |
| 338645 | LOC338645 | NM_001009909 | CAGACCAATTATGATCCTTAA AAGGAAATCTTTAATCAATAA |
| 338657 | DLNB14 | NM_198489 | ATCATTGTCTTTCTTTAGGAAA CAGGACCATCTCTGACATTCA |
| 338661 | LOC338661 | XM_290501 | AACATTTGTTGTTAATATAAT AAGTATCCAGGATGACCTGAA |
| 338692 | LOC338692 | NM_207354 | CACCGTGTTTGAAGTGCCCAA CCGGAGGAGCTTCATCTTCAA |
| 338751 | OR52L1 | NM_001005173 | CTGGTAGGGATTCCAGGTTTA CACCGCTTTGGTCATCATGTA |
| 338879 | RNASE10 | XM_292225 | AACGCTGGTGCTTAGCAACAA CTGAATCTGGAGAGTGATCAA |
| 338949 | LOC338949 | XM_294751 | AACAATGTTTAGAGTCATGAA CAGAAATGGAATAAAATATCAA |
| 339005 | LOC339005 | XM_370838 | CAAAGTCACATCTTTGTGGCA GCGAAGTTAGAGCCTAGTGTA |
| 339022 | LOC339022 | XM_294775 | ATGGATTTATAGAGAACATCA AGAGAATACATTTAAATTAAA |

Table4

| | | | | | | |
|---|---|---|---|---|---|---|
| 339059 | FLJ40448 | NM_182605 | AGGAGAATCACTTTCACTTAA | CACCATCTGGTTGCCTGGCAA | | |
| 339065 | LOC339065 | XM_294794 | AAGCTACGATAGAGTCAGATA | CAGGGTAGTGATGACCTCACA | | |
| 339145 | FLJ44299 | NM_198491 | CAGCTGAGTAGGGCAAATGAA | CAGAGTGGCTTAGAACAGTAA | | |
| 339201 | DKFZp762C2414 | NM_178542 | CCCGGCTGATTGGATTCGTTA | CTCCATGAGGCTGGTCCTGAA | | |
| 339210 | LOC339210 | XM_378687 | CTAATAAACATTGGTATTAAA | CTGCAAAGAAATACTTGATAA | | |
| 339324 | LOC339324 | XM_290838 | AAACATCACAATATTCATACA | AAACATAATATGGAACAATTA | | |
| 339390 | LSECtin | NM_198492 | CTCCTGGAGCTGATTGCCAAA | AATAAATATTTGAGAAATGAA | CAGCCTGAGGAAGCATCAATA | CTGAGGAAGCATCAATAAATA |
| 339398 | LRRN6D | NM_001004432 | CACCTTGAGGCTGTCTGGCAA | ATCATTAAACCTGCTGTCAAA | CACGGCTATAAGATTATTTCA | AGCGCTAGTCAGCTAGTCTAA |
| 339403 | RLN3R2 | NM_181885 | CCCGGGAGAGCCGCCCTTCTA | TTCAGACACCTTCGTCTTCAA | CAGGCGGTGGGTCGCAAGCAA | CCGATGCCTGTCAAATTCCTA |
| 339451 | KLHL17 | NM_198317 | CACGCTTGATGGGAACCTGTA | CAGCCTCAACTCCATCGAGAA | | |
| 339457 | LOC339457 | NM_001003808 | CAGGCGAAGTTTGACACATTT | CAGGGTGTGGTCAGCCGCAAA | | |
| 339483 | LOC339483 | NM_178546 | GCCGCTGACTGTAACATGTAA | TAGGGAGGTCTCAATAATAAA | | |
| 339488 | TFAP2E | NM_178548 | TATGTTGTAATTAAATTTGAA | CTCAAGGAACAAAGAAGATAA | | |
| 339512 | MGC48998 | NM_178550 | CAGGGTGAAATGAGCCTTGAA | AAGGGATAATTTATACTTCAA | | |
| 339559 | ZNF642 | NM_198494 | CCAGGAATTGTTGACTTTCAA | CAAGTGTAAAATTGGTGATTTA | | |
| 339669 | MGC35206 | NM_178552 | CTCCGTCTTCTCAGACTACTA | TAGAGGTGTCTTCTACTCAAA | | |
| 339766 | LOC339766 | XM_291007 | CAGGATCTTCTTCGACAACGA | CGGGAGGAGATGAACCTGCAA | | |
| 339778 | LOC339778 | XM_496547 | AAGGACTTTCTGTCCATCTAA | CACCATCATCCTTACTGATAA | | |
| 339788 | LOC339788 | XM_379074 | TTGGTAGAATTTACCTTATAA | CTGAATGTTTGTACCCTCTAA | | |
| 339799 | LOC339799 | XM_290345 | CGGAGTTGAGCTGATCATGAA | CACAATGAGTCAGAAGATGAA | TACGCATACTATGACACTGAA | CCGCATGAGCATCAAAGCCTA |
| 339843 | LOC339843 | XM_290351 | CTGGGTTGTGAGCTCCTTGAA | TACCATGGAATGGGTCAGAAA | | |
| 339862 | LOC339862 | XM_376212 | CAGCTAAATAATTGCTAAATA | TAGGTTCAACTTCAGCATATA | | |
| 339879 | LOC339879 | XM_291054 | CTGGGCGGCACGCACTCTCCA | TAGGCTGGTGACACGTCTCCA | | |
| 339983 | FLJ37478 | NM_178557 | CAGCCTGAAACCAACACATTT | CACATTCAGCTTAGAAGTGGA | | |
| 339997 | LOC339997 | XM_295126 | TACAGTACATTAGACATTCAA | CAGAGCCTATGTCAATGGCAA | | |
| 340024 | SLC6A19 | NM_001003841 | CCAGAAGATCTCCTACCCGAA | CACGAACATCCTGACCCTCAT | CTCGGTGATTGTGTCCATCAT | CAGGTTCAATAAGGACATCGA |
| 340074 | LOC340074 | XM_379355 | CCGGCATACAGTCACCACCAA | ATGCATCTTAATCATTTACAA | | |
| 340094 | LOC340094 | XM_295155 | AGGCAATAAACTTAAACAATA | AAAGGCCATTACAATATTTAA | | |
| 340096 | LOC340096 | XM_293943 | CTCCGTGGAGAGGGTGCCGGA | CCTGGGAAACCTTGCACTCTA | | |
| 340113 | LOC340113 | XM_379324 | CAGATGTTTGTTTCTGCTCTA | AAGGAGCAGAACATCTGTTAA | | |
| 340146 | SLC35D3 | NM_001008783 | CAGGTATATGAAGAAGGATTA | CTGGAATATAATTCAGAATTA | | |
| 340156 | LOC340156 | XM_373109 | ATCGGGATGCTAAGCAAATAA | AAGCTTCTGGGTAAACACATA | AAGAATAAACATGGACCTTAT | TTGGTGTTGATAAGAAATTAA |
| 340168 | LOC340168 | XM_291161 | AGAGCTAGAGTGATTCCTTAA | ACCAGTTTCCAGCCAATGCAA | | |
| 340265 | GRID2IP | XM_294249 | CAGGACTGTCCGAGTCTACAA | GCGCAGGACTGTCCGAGTCTA | | |
| 340267 | LOC340267 | XM_295195 | CTGGTAGGTTGTGCTCAACTT | TACAGGATATCTTATTTGAAA | | |
| 340348 | MGC50844 | NM_178562 | GCGGACATACTTAGATCCTAA | CAGCATTGTGCCAGAGTGATA | | |
| 340359 | LOC340359 | XM_294387 | AAGGTATGACAGCATCTGCAA | CCGGGAGAAGAGTGAAGCCAA | | |
| 340393 | LOC340393 | XM_291270 | CTGCCTGACTCAGATGCCTAA | CCGGAAACTCAACATCAACTA | | |
| 340485 | ASAH3L | NM_001010887 | TTGGGTCAGATGCTTGATGAA | CAACAAGAAATCATCAGTCAA | | |
| 340515 | LOC340515 | XM_379650 | ACCAATGTATATGCACTTGTA | TAGGAAGAAGTGGTAAGCTAA | | |
| 340526 | RGAG4 | XM_291322 | AAGGATGAAGAAGATGAAGAT | TGGGCAGTCGCTGTCGCATTT | | |
| 340542 | LOC340542 | XM_291335 | AACATTGATATGATAGATGGA | AAAGTTAATAAAGCAATTTAA | | |
| 340595 | FLJ46608 | NM_001004308 | CTGATTTATAACCTGCATTAA | CTGCAGTAGTTTCTAACTCTA | | |

Table4

| ID | Gene | Accession | Sequence |
|---|---|---|---|
| 340654 | LIPL3 | XM_291663 | CAGCTTTACCACTCTGATGAA CAGGCAGTGAATTCTGTGTGAA CAGGATACTCCCTCAAATCAA CAGGACTAAATTGGCTGTTAA |
| 340665 | CYP26C1 | NM_183374 | GCGCTATGGGACAGTGTTCAA CCGGGTGATGCCCTGACCTA CACCTTCGAGCTCGACGGCTA CTGGAGCGTGATGTATAGCAT |
| 340900 | LOC340900 | XM_291767 | AACCTGGTATTGGAAGATGTTA CTGATTTATTAATTAGCTATA |
| 340913 | LOC340913 | XM_291770 | AACAGCCATGTGGAAGTAATA CTGATTCATGTTTCTTACCTAA |
| 341032 | MGC50104 | NM_198498 | AAGGAGTGAGAGTGAAGCATA CCGGGCAGACAAGTAACTCAA |
| 341356 | LOC341356 | XM_292023 | AGGGTGGTGAGAAGAAATTACA ATGGAGTTGGTTTCAAGAAGA |
| 341418 | OR6C4 | NM_001005494 | ATCCCTTCATATATACTTTAA CAGCATGACAACAGGAAATAA |
| 341457 | LOC341457 | XM_292085 | CAGGGAGAAATTTGATGACAA CACCGTGTTCTTTGACACGGA |
| 341511 | LOC341511 | XM_292109 | TAAGGACGTTAAAGCCAACAA CAACATGCTTGTGTTCATTAA |
| 341784 | LOC341784 | XM_497370 | ACCAACGTTCATGCTGAGGTA CGGGATCATGAGGGAGTTCA |
| 341883 | FLJ46156 | NM_198499 | CTGAACCACAATACAATTAAA CCGGTTGTTTGTAATATTTCA |
| 342346 | LOC342346 | XM_296817 | CAGGAGTGCCCTGGCAGGCAA AAGGACGTGAACACCAAGTTA CCGGTAGAAGACATCCTGGAA CACCAGTTCTCGGAAATCAAA ACGGAGGATGCCCATTATGAAA CAGCTGGATGATTCAATTAT |
| 342372 | PKD1L3 | NM_181536 | GAAGCTGTCGGAAACATTCAA ACAGCATCTGATAGTGGTGAA |
| 342538 | MGC71999 | NM_199290 | CCCGCTAGGCTTCCTGGGAAA CTGGATTCTCTGAACACCCAA |
| 342600 | LOC342600 | XM_292624 | AAGGACTTGTTTCACTGCAAA CAGGAAGAGAGTACAATTTAAA |
| 342618 | LOC342618 | XM_496207 | CCAAGTGAACCTATCCGCAAA CAGGGCATCTGCACTGGAGAA |
| 342669 | LOC342669 | XM_292674 | CAGCATGTGGCACAAGAGACA CATCAAGATCGCGGAGATCAA |
| 342853 | LOC342853 | XM_497574 | GGCGCTGGTTTCCATCATCAA AACGTTGTACTCTCTTCAGAA GAGGGAAATACCAGTGCTTTA AACGATCTCATCGTTTCTGAA |
| 342931 | LOC342931 | XM_292796 | AGCCGGAAGGAGAAACCTCAA ACGTGTGCAATAAATCATTTA |
| 342933 | LOC342933 | XM_292723 | CTGAATCTGAGAGGTCTCAA AAGGAATATCTTATGCAGGAA |
| 342934 | LOC342934 | XM_292724 | CCCGAGCAACCTCTCCAGTTA CTCAGTATCTACAACACACAA TAACCTAAAGCTTATCTGTAA CAGGGTATAAGTATGCTGAGA |
| 342945 | HKR2 | NM_181846 | CACTCATTCCTCACACCTAAA ATCGGTTAAGATCGATGGCAA |
| 342969 | LOC342969 | XM_292824 | AACATTGAAGATCAGTACAAA CAGGAGGAGAAACCCTATGAA |
| 342970 | LOC342970 | XM_292810 | ACGCCTGTGCTCTTTCTGCAA CACCCGAAACTCGGCAGCCAA |
| 342977 | NANOS3 | XM_292819 | ACCACTGGGCATAGAGAGAAA ACGGAGGGAGATCTGAATCTA |
| 342979 | LOC342979 | XM_292820 | TTGAGGGAAGGAAATAATTTA AAGAGTTGATTGGCTTATTAA |
| 343035 | C1orf36 | NM_183059 | CACCTTTGTCTCCTATGTGTA CTGGGCTTTCCTGGTTCTCAA |
| 343169 | OR6F1 | NM_001005286 | ATGATTGTAAGGGAACAGCAA CCCATGGAATATGAGCATGAA TGCCACATTGGTGTGATCCTCTA ATGTATAACGATGACCTAGAA |
| 343295 | LOC343295 | XM_497684 | CCCAAGATGCTTATCAACTTA ATGGAAGGTAGGGATAATGTA TACAGGTAATTAGATGATTTA CACAACTGTTATTCTGCCCAA |
| 343406 | OR10R2 | XM_372798 | CAGGGATGGAAGAATACACCA CACCAGAAAGGGAAAGATGAA CAGACATTCACACAAACTTCA CAGAGAGAGGAGTGAGCCCAA |
| 343413 | LOC343413 | NM_001004310 | CACACAGAGTGCAACCAGTAA CGCCTTATTTCTATCACCCAA |
| 343472 | BARHL2 | NM_020063 | ACCAAGGAGAGCTTGATAAAGTA TTGGCACTAGTTGAACAGAAA |
| 343505 | LOC343505 | XM_291607 | AACTGTGAGATTCGTCCTTTA AACCCTTTAATCTATAGTCTT |
| 343563 | OR2T29 | NM_001004694 | CAGACCTGCATTCATATACAT CAGAGGTTGAACATACCCTAA |
| 343629 | LOC343629 | XM_293121 | ATGGAACTTGCCAGAAAGAAA CACAAGGTGATGGACATTAA |
| 343990 | MGC42367 | NM_207362 | CAGCAAAGTTGATATCCTTAA TACCTTCTAGAGACAAATAAA |
| 344018 | FIGLA | NM_001004311 | AAGCTCTTAGTGAGCATTTAA AAGGATATTTCTGAAGAAGAA |
| 344065 | LOC344065 | XM_292895 | AAGCCAGAGGATTGCAACCAA CACGAAGACTGGAATCTGTAA |
| 344165 | LOC344165 | XM_292957 | ACGGAACCAGTCGGTCAGGAA CTGAATTCGAATCACTCGTTTA |
| 344172 | LOC344172 | XM_497885 | AAGAGTTAAGAAGGCATGAGA CAGGATCCGAGCAAAGCTAAA |
| 344328 | LOC344328 | XM_497894 | GAGGATCTATGCAGAGGCCAA CGCCATTGTGCTGCACACCTA CCACTTATGACTAACTTCTTA CAGCACAATATATAGTGGAA |
| 344561 | GPR148 | NM_207364 | CAGGACCAACTTCGCAGATCA AAAGATTCACATGATGAATAA |
| 344709 | LOC344709 | XM_298233 | CAGGACCAACTTCGCAGATCA AAAGATTCACATGATGAATAA |

Table4

| | | | |
|---|---|---|---|
| 344805 | TMPRSS7 | XM_293599 | TAACTATTCAATAACCAAGAA CCCAGCGTTGTGATGGAGTAA CCCGAGCTACTATCCTCCAAA TCGGATTACTCGTCAACCATA |
| 344807 | CD200R2 | NM_001008784 | AAGGATAGAAGCCACTCAGTA CCAAGTCTGTTGATTGATTTA CTGATCATTCTTTATGTGAAA AGCGTTGTCCTTACTGATCAT |
| 344838 | PAQR9 | NM_198504 | AAGGCGTGTGTCATGATACAA ATGCATGGAATGTGAACACAA CAGGGCTTAACCTGCTAGAAA CACACTCTTCGTGCACTTCTA |
| 344892 | RTP2 | NM_001004312 | CAGCGCTGCCTCTGAAGCCTA CACATGATGTGGAGAATGCTA CGGAATGAGAGGATTGCATTA TAGGGTGTCGTACTTACCATA |
| 345051 | LOC345051 | XM_293680 | CAGAGGCTGGGCGATGTTGAA CAGGATGATCCCAGCTTTGAT CCAGCTTTGATTCACTGGAAA CAGCTTCTTACCTCAGTCAAA |
| 345079 | LOC345079 | XM_293687 | AAGGCCAAGAGCAAGGAAATA AAGCTGCTGAGAGTTGGTAAA |
| 345275 | SCDR9 | NM_178135 | AAGAAAGAAGTGGGTGATGTA TACCAAGACATTTGAGGTCAA TCCATCGATGATGGAGAGAAA CTCCCACTACATCAAGACTAA |
| 345378 | LOC345378 | XM_293802 | CTGCTGTTCTGTCTGAATTAA ACCCTAGTAAATCATCTGCTA |
| 345537 | LOC345537 | XM_293868 | TAGAATTTAGGTCCCTTGTTA CTGGATGAGAGATCTTCCTAA |
| 346007 | EGFL11 | NM_198283 | AACGATCCTGAAGATAATAAT ATGTGGTAAATTGGACACTAA GAGGAATAATGCTACATGTAA AAGCTTGGAGCAAGACATATA |
| 346305 | LOC346305 | XM_498257 | CAGGGAGGCTTCTCAGACCTA CACGGTGGAGACCATATTGTT |
| 346517 | OR6V1 | NM_001001667 | CAGGATGCTCTCAGACCTGTT CAGTAGTACCATCTTTCTGTA |
| 346702 | LOC346702 | XM_498271 | CAGAAGGTCATTATTCACAAA AGGCACCAATGGTATCTTCAA AAGAGCCCTGCTCAAAGTTTA CTCGTCATGGTTACCATTTCA |
| 347051 | SLC10A5 | XM_376781 | TAGAAATATCCTAATGCTTAT CAGTGGGATTAGTGTTCTTAA ATGCCTGTCAATTCTTATATA ATCAATTGGAATAGTCATCAA |
| 347088 | GPR144 | NM_182611 | CAGCGAGTGGGCCAAGGCCAA CTGCTGCAAATCTATGAAGTA CCGAGTCAATGCCTTGGCCAA CTGGCTCAATGTGCACACAAA |
| 347292 | LOC347292 | XM_294581 | TATGGCCATGGGCCTCTTCAA AAGCGGCGTGCCATGGAGCTA |
| 347376 | LOC347376 | XM_293312 | CTGAGTGTTGTGGGTGGGCTA AACGTGTTTCAGAATACACCA |
| 347527 | LOC347527 | XM_293401 | CACCGCTGGCTCAGGATCAAA AAGCTCATTATGTGACTCCTA GACGTTGGAATTGCATCCTTA CAGCCATAAGAGAGCATCGTA |
| 347549 | LOC347549 | XM_293416 | CAAGAGGTGGCTGGGAAAGTT CGCCATGGGCAGAGTGAGGAA |
| 347689 | SOX2OT | XR_000282 | CAGGACTTATCAGCTGGGATA AGGAAGGTTGATTGGAAATAA CAGGTGTAGATCACCTATTAT TCCGGGTTCCCAAGAACTAAA |
| 347731 | LRRTM3 | NM_178011 | AAGGCTTGATTTATCAGGCAA CAGAAATAATACATTAACCAA |
| 347732 | CATSPER3 | NM_178019 | CCCAATGGTCTTGGATGATTT CAGACAATGGTGACCATGATA GCCCAACATTCAAGAAATTTA CCGTTTCTTTAAGATAATTAT |
| 347734 | SLC35B2 | NM_178148 | CTCGCAGTTCCTGGTGCTAAT CCCTGTCATGCTGATGGGAAA CCCGATGTGGCAGGCCCTGAA CAGCTTTATGGTACCTGGCTA |
| 347918 | FLJ33915 | NM_182613 | CTGGAGATGTGAATTCCATAA AAGCATCTAAGAGGCAATGAA |
| 348035 | MGC40069 | NM_182615 | CAACAGATGTTCATATTTCTA TACCTTTGATAAGATGGCTAA AACGGGATTATTCAGTAGGAA CCCAGTCACCTGACAAATCTA |
| 348094 | LOC348094 | NM_182703 | CACACTGATGCCAGATGTTAA ATGGACTTTAGTTAATAGTAA |
| 348174 | LOC348174 | NM_182619 | GACACGGCCATCGGTCACTAA CACTAATCTGCAGCACCGGAA |
| 348303 | SELV | NM_182704 | CAGCAATTTCCGAATCACCTA ATCGATGAGGAAATCAAGAAA |
| 348327 | ZNF530 | NM_020880 | CAGGTATCTGTCCTCATTCAA CTGGATGAGTTCAAACCTTCA |
| 348487 | FLJ36766 | NM_182623 | CTGTGTCATTGGCAAGGACAA CCGGGCCCGCGGAGACATGAA |
| 349136 | LOC349136 | NM_198285 | CAGCACAGATGGCACAAGAAA CAGGATCCTGGTTGCCAACAA |
| 349236 | LOC349236 | NM_182635 | ATGCATGACTGGGACAAGAAA AAGGTGAGACTCCACATGGAA |
| 349565 | NMNAT3 | NM_178177 | AAGGTCTAAATCACAAGGATA AAGGGATAAATAATAAATTGA |
| 350383 | GPR142 | NM_181790 | ATGGTGTCCCATGCACAGAAA CAGGAGGCCCTCCTACTACTA AACACTGGACGAGGTCCTCAA CCGGGTCTTCGTCATGCTCTA |
| 353145 | LCE3E | NM_178435 | CTGACGCATGCCTTCCCATAT AGCGATCTTTGGAGGAAACAA |
| 353299 | RGSL1 | NM_181572 | CAGGTCATTAGCCAGAATAAA CCCGCTCTTACTTACAGTATA |
| 353376 | TICAM2 | NM_021649 | CAGGAAGTATTTCTCATTTAG CTCCGTTAACAGGCAGCATAA TCGGGTACTATCACCCAGAAA TTGCCTGAGATGAAACATATA |
| 353514 | LIR9 | NM_021250 | ACGGCTGAGATTCGACAGGTT CAGGAATACCGTCTGGTTAAA |
| 359948 | IRF2BP2 | NM_182972 | CCTGATCTTAGTAGCAGACAA CTCCGTCCTCTATGAACCAAA CCCAATGATTACCCTTAATTA CTGCTTGAATTGTATATATAT |
| 360023 | FRBZ1 | NM_194314 | AAGGCAGATAGTATATATATA AAGCCTAATGTCTGTAATTAA |
| 360132 | FKBP9L | NM_182827 | GAGGACCGTGTATGAGAGAAA CTGGAATATTCTGTCATAAGA |
| 373856 | USP41 | XM_036729 | ATGCAAGCTACTGTGAATTAA CAGCAGGATGACTATCCTTGA |
| 373863 | DND1 | NM_194249 | GAGGGTGAATCCAGAGAACAA CAGCCCTGTGTTCCTCACCAA |

EP 2 295 543 A1

Table4

| ID | Gene | Accession | Seq 1 | Seq 2 | Seq 3 | Seq 4 |
|---|---|---|---|---|---|---|
| 374355 | C10orf96 | XM_495849 | AAAGAGTGAATTGATACAAGA | AAACAGTACAGTGAAATTACA | | |
| 374383 | DKFZp686O24166 | NM_001009913 | CTGGATCAAGTGGCATGAAA | TAGGTTGTTAATACTCGTGAA | | |
| 374387 | DKFZp779M0652 | XM_374877 | CCAGGAAAGATGAACATCCAA | ACCCTGGAAGGTAGCACATAA | | |
| 374618 | TEX9 | NM_198524 | GAGGATGAAAATTCAGAATTTA | ACGAAGATGATTACAGTTTAA | | |
| 374654 | KIF7 | NM_198525 | TCCGTTACCCTGACCCTGGAA | AAAGAACTGGACCCTCATTTA | TACCCTCACTGGGATCAACAA | TTGGCCAAGAATCATCACGAA |
| 374860 | ANKRD30B | XM_371111 | CACTTTGAGCCTGCCACTGAA | CAAGAGCTTGGCGATACAGAA | | |
| 374890 | LOC374890 | XM_373798 | ATCCAGTTCTATGTCCTATAA | CCAGGCAGGACTGACAATAAA | | |
| 374920 | LOC374920 | NM_199341 | CCCGCTGGAGTTCGCCTACTA | ACGGGTAGAGACGAAATGCAA | | |
| 374955 | LOC374955 | NM_198546 | CTGGATATTCCTGAAATGCAT | TAGGAGAATACTGTTTACAAA | | |
| 375035 | LOC375035 | NM_199344 | CTGAATTCCCATGAATACAAA | AAGCAACAATACATTCTCGAA | | |
| 375108 | LOC375108 | NM_198996 | ATGCACTTGCTTGTGAATTAA | TCCAATGGTTTAGGCATTCAA | AAGGATTCCTTCAGAGAGATTTA | ACCATATAGCAGATTAGATTA |
| 375189 | PFN4 | NM_199346 | AAGAATGAATTAAGGGAGCAA | AAGGACTGTATTTCAAGGGAA | | |
| 375295 | LOC375295 | XM_374020 | CCCGCTCAGTTCAATATTTCA | CAGTTAGACCTGGCAGTAAA | | |
| 375298 | CERKL | NM_201548 | ACACTTGATCTTATTAATTTA | CAGCAGAGAAGTGGTACTTTA | | |
| 375318 | AQP12 | NM_198998 | CTCCCAGTGGCGCCTATGAA | CAGGCCGATGGCAGGTCTTAA | | |
| 375341 | FLJ43654 | NM_198562 | AAGGATCTGCTTGATATGATT | ACCAAGTGAAGTATAGTATAA | | |
| 375347 | FLJ44290 | NM_198564 | CTGGGTGGTATGGGAAGTGTA | AAGGATTCAACTATTATTTATA | | |
| 375444 | FLJ32363 | NM_198566 | CTGTAATTTGTCAAACTGAA | CAACATATTATTAAAGTTTAA | | |
| 375484 | FLJ44216 | NM_198567 | CACCAATATTATGTCATTCAA | CCGAAAGGACTTAATAATCAA | | |
| 375757 | C9orf119 | XM_372143 | CTGCACCTTGACATTCAGAAA | CAGAAGGGTGTGGACATGATA | | |
| 376267 | RAB15 | NM_201402 | TCCACCAACCTCAACATTAA | AAGGACTATATTTGTACTGTA | | |
| 377630 | DUB3 | NM_198584 | AAGGGCAATGGGATAAAGCTA | ATGCCACTATAAGTTAATATAA | CTCAGTGATGTTAGATGGAAA | CAGAGGGACACTAAGCATTAA |
| 377677 | CA13 | NM_198585 | CACAGTTGAAGGGACAAGGCAA | CAGCGTCTCACACTCACAGCTA | | |
| 377841 | UNQ2492 | NM_199175 | CCGTGAAAGGATGGAAACAAA | CTGCAGGAGTGAGCAATTTAA | | |
| 378832 | C21orf123 | XM_495990 | CAGAGCTGTCAGAAAGAATAA | ATGAAACATACTCAAGATTTA | | |
| 379025 | FLJ31306 | NM_198692 | GTCCAGCTGCTGAGTGCTCAA | AAGCAGGATTCTCCAGTCTCA | | |
| 386678 | KRTAP10-11 | NM_019110 | TTGGAAGAAGTCATTTGATAA | CGGAATAGAAGTCTTATTGAA | | |
| 387032 | ZNF307 | NM_199358 | AAGCACCTGCTCTCCATGAA | AAGATTAAGCCTAGTTATATA | | |
| 387054 | FOXD4L3 | NM_001002255 | TCCGGTGTTCACCATGGCCAA | TCCGATTTGGTGGGCAACCAA | | |
| 387082 | SUMO4 | NM_199047 | CAGAATATAGTTTCCACTGTA | TACAAGTACGTTACTTATTAA | | |
| 387332 | TBPL2 | NM_001010923 | TACGCAGATAATGATCATTAA | AAGCCTGTTTATGAAATTCAA | | |
| 387357 | C6orf190 | NM_207370 | ACGGGCCTCCGTGACCACCATA | CAGGAGACGTCGGGAGCAGGAA | GACCACCATAGTCTTCATCTA | CAGCGTTGGCGCCAAGCAGAA |
| 387509 | GPR153 | NM_199352 | AACGTCATAGTATACCATCAA | CTGCTAGTATTGGACATATAA | | |
| 387601 | LOC387601 | XM_370537 | TTGAGTTGTCATGTAGATTAA | CAGGCAGTTTGTGAATATTAA | | |
| 387643 | LOC387643 | XM_373474 | CCGGCTCTACTGAACTTCAGA | CTGCTCAGATGTGAGAAGTAA | | |
| 387710 | LOC387710 | XM_373477 | CCGGGTGATAGGCATTAACTA | CAGCATCAATGTGAAGCCAAA | | |
| 387715 | LOC387715 | XM_373478 | GAGGCAGGCCTCGCCATTTAA | CCCGGCTGGAATCCAGCTCAA | | |
| 387717 | LOC387717 | XM_370584 | AACACAATGATTTGACAGGAA | AACAACTAATTTATGGAACAA | | |
| 387720 | LOC387720 | XM_370586 | AAAGATAGACTTCTTCTTAAA | CTGAAGAATGAGGAACATAAA | | |
| 387723 | LOC387723 | NM_001005180 | CACCAGTTCCTTAATCTTAAA | CACAGCGTGATTATTGCTCCA | ACCCTCTTCGCTATCCATCAA | AAGTCTTATTATACTGTCATA |
| 387748 | OR56B1 | XM_370607 | CAGCAGGCACTGAATATACAA | CAGGCCCATTCTTTCTTCATCTA | | |
| 387750 | LOC387750 | XM_370611 | AAGAAGACGCCAAAGAGAAA | AACCTATTTCCTATGAATTCA | | |
| 387753 | LOC387753 | | | | | |

Table4

| | | | |
|---|---|---|---|
| 387758 | LOC387758 | NM_203371 | AAGAAGATTAATGATCACTTA CAGGTCTACATTATTTAATTA |
| 387763 | LOC387763 | XM_373497 | ATGGCTGAAGGCATTTATTTA AGGGAGAGACTTATACTTTAA |
| 387781 | LOC387781 | XM_370634 | CACCTACTAAATACAAATAGA CAGCCAGATATTTGTGGACAA |
| 387804 | LOC387804 | XM_370648 | CAGCCTCATGTGGGTTTGTAA CAGGGCCGTGCTGACACATAA |
| 387820 | LOC387820 | XM_370665 | AACAGAAATGTTAAACTATAA AAACAGGACATCTATGACAAA |
| 387823 | LOC387823 | XM_373519 | CCGTGTGATCCTAAACATGTT ACACCTCTTCCCATCATTTAA |
| 387825 | LOC387825 | XM_370668 | CAAGGTTGTTAGAGACTTCAA CAGAAACATGTTGCATGTTAA |
| 387836 | UNQ5792 | NM_207375 | CAGAAATTGGACAGCCAGTAA AAGGAGATTCTTGGAAATGGA |
| 387849 | REP15 | XM_370686 | AAGGAGTACCTTGGATTTGAA AAGAGAGATAATAAAGTCACA |
| 387851 | AK3L2 | NM_001002921 | AAGCAGTTGCTGCCAGGCTAA AAAGCCAGTCATTGAATTATA |
| 387856 | LOC387856 | XM_370691 | AACCCGTGAATTGTGTAAATA AACAATTTACCTGGAAATTAA |
| 387871 | LOC387871 | XM_373539 | TAGGTATTTAATGTATTAATA AAGGTACTTGAGAAACATTAA |
| 387873 | LOC387873 | XM_373541 | CTGGGATTACAGAAAGAAGAA CCCGGCCAAATGAGCTATTTA |
| 387880 | LOC387880 | XM_370704 | GAGGGAATCATGGCAAGCCAA CTCAATGGTGAACACCCGAAA |
| 387882 | LOC387882 | NM_207376 | CACGACAATCTCATCATATTA ACCAATGTATTTCAACAATTA |
| 387887 | LOC387887 | XM_373549 | CACAGAGTCTTTCCAACTCAA AACGCTGTTTCTAGTGCTTAA |
| 387895 | LOC387895 | XM_373553 | ATGGACAATGAGAAACATGTA CTCAGTAACAATAACAAGAAA |
| 387898 | LOC387898 | XM_373556 | CGCGCAGATAATCTGGCATAA CTCTTGGGACTCAGAAATCAA |
| 387911 | LOC387911 | NM_001007537 | AAAGGCATCAAAGGTGATCAA AAAGGAGATCGAGGAGAGAAA |
| 387917 | LOC387917 | XM_373562 | ACAGGTGAAGAAACCCTTTAA AAGGATGCTCTTGACGCCAAA |
| 387923 | C13orf21 | XM_370724 | AAGGAACTGGCTGGCCCTGAA ACCGGCAACTGAGATGTTTAA |
| 387930 | LOC387930 | XM_370727 | CAACATTTCTGGCAATTTCTA AGGCATCAACATTTCTGGCAA |
| 387937 | LOC387937 | XM_373569 | CTGGACATCATTTAGCATATA CAGGAAAGAGATTACTTGGAA |
| 387950 | LOC387950 | XM_373578 | CCGGGCATTGGTGTTTGCAAA AAGGAGGAAACGGATCACCAA |
| 387974 | LOC387974 | XM_373583 | ATCGGATTCATCTGACATTTA CTGGTTTAAATGCCACCTTTA |
| 387991 | LOC387991 | XM_370763 | CCCAGAAACAAGGCAAATGAA CAGATACATCAAATAAAGTTA |
| 387992 | LOC387992 | XM_373594 | TAACAGATCTTTGAACACTAA CACTTAAAGAAGAGAATTTAA |
| 388007 | SERPINA13 | NM_207378 | CAACATAGACTTTGCCTTCAA CCAAATGCTTTCTATCTCTTA TCCGTGCATGATTGCTTTGAA AAGGATAGTAATGGTGATCAA |
| 388019 | LOC388019 | XM_373611 | CAGCAATACAGTCACATTCCA CAGGGTGTGTTTGCTGGATTA |
| 388021 | FLJ42486 | NM_207379 | CTCAAATGAGAATCAGCTTAA CACGCTCTTCTTCCTATGCAA |
| 388022 | LOC388022 | XM_370779 | CTCCACAGAACTGCCAGTAAA ACACGCTAAGTTTATGTTTAA |
| 388069 | LOC388069 | XM_370829 | TTACAATGCAGTAAACTTTAA AAGGAACCATGGAAATAACAA |
| 388076 | LOC388076 | XM_370833 | AAGAAATATCATGAAAGGAAA AACGATCATCGATGTTGTCTA |
| 388116 | LOC388116 | XM_370856 | CAAAGCAGAAACGGAATTACA CACCTGGTGAATGAAATCAAT |
| 388129 | LOC388129 | XM_373631 | ACGGAGACCCAGGAAAGATAA CAGCCGAGGGCGAGAAATGAA |
| 388142 | LOC388142 | XM_373638 | CAGCCCTCACTAGACTACAAA ACCATAAACCATGCAATACAA |
| 388143 | LOC388143 | XM_370879 | TCCCTGCGCCATCTTCCAGTA AACAGGTGTTAATAAATAAAT |
| 388159 | FAM42B | XM_370893 | ACAGTTGATCGCAAACTAGTA CAGATTGACTATCCAGTTTAT |
| 388166 | LOC388166 | XM_370898 | AAGAGGTTTCAGGCTGCCTTA CTCCCTTAACATAGACATTGA |
| 388174 | LOC388174 | XM_370905 | CAGCAATCTCTGCATGCCTTT CCCAAGAGCTTGTGTATCTTT |
| 388177 | LOC388177 | XM_370909 | AAGCCCTCTCAAAGCACACTAA ATGGGTGATAGTGGCTTTAAA |
| 388180 | LOC388180 | XM_373650 | TGCAATCAGACTAGAAGGAAA CCGGGCATGATTCTCAACTTT |
| 388205 | LOC388205 | XM_370930 | CAGAACCGATTTGGATTTGGA AGCCAGGATTCCAGTACAGAA |

Table4

| | | | |
|---|---|---|---|
| 388214 | LOC388214 | XM_373666 | GCGGCTGGATCTGTAATGAAA TAGGCTGATGTTAATAAATAA |
| 388227 | LOC388227 | XM_373670 | TCCGATGACTTCTGTCCCTAA CCCGCTGTTGGGAGTCCTTTA |
| 388231 | LOC388231 | XM_373671 | CAGGAAGAAACCTCATACCCA CAGCATGTTTCTGATCATATA |
| 388248 | LOC388248 | XM_370968 | CAGGGATGGAAGTGAAGTGAT CCAGGTCTTAGGAGAAGAAA |
| 388255 | LOC388255 | XM_370973 | CAGCATCTGAATTACACTTAT CAGAGTTGTGTTCACGAAGAA |
| 388276 | LOC388276 | XM_373685 | CAGAATGCCAAGCTCCAGTAA CAGAAGGAGCTTGGGAATACA |
| 388295 | LOC388295 | XM_373693 | CAGGCAGAATTAGTGACCGAT CCCAATAAGGCTGCTGACTCA |
| 388298 | LOC388298 | XM_370992 | CAGCAGGCTAGTGATGGTTAA CTGGGTGTTTGAAAGAATCAA |
| 388300 | LOC388300 | XM_373695 | TTGGGATTCATGCTAGATCAA TTGGCTGTTTCTAGAAAGATA |
| 388325 | UNQ5783 | NM_207103 | CTCACTGGTAAATAAAGTTAA ATGGTTCTGAGTGATATTAAA |
| 388344 | LOC388344 | XM_371023 | ACCGGTCATGCCCATCCGGAA AAGGGAGACAGTTCTGCTGAA |
| 388364 | UNQ9372 | NM_206832 | CAAGATAGAGTCTGAGTTTAA CACCTGATTGTTAAAGATAAA |
| 388387 | FLJ31222 | NM_207388 | CAGCCTCCAAATTTCTTTAAA CCGGCTGAAATTGTATACTTT |
| 388394 | LOC388394 | NM_203400 | CCGGCGCCGCGCGGCGATGAA TGGAAAGAAATCTTGTAATAA |
| 388419 | LOC388419 | XM_371078 | CAAGTTCATCAGGTACCTGTA CGCCAAGTTCTTCGACGTCAA |
| 388420 | LOC388420 | XM_373749 | TAGCCTTGTTGTTGGGATACAAA CAGCTATTTGGCACATTTCAA |
| 388428 | FLJ44861 | NM_207389 | CGGGTCCTTCAACTACCTCAA CAGGTACATCCTGTCCATCAT |
| 388429 | LOC388429 | XM_373756 | CTGGTGAAGCTGCAGGAACA ATGGGCGTCTCCTAACCGTAA |
| 388489 | LOC388489 | XM_373786 | CACGGTCAGCGTGGCGAAGTA TCAGATCATCTCATGTTGAA | CAGGCTGTGCAGTGTCCTGGAA TAGCGTGTTCACCGAGTGGAA |
| 388491 | LOC388491 | XM_371125 | ATGCAGGCACTTGACATTTAT CACTTGAACATAACAGGAAA |
| 388494 | LOC388494 | XM_373788 | CAGGAAGTATTATCCAATGA CAGAATCACAAGTGAAATAAA |
| 388514 | LOC388514 | XM_371145 | ACCCTATATAAAGATCTTCAA ATCTGCATCATCAACATTATA | CGGGACCCAGTTTATGGGAAA CGCCTTCAGTACTTCCACAA |
| 388524 | LOC388524 | NM_001005472 | ATGGAACAGTACATCTATAAA AAGGAGGAATTCAGGGTGAA | CACGGAGGCATCTTATGTTAA CCCGTCGTAACTTAAAGGGAA |
| 388531 | FLJ45744 | NM_207391 | CAGGAGCTTTATTCCCTATTA CAGCTTTAACTTGGGATTTAA |
| 388532 | LOC388532 | XM_371160 | CTCACAAGTGTCACCATGTA CTGGGATAGCTTCCTGAAATA |
| 388533 | UNQ467 | NM_207392 | CTGGGTGGTCCTGAGGAAGAA CTGATTCTCAACCTACCATAA |
| 388536 | MGC62100 | NM_206894 | CTGGTGGACTTGGAATGTTAA CAGCGATCACATCTTATTAAA |
| 388538 | LOC388538 | XM_371165 | AACCTTGGAGTGTACACTGAA AAGGGCAGACATGGAGAAGAGA |
| 388551 | LOC388551 | XM_371177 | CCGCACAGGCTGCACCATCAA CAGGCCCTAAAGAACGGCCAA |
| 388554 | LOC388554 | XM_371182 | CTGGAGTGGGTCCAATATGAA CACGGCCTTTGTGAAAGCCAA |
| 388564 | LOC388564 | XM_371196 | CTCCCTCCTCACTTGGATAAA CAGCGAGTGAGGCCCAGACAA |
| 388565 | LOC388565 | XM_371197 | CAGCGCTTCGAGCCCATGGAA CAGGGAGAGTCTGAAGCGCCA |
| 388566 | FLJ26175 | NM_001001668 | AAGGCTTTCACTGATCACATA AAGCATAGAGAGGATAAGTAA |
| 388579 | LOC388579 | XM_371207 | AACAAATGTTCAACATTCAAA ATGAGGGAGGTGGATGAACAA | CTGGTGTCTGAATATCAGCAA CCAGCAGAGATGTTTGATGCTAA |
| 388587 | LOC388587 | XM_373822 | CCGGTTAGAAATGGCCGCGGA CTGAGTGGATGTAGATGAGAA |
| 388605 | HS6ST1P | XM_371230 | CAGAGGTAGCTGCGTCCTCAA ACCCATCTGTAGCCTCCCTCAA |
| 388616 | LOC388616 | XM_373837 | TACCATGGGATGGTTACTGTA CCGTGTTATTTGGGAAACTCA |
| 388621 | LOC388621 | XM_371243 | AAGCCGAGATAGCTTCCTGAA AAAGAAAGAAGCCAAAGAGAA |
| 388624 | LOC388624 | XM_371244 | CTGGCACTACTTCTCTCTCAA AAGGATGTTACCAGTGCCTTA |
| 388630 | LOC388630 | XM_371250 | AAGGGAGTGGATGTTAGGGAA CAGGACATCCATGGCCAAATA |
| 388633 | LOC388633 | XM_371253 | AAGCTTGTATAAACACTGACAA CACATCATTCTGAATAGGAA |
| 388648 | LOC388648 | XM_373852 | CCGCCAGCAGCCGGATTACAA CAGGGATGTGCGGGTTCTCAA |
| 388652 | LOC388652 | XM_373854 | CTGGCATTGTTTACTGGAGAA AAGGGAAGTCGACGAAATAAA |

212

Table4

| | | | |
|---|---|---|---|
| 388662 | SLC6A17 | XM_371280 | CACCCTCTCCGTGTCCTACAA CAGCTACAATAAGCAGGACAA GTGGTCGAGAATGCTGAGAAA CTGGATTTATGGAACCAAGAA |
| 388714 | LOC388714 | XM_371326 | CAGGAATATATAAAGACATAT CCGGATGATTACCCAAACTAT |
| 388720 | LOC388720 | XM_371330 | CTGACTTACTGTTTCAACAAA CGGATACAATAGATAATGTAA AAGGTGGATGAGAATGGCAAA TCGGATACAATAGATAATGTA |
| 388730 | LOC388730 | NM_203376 | TTGGTGGTGACTACACCTAAA CTCCCACTTTGTGAAGTTTAA |
| 388818 | LOC388818 | NM_371418 | CAGCTTCTTTGTTCCAGTGAA CACCTTCTAGGGATAAATAAA |
| 388830 | LOC388830 | XM_371418 | CAGGAGGGAAAGTCTAATGAA AAGAATTGTGTTGGTCATGAA |
| 388882 | LOC388882 | NM_001006606 | CACCTTGGTGAGAATCAGGAA CCACAGATACAGATTATTAAA |
| 388885 | LOC388885 | XM_373949 | ACCGTCAGCCACCTCAGTAGA AGGCTACTACGTGAAGGAACA |
| 388900 | LOC388900 | XM_371466 | CAGAGAAGAATTAATGTTGTA CAGGAAGATGGAGGTCATCAA |
| 388903 | LOC388903 | XM_373962 | CGGGAAGCACTTAACCAATGA CAGCCAATCCTAGCTCGTTAA |
| 388907 | LOC388907 | XM_371470 | CAGACAATAAACTTAGGGCCA TTCGTGTAACTGACAGAGATA |
| 388917 | LOC388917 | XM_373970 | CCATATGAAATTGCAGTTAA AGGGAGAGAATCTCAGACTACAA |
| 388922 | LOC388922 | XM_371476 | AAGGATAGCAAGACAAATGAA AAGGAAGTTGAAGGAAATCAA |
| 388927 | LOC388927 | XM_371478 | CAGCTCCTTTCTCAAGCTTTA CAGGGCTTTCAAGTAATGAA |
| 388929 | LOC388929 | XM_371480 | CCAGATTTATTACAACATAAA CGCCATAATGTGATAAATAAA |
| 388931 | LOC388931 | XM_371481 | CACGTTCTTCCAGTGCCCTA ACCCAATGTTCACACAAGTAA |
| 388939 | LOC388939 | XM_371488 | AACCTCTTCATCCCAGCTGAA AAAGAACAGCTGATTAAACTA |
| 388942 | LOC388942 | XM_373981 | CAGGTTGACATCACATATGTA CAGGACGAAGTCACCACTAA |
| 388954 | LOC388954 | XM_371495 | ATGGAACAGTACATCTATAAA AGGAGGAATTTCAGGACGGGA |
| 389002 | ANKRD20B | XM_371535 | AGAAGTAGTCACCGCCATGAA GTTATTATTCATATTAATTTA |
| 389033 | LOC389033 | XM_374010 | GCCAGGCAGCACCCTCTGATA TCGGACTGAGAGCATTCCTT |
| 389055 | LOC389055 | XM_374016 | TAGCAGTGTGTGACACCATAA CGGGAGTGCTCTGCAACTGAA |
| 389058 | SP5 | NM_001003845 | CAGGGCGGTTCCAAACTCTAA CAGCATGATATGTCTCTGTAA |
| 389067 | LOC389067 | XM_374021 | CAACATTATTGTGACTATCAA AAGAGTTATAATTACATTTCA |
| 389070 | LOC389070 | XM_374022 | CAAGTTTCAGTGCTCAATATA AAGAGATCAGTCATTACAATA |
| 389075 | RESP18 | NM_001007089 | TAAGGAAGAAATTATCTATAA CAGATTTGAATCATTAAAGTA |
| 389077 | LOC389077 | XM_374025 | CACCTTACAGGCTGACCTTAA TCCCACAAAGTTAGAAGCAAA |
| 389089 | LOC389089 | XM_374029 | TACAGCATTCTCGACCTGTGA TGGGAGGCTTGTGTTGAGTTA CACCGTGGTCACCGTCTTCTA CACGCCAATAATTAACCCTTT TTGGGTCAAACTTCACACTAA |
| 389090 | OR6B2 | NM_001005853 | CAGGTTTCTGCCCGCCAAGAA CACGTAGTTGCGAGAGCCCAA |
| 389108 | LOC389108 | XM_374038 | CAGCCTGCCCTGCTTTATAAA CAGGCACAGCCTGAAGAGGAA |
| 389118 | LOC389118 | NM_001007540 | AAGCATTGAATGAAGACATT CAGGGCTAATGAACCATCTAA |
| 389123 | IQCF2 | NM_203424 | CCCTGGAGACTCATTCCTACA TCCCGAGGCTACAACACAGAA |
| 389127 | LOC389127 | XM_374045 | GTGCATATTCCTGATCCTAGT AGCCATGCCTGCCATCTTCAT |
| 389145 | LOC389145 | XM_374048 | AAGGCCAGTAAGACCAGGGAA CAGCACTGGCAAGGCCAGTAA CACCAGTATTGTTCTTCTTCA AAGGAACAACTGTATCTTATA |
| 389147 | LOC389147 | XM_371663 | AAGGTATATTCTAGACCTCAT AAATGTTAGTTTATTACTGTA |
| 389151 | LOC389151 | XM_371665 | CCGGTCCTCTTGCCCACTA TCGGGCTTGGATTTGGAGAAA |
| 389153 | LOC389153 | XM_374053 | CCGGCTCTCGGCTCCAAACAA CCGAGTGTGGTTTGCTATCAA |
| 389189 | LOC389189 | XM_374071 | CCGGGCTTCCCTTGATGTTAA ACCGCTGGAGTTAGAAATGTA |
| 389197 | FLJ46481 | NM_207405 | CAGAAAGTCTGAGAAAGGATA CAGGTTGTCATTGGAAGTCCA |
| 389233 | LOC389233 | XM_374091 | AAGGATATCGTCACAGATAT CACAACTAAATGAAAGCCTAA |
| 389239 | LOC389239 | XM_371714 | AGACATGAAATATGACATATA CAGATTGGCTCAGAAACCATA AAGCAACAGGAAGCAAATGAA CACCAAGAGTAGACTGATAAA |
| 389246 | LOC389246 | XM_371718 | CAGGATAGAAATTGGAGGCTA AGGGAATGAATGATTAGCAATAA |
| 389247 | LOC389247 | XM_374099 | |

Table4

| | | | |
|---|---|---|---|
| 389253 | LOC389253 | XM_374104 | CCGGGCAGCTGCTCCCCTA CAGGCGCGGAATCCATCCAAGTA |
| 389288 | LOC389288 | XM_374120 | TGGCACCTGAGAAAGCATTA AACGCCATTCATTTCTCTAAA |
| 389290 | LOC389290 | XM_374121 | AAGGAACTAAGAGTTGCAGAA CAGCTGAGAATACATACCAAA |
| 389293 | LOC389293 | XM_371741 | TCCCAGAATAGCCCACATTAA CACGGGAGAACTCATAATGAA |
| 389305 | LOC389305 | XM_371757 | AAGAATGAACTAAGGTGTCTA AACCTTTGTGAAGCTCAACAA |
| 389308 | LOC389308 | XM_371758 | CAGAATGTCCGGCCTTATATA CAGCGCCTATATTGAATAAA |
| 389321 | LOC389321 | XM_371769 | CAGAATCGAGGCATCTGTTGA CAGCATCGAGAACAAGCTAAT |
| 389332 | LOC389332 | XM_374141 | CGCGCTCAGAGCCGCCAGAAA CGGGCTGTTGTAAAGCCGAAA |
| 389333 | LOC389333 | XM_374142 | CCGGGACGCCTCACAGCCCAA CAGGAAGCTCAGAATGGGTTA |
| 389338 | LOC389338 | XM_374145 | CTCTCCGAATTGAAACACTAA TAAGAAGATTGGCGAAATTTA |
| 389342 | LOC389342 | XM_371781 | ACCAAGTTCAATGCTGATGAA CCAATAAATTCTACTAACTCT |
| 389345 | LOC389345 | XM_374150 | CACAGAGGCTACAACTAGAAA CAGGCATTATCGGGACTATAA |
| 389352 | LOC389352 | XM_371791 | CTGGGAGCAAGAGTCCGGAA CCGGATGTCCAGAATGATGAA |
| 389383 | UNQ3045 | NM_207409 | CAGCTTAGTATCTGCACATAA CAGGGATTGCATCATATTCAT |
| 389389 | LOC389389 | XM_371820 | TCCAACAAACTGTGTCTTTAA TAGGATGGGAAACAAGGTTAA |
| 389390 | LOC389390 | XM_374167 | TTGTTCCAGTTCAATGATTAA ATGGAATCTGTCTCCTTAA |
| 389398 | LOC389398 | XM_374170 | CAGCTATGATACTCAGAGAAA CAGGAATATTTCACCCAAGTA |
| 389413 | LOC389413 | XM_374175 | CTGCATCAACTGCCTCCCTTA CTCACCTTACTTTCACCTTAA |
| 389415 | LOC389415 | XM_374177 | CCAGCCCTTTGGAAGAGAGTA CCAGGCCTTAGCCCTGAGCTA |
| 389425 | LOC389425 | XM_371843 | AAGACTGATCTTTGCTGTGGAAA CTGGCTGTCCTGAAATATTAT CTGATCTTTGCTGGAAAGCAA AAGGTGGATGGATGAGAATGGCAAA |
| 389427 | LOC389427 | XM_371845 | GAGGATCAAATTATAGTTTCA GCGGTTGAATCCAGTCATAAA |
| 389435 | LOC389435 | XM_371853 | AAGAGCTGAGGAGAAGATTAA CAAGCAGGCTGTCACCGTGCA |
| 389458 | LOC389458 | NM_203393 | CCGGATGCTAACACCCAGGAA CCCAGATGCATTTATTAGAAA |
| 389469 | LOC389469 | XM_374203 | CAGGATAGTCAAGGACCTCTA AAGAGAGAATGTAATACACAA |
| 389533 | LOC389533 | XM_374226 | CCACCTTATATTATAGTCAAA CAGGCACTCCTTGATGAAATA |
| 389541 | LOC389541 | NM_001008395 | CGGCTGCACCGCGCATGAAT CTGTCTGTGGTCTTTGGAGAA |
| 389599 | LOC389599 | XM_372002 | CGGGAACGCTGGTAACTACAA CAGGATTTACATGGCTGGGTA |
| 389618 | LOC389618 | XM_372019 | CTGGAATGCCTTTGTACATAT CTTGCCTGAACTTCCCAAGAA |
| 389634 | LOC389634 | XM_374252 | CAGGGATGGCAGTCCCAGGAT CCCGCCGCTACCGGGCAGGAA |
| 389641 | LOC389641 | XM_374260 | TTCAATCTATCTGAAGCTTTA AAGAGTCTTGAAAGTCCTTAA |
| 389643 | HMFN0672 | XM_372035 | AAGAGTCATCTTCAATACCTA TAGAAAGAAATGAAATGATAA |
| 389674 | LOC389674 | XM_372050 | AAGAATTGGATGCAGCTATGA AAGCTTGTGTATCATCCATTA |
| 389676 | LOC389676 | XM_374276 | GAGGATTTAGAGGGCAAGCAA CAGGACTTTGACAGCATTCAT |
| 389727 | LOC389727 | XM_372092 | CTGGCGGTGAATGACTTTGAA CCGGGAGAAGTTCTATGCCAA CCACGCAGAGATGACAGCCAA CACGTACACACGGGAGCGCAA |
| 389734 | LOC389734 | XM_372097 | TAGCATTACACATGATGTTTAT AAGGATTGGAATTAACTTTAT |
| 389753 | LOC389753 | XM_372112 | CCCAATCTCCTTTGGATATAA CTGATTTGTGGTCTTCATTTA |
| 389756 | LOC389756 | XM_497059 | CCCGAGGTGGATGAGGGTGCT CTGCCTCTTGCCTGCAGCGAT |
| 389757 | LOC389757 | XM_372114 | CAAGTCCATCTTTGAACAATA CGGGACCGAGGTGCAGCAGTT |
| 389773 | LOC389773 | XM_374297 | AAGAAATTGTAGCGCCAGTAA AACCTAAATTTAATTAATAAA |
| 389777 | LOC389777 | XM_374300 | AAGATGCTATGAATCCAGAAA TCGCATCTGGATAATCCTTCA |
| 389792 | IER5L | NM_203434 | TCGAGCCATTGTCGCCTTCTA CAGAAACAGCAGAAGCGTTAA |
| 389813 | LOC389813 | XM_372159 | CAACGCCTGCCTCTCACTTCAA GACCGTGTACCAGGACATCAA |
| 389832 | LOC389832 | XM_372190 | CAGGAGAACGCCAGCCCTTAT CTACCATAACACTGAAATAAA |

Table4

| | | | |
|---|---|---|---|
| 389840 | FLJ16518 | XM_372199 | ATCCCTCTATTCGGGAATTAA CTGGATGTTCGCGATGGACAA CTGATTGTTGCTCTGGACAACAA GCCGATCAGCATAACATTAAA |
| 389844 | LOC389844 | XM_372202 | GTCCTTGATTCCAATCATGAA CAGCTGCGAGGTCGCCATCAA |
| 389846 | LOC389846 | XM_374325 | CAGGATTGGGTGGATAAATAT CTGGATGCCAATCGAGATTTA |
| 389848 | LOC389848 | XM_372204 | AAGGAGGAATTTCAGGGTGAA TCCGGGAATACTGGCCAGGGA |
| 389878 | CXorf35 | XM_374333 | ATGGGTGGCGATGTTGAAGAA CAGCACCATACTGGAACTTCA |
| 389907 | LOC389907 | XM_372274 | CCGCCTGCCAACCGCCTTCAA CACGCTGGTCACCGTGGGTGA |
| 389932 | tAKR | XM_372302 | CAACGGCACTGTGAAGAGAAA CTCAGCTTATACACACCTGAA AAGATATATTCTATACCACACAA AAGGTTGCTATGAGTAATGTA |
| 389950 | LOC389950 | XM_372307 | TTGGTGTGTGATGTGGCCTAA CTGGGTAGTGTCTGGACAATGCA |
| 389953 | LOC389953 | XM_372310 | CAGCTAAGGAACACTGTGTAA CAGGAATGGATTCTCCACATA |
| 389988 | LOC389988 | XM_372323 | TACATCCAAAGTTGCCCATAA CTCACTGAGACTGGAGTCGAA CTCAATGACGTGTACAGTTTA CAGCATGAGATACACCAACAA |
| 390003 | LOC390003 | XM_497249 | CACAACTTATGATGTTTATAA ATCCGTTACGTTGGTGACCAA |
| 390020 | LOC390020 | XM_372337 | TTCCAACATCATCACCATCAA ACCCACCTTCTTTGAAATATA |
| 390036 | OR52K1 | NM_001005171 | CCCAGTGATTGCCCATTGCTA CCCATGGTCAATCCTATCATA |
| 390054 | OR52A5 | NM_001005160 | CACAGGTTTGGTTCACACATA TTAGTTATAATCAAATATGAA |
| 390058 | OR51B6 | NM_001004750 | TCGGAGTGACATTGACCACAA ACCCATTTATCTATACAGCATTA |
| 390059 | OR51M1 | NM_001004756 | CACAGATACCACCTTCAATAA CCCAATCATATACAGCATTAA |
| 390063 | OR51I1 | NM_001005288 | ATCCATGTTAACAACATTTAT CAGGCATTCCTGGGATACAAA |
| 390067 | OR52H1 | NM_001005289 | TCCCAAGATGCCTGCCAGAAA TCCCTTTACTTTCCTCCACCAA CAGATCAGAGATAAGGTTATA TACCACCATCTTGACTCCCAA |
| 390072 | OR52N4 | NM_001005175 | ACCAGCTTCATTTATTCTGAA TCCCTTTACTTTCCTCCACCAA |
| 390083 | OR56A3 | NM_001003443 | CCCAAGAAATTAAGCAGGGAA CTGCGATGATGTCACCATCAA |
| 390144 | OR5D16 | NM_001005496 | CTCCCTGATATCACTCTCTTA TAGGCATTGGTATCCATTTAA |
| 390158 | LOC390158 | XM_372396 | GCCCAAGACAGAAGTTATGAA CTGGATGATAGTTCATGTAAT |
| 390167 | OR5M10 | NM_001004741 | CAAGAACATTTGCATCTCTCT TTACATGATGGCTTCCTTAA |
| 390183 | LOC390183 | XM_495875 | TTCCCAGCTTAACAGACTTAA CAGGAGGGAATTTCTGTCTGA |
| 390190 | OR5B2 | NM_001005566 | TGGGACAGTAATCTTCATCTA AAGAAAGTGTTGAGAAGGCAA |
| 390205 | LOC390205 | XM_372416 | CGGCCTCTGGCTCTCACGGCAA CTGCCTCTGGATCGAGGGCAA |
| 390212 | GPR152 | NM_206997 | CCAGCCACAGGCAGACACTAA CAGCTAGATTCTGAGGGTCCA CAAATGGACACTACCATGGAA CAGGACCCATTCTGTCAGCCTA |
| 390221 | LOC390221 | XM_497309 | CCCACTGCTCTGTGCAGTGAA CTGGACCTCAGGGTCTAACAA CTCCTACACCTGCAAGAGCAA CTGCCTGCAAACGGCACTTTA |
| 390226 | LOC390226 | XM_495887 | TACCAGGAGGCTGGCATTCCA ACCAGGTTACCATATACTTCA |
| 390231 | LOC390231 | XM_372423 | CTGGACAAAGAGGAATGACAT AAGACTCCTGTAATTGGGTTA |
| 390233 | LOC390233 | XM_372424 | AAGGAAGGAGGATAATCACTAT CTGAGCGAAGCAGACATGGAA ACCCAGACTATGTGTCATTAA CACTCTGATTACTCAATCTTA |
| 390234 | LOC390234 | XM_372425 | AAGCTGAACCTCAACCAAATA CAAGAGATTTGTTCAATGAAA |
| 390260 | OR6X1 | NM_001005188 | CACAGTAATCACAGAATTCAT AAGACTGGTCATGCAAAGTAA |
| 390264 | OR10G4 | NM_001004462 | ATGGATGCCATGGATGGAGTT GCACATCCTCAGAGGAAATAA |
| 390281 | LOC390281 | XM_497327 | AAGCACCTATATGCCCTTCTA CTGGAAGGACAGGTTGTTAAA |
| 390282 | LOC390282 | XM_372447 | AAGCTGTTGAGAGAAAGGAAA AAGGAAAGTTAAGGCCACCAA CGAGATCATGCTCAACGGTAA AACGTCAGAGTGAAATATGTA |
| 390284 | LOC390284 | XM_372448 | CCAGACAAACTCAGAGCTTCA CTGGTGGTGGTCATCGAAGAA |
| 390335 | LOC390335 | XM_372466 | AAGAAAGAGCTGCCCAGAAA CAGCTCTCATCTTGCAGTACA |
| 390349 | LOC390349 | XM_372472 | CAGGTGTGCAGAGATACTTGA CAGCACCAATGAAGACAGGAA |
| 390377 | LOC390377 | XM_372486 | CACCCACCGTTTCATCACCAA CAGAAGAGCTGGCGTCATGAA |
| 390387 | LOC390387 | XM_372490 | CAGGGTCTCCATGGCCTGTCA ATGACTCTGATTCCACATTAA |
| 390407 | LOC390407 | XM_497376 | AGGTAGATGGTTTGACCTTAA TCGAGACATCAGGACCTTCTA |
| 390421 | LOC390421 | XM_497382 | CTGCCTGCATTTGTTCCTAAA CAGGAGTGAGATCCCCACCCAA |

Table4

| | | | | |
|---|---|---|---|---|
| 390429 | OR4N2 | NM_001004723 | AACAGTGATAAGAGAATTCAT AAGGTGTTTAATAAGCACATA | |
| 390442 | OR11H4 | NM_0010004479 | TTGGATTCCTTGGATACCCAA TGGGACAGTCAGTGTAATGTA | |
| 390443 | RNASE9 | NM_0010001673 | AACGTATTCCTCATACTATAA AAGGAGTGTATTGTAATTTAA | |
| 390445 | OR5AU1 | NM_0010004731 | CAAGAGAAAGTGATCTCTTA CTCCTACTTCTTAATTCTCAA | |
| 390529 | LOC390529 | XM_390529 | GACCCTGGAGGAAGAAGCAAA CAGGCGTTTGCTGCCTCCTTA | |
| 390550 | LOC390550 | XM_374354 | CCCACTCTCATGGATGCCTTA ACACTCAGTATTGGTAGATAA | |
| 390551 | LOC390551 | XM_372556 | CTGAGGTGGGTCTGACTCAGA AAGCAGGTAGGTGCGAATGAA | |
| 390560 | DNM1DN4-1 | XM_497444 | AGCAGTGTTCTTCTTCCTATCA CAGCTTCATCATCCTGGTTCA | |
| 390564 | LOC390564 | XM_372562 | ACCGATGGGTACAGAGCCCA CTCCCTACTCTTTGCGAGGAA | ATGGCGACCTTCTGAACAAA CAGGGTCAGCCGAATGCCAGA |
| 390570 | LOC390570 | XM_372566 | CTGTCTCACCGAAGATGTTAA CACGAATGTACCAGTGAACAA | |
| 390577 | LOC390577 | XM_372569 | CCGAGCTGGGTCGGAGCATGA TGGCCCGGAGTTAGAGGCCAA | |
| 390641 | LOC390641 | XM_497469 | CAGGTGGACATGTAAAGAAGA CAGCAGCTGAAGGACAGATAA | |
| 390667 | LOC390667 | XM_372607 | TTGCTCGTGGAGGAAGACCTT CTGGAGGAACAGTTCCGGAGA | |
| 390736 | LOC390736 | XM_372641 | CGGCTCGATGTCTGCAGGCAA GTGGAGGAAGATGGACAACTA | |
| 390801 | LOC390801 | XM_372676 | CACACTGAACTTCCAGAACTT CACCACTGTGGACAACAACTA | |
| 390842 | LOC390842 | XM_497583 | CTGAGGAACCTGCAAGACTCA ACCAATGACAGTATCCAGTAT | |
| 390919 | LOC390919 | XM_497622 | AAGGGCTTTCCTGCAGCGCAA CAGAGAAAGTCTATACATCTA | |
| 390975 | LOC390975 | XM_372749 | CTGGTCCACGCAGATGTCAAA CTGGTCACCCATCGTCAGAAA | CCCGGCAAACAGTCTGAGGAA ATGAACTAGATCCAAATCCAA |
| 390988 | LOC390988 | XM_372755 | CCGCCTGGAGGAGACCCACAA GACGGCCATGTTCGACTTCAA | |
| 391039 | LOC391039 | XM_497673 | CACCCTCTATGACACAACCTA CAAGGACATGAAGATGCATAA | |
| 391053 | LOC391053 | XM_497689 | ATGATGGATAGTGACACCATA ATGAACAACTCTACAACATAA | |
| 391059 | LOC391059 | XM_372784 | CACCTTTACATGACCCAAGAA TCCTATAATTCACAACCAAA | CAGATTGAAGTTACTTTGTCA CTGAAGGTTCATGGTGCCTTA |
| 391105 | LOC391105 | XM_497715 | CAGCCTCGCTAAGGACTTGAA TTCATCTGAGTTTGAGATTAA | CAGTTTCATTTGACTCCTCAA CTGGATTTAACGGAAGTAATT |
| 391109 | OR10K1 | NM_0010004473 | CACCCTAATATCTGTGTCATA CCAAGACTAATTACACTTCAA | |
| 391123 | LOC391123 | XM_372802 | AAGGTCATTGTCACTGTCCAA ATGAGGCTAGGTGGGAAGGAA | |
| 391126 | LOC391126 | XM_497721 | GTGACTTCCAACCAAATCAAA CCACATTCACAGGAAGATTAT | |
| 391137 | LOC391137 | XM_372805 | TTCCTGCTTCACCACCCTCAA AGGATTCTGCTCAGCCAGTAA | |
| 391142 | LOC391142 | XM_496426 | CAGCAGAGACACTACAATCA CAAGAGGGCACGGGAGGCCAA | |
| 391192 | OR2L3 | NM_0010004687 | CTCCATATTCCTTATTGCCAA CACCCTCACTCCAATGCTCAA | ATGGCTCTAATTGGAAACCTA TAGGATTCTTCCCACCATCAA |
| 391205 | LOC391205 | XM_372838 | CTGGAGGAGCAGCGTCACGCA CGACATCGTCTCTGCTCTGAA | |
| 391209 | LOC391209 | XM_372840 | CTGGTCGATCGCCACTTGTACA CACGGGCTGTGCTGAATGACA | |
| 391253 | C20orf137 | XM_372869 | CCGAAATAAGACACAAGAACA CCGGCTGTAATGGCAACCTTA | |
| 391265 | LOC391265 | XM_497576 | CCCAAGAGGGCAGAGGAACA CGGGACCAAGTCTGTCACTTA | |
| 391340 | LOC391340 | XM_372908 | ATCCCTATATTGAGGAGCAAA CTGCCCATCATCTGTATCCTA | |
| 391347 | LOC391347 | XM_372913 | CAGGCCCTGCTGAGCTAGCAA CAGATATGTGTTGTCCTGTTA | |
| 391370 | LOC391370 | XM_372926 | CAAAGGCTTTGGTGTGTTCAA AAGATTGGGCTTCACCCATAA | |
| 391429 | LOC391429 | XM_372954 | CAGATGGCATAGAGCATCCAA AAGATGGGATAGAGCATCCAA | |
| 391599 | LOC391599 | XM_497949 | CAGGGACTTCATCAGCTGCTA CAGGACCTGTTTGGAGAACGA | CAGGAGATATTTAGGTACCAA TCGGACAAGGATGGAAACTTT |
| 391634 | LOC391634 | XM_496686 | CCACATCTACTGCATGATCAA AAGATTGGATGTTATATATTA | |
| 391711 | LOC391711 | XM_373037 | AAGAGAAGGATGATAAAGAAA AAGAATTATAAGCACATTTCA | |
| 391717 | LOC391717 | XM_496725 | ATCAGGTGAGAGCGTGTGAAA CTGGTGAATTTGAGAAGTGTA | |
| 391722 | LOC391722 | XM_373042 | CACCAGAACAGTGTCATCTT ACAGAGAAATGTGAATAAGAA | |
| 391730 | LOC391730 | XM_497990 | CTGGATGGAATTCCTTCCCAA AAGAACTTGCTCCATCATTAA | |

Table4

| | | | |
|---|---|---|---|
| 391733 | LOC391733 | XM_373053 | GTCAGAGACGGAGAAAGATAA CACCATCACGGCACACTCCAT |
| 391769 | LOC391769 | XM_373079 | CACGCAGCTGCTCCTGCGCAA CTCCGCAGAGAGGGTCCTTAA |
| 391773 | LOC391773 | XM_497999 | CTCATCAACAATTACGGCCCA CAGCGTCATGATAACGTGAAC |
| 391804 | LOC391804 | XM_498008 | CACACTTTCTAGCCCGTACAA CCGAAGGGCGTAGTCACAGAA |
| 391819 | LOC391819 | XM_498013 | CTCAGTTGGAGGAATTGGAA CCAGGACATGGCGGTCAACAA |
| 391827 | LOC391827 | XM_498018 | TGGGTTGATGGTGGAGGTAGA CCCTGGAATCTCTACACTAAA |
| 391833 | LOC391833 | XM_498020 | CCGGACTTCGCTGCTCCCGAA CAGGCCAGCAGCATCCTTATA |
| 392169 | LOC392169 | XM_499232 | ACGGGCGCGGTCAGTGCCTGA ACGGTGAAGACCTGAGATTTA |
| 392222 | LOC392222 | XM_373253 | ACGCACCAGTTTCTTGCTTTA CTGAGTGATGAGGAAATGAAA |
| 392238 | LOC392238 | XM_374372 | AAGGGAAGAATTACACAGGAA CAGGTACACGTAAGAAAGAAA |
| 392262 | LOC392262 | XM_373263 | CACCTATGACACAAATGTCAA CTGCTGAAGAATCAAGTATAA |
| 392267 | LOC392267 | XM_496992 | CACCTCAGCCCTTACCTGGAA TGCCATGGTGTATTACGTCAA |
| 392281 | LOC392281 | XM_497005 | TGGACCCAAATTGCTTACGAT CAGTACATTACTCCTCCAGCA |
| 392309 | OR13J1 | NM_0011004487 | CAAGATCCTGGCAGTGCTGAA CCCTCTGTGTCTCAGCCATGTA |
| 392376 | OR13C2 | NM_001004481 | AAGGGACTTTCTGGTCACCCA AAGGATGTGAAAGAGGCAGTA |
| 392392 | OR1K1 | NM_080859 | CCGTGTGGCCACTGTCATGTA TGGGACAGTCATTGCAGTCTA |
| 392395 | LOC392395 | XM_373319 | CAGCCAGGCCATCGCCAACTA TGGGACAAAGAAAGACCAA |
| 392459 | LOC392459 | XM_373344 | ATGGAGCACCATTTCCATGAA CAGGCTAGCAACAAAGGTGAA |
| 392517 | NCBP2L | XM_373362 | CAGGCCTGTGATTAAGAGCAA CCCAAGTGTTGTAGCCTTAAA |
| 392546 | LOC392546 | XM_373372 | GCGGTCCGCATTACTCACATA CACCCTCTTAACGATGATGGA |
| 392556 | LOC392556 | XM_498396 | CACTCTATGGAGCCAATCTAA CACGATTATAAATGGAGGGCAA |
| 392583 | LOC392583 | XM_374379 | CCGCCTTGTGAGCCAGAGCCA CCAGCGGAGGTCCTCAGGAAA |
| 392702 | LOC392702 | XM_374730 | CTCCTGGGCAGCGGAAACAAA CTCCCGGAATCTGGTGGACAA |
| 392758 | LOC392758 | XM_374498 | TCCAGTGGTCTTTGGAGAACA CGGCTGCACCGCGGGCATGAA |
| 392895 | LOC392895 | XM_499284 | AAGGTGCACCAGTAAGATGA CAGTGGGAAGAGGAATTTGAA |
| 399665 | EEIG1 | NM_203305 | CAGGAAGGTTGGAGCAAGCAA TGGAATCTTCTTGAAAGTTTA |
| 399668 | MGC39606 | NM_203306 | CAGCGTGAAGGGCCTGAACAA CAGGTCCTGGAATGAAGCTGTA |
| 399671 | MGC48595 | NM_203309 | CACTATGTTTATAAAGAGAAA CTGCTTGGATATGAAACATAA |
| 399706 | RP11-529L18.1 | XM_378200 | AAGAGCTTCGTTGTTAGAGAAA AAGACAAATGAGACAGAGCTA |
| 399708 | LOC399708 | XM_378203 | CAGGGTATCAAGAGTCAGTAA AAGGATAGAATTATCCATCTA |
| 399715 | LOC399715 | XM_374766 | ACCAATAGCATAAGTATTAA CAGCCTCAGTACTGTAAGAAA |
| 399763 | LOC399763 | XM_374803 | CGGTATATAAATAGAATTAAA CAGGTGTTTCTGAAAGATTAA |
| 399833 | FLJ44653 | NM_001001678 | AACCCTAAGCCTAAACCTTAA ACCCTTAACCATAACCCTTAA |
| 399851 | LOC399851 | NM_207427 | TGGCTTGAACCCAGAGGTGAA CAGAGTCACACTGGATTTACA |
| 399919 | LOC399919 | XM_378299 | CTCGCTCAGGTGAGCATTCAA TAGAGAGGTAATTAAGTTAAA |
| 399921 | LOC399921 | XM_374904 | CCCATCCAGGAAGGTTATATA CAGGGTCAAAGTTCTGAGCAT |
| 399937 | LOC399937 | XM_374917 | AAGAGACAGAATGACAAGATA CTGAAGCAAATGTACCATAAA CAGCAACTCAATGAAAGCCAA CAGAGTGTGAGTGATACTTTA |
| 399940 | LOC399940 | XM_374920 | AAGAGACAGAATGACAAGATA CTGAAGCAAATGTACCATAAA CCGGCCCGTGTTCTACCTCAA CAGCAACTCAATGAAAGCCAA |
| 399961 | LOC399961 | XM_378317 | CTGAGAAATGAAAGTGTGAAA ATGGTGCTTCCTATAATCCAA |
| 400047 | LOC400047 | XM_378363 | TTGGACTTGACCCTACTGTAA AAGGAGTCTTCAGGAATCAAA |
| 400083 | FLJ43808 | NM_207438 | TACATTTAATATCCCAATTAA AAGGATGAAATTTGACGAATA |
| 400099 | LOC400099 | XM_378399 | AAGGTCTGACAGAAACTTTAA CAGGTTCATGTCCCAATGAAA |
| 400156 | LOC400156 | XM_375035 | CAAGCTGTATGTGAAGCTACA AAGGACAACGCCATTAAGAAA |

Table4

| | | | |
|---|---|---|---|
| 400201 | LOC400201 | XM_378449 | ATGGAGCTATGAAATGGCAAA AAGGATATTACCAACAATGGA |
| 400224 | LOC400224 | XM_375090 | AGGGTGGGTGATGACCTTCTA AAGAGCTTTGAAACCAATAAA |
| 400238 | LOC400238 | XM_378473 | CAGGTCAACTGTGGAATTTGA CACCATTTAGAGGACAATGAA |
| 400263 | LOC400263 | XM_378491 | AAGAGCCATCATGGTGATCAA ACGGAGCAACGTCCAGTTTAA |
| 400353 | LOC400353 | XM_375183 | CAGGCATAACTGAAGGTGAAA CAGGCAGGAGAATCACCTAAA |
| 400400 | LOC400400 | XM_378529 | CAGGGTCTCACCTTACAGCAA CACATCTAGCATGGCATATAA |
| 400446 | LOC400446 | XM_496093 | AAGGCTTTCTTAGGTGTGAAA CTGGTTAAGTTGTGTACTAAA |
| 400475 | LOC400475 | XM_378553 | TTGCAGAGTCTCAATACCCAA TAGCTATAAATTATCATATAT |
| 400481 | LOC400481 | XM_375284 | CAGCAGCAACAACAACAACTA CCCGACCTACCTCCTGTTCAA AGGCACCTTTCTAGAGATTAA CAGGGTGAAGAGAATCTTCTA |
| 400496 | LOC400496 | XM_378562 | ACCAATTTAGGTGTAATTTAA AACGCTTTGCTTTATCATTAA |
| 400523 | LOC400523 | XM_378582 | AACGCAGGATATTGCATCATA CTCGTGGGTCTTCATACCTAA |
| 400552 | LOC400552 | XM_378623 | CCAGAGTGTATTGCTTATAAA ACCGTGTTTGGCAGACATTAA |
| 400569 | MGC88387 | NM_001001683 | CGCGCTCTGGAAGACATTGAA CAGAGTGATAATGGCTACCTA |
| 400586 | LOC400586 | XM_375424 | CCCGCAGAATGGAGGAGATAA CTGGAGAAACATCATCCTTAT |
| 400591 | FLJ44815 | NM_207454 | CAGGATTTCATATATAGCAAA CAGGAGAATGTCAGCAGTGAA |
| 400619 | LOC400619 | XM_378703 | CTGGAGTAACTATTCCACTAA CTGAGTCTTCTCTTTCTTCAA |
| 400623 | LOC400623 | XM_378708 | CTGCGACATTTAGTCCTTTAA ACCAGTGTGTTTGAGCATTAA |
| 400624 | FLJ45079 | NM_001001685 | CAGGGTCTGAACTAAGAGCAA CTGGGTCTGCGTGAAGATAAA |
| 400642 | LOC400642 | XM_378735 | CACATCTTCCATATACTTTAA CCAGAGTTTCTTATCAAGTAA |
| 400651 | LOC400651 | XM_378747 | CAGGCTGAATTTGACGTTAAT AAGGACATACATGCAAATGAA |
| 400658 | FLJ44313 | NM_207460 | TCCGCGAGTCCTCAAATGTAA CAGGGCATTGTCTGTCTACAA |
| 400664 | LOC400664 | XM_375549 | CTGGTGATTCCAGTAGGTGA CGCCCACTAATTCTACATTAT |
| 400668 | PRSSL1 | NM_214710 | CACACCCATTCCACATGCAAA CGCCCTGATGCTGCCCGTGAA |
| 400673 | VMAC | XM_375560 | TTCGAAAGTGCTGCAGTTATA CTGTCTGTGCTCAAGAATAAA |
| 400679 | FLJ46230 | NM_207463 | CCCGGAAGGAAAGGAAAGGAA CTGGACTTTGGTCAAGTACTA |
| 400681 | LOC400681 | XM_375589 | GACCCTATATTTGGAATTTAA TAGATTAAATTGGCTAATTAA |
| 400684 | LOC400684 | XM_378793 | CCACTGTTTATTTCAGCATAA CACGAAATCCTCCCATCTCAA |
| 400685 | LOC400685 | XM_378795 | TAGAGTAATACTGGCCTTATA CTGCTTGTGGTGGAAACATAA |
| 400709 | SIGLECP16 | XM_375634 | CTCCGTGTGGCCTCCCTGGAA AAGGGCAGCATTGGGCATGGA |
| 400721 | LOC400721 | XM_375664 | CACACTGGTCACACCACCTTA GTGGGAAATCATTTAGTTCAA |
| 400729 | LOC400729 | XM_378824 | CGGCTCCTGTCTGACCAGGAA ATGATGTACTAGATCCCTTAA |
| 400764 | LOC400764 | XM_378880 | CAGGGAGCAGAAGACATTAAT AAGGGCTATGAAGAAACATAT |
| 400790 | LOC400790 | XM_375814 | CAGGAGCTCCTGCTACATAAA AAGGAACATATTTCCACATAA |
| 400800 | LOC400800 | XM_378925 | AAGGTACAAATTACAGATATA AAGGAAATGATTTAACTTCAA |
| 400842 | LOC400842 | XM_375914 | CCCATGAAGAATGAAGCACAA CCGAGCGATCGCAGAGACCTA TTCATACAGAATGGCACTAAA CTGGGATTATTGATTACAACA |
| 400846 | LOC400846 | XM_378981 | CACGTACTTTCTGAATCTAAA CACAAAGATGTGAGCAATAAA |
| 400850 | FLJ44790 | NM_001001691 | CCCGGCGTTCCTGGAAGCCAA CCCGCAGCAAGTTAAATCTAA |
| 400865 | LOC400865 | XM_379006 | AAGGCAAACCATAGCCATTAA AAGAATTTGCATCATAATTAA |
| 400876 | LOC400876 | XM_379023 | CAGCTCGGACAGCATCCATAA CTGTCTGAAATTGAATGTTTA |
| 400888 | LOC400888 | XM_375955 | CAGGCATCACCTTGCCTGGAA CCGGTGATTGATGATACTACA |
| 400890 | LOC400890 | XM_379036 | AACCAGAATAATCAGCCCTAA ACGCAGAGATGCACACATTAA |
| 400916 | C22orf16 | NM_213720 | CCCAGATAGCTGGGATTGGAA CACAGATAACCCAAGACACAA |
| 400921 | LOC400921 | XM_376001 | CTGGCTGACGTGCTCCATCCA CATTGTGGACTTGGACATTTA CTGACACTAAGCTCGGCTTCA GCCCTCTACTCAGCATGGATA |

Table4

| | | | Sequence |
|---|---|---|---|
| 400931 | FLJ27365 | NM_207477 | CTGGGTAAGTCTAGCCCAGAA CAGGTGATGCCTGAAGCTCAA |
| 400932 | FLJ46257 | NM_001001693 | ATGCATTCGTTTATCAATCAA CCGGGAAGTGTTCACAAATAT |
| 400934 | FLJ44385 | NM_207478 | AAGAAGGAAACTGGACACATA CAGGACAATTATTAAGTTCCA |
| 400937 | LOC400937 | XM_379068 | CAGGATCTACGTTATAATTAT CACAATTATGAAATAATGTAA |
| 400944 | LOC400944 | XM_379077 | CACCAACTCCTTGTATTGCAA CTGGGATGGAATCCTAATGAA |
| 400945 | LOC400945 | XM_379078 | CAGTTTATAATATGCATTCA CAGCAATTTAATATAATAATGCA |
| 400964 | LOC400964 | XM_379106 | CTGGGATTACACAGTCGGTAA CAGGTTAATGAAATAATTAA |
| 400997 | FLJ44006 | NM_001001696 | ATGGAGATATACAATTGTTAA AAGGTCTTAATGAGAATGAAA |
| 401025 | LOC401025 | XM_379149 | CCCATCCTGAGTGACTACTAA CCAGCATGTCTTTCCACTATA |
| 401034 | LOC401034 | XM_376179 | CCACCGGAGACTCAAGATGAA CACACTCACAGTTACCAGCTA |
| 401037 | LOC401037 | XM_379161 | CCAGTGCAGCTAGAAATGTAA TCCCTCAGAATTTACATCTAA |
| 401050 | LOC401050 | XM_379173 | TTAGATAATAATGGAACTGAA CCCAGTTTAGTTTATATATAA |
| 401054 | LOC401054 | XM_376207 | TTGGCTGGACACAATCCTGAA CAGTTGACCTTGGTGAACTA |
| 401056 | LOC401056 | XM_379180 | TAGGACTCTGGAGAAGATCAA TGGAAGCTCTTTGAAACCAA |
| 401058 | LOC401058 | XM_379182 | AAGAATACATAAGAAGTCAAA TACAATATATTCTTAATTTGA |
| 401059 | LOC401059 | XM_379184 | TACATTCTGAGTTGAAGGAAA TCCAAGTATGATTGGAAATTA TAGATATTTATTATTCCAGGTA ACGAAATTCATTGGCAGATTA |
| 401078 | LOC401078 | XM_379200 | CTGCGTGGTGGCAGTTGTTGA CGGGTTTGTAGTTACCTATAA |
| 401081 | LOC401081 | XM_379206 | AAGCAGTATTTGTATTAGAA CTGATAGAAGAAGACATTAAA |
| 401087 | LOC401087 | XM_376267 | AACAAGTACGAATTGCCAAA CTCCTTGTGTTTCATTGGCTA ACGAATTTGCCAAATGGACAA ACGGTCTATGTGGCACAAGA |
| 401089 | LOC401089 | XM_376269 | CACGTTCCAGATACACTTTAA TTGGATTTCTATCACCATAAA |
| 401097 | LOC401097 | XM_376281 | CGCCAACTGTACAACCTTAA GCGGGCTGCCTCTGACATTTA |
| 401101 | LOC401101 | XM_379234 | AACGATCTGCATGAAGATAA CAGCTGTGACTTGATAAACAA |
| 401103 | LOC401103 | XM_379235 | CAGGACCCTCGAATCCTATAA ATGTAGTAATATACTCAATAA |
| 401114 | FLJ35816 | NM_207489 | CTGACTTTACTCTGAGATCAA AAAGTAATGACCAAGATAAA |
| 401123 | FLJ45721 | NM_207490 | TAGGAAGTAATTACTAATAAT AACCATGTAGCTCATAAGTAA |
| 401149 | LOC401149 | XM_379273 | CCGGGACAACAGGCTCTCCAA CTGGCTGTGAATCCCAATGAA |
| 401165 | LOC401165 | XM_379299 | TAGGGTTAATGCTACAATGAA AAGGCAAAATTCTATCTTCAAA |
| 401172 | FLJ33360 | NM_001001702 | CAGGCTTTGCTTCTCTCCCAA AGGAACCTTACTGATAATTTA |
| 401176 | LOC401176 | XM_379318 | AACCAAGAGTTATAAAACTATA AACGTAAATATTATTACGTTA |
| 401180 | LOC401180 | XM_379325 | CACGTCCCTCCTTACTCCCGAA CAGAAACTAAACCGACCACCAA |
| 401206 | LOC401206 | XM_376420 | AACCAGCTGTACATTTGGAAA GACGAGAAGAAGGACGCTGGA |
| 401207 | LOC401207 | XM_376423 | AAGGGAGGAGACAGACAATAAA AAGATACAACTTAACTTTAAA |
| 401210 | LOC401210 | XM_379359 | CCGGTCCACTGTGGAAGGCAA CAGCCAGAAATCTCGCATGTAA |
| 401215 | LOC401215 | XM_379368 | AAGCTCAGGCTGTGAAATTAA AACGATAATTTGTGAAAGTAA |
| 401217 | FLJ40453 | NM_001007542 | AAGAGAATTAGGTGAAGAATTA AACAATCACTATAGTAAGATA |
| 401220 | LOC401220 | XM_379378 | CTGGTTGACCTTGCAAATTGA AAGCAGATGCTTGAGTAAGCTAA |
| 401224 | AACSL | XM_376454 | ATCAATCTATATTATCTTAAA AAGGTTAGTTGAGTAAATTAA |
| 401236 | LOC401236 | XM_376469 | CCAAATGGACTGAACTTTAA CAGGAGAACCATCACAAGCTA |
| 401254 | LOC401254 | XM_379406 | ATGCCTGAAGTATGGCATGTA AAGTGATGTCATTAAGCAGAA |
| 401262 | CRIP3 | NM_206922 | CCCATTTCATCTAGAAAGTAA TAGAGGTTTGTTAATCTAATA |
| 401278 | FLJ44955 | NM_207500 | CTGGTAGGTAAGATCATGTAA GACCATGATACTAACAATAAA |
| 401281 | FLJ27255 | NM_207501 | |
| 401308 | LOC401308 | XM_376576 | TGCCCAGTTTGAAGAATTGAA ATCCTCTATTGTTCTGACAAA |

Table4

| | | | | | | |
|---|---|---|---|---|---|---|
| 401328 | LOC401328 | XM_379485 | AACGTTTAACACAGACATTAA | AACCTAATAGTCTTAGCCAAA | | |
| 401400 | LOC401400 | XM_379518 | CTGGAATTCTACAGCCATGAT | CTGCTGGGAGAAAGAATGGAA | | |
| 401431 | LOC401431 | NM_001008745 | ATGACTTTGATTTCTGCATAA | CAGGGAATTATTCACATGGCA | | |
| 401470 | LOC401470 | XM_376787 | AAGGGCAAGGCCATTAAGAAA | CCGCCACCACGCGCAGCCTAT | | |
| 401472 | FLJ45248 | NM_207505 | AGGGTATTTCCTAGCAAGAAA | TAGGAAGAATTTCCTGACAAA | | |
| 401476 | LOC401476 | XM_379603 | AAGCAATTTGCTCAAGATAAT | ACCCATCAAGAGATTAAGTAA | | |
| 401477 | LOC401477 | XM_379605 | CAGGATGCTGTCACAATTACA | TCAGGAGTAATTGTTAACTAA | | |
| 401480 | LOC401480 | XM_379608 | CAGCTTTGGAGGTCCTAGGAAA | CAGAGTTTCCTTCCCAGCTAA | | |
| 401495 | LOC401495 | XM_379625 | TACCAGGAAACTTGAGACCAA | CACACCCTTACCAGACTTCAA | | |
| 401497 | LOC401497 | XM_376822 | CACAACAACAGTATAAACATAA | TTCAATAAAGTTATCAATAAA | | |
| 401499 | LOC401499 | XM_379628 | AACGGGAAAGCTCTTGGAACA | TTGAGCCAGTTTACAAAGAAA | | |
| 401508 | FLJ45202 | NM_207507 | CTCAAAGGCTGTCCACAAGAA | AACCCTGTCTGTCTCTACTAA | | |
| 401537 | LOC401537 | XM_376888 | CCAGATGAAACAGTACATCTA | CACTCACATCACAAATCTGAA | CACGGAGGCATCTTATGTTAA | CAGACTGGTTTGAAGGAAGAA |
| 401539 | LOC401539 | XM_379648 | AAGACGTAATAGAGATTTCAA | CTCGTAGTCAGTGACAAGTAA | | |
| 401550 | C9orf148 | XM_379667 | AAGAAGAATCCTAACCTCAAA | AAGGGTCGTGGTGGAAATCAA | | |
| 401584 | LOC401584 | XM_376986 | ACCAAGTACTCCAGATTAATA | CAGCTTGAACTGTTGCCTTAA | | |
| 401607 | LOC401607 | XM_379717 | ATTGATCAGCCAGCAGTCAAA | CACTCGCCAATTAGAGATTGA | | |
| 401679 | LOC401679 | XM_377185 | CTGCAAGGCGTGCCACCTGAA | CCGGTGCTCCTGCCTTGTGAA | | |
| 401704 | LOC401704 | XM_377231 | CTACTGGATGATGACAATGAA | AAGCAGTAGATATGAATAATA | | |
| 401713 | LOC401713 | XM_377259 | TGCCAATATGGTCAACAGGAA | TCACAAGACCTTCAATTTAAA | CCAGGCCGCTTTGGAAGCGAA | CCCTCGAGAGTTGGAAGTCAA |
| 401855 | LOC401855 | XM_497509 | CTGGCCTACAAGAACACATAA | AAAGTGGGTCACCAAATGCTA | | |
| 401876 | LOC401876 | XM_497534 | CATGGTATCAAGGGAAATATA | CCCACTTGAATTGCTCAACAA | | |
| 401891 | LOC401891 | XM_377488 | CTGCTCGTAATGGATCAACAA | CAGATTTAACTGCAATAGATA | | |
| 401895 | LOC401895 | XM_377500 | AACAGACCTTCCGCAACTTCA | CAGCAAGACGGCAGAGGTACA | | |
| 401896 | LOC401896 | XM_377511 | ACCAAGTTCAATGCTGATGAA | ACGCTCGGAAGGGTTGACATT | | |
| 401921 | LOC401921 | XM_497630 | CAGTCTTCGGTGGTCAAGAAA | CCCGGTCACTACACTATGAAA | | |
| 401943 | LOC401943 | XM_497659 | TGGGAGGACGAGAGAAGAGCCAA | AAGCCCTTGAGAATGAACTA | | |
| 401945 | LOC401945 | XM_377579 | ACCGGATATGCAGCTCTGAAA | CCGGAGAAATCTGCAGGTAAA | | |
| 401980 | LOC401980 | XM_497729 | CTGAGTGACGTTAATCACTCA | CGGGAAGCTGCTGGTGATCAA | | |
| 402011 | LOC402011 | XM_497758 | CACAATATTGGTGGTATCAAT | TACCATCAACTGTCCAACTA | | |
| 402036 | LOC402036 | XM_377696 | CACGGTGCTCTGGACCGTCTT | CCTCATCAACATTGACTTTCA | CTGGAGAATGACAGCCACACA | CACGGAGGTGAGAGAGAGCAA |
| 402037 | LOC402037 | XM_497793 | CTGGGCCTGCCTGGACCATAA | AAGGATGAGAGGAAGCAGAA | CAGACGCTGAGGATTCTTGTA | CCCGAAAGATGTGGGCATTAA |
| 402055 | LOC402055 | XM_377713 | CCAGGATTGAAGGAAGGGAAA | CAGGAGTTTGCTAGTGGGACA | | |
| 402057 | LOC402057 | XM_377716 | AAGAAGCTCCGCAACAAGATA | CACGCATCTGATGAAAATGGAT | | |
| 402072 | LOC402072 | XM_377741 | CTGGTACTTCTTATGACACTA | AACATGGTCTATAATGTGTAA | | |
| 402094 | LOC402094 | XM_377755 | CCGGGCATCTGCATTCCGGAA | CAGAAGGAAGAAAGTGCTA | | |
| 402095 | LOC402095 | XM_377756 | CTGTCTCAGGAGATTCTCAAA | CACCCAAGAGATTATCCTGAA | | |
| 402112 | LOC402112 | XM_496610 | AAGCCTGAGCCCATGGAGAAA | ACGGTTATGTATGTAGCAAGA | | |
| 402146 | LOC402146 | XM_497943 | GACAAGAAACTAGAGCAATAA | CCAGCATGTGGAATTCTTGAA | | |
| 402184 | LOC402184 | XM_497977 | CTCCTTGTGCCCACAGCCCA | AAGCCATTCCTCATGCCCAGAA | | |
| 402196 | LOC402196 | XM_497991 | CCGCTGCAGCCCAAGCATTTA | CTGCAGAAGAATCATGTTCTA | | |
| 402199 | LOC402199 | XM_377875 | CCGCTGCAGCCCAAGCATTTA | CTGCAGAAGAATCATGTTCTA | | |
| 402200 | LOC402200 | XM_377877 | TGCCATGGCTGGAATAGCCAA | CCGCTGCTGCGAGCCCAAGCATTTA | | |

Table4

| | | | Sequence |
|---|---|---|---|
| 402253 | LOC402253 | XM_377935 | TCGCCTCAGATCTGACCAGAA TCAGAGTGTTCAAATGTGTA |
| 402330 | LOC402330 | XM_378015 | GAACTACGATAAGTTCTCATA GAGAACTACGATAAGTTCTCA |
| 402353 | LOC402353 | XM_378043 | ACGGGACGGCTGCCCAATGAA CGCGGATAATCGAGACTGCAA |
| 402367 | LOC402367 | XM_378064 | CAGGCACATGCTCCTGCTTCA CCCAAGCATTCCGACAGGAA |
| 402388 | LOC402388 | XM_378103 | CAGATGTGCATTGGAGACTCT GACGTGGGCATTGGAGACTGA |
| 402397 | LOC402397 | XM_497102 | TCTGGAAGTATGTATCTACCA CAGGATTTGATGGTGCTAGTA |
| 402485 | LOC402485 | XM_380104 | CTCATTGACAAGATTAACATA AACGTTTAACACAGACATTAA |
| 402504 | LOC402504 | XM_380107 | CAGAGCTGGATCTACCATGAA AAAGATGACTGAAGAATGGCA |
| 402522 | LOC402522 | XM_379849 | CGCCGAGGATTTCAGAAGATA CAGGAGGGTTGGACACGGCAA |
| 402523 | LOC402523 | XM_380114 | AAGGGCATACATGGTAATTAT AACGTGCTAAGTATCAGTAAA |
| 402532 | LOC402532 | XM_380117 | CGGAGAAAGACTGGTCTTGAA CAGACTTGGTGTTATGGTAAA |
| 402563 | LOC402563 | XM_380128 | GCCGGGAGGTTGAGTAGAGAA CAGAAGGAGTGGAGACATTGA |
| 402604 | LOC402604 | XM_379939 | CAGGAATTCCTTATGTGTGAA ACGGTACTGAACAGACAATTA ACCAATGTTTACAAATATGTA CACCATGAAATATGTCTTCTA |
| 402634 | LOC402634 | XM_379987 | CAGAAGGTGGAGAGGTCATCA CCGGCGATGCTGCGTTGCCAA |
| 402641 | LOC402641 | XM_379995 | AAGACATGAGGGAATCATTAA ACAGCTGGATATAAACCTGAA CAGAAGAAGTCTTGTCATCAA TTCCAGGTTATTGATAAGAAA |
| 402669 | LOC402669 | XM_380011 | AGGCACCACCTCTGAAGACAA CAGGTGGTGTCTATGATGCAA |
| 402716 | LOC402716 | XM_380072 | AACACTGGTTATGGGAACAAA CAGGGTTTGGAGAACATTCAA |
| 403282 | OR6C65 | NM_001005518 | CCCGTGATTATGGGCCTCCAA CTCCTACACACTCATCCTTAA |
| 404033 | FLJ34077 | XM_495846 | AAGGGAAACAAGAGCATAAAT CAGCCTGATGATGCAGTAGAA |
| 404550 | MGC17624 | NM_206967 | CCCAGATGATGGAGAAATCGA CCGCAGTGAGCCCACCAAGAA |
| 404552 | SCGB1D4 | NM_206998 | AAGAAACGACTCTCATTGAAA CTCCAAGTTGCCAAACTTAAT |
| 404785 | POTE14 | NM_001005356 | CAGAAGCAACTTTCTGAAGAA CAGGAAGAAATTGTCATGCTA |
| 407835 | LOC407835 | NR_002144 | CAGGGAGATCAGCATCTGCAT CAGCCCGTGTCGACAGGGAA CTGCTGGACTATATTGTGAAA TTCCAGGAGTTTGTCAATAAA |
| 408263 | MGC27121 | NM_001001343 | AAACAAGATAATATTAAATAA CAGCCTTATATTACTCCATAA |
| 414153 | SNRPEL1 | XM_034623 | AACCTCATCTTCAGATACTTA TTGCAAGTGTGCTCTTTTGTGA |
| 414918 | MGC33692 | NM_001001794 | CCGCGACAAGGCGCTGCTCAA CACCATTGTCTCAGCAGTAAA |
| 415116 | PIM3 | NM_001001852 | CACCGCGACATTAAGGACGAA GAGCGTTTAATTTATTCAGTA CTGTCAGAAGATGAACATGTA ATGAACATGTATAGTGGCTAA |
| 439919 | LOC439919 | XM_498448 | TGGGTATTAATTCTCACTGTA CTGTATAAGTGAAGACATTAA |
| 439928 | LOC439928 | XM_498427 | AACCAGCAGAACAAATACAAA TTCTTACTTTGATGGGAACAA |
| 439952 | LOC439952 | XM_495799 | ATGCTGAAACATCGAAGTCCA CCGAGAAATCAACGACCTATA |
| 439957 | LOC439957 | XM_495805 | TACCCTTACCATCAATGCCAA CCGGGAGACAGCCAGACTCAA |
| 439959 | LOC439959 | XM_495806 | CACCAAGACTATCAATTTGAT AGGGAAATTCATCTATTTATA AAGATGCAGGATCATGGCCAA TAGGTATTCAATAGCATTAGA |
| 439982 | LOC439982 | XM_495828 | CAGAATCAGATGTTCCAGCTT ACGGGCTTATTGAGACCCTCA |
| 439985 | LOC439985 | XM_495832 | AAAGCGGATACATTGTCTATTA AAAGATAAGAAGAAGAAGTAA |
| 439994 | LOC439994 | XM_498491 | CAATGTCTTTCTTGTATTAAA TACAGATAGGATTACACTAAA |
| 440000 | LOC440000 | XM_498494 | CCGGCTGCTCGTCACCGCGAA GACAATTACTTGACTGGAAA |
| 440004 | LOC440004 | XM_498497 | ATGGTATTTCTGGTTATTAAA CCGGAGTTGGTTCTGACTTTAA |
| 440021 | KRTAP5-2 | NM_001004325 | CTCTCATGTCTGTAAATCAAA AATGAATTTCCTTGCAATAAA |
| 440042 | LOC440042 | XM_498516 | ATAGTAGATTCTATCAATCAA CAGGTGAACAGAAGTTCTCAA |
| 440050 | LOC440050 | XM_498520 | AGGGTCCATTCCTCACTATAA AGGAGAGTCCACCCTAATTAA |
| 440055 | LOC440055 | XM_495885 | CAAGATTCAGGTTCACCCGTA CAGGTTCACCCGTAACCCACT |
| 440058 | LOC440058 | XM_498524 | TTGGAAGAATTGTGTTATATA AAGGGTAGACTGAGTCTTGAA |
| 440066 | LOC440066 | XM_495897 | TAGGTCAAATCAGTCACTCA CACCTGCATTGTGCAAGGAAA |

Table4

| | | | |
|---|---|---|---|
| 440071 | LOC440071 | XM_498531 | CCGGAGAGGCCGAGTGATCTA CACAATTCTCTCTCTCCTTAA |
| 440077 | FLJ16353 | NM_001004328 | CAGCACATAGTTAGTGCTCCA TTGACAAATCAATATATTAAA |
| 440079 | LOC440079 | XM_498535 | TGCAAGGTACTTGACACGCAA AAGAGAGAATAGATAACTTCA |
| 440083 | LOC440083 | XM_498536 | AAGAAGATGAGAGTGATTCAA AAGGAGACAACAAGTCCCGAA |
| 440093 | LOC440093 | XM_495919 | ATGCATGTTTCTGTATGTTAA ACCAAGCAGACTGCTCGTAAA |
| 440098 | LOC440098 | XM_498543 | ATCGGGTAAAGGAGTCGATAA CTGGAGACAAGTGGACCCTAA |
| 440101 | LOC440101 | XM_498545 | CAGAAGTTTATTTCTCACTTA CACATAGATTACACTAAATAA |
| 440102 | LOC440102 | XM_498546 | GAGAATGTTGCTGCCACTCAA AAAGAAAGGAATATCATTATA |
| 440106 | LOC440106 | XM_495929 | AAGAGGGTCCATCGCCATCAT AGGGCTGGAAGTAGGCCTGAA |
| 440112 | LOC440112 | XM_498548 | AAGATGTTGTTTCAACCTGTA AAGCTTTAAGTTGCAAATGAA |
| 440134 | LOC440134 | XM_498562 | CCACTCCATAGTGAATGGTTA TTGATTGTCTTTCATCGGAAA |
| 440138 | LOC440138 | NM_001004127 | AACGGTCAACAGATACTAGAA CTCATTGTATGTGGAACTTTA |
| 440142 | LOC440142 | XM_495960 | ATCCAGTTCATCTCACTTCAA AAGCCTTTGAGAAGCATGAAA |
| 440151 | LOC440151 | XM_498568 | CACCAGCATTCCAGAGTCCAA CTGGAAATAAATAATTCATAA |
| 440153 | LOC440153 | XM_495967 | CAACATATGCACTGACTATAA CTCACTCTTTACTACAACATA CAGCAACTTCACAACCCAGTA CAGGCCTCAGATAGACGCCAA |
| 440155 | LOC440155 | XM_498569 | TACAAATGGAGACACAGGAAA CCGGCTGCTGCTTACACCTTT |
| 440157 | LOC440157 | XM_495969 | AAGGGAAGCCAGGCTGAGATA CAGAGTCTGCCTCCATGAGAA |
| 440186 | LOC440186 | XM_498579 | AGCCTCGGAAATTTCCTTTAA CCCGGAAAGCAGGGCATTCAA |
| 440190 | LOC440190 | XM_498580 | AAGGAACTTTGGGAAGATGAT CCGGGCCTCCTTGAAGGTTGA |
| 440191 | LOC440191 | XM_498582 | TAGCATAAGATGCTTCCTTAA TACTTAATAAATAAACATTTA |
| 440228 | LOC440228 | XM_496031 | CTCCTGGGTAGATGCATATAA CAGCCCAAGGACAGCCAATTA |
| 440232 | LOC440232 | XM_496036 | CTGTGCCTGGCCTGTATTGTA AAGCAGGTGGATGCAAATGAA |
| 440233 | LOC440233 | XM_496037 | CAAGAAGGCAAAGACAATAAA CACCTGGAAGCTGCCATCTAA |
| 440242 | LOC440242 | XM_496040 | TGCAGAGATCCAGGATACAAA ATACAACAGCTTCTTAGGTAA |
| 440243 | LOC440243 | XM_496041 | CACCTGGAAGCTGCCATCTAA GTGCATCCTCACGTAAGTGCA |
| 440245 | LOC440245 | XM_496042 | CCCAAACGAGGTGATCGACTT CTCCTTTCGGTGCATGAACAA |
| 440252 | LOC440252 | XM_496049 | AACAAGAAACAGAAAGCCAAA AGAGATGAGTATTCTGAACAT |
| 440254 | LOC440254 | XM_498602 | CAGCTGCTGCGGCGCCATGAT TTGCAAATAATGAGAAGCCGA |
| 440266 | LOC440266 | XM_496062 | CTGCAGCAAAGCACATACAGA GATGAGTGTGCTGAACATATA |
| 440274 | LOC440274 | XM_498608 | CTTGAGAATTATGTAGAATAA CAGAGGTCATCTTTAAGTAAA |
| 440277 | LOC440277 | XM_498610 | AACATTGGCCAGGGAAGTTAA CAGGATGGTGACTTGTCTTTA |
| 440284 | LOC440284 | XM_498614 | ACGGAAAGCCTTTATAATGTA CAGAATGTTATTAATCTTTAA |
| 440298 | LOC440298 | XM_496086 | CCGGGACCCTGCAGAAATGAA ACCCGGGATCCTCAAATAGAA |
| 440301 | LOC440301 | XM_498620 | ATAAATTCTCACACACACATA CTGCCCTGTTCTATAGCTATA |
| 440303 | DNM1DN11-13 | XM_498621 | TTGACCGTTGCTGAAACTTGT CTGAGCATTCATCTGAACTAT |
| 440307 | LOC440307 | XM_496092 | ACGCAGATCACTGGCCATCAA ATCGAGGACATCATCATCAAA |
| 440317 | LOC440317 | XM_496097 | AACCCTAGTGTTGGTACAGCA CAGAGCGTTTCAGGCAATGAA |
| 440329 | LOC440329 | XM_498628 | TCCCAAGTCAAAGACACTCCA CGGCTTGAAGGAACAGGGCTA |
| 440334 | LOC440334 | XM_498631 | TTGTAACTACTTGAAGTTAAA ACGGTCTTTCTTTGCCAACTA |
| 440344 | LOC440344 | XM_496123 | CACGGGAGGTGACATGAGCAA GAAGGAGAAATTCCAAAGAAA CAAAGAAATGTAGTTTATTTA CTGGCTGTGAAGGACCCTTTA |
| 440361 | LOC440361 | XM_496145 | AGGGAGCAGTGAGGACACCAA CACAACGATGTGCTTAGTAAA |
| 440370 | LOC440370 | XM_496158 | CTCCTTGTATCTTCAAATGAA ATGGTATACTTTATTAAGAAA |
| 440372 | LOC440372 | XM_496161 | GCCGATGGAGATGCCTATGAA CACGGAGAATGACCAGCTCAA ATGGACAAGAAGAAAGAGTAA CGCGGGCGTCTAGGCAAGAAA |

Table4

| | | | |
|---|---|---|---|
| 440374 | LOC440374 | XM_370980 | ACCTATTTATGAATATACAAA AAACCATTAAACAGATTTCTA |
| 440380 | LOC440380 | XM_498645 | AACCCAAGAAAGTCAGAAA ATGGAGGAATGAGGAGTTCAA |
| 440387 | LOC440387 | XM_496169 | TACCACCACGCCCGCTGTGTA CTGCCTTAAGATTTCCCATTA |
| 440391 | LOC440391 | XM_498650 | ATGGTATTGGTGTAACACATGTA AAGGATCGCATGGCAGAACTA |
| 440414 | LOC440414 | XM_496194 | CGCGGATAGTGTGCAGGCATA CCTGGTGTTGTCAATCATGAA |
| 440426 | LOC440426 | XM_498662 | TCCCAAGTGGCTAAGACTTAA CAGGATTAGCTTGTGGTTTAT |
| 440432 | LOC440432 | XM_498666 | CCGGGCATACTTAGTATCTTT CTGGTTGGTAAAGAACATTAA |
| 440434 | LOC440434 | XM_496214 | GCCGCAGGCGTTATTCCTAAA CAGCCGAAAGAGCAACTCATA |
| 440435 | GPR158L1 | NM_001004334 | AACCAAGAAAATGGCCGCAAA CAGGTGTGTATAAATCTGACA ACAGAAGAATTCACTACTGAA CACCATCATACTCAAGCTTTA |
| 440447 | LOC440447 | XM_496231 | TCCCAGGGTCTCTACAGATTT AAGGAGTTGCAGGAAGCATTT |
| 440449 | LOC440449 | XM_498675 | CTAGATGAAGAGTTATCTTAA CTGGAGTTCCTTGACTAGGAA |
| 440455 | LOC440455 | XM_498678 | CCCGAGGCTCCACGTACTCAA CGCGCTTGTGCAGGGAACAGAA |
| 440461 | LOC440461 | XM_498680 | CTTGTGATGTGTGGAGATCAA CTCCCTGGTTATACTGTTATA |
| 440462 | LOC440462 | XM_498681 | CCGGGCCTTGTGAACCTGCAA CACCCTGGTCTTCTCCTGTAA |
| 440472 | LOC440472 | XM_496255 | ATGGCTATAACAACAAACTAA CAGACTCTTCTTAGAAGTCTA |
| 440484 | LOC440484 | XM_496268 | CAGGATCATCCATACCCTAAA CACTGTAACATAATCCATTAA |
| 440493 | LOC440493 | XM_498698 | GCGGCTCAGTTTCACTAACAA CCCGACTTTCCGTGCAATAAA |
| 440495 | LOC440495 | XM_498700 | GGCGACATATACTAACAACAA CTGGTGTTACATTATCAACAT |
| 440498 | LOC440498 | XM_498704 | ATGGTGCAAGCTGGCATTGAA CTGGTAGATTATCATGGAGTA |
| 440501 | LOC440501 | XM_498706 | CGGAGCAGAGTTCTCCCACGA CAGATTTCAGTGGTACTGCGA |
| 440508 | LOC440508 | XM_496285 | CAGGCTACTCCCCACTGGTTA CTCCATAAGCCTCAGAATGAA |
| 440526 | LOC440526 | XM_498718 | CAGCAACCACGCCACCATGAA CTGAACAGGGTTCTAACCAAA |
| 440536 | LOC440536 | XM_498721 | CAGGCATCACATCCAGACTCA AGCCTTTGTAGGAACGAGAAA |
| 440546 | LOC440546 | XM_496328 | AAGCAGAGAAATGCAAACTAA TTGCTTCAAATAGGGCCAGTA |
| 440551 | LOC440551 | XM_496331 | GAGGGAGATGCGCTCCCTGGA CCCGCCTCTCGTGTGTCTCTA |
| 440552 | LOC440552 | XM_496332 | CTCCAAATATCTCATCAACAA AACCAACACACTAACCATATA |
| 440554 | LOC440554 | XM_498731 | AAGCACAGTTTCCTATGACAA CACGTCACTCAGAAAGTTGAA |
| 440563 | LOC440563 | XM_496344 | TCGAAACACCAGCGCATATCA ACCAGCTTCATTTGATCAAGA |
| 440567 | LOC440567 | XM_496348 | GAGCTCTATGATGAGCATGTA CTGCTCGTGTTAAATCTAGAA |
| 440568 | LOC440568 | XM_496349 | AAGGTCTTAAGTGCAGCAGGA AAGCTCTTTGTTGGAGAAGGA |
| 440572 | LOC440572 | XM_498736 | CCGTTTAATATCAGAGAGAAA AACCATATTTATGAAAGTAAA |
| 440583 | LOC440583 | XM_498743 | CCGGGATGACCTAAGGAACAA CAGAGAGTGATGACTGCTTAA |
| 440586 | LOC440586 | XM_496361 | TCAGGTTATGTTGGAGCTAAA AAGCGAATTTATGATCTGAAA |
| 440587 | LOC440587 | XM_496362 | CAGGCAGTCATGTGGCCCGAA CCCAAACATCTTATTGATACT |
| 440596 | LOC440596 | XM_498746 | TTGGGTTTCCTTCAGTCTCAA GTGGGTGAAGTGGAAGAACAA |
| 440606 | LOC440606 | XM_496385 | CTGGAACATGTAGGCCTTGTA TAGCTACAGCATATTATTTAA |
| 440607 | LOC440607 | NM_001004340 | ACTGTGAAAGGCCTCCAGTTA CAGCAGGAATATCACAATACA |
| 440609 | LOC440609 | XM_498754 | AAGCCTTGTTAACTTGCTGAA CACATCGTGTTTGGGATCAAA |
| 440665 | LOC440665 | XM_498809 | CACCAAGAAGTTTCTGACAAT GAGAAGTGTTCCTCTTCAGTAA |
| 440667 | LOC440667 | XM_498810 | CAGCAGCTGTTGCCAAATGAA CACGCGGGTTCTGCCCTCAAA |
| 440678 | LOC440678 | XM_496401 | AAGGATGTCATCAAAGTAGTT TAGGAGATCTTTCACTTGTAA |
| 440683 | LOC440683 | XM_496404 | GAACATAAGTTTCTATTTCAA CAGAGGGCGCTTCAGGAAGCAA AGGAATGAACATAAGTTTCTA GTGCAATTCGTCAGAACTTAA |
| 440684 | LOC440684 | XM_496405 | CTCCTTGTCAGGAGCACCTAA AGGGATTATTACTCACTGCAA CTGCAACTTAGCCTAGCATTA TAGCATTAAGTTGCTTGACTA |

Table4

| | | | |
|---|---|---|---|
| 440692 | LOC440692 | XM_498820 | AAGCAGATCAGTGCTCAAGTA TAGGAGTTGTAGAGCAGAGAA |
| 440696 | LOC440696 | XM_496420 | AAGCCAATTGATGACATATAT AAGCGTAAAGCTTGAAATATA |
| 440705 | LOC440705 | XM_496427 | CACCTTCATGCTCAGCATTCA TCCGAATTTCTTGATCAAGAA |
| 440707 | LOC440707 | XM_498827 | CCGAGACTCAGACTTACCCAA CTGCATCTTCTCATCCCATAT |
| 440722 | LOC440722 | XM_498831 | AGGCATCACGTCCACATGCAA CAGTGTGGCATGGGCATGTAA |
| 440724 | LOC440724 | XM_498833 | AAGGGAAAGGGTGACCACATA CACTGTGAAGAATATATTAAA |
| 440725 | LOC440725 | XM_498834 | TTGCATCAAATGGGACCTTAA ACGAGACTTACAAATAATAAA |
| 440730 | TRIM67 | NM_001004342 | CTGGCTTTAATAGTGATTCAT TACCTAAGTTCTGGTATCTAA CCGGGAAGTGTACGTCGGTAA CACAACTATCTCAGGCCTTAA |
| 440747 | LOC440747 | XM_380170 | ATCCAATTCTCTACACCATAA ATGCAGAATCAACAACAACAA |
| 440752 | LOC440752 | XM_498844 | CTGCTTGAAGCTGGCATTTGA ATGGCTGGTTACTGTGCTAAA |
| 440757 | MGC44328 | NM_001004344 | ACAAGTGTTAATCCCAATAAA CAGGGAAGCACACAGTCACTAA |
| 440764 | LOC440764 | XM_496476 | CACGGTGCTCCTGGAGGCCTA CACGCTCACGGTCAAGTATGA |
| 440765 | LOC440765 | XM_498851 | CAGCAGTAACTTATTAAATCT CACCAGCCGAGTTGCTAGCTAA |
| 440766 | LOC440766 | XM_498852 | ACCCATGAACATGTACAATTA ATGGCTAATAATCACATGATA |
| 440771 | LOC440771 | XM_498856 | CCGGTAGCCAGTCAGAATTTA CACGGTCAAGACGGAAGCAAA |
| 440772 | LOC440772 | XM_498857 | AAGCAAATGGCTCATTTCCAA AACCTCTAAGATTTGAATGAA |
| 440773 | LOC440773 | XM_496481 | CAGGTAGATGATAAGGAGATT AAAGAAGAATCTGAAGTGAAA |
| 440786 | LOC440786 | XM_496488 | CTGGCGTTAAAGAGGTCCATA TAGAATATGGAGGCCCTGAAA |
| 440787 | LOC440787 | XM_496489 | TAGACTCAGAGAGATGGCAAA CAGAACAAACATAGTACGGCA |
| 440788 | LOC440788 | XM_496490 | ACGGAGCATTACATTCCCAGA CCCGGAATCAATGGGAATGAA |
| 440790 | LOC440790 | XM_498869 | AACATTCTTCATTATGCATAA CAGCAGGTGTTGCTTAGTGAA |
| 440793 | LOC440793 | XM_496494 | AAGGTCTTCTCAAATATTGTA CTGCAGCGGAGCCATGGCCCA |
| 440804 | LOC440804 | XM_036936 | AACAGAGATCCTGGATACCAA CTGGAGGAAGACAGCTTATTA |
| 440815 | LOC440815 | XM_498875 | GCAGATGGCGAGCATGGCAAA AAGGGAGACCAAAGTTGGAAA |
| 440824 | LOC440824 | XM_496523 | CAGATCCATGCTGATCCCGCA CAGGGCGCTGAGTACCATGTA |
| 440839 | LOC440839 | XM_496539 | TTGAGGGATTTCAGACTTCAA AAGTCTTATTTAAGAAATATA |
| 440843 | LOC440843 | XM_498885 | TTGAATTTGTGTTCAAAGTAA CTGGGAAGAAAGCAACACAAA |
| 440854 | CAPN14 | XM_496548 | CTGGAGTGACAGTTCAAGTAA CAGGATGAATTCTTCACCAAA |
| 440855 | LOC440855 | XM_498894 | CTGGACATATTTCTTCTGGAA CCGGAACAGAGATTTACTCAA |
| 440856 | LOC440856 | XM_498895 | ACGAACGTAATTTGTTTCTGA AAGGGAGAGAAGAGAGTTGAA |
| 440857 | LOC440857 | XM_498896 | TCCAGTCTGTTTACTATACAT GGGACTCAAAGTATTAGCTAA |
| 440859 | LOC440859 | XM_498898 | CCGGGAAGCCTGGCTCCGAAA CACATTACAGTTAATCTGTAA |
| 440866 | LOC440866 | XM_496551 | CAGAAAGTTTGTGAAGATCTT ATGGTCTGACTTTCACAGAAA CCGGTTCATCTAATACAGACA CAGATTTGTCAAGGTTTGAAA |
| 440867 | FLJ16124 | NM_001004345 | AAGGAAGGAATTTATCTACCA CACGAGAAATATTACGTTGTA |
| 440872 | LOC440872 | XM_496559 | AAAGAGATACGTATGTACTTA CAGTGGCTGTGAAGAATATAA |
| 440900 | LOC440900 | XM_498909 | ATCCGTCACACTAGATGCATA CCGGAGCCGTTTACAGTTTGA |
| 440923 | LOC440923 | XM_498916 | AACAGTGAATAAGCACCATAA TAGGAGGAAGGCCACAACTAA |
| 440925 | LOC440925 | XM_496609 | CAGAGGTTAGCCTAACTACAA ATAGGTTTAAGAGTTAATATA |
| 440927 | LOC440927 | XM_496612 | AAGATCAATGCCCTCATTAAA AAGAAAGTGGAAGCAAAGAAA |
| 440930 | LOC440930 | XM_498918 | CCGCTAGGTGTAGGACCGCTA TTGGCAGAATACTCAGGTCAA |
| 440937 | LOC440937 | XM_498922 | ACCTGAGAATTCAATGATGTA CCCAACCTACAGGAAGCATAA |
| 440940 | LOC440940 | XM_498923 | CAGGGCGACAGTGTAGGGCCA AGCCTGCGTCTCAGCAGCAAA |
| 440954 | LOC440954 | XM_498932 | CTCATTTACCTTCCACATAAA AAGGAACAGATGTACAGTAAA |

EP 2 295 543 A1

Table4

| | | | | |
|---|---|---|---|---|
| 440959 | LOC440959 | XM_498934 | CTGCACCAATTCGCTCATTTA CAGCCAGTTCATAGACACCAA | |
| 440960 | LOC440960 | XM_498935 | CGGGAGAATATGCAGCAATAA CCGGCTCAGGTATTTATTGAA | |
| 440965 | LOC440965 | XM_498938 | CGGCAGACACACCACGTTCCA TAGGTCTATCAGTGCTTTGGA | |
| 440970 | LOC440970 | XM_496650 | AAGGAAGAGAGTGATTATATA CAACATCTTATTGCTATTTAA | |
| 440971 | LOC440971 | XM_496651 | ATGGAGTAAACTACAGCTTAA TAGGATATATTTGTCCCTTAA | |
| 440978 | LOC440978 | XM_496659 | CAGGGTAATCACGGAATCCCA AAGGGAGAGATTGGTGATGTA | |
| 440990 | LOC440990 | XM_498952 | GAGGATGTATGTGATGAGTAA CTGCTGTAAACCAATGTTAAA | |
| 440992 | LOC440992 | XM_496668 | TTCGCTCTCGCCGCCGAGGAA AAGGAGGAGTTCGAATGCCTT | |
| 440995 | LOC440995 | XM_498955 | CCAGAACTGGCAGCCCTTAAA TAGCGTGCCGTGGCCATTGAA | |
| 440997 | LOC440997 | XM_498957 | AAGGTTCATAGTTGTGTTGTA TAGGCTATTTCAAGAAATTAA | |
| 440998 | LOC440998 | XM_498958 | TGCAGAATTCATTATAATAAA CAGGTGAGACTTCACCGTGAA | |
| 441022 | LOC441022 | XM_496700 | CACCCTATGGATGAACAGGAT CACAAGCATCTGATGAAGCAA | |
| 441023 | LOC441023 | XM_498970 | TCCCTCCATCCTTGCCCTAAA ACCATGAGTTGTCACATCTAA | |
| 441027 | LOC441027 | XM_496707 | CGGAATGACCTTACTTGGTAA TACCATTAAATTCAGCTGAAA | |
| 441030 | LOC441030 | XM_498973 | AAGAATCTTTACAAAGCTGAA AAGGATGAGAGTGGATGTCAA | |
| 441035 | LOC441035 | XM_496713 | CAACTTGGTAAGAACACATAA GTGAGAAATAATACTCATAAA | |
| 441042 | LOC441042 | XM_498981 | AAGGTTTGCTCTAACCTAGAA TAGCAGGAATTCTTATATCAA | |
| 441050 | LOC441050 | XM_496721 | CTGGAGTAAGAAATATTTCAT CTGGCTTAGTATGGACTTCAA | |
| 441053 | LOC441053 | XM_496723 | ACCATTTGCAATGAAGAGGAA AGGAAAGGAATTTGACATTAA | |
| 441059 | LOC441059 | XM_498991 | AACTCCCAATTTGGAAAGTAA CTGCTAAAGAATAAATGTAAA | |
| 441068 | LOC441068 | XM_496743 | CTACAAGAAATCGAAGAGAAA CACCAAGGAAATGAAAGTTCA | |
| 441069 | LOC441069 | XM_498996 | CGGGACCTGAACCGAGAGCAA CAGGGTTCCATCAGGAAGGAA | |
| 441071 | LOC441071 | XM_498997 | CAGCTTGAACAACTCAGCTAA CCCATGGATATTGAAAGGATA | |
| 441073 | LOC441073 | XM_496746 | AAGAGCTGAGACAGAAGTACA AAACTGGACAAAGACTGTGAA | |
| 441077 | LOC441077 | XM_496748 | AGGGTTCAACCTCCAACCTTA CCTGAGATCGATAAAGCTTAA | |
| 441093 | LOC441093 | XM_499007 | AAGATGGAGTTTGGAAGGATA CTGGAAGATTCTGTTCATGTA | |
| 441099 | LOC441099 | XM_496770 | AAGCCTCAGAGTATTTCGAAA AACATTTCACTTTACATCTAA | |
| 441113 | LOC441113 | XM_496782 | AACCACAGATCTCGTAGGAAA CACGATGTATTTATTTGATTT | |
| 441117 | LOC441117 | XM_496786 | GTGGAAGAATTCATATATGAA AAGGATCTTAGCTTTGAGAAA | |
| 441118 | LOC441118 | XM_496787 | CTGGAAGAGTAGAAACAGAGA CAGGTGGTGTTTGGGTGCATA | |
| 441128 | LOC441128 | XM_496793 | AAGCCCAAATAGAGAACAGTA AAGAGGCACCTAATTCCTTAA | |
| 441129 | LOC441129 | XM_499026 | CCCACTCACCGCACACCATCA CAGGGCGCCTCTAGTCCCTCA | |
| 441140 | FLJ45422 | NM_001004349 | TTGGGAGGTGATGTCATATAA CAGACGGTACATACACTCTAA | |
| 441141 | LOC441141 | XM_499031 | CAGAATGACTACCCATGCAAA AAGACAGGTACTGGAAGATAA | |
| 441157 | LOC441157 | XM_499037 | CCGGGATTCAGAATTGATCCA CTGCACGTCACGGCTACTCAA | |
| 441162 | LOC441162 | XM_499039 | ATGGGACATGCATATAAACTA TACCTAATACACTGCAATATA | |
| 441165 | LOC441165 | XM_496820 | CACCCTTAAACTACAAAGAGA AAAGAAGACATCACCCTTAAA CACCTGCTACTCAGAAAGCTA TTGGAAGAGATTCTTATTAAA |
| 441167 | LOC441167 | XM_499042 | CAGGCAAGCTTTCAACTATAA CTGAAGGTAATCAGAGATTAA | |
| 441171 | LOC441171 | XM_499044 | CCCAGAAACACTGTACTTTAT ATGATGAATCACCAACACTAA | |
| 441173 | FLJ39824 | XM_496827 | CAGGACCATTTGACTATTTCA TAAGTTATTTATACTCATTTA | |
| 441189 | LOC441189 | XM_496843 | CCGGGCCTCAGTTTCCCGGAA CCCGGAGGAGATCGCGCGCAA | |
| 441199 | LOC441199 | XM_496855 | AAGGATACTTGTTAACCTTAA TCCAGCTTTGTTGGTATTCAA | |
| 441209 | LOC441209 | XM_496859 | CTGGCTGTCAATTTATCAGTA AGGAAGATTGTTGGTGATGAA | |

Table4

| | | | | |
|---|---|---|---|---|
| 441215 | LOC441215 | XM_496862 | TTGCAAAGAGGAAGAAGATCAA | CAGGGTGTATGAGGAGAGAAA |
| 441220 | LOC441220 | XM_496867 | CAGCATGTCTTCAGCCAGGAA | CTGGTGGTTGTACTCAGATAA |
| 441221 | LOC441221 | XM_496868 | CTGAGAAATGGAATAATCGTA | CAGGATGAACGGCGCCAGGTA |
| 441235 | LOC441235 | XM_496880 | CAGGTCATATCCTGAGATCAA | CACTGTCCTGATTATAAATAA |
| 441243 | LOC441243 | XM_376622 | AGGCCAAAGCTTTGTATTTCA | ACAGAGTGGTGTGGAGAATAA |
| 441268 | LOC441268 | XM_496908 | TTGCTCCATACAGACCTCCCA | CTGCTGATTTCTTCACTTGCT |
| 441307 | LOC441307 | XM_499093 | AAGGATGTTTGGAGGCTTCAA | CCGGCCTTCGTTTCCATTCAA |
| 441309 | LOC441309 | XM_496942 | CTGAATGATATTTAAGCTGAA | GGCGACCTTGCTGCCAGGACAA |
| 441313 | LOC441313 | XM_496945 | GACGTTGTAGTTGGCGTGGTT | CTGGAATGCCTTTGTACACATAT |
| 441328 | LOC441328 | XM_496957 | ACCAAAGAGCGTGGAAGGAAA | TCCGGATCAGAAGGAGCAGGA |
| 441333 | LOC441333 | XM_496962 | TAGGGAAGGGTGGTTCTGCAA | CACCTTGGATCTAGGAGACAA |
| 441336 | LOC441336 | XM_499101 | CACCAGGACCTGAAATGTTAT | CCAGCTGGATATCACGGGAAA |
| 441377 | LOC441377 | XM_496991 | AAGGAACAACGGTCACACCAA | CCCACCTTGATTTAGACCTGT |
| 441386 | GD:PTPRD | XM_497007 | CCCAAGCTGTATGTGAAGCTA | AAGGACAAGGCCATTAAGAAA |
| 441395 | LOC441395 | XM_497014 | ACAGATCCTGGTCAGAGTAAA | CAGGTGCTGGTTATGCTTTAT |
| 441401 | LOC441401 | XM_499136 | CAGCCGGTCAAAGCAGCAGAA | ATGACAGATTATGAAATGAA |
| 441421 | LOC441421 | XM_497042 | AAGGCGTGCTGTCGCCTGCAA | AAGAGGTTTCTGGGTCATCTA |
| 441424 | LOC441424 | XM_497044 | AACACCCATAAGAATTCCTAT | CAGAAGGAACAAGCACACAATAT |
| 441432 | LOC441432 | XM_376852 | CAGGAAATATGATGCGAGGAA | CAGGGTGTACCATGGCATGAA |
| 441447 | LOC441447 | XM_497061 | AAGGAGGAATTTCAGGGTGAA | TAGCACGAATTTCCTATGACCA |
| 441450 | LOC441450 | XM_499151 | CTGGGTTCTGCTGACATGTGAA | TAGGACGAATTTCTTGCTCCA |
| 441454 | LOC441454 | XM_499153 | CAGCTCTGAAATCATTGCCAA | TTGGTTTATATGATCTAGTTA |
| 441455 | LOC441455 | XM_497068 | AACTGCTGTGGAAACCAAGAA | GTGCCTCTCCATGGAGATTCA |
| 441458 | LOC441458 | XM_499155 | CTAAGTGGAGTGGACCATAAA | ATGATAATAGCTTACCACAAA |
| 441460 | LOC441460 | XM_499156 | TGGGAACAATAAAGCACTTCA | CTGCACCTATTGTCACAGCAA |
| 441464 | LOC441464 | XM_499160 | CTGGGATTAAGACACAAACAA | CAGGGACAACTTGGATCTTCA |
| 441467 | LOC441467 | XM_499164 | CTGCTTGGCGGTAGACCTTAA | CAGGATCCTAACGAAGACCAA |
| 441468 | LOC441468 | XM_499165 | AAGCAAGGTCTCGCTGTGTTA | TAGGACTTTCTTTGCATTCAT |
| 441471 | LOC441471 | XM_351854 | AAGCGTCTTCGTTCTTGCTCAT | CTGCACCTCACTGGAGCGGAA |
| 441474 | LOC441474 | XM_499168 | CGCCGCCTGCTTCACTTCTTA | CCCAGGAGATAGAACCTTCTA |
| 441483 | LOC441483 | XM_497093 | GGCCCTGATAAGGCCACCCAA | CCACGCTGGGTTAAGTGACA |
| 441490 | LOC441490 | XM_497100 | ATGGAATCAGCGACCACACTA | TTTGACCATTGTTAACAATAA |
| 441491 | LOC441491 | XM_499175 | CCCAGTGGAGCACGTCCTCAA | CTCCAGAAACATGAGCACAAA |
| 441494 | LOC441494 | XM_499177 | CAGAGGCATGAAGCTAGAATA | CAGGTTCATCTCATTAGTGGA |
| 441515 | LOC441515 | XM_497146 | CTCACTAAAGCTCTAGTGCAA | CAGCACATTCAGTTAATGTTA |
| 441525 | SPANX-N4 | NM_001009613 | CTGGAGAATAACCAGCCTACA | CAGAGTTTGAAAGAGACAGAA |
| 441528 | LOC441528 | XM_497181 | CTGGCCCAGCATTTAAACTTA | CTGAATATTGTGCCATGAAAA |
| 441529 | LOC441529 | XM_497182 | CTGGAGGAATCACCCAGTGAA | CAGGCCACTCAGGATATCCAA |
| 441531 | LOC441531 | XM_497185 | AAGCCATTCCTCATACCAATA | AAAGTATAGTATATATTAATA |
| 441540 | LOC441540 | XM_351948 | CTGGCACCCTTCAGATCCTTT | CCTTACGGATTTCGCGGTTTA |
| 441542 | LOC441542 | XM_497201 | CAGCATTCTGACGCTGAAGTA | CAGGACATATGAGACTTATTA |
| 441551 | LOC441551 | XM_497216 | TCGACCAATTGCCAACGTTA | CTGGGAATATGGCTCCTGAAA |
| 441560 | LOC441560 | XM_497230 | GCGGATGAGCACCAACCCTGA | CAGACGCCTTGTATCTCGCAA |

Table4

| 441561 | LOC441561 | XM_497231 | ATGGACCAAGTGGAAGGCAAA CAAGAGATTATATGACATGAA |
|---|---|---|---|
| 441569 | LOC441569 | XM_499192 | ATCAGAAGACATAGCCAGAAA CTGCAAGAAAGGCCTCAACAA |
| 441599 | LOC441599 | XM_497281 | ATGGGTGACTCTTAAAGGTTA CTGAATCTAGAGCTCATTAAA |
| 441631 | LOC441631 | XM_497334 | ATGGTCCATTATAAGACTGAA CAGCATGATGCCATTGCTGAA |
| 441634 | LOC441634 | XM_497338 | CTGGAGCTCACTCGCATTCAT CGGGAGTTGAAGGACTTGGAA |
| 441636 | LOC441636 | XM_497341 | CAGGAACAACTGAACCATCAA ATCCATATTGATGACAATAAA |
| 441663 | LOC441663 | XM_497381 | TAGCCGCAAACTAGACCCAAA |
| 441665 | LOC441665 | XM_497385 | CCGGGACCTGTGACCCACTCA CAGCTGAGACCTGTCCTGTGA |
| 441682 | LOC441682 | XM_497406 | CACCATCCAAGGTATCGTGTA CAGGCCTAGCATAGTCCTTTA |
| 441696 | LOC441696 | XM_499197 | CTCAGACACATGCCTCCGCGTAA CACAAAGAGTTTGTTACTGTT |
| 441706 | LOC441706 | XM_497431 | TGGGATGACAGATGCATTCAA CCAGGATATCTAATTCGCTAA |
| 441709 | LOC441709 | XM_497434 | TCTGGTATTTATTACTGAGAA CACCCTAAGTCTGCACTTCAA |
| 441740 | LOC441740 | XM_497477 | ACAGCTGTACATATTATACTA CAGGAAACTGCCAGAAAGTAA |
| 441754 | LOC441754 | XM_497493 | TTGGAACATCGGCAACACAAA CAGGATGAAGCTCATGCCTCA |
| 441755 | LOC441755 | XM_497494 | CAGGCAATGGAAGATGCATAT TAGATGAAACTTACAATATAA |
| 441759 | LOC441759 | XM_497498 | CTGGCCGAGTTGCACCCTTGA ACGGACGATGGAGGTCTGTGA |
| 441790 | LOC441790 | XM_497536 | TCCAAACAGATTCCTTGGGAA CAGCTTGTGATTGAGGACCTA |
| 441798 | LOC441798 | XM_497553 | TCCGCGGTCTTTACCCTGGAA CAGGAGCATGCAGGCCTACAA |
| 441816 | LOC441816 | XM_497584 | CAGCAGGTGTGGGAAATCCTA ATGGACTTGGTCAACATCAAA |
| 441821 | LOC441821 | XM_497592 | AAAGATTGCTCCAAAGATTAA CAGCAATATCCCATCCACAAA |
| 441826 | LOC441826 | XM_499202 | CTGGGAATTCTGGATGTTCCA CTGGGTATTCTGGGCATTCCA |
| 441827 | LOC441827 | XM_499203 | AAGCCTATCTAGATCTACTAA CAGCCTATCTAGACCTACTAA |
| 441833 | LOC441833 | XM_497603 | CTCTCGGACATTAGTCACCAA TCCCGAGAATGTGACATCTAA |
| 441842 | LOC441842 | XM_497614 | AAGCTTCTAACGGGTTCTCAA CTGAATGTGAGATAATTTATA |
| 441847 | LOC441847 | XM_497623 | ACCCACCTGTTAGGCATAAAA TCCAACCTCATTAAATATAAA |
| 441848 | LOC441848 | XM_497624 | CAGTTGAAGATCAATAAGAAA ATGCATCGAGTTCACACTAAA |
| 441866 | LOC441866 | XM_497645 | ACCCTGATTTGTCCTGATAAA TCCTGATAAAGAGAAGGCATA |
| 441874 | LOC441874 | XM_497654 | CACGTCAATTACCGCGAGGAA CAGTTCTGTATCAGTCTTGAA |
| 441898 | LOC441898 | XM_497698 | AAGCTCCTTCTCCTATTATTA TAGGCAGAATCCCGTGAAGAA |
| 441915 | LOC441915 | XM_497724 | CCAGTTACATCTGGTGATCAA AACGCTTATGGTATGAAGAAA |
| 441916 | LOC441916 | XM_497726 | ATCCAGAGTATGGAGAAGTAA CAAATCCAATATATTAAGAAA |
| 441922 | LOC441922 | XM_497733 | CAGGAGAGACAGGACCCATTA CAGGAGAGACAGGACCTATTA |
| 441925 | LOC441925 | XM_497740 | CAAGTTCATGCTTATAAATT CAGGGACTACAGTGCCTTGAA |
| 441928 | LOC441928 | XM_497743 | TGGGAAATATGAGGTCATGAA CTGGAGGTACCTGCAGCGCGAA |
| 441955 | LOC441955 | XM_497773 | AGGGATGAGGTAGCAGAGAAA AAGGCTATGAGAATACACCAA |
| 441959 | LOC441959 | XM_497777 | CAGACCATGACAACAACATCA TGAGCAGATCGCGGAGATCAA |
| 441994 | LOC441994 | XM_497814 | TCGACAGACCCTGGAGGTGTA CAGCACCATATCCGACATCAT |
| 442008 | LOC442008 | XM_497828 | GACGCAAGAACTTAGCAAA CAGAATCAAATGATCCTGCAA |
| 442011 | LOC442011 | XM_497831 | AAGCTTATTGATGGCATTCAT ACGGCTTCATTAACAGTGAA |
| 442016 | LOC442016 | XM_497838 | CAGATAGATCTGTTTCAAGAA AAGGGAAATGCTTGATGGTTA |
| 442020 | LOC442020 | XM_497844 | CAGCATCATGAGCCAGTTTCA CAGGCTGACTCGTACGCAAA |
| 442022 | LOC442022 | XM_497846 | CGAGCAACTCTCTGGGAACTA TGAGGTGGTGTCCTTCCGTAA |
| 442044 | LOC442044 | XM_497872 | CCAGGTGTCAAGGACAGCAAA CAGCTCCATTGTCTCCCATAA |

Table4

| | | | |
|---|---|---|---|
| 442081 | LOC442081 | XM_497923 | CCGCCAGACATTCCAATTGGA CACGGGCTACATGACCTGTGA |
| 442093 | LOC442093 | XM_497939 | ATCGTCATTGTTAATGAGCTA AAGGCTGATTACGAGAGCAAA |
| 442099 | LOC442099 | XM_497951 | TCGGACAGACCTCCACTTCAA AACCCTCAGCTTCCATTTAAA |
| 442108 | LOC442108 | XM_497964 | AAGGGACATGTGGAAGTAATA CAGATTAGTCAAGAAGATGAA |
| 442119 | LOC442119 | XM_497984 | CAGGATTTGAAGGAAGCCACA AACGACATCTTTGGCACCAAA |
| 442121 | LOC442121 | XM_497986 | CAGAATAGCTTTGCACATACA CAGCTGGACTTTAAGAATGTA |
| 442124 | LOC442124 | XM_497989 | CCCGCCTTCGTTATCATTTGT CACCTTCTACCTCTTATTCCA |
| 442140 | LOC442140 | XM_498021 | CTCATGGCTCTTGGAAGGAAA CACCCTGAAGAAACCATCAA |
| 442141 | LOC442141 | XM_498022 | CTCAAGGAGCCTCATATGGAA CCGGAGCATGGCAGAAACTCCA |
| 442145 | LOC442145 | XM_498027 | CCGGGTCCTAAACTCTGGATA TAGGCTCTGGGTATAAGCTTT |
| 442158 | LOC442158 | XM_498040 | TGCCAGCAAAGTCCATGTAAA AGGAACAATAATTGTGCCAAA |
| 442174 | LOC442174 | XM_498056 | CTGGCTCTGTGAATTCGAATA CTGGTGGGAGTGAGAATTGAA |
| 442208 | LOC442208 | XM_498091 | GCGGATCAGGTTCAAAGTGAT CAGGACCAACATCCTCTCCAT |
| 442209 | LOC442209 | XM_498092 | AACAATAAAGGCGAGATGAAA CAGATCAAGGATCAAGGGAT |
| 442228 | LOC442228 | XM_498111 | ACGGAAGTACTTGGCATATAA CACCATGTAGTTGTAATTCAA |
| 442243 | LOC442243 | XM_498127 | CTGACACCTCAGGGTCAGAAA CAGAGGCACCTATACCACCAA |
| 442272 | LOC442272 | XM_498157 | AAGCAGAAGGACACACAGATGTA CCGAACCAGATGAAACCCAAA |
| 442339 | LOC442339 | XM_498230 | GTGACTTTGAAGATAGCTTCA CCGGTGAGTGGCAAACAACGA |
| 442350 | LOC442350 | XM_498243 | CAGAAACAGGGAAATCCTGAA CGGAATGGAAATACTTTCTAA |
| 442375 | LOC442375 | XM_498266 | ATGGCTTTGCAAAGGAGCCAA TGGCATCATCTCAGGAAGAAA |
| 442397 | LOC442397 | XM_498297 | CCGTAACAGATTTCCATGAA CAGGTAGTAGTGACACATATT |
| 442398 | LOC442398 | XM_498298 | GTGCGAGATGAGAAATCTCAA CCGGGACAACTGCACCAGTGA |
| 442400 | LOC442400 | XM_498299 | AACGACAACATGAAGTTCGAA CAGGACGCTTTAGCACTCGA |
| 442401 | LOC442401 | XM_378044 | CCGCCTCTTGGCACCTTAA CAGCGAGATGACCAAGACGAA |
| 442410 | LOC442410 | XM_498312 | CGGTGTGAGCATGGCAGAGAA CAGGGCCATTCCATTGTCAT |
| 442429 | LOC442429 | XM_498335 | CTGGCCAAGAAGCCATTCACA CAGAGCCTGCGCAGTGTGGTA |
| 442451 | LOC442451 | XM_498366 | CATGATGAACACAGTGTGAAA CAGCTGAAAGTCAGGATAGTA |
| 442454 | LOC442454 | XM_498369 | TGCATTGGACCTGACCATGAA TACCTTGAACTGTATGTGCAA |
| 442481 | LOC442481 | XM_498410 | CAGGATGGAGGCTACGAAGGT CCGACCCACAGGCTCCAGTAA |
| 442524 | LOC442524 | XM_499272 | CAGACTTGAGGGCCCAGACAA CTCGAAGATGCCAGTATTTCA |
| 442535 | LOC442535 | XM_499279 | TACTATGACGTCTCCAACTCA ACTGTTTGTCATAGTGCCGTA |
| 442562 | LOC442562 | XM_499306 | CAGTCACAATGAGTAATTTAA CCCGTCGAACACAGCCACCTA |
| 442573 | LOC442573 | XM_499315 | ATCGGGTATTGGCAGCGTGGCAAA AGGGTGGAGCACAACGTCAA |
| 442578 | LOC442578 | XM_499318 | TGGAATTGGCAGCGTGGCAAA TCCACCTTACATGCAGTTGTA |
| 442606 | LOC442606 | XM_499352 | GAACAACATCATGGAGTATTA CCGTGCCTTCTTCCACTACAA |
| 442608 | LOC442608 | XM_499354 | CTTCCACTTCCTGGCCAGGAA TGCAGTCCAGGAGGATTTGAA |
| 442616 | LOC442616 | XM_499536 | TTGGACATGCATATTCCCAAA CCCGGCCGGTTATTCCGTTTAA |
| 442622 | LOC442622 | XM_499365 | CAAGGAGAAACTGGACTAATA CTGGAGGGCCTTTGACTTCAA |
| 442625 | LOC442625 | XM_499368 | CAGTTACATGTTCTCATTTCA CTCAAGCGGTACTACACATAA |
| 442696 | LOC442696 | XM_499441 | AAGCTCCACAATTGCCAGGAA AGCTGTGACCTTAATAATTTA |
| 442699 | LOC442699 | XM_499444 | AAGAATTTGACAAGAAGGATA CTGCCTTGTCAAGGCCATTAA |
| 442708 | LOC442708 | XM_499454 | ACAGATCACATTGCATGCAAA TAGGAACAATGTGGCCTTCAA |
| 442709 | LOC442709 | XM_499455 | | |

Table4

442711    LOC442711       XM_499457    CAGGAACATCAGCTTAACCAT CTGGTGCATTTAGGCCACCTA
442721    LOC442721       XM_499467    CACCACAGAAGTCAATTTGAA AAAGTCAAATAGAGAACATAA
445328    FLJ43692     NM_001003702    ATGTGTTTCATCAGCACTCAA CTGGATGATTACTATCTCTTT
445449    HBM          NM_001003938    TTGGTCTGTGCCTGTCAATAA CAGGACGAGTTCACCGTGCAA
474338    LOC474338       NR_002190    CAGAGAATTGCTGATAATCAT CTCATTATTCATTATTGTTTA AAGGAAGGTGAATATATTAAA CAGGCTTGTGGTGATAAATAA
474343    SPIN-2       NM_001006681    CAGCAGGGAGGCGGAGGGCTA CAAACTTGTGTGTACATGAAA
474344    GIMAP6       NM_001007224    AGCGATCAATTTCCCATTGAA CCCATTGAACTGAACATGCAA
494143    LOC494143    NM_001008708    CAGAACCTCAATTGCATATAA CTGGAAGACATTGCTGAACAA
497049    FLJ25758     NM_001011541    CAGGCTGTTCCTAGTCATGGA CACCCTCGAATTCTTCTTGAA
503497    MS4A13       NM_001012417    CTGCAGTAACTCTAACAATAA CTGGAATTATAAGTACTTTAA

Europäisches
Patentamt
European
Patent Office
Office européen
des brevets

**PARTIAL EUROPEAN SEARCH REPORT**    Application Number

which under Rule 63 of the European Patent Convention EP 09 17 0077
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EMIKO NAKAJIMA ET AL:  "A Naturally Occurring Variant of Porcine Mx1 Associated with Increased Susceptibility to Influenza Virus In Vitro" BIOCHEMICAL GENETICS, KLUWER ACADEMIC PUBLISHERS-PLENUM PUBLISHERS, NE, vol. 45, no. 1-2, 4 January 2007 (2007-01-04), pages 11-24, XP019477768 ISSN: 1573-4927 * the whole document * ----- | 1-6,8, 10-19, 21-23 | INV. C12N7/02 |
| X | STAEHELI P ET AL:  "Influenza virus-susceptible mice carry Mx Genes with a large deletion or a nonsense mutation." MOL. CELL. BIOL., vol. 8, no. 10, 1988, pages 4518-4523, XP002569906 * the whole document * ----- -/-- | 1-6,8, 10-19, 21-23 | |

TECHNICAL FIELDS
SEARCHED      (IPC)

C12N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 22 February 2010 | Galli, Ivo |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...................................................................................................

& : member of the same patent family, corresponding
document

EPO FORM 1503 03.82 (P04E07)

Europäisches Patentamt
European Patent Office
Office européen des brevets

**PARTIAL EUROPEAN SEARCH REPORT**

Application Number

EP 09 17 0077

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | HOFFMANN H.H., PALESE P. & SHAW M.L.: "Modulation of influenza virus replication by alteration of sodium ion transport and protein kinase C activity." ANTIVIRAL RES, vol. 80, 2008, pages 124-134, XP002569907 * the whole document * ----- | 1-2,4, 8-23 | |
| A | US 6 344 354 B1 (WEBSTER ROBERT G [US] ET AL) 5 February 2002 (2002-02-05) * the whole document * ----- | 1-23 | |
| T | KARLAS A. ET AL.: "Genome-wide RNAi screen identifies human host factors crucial for influenza virus replication." NATURE, vol. 463, 11 February 2010 (2010-02-11), pages 818-822, XP002569908 * the whole document * ----- | | TECHNICAL FIELDS SEARCHED (IPC) |

EPO FORM 1503 03.82 (P04C10)

Europäisches
Patentamt

European
Patent Office

Office européen
des brevets

**INCOMPLETE SEARCH**
**SHEET C**

Application Number

EP 09 17 0077

```
Claim(s) completely searchable:
      1-23

Claim(s) not searched:
      24-25

Reason for the limitation of the search:

Claims 24-25 concern a pharmaceutical composition comprising an activator
of MxA (claim 24), for instance an inhibitor of miR-141 etc (claim 26).
These are reach-through claims for which there is no support and no
disclosure (Art. 83/84).  In fact, the subject-matter is so undetermined
that a search could not be carried out.
```

**EP 2 295 543 A1**

## ANNEX TO THE EUROPEAN SEARCH REPORT
## ON EUROPEAN PATENT APPLICATION NO.

EP 09 17 0077

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2010

| Patent document cited in search report | Publication date | Patent family member(s) | Publication date |
|---|---|---|---|
| US 6344354 B1 | 05-02-2002 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0244321 A

**Non-patent literature cited in the description**

- **Bartel D.** *Cell,* 2009, vol. 136, 215-233
- **Köhler et al.** *Nature,* 1975, vol. 256, 495-497
- **Kozbor et al.** *J. Immunol. Methods,* 1985, vol. 81, 31-42
- **Cote et al.** *PNAS,* vol. 80, 2026-2030
- **Cole et al.** *Mol. Cell Biol.,* 1984, vol. 62, 109-120
- **Morrison et al.** *PNAS,* 1984, vol. 81, 6851-6855
- **Takeda et al.** *Nature,* 1985, vol. 314, 452-454
- **Orlandi et al.** *PNAS,* 1989, vol. 86, 3833-3837
- **Winter et al.** *Nature,* 1991, vol. 349, 293-299
- **Burton.** *PNAS,* 1991, vol. 88, 11120-11123
- **Huse et al.** *Science,* 1989, vol. 254, 1275-1281
- **T. D. Carroll et al.** *J. Immunol.,* 2008, vol. 180, 2385
- **K. Subbarao et al.** *Science,* 1998, vol. 279, 393
- **E. C. Claas et al.** *Lancet,* 1998, vol. 351, 472
- **C. Fraser et al.** *Science,* 2009
- **T. Wolff et al.** *Biol. Chem.,* 2008, vol. 389, 1299
- **H. Kato et al.** *Nature,* 2006, vol. 441, 101
- **B. Opitz et al.** *Cell Microbiol.,* 2007, vol. 9, 930
- **J. Lindenmann.** *Virology,* 1962, vol. 16, 203
- **O. Haller ; H. Arnheiter ; I. Gresser ; J. Lindenmann.** *J. Exp. Med.,* 1979, vol. 49, 601
- **T. M. Tumpey et al.** *J. Virol.,* 2007, vol. 81, 10818
- **O. Haller ; P. Staeheli ; G. Kochs.** *Biochimie,* 2007, vol. 89, 812
- **J. Pavlovic et al.** *J. Virol.,* 1995, vol. 69, 4506
- **B. G. Hale ; R. E. Randall ; J. Ortin ; D. Jackson.** *J. Gen. Virol.,* 2008, vol. 89, 359
- **E. Gottwein ; B. R. Cullen.** *Cell Host. Microbe,* 2008, vol. 3, 375
- **B. R. Cullen.** *Nature,* 2009, vol. 457, 421
- **C. L. Jopling ; M. Yi ; A. M. Lancaster ; S. M. Lemon ; P. Sarnow.** *Science,* 2005, vol. 309, 1577
- **A. Lutz ; J. Dyall ; P. D. Olivo ; A. Pekosz.** *J. Virol. Methods,* 2005, vol. 126, 13
- **P. A. Gregory et al.** *Nat. Cell Biol.,* 2008, vol. 10, 593
- **C. Llave ; Z. Xie ; K. D. Kasschau ; J. C. Carrington.** *Science,* 2002, vol. 297, 053
- **D. Holzinger et al.** *J. Virol.,* 2007, vol. 81, 7776
- **T. Ronni et al.** *J. Immunol.,* 1997, vol. 158, 2363
- **B. P. Lewis ; C. B. Burge ; D. P. Bartel.** *Cell,* 2005, vol. 120, 15
- **T. Ronni et al.** *J. Interferon Cytokine Res.,* 1998, vol. 18, 773
- **M. Selbach et al.** *Nature,* 2008, vol. 455, 58
- **D. Baek et al.** *Nature,* 2008, vol. 455, 64